(19) Europäisches Patentamt
European Patent Office
Office européen des brevets

(11) EP 3 260 541 B1

(12) # EUROPEAN PATENT SPECIFICATION

(45) Date of publication and mention of the grant of the patent:
**15.05.2019 Bulletin 2019/20**

(21) Application number: **17001061.5**

(22) Date of filing: **27.03.2013**

(51) Int Cl.:
***C12N 15/67*** *(2006.01)*

(54) **ARTIFICIAL NUCLEIC ACID MOLECULES FOR IMPROVED PROTEIN OR PEPTIDE EXPRESSION**

KÜNSTLICHE NUKLEINSÄUREMOLEKÜLE ZUR VERBESSERTEN PROTEIN- ODER PEPTIDEXPRESSION

MOLÉCULES D'ACIDE NUCLÉIQUE ARTIFICIELLES POUR UNE EXPRESSION PROTÉIQUE OU PEPTIDIQUE AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.03.2012 PCT/EP2012/001336**
**08.06.2012 PCT/EP2012/002447**

(43) Date of publication of application:
**27.12.2017 Bulletin 2017/52**

(62) Document number(s) of the earlier application(s) in accordance with Art. 76 EPC:
**13716727.6 / 2 831 241**

(73) Proprietor: **CureVac AG**
**72076 Tübingen (DE)**

(72) Inventor: **THESS, Andreas**
**72127 Kusterdingen (DE)**

(74) Representative: **Graf von Stosch, Andreas et al**
**Graf von Stosch**
**Patentanwaltsgesellschaft mbH**
**Prinzregentenstraße 22**
**80538 München (DE)**

(56) References cited:
**WO-A1-2012/019630**

- **LEDDA M ET AL: "Effect of 3'UTR length on the translational regulation of 5'-terminal oligopyrimidine mRNAs", GENE, ELSEVIER, AMSTERDAM, NL, vol. 344, 3 January 2005 (2005-01-03), pages 213-220, XP027872598, ISSN: 0378-1119 [retrieved on 2005-01-03]**
- **CHAKRABARTI PARTHA ET AL: "The mammalian target of rapamycin complex 1 regulates leptin biosynthesis in adipocytes at the level of translation: The role of the 5 '-untranslated region in the expression of leptin messenger ribonucleic acid", MOLECULAR ENDOCRINOLOGY, vol. 22, no. 10, October 2008 (2008-10), pages 2260-2267, XP002689369, ISSN: 0888-8809**
- **ZHU JIANFENG ET AL: "Binding of the La autoantigen to the 5' untranslated region of a chimeric human translation elongation factor 1A reporter mRNA inhibits translation in vitro", 31 October 2001 (2001-10-31), BIOCHIMICA ET BIOPHYSICA ACTA, VOL. 1521, NR. 1-3, PAGE(S) 19-29, XP002689370, ISSN: 0006-3002 * page 20, left-hand column, paragraph 3 - page 20, right-hand column, paragraph 1; figure 1 ***
- **V. IADEVAIA ET AL: "All translation elongation factors and the e, f, and h subunits of translation initiation factor 3 are encoded by 5'-terminal oligopyrimidine (TOP) mRNAs", RNA, vol. 14, no. 9, 1 January 2008 (2008-01-01), pages 1730-1736, XP055044697, ISSN: 1355-8382, DOI: 10.1261/rna.1037108**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 260 541 B1

- **SÁNCHEZ R ET AL: "The oligo(A) tail on histone mRNA plays an active role in translational silencing of histone mRNA during Xenopus oogenesis", MOLECULAR AND CELLULAR BIOLOGY, AMERICAN SOCIETY FOR MICROBIOLOGY, WASHINGTON, US, vol. 24, no. 6, 1 March 2004 (2004-03-01), pages 2513-2525, XP002616167, ISSN: 0270-7306, DOI: 10.1128/MCB.24.6.2513-2525.2004**
- **SHEN ET AL: "Structures required for poly(A) tail-independent translation overlap with, but are distinct from, cap-independent translation and RNA replication signals at the 3' end of Tobacco necrosis virus RNA", VIROLOGY, ACADEMIC PRESS,ORLANDO, US, vol. 358, no. 2, 25 January 2007 (2007-01-25), pages 448-458, XP005853995, ISSN: 0042-6822, DOI: 10.1016/J.VIROL.2006.08.054**

## Description

The invention relates to artificial nucleic acid molecules comprising a 5'UTR element as defined in the claims, an open reading frame, and optionally a histone stem-loop, a 3'UTR element, a poly(A) sequence and/or a polyadenylation signal. The invention relates further to a vector comprising a 5'UTR element as defined in the claims, an open reading frame and/or a cloning site, to a pharmaceutical composition comprising the artificial nucleic acid molecule or the vector, and to a kit comprising the artificial nucleic acid molecule, the vector and/or the pharmaceutical composition, preferably for use in the field of gene therapy and/or genetic vaccination.

Gene therapy and genetic vaccination belong to the most promising and quickly developing methods of modern medicine. They may provide highly specific and individual options for therapy of a large variety of diseases. Particularly, inherited genetic diseases but also autoimmune diseases, cancerous or tumour-related diseases as well as inflammatory diseases may be the subject of such treatment approaches. Also, it is envisaged to prevent (early) onset of such diseases by these approaches.

The main conceptual rational behind gene therapy is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to misregulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product. However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

Genetic vaccination allows to evoke a desired immune response to selected antigens, such as characteristic components of bacterial surfaces, viral particles, tumour antigens or the like. Generally, vaccination is one of the pivotal achievements of modern medicine. However, effective vaccines are currently available only for a smaller number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen *in vivo.* Genetic vaccines are expressed upon administration to a patient and uptake by competent cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *in vitro* transfection of such cells, and re-administration of the treated cells to the patient.

DNA as well as RNA may be used as nucleic acid molecules for administration in the context of gene therapy or genetic vaccination. DNA is known to be relatively stable and easy to handle. However, the use of DNA bears the risk of undesired insertion of the administered DNA-fragments into the patient's genome potentially resulting in loss of function of the impaired genes. As a further risk, the undesired generation of anti-DNA antibodies has emerged. Another drawback is the limited expression level of the encoded peptide or protein that is achievable upon DNA administration and its transcription/translation. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA for gene therapy or genetic vaccination, the risk of undesired genomic integration and generation of anti-DNA antibodies is minimized or avoided. However, RNA is considered to be a rather unstable molecular species which may readily be degraded by ubiquitous RNAses.

*In vivo*, RNA-degradation contributes to the regulation of the RNA half-life time. That effect was considered and proven to fine tune the regulation of eukaryotic gene expression (Friedel et al., Conserved principles of mammalian transcriptional regulation revealed by RNA half-life, Nucleic Acid Research, 2009, 1-12). Accordingly, each naturally occurring mRNA has its individual half-life depending on the gene from which the mRNA is derived. It contributes to the regulation of the expression level of this gene. Unstable RNAs are important to realize transient gene expression at distinct points in time. However, long-lived RNAs may be associated with accumulation of distinct proteins or continuous expression of genes. *In vivo*, the half life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that the product encoded by the RNA-sequence shall accumulate *in vivo.* On the other hand, the RNA has to maintain its structural and functional integrity when prepared for a suitable dosage form, in the course of its storage, and when administered. Thus, considerable attention was dedicated to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the translated region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising elements. For example, they may comprise so-called untranslated regions (UTR) at their 5'-end (5'UTR) and/or at their 3'-end (3'UTR) as well as other structural features, such as a 5'-cap structure or a 3'-poly(A) tail. Both, 5'UTR and 3'UTR are typically transcribed from the genomic DNA and are, thus, an element of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenine nucleotides added to the 3'-end of the transcribed mRNA. It may comprise up to about 400 adenine nucleotides. It was found that the length of such a 3'-poly(A) tail is a potentially critical element for the stability of the individual mRNA.

Nearly all eukaryotic mRNAs end with such a poly(A) sequence that is added to their 3' end by the ubiquitous cleavage/polyadenylation machinery. The presence of a poly(A) sequence at the 3' end is one of the most recognizable features of eukaryotic mRNAs. After cleavage, most pre-mRNAs, with the exception of replication-dependent histone transcripts, acquire a polyadenylated tail. In this context, 3' end processing is a nuclear co-transcriptional process that promotes transport of mRNAs from the nucleus to the cytoplasm and affects the stability and the translation of mRNAs. Formation of this 3' end occurs in a two step reaction directed by the cleavage/polyadenylation machinery and depends on the presence of two sequence elements in mRNA precursors (pre-mRNAs); a highly conserved hexanucleotide AAUAAA (polyadenylation signal) and a downstream G/U-rich sequence. In a first step, pre-mRNAs are cleaved between these two elements. In a second step tightly coupled to the first step the newly formed 3' end is extended by addition of a poly(A) sequence consisting of 200-250 adenylates which affects subsequently all aspects of mRNA metabolism, including mRNA export, stability and translation (Dominski, Z. and W. F. Marzluff (2007), Gene 396(2): 373-90.).

The only known exception to this rule are the replication-dependent histone mRNAs which terminate with a histone stem-loop instead of a poly(A) sequence. Exemplary histone stem-loop sequences are described in Lopez *et al.* (Dávila Lopez, M., & Samuelsson, T. (2008), RNA (New York, N.Y.), 14(1), 1-10. doi:10.1261/rna.782308.).

The stem-loops in histone pre-mRNAs are typically followed by a purine-rich sequence known as the histone downstream element (HDE). These pre-mRNAs are processed in the nucleus by a single endonucleolytic cleavage approximately 5 nucleotides downstream of the stem-loop, catalyzed by the U7 snRNP through base pairing of the U7 snRNA with the HDE.

Due to the requirement to package newly synthesized DNA into chromatin, histone synthesis is regulated in concert with the cell cycle. Increased synthesis of histone proteins during S phase is achieved by transcriptional activation

of histone genes as well as posttranscriptional regulation of histone mRNA levels. It could be shown that the histone stem-loop is essential for all posttranscriptional steps of histone expression regulation. It is necessary for efficient processing, export of the mRNA into the cytoplasm, loading onto polyribosomes, and regulation of mRNA stability.

In the above context, a 32 kDa protein was identified, which is associated with the histone stem-loop at the 3'-end of the histone messages in both the nucleus and the cytoplasm. The expression level of this stem-loop binding protein (SLBP) is cell-cycle regulated and is highest during S-phase when histone mRNA levels are increased. SLBP is necessary for efficient 3'-end processing of histone pre-mRNA by the U7 snRNP. After completion of processing, SLBP remains associated with the stem-loop at the end of mature histone mRNAs and stimulates their translation into histone proteins in the cytoplasm. (Dominski, Z. and W. F. Marzluff (2007), Gene 396(2): 373-90). Interestingly, the RNA binding domain of SLBP is conserved throughout metazoa and protozoa (Dávila López, M., & Samuelsson, T. (2008), RNA (New York, N.Y.), 14(1), 1-10. doi:10.1261/rna.782308) and it could be shown that its binding to the histone stem-loop sequence is dependent on the stem-loop structure and that the minimum binding site contains at least 3 nucleotides 5' and 2 nucleotides 3' of the stem-loop (Pandey, N. B., et al. (1994), Molecular and Cellular Biology, 14(3), 1709-1720 and Williams, A. S., & Marzluff, W. F., (1995), Nucleic Acids Research, 23(4), 654-662.).

Even though histone genes are generally classified as either "replication-dependent", giving rise to mRNA ending in a histone stem-loop, or "replacement-type", giving rise to mRNA bearing a poly(A)-tail instead, naturally occurring mRNAs containing both a histone stem-loop and poly(A) or oligo(A) 3' thereof have been identified in some very rare cases. Sanchez *et al.* examined the effect of naturally occurring oligo(A) tails appended 3' of the histone stem-loop of histone mRNA during Xenopus oogenesis using Luciferase as a reporter protein and found that the oligo(A) tail is an active part of the translation repression mechanism that silences histone mRNA during oogenesis and its removal is part of the mechanism that activates translation of histone mRNAs (Sanchez, R. and W. F. Marzluff (2004), Mol Cell Biol 24(6): 2513-25).

Furthermore, the requirements for regulation of replication dependent histones at the level of pre-mRNA processing and mRNA stability have been investigated using artificial constructs coding for the marker protein alpha globin, taking advantage of the fact that the globin gene contains introns as opposed to the intron-less histone genes. For this purpose constructs were generated in which the alpha globin coding sequence was followed by a histone stem-loop signal (histone stem-loop followed by the histone downstream element) and a polyadenylation signal (Whitelaw, E., et al. (1986). Nucleic Acids Research, 14(17), 7059-7070; Pandey, N. B., & Marzluff, W. F. (1987). Molecular and Cellular Biology, 7(12), 4557-4559; Pandey, N. B., et al. (1990). Nucleic Acids Research, 18(11), 3161-3170).

Also, it was shown that the 3'UTR of $\alpha$-globin mRNA may be an important factor for the well-known stability of $\alpha$-globin mRNA (Rodgers et al., Regulated $\alpha$-globin mRNA decay is a cytoplasmic event proceeding through 3'-to-5' exosome-dependent decapping, RNA, 8, pp. 1526-1537, 2002). The 3'UTR of $\alpha$-globin mRNA is obviously involved in the formation of a specific ribonucleoprotein-complex, the $\alpha$-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

Irrespective of factors influencing mRNA stability, effective translation of the administered nucleic acid molecules by the target cells or tissue is crucial for any approach using nucleic acid molecules for gene therapy or genetic vaccination. Along with the regulation of stability, also translation of the majority of mRNAs is regulated by structural features like UTRs, 5'-cap and 3'-poly(A) tail. In this context, it has been reported that the length of the poly(A) tail may play an important role for translational efficiency as well. Stabilizing 3'-elements, however, may also have an attenuating effect on translation.

Further regulative elements, which may have an influence on expression levels, may be found in the 5'UTR. For example, it has been reported that the 5'UTR of leptin mRNA is involved in the regulation of its translation (Chakrabarti P. et al., The Mammalian Target of Rapamycin Complex 1 Regulates Leptin Biosynthesis in Adipocytes at the Level of Translation: The Role of the 5'-Untranslated Region in the Expression of Leptin Messenger Ribonucleic Acid; Molecular Endocrinology, 2008, 22(10):2260-2267). It was further reported that the synthesis of particular proteins, e.g. proteins belonging to the translational apparatus, may be regulated not only at the transcriptional but also at the translational level. For example, translation of proteins encoded by so called 'TOP-genes' may be downregulated by translational repression. Therein, the term 'TOP-gene' relates to a gene corresponding to an mRNA that is characterized by the presence of a TOP sequence at the 5'end and in most cases by a growth-associated translation regulation (Iadevaia et al., All translation elongation factors and the e, f, and h subunits of translation initiation factor 3 are encoded by 5'-terminal oligopyrimidine (TOP) mRNAs; RNA, 2008, 14:1730-1736). In this context, a TOP sequence - also called the '5'-terminal oligopyrimidine tract' - typically consists of a C residue at the cap site, followed by an uninterrupted sequence of up to 13 or even more pyrimidines (Avni et al., Vertebrate mRNAs with a 5'-terminal pyrimidine tract are Candidates for translational repression in quiescent cells: characterization of the translational cis-regulatory element, Molecular and Cellular Biology, 1994, p. 3822-3833). These TOP sequences are reported to be present in many mRNAs encoding components of the translational machinery and to be responsible for selective repression of the translation of these TOP containing mRNAs due to growth arrest (Meyuhas, et al., Translational Control of Ribosomal Protein mRNAs in Eukaryotes, Translational Control. Cold Spring Harbor Monograph Archive. Cold Spring Harbor Laboratory Press, 1996, p. 363-388). The mechanism of translational regulation of TOP mRNAs is also the subject of another study, which reported that TOP mRNA translation may be modulated through binding of the La autoantigen to the 5'-untranslated region of said mRNA (Zhu J., Binding of the La autoantigen to the 5' untranslated region of a chimeric human translation elongation factor 1A reporter mRNA inhibits translation in vitro; Biochemica et Biophysica Acta, 2001, 1521:19-29). The results obtained in a further study indicate that the length of a 3'-UTR may also play a role in the translational regulation of 5'TOP mRNAs (Ledda, M. et al., Effect of 3' UTR length on the translational regulation of 5'-terminal oligopyrimidine mRNAs; Gene, 2005, 344:213-220).

It is the object of the invention to provide nucleic acid molecules which may be suitable for application in gene therapy and/or genetic vaccination. Particularly, it is the object of the invention to provide artificial nucleic acid molecules, such as an mRNA species, which provide for increased protein production from said artificial nucleic acid molecules, preferably which exhibit increased translational efficiency. Another object of the present invention is to provide nucleic acid molecules coding for such a superior mRNA species which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject-matter.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naive antigen-specific T cells or different immune cells able to induce an antigen-specific immune response by antigen-presenting cells. This occurs in the lymphoid tissues and organs through which naïve T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized

by the immune system, preferably by the adaptive immune system, and is capable of triggering an antigen-specific immune response, e.g. by formation of antibodies and/or antigen-specific T cells as part of an adaptive immune response.

Typically, an antigen may be or may comprise a peptide or protein which may be presented by the MHC to T-cells.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term 'wild type' may be understood as a sequence occurring in nature. Further, the term 'artificial nucleic acid molecule' is not restricted to mean 'one single molecule' but is, typically, understood to comprise an ensemble of identical molecules. Accordingly, it may relate to a plurality of identical molecules contained in an aliquot.

Bicistronic RNA, multicistronic RNA: A bicistronic or multicistronic RNA is typically an RNA, preferably an mRNA, that typically may have two (bicistronic) or more (multicistronic) open reading frames (ORF). An open reading frame in this context is a sequence of codons that is translatable into a peptide or protein.

Carrier / polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may - for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein which is positively charged under physiological conditions, particularly under physiological conditions *in vivo.* A 'cationic peptide or protein' may contain at least one positively charged amino acid, or more than one positively charged amino acid, e.g. selected from Arg, His, Lys or Orn. Accordingly, 'polycationic' components are also within the scope exhibiting more than one positive charge under the conditions given.

5'-cap: A 5'-cap is an entity, typically a modified nucleotide entity, which generally 'caps' the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic-acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single stranded or double stranded. In the double stranded form, the nucleotides of the first

strand typically hybridize with the nucleotides of the second strand, e.g. by A/T-base-pairing and G/C-base-pairing.

Epitope: Epitopes (also called 'antigen determinant') can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an artificial nucleic acid molecule as defined herein, directly to the patient - by whatever administration route - or *in vitro* to isolated cells/tissues of the patient, which results in transfection of the patient's cells either *in vivo*/*ex vivo* or *in vitro;* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject, preferably a patient, or to isolated cells of a subject, preferably a patient, which usually results in transfection of the subject's cells either *in vivo* or *in vitro;* b) transcription and/or translation of the introduced nucleic acid molecule; and optionally c) re-administration of isolated, transfected cells to the subject, preferably the patient, if the nucleic acid has not been administered directly to the patient.

Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component which is able to induce an immune response is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers which are covalently linked to each other by phosphodiester-bonds of a sugar/phosphate-backbone. The

term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG or AUG), at its 5'-end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG or AUG) and which preferably terminates with a stop codon (e.g., TAA, TGA, or TAG or UAA, UAG, UGA, respectively). The open reading frame may be isolated or it may be incorporated in a longer nucleic acid sequence, for example in a vector or an mRNA. An open reading frame may also be termed 'protein coding region'.

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation.

Protein A protein typically comprises one or more peptides or polypeptides. A protein is typically folded into 3-dimensional form, which may be required for to protein to exert its biological function.

Poly(A) sequence: A poly(A) sequence, also called poly(A) tail or 3'-poly(A) tail, is typically understood to be a sequence of adenine nucleotides, e.g., of up to about 400 adenine nucleotides, e.g. from about 20 to about 400, preferably from about 50 to about 400, more preferably from about 50 to about 300, even more preferably from about 50 to about 250, most preferably from about 60 to about 250 adenine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed 'restriction enzyme recognition site', is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. *In vivo,* transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, a 5'UTR, an open reading frame, a 3'UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be

the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term 'transfection' refers to the introduction of nucleic acid molecules, such as DNA or RNA (e.g. mRNA) molecules, into cells, preferably into eukaryotic cells. In the context of the present invention, the term 'transfection' encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term 'vector' refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid which is suitable for storing, transporting, and/or administering a pharmaceutically active compound.

3'-untranslated region (3'UTR): A 3'UTR is typically the part of an mRNA which is located between the protein coding region (i.e. the open reading frame) and the poly(A) sequence of the mRNA. A 3'UTR of the mRNA is not translated into an amino acid sequence. The 3'UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the premature mRNA and optional endo- or exonuclease cleavages etc. In the context of the present invention, a 3'UTR corresponds to the sequence of a mature mRNA which is located 3' to the stop codon of the protein coding region, preferably immediately 3' to the stop codon of

the protein coding region, and which extends to the 5'-side of the poly(A) sequence, preferably to the nucleotide immediately 5' to the poly(A) sequence. The term "corresponds to" means that the 3'UTR sequence may be an RNA sequence, such as in the mRNA sequence used for defining the 3'UTR sequence, or a DNA sequence which corresponds to such RNA sequence. In the context of the present invention, the term "a 3'UTR of a gene", such as "a 3'UTR of an albumin gene", is the sequence which corresponds to the 3'UTR of the mature mRNA derived from this gene, i.e. the mRNA obtained by transcription of the gene and maturation of the pre-mature mRNA. The term "3'UTR of a gene" encompasses the DNA sequence and the RNA sequence of the 3'UTR.

5'-untranslated region (5'UTR): A 5'UTR is typically understood to be a particular section of messenger RNA (mRNA). It is located 5' of the open reading frame of the mRNA. Typically, the 5'UTR starts with the transcriptional start site and ends one nucleotide before the start codon of the open reading frame. The 5'UTR may comprise elements for controlling gene expression, also called regulatory elements. Such regulatory elements may be, for example, ribosomal binding sites or a 5'-Terminal Oligopyrimidine Tract. The 5'UTR may be posttranscriptionally modified, for example by addition of a 5'-cap. In the context of the present invention, a 5'UTR corresponds to the sequence of a mature mRNA which is located between the 5'cap and the start codon. Preferably, the 5'UTR corresponds to the sequence which extends from a nucleotide located 3' to the 5'-cap, preferably from the nucleotide located immediately 3' to the 5'cap, to a nucleotide located 5' to the start codon of the protein coding region, preferably to the nucleotide located immediately 5' to the start codon of the protein coding region. The nucleotide located immediately 3' to the 5'cap of a mature mRNA typically corresponds to the transcriptional start site. The term "corresponds to" means that the 5'UTR sequence may be an RNA sequence, such as in the mRNA sequence used for defining the 5'UTR sequence, or a DNA sequence which corresponds to such RNA sequence. In the context of the present invention, the term "a 5'UTR of a gene", such as "a 5'UTR of a TOP gene", is the sequence which corresponds to the 5'UTR of the mature mRNA derived from this gene, i.e. the mRNA obtained by transcription of the gene and maturation of the pre-mature mRNA. The term "5'UTR of a gene" encompasses the DNA sequence and the RNA sequence of the 5'UTR.

5'Terminal Oligopyrimidine Tract (TOP): The 5'terminal oligopyrimidine tract (TOP) is typically a stretch of pyrimidine nucleotides located at the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as 5' TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence which represents a 5'UTR or at the 5'end of a sequence which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule according to the present invention, the 5'UTR element of the artificial nucleic acid molecule according to the present invention, or the nucleic acid sequence which is derived from the 5'UTR of a TOP gene as described herein. In other words, a stretch of 3 or more pyrimidine nucleotides which is not located at the 5'-end of a 5'UTR or a 5'UTR element but anywhere within a 5'UTR or a 5'UTR element is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene, which preferably extends from the nucleotide located 3' to the 5'cap to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about

100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID NOs. 1-1363, 1435, 1461 and 1462.

In a first aspect, the present invention relates to an artificial nucleic acid molecule comprising:

a. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence; and

b. at least one open reading frame (ORF).
Preferably, the artificial nucleic acid molecule may further comprise:

c. at least one histone stem-loop.

Such an artificial nucleic acid molecule may be DNA or RNA. In case the artificial nucleic acid molecule is DNA it may be used for providing RNA, preferably an mRNA with a corresponding sequence as is described further below. The inventive artificial nucleic acid molecule is particularly useful in gene therapy and genetic vaccination because it may provide increased and/or prolonged protein production of the protein encoded by the open reading frame.

In this context, the term '5'UTR element' preferably refers to a nucleic acid sequence which represents a 5'UTR of an artificial nucleic acid sequence, such as an artificial mRNA, or which codes for a 5'UTR of an artificial nucleic acid molecule. Thus, preferably, a 5'UTR element may be the 5'UTR of an mRNA, preferably of an artificial mRNA, or it may be the transcription template for a 5'UTR of an mRNA. Thus, a 5'UTR element preferably is a nucleic acid sequence which corresponds to the 5'UTR of an mRNA, preferably to the 5'UTR of an artificial mRNA, such as an mRNA obtained by transcription of a genetically engineered vector construct. Preferably, a 5'UTR element in the sense of the present invention functions as a 5'UTR or codes for a nucleotide sequence that fulfils the function of a 5'UTR. The term '5'UTR element' furthermore refers to a fragment or part of a 5'UTR of an artificial nucleic acid sequence, such as an artificial mRNA, or which codes for a part or fragment of a 5'UTR of an artificial nucleic acid molecule. This means that the 5'UTR element in the sense of the present invention may be comprised in the 5'UTR of an artificial nucleic acid sequence, such as an artificial mRNA, or which codes for a 5'UTR of an artificial nucleic acid molecule.

The 5'UTR elements as defined herein by way of their SEQ ID NOs may comprise or consist of a nucleic acid sequence that is derived from the 5'UTR of a TOP gene or from a variant of the 5'UTR of a TOP gene.

The term 'a nucleic acid sequence which is derived from the 5'UTR of a TOP gene' preferably refers to a nucleic acid sequence which is based on the 5'UTR sequence of a TOP gene or on a fragment thereof. This term includes sequences corresponding to the entire 5'UTR sequence, i.e. the full length 5'UTR sequence of a TOP gene, and sequences corresponding to a fragment of the 5'UTR sequence of a TOP gene. Preferably, a fragment of a 5'UTR of a TOP gene consists of a continuous stretch of nucleotides corresponding to a continuous stretch of nucleotides in the full-length 5'UTR of a TOP gene, which represents at least 20%, preferably at least 30%, more preferably at least 40%, more preferably at least 50%, even more preferably at least 60%, even more preferably at least 70%, even more preferably at least 80%, and most preferably at least 90% of the full-length 5'UTR of a TOP gene. Fragments in the sense of the present invention, are functional fragments as described herein. A particularly preferred fragment of a 5'UTR of a TOP gene is a 5'UTR of a TOP gene lacking the 5'TOP motif. The term '5'UTR of a TOP gene' preferably refers to the 5'UTR of a naturally occurring TOP gene.

The terms 'variant of the 5'UTR of a TOP gene' and 'variant thereof' in the context of a 5'UTR of a TOP gene refers to a variant of the 5'UTR of a naturally occurring TOP gene, preferably to a variant of the 5'UTR of a vertebrate TOP gene, preferably to a variant of the 3'UTR of a mammalian TOP gene, more preferably to a variant of the 3'UTR of a human TOP gene. Such variant may be a modified 5'UTR of a TOP gene. For example, a variant 5'UTR may exhibit one or more nucleotide deletions, insertions, additions and/or substitutions compared to the naturally occurring 5'UTR from which the variant is derived. Preferably, a variant of a 5'UTR of a TOP gene is at least 95% identical to the naturally occurring 5'UTR the variant is derived from. Preferably, the variant is a functional variant as described herein.

The term "a nucleic acid sequence that is derived from a variant of the 5'UTR of a TOP gene" may refer to a nucleic acid sequence which is based on a variant of a 5'UTR sequence of a TOP gene or on a fragment thereof. This term includes sequences corresponding to the entire variant 5'UTR sequence, i.e. the full length variant 5'UTR sequence of a TOP gene, and sequences corresponding to a fragment of the variant 5'UTR sequence of a TOP gene. Preferably, a fragment of a variant of the 5'UTR of a TOP gene consists of a continuous stretch of nucleotides corresponding to a continuous stretch of nucleotides in the full-length variant 5'UTR of a TOP gene, which represents at least 20%, preferably

at least 30%, more preferably at least 40%, more preferably at least 50%, even more preferably at least 60%, even more preferably at least 70%, even more preferably at least 80%, and most preferably at least 90% of the full-length variant 5'UTR of a TOP gene. Such a fragment of a variant, in the sense of the present invention, is preferably a functional fragment as described herein.

Thus, 5'UTR elements of the artificial nucleic acid molecule as defined herein by way of their SEQ ID NOs may comprise or consist of a fragment of the 5'UTR of a TOP gene or of a fragment of a variant of the 5'UTR of a TOP gene or may comprise or consist of the entire 5'UTR of a TOP gene or may comprise or consist of a variant of the 5'UTR of a TOP gene.

The 5'UTR element is preferably suitable for increasing protein production from the artificial nucleic acid molecule.

Preferably, the at least one 5'UTR element is functionally linked to the ORF. This means preferably that the 5'UTR element is associated with the ORF such that it may exert a function, such as a protein production increasing function for the protein encoded by the ORF or a stabilizing function on the artificial nucleic acid molecule. Preferably, the 5'UTR element and the ORF are associated in 5'→3' direction. Thus, preferably, the artificial nucleic acid molecule comprises the structure 5'-5'UTR element-(optional)linker-ORF-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the 5'UTR element and the at least one open reading frame are heterologous. The term 'heterologous' in this context means that the open reading frame and the 5'UTR element are not occurring naturally (in nature) in this combination. Preferably, the 5'UTR element is derived from a different gene than the open reading frame. For example, the ORF may be derived from a different gene than the 5'UTR element, e.g. encoding a different protein or the same protein but of a different species etc. For example, the ORF does not encode the protein which is encoded by the gene from which the 5'UTR element is derived.

In a preferred embodiment, the 5'UTR element as defined herein by way of its SEQ ID NO, preferably the artificial nucleic acid molecule, does not comprise a complete TOP-motif or 5'TOP sequence. Thus, preferably, the 5'UTR element, preferably the artificial nucleic acid molecule, does not comprise the complete TOP-motif of the TOP gene from which the nucleic acid sequence of the 5'UTR element is derived. For example, the 5'UTR element or the artificial nucleic acid molecule according to the present invention may comprise 1, 2, 3, 4, 5, 6, 7, 8, 9, 10 or more pyrimidine residues of the TOP-motif or 5'TOP, preferably 1, 2, 3, 4, 5, 6, 7, 8, 9, 10 or more pyrimidine residues of the TOP-motif located at the 3'side of the TOP-motif or 5'TOP. For example, the 5'UTR element may comprise or consist of a nucleic acid sequence which starts at its 5'end with a pyrimidine residue that corresponds to residue 2, 3, 4, 5, 6, 7, 8, 9, 10 etc. of the TOP-motif or 5'TOP of the TOP gene from which the nucleic acid sequence of the 5'UTR element is derived.

It is particularly preferred that the 5'UTR element as defined herein by way of its SEQ ID NO, preferably the artificial nucleic acid molecule according to the present invention, does not comprise a TOP-motif or 5'TOP. For example, the nucleic acid sequence of the 5'UTR element which is derived from a 5'UTR of a TOP gene starts at its 5'-end with a nucleotide located at position 1, 2, 3, 4, 5, 6, 7, 8, 9 or 10 downstream of the 5'terminal oligopyrimidine tract (TOP) of the 5'UTR of a TOP gene. Position 1 downstream of the 5'terminal oligopyrimidine tract (TOP) is the first purine based nucleotide 3' of the TOP-motif or the 5'TOP. Accordingly, position 1 downstream of the 5'terminal oligopyrimidine tract is the first nucleotide following the 3'-end of the 5'terminal oligopyrimidine tract in 5'-3'-direction. Likewise, position 2 downstream of the 5'TOP is the second nucleotide following the end of the 5'terminal oligopyrimidine tract, position 3 the third nucleotide and so on.

Therefore, the 5'UTR element preferably starts 5, 10, 15, 20, 25, 30, 40 or 50 nucleotides downstream of the transcriptional start site of the 5'UTR of a TOP gene.

In some embodiments, the nucleic acid sequence of the 5'UTR element as defined herein by way of its SEQ ID NO is derived from a 5'UTR of a TOP gene and terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'UTR element does not comprise any part of the protein coding region. Thus, preferably, the only protein coding part of the inventive artificial nucleic acid molecule is provided by the open reading frame. However, the open reading frame is preferably derived - as said above - from a gene that is different to the gene the 5'UTR element is derived from.

It is particularly preferred that the 5'UTR elementas defined herein by way of its SEQ ID NO does not comprise a start codon, such as the nucleotide sequence A(U/T)G. Thus, preferably, the artificial nucleic acid molecule will not comprise any upstream AUGs (or upstream ATGs in case it is a DNA molecule). In other words, in some embodiments, it may be preferred that the AUG or ATG, respectively, of the open reading frame is the only start codon of the artificial nucleic acid molecule.

Additionally, it is preferred that the 5'UTR element does not comprise an open reading frame. Thus, preferably, the artificial nucleic acid molecules according to the invention will not comprise any upstream open reading frames.

The nucleic acid sequence of the 5'UTR element as defined in the claim may be derived from a eukaryotic TOP

gene, preferably a plant or animal TOP gene, more preferably a chordate TOP gene, even more preferably a vertebrate TOP gene, most preferably a mammalian TOP gene, such as a human or mouse TOP gene.

Artificial nucleic acid molecules according to the present invention comprise a 5'UTR element as defined herein by way of its SEQ ID NO, which may comprise or consist of a nucleic acid sequence which is derived from the 5'UTR of a TOP gene or which is derived from a variant of the 5'UTR of a TOP gene, wherein the TOP gene is a plant or animal TOP gene, more preferably a chordate TOP gene, even more preferably a vertebrate TOP gene, most preferably a mammalian TOP gene, such as a human or mouse TOP gene and which optionally does not comprise the nucleotide sequence A(U/T)G and optionally does not comprise an open reading frame; at least one open reading frame (ORF); and optionally at least one histone-stem loop; wherein optionally the 5'UTR element does not comprise a TOP motif and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) of the 5'UTR of a TOP gene and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene or mRNA it is derived from.

Furthermore, the artificial nucleic acid molecule according to the present invention may comprise more than one 5'UTR elements as described above. For example, the artificial nucleic acid molecule according to the present invention may comprise one, two, three, four or more 5'UTR elements, wherein the individual 5'UTR elements may be the same or they may be different. For example, the artificial nucleic acid molecule according to the present invention may comprise two essentially identical 5'UTR elements as described above.

Furthermore, in addition to the at least one 5'UTR element as defined in the claims, the artificial nucleic acid molecule may also comprise another 5'UTR element as described herein.

For example, the artificial nucleic acid molecule may comprise a further 5'UTR element, which comprises or consists of a nucleic acid sequence which is derived from a nucleic acid sequence selected from the group consisting of SEQ ID NOs. 1-1363, SEQ ID NO. 1435, SEQ ID NO. 1461 or SEQ ID NO. 1462, from the homologs of SEQ ID NOs. 1-1363, SEQ ID NO. 1435, SEQ ID NO. 1461 or SEQ ID NO. 1462, from a variant thereof, or a corresponding RNA sequence. The term "homologs of SEQ ID NOs. 1-1363, SEQ ID NO. 1435, SEQ ID NO. 1461 or SEQ ID NO. 1462, " refers to sequences of other species than Homo sapiens (human) or Mus musculus (mouse), which are homologous to the sequences according to SEQ ID NOs. 1-1363, SEQ ID NO. 1435, SEQ ID NO. 1461 or SEQ ID NO. 1462. For example, SEQ ID NO. 1 relates to a sequence comprising the 5'UTR of Homo sapiens alpha 2 macroglobulin (A2M). A homolog of SEQ ID NO. 1 in the context of the present invention is any such sequence derived from an alpha 2 macroglobulin (A2M) gene or mRNA of another species than Homo sapiens (human), such as any vertebrate, preferably any mammalian alpha 2 macroglobulin (A2M) gene other than the human alpha 2 macroglobulin (A2M) gene, such as a mouse, rat, rabbit, monkey etc. alpha 2 macroglobulin (A2M) gene.

In a preferred embodiment, the artificial nucleic acid molecule may comprise a further 5'UTR element, which comprises or consists of a nucleic acid sequence which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, from the homologs of SEQ ID NOs. 1-1363, SEQ ID NO. 1435, SEQ ID NO. 1461 or SEQ ID NO. 1462, from a variant thereof, or a corresponding RNA sequence. It is particularly preferred that the 5' UTR element is derived from a nucleic acid sequence extending from the nucleotide position immediately 3' to the 5'-TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, from the homologs of SEQ ID NOs. 1-1363, SEQ ID NO. 1435, SEQ ID NO. 1461 or SEQ ID NO. 1462, from a variant thereof, or a corresponding RNA sequence.

In a preferred embodiment, the artificial nucleic acid molecule may comprise a further 5'UTR element, which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence of a nucleic acid sequence, selected from SEQ ID NOs. 1-1363, SEQ ID NO. 1435, SEQ ID NO. 1461 or SEQ ID NO. 1462, or a corresponding RNA sequence, or wherein the 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from nucleotide position 5 to the nucleotide position immediately 5' to the start codon (located at the 3' end of the sequences), e.g. the nucleotide position immediately 5' to the ATG sequence of a nucleic acid sequence, selected from SEQ ID NOs. 1-1363, SEQ ID NO. 1435, SEQ ID NO. 1461 or SEQ ID NO. 1462, or a corresponding RNA sequence,

wherein, preferably, the fragment is as described above, i.e. being a continuous stretch of nucleotides representing at least 20% etc. of the full-length 5'UTR the fragment is derived from.

Preferably, the artificial nucleic acid molecule may comprise a further 5'UTR element, which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, or a corresponding RNA sequence, or wherein the 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1435, SEQ ID NO. 1461 or SEQ ID NO. 1462, or a corresponding RNA sequence, wherein, preferably, the fragment is as described above, i.e. being a continuous stretch of nucleotides representing at least 20% etc. of the full-length 5'UTR the fragment is derived from.

Preferably, the above defined fragments and variants (e.g. exhibiting at least 40% identity) of the sequences according to SEQ ID NOs. 1-1363, SEQ ID NO. 1435, SEQ ID NO. 1461 or SEQ ID NO. 1462, are functional fragments and variants as described herein.

In a particularly preferred embodiment, the artificial nucleic acid molecule may comprise a further 5'UTR element, which comprises or consists of a nucleic acid sequence which is derived from a 5'UTR of a TOP gene encoding a ribosomal protein or from a variant of a 5'UTR of a TOP gene encoding a ribosomal protein. Particularly preferred 5'UTR elements comprise or consist of a nucleic acid sequence which are derived from a 5' UTR of a TOP gene coding for a ribosomal protein selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, UBA52. Particularly preferred are nucleic acid sequences which are derived from a 5' UTR of TOP genes vertebrate coding for ribosomal proteins, such as mammalian ribosomal proteins e.g. human or mouse ribosomal proteins.

For example, the artificial nucleic acid molecule may comprise a further 5'UTR element, which comprises or consists of a nucleic acid sequence which is derived from a 5'UTR of a nucleic acid sequence according to any ofSEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the artificial nucleic acid molecule may comprise a further 5'UTR element, which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; or a corresponding RNA sequence, preferably lacking the 5'TOP motif, or wherein the 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310,

1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360; or a corresponding RNA sequence, wherein, preferably, the fragment is as described above, i.e. being a continuous stretch of nucleotides representing at least 20% etc. of the full-length 5'UTR, preferably lacking the 5'TOP motif. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the artificial nucleic acid molecule may comprise a further 5'UTR element, which comprises or consists of a nucleic acid sequence which is derived from a 5'UTR of a TOP gene encoding a ribosomal Large protein (RPL) or from a variant of a 5'UTR of a TOP gene encoding a ribosomal Large protein (RPL). For example, the 5'UTR element comprises or consists of a nucleic acid sequence which is derived from a 5'UTR of a nucleic acid sequence according to any of SEQ ID NOs: 67, 259, 1284-1318, 1344, 1346, 1348-1354, 1357, 1461 and 1462, a corresponding RNA sequence, a homolog thereof, or a variant thereof as described herein, preferably lacking the 5'TOP motif.

Preferably, the artificial nucleic acid molecule may comprise a further 5'UTR element, which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to any of SEQ ID NOs. 67, 259, 1284-1318, 1344, 1346, 1348-1354, 1357, and 1358 or a corresponding RNA sequence, preferably lacking the 5'TOP motif, or wherein the 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the 5'UTR of a nucleic acid sequence according to SEQ ID NOs: 67, 259, 1284-1318, 1344, 1346, 1348-1354, 1357, 1461 and 1462 or a corresponding RNA sequence, wherein, preferably, the fragment is as described above, i.e. being a continuous stretch of nucleotides representing at least 20% etc. of the full-length 5'UTR, preferably lacking the 5'TOP motif. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In a particularly preferred embodiment, the artificial nucleic acid molecule may comprise a further 5'UTR element, which comprises or consists of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit Vic gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene.

Accordingly, in a particularly preferred embodiment, the artificial nucleic acid molecule may comprise a further 5'UTR element, which comprises or consists of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID No. 1368, or SEQ ID NOs 1452-1460 or a corresponding RNA sequence, or wherein the 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even

more preferably of at least about 95%, even more preferably of at least about 99% to the nucleic acid sequence according to SEQ ID No. 1368, or SEQ ID NOs 1452-1460 wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the at least one 5'UTR element exhibits a length of at least about 20 nucleotides or more, preferably of at least about 30 nucleotides or more, more preferably of at least about 40 nucleotides or more. However, it may be preferred if the 5'UTR element of the artificial nucleic acid molecule is rather short. Accordingly, it may have a length of less than about 200, preferably less than 150, more preferably less than 100 nucleotides. For example, the 5'UTR may have a length of less than about 25, 30, 35, 40, 45, 50, 55, 60, 65, 70, 80, 85, 90, 95, 100, 105, 110, 115, 120, 125, 130, 135, 140, 145, 150, 155, 160, 165, 170, 175, 180, 185, 190, 195, 200 nucleotides Preferably, the 5'UTR element may have a length of about 20-25, 26-30, 31-35, 36-40, 41-45, 46-50, 51-55, 56-60, 61-65, 66-70, 71-80, 81-85, 86-90, 91-95, 96-100, 101-105, 106-110, 111-115, 116-120, 121-125, 126-130, 131-135, 136-140, 141-145, 146-150, 151-155, 156-160, 161-165, 166-170, 171-175, 176-180, 181-185, 186-190, 191-195, 196-200 or more nucleotides. For example, the 5'UTR element may have a length of about 20, 26, 31, 36, 41, 46, 51, 56, 61, 66, 71, 81, 86, 91, 96, 101, 106, 111, 116, 121, 126, 131, 136, 141, 146, 151, 156, 161, 166, 171, 176, 181, 186, 191 or 196 nucleotides. Preferably, the 5'UTR element may have a length from about 20, 30, 40 or more to less than about 200 nucleotides, more preferably from about 20, 30, 40 or more to less than about 150 nucleotides, most preferably from about 20, 30, 40 or more to less than about 100 nucleotides.

Preferred 5'UTR elements are derived from a 5' UTR of a TOP gene selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a variant thereof.

In some embodiments, the artificial nucleic acid molecule comprises (in addition to the at least one 5'UTR element defined in the claims) a 5'UTR element which comprises or consists of a nucleic acid sequence which is derived from the 5'UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a variant thereof, wherein preferably the 5'UTR element does not comprise a TOP motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its 3'-end with a nucleotide located at position 1, 2, 3, 4, 5, 6, 7, 8, 9 or 10 upstream of the start codon (A(U/T)G) of the gene it is derived from.

In a particularly preferred embodiment, the artificial nucleic acid molecule further comprises a histone stem-loop.

Accordingly, it is particularly preferred that the artificial nucleic acid molecule according to the present invention comprises:

a. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;

b. at least one open reading frame (ORF), and

c. at least one histone stem-loop.

The combination of a 5'UTR element as described above with a histone stem-loop may have a particularly advantageous effect in providing prolonged and possibly also enhanced translation of an RNA molecule.

In the context of the present invention, such a histone stem-loop is typically derived from a histone gene and comprises an intramolecular base pairing of two neighbored entirely or partially reverse complementary sequences, thereby forming a stem-loop. A stem-loop can occur in single-stranded DNA or, more commonly, in RNA. The structure is also known as a hairpin or hairpin loop and usually consists of a stem and a (terminal) loop within a consecutive sequence, wherein the stem is formed by two neighbored entirely or partially reverse complementary sequences separated by a short sequence as sort of spacer, which builds the loop of the stem-loop structure. The two neighbored entirely or partially reverse complementary sequences may be defined as e.g. stem-loop elements stem1 and stem2. The stem loop is formed when these two neighbored entirely or partially reverse complementary sequences, e.g. stem-loop elements stem1 and stem2, form base-pairs with each other, leading to a double stranded nucleic acid sequence comprising an unpaired loop at its terminal ending formed by the short sequence located between stem-loop elements stem1 and stem2 on the consecutive sequence. The unpaired loop thereby typically represents a region of the nucleic acid which is not capable of base pairing with either of these stem-loop elements. The resulting lollipop-shaped structure is a key building block of many RNA secondary structures. The formation of a stem-loop structure is thus dependent on the stability of the resulting stem and loop regions, wherein the first prerequisite is typically the presence of a sequence that can fold back on itself to form a paired double strand. The stability of paired stem-loop elements is determined by the length, the number of mismatches or bulges it contains (a small number of mismatches is typically tolerable, especially in a long double strand), and the base composition of the paired region. In the context of the present invention, optimal loop length is 3-10 bases, more preferably 3 to 8, 3 to 7, 3 to 6 or even more preferably 4 to 5 bases, and most preferably 4 bases.

Preferably, the at least one histone stem-loop is functionally associated to the ORF. This means that the at least one histone stem-loop is preferably positioned within the artificial nucleic acid molecule such that it is able to exert its function, for example, its function of increasing protein production from the ORF or stabilizing the artificial nucleic acid molecule.

Preferably, the histone stem-loop is located 3' to the ORF. For example, the histone stem-loop may be connected to the 3'-end of the ORF directly or via a linker, for example via a stretch of nucleotides, such as 2, 4, 6, 8, 10 etc. nucleotides, e.g. comprising one or more restriction sites, or the histone stem-loop may be located within or between or downstream of other structures located 3' to the ORF, such as within a 3'UTR element, or between a poly(A) sequence and a poly(C) sequence, or down-stream of a poly(A) and/or a poly(C) sequence, or the histone stem-loop may be located at the 3'-end of the artificial nucleic acid molecule. The term "located at the 3'-end" also includes embodiments, wherein the histone stem-loop is followed in 3'-direction by few nucleotides which remain, e.g., after a restriction enzyme cleavage.

Preferably, the 5'UTR element and the histone stem-loop are chosen and positioned such that they exert at least an an additive, preferably a synergistic function on protein production from the ORF of the artificial nucleic acid molecule. Preferably, protein production from the ORF is increased at least in an additive, preferably in a synergistic way by the 5'UTR element and the histone stem-loop. Thus, the protein amount of the protein encoded by the ORF, such as a reporter protein, e.g. luciferase, at a certain time point after initiation of expression of the ORF, e.g. after transfection of a test cell line, is at least the same, preferably higher than what would be expected if the protein production increasing effects of the 5'UTR element and the histone stem-loop were purely additive. The additive, preferably synergistic effect may, for example, be determined by the following assay. Four artificial nucleic acid molecules, e.g. mRNAs, comprising an ORF encoding, e.g. a reporter protein such as luciferase, are generated, i.e. (i) lacking a 5'UTR element and a histone stem-loop (E0), (ii) containing a 5'UTR element derived from a 5'UTR of a TOP gene or of a variant thereof (E1), (iii) containing a histone stem-loop (E2), and (iv) containing both the 5'UTR element and the histone stem-loop (E1E2). Expression of the ORF contained in the artificial nucleic acid molecules is initiated, for example, by transfecting a test cell line, such as a mammalian cell line, e.g. HELA cells, or primary cells, e.g. HDF cells. Samples are taken at specific time points after initiation of expression, for example, after 6 hours, 24 hours, 48 hours, and/or 72 hours and the amount of protein produced by expression of the ORF contained in the artificial nucleic acid molecules is measured, for example, by an ELISA assay or a luciferase test, depending on the type of protein encoded by the ORF. The predicted amount of protein at a certain time point after initiation of expression obtained by construct E1E2 if the effects of the 3'UTR element and the 5'UTR element were purely additive (PPA) may be calculated as follows:

$$PPA_x = (E1_x - E0_x) + (E2_x - E0_x) + E0_x,$$

E0 is the amount of protein obtained for the construct E0 (lacking a 5'UTR and a histone stem-loop), E1 is the amount of protein obtained for the construct E1, E2 is the protein amount obtained for the construct E2, and x is the time point after initiation of expression. The effect on increasing protein production is additive if $E1E2_x = PPA_x$, and

synergistic in the sense of the present invention if $E1E2_x > PPA_x$, wherein $E1E2_x$ is the amount of protein obtained from construct E1E2 at time point x. Preferably, E1E2 is at least 1.0, more preferably at least 1.1, more preferably at least 1.3, more preferably at least 1.5, even more preferably at least 1.75 times PPA at a given time point post initiation of expression, such as 24 hours, 48 hours or 72 hours post initiation of expression.

Thus, in a preferred embodiment, the present invention provides an artificial nucleic acid molecule comprising (a.) at least one 5'-untranslated region element (5'UTR element) which comprises or consists of a nucleic acid sequence which has an identity of at least 95% to a nucleic acid sequence according to SEQ ID NOs. 1368, 1452, 1453, 1454, 1455, 1456, 1457 or 1458, or a corresponding RNA sequence, or a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence; (b.) at least one open reading frame (ORF); and (c.) at least one histone stem-loop as described herein, wherein the histone stem-loop and the 5'UTR element act at least additively, preferably synergistically to increase protein production from the ORF, preferably wherein E1E2 ≥ PPA, preferably E1E2 is at least PPA, more preferably E1E2 is at least 1.1 times PPA, more preferably E1E2 is at least 1.3 times PPA, even more preferably wherein E1E2 is at least 1.5 times PPA at a given time point post initiation of expression of the ORF, for example 24 hours, preferably 48 hours post initiation of expression, wherein E1E2 and PPA are as described above.

Furthermore, it is preferred that the at least one histone stem-loop and the at least one 5'UTR element have an at least additive, preferably a synergistic effect on total protein production from the artificial nucleic acid molecule in a certain time span, such as within 24 hours, 48 hours, or 72 hours post initiation of expression. The additive, preferably the synergistic effect may be determined as described above, with the difference that the area under the curve (AUC) for the amount of protein over time predicted for E1E2 if the effects are additive is compared to the actual AUC measured for E1E2.

In a preferred embodiment of the present invention, the inventive artificial nucleic acid molecule comprises or codes for (a.) at least one 5'UTR element as described above, (b.) at least one open reading frame; and (c.) at least one histone stem-loop, preferably according to at least one of the following formulae (I) or (II):

formula (I) (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

formula (II) (stem-loop sequence with stem bordering elements):

$$\underbrace{N_{1-6}}_{\substack{\text{stem1}\\\text{bordering element}}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\substack{\text{stem2}\\\text{bordering element}}}$$

wherein:

stem1 or stem2 bordering element $N_{1-6}$ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;

stem1 $[N_{0-2}GN_{3-5}]$ is reverse complementary or partially reverse complementary with element stem2, and is a consecutive sequence of between 5 to 7 nucleotides;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and

wherein G is guanosine or an analogue thereof, and may be optionally replaced by a cytidine or an analogue thereof, provided that its complementary nucleotide cytidine in stem2 is replaced by guanosine;

loop sequence [$N_{0-4}$(U/T)$N_{0-4}$] is located between elements stem1 and stem2, and is a consecutive sequence of 3 to 5 nucleotides, more preferably of 4 nucleotides;
wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;

stem2 [$N_{3-5}$C$N_{0-2}$] is reverse complementary or partially reverse complementary with element stem1, and is a consecutive sequence of between 5 to 7 nucleotides;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;
wherein $N_{0-2}$ is a consecutive sequence of 0 to 2, preferably of 0 to 1, more preferably of 1 N, wherein each N is independently from another selected from a nucleotide selected from A, U, T, G or C or a nucleotide analogue thereof; and
wherein C is cytidine or an analogue thereof, and may be optionally replaced by a guanosine or an analogue thereof provided that its complementary nucleotide guanosine in stem1 is replaced by cytidine;
wherein stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one ore more bases in one stem do not have a complementary base in the reverse complementary sequence of the other stem.

In the above context, a wobble base pairing is typically a non-Watson-Crick base pairing between two nucleotides. The four main wobble base pairs in the present context, which may be used, are guanosine-uridine, inosine-uridine, inosine-adenosine, inosine-cytidine (G-U/T, I-U/T, I-A and I-C) and adenosine-cytidine (A-C).

Accordingly, in the context of the present invention, a wobble base is a base, which forms a wobble base pair with a further base as described above. Therefore, non-Watson-Crick base pairing, e.g. wobble base pairing, may occur in the stem of the histone stem-loop structure according to the present invention.

In the above context, a partially reverse complementary sequence comprises maximally two, preferably only one mismatch in the stem-structure of the stem-loop sequence formed by base pairing of stem1 and stem2. In other words, stem1 and stem2 are preferably capable of (full) base pairing with each other throughout the entire sequence of stem1 and stem2 (100% of possible correct Watson-Crick or non-Watson-Crick base pairings), thereby forming a reverse complementary sequence, wherein each base has its correct Watson-Crick or non-Watson-Crick base pendant as a complementary binding partner. Alternatively, stem1 and stem2 are preferably capable of partial base pairing with each other throughout the entire sequence of stem1 and stem2, wherein at least about 70%, 75%, 80%, 85%, 90%, or 95% of the 100% possible correct Watson-Crick or non-Watson-Crick base pairings are occupied with the correct Watson-Crick or non-Watson-Crick base pairings and at most about 30%, 25%, 20%, 15%, 10%, or 5% of the remaining bases are unpaired.

According to a preferred embodiment of the invention, the at least one histone stem-loop sequence (with stem bordering elements) of the inventive nucleic acid sequence as defined herein comprises a length of about 15 to about 45 nucleotides, preferably a length of about 15 to about 40 nucleotides, preferably a length of about 15 to about 35 nucleotides, preferably a length of about 15 to about 30 nucleotides and even more preferably a length of about 20 to about 30 and most preferably a length of about 24 to about 28 nucleotides.

Furthermore, the at least one histone stem-loop sequence (without stem bordering elements) of the inventive artificial nucleic acid molecule as defined herein may comprise a length of about 10 to about 30 nucleotides, preferably a length of about 10 to about 20 nucleotides, preferably a length of about 12 to about 20 nucleotides, preferably a length of about 14 to about 20 nucleotides and even more preferably a length of about 16 to about 17 and most preferably a length of about 16 nucleotides.

Preferably, the inventive artificial nucleic acid molecule may comprise or code for (a.) at least one 5'UTR element as described above; at least one open reading frame; and (c.) at least one histone stem-loop sequence according to at least one of the following specific formulae (Ia) or (IIa):

formula (Ia) (stem-loop sequence without stem bordering elements):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (IIa) (stem-loop sequence with stem bordering elements):

$$\underbrace{N_{2-5}}_{\text{stem1 bordering element}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\text{stem2 bordering element}}$$

wherein N, C, G, T and U are as defined above.

Preferably, the inventive artificial nucleic acid molecule may comprise or code for (a.) at least one 5'UTR element as described above; at least one open reading frame; and (c.) at least one histone stem-loop sequence according to at least one of the following specific formulae (Ib) or (IIb):

formula (Ib) (stem-loop sequence without stem bordering elements):

$$\underbrace{[N_1GN_4]}_{\text{stem1}}\ \underbrace{[N_2(U/T)N_1]}_{\text{loop}}\ \underbrace{[N_4CN_1]}_{\text{stem2}}$$

formula (IIb) (stem-loop sequence with stem bordering elements):

$$\underbrace{N_{4-5}}_{\text{stem1 bordering element}}\ \underbrace{[N_1GN_4]}_{\text{stem1}}\ \underbrace{[N_2(U/T)N_1]}_{\text{loop}}\ \underbrace{[N_4CN_1]}_{\text{stem2}}\ \underbrace{N_{4-5}}_{\text{stem2 bordering element}}$$

wherein N, C, G, T and U are as defined above.

Preferably, the inventive artificial nucleic acid molecule may comprise or code for (a.) at least one 5'UTR element as described above; at least one open reading frame; and (c.) at least one histone stem-loop sequence according to at least one of the following specific formulae (Ic) to (Ih) or (IIc) to (IIh), shown alternatively in its stem-loop structure and as a linear sequence representing histone stem-loop sequences as generated according to Example 1:

formula (Ic): (metazoan and protozoan histone stem-loop consensus sequence without stem bordering elements):

```
  N U
N     N
  N-N
  N-N
  N-N
  N-N
  G-C
  N-N  (stem-loop structure)
```

NGNNNNNNUNNNNNCN
(linear sequence) (SEQ ID NO: 1391)

formula (IIc): (metazoan and protozoan histone stem-loop consensus sequence with stem bordering elements):

```
                N U
              N     N
                N-N
                N-N
                N-N
                N-N
                G-C
N*N*NNNN-NNNN*N*N*  (stem-loop structure)
```

N*N*NNNNGNNNNNNUNNNNNCNNNN*N*N*
(linear sequence) (SEQ ID NO: 1392)

formula (Id): (without stem bordering elements)

```
  N U
N     N
  N-N
  N-N
  N-N
  N-N
  C-G
  N-N  (stem-loop structure)
```

NCNNNNNNUNNNNNGN
(linear sequence) (SEQ ID NO: 1393)

formula (IId): (with stem bordering elements)

```
        N U
       N   N
        N-N
        N-N
        N-N
        N-N
        C-G
N*N*NNNN-NNNN*N*N*  (stem-loop structure)
```

N*N*NNNNCNNNNNNUNNNNNGNNNN*N*N*
(linear sequence) (SEQ ID NO: 1394)

formula (Ie): (protozoan histone stem-loop consensus sequence without stem bordering elements)

```
 N U
N   N
 N-N
 N-N
 N-N
 N-N
 G-C
 D-H  (stem-loop structure)
```

DGNNNNNNUNNNNNNCH
(linear sequence) (SEQ ID NO: 1395)

formula (IIe): (protozoan histone stem-loop consensus sequence with stem bordering elements)

```
       N U
      N   N
       N-N
       N-N
       N-N
       N-N
       G-C
N*N*NNND-HNNN*N*N*  (stem-loop structure)
```

N*N*NNNDGNNNNNNUNNNNNNCHNNN*N*N*
(linear sequence) (SEQ ID NO: 1396)

formula (If): (metazoan histone stem-loop consensus sequence without stem bordering elements)

```
  N U
 N   N
  Y-V
  Y-N
  B-D
  N-N
  G-C
  N-N  (stem-loop structure)
```

NGNBYYNNUNVNDNCN
(linear sequence) (SEQ ID NO: 1397)

formula (IIf): (metazoan histone stem-loop consensus sequence with stem bordering elements)

```
             N U
            N   N
             Y-V
             Y-N
             B-D
             N-N
             G-C
N*N*NNNN-NNNN*N*N*  (stem-loop structure)
```

N*N*NNNNGNBYYNNUNVNDNCNNNN*N*N*
(linear sequence) (SEQ ID NO: 1398)

formula (Ig): (vertebrate histone stem-loop consensus sequence without stem bordering elements)

```
  N U
 D   H
  Y-A
  Y-B
  Y-R
  H-D
  G-C
  N-N  (stem-loop structure)
```

NGHYYYDNUHABRDCN
(linear sequence) (SEQ ID NO: 1399)

formula (IIg): (vertebrate histone stem-loop consensus sequence with stem bordering elements)

N U
D H
Y-A
Y-B
Y-R
H-D
G-C
N*N*HNNN-NNNN*N*H* (stem-loop structure)

N*N*HNNNGHYYYDNUHABRDCNNNN*N*H*
(linear sequence) (SEQ ID NO: 1400)

formula (Ih): (human histone stem-loop consensus sequence (Homo sapiens) without stem bordering elements)

Y U
D H
U-A
C-S
Y-R
H-R
G-C
D-C (stem-loop structure)

DGHYCUDYUHASRRCC
(linear sequence) (SEQ ID NO: 1401)

formula (IIh): (human histone stem-loop consensus sequence (Homo sapiens) with stem bordering elements)

Y U
D H
U-A
C-S
Y-R
H-R
G-C
N*H*AAHD-CVHB*N*H* (stem loop structure)

N*H*AAHDGHYCUDYUHASRRCCVHB*N*H*
(linear sequence) (SEQ ID NO: 1402)

wherein in each of above formulae (Ic) to (Ih) or (IIc) to (IIh):
N, C, G, A, T and U are as defined above;
each U may be replaced by T;
each (highly) conserved G or C in the stem elements 1 and 2 may be replaced by its complementary nucleotide base C or G, provided that its complementary nucleotide in the corresponding stem is replaced by its complementary nucleotide in parallel; and/or
G, A, T, U, C, R, Y, M, K, S, W, H, B, V, D, and N are nucleotide bases as defined in the following Table:

| abbreviation | Nucleotide bases | remark |
|---|---|---|
| G | G | Guanine |
| A | A | Adenine |
| T | T | Thymine |
| U | U | Uracile |
| C | C | Cytosine |
| R | G or A | Purine |
| Y | T/U or C | Pyrimidine |
| M | A or C | Amino |
| K | G or T/U | Keto |
| S | G or C | Strong (3H bonds) |
| W | A or T/U | Weak (2H bonds) |
| H | A or C or T/U | Not G |
| B | G or T/U or C | Not A |
| V | G or C or A | Not T/U |
| D | G or A or T/U | Not C |
| N | G or C or T/U or A | Any base |
| * | Present or not | Base may be present or not |

In this context, it is particularly preferred that the histone stem-loop sequence according to at least one of the formulae (I) or (Ia) to (Ih) or (II) or (IIa) to (IIh) of the present invention is selected from a naturally occurring histone stem-loop sequence, more particularly preferred from protozoan or metazoan histone stem-loop sequences, and even more particularly preferred from vertebrate and mostly preferred from mammalian histone stem-loop sequences especially from human histone stem-loop sequences.

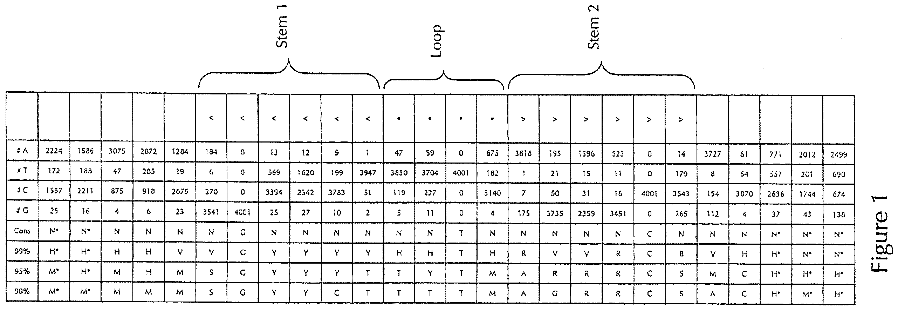

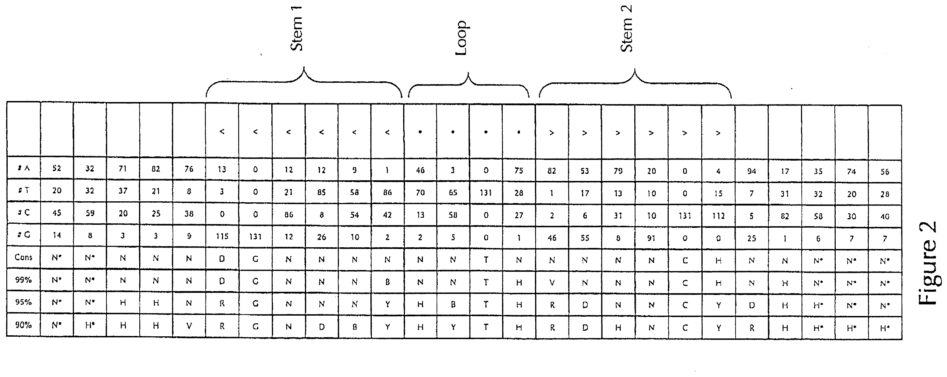

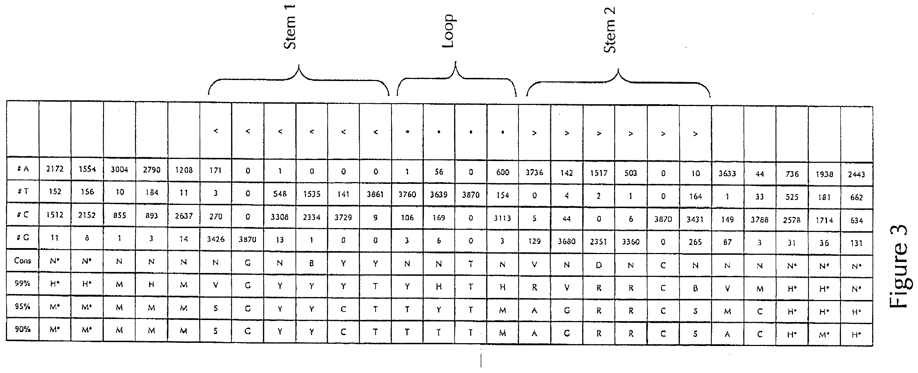

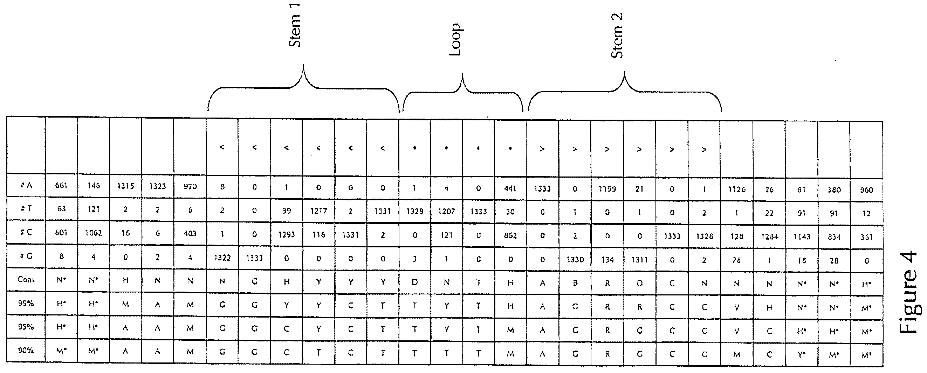

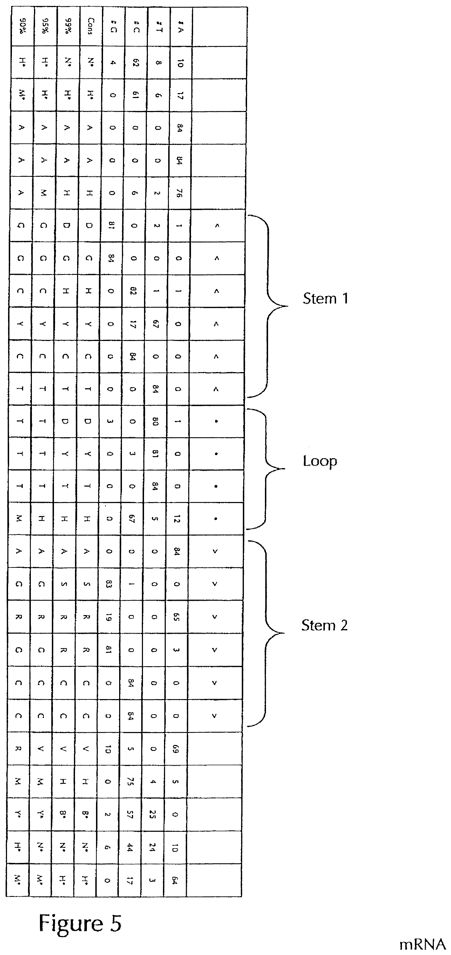

Further preferably, the histone stem-loop sequence according to at least one of the specific formulae (I) or (Ia) to (Ih) or (II) or (IIa) to (IIh) of the present invention is a histone stem-loop sequence comprising at each nucleotide position the most frequently occurring nucleotide, or either the most frequently or the second-most frequently occurring nucleotide of naturally occurring histone stem-loop sequences in metazoa and protozoa (Fig. 1), protozoa (Fig. 2), metazoa (Fig. 3), vertebrates (Fig. 4) and humans (Fig. 5) as shown in figures 1-5. In this context, it is particularly preferred that at least 80%, preferably at least 85%, or most preferably at least 90% of all nucleotides correspond to the most frequently occurring nucleotide of naturally occurring histone stem-loop sequences.

Further preferably, the histone stem-loop sequence according to at least one of the specific formulae (I) or (Ia) to (Ih) of the present invention may be selected from following histone stem-loop sequences or corresponding RNA sequences (without stem-bordering elements) representing histone stem-loop sequences as generated according to Example 1:

VGYYYYHHTHRVVRCB (SEQ ID NO: 1403 according to formula (Ic))
SGYYYTTYTMARRRCS (SEQ ID NO: 1404 according to formula (Ic))
SGYYCTTTTMAGRRCS (SEQ ID NO: 1405 according to formula (Ic))
DGNNNBNNTHVNNNCH (SEQ ID NO: 1406 according to formula (Ie))
RGNNNYHBTHRDNNCY (SEQ ID NO: 1407 according to formula (Ie))
RGNDBYHYTHRDHNCY (SEQ ID NO: 1408 according to formula (Ie))
VGYYYTYHTHRVRRCB (SEQ ID NO: 1409 according to formula (If))
SGYYCTTYTMAGRRCS (SEQ ID NO: 1410 according to formula (If))
SGYYCTTTTMAGRRCS (SEQ ID NO: 1411 according to formula (If))
GGYYCTTYTHAGRRCC (SEQ ID NO: 1412 according to formula (Ig))
GGCYCTTYTMAGRGCC (SEQ ID NO: 1413 according to formula (Ig))
GGCTCTTTTMAGRGCC (SEQ ID NO: 1414 according to formula (Ig))

DGHYCTDYTHASRRCC (SEQ ID NO: 1415 according to formula (Ih))
GGCYCTTTTHAGRGCC (SEQ ID NO: 1416 according to formula (Ih))
GGCYCTTTTMAGRGCC (SEQ ID NO: 1417 according to formula (Ih))

Furthermore, in this context, following histone stem-loop sequences (with stem bordering elements) as generated according to Example 1 according to one of specific formulae (II) or (IIa) to (IIh) and the corresponding RNA sequences are particularly preferred:

H*H*HHVVGYYYYHHTHRVVRCBVHH*N*N*
(SEQ ID NO: 1418 according to formula (IIc))

M*H*MHMSGYYYTTYTMARRRCSMCH*H*H*
(SEQ ID NO: 1419 according to formula (IIc))

M*M*MMMSGYYCTTTTMAGRRCSACH*M*H*
(SEQ ID NO: 1420 according to formula (IIc))

N*N*NNNDGNNNBNNTHVNNNCHNHN*N*N*
(SEQ ID NO: 1421 according to formula (lie))

N*N*HHNRGNNNYHBTHRDNNCYDHH*N*N*
(SEQ ID NO: 1422 according to formula (IIe))

N*H*HHVRGNDBYHYTHRDHNCYRHH*H*H*
(SEQ ID NO: 1423 according to formula (lie))

H*H*MHMVGYYYTYHTHRVRRCBVMH*H*N*
(SEQ ID NO: 1424 according to formula (IIf))

M*M*MMMSGYYCTTYTMAGRRCSMCH*H*H*
(SEQ ID NO: 1425 according to formula (IIf))

M*M*MMMSGYYCTTTTMAGRRCSACH*M*H*
(SEQ ID NO: 1426 according to formula (IIf))

H*H*MAMGGYYCTTYTHAGRRCCVHN*N*M*
(SEQ ID NO: 1427 according to formula (IIg))

H*H*AAMGGCYCTTYTMAGRGCCVCH*H*M*
(SEQ ID NO: 1428 according to formula (IIg))

M*M*AAMGGCTCTTTTMAGRGCCMCY*M*M*
(SEQ ID NO: 1429 according to formula (IIg))

N*H*AAHDGHYCTDYTHASRRCCVHB*N*H*
(SEQ ID NO: 1430 according to formula (IIh))

H*H*AAMGGCYCTTTTHAGRGCCVMY*N*M*
(SEQ ID NO: 1431 according to formula (IIh))

H*M*AAAGGCYCTTTTMAGRGCCRMY*H*M*
(SEQ ID NO: 1432 according to formula (IIh))

A particular preferred histone stem-loop sequence is the sequence according to SEQ ID NO: 1433 (CAAAGGCTCTTTTCAGAGCCACCA) or the corresponding RNA sequence.

Thus, in a particularly preferred embodiment, the artificial nucleic acid molecule according to the present invention comprises (a.) at least one 5'UTR element as described above; (b.) at least one open reading frame; and (c.) at least one histone-stem loop which comprises or consists of a sequence having a sequence identity of at least about

75%, preferably of at least about 80%, preferably at least about 85%, more preferably at least about 90%, even more preferably at least about 95% to the sequence according to SEQ ID NO. 1433 or the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

According to a further preferred embodiment, the inventive artificial nucleic acid molecule comprises or codes for at least one histone stem-loop sequence showing at least about 80%, preferably at least about 85%, more preferably at least about 90%, or even more preferably at least about 95% sequence identity with the not to 100% conserved nucleotides in the histone stem-loop sequences according to at least one of specific formulae (I) or (Ia) to (Ih) or (II) or (IIa) to (IIh) or with a naturally occurring histone stem-loop sequence.

Furthermore, the artificial nucleic acid molecule according to the present invention may comprise more than one histone stem-loop as described herein. For example, the artificial nucleic acid molecule according to the present invention may comprise one, two, three, four or more histone stem-loops, wherein the individual histone stem-loops may be the same or they may be different. For example, the artificial nucleic acid molecule according to the present invention may comprise two histone stem-loops, wherein each histone stem-loop sequence may be selected from the group consisting of SEQ ID NOs. 1391-1433.

In a particularly preferred embodiment, the present invention provides an artificial nucleic acid molecule comprising:

a. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence, or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;
b. at least one open reading frame (ORF); and
c. at least one histone stem-loop, wherein preferably the sequence of the histone stem-loop is selected from the group consisting of sequences according to formulae (I) or (Ia) to (Ih) or (II) or (IIa) to (IIh), such as a sequence selected from the group consisting of SEQ ID NOs: 1391-1433, preferably from the group consisting of SEQ ID NOs. 1403-1433.

Preferably, the artificial nucleic acid molecule according to the present invention may comprise a 5'UTR element which comprises or consists of a nucleic acid sequence which has an identity of at least about 95%, preferably of at least about 99% to the nucleic acid sequence according to SEQ ID NO: 1368 or SEQ ID NOs: 1452-1460 and a histone stem-loop sequence selected from the group consisting of SEQ ID NOs: 1403-1433, e.g. according to SEQ ID NO: 1433, or wherein the histone histone-stem loop comprises or consists of a sequence having a sequence identity of about 90%, even more preferably at least about 95% to the sequence according to SEQ ID NO. 1433 or the corresponding RNA sequence, wherein positions 6, 13 and 20 of the sequence having a sequence identity of at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433.

In some embodiments, the histone stem-loop sequence according to component (c.) is not derived from a mouse histone gene, e.g. from mouse histone gene H2A614. In one embodiment, the artificial nucleic acid molecule of the invention neither contains a mouse histone stem-loop sequence nor contains mouse histone gene H2A614. Furthermore, in one embodiment, the inventive artificial nucleic acid molecule does not contain a stem-loop processing signal, more specifically, a mouse histone processing signal and, most specifically, does not contain mouse histone stem-loop processing signal H2kA614. Also, in one embodiment, the inventive nucleic acid molecule may contain at least one mammalian histone gene. However, in one embodiment, the at least one mammalian histone gene is not Seq. ID No. 7 of WO 01/12824.

Preferably, the inventive artificial nucleic acid molecule comprises no histone downstream element (HDE).

The term "histone downstream element (HDE)" refers to a purine-rich polynucleotide stretch of about 15 to 20 nucleotides 3' of naturally occurring stem-loops, which represents the binding site for the U7 snRNA involved in processing of histone pre-mRNA into mature histone mRNA. For example in sea urchins the HDE is CAAGAAAGA (Dominski, Z. and W. F. Marzluff (2007), Gene 396(2): 373-90).

Preferably, the artificial nucleic acid molecule according to the present invention further comprises a poly(A) sequence or a poly(A) signal.

Therefore, it is particularly preferred that the inventive artificial nucleic acid molecule comprises or codes for (a.) at least one 5'UTR element as described above, (b.) at least one open reading frame, preferably encoding a peptide or protein; (c.) at least one histone stemloopas described herein, and (d.) a poly(A) sequence or a polyadenylation signal.

A polyadenylation signal is defined herein as a signal which conveys polyadenylation to a (transcribed) mRNA by specific protein factors (e.g. cleavage and polyadenylation specificity factor (CPSF), cleavage stimulation factor (CstF), cleavage factors I and II (CF I and CF II), poly(A) polymerase (PAP)).

Preferably, the polyadenylation signal comprises the consensus sequence NN(U/T)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. The polyadenylation signal is preferably located within the artificial nucleic acid molecule such that the above described machinery is able to effect polyadenylation of the artificial nucleic acid molecule. For example, the polyadenylation signal may be located less than about 50 nucleotides, more preferably less than about 30 nucleotides, most preferably less than about 25 nucleotides, for example 21 nucleotides, upstream of the 3'-end of the artificial nucleic acid molecule.

Additionally or alternatively to the polyadenylation signal, in some embodiments, the artificial nucleic acid molecule according to the present invention may further comprise a poly(A) sequence. The length of the poly(A) sequence may vary. For example, the poly(A) sequence may have a length of about 20 adenine nucleotides up to about 400 adenine nucleotides, such as about 20 adenine nucleotides up to about 300 adenine nucleotides, preferably about 40 to about 200 adenine nucleotides, more preferably about 50 to about 100 adenine nucleotides, such as about 60, 70, 80, 90 or 100 adenine nucleotides. The term about refers to a deviation of ± 10%.

The poly(A) sequence is preferably located 3' to the ORF. For example, the poly(A) sequence may be connected to the 3'-end of the ORF directly or via a linker, for example via a stretch of nucleotides, such as 2, 4, 6, 8, 10, 20 etc. nucleotides, such as via a linker of 1-50, preferably 1-20 nucleotides, e.g. comprising one or more restriction sites, or the poly(A) sequence may be located within or between or downstream of other structures located 3' to the ORF, such as between a 3'UTR element and a poly(C) sequence, or downstream of a 3'UTR element and/or a poly(C) sequence, or the poly(A) sequence may be located at the 3'-end of the artificial nucleic acid molecule. The term "located at the 3'-end" also includes embodiments, wherein the poly(A) sequence is followed in 3'-direction by few nucleotides which remain, e.g. after a restriction enzyme cleavage.

It is particularly preferred that the inventive artificial nucleic acid molecule comprises in 5'-to 3'-direction or codes in 5'- to 3'-direction for

(a.) at least one 5'UTR element as defined herein by way of its SEQ ID NO;
(b.) at least one open reading frame, preferably encoding a peptide or protein;
(c.) at least one histone stem-loop, optionally without a histone downstream element 3' to the histone stem-loop, as described herein; and
(d.) a poly(A) sequence and/or a polyadenylation signal.

In another particularly preferred embodiment, the inventive nucleic acid molecule according to the present invention comprises in 5'- to 3'-direction or codes in 5'- to 3'-direction for:

(a.) at least one 5'UTR element as defined herein by way of its SEQ ID NO;
(b.) at least one open reading frame, preferably encoding a peptide or protein;
(d.) a poly(A) sequence; and
(c.) at least one histone stem-loop as described herein.

Thus, the poly(A) sequence and the histone stem-loop of an artificial nucleic acid molecule according to the present invention may be positioned in any desired order from 5' to 3'. Particularly, the poly(A) sequence may be located 5' as well as 3' of the histone stem-loop.

Accordingly, in one embodiment, the artificial nucleic acid molecule according to the present invention comprises

a. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;

(b.) at least one open reading frame (ORF);

(c.) a histone stem-loop; and

(d.) a poly(A) sequence and/or a polyadenylation signal, wherein the poly(A) sequence is located 5' or 3' of the histone stem-loop.

In a further preferred embodiment, the artificial nucleic acid molecule according to the present invention further comprises a poly(C) sequence. A poly(C) sequence in the context of the present invention preferably consists of about 10 to about 200 cytidine nucleotides, more preferably of about 10 to about 100 cytidine nucleotides, more preferably of about 10 to about 50 cytidine nucleotides, even more preferably of about 20 to about 40 cytidine nucleotides, such as about 20, about 25, about 30, about 35, about 40, preferably about 30 cytidine nucleotides. The poly(C) sequence is preferably located 3' to the ORF of the artificial nucleic acid molecule. For example, the poly(C) sequence may be connected to the 3'-end of the ORF directly or via a linker of a stretch of nucleotides, such as 2, 4, 6, 8, 10, 20 etc. nucleotides, such as via a linker of 1-50, preferably of 1-20 nucleitides, e.g. comprising one or more restriction sites, or the poly(C) sequence may be located within, between or downstream of any other structures located 3' to the ORF. For example, the poly(C) sequence may be part of a 3'UTR element or may be located between a poly(A) sequence and a histone stem-loop, or the poly(C) sequence may be located at the 3'-end of the artificial nucleic acid molecule. The term "located at the 3'-end" also includes embodiments, wherein the poly(C) sequence is followed in 3'-direction by a few nucleotides which remain, e.g., after a restriction enzyme cleavage. In a particularly preferred embodiment, the poly(C) sequence is located between a poly(A) sequence and a histone stem-loop.

In a particularly preferred embodiment, the poly(C) sequence is located 5' to the histone stem-loop.

Thus, in a particularly preferred embodiment, the artificial nucleic acid molecule according to the present application comprises the structure 5'-[ORF]-[optional linker]-[3'UTR element]-[optional linker]-[poly(A) sequence]-[optional linker]-[poly(C) sequence]-[optional linker]-[histone stem-loop]-3', wherein the optional linkers may be independently of each other present or absent and may be a stretch of 1-50 nucleotides, e.g. comprising one or more restriction sites.

In a further embodiment, the artificial nucleic acid molecule according to the present invention further comprises a 3'UTR element. Thus, in some embodiments, the artificial nucleic acid molecule according to the present invention may comprise at least one 5'UTR element as described above, at least one open reading frame, at least one histone stem-loop as described herein and at least one 3'UTR element as described herein. Furthermore, in some embodiments, the artificial nucleic acid molecule according to the present invention may comprise at least one 5'UTR element as described above, at least one open reading frame, at least one histone stem-loop as described herein, at least one 3'UTR element as described herein, and a poly(A) sequence and/or a polyadenylation signal as described herein. In some embodiments, the histone stem-loop may be part of the 3'UTR element.

The term '3'UTR element' refers to a nucleic acid sequence which comprises or consists of a nucleic acid sequence that is derived from a 3'UTR or from a variant of a 3'UTR. A 3'UTR element in the sense of the present invention may represent the 3'UTR of an mRNA, e.g., in the event that the artificial nucleic acid molecule is an mRNA, or it may represent a sequence in a nucleic acid construct, such as a vector construct, that when transcribed represents the 3'UTR of the transcription product, such as the mRNA. Thus, in the sense of the present invention, preferably, a 3'UTR element may be the 3'UTR of an mRNA, preferably of an artificial mRNA, or it may be the transcription template for a 3'UTR of an mRNA. Thus, a 3'UTR element preferably is a nucleic acid sequence which corresponds to the 3'UTR of an mRNA, preferably to the 3'UTR of an artificial mRNA, such as an mRNA obtained by transcription of a genetically engineered vector construct. Preferably, the 3'UTR element fulfils the function of a 3'UTR or encodes a sequence which fulfils the function of a 3'UTR. The term '3UTR element' furthermore refers to a fragment or part of a 3'UTR of an artificial nucleic acid sequence, such as an artificial mRNA, or which codes for a part or fragment of a 3'UTR of an artificial nucleic acid molecule. This means that the 3'UTR element in the sense of the present invention may be comprised in the 3'UTR of an artificial nucleic acid sequence, such as an artificial mRNA, or which codes for a 3'UTR of an artificial nucleic acid molecule.

In the context of the present invention, the 3'UTR element may be derived from any 3'UTR of a gene or from a variant thereof, such as from a 3'UTR which is naturally associated with the ORF of the artificial nucleic acid molecule according to the present invention or any other 3'UTR of a naturally occurring gene or of a variant thereof.

Preferably, the 3'UTR element is functionally linked to the ORF. This means preferably that the 3'UTR element is associated with the ORF such that it may exert a function, such as a stabilizing function on the expression of the ORF or a stabilizing function on the artificial nucleic acid molecule. Preferably, the ORF and the 3'UTR element are associated in 5'→73' direction. Thus, preferably, the artificial nucleic acid molecule comprises the structure 5'-ORF-(optional)linker-3'UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the at least one 5'UTR element and the at least one 3'UTR element are functionally linked to the ORF. This means preferably that the 5'UTR element and the 3'UTR element are associated with the ORF such that they may exert a function, preferably in an additive, more preferably in a synergistic manner, such as a stabilizing function on the expression of the ORF, a protein production increasing function for the protein encoded by the ORF, or a stabilizing

function on the artificial nucleic acid molecule. Preferably, the 5'UTR element, the ORF, and the 3'UTR element are associated in 5'→3' direction. Thus, preferably, the artificial nucleic acid molecule comprises the structure 5'-5'UTR element-(optional)linker-ORF-(optional)linker-3'UTR element-3', wherein the linker may be present or absent. For example, the linker may be one or more nucleotides, such as a stretch of 1-50 or 1-20 nucleotides, e.g., comprising or consisting of one or more restriction enzyme recognition sites (restriction sites).

In a particularly preferred embodiment, the 5'UTR element and the 3'UTR element are heterologous, e.g. preferably the 5'UTR and the 3'UTR are derived from different genes of the same or of different species. Preferably, the 3'UTR is not derived from the TOP gene the 5'UTR is derived from.

In a preferred embodiment, the 3'UTR element is chosen such that it exerts at least an additive, preferably a synergistic function with the 5'UTR element on the protein production from the ORF of the artificial nucleic acid molecule. Preferably, the protein production is increased in at least an additive, preferably a synergistic way by the 3'UTR element and the 5'UTR element. Thus, the protein amount of the protein encoded by the ORF, such as a reporter protein, e.g. luciferase, at a certain time point after initiation of expression of the ORF, e.g. after transfection of a test cell or cell line, is preferably at least the same, preferably higher than what would be expected if the protein production increasing effects of the 3'UTR element and the 5'UTR element were purely additive. The additive, preferably the synergistic effect may, for example, be determined by the following assay. Four artificial nucleic acid molecules, e.g. mRNAs, comprising an ORF encoding, e.g. a reporter protein such as luciferase, are generated, i.e. (i) lacking UTR elements (E0), (ii) containing a 5'UTR element derived from a 5'UTR of a TOP gene or of a variant thereof (E1), (iii) containing a test 3'UTR element (E2), and (iv) containing both the 5'UTR element and the test 3'UTR element (E1E2). Expression of the ORF contained in the artificial nucleic acid molecules is initiated, for example, by transfecting a test cell line, such as a mammalian cell line, e.g. HELA cells, or primary cells, e.g. HDF cells. Samples are taken at specific time points after initiation of expression, for example, after 6 hours, 24 hours, 48 hours, and 72 hours and the amount of protein produced by expression of the ORF contained in the artificial nucleic acid molecules is measured, for example, by an ELISA assay or a luciferase test, depending on the type of protein encoded by the ORF. The predicted amount of protein at a certain time point after initiation of expression obtained by construct E1E2 if the effects of the 3'UTR element and the 5'UTR element were purely additive (PPA) may be calculated as follows:

$$PPA_x = (E1_x - E0_x) + (E2_x - E0_x) + E0_x,$$

E0 is the amount of protein obtained for the construct E0 (lacking UTRs), E1 is the amount of protein obtained for the construct E1, E2 is the protein amount obtained for the construct E2, and x is the time point after initiation of expression. The effect on increasing protein production is additive if E1 $E2_x = PPA_x$ and synergistic in the sense of the present invention if $E1E2_x > PPA_x$, wherein $E1E2_x$ is the amount of protein obtained from construct E1E2 at time point x. Preferably, E1E2 is at least 1.0, preferably at least 1.1, more preferably at least 1.3, more preferably at least 1.5, even more preferably at least 1.75 times PPA at a given time point post initiation of expression, such as 24 hours, 48 hours or 72 hours post initiation of expression.

Thus, in a preferred embodiment, the present invention provides an artificial nucleic acid molecule comprising (a.) at least one 5'-untranslated region element (5'UTR element) which comprises or consists of

a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence; (b.) at least one open reading frame (ORF); (c.) at least one histone stem-loop, and at least one 3'UTR element, wherein preferably the 3'UTR element and the 5'UTR element act at least additively, preferably synergistically to increase protein production from the ORF, preferably wherein E1E2 ≥ PPA, preferably E1E2 is at least 1.0 times PPA, preferably E1E2 is at least 1.1 times PPA, more preferably E1E2 is at least 1.3 times PPA, even more preferably wherein E1E2 is at least 1.5 times PPA at a given time point post initiation of expression of the ORF, for example 24 hours, preferably 48 hours post initiation of expression, wherein E1E2 and PPA are as described above.

Furthermore, it is preferred that the 3'UTR element and the 5'UTR element have at least an additive, preferably a synergistic effect on the total protein production from the artificial nucleic acid molecule in a certain time span, such as within 24 hours, 48 hours, or 72 hours post initiation of expression. The additive or the synergistic effect may be determined as described above, with the difference that the area under the curve (AUC) for the amount of protein over time predicted for E1E2 if the effects were purely additive is compared to the actual AUC measured for E1E2.

In a preferred embodiment, the 3'UTR element comprises or consists of a nucleic acid sequence which corresponds to the 3'UTR of a stable mRNA or to a functional variant of the 3'UTR of a stable mRNA, or to a functional fragment of said 3' UTR or of said functional variant. Thus, in a preferred embodiment, the 3'UTR element comprises or consists of a sequence which is derived from a gene providing a stable mRNA or from a functional variant of a 3'UTR of a gene providing a stable mRNA. The term "stable mRNA", preferably refers to mRNAs which exhibit a longer half-life in mammalian cells than the average half-life of mRNA molecules in mammalian cells. Preferably, a stable mRNA in the sense of the present application refers to an mRNA which exhibits a half-life of more than 5 hours, preferably more than 8 hours, in a mammalian cell, such as in a mammalian cell line, e.g. in HELA cells, or in primary cells, e.g. in HDF cells, preferably determined by using a transcription inhibitor such as actinomycin D.

For example, the half-life of an mRNA in mammalian cells, such as HELA or HDF cells, may be determined by culturing the cells in presence of a transcription inhibitor, e.g. actinomycin D, 5,6-dichloro-1-β-D-ribofuranosylbenzimidazole (DRB), or α-amanitin, harvesting the cells at different time points after inhibition of transcription, and determining the amount of the mRNA present in the cell samples by methods well known to the person skilled in the art, e.g. by quantitative RT-PCR. The half-life of a particular mRNA may be calculated based on the amounts of the particular mRNA measured at the different time points post inhibition of transcription. Alternatively, pulse-chase methods, e.g. using radioactively labelled nucleotides, or constructs comprising inducible promoters may be used for determining the half-life of an mRNA in mammalian cells.

It is particularly preferred that the enhanced stability of a stable mRNA in the sense of the present invention is affected by its 3'UTR. Thus, preferably, the 3'UTR element comprises or consists of a 3'UTR of a stable mRNA, or of a functional fragment or a functional variant thereof, which exhibits a half-life of more than 5 hours, preferably more than 8 hours, in a mammalian cell, such as in a mammalian cell line, e.g. in HELA cells, or in mammalian primary cells, such as HDF cells, preferably determined by using a transcription inhibitor such as actinomycin D, wherein the enhanced stability of said stable mRNA is effected by its 3'UTR. The ability of a 3'UTR for enhancing stability may be tested as described herein, e.g. by using a reporter open reading frame such as a luciferase encoding open reading frame. Alternatively, an artificial construct encoding the test stable mRNA may be generated, wherein the 3'UTR of the stable mRNA is replaced with a reference 3'UTR, such as a 3'UTR of a short lived mRNA, e.g. a Myc 3'UTR. The stability of the wild type stable mRNA and the 3'UTR modified mRNA may be determined as described above. In the event the 3'UTR modified mRNA exhibits a shorter half-life than the wild type stable mRNA, it may be concluded that a stability enhancing effect is exerted by the 3'UTR of the stable mRNA.

In a particularly preferred embodiment, the 3'UTR element comprises or consists of a 3'UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene, or from a functional variant of a 3'UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene or from a functional fragment of said 3'UTR or of said variant. In a particularly preferred embodiment, the 3'UTR element comprises or consists of a 3'UTR of an albumin gene, preferably a vertebrate albumin gene, more preferably a mammalian albumin gene, most preferably a human albumin gene or from a functional fragment of said 3'UTR or of said variant. In another particularly preferred embodiment, the 3'UTR element comprises or consists of a 3'UTR of an α-globin gene, preferably a vertebrate α-globin gene, more preferably a mammalian α-globin gene, most preferably a human α-globin gene or a functional fragment or a functional variant thereof. For example, the 3'UTR element may comprise or consist of the center, α-complex-binding portion of the 3'UTR of an α-globin gene, such as of a human α-globin gene.

Preferably, the at least one 3'UTR element comprises or consists of a 3'UTR of a vertebrate albumin gene, a vertebrate α-globin gene, a vertebrate β-globin gene, a vertebrate tyrosine hydroxylase gene, a vertebrate lipoxygenase gene, and a vertebrate collagen alpha gene, such as a vertebrate collagen alpha 1(I) gene, or a functional variant thereof, preferably the 3'UTR of a mammalian albumin gene, a mammalian α-globin gene, a mammalian β-globin gene, a mammalian tyrosine hydroxylase gene, a mammalian lipoxygenase gene, and a mammalian collagen alpha gene, such as a mammalian collagen alpha 1(I) gene, or a functional variant thereof, more preferably the 3'UTR of a human albumin gene, a human α-globin gene, a human β-globin gene, a human tyrosine hydroxylase gene, a human lipoxygenase gene, and a human collagen alpha gene, such as a human collagen alpha 1(I) gene, or a functional variant thereof, even more preferably the 3'UTR of the human albumin gene according to GenBank Accession number NM_000477.5 or a functional variant thereof. In a preferred embodiment, the 3'UTR element is not derived from the 3'UTR of a *Xenopus* albumin gene. Preferably, the 3'UTR element does not comprise a poly(A) limiting element B (PLEB) of a 3'UTR from a *Xenopus* albumin gene. Preferably, the 3'UTR element does not consist of a PLEB of a 3'UTR from a *Xenopus* albumin gene.

In one embodiment, the 3'UTR element and the at least one open reading frame are heterologous, e.g. preferably the 3'UTR element and the ORF are derived from different genes of the same or of different species. Preferably, the ORF does not encode an α-globin protein if the 3'UTR element is derived from an α-globin gene. Preferably, the ORF does not encode a β-globin protein if the 3'UTR element is derived from a β-globin gene. Preferably, the ORF does not encode an albumin protein if the 3'UTR element is derived from an albumin gene. Preferably, the ORF does not encode

a tyrosine hydroxylase protein if the 3'UTR element is derived from a tyrosine hydroxylase gene. Preferably, the ORF does not encode a lipoxygenase protein if the 3'UTR element is derived from a lipoxygenase gene. Preferably, the ORF does not encode a collagen alpha protein if the 3'UTR element is derived from a collagene alpha gene.

In one embodiment, the artificial nucleic acid molecule may consist of at least two sequence parts that are derivable from two different genes, the 5'UTR element which is derivable from a TOP gene and the open reading frame and the 3'UTR which may be derivable from the gene encoding the desired protein product. More preferably, the artificial nucleic acid molecule consists of three sequence parts that are derivable from three different genes: the 5'UTR element which is derivable from a TOP gene, the open reading frame which is derivable from the gene encoding the desired gene product and the 3'UTR element which may be derivable from a gene that relates to an mRNA with an enhanced half-life, for example a 3'UTR element as defined and described below.

In some embodiments, the 3'UTR element consists of a histone stem-loop. In some embodiments, the 3'UTR element of the artificial nucleic acid molecule may comprise a histone stem-loop in addition to the nucleic acid sequence derived from the 3'UTR of a gene, such as of a gene providing a stable mRNA, such as of an albumin gene, an α-globin gene, a β-globin gene, a tyrosine hydroxylase gene, a lipoxygenase gene, or a collagen alpha gene, such as a collagen alpha 1(I) gene as described above. Such artificial nucleic acid molecule according to the present invention, for example, may comprise in 5'-to-3'-direction a 5'UTR element, an ORF, a 3'UTR element, preferably comprising a polyadenylation signal, a histone stem-loop and an optional poly(A) sequence. It may also comprise in 5'-to-3'-direction a 5'UTR element as described above, an ORF, a 3'UTR element, e.g. comprising a polyadenylation signal, a poly(A) sequence and a histone stem-loop.

The term 'a nucleic acid sequence which is derived from the 3'UTR of a [...] gene' preferably refers to a nucleic acid sequence which is based on the 3'UTR sequence of a [...] gene or on a part thereof, such as on the 3'UTR of an albumin gene, an α-globin gene, a β-globin gene, a tyrosine hydroxylase gene, a lipoxygenase gene, or a collagen alpha gene, such as a collagen alpha 1(I) gene, preferably of an albumin gene or an α-globin gene on a part thereof. This term includes sequences corresponding to the entire 3'UTR sequence, i.e. the full length 3'UTR sequence of a gene, and sequences corresponding to a fragment of the 3'UTR sequence of a gene, such as an albumin gene, α-globin gene, β-globin gene, tyrosine hydroxylase gene, lipoxygenase gene, or collagen alpha gene, such as a collagen alpha 1(I) gene, preferably of an albumin or α-globin gene. A fragment in this context preferably consists of a continuous stretch of nucleotides corresponding to a continuous stretch of nucleotides in the full-length 3'UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length 3'UTR. Such a fragment, in the sense of the present invention, is a functional fragment as described herein. The term '3'UTR of a [...] gene' preferably refers to the 3'UTR of a naturally occurring gene, such as of a naturally occurring albumin gene, α-globin gene, β-globin gene, tyrosine hydroxylase gene, lipoxygenase gene, or collagen alpha gene, such as a collagen alpha 1(I) gene, preferably of a naturally occurring albumin or α-globin gene.

The terms 'variant of the 3'UTR of a [...] gene' and 'variant thereof' in the context of a 3'UTR refers to a functional variant of the 3'UTR of a naturally occurring gene, such as a naturally occurring albumin gene, a naturally occurring α-globin gene, a naturally occurring β-globin gene, a naturally occurring tyrosine hydroxylase gene, a naturally occurring lipoxygenase gene, or a naturally occurring collagen alpha gene, such as a collagen alpha 1(I) gene, preferably to a variant of the 3'UTR of a vertebrate albumin gene, a vertebrate α-globin gene, a vertebrate β-globin gene, a vertebrate tyrosine hydroxylase gene, a vertebrate lipoxygenase gene, and a vertebrate collagen alpha gene, such as a vertebrate collagen alpha 1(I) gene, preferably to a variant of the 3'UTR of a mammalian albumin gene, a mammalian α-globin gene, a mammalian β-globin gene, a mammalian tyrosine hydroxylase gene, a mammalian lipoxygenase gene, and a mammalian collagen alpha gene, such as a mammalian collagen alpha 1(I) gene, or to a variant of the 3'UTR of a human albumin gene, a human α-globin gene, a human β-globin gene, a human tyrosine hydroxylase gene, a human lipoxygenase gene, and a human collagen alpha gene, such as a human collagen alpha 1(I) gene. Such variant may be a modified 3'UTR of a gene. For example, a variant 3'UTR may exhibit one or more nucleotide deletions, insertions, additions and/or substitutions compared to the naturally occurring 3'UTR from which the variant is derived. Preferably, a variant of a 3'UTR is at least 40%, preferably at least 50%, more preferably at least 60%, more preferably at least 70%, even more preferably at least 80%, even more preferably at least 90%, most preferably at least 95% identical to the naturally occurring 3'UTR the variant is derived from. The variant is a functional variant as described herein.

The term 'a nucleic acid sequence which is derived from a variant of the 3'UTR of a [...] gene' preferably refers to a nucleic acid sequence which is based on a variant of the 3'UTR sequence of a gene, such as on a variant of the 3'UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'UTR of a gene, i.e. the full length variant 3'UTR sequence of a gene, and sequences corresponding to a fragment of the variant 3'UTR sequence of a gene. A fragment in this context preferably consists of a continuous stretch of nucleotides corresponding to a continuous stretch of nucleotides in the full-length variant 3'UTR, which represents at least 20%, preferably at least 30%, more preferably at least

40%, more preferably at least 50%, even more preferably at least 60%, even more preferably at least 70%, even more preferably at least 80%, and most preferably at least 90% of the full-length variant 3'UTR. Such a fragment of a variant, in the sense of the present invention, is a functional fragment of a variant as described herein.

The terms 'functional variant', 'functional fragment', and 'functional fragment of a variant' (also termed 'functional variant fragment') in the context of the present invention, mean that the fragment of the 5'UTR or the 3'UTR, the variant of the 5'UTR or the 3'UTR, or the fragment of a variant of the 5'UTR or the 3'UTR of a gene fulfils at least one, preferably more than one, function of the naturally occurring 5'UTR or 3'UTR of the gene of which the variant, the fragment, or the fragment of a variant is derived. Such function may be, for example, stabilizing mRNA and/or stabilizing and/or prolonging protein production from an mRNA and/or increasing protein production from an mRNA, preferably in a mammalian cell, such as in a human cell. It is particularly preferred that the variant, the fragment, and the variant fragment in the context of the present invention fulfil the function of stabilizing an mRNA, preferably in a mammalian cell, such as a human cell, compared to an mRNA comprising a reference 5'UTR or lacking a 5'UTR and/or a 3'UTR, and/or the function of stabilizing and/or prolonging protein production from an mRNA, preferably in a mammalian cell, such as in a human cell, compared to an mRNA comprising a reference 5'UTR or lacking a 5'UTR and/or a 3'UTR, and/or the function of increasing protein production from an mRNA, preferably in a mammalian cell, such as in a human cell, compared to an mRNA comprising a reference 5'UTR or lacking a 5'UTR and/or a 3'UTR. A reference 5'UTR may be, for example, a 5'UTR naturally occurring in combination with the ORF. Furthermore, a functional variant, a functional fragment, or a functional variant fragment of a 5'UTR or of a 3'UTR of a gene preferably does not have a substantially diminishing effect on the efficiency of translation of the mRNA which comprises such variant of a 5'UTR and/or such variant of a 3'UTR compared to the wild type 5'UTR and/or 3'UTR from which the variant is derived. A particularly preferred function of a "functional fragment", a "functional variant" or a "functional fragment of a variant" of the 3'UTR of a gene, such as an albumin gene, $\alpha$-globin gene, $\beta$-globin gene, tyrosine hydroxylase gene, lipoxygenase gene, or collagen alpha gene, such as a collagen alpha 1(I) gene, in the context of the present invention is the stabilization and/or prolongation of protein production by expression of an mRNA carrying the functional fragment, functional variant or functional fragment of a variant as described above. A particularly preferred function of a "functional fragment", a "functional variant" or a "functional fragment of a variant" of the 5'UTR in the context of the present invention is the protein production increasing function.

Preferably, the efficiency of the one or more functions exerted by the functional variant, the functional fragment, or the functional variant fragment, such as mRNA and/or protein production stabilizing efficiency and/or the protein production increasing efficiency, is at least 40%, more preferably at least 50%, more preferably at least 60%, even more preferably at least 70%, even more preferably at least 80%, most preferably at least 90% of the mRNA and/or protein production stabilizing efficiency and/or the protein production increasing efficiency exhibited by the naturally occurring 5'UTR and/or 3'UTR of which the functional variant, the functional fragment or the functional variant fragment is derived.

In the context of the present invention, a fragment or part of the 3'UTR of a gene, such as an albumin gene, $\alpha$-globin gene, $\beta$-globin gene, tyrosine hydroxylase gene, lipoxygenase gene, or collagen alpha gene, such as a collagen alpha 1(I) gene, or of a variant thereof preferably exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides. Such fragment of the 3'UTR of a gene or of a variant of the 3'UTR of a gene is a functional fragment as described above.

In the context of the present invention, a fragment or part of the 5'UTR of a TOP gene or of a variant thereof preferably exhibits a length of at least about 20 nucleotides, preferably of at least about 30 nucleotides, more preferably of at least about 50 nucleotides. Such fragment of the 5'UTR of a TOP gene or of a variant of the 5'UTR of a TOP gene is a functional fragment as described above.

In some embodiments, the 3'UTR element of the artificial nucleic acid molecule according to the present invention comprises or consists of a "functional fragment", a "functional variant" or a "functional fragment of a variant" of the 3'UTR of a gene, such as of an albumin gene, $\alpha$-globin gene, $\beta$-globin gene, tyrosine hydroxylase gene, lipoxygenase gene, or collagen alpha gene, such as a collagen alpha 1(I) gene, or of a functional variant thereof.

In some embodiments, the at least one 5'UTR element of the artificial nucleic acid molecule according to the present invention comprises or consists of a "functional fragment", a "functional variant" or a "functional fragment of a variant" of the 5'UTR of a TOP gene. Preferably, the 3'UTR element of the artificial nucleic acid molecule according to the present invention increases the stability of the artificial nucleic acid molecule, e.g. increases the stability of an mRNA according to the present invention, compared to a respective mRNA (reference mRNA) lacking a 3'UTR element. Preferably, the at least one 3'UTR element of the artificial nucleic acid molecule according to the present invention increases the stability of protein production from the artificial nucleic acid molecule according to the present invention, e.g. from an mRNA according to the present invention, compared to a respective mRNA lacking a 3'UTR element. Preferably, the at least one 3'UTR element of the artificial nucleic acid molecule according to the present invention prolongs protein production from the artificial nucleic acid molecule according to the present invention, e.g. from an mRNA according to the present invention, compared to a respective mRNA lacking a 3'UTR element. Preferably, the at least one 3'UTR element of the artificial nucleic acid molecule according to the present invention increases the protein production from

the artificial nucleic acid molecule according to the present invention, e.g. from an mRNA according to the present invention, compared to a respective mRNA lacking a 3'UTR element. Preferably, the at least one 3'UTR element of the artificial nucleic acid molecule according to the present invention does not negatively influence translational efficiency of an mRNA compared to the translational efficiency of a respective mRNA lacking a 3'UTR element. The term 'respective mRNA' in this context means that - apart from the different 3'UTR - the reference mRNA is comparable, preferably identical, to the mRNA comprising the 3'UTR element.

Preferably, the at least one 5'UTR element of the artificial nucleic acid molecule according to the present invention increases the stability of the artificial nucleic acid molecule, e.g. increases the stability of an mRNA according to the present invention, compared to a respective mRNA (reference mRNA) lacking a 5'UTR element or comprising a reference 5'UTR element, such as a 5'UTR naturally occurring in combination with the ORF. Preferably, the at least one 5'UTR element of the artificial nucleic acid molecule according to the present invention increases protein production from the artificial nucleic acid molecule according to the present invention, e.g. from an mRNA according to the present invention, compared to a respective mRNA lacking a 5'UTR element or comprising a reference 5'UTR element, such as a 5'UTR naturally occurring in combination with the ORF. The term 'respective mRNA' in this context means that - apart from the different 5'UTR - the reference mRNA is comparable, preferably identical, to the mRNA comprising the inventive 5'UTR element.

Preferably, the histone stem-loop of the artificial nucleic acid molecule according to the present invention increases the stability of the artificial nucleic acid molecule, e.g. increases the stability of an mRNA according to the present invention, compared to a respective mRNA (reference mRNA) lacking a histone stem-loop. Preferably, the histone stem-loop of the artificial nucleic acid molecule according to the present invention increases protein production from the artificial nucleic acid molecule according to the present invention, e.g. from an mRNA according to the present invention, compared to a respective mRNA lacking a histone stem-loop. The term 'respective mRNA' in this context means that - apart from the histone stem loop - the reference mRNA is comparable, preferably identical, to the mRNA comprising the a histone stem-loop.

Preferably, the at least one 5'UTR element and the at least one 3'UTR element act synergistically to increase protein production from the artificial nucleic acid molecule according to the present invention, e.g. from an mRNA according to the present invention, as described above.

Preferably, the at least one 5'UTR element and the histone stem-loop act synergistically to increase protein production from the artificial nucleic acid molecule according to the present invention, e.g. from an mRNA according to the present invention, as described above.

The term 'stabilizing and/or prolonging protein production from an mRNA' preferably means that the protein production from the mRNA is stabilized and/or prolonged compared to the protein production from a reference mRNA, e.g. lacking a 3'UTR element.

'Stabilized protein expression' in this context preferably means that there is more uniform protein production from the artificial nucleic acid molecule according to the present invention over a predetermined period of time, such as over 24 hours, more preferably over 48 hours, even more preferably over 72 hours, when compared to a reference nucleic acid molecule, for example, lacking a 3'UTR element. Thus, the level of protein production, e.g. in a mammalian system, from the artificial nucleic acid molecule comprising a 3'UTR element according to the present invention, e.g. from an mRNA according to the present invention, preferably does not drop to the extent observed for a reference nucleic acid molecule. For example, the amount of a protein (encoded by the ORF) observed 6 hours after initiation of expression, e.g. 6 hours post transfection of the artificial nucleic acid molecule according to the present invention into a cell, such as a mammalian cell, may be comparable to the amount of protein observed 48 hours after initiation of expression, e.g. 48 hours post transfection. Thus, the ratio of the amount of protein encoded by the ORF, such as of a reporter protein, e.g., luciferase, observed at 48 hours post initiation of expression, e.g. 48 hours post transfection, to the amount of protein observed 6 hours after initiation of expression, e.g. 6 hours post transfection, is preferably above 0.4, preferably above 0.5, more preferably above 0.6, even more preferably above 0.7, e.g. between about 0.4 and about 4, preferably between about 0.65 and about 3, more preferably between about 0.7 and 2 for a nucleic acid molecule according to the present invention. Thus, in one embodiment, the present invention provides an artificial nucleic acid molecule as described above, wherein the ratio of the (reporter) protein amount observed 48 hours after initiation of expression to the (reporter) protein amount observed 6 hours after initiation of expression, preferably in a mammalian expression system, such as in mammalian cells, is preferably between about 0.4 and 4, preferably between about 0.65 and about 3, more preferably between about 0.7 and 2.

'Increased protein expression' in the context of the present invention may refer to increased protein expression at one time point after initiation of expression compared to a reference molecule or to an increased total protein production within a certain time period after initiation of expression. Thus, the protein level observed at a certain time point after initiation of expression, e.g. after transfection, of the artificial nucleic acid molecule according to the present invention, e.g. after transfection of an mRNA according to the present invention, for example, 24, 48, or 72 hours post transfection, or the total protein produced in a time span of, e.g. 24, 48 or 72 hours, is preferably higher than the protein level observed

at the same time point after initiation of expression, e.g. after transfection, or the total protein produced within the same time span, for a reference nucleic acid molecule, such as a reference mRNA comprising a reference 5'UTR element or lacking a 5'UTR element and/or 3'UTR element and/or a histone stem-loop. As set forth above, it is a particularly preferred function of the 5'UTR element and the histone stem-loop to effect an increase in protein production from the artificial nucleic acid molecule. Preferably, the increase in protein production effected by the 5'UTR element and the histone stem-loop compared to a reference nucleic acid molecule lacking such 5'UTR element and a histone stem-loop at a given time point post initiation of expression is at least 1.5-fold, more preferably at least 2-fold, more preferably at least 3-fold, more preferably at least 4-fold, more preferably at least 5-fold, even more preferably at least 10-fold, even more preferably at least 15-fold of the protein production observed for a reference nucleic acid molecule lacking the 5'UTR element and a histone stem-loop. The same holds preferably for the total protein production in a given time period, for example in a time period of 24, 48 or 72 hours post initiation of expression.

Said increase in stability of the artificial nucleic acid molecule, said increase in stability of protein production, said prolongation of protein production and/or said increase in protein production is preferably determined by comparison with a respective reference nucleic acid molecule lacking a 5'UTR element and/or a 3'UTR element and/or a histone stem-loop, e.g. an mRNA lacking a 5'UTR element and/or a 3'UTR element and/or a histone stem-loop, or a reference nucleic acid molecule comprising a reference 5'UTR element and/or a reference 3'UTR element, such as a 3'UTR and/or a 5'UTR naturally occurring with the ORF or a 5'UTR and/or a 3'UTR of a reference gene.

The mRNA and/or protein production stabilizing effect and efficiency and/or the protein production increasing effect and efficiency of the variants, fragments and/or variant fragments of the 3'UTR of an albumin gene as well as the mRNA and/or protein production stabilizing effect and efficiency and/or the protein production increasing effect and efficiency of the 3'UTR element, the at least one 5'UTR element, or the histone stem-loop of the artificial nucleic acid molecule according to the present invention may be determined by any method suitable for this purpose known to the skilled person. For example, artificial mRNA molecules may be generated comprising a coding sequence for a reporter protein, such as luciferase, and no 3'UTR and/or no 5'UTR and/or no histone stem-loop, a 5'UTR derived from a TOP gene and/or a 3'UTR derived from a gene as described above and/or a histone stem-loop as described above, a 5'UTR derived from a reference gene and/or a 3'UTR derived from a reference gene (i.e., a reference 3'UTR or a reference 5'UTR, such as a 5'UTR or a 3'UTR naturally occurring with the ORF), as 3'UTR a functional variant of a 3'UTR of a gene as described above, as 3'UTR a functional fragment of a 3'UTR of a gene as described above, or as 3'UTR a functional fragment of a variant of a 3'UTR of a gene as described above, as 5'UTR a functional variant of a 5'UTR of a TOP gene, as 5'UTR a functional fragment of a 5'UTR of a TOP gene, or as 5'UTR a functional fragment of a variant of a 5'UTR of a TOP gene. Such mRNAs may be generated, for example, by *in vitro* transcription of respective vectors such as plasmid vectors, e.g. comprising a T7 promoter and a sequence encoding the respective mRNA sequences. The generated mRNA molecules may be transfected into cells by any transfection method suitable for transfecting mRNA, for example they may be electroporated into mammalian cells, such as HELA or HDF cells, and samples may be analyzed certain time points after transfection, for example, 6 hours, 24 hours, 48 hours, and 72 hours post transfection. Said samples may be analyzed for mRNA quantities and/or protein quantities by methods well known to the skilled person. For example, the quantities of reporter mRNA present in the cells at the sample time points may be determined by quantitative PCR methods. The quantities of reporter protein encoded by the respective mRNAs may be determined, e.g., by ELISA assays or reporter assays such as luciferase assays depending on the reporter protein used. The effect of stabilizing protein expression and/or prolonging protein expression may be, for example, analyzed by determining the ratio of the protein level observed 48 hours post transfection and the protein level observed 6 hours post transfection. The closer said value is to 1, the more stable the protein expression is within this time period. Said value may also be above 1 if the protein level is higher at the later time point. Such measurements may of course also be performed at 72 or more hours and the ratio of the protein level observed 72 hours post transfection and the protein level observed 6 hours post transfection may be determined to determine stability of protein expression.

Preferably, the 3'UTR element of the artificial nucleic acid molecule according to the present invention comprises or consists of a nucleic acid sequence which has an identity of at least about 95%, preferably of at least about 99%, more preferably of 100% to a nucleic acid sequence selected from SEQ ID NOs. 1369-1377 and 1434 and corresponding RNA sequences, wherein the variants of the sequences according to SEQ ID NOs. 1369-1377 and 1434 are functional variants as described above. SEQ ID NOs. 1369, 1371 and 1434, functional variants thereof, and corresponding RNA sequences are particularly preferred.

The 3'UTR element of the artificial nucleic acid molecule according to the present invention may also comprise or consist of a fragment of a nucleic acid sequence which has an identity of at least about 95%, preferably of at least about 99%, more preferably of 100% to the nucleic acid sequence according to SEQ ID No. 1369-1377 and 1434 and of corresponding RNA sequences, wherein the fragment is a functional fragment or a functional variant fragment as described above. Preferably, the fragment is as described above, i.e. being a continuous stretch of nucleotides representing at least 20% etc. of the full-length 3'UTR the fragment is derived from. Such fragment preferably exhibits a length of at least about 40 nucleotides, preferably of at least about 50 nucleotides, preferably of at least about 75 nucleotides,

more preferably of at least about 100 nucleotides, even more preferably of at least about 125 nucleotides, most preferably of at least about 150 nucleotides.

For example, such fragment may exhibit a nucleic acid sequence according to SEQ ID Nos. 1378-1390, such as:

AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATT (SEQ ID No. 1378)

CATCACATTT AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG (SEQ ID No. 1379)

AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC (SEQ ID No. 1380)

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT (SEQ ID No. 1381)

TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT (SEQ ID No. 1382)

AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT (SEQ ID No. 1383)

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT (SEQ ID No. 1384)

AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA (SEQ ID No. 1385)

ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA AAAATGGAAA (SEQ ID No. 1386)

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1387)

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1388)

CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No. 1389)

AAGCTTATTC ATCTGTTTTT CTTTTTCGTT GGTGTAAAGC CAACACCCTG TCTAAAAAAC (SEQ ID No. 1390)
or the corresponding RNA sequence, or a nucleic acid sequence which is at least 95%, more preferably at least about 99% identical to said nucleic acid sequences or the corresponding RNA sequence. Thus, the at least one 3'UTR element of the artificial nucleic acid molecule according to the present invention may comprise or consist of a nucleic acid fragment as described above. Obviously, the thymidine nucleotides comprised in the fragments according to SEQ ID Nos. 1378-1390 may be replaced by uridine nucleotides.

Said variants, fragments or variant fragments are functional variants, functional fragments, or functional variant fragments as described above, exhibiting at least one function of the nucleic acid sequence according to SEQ ID Nos. 1369-1377 and 1434, such as stabilization of the artificial nucleic acid molecule according to the invention, stabilizing and/or prolonging protein expression from the artificial nucleic acid molecule according to the invention, and/or increasing protein production, preferably with an efficiency of at least 40%, more preferably of at least 50%, more preferably of at least 60%, even more preferably of at least 70%, even more preferably of at least 80%, most preferably of at least 90% of the stabilizing efficiency and/or protein production increasing efficiency exhibited by the nucleic acid sequence according to SEQ ID Nos. 1369-1377 and 1434. Variants, fragments or variant fragments are functional variants, functional fragments, or functional variant fragments which exhibit the function of acting synergistically with the 5'UTR element to increase protein production from the artificial nucleic acid molecule.

Preferably, the 3'UTR element of the artificial nucleic acid molecule according to the present invention exhibits a length of at least about 40 nucleotides, preferably of at least about 50 nucleotides, preferably of at least about 75 nucleotides, more preferably of at least about 100 nucleotides, even more preferably of at least about 125 nucleotides, most preferably of at least about 150 nucleotides. For example, the 3'UTR may exhibit a length of about 50 to about 300 nucleotides, preferably of about 100 to about 250 nucleotides, more preferably of about 150 to about 200 nucleotides.

Furthermore, the artificial nucleic acid molecule according to the present invention may comprise more than one 3'UTR elements as described above. For example, the artificial nucleic acid molecule according to the present invention may comprise one, two, three, four or more 3'UTR elements, wherein the individual 3'UTR elements may be the same or they may be different. For example, the artificial nucleic acid molecule according to the present invention may comprise two essentially identical 3'UTR elements as described above, e.g. two 3'UTR elements comprising or consisting of a nucleic acid sequence which is derived from the 3'UTR of an albumin gene or an α-globin gene or from a functional variant of the 3'UTR of an albumin gene or of an α-globin gene, such as a nucleic acid sequence according to SEQ ID No. 1369, 1371, 1376, or 1434, functional variants thereof, functional fragments thereof, or functional variant fragments thereof as described above.

In a preferred embodiment, the artificial nucleic acid molecule comprises ((a.) at least one 5'-untranslated region element (5'UTR element) which comprises or consists of

a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence,

, (b.) at least one open reading frame, (c.) at least one histone stem-loop as described herein, such as at least one histone stem-loop according to SEQ ID NOs. 1391-1433, optionally (d.) a poly(A) sequence or a poly(A) signal, optionally (e.) a poly(C) sequence, and optionally (f.) at least one 3'UTR element, preferably derived from a gene providing a stable

mRNA, e.g., which comprises or consists of a nucleic acid sequence which is derived from the 3'UTR of an albumin gene or an α-globin gene, such as a sequence selected from the group consisting of SEQ ID NOs: 1369, 1371, and 1434 or a functional variant thereof as described herein, or a functional fragment of said 3' UTR or said functional variant.

Preferably, the sequence of elements of the artificial nucleic acid molecule in 5'-to-3'-direction is 5'-[at least one 5'UTR]-[ORF]-[optional at least one 3'UTR]-[optional poly(A) sequence]-[optional poly(C) sequence]-[at least one histone stem-loop]-3'.

For example, the artificial nucleic acid molecule may comprise (a.) at least one 5'UTR element as defined herein by way of its SEQ ID NO, which may comprise or consist of a nucleic acid sequence which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cytochrome c oxidase subunit VIc gene (COX6C), or a mammalian N-acylsphingosine amidohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'UTR element does not comprise the 5'TOP of said gene(b.) at least one open reading frame, (c.) at least one histone stem-loop, such as at least one histone stem-loop according to SEQ ID NOs. 1391-1433, optionally (d.) a poly(A) sequence and/or a poly(A) signal, optionally (e.) a poly(C) sequence, and optionally (f.) at least one 3'UTR element as defined herein which comprises or consists of a nucleic acid sequence which is derived from an albumin gene or an α-globin gene, such as a sequence selected from the group consisting of SEQ ID NOs: 1369, 1371, and 1434 or a functional variant thereof as described herein.

In a particularly preferred embodiment, the artificial nucleic acid molecule according to the present invention comprises:

(a.) at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;

(b.) at least one open reading frame,

(c.) at least one histone stem-loop as described herein, such as a histone stem-loop sequence according to any one of SEQ ID NOs. 1391-1433, preferably a histone stem-loop sequence having a sequence identity of at least about 75%, preferably of at least about 80%, preferably at least about 85%, more preferably at least about 90%, even more preferably at least about 95% to the sequence according to SEQ ID NO. 1433 or a corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or the corresponding RNA sequence are conserved, i.e. are identical to the nucleotides at positions 6, 13 and 20 of SEQ ID NO. 1433,

(d.) optionally, a poly(A) sequence or a poly(A) signal as described herein,

(e.) optionally, a poly(C) sequence, and

(f.) optionally, a 3'UTR element as defined herein, preferably a 3'UTR element which is derived from a gene providing a stable mRNA, such as a 3'UTR element which comprises or consists of a nucleic acid sequence which has an identity of at least about 95%, preferably of at least about 99%, more preferably of 100% to the nucleic acid sequence according to SEQ ID No. 1369, 1371, or 1434 or a corresponding RNA sequence.

Thus, in a particularly preferred embodiment, the present invention provides an artificial nucleic acid molecule comprising a 5'UTR element which comprises or consists of a nucleic acid sequence which has an identity of at least about 95% to the nucleic acid sequence according to SEQ ID No. 1368 or SEQ ID NOs. 1452-1460, or a corresponding RNA sequence, a histone stem-loop comprising a sequence which has an identity of at least about 90% to the sequence according to SEQ ID NO. 1434 or a corresponding RNA sequence, optionally a poly(A) sequence and/or a poly(A) signal as described herein, optionally a poly(C) sequence, and optionally a 3'UTR element which comprises or consists of a nucleic acid sequence which has an identity of at least about 95% to the nucleic acid sequence according to SEQ ID No. 1369, 1371 or 1434.

Preferably, the artificial nucleic acid molecule according to the present invention does not contain one or two or at least one or all but one or all of the components of the group consisting of: a sequence encoding a ribozyme (preferably a self-splicing ribozyme), a viral nucleic acid sequence, a histone stem-loop processing signal, in particular a histone stem-loop processing sequence derived from mouse histon H2A614 gene, a Neo gene, an inactivated promoter sequence and an inactivated enhancer sequence. Even more preferably, the nucleic acid according to the invention does not contain a ribozyme, preferably a self-splicing ribozyme, and one of the group consisting of: a Neo gene, an inactivated promotor sequence, an inactivated enhancer sequence, a histon stem-loop processing signal, in particular a histon-stem loop processing sequence derived from mouse histon H2A614 gene. Accordingly, the nucleic acid may in a preferred mode neither contain a ribozyme, preferably a self-splicing ribozyme, nor a Neo gene or, alternatively, neither a ribozyme, preferably a self-splicing ribozyme, nor any resistance gene (e.g. usually applied for selection). In an other preferred mode, the nucleic acid molecule of the invention may neither contain a ribozyme, preferably a self-splicing ribozyme, nor a histone stem-loop processing signal, in particular a histone stem-loop processing sequence derived from mouse histone H2A614 gene.

Furthermore, it is preferred that the inventive artificial nucleic acid molecule according to the present invention does not comprise an intron.

The artificial nucleic acid molecule according to the present invention may be RNA, such as mRNA, DNA, such as a DNA vector, or may be a modified RNA or DNA molecule. It may be provided as a double-stranded molecule having a sense strand and an anti-sense strand, for example, as a DNA molecule having a sense strand and an anti-sense strand.

The invention also provides an artificial nucleic acid molecule which is an mRNA molecule comprising a, 5'UTR element, an open reading frame, a histone stem-loop as described herein, an optional 3'UTR element as described herein and an optional poly(A) sequence.

The artificial nucleic acid molecule according to the present invention may further comprise a 5'-cap. The optional 5'-cap is preferably attached to the 5'-side of the 5'UTR element.

The invention provides an artificial nucleic acid molecule which may be a template for an RNA molecule, preferably for an mRNA molecule, which is stabilised and optimized with respect to translation efficiency. In other words, the artificial nucleic acid molecule may be a DNA or RNA which may be used for production of an mRNA. The obtainable mRNA, may, in turn, be translated for production of a desired peptide or protein encoded by the open reading frame. If the artificial nucleic acid molecule is a DNA, it may, for example, be used as a double-stranded storage form for continued and repetitive *in vitro* or *in vivo* production of mRNA.

Potential transcription systems are *in vitro* transcription systems or cellular transcription systems etc. Accordingly, transcription of an artificial nucleic acid molecule according to the invention, e.g. transcription of an artificial nucleic acid molecule comprising a 5'UTR element, an open reading frame, a histone stem-loop, a 3'UTR element, and a polyadenylation-signal, may result in an mRNA molecule comprising a 5'UTR element, an open reading frame, a histone stem-loop, a 3'UTR element and a poly(A) sequence.

For example, the artificial nucleic acid molecule according to the present invention may comprise a nucleic acid sequence corresponding to the DNA sequence

```
CATCACATTT AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG TCTAAAAAAC
ATAAATTTCT TTAATCATTT TGCCTCTTTT CTCTGTGCTT CAATTAATAA AAAATGGAAA
```

GAATCTAGAT CTAAAAAAAA AAAAAAAAAA AAAAAAAAAA AAAAAAAAAA AAAAAAAAAA AAAAAAAAAA AAAAAA (SEQ ID No. 1377).

Transcription of such a sequence may result in an artificial nucleic acid molecule comprising a corresponding RNA sequence.

Such artificial RNA molecule may also be obtainable *in vitro* by common methods of chemical synthesis without being necessarily transcribed from a DNA progenitor.

In a particularly preferred embodiment, the artificial nucleic acid molecule according to the present invention is an RNA molecule, preferably an mRNA molecule comprising in 5'-to-3'-direction a 5'UTR element as described above, an open reading frame, an optional 3'UTR element as described above, an optional poly(A) sequence, an optional poly(C) sequence, and a histone stem-loop as described herein.

In some embodiments, the artificial nucleic acid molecule comprises further elements such as an IRES-motif. An internal ribosome entry side (IRES) sequence or IRES-motif may separate several open reading frames, for example if the artificial nucleic acid molecule encodes for two or more peptides or proteins. An IRES-sequence may be particularly helpful if the mRNA is a bi- or multicistronic RNA.

Furthermore, the artificial nucleic acid molecule may comprise additional 5'-elements such as a promoter or enhancer sequence. The promoter may drive and or regulate transcription of the artificial nucleic acid molecule according to the present invention, for example of an artificial DNA molecule according to the present invention.

In preferred embodiments, the invention provides artificial nucleic acid molecules, preferably mRNA molecules, comprising in 5'-to-3'-direction at least one of the following structures:

5'-cap - 5'UTR element - ORF - 3'UTR element - histone stem-loop - poly(A) sequence

5'-cap - 5'UTR element - ORF - 3'UTR element - poly(A) sequence - histone stem-loop

5'-cap - 5'UTR element - ORF - IRES - ORF - 3'UTR element - histone stem-loop - poly(A) sequence

5'-cap - 5'UTR element - ORF - IRES - ORF - 3'UTR element - poly(A) sequence - histone stem-loop

5'-cap - 5'UTR element - ORF - 3'UTR element - poly(A) sequence - poly(C) sequence - histone stem-loop

5'-cap - 5'UTR element - ORF - IRES - ORF - 3'UTR element - poly(A) sequence - poly(C) sequence - histone stem-loop

5'-cap - 5'UTR element - ORF - IRES - ORF - 3'UTR element - histone stem-loop - poly(A) sequence - poly(C) sequence

More preferably, the inventive artificial nucleic acid molecule comprises or codes for (a.) a 5'UTR-element; (b.) an open reading frame, preferably encoding a peptide or protein; (c.) at least one histone stem-loop, optionally (d.) a poly(A) sequence and/or polyadenylation signal; (e.) optionally a poly(C) sequence; and (f.) optionally a 3'UTR element, preferably for increasing the expression level of an encoded protein, wherein the encoded protein is preferably no histone protein, no reporter protein and/or no marker or selection protein, as defined above. The elements (c.) to (f.) of the inventive artificial nucleic acid molecule may occur in the inventive artificial nucleic acid molecule in any sequence, i.e. the elements (a.), (b.), (c.), (d.), (e.) and (f.) may, for example, occur in the sequence (a.), (b.), (c.), (d.), (e.) and (f.), or (a.), (b.), (d.), (c.), (e.) and (f.), or (a.), (b.), (c.), (d.), (f.) and (e.), or (a.), (b.), (d.), (c.), (f.) and (e.), or (a.), (b.), (e.), (d.), (c.) and (f.), or (a.), (b.), (e.), (d.), (f.) and (c.), or (a.), (b.), (c.), (f.), (e.) and (d.) etc., wherein further elements as described herein, may also be contained, such as a 5'-CAP structure, stabilization sequences, IRES sequences, etc. Each of the elements (a.) to (f.) of the inventive artificial nucleic acid molecule, particularly b), may occur in di- or multicistronic constructs and/or each of the elements (a.), (c.) and (f.) may also be repeated at least once, preferably twice or more in the inventive artificial nucleic acid molecule. As an example, the inventive artificial nucleic acid molecule may comprise its sequence elements (a.), (b.), (c.) and optionally (d.) in e.g. the following order. In all cases the artificial nucleic acid molecule may additionally comprise one or more optional 3'UTR element(s) and/or a poly(C) sequence as defined herein:

5'UTR - ORF - histone stem-loop - 3'; or
5'UTR - ORF - ORF - histone stem-loop - 3'; or
5' UTR - ORF - IRES - ORF - histone stem-loop - 3'; or
5' UTR - ORF - histone stem-loop - poly(A) sequence - 3'; or

5’ UTR - ORF - histone stem-loop - polyadenylation signal - 3’; or
5’ UTR - ORF - ORF - histone stem-loop - polyadenylation signal - 3’; or
5’ UTR - ORF - histone stem-loop - histone stem-loop - 3’; or
5’ UTR - ORF - histone stem-loop - histone stem-loop - poly(A) sequence - 3’; or
5’ UTR - ORF - histone stem-loop - histone stem-loop - polyadenylation signal- 3’; or
5’ UTR - ORF - histone stem-loop - poly(A) sequence - histone stem-loop - 3’; or
5’ UTR - ORF - poly(A) sequence - histone stem-loop - 3’; or
5’ UTR - ORF - poly(A) sequence - histone stem-loop - histone stem-loop - 3’;etc.

It is preferred that the above sequences comprise a poly(C) sequence. Preferably, this poly(C) sequence is located 5’ to the histone stem-loop, preferably between the poly(A) sequence and the histone stem-loop sequence.

In this context, it is particularly preferred that the inventive artificial nucleic acid molecule comprises or codes for a) a 5’UTR element, b) an open reading frame, preferably encoding a peptide or protein; c) at least one histone stem-loop, and d) a poly(A) sequence or polyadenylation sequence; preferably for increasing the expression level of an encoded protein, wherein the encoded protein is preferably no histone protein, no reporter protein (e.g. Luciferase, GFP, EGFP, β-Galactosidase, particularly EGFP) and/or no marker or selection protein (e.g. alpha-Globin, Galactokinase and Xanthine:Guanine phosphoribosyl transferase (GPT)).

The open reading frame of the artificial nucleic acid molecule is not particularly limited. For example, the open reading frame may encode a protein or peptide that may be used for therapy of a disease. The particular choice of the protein or peptide depends on the disease to be treated and is not the subject of the invention. Accordingly, the artificial nucleic acid molecule may be for use in treatment of a disease that is treatable with the protein or peptide that is encoded by the open reading frame. The open reading frame may also encode a protein or peptide that may be used as an antigen for vaccination. Again, the particular choice of the protein or peptide depends on the disease or infection to be prevented. Accordingly, the artificial nucleic acid molecule may be for use in prevention of a disease by inducing a specific immune response.

However, the encoded protein is preferably no histone protein. In the context of the present invention, such a histone protein is typically a strongly alkaline protein found in eukaryotic cell nuclei, which package and order the DNA into structural units called nucleosomes. Histone proteins are the chief protein components of chromatin, act as spools around which DNA winds, and play a role in gene regulation. Without histones, the unwound DNA in chromosomes would be very long (a length to width ratio of more than 10 million to one in human DNA). For example, each human cell has about 1.8 meters of DNA, but wound on the histones it has about 90 millimeters of chromatin, which, when duplicated and condensed during mitosis, result in about 120 micrometers of chromosomes. More preferably, in the context of the present invention, such a histone protein is typically defined as a highly conserved protein selected from one of the following five major classes of histones: H1/H5, H2A, H2B, H3, and H4", preferably selected from mammalian histone, more preferably from human histones or histone proteins. Such histones or histone proteins are typically organised into two super-classes defined as core histones, comprising histones H2A, H2B, H3 and H4, and linker histones, comprising histones H1 and H5.

In this context, linker histones, are preferably excluded from the scope of protection of the pending invention, preferably mammalian linker histones, more preferably human linker histones, are typically selected from H1, including H1F, particularly including H1F0, H1FNT, H1FOO, H1FX, and H1H1, particularly including HIST1H1A, HIST1H1B, HIST1H1C, HIST1H1D, HIST1H1E, HIST1H1T.

Furthermore, in some embodiments, core histones which are preferably excluded from the scope of protection of the pending invention, preferably mammalian core histones, more preferably human core histones, are typically selected from H2A, including H2AF, particularly including H2AFB1, H2AFB2, H2AFB3, H2AFJ, H2AFV, H2AFX, H2AFY, H2AFY2, H2AFZ, and H2A1, particularly including HIST1H2AA, HIST1H2AB, HIST1H2AC, HIST1H2AD, HIST1H2AE, HIST1H2AG, HIST1H2AI, HIST1H2AJ, HIST1H2AK, HIST1H2AL, HIST1H2AM, and H2A2, particularly including HIST2H2AA3, HIST2H2AC; H2B, including H2BF, particularly including H2BFM, H2BFO, H2BFS, H2BFWT H2B1, particularly including HIST1H2BA, HIST1H2BB, HIST1H2BC, HIST1H2BD, HIST1H2BE, HIST1H2BF, HIST1H2BG, HIST1H2BH, HIST1H2BI, HIST1H2BJ, HIST1H2BK, HIST1H2BL, HIST1H2BM, HIST1H2BN, HIST1H2BO, and H2B2, particularly including HIST2H2BE; H3, including H3A1, particularly including HIST1H3A, HIST1H3B, HIST1H3C, HIST1H3D, HIST1H3E, HIST1H3F, HIST1H3G, HIST1H3H, HIST1H3I, HIST1H3J, and H3A2, particularly including HIST2H3C, and H3A3, particularly including HIST3H3; H4, including H41, particularly including HIST1H4A, HIST1H4B, HIST1H4C, HIST1H4D, HIST1H4E, HIST1H4F, HIST1H4G, HIST1H4H, HIST1H4I, HIST1H4J, HIST1H4K, HIST1H4L, and H44, particularly including HIST4H4, and H5.

Preferably, the protein encoded by the open reading frame is no reporter protein (e.g. Luciferase, Green Fluorescent Protein (GFP), Enhanced Green Fluorescent Protein (EGFP), β-Galactosidase) and no marker or selection protein (e.g. alpha-globin, Galactokinase and Xanthine:guanine phosphoribosyl transferase (GPT)). Preferably, the artificial nucleic acid molecule of the invention does not contain a (bacterial) Neo gene sequence (Neomycin resistance

gene).

Preferably, the ORF does not code for a protein selected from the group consisting of albumin proteins, α-globin proteins, β-globin proteins, tyrosine hydroxylase proteins, lipoxygenase proteins, and collagen alpha proteins.

In a preferred embodiment, the open reading frame does not code for human albumin, provided that the 3'UTR element is identical to the 3'UTR of human albumin. In some further embodiment, it is preferred that the open reading frame does not code for human albumin according to GenBank Accession number NM_000477.5 provided that the 3'UTR element is identical to the 3'UTR of human albumin. In some further embodiments, it is preferred that the open reading frame does not code for human albumin or variants thereof provided that the 3'UTR element is a sequence which is identical to SEQ ID No. 1369 or to a corresponding RNA sequence.

Furthermore, in some embodiments, it is preferred that the open reading frame does not code for a reporter protein selected from the group consisting of globin proteins, luciferase proteins, GFP proteins or variants thereof, for example, variants exhibiting at least 70% sequence identity to a globin protein, a luciferase protein, or a GFP protein.

Preferably, the artificial nucleic acid molecule, preferably the open reading frame, is at least partially G/C modified. Thus, the inventive artificial nucleic acid molecule may be thermodynamically stabilized by modifying the G (guanosine)/C (cytidine) content of the molecule. The G/C content of the open reading frame of an artificial nucleic acid molecule according to the present invention may be increased compared to the G/C content of the open reading frame of a corresponding wild type sequence, preferably by using the degeneration of the genetic code. Thus, the encoded amino acid sequence of the nucleic acid molecule is preferably not modified by the G/C modification compared to the coded amino acid sequence of the particular wild type sequence. The codons of a coding sequence or a whole nucleic acid molecule, e.g. an mRNA, may therefore be varied compared to the wild type coding sequence, such that they include an increased amount of G/C nucleotides while the translated amino acid sequence is maintained. In respect to the fact that several codons code for one and the same amino acid (so-called degeneration of the genetic code), the most favourable codons for the stability can be determined (so-called alternative codon usage).

Depending on the amino acid to be encoded by the coding region of the inventive nucleic acid molecule as defined herein, there are various possibilities for modification of the nucleic acid sequence, e.g. the open reading frame, compared to its wild type coding region. In the case of amino acids which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present.

In contrast, codons which contain A and/or U/T nucleotides may be modified by substitution of other codons which code for the same amino acids but contain no A and/or U/T. For example

the codons for Pro can be modified from CC(U/T) or CCA to CCC or CCG;
the codons for Arg can be modified from CG(U/T) or CGA or AGA or AGG to CGC or CGG;
the codons for Ala can be modified from GC(U/T) or GCA to GCC or GCG;
the codons for Gly can be modified from GG(U/T) or GGA to GGC or GGG.

In other cases, although A or (U/T) nucleotides cannot be eliminated from the codons, it is however possible to decrease the A and (U/T) content by using codons which contain a lower content of A and/or (U/T) nucleotides. Examples of these are:

The codons for Phe can be modified from (U/T)(U/T)(U/T) to (U/T) (U/T)C;
the codons for Leu can be modified from (U/T) (U/T)A, (U/T) (U/T)G, C(U/T) (U/T) or C(U/T)A to C(U/T)C or C(U/T)G;
the codons for Ser can be modified from (U/T)C(U/T) or (U/T)CA or AG(U/T) to (U/T)CC, (U/T)CG or AGC;
the codon for Tyr can be modified from (U/T)A(U/T) to (U/T)AC;
the codon for Cys can be modified from (U/T)G(U/T) to (U/T)GC;
the codon for His can be modified from CA(U/T) to CAC;
the codon for Gln can be modified from CAA to CAG;
the codons for Ile can be modified from A(U/T)(U/T) or A(U/T)A to A(U/T)C;
the codons for Thr can be modified from AC(U/T) or ACA to ACC or ACG;
the codon for Asn can be modified from AA(U/T) to AAC;
the codon for Lys can be modified from AAA to AAG;
the codons for Val can be modified from G(U/T)(U/T) or G(U/T)A to G(U/T)C or G(U/T)G;
the codon for Asp can be modified from GA(U/T) to GAC;
the codon for Glu can be modified from GAA to GAG;
the stop codon (U/T)AA can be modified to (U/T)AG or (U/T)GA.

In the case of the codons for Met (A(U/T)G) and Trp ((U/T)GG), on the other hand, there is no possibility of sequence modification without altering the encoded amino acid sequence.

The substitutions listed above can be used either individually or in all possible combinations to increase the G/C content of the open reading frame of the inventive nucleic acid sequence as defined herein, compared to its particular wild type open reading frame (i.e. the original sequence). Thus, for example, all codons for Thr occurring in the wild type sequence can be modified to ACC (or ACG).

Preferably, the G/C content of the open reading frame of the inventive artificial nucleic acid molecule as defined herein is increased by at least 7%, more preferably by at least 15%, particularly preferably by at least 20%, compared to the G/C content of the wild type coding region. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the inventive artificial nucleic acid molecule or a fragment, variant or derivative thereof are substituted, thereby increasing the G/C content of said open reading frame.

In this context, it is particularly preferable to increase the G/C content of the open reading frame of the inventive nucleic acid sequence as defined herein, to the maximum (i.e. 100% of the substitutable codons), compared to the wild type open reading frame.

Furthermore, the open reading frame is preferably at least partially codon-optimized. Codon-optimization is based on the finding that the translation efficiency may be determined by a different frequency in the occurrence of transfer RNAs (tRNAs) in cells. Thus, if so-called "rare codons" are present in the coding region of the inventive artificial nucleic acid molecule as defined herein, to an increased extent, the translation of the corresponding modified nucleic acid sequence is less efficient than in the case where codons coding for relatively "frequent" tRNAs are present.

Thus, the open reading frame of the inventive nucleic acid sequence is preferably modified compared to the corresponding wild type coding region such that at least one codon of the wild type sequence which codes for a tRNA which is relatively rare in the cell is exchanged for a codon which codes for a tRNA which is comparably frequent in the cell and carries the same amino acid as the relatively rare tRNA. By this modification, the open reading frame of the inventive artificial nucleic acid molecule as defined herein, is modified such that codons for which frequently occurring tRNAs are available may replace codons which correspond to rare tRNAs. In other words, according to the invention, by such a modification all codons of the wild type open reading frame which code for a rare tRNA may be exchanged for a codon which codes for a tRNA which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system in which the nucleic acid molecule according to the present invention is to be expressed, preferably with respect to the system in which the nucleic acid molecule according to the present invention is to be translated. Preferably, the codon usage of the open reading frame is codon-optimized according to mammalian codon usage, more preferably according to human codon usage. Preferably, the open reading frame is codon-optimized and G/C-content modified.

For further improving degradation resistance, e.g. resistance to *in vivo* degradation by an exo- or endonuclease, and/or for further improving protein production from the artificial nucleic acid molecule according to the present invention, the artificial nucleic acid molecule may further comprise modifications, such as backbone modifications, sugar modifications and/or base modifications, e.g., lipid-modifications or the like. Preferably, the transcription and/or the translation of the artificial nucleic acid molecule according to the present invention is not significantly impaired by said modifications.

Nucleotide analogues/modifications that may be used in the context of the present invention may be selected, for example, from 2-amino-6-chloropurineriboside-5'-triphosphate, 2-aminoadenosine-5'-triphosphate, 2-thiocytidine-5'-triphosphate, 2-thiouridine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-iodocytidine-5'-triphosphate, 5-iodouridine-5'-triphosphate, 5-methylcytidine-5'-triphosphate, 5-methyluridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

Further, lipid-modified artificial nucleic acid molecules may typically comprise at least one linker which is covalently linked with the artificial nucleic acid molecule, and at least one lipid which is covalently linked with this linker. Alternatively, a lipid-modified artificial nucleic acid molecule may comprise at least one artificial nucleic acid molecule as defined herein and at least one, preferably bifunctional lipid which is covalently linked, preferably without a linker, with that artificial nucleic acid molecule. According to a third alternative, a lipid-modified artificial nucleic acid molecule may comprise an artificial nucleic acid molecule as defined herein, at least one linker which is covalently linked with that artificial nucleic acid molecule, at least one lipid which is covalently linked with this linker, and additionally at least one, preferably bifunctional lipid which is covalently linked, preferably without a linker, with the artificial nucleic acid molecule.

In a further aspect, the present invention provides a vector comprising

a. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;

(b.) at least one open reading frame and/or at least one cloning site; and

(c.) optionally, at least one histone stem-loop.

The cloning site may be suitable for accepting an open reading frame, i.e. an open reading frame coding for a protein or peptide to be expressed may be cloned into the vector via the cloning site.

The at least one 5'UTR element, the at least one ORF, and the at least one optional histone stem-loop are as described herein for the artificial nucleic acid molecule according to the present invention. The cloning site may be any sequence that is suitable for introducing an open reading frame or a sequence comprising an open reading frame, such as one or more restriction sites.

Thus, the vector comprising a cloning site is preferably suitable for inserting an open reading frame into the vector. Preferably, it may be suitable for inserting an open reading frame between the 5'UTR element and a desired 3' structure such as a histone stem loop, a polyl(A) sequence, a polyadenylation signal and/or a 3'UTR element, more preferably it is suitable for insertion 5' to the 3' structure and 3' to the 5'UTR element. For example the 3' structure may comprise a histone stem-loop, a poly(A) sequence or a polyadenylation signal and/or a 3'UTR element as described above. Thereby the histone stem loop, the poly(A) sequence and/or the polyadenylation signal and the 3'UTR element may occur in any order that may be desired. Preferably, the cloning site or the ORF is located 5' to the 3'UTR structure, preferably in close proximity to the 5'-end of the histone stem-loop, poly(A) sequence, polyadenylation signal and/or a 3'UTR element as described above. For example, the cloning site or the ORF may be directly connected to the 5'-end of the histone stem-loop, poly(A) sequence, polyadenylation signal and/or a 3'UTR element or they may be connected via a stretch of nucleotides, such as by a stretch of 2, 4, 6, 8, 10, 20 etc. nucleotides as described above for the artificial nucleic acid molecule according to the present invention. Preferably, the cloning site or the ORF is located 3' to the 5'UTR element, preferably in close proximity to the 3'-end of the 5'UTR element. For example, the cloning site or the ORF may be directly connected to the 3'-end of the 5'UTR element or they may be connected via a stretch of nucleotides, such as by a stretch of 2, 4, 6, 8, 10, 20 etc. nucleotides as described above for the artificial nucleic acid molecule according to the present invention.

Preferably, the vector according to the present invention is suitable for producing the artificial nucleic acid molecule according to the present invention, preferably for producing an artificial mRNA according to the present invention, for example, by optionally inserting an open reading frame or a sequence comprising an open reading frame into the vector and transcribing the vector. Thus, preferably, the vector comprises elements needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognizes by a polymerase, such as by an RNA polymerase, e.g. by a eukaryotic, prokaryotic, viral, or phage RNA polymerase. In a preferred embodiment, the vector comprises a phage RNA polymerase promoter such as an SP6 or T7, preferably a T7 promoter. Preferably, the vector is suitable for *in vitro* transcription using a phage based *in vitro* transcription system, such as a T7 RNA polymerase based *in vitro* transcription system. The vector may further comprise a poly(A) sequence and/or a polyadenylation signal and/or a poly(C) sequence as described above for the artificial nucleic acid molecule according to the present invention.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector.

In a preferred embodiment, the vector according to the present invention comprises or codes for the artificial nucleic acid molecule according to the present invention.

Preferably, a vector according to the present invention comprises a sequence having an identity of at least about 95%; preferably of at least about 99% to a sequence according to any one of SEQ ID NOs. 1368 or 1452-1460, or a functional fragment thereof which has an identity of at least 95% to a nucleic acid sequence according to SEQ ID NOs. 1368, 1452, 1453, 1454, 1455, 1456, 1457 or 1458, , or a corresponding RNA sequence.

Preferably, the vector is a circular molecule. Preferably, the vector is a double-stranded molecule, such as a

double stranded DNA molecule. Such circular, preferably double stranded DNA molecule may be used conveniently as a storage form for the inventive artificial nucleic acid molecule. Furthermore, it may be used for transfection of cells, for example, cultured cells. Also it may be used for *in vitro* transcription for obtaining an artificial RNA molecule according to the invention.

Preferably, the vector, preferably the circular vector, is linearizable, for example, by restriction enzyme digestion. In a preferred embodiment, the vector comprises a cleavage site, such as a restriction site, preferably a unique cleavage site, located immediately 3' to the open reading frame or - if present - to the histone stem-loop, or - if present - to the poly(A) sequence or the polyadenylation signal, or - if present - to the 3'UTR element, or - if present - to the poly(C) sequence. Thus, preferably, the product obtained by linearizing the vector terminates at the 3'end with the 3'-end of the open reading frame, or - if present - with the 3'-end of the histone stem loop, or - if present - with the 3'-end of the poly(A) sequence or the 3'-end of the polyadenylation signal, or - if present - with the 3'-end of a 3'UTR element, plus some optional nucleotides, e.g. remaining from the restriction site after cleavage.

In a further aspect, the present invention relates to a cell comprising the artificial nucleic acid molecule according to the present invention or the vector according to the present invention. The cell may be any cell, such as a bacterial cell, insect cell, plant cell, vertebrate cell, e.g. a mammalian cell. Such cell may be, e.g., used for replication of the vector of the present invention, for example, in a bacterial cell. Furthermore, the cell may be used for transcribing the artificial nucleic acid molecule or the vector according to the present invention and/or translating the open reading frame of the artificial nucleic acid molecule or the vector according to the present invention. For example, the cell may be used for recombinant protein production.

The cells according to the present invention are, for example, obtainable by standard nucleic acid transfer methods, such as standard transfection methods. For example, the artificial nucleic acid molecule or the vector according to the present invention may be transferred into the cell by electroporation, lipofection, e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of a human subject, a domestic animal, a laboratory animal, such as a mouse or rat cell. Preferably the cell is a human cell. The cell may be a cell of an established cell line, such as a CHO, BHK, 293T, COS-7, HELA, HEK etc. cell, or the cell may be a primary cell, such as a HDF cell, preferably a cell isolated from an organism. In a preferred embodiment, the cell is an isolated cell of a mammalian subject, preferably of a human subject. For example, the cell may be an immune cell, such as a dendritic cell, a cancer or tumor cell, or any somatic cell etc., preferably of a mammalian subject, preferably of a human subject.

In a further aspect, the present invention provides a pharmaceutical composition comprising the artificial nucleic acid molecule according to the present invention, the vector according the present invention, or the cell according to the present invention. The pharmaceutical composition according to the invention may be used, e.g., as a vaccine, for example, for genetic vaccination. Thus, the ORF may, e.g., encode an antigen to be administered to a patient for vaccination. Thus, in a preferred embodiment, the pharmaceutical composition according to the present invention is a vaccine. Furthermore, the pharmaceutical composition according to the present invention may be used, e.g., for gene therapy.

Preferably, the pharmaceutical composition further comprises one or more pharmaceutically acceptable excipients, vehicles, fillers and/or diluents. In the context of the present invention, a pharmaceutically acceptable vehicle typically includes a liquid or non-liquid basis for the inventive pharmaceutical composition. In one embodiment, the pharmaceutical composition is provided in liquid form. In this context, preferably, the vehicle is based on water, such as pyrogen-free water, isotonic saline or buffered (aqueous) solutions, e.g phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the inventive pharmaceutical composition. The term "compatible" as used herein preferably means that these components of the inventive pharmaceutical composition are capable of being mixed with the inventive nucleic acid, vector or cells as defined herein in such a manner that no interaction occurs which would substantially reduce the pharmaceutical effectiveness of the inventive pharmaceutical composition under typical use conditions.

The pharmaceutical composition according to the present invention may optionally further comprise one or more additional pharmaceutically active components. A pharmaceutically active component in this context is a compound that exhibits a therapeutic effect to heal, ameliorate or prevent a particular indication or disease. Such compounds

include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the inventive pharmaceutical composition may comprise a carrier for the artificial nucleic acid molecule or the vector. Such a carrier may be suitable for mediating dissolution in physiological acceptable liquids, transport and cellular uptake of the pharmaceutical active artificial nucleic acid molecule or the vector. Accordingly, such a carrier may be a component which may be suitable for depot and delivery of an artificial nucleic acid molecule or vector according to the invention. Such components may be, for example, cationic or polycationic carriers or compounds which may serve as transfection or complexation agent.

Particularly preferred transfection or complexation agents in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*), pAntp, pIsl, FGF, Lactoferrin, Transportan, Buforin-2, Bac715-24, SynB, SynB(1), pVEC, hCT-derived peptides, SAP, or histones.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (III):

$$\{(Arg)_l;(Lys)_m;(His)_n;(Orn)_o;(Xaa)_x\}; \qquad \text{formula (III)}$$

wherein l + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula $\{(Arg)_l;(Lys)_m;(His)_n;(Orn)_o;(Xaa)_x\}$ (formula (III)) as shown above and which comprise or are additionally modified to comprise at least one -SH moeity, may be, without being restricted thereto, selected from subformula (IIIa):

$$\{(Arg)_l;(Lys)_m;(His)_n;(Orn)_o;(Xaa')_x(Cys)_y\} \qquad \text{subformula (IIIa)}$$

wherein $(Arg)_l;(Lys)_m;(His)_n;(Orn)_o$; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (IIIb):

$$Cys_1\ \{(Arg)_l;(Lys)_m;(His)_n;(Orn)_o;(Xaa)_x\}\ Cys_2 \qquad \text{subformula (IIIb)}$$

wherein empirical formula $\{(Arg)_l;(Lys)_m;(His)_n;(Orn)_o;(Xaa)_x\}$ (formula (III)) is as defined herein and forms a core of an amino acid sequence according to (semiempirical) formula (III) and wherein $Cys_1$ and $Cys_2$ are Cysteines proximal to, or terminal to $(Arg)_l;(Lys)_m;(His)_n;(Orn)_o;(Xaa)_x$.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-($\alpha$-trimethylammonio-acetyl)diethanolamine chloride, CLIP1: rac-[(2,3-dioctadecyloxypropyl)(2-hydroxyethyl)]-dimethylammonium chloride,

CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

In this context, it is particularly preferred that the inventive artificial nucleic acid molecule or the inventive vector is complexed at least partially with a cationic or polycationic compound, preferably cationic proteins or peptides. Partially means that only a part of the inventive artificial nucleic acid molecule or the inventive vector is complexed with a cationic or polycationic compound and that the rest of the inventive artificial nucleic acid molecule or the inventive vector is in uncomplexed form ("free"). Preferably the ratio of complexed nucleic acid to: free nucleic acid is selected from a range. of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of complexed nucleic acid to free nucleic acid is selected from a ratio of about 1:1 (w/w).

The pharmaceutical composition according to the present invention may optionally further comprise one or more adjuvants, for example, adjuvants for stimulating the innate immune system or for enhancing cellular uptake of the artificial nucleic acid molecule or vector. In this context, an adjuvant may be understood as any compound, which is suitable to initiate or increase an immune response of the innate immune system, i.e. a non-specific immune response. In other words, when administered, the inventive pharmaceutical composition preferably elicits an innate immune response due to the adjuvant, optionally contained therein. Preferably, such an adjuvant may be an adjuvant supporting the induction of an innate immune response in a mammal. Such an adjuvant may be, for example, an immunostimulatory nucleic acid, i.e. a nucleic acid that may bind to a Toll-like-receptor or the like, preferably an immunostimulatory RNA.

Such adjuvants, preferably such immunostimulatory nucleic acids, may induce an innate, i.e. unspecific, immune response which may support a specific, i.e. adaptive, immune response to the peptide or protein, i.e. the antigen, encoded by the artificial nucleic acid molecule of the pharmaceutical composition, preferably the vaccine.

The inventive pharmaceutical composition may also additionally comprise any further compound, which is known to be immunostimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Further additives which may be included in the inventive pharmaceutical composition are, e.g., emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives etc.

The pharmaceutical composition according to the present invention preferably comprises a "safe and effective amount" of the components of the pharmaceutical composition, particularly of the inventive nucleic acid sequence, the vector and/or the cells as defined herein. As used herein, a "safe and effective amount" means an amount sufficient to significantly induce a positive modification of a disease or disorder as defined herein. At the same time, however, a "safe and effective amount" preferably avoids serious side-effects and permits a sensible relationship between advantage and risk. The determination of these limits typically lies within the scope of sensible medical judgment.

In a further aspect, the present invention provides the artificial nucleic acid molecule according to the present invention, the vector according to the present invention, the cell according to the present invention, or the pharmaceutical composition according to the present invention for use as a medicament, for example, as vaccine (in genetic vaccination) or in gene therapy.

The artificial nucleic acid molecule according to the present invention, the vector according to the present invention, the cell according to the present invention, or the pharmaceutical composition according to the present invention are particularly suitable for any medical application which makes use of the therapeutic action or effect of peptides, polypeptides or proteins, or where supplementation of a particular peptide or protein is needed. Thus, the present invention provides the artificial nucleic acid molecule according to the present invention, the vector according to the present invention, the cell according to the present invention, or the pharmaceutical composition according to the present invention for use in the treatment or prevention of diseases or disorders amenable to treatment by the therapeutic action or effect of peptides, polypeptides or proteins or amenable to treatment by supplementation of a particular peptide, polypeptide or protein. For example, the artificial nucleic acid molecule according to the present invention, the vector according to the present invention, the cell according to the present invention, or the pharmaceutical composition according to the present invention may be used for the treatment or prevention of genetic diseases, autoimmune diseases,

cancerous or tumour-related diseases, infectious diseases, chronic diseases or the like, e.g., by genetic vaccination or gene therapy.

In particular, such therapeutic treatments which benefit from a stable, prolonged and/or increased presence of therapeutic peptides, polypeptides or proteins in a subject to be treated are especially suitable as medical application in the context of the present invention, since the 5'UTR element in particular in combination with a histone stem-loop provides for increased protein expression from the ORF of the inventive nucleic acid molecule. Thus, a particularly suitable medical application for the artificial nucleic acid molecule according to the present invention, the vector according to the present invention, the cell according to the present invention, or the pharmaceutical composition according to the present invention is vaccination, for example against infections or tumours. Thus, the present invention provides the artificial nucleic acid molecule according to the present invention, the vector according to the present invention, the cell according to the present invention, or the pharmaceutical composition according to the present invention for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the ORF may be selected. For example, the open reading frame may code for a protein that has to be supplied to a patient suffering from total lack or at least partial loss of function of a protein, such as a patient suffering from a genetic disease. Additionally, the open reading frame may be chosen from an ORF coding for a peptide or protein which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases, allergies, infections, chronic diseases or the like. Furthermore, the open reading frame may code for an antigen or immunogen, e.g. for an epitope of a pathogen or for a tumour antigen. Thus, in preferred embodiments, the artificial nucleic acid molecule or the vector according to the present invention comprises an ORF encoding an amino acid sequence comprising or consisting of an antigen or immunogen, e.g. an epitope of a pathogen or a tumour-associated antigen, a 5'UTR element as described above, preferably a histone stem-loop as described herein, and optional further components, such as a 3'UTR element and/or a poly(A) sequence and/or a poly(C) sequence etc. as described herein.

In the context of medical application, in particular, in the context of vaccination, it is preferred that the artificial nucleic acid molecule according to the present invention is RNA, preferably mRNA, since DNA harbours the risk of eliciting an anti-DNA immune response and tends to insert into genomic DNA. However, in some embodiments, for example, if a viral delivery vehicle, such as an adenoviral delivery vehicle is used for delivery of the artificial nucleic acid molecule or the vector according to the present invention, e.g., in the context of gene therapeutic treatments, it may be desirable that the artificial nucleic acid molecule or the vector is a DNA molecule.

The artificial nucleic acid molecule according to the present invention, the vector according to the present invention, the cell according to the present invention, or the pharmaceutical composition according to the present invention may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the artificial nucleic acid molecule according to the present invention, the vector according to the present invention, the cell according to the present invention, or the pharmaceutical composition according to the present invention is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the inventive pharmaceutical composition may be aqueous or oleaginous suspension. These suspensions may be formulated according to techniques known in the art using suitable dispersing or wetting agents and suspending agents.

The artificial nucleic acid molecule according to the present invention, the vector according to the present invention, the cell according to the present invention, or the pharmaceutical composition according to the present invention may also be administered orally in any orally acceptable dosage form including, but not limited to, capsules, tablets, aqueous suspensions or solutions.

The artificial nucleic acid molecule according to the present invention, the vector according to the present invention, the cell according to the present invention, or the pharmaceutical composition according to the present invention may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the artificial nucleic acid molecule according to the present invention, the vector according to the present invention, the cell according to the present invention, or the pharmaceutical composition according to the present invention may be formulated in a suitable

ointment suspended or dissolved in one or more carriers.

In one embodiment, the use as a medicament comprises the step of transfection of mammalian cells, preferably *in vitro* transfection of mammalian cells, more preferably *in vitro* transfection of isolated cells of a subject to be treated by the medicament. If the use comprises the *in vitro* transfection of isolated cells, the use as a medicament may further comprise the (re)administration of the transfected cells to the patient. The use of the inventive artificial nucleic acid molecules or the vector as a medicament may further comprise the step of selection of successfully transfected isolated cells. Thus, it may be beneficial if the vector further comprises a selection marker. Also, the use as a medicament may comprise *in vitro* transfection of isolated cells and purification of an expression-product, i.e. the encoded peptide or protein from these cells. This purified peptide or protein may subsequently be administered to a subject in need thereof.

The present invention also provides a method for treating or preventing a disease or disorder as described above comprising administering the artificial nucleic acid molecule according to the present invention, the vector according to the present invention, the cell according to the present invention, or the pharmaceutical composition according to the present invention to a subject in need thereof.

Furthermore, the present invention provides a method for treating or preventing a disease or disorder comprising transfection of a cell with an artificial nucleic acid molecule according to the present invention or with the vector according to the present invention. Said transfection may be performed *in vitro* or *in vivo.* In a preferred embodiment, transfection of a cell is performed *in vitro* and the transfected cell is administered to a subject in need thereof, preferably to a human patient. Preferably, the cell which is to be transfected *in vitro* is an isolated cell of the subject, preferably of the human patient. Thus, the present invention provides a method of treatment comprising the steps of isolating a cell from a subject, preferably from a human patient, transfecting the isolated cell with the artificial nucleic acid molecule according to the present invention or the vector according to the present invention, and administering the transfected cell to the subject, preferably the human patient.

The method of treating or preventing a disorder according to the present invention is preferably a vaccination method and/or a gene therapy method as described above.

As described above, the 5'UTR element, preferably, the histone stem-loop, and optionally the poly(A)sequence and/or the 3'UTR element are capable of increasing protein production from an artificial nucleic acid molecule, such as an mRNA or vector, comprising these elements and an ORF, preferably in an at least additive, preferably in a synergistic manner. Thus, in a further aspect, the present invention relates to a method for increasing protein production from an artificial nucleic acid molecule comprising the step of associating the artificial nucleic acid molecule, preferably an ORF contained within the artificial nucleic acid molecule, with (i) at least one 5'-untranslated region element (5'UTR element) which comprises or consists of a nucleic acid sequence which has an identity of at least 95% to a nucleic acid sequence according to SEQ ID NOs. 1368, 1452, 1453, 1454, 1455, 1456, 1457 or 1458, or a corresponding RNA sequence, or a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence, preferably (ii) at least one histone stem-loop as described herein, and optionally one or more further elements, such as a poly(A)sequence and/or polyadenylation signal, and/or a poly(C) sequence, and/or a 3'UTR element, which comprises or consists of a nucleic acid sequence derived from the 3'UTR of a chordate gene, preferably a vertebrate gene, more preferably a mammalian gene, most preferably a human gene, or from a functional variant of the 3'UTR of a chordate gene, preferably a vertebrate gene, more preferably a mammalian gene, most preferably a human gene as described above.

Associating the artificial nucleic acid molecule or the vector with a 5 'UTR element and preferably a histone stem-loop as well as optional further elements in the context of the present invention preferably means functionally associating or functionally combining an artificial nucleic acid molecule, e.g. comprising an ORF, such as an mRNA or a vector, with the 5'UTR element and optionally the histone stem-loop and/or the poly(A) sequence and/or the 3'UTR element. This means that the artificial nucleic acid molecule, preferably the ORF contained within the artificial nucleic acid molecule, the 5'UTR element and preferably the histone stem-loop and the optional further elements, such as the poly(A)sequence and/or the 3'UTR element as described above, are associated or coupled such that the function of the 5'UTR element and the histone stem-loop and the optional further elements, e.g. protein production increasing function, is exerted. Typically, this means that the 5'UTR element and the histone stem-loop and optionally the poly(A)sequence and/or the 3'UTR element are integrated into the artificial nucleic acid molecule, preferably into the mRNA molecule or the vector, such that the open reading frame is positioned between the 5'UTR element and the optional histone stem-loop and the optional poly(A)sequence and/or the optional 3'UTR element.

The product of said method is preferably the artificial nucleic acid molecule according to the present invention or the vector according to the present invention. Thus, e.g. the nature and sequence of the elements, such as the 5'UTR element, the histone stem-loop, the poly(A) sequence, the polyadenylation signal, the poly(C) sequence, and the 3'UTR element are as described above for the artificial nucleic acid molecule according to the present invention or the vector according to the present invention.

In a further aspect, the present invention provides the use of at least one 5'-untranslated region element (5'UTR

element), which comprises or consists of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence,

or

a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence, preferably at least one histone stem-loop, and optionally further elements, such as a poly(A)sequence and/or a polyadenylation signal, and/or a poly(C) signal), and/or a 3'UTR element which comprises or consists of a nucleic acid sequence derived from the 3'UTR of a chordate gene, preferably a vertebrate gene, more preferably a mammalian gene, most preferably a human gene, or from a functional variant of the 3'UTR of a chordate gene, preferably a vertebrate gene, more preferably a mammalian gene, most preferably a human gene as described above for increasing protein production from an artificial nucleic acid molecule, such as an mRNA or a vector.

The use according to the present invention preferably comprises associating the artificial nucleic acid molecule with the 5'UTR element, preferably the histone stem-loop and optional further elements, such as a poly(A)sequence or 3'UTR element etc., as described above.

The compounds and ingredients of the inventive pharmaceutical composition may also be manufactured and traded separately of each other. Thus, the invention relates further to a kit or kit of parts comprising an artificial nucleic acid molecule according to the invention, a vector according to the present invention, a cell according to the invention, and/or a pharmaceutical composition according to the invention. Preferably, such kit or kit of parts may, additionally, comprise instructions for use, cells for transfection, an adjuvant, a means for administration of the pharmaceutical composition, a pharmaceutically acceptable carrier and/or an pharmaceutically acceptable solution for dissolution or dilution of the artificial nucleic acid molecule, the vector, the cells or the pharmaceutical composition.

The following Figures, Sequences and Examples are intended to illustrate the invention further. They are not intended to limit the subject-matter of the invention thereto.

Figure 1: shows the histone stem-loop consensus sequence generated from metazoan and protozoan stem-loop sequences (as reported by Dávila López, M., & Samuelsson, T. (2008), RNA (New York, N.Y.), 14(1), 1-10. doi:10.1261/rna.782308). 4001 histone stem-loop sequences from metazoa and protozoa were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 2: shows the histone stem-loop consensus sequence generated from protozoan stem-loop sequences (as reported by Dávila López, M., & Samuelsson, T. (2008), RNA (New York, N.Y.), 14(1), 1-10. doi:10.1261/rna.782308). 131 histone stem-loop sequences from protozoa were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 3: shows the histone stem-loop consensus sequence generated from metazoan stem-loop sequences (as reported by Dávila López, M., & Samuelsson, T. (2008), RNA (New York, N.Y.), 14(1), 1-10. doi:10.1261/rna.782308). 3870 histone stem-loop sequences from metazoa were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 4: shows the histone stem-loop consensus sequence generated from vertebrate stem-loop sequences (as reported by Dávila López, M., & Samuelsson, T. (2008), RNA (New York, N.Y.), 14(1), 1-10. doi:10.1261/rna.782308). 1333 histone stem-loop sequences from vertebrates were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

Figure 5: shows the histone stem-loop consensus sequence generated from human (Homo sapiens) stem-loop

sequences (as reported by Dávila López, M., & Samuelsson, T. (2008), RNA (New York, N.Y.), 14(1), 1-10. doi:10.1261/rna.782308). 84 histone stem-loop sequences from humans were aligned and the quantity of the occurring nucleotides is indicated for every position in the stem-loop sequence. The generated consensus sequence representing all nucleotides present in the sequences analyzed is given using the single-letter nucleotide code. In addition to the consensus sequence, sequences are shown representing at least 99%, 95% and 90% of the nucleotides present in the sequences analyzed.

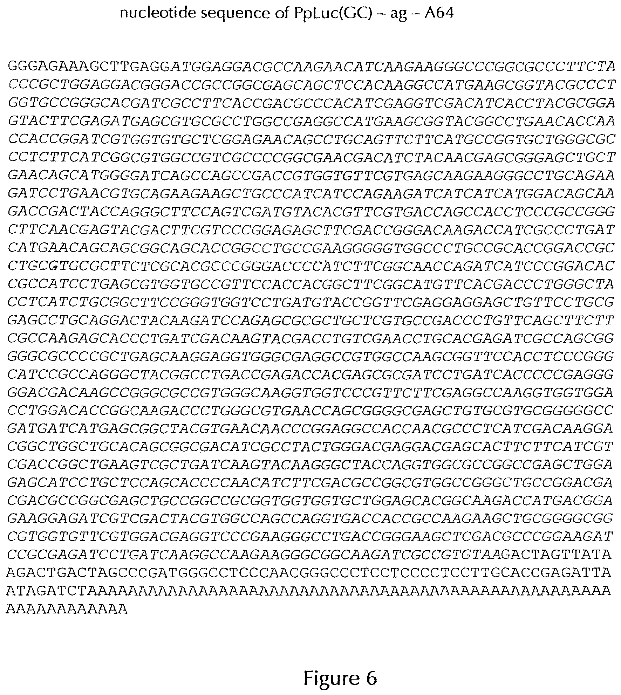

Figure 6 shows the nucleotide sequence of a *Photinus pyralis* luciferase encoding nucleic acid molecule PpLuc(GC) - ag - A64. This artificial construct does not comprise a 5'UTR element or a histone stem loop. The coding region for PpLuc(GC) is depicted in italics. The sequence depicted in Figure 6 corresponds to SEQ ID No. 1364.

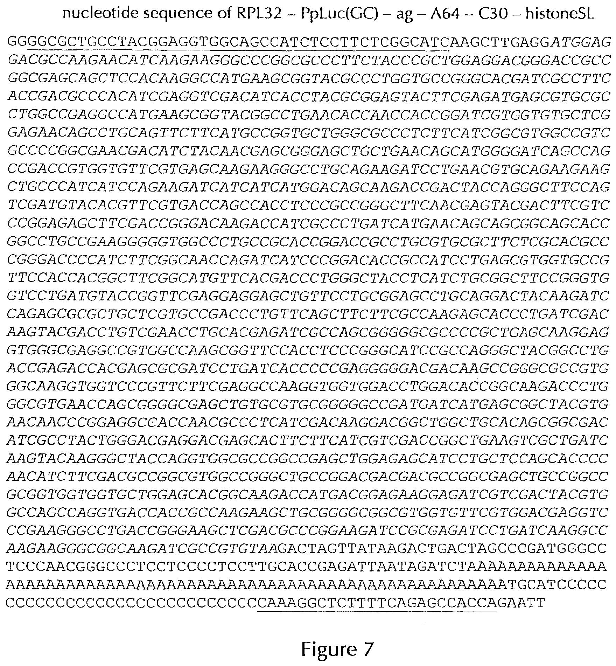

Figure 7 shows the nucleotide sequence of RPL32 - PpLuc(GC) - ag - A64-C30 - histoneSL. The 5'UTR of human ribosomal protein Large 32 lacking the 5' terminal oligopyrimidine tract was inserted 5' of the ORF. A histoneSL was appended 3' of A64 poly(A). The coding region for PpLuc(GC) is depicted in italics. The 5'UTR element sequence and the histone stem-loop sequence are underlined. The sequence depicted in Figure 7 corresponds to SEQ ID No. 1365.

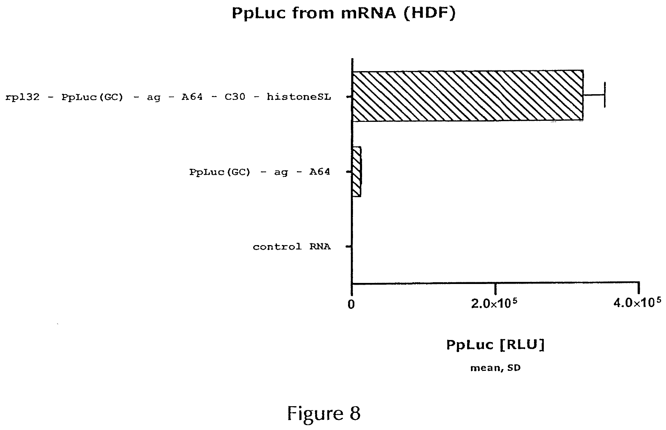

Figure 8 shows that the combination of the 5'UTR element derived from the 5'UTR of the TOP gene RPL32 and a histone stem-loop increases protein production from mRNA strongly. The effect of the combination of the 5'UTR element and the histone stem-loop on luciferase expression from mRNA was examined. To this end, different mRNAs were transfected into human dermal fibroblasts (HDF) by lipofection. Luciferase levels were measured at 24 hours after transfection. Luciferase was clearly expressed from mRNA having neither 5'UTR element nor histoneSL. Strikingly however, the combination of 5'UTR element and histoneSL strongly increased the luciferase level. The magnitude of the rise in luciferase level due to combining 5'UTR element and histoneSL in the same mRNA indicates that they are acting synergistically. Data are graphed as mean RLU ± SD (relative light units ± standard deviation) for duplicate transfections. RLU are summarized in Example 5.1.

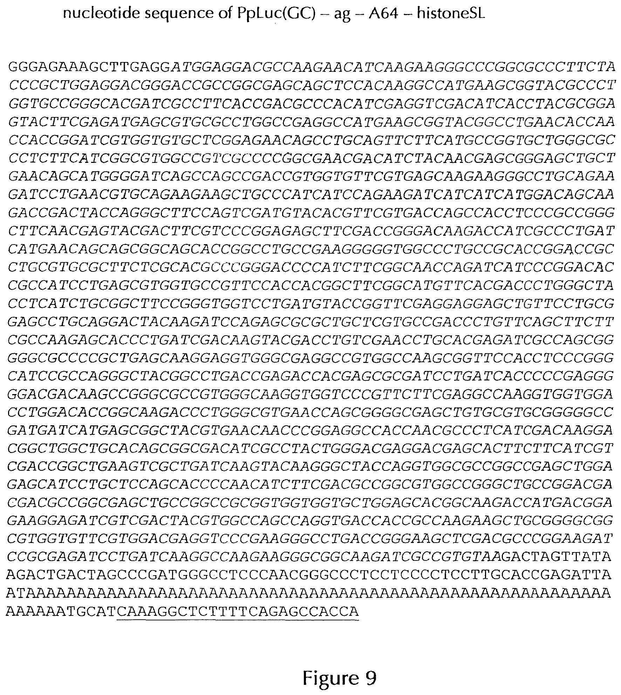

Figure 9 shows the nucleotide sequence of PpLuc(GC) - ag - A64 - histoneSL. A histoneSL was appended 3' of A64 poly(A). The coding region for PpLuc(GC) is depicted in italics. The histone stem-loop sequence is underlined. The sequence depicted in Figure 9 corresponds to SEQ ID No. 1464.

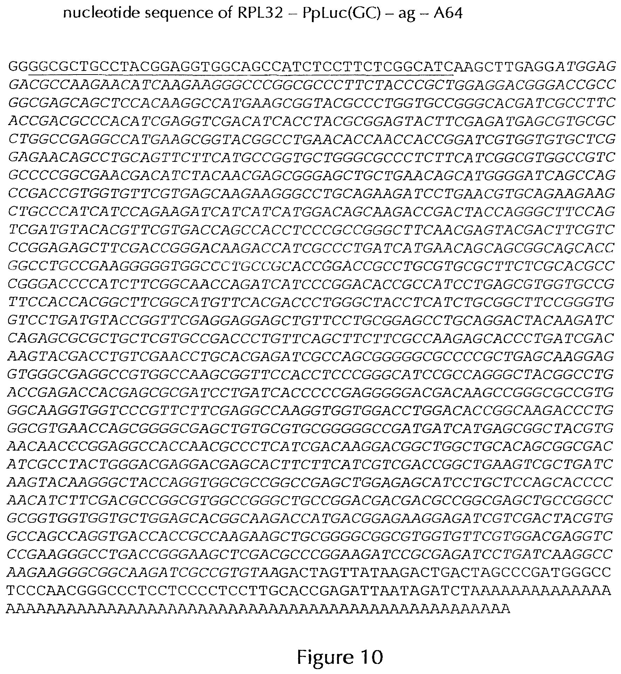

Figure 10 shows the nucleotide sequence of rpl32 - PpLuc(GC) - ag - A64. The 5'UTR of human ribosomal protein Large 32 lacking the 5' terminal oligopyrimidine tract was inserted 5' of the ORF. The coding region for PpLuc(GC) is depicted in italics. The 5'UTR element sequence is underlined. The sequence depicted in Figure 10 corresponds to SEQ ID No. 1463.

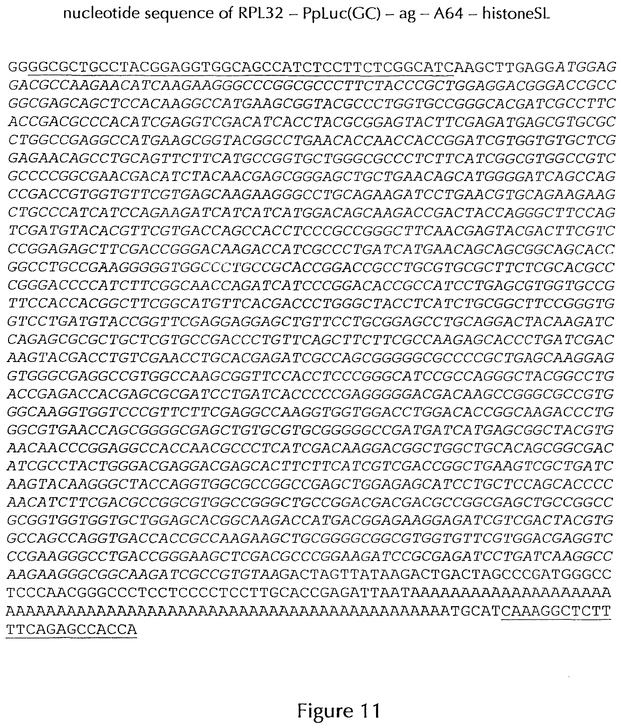

Figure 11 shows the nucleotide sequence of rpl32 - PpLuc(GC) - ag - A64 - histoneSL. The 5'UTR of human ribosomal protein Large 32 lacking the 5' terminal oligopyrimidine tract was inserted 5' of the ORF. A histoneSL was appended 3' of A64 poly(A). The coding region for PpLuc(GC) is depicted in italics. The 5'UTR element sequence and the histone stem-loop sequence are underlined. The sequence depicted in Figure 11 corresponds to SEQ ID No. 1480.

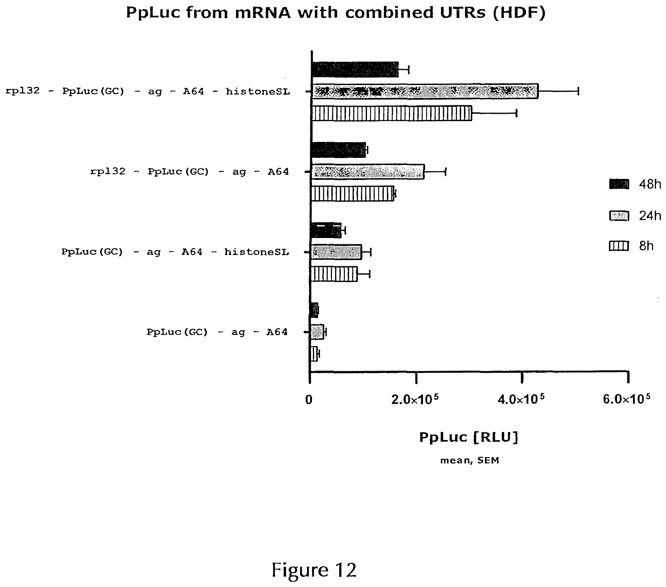

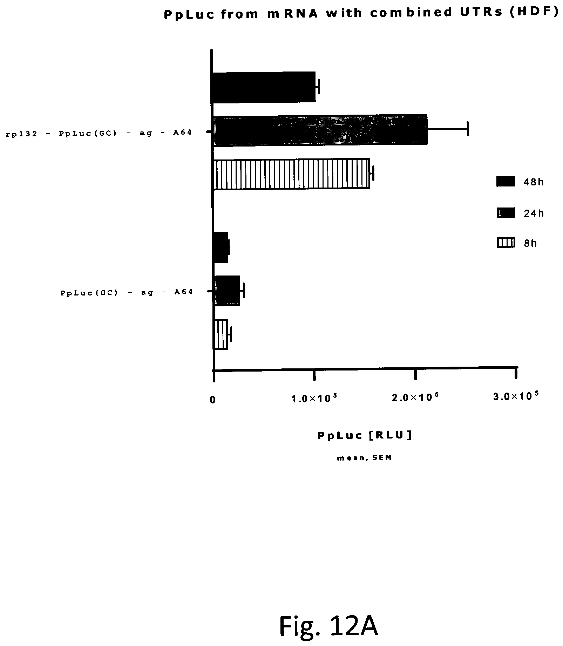

Figure 12 is a graphical representation of the effect of the 5'UTR element derived from the 5'UTR of the TOP gene RPL32, the histone stem-loop, and the combination of the 5'UTR element and the histone stem-loop on luciferase expression from mRNA. A variety of mRNAs were transfected into human dermal fibroblasts (HDF) by lipofection. Luciferase levels were measured at 8, 24, and 48 hours after transfection. Both, either the histone stem-loop or the 5'UTR element increase luciferase levels compared to mRNA lacking both these elements. Strikingly, the combination of 5'UTR element and histone stem-loop further strongly increases the luciferase level, much above the level observed with either of the individual elements, thus acting synergistically. Data are graphed as mean RLU ± SEM (relative light units ± standard error) for triplicate transfections. RLU are summarized in Example 5.2.

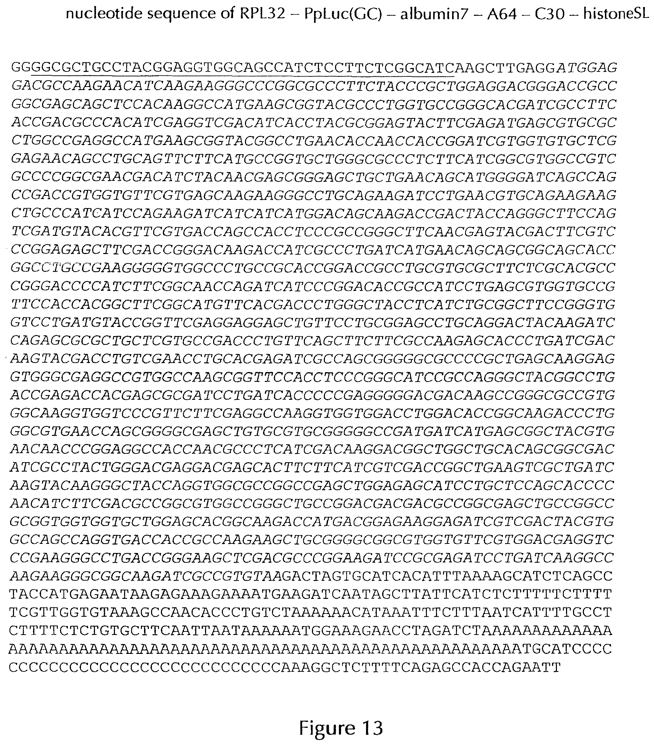

Figure 13 shows the nucleotide sequence of rpl32 - PpLuc(GC) - albumin7-A64 - C30 - histoneSL. The albumin7 3'UTR element replaced the alpha-globin 3'UTR element in the construct shown in Figure 7 (which contains the rpl32 5'UTR element). The 5'UTR element sequence is underlined. The sequence depicted in Figure 13 corresponds to SEQ ID No. 1481.

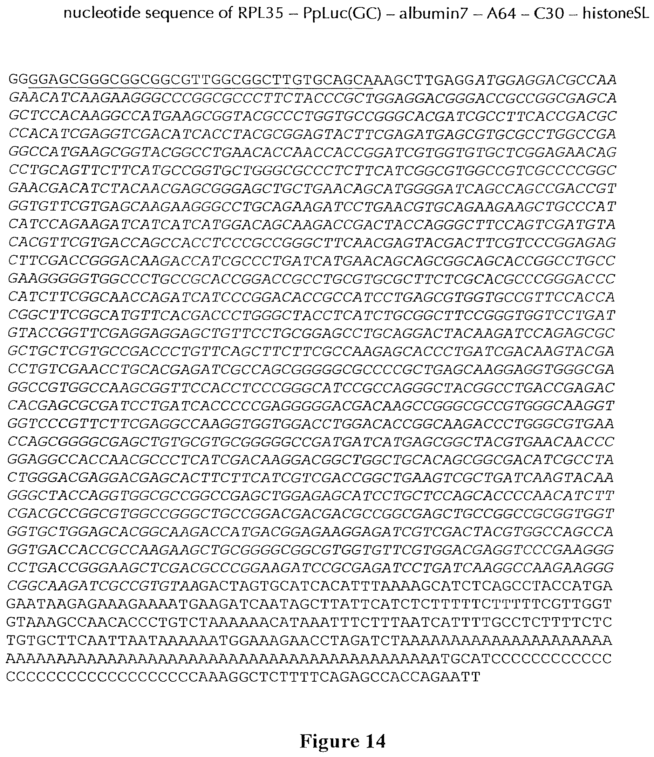

Figure 14 shows the nucleotide sequence of rpl35 - PpLuc(GC) - albumin7-A64 - C30 - histoneSL. The 5'UTR of human ribosomal protein Large 35 lacking the 5' terminal oligopyrimidine tract replaced the rpl32 5'UTR element in the construct shown in Figure 13. The 5'UTR element sequence is underlined. The sequence depicted in Figure 14 corresponds to SEQ ID No. 1436.

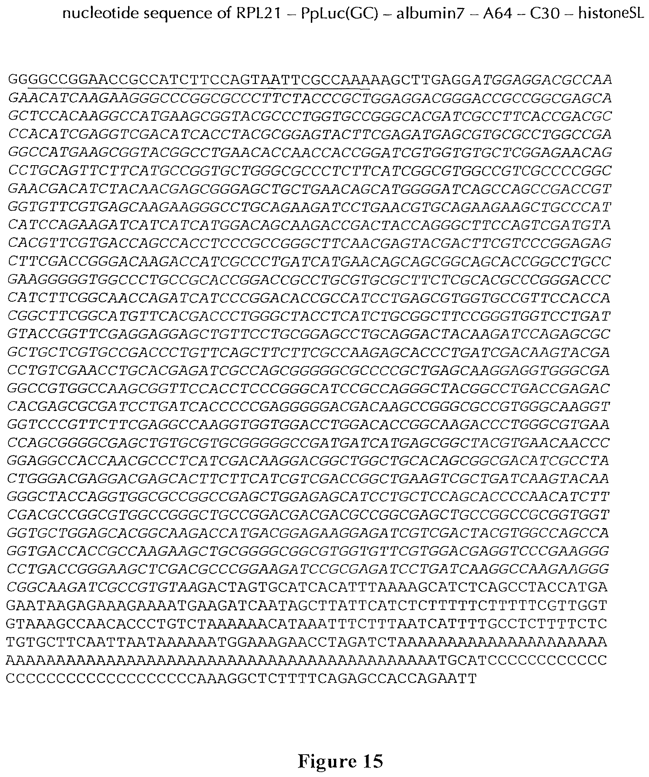

Figure 15 shows the nucleotide sequence of rpl21 - PpLuc(GC) - albumin7-A64 - C30 - histoneSL. The 5'UTR of human ribosomal protein Large 21 lacking the 5' terminal oligopyrimidine tract replaced the rpl32 5'UTR element in the construct shown in Figure 13. The 5'UTR element sequence is underlined. The sequence depicted in Figure 15 corresponds to SEQ ID No. 1437.

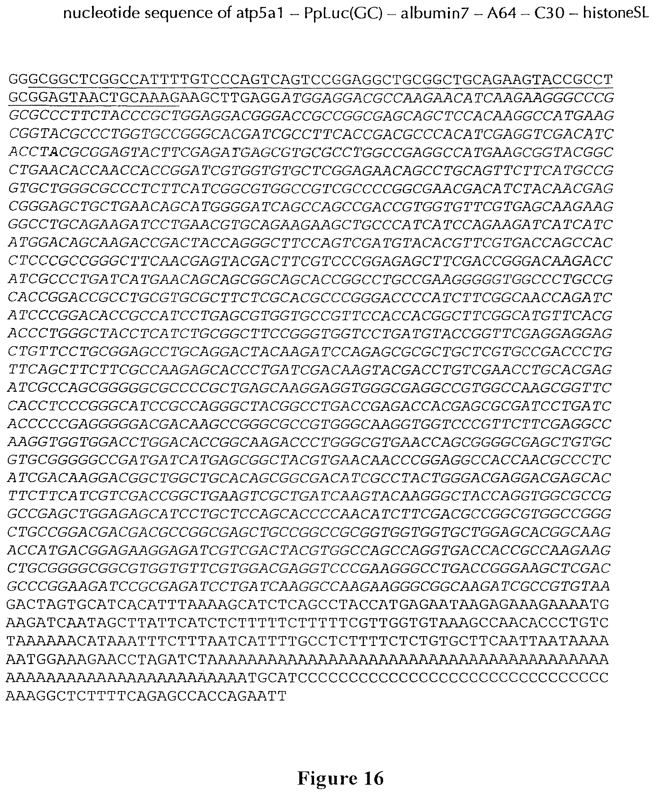

Figure 16 shows the nucleotide sequence of atp5a1 - PpLuc(GC) - albumin7-A64 - C30 - histoneSL. The 5'UTR of human ATP synthase, H+ transporting, mitochondrial F1 complex, alpha subunit 1 lacking the 5' terminal oligopyrimidine tract replaced the rpl32 5'UTR element in the construct shown in Figure 13. The 5'UTR element sequence is underlined. The sequence depicted in Figure 16 corresponds to SEQ ID No. 1438.

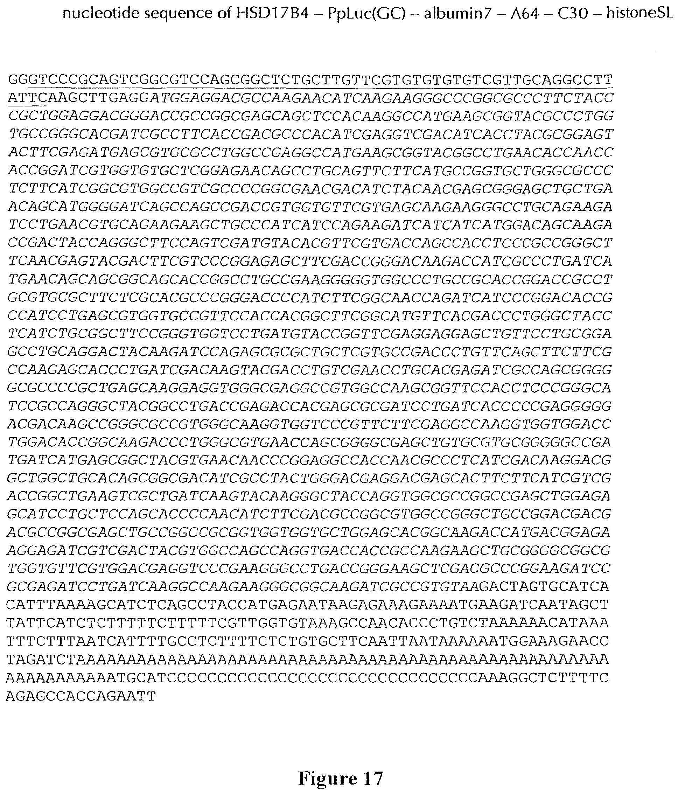

Figure 17 shows the nucleotide sequence of HSD17B4 - PpLuc(GC) - albumin7 - A64 - C30 - histoneSL. The 5'UTR of human hydroxysteroid (17-beta) dehydrogenase 4 lacking the 5' terminal oligopyrimidine tract replaced the rpl32 5'UTR element in the construct shown in Figure 13. The 5'UTR element sequence is underlined. The sequence depicted in Figure 17 corresponds to SEQ ID No. 1439.

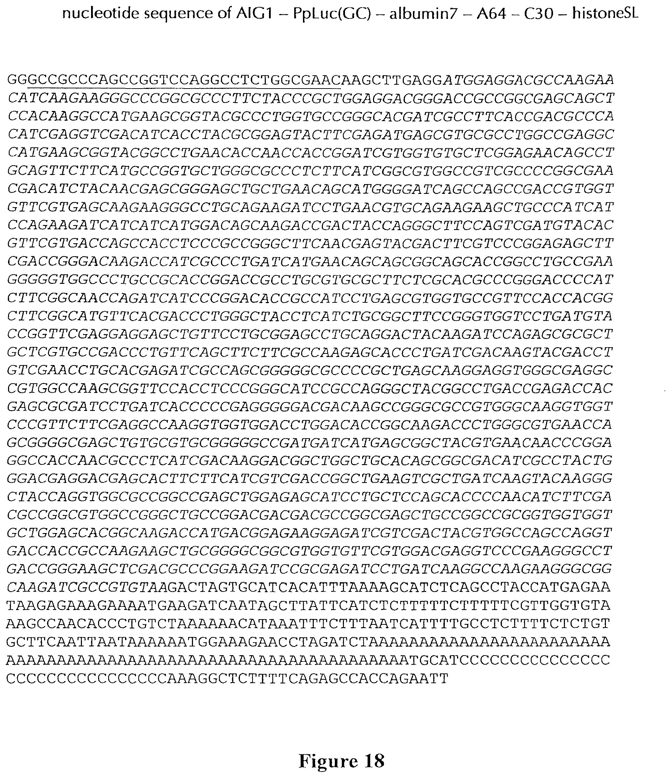

Figure 18 shows the nucleotide sequence of AIG1 - PpLuc(GC) - albumin7 - A64 - C30 - histoneSL. The 5'UTR of human androgen-induced 1 lacking the 5' terminal oligopyrimidine tract replaced the rpl32 5'UTR element in the construct shown in Figure 13. The 5'UTR element sequence is underlined. The sequence depicted in Figure 18 corresponds to SEQ ID No. 1440.

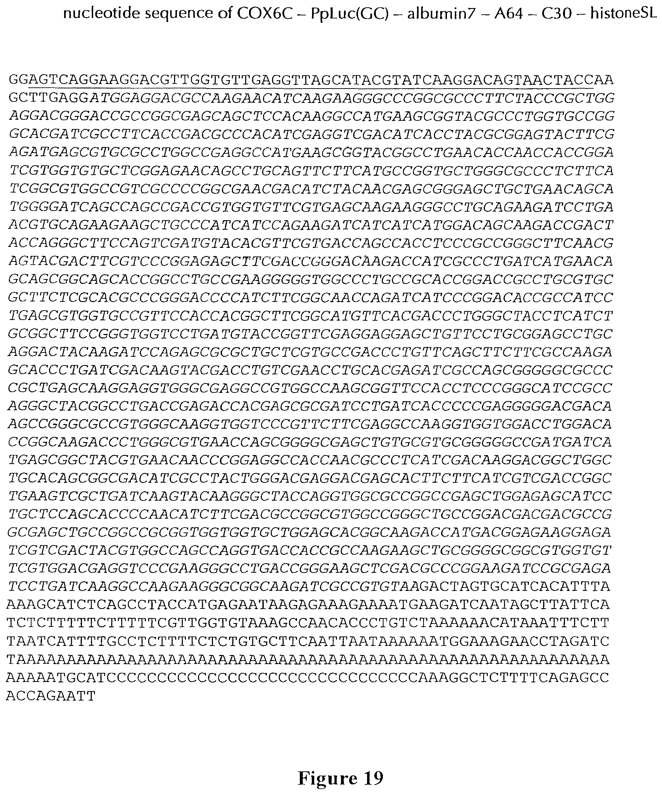

Figure 19 shows the nucleotide sequence of COX6C - PpLuc(GC) - albumin7 - A64 - C30 - histoneSL. The 5'UTR of human cytochrome c oxidase subunit VIc lacking the 5' terminal oligopyrimidine tract replaced the rpl32 5'UTR element in the construct shown in Figure 13. The 5'UTR element sequence is underlined. The sequence depicted in Figure 19 corresponds to SEQ ID No. 1441.

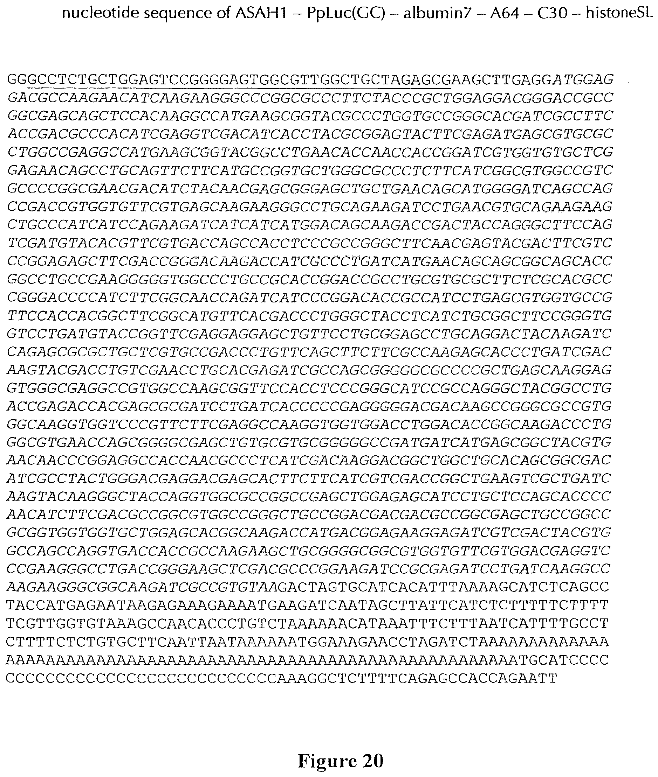

Figure 20 shows the nucleotide sequence of ASAH1 - PpLuc(GC) - albumin7 - A64 - C30 - histoneSL. The 5'UTR of human N-acylsphingosine amidohydrolase (acid ceramidase) 1 lacking the 5' terminal oligopyrimidine tract replaced the rpl32 5'UTR element in the construct shown in Figure 13. The 5'UTR element sequence is underlined. The sequence depicted in Figure 20 corresponds to SEQ ID No. 1442.

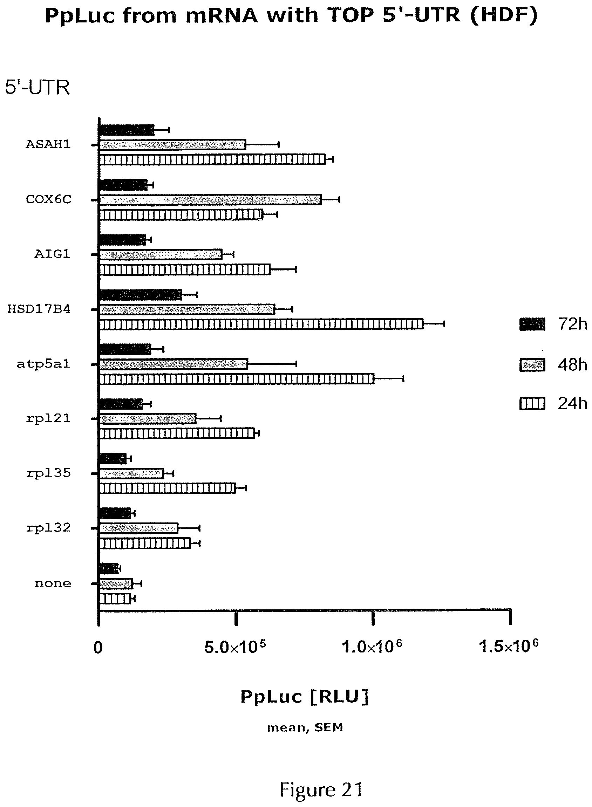

Figure 21 is a graphical representation of the effect of the 5'UTR element derived from the TOP genes RPL32, RPL35, RPL21, ATP5A1, HSD17B4, AIG1, COX6C and ASAH1 on luciferase expression from mRNA. The mRNAs were transfected into human dermal fibroblasts (HDF) by lipofection. Luciferase levels were measured at 24, 48, and 72 hours after transfection. The 5'UTR elements strongly increase luciferase levels compared to mRNA lacking a 5'UTR element. Data are graphed as mean RLU ± SEM (relative light units ± standard error) for triplicate transfections. RLU are summarized in Example 5.3.

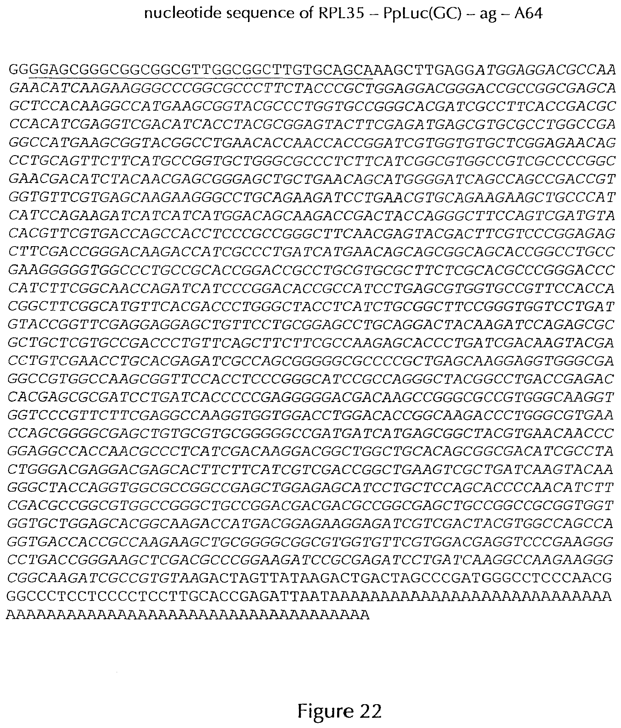

Figure 22 shows the nucleotide sequence of rpl35 - PpLuc(GC) - ag - A64. The 5'UTR of human ribosomal protein Large 35 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 22 corresponds to SEQ ID No. 1466.

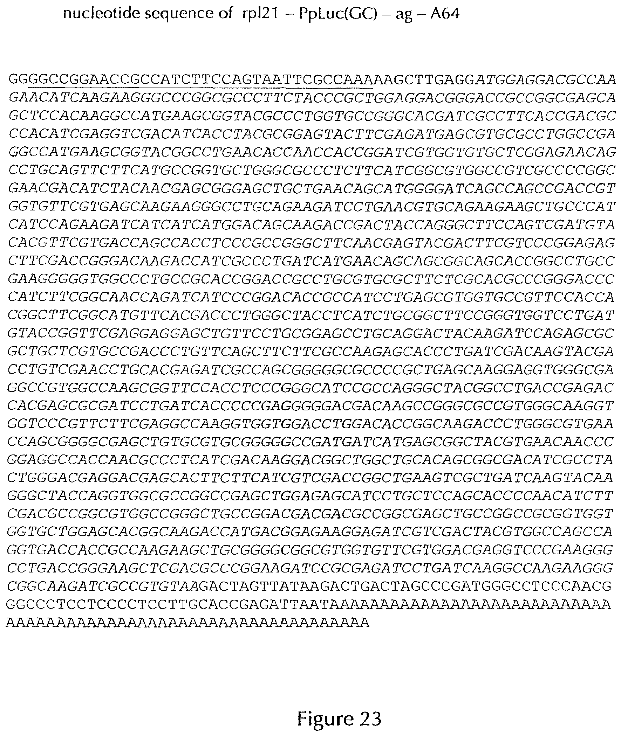

Figure 23 shows the nucleotide sequence of rpl21 - PpLuc(GC) - ag - A64. The 5'UTR of human ribosomal protein Large 21 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 23 corresponds to SEQ ID No. 1467.

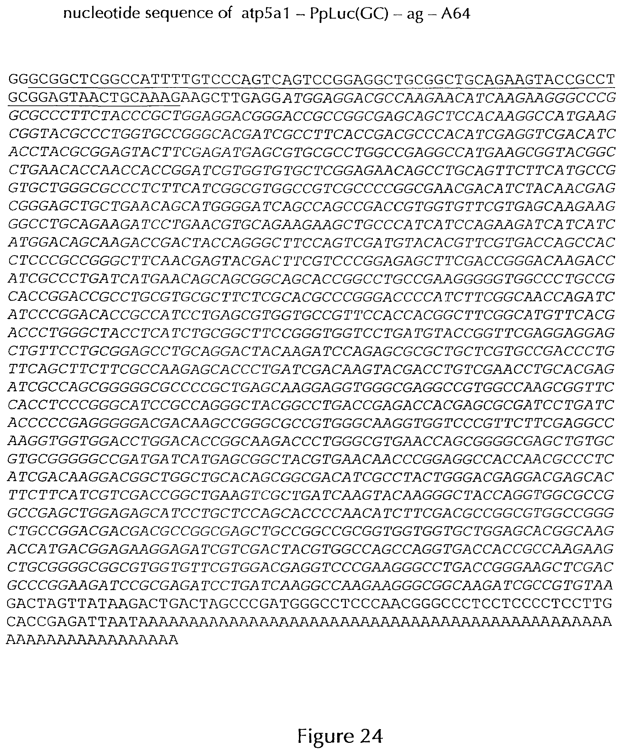

Figure 24 shows the nucleotide sequence of atp5a1 - PpLuc(GC) - ag - A64. The 5'UTR of human ATP synthase, H+ transporting, mitochondrial F1 complex, alpha subunit 1 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 24 corresponds to SEQ ID No. 1468.

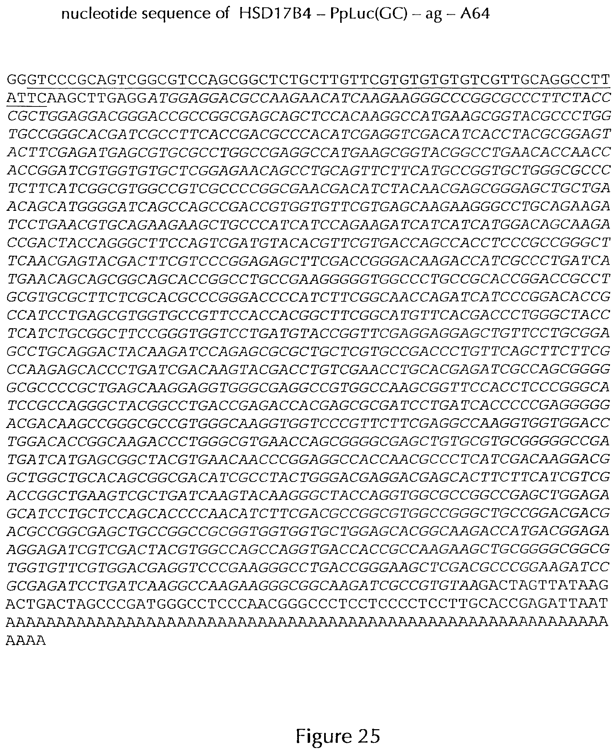

Figure 25 shows the nucleotide sequence of HSD17B4 - PpLuc(GC) - ag - A64. The 5'UTR of human hydroxysteroid (17-beta) dehydrogenase 4 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 25 corresponds to SEQ ID No. 1469.

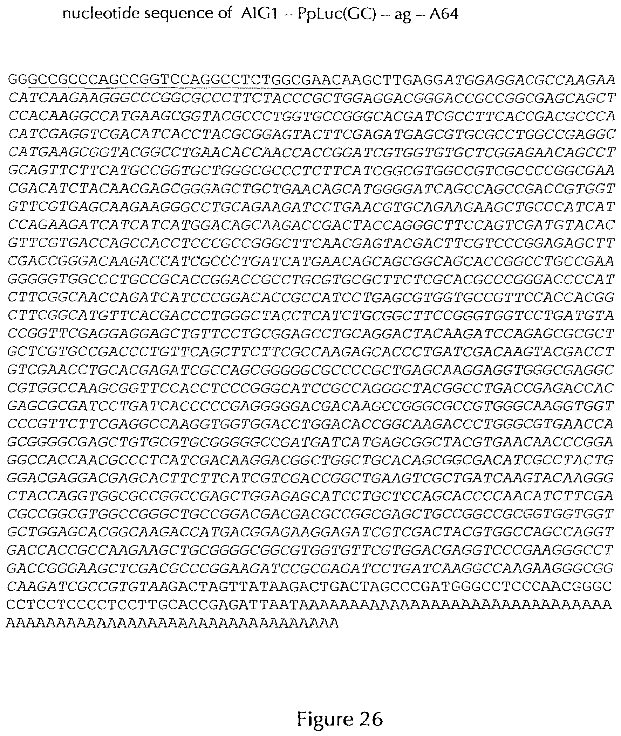

Figure 26 shows the nucleotide sequence of AIG1 - PpLuc(GC) - ag - A64. The 5'UTR of human androgen-induced 1 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 26 corresponds to SEQ ID No. 1470.

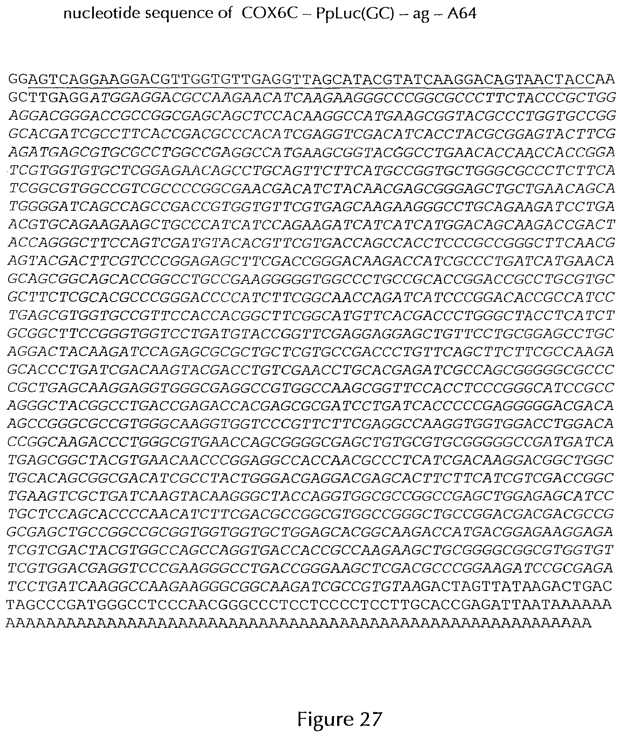

Figure 27 shows the nucleotide sequence of COX6C - PpLuc(GC) - ag - A64. The 5'UTR of human cytochrome c oxidase subunit VIc lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 27 corresponds to SEQ ID No. 1471.

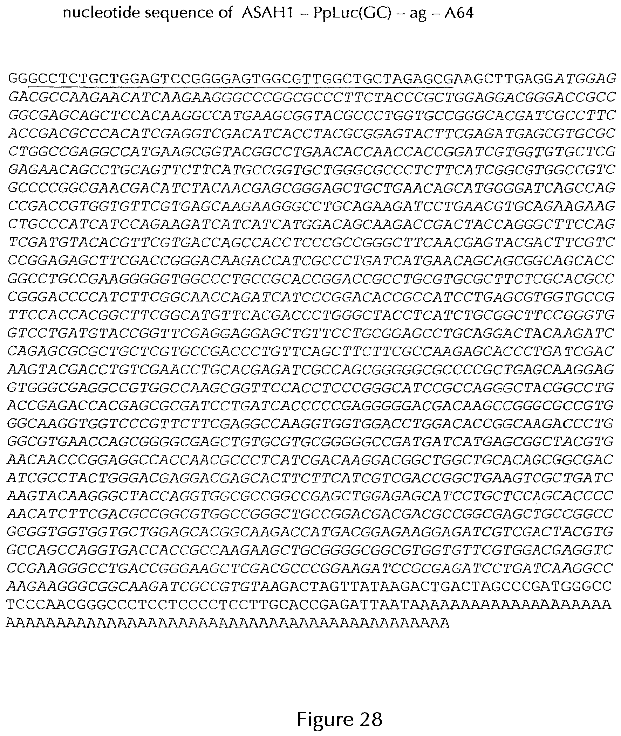

Figure 28 shows the nucleotide sequence of ASAH1 - PpLuc(GC) - ag - A64. The 5'UTR of human N-acylsphingosine amidohydrolase (acid ceramidase) 1 lacking the 5' terminal oligopyrimidine tract replaced the rpl32 5'UTR element in the construct shown in Figure 10. The coding region for PpLuc(GC) is depicted in italics. The 5'UTR element sequence is underlined. The sequence depicted in Figure 28 corresponds to SEQ ID No. 1472.

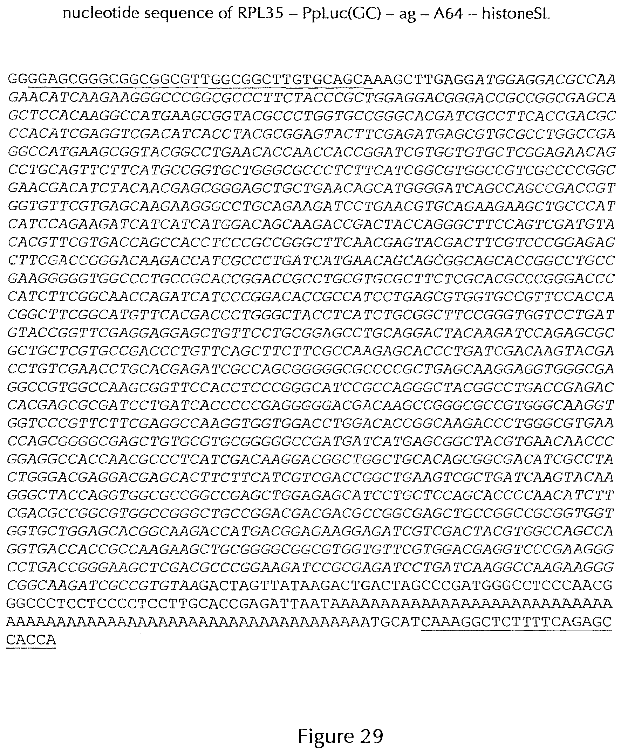

Figure 29 shows the nucleotide sequence of rpl35 - PpLuc(GC) - ag - A64 - histoneSL. The 5'UTR of human ribosomal protein Large 35 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 29 corresponds to SEQ ID No. 1473.

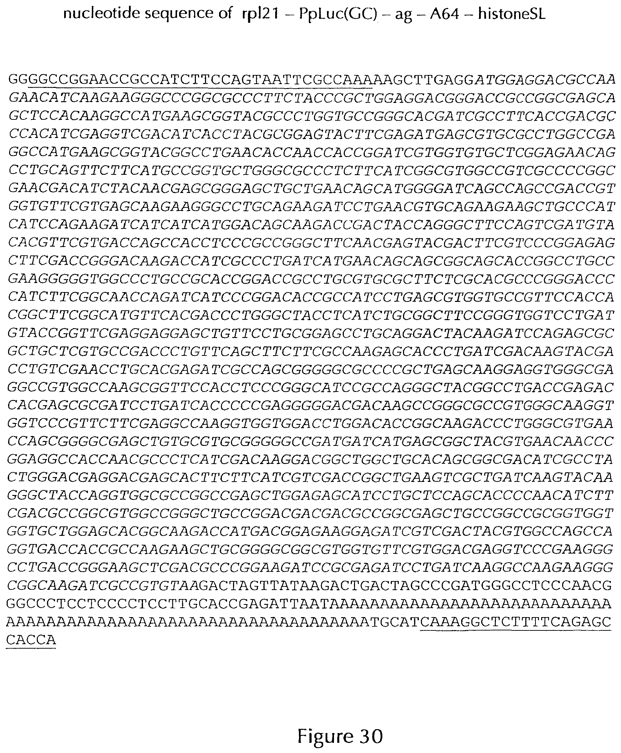

Figure 30 shows the nucleotide sequence of rpl21 - PpLuc(GC) - ag - A64 - histoneSL. The 5'UTR of human ribosomal protein Large 21 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 30 corresponds to SEQ ID No. 1474.

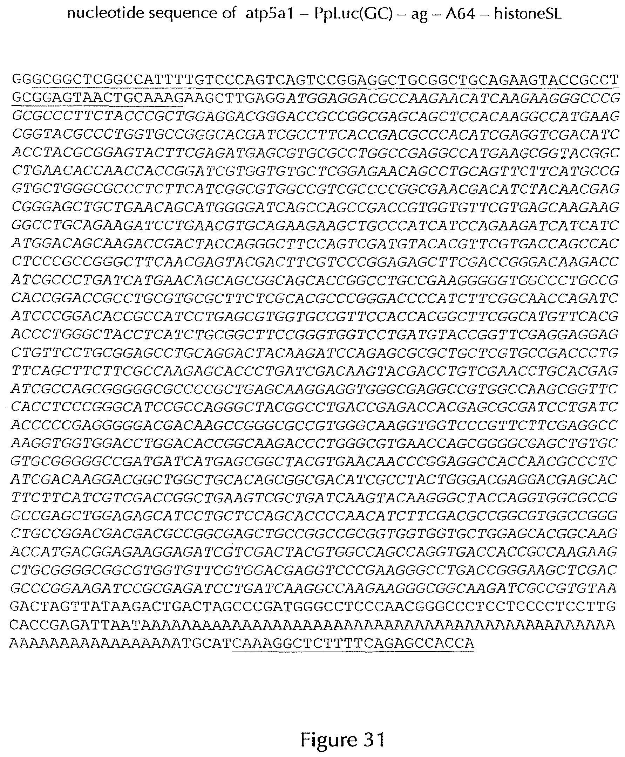

Figure 31 shows the nucleotide sequence of atp5a1 - PpLuc(GC) - ag - A64 - histoneSL. The 5'UTR of human ATP synthase, H+ transporting, mitochondrial F1 complex, alpha subunit 1 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 31 corresponds to SEQ ID No. 1475.

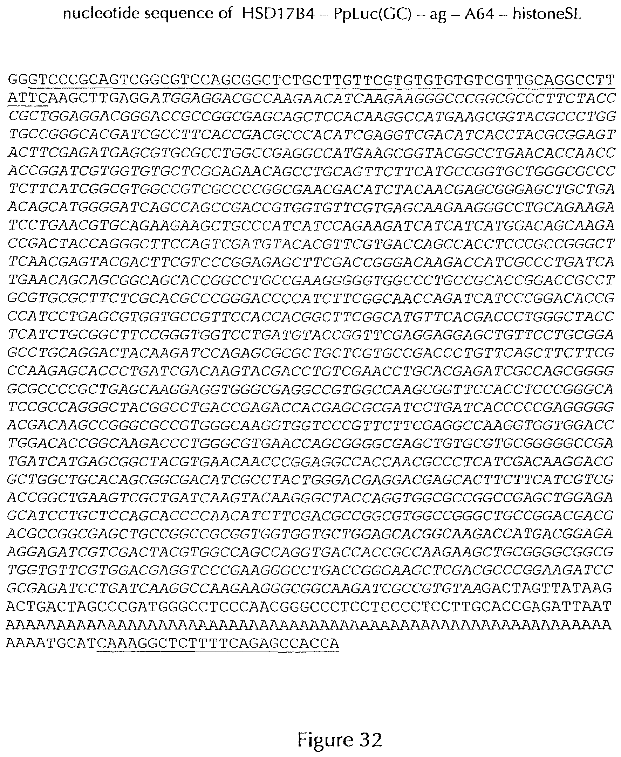

Figure 32 shows the nucleotide sequence of HSD17B4 - PpLuc(GC) - ag - A64 - histoneSL. The 5'UTR of human hydroxysteroid (17-beta) dehydrogenase 4 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 32 corresponds to SEQ ID No. 1476.

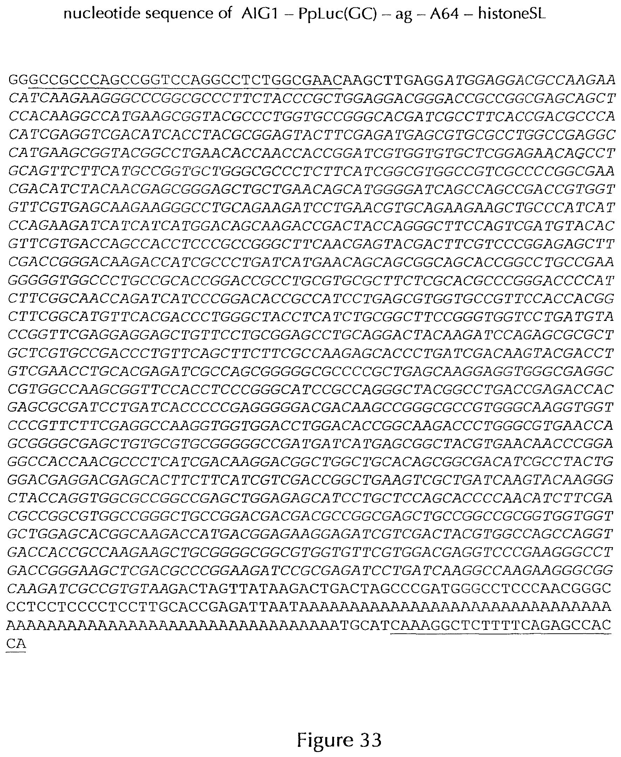

Figure 33 shows the nucleotide sequence of AIG1 - PpLuc(GC) - ag - A64 - histoneSL. The 5'UTR of human androgen-induced 1 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 33 corresponds to SEQ ID No. 1477.

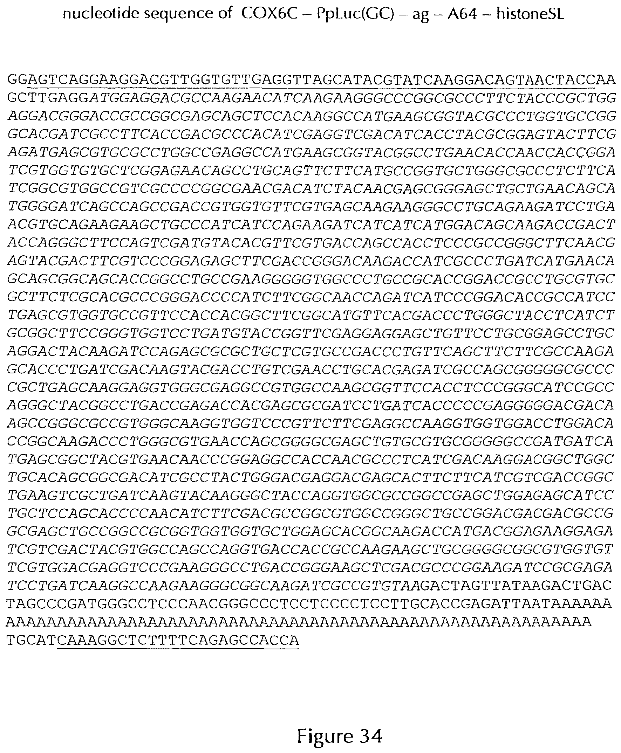

Figure 34 shows the nucleotide sequence of COX6C - PpLuc(GC) - ag - A64 - histoneSL. The 5'UTR of human cytochrome c oxidase subunit VIc lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 34 corresponds to SEQ ID No. 1478.

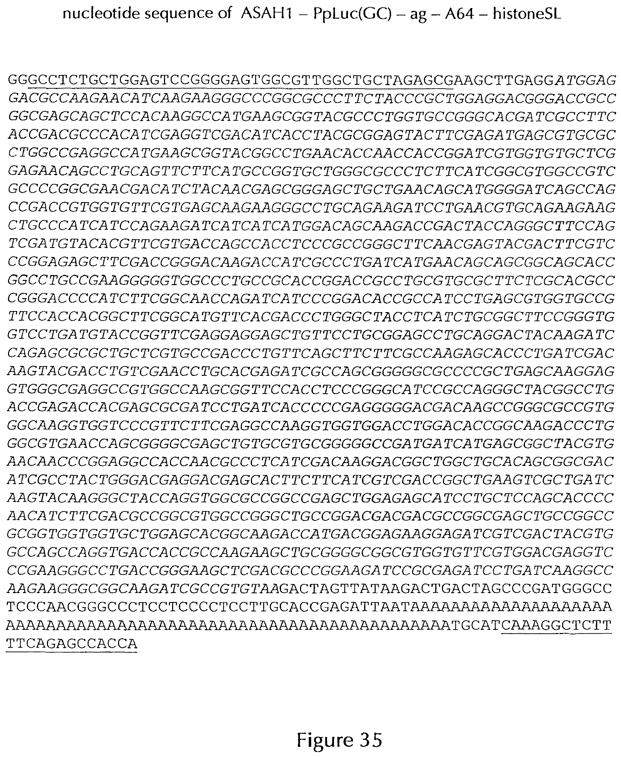

Figure 35 shows the nucleotide sequence of ASAH1 - PpLuc(GC) - ag - A64 - histoneSL. The 5'UTR of human N-acylsphingosine amidohydrolase (acid ceramidase) 1 lacking the 5' terminal oligopyrimidine tract replaced the rpl32 5'UTR element in the construct shown in Figure 11. The coding region for PpLuc(GC) is depicted in italics. The 5'UTR element sequence and the histone stem-loop sequence are underlined. The sequence depicted in Figure 35 corresponds to SEQ ID No. 1479.

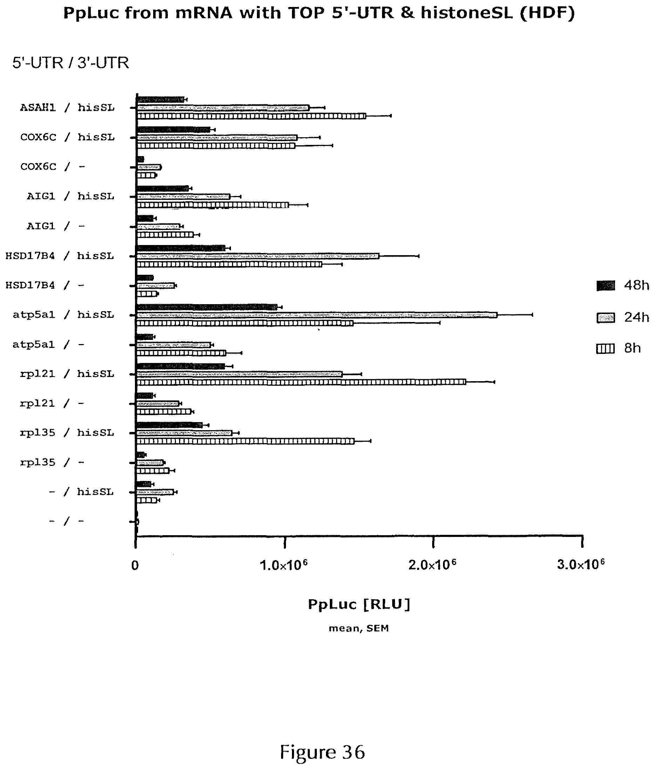

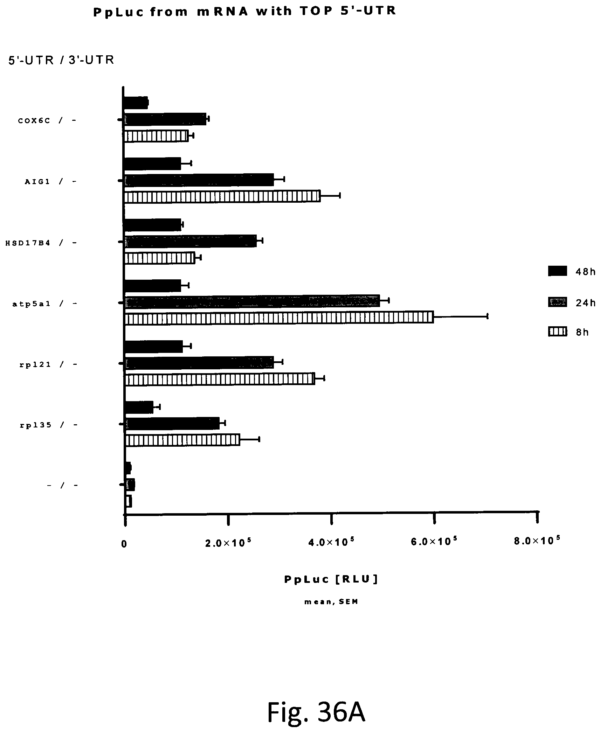

Figure 36 is a graphical representation of the effect of 5'UTR elements derived from 5'UTRs of the TOP genes RPL35, RPL21, ATP5A1, HSD17B4, AIG1, COX6C and ASAH1, the histone stem-loop, and the combination of 5'UTR elements and histone stem-loop on luciferase expression from mRNA. The different mRNAs were transfected into human dermal fibroblasts (HDF) by lipofection. Luciferase levels were measured at 8, 24, and 48 hours after transfection. Both, either the histone stem-loop or the 5'UTR elements increase luciferase levels compared to mRNA lacking both these elements. Strikingly, the combination of 5'UTR elements and histone stem-loop further strongly increases the luciferase level, much above the level observed with either of the individual elements, thus acting synergistically. Data are graphed as mean RLU ± SEM (relative light units ± standard error) for triplicate transfections. The synergy between 5'UTR elements and histone stem-loop is summarized in example 5.4.

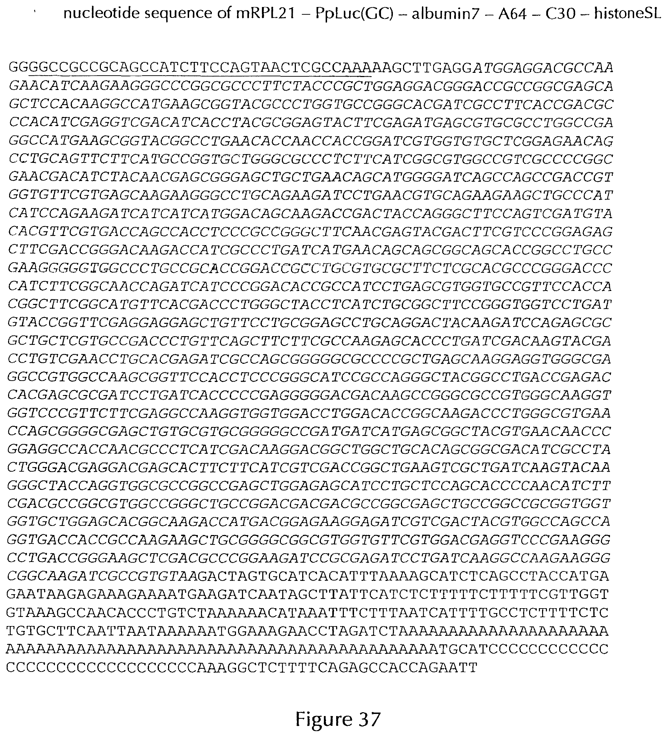

Figure 37 shows the nucleotide sequence of mrpl21 - PpLuc(GC) - albumin7-A64 - C30 - histoneSL. The 5'UTR of murine ribosomal protein Large 21 lacking the 5' terminal oligopyrimidine tract replaced the rpl32 5'UTR element in the construct shown in Figure 13. The 5'UTR element sequence is underlined. The sequence depicted in Figure 36 corresponds to SEQ ID No. 1443.

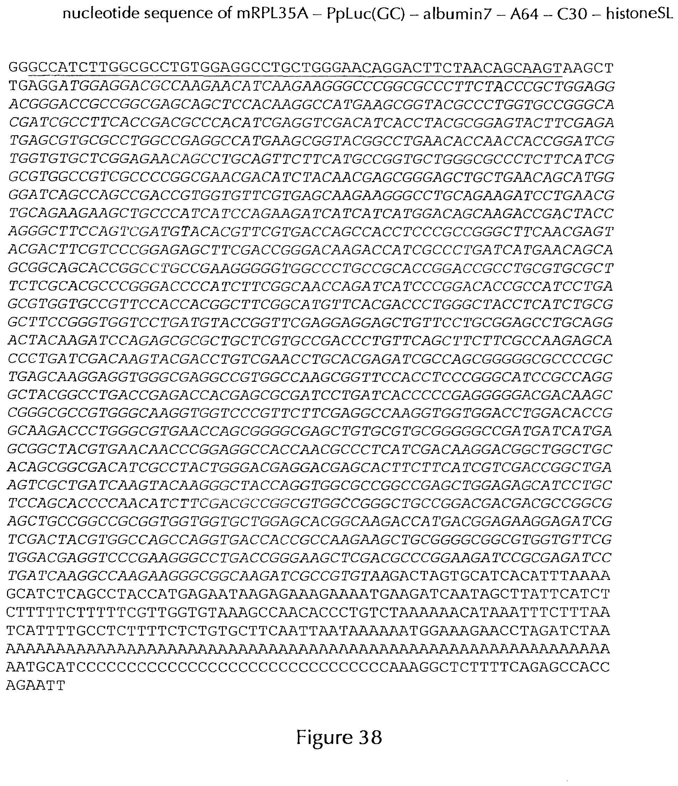

Figure 38 shows the nucleotide sequence of mrpl35A - PpLuc(GC) - albumin7 - A64 - C30 - histoneSL. The 5'UTR of murine ribosomal protein Large 35A lacking the 5' terminal oligopyrimidine tract replaced the rpl32 5'UTR element in the construct shown in Figure 13. The 5'UTR element sequence is underlined. The sequence depicted in Figure 37 corresponds to SEQ ID No. 1444.

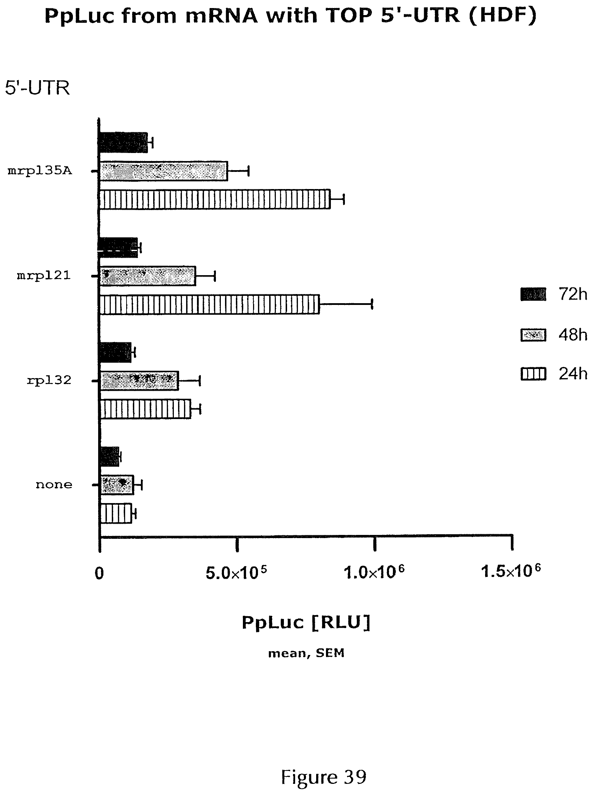

Figure 39 is a graphical representation of the effect of the 5'UTR elements derived from 5'UTRs of mouse TOP genes on luciferase expression from mRNA. mRNAs containing either a mouse or a human 5'UTR element were transfected into human dermal fibroblasts (HDF) by lipofection. Luciferase levels were measured at 24, 48, and 72 hours after transfection. Mouse 5'UTR elements strongly increase luciferase levels compared to mRNA lacking a 5'UTR element, similarly as the human 5'UTR element. Data are graphed as mean RLU ± SEM (relative light units ± standard error) for triplicate transfections. RLU are summarized in Example 5.5.

| | |
|---|---|
| SEQ ID No. 1364 | PpLuc(GC) - ag - A64 (Fig. 6) |
| SEQ ID No. 1365 | RPL32 - PpLuc(GC) - ag - A64 - C30 - histoneSL (Fig. 7) |
| SEQ ID No. 1366 | fragment of the 5'UTR of human ribosomal protein Large 32 |
| SEQ ID No. 1367 | fragment of the 5'UTR of human ribosomal protein Large 32 |
| SEQ ID No. 1368 | 5'UTR of human ribosomal protein Large 32 lacking the 5' terminal oligopyrimidine tract |
| SEQ ID No. 1369 | Human albumin 3'UTR |
| SEQ ID No. 1370 | 3'UTR of Homo sapiens hemoglobin, alpha 1 (HBA1) |
| SEQ ID No. 1371 | 3'UTR of Homo sapiens hemoglobin, alpha 2 (HBA2) |
| SEQ ID No. 1372 | 3'UTR of Homo sapiens hemoglobin, beta (HBB) |
| SEQ ID No. 1373 | 3'UTR of Homo sapiens tyrosine hydroxylase (TH) |
| SEQ ID No. 1374 | 3'UTR of Homo sapiens arachidonate 15-lipoxygenase (ALOX15) |

(continued)

| | |
|---|---|
| SEQ ID No. 1375 | 3'UTR of Homo sapiens collagen, type I, alpha 1 (COL1A1) |
| SEQ ID No. 1376 | albumin7 3'UTR |
| SEQ ID No. 1377 | Human albumin 3'UTR + poly(A) sequence |
| SEQ ID No. 1378 | Human albumin 3'UTR fragment 1 |
| SEQ ID No. 1379 | Human albumin 3'UTR fragment 2 |
| SEQ ID No. 1380 | Human albumin 3'UTR fragment 3 |
| SEQ ID No. 1381 | Human albumin 3'UTR fragment 4 |
| SEQ ID No. 1382 | Human albumin 3'UTR fragment 5 |
| SEQ ID No. 1383 | Human albumin 3'UTR fragment 6 |
| SEQ ID No. 1384 | Human albumin 3'UTR fragment 7 |
| SEQ ID No. 1385 | Human albumin 3'UTR fragment 8 |
| SEQ ID No. 1386 | Human albumin 3'UTR fragment 9 |
| SEQ ID No. 1387 | Human albumin 3'UTR fragment 10 |
| SEQ ID No. 1388 | Human albumin 3'UTR fragment 11 |
| SEQ ID No. 1389 | Human albumin 3'UTR fragment 12 |
| SEQ ID No. 1390 | Human albumin 3'UTR fragment 13 |
| SEQ ID NO. 1391 | Sequence according to formula (Ic) |
| SEQ ID NO. 1392 | Sequence according to formula (IIc): |
| SEQ ID NO. 1393 | Sequence according to formula (Id): |
| SEQ ID NO. 1394 | Sequence according to formula (IId) |
| SEQ ID NO. 1395 | Sequence according to formula (Ie) |
| SEQ ID NO. 1396 | Sequence according to formula (IIe) |
| SEQ ID NO. 1397 | Sequence according to formula (If) |
| SEQ ID NO. 1398 | Sequence according to formula (IIf) |
| SEQ ID NO. 1399 | Sequence according to formula (Ig) |
| SEQ ID NO. 1400 | Sequence according to formula (IIg) |

(continued)

| | |
|---|---|
| SEQ ID NO. 1401 | Sequence according to formula (Ih) |
| SEQ ID NO. 1402 | Sequence according to formula (IIh) |
| SEQ ID NO. 1403 | Sequence according to formula (Ic) |
| SEQ ID NO. 1404 | Sequence according to formula (Ic) |
| SEQ ID NO. 1405 | Sequence according to formula (Ic) |
| SEQ ID NO. 1406 | Sequence according to formula (Ie) |
| SEQ ID NO. 1407 | Sequence according to formula (Ie) |
| SEQ ID NO. 1408 | Sequence according to formula (Ie) |
| SEQ ID NO. 1409 | Sequence according to formula (If) |
| SEQ ID NO. 1410 | Sequence according to formula (If) |
| SEQ ID NO. 1411 | Sequence according to formula (If) |
| SEQ ID NO. 1412 | Sequence according to formula (Ig) |
| SEQ ID NO. 1413 | Sequence according to formula (Ig) |
| SEQ ID NO. 1414 | Sequence according to formula (Ig) |
| SEQ ID NO. 1415 | Sequence according to formula (Ih) |
| SEQ ID NO. 1416 | Sequence according to formula (Ih) |
| SEQ ID NO. 1417 | Sequence according to formula (Ih) |
| SEQ ID NO. 1418 | Sequence according to formula (IIc) |
| SEQ ID NO. 1419 | Sequence according to formula (IIc) |
| SEQ ID NO. 1420 | Sequence according to formula (IIc) |
| SEQ ID NO. 1421 | Sequence according to formula (IIe) |
| SEQ ID NO. 1422 | Sequence according to formula (IIe) |
| SEQ ID NO. 1423 | Sequence according to formula (IIe) |
| SEQ ID NO. 1424 | Sequence according to formula (IIf) |
| SEQ ID NO. 1425 | Sequence according to formula (IIf) |
| SEQ ID NO. 1426 | Sequence according to formula (IIf) |

(continued)

| | |
|---|---|
| SEQ ID NO. 1427 | Sequence according to formula (IIg) |
| SEQ ID NO. 1428 | Sequence according to formula (IIg) |
| SEQ ID NO. 1429 | Sequence according to formula (IIg) |
| SEQ ID NO. 1430 | Sequence according to formula (IIh) |
| SEQ ID NO. 1431 | Sequence according to formula (IIh) |
| SEQ ID NO. 1432 | Sequence according to formula (IIh) |
| SEQ ID NO. 1433 | Example histone stem-loop sequence |
| SEQ ID NO. 1434 | Center, $\alpha$-complex-binding portion of the 3'UTR of an $\alpha$-globin gene |
| SEQ ID NO. 1435 | ATP synthase lipid-binding protein, mitochondrial (atp5g2) |
| SEQ ID NO. 1436 | RPL35 - PpLuc(GC) - albumin7 - A64 - C30 - histoneSL (Fig. 14) |
| SEQ ID NO. 1437 | RPL21 - PpLuc(GC) - albumin7 - A64 - C30 - histoneSL (Fig. 15) |
| SEQ ID NO. 1438 | ATP5A1 - PpLuc(GC) - albumin7 - A64 - C30 - histoneSL (Fig. 16) |
| SEQ ID NO. 1439 | HSD17B4 - PpLuc(GC) - albumin7 - A64 - C30 - histoneSL (Fig. 17) |
| SEQ ID NO. 1440 | AlG1 - PpLuc(GC) - albumin7 - A64 - C30 - histoneSL (Fig. 18) |
| SEQ ID NO. 1441 | COX6C - PpLuc(GC) - albumin7 - A64 - C30 - histoneSL (Fig. 19) |
| SEQ ID NO. 1442 | ASAH1 - PpLuc(GC) - albumin7 - A64 - C30 - histoneSL (Fig. 20) |
| SEQ ID NO. 1443 | mRPL21 - PpLuc(GC) - albumin7 - A64 - C30 - histoneSL (Fig. 37) |
| SEQ ID NO. 1444 | mRPL35A - PpLuc(GC) - albumin7 - A64 - C30 - histoneSL (Fig. 38) |
| SEQ ID NO. 1445 | RPL35 - PpLuc(GC) - A64 - C30 - histoneSL |
| SEQ ID NO. 1446 | RPL21 - PpLuc(GC) - A64 - C30 - histoneSL |
| SEQ ID NO. 1447 | ATP5A1 - PpLuc(GC) - A64 - C30 - histoneSL |
| SEQ ID NO. 1448 | HSD17B4 - PpLuc(GC) - A64 - C30 - histoneSL |
| SEQ ID NO. 1449 | AlG1 - PpLuc(GC) - A64 - C30 - histoneSL |
| SEQ ID NO. 1450 | COX6C - PpLuc(GC) - A64 - C30 - histoneSL |
| SEQ ID NO. 1451 | ASAH1 - PpLuc(GC) - A64 - C30 - histoneSL |
| SEQ ID NO. 1452 | 5'UTR of human ribosomal protein Large 35 (RPL35) lacking the 5' terminal oligopyrimidine tract |

(continued)

| | |
|---|---|
| SEQ ID NO. 1453 | 5'UTR of human ribosomal protein Large 21 (RPL21) lacking the 5' terminal oligopyrimidine tract |
| SEQ ID NO. 1454 | 5'UTR of human ATP synthase, H+ transporting, mitochondrial F1 complex, alpha subunit 1, cardiac muscle (ATP5A1) lacking the 5' terminal oligopyrimidine tract |
| SEQ ID NO. 1455 | 5'UTR of human hydroxysteroid (17-beta) dehydrogenase 4 (HSD17B4) lacking the 5' terminal oligopyrimidine tract |
| SEQ ID NO. 1456 | 5'UTR of human androgen-induced 1 (AIG1) lacking the 5' terminal oligopyrimidine tract |
| SEQ ID NO. 1457 | 5'UTR of human cytochrome c oxidase subunit VIc (COX6C) lacking the 5' terminal oligopyrimidine tract |
| SEQ ID NO. 1458 | 5'UTR of human N-acylsphingosine amidohydrolase (acid ceramidase) 1 (ASAH1) lacking the 5' terminal oligopyrimidine tract |
| SEQ ID NO. 1459 | 5'UTR of mouse ribosomal protein Large 21 (mRPL21) lacking the 5' terminal oligopyrimidine tract |
| SEQ ID NO. 1460 | 5'UTR of mouse ribosomal protein large 35A (mRPL35A) lacking the 5' terminal oligopyrimidine tract |
| SEQ ID NO. 1461 | Mouse ribosomal protein Large 21 (mRPL21) |
| SEQ ID NO. 1462 | Mouse ribosomal protein large 35A (mRPL35A) |
| SEQ ID NO. 1463 | RPL32 - PpLuc(GC) - ag - A64 (Fig. 10) |
| SEQ ID NO. 1464 | PpLuc(GC) - ag - A64 - histoneSL (Fig. 9) |
| SEQ ID NO. 1465 | PpLuc(GC) - albumin7 - A64 - C30 - histoneSL |
| SEQ ID NO. 1466 | RPL35 - PpLuc(GC) - ag - A64 (Fig. 22) |
| SEQ ID NO. 1467 | RPL21 - PpLuc(GC) - ag - A64 (Fig. 23) |
| SEQ ID NO. 1468 | atp5a1 - PpLuc(GC) - ag - A64 (Fig. 24) |
| SEQ ID NO. 1469 | HSD17B4 - PpLuc(GC) - ag - A64 (Fig. 25) |
| SEQ ID NO. 1470 | AIG1 - PpLuc(GC) - ag - A64 (Fig. 26) |
| SEQ ID NO. 1471 | COX6C - PpLuc(GC) - ag - A64 (Fig. 27) |
| SEQ ID NO. 1472 | ASAH1 - PpLuc(GC) - ag - A64 (Fig. 28) |
| SEQ ID NO. 1473 | RPL35 - PpLuc(GC) - ag - A64 - histoneSL (Fig. 29) |
| SEQ ID NO. 1474 | RPL21 - PpLuc(GC) - ag - A64 - histoneSL (Fig. 30) |
| SEQ ID NO. 1475 | atp5a1 - PpLuc(GC) - ag-A64 - histoneSL (Fig. 31) |
| SEQ ID NO. 1476 | HSD17B4 - PpLuc(GC) - ag - A64 - histoneSL (Fig. 32) |
| SEQ ID NO. 1477 | AIG1 - PpLuc(GC) - ag - A64 - histoneSL (Fig. 33) |
| SEQ ID NO. 1478 | COX6C - PpLuc(GC) - ag - A64 - histoneSL (Fig. 34) |

(continued)

| | |
|---|---|
| SEQ ID NO. 1479 | ASAH1 - PpLuc(GC) - ag - A64 - histoneSL (Fig. 35) |
| SEQ ID NO. 1480 | RPL32 - PpLuc(GC) - ag - A64 - histoneSL (Fig. 11) |
| SEQ ID NO. 1481 | RPL32 - PpLuc(GC) - albumin7 - A64 - C30 - histoneSL (Fig. 13) |

## Examples

### 1. Preparation of DNA-templates

A vector for *in vitro* transcription was constructed containing a T7 promoter followed by a GC-enriched sequence coding for *Photinus pyralis* luciferase (PpLuc(GC)) and an A64 poly(A) sequence. The poly(A) sequence was followed by a restriction site used for linearization of the vector before *in vitro* transcription. mRNA obtained from this vector accordingly by *in vitro* transcription is designated as "PpLuc(GC) - A64".

This vector was modified to include untranslated sequences 5' or 3' of the open reading frame. In summary, vectors comprising the following mRNA encoding sequences have been generated:

| | |
|---|---|
| SEQ ID No. 1364 | PpLuc(GC) - ag - A64 (Fig. 6) |
| SEQ ID No. 1365 | RPL32 - PpLuc(GC) - ag - A64 - C30 - histoneSL (Fig. 7): |
| SEQ ID NO. 1436 | RPL35 - PpLuc(GC) - albumin7 - A64 - C30 - histoneSL (Fig. 14) |
| SEQ ID NO. 1437 | RPL21 - PpLuc(GC) - albumin7 - A64 - C30 - histoneSL (Fig. 15) |
| SEQ ID NO. 1438 | ATP5A1 - PpLuc(GC) - albumin7 - A64 - C30 - histoneSL (Fig. 16) |
| SEQ ID NO. 1439 | HSD17B4 - PpLuc(GC) - albumin7 - A64 - C30 - histoneSL (Fig. 17) |
| SEQ ID NO. 1440 | AIG1 - PpLuc(GC) - albumin7 - A64 - C30 - histoneSL (Fig. 18) |
| SEQ ID NO. 1441 | COX6C - PpLuc(GC) - albumin7 - A64 - C30 - histoneSL (Fig. 19) |
| SEQ ID NO. 1442 | ASAH1 - PpLuc(GC) - albumin7 - A64 - C30 - histoneSL (Fig. 20) |
| SEQ ID NO. 1443 | mRPL21 - PpLuc(GC) - albumin7 - A64 - C30 - histoneSL (Fig. 37) |
| SEQ ID NO. 1444 | mRPL35A - PpLuc(GC) - albumin7 - A64 - C30 - histoneSL (Fig. 38) |
| SEQ ID NO. 1445 | RPL35 - PpLuc(GC) - A64 - C30 - histoneSL |
| SEQ ID NO. 1446 | RPL21 - PpLuc(GC) - A64 - C30 - histoneSL |
| SEQ ID NO. 1447 | ATP5A1 - PpLuc(GC) - A64 - C30 - histoneSL |
| SEQ ID NO. 1448 | HSD17B4 - PpLuc(GC) - A64 - C30 - histoneSL |
| SEQ ID NO. 1449 | AIG1 - PpLuc(GC) - A64 - C30 - histoneSL |
| SEQ ID NO. 1450 | COX6C - PpLuc(GC) - A64 - C30 - histoneSL |
| SEQ ID NO. 1451 | ASAH1 - PpLuc(GC) - A64 - C30 - histoneSL |
| SEQ ID NO. 1463 | RPL32 - PpLuc(GC) - ag - A64 (Fig. 10) |
| SEQ ID NO. 1464 | PpLuc(GC) - ag - A64 - histoneSL (Fig. 9) |
| SEQ ID NO. 1465 | PpLuc(GC) - albumin7 - A64 - C30 - histoneSL |
| SEQ ID NO. 1466 | RPL35 - PpLuc(GC) - ag - A64 (Fig. 22) |
| SEQ ID NO. 1467 | RPL21 - PpLuc(GC) - ag - A64 (Fig. 23) |
| SEQ ID NO. 1468 | atp5a1 - PpLuc(GC) - ag - A64 (Fig. 24) |
| SEQ ID NO. 1469 | HSD17B4 - PpLuc(GC) - ag - A64 (Fig. 25) |
| SEQ ID NO. 1470 | AIG1 - PpLuc(GC) - ag - A64 (Fig. 26) |
| SEQ ID NO. 1471 | COX6C - PpLuc(GC) - ag - A64 (Fig. 27) |
| SEQ ID NO. 1472 | ASAH1 - PpLuc(GC) - ag - A64 (Fig. 28) |
| SEQ ID NO. 1473 | RPL35 - PpLuc(GC) - ag - A64 - histoneSL (Fig. 29) |
| SEQ ID NO. 1474 | RPL21 - PpLuc(GC) - ag - A64 - histoneSL (Fig. 30) |
| SEQ ID NO. 1475 | atp5a1 - PpLuc(GC) - ag - A64 - histoneSL (Fig. 31) |
| SEQ ID NO. 1476 | HSD17B4 - PpLuc(GC) - ag - A64 - histoneSL (Fig. 32) |
| SEQ ID NO. 1477 | AIG1 - PpLuc(GC) - ag - A64 - histoneSL (Fig. 33) |
| SEQ ID NO. 1478 | COX6C - PpLuc(GC) - ag - A64 - histoneSL (Fig. 34) |

(continued)

| | |
|---|---|
| SEQ ID NO. 1479 | ASAH1 - PpLuc(GC) - ag - A64 - histoneSL (Fig. 35) |
| SEQ ID NO. 1480 | RPL32 - PpLuc(GC) - ag - A64 - histoneSL (Fig. 11) |
| SEQ ID NO. 1481 | RPL32 - PpLuc(GC) - albumin7 - A64 - C30 - histoneSL (Fig. 13) |

**2. *In vitro* transcription**

The DNA-template according to Example 1 was linearized and transcribed *in vitro* using T7-Polymerase. The DNA-template was then digested by DNase-treatment. mRNA transcripts contained a 5'-CAP structure obtained by adding an excess of N7-Methyl-Guanosine-5'-Triphosphate-5'-Guanosine to the transcription reaction. mRNA thus obtained was purified and resuspended in water.

**3. Luciferase expression by mRNA lipofection**

Human dermal fibroblasts (HDF) were seeded in 24 well plates at a density of $5x10^4$ cells per well. The following day, cells were washed in opti-MEM and then transfected with 50 ng per well of Lipofectamine2000-complexed PpLuc-encoding mRNA in opti-MEM. As a control, mRNA not coding for PpLuc was lipofected separately. mRNA coding for *Renilla reniformis* luciferase (RrLuc) was transfected together with PpLuc mRNA to control for transfection efficiency (20 ng of RrLuc mRNA per well). 90 minutes after start of transfection, opti-MEM was exchanged for medium. 24, 48, 72 hours after transfection, medium was aspirated and cells were lysed in 200 μl of lysis buffer (25 mM Tris, pH 7.5 (HCl), 2 mM EDTA, 10% glycerol, 1% Triton X-100, 2 mM DTT, 1 mM PMSF). Lysates were stored at -20°C until luciferase activity was measured.

Alternatively, HDF were seeded in 96 well plates one to three days before transfection at a density of $10^4$ cells per well. Immediately before lipofection, cells were washed in opti-MEM. Cells were lipofected with 25 ng of PpLuc-encoding mRNA per well complexed with Lipofectamine2000. In some experiments, mRNA coding for *Renilla reniformis* luciferase (RrLuc) was transfected together with PpLuc mRNA to control for transfection efficiency (2.5 ng of RrLuc mRNA per well). 90 minutes after start of transfection, opti-MEM was exchanged for medium. At various time points post transfection, medium was aspirated and cells were lysed in 100 μl of lysis buffer (Passive Lysis Buffer, Promega). Lysates were stored at -80°C until luciferase activity was measured.

**4. Luciferase measurement**

Luciferase activity was measured as relative light units (RLU) in a BioTek SynergyHT plate reader. PpLuc activity was measured at 15 seconds measuring time using 50 μl of lysate and 200 μl of luciferin buffer (75 μM luciferin, 25 mM Glycylglycin, pH 7.8 (NaOH), 15 mM MgSO4, 2 mM ATP). RrLuc activity was measured at 15 seconds measuring time using 50 μl of lysate and 200 μl of coelenterazin buffer (40 μM coelenterazin in phosphate buffered saline adjusted to 500 mM NaCl).

Alternatively, luciferase activity was measured as relative light units (RLU) in a Hidex Chameleon plate reader. PpLuc activity was measured at 2 seconds measuring time using 20 μl of lysate and 50 μl of luciferin buffer (Beetle-Juice, PJK GmbH). RrLuc activity was measured at 2 seconds measuring time using 20 μl of lysate and 50 μl of coelenterazin buffer (Renilla-Juice, PJK GmbH).

**Results**

**5.1 The combination of 5'UTR elements derived from 5'UTRs of TOP genes and histone stem-loop increases protein expression strongly**.

To investigate the effect of the combination of a 5'UTR element derived from a 5'UTR of a TOP gene and a histone stem-loop (histoneSL) on protein expression from mRNA, mRNAs with different UTRs were synthesized: mRNAs either lacked both 5'UTR element and histoneSL, or contained both 5'UTR element and histoneSL. Luciferase-encoding mRNAs or control mRNA were transfected into human dermal fibroblasts (HDF). Luciferase levels were measured at 24 hours after transfection (see following Table 1 and Figure 8).

Table 1:

| mRNA | RLU at 24 hours |
|---|---|
| control RNA | 588 |
| PpLuc(GC) - ag - A64 | 12246 |
| RPL32 - PpLuc(GC) - ag - A64 - C30 - histoneSL | 319840 |

Luciferase was clearly expressed from mRNA having neither 5'UTR element nor histoneSL. Strikingly however, the combination of 5'UTR element and histoneSL strongly increased the luciferase level. The magnitude of the rise in luciferase level due to combining 5'UTR element and histoneSL in the same mRNA indicates that they are acting synergistically.

**5.2 The combination of 5'UTR elements derived from 5'UTRs of TOP genes and histone stem-loop increases protein expression from mRNA in a synergistic manner.**

To investigate the effect of the combination of a 5'UTR element derived from a 5'UTR of a TOP gene and histone stem-loop on protein expression from mRNA, mRNAs with different UTRs were synthesized: mRNAs either lacked both 5'UTR element and histone stem-loop, or contained either a 5'UTR element or a histone stem-loop, or both 5'UTR element and histone stem-loop. Luciferase-encoding mRNAs were transfected into human dermal fibroblasts (HDF). Luciferase levels were measured at 8, 24, and 48 hours after transfection (see following Table 2 and Figure 12).

Table 2:

| mRNA | RLU at 8 hours | RLU at 24 hours | RLU at 48 hours |
|---|---|---|---|
| PpLuc(GC)-ag-A64 | 13110 | 25861 | 14362 |
| PpLuc(GC)-ag-A64-histoneSL | 88640 | 97013 | 57026 |
| rpl32-PpLuc(GC)-ag-A64 | 155654 | 212245 | 102528 |
| rpl32-PpLuc(GC)-ag-A64-histoneSL | 301384 | 425825 | 161974 |

Luciferase was clearly expressed from mRNA having neither 5'UTR element nor histone stem-loop. Both, either the histone stem-loop or the 5'UTR element increased luciferase levels compared to mRNA lacking both these elements. Strikingly however, the combination of 5'UTR element and histone stem-loop further strongly increased the luciferase level, much above the level observed with either of the individual elements. The magnitude of the rise in luciferase level due to combining 5'UTR element and histone stem-loop in the same mRNA demonstrates that they are acting synergistically.

The synergy between 5'UTR element and histone stem-loop was quantified by dividing the signal from mRNA combining both elements by the sum of the signal from mRNA lacking both elements plus the rise in signal effected by the 5'UTR element plus the rise in signal effected by the histone stem-loop. This calculation was performed for the three time points individually and for total protein expressed from 0 to 48 hours calculated from the area under the curve (AUC) (see following Table 3).

Table 3:

| 8 h | | | | | |
|---|---|---|---|---|---|
| **rpl32** | **histoneSL** | **RLU** | **∆ RLU** | **RLU predicted (additive)** | **synergy** |
| - | - | 13110 | | | |
| - | + | 88640 | 75530 | | |
| + | - | 155654 | 142544 | | |
| + | + | 301384 | | 231184 | 1,30 |
| 24 h | | | | | |
| **rpl32** | **histoneSL** | **RLU** | **∆ RLU** | **RLU predicted (additive)** | **synergy** |
| - | - | 25861 | | | |

(continued)

| | | | | | |
|---|---|---|---|---|---|
| 24 h | | | | | |
| - | + | 97013 | 71152 | | |
| + | - | 212245 | 186384 | | |
| + | + | 425825 | | 283397 | 1,50 |
| 48 h | | | | | |
| **rpl32** | **histoneSL** | **RLU** | **Δ RLU** | **RLU predicted (additive)** | **synergy** |
| - | - | 14362 | | | |
| - | + | 57026 | 42664 | | |
| + | - | 102528 | 88166 | | |
| + | + | 161974 | | 145192 | 1,12 |
| AUC 0 - 48 hours | | | | | |
| **rpl32** | **histoneSL** | **RLU** | **Δ RLU** | **RLU predicted (additive)** | **synergy** |
| - | - | 846881 | | | |
| - | + | 3688000 | 2841119 | | |
| + | - | 7343000 | 6496119 | | |
| + | + | 14080000 | | 10184119 | 1,38 |

The synergy thus calculated specifies how much higher the luciferase level from mRNA combining 5'UTR element and histone stem-loop is than would be expected if the effects of 5'UTR element and histone stem-loop were purely additive. This result confirms that the combination of 5'UTR element and histone stem-loop effects a markedly synergistic increase in protein expression.

**5.3 5'UTR elements derived from 5'UTRs of TOP genes increase protein expression from mRNA.**

To investigate the effect of 5'UTR elements derived from 5'UTRs of TOP genes on protein expression from mRNA, mRNAs with one of different 5'UTR elements were synthesized. In addition, mRNAs contained the albumin7 3'UTR element. Luciferase-encoding mRNAs were transfected into human dermal fibroblasts (HDF). Luciferase levels were measured at 24, 48, and 72 hours after transfection (see following Table 4 and Figure 21).

Table 4:

| 5'UTR | RLU at 24 hours | RLU at 48 hours | RLU at 72 hours |
|---|---|---|---|
| none | 114277 | 121852 | 68235 |
| rpl32 | 332236 | 286792 | 114148 |
| rpl35 | 495917 | 234070 | 96993 |
| rpl21 | 563314 | 352241 | 156605 |
| atp5a1 | 1000253 | 538287 | 187159 |
| HSD17B4 | 1179847 | 636877 | 299337 |
| AIG1 | 620315 | 446621 | 167846 |
| COX6C | 592190 | 806065 | 173743 |
| ASAH1 | 820413 | 529901 | 198429 |

Luciferase was clearly expressed from mRNA lacking a 5'UTR element. Strikingly however, all 5'UTR elements strongly increased the luciferase level.

**5.4 The combination of 5'UTR elements derived from 5'UTRs of TOP genes and histone stem-loop increases protein expression from mRNA in a synergistic manner.**

To investigate the effect of the combination of 5'UTR elements derived from the 5'UTRs of TOP genes and histone stem-loop on protein expression from mRNA, mRNAs with different UTRs were synthesized: mRNAs either lacked both 5'UTR element and histone stem-loop, or contained a histone stem-loop, or contained one of different 5'UTR elements derived from 5'UTRs of TOP genes, or contained both one of different 5'UTR elements and a histone stem-loop. In addition, mRNAs contained the alpha-globin 3'UTR element. Luciferase-encoding mRNAs were transfected into human dermal fibroblasts (HDF). Luciferase levels were measured at 8, 24, and 48 hours after transfection (see Figure 36). Luciferase was clearly expressed from mRNA having neither 5'UTR element nor histone stem-loop. The histone stem-loop increased the luciferase level. All 5'UTR elements also increased the luciferase level. Strikingly however, the combinations of 5'UTR element and histone stem-loop further strongly increased the luciferase level, much above the level observed with either of the individual elements. The magnitude of the rise in luciferase level due to combining 5'UTR element and histone stem-loop in the same mRNA demonstrates that they are acting synergistically.

The synergy between 5'UTR element and histone stem-loop was quantified by dividing the signal from mRNA combining both elements by the sum of the signal from mRNA lacking both elements plus the rise in signal effected by the 5'UTR element plus the rise in signal effected by the histone stem-loop. This calculation was performed for total protein expressed from 0 to 48 hours calculated from the area under the curve (AUC) (see following Table 5).

Table 5:

| TOP 5'UTR | Synergy with histone stem-loop |
|---|---|
| 35L | 2,50 |
| 21L | 3,25 |
| atp5a1 | 3,00 |
| HSD17B4 | 3,55 |
| AIG1 | 1,52 |
| COX6C | 3,19 |

The synergy thus calculated specifies how much higher the luciferase level from mRNA combining 5'UTR element and histone stem-loop is than would be expected if the effects of 5'UTR element and histone stem-loop were purely additive. The luciferase level from mRNA combining 5'UTR element and histone stem-loop was up to more than three times higher than if their effects were purely additive. This result confirms that the combination of 5'UTR element and histone stem-loop effects a markedly synergistic increase in protein expression.

**5.5 5'UTR elements derived from 5'UTRs of mouse TOP genes increase protein expression from mRNA.**

To investigate the effect of TOP 5'UTR elements derived from 5'UTRs of mouse TOP genes on protein expression from mRNA, mRNAs with two different mouse 5'UTR elements were synthesized. In addition, mRNAs contained the albumin7 3'UTR element. Luciferase-encoding mRNAs were transfected into human dermal fibroblasts (HDF). For comparison, mRNA containing the human rpl32 5'UTR element was transfected. Luciferase levels were measured at 24, 48, and 72 hours after transfection (see following Table 6 and Figure 39).

Table 6:

| 5'UTR | RLU at 24 hours | RLU at 48 hours | RLU at 72 hours |
|---|---|---|---|
| none | 114277 | 121852 | 68235 |
| rpl32 | 332236 | 286792 | 114148 |
| mrpl21 | 798233 | 351894 | 139249 |
| mrpl35A | 838609 | 466236 | 174949 |

Luciferase was clearly expressed from mRNA lacking a 5'UTR element. Both mouse 5'UTR elements strongly increased the luciferase level, similarly as the human 5'UTR element.

**Sequences:**

Homo sapiens alpha-2-macroglobulin (A2M): gctccttctttctgcaacatg (Seq ID No: 1)

Homo sapiens acyl-CoA dehydrogenase, C-4 to C-12 straight ch ain (ACADM):

ggctctctttccgcgctgcggtcagcctcggcgtcccacagagagggccagaggtggaaa
cgcagaaaaccaaaccaggactatcagagattgcccggagaggggatg
(Seq ID No: 2)

Homo sapiens arylsulfatase E (chondrodysplasia punctata 1) (ARSE):

cttcctcttcttgatcggggattcaggaaggagcccaggagcagaggaagtagagagaga
gacaacatg (Seq ID No: 3)

Homo sapiens Bruton agammaglobulinemia tyrosine kinase (BTK) :

tgtccttcctctctggactgtaagaatatgtctccagggccagtgtctgctgcgatcgag
tcccaccttccaagtcctggcatctcaatgcatctgggaagctacctgcattaagtcagg
actgagcacacaggtgaactccagaaagaagaagctatg (Seq ID No: 4)

Homo sapiens complement component 2 (C2): tgaccttttccctcccgcggctctctacctctcgccgcccctagggaggacaccatg (Seq ID No: 5)

Homo sapiens cyclin-dependent kinase 4 (CDK4):

gggcctctctagcttgcggcctgtgtctatggtcgggccctctgcgtccagctgctccgg
accgagctcgggtgtatggggccgtaggaaccggctccggggccccgataacgggccgcc
cccacagcaccccgggctggcgtgagggtctcccttgatctgagaatg
(Seq ID No: 6)

Homo sapiens cytochrome P450, family 17, subfamily A, polype ptide 1 (CYP17A1): agctcttctactccactgctgtctatcttgcctgccggcacccagccaccatg (Seq ID No: 7)

Homo sapiens endoglin (ENG):

cttcctctacccggttggcaggcggcctggcccagccccttctctaaggaagcgcatttc
ctgcctccctgggccggccgggctggatg (Seq ID No: 8)

Homo sapiens excision repair cross-complementing rodent repa ir deficiency, complementation group 3 (ERCC3): tcttctctctgctgctgtagctgccatg (Seq ID No: 9)

Homo sapiens excision repair cross-complementing rodent repa ir deficiency, complementation group 5 (ERCC5):

ctgtctttcttccgggaggcggtgacagctgctgagacgtgttgcagccagagtctctcc
gctttaatgcgctcccattagtgccgtcccccactggaaaaccgtggcttctgtattatt
tgccatctttgttgtgtaggagcagggagggcttcctcccggggtcctaggcggcggtgc
agtccgtcgtagaagaattagagtagaagttgtcggggtccgctcttaggacgcagccgc
ctcatg (Seq ID No: 10)

Homo sapiens ferritin, light polypeptide (FTL):

cgtcccctcgcagttcggcggtcccgcgggtctgtctcttgcttcaacagtgtttggacg gaacagatccggggactctcttccagcctccgaccgccctccgatttcctctccgcttgc aacctccgggaccatcttctcggccatctcctgcttctgggacctgccagcaccgttttt gtggttagctccttcttgccaaccaaccatg (Seq ID No: 11)

Homo sapiens galactosylceramidase (GALC): ccgcctccctgggcgccggagtcatgtgacccacacaatg (Seq ID No: 12)

Homo sapiens gap junction protein, alpha 1, 43kDa (GJA1):

ttttctttcattaggggaaggcgtgaggaaagtaccaaacagcagcggagttttaaact ttaaatagacaggtctgagtgcctgaacttgccttttcattttacttcatcctccaagga gttcaatcacttggcgtgacttcactacttttaagcaaaagagtggtgcccaggcaacat g (Seq ID No: 13)

Homo sapiens gap junction protein, beta 1, 32kDa (GJBl):

cattctctgggaaagggcagcagcagccaggtgtggcagtgacagggaggtgtgaatgag gcaggatg (Seq ID No: 14)

Homo sapiens glucose-6-phosphate isomerase (GPI): cgctccttcctcctcggctcgcgtctcactcagtgtaccttctagtcccgccatg (Seq ID No: 15)

Homo sapiens hydroxyacyl-CoA dehydrogenase/3-ketoacyl-CoA th iolase/enoyl-CoA hydratase (trifunctional protein), alpha subunit (HADHA): ctgtcctcttcagctcaagatg (Seq ID No: 16)

Homo sapiens hydroxyacyl-CoA dehydrogenase/3-ketoacyl-CoA th iolase/enoyl-CoA hydratase (trifunctional protein), beta subunit (HADHB):

gggccctttctgggcaggacccgccccttggtcccgcagagccttggtacttggacctga accttgctccgagagggagtcctcgcggacgtcagccaagattccagaatg (Seq ID No: 17)

Homo sapiens complement factor H (CFH):

cttccttttgcagcaagttctttcctgcactaatcacaattcttggaagaggagaactgg acgttgtgaacagagttagctggtaaatgtcctcttaaaagatccaaaaaatg (Seq ID No: 18)

Homo sapiens sarcoglycan, gamma (35kDa dystrophin-associated glycoprotein) (SGCG):

agccctttctccagggacagttgctgaagcttcatcctttgctctcattctgtaagtcat agaaaagtttgaaacattctgtctgtggtagagctcgggccagctgtagttcattcgcca gtgtgcttttcttaatatctaagatg (Seq ID No: 19)

Homo sapiens lipase A, lysosomal acid, cholesterol esterase (LIPA) :

ggtcccctatccgcaccccggcccctgagagctggcactgcgactcgagacagcggcccg gcaggacagctccagaatg (Seq ID No: 20)

Homo sapiens lipoprotein lipase (LPL):

ccccctcttcctcctcctcaagggaaagctgcccacttctagctgccctgccatcccctt taaagggcgacttgctcagcgccaaaccgcggctccagccctctccagcctccggctcag ccggctcatcagtcggtccgcgccttgcagctcctccagagggacgcgccccgagatg (Seq ID No: 21)

Homo sapiens mutL homolog 1, colon cancer, nonpolyposis type 2 (E. coli) (MLH1): ggctcttctggcgccaaaatg (Seq ID No: 22)

Homo sapiens Niemann-Pick disease, type C1 (NPC1):

cttccttcctgaccggcgcgcgcagcctgctgccgcggtcagcgcctgctcctgctcctc cgctcctcctgcgcggggtgctgaaacagcccggggaagtagagccgcctccggggagcc caaccagccgaacgccgccggcgtcagcagccttgcgcggccacagcatg (Seq ID No: 23)

Homo sapiens peroxisomal biogenesis factor 12 (PEX12):

gcgcctctcttccgccaggcatcccagaggtcctggtggtttcatttccgggtgcggctt ctgtcataaagcggagacctcccttcaaacgtggcgtcgtgggttgtttgcgcctcgcct

ggggtcagcgagcaaggacgggcgcgggcggggatactcaaagccaacagctggagtcag cccttgtgtcccgggctcacagtggcacgactgaatcctcagagtcggctggcttttgag ctctcacgattggggaggagggggcgtttctggttcgcagctccagaggattgcgttcct tcccccatacctgtcccccacagtcacgctctgccctgacgtgcagcatttgacaagtta ccccctcgccacatactacttccacccacgtccgagttaactttgttcttaaccttcttg agactaccctcggcctccaggtctttttttcccagttcatttttgcccataagattgagt ttcgagtttcagatatcatgcagaaagtttacctttaagactgagcacccatctgatact cttcctcccgaaaaagttcatgctcacgagagagtttgtgggaaaagtgaaagccagtac acgcaggaaactatg (Seq ID No: 24)

Homo sapiens peroxisomal biogenesis factor 6 (PEX6): cgctccttcaccctcctcgttggtgtcctgtcaccatg (Seq ID No: 25)

Homo sapiens phosphofructokinase, muscle (PFKM):

gagccttcttgtcagcatctgttagtggaggttgggaagcctctcctccttcccccctccc tctttgcctccacctggctcctccccatgttcgtccatcacccctccccccctttcccaag gacaatctgcaagaaagcagcggcggaggagagctaagactaaaagagtggatcatg (Seq ID No: 26)

Homo sapiens serpin peptidase inhibitor, clade A (alpha-1 antiproteinase, antitrypsin), member 1 (SERPINA1) : ctgtctcctcagcttcaggcaccaccactgacctgggacagtgaatcgacaatg (Seq ID No: 27)

Homo sapiens phosphatase and tensin homolog (PTEN):

agttctctcctctcggaagctgcagccatgatggaagtttgagagttgagccgctgtgag
gcgaggccgggctcaggcgagggagatgagagacggcggcggccgcggcccggagcccct
ctcagcgcctgtgagcagccgcgggggcagcgccctcggggagccggccggcctgcggcg
gcggcagcggcggcgtttctcgcctcctcttcgtcttttctaaccgtgcagcctcttcct
cggcttctcctgaaagggaaggtggaagccgtgggctcgggcgggagccggctgaggcgc
ggcggcggcggcggcacctcccgctcctggagcgggggggagaagcggcggcggcggcgg
ccgcggcggctgcagctccagggagggggtctgagtcgcctgtcaccatttccagggctg
ggaacgccggagagttggtctctccccttctactgcctccaacacggcggcggcggcggc
ggcacatccagggacccgggccggttttaaacctcccgtccgccgccgccgcaccccccg
tggcccgggctccggaggccgccggcggaggcagccgttcggaggattattcgtcttctc
cccattccgctgccgccgctgccaggcctctggctgctgaggagaagcaggcccagtcgc
tgcaaccatccagcagccgccgcagcagccattacccggctgcggtccagagccaagcgg
cggcagagcgaggggcatcagctaccgccaagtccagagccatttccatcctgcagaaga
agccccgccaccagcagcttctgccatctctctcctcctttttcttcagccacaggctcc
cagacatg (Seq ID No: 28)

Homo sapiens solute carrier family 3
(cystine, dibasic and neutral amino acid transporters, activ ator of cystine, dibasic and neutral amino acid transport), member 1 (SLC3A1): cctcccttactgcaggaaggcactccgaagacataagtcggtgagacatg (Seq ID No: 29)

Homo sapiens aldehyde dehydrogenase 3 family, member A2 (ALDH3A2) : ccgcctcccactccccagcgcccccggaccgtgcagttctctgcaggaccaggccatg (Seq ID No: 30)

Homo sapiens bleomycin hydrolase (BLMH):

gtttctcccagcctcagcctccccgccgccgccgccgccgccgccgagccggtttcc
tttttccggcgctccgggtgcgagagacaggtcgggccccctaggcagcgagccgcagcg
caatcccggcgctcgcccaaggaccctggaagctaccgttaccccgccgggcagcgtggg
cgccatg (Seq ID No: 31)

Homo sapiens cathepsin K CTSK):

cctcctcctcttacccaaattttccagccgatcactggagctgacttccgcaatcccgat
ggaataaatctagcacccctgatggtgtgcccacactttgctgccgaaacgaagccagac
aacagatttccatcagcaggatg (Seq ID No: 32)

Homo sapiens GM2 ganglioside activator (GM2A):

gcttctttgcgtaaccaatactggaaggcatttaaaggcacctctgccgccacagacctt
gcagttaactccgccctgacccacccttcccgatg (Seq ID No: 33)

Homo sapiens hydroxysteroid (17-beta) dehydrogenase 4 (HSD17B4) :

ccgcctcctcctgtcccgcagtcggcgtccagcggctctgcttgttcgtgtgtgtgtcgt
tgcaggccttattcatg (Seq ID No: 34)

Homo sapiens neutrophil cytosolic factor 2 (NCF2):

ctctctctgcttctttccttttctctctcatggtagggttatgagtcagttgccaaaagg tggggacatttcctgatgcatttgcaacactgagaagttatcttaagggaggctgggccc cattctactcatctggcccagaaagtgaacaccttgggggccactaaggcagccctgcta ggggagacgctccaacctgtcttctctctgtctcctggcagctctcttggcctcctagtt tctacctaatcatg (Seq ID No: 35)

Homo sapiens 3-oxoacid CoA transferase 1 (OXCT1) : cagcctcctcctgcctcaccgcccgaagatg (Seq ID No: 36)

Homo sapiens sulfite oxidase (SUOX):

ccgccccttctcgagaactcgcagagctgggctggtaaaattgcagtgctgaagacactg gacccgcaaaaggctgtccctcccaaacctgggattctgggctcactgagttcacctgcg agtcagccctacctgcactgctctggtctagtacaaacaggctgctggcattgagggacg gagtctccaactcctggcctctagcagtcctcctgtgtaggtctcccaaagtgctagtgt gtccggaattggtgggttcttggtctcactgacttcaagaatgaagccgcggaccctcgc agtctgctacaatg (Seq ID No: 37)

Homo sapiens albumin (ALB): ttttctcttctgtcaaccccacacgcctttggcacaatg (Seq ID No: 38)

Homo sapiens arylsulfatase A (ARSA):

ctccctctagcgccttccccccggcccgactccgctggtcagcgccaagtgacttacgcc cccgaccctgagcccggaccgctaggcgaggaggatcagatctccgctcgagaatctgaa ggtgccctggtcctggaggagttccgtcccagcccgcggtctcccggtactgtcgggccc

cggccctctggagcttcaggaggcggccgtcagggtcggggagtatttgggtccggggtc tcagggaagggcggcgcctgggtctgcggtatcggaaagagcctgctggagccaagtagc cctccctctcttgggacagacccctcggtcccatg (Seq ID No: 39)

Homo sapiens elastin (ELN):

ctccctccctctttccctcacagccgacgaggcaacaattaggctttggggataaaacga ggtgcggagagcgggctggggcatttctccccgagatg (Seq ID No: 40)

Homo sapiens hemoglobin, alpha 2 (HBA2): cactcttctggtccccacagactcagagagaacccaccatg (Seq ID No: 41)

Homo sapiens hexosaminidase B (beta polypeptide) (HEXB):

cttcctctgatccgggccgggcgggaagtcgggtcccgaggctccggctcggcagaccgg gcggaaagcagccgagcggccatg (Seq ID No: 42)

Homo sapiens mannosidase, alpha, class 2B, member 1 (MAN2B1): cggcctttccagggccggggaaccccaggaggaagctgctgagccatg (Seq ID No: 43)

Homo sapiens recombination activating gene 2 (RAG2):

cactctctttacagtcagccttctgcttgccacagtcatagtgggcagtcagtgaatctt ccccaagtgctgacaattaatacctggtttagcggcaaagattcagagaggcgtgagcag cccctctggccttcagacaaaaatctacgtaccatcagaaactatg (Seq ID No: 44)

Homo sapiens CD53 molecule (CD53):

tctcctttacacaaatagccccggatatctgtgttaccagccttgtctcggccacctca aggataatcactaaattctgccgaaaggactgaggaacggtgcctggaaaagggcaagaa tatcacggcatg (Seq ID No: 45)

Homo sapiens Fc fragment of IgG, low affinity IIIa, receptor (CD16a) (FCGR3A): tggtccctttagggctccggatatctttggtgacttgtccactccagtgtggcatcatg (Seq ID No: 46)

Homo sapiens interleukin 1, beta (IL1B) :

aaacctcttcgaggcacaaggcacaacaggctgctctgggattctcttcagccaatcttc attgctcaagtgtctgaagcagccatg (Seq ID No: 47)

Homo sapiens CD4 molecule (CD4):

ctgtctctcttcatttaagcacgactctgcagaaggaacaaagcaccctccccactgggc tcctggttgcagagctccaagtcctcacacagatacgcctgtttgagaagcagcgggcaa gaaagacgcaagcccagaggccctgccatttctgtgggctcaggtccctactggctcagg cccctgcctccctcggcaaggccacaatg (Seq ID No: 48)

Homo sapiens serpin peptidase inhibitor, clade A (alpha-1 antiproteinase, antitrypsin), member 5 (SERPINA5):

agccctctgccctttctgagcccgagggactgccacctccactgtgtgcacactcagcta cgggacacatttcaggtatccaaggcagcagaggtgagtgggtcccccgagctctgtgac

cttatgctccacactaactctggcagagcctccgtttcctcatagaacaaagaacagcca ccatg (Seq ID No: 49)

Homo sapiens vitronectin (VTN):

tgccctccttccctgtctctgcctctccctcccttcctcaggcatcagagcggagacttc agggagaccagagcccagcttgccaggcactgagctagaagccctgccatg (Seq ID No: 50)

Homo sapiens aldehyde dehydrogenase 9 family, member A1 (ALDH9A1) :

ccgcccctcccgcggccccgcccctcccgcggcccgtcagcctctgccgcggagctgcgt ccgccactcatg (Seq ID No: 51)

Homo sapiens annexin A1 (ANXA1) :

cttcctttaaaatcctataaaatcagaagcccaagtctccactgccagtgtgaaatcttc
agagaagaatttctctttagttctttgcaagaaggtagagataaagacacttttttcaaaa
atg (Seq ID No: 52)

Homo sapiens ATPase, Na+/K+ transporting, alpha 1 polypeptid e (ATP1A1) : ttttctctctgattctccagcgacaggacccggcgccgggcactgagcaccgccaccatg (Seq ID No: 53)

Homo sapiens ATPase, Na+/K+ transporting, alpha 2 polypeptid e (ATP1A2) :

ctttctctgtctgccagggtctccgactgtcccagacgggctggtgtgggcttgggatcc
tcctggtgacctctcccgctaaggtccctcagccactctgccccaagatg
(Seq ID No: 54)

Homo sapiens calcium channel, voltage-dependent, beta 3 subu nit (CACNB3):

ccctccttcgcgctctctcgctccctgccgccgcccgcagggctgcggggctcggtggca
tctcccgggcgcggcccgcagtccttgcccctgcctccgggccgctcccgcccccggcgc
cgctcgctcccccgacccggactcccccatg (Seq ID No: 55)

Homo sapiens cholinergic receptor, nicotinic, alpha 7 (neuronal) (CHRNA7):

gtgcctctgtggccgcaggcgcaggcccgggcgacagccgagacgtggagcgcgccggct
cgctgcagctccgggactcaacatg (Seq ID No: 56)

Homo sapiens cytochrome P450, family 51, subfamily A, polype ptide 1 (CYP51A1) :

gcttctctcgttccgtcgattgggaggagcggtggcgacctcggccttcagtgtttccga
cggagtgaatg (Seq ID No: 57)

Homo sapiens glutamate decarboxylase 1 (brain, 67kDa) (GAD1) :

atctctctcttctcctggcgctcgcgtgcgagagggaactagcgagaacgaggaagcagc
tggaggtgacgccgggcagattacgcctgtcagggccgagccgagcggatcgctgggcgc
tgtgcagaggaaaggcgggagtgcccggctcgctgtcgcagagccgagcctgtttctgcg

ccggaccagtcgaggactctggacagtagaggccccgggacgaccgagctgatg
(Seq ID No: 58)

Homo sapiens gamma-glutamyl carboxylase (GGCX):

aattctcctggcggcctccgttcagacgcggcagctgtgacccacctgcctcctccgcag
agcaatg (Seq ID No: 59)

Homo sapiens glutamate receptor, metabotropic 3 (GRM3):

tcccctctttccccaacctcctccctctcttctactccacccctccgttttcccactccc
cactgactcggatgcctggatgttctgccaccgggcagtggtccagcgtgcagccgggag
ggggcaggggcagggggcactgtgacaggaagctgcgcgcacaagttggccatttcgagg
gcaaaataagttctcccttggatttggaaaggacaaagccagtaagctacctcttttgtg
tcggatgaggaggaccaaccatgagccagagcccgggtgcaggctcaccgccgccgctgc
caccgcggtcagctccagttcctgccaggagttgtcggtgcgaggaattttgtgacaggc
tctgttagtctgttcctcccttatttgaaggacaggccaaagatccagtttggaaatgag
agaggactagcatgacacattggctccaccattgatatctcccagaggtacagaaacagg
attcatgaagatg (Seq ID No: 60)

Homo sapiens guanylate cyclase 1, soluble, alpha 3 (GUCY1A3) :

ggttcctttggggtgatcaaagagggagacacagacacagagagacaaaggcaaggagga
ctgtctgggagccacgcgggcgatacagtttccgaggcacgccgcgtcccgcctagcctg
ttgaacaggtagacatgagcgacccaagctgcggatttgcgaggcgcgccctggagctgc
tagagatccggaagcacagccccgaggtgtgcgaagccaccaagtcaagttcctaacgag
tcttcagaggaggcagcaggaagctcagagagctgcaaagcaaccgtgcccatctgtcaa
gacattcctgagaagaacatacaagaaagtcttcctcaaagaaaaaccagtcggagccga
gtctatcttcacactttggcagagagtatttgcaaactgatttcccagagtttgaacgg
ctgaatgttgcacttcagagaacattggcaaagcacaaaataaaagaaagcaggaaatct
ttggaaagagaagactttgaaaaaacaattgcagagcaagcagttgcagcaggagttcca
gtggaggttatcaaagaatctcttggtgaagaggtttttaaaatatgttacgaggaagat
gaaaacatccttggggtggttggaggcacccttaaagatttttaaacagcttcagtacc
cttctgaaacagagcagccattgccaagaagcaggaaaaaggggcaggcttgaggacgcc
tccattctatgcctggataaggaggatgattttctacatgtttactacttcttccctaag
agaaccacctccctgattcttcccggcatcataaaggcagctgctcacgtattatatgaa
acggaagtggaagtgtcgttaatg (Seq ID No: 61)

Homo sapiens 3-hydroxy-3-methylglutaryl-CoA reductase (HMGCR) :

ggctccttccgctccgcgactgcgttaactggagccaggctgagcgtcggcgccggggtt
cggtggcctctagtgagatctggaggatccaaggattctgtagctacaatg
(Seq ID No: 62)

Homo sapiens IMP (inosine 5'-monophosphate) dehydrogenase 2 (IMPDH2) : aggtctctgcggcgcggtcctcggagacacgcggcggtgtcctgtgttggccatg (Seq ID No: 63)

Homo sapiens leukotriene A4 hydrolase (LTA4H):

acttcctttcccggcgtgcaccgcgaatccctcctcctcttctttacctctctccctcct

cctcaggttctctatcgacgagtctggtagctgagcgttgggctgtaggtcgctgtgctg
tgtgatcccccagagccatg (Seq ID No: 64)

Homo sapiens neuropeptide Y receptor Y1 (NPY1R) :

ccttctttaataagcaggagcgaaaaagacaaattccaaagaggattgttcagttcaagg
gaatgaagaattcagaataatttggtaaatggattccaatatggggaataagaataagc
tgaacagttgacctgctttgaagaaacatactgtccatttgtctaaaataatctataaca
accaaaccaatcaaaatg (Seq ID No: 65)

Homo sapiens pyruvate dehydrogenase (lipoamide) beta (PDHB): cggcccctctgttgtcgtttggcagcggatagaggacacgaccaagatg (Seq ID No: 66)

Homo sapiens ribosomal protein L36a-like (RPL36AL) :

cttccctttcctgttaggcgagagctgcgaaaggcgagagctgcgaagggccaggtgtcg
ggcgctgtttctcgttttcatcatatagacaaaacagccctgctgcaaagatg
(Seq ID No: 67)

Homo sapiens ATPase, Ca++ transporting, type 2C, member 1 (ATP2C1) :

gcttcttctcacgccgggagcaggctcccgcctcgcaccgctgccccgcgagcagctcct
cttctcccgaggcgcgcggggcgcccccgcgagccccgcggctgagaccccgcagcctgg
aggagggctgtccggggctttggatgctgctgctaggggtggtgggagcagccgtgggac
gcgtggccgggagcggggtgacagcctgggattccgggggcttctcttccttgtcctcc
tcctctcctctctattcccagtgtggccgtggctgacactaaagactttgtagccatcaa
cccgagtgcagtttcgatggaaaatg (Seq ID No: 68)

Homo sapiens UDP-glucose pyrophosphorylase 2 (UGP2):

ccgcctctttcattgaagaaatttaagttcgtgtggttttaccttttccgggagtctcca
gctggccctcatttgtgtccggagctcaggagttcccaaaccgactcagtcgcaccaagt
ttccgtcttttggaattggggaaggagtttctttctttcttttctttttttcttgagccag
ttttaatcgctttgaataaatactcccttaagtagttaaatataggaggagaaagaatac
atcggttgttaaagcaggagaggaagagagacctgccctgtagcgtgactcctctagaaa
aaaaaaaaaaaagccggagtatttactaagcccctaaaatg (Seq ID No: 69)

Homo sapiens ATPase, Na+/K+ transporting, beta 1 polypeptide (ATP1B1) :

cctcctcctgctcctgccttggctcctccgccgcgcgtctcgcactccgagagccgcagc
ggcagcggcgcgtcctgcctgcagagagccaggccggagaagccgagcggcgcagaggac
gccagggcgcgcgccgcagccacccaccctccggaccgcggcagctgctgacccgccatc
gccatg (Seq ID No: 70)

Homo sapiens glycoprotein M6B (GPM6B):

ctgtctttatggaccagtaggcagagcgaaattgacgctgacaagactttgcatcttgg
aagggactgtaatctactgtagtgaagaacagagcctctcaatcagacgggtgtaaataa
gagacggaggggagtccaaaagaaaaggaagaggaggaaaaacaagtgtgtgttgggggg
aacaggggaaaagcattttggtggatggtatg (Seq ID No: 71)

Homo sapiens wntless homolog (Drosophila) (WLS):

gctcctttaagcgtccacaggcggcggagcggccacaatcacagctccgggcattggggg

aacccgagccggctgcgccgggggaatccgtgcgggcgccttccgtcccggtcccatcct
cgccgcgctccagcacctctgaagtttgcagcgcccagaaaggaggcgaggaaggaggg
agtgtgtgagaggagggagcaaaaagctcaccctaaaacatttatttcaaggagaaaaga
aaaaggggggcgcaaaaatg (Seq ID No: 72)

Homo sapiens flavin containing monooxygenase 3 (FMO3) :

ttttctctttcaaactgcccagacggttggacaggacgtagacacacagaagaaaagaag
acaaagaacgggtaggaaaattaaaaggttaccatg (Seq ID No: 73)

Homo sapiens multiple C2 domains, transmembrane 1 (MCTP1):

cagcctcttttgccggtattcagtgaagaaagcaagtctaaatatgcagttctctcactg
gagtgaaagatgttttgttcatttctaatcaactatg (Seq ID No: 74)

Homo sapiens structural maintenance of chromosomes 4 (SMC4):

ccgcctctcggcgagcccgccctcttctgaagaggcgtttctggaccactgagccccgcc
tcccactgtgagcggaaccctaccgttttaaaaaaatctttttcaaaacttgccaggtt
gtctttccaaatatttttaataatagtgctgctgctgtagaccacagagaaaagaatccc
tcgctcttccttttcacttagtagaaacttctaccgcgtaggtcccgccaggagttcgcg
catgcgcaggagcgacaataagatggcggtgataatcgccgcactttttttcaaattagt
ggatcccagaaatcattgcgcgcatttgtaacgaatttccgttcgagtttgtattttagg
cgccattttcgagtgaaggacccggagccgaaacaccggtaggagcggggaggtgggtac
tacacaaccgtctccagccttggtctgagtggactgtcctgcagcgaccatg
(Seq ID No: 75)

Homo sapiens GLE1 RNA export mediator homolog (yeast) (GLE1) :

tggccttcccggcggctgattcgagggcttgtttggtcagaagggggcgtcagagaagc
tgccccttagccaaccatg (Seq ID No: 76)

Homo sapiens tripartite motif containing 6 (TRIM6):

gagtctttcggcctgggtggaggacgcggctgcttcaagtccttggctctgatccaggcc
acagattccaggattctacaggcaggaaacatcttagaaatcagggttgggcaggcagga
gccaggagagtagctacaatg (Seq ID No: 77)

Homo sapiens ecotropic viral integration site 2A (EVI2A):

tatcctttttactgcagatttactttaaggctcatattctccaagtctattctgcttta
aaaagaagacaagaaaagaagtggtttatcaaaatcacgttataatcagattttgaccaa
gcatttgtaagtatacaaatgtcagccaatgacatataacaaccatttcttataaaacc
ttgatgttcaaaagcctgactagcagtggcatccatg (Seq ID No: 78)

Homo sapiens heterogeneous nuclear ribonucleoprotein L (HNRNPL) :

tgctcttttcgatccgggacggccggtcaggctcgccgccgagctggagaactacgatga
cccgcacaaaacccctgcctccccagttgtccacatcaggggcctgattgacggtgtggt
ggaagcagaccttgtggaggccttgcaggagtttggacccatcagctatgtggtggtaat
g (Seq ID No: 79)

Homo sapiens mitochondrial translational initiation factor 2 (MTIF2) :

cattcttccgggtccagaaggtgatctccgcccgtgctcagaatccaggggcccggggct

gtagattccttgacaaggatatcctagcggcgaaacaacaccgtactgggagtcagaacg
tctgggttctagtcttgactgccattaactagcggtatgacattggagaagctttttga
cccttctggatttccgtttccttttctgtaaaatgaggagcttggaagatccggaaaatg
aggcccataggaaacaagtgacttgctgagtccagataacactgactgtcagagagaaac
atg (Seq ID No: 80)

Homo sapiens nuclear factor of kappa light polypeptide gene enhancer in B-cells inhibitor, zeta (NFKBIZ):

tggcctcctcttgccacgaggtcagacggcgagttcttagagaaaaggctgcttagctg
ctgcttatcatgtaacctcaaaaggaaactgatcgtctttctcatgctgtcacgtacttg
ggttattatcgctgattacagctggaaacaattgatttgctcttacgtatttgtgtgact
tgactcttcaaacacaaaggttaacaggaagatctcgagggccctggctgaacttcacct
tttggctttcttggcctgatgctgaactctcgaggttgagccccatatg
(Seq ID No: 81)

Homo sapiens v-erb-b2 erythroblastic leukemia viral oncogene homolog 3 (avian) (ERBB3):

atccctccccggactccggctccggctccgattgcaatttgcaacctccgctgccgtcgc
cgcagcagccaccaattcgccagcggttcaggtggctcttgcctcgatgtcctagcctag
gggcccccgggccggacttggctgggctcccttcaccctctgcggagtcatg
(Seq ID No: 82)

Homo sapiens podoplanin (PDPN): ccgcctcctcgggagagataaatg (Seq ID No: 83)

Homo sapiens ribonucleotide reductase M1 (RRM1):

gcgcccctttgtgcgtcacgggtggcgggcgcgggaaggggatttggattgttgcgcctc
tgctctgaagaaagtgctgtctggctccaactccagttctttcccctgagcagcgcctgg
aacctaacccttcccactctgtcaccttctcgatcccgccggcgctttagagccgcagtc
cagtcttggatccttcagagcctcagccactagctgcgatg (Seq ID No: 84)

Homo sapiens solute carrier family 2 (facilitated glucose transporter), member 4 (SLC2A4):

gcgtctttcccccagccccgctccaccagatccgcgggagccccactgctctccgggtc
cttggcttgtggctgtgggtcccatcgggcccgccctcgcacgtcactccgggaccccg
cggcctccgcaggttctgcgctccaggccggagtcagagactccaggatcggttctttca
tcttcgccgcccctgcgcgtccagctcttctaagacgagatg (Seq ID No: 85)

Homo sapiens steroid-5-alpha-reductase, alpha polypeptide 1 (3-oxo-5 alpha-steroid delta 4-dehydrogenase alpha 1) (SRD5A1) :

aaccctttctgcagagtcccggcagtgcgggactccggtagccgcccctccggtagccgc
ccctcctgcccccgcgccgccgccctatatgttgcccgccgcggcctctggggcatggag
cacgctgcccagccctggcgatg (Seq ID No: 86)

Homo sapiens thromboxane A synthase 1 (platelet) (TBXAS1):

gttccctttctacctgcagagcacggttcccataagggcggcgagatcagcctcctgtc
tcatctggaagaccaccactctggggtctcagaggaatg (Seq ID No: 87)

Homo sapiens transketolase (TKT): ctatctctgtgtgtccgcgtgtgcgcccggtccccgcctgccgcaccatg (Seq ID No: 88)

Homo sapiens tumor necrosis factor receptor superfamily, mem ber 1A (TNFRSF1A) :

cctcctcctccagctcttcctgtcccgctgttgcaacactgcctcactcttcccctccca
ccttctctcccctcctctctgctttaatttctcagaattctctggactgaggctccagt
tctggcctttggggttcaagatcactgggaccaggccgtgatctctatgcccgagtctca
accctcaactgtcaccccaaggcacttgggacgtcctggacagaccgagtcccgggaagc
cccagcactgccgctgccacactgccctgagcccaaatgggggagtgagaggccatagct
gtctggcatg (Seq ID No: 89)

Homo sapiens tubulin, beta 2A class IIa (TUBB2A):

aggtctctgcgcagcccagcccgccggtccacgccgcgcaccgctccgagggccagcgcc
acccgctccgcagccggcaccatg (Seq ID No: 90)

Homo sapiens actin, beta (ACTB): tcgcctttgccgatccgccgcccgtccacacccgccgccagctcaccatg (Seq ID No: 91)

Homo sapiens adenylosuccinate synthase (ADSS):

ggctccttcttcctctgcatgtggctggcggccgcagagcagttcagttcgctcactcct
cgccggccgcctctccttcgggctctcctcgcgtcactggagccatg
(Seq ID No: 92)

Homo sapiens alanyl (membrane) aminopeptidase (ANPEP): cgttctctgcctggcctgaggctccctgagccgcctccccaccatcaccatg (Seq ID No: 93)

Homo sapiens beaded filament structural protein 1, filensin (BFSP1):

gcctcctttctttctcagcccagacctggccctctggagagggttttggagtcctgggta
ggcagggtacctcaggcagcaggcagcacaccttggatgtgagctgaatggattttcaaa
tttcacagaaggagcctccatgctggagaaagtatgtatg (Seq ID No: 94)

Homo sapiens basic transcription factor 3 (BTF3):

cggcctccctttagctgccatcttgcgtccccgcgtgtgtgcgcctaatctcaggtggtc
cacccgagaccccttgagcaccaaccctagtcccccgcgcggccccttattcgctccgac
aagatg (Seq ID No: 95)

Homo sapiens complement component 1, q subcomponent binding protein (C1QBP) : ttgtcctttgcatctgcacgtgttcgcagtcgtttccgcgatg (Seq ID No: 96)

Homo sapiens calsequestrin 1 (fast-twitch, skeletal muscle) (CASQ1) :

tttcctttcttaatatggcgatgagctcttaggccagtgtggggaccggggctgaggtgc
cctggacactggaggaggggagggaaggagcccctgggagcctggggtagaagtgtagg
aggtgggaggattccggcccgcatggagctgtcctggcctcagaaggttatccgtctctc

ctgccaaccatggagacatatttagacaggaccaggtggggactgaggggtgccaatttc
agggggcagctccggttccctccccgcccccctgctcctattcctccacctgacccttttt
cccttggctctgtcggcagtttctccaggacccagcagtgccctctgtccactgctctgg
gccattccccaatccccccctcccacttgagcccctaactcagaatctgggacccaggggc
ccctccctaccccagctaacctcttctggaccaggagagccaacccagatcccactacct
ccatg (Seq ID No: 97)

Homo sapiens caveolin 3 (CAV3):

gtctctctgcccctctctgccccaagtattttcagccccagccggccacacagctcggat
ctcctcctgtggatccccccagctctgcgatg (Seq ID No: 98)

Homo sapiens serpin peptidase inhibitor, clade H (heat shock protein 47), member 1, (collagen binding protein 1) (SERPINH1):

aggtctttggctttttttggcggagctggggcgccctccggaagcgtttccaactttcca
gaagtttctcgggacgggcaggagggggtggggactgccatatatagatcccgggagcag
gggagcgggctaagagtagaatcgtgtcgcggctcgagagcgagagtcacgtcccggcgc
tagcccagcccgacccaggcccaccgtggtgcacgcaaaccacttcctggccatg
(Seq ID No: 99)

Homo sapiens CD68 molecule (CD68):

tttcctcctttccaagagagggctgagggagcagggttgagcaactggtgcagacagcct
agctggactttgggtgaggcggttcagccatg (Seq ID No: 100)

Homo sapiens cell division cycle 20 homolog (S. cerevisiae) (CDC20) :

gggtccctttctgtcccctgagcaccgtcgcctcctttcctccagggctccgtaggcacc
aactgcaaggacccctccccctgcgggcgctcccatg (Seq ID No: 101)

Homo sapiens cadherin 13, H-cadherin (heart) (CDH13):

gagcctctcctcaaagcctggctcccacggaaaatatgctcagtgcagccgcgtgcatga
atgaaaacgccgccgggcgcttctagtcggacaaaatg (Seq ID No: 102)

Homo sapiens regulator of chromosome condensation (RCC1) and BTB (POZ) domain containing protein 2 (RCBTB2):

cgctcccttcgtttccgtctcggccgggcacccgagcgcatcccgccgaggccgggccgt
ttcagggggaggcgccaactcatcgcggcgccgggcccctgaccgtgcagtaaccgctac
ccaggaggcggagcggacaaggctccggcctgcgaggagtcacattaactttgctctaga
agacaactttacaaggatctaaaggaacaggattaaagatgactgaatactgggttcca
gaaatttaaaacaatcagcttagcaaatcatatattcttctgtggagctgagaattgatg
tccgctcttccccgtgatttggaactttccaatcccagagaaaagttgacaaaggactg
cccaggactgagtccatatg (Seq ID No: 103)

Homo sapiens cold inducible RNA binding protein (CIRBP):

ccccccctcactcgcgcgttaggaggctcgggtcgttgtggtgcgctgtcttcccgcttg
cgtcagggacctgcccgactcagtggccgccatg (Seq ID No: 104)

Homo sapiens LIM domain binding 2 (LDB2):

cctcctctcctctccctctcctctcctgctatagagggctccgacagcagttcccagcca
gcgtgttcagcctgcctgcctgcctgcctctgtgtgtgtgtgagcgtgtgtgcgtgcgtc

tactttgtactgggaagaacacagcccatgtgctctgcatggacgttactgatactctgt
ttagcttgattttcgaaaagcaggcaagatg (Seq ID No: 105)

Homo sapiens chloride channel, nucleotide-sensitive, 1A (CLNS1A) :

ctgcctcttccagggcgggcggtgtggtgcacgcattgctgtgctccaactccctcaggg
cctgtgttgccgcactctgctgctatg (Seq ID No: 106)

Homo sapiens collapsin response mediator protein 1 (CRMP1):

cctcctccttctcccgccctcctcgccgatccgggcggtgctggcagccggagcggcggc
gggcgggccgagcagccggggcagccgcgcgtgggcatccacgggcgccgagcctccgtc
cgtgtctctatccctcccgggcctttgtcagcgcgcccgctgggagcggggccgagagcg
ccggttccagtcagacagccccgcaggtcagcggccgggccgagggcgccagagggggcc
atg (Seq ID No: 107)

Homo sapiens catenin (cadherin-associated protein), delta 1 (CTNND1) :

ttgcctttggctgggtgcaacttccattttaggtgttggatctgaggggaaaaaaaaga
gagagggagagagagagaaagaagagcaggaaagatcccgaaaggaggaagaggtggcga
aaaatcaactgccctgctggatttgtctttctcagcaccttggcgaagccttgggtttct
ttcttaaaggactgatttttagaactccacatttgaggtgtgtggcttttgaagaaaatg
tatgtactgacgggaaaaggaggataagcaagtcgaattttgtcttacgctctctcctt
cctgcttcctccttgctgtggtggctgggatgcttcttccatgatttttgaatctagac
tgggctgttctctgtgttaaaccaatcagttgcgaccttctcttaacagtgtgaagtgag
gggtctctctccctccttctccttcctctgtgattcaccttccttttaccctgccctg
cggcggctccgccccttaccttcatg (Seq ID No: 108)

Homo sapiens diacylglycerol kinase, alpha 80kDa (DGKA):

ccgtcccctccagcccagctcgggctccagctccagcgccggcgcttcagctgcgaccgc gagccctctcaagcaagatataacttccccaagtcacacagtggtatcagagctaagaat gggacccagatatgactgatctagttctgttccaaaaccgtgctgtattatattaacgcc taccctctgaagaggtccaagcaacggaagtactactacgaagctgcctttctggccatc cttgagaaaaatagacagatgagttcctgccagtgagtccctaggcctccatctctctcc cttgctgtaccaccttcaccaccatccatgcgaccccaagagccttaatgactctagaag agactccaggcaggggaagctgaaaggacctttcactccctacttttggccagggccttc tgtgccacctgccaagaccagcaggcctaccctctgaagaggtccaagcaacggaagtac tactacgaagctgcctttctggccatccttgagaaaaatagacagatg
(Seq ID No: 109)

Homo sapiens aspartyl-tRNA synthetase (DARS):

cgatctttctggagccgcacctccacgcggagtccgagcgcgtgtgctgagaccccaggg tcgggagggcggagactgggagggagggagaagcccctttggcctgccttacggaagcct gcgagggagggtggtgtccactgcccagttccgtgtcccgatg (Seq ID No: 110)

Homo sapiens dynein, cytoplasmic 1, intermediate chain 2 (DYNC1I2) :

agttcttctcgatcgtgtcagtttgtaaggcgagggcggaagttggattcctggcctgag aatattaggcgtagttttccagtttttggcaaagcggaaatacttaaggcccctgggttg

actgggttctttgttttatctaccggcttctgctttacgacaggtcacaaacatg
(Seq ID No: 111)

Homo sapiens dedicator of cytokinesis 1 (DOCK1) :

tttcctccccatcctgtcgcggctcgaaaggaatggaaaatggcggcctagacgcggagt ttcctgcccgacccgcggcggctccggcggcgccatg (Seq ID No: 112)

Homo sapiens dihydropyrimidinase-like 2 (DPYSL2):

ctctctctttttttttccgccctagctggggctgtgttggaggagaggaagaaagagagac agaggattgcattcatccgttacgttcttgaaatttcctaatagcaagaccagcgaagcg gttgcaccctttttcaatcttgcaaaggaaaaaaacaaaacaaaacaaaaaaaaacccaagt ccccttcccggcagtttttgccttaaagctgccctcttgaaattaatttttttcccaggag agagatg (Seq ID No: 113)

Homo sapiens developmentally regulated GTP binding protein 2 (DRG2) :

tgttctctttggcttccgggcgcacgctactctgtcgccgccgtcagaccggaattgccg gtgccgccgccaccgctgtctgtgcgcccacctctgctgctaccatg
(Seq ID No: 114)

Homo sapiens eukaryotic translation elongation factor 1 alph a 1 (EEF1A1) :

cgttctttttcgcaacgggtttgccgccagaacacaggtgtcgtgaaaactacccctaaa agccaaaatg (Seq ID No: 115)

Homo sapiens eukaryotic translation elongation factor 1 gamm a (EEF1G) :

tctcctctttccccctcccttctctcccgggcggcttactttgcggcagcgccgagaacc ccaccccctttctttgcggaatcaccatg (Seq ID No: 116)

Homo sapiens eukaryotic translation initiation factor 2, sub unit 3 gamma, 52kDa (EIF2S3): atttccttcctcttttggcaacatggcgggc (Seq ID No: 117)

Homo sapiens eukaryotic translation initiation factor 4B (EIF4B): gggtcttttgcgttctctttccctctcccaacatg (Seq ID No: 118)

Homo sapiens eukaryotic translation initiation factor 4 gamm a, 2 (EIF4G2): tattcttttgaagattcttcgttgtcaagccgccaaagtg (Seq ID No: 119)

Homo sapiens epithelial membrane protein 1 (EMP1):

cttcccctcagtgcggtcacatacttccagaagagcggaccagggctgctgccagcacct gccactcagagcgcctctgtcgctgggacccttcagaactctctttgctcacaagttacc aaaaaaaaaagagccaacatg (Seq ID No: 120)

Homo sapiens fibrillarin (FBL):

cgctcttttccacgtgcgaaagccccggactcgtggagttgtgaacgccgcggactccgg agccgcacaaaccagggctcgccatg (Seq ID No: 121)

Homo sapiens exostoses (multiple)-like 2 (EXTL2):

ctgtcccttgctccaggcgctcactttgcgggcggcactttttccaggttgttaatccag ctaatggagaaggatagatgcacgctacttggtttagaaaaaaaaacaaaaatgagcaaa cgagacgccccttccgttttatgataactaagctgcagggaaataaatcggctggcccta ctgcaatctactgcactcgagaaacatcacagaaaattctttgatttatcttaatagtga caagtgagcctgcttctgtcaattactgaagctataaggagatttttaaaaattaaact tcaacacaatg (Seq ID No: 122)

Homo sapiens solute carrier family 37 (glucose-6-phosphate transporter), member 4 (SLC37A4):

ccgcctctgttcaggacactgggtccccttggagcctccccaggcttaatgattgtccag aaggcggctataaagggagcctgggaggctgggtggaggagggagcagaaaaaacccaac tcagcagatctgggaactgtgagagcggcaagcaggaactgtggtcagaggctgtgcgtc ttggctggtagggcctgctcttttctaccatg (Seq ID No: 123)

Homo sapiens GDP dissociation inhibitor 2 (GDI2) :

agccctcccctcctcgctccctcccctcctctccccgcccagttcttctcttcccgtctg aggtggcggtcggtctcgccttgtcgccagctccattttcctctctttctcttcccccttt ccttcgcgcccaagagcgcctcccagcctcgtagggtggtcacggagcccctgcgcctttt tccttgctcgggtcctgcgtccgcgcctgccccgccatg (Seq ID No: 124)

Homo sapiens UDP-Gal:betaGlcNAc beta 1,4- galactosyltransfer ase, polypeptide 1 (B4GALT1): caccccttcttaaagcggcggcgggaagatg (Seq ID No: 125)

Homo sapiens GDP-mannose 4,6-dehydratase (GMDS):

ggccctccctgcacggcctcccgtgcgcccctgtcagactgtggcggccggtcgcgcggt
gcgctctccctccctgcccgcagcctggagaggcgcttcgtgctgcacacccccgcgttc
ctgccggcaccgcgcctgccctctgccgcgctccgccctgccgccgaccgcacgcccgcc
gcgggacatg (Seq ID No: 126)

Homo sapiens histone deacetylase 2 (HDAC2):

ggccccctcctcgcgagttggtgccgctgccacctccgattccgagctttcggcacctct
gccgggtggtaccgagccttcccggcgccccctcctctcctcccaccggcctgcccttcc
ccgcgggactatcgcccccacgtttccctcagcccttttctctcccggccgagccgcggc
ggcagcagcagcagcagcagcaggaggaggagcccggtggcggcggtggccggggag
cccatg (Seq ID No: 127)

Homo sapiens protein arginine methyltransferase 2 (PRMT2):

gggccttcccggctgacggcctgcgtgcactgcgcttgcgcgggttgagggcggtggctc
aggctcctggaaaggaccgtccacccctccgcgctggcggtgtggacgcggaactcagcg
gagaaacgcgattgagagcagtgtgtggattacactatcactggaaaaatacgaattgag
aagaaggaaaagactggaagatgcagaccttggttcctgttagtggaaacactgtaaggt
cccagaaatggaaaagaaaatgaaataaatcagcagttatgaggcagagcctaagagaac
tatg (Seq ID No: 128)

Homo sapiens immunoglobulin (CD79A) binding protein 1 (IGBP1) : gttcctctctccccaagatg (Seq ID No: 129)

Homo sapiens eukaryotic translation initiation factor 3, sub unit E (EIF3E): actcccttttctttggcaagatg (Seq ID No: 130)

Homo sapiens activated leukocyte cell adhesion molecule (ALCAM) :

gtccctctactcagagcagcccggagaccgctgccgccgctgccgctgctaccaccgctg
ccacctgaggagacccgccgcccccccgtcgccgcctcctgcgagtccttcttagcacct
ggcgtttcatgcacattgccactgccattattattatcattccaatacaaggaaaataaa
agaagataccagcgaaaagaaccgcttacacctttccgaattactcaagtgtctcctgga
aacagagggtcgttgtccccggaggagcagccgaagggcccgtgggctggtgttgaccgg
gagggaggaggagttgggggcattgcgtggtggaaagttgcgtgcggcagagaaccgaag
gtgcagcgccacagcccaggggacggtgtgtctgggagaagacgctgcccctgcgtcggg
acccgccagcgcgcggcaccgcggggcccggggacgacgccccctcctgcggcgtggact
ccgtcagtggcccaccaagaaggaggaggaatatg (Seq ID No: 131)

Homo sapiens acyloxyacyl hydrolase (neutrophil) (AOAH):

ttttctttatcctgcagtctttacctcagcagaaccgcacaccacagactccctccagct
ctttgtgtgtggctctctcagggtccaacaagagcaagctgtgggtctgtgagtgtttat
gtgtgctttattcacttcacacttattgaaaagtgtgtatgtgagagggtggggtgtgt
gtgtcaaagagagtgaggaagagaaggagagagagatcaattgattctgcagcctcagct
ccagcatccctcagttgggagcttccaaagccgggtgatcacttggggtgcatagctcgg
agatg (Seq ID No: 132)

Homo sapiens ADP-ribosylation factor 1 (ARF1):

ccgccccttacccggcgtgccccgcgcccggaggcgctgacgtggccgccgtcagagccg ccatcttgtgggagcaaaaccaacgcctggctcggagcagcagcctctgaggtgtccctg gccagtgtccttccacctgtccacaagcatg (Seq ID No: 133)

Homo sapiens ADP-ribosylation factor 6 (ARF6) :

gcgcctttccggcagcggcggcggcagaactgggaggaggagttggaggccggagggag cccgcgctcggggcggcggctggaggcagcgcaccgagttcccgcgaggatccatgacct gacggggccccggagccgcgctgcctctcgggtgtcctgggtcggtggggagcccagtgc tcgcaggccggcgggcgggccggagggctgcagtctccctcgcggtgagaggaaggcgga ggagcgggaaccgcggcggcgctcgcgcggcgcctgcgggggaagggcagttccgggcc gggccgcgcctcagcagggcggcggctcccagcgcagtctcagggcccgggtggcggcgg cgactggagaaatcaagttgtgcggtcggtgatgcccgagtgagcgggggcctgggcct ctgcccttaggaggcaactcccacgcaggccgcaaaggcgctctcgcggccgagaggctt cgtttcggtttcgcggcggcggcggcgttgttggctgaggggacccgggacacctgaatg cccccggccccggctcctccgacgcgatg (Seq ID No: 134)

Homo sapiens ras homolog family member A (RHOA):

cgccctcccgccgccgcccgccctcgctctctcgcgctaccctcccgccgcccgcggtcc tccgtcggttctctcgttagtccacggtctggtcttcagctacccgccttcgtctccgag tttgcgactcgcggaccggcgtccccggcgcgaagaggctggactcggattcgttgcctg agcaatg (Seq ID No: 135)

Homo sapiens ras homolog family member G (RHOG):

cggcctcccgctctcacttccttctcgagcccggagccgctgccgccgcccccagctccc

ccgcctcggggagggcaccaggtcactgcagccagaggggtccagaagagagaggaggca ctgcctccactacagcaactgcacccacgatg (Seq ID No: 136)

Homo sapiens ATP synthase, H+ transporting, mitochondrial F1 complex, O subunit (ATP50) : ctctcttcccactcgggtttgacctacagccgcccgggagaagatg (Seq ID No: 137)

Homo sapiens B lymphoid tyrosine kinase (BLK):

ccacctctgtctgctgccggcagaaagccacaagccatgaaaactgattgagatgagaag aattcatctgggactggcttttgctttaggatggtgttggaagttgctcgttgtcgctag gagcctgctccactgtaagggtgtcaggatctgaagagctatggtgaaacaccactgaag cattgccaaggatg (Seq ID No: 138)

Homo sapiens B-cell translocation gene 1, anti-proliferative (BTG1) :

gcatctcttcgcctctcggagctggaaatgcagctattgagatcttcgaatgctgcggag ctggaggcggaggcagctggggaggtccgagcgatgtgaccaggccgccatcgctcgtct cttcctctctcctgccgcctcctgtctcgaaaataactttttagtctaaagaaagaaag acaaaagtagtcgtccgcccctcacgccctctcttcctctcagccttccgcccggtgagg aagcccggggtggctgctccgccgtcggggccgcgccgccgagccccagccgccccgggc cgcccccgcacgccgcccccatg (Seq ID No: 139)

Homo sapiens calcium modulating ligand (CAMLG):

cggcctctagtcatcgccctcgcagcggcggccaacatcaccgccactgccacccctccc agactgtggacgggaggatg (Seq ID No: 140)

Homo sapiens calnexin (CANX):

aggcctcttggttctgcggcacgtgacggtcggccgcctccgcctctctctttactgcg gcgcggggcaaggtgtgcgggcgggaaggggcacgggcacccccgcggtccccgggaggc tagagatcatg (Seq ID No: 141)

Homo sapiens calpain 2, (m/II) large subunit (CAPN2): cgacctttctctgcgcagtacggccgccgggaccgcagcatg (Seq ID No: 142)

Homo sapiens caveolin 1, caveolae protein, 22kDa (CAV1):

gcgccttttttcccccatacaatacaagatcttccttcctcagttcccttaaagcaca gcccagggaaacctcctcacagttttcatccagccacgggccagcatg (Seq ID No: 143)

Homo sapiens CD1d molecule (CD1D) :

cgacctctttgcagctcgcacagctaagggcgagggcgcccttcggcagaagcagcaaac cgccggcaagcccagcgaggagggctgccggggtctgggcttgggaattggctggcaccc agcggaaagggacgtgagctgagcggcgggggagaagagtgcgcaggtcagagggcggcg cgcagcggcgctccgcgaggtccccacgccgggcgatatg (Seq ID No: 144)

Homo sapiens CD22 molecule (CD22):

tctccttttgctctcagatgctgccagggtccctgaagagggaagacacgcggaaacagg cttgcacccagacacgacaccatg (Seq ID No: 145)

Homo sapiens CD37 molecule (CD37) :

cttcctctttgggggttcttcctttctctctcagctctccgtctctctttctctctcagc ctctttctttctccctgtctcccccactgtcagcacctcttctgtgtggtgagtggaccg cttaccccactaggtgaagatg (Seq ID No: 146)

Homo sapiens CD38 molecule (CD38):

gcctctctcttgctgcctagcctcctgccggcctcatcttcgcccagccaaccccgcctg gagccctatg (Seq ID No: 147)

Homo sapiens CD48 molecule (CD48):

cggccttttctagccaggctctcaactgtctcctgcgttgctgggaagttctggaagga agcatg (Seq ID No: 148)

Homo sapiens chromogranin B (secretogranin 1) (CHGB): cttcctttccgcacaggggccgccgagcggggccatg (Seq ID No: 149)

Homo sapiens chloride channel, voltage-sensitive 3 (CLCN3):

ttccccttccgtgggtcagggccggtccggtccggaacctgcagcccctttcccagtgtt
ctagttcgcccgtgacccggaataatgagcaaggagggtgtggtgggttgaaagccatcc
tactttactcccgagttagagcatggattcagttttagtcttaaggggaagtgagattg
gagatttttatttttaattttgggcagaagcaggttgactctagggatctccagagcgag
aggatttaacttcatgttgctcccgtgtttgaaggaggacaataaaagtcccaccgggca
aaattttcgtaacctctgcggtagaaaacgtcaggtatcttttaaatcgcgatagttttc
gctgtgtcaggctttcttcggtggagctccgagggtagctaggttctaggtttgaaacag
atgcagaatccaaaggcagcgcaaaaaacagccaccgatttgctatgtctctgagctgc
gagataatcagacagctaaatg (Seq ID No: 150)

Homo sapiens colipase, pancreatic (CLPS):

ttccccttccgtgggtcagggccggtccggtccggaacctgcagcccctttcccagtgtt
ctagttcgcccgtgacccggaataatgagcaaggagggtgtggtgggttgaaagccatcc
tactttactcccgagttagagcatggattcagttttagtcttaaggggaagtgagattg
gagatttttatttttaattttgggcagaagcaggttgactctagggatctccagagcgag
aggatttaacttcatgttgctcccgtgtttgaaggaggacaataaaagtcccaccgggca
aaattttcgtaacctctgcggtagaaaacgtcaggtatcttttaaatcgcgatagttttc
gctgtgtcaggctttcttcggtggagctccgagggtagctaggttctaggtttgaaacag
atgcagaatccaaaggcagcgcaaaaaacagccaccgatttgctatgtctctgagctgc
gagataatcagacagctaaatg (Seq ID No: 151)

Homo sapiens cytochrome c oxidase subunit IV isoform 1 (COX4I1) :

ctaccctttccgctccacggtgacctccgtgcggccgggtgcgggcggagtcttcctcg
atcccgtggtgctccgcggcgcggccttgctctcttccggtcgcgggacaccgggtgtag
agggcggtcgcggcgggcagtggcggcagaatg (Seq ID No: 152)

Homo sapiens cytochrome c oxidase subunit VIIc (COX7C) :

ctttcttttcagtccttgcgcaccggggaacaaggtcgtgaaaaaaaaggtcttggtgag
gtgccgccatttcatctgtcctcattctctgcgcctttcgcagagcttccagcagcggta
tg (Seq ID No: 153)

Homo sapiens activating transcription factor 2 (ATF2):

cagccttttcctccaggggtgctttgtaaacacggctgtgctcagggctcgcgggtgacc
gaaaggatcatgaactagtgacctggaaagggtactagatggaaacttgagaaaggactg
cttattgataacagctaaggtattcctggaagcagagtaaataaagctcatggcccacca
gctagaaagtattcttgccatgagaaaaagaatgtgataagttattcaacttatg
(Seq ID No: 154)

Homo sapiens casein kinase 1, alpha 1 (CSNK1A1) :

agatccctttcccagagtgctctgcgccgtgaagaagcggctcccggggactgggggcat
tttgtgttggctggagctggagtaacaagatggcgtcgtccgcggagtgacaggggtccc
tctgggccggagccggcggcagtggtggcagcggtatcgccgccctagctcaccgcgccc
cttttccagcccgcgacgtcgccgcgcaagcgaggcagcggcggccgccgagaaacaagt
ggcccagcctggtaaccgccgagaagcccttcacaaactgcggcctggcaaaaagaaacc
tgactgagcggcggtgatcaggttcccctctgctgattctgggccccgaaccccggtaaa
ggcctccgtgttccgtttcctgccgccctcctccgtagccttgcctagtgtaggagcccc
gaggcctccgtcctcttcccagaggtgtcggggcttggccccagcctccatcttcgtctc
tcaggatg (Seq ID No: 155)

Homo sapiens catenin (cadherin-associated protein), beta 1, 88kDa (CTNNB1):

aagcctctcggtctgtggcagcagcgttggcccggccccgggagcggagagcgaggggag
gcggagacggaggaaggtctgaggagcagcttcagtccccgccgagccgccaccgcaggt
cgaggacggtcggactcccgcggcgggaggagcctgttcccctgagggtatttgaagtat
accatacaactgttttgaaaatccagcgtggacaatg (Seq ID No: 156)

Homo sapiens dCMP deaminase (DCTD):

ccgcctcctcccccgacttccttccctgagcacggcggcggcggggacgagcaccggcct
gcgcgcggagccggcaccggatgacccaacatg (Seq ID No: 157)

Homo sapiens damage-specific DNA binding protein 1, 127kDa (DDB1) :

ctgtcttttcgcttgtgtccctctttctagtgtcgcgctcgagtcccgacgggccgctcc
aagcctcgacatg (Seq ID No: 158)

Homo sapiens desmin (DES):

ctgtctcccctcgccgcatccactctccggccggccgcctgcccgccgcctcctccgtgc
gcccgccagcctcgcccgcgccgtcaccatg (Seq ID No: 159)

Homo sapiens deoxyhypusine synthase (DHPS):

cgttccctacttcctgtgctcttgcggagacgcgcgcgtcggggtttaacgcgtttctgg
gccgccgtaagcccggcctaggggcagctttgactcgagagccggctataggcgcatg
(Seq ID No: 160)

Homo sapiens dihydrolipoamide S-acetyltransferase (DLAT):

caccctttcggatgcctcccctagaaccctaccactttccacccctttccgtctgttatt
tctcccaaacttgcgcccgcacaggcccctctggaacactcctgccccgtagtgcccctc
gtccccgctccgtagagaaagagcgtgcgtgccgcgcatttctggcctggggagcgggtg
gagtaaacctgcgggaaccattttacgacaacgtgcggctgtgcggtgtggctgacggca
acgccgctgctcttggagaggtcactccggagacggcgttggttttggggtgtgggggt
tggtggcactatg (Seq ID No: 161)

Homo sapiens down-regulator of transcription 1, TBP-binding (negative cofactor 2) (DR1):

ccttccctggcatctggagggaccaccgttgccgcgtcttcggcttccacgatctgcgtt cggctacgcggccacggcggcagccactgcgactcccactgtgcctggctctgtccata ttagttcccaggcggccgtcgccgttccagcagcggcagcggcagcggcagcggcggaca tgttgtgaggcggcggcgcgggtgtctgaaggatggtttggccgaggcggcggcaacggc tgctggcggcggcggcagcggcagcggggcctcgggctctatagagccgagcccgctggg tacccgcccggtaccgcggcgaggccagtgcccctggatcttgcctctgctccgacgccg ttggggaccagttaggcgacagcgcccgcccctctgaggagacacgaaggtggttcccca gccgctcaaatttccggaccaccgcgctttcccctcctcagcctgggctgtgctctctct agaatcctcgggccccccactttcttcccaaactcatcctaaatctctcacacacgcgagt gttcccagccctcaagccagctgctcctccgttcatttctgcaccctcttcgcaaagca ccccccgggatcactctccgagggcgactttttgagaaatctcggtggagtagtggacca gagctggggagtttttaaaagccggggcgcgagaaacaggaaggtactatg (Seq ID No: 162)

Homo sapiens endothelin receptor type A (EDNRA):

ttttctttttcgtgcgagccctcgcgcgcgcgtacagtcatcccgctggtctgacgattg tggagaggcggtggagaggcttcatccatcccacccggtcgtcgccggggattggggtcc cagcgagacctccccgggagaagcagtgcccaggaggttttctgaagccggggaagctgt gcagccgaagccgccgccgcgccggagcccgggacaccggccaccctccgcgccacccac cctcgccggctccggcttcctctggcccaggcgccgcgcggacccggcagctgtctgcgc acgccgagctccacggtgaaaaaaaagtgaaggtgtaaaagcagcacaagtgcaataaga gatatttcctcaaatttgcctcaagatg (Seq ID No: 163)

Homo sapiens eukaryotic translation elongation factor 1 alph a 2 (EEF1A2) :

cagtccctctggctgagacctcggctccggaatcactgcagccccccctcgccctgagcca gagcaccccgggtcccgccagcccctcacactcccagcaaaatg (Seq ID No: 164)

Homo sapiens eukaryotic translation elongation factor 2 (EEF2) :

cgttctcttccgccgtcgtcgccgccatcctcggcgcgactcgcttctttcggttctacc tgggagaatccaccgccatccgccaccatg (Seq ID No: 165)

Homo sapiens eukaryotic translation initiation factor 4A2 (EIF4A2): ctgtcttttcagtcgggcgctgagtggtttttcggatcatg (Seq ID No: 166)

Homo sapiens egf-like module containing, mucin-like, hormone receptor-like 1 (EMR1) : gtttcttttctttgaatgacagaactacagcataatg (Seq ID No: 167)

Homo sapiens enolase 2 (gamma, neuronal) (ENO2) :

gcgcctcctccgcccgccgcccgggagccgcagccgccgccgccactgccactcccgctc tctcagcgccgccgtcgccaccgccaccgccaccgccactaccaccgtctgagtctgcag tcccgagatcccagccatcatg (Seq ID No: 168)

Homo sapiens esterase D (ESD):

ccgcctttacttcggcccgcttcttctggtcactccgccaccgtagaatcgcctaccat
ttggtgcaagcaaaaagcaatcagcaattggacaggaaaagaatg
(Seq ID No: 169)

Homo sapiens Finkel-Biskis-Reilly murine sarcoma virus (FBR-MuSV) ubiquitously expressed (FAU): cttcctctttctcgactccatcttcgcggtagctgggaccgccgttcagtcgccaatatg (Seq ID No: 170)

Homo sapiens Friend leukemia virus integration 1 (FLI1) :

ctgtctctttcgctccgctacaacaacaaacgtgcacaggggagtgagggcagggcgctc
gcaggggcacgcagggagggcccagggcgccagggaggccgcgccgggctaatccgaag
gggctgcgaggtcaggctgtaaccgggtcaatgtgtggaatattgggggctcggctgca
gacttggccaaatg (Seq ID No: 171)

Homo sapiens fibromodulin (FMOD):

gccccttttcacaatatttgattaggaatttggggcgggaccctggtctggcacaggcac
gcacactctcagtagactctttcactcctctctcttcctctctcacacgttctccaac
ccaaggaggccagacagagggacgtggtcactctctgaaaagttcaacttgagagacaaa
atg (Seq ID No: 172)

Homo sapiens ferritin, heavy polypeptide 1 (FTH1):

cgttcttcgccgagagtcgtcggggtttcctgcttcaacagtgcttggacggaacccggc
gctcgttccccaccccggccggccgcccatagccagccctccgtcacctcttcaccgcac
cctcggactgccccaaggcccccgccgccgctccagcgccgcgcagccaccgccgccgcc
gccgcctctccttagtcgccgccatg (Seq ID No: 173)

Homo sapiens glyceraldehyde-3-phosphate dehydrogenase (GAPDH) :

cgctctctgctcctcctgttcgacagtcagccgcatcttcttttgcgtcgccagccgagc
cacatcgctcagacaccatg (Seq ID No: 174)

Homo sapiens glycyl-tRNA synthetase (GARS): caccctctctggacagcccagggccgcaggctcatg (Seq ID No: 175)

Homo sapiens glutamic-oxaloacetic transaminase 2, mitochondr ial (aspartate aminotransferase 2) (GOT2):

ctgtccttaccttcagcaggagccggttccctgtgtgtgtgtccgctcgccctctgctcc
gtcctgcggctgcccactgccctcctacggtccaccatg (Seq ID No: 176)

Homo sapiens general transcription factor IIF, polypeptide 1 , 74kDa (GTF2F1) :

gcgcctcttccggttacctttcccagcgccagaggcgcctaggttggggtcctcgctc
aggcacagagacccgacaccgagcggcggcttccccgggatcgagggacgcgcacgccag
aggagacgaaaggaacccgggtcggaccagatcggaaccactgaccattgcccatg
(Seq ID No: 177)

Homo sapiens glycogen synthase 1 (muscle) (GYS1):

cggcctccttctgcctaggtcccaacgcttcggggcaggggtgcggtcttgcaataggaa gccgagcgtcttgcaagcttcccgtcgggcaccagctactcggccccgcaccctacctgg tgcattccctagacacctccggggtccctacctggagatccccggagccccccttcctgc gccagccatg (Seq ID No: 178)

Homo sapiens major histocompatibility complex, class I, C (HLA-C): cattctccccagaggccgagatg (Seq ID No: 179)

Homo sapiens major histocompatibility complex, class II, DP beta 1 (HLA-DPB1):

gctccctttagcgagtccttcttttcctgactgcagctcttttcattttgccatcctttt ccagctccatg (Seq ID No: 180)

Homo sapiens 3-hydroxy-3-methylglutaryl-CoA synthase 1 (soluble) (HMGCS1):

ctgtcctttcgtggctcactccctttcctctgctgccgctcggtcacgcttgctctttca ccatg (Seq ID No: 181)

Homo sapiens hippocalcin (HPCA):

ccgccttccctgcgcagtcggtgtctccgcgtcgctgggtgggacttggctcggcggcca tg (Seq ID No: 182)

Homo sapiens hydroxysteroid (17-beta) dehydrogenase 2 (HSD17B2) :

ctcccttcttgactctctgttcacagaactcaggctgcctccagccagcctttgcccgct agactcactggccctgagcacttgaaggtgcagcaagtcactgagaatg (Seq ID No: 183)

Homo sapiens heat shock 60kDa protein 1 (chaperonin) (HSPD1):

ctgtccctcactcgccgccgacgacctgtctcgccgagcgcacgccttgccgccgccccg cagaaatg (Seq ID No: 184)

Homo sapiens intercellular adhesion molecule 3 (ICAM3):

ccgccttttcccctgcctgcccttcgggcacctcaggaaggcaccttcctctgtcagaat g (Seq ID No: 185)

Homo sapiens inositol polyphosphate-1-phosphatase (INPP1) :

cgtcctctggccgcgcctgcggccgcacgcccagcgcccctcgcctaacctcgcgcccgg gccgcgcctcctcctcctcctgctccccgccgcttccgtttctcgagggaaaggctgctg cctcctgctctgtcctcatccccggcttagctgacggcccagagggtgggtgccaattcc accagcagctgcaactgaaaagcaaggttcagaaatg (Seq ID No: 186)

Homo sapiens interferon regulatory factor 2 (IRF2):

gtttcctctccttgttttgctttcgatctggactgttctcaggcaagccggggagtaact tttagttttgctcctgcgattattcaactgacgggctttcatttccatttcacataccct agcaacacttataccttgcggaattgtattggtagcgtgaaaaagcacactgagagggc accatg (Seq ID No: 187)

Homo sapiens inter-alpha-trypsin inhibitor heavy chain 2 (ITIH2) :

ttttcttctttttcttctttcttaaagcgaactgtactcctctgctgttcctttgaact tggttcagtaggaagaagtgatatcctccccagaccatctgctttggggagcttggcaaa actgtccagcaaaatg (Seq ID No: 188)

Homo sapiens karyopherin (importin) beta 1 (KPNB1):

ccgccttcctccctccctcgctccctccctgcgcgccgcctctcactcacagcctccctt ccttctttctccctccgcctcccgagcaccagcgcgctctgagctgcccccagggtccct cccccgccgccagcagcccatttggagggaggaagtaagggaagaggagaggaaggggag ccggaccgactacccagacagagccggtgaatgggtttgtggtgacccccgcccccacc ccaccctcccttcccacccgacccccaacccccatccccagttcgagccgccgcccgaaa ggccgggccgtcgtcttaggaggagtcgccgccgccgccacctccgccatg (Seq ID No: 189)

Homo sapiens karyopherin alpha 3 (importin alpha 4) (KPNA3):

ctctcccctcctcccctcccgctccaagattcgccgccgccgccgccgcagccgcagg agtagccgccgccggagccgcgcgcagccatg (Seq ID No: 190)

Homo sapiens keratin 19 (KRT19):

gctcctcccgcgaatcgcagcttctgagaccagggttgctccgtccgtgctccgcctcgc catg (Seq ID No: 191)

Homo sapiens laminin, beta 1 (LAMB1) :

attcccttctttgggctcgggggctcccggagcagggcgagagctcgcgtcgccggaaag gaagacgggaagaaagggcaggcggctcggcgggcgtcttctccactcctctgccgcgtc cccgtggctgcagggagccggcatg (Seq ID No: 192)

Homo sapiens ribosomal protein SA (RPSA):

ctgtcttttccgtgctacctgcagaggggtccatacggcgttgttctggattcccgtcgt aacttaaaggaaattttcacaatg (Seq ID No: 193)

Homo sapiens lymphocyte cytosolic protein 1 (L-plastin) (LCP1) :

ttttctttcctggctgatgatttgtcattctagtcacttcctgccttgtgaccacacacc caggcttgacaaagctgttctgcagatcagaaagaaggggttcctggtcatacaccagta ctaccaaggacagctttttcctgcaagatctgttacctaaagcaataaaaaatg (Seq ID No: 194)

Homo sapiens lectin, galactoside-binding, soluble, 1 (LGALS1) :

ccatctctctcgggtggagtcttctgacagctggtgcgcctgcccgggaacatcctcctg
gactcaatcatg (Seq ID No: 195)

Homo sapiens SH2 domain containing 1A (SH2D1A) :

ttctctctttttgcacatctggctgaactgggagtcaggtggttgacttgtgcctggct
gcagtagcagcggcatctcccttgcacagttctcctcctcggcctgcccaagagtccacc
aggccatg (Seq ID No: 196)

Homo sapiens mannosidase, alpha, class 2A, member 1 (MAN2A1) :

tgttcctttcccctccgcttctctgacctagctgcgcggccccggcccgggagctgccga

acccgcgcctcccctgggtgaggaggacacgcctgccctcgtcgagaaaacttttcctgc
cgactcagttggggcggcggtggcaggaagtgcgggcagcgacctctcctccgcctgccc
cgcgcgccctgccggaggtcggcgctgagcttgcgatcaagtttgtgggggcccccccttc
ccagttgccggcgagtctcgcctcgagagggggcgcccgaccccggggagggcggcaggcc
agggcgaaggccaagggcgtgtggtggcgccggagactaggtgcggagcaaggcggggac
tcgcacccgcatccgagagcgcggaggtcgcgcagcccgggagaagggagcctccggcgg
ctgcttcctagagtccacagtgcgctgtctcctttggctgaggagagtgtcctggccccg
agtctatcgaggaaaatg (Seq ID No: 197)

Homo sapiens myelin basic protein (MBP):

ccgcctcttttcccgagatgccccggggagggaggacaacaccttcaaagacaggccctc
tgagtccgacgagctccagaccatccaagaagacagtgcagccacctccgagagcctgga
tgtgatg (Seq ID No: 198)

Homo sapiens melanocortin 1 receptor
(alpha melanocyte stimulating hormone receptor) (MC1R):

cattcttcccaggacctcagcgcagccctggcccaggaaggcaggagacagaggccagga
cggtccagaggtgtcgaaatgtcctggggacctgagcagcagccaccagggaagaggcag
ggagggagctgaggaccaggcttggttgtgagaatccctgagcccaggcggtagatgcca
ggaggtgtctggactggctgggccatgcctgggctgacctgtccagccagggagagggtg
tgagggcagatctgggggtgcccagatggaaggaggcaggcatgggggacacccaaggcc
ccctggcagcaccatgaactaagcaggacacctggagggggaagaactgtggggacctgga
ggcctccaacgactccttcctgcttcctggacaggactatg (Seq ID No: 199)

Homo sapiens malic enzyme 1, NADP(+)-dependent, cytosolic (ME1) :

gggcctttcccagtgcggccgccgccgccacagctgcagtcagcaccgtcaccccagcag
catccgccgcctgcaccgcgcgtgcggcccgccccggcctgaccccgccgccgaacccgg
cgccagccatg (Seq ID No: 200)

Homo sapiens myocyte enhancer factor 2C (MEF2C):

agctctctgctcgctctgctcgcagtcacagacacttgagcacacgcgtacacccagaca
tcttcgggctgctattggattgactttgaaggttctgtgtgggtcgccgtggctgcatgt
ttgaatcaggtggagaagcacttcaacgctggacgaagtaaagattattgttgttatttt
tttttctctctctctctcttaagaaaggaaaatatcccaaggactaatctgatcggg
tcttccttcatcaggaacgaatgcaggaatttgggaactgagctgtgcaagtgctgaaga
aggagatttgtttggaggaaacaggaaagagaaagaaaaggaaggaaaaaatacataatt
tcagggacgagagagagaagaaaaacggggactatg (Seq ID No: 201)

Homo sapiens mannosyl
(alpha-1,3-)-glycoprotein beta-1,2-N-acetylglucosaminyltrans ferase (MGAT1) :

agcccttcttggggaagtcagctacccagcagcctgtagtcctcggctacccaccctcac
cgcctggggtcccatggtgagacagctgggtgggcatcaggcttctgcagagggccaggc
cggagggagctgggcgagggagtggggctggctcctggcttgcaccggcctcgtggaatc
caggcctcagacctgatcgctggcgaaactggctctgtgcgctggagcccctggtcttct
gcgtctgtcctcctcccggccagactttactcctggctcagcgacaggtatttgctatgg
aagagctgtccctccctcccctcggtggcctgggtccacctccacctcctcttcaggtc
cgcaccttcctcccctttaaaacaccagccgggcgcagacccgttctaggcttttccatg

gtgcttccgccaaagcttgtgaccgagtccttcccgcctagggctggtgggcctcccctg
ctggtaggtctctcttcgctttctttactcagaactgaagctctcattccccacccacca
aggaaaaacaaaagggaagaagccacagctggccccggcttgctttggcacaggtgtttc
cccccggccccccgtcgggcaccctggttcctgttctgtccctgccccacgcgaccctgg
ggctcccacccgggctcctcagcctcccctgggttggggtgggggactggctcccagcc
cttggcctagggtttggtgaacgcctttcctggactgcgggcccacttcaggcgcggctc
caggctgggcagctgcgctggagggccgagggcaggggtggggtcgggcgtccaccctca
gggttgcgccagggagccggaaagccgactcccgaagttggggtcctgggaaaacttggg
tcctgggttgactgagaagcggcggggaaaggaggcgggccaggaggagggggcctggcg
gacgccggccgggggcggggcgcggcggggctgtcggtcacgcccctcagtccgccccg
ccccgccccgcctgccggggaagggccacgttgcccgcccggccgtccggccccggcgcg
ccgcagaaagggctggcgagtcgaaaggcgaggcggccgcggcagcgcttgggacgcgcc
tgggcaccgggctcgctccctgcgccccggagcaggccaagttcggggccaggacgtcgg
gaggacctggtgcatggctgcctcctaatcccatagtccagaggaggcatccctaggact
gcgggcaagggagccgggcaagcccagggcagccttgaaccgtcccctggcctgccctcc
ccggtgggggccaggatg (Seq ID No: 202)

Homo sapiens mitogen-activated protein kinase kinase kinase 11 (MAP3K11) :

ctgcctcccgcccccggggccaaagtacaaagggaggaggaagaagggagcggggtcgga
gccgtcggggccaaaggagacggggccaggaacaggcagtctcggcccaactgcggacgc
tccctccacccccctgcgcaaaaagacccaaccggagttgaggcgctgcccctgaaggccc
caccttacacttggcgggggccggagccaggctcccaggactgctccagaaccgagggaa
gctcgggtccctccaagctagccatggtgaggcgccggaggccccgggccccaccccccc
cggcctgaccacactgccctgggtgccctcctccagaagcccgagatgcgggggccggg
agacaacactcctggctccccagagaggcgtgggtctggggctgagggccagggcccgga
tgcccaggttccgggactagggccttggcagccagcggggtggggaccacgggcaccca
gagaaggtcctccacacatcccagcgccggctcccggccatg (Seq ID No: 203)

Homo sapiens membrane protein, palmitoylated 1, 55kDa (MPP1) :

ccgccttctccgcagccccgcaggccccgggccctgtcattcccagcgctgccctgtctt gcgttccagtgttccagcttctgcgagatg (Seq ID No: 204)

Homo sapiens v-myc myelocytomatosis viral oncogene homolog (avian) (MYC) :

ggccctttataatgcgagggtctggacggctgaggacccccgagctgtgctgctcgcggc cgccaccgccgggccccggccgtccctggctcccctcctgcctcgagaagggcagggctt ctcagaggcttggcgggaaaaagaacggagggagggatcgcgctgagtataaaagccggt tttcggggctttatctaactcgctgtagtaattccagcgagaggcagagggagcgagcgg gcggccggctagggtggaagagccgggcgagcagagctgcgctgcgggcgtcctgggaag ggagatccggagcgaataggggcttcgcctctggcccagccctcccgctgatcccccag ccagcggtccgcaacccttgccgcatccacgaaactttgcccatagcagcgggcgggcac tttgcactggaacttacaacacccgagcaaggacgcgactctcccgacgcggggaggcta ttctgcccatttggggacacttccccgccgctgccaggacccgcttctctgaaaggctct ccttgcagctgcttagacgctg (Seq ID No: 205)

Homo sapiens nuclear cap binding protein subunit 1, 80kDa (NCBP1): tggcctctcggttccgcggcgcaccggagggcagcatg (Seq ID No: 206)

Homo sapiens necdin homolog (mouse) (NDN) :

cttcctctccaggaatccgcggagggagcgcaggctcgaagagctcctggacgcagaggc cctgcccttgccagacggcgcagacatg (Seq ID No: 207)

Homo sapiens NADH dehydrogenase (ubiquinone) 1 beta subcomplex, 5, 16kDa (NDUFB5) : ccttcttcctcctgcccgtagtagccatg (Seq ID No: 208)

Homo sapiens NADH dehydrogenase
(ubiquinone) Fe-S protein 4, 18kDa
(NADH-coenzyme Q reductase) (NDUFS4) : ccgtcctttcatcctggcgtttgcctgcagcaagatg (Seq ID No: 209)

Homo sapiens nuclear factor of kappa light polypeptide gene enhancer in B-cells 2 (p49/p100) (NFKB2) :

tgccccttccccggccaagcccaactccggatctcgctctccaccggatctcacccgcca cacccggacaggcggctggaggaggcgggcgtctaaaattctgggaagcagaacctggcc ggagccactagacagagccgggcctagcccagagacatg (Seq ID No: 210)

Homo sapiens non-metastatic cells 2, protein (NM23B) expressed in (NME2) :

gcccctcctccgccgccggctcccgggtgtggtggtcgcaccagctctctgctctcccag cgcagcgccgccgcccggcccctccagcttcccggaccatg (Seq ID No: 211)

Homo sapiens nucleophosmin
(nucleolar phosphoprotein B23, numatrin) (NPM1) :

gcgtcctttccctggtgtgattccgtcctgcgcggttgttctctggagcagcgttctttt atctccgtccgccttctctcctacctaagtgcgtgccgccacccgatg
(Seq ID No: 212)

Homo sapiens 5'-nucleotidase, ecto (CD73) (NT5E) :

cattccttttgtagaaaaacccgtgcctcgaatgaggcgagactcagagaggacccaggc
gcggggcggacccctccaattccttcctcgcgcccccgaaagagcggcgcaccagcagcc
gaactgccggcgcccaggctccctggtccggccgggatgcggccggtacccgctccccgc
cgggaacaacctctccactcttcctgcagggagctggtgccagccgacagccgcgccagg
gccgctccgggtaccagggtcggatcgggtgacgtcgcgaacttgcgcctggccgccaag
ccggcctccaggctgaagaaggacccgccccggccttgacccgggccccgcccctccagc
cggggcaccgagccccggccctagctgctcgcccctactcgccggcactcgcccggctcg
cccgctttcgcacccagttcacgcgccacagctatg (Seq ID No: 213)

Homo sapiens phosphatidylethanolamine binding protein 1 (PEBP1):

gcgtcttcccgagccagtgtgctgagctctccgcgtcgcctctgtcgcccgcgcctggcc
taccgcggcactcccggctgcacgctctgcttggcctcgccatg
(Seq ID No: 214)

Homo sapiens poly(A) binding protein, cytoplasmic 1 (PABPC1) :

gcttccccttctccccggcggttagtgctgagagtgcggagtgtgtgctccgggctcgga
acacacatttattattaaaaaatccaaaaaaaatctaaaaaaatcttttaaaaaacccca
aaaaaatttacaaaaaatccgcgtctcccccgccggagacttttattttttttcttcctc
ttttataaaataacccggtgaagcagccgagaccgacccgcccgcccgcggccccgcagc
agctccaagaaggaaccaagagaccgaggccttcccgctgcccggacccgacaccgccac
cctcgctccccgccggcagccggcagccagcggcagtggatcgaccccgttctgcggccg
ttgagtagttttcaattccggttgattttgtccctctgcgcttgctccccgctcccctc
cccccggctccggcccccagccccggcactcgctctcctcctctcacggaaaggtcgcgg
cctgtggccctgcgggcagccgtgccgagatg (Seq ID No: 215)

Homo sapiens proprotein convertase subtilisin/kexin type 2 (PCSK2) :

cgctctttctctccggtacacacagctccccacattcgcacccctgcccgcgcgccgggc
cgcctgactgcacggcttcccctccagccagatgctggagaacacacactgattcgctgc
tttccaagaccctgttcagtctctttctctatacaaagatttttttaaaaactatatata
agaattctttatttgcaccctccctccgagtcccctgctccgccagcctgcgcgcctcct
agcaccacttttcactcccaaagaaggatg (Seq ID No: 216)

Homo sapiens phosphogluconate dehydrogenase (PGD):

gggtctttccctcactcgtcctccgcgcgtcgccgctcttcggttctgctctgtccgccg
ccatg (Seq ID No: 217)

Homo sapiens phosphoglucomutase 1 (PGM1) :

cgctcccctttcccctcccgccggacctgccaggaggtgggctggcgcggagggagggcc
ctgtcccctgtccctttaaggaggagggccaaacgccggcctagagtgcggcgtagcccc
cacccgccgtgccctcaccccagagcagctgcagcctcagccggccgcccctccgccagc
caagtccgccgctctgacccccggcagcaagtcgccaccatg (Seq ID No: 218)

Homo sapiens solute carrier family 25 (mitochondrial carrier; phosphate carrier), member 3 (SLC25A3) :

cggcctctgtgagccgcaacctttccaagggagtggttgtgtgatcgccatcttagggag
tgagtgtggccgggccttctcctgtggcgggtgtggggagcggagcccagagctcctgtg
gggccgctgctttggcggtgggcccagccgggagcagcctctttcgaaggccgccgtgac
ctcttcaagggcgtggagacgggaaggaaaaggccccggttggggttccagggcgccggt
aacgttaaccggcgccttgcctgtcctctaaccgtcgctccctcctcccctagaaagatg
(Seq ID No: 219)

Homo sapiens pim-1 oncogene (PIM1) :

cctcccctttactcctggctgcggggcgagccgggcgtctgctgcagcggccgcggtggc
tgaggaggcccgagaggagtcggtggcagcggcggcggcgggaccggcagcagcagcagc
agcagcagcagcagcaaccactagcctcctgccccgcggcgctgccgcacgagccccacg
agccgctcaccccgccgttctcagcgctgcccgaccccgctggcgcgccctcccgccgcc
agtcccggcagcgccctcagttgtcctccgactcgccctcggccttccgcgccagccgca
gccacagccgcaacgccacccgcagccacagccacagccacagccccaggcatagccttc
ggcacagccccggctccggctcctgcggcagctcctctgggcaccgtccctgcgccgaca
tcctggaggttgggatg (Seq ID No: 220)

Homo sapiens pyruvate kinase, muscle (PKM2) :

ggatctcttcgtctttgcagcgtagcccgagtcggtcagcgccggaggtgagcggtgcag
gaggctacgccatcagtccccaccaagggccagtcgcccggctagtgcggaatcccggcg
cgccggccggccccgggcacgcaggcagggcggcgcaggatccagggcgtctgggatgca
gtggagctcagagagaggagaacggctcctcacgcctggggcctgctcttcagaagtccc
cagcgccgttccttccagatcaggacctcagcagccatg (Seq ID No: 221)

Homo sapiens pleiomorphic adenoma gene-like 1 (PLAGL1) :

cggcctcctcggcgcagccatcctcttggctgccgcgggcggcaaagcccacggcatctg
ccatttgtcattcagcccgtcggtaccgccccgagccttgatttagacacggctggggcg
tgctctggcctcactctccgggcgggtgctggacggacggacggacggggcagccgtgct
cacagctcagcagcgcggggccttggcgcgcggggcgcttccccgggtcgccgtcatggc
cgcggaggtggcacgcccgagcggcctcgcctgagctccgggggtcgtcgccccgcaggg
attgctgtcacgtctaatgtggctgctgcctcgtgtcacatctgaaactcatctgtacct
cacttagaaagtggttctgattagacaagacttttcgttgcagtcgacagaaacctaatg
ggaccattgaagaattccaaacaggtatttgcataggaatcagaggagttaatcttgtct
cttctcacaggtttgaatcttcagacaaacttctgggaggactcggtccctgcctcgcag
cagatgttccctgtcactcagtaggcatatg (Seq ID No: 222)

Homo sapiens phospholipase D2 (PLD2) : tgctctcttggctccggaacccccgcgggcgctggctccgtctgccagggatg (Seq ID No: 223)

Homo sapiens proteolipid protein 2 (colonic epithelium-enriched) (PLP2) :

ccccttcccggccagacggcgggcaagacagctgggtgtacagcgtcctcgaaaccacg
agcaagtgagcagatcctccgaggcaccagggactccagcccatgccatg
(Seq ID No: 224)

Homo sapiens pinin, desmosome associated protein (PNN) :

cagtcctttcgcgcctcggcggcgcggcatagcccggctcggcctgtaaagcagtctcaa
gcctgccgcagggagaagatg (Seq ID No: 225)

Homo sapiens phosphoribosyl pyrophosphate amidotransferase (PPAT): ggtccttccacgtgctttcggcggcgacatg (Seq ID No: 226)

Homo sapiens protein phosphatase 1, catalytic subunit, gamma isozyme (PPP1CC) :

tgttcttctcgtggttccagtggggagagaaggaggaagtagggagcggggtggcagggg
ggggacccgccgcggctgctgccaccgccgccaccaccgcctctgctcgtggcgtgggaa
aggaggtgtgagtcccgggcgcgagccggcggcggcgccgctgcgggagggtcggcggtg
ggaaggcgatg (Seq ID No: 227)

Homo sapiens protein phosphatase 1, regulatory subunit 8 (PPP1R8) :

cggtcttccagtttcccggcgtgcttagggcgcgccaaatgggaggggggagacgcaagat
g (Seq ID No: 228)

Homo sapiens protein phosphatase 6, catalytic subunit (PPP6C) :

cggcctccgccgctgccgccgccgctgctacagccgccgccgccgctgttgccgcggctt
gttattcttaaaatg (Seq ID No: 229)

Homo sapiens protein kinase C substrate 80K-H (PRKCSH) :

ctttctttctgcagcaggaaccgcggctgctggacaagaggggtgcggtggatactgacc
tttgctccggcctcgtcgtgaagacacagcgcatctccccgctgtaggcttcctcccaca
gaacccgtttcgggcctcagagcgtctggtgagatg (Seq ID No: 230)

Homo sapiens mitogen-activated protein kinase 6 (MAPK6) :

cgccccctcttcctcgccctctctcgcgggtcggggttacatggcggcgactgcggcaaa
gcgagagcctcggagacgccgctgccgccagcacagccggagacctgagccgacactggg
ggcagtccgcgagccccgcactctctcgatgagtcggagaagtcccgttgtatcagagta
agatggacggtagctttgattgtgattgtggtgagctggagccacctgatcactaacaaa
agacatcttctgttaaccaacagccgccagggcttcctgttgaaataaatatatagcaac
aaaggaaaaaaagaagcaaaacggaaatagtgcttaccagcaccttagaatgatgctgct
caggaccagtccaacactgaatgtatctgcactgtgaggagaatgttcatagaagcctgt
tgtgtgcatatttattcacattttgttaaatgttaaatcgtttagcacggtaatctgag
tgcacagtatgtcatttcattccgtttgagtttcttgttttcgttaaatgtctgcagagt
tgctgccccttttcttgaactatgagtactgcaatctttttaattctcaatatgaatagag
ctttttgagctttaaatctaaggggaactcgacaggcctgtttggcatatgcaatgaaca
tcaagaaaccatcttgctgtggaagcataattatttttcttctcccttttttgaaagatct
ttcctttttgatgccagtttcttccttgtttacacaagttcaatttgaaaggaaaaggca
atagtaagggtttcaaaatg (Seq ID No: 231)

Homo sapiens phosphoribosyl pyrophosphate synthetase 2 (PRPS2) :

cctccccttccctacatctagccgccgcgctttcccgctcccgcagcagcagcctcccgc
gtcgctgtcgctgttgcctccgccacctcctccgccgccgcgcgcccctcggagttccgc
gccccaccatg (Seq ID No: 232)

Homo sapiens phosphoribosyl pyrophosphate synthetase-associa ted protein 1 (PRPSAP1):

ttgcctctggctctgaggcggcggcgccgggcgctgcgaaggctcggccgctgtagtcag
tggtgtggggtgcgcaagggcacggacctcggagctctccccgcttgcgccgagtttctc
agcgccttccccacccaaaccggggtctcgcagtcggaagcactcagagtgcagccccgc
gcggggccggtcgtaaccgcgccgcgggccggacgatg (Seq ID No: 233)

Homo sapiens proteasome (prosome, macropain) subunit, beta type, 5 (PSMB5): agttctttctgcccacactagacatg (Seq ID No: 234)

Homo sapiens proteasome (prosome, macropain) 26S subunit, non-ATPase, 13 (PSMD13): tgttcttctgtgccgggggtcttcctgctgtcatg (Seq ID No: 235)

Homo sapiens protein tyrosine phosphatase, receptor type, N (PTPRN) :

cagcccctctggcaggctcccgccagcgtcgctgcggctccggcccgggagcgagcgccc
ggagctcggaaagatg (Seq ID No: 236)

Homo sapiens RAB3A, member RAS oncogene family (RAB3A) :

ctccctttgcaggacgtcacggaggactgcaggggcctgagccgctgctgccgccgccgc
cgcgcagccccacatcaacgcaccggggtcctgtcaccgccaccgccaaaaaagtcaccg
ccgctagggtcgccgttgcatcggtgcagggcaagatg (Seq ID No: 237)

Homo sapiens RNA binding motif, single stranded interacting protein 2 (RBMS2) : ctctctctctctctctcgctcgttccctaa-cattaaagagaaaatg (Seq ID No: 238)

Homo sapiens reticulocalbin 1, EF-hand calcium binding domai n (RCN1) :

gcgcccctctgctccggctcggggcgggcactggcggagggactggccagtcccctcctc
cgcgccggccccaaccctgtcgctgccgccgcgctccgagtccccattcccgagctgccg
ctgttgtcgctcgctcagcgtctccctctcggccgccctctcctcgggacgatg
(Seq ID No: 239)

Homo sapiens radixin (RDX) :

ccgccttttcccgcggaggcgccgagcggccatattgcggagctgtctgcggtggcggcg
gcgcctctcgtctcccgcggcccagcgctcgcaccaccgcttctccctccctgtcgcagc
cgcgccgccgcgcagcgccccagccacacgccggcgggcagaagccgcccgctctccgga
aagtgataacagaattcattgaagtggagaattttaaagaaggtaacaaaaagagaaag
aaaatg (Seq ID No: 240)

Homo sapiens replication factor C (activator 1) 1, 145kDa (RFC1) : tcgccttcttgcacttcgcgggagaagttgttggcgcgaatggatcctgagcctcgataa cagattcctcaaccggcccacccgccagccagccagcgccttcatcctggggctgcgatg (Seq ID No: 241)

Homo sapiens ring finger protein 4 (RNF4) :

gcatctttctcgaggagctctcctgggcggctgaagaaggagcttcttctccggagtgcg
ccggcggtggcgcctgcggacctaactagctccaggttaggccgagctttgcgggaaagc
agcggacttgaaaatactggaaatctgtccggatccaaattattttgcaagccagatgag
taaccagagggcatgaaaggttgagaacatttgacttccctgcaaaccttggtatagatc
acttccttttctgtaggaaaggaaaggcaccaaagagcacaatg
(Seq ID No: 242)

Homo sapiens ribophorin I (RPN1): tgctcttcccggtcatg (Seq ID No: 243)

Homo sapiens ribosomal protein S27a (RPS27A) : cgttcttcctttcgatccgccatctgcggtggagccgccaccaaaatg (Seq ID No: 244)

Homo sapiens secreted and transmembrane 1 (SECTM1) :

cttcctttagcgtgaaccgcgggtgcggtgcctcccgtgaaaataataaattcaccgtca
cgcttgttgtgaacgcgggtggttcccgaaacttggaggcttcccgtaaacccagctcct
tcctcatctgggaggtgggtcccgcgcgggtccgccgcctcctccctggcccctccctct
cgtgtctttcattttcctggggctccggggcgcggagaagctgcatcccagaggagcgcg

tccaggagcggacccgggagtgtttcaagagccagtgacaaggaccaggggcccaagtcc
caccagccatg (Seq ID No: 245)

Homo sapiens small glutamine-rich tetratricopeptide repeat (TPR)-containing, alpha (SGTA) :

ctttcttttgcgcaggcgtcgcgccctggggccggggccgggcggcaccgcggtgcgcaa
gcgcaaccgtcggtgggtcggggatcggtcgcctgagaggtatcacctcttctgggctca
agatg (Seq ID No: 246)

Homo sapiens SH3 domain binding glutamic acid-rich protein 1 ike (SH3BGRL) :

agttctccttccaccttcccccacccttctctgccaaccgctgtttcagcccctagctgg
attccagccattgctgcagctgctccacagcccttttcaggacccaaacaaccgcagccg
ctgttcccaggatg (Seq ID No: 247)

Homo sapiens solute carrier family 1
(glutamate/neutral amino acid transporter), member 4 (SLC1A4) :

cgccctcctacttccccgtctgcgtccgcgttcgcggctcccgtttgcatcatccccgtc
tgcgtccgcgttcgcggctcccgtttgcatcatctccagccggcggctgctccagggagg
ctgggcgcgatcctctccgcccgcggctccaacccgcactctgcgcctctcctcgccttt
ctcgcacctgctcctgcgccaggcccggagacccccggggcggcttcccagaacctgcgg
agcacaactggccgaccgacccattcattgggaaccccgtcttttgccagagcccacgtc
ccctgccacctctagctcggagcggcgtgtagcgccatg (Seq ID No: 248)

Homo sapiens small nuclear RNA activating complex, polypepti de 2, 45kDa (SNAPC2): ctgcctctttctgagcggcatg (Seq ID No: 249)

Homo sapiens sorting nexin 1 (SNX1) : ctatctctcgataaagttgttgttgcggcttccgccgcgggtggaagaagatg (Seq ID No: 250)

Homo sapiens signal recognition particle 54kDa (SRP54) :

ctatctctcatctttccgctcttagctgggagtgctccgcctagtcacttttcttaaggt
ggctcgtcgaggcctgacttcttccccgaaatcacgtccctagacagcctcctattttac
cactaactttactcctgcagttattcagcggtaggaaactgaaaccaaaaaccagtgtaa
gcaagtaaacatctaaactgtttcaggagccgcgtagaaggaacgcggcggtgtgccccg
gaagcggaagtagattctcctatagaaaggctggactacgcggagtggtgacgtttcctc
attgggcggaaggttcgctggcactccgttggtcttccagctggtgggagttgacgacgt
ggtgctgggcgttgggaccctactttatctagttcgggaagttgggttgtggggtcatac
ctgtctgtctgctcccagctttcttgggtttcttccgacggcgtggggcctcgctaagga
attcccggcccctcagggccacggctttagcggtgtcttttgcgagttcttcgtaagtac
atcttaaagctgtcaagatg (Seq ID No: 251)

Homo sapiens signal sequence receptor, beta
(translocon-associated protein beta) (SSR2) :

cggtctttcggatgctgacgctctcttcctgtctttgtggctccggaaaggcgtttggga
tgccaacgatg (Seq ID No: 252)

Homo sapiens signal transducer and activator of transcriptio n 6, interleukin-4 induced (STAT6) :

ttttctttttggtggtggtggtggaagggggggaggtgctagcagggccagccttgaactc
gctggacagagctacagacctatggggcctggaagtgcccgctgagaaagggagaagaca
gcagaggggttgccgaggcaacctccaagtcccagatcatg (Seq ID No: 253)

Homo sapiens suppressor of Ty 4 homolog 1 (S. cerevisiae) (SUPT4H1): tgttcttcccatcggcgaagatg (Seq ID No: 254)

Homo sapiens transcription factor 7 (T-cell specific, HMG-box) (TCF7) :

ggtccttcccctaaaacttggcactgccgatactcccagcccgttccttcccaagtcagg aacttgcaggggaccccttggcaattctttttctctcaagagcagacagccttcagtccc agccgctgccagggctggtgtgtctgacccagctgtggtttttccaggcctgaaggcccc ggagtgcaccagcggcatg (Seq ID No: 255)

Homo sapiens TEA domain family member 4 (TEAD4):

cagtctcctccccgaggtgccggtggccccgccgccactccctccggctccctccctccc gccgcggcgcgcatctcattccagccctcattccgcgcattccagcgtcctcctcgcaca ctcgaggccaggggcgggagggccgcagctccggcgccgccgcgtcccgccaggtgaga ggcgcccgcgcccgccgcacccgccggcgccctcacggccgcgcgccccacgccgccgc agccgaccgctcgcgccgcgtgctcggctgctcttttctttccgccgcccgcgttcccgc cttggacctctgcgctccgacgcgctccgtcccgacctctggcttccctccgcgctccgg cgctgctcgctgcccctctcccgcttccctcctgtccgccccgcgctcccctcctcgctc ccggttgactcactcctccaggaatagggatccccgtgttttcccgtcagtcccattctg ggaaaactcctccctccgcgcgctccgctccgctccgctgggcgcaccggggccggtcgg cgcggggtgggcttggccccgcggccccgccttcactgcgccgcccgtcggccccggccg gagcccggctctgcgcgctgacgccctgtcgtccccgcagaacgatcgccgcggccggaa gagttggcgctcggggcggactccttggaactggcttagcgcacccatcccaccttcccg caccctgggaccggtcggaacgagctgattgcccgctacatcaagctccggacagggaag acccgcaccaggaagcaggtctccagccacatccaggtgctggctcgtcgcaaagctcgc gagatccaggccaagctaaaggaccaggcagctaaggacaaggccctgcagagcatg (Seq ID No: 256)

Homo sapiens G protein-coupled receptor 137B (GPR137B) :

ttttctttcctccagtctcggggctgcaggctgagcgcgatgcgcggagacccccgcggg ggcggcggcggccgtgagccccgatg (Seq ID No: 257)

Homo sapiens tumor protein, translationally-controlled 1 (TPT1) :

cggccttttccgcccgctccccccctccccccgagcgccgctccggctgcaccgcgctcgc tccgagtttcaggctcgtgctaagctagcgccgtcgtcgtctcccttcagtcgccatcat g (Seq ID No: 258)

Homo sapiens ubiquitin A-52 residue ribosomal protein fusion product 1 (UBA52) : ctatcttctttttcttcagcgaggcggccgagctgacgcaaacatg (Seq ID No: 259)

Homo sapiens ubiquinol-cytochrome c reductase core protein I I (UQCRC2) :

cggcctccgccaccatcttgctttcctttaatccggcagtgaccgtgtgtcagaacaatc ttgaatcatg (Seq ID No: 260)

Homo sapiens ubiquitin specific peptidase 1 (USP1) :

ctgcctttcgtgtctctgcagcgtggagactggaaccggcaatttcaaaggacgccacgt
tcaatcgcagcgctggcgcgggcggaggctaaaacacgggggtcctgagactgaggaaaa
cgcgccaagttcccctcggtggcggagtgctaaagaccctagcggttcaggcgttcggcg
agcggggccgctgcttgttgcgctcctggctctcccggggcgggcgcagatgggcgccgc
tcccgggatgtagttggtgttggtgcaagacgggagcgagcggcggtcggggttcccgct
cttgggagcggatggtcactcccccgcggggagggcgagccgaccagattttcctggggc
cggggacccggcgggctcggggcagggactcacctgtcgcacccacactcattcgggttg
gacttgccggcgtcaccgccgcggacttcgctttgggccatgaccagatataattggtga
ttacaactttcctctataaattaactcttgacactccttgggatttgaagaaaaaaatg
(Seq ID No: 261)

Homo sapiens voltage-dependent anion channel 2 (VDAC2) :

gtgtctccttcacttcgccctccagctgctggagctgcagcccgaccgcgagcgtgccaa
gcggcttcagcagctagcggagcggtggcggcggccccctcaggacaccaccagattcc
cctcttcccgcggcctcgccatg (Seq ID No: 262)

Homo sapiens vimentin (VIM):

gcctcttctccgggagccagtccgcgccaccgccgccgcccaggccatcgccaccctccg
cagccatg (Seq ID No: 263)

Homo sapiens very low density lipoprotein receptor (VLDLR):

cccccctccccgctgctcaccccgctctccggccgccgccggtgcgggtgctccgctaccg
gctcctctccgttctgtgctctcttctgctctcggctccccacccccctctcccttccctc
ctctccccttgcctcccctcctctgcagcgcctgcattattttctgcccgcaggctcggc
ttgcactgctgctgcagcccggggaggtggctgggtgggtggggaggagactgtgcaagt
tgtaggggagggggtgccctcttcttccccgctcccttcccccgccaactccttcccctc
cttctccccctttcccctccccgcccccaccttcttcctcctttcggaaggactggtaac
ttgtcgtgcggagcgaacggcggcggcggcggcggcggcggcaccatccaggcgggcacc
atg (Seq ID No: 264)

Homo sapiens wingless-type MMTV integration site family, mem ber 10B (WNT10B) :

agtcctttgctcgccggcttgctagctctctcgatcactccctcccttcctccctccctt
cctcccggcggccgcggcggcgctggggaagcggtgaagaggagtggcccggccctggaa
gaatgcggctctgacaaggggacagaacccagcgcagtctccccacggtttaagcagcac
tagtgaagcccaggcaacccaaccgtgcctgtctcggaccccgcacccaaaccactggag
gtcctgatcgatctgcccaccggagcctccgggcttcgacatg (Seq ID No: 265)

Homo sapiens CCHC-type zinc finger, nucleic acid binding pro tein (CNBP) :

cagcctctaccttgcgagccgtcttccccaggcctgcgtccgagtctccgccgctgcggg
cccgctccgacgcggaagatctgactgcagccatg (Seq ID No: 266)

Homo sapiens zinc finger protein 43 (ZNF43) :

gggcctttgtctctggctgcagttggagctctgcgtctcgtcttcgttcttctgtgtcct ctgctgctagaggtccagcctctgtggctctgtgacctgcgggtattgggggatccacag ctaagacgccaggacccccggaagcctagaaatg (Seq ID No: 267)

Homo sapiens zinc finger protein 74 (ZNF74) :

cagtcctttgtgggagtccggtctgtccacttgccggtccctcagaccgtcggcggtct ctgtccgcttcgggacctgtccgctggtcgctccgcgtccgatggctcctggccgcggaa ccttaggcctggccctggtctccgagcgcgggttcgccggaggagcgtgtggcggggt gtgccggggcgtgagtgcgccgagcatggggctgagcctggtgtggggagtgggtatctg cggagccggcctgaaccccacctcagccgggcgcggggaggggctccgtgcgtgtgatc gtgcagctgtgagcgcgtggccgccccgcggggctccgctgcaggcccctcagccccagg agcagtactcgctcttcagggcctgccctggatcctggaggctacacagctgcccactcc tcctggggaggctgccgtggaggccatg (Seq ID No: 268)

Homo sapiens zinc finger protein 85 (ZNF85) :

gggcctttgtctctcgctgcagcctgagctctaggtcttgttttccctgctttgtgtttt ctgctcgtggacgcccagcctctgtggccctgtggcctgcaggtattgggagatccacag ctaagacgccgggacccctggaagcctagaaatg (Seq ID No: 269)

Homo sapiens zinc finger protein 91 (ZNF91) :

gggcctttgtctctcgctgccgccggagtttccaggtctcgacttcactgctctgtgtcc tctgctccaggaggcccagcctgtgtggccctgtgacctgcaggtattggagagccacag ctaagatg (Seq ID No: 270)

Homo sapiens zinc finger protein 141 (ZNF141):

gggtctttgcgtctggctactaccagaccgcgggttaggggcttcatctctctgcgttct cagttgtgggaggccttggtgattcggccacagcctcagcctccgtcgctctgtgacctg cgggtattggatgattggtagctaagactcccgaatacttcagaagtggggaaatg (Seq ID No: 271)

Homo sapiens zinc finger protein 205 (ZNF205): tgttctttctagctctgaaatagaaaatg (Seq ID No: 272)

Homo sapiens transmembrane protein 187 (TMEM187) :

ctccctttcggagatttgaatttcccccagcgaggcgagtgaggcgaaatacccgtatg gtgatagctggcctttcgcgccaatactgaaaaaggcagaacgttcctccgctggcgcc agccaatcagcaggactcctgccttccttcggggcaaggtcgcagcatctgcctcggaaa tcacgaaatcacggggcttctttctgctggctcagccgggaggcccagagtgttctgcag aggctgcgtattgaaggctgctctctgaagctccctgccccaggtcacgccgccggttcc agatg (Seq ID No: 273)

Homo sapiens histone cluster 2, H2be (HIST2H2BE) : acttcttttcttggctaagccgcgtttgtactgtgtcttaccatg (Seq ID No: 274)

Homo sapiens solute carrier family 25
(mitochondrial carrier; oxoglutarate carrier), member 11 (SLC25A11) :

ccgcctttgcgctgcgcgcctgcgcccgcgccggcttccagcgggtgtcggacctgagag

ctggaggggcgtgcgcgcgccctcgctctgttgcgcgcgcggtgtcaccttgggcgcgag
cggggccgcgcgcgcacgggacccggagccgagggccattgagtggcgatg
(Seq ID No: 275)

Homo sapiens tyrosylprotein sulfotransferase 2 (TPST2) :

cctccccttccccggctggggcggctggagagccgggagtcgctgggtgcgtggggctg
cctcgccgcgtctcgccacgggctctgccagcagacagccttggcacacaggcacaaggg
ctggagcccagagatgagagtgcccaagggagatgtgagcctggcgggctgcccgctaac
ctgtcgctgaagccccagaagcgggccctcaggccaggcctaccctgcctccggcccagc
atg (Seq ID No: 276)

Homo sapiens sorbin and SH3 domain containing 2 (SORBS2) :

aagcctcttttatacatctcttcagggaagagagaagcaatgggcatgttagtatacaat
gatcacagccacgcaggcctgcaagctgccttttggacaggctgttgactgccgttccaa
ttagctgattggagaatgtggaatgcagagtgataatgctgcatatctgctatcaggcag
cagcaaaggtttttgtcttgggaaggcaagctttccctgcaatattatctcagcagctcc
ctagctgcttaccctgaaaacgagggatccaaacggagggtgttgcactctgctaacgct
ggtcctgtgcgtggctgtggcatatgagcggcaggtctgaaaaagcaggtgtgtgctggg
acgggcactggactggaacgcaggcggacgctctcgggtttacctgcttcctgttaacag
attgtgggctcccagggcatatgtctgcacgctgaggccgaggcggagaaggggcttcct
gagcgtcccagtacactgacagagacacttggattggacttaatcttaaacctctggagt
tcaagaccttttaaaaagggctaaataaacaatctctacatgtaaaaggccactgactcc
tacttcctctgtatagagcaactgttgaactcagctgcctgtaggaaaactgaagacttt
aataacaaactctccaaggtgaaaatg (Seq ID No: 277)

Homo sapiens G protein-coupled receptor 65 (GPR65):

gtttctcttcttgacttgatgcaggcacagatttatcaagctcctcagtcaacaaacaca
tcaccggaagaaatatggaaggaaaggaattttaaaaggaaataccaatctctgtgcaaa
caaagccttgtatattcatgtttgcaccaatctactgtgagatttatgaagaaaaacaaa
ttgcggacaactctctatgtacacttacaaatgcctcagttgatgcttgtgggctgtttg
tcagcgttctgtgataatgaacacatggacttctgtttattaaattcagttgacccctttt
agccaattgccaggagcctggattttacttccaactgctgatatctgtgtaaaaattga
tctacatccaccctttaaaagcattgatgaattaattagaactttagacaacaaagaaaa
attgaaaaagaattctcagtaaaagcgaattcgatgttcaaaacaaactacaaagagaca
agacttctctgtttactttctaagaactaatataattgctaccttaaaaaggaaaaaatg
(Seq ID No: 278)

Homo sapiens nipsnap homolog 1 (C. elegans) (NIPSNAP1): gggccttcctgcaacctttgcggctccaacatg (Seq ID No: 279)

Homo sapiens inhibitor of kappa light polypeptide gene enhan

cer in B-cells, kinase complex-associated protein (IKBKAP): gcttctttgcagcgcttcagcgtttcccctggagggcgcctccatccttggaggcctag tgccgtcggagagagagcgggagccgcggacagagacgcgtgcgcaattcggagccgact ctgggtgcggactgtgggagctgactctgggtagccggctgcgcgtggctggggaggcga ggccggacgcacctctgtttgggggtcctcagagattaatgattcatcaaggatagttg tacttgtctcgtgggaatcacttcatcatg (Seq ID No: 280)

Homo sapiens COP9 constitutive photomorphogenic homolog subu nit 3 (Arabidopsis) (COPS3) : ctgccttcgccgctcgggccgcccgggggaaaacatg (Seq ID No: 281)

Homo sapiens pirin (iron-binding nuclear protein) (PIR) :

ccgcctcctctaggccgccggccgcgaagcgctgagtcacggtgaggctactggacccac actctcttaacctgccctccctgcactcgctcccggcggctcttcgcgtcacccccgccg ctaaggctccaggtgccgctaccgcagcgtgagtacctggggctcctgcaggggtccact agccctccatcctctacagctcagcatcagaacactctctttttagactccgatatg (Seq ID No: 282)

Homo sapiens THO complex 5 (THOC5) :

ccttccttacttccggttctctatggtgcgcgggcaagctttgctccgcctccggcagtg gcttactcccggtgccaggttcttggagctgtgaggaggaacaaccatg (Seq ID No: 283)

Homo sapiens RuvB-like 1 (E. coli) (RUVBL1) :

gggcctttgcaaaattgccctagtaacggccgcatggtaactcaggcgccgggcgcactg tcctagctgctggttttccacgctggttttagctcccggcgtctgcaaaatg (Seq ID No: 284)

Homo sapiens Kruppel-like factor 7 (ubiquitous) (KLF7) :

tttcctttttagttgactgaaacaaaacaaaacaaaagggccactggatgtctgccttct tggggggtgagccagacagactgacaaacaaacagccccaactgtgttcggggagggtt tcgcctcccgttttgcccggcagcagcagcatg (Seq ID No: 285)

Homo sapiens USO1 vesicle docking protein homolog (yeast) (USO1) :

gctccccttttgccttcaaccttcgagccgccacgtaatgccacgtccccgcgcatgcgc atcttggccgctgctggcggctgtttccgggcttagagggctggagtggccgccgagttg gaggcggtggtggcagcagtaggagtgtgtagagtgcgggattgggggccaggccctgcg gagggcgggggaagttgtcttcttttttttccggaggggccggtaaacctggtggctgaa cggcaagatg (Seq ID No: 286)

Homo sapiens unc-5 homolog C (C. elegans) (UNC5C) :

ccccctttggcccctgcctttggagaaagtggagtgtggcgcttggttgtcgttatttc ttcggactgcttcgcggtgcacggattcagcttctgcccagtggggctttcagctgtttg cgcgtctctctgtcccctcccctccccccggcacacctctgtctacgatg (Seq ID No: 287)

Homo sapiens RNA terminal phosphate cyclase domain 1 (RTCD1) :

gcttcttccgctttctcgtcaggctcctgcgccccaggcatgaaccaaggtttctgaact actgggcgggagccaacgtctcttctttctcccgctctggcggaggctttgtcgctgcgg gctgggccccagggtgtcccccatg (Seq ID No: 288)

Homo sapiens eukaryotic translation initiation factor 3, sub unit A (EIF3A) :

ggctccttcctttccgtctctggccggctgggcgcgggcgactgctggcgaggcgcgtgg

gaccttacgctggttccccttcgtctcctctcccggcccgggccactagagagttcgctg acgccgggtgagctgagcctgccgccaagatg (Seq ID No: 289)

Homo sapiens eukaryotic translation initiation factor 3, sub unit D (EIF3D) :

gtttcctcttttcctggtttctcaagagtgctgctgctaacgcggtccccggcacgcacc atctgttgccatcccggccggccgaggccattgcagattttggaagatg (Seq ID No: 290)

Homo sapiens eukaryotic translation initiation factor 3, sub unit F (EIF3F): ccgcctccttctttctcgacaagatg (Seq ID No: 291)

Homo sapiens eukaryotic translation initiation factor 3, sub unit G (EIF3G) : cgctctctggccgggcttgggctgcgtggagaatactttttgcgatg (Seq ID No: 292)

Homo sapiens eukaryotic translation initiation factor 3, sub unit H (EIF3H): gtttctctttcttcctgtctgcttggaaagatg (Seq ID No: 293)

Homo sapiens eukaryotic translation initiation factor 3, sub unit I (EIF3I) : aaaccttttccggtcttactcacgttgcggccttcctcgcgtcacagccgggatg (Seq ID No: 294)

Homo sapiens eukaryotic translation initiation factor 3, sub unit J (EIF3J): ctccctctcacacacgctcacacccggctcgagatg (Seq ID No: 295)

Homo sapiens poly(A) binding protein, cytoplasmic 4 (inducible form) (PABPC4) :

ccgcctctctccgccccgggtcgctgccgcctccgccgctttcgggcttcgcagcctgag
gaaaaaaagagaaaaagataaaaaaaatctgaaaacgcttcaaaatcctgaaaaaaaaa
aggaaaagaaaaaacgaatcctcggagaacccgcggggaagtcactttcgtacgcttccg
gcctgccccgcgcccgccgccgcagcgcttggcgtccgtcggtctccgtccgtcggtccg
ggggtgagccgcccgcccggcccgccgtgccctccccccgctcgggccccgagccccgcg
ccccgcgcctgccccggcgcaccacgtgtccgtgctgcccttcgccgcccgcccggggct
cgccgagtcggcgcccacaaagatttggtttccctctgccccggcggttgtaatcttaaa
ccgccggagcccgaggcctatatttatagagaaacgcgtgtccccgaggccgccgtgggc
agcgtccggtcgcctcttaaaggattttacccttcggaaggggattccccgtttaattt
ttttcctactttgatttttgaaatttggagcttcgcaccaggaccgcggagaagtgcaa
agtcgcggggagggccgtattgtgcggagagccttttgtctgcggtgctgcggccgtggg
agccggcccccgcctcccgtttccgtcccgtctccaagcccgccgactccagctcgtcct
cgccgcgccggtgccacctgtgagccgcggcgcgggcccgggctccgaaggcgcccctttt
gtcctgcggcgggcccgataagaagtcctcctggcggggctcggggtggtgggggcggg
gagatg (Seq ID No: 296)

Homo sapiens receptor-interacting serine-threonine kinase 2 (RIPK2) :

agctctttcgcggcgctacggcgttggcaccagtctctagaaaagaagtcagctctggtt
cggagaagcagcggctggcgtgggccatccggggaatgggcgccctcgtgacctagtgtt
gcggggcaaaaagggtcttgccggcctcgctcgtgcaggggcgtatctgggcgcctgagc
gcggcgtgggagccttgggagccgccgcagcaggggggcacacccggaaccggcctgagcg
cccgggaccatg (Seq ID No: 297)

Homo sapiens neuropilin 1 (NRP1) :

ctttcttttctccaagacgggctgaggattgtacagctctaggcggagttggggctcttc
ggatcgcttagattctcctctttgctgcatttccccccacgtcctcgttctcccgcgtct
gcctgcggacccggagaagggagaatg (Seq ID No: 298)

Homo sapiens guanine monphosphate synthetase (GMPS) :

tggtcttctctcccgcggcgctggggcccgcgctccgctgctgttgctccattcggcgct
tttctggcggctggctcctctccgctgccggctgctcctcgaccaggcctccttctcaac
ctcagcccgcggcgccgacccttccggcaccctcccgccccgtctcgtactgtcgccgtc
accgccgcggctccggccctggccccgatg (Seq ID No: 299)

Homo sapiens far upstream element (FUSE) binding protein 1 (FUBP1) :

ttttctttctttcttagctgttagctgagaggaagtctctgaacaggcggcagcggctct
tatagtgcaaccatg (Seq ID No: 300)

Homo sapiens eukaryotic translation initiation factor 2B, su bunit 5 epsilon, 82kDa (EIF2B5):

gatccttttttgtcccctactgcgtgcggtggcagcttccttgcggaagtggtgaccgtga
gagaagaagatg (Seq ID No: 301)

Homo sapiens eukaryotic translation initiation factor 2, sub unit 2 beta, 38kDa (EIF2S2) :

gtttcctttcgctgatgcaagagcctagtgcggtggtgggagaggtatcggcaggggcag
cgctgccgccggggcctggggctgacccgtctgacttcccgtccgtgccgagcccactcg
agccgcagccatg (Seq ID No: 302)

Homo sapiens adaptor-related protein complex 1, sigma 2 subu nit (AP1S2) :

cctcccctctccgcctaagcctgccctatgccagccgggtgtcctccccacagcaccacg
gcttctcttcctcagcacggcgacaggggcttccccttcgccgccgccgccgccgccggc
caagctccgccgcgcccgcggcccgcggccgccatg (Seq ID No: 303)

Homo sapiens suppression of tumorigenicity 13 (colon carcinoma) (Hsp70 interacting protein) (ST13) :

cgccccccttctgcgcggtcacgccgagccagcgcctgggcctggaaccgggccgtagccc
ccccagtttcgcccaccacctccctaccatg (Seq ID No: 304)

Homo sapiens solute carrier family 7 (cationic amino acid transporter, y+ system), member 7 (SLC7A7) :

ctccctttcttaaatgcttggggtgagagagaagagaggctagggtggggcatggaggac
acagagagagagagtgctgtgtattccttccccgctactgtcctgtcctcagctaacttg
ctctgggacagcttccccagggctacagatactgcactcagctgactgtcctttcttctg

ggcccctggtcccagagcagagctgacaaaggagattcctgagagagcaccttcttatca
cagaaagtgctgagccaagagctcctagctgccccttttgcagatgtgaagggccagtga
accttggacccagatggttgcttaatactcctttccccctccctcactccttcctttgcg
ggctgcctcacctcctccacccttcttgcttaaatccataggcatttgtctggccttccc
ttttactgctggctgggaaggaggagcatcagaccacagatcctggaaggcacttctctc
cctgactgctgctcacactgccgtgagaacctgcttatatccaggaccaaggaggcaatg
ccaggaagctggtgaagggtttcctctcctccaccatg (Seq ID No: 305)

Homo sapiens paired box 2 (PAX2) :

ctccctttctcctcaagtcctgaagttgagtttgagaggcgacacggcggcggcggccg
cgctgctcccgctcctctgcctccccatg (Seq ID No: 306)

Homo sapiens 5-aminoimidazole-4-carboxamide ribonucleotide f ormltransferase/IMP cyclohydrolase (ATIC) :

agccctcctacctgcgcacgtggtgccgccgctgctgcctcccgctcgccctgaacccag
tgcctgcagccatg (Seq ID No: 307)

Homo sapiens ATP synthase, H+ transporting, mitochondrial F1 complex, alpha subunit 1, cardiac muscle (ATP5A1) :

ccttctttgcggctcggccattttgtcccagtcagtccggaggctgcggctgcagaagta
ccgcctgcggagtaactgcaaagatg (Seq ID No: 308)

Homo sapiens cyclin G1 (CCNG1) :

cggccccttcggctccgagctgaccctgatcagggccgagttgtctcggcggcgctgccg
aggcctccacccaggacagtcccccctccccgggcctctctcctcttgcctacgagtcccc
tctcctcgtaggcctctcggatctgatatcgtggggtgaggtgagcaggcccggggaggg
tggttaccgctgaggagctgcagtctctgtcaagatg (Seq ID No: 309)

Homo sapiens cadherin 16, KSP-cadherin (CDH16) :

agctctcttcttgcttggcagctggaccaagggagccagtcttgggcgctggagggcctg
tcctgaccatg (Seq ID No: 310)

Homo sapiens cyclin-dependent kinase inhibitor 1B (p27, Kip1) (CDKN1B) :

ttttcttcttcgtcagcctcccttccaccgccatattgggccactaaaaaaagggggctc
gtcttttcggggtgtttttctcccctcccctgtccccgcttgctcacggctctgcgact
ccgacgccggcaaggtttggagagcggctgggttcgcgggacccgcgggcttgcacccgc
ccagactcggacgggctttgccaccctctccgcttgcctggtcccctctcctctccgccc
tcccgctcgccagtccatttgatcagcggagactcggcggccgggccggggcttccccgc
agcccctgcgcgctcctagagctcgggccgtggctcgtcggggtctgtgtcttttggctc
cgagggcagtcgctgggcttccgagaggggttcgggctgcgtaggggcgctttgttttgt
tcggttttgtttttttgagagtgcgagagaggcggtcgtgcagacccgggagaaagatg
(Seq ID No: 311)

Homo sapiens chimerin (chimaerin) 2 (CHN2) :

tctcctcttcttcctttgtgtgtgcgcgagcggagttggggcggagggagaagggggagg
tcgctctgtctgtccgtctcccgccgcctctgcccggtctactcgaagtgcggcgggaga
ggcgggagcccaggagagggtgcgggagctggcggggcggctcggagctgccaggacgcc
ctggtcccagccgcgcacaggggagcgtggacggcagaggggctcggcgggagccgagat
ccgcccgtcccggctgcccctcggcctccctctgctcccacctacccccctgacacccata

gaaaagcgtgcaaaggcgcggagcgggacggaaaccacaaataaatagcggcggcggcag
cgcgtcatctggtggagcaggaagtgcaggcagagtccggaggctggtgctttctgcgcg
tccccaggactttgccatggctgggggccgcggaggctgcgagcggccgggcgagggca
gcggcggcggcgtccgcaccggggctgagcgagcagcgacgcgaggggcgcgcggagatg
(Seq ID No: 312)

Homo sapiens citrate synthase (CS) :

gggcctccttgaggaccccgggctgggcgccgccgccggttcgtctactctttccttcag
ccgcctcctttcaaccttgtcaacccgtcggcgcggcctctggtgcagcggcggcggctc
ctgttcctgccgcagctctctccctttcttacctccccaccagatcccggagatcgcccg
ccatggctttacttactgcggccgcccggctcttgggaaccaaggcacccagtggcaagt
actagctgagcatttgggagatgcttgtcttacttggctgttgcttctcctgctgctggg
gaaaaggaatgcatcttgtcttgttcttgcagcccggcatgccagtgcttcctccacgaa
tttgaaagacatattggctgacctgatacctaaggagcaggccagaattaagactttcag
gcagcaacatggcaagacggtggtgggccaaatcactgtggacatg
(Seq ID No: 313)

Homo sapiens cathepsin S (CTSS) :

atttcttttcaagtcaattgaactgaaatctccttgttgctttgaaatcttagaagagag cccactaattcaaggactcttactgtgggagcaactgctggttctatcacaatg (Seq ID No: 314)

Homo sapiens deoxynucleotidyltransferase, terminal (DNTT) :

cagtctccctcccttctggagacaccaccagatgggccagccagaggcagcagcagcctc ttcccatg (Seq ID No: 315)

Homo sapiens dual specificity phosphatase 3 (DUSP3) : cgctctccgcctcgcttgctcctgccgggcgtgcagggccccgccgccgccatg (Seq ID No: 316)

Homo sapiens coagulation factor II (thrombin) receptor-like 2 (F2RL2) :

catcctttccctgcggaggaccagggcaagtttcctgcctgcacggcacaggagagcaaa cttctacagacagaccaaggcttccatttgctgctgacacatggaactgaggtgaaattg tgctccatgattttacagatttcataacgtttaagagacgggactcaggtcatcaaaatg (Seq ID No: 317)

Homo sapiens Fc fragment of IgG, receptor, transporter, alph a (FCGRT): cgtcctctcagcatg (Seq ID No: 318)

Homo sapiens guanylate binding protein 2, interferon-inducib le (GBP2) :

ttacctctttttcttgtctctcgtcaggtctctgacattgacagagcctggacgttggag gaagccccaggacgttggaggggtaaagtaaaagtccacagttaccgtgagagaaaaaag agggagaaagcagtgcagccaaactcggaagaaaagagaggaggaaaaggactcgacttt cacattggaacaaccttctttccagtgctaaaggatctctgatctggggaacaacaccct ggacatg (Seq ID No: 319)

Homo sapiens G protein pathway suppressor 1 (GPS1) :

cgctctttctcccttcagcagccagccagctctgtgtcagggtcggggggtgcagaaagt caggacagaatg (Seq ID No: 320)

Homo sapiens general transcription factor IIF, polypeptide 2 , 30kDa (GTF2F2) :

gttcctcttttcctcggttcccagtgttctggcaggtaaggaacgccggctcttcgcctc tcagcgcggcttgtcctttgttccggacgcccgctcctcagccctgcggctcctggggtc gctgctgcatcccgcacgcctccaccggctgcagacccatg (Seq ID No: 321)

Homo sapiens glycogenin 1 (GYG1) :

cgctccctcccggtgccggcttctctgagtcaccaacctgaggctgccccggccgcctgc gcacccggcagcaccatg (Seq ID No: 322)

Homo sapiens heat shock 70kDa protein 9 (mortalin) (HSPA9): agctctttgccgtcggagcgcttgtttgctgcctcgtactcctccatttatccgccatg (Seq ID No: 323)

Homo sapiens iron-responsive element binding protein 2 (IREB2) :

cttccttctttcctcccttgccagtccgcctgtcttcctccccgtcttccctgcccggcc tccccccttcttcccccgctggccccctccccggagggataatatggtctccggcgatg (Seq ID No: 324)

Homo sapiens origin recognition complex, subunit 1 (ORC1) :

ccaccttcttttcatttctagtgagacacacgctttggtcctggctttcggcccgtagtt gtagaaggagccctgctggtgcaggttagaggtgccgcatcccccggagctctcgaagtg gaggcggtaggaaacggagggcttgcggctagccggaggaagctttggagccggaagcca tg (Seq ID No: 325)

Homo sapiens RAB1A, member RAS oncogene family (RAB1A) :

cattcctttctttcgattacccgtggcgcggagagtcagggcggcggctgcggcagcaag ggcggcggtggcggcggcggcagctgcagtgacatg (Seq ID No: 326)

Homo sapiens cytohesin 2 (CYTH2) :

gagtcttttcagcgctgaggactggcgctgaggaggcggcggtggctcccggggcgtttg agcgggctcacccgagcccgcgggccaacgcggatccaggcccgactggcgggaccgccc cggattccccgcgggccttcctagccgccatg (Seq ID No: 327)

Homo sapiens COP9 constitutive photomorphogenic homolog subu nit 2 (Arabidopsis) (COPS2): atttctcctccccctcccggccaagatg (Seq ID No: 328)

Homo sapiens solute carrier family 9
(sodium/hydrogen exchanger), member 3 regulator 1 (SLC9A3R1) :

ggtcctctctcggctcctcgcggctcgcggcggccgacggttcctgggacacctgcttgc ttggcccgtccggcggctcagggcttctctgctgcgctcccggttcgctggacgggaaga agggctgggccgtcccgtcccgtccccatcggaaccccaagtcgcgccgctgacccgtcg cagggcgagatg (Seq ID No: 329)

Homo sapiens peptidase (mitochondrial processing) beta (PMPCB): ctaccttccttctagcagaaatg (Seq ID No: 330)

Homo sapiens RAB3D, member RAS oncogene family (RAB3D) :

cggcccttcctccgccttctgggcggagcccgcgcgggatccgggtggctgcaggctgct ggcttctgcggctgcggggtcggggtcgcggccagggccaagccgcagcgagttcacagg cggaacccctgcaggcggcgccccctacgcgaggtcacccctgggaaggagcgcagccca cccggcccctccgcatccgagcaggacgcccgtctcctctccctgaggatttcaggtctc cctgtcccaggaggcttgtgccaagatg (Seq ID No: 331)

Homo sapiens ATP-binding cassette, sub-family B (MDR/TAP) : tcttctctcggttcctctttcctcgctcaagatg (Seq ID No: 332)

Homo sapiens N-acylsphingosine amidohydrolase (acid ceramidase) 1 (ASAH1) : ggctcttctttgcctctgctggagtccggggagtggcgttggctgctagagcgatg (Seq ID No: 333)

Homo sapiens cytochrome c oxidase subunit VIc (COX6C) :

ttttcctttagtcaggaaggacgttggtgttgaggttagcatacgtatcaaggacagtaa
ctaccatg (Seq ID No: 334)

Homo sapiens COX15 homolog, cytochrome c oxidase assembly pr otein (yeast) (COX15) :

gcttctcttttccttggcggaggagggagaccacagagccctgggttgtggaagaggtgg
ctgttccctgtcatcagtatg (Seq ID No: 335)

Homo sapiens c-src tyrosine kinase (CSK) :

ccccttccccgcctttcttccctccgcgacccgggccgtgcgtccgtcccctgcctc
tgcctggcggtccctcctcccctctccttgcacccatacctctttgtaccgcaccccctg
gggacccctgcgcccctccccctccccctgaccgcatggaccgtcccgcaggccgctgat
gccgcccgcggcgaggtggcccggaccgcagtgccccaagagagctctaatggtaccaag
tgacaggttggctttactgtgactcggggacgccagagctcctgagaagatg
(Seq ID No: 336)

Homo sapiens versican (VCAN) :

gagcctttctggggaagaactccaggcgtgcggacgcaacagccgagaacattaggtgtt
gtggacaggagctgggaccaagatcttcggccagccccgcatcctcccgcatcttccagc
accgtcccgcaccctccgcatccttccccgggccaccacgcttcctatgtgacccgcctg
ggcaacgccgaacccagtcgcgcagcgctgcagtgaattttccccccaaactgcaataag
ccgccttccaaggccaagatg (Seq ID No: 337)

Homo sapiens dystroglycan 1 (dystrophin-associated glycoprotein 1) (DAG1) :

gcgcctcttaggcttggcggtggcggcggcggcagcttcgcgccgaatccccggggagcg
gcggtggcggcgtcctggggccaggaggagcgaacacctgccgcggtcctcccgccggcg
ctgggctctgtgtgctccgggatggagcaggtgtgcagagggtgagaacccagctctggg
accaagtcacttgcttccttacttagcaagactatcgacttgagcaaacttggacctggg
atg (Seq ID No: 338)

Homo sapiens DEAD (Asp-Glu-Ala-Asp) box helicase 5 (DDX5) :

cccccctcttttggttacagacgtgagggctctttggagacgtaaacatctccgagtggcg
agggtgggcggggctgggcttgggaaagggcggggtggcttgcttgaggtgtggaaagac
cagaagaaggtgaggtcaagagagtgcagaatgaggcattccaatggtgggtggccctg
acctgagagagtggcgcggggaggggtgaaagcgcggcgatcctggaacgccagcgggcg
ttgcggcctatgcgcgaggggcggggcgattaggtcatagagcggctcccagcgttccct
gcggcgtaggaggcggtccagactataaaagcggctgccggaaagcggccggcacctcat
tcatttctaccggtctctagtagtgcagcttcggctggtgtcatcggtgtccttcctccg
ctgccgcccccgcaaggcttcgccgtcatcgaggccatttccagcgacttgtcgcacgct
tttctatatacttcgttccccgccaaccgcaaccattgacgccatg
(Seq ID No: 339)

Homo sapiens desmoplakin (DSP) :

gctcctctgcgcccttgccgccctccgagccacagctttcctcccgctcctgcccccggc
ccgtcgccgtctccgcgctcgcagcggcctcgggagggcccaggtagcgagcagcgacct
cgcgagccttccgcactcccgcccggttccccggccgtccgcctatccttggcccccctcc
gctttctccgcgccggcccgcctcgcttatgcctcggcgctgagccgctctcccgattgc
ccgccgacatg (Seq ID No: 340)

Homo sapiens glutamyl-prolyl-tRNA synthetase (EPRS) :

cttcctttcgcggggtcctccgtagttctggcacgagccaggcgtactgacaggtggacc
agcggactggtggagatg (Seq ID No: 341)

Homo sapiens acyl-CoA synthetase long-chain family member 4 (ACSL4) :

gctcctcctcgtcccagcgctagcgggcacgcggttccttttgcgagctttccgagtgc
caggcgccggccggctgcgaagacgcggtgggccgcccctccgattgaaatcacagaaga
tattcgtgttcttcttaagagaaaagaggacattttagctttctcagttgaaggcgtac
tttattgtcggcttccaaagattactaacttttatctgtatcactaagattgaactgcct
tggctgtactgctattcttactgctgcttctattattgccttcttcagcacaataaggct
ttcaaaagccaaagaataacaagaaataagcaccattttagaagcctttccactatg
(Seq ID No: 342)

Homo sapiens fibroblast activation protein, alpha (FAP):

tggtccttttcaacggttttcacagatccagtgacccacgctctgaagacagaattagct
aactttcaaaaacatctggaaaaatg (Seq ID No: 343)

Homo sapiens UDP-N-acetyl-alpha-D-galactosamine:polypeptide

N-acetylgalactosaminyltransferase 3 (GalNAc-T3) (GALNT3):
ctgcctctccaggcaacgcgggaggcccagcgggaaggcaggaggcggcggcggaggagg
agctctactgagccgcaactgtggcgacagcaaccggagtcgcagccgccgccacctgca
cctggcgcctagcccacgtccagcgcctgcccggccgccgcttcccgccaccctgccctg
cccacccgccaggtactaccattaaagataccttcttctcagcaaatctatgataaaaaa
tataagtaacagaagaagaaataactgttatttgtcaagtgacaagcttttaatgtcaga
atg (Seq ID No: 344)

Homo sapiens glypican 3 (GPC3) :

acgtctcttgctcctcagggccactgccaggcttgccgagtcctgggactgctctcgctc

cggctgccactctcccgcgctctcctagctccctgcgaagcaggatg
(Seq ID No: 345)

Homo sapiens interleukin enhancer binding factor 2, 45kDa (ILF2) :

acgcctcttcagttgtctgctactcagaggaaggggcggttggtgcggcctccattgttc
gtgttttaaggcgccatg (Seq ID No: 346)

Homo sapiens nucleosome assembly protein 1-like 1 (NAP1L1) :

gggtctttttagcgccatctgctcgcggcgccgcctcctgctcctcccgctgctgctgc cgctgccgccctgagtcactgcctgcgcagctccggccgcctggctccccatactagtcg ccgatatttggagttcttacaacatg (Seq ID No: 347)

Homo sapiens asparaginyl-tRNA synthetase (NARS) :

cgctctctgatgcaacgccggaatcgcggaaaccgccggtgcacgttggagtcataagac ggcgtcggtgttgcagtctgtgtccttggaggtgaccagggccactgcaggcatg (Seq ID No: 348)

Homo sapiens NADH dehydrogenase (ubiquinone) 1 alpha subcomplex, 10, 42kDa (NDUFA10): cgtccccttgggtccttgatcctgagctgaccgggtagccatg (Seq ID No: 349)

Homo sapiens NADH dehydrogenase (ubiquinone) Fe-S protein 2, 49kDa (NADH-coenzyme Q reductase) (NDUFS2) : ttctccttcccgcagtctgcagccggagtaagatg (Seq ID No: 350)

Homo sapiens NADH dehydrogenase (ubiquinone) Fe-S protein 5, 15kDa (NADH-coenzyme Q reductase) (NDUFS5) :

catcctttacggcaggcgtccgcgtcgctagctagtcgttctgaagcggcggccagagaa gagtcaagggcacgagcatcgggtagccatg (Seq ID No: 351)

Homo sapiens phosphoenolpyruvate carboxykinase 2 (mitochondrial) (PCK2) :

ccctcctttttaagcgcctcccgccagcctctgctgtggctcgcttcgccgcgctccctc cttccccgccttccatacctccccggctccgctcggttcctggccaccccgcagcccctg cccaggtgccatg (Seq ID No: 352)

Homo sapiens serpin peptidase inhibitor, clade B (ovalbumin), member 6 (SERPINB6) :

ctcccttcgcgctccggacgggcgacggtagctcgagacccgggactccgcccgcctccc cgcgagtatttgaggtccggggcggctccggcgcctctgcccgccgttctgctcgctcgc tccccgctctggagtctgccatcatg (Seq ID No: 353)

Homo sapiens Rab geranylgeranyltransferase, alpha subunit (RABGGTA) :

ttctctcctcagacttcaagggctaccactggacccttcccctgtcttgaaccctgagcc ggcaccatg (Seq ID No: 354)

Homo sapiens Rab geranylgeranyltransferase, beta subunit (RABGGTB): ctctctcctttccctgttagacatg (Seq ID No: 355)

Homo sapiens small nuclear ribonucleoprotein polypeptide A (SNRPA) :

agttctctccgcacgcgggctggagaagcgggtcctacgcacgctttgttgtcgcgcttt gcctccgtccttgcccctactcccgccttacctgacttccttttcggaggaagatccttg agcagccgacgttgggacaaaggatttggagaaacccagggctaaagtcacgtttttcct cctttaagacttacctcaacacttcactccatg (Seq ID No: 356)

Homo sapiens sterol regulatory element binding transcription factor 2 (SREBF2) :

cgccctttctgtgcggcgcccgggcgcaacgcaaacatggcggcgggtggcacccgtcgg
tgaggcggtgccgggcgggggttgtcgggtgtcatgggcggtggcgacggcaccgccccc
gcgtctccctgagcgggacggcagggggggcttctgcgctgagccgggcgatg
(Seq ID No: 357)

Homo sapiens translin (TSN) :

ctgccctttggacgcgcgcctcggttccgaacgcagcggacggcgcctcaggcagcgcgg
cggacagcccgtcctccggcgcgccgcgagcctcggaggaccctagcgacggtcgtggcg
taagaccgggggacgcggcggtagcggcggccgttgcgattgattgcgctggttgcctg
cggcgtccacttccttggccgcccttgctacactggctgattgttgtgcagccggcgcca
tg (Seq ID No: 358)

Homo sapiens Fanconi anemia, complementation group G (FANCG) :

ccaccctttctcgaggctgtggcctccgcgagagccgagcgggccgcaccgccggccgtg
cgactgccccagtcagacacgaccccggcttctagcccgcctaagcctgtttggggttgc
tgactcgtttcctccccgagtttcccgcgggaactaactcttcaagaggaccaaccgcag
cccagagcttcgcagacccggccaaccagaggcgaggttgagagcccggcgggccgcggg
gagagagcgtcccatctgtcctggaaagcctgggcgggtggattgggaccccgagagaag
caggggagctcggcggggtgcagaagtgcccaggcccctccccgctggggttgggagctt
gggcaggccagcttcacccttcctaagtccgcttctggtctccgggcccagcctcggcca
ccatg (Seq ID No: 359)

Homo sapiens DEAD (Asp-Glu-Ala-Asp) box polypeptide 39B (DDX39B) :

ttccctccttcgtcgctgttgctgccgccatacgcgctctccctgtttagctcttctgtt
agaaatagtatctttgttttcctttgctgttcctcaatcccctactcttcaccccttgtt
ttcacctattttgcgagaacccatccagatccccccttcccttcttcccctgccggcccag
ttatg (Seq ID No: 360)

Homo sapiens RAB11A, member RAS oncogene family (RAB11A): ccgccctttcgctcctcggccgcgcaatg (Seq ID No: 361)

Homo sapiens SPARC-like 1 (hevin) (SPARCL1):

agctctttcccttttggtttgcaagcactgcctgtaaagccctcgcatgagaggccagcc
tgctagggaaatccaggaatctgcaacaaaaacgatgacagtctgaaatactctctggtg
ccaacctccaaattctcgtctgtcacttcagacccccactagttgacagagcagcagaat
ttcaactccagtagacttgaatatgcctctgggcaaagaagcagagctaacgaggaaagg

gatttaaagagtttttcttgggtgtttgtcaaactttattccctgtctgtgtgcagagg
ggattcaacttcaatttttctgcagtggctctgggtccagccccttacttaaagatctgg
aaagcatg (Seq ID No: 362)

Homo sapiens cyclin B2 (CCNB2) :

ctccctttcagtccgcgtccctccctgggccgggctggcactcttgccttccccgtccc tcatg (Seq ID No: 363)

Homo sapiens cytochrome c oxidase subunit VIIa polypeptide 2 like (COX7A2L) :

ggtccttctctggggcggtcgcgttggcagcggatgcgggaagccggactctgggcgtca tg (Seq ID No: 364)

Homo sapiens lysophosphatidic acid receptor 2 (LPAR2) :

cgccctctcagcaacccgcacagggcgcacccggacgctctaccgctcccgccgcagtcg ccgggccatgggcctcgagcccgccccgaacccccgcgagcccgccttgtctgcggcgtg actggaggcccagatg (Seq ID No: 365)

Homo sapiens adaptor-related protein complex 4, mu 1 subunit (AP4M1) :

cgttcttttgttccggggccgcagggcggggcaggcccgactttcgccgtcttcttgtct actctccagaacggccatg (Seq ID No: 366)

Homo sapiens budding uninhibited by benzimidazoles 3 homolog (yeast) (BUB3) :

cttcctctccgcctccttcgcctagcctgcgagtgttctgagggaagcaaggaggcggcg gcggccgcagcgagtggcgagtagtggaaacgttgcttctgaggggagcccaagatg (Seq ID No: 367)

Homo sapiens DEAD (Asp-Glu-Ala-Asp) box helicase 21 (DDX21) : ctacctcttcctctccacgcggttgagaagaccggtcggcctgggcaacctgcgctgaag atg (Seq ID No: 368)

Homo sapiens solute carrier family 33 (acetyl-CoA transporter), member 1 (SLC33A1) :

tgctctctgccgcattgatagcagcgagagctggaggtgttgggtcgggagaccagccgt tcgatcccgccgcaggtaggagctggtttccatcctggcaccacggcacacacctccagc ctcgagcccggcgctgctgcccggggtctccttcaggctctttgacgccgttccagggg gcacctatccaggcatcctctgggcctctagccagaggactggctcccggcttcagcact ccgggctgcagtaagaagtgcccttatcgctctgagccctgccaccatcccgtgaaccac cgaaaccctggtccagcgcgacagccttggacctgggactggacggatccaaaacgctca gcctcggcccccacagacggggctctgcatcgtctctgatatg (Seq ID No: 369)

Homo sapiens G protein-coupled receptor 37 like 1 (GPR37L1) : tgctcttcctgggctggctgtctcctgctcatccagccatg (Seq ID No: 370)

Homo sapiens neuronal regeneration related protein homolog (rat) (NREP) :

ctgtctttctagcatgttgccctttttcaaccacatttgtgtttcaggtgtagagaggag

agagagtgaacagggagcggggcttttgtctgttggtctccctggactgaagagagggag aatagaagcccaagactaagattctcaaaatg (Seq ID No: 371)

Homo sapiens vesicle-associated membrane protein 3 (cellubrevin) (VAMP3) : gcttctctgctgaccctctctcgtcgccgctgccgccgccgcagctgccaaaatg (Seq ID No: 372)

Homo sapiens synaptosomal-associated protein, 29kDa (SNAP29): cctccttctgtttcccagaccgagagccgcgccggcaccatg (Seq ID No: 373)

Homo sapiens lon peptidase 1, mitochondrial (LONP1) :

ccccctcttctccgcgtaggcccagctccctgaagcggctgtttcgagccacgcgcccat
cgggtaccgaggcacgcgccgggcgtcacgtgcgtttcgcggcgagcggaaatgacgcga
gttgtgtgagccgccagtatggccgggctatg (Seq ID No: 374)

Homo sapiens kinesin family member 3B (KIF3B) :

ctgtctctccccatccggggcagcggggaatggctgagccaggggttcgccgcccccgcc
gccgccgccgccgccgccgccgccgccgcccgctttcggctcgggcctcaggaccgt
agcatcctgagacattttgaattgacacttctcaagatttgactggatcagagttcatca
tg (Seq ID No: 375)

Homo sapiens transmembrane 9 superfamily member 2 (TM9SF2) :

cttcctttatctctggcggccttgtagtcgtctccgagactccccacccctccttccctc
ttgacccccctaggtttgattgccctttccccgaaacaactatcatg
(Seq ID No: 376)

Homo sapiens cytosolic iron-sulfur protein assembly 1 (CIAO1) :

gagcctctgtcggccgcggaagcctggagtgggcggtacgcagacgcgcgcggtgagacc
cgctgtctgctcagcggactctgcccgcccccacctccccctgcgtcgggccgacatg
(Seq ID No: 377)

Homo sapiens GRB2-related adaptor protein 2 (GRAP2) :

caccctctttcagagtggtacatggaagacagcacaaagtggatccatactctgaaatgc
agtaactctgatgcttgaatttgtctcccttcttgccagaaaggattctaataactcggt
gtcaaagccaagacataaactcaaccccttctcttccaaaagcttcacgttacagcatg
(Seq ID No: 378)

Homo sapiens leupaxin (LPXN) :

gtacctttctcggggtgtctgcgtaactgcccagacttgccttggtttggtcagatgaca
cctcctctgggactggctagccagcgttcatg (Seq ID No: 379)

Homo sapiens SH3-domain binding protein 5 (BTK-associated) (SH3BP5) :

tttcctctgctccgccgcggccggaggtatccgcatcggcgagctgcgtctcccgggtgt
cggccccggcggctccccgaccgtgcccggctgtggcgaggcggctccagcccagcctgt
ggcagccgcgacccccggggcgctccggagcccactgcgcggcgcgcgtgccggctgcct
gcatg (Seq ID No: 380)

Homo sapiens phosphatidylinositol glycan anchor biosynthesis , class B (PIGB) : ctttcttccgccttaggaaggtggcggccagggatg (Seq ID No: 381)

Homo sapiens lipopolysaccharide-induced TNF factor (LITAF) :

cggccctttctcggggcgcccgagaggccagctcagacctcccggctcgacaggcggcg
cgggcggcggtgagtgcggcgcggggacgccggggcgcggggaccagcgggagacagcgg
ggggccggtggcgccagcacctgctggggccccgggcactgagcccttggctggggcct
cctgggatgccaggggcgcgggtcgggtcgcgggcatcgaggcgcggcggagggcgtgg
gggcccggccggggcggggtccggcctcccagcgctggtcccggccgcgtctccggttgg
gttcagctcctgcgtcccagagtggcccgatcgcgcgtggcggggtcgtccggcccccac
ccgaacgagcgcccttcgcggcccgccgcgtccccctccccggagaggacggcccctggg
ctttttagaaaaaggcgcgattctctctagtgactcaggttgagatttccagaaatatcc
cccgggggttcagaaacaaaaccaaaacaaacaaaaaaaccccaacgaattcccaaatgc
tatttgccaaacatttgacttctaggggcgcgggtacccgcgtttctctccctgcccccg
cgacttcgcgcaagatccgggaaggacacccgaggcccctgggagaccctggggaggtga
aaatcagagagcgaagcgggccgtggcccctaggcctgacccctccccgcggggtaaggc
gggcaccccgcgagcgcaggggtcctcttactgctgatggcacccagctctggcccaga
cgccgctcaccgtccaccgccggtgctgggtaaaatg (Seq ID No: 382)

Homo sapiens etoposide induced 2.4 mRNA (EI24) :

ccaccccttcggctctgggccccgcctcgtggtgccggctggttcttcgcgctcgcccga
cttcccagcggccccgtgcggcccgggcatgcccagtgcgggcgcagcggccccggccct
ggaagcgccccggcggagctggcctgcggtgggctaggggcagggccggagccgcggcgg
cggagctgtggatccttcatgatgagagatttggggacacttctctctcctgtgtgtagt
tgatagtttggtggtgaagagatg (Seq ID No: 383)

Homo sapiens chromosome 14 open reading frame 2 (C14orf2) :

tgacctttccgagttggctgcagatttgtggtgcgttctgagccgtctgtcctgcgccaa
gatg (Seq ID No: 384)

Homo sapiens peroxiredoxin 6 (PRDX6) :

attcctccgcgcgctgggacaggctgcttcttcgccagaaccaaccggttgcttgctgtc
ccagcggcgccccctcatcaccgtcgccatg (Seq ID No: 385)

Homo sapiens solute carrier family 29 (nucleoside transporters), member 1 (SLC29A1) :

ctctcttccgcccggcggcccacaccggtcaggcccggcgcgggctgcgctctccagctg
tggctatggccccagccccgagatgaggagggagagaactaggggcccgcaggcctggga
atttccgtcccccaccaagtccggatgctcactccaaagtctcagcaggcccctgaggga
gggagctgtcagccagggaaaaccgagaacaccatcaccatg (Seq ID No: 386)

Homo sapiens heterogeneous nuclear ribonucleoprotein F (HNRNPF) :

cgaccttcctgccgggccgggcggtccgaggctgctggagtgccgtgagcaggccgcggg
aacgtcgccgtcaccttgtctcggggcctcggcgctgcttcccgccaaaacacgtttacc
gcgcgcccgggcctcccaccttgcggaagggaccccaccaccacttggatttctgttgca
ggttgagaacaaaaacatgcacctggagtttccccggagccctctgcgtggttgagcttc
ggtggaatttcggggctcttggctgccagccgcgcttgcctggtagcaacagaaaccagt

cctgctcgcctccgtggacatttcattaccatccagaagtgtctcccactgaaggcatcc
gtggttgttttaagccacaaaaagccacacccaagatcacctgacacccaccctgaca
agtgtccatg (Seq ID No: 387)

Homo sapiens islet cell autoantigen 1, 69kDa (ICA1) :

ccgcccctttccctcgccttcggctgacgctgacgtcggatgagtgatccggagggacgc
tccgaccgcggccgggaggctcctggggggccggggctccgaggttataatataacttatc
ctctcatgctttttttcctgccccttctccccaaatcatcaacaatagaagaagaagaaaa
catg (Seq ID No: 388)

Homo sapiens PWP2 periodic tryptophan protein homolog (yeast) (PWP2) : gtgtctctgtgggcggccgccgggttgagctgcggcacacgtgcgacggccgtgatg (Seq ID No: 389)

Homo sapiens glutaminyl-tRNA synthetase (QARS) : gtttcttttagtttccggtgtctctgcaatg (Seq ID No: 390)

Homo sapiens stearoyl-CoA desaturase (delta-9-desaturase) (SCD) :

cggcctctgtctcctcccccctcccgcccttacctccacgcgggaccgcccgcgccagtca
actcctcgcactttgcccctgcttggcagcggataaaagggggctgaggaaataccggac
acggtcacccgttgccagctctagcctttaaattcccggctcggggacctccacgcaccg
cggctagcgccgacaaccagctagcgtgcaaggcgccgcggctcagcgcgtaccggcggg
cttcgaaaccgcagtcctccggcgaccccgaactccgctccggagcctcagccccctgga
aagtgatcccggcatccgagagccaagatg (Seq ID No: 391)

Homo sapiens fragile X mental retardation, autosomal homolog 1 (FXR1): cggcctttgcggttccaacatg (Seq ID No: 392)

Homo sapiens musculin (MSC) :

tagccttttcaaaaggcgcagcttaccgcggtgcgcgcggattctggacttgggcgccaa
ctcgtagtccacgctccccggggtcagcagaggggcgctcacgctctcgccacccacctc
gctttctcaccccgcgcttcccggcctgggtttttagtcttccttggagcgctctctggc
ctccgcctccgccagggagcggaaggcggagacagcgagactggccaggggggaggaaag
aggacgcgtgtgggcaagggggacaacgggatg (Seq ID No: 393)

Homo sapiens RNA binding motif protein 8A (RBM8A):

cgacctttcccctctgcgacagtttcccgaggtacctagtgtctgagcggcacagacgag
atctcgatcgaaggcgagatg (Seq ID No: 394)

Homo sapiens heparan sulfate
(glucosamine) 3-O-sulfotransferase 1 (HS3ST1) :

ggtcctctgcgccctggcagccaggagtcgccgccacgaccgccgggtctcagtgggtgc
ctgcgccttctccccgcccgcctgccccgggccatccagaaacttgctctacccgccgcg
ggtgctcggcagtgctgcccatggcccagcccaggagcctatttagggcgccggacgggc
tggacagaggcgcggctcagtaattgaaggcctgaaacgcccatgtgccactgactagga
ggcttccctgctgcggcacttcatgacccagcggcgcgcggcccagtgaagccaccgtgg
tgtccagcatg (Seq ID No: 395)

Homo sapiens solute carrier family 12 (potassium/chloride transporters), member 6 (SLC12A6) :

ctgtctcttgtaggcagggatcacagtctgaaacgacagcaaggaagaggtaggcaggga
aaactaactggaaggaagtttaaatacagaaagagcaaagtattatctaactataacaat
g (Seq ID No: 396)

Homo sapiens apelin receptor (APLNR) :

cttcctccagggtctggagaacccagaggcagctcctcctgagtgctgggaaggactctg
ggcatcttcagcccttcttactctctgaggctcaagccagaaattcaggctgcttgcaga
gtgggtgacagagccacggagctggtgtccctgggaccctctgcccgtcttctctccact
ccccagcatg (Seq ID No: 397)

Homo sapiens calpain 1, (mu/l) large subunit (CAPN1) :

cgctcttcctggttgggccctgccctgagctgccaccgggaagccagcctcagggactgc
agcgacccccaaacacccctcccccaggatg (Seq ID No: 398)

Homo sapiens cyclin C (CCNC) :

cttcctttcgccgtcgccgccgcggagcggagtcgagccgagctgatttgatcgaggagc
gcggttaccggacgggctgggtctatggtcgctccgcgggccgctccgccggctggtgct
tttttatcagggcaagctgtgttccatg (Seq ID No: 399)

Homo sapiens glutamate dehydrogenase 1 (GLUD1) :

cttcctccctagtcgcggggagtctgagaaagcgcgcctgtttcgcgaccatcacgcacc
tcccctccgcttgtggccatg (Seq ID No: 400)

Homo sapiens guanine nucleotide binding protein-like 1 (GNL1) :

cctccttcctcgccgccggggcgccctctcggtgccactggctctcacgtgccagtagcc
caccccgcatcatcctctcgcctcgctcctggagggaagtgactatatctcccccgtccg
ccttccatcgccgccgcggcggtaattctgtcgggcccgcccgctgacgtcacctgctag
ccccgcctcctctagggtcccgggcccctgcggcgggggctgccccgggggcagtcagt
tgaggcggcgggagctcggcggagggcgggccaggtgactggtccgggccatg
(Seq ID No: 401)

Homo sapiens lysophosphatidic acid receptor 4 (LPAR4) :

aggccttttgtgtcctgtttgctaaaggcatgcgggctacagcattcaagagagggagt
cgttaacaaagggaaagagataaatgtaaataagctcacatttacagaatgagcggtttg
cagtaaaaagctgcggcagcccagagtctgctactttaggctgggctaacctttccctgt
aaaaaaaaaaaaaaaaaaaaaaaaaaaaaatggataaaaatatgcacttccaaagggcg
agttgcccatttacatgtttattagctaattatctacaggcatcagcacattctctcatc
tagcacactctttcttggggaggaaaatatttcctaccggtccatagtgtcagagtggtg
aacccctgcagccagcaggcctcctgaaaaaaaagtccatg (Seq ID No: 402)

Homo sapiens G protein-coupled receptor kinase 5 (GRK5) :

gctcctctttgcagaggggaaactcttgggctgagagcaggaataatgcggtaggcaag
gcgggctgctggctccccggctccggcagcagcggcggcagcccgagcagcggcagcag
cagcggcagcaccccaggcgctgacagccccgccggccggctccgttgctgaccgccgac
tgtcaatg (Seq ID No: 403)

Homo sapiens glutamic-pyruvate transaminase (alanine aminotransferase) (GPT) :

agccctttctgtccctcccagtgaggccagctgcggtgaagagggtgctctcttgcctgg
agttccctctgctacggctgccccctcccagccctggcccactaagccagacccagctgt
cgccattcccacttctggtcctgccacctcctgagctgccttcccgcctggtctgggtag
agtcatg (Seq ID No: 404)

Homo sapiens hydroxyacyl-CoA dehydrogenase (HADH) : gggtctcctcgctgtcgccgccgctgccacaccatg (Seq ID No: 405)

Homo sapiens high density lipoprotein binding protein (HDLBP) :

tcttctcctttaccaagatggcggcttgtccctgtttcgccacagttcctaccttatgag
ctcggttttcttatgcttataagagtggaacagcaaaagctggcaggctgacagaggcgg
cctcaggacggaccttctggctactgaccgttttgctgtggttttcccggattgtgtgta
ggtgtgagatcaaccatg (Seq ID No: 406)

Homo sapiens histidine triad nucleotide binding protein 1 (HINT1): gttcctcccttcttccgagcctctcctctggccgccgcgcgggagagaggccgagatg (Seq ID No: 407)

Homo sapiens heat shock 70kDa protein 1A (HSPA1A) :

ctaccttttcgagagtgactcccgttgtcccaaggcttcccagagcgaacctgtgcggc
tgcaggcaccggcgcgtcgagtttccggcgtccggaaggaccgagctcttctcgcggatc
cagtgttccgtttccagcccccaatctcagagcggagccgacagagagcagggaaccggc
atg (Seq ID No: 408)

Homo sapiens nucleolin (NCL) :

cagtctttcgcctcagtctcgagctctcgctggccttcgggtgtacgtgctccgggatct
tcagcacccgcggccgccatcgccgtcgcttggcttcttctggactcatctgcgccactt
gtccgcttcacactccgccgccatcatg (Seq ID No: 409)

Homo sapiens nuclear factor, interleukin 3 regulated (NFIL3) :

ccgcccctttctttctcctcgccggcccgagagcaggaacacgataacgaaggaggccca
acttcattcaataaggagcctgacggatttatcccagacggtagaacaaaaggaagaata
ttgatggattttaaaccagagtttttaaagagcttgagaatacggggaaattaatttgtt
ctcctacacacatagatagggtaaggttgtttctgatg (Seq ID No: 410)

Homo sapiens protein phosphatase 1, regulatory subunit 3C (PPP1R3C) :

cagtctctcccagcgaccgccgcgggggcaaggcctggagctgtggttcgaatttgtgca
ggcagcgggtgctggcttttagggtccgccgcctctctgcctaatg
(Seq ID No: 411)

Homo sapiens protein tyrosine phosphatase, non-receptor type 14 (PTPN14) :

agttctttccaactttttctcggcggagtgagcgcagcgggcgcagactcgggggcaggt
tgctgtgcttctccgggctcagccgcctgctctcctggctcaggtcctcggggagcccta
gacagacatcaagtggccactggcgctccttcccctcccagctgagccatcctccccggc
ctcctcgggcgggacagccccgtgcttaggtttttctcctttctcccccggtgcgcctc
tgctcggactctcgcgccgggatcgcggcggaaacctccctcccctttcgcctcctgcgg
ctccttcccttcgcccctcctccgccagtcactggaatcaattccgtggggaatcggctc
cgccgccgcgaaggacagcctttccgcgcgggactccggggcgccacgggggccatgtaa
gcagctatcttccagagggccacactgggcatggacaccctttttccctgcctggaggagc
acaggtgatagtgtaatttccagtcacgaaactgctaaggccatctcagggggcgtgtgc
gccaggataggcgggcggcgtccgaggaccacatagccatg (Seq ID No: 412)

Homo sapiens selenoprotein P, plasma, 1 (SEPP1) :

ctttcttttaagttgataacaatcagctcaggggtttgctctgcttgcaaggtcactgca
agaatgaacattgaactttggactatacctgaggggtgaggtaaacaacaggactataaa
tatcagagtgtgctgctgtggctttgtggagctgccagagtaaagcaaagagaaaggaag
caggcccgttggaagtggttgtgacaaccccagcaatg (Seq ID No: 413)

Homo sapiens serine hydroxymethyltransferase 2 (mitochondrial) (SHMT2) :

agctcttctcgcgcatgcgttctccgaacggtcttcttccgacagcttgctgccctagac
cagagttggtggctggacctcctgcgacttccgagttgcgatg (Seq ID No: 414)

Homo sapiens tyrosine kinase with immunoglobulin-like and EG F-like domains 1 (TIE1) :

tttcctcttcctccccagcaccgacccacactgaccaacacaggctgagcagtcaggccc
acagcatctgaccccaggcccagctcgtcctggctggcctgggtcggcctctggagtatg
(Seq ID No: 415)

Homo sapiens coiled-coil domain containing 6 (CCDC6) : cctcctttccccagcccgccgcggccatg (Seq ID No: 416)

Homo sapiens nuclear receptor coactivator 4 (NCOA4) :

ggacctttcgcactcgggtcaggggtaaagcagcctgtcgcttgccgggcagctggtgag tcggtgacctggcctgtgaggagcagtgaggagaatg (Seq ID No: 417)

Homo sapiens chromatin assembly factor 1, subunit B (p60) (CHAF1B) :

gtgcctctgactgtccgggtccctccagcatttgcagctttctcctgtcttgaagaagt agaacggtgcccgagaaacgtttttccccttcgagactcaggaggatgaaagtcatcact tgtgaaatagcctggcacaacaaggagcccgtgtacagcctggacttccagcatg (Seq ID No: 418)

Homo sapiens 3'-phosphoadenosine 5'-phosphosulfate synthase 1 (PAPSS1) :

agccccgccccgctcgctggcctgccctcctcttgctaccctcccggcgcagagaacccc ggctgctcagcgcgctccgcggtcatg (Seq ID No: 419)

Homo sapiens Fas apoptotic inhibitory molecule 3 (FAIM3) :

tgccctcctcttgctaccctcccggcgcagagaaccccggctgctcagcgcgctccgcgg tcatg (Seq ID No: 420)

Homo sapiens N-acetylated alpha-linked acidic dipeptidase 2 (NAALAD2) :

cagcctcctgccagcgcgctctctgtttctctgcagccccgaagctcgcgaatgtagcag gcgccccaagctcggtcctcaagaagccatggcggaatccagggggccgtctgtacctttg gatgtgcttggctgctgcgctggcatctttcctgatgggatttatggtgggtaagt (Seq ID No: 421)

Homo sapiens abl-interactor 1 (ABI1) : ctgtctctttaacgcgagaggaagcgatgcagaggggtggaaaatg (Seq ID No: 422)

Homo sapiens potassium voltage-gated channel, Isk-related fa mily, member 3 (KCNE3):

cttccttttctgccttctctcctgctttctagctctgggctttcccagctccgaagtcaa tactgagatcccagatgtgtccagagacatcctgaagaggctcgggggtggaggagcctt agtgtgtccacaaaggactcctgaaactgactgagagccagt (Seq ID No: 423)

Homo sapiens target of myb1 (chicken)-like 1 (TOM1L1) : ggccctctggcgctaccatg (Seq ID No: 424)

Homo sapiens ubiquitin-like modifier activating enzyme 2 (UBA2) :

cgcccttcccccacccgcttccggccgcggctcggttctcccgcctccgcctccgccgcg gctcgtggttgtcccgccatg (Seq ID No: 425)

Homo sapiens scavenger receptor class B, member 2 (SCARB2) :

ctccctccttgcagttggatccctggcgggtgcggcccggcccggcccgtgagcggcgca cagaatg (Seq ID No: 426)

Homo sapiens insulin induced gene 1 (INSIG1):

actcctcctttccccgccccgcctccgttcggagagccggcgggcggcgcctctcggc caggaagcgcctcttggacgcgtgtgaccgatg (Seq ID No: 427)

Homo sapiens kinesin family member C3 (KIFC3):

aggcctcttctgaggctctaggtgccccagtagcagggccttctgcagcaaggccgggaa ctgctgcaccattggtgtgttttaccttaagggactccaggcagcttccttgctgggaag atattcatttgctggggtggggctgggggtgcagaggtaggaagtgctgtggctagaagg cggcctggccagcgagtaggtggtggagcgagtgagagcgtgtgcgctgtaaacagtgtg agtgcatg (Seq ID No: 428)

Homo sapiens LIM domain kinase 2 (LIMK2) :

aggcctcttctgaggctctaggtgccccagtagcagggccttctgcagcaaggccgggaa ctgctgcaccattggtgtgttttaccttaagggactccaggcagcttccttgctgggaag atattcatttgctggggtggggctgggggtgcagaggtaggaagtgctgtggctagaagg cggcctggccagcgagtaggtggtggagcgagtgagagcgtgtgcgctgtaaacagtgtg agtgcatgtgcgccagcgcgtgcaaggacacggtaagggatgtacatgtattgtctcgtg agtaagagcttgtgtgtgtgttgggatgggaagacacgtactggtatgagagcccgcgtg agaagtgtatgtgtgagtactcgcgtggaagttttgcactcgggtttgaggctgtgcaaa agtacgcatggctcaccaggtgtggggctgtgtggctgcctcgtgtgtgccagcccgtg tgcaggcctgttttgtgagagccttcagggaacgcatgagcacgtgtgccagtgcgagtg

cgggacgcggggaggcgggagagaccgagtgggaggccccgcgaaggagtgggagtggga gtgggagtgccggcgggagacctgcgggggcgcgcccggctgacgcgtgcgcgccagtg cgcgtgagtgcgggcgcgcgccgccgcccccccgccggggtcggagccggttgccatggga acgcgccgcggcccgagttaatcatttcctgtggaaagtgtgcgggaggggcgcgagcgg gctggccgaggaggaggcggcggcgtggagctgcctcctgccggcgggccgggccgggcc gagccccgggcgctgcggcgacgcctggatcctgcctccgccaggccggctgcctggtgc cccgaggaggctgctgagccccaggccatg (Seq ID No: 429)

Homo sapiens lectin, mannose-binding, 1 (LMAN1) : cctcctccgcgttccagaatccaagatg (Seq ID No: 430)

Homo sapiens MRE11 meiotic recombination 11 homolog A (S. cerevisiae) (MRE11A) :

cgttctctcccgcggaattcaggtttacggccctgcgggttctcagaggcaagttcagac cgtgttgttttcttttcacggatcctgccctttcttcccgaaaagaagacagccttgggt cgcgattgtggggcttcgaagagtccagcagtgggaatttctagaatttggaatcgagtg cattttctgacatttgagtacagtacccaggggttcttggagaagaacctggtcccagag gagcttgactgaccataaaaatg (Seq ID No: 431)

Homo sapiens nascent polypeptide-associated complex alpha su bunit (NACA) :

cttccttctgcaacaggcgtgggtcacgctctcgctcggtctttctgccgccatcttggt
tccgcgttccctgcacagtaagtactttctgtgccgctactgtctatccgcagccatccg
cctttctttcgggctaagccgccccggggactgagagttaaggagagttggaggctttac
tgggccacagggttcctactcgcccctgggcctccggacaaaatggggtctgcggttggt
gtcctggcaaaagcagggtagaagggctgcggggcgggcccagaatccgagcctgcagag
atgggagcagttgcagtgttgagggcggaagaggagtgcgtcttgttttgggaactgctt
cacaggatccagaaaaggaaatg (Seq ID No: 432)

Homo sapiens claudin 11 (CLDN11) :

cgcccttcgccgctgagctcgcagcctccggcgcccacctccacctccagtgtcccgcct
cgggccgtcgccctccagcggctcgcgagcgtgggagacgtacctggcaggcactgtcc
agcccaggcccaggcacagccgtgaggggcgaggcacggggacatcctggcggccaccat
g (Seq ID No: 433)

Homo sapiens retinoblastoma binding protein 4 (RBBP4) : ccgcccctcccgcaacgctcgaccccaggattcccccggctcgcctgcccgccatg (Seq ID No: 434)

Homo sapiens acyl-CoA synthetase medium-chain family member 3 (ACSM3) :

ccctcttctttagactgccacgaggaaaaagcagatgtgagaactcaaggttcagggctg
ctcttctaagaaacaagtctgccataatctccatctgtgttggaatctgttaactaatga
actggtctctgtgcaaatcctgagtgctaaagcttccaacaagactgatg
(Seq ID No: 435)

Homo sapiens syndecan binding protein (syntenin) (SDCBP) :

cgctctcttacactcgggcctcagaagtccgtgccagtgaccggaggcggcggcggcgag
cggttccttgtgggctagaagaatcctgcaaaaatg (Seq ID No: 436)

Homo sapiens serum/glucocorticoid regulated kinase 1 (SGK1) :

agtccttctcattccttgcccccgcccaaggctctcttcaccttccccgcgggggtcctc
tcgttttctgtctcccaaatgctggcttcccgcctttcctcccccgcttatttacttaat
taaggccctggggctgcaccccaccggcagctccttcgggggtgtggccgaagagctccg
agggcggggctgaccgagccatattcggcgtggccggtggtgattggtgagggcggggc
ctgccgcaggggggcggggcctgcaggtttggcccccgcagggagcgcagctggcgccgct
gggagctggtggcgcggcgcaggtcccggccgagtgtggcgcagcagtggcggcgcttcc
cattcgccatgcgccgggggtgggtgcccgaaggttgcatgatggaatttgaacattact
tcaagaggtttgtatttggattagttaattgggtttgtcctctgctgactgtttcttc
ggatgcatttttggtgtgctcttgagggattaaatg (Seq ID No: 437)

Homo sapiens Wolf-Hirschhorn syndrome candidate 2 (WHSC2) :

cgtccttccggctctcggctttgccacaaagcttcccgaagacgcggccgctacccggag
acgcggtcgccacccagaagcgctctcccgggaagccccgctcgtgggaccgcgccacct
gcgccgcctctgcggcccgcagcccgacgggcgccgccatgttggggtcctagcgaggga
cgcgtaggtgtcttcataagatg (Seq ID No: 438)

Homo sapiens nuclear receptor subfamily 1, group H, member 3 (NR1H3) :

cagtccttttgcaagagctgctaagagcgctgggtaaggagaggaaggggagagacatgg aacttggctggtctgcagggaaatgccactgttttggccgggagtagggggcgggagtgg cgggagaggggggtggccggctggggaggagccagcctggtggagaagctgccctgtggc ggggggtgaggagggggagggctgtggtcaccaggcaggaaggaggggtggcctgacccctc ggcagtccctcccctcagcctttccccaaattgctacttctctggggctccaggtcctgc ttgtgctcagctccagctcactggctggccaccgagacttctggacaggaaactgcacca tcctcttctcccagcaaggggggctccagagactgcccacccaggaagtctggtggcctgg ggatttggtgggtctgctccttag (Seq ID No: 439)

Homo sapiens glypican 6 (GPC6) :

cctcctttctccttccctcttgcctccagtgactgtctccaggatttctctcttcctatt tcaggaggactctcacaggctcccacagcctgtgttaagctgaggtttcccctagatctc gtatatccccaacacatacctccacgcacacacatccccaagaacctcgagctcacacca acagacacacgcgcgcatacacactcgctctcgcttgtccatctccctcccggggggagcc ggcgcgcgctcccacctttgccgcacactccggcgagccgagcccgcagcgctccaggat tctgcggctcggaactcggattgcagctctgaacccccatggtggtttttttaaacacttc ttttccttctcttcctcgttttgattgcaccgtttccatctgggggctagaggagcaagg cagcagccttcccagccagcccttgttggcttgccatcgtccatctggcttataaaagtt tgctgagcgcagtccagagggctgcgctgctcgtcccctcggctggcagaagggggtgac gctgggcagcggcgaggagcgcgccgctgcctctggcgggctttcggcttgaggggcaag gtgaagagcgcaccggccgtggggtttaccgagctggatttgtatgttgcaccatg (Seq ID No: 440)

Homo sapiens peptidylprolyl isomerase F (PPIF) :

cggccttctgggcgcgcgcgacgtcagtttgagttctgtgttctccccgcccgtgtcccg cccgacccgcgcccgcgatg (Seq ID No: 441)

Homo sapiens ARP1 actin-related protein 1 homolog A, centrac tin alpha (yeast) (ACTR1A) : agttccttccccagaaggagagattcctctgccatg (Seq ID No: 442)

Homo sapiens tripartite motif containing 28 (TRIM28) :

ggctctttctgcgagcgggcgcgcgggcgagcggttgtgcttgtgcttgtggcgcgtggt gcgggtttcggcggcggctgaggaagaagcgcgggcggcgccttcgggaggcgagcaggc agcagttggccgtgccgtagcagcgtcccgcgcgcggcgggcagcggcccaggaggcgcg tggcggcgctcggcctcgcggcggcggcggcagcggcccagcagttggcggcgagcg cgtctgcgcctgcgcggcgggccccgcgcccctcctccccccctgggcgcccccggcggc gtgtgaatg (Seq ID No: 443)

Homo sapiens aminoadipate-semialdehyde synthase (AASS) :

cggccttccatcccagtttcttctaggaattcggagcctcccctgcagcgactcggaaga ttcgaggcggcgggggacaagtcggcgccccagagcggacgagtcaccaggtgtcaagat g (Seq ID No: 444)

Homo sapiens cornichon homolog (Drosophila) (CNIH) : ccgcctttctccgctggcaacggcgccgctccccgctcctcctccccagccatg

(Seq ID No: 445)

Homo sapiens M-phase phosphoprotein 10
(U3 small nucleolar ribonucleoprotein) (MPHOSPH10) : ctcccttcccttgcatgctgcattgtgtcgggagttgctgacagccatg (Seq ID No: 446)

Homo sapiens ubiquitin specific peptidase like 1 (USPL1) :

ccgccttcctagtggagacgcgagtggggaggagcagtccgaggggaacgtgggttgaa
cgttgcaactagggtggagatcaagctggaacaggagttccgatcgacccggtaccaaga
aggggagtgcccgcggcagggttcattgaaaaatccttagtgatattgacatgtctcaa
gtgacataaattagccaatgactcggaatg (Seq ID No: 447)

Homo sapiens solute carrier family 23 (nucleobase transporters), member 1 (SLC23A1) :

tggcctttgtcaagtcatcccctcttctcctcaggaactgctcaaacctgtgccccaaag
atg (Seq ID No: 448)

Homo sapiens splicing factor 3b, subunit 4, 49kDa (SF3B4) : ggatctctttcgccatg (Seq ID No: 449)

Homo sapiens DnaJ (Hsp40) homolog, subfamily A, member 2 (DNAJA2):

ctgtctccctcggcctgtgccgccgccgacgccgcttgtgggcccgactccgctctgtct
gcttcgccaccttctccccgagcactgcccggccggccgccatg
(Seq ID No: 450)

Homo sapiens calicin (CCIN) :

catcctctcttccaccctctcttctccctggtcaaccgctctgcaaacaaccatcaatct
gatcccacaggcctgagaaagtctgctctccagtacctgctgctgatctgtttcagccga
caagaggcaccatg (Seq ID No: 451)

Homo sapiens mannosidase, beta A, lysosomal (MANBA) : ctgcctttcgatctctccacatctcggtggcgcgggatctcaagatg (Seq ID No: 452)

Homo sapiens microtubule-associated protein 1B (MAP1B) :

aatcctttctcctgccgcagtggagaggagcggccggagcgagacacttcgccgaggcac
agcagccggcaggatg (Seq ID No: 453)

Homo sapiens malate dehydrogenase 1, NAD (soluble) (MDH1) :

gagcctttctcgctaacaccgctcgccctctccgagtcagttccgcggtagaggtgacc
tgactctctgaggctcattttgcagttgttgaaattgtccccgcagttttcaatcatg
(Seq ID No: 454)

Homo sapiens microfibrillar-associated protein 1 (MFAP1) :

gtttctctatcagtcgcgcagctgtgttcgcggactcaggtggaaggaatttcttctctt
cgttgacgttgctggtgttcactgtttggaattagtcaagtttcgggaatcaccgtcgct
gccatcaacatg (Seq ID No: 455)

Homo sapiens chaperonin containing TCP1, subunit 3 (gamma) (CCT3) :

ggttctctctctccagaaggttctgccggttcccccagctctgggtacccggctctgcat
cgcgtcgccatg (Seq ID No: 456)

Homo sapiens tubulin, alpha la (TUBA1A) : caacctctcctcttcgtctccgccatcagctcggcagtcgcgaagcagcaaccatg (Seq ID No: 457)

Homo sapiens CD164 molecule, sialomucin (CD164) : ctttctcccgaacgccagcgctgaggacacgatg (Seq ID No: 458)

Homo sapiens cysteine-rich secretory protein 3 (CRISP3) : ctctctctgcaccttccttctgtcaatagatg (Seq ID No: 459)

Homo sapiens SMYD family member 5 (SMYD5) :

cggcctccatgtgcgacgtgttctccttctgcgtgggcgtggcgggccgcgcgcgggtct
ccgtggaagtccgtttcgtgagcagcgccaaggtgaggtcggggcgggtcctgccgggag
cctctccccagtccggccatg (Seq ID No: 460)

Homo sapiens kelch repeat and BTB (POZ) domain containing 10 (KBTBD10) :

ctgcctttttacagctagacctgtgtgctgcaaggagctaaggccttcagtgtccccttc
cttacccaggtttctcacagaatg (Seq ID No: 461)

Homo sapiens aldo-keto reductase family 1, member A1 (aldehyde reductase) (AKR1A1) :

ccgccccttgcaccgcccacgtggccagcgccacctgcctcattgtgcccaggagttctc
caaacccgcgctgcggagtgagtgaccaagttccggccagttcgacctcgaggatccaga
ggtggagacggtactacctcccagctctgttttccatccccttcaggtccttcctcggga
ggcggcgaaggcggtccaccctgcgcgtgatcctttatgcccggcccctgcccctccctc
cgggtggaacttcccctcaccgccagacttaagctgaggatcgttggatctctggcggg
gtgcagaactgagcccaggccacagtaccctattcacgctctgtgcttgtgccaaggttt
caagtgatcctcccgcctcagcctgcccaggtgctgagattacatgtatgagccactgca
cctggaaaggagccagaaatgtgaagtgctagctgaaggatgagcagcagctagccaggc
aaagggggcaatg (Seq ID No: 462)

Homo sapiens TRK-fused gene (TFG) :

tgttcttcccccacctgccacgtacagagcccaagttctcgctaggcttgttgggtcagc
gcgattggccggggcccgcgcgagcctgcgagcgaggtgcggcggtcgcgaagggcaacc
gaggggccgtgaccaccgcctccccgcgacgccccagtccagtggcctcgcgtccgccc
attcagcggagacctgcggagaggcggcggccgcggcctccgcaagccgtctttctctag
agttgtatatatagaacatcctggagtccaccatg (Seq ID No: 463)

Homo sapiens 3' (2'), 5'-bisphosphate nucleotidase 1 (BPNT1) :

catccttctcaaaagacttattgacagtgccaaagctcggtactggacacaacgagggac
ctgggtctacgataacgcgcttttgctcctcctgaagtgtctttggtccaacgttgttcc
agagtgtaccatg (Seq ID No: 464)

Homo sapiens guanine nucleotide binding protein (G protein) :

ttttctctctctctttcactgcaaggcggcggcaggagaggttgtggtgctagtttctct aagccatccagtgccatcctcgtcgctgcagcgacacacgctctcgccgccgccatg (Seq ID No: 465)

Homo sapiens major histocompatibility complex, class II, DM alpha (HLA-DMA) :

caccctctcggggagggagttggggaagctgggttggctgggttggtagctcctacctac tgtgtggcaagaaggtatg (Seq ID No: 466)

Homo sapiens transmembrane protein 50B (TMEM50B) :

tctccttcctgcgcgcgcgcctgaagtcggcgtgggcgtttgaggaagctgggatacagc atttaatgaaaaatttatgcttaagaagtaaaaatg (Seq ID No: 467)

Homo sapiens lactoperoxidase (LPO) :

cagtctttcctgctaagcctcagcgtctcctccaagccacatcaaaatctttccttctgg gcctttcccagaagtgaattcttgctggaaggtataaaagaccagctcctccaagcagag caactccctggctgccgtgaaaagacaaggcactgggcagtgatg (Seq ID No: 468)

Homo sapiens NEL-like 2 (chicken) (NELL2) : ctgcctttacaacagagggagacgatggactgagctgatccgcaccatg (Seq ID No: 469)

Homo sapiens nucleobindin 1 (NUCB1) : cgccctctgcggtgaaggagagaccacactgccatg (Seq ID No: 470)

Homo sapiens paired box 9 (PAX9) :

aagcctctttcatcggggcacagacttccttttacttcttccttttgccctctcgcctcc tcctcctgggaagaagcggaggcgccggcggtcggccgggatagcaacaggccgggccac tgaggcggtgcggaaagtttctgtctgggagtgcggaactggggccgggttggtgtactg ctcggagcaatg (Seq ID No: 471)

Homo sapiens cyclin-dependent kinase 16 (CDK16) :

cgccctttattcttgctcggcctcgccacagagagcaaatcagattggctgggcgacaac ctcaaagggcggggctgcacacgttcactacgggaatgaggtagcggtggagggggcagt tgggcggggataggccgtcctagctaaggtggtaaaggccaataactcttcaggctgcct ctcctcgaaaagtcatcttctcgcgaacctttaaaatgccttcctccccaagcacctcaa gggactagaactgagtgcttcatttgtctttttcctccttgcaaaagtcccgtttgcca ccatggggatgtaccaagtgagaccgagtaggggaacgagtggtgattgacgcgccagg ttactggccactgctcacctaggcgctagcaaacttctgccaagatcggaactgagtact aaacagcctccacagttctccctggtgccgtctccggcttggcgccgcatcctcctctgg gctcgcgatggccgcgtcccctcccgctgcggacgggtcctttggtacatg (Seq ID No: 472)

Homo sapiens serpin peptidase inhibitor, clade E (nexin, plasminogen activator inhibitor type 1), member 2

(SERPINE2) :

ctgcctctttccggctgtgaccctcctcgccgccgcttggctgcgtcctccgactcc
ccgcgccgccgagaccaggctcccgctccggttgcggccgcaccgccctccgcggccgcc
ccctggggatccagcgagcgcggtcgtccttggtggaaggaaccatg
(Seq ID No: 473)

Homo sapiens pancreatic lipase-related protein 1 (PNLIPRP1) :

aactcctttccccctgctgtgacgtacaggtgaggtaaacagtactgaagtccagggcgt
cggtgctcactgctctggcaatgcccggtgagactgaattatgtttaaatttattgtaga
tg (Seq ID No: 474)

Homo sapiens peripherin (PRPH) : ggctccttcccagcccccggcctagctctgcgaacggtgactgcccatccttggccgcaa tg (Seq ID No: 475)

Homo sapiens RAD21 homolog (S. pombe) (RAD21) :

gaccctttcccctccccgggccacccagcccgcccaactcccagcggagagcaaggttt
tcttctgttttcatagccagccagaacaatg (Seq ID No: 476)

Homo sapiens signal sequence receptor, delta (SSR4) : ttttcttttcctctaggcagagaagaggcgatg (Seq ID No: 477)

Homo sapiens tissue factor pathway inhibitor (lipoprotein-associated coagulation inhibitor) (TFPI) :

ctccctctttgctctaacagacagcagcgactttaggctggataatagtcaaattcttac
ctcgctctttcactgctagtaagatcagattgcgtttctttcagttactcttcaatcgcc
agtttcttgatctgcttctaaaagaagaagtagagaagataaatcctgtcttcaatacct
ggaaggaaaaacaaaataacctcaactccgttttgaaaaaaacattccaagaactttcat
cagagattttacttagatg (Seq ID No: 478)

Homo sapiens ubiquinol-cytochrome c reductase binding protei n (UQCRB): gcttctctttctggtcaaaatg (Seq ID No: 479)

Homo sapiens mitogen-activated protein kinase kinase kinase 12 (MAP3K12) :

ccgccttttgtgctgcggccgcggagcccccgagggcccagtgttcaccatcataccagg
ggccagaggcgatg (Seq ID No: 480)

Homo sapiens sushi-repeat containing protein, X-linked (SRPX) :

tggtctcttcggtctcctgccgcccccgggaagcgcgctgcgctgccgaggcgagctaag
cgcccgctcgccatg (Seq ID No: 481)

Homo sapiens aminopeptidase puromycin sensitive (NPEPPS) :

cccccctctccctccctccttgcgggccctcctccccttccctcccctccgccccccttccc
cgtaggcagcccgcccgccagtccgcccgcaccgcctccttcccagcccctagcgctccg
gctgggtctctcccccgcccccaggctcccccggtcgctctcctccggcggtcgcccgc
gctcggtggatg (Seq ID No: 482)

Homo sapiens fibulin 5 (FBLN5) :

tcgccttctgcccgggcgctcgcagccgagcgcggccggggaagggctctcctcccagcg
ccgagcactgggccctggcagacgccccaagattgttgtgaggagtctagccagttggtg
agcgctgtaatctgaaccagctgtgtccagactgaggccccatttgcattgtttaacata
cttagaaaatgaagtgttcattttaacattcctcctccaattggtttaatgctgaatta
ctgaagagggctaagcaaaaccaggtgcttgcgctgagggctctgcagtggctgggagga
ccccggcgctctccccgtgtcctctccacgactcgctcggcccctctggaataaaacacc
cgcgagccccgagggcccagaggaggccgacgtgcccgagctcctccgggggtcccgccc
gcgagctttcttctcgccttcgcatctcctcctcgcgcgtcttggacatg
(Seq ID No: 483)

Homo sapiens lysophospholipase I (LYPLA1) : cgctcttccttccgcttgcgctgtgagctgaggcggtgtatg (Seq ID No: 484)

Homo sapiens high mobility group nucleosomal binding domain 4 (HMGN4) :

tcgtcttctctgtcttagggctggtgctggccctgcccacgcctagggctccggcgcgtc
acgggcctcagctgggattcccgcgcccctcggacggccacgagactcggacatctttcc
aggaacagcgtgaggaggacagaagcacccaacaggactgctcaagccacctgcgaacac
tgctgctaccatg (Seq ID No: 485)

Homo sapiens eukaryotic translation initiation factor 3, sub unit M (EIF3M) : agttccctttccggtcggcgtggtcttgcgagt-ggagtgtccgctgtgcccgggcctgc accatg (Seq ID No: 486)

Homo sapiens Sec23 homolog A (S. cerevisiae) (SEC23A) : cctcctcttgacgtggcagaggcggcgccagccatg (Seq ID No: 487)

Homo sapiens cartilage associated protein (CRTAP) :

cgtcctctttcctttccttctccctcccctttcccttccttcgtcccttccttccttcc
tttcgccgggcgcgatg (Seq ID No: 488)

Homo sapiens vesicle amine transport protein 1 homolog (T. californica) (VAT1) :

ccgcccctcccgctggatcccgcagccgcggctcttcccgacgcgttccgccttccccag
ctgtgcactctccatccagctgtgcgctctcgtcgggagtcccagccatg
(Seq ID No: 489)

Homo sapiens importin 7 (IPO7) :

gcttctctttcctttcgcgccggttgccgctgcggagcgcggcgggtccatgtgcgcagt

gagtggcgctattcctggcccagtagcacccgagccccgggtttgaccgagtccgcgctg
cgatg (Seq ID No: 490)

Homo sapiens ATG7 autophagy related 7 homolog (S. cerevisiae) (ATG7) :

gctcctttgcgcacgcgcgccgcttcccagtggcaagcgcgggcaggaccgcgttgcgtc atcggggcgcgcgcctcagagagagctgtggttgccggaagttgagcggcggtaagtgag ccgcggcgggcgagggtgtagtggggtcttgctgggccggttttggaggcctggagtcaa ggggcgagctcgccagggagggcgagggtcacagcaagtctcaggatcctcctctgccag tttctgggtggtccttcctcctccagggactcactgattccggctggcgcccttcgtctg tagccgcgtcccctcagactggttcagtccggggtcttctgacttggaagctcgtgctga tttcctaagtcagcccctcctgtcctcttggtaggcagtgctcagaatcttcagtgttgg aacacgggagatgggacatttggattcccagcctggctgtgtctggatttgctgtctctg gcacgttccttccccatctaagctgcttttccatctgcaaaatgggaatgataatccgcc atttgtttaagtgaggaggttaaataagtttactttctgagaaagaagattctcgattcc ttggttacagggttagaaactaatg (Seq ID No: 491)

Homo sapiens dynactin 2 (p50) (DCTN2) :

cgctccctttgccgccgccttagcccgggacccgaacccagcctctcccctacccgaaca ccggccccggctccaccgaggcccgggtcccccagcccgtctcgccgccgccatg (Seq ID No: 492)

Homo sapiens acidic (leucine-rich) nuclear phosphoprotein 32 family, member B (ANP32B) :

agccccctttccctccatggtttctctccgctcccgtgagtaacttggctccgggggct ccgctcgcctgcccgcacgccgcccgccacccaggaccgcgccgccggcctccgccgcta gcaaacccttccgacggccctcgctgcgcaagccgggacgcctctccccccctccgccccc gccgcggaaagttaagtttgaagagggggaagaggggaacatg (Seq ID No: 493)

Homo sapiens protein C receptor, endothelial (PROCR) :

acttctcttttccctagactgcagccagcggagcccgcagccggcccgagccaggaaccc aggtccggagcctcaacttcaggatg (Seq ID No: 494)

Homo sapiens actin related protein 2/3 complex, subunit 1A, 41kDa (ARPC1A) :

cgctccctctgggcttccgtcctccgcccgcgcccgacggagcctgttcgcgtcgactgc ccagagtccgcgaatcctccgctccgagcccgtccggactcccccgatcccagctttctc tcctttgaaaacactaagaataatg (Seq ID No: 495)

Homo sapiens chaperonin containing TCP1, subunit 4 (delta) (CCT4) :

aggcccccttctccgcctccgcctcctcccgacgccggcgccgctttctggaaggttcgt gaaggcagtgagggcttaccgttattacactgcggccggccagaatccgggtccatccgt ccttcccgagccaacccagacacagcggagtttgccatg (Seq ID No: 496)

Homo sapiens Niemann-Pick disease, type C2 (NPC2) : gcttctttcccgagcttggaacttcgttatccgcgatg (Seq ID No: 497)

Homo sapiens phosphoribosylaminoimidazole carboxylase, phosp horibosylaminoimidazole succinocarboxamide synthetase (PAICS) :

acccctcttttctagagttctgcctcgcttcccggcgcggtcgcagccctcagcccactt
aggataatg (Seq ID No: 498)

Homo sapiens ST6 (alpha-N-acetyl-neuraminyl-2,3-beta-galactosyl-1,3)-N-acetyl

galactosaminide alpha-2,6-sialyltransferase 2 (ST6GALNAC2):
ctcccttctgcctgggacgtcagcggacggggcgctcgcgggccggggctgtatg
(Seq ID No: 499)

Homo sapiens polymerase (RNA) III
(DNA directed) polypeptide C (62kD) (POLR3C) :

aagccctttccgaggatggcaaaggatctgggaatgcttctccaaagatatgtggatgga
cgaaataggtctctggtgatactgaggcggggtggggacggggaggcaaagacttggctt
cttaggaattggaagaaataagtaaacaatgtttggtagcaatttgtaataaggaagtaa
tcataaaattaactacgtccgtttctgattgtgtcaactttgtcaaggagtagaagttta
agaattgaatactgtcctgcaaacaacgtaacctcatctcctgtttgacacaccctgttg
agaagcagtcctttacctcctaaatttctttttcgaaattatcatttcctttatggactg
agaataacactgcctgttcactcccaccgagctgtgaacagtgaccttaattcttccaag
cagggaagtgtagaaactaaggtctgtgacagaccgcaaaatcatctcccaatctttaag
gaaaatcagaatcacgcataatcccatagagataaatttgatgcatagtcttttcctatg
catacattttcctttttttttacaataattgaatttttatatttttttcagcttgcttct
gtcacttaatatattatgagtaatttttttttggtttttttttgttttggagacagaatctc
gcactgtcgcccgggttggagtgcagtggcgcgatctcggctcactgcaacctctgcctc
ccggcttcaagcgattctcctgtctcagcctccctagtagctgggattacaggcacccgc
caccacgcccagctaatttttttgtgtgtttttagtagagaaggggtttcactatattgg
ccaggctggtctcaaactcctgacctcatgatacgcccacctcggtctcccaaagtgcta
ggattacaggcctgagccaccgcgccagcctattatgaataatttctacatgaatacgc
atcgtactaaataactttaaatgttggtgtagtatgccattgtatgggtatggcatcatt
tattgttagacgttagattgtttccactaagtcggtattataaagagaactaatgacttc
attattattagctttttctttctttggacacaatatccaaaaagaaattgttgtttcaaa
gatatgcaagattttaaggcttttgatatgtattgtcaaattgccctccagaaagaat
acatgaatttacactcagcagctctgcttccagcgtgaaagactttctattgtaccattt
tggtgtttttccctagctctcagactccccagtacaatg (Seq ID No: 500)

Homo sapiens influenza virus NS1A binding protein (IVNS1ABP) :

gtgtctcccggtcgcgcgtggaggtcggtcgctcagagctgctgggcgcagtttctccgc
ctgctgcttcggcgcggctgtatcggcgagcgagcgagttcccgcgagttctcggtggcg
ctccccccttcctttcagtctccacggactggcccctcgtccttctacttgaccgctcccg
tcttccgccgccttctggcgctttccgttgggccgattcccgcccgcttcctcctgcttc
ccatcgaagctctagaaatgaatgtttccatctcttcagagatgaaccagattatgatgc
atcattatcacagaagaaattcgtgtctatagcttttaaggacttgattacatcattttc
aagcctgatagttttggaatcaccattagagcttaagacacacctgccttcatttcaacc
acctgtcttcataccctgacgaagtgcaccttttaacactcctttgtccttggattactt
aagagttcccagaaatacatttgccaccaacagagtagccaaatttataaggaaaaatg
(Seq ID No: 501)

Homo sapiens thioredoxin interacting protein (TXNIP) :

acccctcttttctccaaaggagtgcttgtggagatcggatcttttctccagcaattggg
ggaaagaaggcttttctctgaattcgcttagtgtaaccagcggcgtatatttttaggc
gccttttcgaaaacctagtagttaatattcatttgtttaaatcttattttatttttaagc
tcaaactgcttaagaataccttaattccttaaagtgaaataatttttgcaaaggggttt
cctcgatttggagctttttttttcttccaccgtcatttctaactcttaaaaccaactcag
ttccatcatg (Seq ID No: 502)

Homo sapiens ecotropic viral integration site 2B (EVI2B) :

ttttcctttcttagccaaatcaccaaaatgtccagttagaacaagaatttagcattctgc
aaaagaagttaacagctgagataacgaggaaatattctgaaatg
(Seq ID No: 503)

Homo sapiens guanine nucleotide binding protein (G protein), alpha inhibiting activity polypeptide 3 (GNAI3) :

ggttcttctgggcgctaagggagctgacggagagggccaccgcccagcaatagacggtgc
ctcagcctgccgagccgcagtttccgtggtgtgagtgagtccgggcccgtgtcccctctc
ccgccgccgccatg (Seq ID No: 504)

Homo sapiens polymerase (DNA directed), eta (POLH) :

cggcccttcgcagcgggcgcgctgtcagacctcagtctggcggctgcattgctgggcgcg
ccgctctcgtctgatccctgctggggacggttgcccgggcaggatcctttacgatccctt
ctcggtttctccgtcgtcacagggaataaatctcgctcgaaactcactggaccgctccta
gaaaggcgaaaagatattcaggagcccttccatttccttccagtaggcaccgaacccag
catttcggcaaccgctgctggcagttttgccaggtgtttgttaccttgaaaaatg
(Seq ID No: 505)

Homo sapiens solute carrier family 2 (facilitated glucose transporter), member 1 (SLC2A1) :

cgctctctggcaagaggcaagaggtagcaacagcgagcgtgccggtcgctagtcgcgggt
ccccgagtgagcacgccagggagcaggagaccaaacgacgggggtcggagtcagagtcgc
agtgggagtccccggaccggagcacgagcctgagcgggagagcgccgctcgcacgcccgt
cgccacccgcgtacccggcgcagccagagccaccagcgcagcgctgccatg
(Seq ID No: 506)

Homo sapiens zinc finger protein 138 (ZNF138) :

gggtctttgtctcgctgcagcgggtgctgcaggtctggccttcacttttctgcgtcctct
tactcctagaggcccagcctctgtggcgctgtgatctggttattgggagattcacagcta
agacgccaggatcccccggaagcctagaaatg (Seq ID No: 507)

Homo sapiens ubiquitin specific peptidase 3 (USP3) :

ctttctttgacgcaagggctcgagacgcagccgccgtcggccgagcgcccggctagaagc gacaccagacggagcctccggagttcctccgcccccacctcgccgggtcctggagccgca gtcctcccagctgccctcctcgtggccatg (Seq ID No: 508)

Homo sapiens calcium channel, voltage-dependent, gamma subun it 3 (CACNG3) :

ctgtcttttctccagtttgagcgggggtgtcgggagcaggcggagagctttcctgcgagg ctgtggaagcagtgaacactcttctcagcggctcgcctcccagcagtgctatttttgcc

atccgccctcacccccagcacacgcgctcgcacacacacgcacgcacgcacacacacaca cacacacactcacacagagacctctctgggtttctttgccttgagtctcccggggctgtg agaagccaggcgcatctcaaaccgagctggcagctccaggctccggagccatgccctgca cggaccctcgtctttaccacgctcctgaggaatgaaaggaacccagggaccctcagaagg cagcagtgatgcggaccaacccccggagcctgcacccttccgagggccataggcgaccc agggaactggagagagctccagaaaggaaatcccagctttcccaaagtccctgtggatgc tgacaaaaggagacctgaattttggaagagcctgtactaggttacccggctgcagagtg attttcccctccggcactgactctcccctccaaccccagccgtccagagtaccatgaa gaattatg (Seq ID No: 509)

Homo sapiens guanine nucleotide binding protein (G protein), beta 5 (GNB5): ttccctctccgctgcgtccccgcgcgaagatg (Seq ID No: 510)

Homo sapiens chaperonin containing TCP1, subunit 8 (theta) (CCT8) : cttcctccgcggtcttccgagcggtcgcgtgaactgcttcctgcaggctggccatg (Seq ID No: 511)

Homo sapiens prostaglandin E synthase 3 (cytosolic) (PTGES3) :

cgctctttccgcgcggtgcattctggggcccgaggtcgagcccgccgctgccgccgtcgc ctgagggaagcgagaagaggccgcgaccggagagaaaaagcggagtcgccaccggagaga agtcgactccctagcagcagccgccgccagagaggcccgcccaccagttcgcccgtcccc ctgccccgttcacaatg (Seq ID No: 512)

Homo sapiens zinc finger protein 266 (ZNF266) :

ttttcttcctggtggcgtttgggcttaatacagctttggcgaggtcggatgacgggtggg
agccagcggtggaaggggtggcgaaagtaccggtttgccccaggccgccgaggggcctcc
ttagagagaccttgcctgctccgctcgcgtccgccggggccgcgcgggtcctcctggcgc
cgccaggttcaaaaagccactcgagttgtcactgcgacggccctgggccaggagccgttt
cgggatctgtcaaacaacgagttttcgtcgttcgaatcaggttgactggtccttcatccc
cccaatctcccgtacctggcgagtccagctcgtcgcggcaatgctaagaaaagagtgata
tgcaagctgagaccaaaaatatggtatgatttagccatactgaaggggaaggaaataaga
gctgggcaaagcattctgtgaattggctgactccacttctatggtgagagagaggagtgc
atcaaagattactcccagtagagatggtttcagcatgttggccagtctggtctcagactc
ctgacctcaagtgatccacccacctcggcctcccaaaatgctgggattacaggtataagc
cactgtgcctggccaaagataccgttaaccctggataaagagaatggaggttacctctgt
ccgtgtagattcctaagctgtcctggagtgatccttggagtaaaggaaaggtgctttgaa
gcacattcagccatcagccctgtgggatggcagccactgatttgtcctatggtctttaca
gggacccagtctgccttcaagaaaagacagaagtagaaagggtggtggctgactgtctga
caaattgttatcaggtatgcaggaagtatatccttctccaaaatatcatacttgcatcac
caggtagacacatttccttctacacagaattatcttcagagcttcttaaagcaaataaag
cctgcttcaaggactgagtccctagtcgaattcccggaaggagtggagcctgtcatattg
tgtttatctagcatctgctcaagagtgtgctgcagtggagggaaatcagatgacctccca
gtctggttgtgttacatacaatcatgtgtaagaagtgccattcaagccgtgtcactggag
gggactgacagtgagattcagtgacttttgatgatctggctgtggacttcaccccagaag
aatggactttactggacccaactcagagaaacctctacagagatgtgatg
(Seq ID No: 513)

Homo sapiens methylenetetrahydrofolate dehydrogenase (NADP+ dependent) 2, methenyltetrahydrofolate cyclohydrolase (MTHFD2): gcttccctcccggcgcagtcaccggcgcggtctatg (Seq ID No: 514)

Homo sapiens chemokine (C-C motif) receptor 9 (CCR9) :

cttcctttctcgtgttgttatcgggtagctgcctgctcagaacccacaaagcctgcccct
catcccaggcagagagcaacccagctctttccccagacactgagagctggtggtgcctgc
tgtcccagggagagttgcatcgccctccacagagcaggcttgcatctgactgacccacca
tg (Seq ID No: 515)

Homo sapiens heat shock 105kDa/110kDa protein 1 (HSPH1) :

cctccccttttgggtcggtagttcagcgccggcgccggtgtgcgagccgcggcagagtga
ggcaggcaacccgaggtgcggagcgacctgcggaggctgagccccgctttctcccagggt
ttcttatcagccagccgccgctgtccccgggggagtaggaggctcctgacaggccgcggc
tgtctgtgtgtccttctgagtgtcagaggaacggccagaccccgcgggccggagcagaac
gcggccagggcagaaagcggcggcaggagaagcaggcaggggggccggaggacgcagaccg
agacccgaggcggaggcggaccgcgagccggccatg (Seq ID No: 516)

Homo sapiens StAR-related lipid transfer (START) domain containing 10 (STARD10) :

tggtcctttcttttatgattcacaaggaatgaccctcttcatcgcctctcctaattcagt cctcacaacagtccttttacaaatgggacaacaggttagaggaagtcaggcagatttcca gcatcatagagagtaaaggaccagggaaggatcaggattcaaggactgcacccaggctct gcttccagcttgctgtgtgactttgggtaattttgttcccttagggaactgagctttctc atttgtaaatgcaaacaggctgttgggaggatcaaatgagatccaggggtgaaaacagct tagtttactttcaggaatttacccacgcggtatataaaggcaaaatattattatagtcag gtgattgtagattgaggaacccatttcctcattctgcaaattgcaaacctgaggcccaa agagggacaggggcttgccccaggtctcagcaggctgtgagcaagagctaaagcctaatc ctcctgcctttgggcctggagcccttccttgtaccccaggggtcagtgtctttgttggat acaggcttagattgactgactgtaccctgagaacctaggggagtccctgttcccaattct tctcctacccccaccttggcctgatggaggaagaccctgctgtgttgagatgagcaccag agccaagaagctgaggaggatctggagaattctggaggaagaggagagtgttgctggagc tgtacagaccctgcttctcaggtcccaggaaggtggcgtcagcatctgcagccgcgtcga cgttgtcggagcctccgcggaggacccaggagagccggactaggaccagggccctgggcc tccccacactccccatg (Seq ID No: 517)

Homo sapiens UTP14, U3 small nucleolar ribonucleoprotein, ho molog A (yeast) (UTP14A) : ctttccttcggcttccgttcttggtccatgtgagagaagctggctgctgaaatg (Seq ID No: 518)

Homo sapiens SUB1 homolog (S. cerevisiae) (SUB1) : ggttctctgtcagtcgcgagcgaacgaccaagagggtgttcgactgctagagccgagcga agcgatg (Seq ID No: 519)

Homo sapiens minichromosome maintenance complex component 5 (MCM5) :

ccgcctcttgttttcccgcgaaactcggcggctgagcgtggaggttcttgtctcccctg gtttgtgaagtgcggaaaaccagaggcgcagtcatg (Seq ID No: 520)

Homo sapiens RNA binding motif (RNP1, RRM) protein 3 (RBM3) :

tactctttatcaatcgtcttccggcgcagccccgtccctgtttttgtgctcctccgagc tcgctgttcgtccgggtttttacgttttaatttccaggacttgaactgccatg (Seq ID No: 521)

Homo sapiens KDEL (Lys-Asp-Glu-Leu) endoplasmic reticulum protein retention re ceptor 1 (KDELR1) :

ctcccctctcgctctcctccctcttcccggctccagctccgccgccagctccagccttt gctccccctcccaaagtcccctccccggagcggagcgcacctagggtccctcttccgtcc ccccagcccagctacccgttcagaccagcagcctcgggggcacccccccgccagcctgc ctccctcccgctcagccctgccagggttccccagccatg (Seq ID No: 522)

Homo sapiens StAR-related lipid transfer (START) domain containing 3 (STARD3) :

agatcttcttccgctctgaggcgctactgaggccgcggagccggactgcggttggggcgg gaagagccggggccgtggctgacatggagcagccctgctgctgaggccgcgccctccccg ccctgaggtgggggcccaccaggatg (Seq ID No: 523)

Homo sapiens heterogeneous nuclear ribonucleoprotein A0 (HNRNPA0) :

cggcctctttgtgtggtgcccagataggggagcggaggtggcggcggcggcggtagcggt
ggccttggttgtcttccagtctcctcggctcgccctttagccggcaccgctccccttccc
tccccttcctcttccttccttccctccccttccctttttcccttccccgtcggtgag
cggcgggggtggctccagcaacggctgggcccaagctgtgtagaggccttaaccaacgat
aacggcggcgacggcgaaacctcggagctcgcagggcgggggcaaggcccgggccttgga
gatg (Seq ID No: 524)

Homo sapiens chromobox homolog 1 (CBX1) :

ggctcttttgttcggctgaggggagggccgttggccggggcctgcggtacgccgcttcag
tgagggacgccactgcggccacccggcttgctgccttcctgggcgccactcccccaggcg
acccgacgcgacgcgccagcagcgcagcaccgattcctctcggctcttggcgctgctc
tgaggtgaggagcccgctggaggcgggagagctgggggagggggcgcggcggcggcggcg
gcgggagccctgcgtgagggaacgcgctttcgaggcggaggttaggagcggggagcgcgc
ccgggtccagcgtcctgcttctccgcttcccgcgctgagctcttcgcctgtcgctgaggc
gtcggtgccagctgcgtgaaggatggagagggcggggcgcgaatcctgagccagagactg
agtgcttgggggtgggccgagcacttgggggccgctcttcggggcccgggtggtctggaa
caatgttgcttggctgggcggctgcgggatagggcggaaggggacaggcttgaggcttgg
ataggcgtgaggaggcgcatacgaccgcacaacccgaggtttgtaactgtattcggaaga
cgccgggtccggctgggactgccagaggaacctggctttgcaggactacggaggagtaac
gtcgagtgaattggaagagggcccagggccgcacaagcagcgtcaccctttacaccagaa
agctggcgggcactatg (Seq ID No: 525)

Homo sapiens myeloid/lymphoid or mixed-lineage leukemia (trithorax homolog, Drosophila); translocated to, 11 (MLLT11) :

cgcccttcttaggaggggctgcattgcaggggggagagtgaactgacagactcagtcactg
aagagggaaaaggagtgagaagacaaagccgtcaaagccccaacagctttgtatttctcc
agcccggcgcagaccccggagctcccgaggcactccctccatctttggaacacgccagta
attgattgataacaggaagctatg (Seq ID No: 526)

Homo sapiens interferon-induced protein 44-like (IFI44L) :

ttttctttctttcctagagtctctgaagccacagatctcttaagaactttctgtctccaa
accgtggctgctcgataaatcagacagaacagttaatcctcaatttaagcctgatctaac
ccctagaaacagatatagaacaatg (Seq ID No: 527)

Homo sapiens cyclin I (CCNI) :

acttcttcctcccttcccctctcttcccctccctccccagccttccccgcgagcggacgc
ggcagcgcctctgtctcgctttttcttatttttccccccttttccccttttcttttttttt
tttcttttcttttctcccctccccccctttcaccatttcccctcggaggcgctttccccg
ggcaggggcagagccggtctcacccccccgcctctccccggcccccgccgccctatggcga
gagggagccccctcccaacccgggctcgagcggcggcggcctcaggccggggtcatcat
ggaactaattcgctgaccgacccagcggccgcagccgtgcgtcccgctcgagcgccagcg
cccgcgcccgcgccccccgatccgcttcccctttctccctcctcagttggccgagtcgtc
ccgcgcgcaccgcctccgcgcgcctatgagaatgaggtggtaacgggcccccggatgacc
ccgcgtcaccactgtgaggcctacagctctgccggggaggaggaggaggaggaagaggag
gagaaggtagctacagcaagctgggtagcaggcagatccaaaggatatcatg
(Seq ID No: 528)

Homo sapiens methionyl aminopeptidase 2 (METAP2) : cattccctcgcgctctctcgggcaacatg (Seq ID No: 529)

Homo sapiens leukocyte immunoglobulin-like receptor, subfami ly B (with TM and ITIM domains), member 4 (LILRB4) : gtctctttgtcctgccggcactgaggactcatccatctgcacagctggggcccctgggag gagacgccatg (Seq ID No: 530)

Homo sapiens destrin (actin depolymerizing factor) (DSTN) :

gggtctctcggtcccgcagccgtgaggaggacggtctgcatactcgctgcccgccggctc cctcccccgcgtccctgcgaccgccgcggcgaagatg (Seq ID No: 531)

Homo sapiens eukaryotic translation initiation factor 2D (EIF2D) :

gggccctttcgcggccgggccccagcatggctgcccccacggctgagggcctggcagct gctgcgccctcgctttcttgacattccctggcttctgtgctctcttccccaggccacccc agcagacatg (Seq ID No: 532)

Homo sapiens histamine N-methyltransferase (HNMT) : ctgtctttctcagaaaaccaaatatg (Seq ID No: 533)

Homo sapiens ras-related C3 botulinum toxin substrate 1 (rho family, small GTP binding protein Racl) (RAC1) :

gtttctctgcagttttcctcagctttgggtggtggccgctgccgggcatcggcttccagt ccgcggagggcgaggcggcgtggacagcggccccggcacccagcgccccgccgcccgcaa gccgcgcgcccgtccgccgcgccccgagcccgccgcttcctatctcagcgccctgccgcc gccgccgcggcccagcgagcggccctgatgcaggccatcaagtgtgtggtggtgggagac

ggaaacaagaatctcagtgtaacccgagcaaaatcgcgcgtctcagcgttgcttgtatag agctgtaggtaaaacttgcctactgatcagttacacaaccaatgcatttcctggagaata tatccctactgtctttgacaattattctgccaatgttatg (Seq ID No: 534)

Homo sapiens signal recognition particle 72kDa (SRP72) : tcgtctcctccaagatg (Seq ID No: 535)

Homo sapiens zinc finger protein 33B (ZNF33B) :

ccgcctttcctttgtttgtctcacgttttgcgtgggaggcggtcccgggatttcagggg tctaccggctctcttatggcgaatgcaacccgaagagagagtgagctgtatcttcagagt tgtctccgtctttccaagaacagaacaaaatg (Seq ID No: 536)

Homo sapiens zinc finger protein 16 (ZNF16) : gcctcctttccaagcgcgacccgttgaggtccttgtcatg (Seq ID No: 537)

Homo sapiens zinc finger protein 33A (ZNF33A) :

ccgcctttcctttgtttttctcaggttttgcgtgggaggcggtcccgggatttcaaggg tctacgcgcttttctatggcgaatgcaacccgacgagggagtggctgtatcttcagagt tgtctccgtctttccaagaacagaacaaaatg (Seq ID No: 538)

Homo sapiens butyrophilin, subfamily 3, member A3 (BTN3A3) :

ctttctttttcctttcttcggaatgagagactcaaccataatagaaagaatggagaacta
ttaaccaccattcttcagtgggctgtgattttcagaggggaatactaagaaatggtttc
catactggaacccaaaggtaaagacactcaaggacagacattttggcagagctgctcac
tccttgctcagctcagttttctgtgcttggaccctctgggcccatcctggccatg
(Seq ID No: 539)

Homo sapiens butyrophilin, subfamily 2, member A2 (BTN2A2) :

ctctttgggatgctttgttgtctggtggtgactgtgcccatgggtgagttgtatcggaaa
atcgtcatgtgaggatcagaggggaaaagaaaacagaggcctctggtctctgcctgccct
gggtgctcatg (Seq ID No: 540)

Homo sapiens nudix (nucleoside diphosphate linked moiety X)-type motif 21 (NUDT21) :

acgcctcctcttgcgctgtcctgttaatggcgggcagtagccgctgaggggattgcagat
aaccgcttcccgcacggggaaagtctaccctgcctgccactttctgctcgccgtcagcgc
cggagctcgccagcatg (Seq ID No: 541)

Homo sapiens stathmin-like 2 (STMN2) :

tgctctttctctagcacggtcccactctgcagactcagtgccttattcagtcttctctct
cgctctctccgctgctgtagccggaccctttgccttcgccactgctcagcgtctgcacat
ccctacaatg (Seq ID No: 542)

Homo sapiens katanin p60 (ATPase containing) subunit A 1 (KATNA1) :

caccctcttccgccgctcccgcccagcgacctcgctcccggggcgacgccccgcgtgcgc
cagagtcgccgaggtcgtccccggcaccggaagtgaccctggcgggtttgtcttcaaatt
ctcggcgagcaggagccgcgccggcaggtggtgttgacgattgaactgggcagtactggg
gccgtgagcggagagcaaagtgggctggactgggtcaggccctccttcctcgctgccggg

atctccactccgccaatcccctgtgcctggcgttgggcggtttcccgaggagcttgggcc
gccgcagcttacagttgaacatg (Seq ID No: 543)

Homo sapiens butyrophilin, subfamily 3, member A2 (BTN3A2) :

ctttctctttttcctttcttccggatgagaggctaagccataatagaaagaatggagaat
tattgattgaccgtctttattctgtgggctctgattctccaatgggaataccaaggatg
gttttccatactggaacccaaaggtaaagacactcaaggacagacattttggcagagca
tagatg (Seq ID No: 544)

Homo sapiens CLK4-associating serine/arginine rich protein (CLASRP) :

cggcctttcatttccgcttccggtgcgggccgcgcgcgagcgcagcggtgggaggcggcg
accagccggttgaggccccaggcttggcctcaccacaatg (Seq ID No: 545)

Homo sapiens clathrin, light chain A (CLTA) :

ctccctcctggcgcttgtcctcctctcccagtcggcaccacagcggtggctgccgggcgt ggtgtcggtgggtcggttggttttgtctcaccgttggtgtccgtgccgttcagttgccc gccatg (Seq ID No: 546)

Homo sapiens NADH dehydrogenase (ubiquinone) flavoprotein 1, 51kDa (NDUFV1) :

gcgtctctatcgcgccagttcctcagcctcagtgctatgaaggtgacagcgtgaggtgac ccatctggcccgccgcgatg (Seq ID No: 547)

Homo sapiens signal sequence receptor, gamma (translocon-associated protein gamma) (SSR3) :

gggcctttgcccgccttggcggccggctctacgttccctgttctcgcctgcagctccgcc atg (Seq ID No: 548)

Homo sapiens valosin containing protein (VCP) :

gcttcccttccgatgattcggctcttctcggctcagtctcagcgaagcgtctgcgaccgt cgtttgagtcgtcgctgccgctgccgctgccactgccactgccacctcgcggatcaggag ccagcgttgttcgcccgacgcctcgctgccggtgggaggaagcgagagggaagccgcttg cgggtttgtcgccgctgctcgcccaccgcctggaagagccgagccccggcccagtcggtc gcttgccaccgctcgtagccgttacccgcgggccgccacagccgccggccgggagaggcg cgcgccatg (Seq ID No: 549)

Homo sapiens zinc finger protein 195 (ZNF195) :

gggcctttgtcccgacagagctccacttcctgtccccgcggctctgtgtcccctgctagc cgtaggtcgtgtgacccgcaggcaccgggagatccagaagtgaaacgccaggctctctgg aggccaggagatg (Seq ID No: 550)

Homo sapiens testis-specific kinase 2 (TESK2) :

cagtctttcgcggcccgggagctcagcagagctaccagctgccctgttggcttcgctggt cggatcgtcctcctggccccgccaaacaggcgggggagcggccccgactgtggggccat ggcagtagtctcctcgttcgccgccgccgctagcctagctgagtcgccggcttctgcgct aggggctcccaccgcctccgcaggctaaggagccgctgccaccaacgagctgtgagggtt actatgctccctctttgccgccgtctcctcctcttgcccgcgcaggcacccctctggctg ctcagtcctgcctcagtgtcaaaccagaagagaagtaaaattcaacaaaaatttatgtgt

ggagttccttcttaaaagaagaaaaaagtgattatttagactatg (Seq ID No: 551)

Homo sapiens family with sequence similarity 107, member A (FAM107A) :

agccctccttgctagtctgggacttcccggtggagtgaggaacccagcaacacgctcctg acttcccttcccaaggactcgacctgagaaggacacagcagtctctgaatttcatgctct cctctttgatgtgaagaaaatgaaaagctgaacagttgtggaactgtggatagagttaga caataaggccgccatg (Seq ID No: 552)

Homo sapiens serine/threonine kinase receptor associated pro tein (STRAP) :

ccctccctccctttccctccctcgtcgactgttgcttgctggtcgcagactccctgaccc
ctccctcacccctccctaacctcggtgccaccggattgcccttcttttcctgttgcccag
cccagccctagtgtcagggcgggggcctggagcagcccgaggcactgcagcagaagagag
aaaagacaacgacgaccctcagctcgccagtccggtcgctggcttcgccgccgccatg
(Seq ID No: 553)

Homo sapiens mitochondrial ribosomal protein L3 (MRPL3) :

ctttctttccgtcgcagagagcatcggccggcgaccgttccggcggccattgcgaaaact
tccccacggctactgcgtccacgtggcggtggcgtggggactccctgaaagcagagcggc
agggcgcccggaagtcgtgagtcgagtcttcccgggctaatccatg
(Seq ID No: 554)

Homo sapiens zinc fingers and homeoboxes 1 (ZHX1) :

ctcccttcccctccgcccccggacggccgctggggcgcgcgcctctcctcgcaccccca
ccctgagtccccacactccgcggggccaccgagctgctgaggcccctttgcgggcccgcc
gagcggttccgggtttagggttcacaggtcagagttgactccctgaaaagtgcagccggt
ttgaaatgcaagatggcggcggcgtggcgctgagaggcgcggcggcccctgcaggagaag
acagactgctgctttggacctgttggtaatgatggcctgagctaaacatctaactagaag
ggatacccttccatttcaaagaacagaatgctaaggaagctgtggcaagtgattggagtt
gtgcttcaaaaatttcagaaattcagcagtattttatctgccaacaataagctctttact
tgattgcaccatgagaaagctgctaatgagacttgttgagcacaaaaatggacttgaaga
accaaaagccattgttttcaaatgaagaacactgaacagttttaagcctcgatgcttttt
aatcaccactgagcttttcctcataacatcagaatg (Seq ID No: 555)

Homo sapiens calcium binding protein P22 (CHP) :

ccttccttccctccctccttccctcctgtcgccgtctcttctggcgccgctgctcccgga
ggagctcccggcacggcgatg (Seq ID No: 556)

Homo sapiens ecdysoneless homolog (Drosophila) (ECD) :

ctttctctcaggatttccgctggcttcaggttccggtcaggcgtcgggacagagcctgat
ccaggcttcggcggccggtggcagctctcgatcagctctcgcagtcggagaggcggctaa
ggaaaggtgccacagcagagacgcgaaggagaggccctagaacctttttcaaagaagaatg
(Seq ID No: 557)

Homo sapiens V-set and immunoglobulin domain containing 4 (VSIG4) : gagcctctttggtagcaggaggctggaagaaaggacagaagtagctctggctgtgatg (Seq ID No: 558)

Homo sapiens prohibitin 2 (PHB2) :

tgccctttctttcgccagccttacgggcccgaaccctcgtgtgaagggtgcagtacctaa
gccggagcggggtagaggcgggccggcaccccccttctgacctccagtgccgccggcctca
agatcagacatg (Seq ID No: 559)

Homo sapiens signal transducer and activator of transcriptio n 1, 91kDa (STAT1):

ctgccttttctcctgccgggtagtttcgctttcctgcgcagagtctgcggaggggctcgg
ctgcaccgggggggatcgcgcctggcagaccccagaccgagcagaggcgacccagcgcgct
cgggagaggctgcaccgccgcgcccccgcctagcccttccggatcctgcgcgcagaaaag
tttcatttgctgtatgccatcctcgagagctgtctaggttaacgttcgcactctgtgtat
ataacctcgacagtcttggcacctaacgtgctgtgcgtagctgctcctttggttgaatcc
ccaggcccttgttggggcacaaggtggcaggatg (Seq ID No: 560)

Homo sapiens heat shock protein 90kDa alpha (cytosolic), class B member 1 (HSP90AB1) :

agctctctcgagtcactccggcgcagtgttgggactgtctgggtatcggaaagcaagcct
acgttgctcactattacgtataatccttttcttttcaagatg (Seq ID No: 561)

Homo sapiens cancer susceptibility candidate 3 (CASC3) : cgttctccgtaagatg (Seq ID No: 562)

Homo sapiens nuclear cap binding protein subunit 2, 20kDa (NCBP2): gcttctctgcactatg (Seq ID No: 563)

Homo sapiens non-POU domain containing, octamer-binding (NONO) :

cgctcttttctcgggacgggagaggccgtgtagcgtcgccgttactccgaggagatacca
gtcggtagaggagaagtcgaggttagagggaactgggaggcactttgctgtctgcaatcg
aagttgagggtgcaaaaatg (Seq ID No: 564)

Homo sapiens lectin, galactoside-binding, soluble, 9 (LGALS9) :

atttctttgttaagtcgttccctctacaaaggacttcctagtgggtgtgaaaggcagcgg
tggccacagaggcggcggagagatg (Seq ID No: 565)

Homo sapiens chaperonin containing TCP1, subunit 5 (epsilon) (CCT5): cggtctccgccggttgggggaagtaattccggttgtt-gcaccatg (Seq ID No: 566)

Homo sapiens haloacid dehalogenase-like hydrolase domain con taining 1 (HDHD1) : cttcctcctcgcccccacccagacccagaaggcgccaccatg (Seq ID No: 567)

Homo sapiens glutamate dehydrogenase 2 (GLUD2) :

cttccttcctagtcgcggggagtctgagaaagcgcacctgttccgcgaccgtcacgcacc
cctcctccgcctgccgcgatg (Seq ID No: 568)

Homo sapiens general transcription factor IIIC, polypeptide 3, 102kDa (GTF3C3) :

ggttctctgtcccggttcctggggttgcacagacagaccctgtaaacatg
(Seq ID No: 569)

Homo sapiens general transcription factor IIIC, polypeptide 5, 63kDa (GTF3C5) :

gggtccctcgctggctagtaggagagactggtgcttgccccgcccggtggactaactcgc
ttaattttaaataaaaagtcgaggacacggcggtcgttttcccgaagacatgggccctcc
catgggccatttgctccctggaggccctcgcgtcttgctgagcccggggagttaggatga
cgcgagcggtgagggagcccggaacgattccttcgcggaacaattgaggcgaggcctttg
ggagtactttgtgggacggaccctggcgggccctgccagacgcacagggatg
(Seq ID No: 570)

Homo sapiens ancient ubiquitous protein 1 (AUP1):

ccgccttcccaagagcccctgcggccgggcgcgaaaatggcggcggcggcgacggccggg
cgctcctgaagcagcagttatg (Seq ID No: 571)

Homo sapiens coatomer protein complex, subunit gamma 2 (COPG2) :

cggccttcctgcagcctcttccgctcgccggctgcggcgcctgggacggttgcggtgggt
ctgggcgctgggaagtcgtccaagatg (Seq ID No: 572)

Homo sapiens apoptosis antagonizing transcription factor (AATF) :

cggtctctggcggagtcggggaatcggatcaaggcgagaggatccggcagggaaggagct
tcggggccgggggttggccgcacatttacgtgcgcgaagcggagtggaccgggagctgg
tgacgatg (Seq ID No: 573)

Homo sapiens integrator complex subunit 6 (INTS6) : tctcctctttctccaccacctcgggccccggtgtccccggccagcactatg (Seq ID No: 574)

Homo sapiens F-box and leucine-rich repeat protein 4 (FBXL4) :

tcttccttccgggtcgcgctaggccgggcttgcggcggttgtgccgcatctagagagtcg
gggagccgcccccgcacccaggccttctcgcgctgcctggtcgctggtgaagcccgcggc
gcgcgcctctcccggaccctgcagggtaaaagaatgtcacatgtcagcatttgtacctga
agtcagcatgcaaagttcagggtacctggatgaatgccaacttttgcatttcccatgtgt
atcctgtgaccattctatctgggaacatccttcaaagagttcatgcatcttactgaggac
acctgaccttttgaagcttcataattcacatctagatg (Seq ID No: 575)

Homo sapiens guanine nucleotide binding protein
(G protein), gamma 3 (GNG3) :

gctccttctagcatccttcatccttcaggtaccagccatccagacagtgcttgagctgca
gaaactgagaccagacctctggcctggccctccccaggggcctcctttcgtatagtcact
gcttctgcatcagatactttcagctgcaactccctactgggtggggcacccatttcaggc
agaaggttttggtaccctccactgaccctacacccagggctgctactgccgcttgtggct
tcaggatg (Seq ID No: 576)

Homo sapiens histidyl-tRNA synthetase 2, mitochondrial (putative) (HARS2): aggccttttgttcctgtcccggaaagccggcgtcctgccgcgcgatg (Seq ID No: 577)

Homo sapiens interleukin enhancer binding factor 3, 90kDa (ILF3) :

cctcctcctcctcttctcgccattgcagttggacccagcagcccggcgcgcaccgcgtgg
cttttgggggcagaccccggcgggctgtggcaggagggcggcggcggcggctgcggtcga
agaaggggacgccgacaagagttgaagtattgataacaccaaggaactctatcacaattt
gaaaagataagcaaaagtttgatttccagacactacagaagaagtaaaaatg
(Seq ID No: 578)

Homo sapiens polymerase I and transcript release factor (PTRF) :

gtttcctctgctctccgctctcgcccgctagctctcctcccttccgctcctgcttctctc
cgggtctcccgctccagctccagccccacccggccggtcccgcacggctccgggtagcca
tg (Seq ID No: 579)

Homo sapiens 5'-3' exoribonuclease 2 (XRN2):

tgccctctgccgctgctcccgtctctttggttacgctcgtcagccggtcggccgccgcct
ccagccgtgtgccgctatg (Seq ID No: 580)

Homo sapiens 2-hydroxyacyl-CoA lyase 1 (HACL1) :

ccgcctcttccttcccgttgtttaaggcagttggttgccctcctgtccgtcagaggtgca
gtaccagaggtggcgtgctgccgatttcgcgtttgccttgctggatgattccgcttgttt
gccggctgcgtgagtgcttagagcttttcggtggaagatg (Seq ID No: 581)

Homo sapiens zinc finger protein 346 (ZNF346) : ggctctctaccggtgagggtttgcggggaagatg (Seq ID No: 582)

Homo sapiens microtubule-associated protein, RP/EB family, m ember 3 (MAPRE3) :

cagtctctgtgcgttgaagccggagaccgcggcggcctcagcgaggaccctccgccccgg
agccgccggccggagccgcagcctctgccgcagcgcccccgccacctgtcccctccccct
ccgcctccgccggagccgcctcgtgcactctggggtatg (Seq ID No: 583)

Homo sapiens splicing factor 3b, subunit 3, 130kDa (SF3B3) :

gtgccttttccgccgcgcgccaccagaatgtccctgtcttgaggtctaatggcggacgc
cagtatgttggagttggtggtggcttaagttttgaagggaggtagcatccgttggatatc
cacaccatccttctcgctgcaggctttcttggactccgtactgttggtgtaaccaaggcc
tggaggtctgggtggctcaggtttcctgcagccatg (Seq ID No: 584)

Homo sapiens spondin 2, extracellular matrix protein (SPON2) :

ctgcctctcgctggaggccaggccgtgcagcatcgaagacaggaggaactggagcctcat
tggccggcccggggcgccggcctcgggcttaaataggagctccgggctctggctgggacc
cgaccgctgccggccgcgctcccgctgctcctgccgggtgatg (Seq ID No: 585)

Homo sapiens solute carrier family 13 (sodium/sulfate symporters), member 4 (SLC13A4) :

ttttcttttctgctttgcaggcccaggctcaaggcaaattataagtagggaaccaatttg

agggaaagacatgtgaacagagttaaggtaccacgtcctgggagcgaccagcagccccac
ctgaagtccgcatgcaactctgacaagctcaggtgcttgttttaaggaaaggggctacta
gagtcttaccaacagcgagcccaggtgggagatgaaacaggtactccccaaaataggtca
tccgagggaggaaaactgatggagagcacaatgtgctctgagcgtttttaatgtttttaa
gcttttaaatgatttcttcaaggccgagcagcagcagcaaaggtgtggcttaaaggatta
aggggtttctgctgacacctagaatgaagttactctattactaatcaagccgagaggag
gcccactatgcccccgtttatcatcctttcccagttcctttttgctggtcacaaaacgat
gctcatcaatcccacctaaagcaggaggccaggagcccagcctcttgtagaaacagcgag
ggtataactgccctcccgttctgcccccaagacgaaggaggactctcggaagccaagaaa
ggtttaagaagtctttctggatagagagcagtgcccaggcaggaagcctttcgccggcag
agcggggtccaaggacgagctggagaggacagaggcgcgatg (Seq ID No: 586)

Homo sapiens PRP6 pre-mRNA processing factor 6 homolog (S. cerevisiae) (PRPF6) : attcctttccttcctagccttggtcgtcgccgccaccatg (Seq ID No: 587)

Homo sapiens eukaryotic translation initiation factor 3, sub unit K (EIF3K) :

ccacctcttcctgttcccgtccttgaggacgccgtgccgggtcagtgttagcctccagcc
ctggttgtggaaggcgacagaagtcatg (Seq ID No: 588)

Homo sapiens ataxin 10 (ATXN10) :

ccccctcccccgcggcgccgtctcctcctcccgcctgaggcgagtctgggctcagcctag
agctctccggcggcggcgcagcttcagggcagcgcgggctgcagcggcggcggcggttag
ggctgtgtagggcgaggcctccccccttcctcctcgccatcctactcctccctcctcgtca
tcctccccccttcgtcctcctcgccttcctcctcctcgtcaggctcgacccagctgtgagc
ggcaagatg (Seq ID No: 589)

Homo sapiens secretogranin III (SCG3) :

cttccttcctcacttcctctgcaggagggagcgagagtaaagctacgccctggcgcgcag
tctccgcgtcacaggaacttcagcacccacagggcggacagcgctcccctctacctggag
acttgactcccgcgcgccccaaccctgcttatcccttgaccgtcgagtgtcagagatcct
gcagccgcccagtcccggcccctctcccgccccacacccaccctcctggctcttcctgtt
tttactcctcctttttcattcataacaaaagctacagctccaggagcccagcgccgggctg
tgacccaagccgagcgtggaagaatg (Seq ID No: 590)

Homo sapiens polymerase (DNA directed), mu (POLM) :

cttccttccgtctcgctcggagtttccctctgcgttcgctccgcgctgctggaggctgtc
gtcccaatg (Seq ID No: 591)

Homo sapiens epsin 1 (EPN1) :

cctccttctgttgcttcccgtctcctcggcggctcccctcccccgcccggctctccgcgc ccttctgggcggcggggcggcggagccgtcggcgtgcggccctccttgcgttcgtgcgt gcgcccgtggcccggcgcacgtcccgcgacaccgaggccgagcggggcaggggctgacc gccatgacccccagagcccggcgtgaggggccgagatgcggtgacctgccagcacctg ccgcagccttcgtccgggagtcgccccatctctccacgcatcggggccctgtgccccttg ctgctgcagccgggcaccatg (Seq ID No: 592)

Homo sapiens Sec61 alpha 1 subunit (S. cerevisiae) (SEC61A1):

gtgtctctcggcggagctgctgtgcagtggaacgcgctgggccgcgggcagcgtcgcctc acgcggagcagagctgagctgaagcgggacccggagcccgagcagccgccgccatg (Seq ID No: 593)

Homo sapiens Obg-like ATPase 1 (OLA1):

cgttctctcctccttcctccccgcctccagctgccggcaggacctttctctcgctgccgc tgggaccccgtgtcatcgcccaggccgagcacgatg (Seq ID No: 594)

Homo sapiens sorting nexin 12 (SNX12): aggcctctgtcccccacccccttttccccggtcccaggctctccttcggaaagatg (Seq ID No: 595)

Homo sapiens LAG1 longevity assurance homolog 2 (S. cerevisiae) (LASS2):

cggccttttttcccggctgggctcgggctcagctcgactgggctcggcgggcggcggcg gcggcgccggcggctggcggaggagggagggcgagggcgggcgcgggccggcgggcgggc ggaagagggaggagaggcgcggggagccaggcctcggggcctcggagcaaccacccgagc agacggagtacacggagcagcggccccggccccgccaacgctgccgccggctactccctc ttgatgccctcccctttgcccctcactcaggatg (Seq ID No: 596)

Homo sapiens cytohesin 4 (CYTH4): tcatcttttccccagaggcgtcggaatg (Seq ID No: 597)

Homo sapiens transportin 2 (TNPO2):

aattctctctctttggctccctccttccgcgcgagtctctggagaagccgcagcgcgagt tgccgccgctgctgcccggggccgggtaagtgggcctcactcagagcccgaccctcttgg ccccggcttgcgtcgacccccgccgggcaccgagcctgcgccgcgcgcggcccgggcgtc ggggccgcgcccgaccgggaaaggccgggaagccggttgggcccgatcctcctggcagct agaacgggccgggcgggggagggggggaaccgagcagagcttaggggggtggggcctcggag ccaggccatgtcggggctcctcaagaagagggccagtgggactgctggggtcggctgga ggggatctgattgggggaagcgtctggggactgcttggggcctgattgggggacgtcgcg aggatcggcttgccttgcgccatg (Seq ID No: 598)

Homo sapiens makorin ring finger protein 1 (MKRN1): gggcctttgctgtgtgggataaacagtaatg (Seq ID No: 599)

Homo sapiens vinculin (VCL):

ctgtctcttcgccggttcccggccccgtggatcctacttctctgtcgcccgcggttcgcc gccccgctcgccgccgcgatg (Seq ID No: 600)

Homo sapiens DEAH (Asp-Glu-Ala-His) box polypeptide 38 (DHX38) :

cctcctttcctgcccccagactagaggcgggatgtagtctcttaggctaagagtgattg
gtcacaaggagactcggaagtgtctgatcagagccccagaggaggccttgagagcctgtt
ggcgtaccgttccacacttggatccaggaatcgggcgtgttccaggctgctctctatggt
agctttgggcggatagaggggcgcgcaaagtattaagggacaataatggccgctttcaa
ggtgtggattttggctccttgagcctgtctgagcgaggggtggcagcgccggcgccccag
aatccgggacagaagggtcccaagagtcgcgcttggtgagagaaatcccagatcctgtga
tg (Seq ID No: 601)

Homo sapiens osteoglycin (OGN):

catcctctaagcttttaaatattgcttcgatggtctgaatttttatttccagggaaaaag
agagttttgtcccacagtcagcaggccactagtttattaacttccagtcaccttgatttt
tgctaaaatg (Seq ID No: 602)

Homo sapiens NIN1/RPN12 binding protein 1 homolog (S. cerevisiae) (NOB1): gctcccctctcacgcagccaacatg (Seq ID No: 603)

Homo sapiens nudix (nucleoside diphosphate linked moiety X)-type motif 5 (NUDT5) :

catccttttagcaccgcgagaggcgccggtgtttcgagccgtggcaccggcatcggctga
cactgctgcctccagctagttatttcgtcctcttccgttcttcacccctacaccttggag
gtgaacttctcacctgagggctgtaaagactcgtttgaaaatg (Seq ID No: 604)

Homo sapiens WD repeat domain 91 (WDR91): cgtccctcaccgcaccaccccctaaagacgctagcgctgcgatg (Seq ID No: 605)

Homo sapiens nuclear transcription factor Y, gamma (NFYC):

gggcctctgcattgcccgactccgtaggagcgcgggggcggctcctgctcttcctggact
cctgagcagagttgtcgagatg (Seq ID No: 606)

Homo sapiens protein phosphatase 2, regulatory subunit A, al pha (PPP2R1A) :

ccgcccttccttcttctcccagcattgcccccccccacgtttcagcacagcgctggccgca
gtctgacaggaaagggacggagccaagatg (Seq ID No: 607)

Homo sapiens vesicle-associated membrane protein 2 (synaptobrevin 2) (VAMP2):

ccatctttccgtcccgggcagccagcgccagtcggagccagcgcgagccgccgccgccat
cactgccgctgccaagtcctccacccgctgcccccgccatg (Seq ID No: 608)

Homo sapiens transmembrane protein 5 (TMEM5): gattctctttccgcccgctccatggcggtggatgcctgactggaagcccgagtgggatg (Seq ID No: 609)

Homo sapiens UDP-GlcNAc:betaGal beta-1,3-N-acetylglucosaminy ltransferase 3 (B3GNT3):

aactctttcttcggctcgcgagctgagaggagcaggtagaggggcagaggcgggactgtc
gtctgggggagccgcccaggaggctcctcaggccgaccccagaccctggctggccaggat
g (Seq ID No: 610)

Homo sapiens SEC11 homolog A (S. cerevisiae) (SEC11A): gcgccctttcccctgccggtgtcctgctcgccgtccccgccatg (Seq ID No: 611)

Homo sapiens RUN and SH3 domain containing 1 (RUSC1):

ctccctccccgcgccccgtcctctcccgccctacaggccctagcagggcaggcgggaggt

gagcgcggccatcccgctcccggagttccgggatcctggagtccgtagttcgtggtcctt
cgccggtgtccccggagcccagcggctgtggatg (Seq ID No: 612)

Homo sapiens aryl hydrocarbon receptor interacting proten-l ike 1 (AIPL1): cctccctttctcctgcagccatg (Seq ID No: 613)

Homo sapiens tumor necrosis factor, alpha-induced protein 8 (TNFAIP8) :

cctcctttctcccgccggctctaacccgcgcttggctaaggtccgcgggaacccgtgag
ccaccgagagagcagagaactcggcgccgccaaacagcccagctcgcgcttcagcgtccc
ggcgccgtcgcgccactcctccgatg (Seq ID No: 614)

Homo sapiens staphylococcal nuclease and tudor domain contai ning 1 (SND1):

gcgtctctttcgctccgtgtcccgctgctgctcctgtgagcgcccggcgagtccgtcccg
tccaccgtccgcagctggtagccagcctgcccctcgcctcgactccctttcaccaacacc
gacacccacattgacacctccagtccggccagccgctccactcgttgcctttgcatctcc
acacatg (Seq ID No: 615)

Homo sapiens DNA segment on chromosome 4
(unique) 234 expressed sequence (D4S234E):

cgccctcttttggtcgcccccctccccaacccagcactaaggagcaccctgctctggtctc
cgccaccacccagcgcctcctggacccatccccccaaacccttgaacgtcctcaggaccc
ccaggtgagcgcggcgcgctgcgggcggggaccctctctgcacctccccgcacccctggg
ggtcgctctgtccctacggtccccgcctcccctttctcctttctaagcgcctcgcgccca
ggccgccgcccggggtggcgcagcccgcagccctcccgctccgggcgccctccgccgctc
cgagacccccctgggggcgcgtcctctcccgctcccctgttccctcccccggctcagggcg
ggcgcgtggtcccaggggaggctcccgcccagccccgcactcctttgtgcggccgggcgg
gcgctgcgtcaaggtggaggcgcggccacacgcgcgcacccacccgcgcgcacccagccc
ccgggagaggcaggaagggaggcggcggcgcgaggaggagggagcggccgtggagcccaa
tcgttcgctccccttcccgggtccgcgcgcggcgccgcctccgccattgctgcgagcagg
agcaggagacgcggagctcggagcgctcagctgacctgccggagccgggcgtgggctgca
gcctcggagctcccggaacgatg (Seq ID No: 616)

Homo sapiens growth hormone inducible transmembrane protein (GHITM) :

acgtcctttcgatgttgcgtcatgcagtgcgccggaggaactgtgctctttgaggccgac
gctaggggcccggaagggaaactgcgaggcgaaggtgaccggggaccgagcatttcagat
ctgctcggtagacctggtgcaccaccaccatg (Seq ID No: 617)

Homo sapiens stress-associated endoplasmic reticulum protein 1 (SERP1):

tttccttcctctttcactccgcgctcacggcggcggccaaagcggcggcgacggcggcgc
gagaacgacccggcggccagttctcttcctcctgcgcacctgccccgctcggtcagtcag
tcggcggccggcgcccggcttgtgctcagacctcgcgcttgcggcgcccaggcccagcgg
ccgtagctagcgtctggcctgagaacctcggcgctccggcggcgcgggcaccacgagccg
agcctcgcagcggctccagaggaggcaggcgagtgagcgagtccgaggggtggccggggc
aggtggtggcgccgcgaagatg (Seq ID No: 618)

Homo sapiens ADP-ribosylation factor interacting protein 1 (ARFIP1) :

cggtctcctcacttccggcttcgctgctcttggttctggttctggaggctgggttgagag
gtcgccggtccgactgtcctcggcggttggtcagtgtgaatttgtgacagctgcagttgc
tccccgcccccgagcagccgaggagtctaccatg (Seq ID No: 619)

Homo sapiens tumor necrosis factor receptor superfamily, mem ber 21 (TNFRSF21):

ccgccccttcggcgccaccacgtgtgtccctgcgcccggtggccaccgactcagtccctc
gccgaccagtctgggcagcggaggagggtggttggcagtggctggaagcttcgctatggg
aagttgttcctttgctctctcgcgcccagtcctcctccctggttctcctcagccgctgtc
ggaggagagcacccggagacgcgggctgcagtcgcggcggcttctccccgcctgggcggc
cgcgccgctgggcaggtgctgagcgcccctagagcctcccttgccgcctccctcctctgc
ccggccgcagcagtgcacatggggtgttggaggtagatgggctcccggcccgggaggcgg
cggtggatgcggcgctgggcagaagcagccgccgattccagctgccccgcgcgccccggg
cgcccctgcgagtccccggttcagccatg (Seq ID No: 620)

Homo sapiens sushi-repeat containing protein, X-linked 2 (SRPX2) :

ccccctcttctgcagcagacggactgagttcctctaatccctgtgttccttctcccccat
ctttctaaaccccttctctgagagaggaataactatagcttcagggataatatagcttta
aggaaacttttggcagatgtggacgtcgtaacatctgggcagtgttaacagaatcccgga
ggccgggacagaccaggagccactcgttctaggaatgttaaagtagaaggtttttccaa
ttgatgagaggagcagagaggaaggagaaagaggaggagagagaaaaagggcacaaaata
ccataaaacagatcccatatttctgcttcccctcacttttagaagttaattgatggctga
cttctgaaagtcactttcctttgccctggtacttcaggccatatacatcttttcttgtct
ccataatcctccctttcaaggatg (Seq ID No: 621)

Homo sapiens HIV-1 Tat specific factor 1 (HTATSF1):

acctccctttctctgctcagctccagcgtcatttcggcctcttagttcttctgaaccctg
ctcctgagctaggtaggaaacatg (Seq ID No: 622)

Homo sapiens trafficking protein particle complex 2 (TRAPPC2) :

gggtctcttccgcggaaactgacattgcgtttccgttgtcggcctcccactgcaggagcc
atatattgaagaccatg (Seq ID No: 623)

Homo sapiens UDP-N-acetyl-alpha-D-galactosamine:polypeptide N-acetylgalactosaminyltransferase 5 (GalNAc-T5) (GALNT5):

ccacctttcttgggcttgtaggaaggtggacatggctcccggagacaagacaagtgat atgttgaactgttcggtggctggaatcaactgctcctggagtgacctaaggccagtgttt atcagaacttagccagggccagccaagcaggcacagatgctctgctatgaaatgccacgc aggcagagactgacaagcggtaggaactgagctttccccttggactgctgcttcctgctg tgttcaggggagggggtcactttctggcaactctgctgctgctgctgctgctgctgctac ttcagcttcctctccactcaaggtaagcaggctaagggagggcaggctgctagggaaagc tttgtaccatg (Seq ID No: 624)

Homo sapiens transmembrane protein 97 (TMEM97): tggcccctcttctcacatcagcgggtccaggcccaaccgacagactatg (Seq ID No: 625)

Homo sapiens EH-domain containing 2 (EHD2):

cgtcctccccgctccgggccccacccggctcagacggctccggacgggaccgcgagcaca ggccgctccgcgggcgcttcggatcctcgcgggaccccaccctctcccagcctgcccagc ccgctgcagccgccagcgcgccccgtcggcagctctccatctgcacgtctctccgtgaac cccgtgagcggtgtgcagccaccatg (Seq ID No: 626)

Homo sapiens tubulin tyrosine ligase-like family, member 4 (TTLL4) :

cgccctcttcttccagactctcggtctgtccgctgggggcgcgcgcggtgtgtggcaggc ggcagcggcgctggcggccgagtgcgcttgtcacgcgtggcggtgcgtggttgctagggg cgcctgaggctgccgggtagcccagcaggccgagggaggaagtagcgtggagccggtgcc gagccggggcgaagctggatcccctagatagactgtcttcaagctcactgatattttcct ctgcttgatccattgtgctgttgagagcctctagtaaattttcagactgacagacttca aggatgcagctgctactaccggaggtgtgtggcaccttacctcagcaaggccatgagacc gtgtggccatgatgtgggcccctcatg (Seq ID No: 627)

Homo sapiens basic leucine zipper and W2 domains 1 (BZW1) :

acctctccctcctcctggcgttagttccggtcgcagaggagacaccgccgcagttgccgg tacatcggggatttctggctctttcctcttcgccttaaattcgggtgtcttttatg (Seq ID No: 628)

Homo sapiens centrosomal protein 57kDa (CEP57):

ttgccctttctgtgtaagctgtgagcgtaggcggccctgaggggggtgtgttgcaggggtt tccaagcccagcaccagcacccttgccctttttccatcaggggttcagcctagggtccccg ctggtgggcggctcccgagtcttggagaagagcacgagaacctagaccgcccccgaagtg cggagacccccctgggcaggctgaaagatg (Seq ID No: 629)

Homo sapiens family with sequence similarity 115, member A (FAM115A) :

ctgccctttgcctcctgggcggagaagctgcttcctcctgggaacaaccgcctcccgctc ctagcaggttgctactgccccgaacccgcgctgcagggaacagcggggcaaacagtgagt ggggttcagcgtagactctggaccaggagaggcccgcggtgaccgaggcctgggccccgg aaaccaatagagccatg (Seq ID No: 630)

Homo sapiens ATG13 autophagy related 13 homolog (S. cerevisiae) (ATG13):

agccctctttcacccccccccccccggccattaccgaagcggatgaaaacaaacactaacg atggcggcgccgggaagcgaccggctgctgggcttaaggcgggagtgaccgcttaaccag tgagggaagcactgaagagcgccagtcgacgtgggtgcgacaactcgcggagtcttagga gcaaaacgtctggggcctgcgagccaggacccttctgaagccttaggtgtctatcggcga cgtgtacggtcactgcagctccggagcgcggaaccctcagccaggaggcgcggctggtcg gtcccaggtcccggcctccgtaatgagagcccggaaccactctttgtgccgcagcttcgc agcatcttggactcaagtgattctcctgcctcagcctcctgagtagctgggactacagat tcctataggcaatg (Seq ID No: 631)

Homo sapiens sorting nexin 17 (SNX17):

ccgccttcccacatcggatcgcagggctcccaaaatggcgagtgaggctgcggggactcg ctgagcagcggagggggagcgtgcagagccgctgcggccctcacagtccggagcccggcc gtgccgtgccgtagggaacatg (Seq ID No: 632)

Homo sapiens phytanoyl-CoA 2-hydroxylase interacting protein (PHYHIP) :

cgttctttctcccttctctgcctctctcctccacgctgctttgatttcgctcttgcct ctcttcttgcgctgctcagctgggaacatcgtctcaccaggggcagcagcgacgcgctgc acagccagacaggagctggctgcggggcatggaagcagcctccttggcagccgggagagg agcaagcgcacgccactgcccgtgacccaggcgtccggctgctgtcccctgccggggagc tcatccacgcagaggtctctccctgtcctccctgcgagcttttcctctgcagagcccagt ggagccagtccccacaggagacaaccctgacgggagcatg (Seq ID No: 633)

Homo sapiens translocase of outer mitochondrial membrane 20 homolog (yeast) (TOMM20) :

cggcctttctgtgttcctggcccgcggccgtcgggtgtgagctgcgccgaccgctctgag ggttcgtggcccaccgctccttcgcggtccctgccgccaccgtccacgctcagcgttgta gagaagatg (Seq ID No: 634)

Homo sapiens KIAA0141 (KIAA0141):

cggcctttctagccgctgtcccaagggttggtctcgcgctttcggctgcgagctctctgt ggtgctggcagcgacatg (Seq ID No: 635)

Homo sapiens janus kinase and microtubule interacting protei n 2 (JAKMIP2):

ctccctcctttaaacagcttctccgggtctcagcatgggcttccagggcagcgattgagg agaccttaccaaggagcaccacacagtagatgctgagacatcgtactccaggataagaaa cagtaacatggcagcacctgcttgaaagaaattaaaaaccaacagactccatttagaaag gaacaatg (Seq ID No: 636)

Homo sapiens EPM2A (laforin) interacting protein 1 (EPM2AIP1) :

cctcctctccccttgcggcctttctaacgttggccctgctcttgtggcctcccgcagaat g (Seq ID No: 637)

Homo sapiens centrosomal protein 170kDa (CEP170):

cggtctttgccgttaccgctatgtgtggggcgtgtgtggaataacgttattgcccagcgg agctgagggccccggagctcgaccgcagcggcagcgacgacaacagcggcgacgacgacg acgacgaggtgggggaggacggcgtgcgagagactcacgggacgcgacgcgccccgcct cccccgtccggtccctctctccacggtaaggggatgacgtagctttgccaaagacttaga agctaagcagaaaatg (Seq ID No: 638)

Homo sapiens suppressor of Ty 7 (S. cerevisiae)-like (SUPT7L) :

aggcctctcgaggtccagacagccgcccagcccgctctgcgacgcagcagtgaatagtgt ggtacctccttgtctcggttcaggtccagacctccccgtcttccggctgccctgaacgtc aggcgacctcaggaccctgtgattggcgcctgcgccggcggaccgtgaccgaggaaaccc ctggagggacttgggcattccttgggctccgtgcctgttcttcgtgctcctttcgggcaa

ggatctcacattatcagtctttgaccgacacagaatgcctggcatttgataaatgtttgt tgaacttgaagagacatatggacaatg (Seq ID No: 639)

Homo sapiens non-SMC condensin I complex, subunit D2 (NCAPD2) :

ttttccttttcatttcagcctgactgccggaatcagagccgcgggtgagatccccagccc tgtgagcctgtaggagtagaatg (Seq ID No: 640)

Homo sapiens ring finger protein 10 (RNF10):

ggttctttgagatgctgtttggcgactcgtcgccattcccggagcaggtcggcctcggcc caggggcgagtatccgttgctgtgtcggagacactagtccccgacaccgagacagccagc cctctcccctgcctcgcggcgggagagcgtgtccggccggccggccggcggggctcgcgc aacctccctcgcctccccttcccccgcagcctccgccccgccaggcccggcccggactcc cgagccccggcctcctcgtcctcggtcgccgctgccgccgggcttaacagccccgtccgc cgcttctcttcctagtttgagaagccaaggaaggaaacagggaaaaatgtcgccatgaag gccgagaaccgctgccgccgccgacccccgccggccctgaacgccatgagcctgggtccc cgccgcgcccgctccgctccgactgccgtcgccgccgaggcccccgttgatg (Seq ID No: 641)

Homo sapiens PAN2 poly(A) specific ribonuclease subunit homo log (S. cerevisiae) (PAN2):

agcccttcttgattggaagaagcgcctcggaccccggtccttggcgccgtagtggttagg ttgagccctaggcgtggggagaactggggaaactggaatttcccgcggagctgacagcg cttgcgctcccccctactcgttctaattccacgcgctccaaaatatccgccatggagaaat cttggccaggatgtccattctaggcccatcggtgctgtcttgctgaaggttgggtcaggc atctaaagggactgtggtaagggagggtgtgacacaggtgtaagctgccatcgtcatcat g (Seq ID No: 642)

Homo sapiens CD302 molecule (CD302): gctcctctccggccgcgcagccgctgccgcccacccgcacccgccgtcatg (Seq ID No: 643)

Homo sapiens NSA2 ribosome biogenesis homolog (S. cerevisiae) (NSA2):

gactctttcctgtcccggcctgcgtggtgtgggcttgtgggtctttgagacccgaaaatt gagagcgttttcgcactccagcggctgctcctggcggctctgcggccgtcaccatg (Seq ID No: 644)

Homo sapiens DIS3 mitotic control homolog (S. cerevisiae) (DIS3) : acgccttttgctggaagagcgctgctggggttaggattctgcgcggcgaggcaagatg (Seq ID No: 645)

Homo sapiens caspase recruitment domain family, member 8 (CARD8) :

cctcctctgcgagcgttatttcaaaagaagttgagaaccagagaaaccgacctaaggga ttctcccatttggcccgtcctaccctaaagtcaccacctgctgcttttctggagcgctta ccagtgaccaagaggaacagaacacagagcagcctggcagtgtccaagcaacaagcctcc gctcctccttcctgcaccctggggctcctgaaactcacatgggtaaaaaagatacagtaa agacataaataccacatttgacaaatg (Seq ID No: 646)

Homo sapiens epsin 2 (EPN2):

ccgcctctcgagcgctgccggtggccgcagcggcgcacccacgccggcccggaggagcag agtgttcatttctgtgtcgggcacagtgctaagtgctgggtgctcactggtgatgaggca gatgaaggttaccaaacttgtggacaggagcctcatatcagagacgtggacctcactgta gcctggtcatggcttccagcttttcgaatctgaggctccaaaggaggaaatgaccattca gggatcttactccagcttgattacggagactgaaccttcatagggtgcgcacttaccaag gacaggaaggtttctctgtttgaagggctttaaacttataacaaagaaaataaaaatg (Seq ID No: 647)

Homo sapiens pyridoxal-dependent decarboxylase domain contai ning 1 (PDXDC1):

ccgcctctcaaccatcaggttcggcagcccgcggcgccgcctggcagctcctcctcttct ccgccccgccggccgcgggcgcgggggacgtcagcgctgccagcgtggaaggagctgcgg ggcgcgggaggaggaagtagagcccgggaccgccaggccaccaccggccgcctcagccat g (Seq ID No: 648)

Homo sapiens nicotinamide nucleotide adenylyltransferase 2 (NMNAT2) :

ccttcctttctccctctgcagacacaacgagacacaaaaagagaggcaacccctagacca ccgcgaaggacccatctgcaccatg (Seq ID No: 649)

Homo sapiens mitochondrial ribosomal protein S27 (MRPS27): tgttccttttggtacgctccaagatg (Seq ID No: 650)

Homo sapiens leucine-rich repeats and calponin homology (CH) domain containing 1 (LRCH1):

tcccctccttccagcgcctttcggtggagcactgcggcactcagcccgagctgccgtttt cccctcgcggggaacgctgtgaccccccgcaggagcggcggggcggggtgggggggccc gggagaagatg (Seq ID No: 651)

Homo sapiens PAS domain containing serine/threonine kinase (PASK) :

gctcctttccgtggtgtgtagccggcttggcgtgaccctcgcctgatccagttgttagag
ttggaagcttggcagttggcctcccttcttcccatg (Seq ID No: 652)

Homo sapiens megalencephalic leukoencephalopathy with subcor tical cysts 1 (MLC1):

cttcctttcctagttgggttctgacagctccgaggcagtggtttacacaaccaacacgaa
acatttctacgatccacccgattcctcccctcattgatattcaggaagcagctctccttc
ccctgccttcagctcaagtttgctgagcttttgtttcatttgtgaatacttcttgctgga
agtccctcacccagagaccagtgctcccaacggcagagcagcgggggagataaagaactg
gtgacacgtggctgtacattcagcacagctgtggtgtccccaagtgccatg
(Seq ID No: 653)

Homo sapiens RRS1 ribosome biogenesis regulator homolog
(S. cerevisiae) (RRS1):

ctttcttttccggattgggcatcccggcatctgcacgtggttatgctgccggagtttggg
ccgccactgtaggaaaagtaacttcagctgcagccccaaagcgagtgagccgagccggag
ccatg (Seq ID No: 654)

Homo sapiens formin binding protein 4 (FNBP4): cgctctctgctcgcgcttgggctcgcgatg (Seq ID No: 655)

Homo sapiens peptidylprolyl isomerase domain and WD repeat c ontaining 1 (PPWD1): gcgccttttctgacgatgcgaacaacatg (Seq ID No: 656)

Homo sapiens sorting and assembly machinery component 50 hom olog (S. cerevisiae) (SAMM50):

ccgccttctgccctcagcagcagacgctctgtcccgcccgggcagctctgcgaggcagcg
gctggagagggaaccatg (Seq ID No: 657)

Homo sapiens Yip1 domain family, member 3 (YIPF3):

gcttctccttttgtgttccggccgatcccacctctcctcgaccctggacgtctaccttc
cggaggcccacatcttgcccactccgcgcgcggggctagcgcgggtttcagcgacgggag
ccctcaaggacatg (Seq ID No: 658)

Homo sapiens tectonin beta-propeller repeat containing 1 (TECPR1) :

caccctcttgcccggtccccgggagggccggtccgctcctcccggacgccgaggacctac
caccgcgacttcgccccgcccggcgcgggcccaggaccctgatgtcgcttttgaacagcc
cctgcacctggcagccagcgagctactgtagtaggcattgccgactgtttgcataccgga
tgggagtgacagtgtaatagaaaaacaagcaagaaacctttaggtaggactcctaaggc
tcagaggaagttacctccagccgctgccatg (Seq ID No: 659)

Homo sapiens DDB1 and CUL4 associated factor 12 (DCAF12):

ccttccctttcccggctcaagtccttcctctctctttcctttctttccgcctatcttttt
tctgctgccgctccgggtccgggccattttccgggccgggcgcactaaggtgcgcggccc
cggggcccagtatatgacccgccgtcctgctatccttcgcttcccccgccccatgtggct
gcggggccgcggcggcgctgcccactatg (Seq ID No: 660)

Homo sapiens chromosome 3 open reading frame 17 (C3orf17): ccgcctttcgtaagtccccccgcctcgcatg (Seq ID No: 661)

Homo sapiens LETM1 domain containing 1 (LETMD1): caacctcttctctcccgcttctctcgctgtgaagatg (Seq ID No: 662)

Homo sapiens chordin-like 2 (CHRDL2):

ctcccttctgctggaccttccttcgtctctccatctctccctcctttccccgcgttctct
ttccacctttctcttcttcccaccttagacctcccttcctgccctcctttcctgcccacc
gctgcttcctggcccttctccgaccccgctctagcagcagacctcctggggtctgtgggt
tgatctgtggcccctgtgcctccgtgtcctttcgtctcccttcctcccgactccgctcc
cggaccagcggcctgaccctggggaaaggatg (Seq ID No: 663)

Homo sapiens CCR4-NOT transcription complex, subunit 10 (CNOT10) :

actcctctagccggaacctgggggcccggagccggggtaggcacagagttgtcctcggag
gtccaggacagcggccagcccggcggcgggagtcagggccacgccacctgcagggaagaa
cccgagtcgaagcgggaagatg (Seq ID No: 664)

Homo sapiens THUMP domain containing 3 (THUMPD3):

cttcctcttgcagttgaggccggcgccgagccggacttcaggcggatctcgtggcggagc
ccatcttgctccctctcccaggcctttacccgctccctaggattcccgggccctgtaggt
gggagttgggagacgacagtactgcttttaaagagacagtgttagggatcttggaagcac
agccaacatg (Seq ID No: 665)

Homo sapiens nipsnap homolog 3A (C. elegans) (NIPSNAP3A):

gctcctttccactcgggaaaccttcagaggagtctcagaaaggacacggctggctgcttt
tctcagcgccgaagccgcgccatg (Seq ID No: 666)

Homo sapiens CAP-GLY domain containing linker protein 3 (CLIP3) :

gcccctccctctccgcccccacccccctgtcggcgtctgggcctcgtccccttctctctgt
ctcccttgcctcccccatcacgtcccctgacaccgacaccccattgctcccacagtctcc
ccagtctccactttggtccccagcgctgtctgcccgaggatttgcctgaaggctgccccc
aactctgcacccgccccccgagggccaccgaggaccatg (Seq ID No: 667)

Homo sapiens ring finger protein 167 (RNF167):

caccccttcccgaagtttttctgtcacctgtgttaggctccgtcccctttccgcgttttat
ccccgtaccagaaaaggatacatttagtgcctcccacccagctccactaaacgggttgga
tatctcattctttgagttggtgttccttccccggcgcccccatgtagctgggaagtggga
cctggggggtggttggacccctgggatcctaaaggagggggcagggaggcgcagaactccg
cttctgctccttgctaccaggacgcgcggcctcctcagcctctttcctcccgctgccatg
(Seq ID No: 668)

Homo sapiens polymerase (RNA) II
(DNA directed) polypeptide M (POLR2M):

cgttcttccgggaaaatggcgactcccgctcgtgccccggagtcaccgccgtccgcggat
ccggcgctagtagcggggcctgccgaggaagccgagtgcccgccgccgcgccagcctcag
cccgcgcagaatg (Seq ID No: 669)

Homo sapiens dihydroxyacetone kinase 2 homolog (S. cerevisiae) (DAK):

tcgcctctttccgccagcgcccgcaggacccggatgagagcgcacgcttcggggtctccg
ggaagtcgcggcgccttcggatgtggcggatgcggccgtgagccggcgggggaggtgctg
ctgctgcctccactgtactcagacccaggtagcacaggattgtccatcctccagcagctc
agtgcaacggtgtgaactcagcctgtttcagagcctccacaccatg
(Seq ID No: 670)

Homo sapiens RNA polymerase II associated protein 1 (RPAP1):

cgatctctgcggggcaagatggcggcgcccagacaggcctggagcacggatgaataagag
ggaacccccacacggagacactgctggagagagtcgtactggggaggcagctggagcagc
aagatg (Seq ID No: 671)

Homo sapiens torsin A interacting protein 1 (TOR1AIP1):

cctcctctttggtgcctccagccaggaggcgggagcgatccacagcagctgacccagctc
aggcactgcctctctcacagccctcaagacacaccatgggcccagaggcaggtttgctac
acagcagcgacgacgcaggcggcggccccagcgactcgcaactgcctccctgaccacagc
ggccaccgcccaacacccccgagaagccatcgccaccaccggcaggagaacctagggtcc

ataaagccatcttcgcgatcgactaaagctacgtcaacaactatg
(Seq ID No: 672)

Homo sapiens SERPINE1 mRNA binding protein 1 (SERBP1):

ccccctctctcggcccggccatcttgtgggaagagctgaagcaggcgctcttggctcggc
gcggcccgctgcaatccgtggaggaacgcgccgccgagccaccatcatg
(Seq ID No: 673)

Homo sapiens N-acetyltransferase 9 (GCN5-related, putative) (NAT9): caccctttctgcgggggacgatttcgtcggtggtaggctgctaccatg (Seq ID No: 674)

Homo sapiens ribosomal L1 domain containing 1 (RSL1D1): gcgcctcttcacgaggtggaaacaagatg (Seq ID No: 675)

Homo sapiens SH3 domain containing, Ysc84-like 1 (S. cerevisiae) (SH3YL1): cttcctcttcctgggcagcctcgggacggggcgccgcggccgggcgggcagcatg (Seq ID No: 676)

Homo sapiens methylmalonic aciduria (cobalamin deficiency) cblD type, with homocystinuria (MMADHC) :

acttcctttgcctgctcaccgccagcgtaggtgctaccaccgctgccgtcgccgccgcca
ttttgatggcaggaagagtccggttctgggacagctggagacagtggtggtgactgaaat
aactttaccaaaggaaagctattttgcgaactatcttctccagcggagatg
(Seq ID No: 677)

Homo sapiens glioma tumor suppressor candidate region gene 2 (GLTSCR2): agttcttcctttgacaagatg (Seq ID No: 678)

Homo sapiens DDB1 and CUL4 associated factor 8 (DCAF8):

cagtcttctcgagcacatcgtcgcaaacggggccggaaagcgtggcagcgcaggcgcaag
cgcagagagcggaggcggtggtggtggcggccgctggccagttccttcagtgaatctaca
gacctatttctcaggagctcagcctggccttacttcagtgataaaggaggaaaggctg
gctacagcaaacatcattcaagatg (Seq ID No: 679)

Homo sapiens UBX domain protein 1 (UBXN1):

ctttcttctcgtcggtgttcccggctgctatagagccgggtgagagagcgagcgcccgtc
ggcgggtgtcgagggcgggttgcctcgcgctgacccttcccgccctccttctcgtcacac
accaggtccccgcggaagccgcggtgtcggcgccatg (Seq ID No: 680)

Homo sapiens antizyme inhibitor 1 (AZIN1):

ccgccttctcacactttcaggctctgatcgcggccgcagtttttcctttttcttctgcc
gtcgccttctctgcctcttctcatcctttctcgctctgctgctctgcagtgtgacgagtc
cgaatcctcttcccacccagcccgcgcctttcttcttttgcctgcgctgttctatttctc
cttcggccgccgccgccactgctgcacacagctggtgtcggtgccgcgcttttacccccca
agtcgttcccgcagcctatggcccaggccgccttgggtatttctgctcaaggtaaccaca
tccctctttaaaaattccgccgaaaaagagaagacgctttacccgactctttgggccgtt
atctcacggcgaactttctgaccaagtatacaactacccagagggcctaggagaagtgct

gtatagagagcagttcgacttcaacgctgagccaccttgggaacctagctgatgataggg
gggttccatctcccaacttgtccatggaggtcttcacttcagaaatccaagactcatatt
catccagcttggtgtcaagtgggctgttgctgccagaattatcttgtgattatttgagag
atgtatcagtttcttctgaagtacaatcaactgtagaagcctttgtagcagtttgttgca
tattctaaggacccagacataggcttggtggcccgtctcttgtctttcctggtttatgac
tttcggctttgtggaatacggctgagatg (Seq ID No: 681)

Homo sapiens cell division cycle 40 homolog (S. cerevisiae) (CDC40) : gcctcttcttcttccgccctggcagggtctccgcagaagatttgttgccgtcatg (Seq ID No: 682)

Homo sapiens stathmin-like 3 (STMN3): gcgcctctccagcctccgcaggcccaaccgccgccagcaccatg (Seq ID No: 683)

Homo sapiens nudix
(nucleoside diphosphate linked moiety X)-type motif 13 (NUDT13) :

tttcctcttttgtgctgattcctgaggactaggaaggtgccccgaaagaattcagagac
ctgacaatg (Seq ID No: 684)

Homo sapiens calcium homeostasis modulator 2 (CALHM2):

ctctcttttctggagttagattagtctgaagccgccaccagccccaggcccccgtgcaga
agaaaagcgggagggaacggcggaggccgccgctgccctgcaccgccctcctggaggcca
cttggagagtccggccccgaggaggccatggccacaagtgcccacagctggccccaggtt
gccagcgtcgctacagcccagaccaaggcagaataatctccggatgagctggtggcaccg
ctgagcctttggtctcaccagggcttcctgttgctggcaggcggggtggagcggagctgc
tgggaggctgctggataggagaggggtcacggctgcggaagaggaggttcttcgggacac
ccgtggatggacacggcaaggaaacaccaggccaaccacagctggggataaaatagcaca
accacaccctgccgtccagcgcctcccagcctgtgccccttcctagtaccaccagcaacc
atcaatcccgtctcctcctgcctcctctcctgcaatccaccccgccacgactatcgccat
g (Seq ID No: 685)

Homo sapiens NMD3 homolog (S. cerevisiae) (NMD3):

tcttctctgtggcggagacagccaggttggcagctgacggacagccggggtctatttg
ttgcgggttttcagcaaatccagggctggtctggaggcgcgaaaacttaaggcatacaga
acgatg (Seq ID No: 686)

Homo sapiens ATPase, H+ transporting, lysosomal 50/57kDa, V1 subunit H (ATP6V1H):

gcgcctctgtcattctactgcggccgccctggcttccttctacctgtgcggccctcaacg
tctccttggtgcgggacccgcttcactttcggctcccggagtctccctccactgctcaga
cctctggacctgacaggagacgcctacttggctctgacgcggcgccccagcccggctgtg
tccccggcgccccggaccaccctccctgccggctttgggtgcgttgtggggtcccgagga
ttcgcgagatttgttgaaagacattcaagattacgaagtttagatg
(Seq ID No: 687)

Homo sapiens DPH5 homolog (S. cerevisiae) (DPH5):

gggccttttctctgcacggagccggcgcttttgcagttgcttctgcggaaaggtggtagt

taagaatttgtaaaggccagagaactacctacgattctctcagcggtctctcttctcctc
aagtttgaaatg (Seq ID No: 688)

Homo sapiens polymerase (RNA) I polypeptide D, 16kDa (POLR1D) :

cctcctccctccttccgtcctccgcgccttccgtcggtcggtccttgcttcctgcttcgc
ctccgcgcctcgcgctatgggacagagcccccgatccgccagcaccacctgaggatccag
aaaccgccccagcgatg (Seq ID No: 689)

Homo sapiens HMP19 protein (HMP19):

ctgtcctttcagcaccacaagctcgggctgaggagggaggactcctggccgtcctcctcc
tcttcaaattggcttgaatcttctctgaccccccacgagtgcagcacagtctgggaagaa
aggcgtaaggatg (Seq ID No: 690)

Homo sapiens adiponectin receptor 1 (ADIPOR1):

gcgccccttccggcgcggggagggcgctgaagatcggggccgctcggccgcaggccgcct ccagcgccgcgggatgtagcgcgggggaccgcggcccccagcagagcccgcctgcccggc ttgtctaccatcagagggagatctctgccccctggggctgagagaccccaacctttcccc aagctgaagctgcagggtattgaggtaccagccagatg (Seq ID No: 691)

Homo sapiens SH3-domain GRB2-like endophilin B1 (SH3GLB1):

ttttcccttgggacccgggtccacacggcggggtcgcccgtccatctccggctcgcccgc ggggcccatcgtcgacgttagcggccgttctccgagccgactgacccatccttggcgctg ccgccgcgcgcttgttctcctccctcgccccgccttcatcctccccgttcacggaaacga cagctgcggctgcggggctggcgccgcctccctccacctaccacgtctgccctcgccgct ctagccctgcgccccagcccggccgcggcacctccgcctcgccgccgctaggtcggccgg ctccgcccggctgccgcctaggatg (Seq ID No: 692)

Homo sapiens anterior pharynx defective 1 homolog A (C. elegans) (APH1A):

gtcccctcttcggcttccgtagaggaagtggcgcggaccttcatttggggtttcggttcc ccccttccccttccccggggtctgggggtgacattgcaccgcgcccctcgtggggtcgc gttgccaccccacgcggactccccagctggcgcgcccctcccatttgcctgtcctggtca ggcccccacccccttcccacctgaccagccatg (Seq ID No: 693)

Homo sapiens RNA binding motif protein, X-linked 2 (RBMX2): ctgcctttcccgggcgctgattcctgagtgctgagcgcgaacccgaggagatg (Seq ID No: 694)

Homo sapiens family with sequence similarity 82, member B (FAM82B): atctcctttagccccgcccgcctccgtagctgcctgaagtagtgcagggtcagcccgcaa gttgcaggtcatg (Seq ID No: 695)

Homo sapiens UTP11-like, U3 small nucleolar ribonucleoprotei n, (yeast) (UTP11L): tgatcttttccaaggctgtacagacatg (Seq ID No: 696)

Homo sapiens chromosome 14 open reading frame 166 (C14orf166) :

cgccctctcgccgcgtcgccggtgcctgcgcctcccgctccacctcgcttcttctctccc ggccgaggcccgggggaccagagcgagaagcggggaccatg (Seq ID No: 697)

Homo sapiens transmembrane emp24 protein transport domain co ntaining 5 (TMED5):

gcttctctttcggagggagtgttcgccgccgccgcggccgccacctggagtttcttcaga ctccagatttccctgtcaaccacgaggagtccagagaggaaacgcggagcggagacaaca gtacctgacgcctctttcagcccgggatcgccccagcagggatg
(Seq ID No: 698)

Homo sapiens coatomer protein complex, subunit zeta 1 (COPZ1) : gtttcttttgcggctccacgtcggcaccagctgcggggcaagat (Seq ID No: 699)

Homo sapiens mitochondrial ribosomal protein S16 (MRPS16):

ggttctttctgtgtttgttctctgccctgccaaggccgtagagctggtgcgtgcgggtag cggggctctccgaggagccgcacgccggcggcaccatg (Seq ID No: 700)

Homo sapiens charged multivesicular body protein 3 (CHMP3):

ctacctcctttccgcgggccccgcccaggcggctgcccgtgacctgcctgggcgcgggg aactgaaagccggaaggggcaagacgggttcagttcgtcatggggctgtttggaaagacc caggagaagccgcccaaagaactgatatccaaagagaagaagaaaaagtgaaacgatctg tgaaagatgctgccaagaagggccagaaggatgtctgcatagttctggccaaggagatg (Seq ID No: 701)

Homo sapiens RNA binding motif protein 7 (RBM7): cgaccttttggccaggttagggagggggcgacgctgagatg (Seq ID No: 702)

Homo sapiens eukaryotic translation initiation factor 3, sub unit L (EIF3L): cgctctttccggcggtgctcgcaagcgaggcagccatg (Seq ID No: 703)

Homo sapiens zinc finger protein 706 (ZNF706):

ccttcctttccctccggcgtcctctcccggccctctcgcgctgcactgtctctccgacgc aagactgtcccggcccggatatg (Seq ID No: 704)

Homo sapiens androgen-induced 1 (AIG1) : cgccctccttgccgcccagccggtccaggcctctggcgaacatg (Seq ID No: 705)

Homo sapiens interleukin-1 receptor-associated kinase 4 (IRAK4) :

cgccccttcgcggcgcttcctagttcggctggttcttctgtcgccggcttcagcagcccg cgcccgggcaggaatagaagatg (Seq ID No: 706)

Homo sapiens transmembrane protein 66 (TMEM66):

cgttccttcgccgccgccaggggtagcggtgtagctgcgcagcgtcgcgcgcgctaccgc acccaggttcggcccgtaggcgtctggcagcccggcgccatcttcatcgagcgccatg (Seq ID No: 707)

Homo sapiens carboxypeptidase Q (CPQ):

ccgcctctcggccccgcggcctggccggcaagcagggctgcagtcacggggcggcgcgga gggccccagcccagtcaggggtgtggccgccgccaccgtaaggctaggccgcgagcttag tcctgggagccgcctccgtcgccgccgtcagagccgccctatcagattatcttaacaaga aaaccaactggaaaaaaaatg (Seq ID No: 708)

Homo sapiens hydroxysteroid (17-beta) dehydrogenase 12 (HSD17B12): cgctcttttcattcacgaaggtagtgaggcctagtggaaagccatg (Seq ID No: 709)

Homo sapiens protein phosphatase methylesterase 1 (PPME1) : cctcccctcgatg (Seq ID No: 710)

Homo sapiens HemK methyltransferase family member 1 (HEMK1):

ccccctttccggcaggctactgggctccgcccacacacctcccggcctggttcctaaacg
ccagctcggagcaatccccttgggctggagccaaatccctgctgtgatttaaggaagac
cggcaggtccgggcccccaagggtcaaccccacacacatccccgcactttcctgtatgca
ggcctgcgagcgtagagggagtggaattcacagcctccccacccatccgcaggggtctcc
tgggaggaacccaccagcgataggaacactgaagctgggctacggcgtccgcccgagcct
tttcttaaaggcgccgaccccggaagcggggcgtccgagggagcgcgcgacgggccacgc
acgtccgggcgtccagttcggggcagcttctccggctggtgggtgggtggggcagccttt
caggcagggtggcaaccaactatatctgaggaccagagccattttggggcaccagagctt
gtgacctctccatctccacccagctgggtccaggggccactctcagcactcacctcagca
gctgacatcataaagcagacttgggaacctggaagcactctggagaacctttccctgaga
catg (Seq ID No: 711)

Homo sapiens N(alpha)-acetyltransferase 38, NatC auxiliary s ubunit (NAA38):

cgccctttcagttctgcttgctgtcggcaccgctgcgttacccggaaccgccgggccgaa
cagcatg (Seq ID No: 712)

Homo sapiens cleavage and polyadenylation specific factor 3, 73kDa (CPSF3):

ggttcttcctttttatttaccggtggctgtgcttccaatttaggaagaccccggcgacc
tgttcctcacccccgcttcgccctcacactttcgggatg (Seq ID No: 713)

Homo sapiens dynactin 4 (p62) (DCTN4): tcgcctcctccctccccaagatg (Seq ID No: 714)

Homo sapiens hydroxysteroid (17-beta) dehydrogenase 11 (HSD17B11) :

gttcctccttgctctcgcccctactctttctggtgttagatcgagctaccctctaaaagc
agtttagagtggtaaaaaaaaaaaaaacacaccaaacgctcgcagccacaaaagggatg
(Seq ID No: 715)

Homo sapiens YTH domain family, member 2 (YTHDF2):

tagtctttccaggtgttagtcgaaacctcgtggtgcgaccctggtcgtcccaaacccccct
aggccttaatcctggggcggtgggggcggggaggccgtgagcacggcttccgctcctcca
atccgccagagggcgcagcggccggcctctcccttcccggggttcttcgcgccggcccc
ttccgcgtgggtgagtgaatgtgagagtcagcgctcgcgccgcgcgcgccgcccgcctcc

gctgttcggcgctctgctttaggcggtgggggcgggcgcgcgcgtaaaagcatagagac
gggcattgagctcttgggctagagcgtcgccgagtcggagccggagcctgagccgcgcgc
tgtgtctccgctgcgtccgccgaggcccccgagtgtcagggacaaagcctccgcctgct
cccgcagccggggctcatctgccgccgccgccgcgctgaggagagttcgccgccgtcgcc
gcccgtgaggatctgagagccatg (Seq ID No: 716)

Homo sapiens tubulin, epsilon 1 (TUBE1) :

agctctctagcagagcgccgttgctggggggaatgcagaagcggccgcgggctagcaagct
cccggagccggcggcgcaccaccatg (Seq ID No: 717)

Homo sapiens ubiquitin interaction motif containing 1 (UIMC1) :

cctcctttcttcctcagcgggtccgcggcccgctactctccgggaggggcgcttcccga cgccaaggtaggcctctcccgacgccggggcggcccttcctgatgccggggtgtgtctct cgcgacgcgggggtgggctccggacgccggggctggccttgccgaagtcgggggtgggtc cctccggacgccgaagtgggctcgggatgcggggctgggaccctcccgattccggggcgg attccggacgccgggaccggccattactggtgccgggttgggcttctccagatgccgggg ctgggtccttcccaaggttgagacaaaaggatg (Seq ID No: 718)

Homo sapiens TNF receptor-associated protein 1 (TRAP1):

ccgccccttcccatcgtgtacggtcccgcgtggctgcgcgcggcgctctgggagtacgac atg (Seq ID No: 719)

Homo sapiens cereblon (CRBN): cagcctcctttgcgggtaaacagacatg (Seq ID No: 720)

Homo sapiens ribosomal L24 domain containing 1 (RSL24D1): cttcctctcaagcttggcgtttgtttggtggggttacacgcgggttcaacatg (Seq ID No: 721)

Homo sapiens leucine carboxyl methyltransferase 1 (LCMT1):

taccctcttctgttgctttctccctgtggctcgcgccgtcccccgccgcccgtcgacccc gcttccatgtccctggcggacacagctcccaggaacctccacgcccatggccactaggca gagggaatcctctatcacctcctgctgttccacctcgagctgcgacgcagacgacgaggg cgtgcgcggcacctgcgaagatg (Seq ID No: 722)

Homo sapiens RAB14, member RAS oncogene family (RAB14):

ccccccttctttgtggtccggcccattgcgaggtgacaggaaaccctgtgcagggagcg ccgccatcttggaccagcccgaggaagatactgagggagcacaggagcagtcaccgctgc cactgctactgccgctactgctgccggcgcgtctgcacctctcggcctgccagtgtacct gccggcgcctcggtcgaccgcccccgcccccctctcccgctgcgtccgcactcctgttcct ggtcctgacgccccccctcccgcccggaaagctgcccagccaccagcaaccccccagtgcc accatg (Seq ID No: 723)

Homo sapiens Enah/Vasp-like (EVL):

cttccttttcctgtttggttttaagtaggctataaaaatcaagttgctgtcttcagaggg tctgtggtcctctgatcaacataggctggtgggagtacaggactcgcctcctcagggttc cctgtgctgccacttttcagccatg (Seq ID No: 724)

Homo sapiens LIM domain and actin binding 1 (LIMA1):
ctctcttcccctctccctctccctctgccgggtggatgctttctccatgtggcaaggctg taactgttcacagctgtctgaaacagcagtggaccaggagcagcttggagttttaacttt cattttacaaagaacaacatgtttgaatgtttcagcaggcaagttataactggcatctac ttcttgttcttctagaacaccgaaaatctctcccagcactttagaaaggggaccctgact gtgttaaagaagaagtgggagaacccagggctgggagcagagtctcacacagactctcta cggaacagcagcactgagattaggcacagagcagaccatcctcctgctgaagtgacaagc cacgctgcttctggagccaaagctgaccaagaagaacaaatccaccccagatctagactc aggtcacctcctgaagccctcgttcagggtcgatatccccacatcaaggacggtgaggat cttaaagaccactcaacagaaagtaaaaaaatg (Seq ID No: 725)

Homo sapiens ubiquitin-fold modifier conjugating enzyme 1 (UFC1): gtttctcttgcgccctggtccaagatg (Seq ID No: 726)

Homo sapiens coatomer protein complex, subunit beta 1 (COPB1) :

caccccccttccacgtcagccaaggactctggagccgccgccgctgctgcggttcata gccggagtagacggagccgcagtagacggatccgcggctgcaccaaaccactgcccctcg gagcctggtagtgggccacaagcccccagtcccagaggcgtggtgggtcgggcagagtcg gaagaactggctttctagctggaagatgcggaaggggagcgactaggccgcttgcgtctg ggcctggcagaaggaccggattttctggcatccttaaatcttgtgtcaaggattggtta taatataaccagaaaccatg (Seq ID No: 727)

Homo sapiens transmembrane protein 9 (TMEM9):

gggtcttttgcggctgcagcgggcttgtaggtgtccggctttgctggcccagcaagcctg ataagcatg (Seq ID No: 728)

Homo sapiens shisa homolog 5 (Xenopus laevis) (SHISA5):

ctttctttttctccaaaaggggaggaaattgaaactgagtggcccacgatgggaagaggg gaagcccaggggtacaggaggcctctgggtgaaggcagaggctaacatg (Seq ID No: 729)

Homo sapiens transmembrane protein 69 (TMEM69):

gtgcctttccagtggacctgggctgttgttgcggttgttttccttctctccgtgcaacgc tggcaagtctcaaagtcgccacagaaacatgcccctgattcagtgcctctgcttagctgt aacatgttaatcagaactacctggcatcttcctgaacaagactttcaataggggccagta tg (Seq ID No: 730)

Homo sapiens kelch repeat and BTB (POZ) domain containing 4 (KBTBD4) : agatcttcttccgggcggacgtggagccggaagcggaggttccgggctccgggatg (Seq ID No: 731)

Homo sapiens pipecolic acid oxidase (PIPOX):

cgtcctttagccgggagcctgtctttgcttgcctttgcctttgaggctctgtggctgtgg ggctgagtggcatcatg (Seq ID No: 732)

Homo sapiens blocked early in transport 1 homolog (S. cerevisiae)-like (BET1L):

agctctttccccgcgactgcgccacgtctgaggcggctgtggccgcgtcggtgtccgcgt cgaggagccggggcagggcacgatg (Seq ID No: 733)

Homo sapiens zinc finger protein 581 (ZNF581):

ttctctctttcggccggcgccgccagttcctggggcacacccagaggtccccttctcgcc gccgcctgcaactgcgagggtagcccggggccgcttggagtcgcccggacctgagaggct gctgcactggcctcagccagccctccggatg (Seq ID No: 734)

Homo sapiens armadillo repeat containing, X-linked 1 (ARMCX1) :

cgtccttctaatcctagtcttcgtttggtccggttgcactcttcctatagcccagagggc
gagagggcctgtggcctggggaaggaggacgaggttctgcctggatcccagcagtagga
cgctgtgccatttgggaacaaaggaatagtctgcctggaatccctgcagatcttggggcc
ggaggccagtccaacccttggagcaggaagaaacgcaaagttgtcaagaaccaagtcgag
ctgcctcagagccggcccgcagtagctgcagactccgcccgcgacgtgtgcgcgcttctc
tgggccagagcgagcctgttttgtgctcgggttaagagatttgtcccagctataccatg
(Seq ID No: 735)

Homo sapiens spastic paraplegia 21 (autosomal recessive, Mast syndrome) (SPG21):

cggcctcccgcacgcaccgcgcagcctgctgtgcccgtgggtcccgagtgctccgccgcc
cgccccgacccgggcccagccgcctccacggcccgcgctcgtactggagcgaagagcggc
ctcctgaaggaggggaagggacgtgggggcggccacggcaggattaacctccatttcagc
taatcatg (Seq ID No: 736)

Homo sapiens staufen, RNA binding protein, homolog 1 (Drosophila) (STAU1):

tctcccttttttccttcttccttcccctcctcgccgccaccgcccaggaccgccggccgg
gggacgagctcggagcagcagccagagtttattaaccacttaacctctcagaactgaaca
aagacaacattgttcctggaacgccctctttttaaaaaagaaagcataacccctactgta
gaactaaatgcactgtgcatg (Seq ID No: 737)

Homo sapiens adducin 2 (beta) (ADD2):

cggccttttgtcagcgcgcagggccaggagagctctcatttcctcccagcctcgtgcggg
aaatggctttaattctgacggcagggctgtgagggactagcgggaacccgagccttttgt
caaggaactgcggcgtcggtggccagtcatccccgccgccgcggagccgctgcactgctg
ggggatctcccagcagctctgacgagcgcgggctgcagcatgggcagaaaacgctgccct
gcagattagctgggtggatttttaagcgcaccccacccccccaaacccataaaataacaa
aaccaacccgcagtggccgaccggagatagctaagatgccgcgcaggagtttccacctgg
atgtttgaggttgtgtagatgtggccggcacccttgagagtggagctaggggtgcagac
tgagcagtgaacagaaggagccttggacagggctgggccagcctcccgagttccaggagc
gaattgcaaacccaccgggaaaatg (Seq ID No: 738)

Homo sapiens WD repeat domain 1 (WDR1):

ccgccttccggctccagtccccgggctcggcctcggcgaggtgtaattcgcagcgcgggc
cggccccggaggctctcggcgagcgcggcgcggtaacaagtggcgaggatg
(Seq ID No: 739)

Homo sapiens family with sequence similarity 20, member A (FAM20A) :

cgacctctacttccacctctggccccaagtacagcgccagctgcggcctcgggagcgccc gcgggggtgcccgtgcaccggccgcgcctcctccctggcgcgggactcggccgcagctgc ctcggaccccggcacgatcgtgcacaactttcccgaaccgagccccggactgaaccggc tggcggcagccacagcgggtcgagctccaagttgcaggccctcttcgcccacccgctgta caacgtcccggaggagccgcctctcctgggagccgaggactcgctcctggccagccagga ggcgctgcggtattaccggaggaaggtggcccgctggaacaggcctcagttcctgctttt gaaaggaagaggggagtctgtgacccctgaggcctccttgcaactctgttttccaagct ttgcacatcttccgaatttcttcttcaaagtctaccctaatgaaatatcagacaattttc caagtgtgcttcatgaacttctgggaggtgcttcacagtttctgcaaatgattgattgaa ttttcactttgaaaaaatatactttaaggcgacacaagatg (Seq ID No: 740)

Homo sapiens kelch domain containing 4 (KLHDC4): ttttctttcctggtgtcccgtcgcggcttgggacccggcaagatg (Seq ID No: 741)

Homo sapiens calcium channel flower domain containing 1 (CACFD1) :

tgctccctctcccacaaggcagcgcgccggctcggacgcggccggctaccgagccctttg tgagggctgtgagctgcgcctgacggtggcaccatg (Seq ID No: 742)

Homo sapiens zinc finger, CCHC domain containing 8 (ZCCHC8): gaatcttttccacagcccaaaatg (Seq ID No: 743)

Homo sapiens kelch-like 24 (Drosophila) (KLHL24):

gtttcctttgttgtgagctgcggcagagactggtggctggaggagacgccggcgctggag agtgcgctgcgccgcccgccgctgagggaccgcggggttagccactgctggctgcttcca gtgttcgccgagaggtaccgggggtgacagctccgggaccggccgaaaggcgaggaaccg gtgtggaaattaaaagaacacacatatttgactggggctttgatcaaccaaatgctaaa aagccacataaagaagatccctaatagtcatttctcaacaattatatagtcaactgatgt aacaatg (Seq ID No: 744)

Homo sapiens FtsJ homolog 3 (E. coli) (FTSJ3): ctcccccttccaccatg (Seq ID No: 745)

Homo sapiens dymeclin (DYM):

gcttccctcttctctcgccgcctcctggcctccgcaccgacgcggcccgggctggagccg agccggggccgagctgcaggccggaccggagccggatctgtacccgctgagacgtggaaa catggaggcctgagccggtgtgcgccacctgggctgcggcggcgacagcgacttctcctg acccctctgccaccctcccatccgtccgcgggtccgtggagctggagcagatcccccagc cggctgagacaggttgtcttttggaaatgcaggtttaaggacaaattatctgcttaagct agaagatg (Seq ID No: 746)

Homo sapiens zinc finger protein 280D (ZNF280D):

cctcctctttctcctcctcctcagggctccagtcaggccgatccgctccgctcacggaag gaaaacagaaataacttgctggcttgtctggagtcacatgtacttaggtgacaatttaca gaaagtcatctctgcagcttgatg (Seq ID No: 747)

Homo sapiens ankyrin repeat domain 10 (ANKRD10):

cgttcctttgtgctgcggcggcggcttctcgagtcctccccgacgcgtcctctaggccag cgagccccgcgctctccggtgacggaccatg (Seq ID No: 748)

Homo sapiens SWT1 RNA endoribonuclease homolog (S. cerevisiae) (SWT1) :

ctctcctttggcttggggctccggagttgccactgccgccggcgctggtaagcttttcag gatg (Seq ID No: 749)

Homo sapiens leucine rich repeat containing 49 (LRRC49):

tgacctctttcgggtctctttgaatctccgctgtagcgtcacctggaaggcagatctaac agagaacctggactgtctcctatcatg (Seq ID No: 750)

Homo sapiens F-box and leucine-rich repeat protein 12 (FBXL12) :

ccgccttctggacttggtcttagttcccagtcgcggccaaatcacgcctcagccacctcc cgcaagcctctcactgcctcagccacgctttccaggctggtttctggtccccatccgcgg ctggtccggccctgggaccgaatcacttcccagcgagaggaaggtcaaatttctcgaccg gctacgggaaggtcgcggccgccgccctgtcagccgcctcggcgcccccaggacccctcg ggtctctttaaccggaagcggaagtgcgtgtcggcgggatcatg (Seq ID No: 751)

Homo sapiens WD repeat domain 55 (WDR55): cagtccttctcagcatg (Seq ID No: 752)

Homo sapiens zinc finger protein 3 (ZNF3):

cgttctttgttctgtccccggtgtgtgggtctgtgacagggtccaacagggcctggtccg tgtccggtcccccaaatctgtcgtccctgcccccaggcattggcatcaacaaaagtcaga attcccgggaacttgaacagaggctgctaaattcccagtaattgctcctttggccttcta gggactgacttcaaagaaggaaggaaagaatcaggcagtgcttcctcattctcttttaaa acccgcttcccgctgagtctgcacccaggagaccagagagcaccttgcccttccatg (Seq ID No: 753)

Homo sapiens tetratricopeptide repeat domain 27 (TTC27):

ggttcttctcctaggcggaagccagaccagagagcgtgcgtgtttttcccagggtgcccc gcgctgctgttatggccgcctccttgaggtagtatccgcacatggaattctagggccgca ggtgtatttacggtaactgtcgccactagatttcagcgcctttggactctcctgttttca ctttcttttgttgactcccgtgtggccctcgtgggagcctgttttggctgcagcggtgtc tggggtgatg (Seq ID No: 754)

Homo sapiens THUMP domain containing 1 (THUMPD1): gtttctctttcctctcagtttgcgcacaccatg (Seq ID No: 755)

Homo sapiens ankyrin repeat and KH domain containing 1 (ANKHD1) :

tgctcttctcgttcccgagatcagcggcggcggtgaccgcgagtgggtcggcaccgtctc cggctccgggtgcgaacaatg (Seq ID No: 756)

Homo sapiens syntabulin (syntaxin-interacting) (SYBU): cctcctcctggacggcggcagcggcggcgcgaggagccggcgggcagcggcgcgatg (Seq ID No: 757)

Homo sapiens coiled-coil-helix-coiled-coil-helix domain cont aining 3 (CHCHD3):

gcgccttctccttgcttctggggtcgtggccttgctcccgctgtgcgggaaaagaatcc aggcccttccacgcgcgtgtgggtgcggggccccgaagtgctcgtggttccccgctagg tctccgctggggcaggaaccggaatcatg (Seq ID No: 758)

Homo sapiens HAUS augmin-like complex, subunit 4 (HAUS4):

cctccttcgtcgcggcctctagtgcactttcggctccttccccttcccgggcctttcagc ttggtctttccgggcctcgcttcccccagcccctgcgcccggcccgaacgagaggttccg gagccccggcgcggcgggttctggggtgtagacgctgctggccagcccgccccagccga ggttctcggcaccgccttgagagcttcagctgccccaggattagaatcccaagaaaatca aatg (Seq ID No: 759)

Homo sapiens solute carrier family 41, member 3 (SLC41A3):

ccgcctctttcccgccgccgcctgggaggggacccgggctgccaggcgcccagctgtgcc cagatg (Seq ID No: 760)

Homo sapiens phosphatidylinositol glycan anchor biosynthesis , class V (PIGV):

cttcctttccagcctcccgccctcgtctgcttccggccctgtggcctggtggggctctgc aggctccctcgggagtggtccttgggccgtggcccctctgggaggcctgaggagctcaa tcctggtagcaacacccctgaattcctggtggtgaaaggatg (Seq ID No: 761)

Homo sapiens poly (ADP-ribose) polymerase family, member 16 (PARP16) :

agttcctttatccctgggcccaacctccccgccgacccgcggtccaggcctcggtctctc tcttcggcggcgagccgcggcccagaccccggcagaggacacttgtcggcacgttctcac ccctgtcatctcagccccctgcctagctccaccccaggcttgggaacccggcccctgacg gcccattgtccgcgggcccagcccccgcgctgaacgcacgctcgcccttgcccctaacca gcgcgtctaccccggcaacgcgcagtgacctgggatg (Seq ID No: 762)

Homo sapiens thioredoxin-like 4B (TXNL4B):

gtttcttttctgcgcttgtgcgttttctgttcggtttccttcccgctagcggggccacga gggttgctaggcaacagcccctgggtgacttggtcttagggtcctgtccggcttggggct gatgaaaggagctgtccgcgcccgggctcttccgagaagtggttgctgacagccacaaag tgaaagggagtgaggcggcgtggacgagtaaggagtgacagtgaggattcacatttgggt tatttcaagatg (Seq ID No: 763)

Homo sapiens slingshot homolog 3 (Drosophila) (SSH3):

cgtccttcctggtcctgcgggtccaggactgtccgcggggttgagggaaggggccgtgcc cggtgccagcccaggtgctcgcggcctggctccatg (Seq ID No: 764)

Homo sapiens zinc finger protein 692 (ZNF692):

ctccctctggggcgcgggcctcagttccgggctacagcagccgacgccgagaggcaccgt
ttcttcttaaaagagaaacgctgcgcgcgcgaggtgggcccctgtcttccagcagctccg
ggcctgctcgctaggcccgggaggcgcaggcgcaggcgcagtgggggtgagggcgcgtgg
gggcgcacagcctctggtgcacatg (Seq ID No: 765)

Homo sapiens tRNA-histidine guanylyltransferase 1-like (S. cerevisiae) (THG1L): tggccctttcctttccgcgtgtagaatg (Seq ID No: 766)

Homo sapiens solute carrier family 25, member 38 (SLC25A38):

tctccccttctacagagttcctccggcgcttcctccaccccgggatacacagaacctcat
ctcctacggtgctgaagcctgcagcagggcaggatgggcaggagagcagagccgcggagt
ctgcggcgcgggtgaagagcggcgcgtaattcccgcagcaagattgttccgcgcccgcag
cccctggactagcaggatccgaaccccggcggctgcgtgcttataggcgcagacgtcaga
gagcccgcggcttaaagcgcgtcgcctggctagcgccacccccctagccttcttcaaggcc
tccagggctgggcccaagcgcccgtcgacggcaccctgggcccagaggactcgcgggcct
catctccaatg (Seq ID No: 767)

Homo sapiens WD repeat domain 13 (WDR13):

agttctttctgatagcaggcagccatcttgcctggagcctgagaaagggaggagagacag
aaggaaccggcgacagtggtctcagggccgctccgggggcctcaagaaccggaggcagc
cccggaggtggtccccgatcccgggctatgctcttggatctgagaaggaaggcggaggg
cggcggggacaagatgggtggagaatgtcaagcaaggaatgctaggcgggggagggcgt
tgctatggcgactggggaggggcggtgtctgttctgaatcgctgtgtgtcacccggcgc
tgcccaggaagggcagggctggggtgatgaccatggtaacacccgggggggagttcgtga
catctccggcgcggagggactcgatgtctatggcaatggtcgcctggtggaagggacgga
actagatcccttcgctcgggacgctcacattccaggcccttgtcctgcaggctgccgcgg
gcggacacgccagaggaggaggccggggaatg (Seq ID No: 768)

Homo sapiens chromosome 1 open reading frame 123 (C1orf123):

ccgccttttacgacgcgccggaaagcaacggcaagggcggcagccagcaccgggcggaga
gggctaccatg (Seq ID No: 769)

Homo sapiens chromosome 20 open reading frame 11 (C20orf11):

ctgcctccttctactcgggcgccccggcggccgccacctctccccagcccaggagaggct
gcggagccgcagccgcccagaccgcgcagcgcgggaggcaggttccgcacgaaataaatc
agaatg (Seq ID No: 770)

Homo sapiens zinc finger protein 446 (ZNF446):

ttccccttttggggacagatcccgaagttcgagcatccctcggataggccgggtgtcagg
cctggtctctcaggcccgtccaggcccatcttgacgattccaagaccaccccccttgagca
agaatg (Seq ID No: 771)

Homo sapiens mitofusin 1 (MFN1):

ccgccctttgccactcccccctgcctcctctccgcctttaacttctcgggaagatgaggca gtttggcatctgtggccgagttgctgttgccgggtgatagttggagcggagacttagcat aatg (Seq ID No: 772)

Homo sapiens phosphotyrosine interaction domain containing 1 (PID1) :

agtcctctcgcagctgcgccaggacagccggcgcgcggccgtgcccacaagttgccggca gctgagcgccgcgcctcctcctgctcgcagcccccctacgcccacccggcggcggtggcca gcgccaggacgcacatcccgcggacaccgaccccagatgtaaagcgggaccccagcccct cgccccccggcgcgatcgacagtctcgccagcgtctcctctgccaaaacccagggctgga agatgtggcagccggccacggagcgcctgcaggagagatttgcagacacagaagcggcac agagaaggccattgtgaagatcaaggcagaaaccggagttatggcatcataagccaagga atg (Seq ID No: 773)

Homo sapiens pleckstrin homology domain interacting protein (PHIP) :

tttcctcctcctcctcctccgcctccgccgccgttgcttgaatggtggagccgaagctcg gctcgtgaacacacactgacagctatagggcaggcggcggcaccgtccccgcttcccctc ggcggcggggtgtcccgtcggcggccctgaagtgacccataaacatg (Seq ID No: 774)

Homo sapiens LIM and senescent cell antigen-like domains 2 (LIMS2) :

tggcctttttttgggcgtctccctgctccgcggcccgggctggcgggcgggcgctcggctg gcggctgcagcagcagagggagacccgcggcaaccccggcaacccagggctcggcgtcgc tgccaccatg (Seq ID No: 775)

Homo sapiens SCY1-like 2 (S. cerevisiae) (SCYL2):

aggtcttttagtctttttcccccctcccttactcttcgtccccggtccctcccctccccac ccctttccttctagctccgacgtttgcggccgcgggggcggcggaggatatggagtaaag ccagagtcagtggccaggcacgaaggcagagcaggaacagccaggaggcgtttattaggg gggcgggggggaaagagccccagcaccgcccctcctggaagaaggaagaggtaagtgaccg gccgccggcaccgaccgacctccctcaccggcggctctctcgcctgggctcccggagccg gcgaggagggaatggaggactcgcgcccgggttaggcctcccagggccgctcaggctggt gggtgttgcctggtgacgggcctgccggcggccggccgggcgatcggcggtcggcgcccg cgcaaagcggggctggacgagcagcgagctccggggagcggatccgagagggccgagtcc tcgaaagaggccttgaggcgacgggagacccgggatcgaagtcagctgccggagggagag ccccccatgccggctcgagagctcgggtttcggtggtggagaacgtagtacctttcgggg acattggacactactctaggaccgggtaactataactacccaatattgcagccatg (Seq ID No: 776)

Homo sapiens ring finger protein 31 (RNF31):

caccctctctcctagtacttcctgttctcggctaaccctggcgctgggccgggggctgga gagtgaccgtggtctgagtgacctggggcggctgcgtgggccggggtgggcctcaaagcc gggcaccagacgggaggggcggcgctcgggccgcgcgctgcccgcgccgggtcctggcgg gcggcgaggctggggctgactcctgcctcaggatg (Seq ID No: 777)

Homo sapiens mediator complex subunit 9 (MED9): cgacctctggctaacctacccccggagccatg (Seq ID No: 778)

Homo sapiens ATP5S-like (ATP5SL): cggccccttccggttacgaaaccttagcaagatg (Seq ID No: 779)

Homo sapiens GPN-loop GTPase 2 (GPN2):

tctccttttgcgcgacacggtctcagctgttccgcctgaggcgagtgacgctggccgcca
acgaggtatacgtactgggaccctcgccctcagtctcgtctccggcgcggctacctgccc
cgttttccctgtgagttgacctgctccgggccgcgggccgccaatg
(Seq ID No: 780)

Homo sapiens transmembrane protein 48 (TMEM48): cggtctcctgtacgccctagactaggggccgccatctccatg (Seq ID No: 781)

Homo sapiens ankyrin repeat and zinc finger domain containin g 1 (ANKZF1):

ttgtcctcttcgctgctccgtagtgacggggattgttgtgttgcagaaatccggcaatcg
acctgaggacttgcgagccgctcagctcccgggacgtttggagctgctgctaaataattt
ctgctcagccatg (Seq ID No: 782)

Homo sapiens notchless homolog 1 (Drosophila) (NLE1): ggctctttctcctccacgtggggacgcaggatg (Seq ID No: 783)

Homo sapiens cell division cycle associated 8 (CDCA8):

cgctctctctcactggcacagcgaggttttgctcagcccttgtctcgggaccgcagcctc
cgccgagcgccatg (Seq ID No: 784)

Homo sapiens polymerase (RNA) III
(DNA directed) polypeptide E (80kD) (POLR3E) :

cgctccccccacgtgtccgccggagtttctccaccagcaacatggccgccgcctgagag
gagagccgggccgccgccgtctctgcagcccgcgggtaactggccgttgccgccgtccg
cgctcggcccccgcggagagatcgagctgaaggactgcgcggctggctctcctctagtat
g (Seq ID No: 785)

Homo sapiens armadillo repeat containing 1 (ARMC1):

gagcctttgcccgccagcgccttcgctctttggctccctgagttagtccggttgcttgcg
atcgccgcggccggggctgcgaaccgaagggctcgctccgcgccgcctgggtctctacct
catccgtaggtgtggccctgatggtgtggcaggctctggactcctaaagctctggagcga
atttaagatttattcatgtgcatggcatagaagatg (Seq ID No: 786)

Homo sapiens transmembrane protein 33 (TMEM33):

ccgtctttctggaaacaccgctttgatctcggcggtgcgggacaggtacctcccggctgc
tgcgggtgccctggatccagtcggctgcaccaggcgagcgagacccttccctggtggagg
ctcagagttccggcagggtgcatccggcctgtgtgtggcgcgaggcagggaagccggtac
ccgggtcctggccccagcgctgacgttttctctcccctttcttctcttcgcggttgcg
gcgtcgcagacgctagtgtgagcccccatg (Seq ID No: 787)

Homo sapiens pyridoxamine 5'-phosphate oxidase (PNPO):

ccttccttccccggggtagaagtccagggtgagaaattggttccgaactcaaaggaaccc
agtgccgggccacagccgggtcacgtggccggcggccccccatg
(Seq ID No: 788)

Homo sapiens golgi phosphoprotein 3-like (GOLPH3L) :

attccttctctgcatcgaaggatcaggaagtttgtgctctctgcgtggctaagtttttca
cctactaggacggggtggggtggggagaacaggtgtccttctaaaatacagcacaagct
acagcctgcgtccagccataacccaggagtaacatcagaaacaggtgagaatg
(Seq ID No: 789)

Homo sapiens regulator of chromosome condensation (RCC1) and BTB (POZ) domain containing protein 1 (RCBTB1):

cgctcctcctcttcgctgccggtgggcaccgccgctcgctcgcacttctgcgcccattgg
agcttcggagatccctgcggtcccgcgggacggcgcggcagcagctgacctcgcagacag
gatcttgctctcttgcccagactggaatacagtggtgtgaacacggctcactgcagcctc
aacctcctggactcagagatgtcggcttatttataggaattgcttgaagccagagtcatg
(Seq ID No: 790)

Homo sapiens leprecan-like 1 (LEPREL1):

cgtccctttaagagcggctggccaggcacggcctccgcctctcagtacgcggagcgccgg
cggtcacctggggctcgcggagcggccagatcgcggcggagtcggcgcgcttccccgagg
gaaggtgggagaggggacccggacgcgaggtgccccgaagccctctcgagcgtaaccgtc
ccgcgcctctctgaggcggaggatg (Seq ID No: 791)

Homo sapiens hedgehog acyltransferase (HHAT):

ctgtctcttggctcaggcttggaggcctccgagcagcaacatcgtcccaattataccccg
ttggagcatcttcagatcttccactcttttcacaacgcaatcaaaatcttcgtacccatt
ttgcagtagtgatctctgtaagttgctttacaattcataaagtttattctatttgatctt
cactctaatttacaaagaaaagcagggaagtctatttctgttttacagaggtgtacaggg
aggctcacaggggctaagttcacacagtaagccctcgaagctgccagggctgcaaagccc
accctctttccaccgcaccgaactacctcctttcgcctacaaaacgtaggtggggaccac
tggtgttggaatgacggcccacctcgagtttcaggtgacttccactctgcaattaacttg
caggcagccccagacctgcaatgaacacacgggtgggggagagatatgcacgccagggtc
agtgggaaccaacagccgaggggtgagcggggctaggggccccgggccgccggcggggca
aacgcggttcagaaacgcaggccgcgctctggcccgcccccctgcagcagcacggcctgct
cgccatcgcccggagagcgccgcgggttcccgagtccgggcgcggagggcgcgcgggcac
ggcggcaggggcgtgctcggaggacgcgcgctgcgctgctcctccaaagggcagctccgg
gggaaagagggtggcgtcccggggaagcccgcagccgccgccgatgtcgctgggactcgg
aagtgccgaaagaggggtgttgggaactcgcggcgcgcgtgaacgttgccgtcgccgccg
cccgggacagcccggagaaactctcagcgtaggcatcgggaaccttcgtgccaaggagcc
atg (Seq ID No: 792)

Homo sapiens chromosome 11 open reading frame 57 (C11orf57) :

cctccttttctcccaaaccacttcttccccctacccccgccacgcgaggctgcggcg
cacggtatgggtgtgtttgtgtgtatttgtgtggggagggcgtttggagggaaggttacc
gggagctccgaggccgctggggaacagggatcccggtgacaaagatggggatatttcctc
tgtcttccacttggaaacctcaacccccgcttcaggctccctagatactttctggggccc
aaccgaaggccgtagccatccaaagcgttcccagcctttctggggagtgaaacttacccc
cggggttcgtcctagaggagcgtgagcggggaatgcccaggtcaaccgggctgtccgaat
tccgccccggctcagcctccggcctcagtccgggagagagatctgcctgtcggtctgggc
tggggaaacgcggcagtggcctgggccacaggtgagggcagagtaaccagtgggaaggc
tgcgttttcacgaaggactcgggtgaagctgcagagctgcctttgagccctgactccttg
gcttcctgggtcggaggagatcttgtaatggagtggttcttcgtctcactagcaagatgc
ctgatttcctcaggatcaagggattgaagaatg (Seq ID No: 793)

Homo sapiens high mobility group 20A (HMG20A):

agtccttcgccgcattggggcaaaataatcccttcattttgtgaaggtaccgtggaaaa
tatttcatttttcttctcaccggagcaattgtaaatgctatgcggtaagaggagttacct
gtggaaaggtggttaagagattaggtaaagaaaaggaaaggacaccaaaataaagtgctg
cggaagaattttgtccagctgtgagacgacgagtgcgtgaagtgaaggcgattgagagg
ggctgagggaattgtcctctgtggaagggactttcttttggccctaggccccttcctgcc
cctgtcgtcagcagagtctctacaaggaagataacggactgtaaaattctataaagcaaa
gctacacatcacttgacaccatacaccatcttggttacataatgaagagagatg
(Seq ID No: 794)

Homo sapiens checkpoint with forkhead and ring finger domain s, E3 ubiquitin protein ligase (CHFR):

atgtctcttgacagcggcggcggcgcagccggttccgggttcggcgcggggcggggatgt
gaatcccgatg (Seq ID No: 795)

Homo sapiens nucleoporin 133kDa (NUP133):

ccatctcttcccttaggtgtttaagttccgcgcgcaggccaggctgcaacctgacggcca
gatccctcgctgtcctagtcgctgctccttggagtcatg (Seq ID No: 796)

Homo sapiens CNDP dipeptidase 2
(metallopeptidase M20 family) (CNDP2): cttccttccaagaaccttcgagatctgcggtctggggtctggttgaaagatg (Seq ID No: 797)

Homo sapiens oxoglutarate dehydrogenase-like (OGDHL):

gcaccccttccgcgcagcccccctgacctgcagcctccggacctcgctgcagcgcggaccc
ggcccgcccgcccgaatg (Seq ID No: 798)

Homo sapiens transmembrane protein 30A (TMEM30A):

ccgcctcttccgctctacagcggaggtggctgtggcggtggcgctggtggctgcggcggc
ggcggcggcagcggcgctcgagcggttcctgtcaggtcagccggcgggccccctgggtg
gtccacctgcaaatcgcggagcggcgccccaggatcgatg (Seq ID No: 799)

Homo sapiens elongation protein 2 homolog (S. cerevisiae) (ELP2): gcgtctcttgtttgtgcggctgaccagttggcgacatg (Seq ID No: 800)

Homo sapiens WD repeat domain 12 (WDR12):

cgttcttttctttgtatttccgcctctcgcctctctaaaagccgcagttagaggcgag
atttaggaaaaacctctgccgagtgagcctctggttgggaatatgtatgagaaaaaaaa
ctggcaaggcgttagtcaagcaaagctgaaggcagaggaaatttgatatctggctggagt
ctagaggatttaatgcaaataagatactctgagggcagcgtggcaaaaaaagactacaat
tcccggtggtcacagcgtttgagaagcgatgctttctgagacttgtagtaactaggagct
gtgtttgaactatccaggctcaggacagcctcttgaaaaaaaatttttattaataaagc
ggatttgagtgggatctttttcctaatcgattacgggcccacacgtatgggaagaattct
aacaatgattaaagggacatgctacctttacgactatcctttctaatcgatgactccta
aatctaggagtaggtagtcgatgtttgtggtctgggcgtctgtagaagggcaacctcgtg
ctttctgcagaggagaccggagggcagaaggcagagtccaggcttagactgcagttcctc
gcttacctgtgcagtctaattttgagctgcctctttgtagtcttaaaaggcaggagcttc
gtgttgtgggtctgctaacccgtacgtttccgtgggcaagtcgtgtgtactcctcgccat
g (Seq ID No: 801)

Homo sapiens tetratricopeptide repeat domain 17 (TTC17):

cgacctcttcaagatggcgggcgccggagactagcttccgcttccggtgtgagcggcccg
gccgggggggcaagatg (Seq ID No: 802)

Homo sapiens proline rich 11 (PRR11):

ttttctttatggcgtgggagaggccacagcccggactccatcgactcccccggctcttag
actaaaatcatg (Seq ID No: 803)

Homo sapiens TBC1 domain family, member 23 (TBC1D23) :

ctccctctttcttcccctctggggaagctcagtgctggacttccgaagaccttttacgac

attgagtctcggagttggtctcagcgccggatccacttttcggcaaagtgacgtggacgt
caacagcaatg (Seq ID No: 804)

Homo sapiens leucine rich repeat neuronal 3 (LRRN3):

gctcctctctggggagtggagggtgttcagttattaatgaccgctgagcaggcagcacca
tgtcagtgtgacaactgatcgggtgaacgatgcaccactaaccaccatggaaacaaggaa
aaataaagccagctcacaggatctctcttcactggattgagagcctcagcctgccgactg
agaaaaagagttccaggaaaaagaaggaatcccggctgcagcctcctgccttcctttata
ttttaaaatagagagataagattgcgtgcatgtgtgcatatctatagtatatattttgta
cactttgttacacagacacacaaatgcacctatttataccgggcaagaacacaaccatgt
gattatctcaaccaaggaactgaggaatccagcacgcaaggacatcggaggtgggctagc
actgaaactgcttttcaagcatcatgctgctattcctgcaaatactgaagaagcatggga
tttaaatatttacttctaaataaatgaattactcaatctcctatgaccatctatacata
ctccaccttcaaaaagtacatcaatattatatcattaaggaaatagtaaccttctcttct
ccaatatgcatgacattttggacaatgcaattgtggcactggcacttatttcagtgaag
aaaaactttgtggttctatggcattcatcatttgacaaatgcaagcatcttccttatcaa
tcagctcctattgaacttactagcactgactgtggaatccttaagggcccattacatttc
tgaagaagaaagctaagatg (Seq ID No: 805)

Homo sapiens MIS18 binding protein 1 (MIS18BP1):

ggccctctctccgcgcggagccgagccggaactgcggcagtctctccctgccaggctctt
catccaaggtttctgtggatcccttctgaagttctatctgaaaattgcgcttaagtgaat
tttctgttagaagaacttggttgctactttcttgtcaagatg (Seq ID No: 806)

Homo sapiens LMBR1 domain containing 1 (LMBRD1):

ccgcccctttaacctttagggtgcgcgggtgcagtatatctcgcgctctctcccctttcc
ccctcccctttccccaccccgggcgctcaggttggtctggaccggaagcgaagatg
(Seq ID No: 807)

Homo sapiens ST6 (alpha-N-acetyl-neuraminyl-2,3-beta-galactosyl-1,3)-N-acetyl galactosaminide alpha-2,6-sialyltransferase 1 (ST6GALNAC1): cttcctctagaacccgacccaccaccatg (Seq ID No: 808)

Homo sapiens spermatogenesis associated 7 (SPATA7):

gctcctcttttccagtcctccactgccggggctgggcccggccgcgggaaggaccgaagg
ggatacagcgtgtccctgcggcggctgcaagaggactaagcatg
(Seq ID No: 809)

Homo sapiens docking protein 5 (DOK5):

cctcctccttcctcctcctcctcctccttcttctcctccttctcggccgggaggaggcag
ggctggatccctcagccgccgccgctcctcctcctggcaggccggccgcggagtcagctg
acgccggcgctccagcctcgcctccccgcgccgcgctctgcgctccccgaaagtggctgc
aagccggccgcccactgtcagggttgggggacagagaaagtgatgtgcgccttctaaag
cctcgcccagcgccgccgaagcagcttcacctctccaactttctcccaccgactgcttgt
cttgaccctgccctccaccctccccagagccacttcgggtgcgcgctcttgggtaaaggg
ggggtcaccggctgtctgggatg (Seq ID No: 810)

Homo sapiens glycosyltransferase 8 domain containing 1 (GLT8D1) :

tctcctccatcgcctgcagtaagggcggccgcggcgagcctttgaggggaacgacttgtc
ggagccctaaccaggggtatctctgagcctggtgggatccccggagcgtcacatcacttt
ccgatcacttcaaagtggttaaaaactaatatttatatgacagaagaaaaagatg
(Seq ID No: 811)

Homo sapiens cullin-associated and neddylation-dissociated 1 (CAND1) :

tggccttttgccctagggagcgagtgcggagcgagtgggagcgagacggccctgagtgga
agtgtctggctccccgtagaggcccttctgtacgccccgccgcccatgagctcgttctca
cgcgaacagcgccgtcgttaggctggctctgtagcctcggcttaccccgggacaggccca
cgcctcgccagggagggggcagcccgtcgaggcgcctccctagtcagcgtcggcgtcgcg
ctgcgaccctggaagcgggagccgccgcgagcgagaggaggagctccagtggcggcggcg
gcggcggcagcggcagcgggcagcagctccagcagcgccagcaggcgggatcgaggccgt
caacatg (Seq ID No: 812)

Homo sapiens BRICK1, SCAR/WAVE actin-nucleating complex subu nit (BRK1): cgctcttcctcaggcggcggccatg (Seq ID No: 813)

Homo sapiens zinc finger CCCH-type containing 15 (ZC3H15):

cggtcttcctcctcgtcctgccgcagggccagaacccctgacggtattcagctgcgcgta
agtctggccggtgccatctgtctccgcaatg (Seq ID No: 814)

Homo sapiens polo-like kinase 1 substrate 1 (PLK1S1) :

cggtctccttcggcaaccccggccgaacggccacccagaggctgtgctgagctggcgcag
cggcagcagcatg (Seq ID No: 815)

Homo sapiens dysbindin (dystrobrevin binding protein 1) domain containing 2 (DBNDD2) :

gtttctttcctacgcagccgctcctgccgccgtggtcgctggagctttgcctctctaggc
cggcagcgcctctcctccatggtcctgtctgtcagcgctgttttgggagcccgccggtga
ggccgggccacgctcagacacttcgatcgtcgagtctgtcactgggcatg
(Seq ID No: 816)

Homo sapiens KIAA1704 (KIAA1704): gattctttttggatagggttgacgttcgtggatagactcatatctgtgaccagtgtccgc caccgcggatg (Seq ID No: 817)

Homo sapiens solute carrier family 25, member 37 (SLC25A37): ccccctccctgcccacctcctgcagcctcctgcgccccgccgagctggcggatg (Seq ID No: 818)

Homo sapiens myoneurin (MYNN):

cgtcctcccaagatggcggagacagagtgaagaaactgtgttccccccttgggttgctat
cgatcaagggtaaaattccattctgatatcaaaatg (Seq ID No: 819)

Homo sapiens vacuolar protein sorting 33 homolog B (yeast) (VPS33B) :

gcttctttttctggtagaaggcggggttctcctcgtacgctgcggagtctctgcggggtg
tagaccggaatcctgctgacgggcagagtggatcagggagggagggtcgagacacggtgg

ctgcaggtctgagacaaggctgctccgaggtagtagctctcttgcctggaggtggccatt
cattcctggagtgctgctgaggagcgagggcccatctggggtctctggaagtcggtgccc
aggcctgaaggatagccccccttgcgcttccctgggctgcggccggccttctcagaacga
agggcgtccttccaccccgcggcgcaggtgaccgctgccatg (Seq ID No: 820)

Homo sapiens zinc finger, C4H2 domain containing (ZC4H2):

aggcctctccaagcccctaccgcacaggctcatagccccaagcccggaggaggtggctac
attgtgtctattgtatcccttggctggtgtatttgtacatctctcgggacgtgaaattga
cagtgaaaagtatg (Seq ID No: 821)

Homo sapiens BAII-associated protein 2-like 1 (BAIAP2L1):

cttcctctggcggcgtccggccgcttctcctctgctcctcgaagaaggccagggcggcgc
tgccgcaagttttgacattttcgcagcggagacgcgcgcgggcactctcggccgacggc
tgcggcggcggccgaccctccagagccccttagtcgcgccccggccctcccgctgcccgg
agtccggcggccacgaggcccagccgcgtcctcccgcgcttgctcgcccggcggccgcag
ccatg (Seq ID No: 822)

Homo sapiens solute carrier family 25, member 40 (SLC25A40):

cgtccttctcgcgcctcgctctggccctgcaggttgtgtttccgcctctaccccgcctcc
attccgttgctctctcagtctcagacccgggctctcggtccgccgcttcaggtcttggcg
cagcctcagagagttggcgcggctctgtgttgaccaaacctagtggatgcagttagcgcc
ggagcccggccccgcccgtcaccagggttattcccgccttctaggtttgccaggactgcc
ggccctgcagctgccttctgccccaggtttttggctactgatgttacaaacaataaaata
ttggagcatagagttgaagaacagactcaaaccaggtttttatttaattagttaaaaata
tg (Seq ID No: 823)

Homo sapiens protocadherin alpha subfamily C, 2 (PCDHAC2):

tttccttttccctcccccctggagctgtagcggcagcagcagcaggaagccgagccgggtt
gagcgactcggaggcgagcggaggagctggaatatggggagtcagcgaggacggtggggc
caggagcccttgggaggcctacggagggagcggccccaggcgctttctagagcgtgagc
ggtgggggagcaggcgcagggtggcacgagcggaggcggggcccgggcgtggggcacggc
tggggaagctgccgcctccggccctgcccggctgcctccgccgcggccagtggctatg
(Seq ID No: 824)

Homo sapiens chondroitin polymerizing factor 2 (CHPF2):

gttccttttgggttagctttggcagtattgagttttacttcctcctcttttagtggaa
gacagaccataatcccagtgtgagtgaaattgattgtttcatttattaccgttttggctg
ggggttagttccgacaccttcacagttgaagagcaggcagaaggagttgtgaagacagga
caatcttcttggggatgctggtcctggaagccagcgggcctcgctctgtctttggcctca
ttgaccccaggttctctggttaaaactgaaagcctactactggcctggtgcccatcaatc
cattgatccttgaggctgtgcccctggggcacccacctggcagggcctaccaccatg
(Seq ID No: 825)

Homo sapiens thioredoxin-related transmembrane protein 3 (TMX3) :

gcttctcttccgctccgggtcggctccgtttccctttccgggcgggcaggcggcggaccc
cagtgtctttatccctcttttgcacagtcagcttctgcagctctcccgggctagcatg
(Seq ID No: 826)

Homo sapiens ras homolog family member F (in filopodia) (RHOF) :

cgacctcttggctccgctagtgcccggcgcgccgccgccagtgctgcgggctccgggcaa
tg (Seq ID No: 827)

Homo sapiens amyloid beta
(A4) precursor protein-binding, family B, member 1 interacti ng protein (APBB1IP) :

ctttctctcaggaaactccactcccaactgacaggtgctatttccagccagtcctatgct
gttgcaaatagtgagtccatgaatgccctctgccgtgtgcattacttattttcatcagca
gatcttcgtaacacactcctggaagtgggatgacggggtcaaaaggcgaatccatacata
agttaaatagatattgctcaattctcttccacggggttcagaccattttggatttctacg
agcaatgaagacagtgctattcctctacaccctggccggccaactgagcgtggttaaacg
tggggagggaggagggtgaggttaccaacctgatggttgagaaagggcctccgcccagcg
cgcccttcctccacccccacccgagagacagctgaactccggccgggacgcgcgtgttgc
cagtccagccctgcaccgcgtcccctgagggcgggctgcaggcggccgggaagccttgca
caaccggcccaaaagaggaagcccagaaagtgctgaagtaaacactttgggagaccgttg
caacataaagcggcctctcagtctttggtggaaccatcactaggccccaatcccttagtc
cctcttgcgtcgaggctgcaaaatggttccattcgccaggagacgctcctgagagaaggg
cgcgcgcggcacaggggccttccttgcacctcggagcaaagcagctcggatagcgccaca
cgtctgcgcgctgcgtgggaagggcagggctgacagcacttcctccccggggcagcgacc
tggagcccgggtgcggcagtctgcaccgcgcgtcgctttcccggccggagtctcgccgcc
ttcccgcgccccgcagcgccccgcagagcagtcgagatg (Seq ID No: 828)

Homo sapiens roundabout, axon guidance receptor, homolog 4 (Drosophila) (ROBO4) :

ccttccctcttcactgtgagctcagagcagcaggacaaagtgctcgggacaaggacatag
ggctgagagtagccatg (Seq ID No: 829)

Homo sapiens translocase of outer mitochondrial membrane 7 h omolog (yeast) (TOMM7) :

acctcctttccctttcggattcccgacgctgtggttgctgtaaggggtcctccctgcgcc
acacggccgtcgccatg (Seq ID No: 830)

Homo sapiens major histocompatibility complex, class II, DR alpha (HLA-DRA):

ttttcttttattcttgtctgttctgcctcactcccgagctctactgactcccaacagagc gcccaagaagaaaatg (Seq ID No: 831)

Homo sapiens protein arginine methyltransferase 8 (PRMT8): cctcctctactatctcggtatcaccaaacccttgccggctcttatg (Seq ID No: 832)

Homo sapiens adducin 3 (gamma) (ADD3):

ctgcctcttatgaagcaatactagagaggaaaaacaaaacccattcctttaagaaagatt ccgcctcctctcataagcaagcgcctaatggtaattgtagagtttactaagtcaaacact tactactcagcattgagagaagctgctgctgctaatgctgctgctgctgctgccgccgcc gccgctgctgctgctgctgttggtctgaggctgcagtaggtttctgtgcagcattgcaga atccacacctagagaacagaagacacagacacgtacgtctactacccttgttagaaggaa

gctttggatcttcggtggataacaagagtaatccacagacttaaaacatg (Seq ID No: 833)

Homo sapiens BarH-like homeobox 1 (BARHL1) :

agcccttttggatctaatgcgcagaggaggttggcccagagctcccgggctcccccaagg ctgaactccgtccaaggtgcccgcaggctccctgcccgccttccccatgccagcccgcag ctaggggcaggggcagcggcggctggggttgggggtgggtggggagcttttggggaggac aggtcgcagcttggctatg (Seq ID No: 834)

Homo sapiens intraflagellar transport 46 homolog (Chlamydomonas) (IFT46):

ttatcttttgcctagcgactgacaacaggctggttgcttggcgtggaatcctaaagtgg cctggctttgagactggagtgagaccccagccctaggctggggttctttccattatagag gagacggattcagaaggctacagaccaaggttgttgaaaaccagacatatgatgagcgt ctagagattaacgactccgaagaggttgcaagtatttatactccaaccccaagacaccaa ggacttcctcgttctgcccatcttcctaacaaggctatg (Seq ID No: 835)

Homo sapiens carbonic anhydrase X (CA10):

cccccttttcgggaggagggaggcagggacttgcaggcaagagttgcacctggtctagga
acctgcagagaaaagaactctggggtaagtagtgttctggcactggcacggaaaggggta
aagggtgggggcatgagagggacgaaatggagagggcagggaatgaattatgcaaaaaa
atctccaatatttcgcagcggagggagagcacagcacagcactcccaggatgagtcctgc
ctgggtctcccgcgccgaacccgcagcacgaagttctttttaagaagagaaactcgaaaa
tcctggagggtaacagaggcagccagggcggggcggagtgcggaggcggctgccagggac
tggggccgaggcggcggccaaggtggcctgaagctgtgacacccagcctcctcctcctcc
tcctcatggccgcgctcagcctcacctccccgcccgggcctcctgcctccgcccccgggt
gccgggctgcggagctgacgctgggacgcccggcggcggcgaggacgctcacctggccaa
gcctccttctcctcctcccccctcccgcccccacctgtcctcctcctctctgagttgggaa
gcgtagggatccgtaggcgaggaaataacgacccctgcagttgtattgcggaaaatctcg
acagcggcgctagttgcgggcgatggaagccaggcaactgggggttctggggagttcagg
aaaatagcagaggagcaggaagggcgcgcgcgacctggagagtctgtgtgcccccaccgc
gccccagtccccggggcccagcccttcccctcggcgccctggacgcactgccggaacccg
gctgagaggctgcaggctgcgcgcggacctggggagcagggagggtcggcggaggctgcc
ggcggctggcggtttcgggcaataatccctgcctctctttctctgtgtgtctgctgtgtc
tgctccttccccgccccccggaagcaggagaagaactgccccggagcgcagcagccaccc
tccgaccatgccccggtgagggggggcggacttcgagggcaacttgccgcggactgcctgg
gcttagccagcgagctacgcgctcccgggagcccggaattgcacggcgcagcccggcggg
gggctatcgtctatgtcttcttggggcgccagacgaatcggggtctcgtttttgctggaa
gagcccagtgttggtggcttcaggtggctgctgccgccgccgccgccgccgccgctgcta
gtgcggtttccgccgctggtgcgaagagaagagacacgcgagcggggagacctccaaggc
agcgaggcatcggacatgtgtcagcacatctggggcgcacatccgtcgagcccgaggga
gatttgccggaacaattcaaactgcgatattgatcttgggggtgactgtccctggccggc
tgtcgggtgggagtgcgagtgtgcactcgctcggaagtgtgtgcgagtgtgtatgtgtgt
gtgccgtgtcgggctccccccttccccccgttttcccgtcgagtgatgcacttggaatga
gaatcagaggatg (Seq ID No: 836)

Homo sapiens dual specificity phosphatase 22 (DUSP22):

cctcctccctgtaacatgccatagtgcgcctgcgaccacacggccggggcgctagcgttc
gccttcagccaccatg (Seq ID No: 837)

Homo sapiens olfactomedin-like 3 (OLFML3): gttccttctactctggcaccactctccaggctgccatg (Seq ID No: 838)

Homo sapiens phosphoribosyl transferase domain containing 1 (PRTFDC1): ccgtcttcccttcccgcgttccccgggagaaacatg (Seq ID No: 839)

Homo sapiens translocase of outer mitochondrial membrane 22 homolog (yeast) (TOMM22): cctcctttccgcttccggtgtcccctacagtcatg (Seq ID No: 840)

Homo sapiens arrestin, beta 1 (ARRB1) :

gctcctcctgctggctggggatttccagcctgggcgctgacgccgcggacctccctgcg
accgtcgcggaccatg (Seq ID No: 841)

Homo sapiens cytokine induced apoptosis inhibitor 1 (CIAPIN1) :

cctcctctcgcgagaggcgcaaggcgtggagtcgacggctggagagaagccgggagcgag cccaggcggcagtcttgattccctttggccagcagtttttaggtctgtcagtactgcac tgcaagaatg (Seq ID No: 842)

Homo sapiens leucine zipper transcription factor-like 1 (LZTFL1) :

taccctccttccccatttctgtggtccaactaccctcggcgatcccaggcttggcgggg caccgcctggcctctcccgttcctttaggctgccgccgctgcctgccgccatg (Seq ID No: 843)

Homo sapiens phospholipid scramblase 4 (PLSCR4):

agccctcccttccgcgcgcttactttgtttataacttgaaaatcctctccgtctccctt ccctgcctcctttcctttccctttcctctgccagtacaactagacccggcgtctggcgtc cccggtgcccagcattctgcggggcaggcggattaattggaattcttcaaaatg (Seq ID No: 844)

Homo sapiens ectonucleoside triphosphate diphosphohydrolase 7 (ENTPD7):

cctccttccggctgggcaaggggccgcggggagcagctcgggactgaaccgagaggtgcc gaaggaaccggcgggccgcttgatcccgctgcagacgtaggagatgcctgggacaaggag gccaccttctcagggcaaaagaaaaagaaggtgacaggcgttgagaccaccgaagggaac ccatg (Seq ID No: 845)

Homo sapiens fascin homolog 3, actin-bundling protein, testi cular (Strongylocentrotus purpuratus) (FSCN3):

agttctctctgggaacatctggtgggtactacaggccctattccaggccctatggcctgt ggaacctcaccacgggggggagggctgggccagacggagacatcacctgtggtgtcagcc ccatg (Seq ID No: 846)

Homo sapiens X-prolyl aminopeptidase (aminopeptidase P) 1, soluble (XPNPEP1):

cctccttcgcgccggcccttccgcgggtgatcagctggtctgcgctcccctgacgtgggc tggggcacgtcaccgccgaatg (Seq ID No: 847)

Homo sapiens REX4, RNA exonuclease 4 homolog (S. cerevisiae) (REXO4) :

gggtctcttccggagtcttttcctggacggggtccctgcggtgggtgtgtttcggcctgg cctgggcaggcgcttgtgctgccagggcgccgggcccggggaggccggggtctcgggtgg ccgccggcccaggcgctggacggcagcaggatg (Seq ID No: 848)

Homo sapiens LYR motif containing 4 (LYRM4): ttttctttccaaaatg (Seq ID No: 849)

Homo sapiens DEAD (Asp-Glu-Ala-Asp) box polypeptide 24 (DDX24) : ggttcttcactcgcgactgacggagctgcggtggcgtctccacacgcaaccatg (Seq ID No: 850)

Homo sapiens transmembrane protein 159 (TMEM159):

ccttcttcctcttgttcctcctcctgcctctcttcgcttcgcctgcaaacgcggtggggg ctgctcggcggtcaggagcaggttaccctccgtctgcatgcccaccatcaaggtatgagg atggtagaagctctcgtcgaaccagatggatgaagaccactaacggcttttgtttcctct ggtaacagcaagagacagagcgacatgagagattggaccgcgggctgcactggagaattt actggtaggataattcatccctaaagagattgaagtgagcttcagaatg (Seq ID No: 851)

Homo sapiens NDRG family member 4 (NDRG4):

cggcctccgcccctgcagccgcgggcacgcggaggggctcctggctgcccgcacctgcac ccgcgcgtcggcggcgccgaagccccgctccccgcctgcgcgtctgtctcgtccgcatct ccgcggcctcctgctccacgacgtgaccatg (Seq ID No: 852)

Homo sapiens pre-B-cell leukemia homeobox interacting protei n 1 (PBXIP1):

ttttcttctcgggctgcaaacaaagggaagcctgcaacaagttaagctgaagaccgaagc aagagctggttcaggtggcagccacagcagcctcagggacctcagcaactatg (Seq ID No: 853)

Homo sapiens twisted gastrulation homolog 1 (Drosophila) (TWSG1) :

ctgtctctttaaggtgcccgaggctcgcgggcgctgcgctgaggggacggcgggaggcgc ggcctggcctcgcactcaaagccgccgcagcgcgccccgggctcggccgacccggcgggg atctaggggtgggcgacttcgcgggaccgtggcgcatgtttcctgggagttactgatcat cttctttgaagaaacatg (Seq ID No: 854)

Homo sapiens zinc finger protein 286A (ZNF286A):

gtcccctttgtgaggcccgggatgggaggtgcccggttcccccagggacagcttcaagcg gtagggacagacatctgaggacccagcctcagggatgctgtccccgggcttccaggctcc agcgccgtaggactgaggcagactccacggtgagaaagagacccgatctaacccaggcct ttcatcagagcccaggagggaaggcaggaagtgggaccacgaggcccgggggcttctaa ctcgtctggccagggagatctgaattggggtgaagagcagaatctccagaacaaggagga ggtggtgatcatg (Seq ID No: 855)

Homo sapiens S100 calcium binding protein A14 (S100A14):

gctcctcctgtcttgtctcagcggctgccaacagatcatgagccatcagctcctctgggg

ccagctataggacaacagaactctcaccaaaggaccagacacagtgggcaccatg (Seq ID No: 856)

Homo sapiens ANKHD1-EIF4EBP3 readthrough (ANKHD1-EIF4EBP3):

tgctcttctcgttcccgagatcagcggcggcggtgaccgcgagtgggtcggcaccgtctc cggctccgggtgcgaacaatg (Seq ID No: 857)

Homo sapiens KIAA1143 (KIAA1143) : ctgtctttacccagagctaccatg (Seq ID No: 858)

Homo sapiens neuroligin 4, X-linked (NLGN4X):

ctctcttttcttgcagaaccgtctctctcccttctctgtctcttagcacagagctctta
ttcagccactagcttggcccttcctgcttcaattgtaatgcttgttctgcccgtccacag
actattggcggcagaaacaacgaatttcctccaaactaggcggtgttggtggctcttgca
ttcctctggatgaggaaatctagttgggggttccagaaggggaaggctcctgggctttc
aatacatcctcctgaatcatacctcgtttcgggttccctagaaaaatctggacgtgtaaa
aagaactcttaacggccgatgcagctcttccaaagctaaggctgccttggagttttcata
agaaattgtccctggaggtgttggatgatcacagcttccttggagcattgcagttgctgg
aatccagtttcaggattaagggagggctgcctccttgcaatgggctgccaagaaaacggc
tgtgcttgttcttaacctcaggctctgtctgtgatcagtctgagagtctctcccaggtct
actgctccctggaaagccctatctctctgcaggctcgcctctgggctttgtctccttgga
gccacatcactgggacagctgtggatgtggatgcagatttgaaccatg
(Seq ID No: 859)

Homo sapiens mitochondrial antiviral signaling protein (MAVS) :

ccgcctcctcgctgcgggaagggtcctgggccccgggcggcggtcgccaggtctcagggc
cgggggtacccgagtctcgtttcctctcagtccatccacccttcatggggccagagccct
ctctccagaatctgagcagcaatg (Seq ID No: 860)

Homo sapiens serine incorporator 1 (SERINC1): ctgtctccatcttgtctgtatccgctgctcttgtgacgttgtggagatg (Seq ID No: 861)

Homo sapiens KIAA1324 (KIAA1324):

cctcccctttttttccgccttctgccagcagaagcagcagccgcagcacctgagccgcta
ctgccgctcactcaggacaacgctatg (Seq ID No: 862)

Homo sapiens synaptotagmin IV (SYT4):

ggacctccctctttgcctcctccctgttccaggagctggtgccctgggctctgcgctgtt
gttttcagcgctccgaaagccggcgcttgagatccaggcaagtgaatccagccaggcagt
tttcccttcagcacctcggacagaacacgcagtaaaaaatg (Seq ID No: 863)

Homo sapiens pyruvate dehyrogenase phosphatase catalytic sub unit 2 (PDP2):

cttccttctggagctgggtcctgactagggaccgcctgggtgaggtgaggacctggtggc
cgcagttgtggcactgtgcgcaggcgctgaactgaccggacggagcgggcggctgtggcc
tcgccagctggtttaaaaatatccttttttgctgaaggaacacatttgctggtatagttt
cagaatg (Seq ID No: 864)

Homo sapiens gephyrin (GPHN):

ctatcctttcctctcagtcctgccatctagctgccttgggtctcgcgctccgcagagcgt
tccgacactctccggcctcgttctgccgcctccgcgcgctctccccgtgcggccaccgcg
ccccccaagcttgcctccttcttgccggacttggggccgcgcgccctgactccttcccct
cccgcggacccgcgcactcccggcgcggcctctcccccacgcaggccaccgtgcactctg
tggcctccccctccttccccgctctcctcgcgcttctctggctccctagctgtcgcgctc
tcctcggcgagcgcgctcccggcccgcgcgctccgggctccggtttctcccggctcctgt
cagtgcggtgactgcgctgggaaacatg (Seq ID No: 865)

Homo sapiens deltex homolog 2 (Drosophila) (DTX2):

ccttctcctgagagtcggagccacagccagagccctgcccaggccgagccggagctgcag
cccgagcgcggtggtgccctcagccccgtcctcttgtcctcctcagcctcggtgccttgg
aatttgtgtcgctgagtcagcaagcctttcagatttgcccggtttttgttgtttgtggtt
tgtatcaagatgggaactcaaacaagtcattcctcctaaggagctggtgtcttcatccag
aagggacagtttgtgccagctctccagagagaaaaggatctggtactgttctggagtggc
ctgtagcagacactgaaccaccagccagctgcatttgttgtcctggaagtcattgccaac
tctgccagtcacactggggtccccagagaagtcaagatctgccggaggcgctggcaatg
accccgggactccaggccagaggggtctgaagctgtttgggaaagcagcgggactccttg
ggaagatg (Seq ID No: 866)

Homo sapiens melanoma antigen family E, 1 (MAGEE1):

ctgcctttttcaccacctctaatttcagcttcagcagttgcttggaactttggttctggc
agcagcagcaacatcattaccgctagcggcagttttgtgccgaggcacctacacacctcc
cgtcctctctgccagatcgcgggcctgtcggtgtctgctcctacacgccaacgccggtgg
gcaggaccatg (Seq ID No: 867)

Homo sapiens G protein-coupled receptor 107 (GPR107):

cgccctttcaccccggacgtgggcgggagaggaagcggctggtgatgctggaacaaacat
g (Seq ID No: 868)

Homo sapiens PDZ and LIM domain 1 (PDLIM1):

cgctctttctccgacagctgccggggggtgccctgcaagctgttccgcgcgtcctgcccgt
ctgtccccgcgggtcgtcgcccgccacagccgcgccatg (Seq ID No: 869)

Homo sapiens thymosin beta 10 (TMSB10):

cgctcttttgtttcttgctgcagcaacgcgagtgggagcaccaggatctcgggctcggaa
cgagactgcacggattgttttaagaaaatg (Seq ID No: 870)

Homo sapiens phospholipid scramblase 1 (PLSCR1):

agacccttttcagacccttttccggctgacttctgagaaggttgcgcagcagctgtgccc
ggcagtctagaggcgcagaagaggaagccatcgcctggccccggctctctggaccttgtc
tcgctcgggagcggaaacagcggcagccagagaactgttttaatcatg
(Seq ID No: 871)

Homo sapiens eukaryotic translation elongation factor 1 beta 2 (EEF1B2) :

gggtccttttcctctcttcagcgtggggcgcccacaatttgcgcgctctctttctgctg ctccccagctctcggatacagccgacaccatg (Seq ID No: 872)

Homo sapiens pyrophosphatase (inorganic) 1 (PPA1) :

ggctctctccttgtcagtcggcgccgcgtgcgggctggtggctctgtggcagcggcggcg gcaggactccggcactatg (Seq ID No: 873)

Homo sapiens X-ray repair complementing defective repair in Chinese hamster cells 5 (double-strand-break rejoining) (XRCC5) :

ggctctttccgctatctgccgcttgtccaccggaagcgagttgcgacacggcaggttccc gcccggaagaagcgaccaaagcgcctgaggaccggcaacatg (Seq ID No: 874)

Homo sapiens GATA zinc finger domain containing 1 (GATAD1):

gatccctttcccagtcctgcttcccagtgcctcgggccagggaatcctggcctccgcctg cggagccggcggaacccgcttcccgcctccacggggcagcgccagcggcctggtcctttc accggcagctccgtgccgacgctctcaccgctcttcctatcgccgggagtggcgggccga ccaggggcggccgggctaccgtccgccattcccgtgtctctgcgcccgcgggggccgcc cgagccggccaccatg (Seq ID No: 875)

Homo sapiens enolase-phosphatase 1 (ENOPH1) :

ccgccttttccagttccaggtgtgcagaagtgtcctctccccacgcgcggcgggctgcac ttggtcgctggctccgagatcgcgcggggccgccggaagcccaagacggtaccgggggcc gcagccgcagccggcgccgccctccgccctccccaacagcaggccgagtcccgtagcatc cggtagggaaatg (Seq ID No: 876)

Homo sapiens regulation of nuclear pre-mRNA domain containin g 1B (RPRD1B) :

agctctttccgggggcccggggaactactctccttgcctcgctctgtctccttcgaagtg ctctgcgcgaggttcagagcggccgccgcctccaaagggacggttttctagagctccgac gcctctcggtgcccctctgctccggcccttgccctttgacctcgctctcgcggcagggtg agaggtcgggtggccatcttgtggcggcggcgcgggcggctgttactgcggagacccatc cccTCcccttctcgcacccctggcagtctgtcagtcggtaaaaagtcccgcagcctgtc aggtgaggccccggcctcgtgccgtcgctcttcccgccgcactgggcggcccaggccgct ccctgccgggcctcactgccgccaccatg (Seq ID No: 877)

Homo sapiens family with sequence similarity 60, member A (FAM60A) :

ctatctttctagacaaggcagttgaggaggagggagcgcttgaggggactggcctggcg tgcactccgcacctcggggacattattgcgcgtggaacggctgcttttggaaggcacaac ttcctgaatggaccatgactcccaccaaagatccctgtctctgattcaccaaacagcttc aaccctgaaaccaggacgagaagttgacaacatctgagtggacagctaattgacctaaga cttcagaccagactattgcccagaagaaaagatg (Seq ID No: 878)

Homo sapiens MIDI interacting protein 1 (MID1IP1) :

gggcctttatctcggtgctgccgggggaggcgggaggaggagacaccaggggtggccct
gagcgccggcgacacctttcctggactataaattgagcacctgggatgggtaggggcca
acgcagtcaccgccgtccgcagtcacagtccagccactgaccgcagcagcgcccttgcgt
agcagccgcttgcagcgagaacactgaattgccaacgagcaggagagtctcaaggcgcaa
gaggaggccagggctcgacccacagagcaccctcagccatcgcgagtttccgggcgccaa
agccaggagaagccgcccatcccgcagggccggtctgccagcgagacgagagttggcgag
ggcggaggagtgccgggaatcccgccacaccggctatagccaggcccccagcgcgggcct

tggagagcgcgtgaaggcgggcatccccttgacccggccgaccatccccgtgcccctgcg
tccctgcgctccaacgtccgcgcggccaccatg (Seq ID No: 879)

Homo sapiens transmembrane protein 35 (TMEM35):

ctctccctttgtcattctagctgcctgctgcctccgcagcgtccccccagctctccctgt
gctaactgcctgcaccttggacagagcgggtgcgcaaatcagaaggattagttgggacct
gccttggcgaccccatg (Seq ID No: 880)

Homo sapiens Fc fragment of IgG, low affinity IIa, receptor (CD32) (FCGR2A): cttcctcttttctaagcttgtctcttaaaacccactggacgttggcacagtgctgggatg (Seq ID No: 881)

Homo sapiens tribbles homolog 2 (Drosophila) (TRIB2):

ctttctcttttgtttggcttctaacgcgttgggactgagtcgccgccgtgagctccccg
aagactgcacaaactaccgcgggctcctccgccccgtctgcgattcggaagccggcctgg
gggtcgcgtcgggagccctggcgctgcagctccgcaccttagcagcccgggtactcatcc
agatccacgccggggacacacacacagagtaactaaaagtgcggcgattctgcacatcgc
cgactgctttggggtaacaaaaagacccgagttgcctgccgaccgaggacccccgggagc
cgggctcggagcagacgaggtatccggcggcgcccatttgggggcttctaactctttctc
cacgcagcccctcttctgtcccctcccctctcgctccctttaaaatcagtggcaccgag
gcgcctgcagccgcactcgccagcgactcatctctccagcgggtttttttttgtttgtcg
tgtgcgatcctcacactcatg (Seq ID No: 882)

Homo sapiens family with sequence similarity 3, member A (FAM3A) :

cgtcctctccgggggcggagcgggtcggcgggcctgacagggaacctccctgaccgagcc
cacgtctccccacggccagagaaatctccggcccggcccgcatcgccagcccccaggccc
ggaggaacggcccgagcccaggagaaccacatcttcgtcccagccccggaggctcctgtg
ggcaagatcgtgagccaacgggttcctgaggcccctcctggccaggcagggtttccccgc
gcgtttccgaggagccctgcctggccgggcggctggacaaacaggtcgtagcaccgatcg
cgcccgcccccagcaggggtcccgcacaggcttgcccctgacccccacccaaacctgtcc
ttccgctttgcccccaaacagtgcacttgccggcggtcccaacccagcaggagaagtgga
catg (Seq ID No: 883)

Homo sapiens exocyst complex component 4 (EXOC4) : ggctctccccgcgtccaagatg (Seq ID No: 884)

Homo sapiens ELOVL fatty acid elongase 5 (ELOVL5) :

gcgccttcctcttcccatcgcgcgggtcctagccaccggtgtctccttctacatccgcct ctgcgccggctgccacccgcgctccctccgccgccgccgccttgctgctgctcaaagctg ctgccgccccttgggctaaaaggttttcaaatg (Seq ID No: 885)

Homo sapiens apolipoprotein B mRNA editing enzyme, catalytic polypeptide-like 3G (APOBEC3G) :

ctttctctttccctttgcaattgccttgggtcctgccgcacagagcggcctgtctttatc agaggtccctctgccaggggggagggccccagagaaaaccagaaagagggtgagagactga ggaagataaagcgtcccagggcctcctacaccagcgcctgagcaggaagcgggaggggcc atgactacgaggccctgggaggtcactttagggagggctgtcctaaaaccagaagcttgg

agcagaaagtgaaaccctggtgctccagacaaagatcttagtcgggactagccggccaag gatg (Seq ID No: 886)

Homo sapiens gamma-aminobutyric acid (GABA) B receptor, 1 (GABBR1) :

gctcctcctcctcccctccgtcggtcagtcagtccgcgaggagagtccgcggtggcggcg acggtggcgagagccgcgggggccgtaggaagccaaccttccctgcttctccggggccct cgccccctcctccccacaaaatcagggatggaggcgcctccccggcaccctcttagcagc cctccccaggaaaagtgtccccccctgagctcctaacgctccccaacagctacccctgccc cccacgccatg (Seq ID No: 887)

Homo sapiens cofilin 2 (muscle) (CFL2):

cctccttctcctcccagtgccacagagccgaagcccgagctgccgccgcagccacagccg agggcactatg (Seq ID No: 888)

Homo sapiens DEAH (Asp-Glu-Ala-His) box polypeptide 35 (DHX35): tgaccttttaccccaacatg (Seq ID No: 889)

Homo sapiens resistance to inhibitors of cholinesterase 8 ho molog A (C. elegans) (RIC8A): ccgccttccccggcgcgccatg (Seq ID No: 890)

Homo sapiens FK506 binding protein 10, 65 kDa (FKBP10):

agttctttgtagtgcctccctcagactctaacacactcagcctggccccctcctcctatt gcaacccctcccccgctcctcccggccaggccagctcagtcttcccagcccccattcca cgtggaccagccagggcgggggtagggaaagaggacaggaagagggggagccagttctgg gaggcgggggaaggaggttggtggcgactccctcgctcgccctcactgccggcggtccc aactccaggcaccatg (Seq ID No: 891)

Homo sapiens small ArfGAP 1 (SMAP1):

cctcctcccgttccagctgccgctgccgcttcctggctgagtccgcccgcggtcccggc ggcgccaggtgcgttcactctgcccggctccagccagcgtccgccgccgccgtagctgcc ccaggctccccgccccgctgccgagatg (Seq ID No: 892)

Homo sapiens chromosome 14 open reading frame 93 (C14orf93):

cctcctttttgcacacacacgaatacaaagagccatacgaccttcggatgccggaaggtc cttctgaatcccttccctgttccttaggttgcactagtcggggttccatgctgggggc agaaggaatgctctctaccgtctgaaaccgttcatcaggaaggccttgatttgtgatgtg ctaggagagcacaggatctgcaaatagaaggcacctgtctcccttctgcaggccgaggag aggccgccatggactgtgtgcttcttcatggcttgtttactcttctttcacagaccctac agcttggggcctgggctcctctgaccatcctcattgagaaaggaaagtgagtccagagaa gttgatgcttcctacctgttggagcggcccagcagtgtaagcgtggttgttactgcccca tccgccatg (Seq ID No: 893)

Homo sapiens brevican (BCAN):

cgccctcttccgaatgtcctgcggccccagcctctcctcacgctcgcgcagtctccgccg cagtctcagctgcagctgcaggactgagccgtgcacccggaggagacccccggaggaggc gacaaacttcgcagtgccgcgacccaaccccagccctgggtagcctgcagcatg (Seq ID No: 894)

Homo sapiens H2.0-like homeobox (HLX):

cggcctctcttcctcagtgcgggcggagaagcgaaagcggatcgtcctcggctgccgccg ccttctccgggactcgcgcgcccctccccgcgcgcccacccacccagtccggctggactg cggcagccgcgcggctcaccccggcaggatg (Seq ID No: 895)

Homo sapiens v-rel reticuloendotheliosis viral oncogene homo log A (avian) (RELA):

ccgcctctggcgaatggctcgtctgtagtgcacgccgcgggcccagctgcgaccccggcc ccgcccccgggaccccggccatg (Seq ID No: 896)

Homo sapiens zinc finger protein 277 (ZNF277): cctcccttttcttttctgccgggtaatg (Seq ID No: 897)

Homo sapiens globoside alpha-1,3-N-acetylgalactosaminyltrans ferase 1 (GBGT1):

cttcctcttttctgtctggcccgcggccccgctgcctgccctgctccaggctccacctgc gccgccgatcgcccgggtatcgcgggggcccaggccagctgagtccgttttccgcgccgg ggtggcgcccctccaaccgtcctaacgccgggccggcagcaaggagtgttcctgggacct cagagaccaggctcagagcctgacatccctgcgaggggacagcctcatccgcccaggcca gtgggggtctctacaagtgcccaggctcaggtgcagcccccagcaatg (Seq ID No: 898)

Homo sapiens FXYD domain containing ion transport regulator 6 (FXYD6): ggtcctcctgggagtctcggaggggaccggctgtgcagacgccatg (Seq ID No: 899)

Homo sapiens nuclear RNA export factor 3 (NXF3):

tcctctctatgcttggggaaggaacttcctgtaagcaaggcttgaggcttgctctcgcct tcgtcagcagccctcctcaatcttctccaaactcccgtccccaggccacacagattctcc tcaagagagccctataaggacattggtaaaatg (Seq ID No: 900)

Homo sapiens chromosome 14 open reading frame 133 (C14orf133) :

attcccttccgccccttctctaagctgcacagcctgaatagaagggctggtccagcggc
ggcggaggctggcgctgtcctgagaggagggctctgtgcggaagagtcagggcgaccct
tgggcgctggagtacgcttgggactggggctgcgagtgagcaccagcgattggttcggaa
gcggacatttggttcagaacgagcatttaactctgccagggatccgctgggctctgacga
ctgcggtagatccatggcttcctggacgttcacccgtagagtcatcctagcttaactctt
gttccctggtctcagttcacaagcctcacctgtatcttcctggctcggaagataattgaa
accaagtctgacttctcaatg (Seq ID No: 901)

Homo sapiens X-prolyl aminopeptidase (aminopeptidase P) 3, putative (XPNPEP3): ctttctcttcccgacgcgtgagttaggccgtaatg (Seq ID No: 902)

Homo sapiens death inducer-obliterator 1 (DIDO1) :

ggccctctggcaagatggctgctgcggaggcgttggagcgcggaaatctggaaccgggat
ggcgacgtctacactgagtcggaggcgaaggagcttactccacgggaacagcctctagat
aatctgagttgttgaaaatacgaagcctgttactcgtgaacagtggctgacaacagtgtt
gttgtgagcctggctgtctgcttggacccagaggtttcgtctgccagggtttttggttgt

atttaggatttcagggaaaagtgtccaagctttcagtgttggagcaggtatg
(Seq ID No: 903)

Homo sapiens PERP, TP53 apoptosis effector (PERP):

cggcctcttcgctttgtggcggcgcccgcgctcgcaggccactctctgctgtcgcccgt
cccgcgcgctcctccgacccgctccgctccgctccgctcggccccgcgccgcccgtcaac
atg (Seq ID No: 904)

Homo sapiens tubulointerstitial nephritis antigen-like 1 (TINAGL1) :

tcctctcttgactttgagcgtccggcggtcgcagagccaggaggcggaggcgcgcgggcc
agcctgggccccagcccacaccttcaccagggcccaggagccaccatg
(Seq ID No: 905)

Homo sapiens eukaryotic translation initiation factor 4H (EIF4H): ggttcctctcggagcggagacggcaaatg (Seq ID No: 906)

Homo sapiens non-SMC condensin I complex, subunit G (NCAPG):

ccccctctcgcgggaattatttgaacgttcgagcggtaaatactccctggggctgtcata
gaagactactcggagagcgctgcctctgggttggcgggctggcaggctgtagccgagcgc
gggcaggactcgtcccggcagggttccagagccatg (Seq ID No: 907)

Homo sapiens MMS19 nucleotide excision repair homolog (S. cerevisiae) (MMS19): tatcccctcccacggtctctagttcgcgttatg (Seq ID No: 908)

Homo sapiens DnaJ (Hsp40) homolog, subfamily C, member 1 (DNAJC1) :

ctgcctctacagctgtgtgtaggcctgggggcgagggtcttcggaacgtagcgctggctg
cggccccgcccgcctacccacccgcccgtccggcagccggctcccgccgcctccgcgctc
tgtctggggccagccacctggcgggccgctccggtgcgcctgcccgcgcttttcactgac
aggcgctgttccccacagccagcgccgcccgccacgtcccagctctcggccaacggagct
gcgcggcgggtgacctttccgagcccagcgcgatg (Seq ID No: 909)

Homo sapiens stimulated by retinoic acid gene 6 homolog (mouse) (STRA6):

ctaccctttcatctctgcaactccttcctccctgggcctcccttctggtgtgtctgtggg
tctgtctaggtgggcttgggaaaggggaaggaaggggcgtctctttaggcagctcagact
ggacaagccttctttgaaaatggtcctttgaacacacgcctgctggtggttggtcagaca
gatgcgccagcgggagccccggggccccaaggggacagctatctctgcaggaccagtgcg
atg (Seq ID No: 910)

Homo sapiens 5-azacytidine induced 2 (AZI2):

cagccccttttccggctgagagctcatccacacttccaatcactttccggagtgcttccc
ctccctccggcccgtgctggtcccgacggcgggcctgggtctcgcgcgcgtattgctggg
taacgggccttctctcgcgtcggcccggcccctcctgcctcggctcgtccctccttccag
aacgtcccgggctcctgccgagtcagaagaaatgggactccctccgcgacgtgcccggag
cagctcccttcgctgtggaagcggcggtgtcttcgaagaaaccggaagcccgtggtgacc
cctggcgacccggtttgttttcggtccgtttccaaacactaaggaatcgaaactcggcgg
ccttgggggcggccctacgtagcctggcttctggttgtcatg (Seq ID No: 911)

Homo sapiens polymerase (RNA) I polypeptide E, 53kDa (POLR1E) :

acgccttttccggcccgcagcgcggcctgggctcccgcgtgtttaaaagtgcgcttgtgg
ctgctgctgtcttaactcctgtgcttggcggacagacaggcgagatg
(Seq ID No: 912)

Homo sapiens mitochondrial ribosomal protein S25 (MRPS25):

agtcctttctcgtcgctgctcggctcgcggcccgtggggtcggccccgccaccgttgccg
ccatg (Seq ID No: 913)

Homo sapiens TRM2 tRNA methyltransferase 2 homolog A (S. cerevisiae) (TRMT2A):

cggcctccgccgcacgcgctggcggactaagagtggctggcgaagcgagcggccggcgcg
ggcccctggcgggcggcggtacagccccaagcctgagacccggacctgagcatcgcagg
ttcgagtcccgccccgcctggggcgaagccgggggtggcggcgacctcgcggcgttgcac
cggctctgtgagcacctcccctctgagcacttcccttgtgacaggccacttcccttgtga
caggcccaggacgaggtggccaggcggcccccatggcgtccctggtctaggcggagaacc
gcctgggcgatg (Seq ID No: 914)

Homo sapiens lipid phosphate phosphatase-related protein typ e 2 (LPPR2):

ccctccctccacctcggagtctgcgcggcgcggccaggcccggccgaccgcgtctcggtc
ttcgcgtctgccagcctggctggcagtccgtctgtccatcccgccgcgccggggcagtct
aggcggagcgggggctcaggcggcggcggcctcgacgcgagtgagtgtcgtggttggggt
gctggacccagagtgcctaccctcgcctgcctgggcctcagtttccacatctgcacaatg
ggggtgaccatccctgccctgctggctgccaggagcggctgtgagtcttcaggcgtggat
gcagcctgggggaagccatagggcgctttcacaggcctggccttcaccatg
(Seq ID No: 915)

Homo sapiens chromosome 11 open reading frame 1 (C11orf1) : gaacctttttcacctcgtctgaaatg (Seq ID No: 916)

Homo sapiens microtubule associated monoxygenase, calponin a nd LIM domain containing 1 (MICAL1):

cgccctcccacccgctcagacctggttgccagcccaacaggaagcggcccctcccggctt
cggagccgccgccactcatctctgcccagctgctgccctccccaggaggcctccatg
(Seq ID No: 917)

Homo sapiens kinesin light chain 2 (KLC2):

gctcctttaaggcagcgaacgggccaagagaagcgtgtttcgccccctccgacgccaccg
aggtagcggcttcacctttaaggcggcgcgggggctgctgggaaggccggcgggatggag
gcggcgggaccggctcgcgggtgcgggtccgggtgaagcgggaggcagccagagtcggag
ccgggcccgagcaccaggcgcaggcccggcgcccgcctgcccgcaccctcgtcctcacag
acgccacagccatg (Seq ID No: 918)

Homo sapiens DNA cross-link repair 1B (DCLRE1B) :

acttcctttttctgcccactctggtaacttattgctctgctgggctctttcccttagggt
ctctggccctgttcttgccccagcatgacttttatcgggacgccgttgtggaagcctcac
gcaggagccctgcccccgtggagaagatcccactggtgactccaaccctaccaccatg
(Seq ID No: 919)

Homo sapiens armadillo repeat containing, X-linked 5 (ARMCX5) :

gctcctcccactgccgttgtgggtaacgcggacgtggaagaacctcgtctgcggaggaaa
aggtagatgttaaatggtaactacgcgcgaggttctgaggagccctgggaacaggaagga
gaaaagaataccaaaagtgacaacagtttgccaatcgcagtctttaatctgataaagcgg
ttatctcgtcttgagtcccaggtgccgagtcaatccccatacacagccgccgccattgcc
tcgagtccttgtgtctgactgtctgttcctgctgctgtatgacacagcacctcgaggcaa
ggaaataagaaaactgcctctgatccaagcagagaaggtctgcctgtagatctgctgtag
ggcttgtcaccattggaagcaaggtcctacttcagtggcagatctggtggccttggagtg
gctgaagaccaccaccctccacagggctgggcccatgcacagccatccttccctaccttg
agtgagcttcctctgcatgttttctatatcactggcagagcctgtagttggaaaggggac
agagtgactactggactttgtgtgaaaacaccaaccgggacaaaacttcagtcaaggctg
agacgggtgggggtatataacttgtccttacgttaaacttggaacatg
(Seq ID No: 920)

Homo sapiens chromosome 12 open reading frame 43 (C12orf43): aatcctttgcggtggttcaagatg (Seq ID No: 921)

Homo sapiens vacuolar protein sorting 33 homolog A
(S. cerevisiae) (VPS33A):

ggtcctcccgtaggaaccggcggactcggttggcgttgtggggcagggggtggtggagca
agatg (Seq ID No: 922)

Homo sapiens arginine/serine-rich coiled-coil 2 (RSRC2):

gggcctcctcgcctttgtgccatccgggtctctcgcgcgagcgatttagtctgaggcgaa
gcttcggagcggccggtactgttgaaagcgacaagtggaggcgccgctctagcggccggg
actctgaactatggcggctagtgatacagagcgagatggactagccccagaaaagacatc
accagatagagataagaaaaaagagcagtcagaagtatctgtttctcctagagcttcaaa
acatcattattcaagatcacgatcaaggtcaagagaaagaaaacgaaagtcagataatga
aggaagaaaacacaggagccggagcagaagcaaagagcgtgcttatgcgcgaagagactg
aactgaagacgctgcagactcagatagcaaaataataagcctacttcatgataagggaag
aagacatgaatccaaagataaatcctctaagaaacataagtctgaggaacataatgacaa
agaacattcttctgataaaggaagagagcgactaaattcatctgaaaatggtgaggacag
gcacaaacgcaaagaaagaaagtcatcaagaggcagaagtcactcaagatctaggtctcg
tgaaagacgccatcgtagtagaagcagggagcggaagaagtctcgatccaggagtaggga
gcggaagaaatcgagatccagaagcagagagaggaagaaatcgagatccagaagcaggga
aagaaaacggcggatcaggtctcgttcccgctcaagatcaagacacaggcataggactag
aagcaggagtaggacaaggagtaggagtcgagatagaaagaagagaattgaaaagccgag
aagatttagcagaagtttaagccggactccaagtccacctcccttcagaggcagaaacac
agcaatg (Seq ID No: 923)

Homo sapiens integrator complex subunit 3 (INTS3):

ccgccttcccacccccgcccttccactatggccgcttctgtgtggtgtggggagacgct
ggtcctccccgtcctcccatagcgcttattgcctcaccctcacccccctaggggccggatc
caaaggcgctgcactccccaagccttggggcatcagccaggaaggtttcctacctcctaa
ttcaggggcaggactcctcttttccccccacggggaaaagaggcagaaacttaggggttt
ccctcctttcttagggtcagacgctcttagggtccacttcttcaggggcggaagcctctc
ctacccttcccataggggcacaggcctttaccccactgtacttcggagccaacgcctttc
cctcagcactgccaccccagagtcaggacccagaggactgtgccttcgcccccaacgcag

gcgcggccttttggagaggagggaggagtggagaggacaggggcccttgctctcccctcc
ccaacttgttcctcttgccccccagtccctggcaatccagagatcccgatatctaggact
gtccatccatccactccctgacctttcccggctcctggctgcagccatg
(Seq ID No: 924)

Homo sapiens spermatogenesis associated, serine-rich 2 (SPATS2) :

tctcctttcctcttctcagacccgggagcgtccgggacgcggagcccggagctggggcga
cgaggcgattgcgggggcctgggctagctgctggctaccaatattctactttctgtctct
atgaatgtgactaccctggttacctcatataatctccctggaaaaggagacatgaatgtc
tgcaatgatacttcctgacaagaagttgatacaagaaaaggaaaggagattaacagctag
tgagcagaatttcgaacagcaggatttcgtatttttttgcttccaactgcacacttccgtt
gcccacttttaaatcagagatacctacactcaaaacccagacaaggcaaaaggatacttt
tcttgtatattttttgagatcgaagaaacgacaatg (Seq ID No: 925)

Homo sapiens fibroblast growth factor receptor 1 (FGFR1):

ccgcccctttcacctcctggctccctcccgggcgatccgcgccccttgggtctcccctcc
cttccctccgtccgcgtctcctgcgcccctccctgcgctcgtcccgccgctcttcccgc
cgcccaacttttcctccaactcgcgctcgggagctggcgaggcggcggcggctcctcagg
tcagtttgaaaaggaggatcgagctcactgtggagtatccatggagatgtggagccttgt
caccaacctctaactgcagaactgggatg (Seq ID No: 926)

Homo sapiens FUN14 domain containing 2 (FUNDC2): ctccctcttccgctgccgccgtgggaatg (Seq ID No: 927)

Homo sapiens ganglioside induced differentiation associated protein 1-like 1 (GDAP1L1) : cctccttctttcctgcctctgattccgggctgtcatg (Seq ID No: 928)

Homo sapiens chromosome 19 open reading frame 43 (C19orf43) : agtcctttgcgcggcacctggcgacaaaatg (Seq ID No: 929)

Homo sapiens MIS12, MIND kinetochore complex component, homo log (S. pombe) (MIS12):

ccctctcttctccaccagccaacgtccgggaaaaacgagtaagtacaggttccttctgcc
aatccccgccggccacagctaactttcccgcccggcccctttctgtcataattgaggtgt
ccacaaccagccaatcaggaacgcgagagtatcccgcgtttgctttcgctcgccgaggcg
cgtatcagtcggaattttggggagccaaccgcgccgtctgtccctggcaagccagcggcg
gtttaaaggaggtggcgggaagcctgtgtgtgcttcaaatcgtcaccctcatggtcgctc
cggtaagtgctgcggggcagcattttctctgaggaggagcggggacgggcgagactggca
taagcgtcttcgcgagggagcaaggcggcctgtgggtcggcctcaccccggcctccgacc
tgaagatcccagcatgcagcgcgggcgcggggcccgacggaagccgggagccggccggaa
gcagttcctgcgctctggcttctgggtcctgtcctgcgcgatcgcggggtcttagacagc
tcaactcgccgagatgacctgggcacctctgcgttgaatcggcaaatactgatcaagccg
catttattctgctctcaggaactctaagtctagcagagaagatgaggcggtagaagttca
tcaatggcttggctggaggacaagcaaattgaggacattggcaacggagtgatcaaaatg
atagatcatgaggcctaaaatgaataaggaaagaagagaagtggcagaggctgagaacag
aaagagagggtggaggggctgtaaatcttgaagattagggtataatatgagtatatgggt
aagaattggaagaattgtgtaggaggcagtagtcaaaaagtagaagcagtttggaagagt

agttacaaatatcaagagccaggtggctaaaaggtggagctataggtcattgaagctcaa
gaaactgagtctctagggcattggttaagtcatctgtctagacttcaaagttgtctagga
tgataattcagaagactgatctgtgccaaagtcacaggtttttcacgactgaaaacaaca
tagcaaaataagccaagatg (Seq ID No: 930)

Homo sapiens DEAD (Asp-Glu-Ala-Asp) box polypeptide 50 (DDX50) :

cttcctttcacgctgtcgctgcccgtaggtggttgtggccactgtgcccggagggaggcg
gcggtggccagtaatg (Seq ID No: 931)

Homo sapiens chromosome 7 open reading frame 25 (C7orf25):

cggcctctgcgtgcacgcgcctgcgtgctcgcgctcgcggttctggcgctgccggaataa
tgctgacagcatg (Seq ID No: 932)

Homo sapiens KxDL motif containing 1 (KXD1):

ccgccctttcctgtcgtgacttaacgcacgcaagcggctccagggtacgtccccgccacg
cgcgctcgcaggatcggtgcgtggtgacgtttcgccggcgcgggcgccatcccggaagcg
cgagcaaggccgccagatgtgcaggcagcggaggaggagaaagagatg
(Seq ID No: 933)

Homo sapiens defective in sister chromatid cohesion 1 homolo g (S. cerevisiae) (DSCC1):

acttctttcttgcccgccaagcccgcagccacccgggcgcggcgggactcctagacccgg
cgctgcgatg (Seq ID No: 934)

Homo sapiens zinc finger protein 426 (ZNF426):

cgttcctttgtgacgccggctgtgagcgcctgagagtctttttgcctttcagagttaag
gcctcactggcctgggaaaataattgctgcctttgcatccgcgttggctccgtccccag
gatcttcccggttcagggacctggcgatttctgagtgttccggaatcccaataaccctgt
ttaaagaggaatggagattgccactgtccatttagattaatgaggtgtcctgaagtgatg
gtgacatcaatgaaaggagggttctgacacgttctcacctcgcgggatg
(Seq ID No: 935)

Homo sapiens TATA box binding protein (TBP)-associated factor, RNA polymerase I, D, 41kDa (TAF1D):

caacccttttcttccgcacggttggaggaggtcggctggttatcgggagttggagggctg
aggtcgggagggtggtgtgtacagagctctaggacaccaggccagtcgcgggttttgggc
cgaggcctgggttacaagcagcaagtgcgcggttggggccactgcgaggccgttttagaa
aactgtttaaaacaaagagcaattgatg (Seq ID No: 936)

Homo sapiens PHD finger protein 1 (PHF1):

ccgcctcctcctcctgccgctgccgctgctttggctgctgcgtcatacgccccagagccg
ccgggacggaggggctgggcctggggacccccggcctccgcctgcacgcccccccacgc
ccggacgtgccctctccgcgcgggggactcgcctaggtctcctacgtctgcccctgcccg
gctcccggcggccccagctgtcaccggccccccaggatgcaatg
(Seq ID No: 937)

Homo sapiens family with sequence similarity 134, member A (FAM134A) :

ccccttccgcctgacgcgcccccggcggcggccgcgcagccctggctcctcgcgggctc
gggcggcggctgcggcggggctatg (Seq ID No: 938)

Homo sapiens membrane bound O-acyltransferase domain contain ing 7 (MBOAT7):

ccgcctcctttccggagcccgtctgttccccttcgggtccaaagcttttggctcctcctt
gttccgagcccgaaggcccgccccttcacgtactcggagctcggatcccagtgtggacct
ggactcgaatcccgttgccgactcgcgctctcggcttctgctccggggcttcttccctgc
ccgcccggggccctgaccgtggcttcttccccggcctgatctgcgcagcccggcgggcgc
ccagaaggagcaggcggcgcgggggcgcgctgggcgggggaggcgtggccggagctgcgg
cggcaagcgggctgggactgctcggccgcctcctgcccggcgagcagctcagaccatg
(Seq ID No: 939)

Homo sapiens major facilitator superfamily domain containing 11 (MFSD11):

acgccccttttttgctcagccgtcagccccgtctccgtctgaagagtgcttctgccctca
tttgcctctccctgtgaccccggccccctcagactccgctgcgtcgtctctcggccccgt
ccagccgttcctgactgctcttcgccggagtccgcttcccaaccccctttcgccagagcc
cgagagctccgtcggctctgcgtcctggcggattgtcagtggcttcgccccgaggagagc
tgactgccctgggctgctgcctccggcagagctgagccaaaatg
(Seq ID No: 940)

Homo sapiens thiamine triphosphatase (THTPA):

ctcccttccccctctgtgggtcccgcgaggagactctcgggctttgaggtgagacctgaa
gttccgctggccggtagtgtagcaggaaagggcaggtcctcccgggtcgtgagccagtag
cctcctggggtggcaaggtgtagagagggggcgttgaaaggacacccgctacccggcct
gctttctaggggtctctttggattgaggacatcagcagcagtggaagggatttactgga
gacctgtcactgtcagagccttaaaatatcaccgacggggccttaatgtcaccgaggtag
agagaaaagggcagtagccctagagactattgcgacacagtgtgcccctcataagtttt
ccagggaggggttctgtactgagttgacgccccaggagctgagcaccaggctttgcatcc
ttgggaactcagcaaacgtttgttcagccaattgcaggtagcatg
(Seq ID No: 941)

Homo sapiens acyl-CoA synthetase short-chain family member 3 (ACSS3) :

tactcccttccctcaggccccaggaagttgcaagagtaccatttgtcgcacactcgggga
ccgcgggtggccggaggagatg (Seq ID No: 942)

Homo sapiens chromosome 6 open reading frame 211 (C6orf211):

gctcctccttcgcggcggtaccgcctctgtttctgcggcgattgaacagccgagctttgc
ggccgggatcgcggaaagtgatg (Seq ID No: 943)

Homo sapiens transmembrane protein 204 (TMEM204):

atttcctctctgctgagagccagggaaggcgagctctgcgcacacgggcgtccctgcagc
agccactctgctttccaggaccggccaactgccctggaggcatccacacaggggcccagg
cagcacagaggagctgtgaacccgctccacaccggccaccctgcccggagcctggcactc
acagcaggccggtgctaaggagtgtggcgcgggctcgactcccactgctgccggcctccc
gagtgactctgttttccactgctgcaggcgagaagaggcacgcgcggcacaggccggcct
ccgcttcccgggaagacggcgcactcctggccctgggttcttgctgctgcccaccctctg

ctccctgggatgggccccgaggcgagcagcttcagcacaggcctggccctgctccaggtg
caggaaggaggataaggccgggccgagaggcggcacacctggaccatcccatgggcctcc
gcccgcgccgccccgaggatgagtggtgatgtcctctagccacccctagcagcgtcggct
ctccctggacgtgcggccgcggactgggacttggctttctccggataagcggcggcaccg
gcgtcagcgatg (Seq ID No: 944)

Homo sapiens DEAH (Asp-Glu-Ala-His) box polypeptide 40 (DHX40) :

tcgtctttcccctcccatctcctcagatcggtggacgtgctcgcctccactcggggccag
gtctatg (Seq ID No: 945)

Homo sapiens importin 4 (IPO4) : cctccccttttcggcccagtagcggcggctcagttgctgccatg (Seq ID No: 946)

Homo sapiens N-acetyltransferase 10 (GCN5-related) (NAT10):

ccttctctttcggagttgttccgtgctcccacgtgcttccccttctccactggctgggat cccccgggctcggggcgcagtaataattttcaccatg (Seq ID No: 947)

Homo sapiens lin-28 homolog A (C. elegans) (LIN28A):

aaccctttgccttcggacttctccggggccagcagccgcccgaccaggggcccggggcca cgggctcagccgacgaccatg (Seq ID No: 948)

Homo sapiens CAP-GLY domain containing linker protein family , member 4 (CLIP4):

cggcctttcctccgcgcccccgcgtccccagccggccgctccgagaggacccggaggagg caggtggctttctagaagatg (Seq ID No: 949)

Homo sapiens zinc finger, AN1-type domain 1 (ZFAND1): ccgccccttacggcgccggagagatg (Seq ID No: 950)

Homo sapiens GTPase, IMAP family member 6 (GIMAP6):

cctcccttttctacttccgaggctgcaaagtgcaacagcagactcttctgactcaggaa ggccggtgctcctacccacttcctgttcctccatctccagcggacactgctctttcaagg gcaggtctccagcccagctctctgaaaacattttgctgaaaatataagcaaacatcggcc ttgtcctccttgtgttcatacactgtggaagcttttctctgcctcctccgtgagagtgcg tggccgggagaccagaaacgtggtcctttctcttgcctgtgagctggtgcagagatg (Seq ID No: 951)

Homo sapiens thioredoxin domain containing 15 (TXNDC15):

cttcctccggctggcagcacgactcgcgtagccgtgcgccgattgcctctcggcctgggc aatg (Seq ID No: 952)

Homo sapiens asparagine-linked glycosylation 9, alpha-1,2-ma nnosyltransferase homolog (S. cerevisiae) (ALG9): aattctttttccccaggcttgccatg (Seq ID No: 953)

Homo sapiens glutathione S-transferase, C-terminal domain co ntaining (GSTCD):

acttcccttttccggtccgccggattatgaatgacggccggcgcgagtattttccacat

aaggtggctgtcgtttttctcctggcgtctgtggaggcgagtggtctgcgggcagcagct cccagaggcagccttggaattccagctcggactgggcgggaaggcgcaggcggcccaggt cgccgacacgctcacgcaccctccctgcctggccgcgcctctgcgaccaggtgacccaat gaaagaagaaaatg (Seq ID No: 954)

Homo sapiens CXADR-like membrane protein (CLMP):

actccttttctttccaaacagggaaaagtgttccacgaagcggtagcgcctttccgcct
cgcgttttcctccctgaccctggtcccggctcccgtccgggcgccagctggtggggcgag
cgccgggagcccatctgcccccaggggcacggggcgcggggccggctcccgcccggcaca
tggctgcagccacctcgcgcgcaccccgaggcgccgcgcccagctcgcccgaggtccgtc
ggaggcgcccggccgccccggagccaagcagcagctgagcggggaagcgcccgcgtccgg
ggatcgggatg (Seq ID No: 955)

Homo sapiens nonhomologous end-joining factor 1 (NHEJ1):

cctcctcttgcggtgggggaaagcggcctcttactctaggcctttcggtttgcgcgagc
gggcaggaaagcgtgcgtgcggctaagagagtgggcgctctcgcggccgctgacgatg
(Seq ID No: 956)

Homo sapiens gametogenetin binding protein 2 (GGNBP2):

cctccttcttccactccccgcggcgcgagcggctgactgcccgtagaggaaacgacattc
ggagctgcgctcccgcccaggccggccctgacgcgggcctcgtcagccagtaacagggag
cagaggtgggagttagcgaggcgaccacgaaaacggtgaaggtcggaaccgacagcctcc
tccgagaagggcaggagctgggaggaggcggcagcggcggcggcagaaacagcagcggcg
gcggcggcggcagctgggaggaggtggtgacggtggcaacggcagcgtcggggacgatg
(Seq ID No: 957)

Homo sapiens zinc finger protein 672 (ZNF672):

ctttctcttttagccccgcctgcttcccggctccagctggggccggagaggctgagtggt
tggtacgctgctcgctggcctcccagtcttcccagcaaccggtgacactgcccgcgccag
actgaccactagccgacgcgggcgagagggacaggagcgtgacctccccatcccgagggg
ccggacgctcgggcgcctccccgctccccccactcggaggccgcgcgcgccgttagcccc
ttcctcgctcccccgccccagtcccgcagtccgggaggcgggggtcggcagccggctgag
tgggaaccgcgcggtgtctgaggaggcagtcggcgaccggtttccacttcaagcgtgacc
cttttgcctgtgggatgagctccagcatggggtgaggtacagaagagagacttgaagagc
gtgccttgggactcaagcgccaaacctgtaccctagcgagtgtcctactccgcatccgta
atggaaggaaatgcacatcttactccagaggcacaagaggaggacatcccatgcggctac
tcctgcccagcgtggtggggcagcagaagctccagagcccagacttgcaggctcacggtg
cagggtgaacctggccacagctcaccctggaacagccacaatgtctgccccttagagaag
aaccctgaaatcagaccagtttttgcggcctcccccttttcctctctgttacagtgccctt
tccaggccttaagagaagtaaaacttagctgcagcgccaggaggtggaccccagagtgtg
agtggcacgcttccctgtgaacccgtcctcaccatg (Seq ID No: 958)

Homo sapiens N(alpha)-acetyltransferase 60, NatF catalytic s ubunit (NAA60):

ccgcctccgtcccggctgcggcccctgccggttacataactcgttgcgggctccgcgcgg
tcccacttcccggctcccttcgcctccaggatgcgctgagccctacaacacccccagcgg
ccgccggctcccccacgaggtgtgaatg (Seq ID No: 959)

Homo sapiens transcription elongation factor A (SII)-like 4 (TCEAL4) :

tgccctctgtccccgcggctgggtctcgtctgctccggttcctgggctcctaattcttgg tccagcttcttccaggtcagtgtgcgggccttccacgctgccagcggaacactggaatgg cggaaggggaacgggtctgcgcgtctgttgttcccagcgctctgcgaagcctgaaaagga ggagcaacctgtccagaatccccgcaggacaggaaaaggaggggaaatctcgacatg (Seq ID No: 960)

Homo sapiens progestin and adipoQ receptor family member VI (PAQR6) :

tcccctttgtctccccactccccgcccaggcctggcccgcctgcctggccactcttcctc catcagcctggctggcagcagccttggactccgcccgtggagccctgggcctgttgaccc accagcttaggagcacccaccaagctctgggtaaggaagctcaccttctggggctcttct gggaaaatagaggtcaacgtggaggtaccaggccaccatgctcagtctcaagctgcccca acttcttcaagtccaccaggtcccccgggtgttctgggaagatggcatcatgtctggcta ccgccgccccaccagctcggctttggactgtgtcctcagctccttccagatgaccaacga gacggtcaacatctggactcacttcctgcccacctggtgaggggaggctctgccccaggc cgcggccttgagctcagaggggtacccaggcgggcagggaccgtccaggcccacgggct gcagcggcagtcgcggggtccgcggcggcctgagcacgcgcccgccgcaggtacttcct gtggcggctcctggcgctggcgggcggccccggcttccgtgcggagccgtaccactggcc gctgctggtcttcctgctgcccgcctgcctctaccccttcgcgtcgtgctgcgcgcacac cttcagctccatg (Seq ID No: 961)

Homo sapiens DENN/MADD domain containing 2D (DENND2D) :

catccttcttgctcaaccactgggtgcacaggatggaaacttctattccctctctggaag acagcgcgtggcttggcttcacagagttgtggctggagaccgaagcagcccctttctcag gcttactgtcaccagtctgtctgtgttaggggagaggggagtccgctctgtcctgaaggc ccagagatg (Seq ID No: 962)

Homo sapiens family with sequence similarity 188, member A (FAM188A) :

ccttcttctttcctgcctcaccttccaattcgtttgccgccgccgtcccgcagctgctgt ttccggagttgccccttccccatgttccggggcaggagtccgcaaagcgaagatccgccc gccggttcctcatcatg (Seq ID No: 963)

Homo sapiens neurensin 2 (NRSN2):

ccgcctttgctcggcggagacagcaggcagagagatgaggaaactgagacccagaaaggt ggaagcacttgtctaaggtcacgcctccaggaagcagtgtgtccacgactccagtccaag tggtcaggctccagagcccacagtcccaggggtccatg (Seq ID No: 964)

Homo sapiens tripartite motif containing 46 (TRIM46):

agccctcctcacacccccactgggctcctgcattaagcccggggttcgcagccgcagccg ggatcgggcacccaggggcgggcgggcacggtagggccatg (Seq ID No: 965)

Homo sapiens target of EGR1, member 1 (nuclear) (TOE1) :

catcctctctgggaatttaccgatgcccagaacgcccttctttcccccacacgaccctct
cctagtctaactcctgggcgtgctttaagctcagctcaggcagcgtcaccttctctggaa
agcccaaacccagccaccccactacccgctacccgcggcccacgctgatgaagacagcag
aacacggaggccccgcgttcccgccgcgagagcaggagagaaagattacctcccgcgagc

tctagcgcgcccggctttccggcgcactccagggggcgtggctcgggtccacccgggctg
cgagccggcagcacaggccaataggcaattagcgcgcgccaggctgccttccccgcgccg
gacccgggacgtctgaacggaagttcgacccatcggcgacccgacggcgagaccccgccc
catccccgactgcctgaaccgcgccaggagacggaccgcaagtccagcgtacccacagac
gactcaggcgggagacgagcggtgtcatg (Seq ID No: 966)

Homo sapiens DBF4 homolog B (S. cerevisiae) (DBF4B):

cgttcttttaggggtggagccggcaggaaatttaaactgaagccgcggccgaaaacgcca
agagattgatgctgtagctgccctgagataaccaggactgtggaatcgggaagagctcat
ggagctcgcgaatgtaatacggaggcctctgaggaaggagtacggaggccgagaaggagc
cggcatttgatg (Seq ID No: 967)

Homo sapiens myc target 1 (MYCT1): atttccttttatg (Seq ID No: 968)

Homo sapiens myosin XIX (MYO19):

ggttcctttcctcactgcacgctcttgcccctcctcttttctctcctgcccgtgttcttc
ccgccgcctgacctggcccgcccgcctttccagtctggccgggcgggggcctgaagcacg
gcggctcgggccgtgggaccgtgttcacaccctttccagaaattcttggctggtaaccgc
gaaaccgactggagcaggagctgggagaactggagaaaactgctctaatctcacttgact
ccagctaggagctgatgctgcatcgtaataacatttgcagagcgctttcacaggcgctgg
agtgacttgtctgagattcctccagaactgagccctttgttggaaccataccccagccca
tggtcccatgactaggtggatagtactccttgtacctcctgcaacccagaaccctggctg
accactttgaaggaggatg (Seq ID No: 969)

Homo sapiens KIAA0226-like (KIAA0226L):

cctccccttctgctgttaccgggagcgcggtggccacggaacgctgcccggagccgcgc
gagggaggacccgacgcgcggcgtttacccagcgcagcgttccaccgctcgggtttggct
ggataaaataaaaaatggggatattgacctcctgtcactactgcatggactttgatggtt
tccaatcattactttctcctctgtgtcaatctgcctcttcgagaaattcatactcctgaa
tagctctccagacccccagctggccatgtggtgagttcagggcccaaatcaagtagtacc
agcaatcagggaactcctatctgttttgaatggattcacaccagccacaagcctggaaag
atg (Seq ID No: 970)

Homo sapiens MUS81 endonuclease homolog (S. cerevisiae) (MUS81):

ctccctcttcccccgccccgccctgggccaggtgttcgaatcccgactccagaactggcg
gcgtcccagtcccgcgggcgtggagcgccggaggacccgccctcgggctcatg
(Seq ID No: 971)

Homo sapiens zinc finger protein 430 (ZNF430):

gggcctttgtccctcgctgtggcctgagctccaggtctcgtcttcagcgctctgtgtcct ctgctcctagaggtccaggctctgtggccctgtgacccgcaggtattgggagatctacag ctaagacgccaggaacccctggaagcctagaaatg (Seq ID No: 972)

Homo sapiens mutS homolog 5 (E. coli) (MSH5):

gctcctttgcaggctcgtggcggtcggtcagcggggcgttctcccacctgtagcgactc aggttactgaaaaggcgggaaaacgctgcgatggcggcagctgggggaggaggaagataa gcgcgtgaggctggggtcctggcgcgtggttggcagaggcagagacataagacgtgcacg

actcgccccacagggccctcagaccccttccttccaaaggagcctccaagctcatg (Seq ID No: 973)

Homo sapiens proline rich 3 (PRR3): gccccttcctcactaccctccaaatcccgctgcagccattgccgcagacacgatg (Seq ID No: 974)

Homo sapiens sirtuin 2 (SIRT2):

cgccctttaccaacatggctgctgacgccacgccttctgggactcgtagtccggtcctcg cgcgctttcttacctaactggggcgctctgggtgttgtacgaaagcgcgtctgcggccgc aatgtctgctgagagttgtagttctgtgccctatcacggccactcccatttctggtgccg tcacgggacagagcagtcggtgacaggacagagcagtcggtgacgggacacagtggttgg tgacgggacagagcggtcggtgacagcctcaagggcttcagcaccgcgcccatggcagag ccagaccgactcagattcagactctgagggaggagccgctggtggagaagcagacatg (Seq ID No: 975)

Homo sapiens KIAA1715 (KIAA1715):

ttgtctctctgtcagtggcggctgctgcctgctctggaggcaggctgggcggtggcggcc gagactggcgggggtggacgcccgggccgggctgcgcccgcttcttgcagctgtgaattc ctttggacaattgatgatatttatcattgtgcccagtttctacaaataaaagatg (Seq ID No: 976)

Homo sapiens proline-rich transmembrane protein 1 (PRRT1):

ctgccttcatctctccatctctgcgctgctgccggctgcgccatccagcacccagactcc agcaccggccgaggacccccactccggctgcagggaccctgtcccagcgagaccgcaggc atg (Seq ID No: 977)

Homo sapiens t-complex 1 (TCP1) :

ccgccccttccccggagcctcacttccgtcacagtcctgtttctctcccctgttgtccctg cctcttttccttcccgccgtgccccgcggccgggccggggcagccgggaagcggtggg gtggtgtgttacccagtagctcctgggacatcgctcgggtacgctccacgccgtcgcagc cactgctgtggtcgccggtcggccgagggccgcgatactggttgcccgcggtgtaagca gaattcgacgtgtatcgctgccgtcaagatg (Seq ID No: 978)

Homo sapiens Yip1 domain family, member 5 (YIPF5):

cgttctttggccctgtgacacgtagcaacggggctggttcagggtctgaaacagagtttg
ggggttgtttgggattagtgaagctactgcctttgccgccagcgcagcctcagagtttga
ttatttgcaatg (Seq ID No: 979)

Homo sapiens glucose-fructose oxidoreductase domain containi ng 2 (GFOD2) :

cctccctttccagagcccccagttccttagaaaccaggcggcgcgttcccggtggcggcg
ccctggactcccgggcccgcgcatccccgccagccttccttaaggcggatgggtggcccc
cgagaccccgtcggacccatggtttccagtgcagcgcggagtgggcgatgccagcgtgcc
aggagccatgtctgaccaggacgtttggaagatcatatccatgccagaggctcttgtgag
gagatgagttggtaaagagagaggctgggatg (Seq ID No: 980)

Homo sapiens apolipoprotein L, 2 (APOL2):

ttccctttcgaattccagggtatatctgggaggccggaggacgtgtctggttattacaca
gatgcacagctggacgtgggatccacacagctcagaacagttggatcttgctcagtctct

gtcagaggaagatcccttggacaagaggaccctgccttggtgtgagagtgagggaagagg
aagctggaacgagggttaaggaaaaccttccagtctggacagtgactggagagctccaag
gaaagcccctcggtaacccagccgctggcaccatg (Seq ID No: 981)

Homo sapiens microtubule-associated protein 4 (MAP4):

ccgcctccctgcgccccgcccctccggctagctcgctggctcccggctcctcccgacgtc
tcctacctcctcacggctcttcccggcgctctcctggctcccttctgccccagctccgtc
tcggcggcggcgggcagttgcagtggtgcagaatg (Seq ID No: 982)

Homo sapiens exonuclease NEF-sp (LOC81691):

cttccttctttgccaggcagacgcccgttgtagccgttggggaaccgttgagaatccgcc
atg (Seq ID No: 983)

Homo sapiens ST6 (alpha-N-acetyl-neuraminyl-2,3-beta-galactosyl-1,3)-N-acetyl galactosaminide alpha-2,6-sialyltransferase 5 (ST6GALNAC5):

ctgtctctaatctctgcaacagccgcgcttcccgggtcccgcggctcccgcgcgcgatct
gccgcggccggctgctgggcaaaaatcagagccgcctccgccccattacccatcatggaa
accctccaggaaaaagtggccccggacgcgcgagcctgaggattctgcacaaaagaggtg
cccaaaatg (Seq ID No: 984)

Homo sapiens heterogeneous nuclear ribonucleoprotein A1 (HNRNPA1) :

tgctcctttctgcccgtggacgccgccgaagaagcatcgttaaagtctctcttcaccctg
ccgtcatg (Seq ID No: 985)

Homo sapiens zinc finger protein 93 (ZNF93):

gggtcctttgtctctcggtgcagccggagctccaggtctcctcttcactactctgtgtcc
tgtgctcctacaggcccagcctctgtggccctgtgacctgcaggtattgggagatccaca
gctaagacaccaggacccctggaagcctagaaatg (Seq ID No: 986)

Homo sapiens N-terminal EF-hand calcium binding protein 3 (NECAB3) :

cggcctctagccacaccgagtccgccgcggcgtccagggtcggcagcaaccgcagccgag
cccgagcgggtggcggcgccatg (Seq ID No: 987)

Homo sapiens splicing factor 3b, subunit 5, 10kDa (SF3B5):

cattcttctgcgacggcgcggacctggagcttccgcgcggtggcttcactctcctgtaaa
acgctagagcggcgagttgttacctgcgtcctctgacctgagagcgaaggggaaagcggc
gagatg (Seq ID No: 988)

Homo sapiens INO80 complex subunit B (INO80B) : gtccccttttcctcgcaggacctcatg (Seq ID No: 989)

Homo sapiens polyamine modulated factor 1 binding protein 1 (PMFBP1) :

ctttcttcctcttggcttatattagggataggggatgtggtttgttacaaaggatgagta
ttttgatagcttctcattccttgaactattctgcaggtttataacaaagctcagaaaata
ctaaaggttaaaggagaattgagagctgccaaggaaatg (Seq ID No: 990)

Homo sapiens pseudouridylate synthase 3 (PUS3):

cttcctttctcggaaacgcggcgcggccggctgccggaaaacagggcagacctgtatggt
tcgtttattcctggggttgtcatatcatg (Seq ID No: 991)

Homo sapiens heterogeneous nuclear ribonucleoprotein D (AU-rich element RNA binding protein 1, 37kDa) (HN-RNPD):

tattctttttagtgcagcgggagagagcgggagtgtgcgccgcgcgagagtgggaggcg
aagggggcaggccagggagaggcgcaggagcctttgcagccacgcgcgcgccttccctgt
cttgtgtgcttcgcgaggtagagcgggcgcgcggcagcggcggggattactttgctgcta
gtttcggttcgcggcagcggcgggtgtagtctcggcggcagcggcggagacactagcact
atg (Seq ID No: 992)

Homo sapiens GABA(A) receptor-associated protein like 1 (GABARAPL1):

atttctccatctggctctcctctacctccaggcaggctcacccgagatccccgccccgaa
cccccccctgcacactcggcccagcgctgttgcccccggagcggacgtttctgcagctatt
ctgagcacaccttgacgtcggctgagggagcgggacagggtcagcggcgaaggaggcagg
ccccgcgcggggatctcggaagccctgcggtgcatcatg (Seq ID No: 993)

Homo sapiens chromosome 22 open reading frame 13 (C22orf13):

ccttcctttccccagtgttgagcgcggtctcgcctccgcttcctcctcactccgcctgcc
ggctgggaaactagggcaccagtacgatagttccggcaccggaaaagagggctgatgact
gggcccggggccgccgcaacgaccttggggccggcaaagagccagagagggtgctcac
acttccaagcaccccacaccaaggacaggctggacggcaaggcggagacgcggggcttgg
gccctcagaccggggacagcaggaggttgggccaagggccaggacttcccgtcacaattt
catttgttgatcccggcaccgccaggtaagggggccctgagtgaggctaggtatctggt
acggataaagttaggtatagagtagagcggctgcccgctcagggttatccctaaagacag
ttggaggagagttgcttggggcctcggggatgcactgggcgggatcagggcttacaccta
ggactggcaaaagagcgggacccggcagaggcggggcttgccgaagggacgagcctctat
tcaggaaatgcacgagctttggggcggggctcaaagaaaggggcggggcttccggggccc
gcgtcctggtgagctgcgcgtctgcgcgaggattggcgagagggtggggccactcaacg
ctgaggcggcgaatggccggagcagacttaaatcaagaggctggggacctctaagatcaa
agtttggggcggggcctaaggagggggcggggcctccagattcgagacctggaagggctg
gggcggcgcttggggcggccctgccgccgcctcccgttctcccctccgcagcggcggcgg
tggcggagaaggaactcgacacgcaccgaccgccctcccgccccagccgaagcggaagct
gtagcccgctctgggccggggccatgggcgccccgcgccgcccgggtcatg
(Seq ID No: 994)

Homo sapiens lon peptidase 2, peroxisomal (LONP2): ggctcttttgacagcccccagtgcgaaaggctgccagcatg (Seq ID No: 995)

Homo sapiens RNA binding motif protein 4B (RBM4B): ggttctctctgacgtgggagccgccgtcgctgccgccacccggaggctcttgtcaggatg (Seq ID No: 996)

Homo sapiens protocadherin alpha 3 (PCDHA3):

aggtctttctccacaaaagaaataacagcgtgcattacgtattcagatactgctttgctt
catcctctctaaaatttaacaccgaggagtttaagaaatgaagataaggaactcgaatta

tttttaaactttggatcaatgtaaaggcaatctaatatttggaaaatacttgcaatg
(Seq ID No: 997)

Homo sapiens RAB34, member RAS oncogene family (RAB34):

gcctctccttgggccccttctctccccctttcccctccctgctggttcctggcatcgcca
gatgctgcgcagcagtctccgattccccatcaccaattcggctggcgtctccgagaccgc
ggactcccgtagggtccccgtggccccgagttgtagtcgggacaccccggccgcgggtga
tcgtcgggtctccacgcgcccgggtcgctgacgcggatccggcctcggcgccttctcagg
gcgccctgcaaggccgcaggcaggatg (Seq ID No: 998)

Homo sapiens cell division cycle associated 7 (CDCA7):

gctcctcctgctgtgggaccgctgaccgcgcggctgctccgctctccccgctccaagcgc
cgatctgggcacccgccaccagcatg (Seq ID No: 999)

Homo sapiens ArfGAP with GTPase domain, ankyrin repeat and P H domain 3 (AGAP3):

gggtcttttaggagagcactgctgcagccggcagtggagagcctgggcagggagacaggg agaaaactccggcagcagggtggtctctagggctgacctcggagcctggggacaggggag cctatgccgcactgaaggcgggacgctgtaagcgaggagcagctgggcctgggcggactc ctcggccaatcagcctcggtcagcagcaccctcaggcgcagggcactgtttgggcattgc ctagagatccgacaccccgcccagatcagcgcagggaggcgaaagcgacagccgggcgcg ggaggagaccagggcagctgtcccctccgcgagggtggccctcgaggcaatgcgggtggg ggctggtgaggaggcggaagggccgaggctgagtgggagggggccggggcgccaggctgg agcgcgcggctcgggggtggaggctgcagagccagcgagcgagcgaggggcgggggcgcc cgggccggcgcgcaggagggggcgggggcggcggggagggggggctcgggctgcgtgtgccg gagccggcgggggcggcggtgcgtgcgcatgacgcggggggagggcctgggccgcgcgct cccggtcccgttgttgttgccgctggaggctgctccgaggcagcgggatcacggcgctgg gaagcgctcggcagcggcggccacagcgtgcgcggcggcgcctcctggcctcggcctccg gcccccggcccccggctccatgcgctagccccgcgccgccagcccagtagtcccggcccc gccagccccgcgctcccgctcgccgctgccgccgccgccgccgccgccgcctccgccgcg ccgccccgggcccgcctcggccccacggctccgaagccatg (Seq ID No: 1000)

Homo sapiens potassium channel tetramerisation domain contai ning 10 (KCTD10): ctgcctctctcagtccgggtttggagactcctgcgtcctccgacttttcatg (Seq ID No: 1001)

Homo sapiens cyclin B1 (CCNB1):

cattctctgcgaccggcagccgccaatgggaagggagtgagtgccacgaacaggccaata aggagggagcagtgcggggtttaaatctgaggctaggctggctcttctcggcgtgctgcg gcggaacggctgttggtttctgctgggtgtaggtccttggctggtcgggcctccggtgtt ctgcttctccccgctgagctgctgcctggtgaagaggaagccatg (Seq ID No: 1002)

Homo sapiens eukaryotic translation initiation factor 2A, 65 kDa (EIF2A): gtttctctttccgggacaacatg (Seq ID No: 1003)

Homo sapiens protocadherin gamma subfamily B, 7 (PCDHGB7):

cagcctctagcctgggattccctgcgcagccaacaacagaaaagaaaaccagctcccaca cagaggctcccggctgcgcagaccttgcccagcacaccagattgccagctccgagacccg

ggactcctcctgtcctgggccgaatgctcttttagcgcggtagagtgcactttctccaac tggaaaagcggggacccagcgagaacccgagcgaacgatg (Seq ID No: 1004)

Homo sapiens acyl-CoA dehydrogenase family, member 11 (ACAD11):

ggctctttcggcttccttcctcgctgggccggctaaacccggccgcagcagcaccggggt gataagtgtccagggcaggaggccagcgatgttgccttgctaaccgggtatctaagagaa acagggtctttttattcttaggctcgacagtctgacggcccttttctgaacgggaccct gcaggtcttccgcctgctgttgcattaaatttggggggtggaagaggcttctgcgttgttc cttacccgcaacgatgaccatggctttgccttctttaaaattgaggcctccaactctgac gctgactggagaattgaaacccgaacacacattgggctcttttggcacttgactagagct aaaacctcgggattcagcgggcaagcgttgctcagcaacggcgcgtaggctgtgtgcggt tggctggagccagaccccaccccggcctcggcccatgctctagaggggacgttgccccaa tcctgaaggacttcggcactcgagacctgtggatgccgcgttgctgtggcctgcgggggt gatcatg (Seq ID No: 1005)

Homo sapiens zinc finger, CCHC domain containing 7 (ZCCHC7):

ccgtccctctacgcgttttggttcccggttggtgcttcctgttcgcagctgcggcacttc aaggttactgactttttatg (Seq ID No: 1006)

Homo sapiens zinc finger, MYND-type containing 12 (ZMYND12):

gggcctttctggacttggactccttgggagtcgtttctcggccatttgacccgtgggact tgtgggttttgtgctgctttttctttctttcttccccttttccaacttcagcaatacacc cagatgttagtcgagtcacgtcccgccgccctctgcccttgaaatgctggcaagtacgca gccccgcgatcgtcacgtgacgccggggttcagcgtatccttgctgggcaaccgtcttag agaccagcactgctggctgcaccatg (Seq ID No: 1007)

Homo sapiens forty-two-three domain containing 1 (FYTTD1):

cgctccctcggtgcggcgggctgcgtgcgcgagtgggaggtggcaggcctgcgactccgg ccttgtccgcgcccgctctcggcgcgacgtctccagccatg (Seq ID No: 1008)

Homo sapiens SH3-domain GRB2-like (endophilin) interacting protein 1 (SGIP1):

ctccctttctctcagcatcttcttggtagcctgcctgtaggtgaagaagcaccagcagca tccatggcctgtcttttggcttaacacttatctcctttggctttgacagcggacggaata gacctcagcagcggcgtggtgaggacttagctgggacctggaatcgtatcctcctgtgtt ttttcagactccttggaaattaaggaatgcaattctgccaccatg (Seq ID No: 1009)

Homo sapiens GTPase activating Rap/RanGAP domain-like 3 (GARNL3): cagcccttttgcaaatg (Seq ID No: 1010)

Homo sapiens DCN1, defective in cullin neddylation 1, domain containing 5 (S. cerevisiae) (DCUN1D5):

gagcctcttgcttgctgtgactggtggagctgccgcgctgtccgcgttatctcctcccgg tgagaacgaaccgcagtgtccaccggcgaggagccagccctgtcccggtcagagaaagac gacgaggatacctgggagcgggcggcggccgggctgggccgcgccggtgcgggctggcga ctctgctcctccgcttgctgctgtctctgggaactgggtgccagcgctgaggggcttcca gcggacagggaccccttccccggctcccctgcccaccctgccggggagggcggaagatg (Seq ID No: 1011)

Homo sapiens alkB, alkylation repair homolog 7 (E. coli) (ALKBH7): tgccctctctcatgaccccgctccgggattatg (Seq ID No: 1012)

Homo sapiens nitric oxide associated 1 (NOA1): ccgcccctttggagctacttcctcatg (Seq ID No: 1013)

Homo sapiens BTB (POZ) domain containing 10 (BTBD10):

tcgcctcttcgcattgtgagctctcgcggtaagaggctgaggagccggcctgcaacctgc cggggcggctccgctacgcgcagccgcctcagtggcttcctccacagccacctccggagg gatctggctgaggaggaagtggaggtgtcactggccccggcctttgccccaatcttgtgt gggcactgaaggggactacaggttcgagagttatgggtgctacatgtgtgctttcagag cagtagtgtgaggaagcttggagtgggatg (Seq ID No: 1014)

Homo sapiens zinc finger protein 397 (ZNF397):

cggtctttgtggcttgcagctcggggtgggtggctcatttcctggccgctcctggcttc gcggaaagaagagattactcacactccttcgcaagcacagaaccagttgtactgagcttt ttgctaagctgtttcagccaagaatg (Seq ID No: 1015)

Homo sapiens mitochondrial ribosomal protein L45 (MRPL45): gctcccttcccggcggcctttgcgggaacaagatg (Seq ID No: 1016)

Homo sapiens AKT1 substrate 1 (proline-rich) (AKT1S1):

cttccttctccatattgtatactggaattgaagccaaggaggtaccattttgctcgaggg catggcctaagccggtcagctaaggccatgttaatacggggctgtcccatctctctgcgg ggcgcgacagctggaagagccgaacggataagagaagaggaggtgagaggagctgtacac cacaagaggcactgagggactcaggataacgggatgaagccgtcagtgcccccagaaacg aagcggccccggacgaatttctgagtcaccgtcgcgagaaagcgggctgagccgccattt tgaagcctggcaaaccgaagcaagaaatgctgccgtgttggatctttgccagccttcgtg ccgaatgggagcaggttggagggagggagagccaatatacactatgggctgattaagccc ggttggctgccatgttgttaacgagcaccgatttcctctacttttgtcgaagaagtttat tgtgggtcagggacgtcaggtcgcttgccttcgtttactgtggtcatgattgagcatatg aggacggccattattgttgggggcaaatggaaatgctctaggcggggccatttttcttag gggcaagctgtcgtcacccttgtcaactggttcggatgaagcccctgtggccgccatctt gatctcgggcggccccgataagggaggcggagtgtgcggagaggaggcggggcaactgcg cggacgtgacgcaaggcgccgccatgtcttttgagggcggtgacggcgccgggccggcca tgctggctacgggcacggcgcggatg (Seq ID No: 1017)

Homo sapiens transmembrane protein 101 (TMEM101): ctgccctttcccaagatg (Seq ID No: 1018)

Homo sapiens eukaryotic translation elongation factor 1 delt a (guanine nucleotide exchange protein) (EEF1D):

ggccctccctttcatcagtcttcccgcgtccgccgattcctcctccttggtcgccgcgtc cttggctggcgttagagacagggtttcaacgtgttagccaggatggtctcagtctccaga ccctgtgatccgcccgcctcggcctcccaaagtgttgggattacaggtgtgagccaccgt gcctggccgaggctccttcttttatg (Seq ID No: 1019)

Homo sapiens ADP-ribosylation factor GTPase activating prote in 2 (ARFGAP2):

cgccctccccgccgtggattggcccgcggcgggacccgtcagccgcggttgtgtctggga aggagagaaaatg (Seq ID No: 1020)

Homo sapiens junctophilin 4 (JPH4):

atttctctcctccctggggtctcagtgcatctccttctcctctctgcctgcctcctccc
tcaccgaagggttagcggacacccatccttttctgcttggggaccccaccaccacccgca
acactgccgctgtctcttcttcaccgtatccttctctacccaccctcttctctcttctct
tctccctgcccctttaaatctgcctggcccagcctcccccgtgatgctgggatggagcaa
acattgatttgtgctgggatggaatcggaattttgatttattttcctctcccaaccata
agaagaaaaaaataataaaaacacccctcttgagagccccctcccccttgcatccagc
tcccagctcttcttccctatctccatccaaggcagatttttcccctacactattctcat
cttcccccacccttgccactacctcgccccccccacccagcctgctcctccagctggggag
agaggggactctccggactcccccacctttcctctctgggttggagcagtctctccggaa
ggggagggggcttggcttgtccgggcgaggtgggagtggaggtatcctgccatggatgct
gtgccggggaggcagcctgagccccagcccacatgagacgccgaagaaccggggcagagg
ggtcctgacagcagccagggaaacgggtgccctacgattctgcccagcccccctctcagga
cccccaaactgccatccacactcgacacttcggggttctagccactcaggatgagggtcc
ggccctgcctgccctcgctggggcccccccgcccggccccggtctaactgcccccgcccc
gaggcctcgcccggctccaaggcccccagcaggctctccagtcccaggatgcgctgagcc
gccgggggctgaggccgcgccaactacatgcatg (Seq ID No: 1021)

Homo sapiens embryonal Fyn-associated substrate (EFS):

ttttctttctcctcctccaaccttggcggaggccacgactcaggcgccacagctgggggc
tagaggccgcggaccatggtgcggggcagccaccgctgaagtcagcaaaaccgagcctgg
cctgaggcaggctgcgcgggaggccaaagccatg (Seq ID No: 1022)

Homo sapiens GH3 domain containing (GHDC):

cgctccttctttctggccggatgtgtgctgagacccagagtcacccaggggtctccgtca
cgtgccaggagtaggcagaagtgggctgtgacagatcaggaaacagagctcagtgcagcc
cactaaattgctcagggccctacagctaacaagcggcagaggcaggatctgcactcagga
gctgcttggagatg (Seq ID No: 1023)

Homo sapiens acrosin binding protein (ACRBP):

ggctctctctgcggcttggcccgttagaggcggcttgtgtccacgggacgcgggcggatc
ttctccggccatg (Seq ID No: 1024)

Homo sapiens jagunal homolog 1 (Drosophila) (JAGN1):

agttctcttcacggagccgcgcggctgcgggggcgcaaatagggtcagtgggccgcttgg
cggtgtcgttgcggtaccaggtccgcgtgaggggttcgggggttctgggcaggcacaatg
(Seq ID No: 1025)

Homo sapiens ligand of numb-protein X 1, E3 ubiquitin protei n ligase (LNX1):

gttcctttcctgggcatcagcttgcctgctctcagcctaagctctctcgccaaccgtggt
ggctccttgcgttcctacatcctctcatctgagaatcagagagcataatcttcttacggg
cccgtgatttattaacgtggcttaatctgaaggttctcagtcaaattctttgtgatctac
tgattgtgggggcatggcaaggtttgcttaaaggagcttggctggtttgggcccttgtag
ctgacagaaggtggccagggagaaggcagcacactgctcggagaatg
(Seq ID No: 1026)

Homo sapiens cyclin-dependent kinase 2 interacting protein (CINP): tctccttctacggatatctgtggaccttatg (Seq ID No: 1027)

Homo sapiens splA/ryanodine receptor domain and SOCS box con taining 2 (SPSB2):

gcttctttccgcccggctccttcagaggcccggcgacctccagggctgggaagtcaaccg
agctcccttccaggtcaatccaaactggagctcaactttcagaagagaaagacgccccag
caagcctctttcggggagtcctctagctcctcacctccatg (Seq ID No: 1028)

Homo sapiens Berardinelli-Seip congenital lipodystrophy 2 (seipin) (BSCL2):

cctcctcctttcctccctctactctgacacagcacttagcacctgaatcttcgtttctct
cccagggaccctccattttccatatccaggaaaatgtgatgcgccacaggtatcagcgtc
tggatcgccacttcacgttttagccacaagtgactcagtggaagatccagagtcaacaga
ggctcgtcaggaagatg (Seq ID No: 1029)

Homo sapiens tubulin, alpha 1c (TUBA1C):

caccctttcactacttctcccccggactccttggtagtctgttagtgggagatccttgtt
gccgtcccttcgcctccttcaccgccgcagaccccttcaagttctagtcatg
(Seq ID No: 1030)

Homo sapiens 1-acylglycerol-3-phosphate O-acyltransferase 9 (AGPAT9):

tttccttcctctcttcccttcgcagaggtgagtgccgggctcggcgctctgctcctggag
ctcccgcgggactgcctggggacagggactgctgtggcgctcggccctccactgcggacc
tctcctgagtgggtgcgccgagtcatg (Seq ID No: 1031)

Homo sapiens 1-acylglycerol-3-phosphate O-acyltransferase 1 (lysophosphatidic acid acyltransferase, alpha) (AGPAT1):

gcccctttctttccttcgcttcctcttttagagaatgtccggattgctattggactttgg
agcgtatggctccaaatcaactcattggctaaaacttgacggaaaatggtggttaggtgg
ccagaatg (Seq ID No: 1032)

Homo sapiens abhydrolase domain containing 14B (ABHD14B):

cggcctcttcccagcgttcctcctccggccccaggtcaccgccagcacgcgcctgcttcc
cgtctgcgcgagtccacgcagctccccagatcaagaagctgaggccccaggttacacact
aaagtaaatggcagaggcagaaataacacctatgtcctcctgaccccaaggcatgttctt
aaagttctggaaacctcctggaggcttccttgctgctcctctgggactgccaccctgggc
agggtgttctgtggcccctcatcatcgtggttttgaaccacaggcccttcaccagcacag
cagcagcaggcatg (Seq ID No: 1033)

Homo sapiens protein tyrosine phosphatase, non-receptor type 5 (striatum-enriched) (PTPN5):

catcctcccgccagcctgcccgcctgctcgccggcgcccggagcccgctctggccgcttg
ctttttgctgagaaagcttcctgccctggaagatggcacccttccccatccagacacctt
gggaatg (Seq ID No: 1034)

Homo sapiens carbonyl reductase 4 (CBR4):

cttcctccttttcacggcgtcttgcattactattgtgcggctgcaggaggtgtcgagcgg
cgttatttttttttgcggtttgcctttttttttctttttttttttttggaaccgcggtt

gtttaaaagcctgagggaacctggagaggggctcccactccctaccctctttcctccgag
tttgtgactccgagatg (Seq ID No: 1035)

Homo sapiens zinc finger CCCH-type containing 10 (ZC3H10):

ggctctttgtcgaagctagaggaccggcaggcggcagcagcaactacggcggcggcggca
gaacccagcagcgatgtggaggtggagacccacaggagccccggacttcacctgagctac
ctcagtggtcaccaagagtggcaagataaagaaaaccctgagttgggcgggaccaggatg
(Seq ID No: 1036)

Homo sapiens poly (ADP-ribose) polymerase family, member 10 (PARP10):

ccgtctttcagtttcacttttgttttcctgctcccagcagggttaggcttgctgaggggc
aggcacaggagtcctggctgagctcatggcctgaggctgcctagcggccacggggaatg
(Seq ID No: 1037)

Homo sapiens RNA pseudouridylate synthase domain containing 4 (RPUSD4): ccgcccttccttgtaagatg (Seq ID No: 1038)

Homo sapiens family with sequence similarity 73, member B (FAM73B):

ctgcccttccgcagcgatggcatcccgggtgagtatcggccccggccgagcccccaaggc
gggcgggcagcgcggcagggccgggacttgagcggaggaccgagtaggcgcaggtgtccg
ggcccaacaggaccaggaaggtgtcggggttggaatgagtgggtacccgggccggggacg
gtgcgagagggtgccttgcttgggagcggaacgagaaggtacttgggtcagggaggtgat
gcccgggcctggaacgtggcggggattggagcaggcgcgcaggtacccgatccgaggcgg
ggagagcacccgggatggaaggagcaggcgtgcgggccgtgagcggcgccagagggtacc
tggctctgtggaggggccctctggtatgtgtgtccctgtccttctggggcgtggatggtg
cctgggacccagctggcaaccagttgaagacgttctccttggaagctcttggccctgagg
actttgcctggggcattggccctgccatg (Seq ID No: 1039)

Homo sapiens protein phosphatase 1, regulatory subunit 15B (PPP1R15B):

gcgtctcttccggcgtctagggggggtgtcctgccggcgcgcgggccctgcggccatttg
ggcttcgcttccaccgcaccagccggcctacccagtccttccggtatcgcgttgctcagg
ggcttttcaaccctctgtcagtcggaaaaccatcgccgaggccgtgggggactcctatc
catggtgttgaagcgtcgagccgactagggaacctccttccccgccaggatggaagtcgc
atcagtcgccgcctattgcgcgggctgttcttccctgtgttctgccgcccgctgccgcat
tcgctgccctctgtggcttttctgctggctcgaagatcggcctggagcagcgacgccacc
gctgggcaaggccgagactctgtaggcttcctccgaatcccgtcgacctccagccgctga
gcgccgcggccctacctgagagactgtcaagaaaaaggagatg
(Seq ID No: 1040)

Homo sapiens family with sequence similarity 104, member A (FAM104A): ccctctcttcgcggagcggcgccgcgtagcttccatccgccagctgccatg (Seq ID No: 1041)

Homo sapiens PRP38 pre-mRNA processing factor 38 (yeast) domain containing A (PRPF38A):

agccctttacactacggtgtttccggcttcaagatggtcgcctaagctgtttagtgaaac

ttcttccacctttctccattcctctaggtgctttttctgaacctggatgtgaggcattaa
aggatccgacggaaatagaattgaaggcattctaaaatg (Seq ID No: 1042)

Homo sapiens synaptotagmin-like 1 (SYTL1):

cctcctccgtgtggggcagctgctggctgggctgcctgttgagtcagccttcttccctca
cggctcttctcccggtccctgaaactcggctgccaggggagctggagccacctgcgaagg
tgtcctcccatactggacccctacaggaagctccgtgtgcccagctggggcacagcccca
gctgatg (Seq ID No: 1043)

Homo sapiens ubiquitin associated and SH3 domain containing B (UBASH3B):

gctccttttcctttttgatccattcaaaattactcattgcaaattcccggactgctagg
cgaggagagggaagggggcggaggagacagggctactgcaggcgcagagctgggggcagc
cgggggcccgagtggctgaggctggtcccgcagcggccgcttgccggcgttctggctcct
gtggcctcaccaggaagcgtcagagtcccgacactggggaagctcggagcgccgcctccg
ctgccgccgcctcctgcctggctctgggtccccgagcccccttcccctggcccagcccgac
tccctcctccttcccgaaccatccggctcgggctccttccctggcgatggctggccgctg
agccatg (Seq ID No: 1044)

Homo sapiens transmembrane protein 241 (TMEM241):

ccgtctctgggcggctgctgccgctgccgctgctgctgctgcgggggtcgggcggcggcc
aggggatttgggcaggcaccgtggatccccgagaaggggacgagttgacagatg
(Seq ID No: 1045)

Homo sapiens ataxia cerebellar Cayman type (ATCAY):

gagcctctgccagccctgagctgggaagaagcagctacctcggaggcagggcgcgcaggc
gggcggcgatgagagggggcgcagccgcagccccgcgctggggagcccaccgctaaccct
gcaccccacccacccctgcacaaaagagctggcgggcgctggccacgtcgccctgggtga
ccttcctcggatgcagaatccgcccctgcgagcatcctcttcctcctaggctctgaaggc
ccggggagcgtgagcgatgcccagctgcacccgggcagggctcgcctttgtttgccagta
aggaggagaggctgtctcagctgcagaggggtcatccctgcttcaagccagtgcctcttc
ccagctcccatg (Seq ID No: 1046)

Homo sapiens ELL associated factor 1 (EAF1):

attcctctctcacccccacgcagaggagagaacttgcttctggacccgggtgggtgccgg
ctcggctctccttgtcttccagagcggtggcccggaagcacagtcctcccagacgccagc
gccagaagctcggatcgcggctgcaccgggagagcgccgatctgggtgcgaggcaggtgc
ggggccatg (Seq ID No: 1047)

Homo sapiens tripartite motif containing 5 (TRIM5):

gttcctctaggaaaattcctttgtgcagatcaggcccgtggattggtgagtgaatcctaa
ccacgtcttccctggcctgtcttcactcttctccccagaatcaccacttctgcactggtg
tctgaaggtgtattgagtgattttgtggagggcagaagtaggaagtctttgggacaaaac
tgtatttaccttgggatctgtgaacaagaggaacctcagcagccaggacaggcaggagca
gtggaatagctactatg (Seq ID No: 1048)

Homo sapiens wingless-type MMTV integration site family, mem ber 3A (WNT3A): cgccctctcgcgcggcgatg (Seq ID No: 1049)

Homo sapiens chromosome 16 open reading frame 45 (C16orf45):

ctccctccctgcagcccgcaacgggaatggagtaaagggagacccgtcgacctggccacg
gggatcagcgatg (Seq ID No: 1050)

Homo sapiens zinc finger protein 502 (ZNF502):

cattcttccggtttcagaagttaaggctggtgtcctggccccagtccacctctgggagcg
cctgcgccgctccgcggagagtccgtggatctcacagtgaaaaatgtttgctgacccttg
acattgacaaactgctgacagctcagatgatccatgattggaaggatgtggtcatcacca
agatgtctttctttctccggttcccagttttccagacctgaagtgttttccaatcaaagc
gaagagacgatctgtggatg (Seq ID No: 1051)

Homo sapiens armadillo repeat containing 6 (ARMC6):

ggctctcttgcgcaagcgcgctgtccgcttcttctgggcggacgctctggaggcaaaaca
tttccctgctggggcggcgaccaccgtgagcgtcccggaaggggcggcaaagacgcctc
cgtcgcgcacgaggtggcctcgttggctttaccttggttcgcggtcgtccttggttatcg
tgagcgtccgcgagtctctgggaggccaagcctaggggcgccacagcgcctgcgcgcgta
cggcggccggaaggggctagaggcggctccctgggtgacaaccgcgcgccccacctttcc
ccacgtggccgcgaagaccggctcaggagcatctatcggctgcacgccaacatcaacaca
ggcgaagatg (Seq ID No: 1052)

Homo sapiens post-GPI attachment to proteins 3 (PGAP3): gctcctcccccggcggcgagccagggagaaaggatg (Seq ID No: 1053)

Homo sapiens histone cluster 3, H2a (HIST3H2A):

tgccctcttgttttagtctcgcttttcggttgccgttgtctttttccttgactcggaa
atg (Seq ID No: 1054)

Homo sapiens ethanolaminephosphotransferase 1 (CDP-ethanolamine-specific) (EPT1):

ggctctcctaccttctcgggcagcccagtctttgccatccttgcccagccggtgtggtgc
ttgtgtgtcacagccttgtagccgggagtcgctgccgagtgggcgctcagttttcgggtc
gtcatg (Seq ID No: 1055)

Homo sapiens F-box and leucine-rich repeat protein 5 FBXL5:

ccgcctctgccccgcggcgagggtgtctatggagaggcggcggccgcggctgctgaggcg gaggctgaggcagtggcgatggcgccctttcctgaagaagtggacgtcttcaccgcccca cactggcggatgaagcagctggtggggctctactgcgacaagctttctaaaaccaatttt tccaacaacaacgatttccgtgctcttctgcagtctttgtatgctactttcaaggagttc aaaatgcatgagcagattgaaaatgaatacattattggtttgcttcaacaacgcagccag accatttataatgtacattctgacaataaactctccgagatgcttagcctctttgaaaag ggactgaagaatgttaagcctactactgttgactggaagccttaccaataacataaaaca atcgaataacaattatttcatgtattatatgtaaaatatatatactggattcttacagta agaatgaatatgaacagttaaattatgcaaaacaactgaaagagagattggaggctttta caagagattttcttcctcacatg (Seq ID No: 1056)

Homo sapiens major histocompatibility complex, class II, DP alpha 1 (HLA-DPA1):

ctgcctccactcggcctcagttcctcatcactgttcctgtgctcacagtcatcaattata gaccccacaacatg (Seq ID No: 1057)

Homo sapiens secretory carrier membrane protein 1 (SCAMPI): tcgtctctctctgcgcctgggtcgggtgggtgacgccgagagcca-gagagatg (Seq ID No: 1058)

Homo sapiens chromosome 15 open reading frame 57 (C15orf57):

ccgcccctcccgatttcctccgggctacaggcgacagagctgagccaagcgtttactggg cagctgttacggtaagtgaggaggggctggggtgcccagcgttttggatctcccactctg gcccggccccggaataccacatagaggccttgggacctgattcatcccgtccagacagcc ctagagacctgagcgactgaggcctgggatctggacgccggaatttcctgcgtggttctg gacgccctgccctgggctcagattccaaatg (Seq ID No: 1059)

Homo sapiens WD repeat and FYVE domain containing 2 (WDFY2):

cctcctcttgtagtggcgccggcttgcatcccaggtcgtggcggttttggtgcctgaagc agggagcgcggagtcgttcccgagagaggcggccaggctatgctcgccggtttccggcgt tccgctccggccagccagagtctctgtctcaacctgtgtccgtgctccagcagtctcctc agcccggccccgcggcgcggttggcggcggcgccccaggcgcgccccctcctccgatg (Seq ID No: 1060)

Homo sapiens topoisomerase (DNA) I, mitochondrial (TOP1MT): cgctctttcccggaggctggcagatg (Seq ID No: 1061)

Homo sapiens intraflagellar transport 122 homolog (Chlamydomonas) (IFT122):

ctttccctttcggacatgcgcgctcggagcaaggcgccctcgcactcagcttaccgcgca tgtacgttgccaggggtaacgcaggtagccaaagtggcttgtggagtggcgaccgttagt gaggcggttgctgagacagacgctgaggcgggtaggaggagcccgagccgtaaggaagc cgtgatg (Seq ID No: 1062)

Homo sapiens mitochondrial ribosomal protein L53 (MRPL53): agttcttccggggcggaggtcaccatg (Seq ID No: 1063)

Homo sapiens T-cell activation RhoGTPase activating protein (TAGAP) :

ccgccccttcgcttataatgcagagcatgtgaagggagaccggctcggtctctctctc
ccagtggactagaaggagcagagagttatgctgtttctcccattctttacagctcaccgg
atgtaaaagaactctggctagagaccctccaaggacagaggcacagccacacgggagtga
aatccacccctggacagtcagccgcaatactgatgaagctgagaagcagccacaatgctt
caaaaacactaaacgccaataatatggagacactaatcgaatgtcaatcagagggtgata
tcaaggaacatcccctgttggcatcatgtgagagtgaagacagtatttgccagctcattg
gacattctcactattctatgccttaaaggcccttcaacggaagggatattcaggagagca
gccaacgagaaagcccgtaaggagctgaaggaggagctcaactctggggatgcggtggat
ctggagaggctccccgtgcacctcctcgctgtggtctttaaggacttcctcagaagtatc
ccccggaagctactttcaagcgacctctttgaggagtggatg (Seq ID No: 1064)

Homo sapiens phosphoserine aminotransferase 1 (PSAT1): ggtcctccttggctgactcaccgccctggccgccgcaccatg (Seq ID No: 1065)

Homo sapiens CD97 molecule (CD97):

ccccctccttcataaagtcctggcctcgggacagcctgcacagctgcctagcctgtggag

acgggacagccctgtcccactcactcttcccctgccgctcctgccggcagctccaacca
tg (Seq ID No: 1066)

Homo sapiens protein tyrosine phosphatase, non-receptor type 2 (PTPN2):

cgctctccccggatcgtgcggggcctgagcctctccgccggcgcaggctctgctcgcgcc
agctcgctcccgcagccatg (Seq ID No: 1067)

Homo sapiens chromosome 20 open reading frame 112 (C20orf112):

gcccctctccccgggcagccgcggcggcagcagcagcagcagcagctggagctgtggggc
tgtcaccgccgcccgccccgctcactcgcggatcccgaccgcccatctccgcctcgcttc
cagcccaggatgagacttctgtgagcagcgaggattttgatatg
(Seq ID No: 1068)

Homo sapiens APEX nuclease
(multifunctional DNA repair enzyme) 1 (APEX1):

cacccttctttgtgctcgggttaggaggagctaggctgccatcgggccggtgcagatacg
gggttgctcttttgctcataagaggggcttcgctggcagtctgaacggcaagcttgagtc
aggacccttaattaagatcctcaattggctggagggcagatctcgcgagtagggcaacgc
ggtaaaaatattgcttcggtgggtgacgcggtacagctgcccaagggcgttcgtaacggg
aatg (Seq ID No: 1069)

Homo sapiens intermediate filament family orphan 1 (IFFO1):

tttcctcttgagccatcatgcacatctgactgcagccccagcgagcccttccttccttgt
ctgactgctcttcttctcgatttcttcttgttctgccttctcggtttgcagccctgaccc
ccgctgtgtgtctggcccttggtgactgtccgtgtttctgttcctgtcattgtaactgtg
acttttctctctgtctgcccccccttcctactggttcatgcttctcccccattcccaccc
tctctgcccggcctcccgctcccgccctttctcctcatgcacccggcctcgtctctgtag
tctctgcacttgtctcccattaaggtcccatccatg (Seq ID No: 1070)

Homo sapiens neuralized homolog 2 (Drosophila) (NEURL2):

cagtcttcctcccgccccttctttggtccctacggacctgggggcggtggcggtcaatg
ccgggtcaaggtccgcgggcctcgcagatcgtagcccgggcgcacgcgatcagatgatcc
tgttgtggacggctaagttgtaggcgggatggctgagaaagcggcgctaggacccccggg
cagaggctcggggaagggagtcagggggaaatgccttacaaggtcgccttgcggtcacc
atcattgcccgccgcccaaaatagcccccggcgccagctggcctgccctatggccgagag
atg (Seq ID No: 1071)

Homo sapiens drebrin 1 (DBN1):

ctccctctttccctccctcctcctccgtccgcccgtccgtccgcgcgtctgtccgttcgg
cccggtccggcccgaagcatg (Seq ID No: 1072)

Homo sapiens WW domain containing adaptor with coiled-coil (WAC):

cagcctcccttatttagtccgcgatggcttccctcgcgccccaccgtcctcttccggaag
gcggctccctccctgcgcagcccggagcccctgagatcagcctcgagcaggcgcccgagc
gagactatccctaaacgggaacggcggtggccgactcgcgagtgaggaaaagaaggaaag
ggcagactggtcgcgaagagaagatccaggcctcagaggaggagaaaggccggagccagc
cgaggtttgccgagggcggtgttccggacccgcgcggtgcggggaggaaggccgagggtg

ggagaggaggggcccggcggaaactgccgaggtttcccgaaggcggcagcgtccgagttg
cccggatgtagttggtggagcggcagcggcggcaccagcggcggcggcggcggcgggagg
aggaggaggagaagaaggaccaggcggcggcagcagcggcggcggcgggggggaggagggg
aggaggcggcggagcaggaggaggagaaggcggaggaggcagtcgctctccgcggggctg
agccggacgcgtcgtcttgccccccctcccccggttcgcggtgccgccgtgtagttggcg
ccgctgccccggctgagagtgagcgtggtgtcgacggagggagatggcccgggagcgccg
gcgccagtaactgggagctgatgagagtcgccgagggcgcgccgggcccaggtgccgggg
ctgcccgccgcccgccgccgccgccgcctgcgcgcccgcccgcctttcgcggccgctctc
ccccctccccgacacacactcacaggccgggcattgatg (Seq ID No: 1073)

Homo sapiens kelch-like 6 (Drosophila) (KLHL6): cgctccttcagtctcgatg (Seq ID No: 1074)

Homo sapiens GTPase, IMAP family member 1 (GIMAP1):

cagccttctgcactcacagccgaagggaaagcagcaggttggggcttcttgtggccaact
tcagagcctgtcaccaggaaaggtaagcatg (Seq ID No: 1075)

Homo sapiens RAB24, member RAS oncogene family (RAB24):

cgccctctagccccctcccgcgggagtcgcggcgctgcgggtaggagccgggttgcggga gaccccaggttcggttgggattcccagccagaacggagcttaagccgggcaggcgagcga atgacggagtagcgagctgcacggcggcgtgctgcgctgttgaggacgctgtcccgcgcg ctcccaggccgccccgaggcttggggtcttcgaaggataatcggcgcccggggccgaaca gcgggggcacacggggcgctgccgaagtgcaaggccacggccagagctcgagcccgacgc gctgtctggagtcgtaggaccctgacgtggctgaagcggccccgggagcatg (Seq ID No: 1076)

Homo sapiens adaptor-related protein complex 2, alpha 1 subu nit (AP2A1):

agccctccccgcggccggctcggctccttggcgctgcctggggtcctttccgcccggtcc ccgcttgccagcccccgctgctctgtgccctgtccggccaggcctggagccgacaccacc gccatcatg (Seq ID No: 1077)

Homo sapiens copine IV (CPNE4):

ctccctcttttctcagtaccctcctctttactctccgagttaactgagagccgacctgac atctccaacattttcaccctcttcccccacccccatcaccgagaatggagtcagggtttc cggagagaccgaactctgctctcagcacctttcccagccgctgttgctaaactgacctcg gaggacgagaggggaaggaggtgcgacgccccttacatcagtacataactaccacaccaa ccacctccacttcaaagccggattttgcatcctgggggcgggacagacctcgtcccgggc tgaattctctctccactcttcgagattggcacacccagaatg (Seq ID No: 1078)

Homo sapiens synaptosomal-associated protein, 25kDa (SNAP25):

ctgtctttccttccctccctgctcggcggctccaccacagttgcaacctgcagaggcccg gagaacacaaccctcccgagaagcccaggtccagagccaaacccgtcactgaccccccag cccaggcgcccagccactccccaccgctaccatg (Seq ID No: 1079)

Homo sapiens cAMP responsive element binding protein 3-like 4 (CREB3L4):

aggtctcttgactctttccgcctttgtttacaaccctgccatgatctccctcttgcaaaa gcgagggctacagaacaggcattcaggagtcctgtgctccagtcacagccttttctgttc

ttcagctaggagacaccaaaccctcaggaagatttactatagctaagagaaaactgcagc agaaagggcgcggctacctacttcttaaattccgtttgtggaccctcagactcttagtcc cctactcccagatacagcggccctaccgtggctcctggcaaggtggcatccacttttgta gtaagcatg (Seq ID No: 1080)

Homo sapiens leucine-rich pentatricopeptide repeat containin g (LRPPRC): ctgtccttctggcggagcgtgcttcccgctgcggggacgttcgagcaatg (Seq ID No: 1081)

Homo sapiens zinc finger protein 418 (ZNF418):

cgttctctggtagcgaccattttggttaatgttgggtgtgtttctgcggtttgtgaggtg agaggcgctggagctatgggtccgaaccgcggtgtctgaacccagaaggtgaagagtcct tcttgctgcacagaggcagatcttaggccccgtaacggcgcccgccgctcccggcagtgc tttccccgcgtactcgggatggcggcggccgcgctgaggctcccggctcaggcatcatct ggctgcaaagaagagaacacactgtgtttgagggaggaggaaggaggatcagagtttaaa ctcctgccataatg (Seq ID No: 1082)

Homo sapiens tetratricopeptide repeat domain 14 (TTC14):

gtttcttccgcttcctgtaccacccggctcaagtagcggacacggaacagggaactatca gcccgtcggcctccgggccctgcattctctagccatg (Seq ID No: 1083)

Homo sapiens BMP binding endothelial regulator (BMPER):

agccctttcgactgtgagctgcggcagctgagcagaggcggcggcgcgggacctgcagt cgccagggattccctccaggtgacgatg (Seq ID No: 1084)

Homo sapiens zinc finger protein 384 (ZNF384):

ccccctttcgtttccggcgctcccgccttctctccgcagagctcttctctgagcctgtt gggggagggaggggggcgtggaggaactggggttcgcgggagcacgagctgcagcacca cttccgggtgagtgcaagggggagggcagcaaggagggggggccacccactacctcgcgcc cccgccctgcgggtgtctcgcgcgcgttccgtgcgtgtgagtgtgtgggtctgtctcgct ccagaagtgcgtgcccgcgcgctgcgccttgcgctttttcccctccctcgccccttcctg gtcctcccaccctcctcggctccctcctttcccagcaaacgccgcccctcccgcgccctg gctcaggctctggcgccgccgcagccgtcgccgcccgaaagttcaggagccctggaaagg agaaggaataagacggcaggaggaagagagagagagggtagaatg (Seq ID No: 1085)

Homo sapiens RAD51-like 3 (S. cerevisiae) (RAD51L3):

ctctcctttctcctccggcagccagcgcgcctgtgtcctctctaggaaggggtaggggag gggcgtctggagaggacccccccgcgaatgcccacgtgacgtgcagtccccctggggctgt tccggcctgcggggaacatg (Seq ID No: 1086)

Homo sapiens CD99 molecule-like 2 (CD99L2):

gctcctcctcccgctcctcctcggcctccccttcgggcgctctcgcgctaactgtgctcc tccggggccctccgcctgctcccagccatg (Seq ID No: 1087)

Homo sapiens glucosamine-6-phosphate deaminase 2 (GNPDA2):

gcgcctttatctgcatccgggtccgtgggattcgcgctccactggtcagctggggtcgct ctcgggtggttgggtgttgcttgttcccgctgttccagcgtcgaagaaccattgggtctg ccggtttgaacttgttctggaagctgtgcgtcaccgtaatg (Seq ID No: 1088)

Homo sapiens methionyl-tRNA synthetase 2, mitochondrial (MARS2): ccgcctcctccgcttgcggccggtctgcaccatg (Seq ID No: 1089)

Homo sapiens chromosome 12 open reading frame 57 (C12orf57):

tttcctttccgctcccaggggcgttgggaacggttgtaggacgtggctctttattcgtga gttttccatttacctccgctgaacctagagcttcagacgccctatg (Seq ID No: 1090)

Homo sapiens tRNA-yW synthesizing protein 3 homolog (S. cerevisiae) (TYW3):

ggacctttcggccaccgctcgcttcaatatggctgcccccagggagagacgaggctacc atgaaggagccgagcgcagaccctgagtccgtcacccatg (Seq ID No: 1091)

Homo sapiens Sp1 transcription factor (SP1):

ctccctcctccttacccccccctccctgtccggtccgggttcgcttgcctcgtcagcgtc cgcgtttttcccggcccccccaacccccccggacaggaccccccttgagcttgtccctca gctgccaccatg (Seq ID No: 1092)

Homo sapiens histidine triad nucleotide binding protein 3 (HINT3):

cgccctctagtggcagccggttttgaggccggcctccggctttgaagttcctcaccgcgt ctccttccctctccccaaagcctggatcaccgcccagcgtcaggcgaggggcgacgtctc gaggtaaaacggaggaggtgcgggacgcggagactgcgcgggcccggtagccctggagag gccgaggctctaggccgcgaggggcgggtgcaatg (Seq ID No: 1093)

Homo sapiens M-phase specific PLK1 interacting protein (MPLKIP):

agttctctgcggagggccggttgatacagttccggtgggagaacgcggctgcgaggtttt cggctttggctcctgatatg (Seq ID No: 1094)

Homo sapiens palmitoyl-protein thioesterase 2 (PPT2):

caccccttcccccccgccaccgtgggttccagacttgggataagtaaacagcgggtggagcg aggcctacggacccaggccaggtgggagtctgcactcttcaaggggcctgggctgctgct cacgggtattaaagaactccgcgttgttcatggctgaggcgatgcattaggaagatcctg gacctagagaacaagtcccccgaacgctgagttggaggcgggacttcgggtgcgcgttgg cgggagcatg (Seq ID No: 1095)

Homo sapiens BCL2-like 14 (apoptosis facilitator) (BCL2L14):

aagcctcttttcaggctgagtcctaaacctgaagaaagtttagagcctggggctctaaac tacctgagtctttccaaacgacaagccaagaagacctgttgaaagtttcctcttaagttt cgtggagagagactcaggtatagaaatatccttactgccacctgacctgaagcagaagaa atcacagacagcttccagaccaggcccaacatg (Seq ID No: 1096)

Homo sapiens galactose mutarotase (aldose 1-epimerase) (GALM):

acgccccttctcctgtaaacttgggtcgcctctagcttagcgagcgctggagtttgaaga gcgggcagtggctgcacacgccaaactttccctatg (Seq ID No: 1097)

Homo sapiens carboxymethylenebutenolidase homolog (Pseudomonas) (CMBL):

cttccttcccttccccgactttgcagatttctcttcccccaggcctccctcctccacctc
tccgcccccctccgggcttggctctcccaggaggctacgactggagccactggtcccgcag
gatccccgcgtcctcggtcgccgcgtccacgtccctctcgcgtccccgcccggcgccacg
ccgcctcctctgggttcggcctccgcgcggtgcagcgcagtctcaggccgcgggacaagc
ccgacttaaatctctgcaatg (Seq ID No: 1098)

Homo sapiens chromosome 7 open reading frame 31 (C7orf31):

cgtccttctcccgcccccgcccctgcctgccagctccaccgggccgtaggtgcggacgac
ctcaaaattcctcggcccgcgaaggccgccagctgcggggaggggaggggaggcgcggtc
ccgcagcgcccccaggctcatgtcccaggtatgtccagacccccgaggcaccgcttgcag
ggcagtgacagcccgtgaggctcggcctcgacccctggcacccttggtcccagctacgcc
ggctcctggccttcccccaagtccgagagagaggtgggattctccccgacgcagttggaa
accgggaatcccctttagggtcccgttcgtgctgcactactgactccaccatctgcaaag
ggattcttgtccagaatccccgaaggctttaggacagcgcttattttgttgaatgaagag
tctctaattttcggaaagaccacaggctaaaagtcaagttgtgccttttagccaagaag
catg (Seq ID No: 1099)

Homo sapiens secretory carrier membrane protein 5 (SCAMP5): cggcctttcggcagccgaacggccgcggcagttcaggacaaagaggtgtgggcaggccac tgggccagctggtaacatcatg (Seq ID No: 1100)

Homo sapiens mitogen-activated protein kinase 10 (MAPK10):

tgctcctttcggttgccatagcaaccccattccccaagccctctgtccgtctcctctggt
aggttccacaatggtacaggcagcatcacgctgcacaatggtttccaggcagtgaaagag
ggtgattcagcaagccactcttcttctatttctttaacctccccttcacttttatttt
tatgggggtgggtggtgcttgctatatgcttaccttttttcttttcttttttcatttttac
aaatttcctttttttgtcctcacccctcaattcctaggggcttgagtgagtttaagattgg
gttttcttggaaatcacctgtccatcgttaattttaaacaatctccatatctccaaagaa
tctcttccatgttagtctggaatgtggttaatgaaaaacaagtagggaggatttctgggg
caaacactgccggatcaggatcgtagttctcaggcacggaatggctagtgtgagaaacac
caacagcaggcccatctcagatcttcactatggcaacttatgcaagaaactgttgaatta
gacccgtttcctatagatgagaaaccatacaagctgtggtatttatgagcctccatttct
tatactactgcagtgaaccaacattggatgtgaaaattgccttttgtcaggtgtgtgttc
cttacaggtaaaacaaggattcgataaacaagtggatgtgtcatatattgccaaacatt
acaacatg (Seq ID No: 1101)

Homo sapiens beta-site APP-cleaving enzyme 2 (BACE2):

cgtcctccccgccgccgccggtcccggtgcgcgcccatccctgcccgcagccccgcgcgc
cggccgagtcgctgagccgcggctgccggacgggacgggaccggctaggctgggcgcgcc
ccccgggccccgccgtgggcatg (Seq ID No: 1102)

Homo sapiens SWI/SNF related, matrix associated, actin depen dent regulator of chromatin, subfamily d, member 1 (SMARCD1):

acgccttttccgctagtcgccccgctctatcccatagtctcgctgccctgagcctcccgt
gccggccggccggccgggggaacaggcgggcgctcgggggcgctcgggggcgggggga

gttccggttccggttctttgtgcggctgcatcggcggctccgggaagatg (Seq ID No: 1103)

Homo sapiens family with sequence similarity 175, member A (FAM175A): cgtcctcttgtgtagcctgaggcggcggtagcatg (Seq ID No: 1104)

Homo sapiens adenosine deaminase domain containing 1 (testis-specific) (ADAD1):

aggcctcttttgaaagatgcggccctgaccctgtgaacctcgcgcagagcggcctgaagcgagaggttgaggctgggaggtgagaaaatg (Seq ID No: 1105)

Homo sapiens acyl-CoA synthetase short-chain family member 2 (ACSS2):

gcccctctacggaggccccgcctctagttcggcctgttttctcagtcccggcacccgccgcgaccgcaaaggcggccgcggttctaggaacttgacgtgatg (Seq ID No: 1106)

Homo sapiens multiple coagulation factor deficiency 2 (MCFD2):

cttcccttactcaccggtgtccggaaaggtgaacgctgcgctcgggctgcctcgcctgttacctccgccgccgggcatg (Seq ID No: 1107)

Homo sapiens SPOC domain containing 1 (SPOCD1):

gctccttttcagctagtgggtggaaccccaggagggaaaactcagggaagcccagggcccgtgttgtgcttttggcccaggtaggtggacagacatg (Seq ID No: 1108)

Homo sapiens LY6/PLAUR domain containing 1 (LYPD1):

agttccttcagtctcagccgccaactccggaggcgcggtgctcggcccgggagcgcgagcgggaggagcagagacccgcagccgggagcccgagcgcgggcgatgcaggctccgcgagcggcacctgcggctcctctaagctacgaccgtcgtctccgcggcagcagcgcgggccccagcagcctcggcagccacagccgctgcagccggggcagcctccgctgctgtcgcctcctctgatgcgcttgccctctcccggccccgggactccgggagaatg (Seq ID No: 1109)

Homo sapiens cytochrome b5 domain containing 1 (CYB5D1):

cattctttcatactgcctcctcccttgtttttctgtctcagagagatagtctgtcctaaatatcccatgtagcccaggccactgaattaaaacggagcgtattcgttctctgccccacccgcaactcctgaaagcggcgcaactcaattacttgatccttatatgccccacgcgggactcatactacgtttcccgtgaacacgtgcagtccaaaccccgcccctgatatttatctcagtggacggtggccggaaaaggacaatggtttccatgtcagcggataaacgctctcccctcggctcccggacgcgacggaggtcgtagtagtagtgagtacgtgctgaggagcaaaggagtaaccaagagatccagtgaccgacagagcaagagccatg (Seq ID No: 1110)

Homo sapiens synaptoporin (SYNPR):

tctcctcctttgcttcataaaaagagggacaagtggctggtgctgtggacagagaagctttatttttagtatgagacaacctctatttctttcaggagagggaagttggattatcaattcttttgtaaatg (Seq ID No: 1111)

Homo sapiens heterogeneous nuclear ribonucleoprotein U-like 1 (HNRPUL1):

ccccccctttccccttcgcctcctgacaggaaaggtttaaggggacagagccctggga

ggccgggccgggctcggggccaccccgggggcccgggccatg
(Seq ID No: 1112)

Homo sapiens schlafen family member 5 (SLFN5):

ggttctctgctctggacttgggaggctccgttgcctgctcccggagggagacgcgctgcc
gaggagaacccagcgggagaacatttcaggataggaataggccaagtgctgagaagatg
(Seq ID No: 1113)

Homo sapiens MAS-related GPR, member F (MRGPRF):

ccatctcttccagcaggagagggctctactctgagctcctattttccaaggctccgggcc
gcgctcggcgctggcctgctgccccggcgggtccgccggccggaggcgggagtcacagga
agagccctccacaaaaggaggcctcggcggatcaggacagctgcaggtgggtgtgcagac
tggtgagctgccagcaggggcccagacgcgccaggcctggagatg
(Seq ID No: 1114)

Homo sapiens ubiquitin-like domain containing CTD phosphatas e 1 (UBLCP1):

cggtctctcagcggccggtttctgcgtccgctgccgcaggttccaccgcgctccaggtat
ttttttttctgaaggaaagctgcttcctcatatgtttcaagaatg
(Seq ID No: 1115)

Homo sapiens Rab interacting lysosomal protein-like 2 (RILPL2):

cctccttttccgttgtcccttcgcgccccaaaccacatcctggagcgcactctccagcgt
ggctggcagcggggacggtgcgccggggcgcaggcccaagagtcgcgtgcgcggcccctt
gcaccatccccccgggcccacccccgggccgcgctgattgggcaggtagggactctgccc
agcggaaagttttgggtgccgggaggaagtctaacctttgggagactccaagacagcagc
tccgaggtcggcgggggtctgggtggccatg (Seq ID No: 1116)

Homo sapiens zinc finger with UFM1-specific peptidase domain (ZUFSP) :

acttcttttccgtgggagtaaggaagtgcttttgaatgaggtactgagggccaaggtgtt
ggaagttcctaattctttcctcggttaactgtgaaactctgcgtattgggaaggcctggc
ctcagtcatcaggccaggagaggtactggacgccgcgcacgcactcgtctgccagcgagg
cccaaaggggaagcctagcggagctcagtgtggcagctgctggcctctgggccgctactt
gtcaataccatg (Seq ID No: 1117)

Homo sapiens mitogen-activated protein kinase kinase 5 (MAP2K5):

ccgccttcctcctcctcctctcgccgctaccgccgtcgccgccgccgcagccgccgccgg
tccgcgcggcctcgggtggccggagctcagcctgcgcgcgccgcgccctgtgtctccggg
tggggcagaagactcgccccttgaacctcccgcggggactctccgtggtgtggcggccct
ggggctctttcttaatagccccggactgagtcccctccagtcgaggaccctctcctagtc
cactgacgagcggtggacacctgccgctgtatctcccccaaaccgagtccttgccctgct
gcctcctcatacccacacggcggcagagaccttcaccatagcgttcgctcaactccagaa
ccttccgacctccgctagttcctgcgggcctttgcccgcttcccggtgcaccctccccgg
gagacacctcagacccccgacagcctgggcaggctcggtgcctgcgggtgcgttcctgat
caccccctcccctcttccctccccctcatcctccattcccttgttttcaccctctgtcctc

tgcccgtcactccccttgtcacctcttggagcccccctcctaaccagcggccagtgggttt
cccataccccaggatgtgagcctctttaacctgtaatg (Seq ID No: 1118)

Homo sapiens solute carrier family 2 (facilitated glucose transporter), member 12 (SLC2A12):

cactcttctttagcatgctattatggggaaagtgaccactcctgggagcgggggtggtcg
gggcggtttggtggcggggaagcggctgtaacttctacgtgaccatg
(Seq ID No: 1119)

Homo sapiens mitochondrial ribosomal protein L30 (MRPL30):

cttcctctgctctgcttcccttcggaggaaaatttcaggctgaaggtttagcgggtgccg
cctctaaagagagcaatcactacacttatg (Seq ID No: 1120)

Homo sapiens tripartite motif containing 11 (TRIM11):

gctcctcttcctgccggcatccgggatccctacgtcccgcgtcccccgagcgctcggagc
ctacgcgcccagcgctaccgaaacccagagtcctgcgccctggagtccccgcgccccgga
gcccgagcacccgggagtcccgagcctcgcgccccggagtgcccgagcctgcgccgccgc
acccggataccccgcgtccccgcgagctgccgaggccgcccgccgccgccccgcggacag
taccgccttcctcccctctgtccgcgccatg (Seq ID No: 1121)

Homo sapiens proline-rich transmembrane protein 2 (PRRT2):

ctccctccctagctgacttgctccctcccgggctgcggctgctgcaaaagccagcagcgg
cagcgggagctgtccggaggccggcgtcgagggtttgccgctgtctctgctattccatcc
tccccataggggctctctcccctctcccatctcaagatg (Seq ID No: 1122)

Homo sapiens zinc finger protein 626 (ZNF626):

cggcctttgtctctcgctgcagtcagagctccaggtctggttcttctcctaaaggcccag
gctgtgtggccccgtgtcctgcaggtattgggagatccacagctaagacaccgggacctc
ctggaagccaaaaatg (Seq ID No: 1123)

Homo sapiens solute carrier family 25, member 43 (SLC25A43): cggtcttccgggcccgggtcggggctcgatg (Seq ID No: 1124)

Homo sapiens crystallin, zeta (quinone reductase)-like 1 (CRYZL1):

ggctctctgacgaaggactggaaggtggcggtggtgaaggtgcaggccgttggggcggct
cagaggcaggtgactatg (Seq ID No: 1125)

Homo sapiens mitogen-activated protein kinase kinase kinase 7 (MAP3K7):

ctgcctctacccccgccacggatcgccgggtagtaggactgcgcggctccaggctgaggg
tcggtccggaggcgggtgggcgcgggtctcacccggattgtccgggtggcaccgttcccg
gccccaccgggcgccgcgagggatcatg (Seq ID No: 1126)

Homo sapiens septin 6 (SEPT6):

ctttctctttgtcggaggagctcctctgtttcctgtgcagtagctcccgttgcggcggca
cccgtggcagccctggcggacgcaggagcgatg (Seq ID No: 1127)

Homo sapiens myotrophin (MTPN):

ctgcctctcctcggccaggcggaacctctctgctgggcccggtggccgcaaaagaacttt

ctttctcccgcccgaacggtcgccgcggccaactgcctcgcccgcctggcagcctaaccc
tccttctcttcttctcctctccggcttcgcgcggccctgcctccctctcgcccggcggca
tccgcttgctgctgccaccgcctcctcatcttctgcccggccaaccggcctgccccgctg
cagtgatg (Seq ID No: 1128)

Homo sapiens annexin A11 (ANXA11):

ccctcccttgcactgcctctggcacctggggcagccgcgcccgcggagttttccgcccgg
cgctgacggctgctgcgcccgcggctccccagtgccccgagtgccccgcgggccccgcga
gcgggagtgggacccagcccctaggcagaacccaggcgccgcgcccgggacgcccgcgga
gagagccactcccgcccacgtcccatttcgcccctcgcgtccggagtccccgtggccagg
gattattggacctgcctggtttaaactattgtcttagttaattttgtgctgctctaacaa
aatatcacagactgagtaatttataagcaatagtagcttatttggctcacagttctggag
gctgagaagatcgtgaggctgcatctggcaagggccttcttgctgcttcataacatggca
gaagacatcatgcgggtgtgtgtctggggaagagacttacagaagtggagttgctgagtc
aaagatctaaccatg (Seq ID No: 1129)

Homo sapiens RNA binding protein, fox-1 homolog (C. elegans) 1 (RBFOX1):

ttttctttctttcctctcccggcgttgatgagtgcttggctcctgacagaagggatttgg
ctcccagctttgtagttcggaagaagttgggtctatagatttccccctaactctccattg
atgtgttgagcttcagagggaataataactctacgtaaagcatg
(Seq ID No: 1130)

Homo sapiens prefoldin subunit 5 (PFDN5): cttcctcttcgttaagtcggccttcccaacatg (Seq ID No: 1131)

Homo sapiens high mobility group AT-hook 1 (HMGA1):

cgctcttttaagctcccctgagccggtgctgcgctcctctaattgggactccgagccgg
ggctatttctggcgctggcgcggctccaagaaggcatccgcatttgctaccagcggcggc
cgcggcggagccaggccggtcctcagcgcccagcaccgccgctcccggcaacccggagcg
cgcaccgcaggccggcggccgagctcgcgcatcccagccatcactcttccacctgctcct
tagagaagggaagatg (Seq ID No: 1132)

Homo sapiens zinc finger protein 323 (ZNF323):

cggcctttgcggttgatcggtcattggggtgctgcagccccgccacctgttccgtagctt
gccggtgccccgaaggtgtcttctcctaaggaagattaaatcagaaattttaaatcaca
gttatccctttacttaaagccagagtaagccttccaaattaaccccaggaatg
(Seq ID No: 1133)

Homo sapiens tumor protein p53 inducible protein 3 (TP53I3):

ctttctcttctcttagcagcacccagcttgcccacccatgctcaagatgggcgggatgcc
agcctgttacataaatgtgccaaaagcctggccatgcctggaaaatggaccaatccgccc
gccaagaggttgggtctcgttccctagagagaaggaagtttcctctccttgaagtgagag
ctagaatcgcactttctgtcaagctgagagaaagactcttttccagaggctaaaaggaca
agaaaatctgatttgcttgcttctaactttgcgttttaaaggggaaggaggaaaggaaa
gagggggagggtggttctgcttagccccacccctccggctaccccaggtccagccgtcca
ttccggtggaggcagaggcagtcctggggctctggggctcgggctttgtcaccgggaccc
gcaggagccagaaccactcggcgccgcctggtgcatgggaggggagccgggccaggaaca
atatg (Seq ID No: 1134)

Homo sapiens ceramide synthase 5 (CERS5):

ccgcctccccgcgggttccgttggctgtggcggcagctgacgcttgtggcggcggtggct
tcggggtgggcgtaagatg (Seq ID No: 1135)

Homo sapiens TRAF3 interacting protein 2 (TRAF3IP2):

tgttcttctacttacctgggcccggagaaggtggagggagacgagaagccgccgagagcc
gactaccctccgggcccagtctgtctgtccgtggtggatctaagaaactagaatg
(Seq ID No: 1136)

Homo sapiens Smith-Magenis syndrome chromosome region, candi date 7 (SMCR7):

ggtccttcacgttccattcccaggctggtctgagctccggggccgtggtcccgctgcctc
ctccggtcgtcgtgcggaagctgcgacgcaggcagaccatg (Seq ID No: 1137)

Homo sapiens mitochondrial ribosomal protein L10 (MRPL10):

cattcttccggtggagatggctgcggccgtggcggggatgctgcgagggggtctcctgcc
ccaggcgggctagagtgcagtggcatg (Seq ID No: 1138)

Homo sapiens proteasome
(prosome, macropain) subunit, alpha type, 1 (PSMA1):

acttctctgtagatcgctgagcgatactttcggcagcacctccttgattctcagttttgc tggaggccgcaaccaggcccgcgccgccaccatg (Seq ID No: 1139)

Homo sapiens sorting nexin 5 (SNX5):

cggtctttctctagacgcgtcttgctgggagagtgtccgttgcttcccgtccgtgtcgcg gccctgcggttggcggcctcctcgtggagcggagcaaggccaggcggcccctgctcgagt cccgcgtcgccatg (Seq ID No: 1140)

Homo sapiens zinc finger protein 276 (ZNF276):

gggccccctccgcgcgtactgcgggccccacgggtgttagtggcgggggcggcagagtcc gggtgggttgtcgcgacggagccgggcctcttcgccgtcttgagacggggctggcgagaa gggcccctcacggagttgccatgggcgtctaaccgcggcagccaggcccctctctacgtg agaccccggccccccctcccctttctgcagcccgcccgccacctgcgcgccgcgtggcctc cgccggcgcctgcccgccccgcgcctccgtctcccacggagcaggccgggctctcgccat g (Seq ID No: 1141)

Homo sapiens zinc finger protein 561 (ZNF561):

ccatcttttccggcgctggctcctctccgtcagtgcggtttcgcctttatggtggtggag tctgcccaggctgtggaccgcaaataaccctgtacaaagaggaatggagattgcctctat ccacctagattcataagctggcctgaggtgatcttggcatcaaggaagggatgcacatca tcacaccatcagcttcagagaatg (Seq ID No: 1142)

Homo sapiens mucin 7, secreted (MUC7):

ctttctcttcttttgcttctagttaccatcctcaaaggattggctaaaagcaagcaactg gattgaacaccctaagaagaaagattcacactgcaccaggagacatcagaaagaatg (Seq ID No: 1143)

Homo sapiens threonyl-tRNA synthetase (TARS):

gcgcctttcgattgcatcagctggtccagccgaggccaagtcccgggcgctagcccacct

cccacccgcctcttggctcctctcctctaggccgtcgctttcgggttctctcatcgcttc gtcgttcgccaatg (Seq ID No: 1144)

Homo sapiens ATPase, Na+/K+ transporting, alpha 3 polypeptid e (ATP1A3):

cagcctctgtgcggtgggaccaacggacggacggacggacgcgcgcacctaccgaggcgc gggcgctgcagaggctcccagcccaagcctgagcctgagcccgccccgaggtccccgccc cgcccgcctggctctctcgccgcggagccgccaagatg (Seq ID No: 1145)

Homo sapiens chromosome 11 open reading frame 46 (C11orf46):

cgtcctctcagtggtagcgcggggactggctgggaagcggtcggtcgagtgtggcctgtg
tggactcgcatcttgcccgaagccgggcggaggagagctcaagctaagggtgatcagccc
atgacctaaacctccagacaaaataaaacggaaaatttgctagaatcaagaatg
(Seq ID No: 1146)

Homo sapiens chromosome 17 open reading frame 45 (C17orf45):

tgacctttcattcccgttgttatggaggtaggctctctaggaatctgggagtagtagct
gggggcaagagcaaataaagagctcgagcttctgtggtctctggggagatg
(Seq ID No: 1147)

Homo sapiens AHA1, activator of heat shock 90kDa protein ATP ase homolog 2 (yeast) (AHSA2):

gggccttctggcagtttctgggagctgcgaacgcgccgccccggggctcggcggccggaa
acgctggcttcggagccttaggcgccgcggcctttccttgttttccgcccagtccacgcc
gccatggccaagtggggccaggggaacccccactggatcgtggaggagcgggaggacggg
accaacgtgaacaactggcgctggcgcggctggcggcggcctccttccgggatctgggga
gggccgggccgcgggagccggggctgccctggggtctgtgcggggccgcggggccagggg
gtcagggggccgccccccctcagctgctggacgcagggctcggccttcgcctctcggctc
gggagagtccttgagtacggagaccggctaggagggttgcagctgcctctttttgaaagt
tgggttgggccccaagagtgacttccgacagacctttccactcccaccgtctgtggcctg
agggccttcccttctcctcccgcccacccctctggatgtttcggggagttagaagggagc
tggattgagagactgtgttaggggcgggggtatggaacgtagtggaaagggcagaaattt
ggatctcagttcgcgcccaccccgcaggcgcctcccgcgagccgggccctctgtgagtga
gacaagctccccttcctttacgcgcctcacctggcgcgtggggagaggtcggcagccctc
cgccgcagaacctccggaagggatgtcctctgccctgcgcctctggccggggctgtggtc
cctccaggccgtcgaggggatgctgaggccggtccccagaggagcatgacttggctggtc
cggaggagctctgagggcatgggcaatcttggctcgctgcaacctcagcttccagagttc
aagcgagtctcctgcttcagcctcatgagtagctgggactacagatgcgtgccactacgt
ccgtctgatgtttgtatttttagtagagacagggtttcaccatgttggtcaggctgctct
cgaactccagatctcgtgatccgcccgcctgggcctactaaagtgctgggattacaggcg
tgagctagatctgactttctagtgtcctagccttggcccgatggacatgtcatttctctc
agctcgtttctgtcccctaaagtgagaatattgcctgggaagattacattagacgatgta
tatgcgaagacacttgatagctggtattgtcatgattctgattagttcactactgctact
ttccctgtggcctaggctttgcctatttccagtgggcgagctagctagatcctcctccct
taaataagccagtgtttttaagacagaatactacttgcatagtggacaataatatcttaa
agaactgagcaggatgaaaagaatttgatagaaagcaggtttgaggagcacattggaggt
tggcaggtttcgaggctgcttgagaggacttgggccgatctgggctgggcttggacgtga
ccctggcacccaggcaggtggatcccagctggggcttccattcacgactttctggtccct
ggcaggacagagcgggatgccaccagcttgtccaaaggaagttccaggagctcctggtg

ggcatcgttgtggagaatgacgctggccgcggcgagatcaacgagttgaagcaggtggaa
ggggaggcttcgtgcagcagccgcaaaggaaagctgattttcttctatgagtggaacatc
aaactgggctggaaaggcatcgttaaagaatctggagtgaagcacaaggattgattgaa
atacccaatctttctgaggaaaatgaagtagatgacactgagaatttacaacgggaatg
(Seq ID No: 1148)

Homo sapiens GrpE-like 2, mitochondrial (E. coli) (GRPEL2): ctgcctctcagcccaaattggaaacatg (Seq ID No: 1149)

Homo sapiens xyloside xylosyltransferase 1 (XXYLT1):

ccgcccctttcatggccgccgcctggcgccggggctaagtggccgccggcgtccgggtac
ccgagggctctcccgcgttgctggcaccgctggcgccgcggtctcgtagcgcatg
(Seq ID No: 1150)

Homo sapiens chromosome 7 open reading frame 60 (C7orf60):

cctcctctggctgctgcctccgcagctccctcctcctaccccacctcctccatctgggga
gcgtctgcgggggcctgaggggcggcggcggcggcggctgcgatatg
(Seq ID No: 1151)

Homo sapiens tetratricopeptide repeat domain 39B (TTC39B):

ccctcctttgcgctgggctgagcccagagccgagagcaggggtcggctctgagttccctg
cttggtttttgggtggcagcagccagaggaggaatatg (Seq ID No: 1152)

Homo sapiens motile sperm domain containing 2 (MOSPD2):

caccccttctctgtctacctctgggcgggactgccgggtgatgagatactcggtcggcgac
ggtagaacgggcgacggcgacaaccgcaatcacatccacgacggtgatcatg
(Seq ID No: 1153)

Homo sapiens major facilitator superfamily domain containing 6-like (MFSD6L):

ggcccctttcggtccaacggcaggacctgggggctgtggccgggggcggccgttgacctg
gtgaccgcggcgccgccccagaccgggggcgcagtcccactcgctccgagccccggtccc
ccaagcctccctcccgggtacctggggccgcgcccgccctgcgcccagctccgccctccg
tcggcccaggcctgacagagcccggcagccatg (Seq ID No: 1154)

Homo sapiens consortin, connexin sorting protein (CNST):

cttcctctctagccgccagtgctctatgctccgcggtcgcgggccgccagcctccagccg
gccagccgcgaggggtgcgcagagggaggcggggcggaaaggcgagaggtgtctcctcca
ccggagccaggggagacccgagcaagctccgtgacagcacgtcggccgccatgtcgccga
gtggggctggaaacagacccggcgcccagcggtagccctccttgcgcctccgattcccag
acatggaaggtctttaatgtaactttaaatggttcaccaaaggatgctctaatg
(Seq ID No: 1155)

Homo sapiens zinc finger protein 92 (ZNF92):

gggcctttgtctctcgctgcagccggcgctccacgtctagtcttcactgctctgcgtcct
gtgctgataaaggctcgccgctgtgaccctgttacctgcaagaacttggaggttcacagc
taagacgccaggaccccctggaagcctagaaatg (Seq ID No: 1156)

Homo sapiens DnaJ (Hsp40) homolog, subfamily C, member 18 (DNAJC18):

ccccccttctctttcagcctcgggcacgggggaggctcggcggacctgctgattgggaacc
gatatg (Seq ID No: 1157)

Homo sapiens polymerase (RNA) I polypeptide D, 16kDa (POLR1D):

cctcctccctccttccgtcctccgcgccttccgtcggtcggtccttgcttcctgcttcgc
ctccgcgcctcgcgctatgggacagagcccccgatccgccagcaccacctgaggatccag
aaaccgccccagcgatg (Seq ID No: 1158)

Homo sapiens ring finger protein 182 (RNF182):

acctccctcccctcccaggcgccgccgcagccggagcggctcccgggccctgggccgccg
ccggccaggaagaaatacttgtgttggctgcatttccagggatgctaccagagctcaagg
ctgtcacctggtcttgcccagaagagccgttcttagaggcaggacttgatgaaggctttc
ctgctgatggaataggtttgctagagctggccttggaattagaacccttcatgtggcctt
tataaatatgcgtttgagacagagttatatgcagaagttgaaaatgcctggaagatttct
ggtttctttcactacttatcctgccttttgcatcgctgccagatttggatgatatgata
ttcagaggggcaccttaatcaaagccattcttcaacaagacccacctggcataagattgc
acacataattcaagatg (Seq ID No: 1159)

Homo sapiens transmembrane protein 18 (TMEM18):

cctcctctgtggattctggccaggccgggttcggcggttgctgtgagagcgggcttccca
acaccatg (Seq ID No: 1160)

Homo sapiens Hermansky-Pudlak syndrome 4 (HPS4):

aggcctctctgccgcgcgcgcaggtacggggcagaagtcgcaggtacccagctgctgccc
acatttctggtccagagtcccgaaccccgagcactgggatgcctggctactccgagccaa
ggcactgatgtttgaactggaaacttcaaaacgtttaataagagtcttcaggatgggttt
gaactagacaagctagaaatttctttagaacaccagctctagcatgcatctcccactttt
ggctttcctggagaggagcttgaagaggtggttctgcagacagccacagtgatacttagg
aaaccagaggaatggatttgacttttctgctaggattctctgttatagtttctccctgag
ttgtaagaggcatggaaatatacatgaaactgaagaacctgcaaggaagggaagtggaac
tttccatgctgagtgaaaactaaccaagtggcagttgtgactgaaaacactgaaacctac
cacgtccagattcactggattggggatagaggaacggtcacagctagggagaaagaagt
gataccggaaaagaaaacctaaatgaagagaatgaggatgactgcacagtagatg
(Seq ID No: 1161)

Homo sapiens PTK7 protein tyrosine kinase 7 (PTK7): agctccttttcctgagcccgccgcgatg (Seq ID No: 1162)

Homo sapiens kelch repeat and BTB (POZ) domain containing 6 (KBTBD6):

agttctcctgggcgcctagcattgtcgcccacgctgcagtagcggcttctgcggctccaa
gccagcgggtcctgtgaaggcgagcagacgcggagaaaggacgcgggagtgagagagggt
gagtcagccactgtctaaacgataacggaggcggctctgcggggtagggttgaattcag
taaatgggctcgtgctgctgtctcttcggagacgctgctatcttagcgtcagcgagggaa
ggttgaggaggagccagagccgggtcctgcagcgtttctcgccatcagcgcccgtcgcca
tctccaccatg (Seq ID No: 1163)

Homo sapiens sperm antigen with calponin homology and coiled -coil domains 1 (SPECC1):

ctttctttgactggagcggacccgccggacgcaaccgcctcgccagccggagccagcgcg agctcggcacggtggacacccggtccgaggccggcaagccggctggtgcccgagtcggcc aagcatg (Seq ID No: 1164)

Homo sapiens ST6 (alpha-N-acetyl-neuraminyl-2,3-beta-galactosyl-1,3) -N-acetyl

galactosaminide alpha-2,6-sialyltransferase 3 (ST6GALNAC3): ggtccccttatttggatctgcgggaatgtggctggagaggtcctgccgtggtaccagcc tccagcctgcccccaggactgcccctgacccaggcgcgcccgctgctcggtggcaggagg gccggcggagcgccatg (Seq ID No: 1165)

Homo sapiens transportin 1 (TNPO1):

gattctctttgttccgcagccatttcaggccccggacaggaggcagtgccgcttcggccg aaggcccgagcgcccgaggcgtctgggatg (Seq ID No: 1166)

Homo sapiens heat shock 70kDa protein 8 (HSPA8): cttccttcgttattggagccaggcctacaccccagcaaccatg (Seq ID No: 1167)

Homo sapiens hyaluronoglucosaminidase 1 (HYAL1):

ggctccttcctccaggagtctctggtgcagctggggtggaatctggccaggccctgctta ggcccccatcctggggtcaggaaatttggaggataaggcccttcagccccaaggacatcc tggctgccatacctgctcctgacttctcagggctggcagtcatcgactgggaggcatggc gcccacgctgggccttcaactgggacaccaaggacatttaccggcagcgctcacgggcac tggtacaggcacagcaccctgattggccagctcctcaggtggaggcagtagcccaggacc agttccagggagctgcacgggcctggatg (Seq ID No: 1168)

Homo sapiens STE20-related kinase adaptor alpha (STRADA):

agtcctcccggtcgccccactgcgcatggcacgttgcgtactcccctcccagcaaccggt ctggcggcggcgcggcagtaaaactgaggaggcggagccaagacggtcggggctgcttgc taactccaggaacaggtttaagtttttgaaactgaagtaggcctacacagtaggaactca tg (Seq ID No: 1169)

Homo sapiens transmembrane protein 161B (TMEM161B):

ccctctctttcgctgtttgagagtctctcggctcaaggaccgggaggtaagaggtttggg actgccccggcaactccagggtgtctggtccacgacctatcctaggcgccatg (Seq ID No: 1170)

Homo sapiens Usher syndrome 1C (autosomal recessive, severe) (USH1C):

ggctctttccagctcctggcagccgggcacccgaaggaacgggtcgtgcaacgacgcagc tggacctggcccagccatg (Seq ID No: 1171)

Homo sapiens interleukin 12 receptor, beta 1 (IL12RB1):

cagtcttttctccttgctcagcttcaatgtgttccggagtggggacggggtggctgaacc
tcgcaggtggcagagaggctcccctggggctgtggggctctacgtggatccgatg
(Seq ID No: 1172)

Homo sapiens Meis homeobox 2 (MEIS2):

atcccttcctctcttttctgttcgccctcttctccctgctctttttccctttccaccccc

ctcctctgttctccctcacctcctgcgcccctccccttcccgggttctgacagtacga
tgagctgccccattacggcgggatg (Seq ID No: 1173)

Homo sapiens G elongation factor, mitochondrial 2 (GFM2):

ttttcttttcgtttagatacattgccttttgcctaggctggcgtcgagacttgaggccgt
tgcagactttggcgcggctcgcgcctcctgcttcaagagcccagcggtgagagctggcct
gcggcacgcggcctaatgccagacagtaacagtttggaggatcaagatg
(Seq ID No: 1174)

Homo sapiens lamin A/C (LMNA):

gagcctttgccccggcgtcggtgactcagtgttcgcgggagcgccgcacctacaccagcc
aacccagatcccgaggtccgacagcgcccggcccagatccccacgcctgccaggagcaag
ccgagagccagccggccggcgcactccgactccgagcagtctctgtccttcgacccgagc
cccgcgccctttccgggacccctgccccgcgggcagcgctgccaacctgccggccatg
(Seq ID No: 1175)

Homo sapiens calcium/calmodulin-dependent protein kinase II delta (CAMK2D):

cgctctttctctcgccgcgccgtcttgaagccgcgcgggctcgtgagcagcgcgaggccg
ccaaggtgcctcgcttcgccggagccgctgccgcccgccggagggaagccggcctcgggc
gcgcacgctcgtcggagccccggcgcgccccgcgcctgagcctgctgacagcggccgctg
ggctcaggctgtccgctctgggctccgcggcctcggccccgctgcactccacctccgccc
cctcggactccctcccctctgcttctactcctcctgctccagtgcggatcgtttcgcaac
tgcttgccactcgtcccgtgcctggctgtttttccatttcccggccccctcttcttgagt
actttacccccctgcatttggggacagggactggaaaaggggcgggtggagcgtccagtgg
agaagaaggaagcgaggcccgcaggaggaggaggatcggcggactgtggggaggagaccc
cacgccaccctttctggtcatctcccctcccgccccgcccctgcgcacactccctcgcgg
gcgagctactttcggaccaggaaagtaagagcggccctgggtgacagcgccgcgggggcca
gtcccggggttagccgcgcgtctgctcgcttctggtccgtcgcgctcccagccagggcac
agcccggaccgaggatg (Seq ID No: 1176)

Homo sapiens calcium/calmodulin-dependent protein kinase II gamma (CAMK2G):

ccgtctcctcctcttgctccctcggccgggcggcggtgactgtgcaccgacgtcggcgcg
ggctgcaccgccgcgtccgcccgcccgccagcatg (Seq ID No: 1177)

Homo sapiens interleukin 15 (IL15):

ttttcttttcgccaggggttgggactccgggtggcaggcgcccgggggaatcccagctga
ctcgctcactgccttcgaagtccggcgccccccgggagggaactgggtggccgcaccctc
ccggctgcggtggctgtcgccccccaccctgcagccaggactcgatggagaatccattcc
aatatatggccatgtggctctttggagcaatgttccatcatgttccatgctgctgacgtc
acatggagcacagaaatcaatgttagcagatagccagcccatacaagatcgttttcaact
agtggccccactgtgtccggaattgatgggttcttggtctcactgacttcaagaatgaag
ccgcggaccctcgcggtgagtgttacagctcttaaggtggcgcatctggagtttgttcct
tctgatgttcggatgtgttcggagtttcttccttctggtgggttcgtggtctcgctggct
caggagtgaagctacagaccttcgcggaggcattgtggatggatggctgctggaaacccc
ttgccatagccagctcttcttcaatacttaaggatttaccgtggctttgagtaatgagaa
tttcgaaaccacatttgagaagtatttccatccagtgctacttgtgtttacttctaaaca
gtcattttctaactgaagctggcattcatgtcttcattttgggatgcagctaatataccc

agttggcccaaagcacctaacctatagttatataatctgactctcagttcagttttactc
tactaatgccttcatg (Seq ID No: 1178)

Homo sapiens protein O-fucosyltransferase 1 (POFUT1): gtccctccttccctccccgactgtgcgccgcggctggctcgggttcccgggccgacatg (Seq ID No: 1179)

Homo sapiens calpain 3, (p94) (CAPN3):

cactctctttctctctccctctggcatgcatgctgctggtaggagacccccaagtcaaca
ttgcttcagaaatcctttagcactcatttctcaggagaacttatggcttcagaatcacag
ctcggttttaagatggacataacctgtacgaccttctgatgggctttcaactttgaact
ggatgtggacacttttctctcagatgacagaattactccaacttcccctttgcagttgct
tcctttccttgaaggtagctgtatcttatttctttaaaaagctttttcttccaaagcca
cttgccatg (Seq ID No: 1180)

Homo sapiens PTK2B protein tyrosine kinase 2 beta (PTK2B):

agcccttttactcagccacagcctccggagccgttgcacacctacctgcccggccgactt
acctgtacttgccgccgtcccggctcacctggcggtgcccgaggagtagtcgctggagtc
cgcgcctccctgggactgcaatgtgccgatcttagctgctgcctgagaggatg
(Seq ID No: 1181)

Homo sapiens ST6 beta-galactosamide alpha-2,6-sialyltranfera se 1 (ST6GAL1):

cttccttccttctccagtcccttccactgtgcgtcttctgtcccccgttcttccccagcg gacccctctttcgagactccctagtggggtccccagctcccgggcgatcctgcccttgcc gagcgcgttttctggagtcacctggggagggagtcctgggcagggccgggctggggaa gacgcctggggcactgcccggcgttaacaaaggagccgataccgaccggcgtggcgcg gagcggcggccgccaccgagcgtgctgagcaaccgcagcctccgcggccgagagtgcag cgagcaaggggagagccagttgcgcagagccctgcaaccagcagtccagggagaagtggt gaatgtcatggagcccagctgaaatggactggccccttgagcctgtcccaagccctggt gccaggtgtccatccccgtgctgagatgagttttgatcatcctgagaaaaatggccttg gcctgcagacccaataaaccttccctcccatggataatagtgctaattcctgaggacctg aagggcctgccgcccctggggattagccagaagcagatgatcatgacgcagtcctgagg tttaatgggcacccacagccaacttccaacaagatgtgggcacaaaaactaccattcgc ctgatg (Seq ID No: 1182)

Homo sapiens ubiquitin-conjugating enzyme E2Q family member 2 (UBE2Q2): ctccccttccgcgcccggctccccttccgcgcccctcccgccggagatgaggggaagatg (Seq ID No: 1183)

Homo sapiens membrane magnesium transporter 1 (MMGT1): gcttcttttgctgggctgctgctccttcggcatcatg (Seq ID No: 1184)

Homo sapiens PAP associated domain containing 4 (PAPD4):

cggtcttccgggtgtctttgacagggttttctacgccgctttttcggcgactttttgctc ttccgctttttgccaccgcccccaaccttctatatccttgcagcccctaccttttcttgt gttgctcctcccctggcagccgtgaggggggttagatctcagccggagccggagctgggc ctagctgtcccacgggccaccactacctcctttggttcgggagaaagctacgaccaagta cgcccagctcgggccttagaacttctgaacggcagtgcgggtaggccctgcttagccct

tcccggaggacacctgaccaaaagaggaagatagtcttgggacccttgcatggtgtttca aagggtggtgaagaactaaggtagaagaatacatgttcacttccagtgaacaagagcatg (Seq ID No: 1185)

Homo sapiens chromosome 3 open reading frame 23 (C3orf23):

ctcccttctggtgtactgggtgggaggtggaactagtcggacaaagccctcgcgtcggac ccttgccagaactcaattaatggatgcctcgaagttgacgtacatatatattcagaaatg (Seq ID No: 1186)

Homo sapiens mucosa associated lymphoid tissue lymphoma tran slocation gene 1 (MALT1):

cgcccctttgcgcggctggcgcggccagccggccaggctcccctcggcaaacctgtctaa ttggggcggggagcggagcttcctcctctgagggccgtgccgcgctgccagatttgttct tccgcccctgcctccgcggctcggaggcgagcggaaggtgccccggggccgaggcccgtg acggggcgggcgggagccccggcagtccggggtcgccggcgagggccatg (Seq ID No: 1187)

Homo sapiens UDP glycosyltransferase 3 family, polypeptide A 2 (UGT3A2):

ctacctctacccacagccagtgcctttggcgcactgaggtgcacagggtcccttagccgg gcgcagggcgcgcagcccaggctgagatccgcggcttccgtagaagtgagcatg (Seq ID No: 1188)

Homo sapiens sodium channel, voltage-gated, type IV, beta su bunit (SCN4B):

cctcctctcgctctctgcccgctaactttcccgagccccgaccggcggcgcagagctccg gggtagctttgtggccgaacgccgacctcgggcggagagcgcggctgtgcccagtatccc atccccgcgacccccgcgcgctccggagagaacaggactatg (Seq ID No: 1189)

Homo sapiens JAZF zinc finger 1 (JAZF1):

tcccctctgcctcccggtggctcctcgctctccttccatctctcgcccccctctccctc cgtcccgtcctcgccgctcccctcaccccgcctctctcccccctcccccagcccctcctct cctcaccccacccggcctccctccctccctcgcccgcccggcgctcgcagagccgacacc agggggggctctcgatgtagcaccatg (Seq ID No: 1190)

Homo sapiens chromosome 15 open reading frame 55 (C15orf55):

ttcccttccttggatccctgtgcacctactggagccaggttactctgggtcctggacctg actgcctcattctggaggcttccagacagccacagttagtgcccaaacctgagaggatg (Seq ID No: 1191)

Homo sapiens ras homolog family member C (RHOC): cgccctctcttcctgcagcctgggaacttcagccggctggagccccaccatg (Seq ID No: 1192)

Homo sapiens CTP synthase II (CTPS2):

cattctctttccttttccttctctcctgagcgctcctgcagttcctggggcgtagtaggg gatccacaagcgtttgtgaccagtgaagttctttacaagggtgagatctgcacggagga cccgagcgagggtctcggcttgccaggaagccggggttccccgggaagcgtggagttcac ccgcgcactcgaagtgcctttgcaaaattatatctgggtgttggcacccagccactattc tgccaatg (Seq ID No: 1193)

Homo sapiens PRP4 pre-mRNA processing factor 4 homolog B (yeast) (PRPF4B):

agctcttttccttcttcctccacttcccctaccctccaccgtccgggagccgccgccacc gccgccgaggagtcaggaagttcaagatg (Seq ID No: 1194)

Homo sapiens molybdenum cofactor synthesis 2 (MOCS2):

gcgcctttgcggccgtgattcggtcccgctgtcctaggcgggatggtgccgctgtgccag gtaagggtggcgggtgtgcgtgcgggcctgggtgcggagccctcctcgacgtgtctctcc cgccctttccctccacatacccagccttggtcagtcggacctccccactagcccccaacc tggccggcgtcttgggttcgggggcgcccccgcccccgcccccgggcccttcctgtctcc gggctttactgcgactgccccagcagaagtcgggtcctctccgagaactcttgtcagctc acggcagcaaggacggactcgttctgaaggcgcctccacctttatgaccacctctttcc cagattattcgttttgatgaagctaaaattttaatctaaaaagaaatgcacctcatggag aattcttgtgaagaactgtgcttcatctgtggatttctacacccttgatcatttgcaaac ctgtaattatttcgtaaagagttgtttgcacggagtgacaggttgaagtattgtattttg caaaaagtgctgaaataacaggagttcgttcagagaccatttctgtgcctcaagaaataa aagcgttgcagctgtggaaggagatagaaactcgacatcctggattggctgatgttagaa atcagataatatttgctgttcgtcaagaatatg (Seq ID No: 1195)

Homo sapiens cat eye syndrome chromosome region, candidate 1 (CECR1): tttccttttccggaggggagatg (Seq ID No:

1196)

Homo sapiens solute carrier family 13 (sodium-dependent citrate transporter), member 5 (SLC13A5): ctgcccctcactcgtctcgcccgccagtctccctcccgcgcgatg (Seq ID No: 1197)

Homo sapiens armadillo repeat containing, X-linked 3 (ARMCX3):

agtccttcttgtcctggtcgttgttcccgtctgagtaccagctccccactgccctgaggg
cgggccggcctgcggcggagggaaaaaggaagaggagaaggaaattgtcccgaatccctg
cagtgggtccaagcctctcccgggtggccagtctttctgtaggttgcggcacaacgccag
gcaaaagaagaggaaggaatttaatcctaatcggtggaggtcgatttgagggtctgctgt
agcaggtggctccgcttgaagcgagggaggaagtttcctccgatcagtagagattggaaa
gattgttgggagtggcacaccactagggaaaagaagaaggggcgaactgcttgtcttgag
gaggtcaacccccagaatcagctcttgtggccttgaagtggctgaagacgatcaccctcc
acaggcttgagcccagtcccacagccttcctcccccagcctgagtgactactctattcct
tggtccctgctattgtcggggacgattgcatg (Seq ID No: 1198)

Homo sapiens armadillo repeat containing, X-linked 2 (ARMCX2):

cgtcctcctctgggtaccaactctattgcgcagctcgctgccgtgcgtttaacccaggcg
aggaggaggaggagaaaattcccccagattcgggcaggcccgcaccccacattccgtcct
gttttgagaggaggagggaagagaaataaacgtggcagcgcatagaaggccagcagggag
actgctttccagacacctccggcccacacagccgttcaccccccgtcttttcagtcctgg
aaaaggaattcggtctgtccttaggatgaagctctaactgaactgaagtaaggagaaaca
gccttgaatctttggagggtctgtcttccttttgggctctgtgcaactgcagctacagtg
gaaaaaagcaaactgctcttgatcccaggccctgcctaagcctcagcagaacttgtaagc

ctaaactgaagagcctcacccggacgagcaggcatcccttaaccttaagcaatccagttc
cacgccctggatcagtgaataaccccagctgcaccatg (Seq ID No: 1199)

Homo sapiens UBA domain containing 2 (UBAC2):

cgccctctggggctccgagcccggcgggaccatgttcaccagcaccggctccagtgggct
ctgtgagtaccggcctccgccatcctggctgcccccctacacgccaccctaggcacctctt
tgaggaggctggggcagcggggaccctcgggtttgccggaggtggtggggccgaccctcc
agacccgcgtccgaaccctgctagttcccggtcttgggggtcagcggaaaccgcccccat
ttcggcctggagggggcgaatggggacaaagccccgccgcccgccccgaccccacctggta
tccccaggtgctctgcccaggagtctcttggggccgctgcaagtgggcaggtgccctggt
gttctcgtgggccggccccaggccctttgcggagcgtgtgccgcgctgaaggaaggggcc
gtccccccttaccatgccccattcttttaggcttgggggaccgaactaactccccccgccc
ccacttgcaaagttcagcctccgctttagaagctgacctctcagtttcacttggatg
(Seq ID No: 1200)

Homo sapiens cancer susceptibility candidate 4 (CASC4):

cctcctccctcggccggccctggggccgtgtccgccgggcaactccagccgaggcctggg
cttctgcctgcaggtgtctgcggcgaggcccctagggtacagcccgatttggccccatg
(Seq ID No: 1201)

Homo sapiens protein phosphatase, Mg2+/Mn2+ dependent, 1G (PPM1G):

cgctccctcacagctcccgtcccgttaccgcctcctggccggcctcgcgcctttcaccgg caccttgcgtcggtcgcgccgcggggcctgctcctgccgcgcgcacccccggggcttcgg ctccggcacgggtcgcgcccagctttcctgcacctgaggccgccggccagccgccgccat g (Seq ID No: 1202)

Homo sapiens StAR-related lipid transfer (START) domain containing 13 (STARD13):

ctttctttttaaaaatcgctgggtctgttgagctgtcctgggctgggtgccttgctcttt gactgagactggagacagacggcaacagccacaggcagactgaggtggcaataggaaatc tgccgagatg (Seq ID No: 1203)

Homo sapiens tubulin, beta class I (TUBB):

gattctcccgcctcccagccccggcgcacgcgcgccccgcccagcctgctttccctccgc gccctcccctctcctttctccctctcagaaccttcctgccgtcgcgtttgcacctcgctg ctccagcctctggggcgcattccaaccttccagcctgcgacctgcggagaaaaaaaatta cttattttcttgccccatacataccttgaggcgagcaaaaaaattaaattttaaccatg (Seq ID No: 1204)

Homo sapiens cytochrome P450, family 4, subfamily X, polypep tide 1 (CYP4X1):

tttccttcttcccgcgagtcagaagcttcgcgagggcccagagaggcggtggggtgggcg accctacgccagctccgggcgggagaaagcccaccctctcccgcgccccaggaaaccgcc ggcgttcggcgctgcgcagagccatg (Seq ID No: 1205)

Homo sapiens doublecortin (DCX):

ttttctttctctcagcatctccacccaaccagcagaaaaccggtgagtggggcttttaag tgattttcaagaagaatgtaacagatgtcaaacgggaaaagcacaaggcaaagcctgctc tctctgtctctctgtctcctcttctcctttttgccttattctatccgatttttttcccta

agcttctacctgggatttcctttggaaaagtctctgaggttccaccaaaatatg (Seq ID No: 1206)

Homo sapiens protein phosphatase 2, regulatory subunit B', g amma (PPP2R5C):

ttgtcttttttttttaaactaaaatggaggctggtttcttgccttaaggagcccattgc ctttcccgctgaagtctagatg (Seq ID No: 1207)

Homo sapiens solute carrier family 9, subfamily B (cation proton antiporter 2), member 2 (SLC9B2):

ccacctttccggggaagccacgcgcaccaggcatcgcacgcggctctgcacccgcgccg ccggacctgaaacccggcggagggcacacggggctgccgctgcgggccccggaccaaccc atgcttactccggagcctgtaccggcgccgacgggtcggacctccctgcgcggtgtcgcc cagcgggttcgtgcgaaaggcggggccgactacacgcggtgccgcgccctgagaccgttt atctgcagtcaacgcagcctcccggctcagcctgggaagatgcgcgaatcgggaacccca gagcgcggtggctagaccgggctccgccgcctcccccacagcccctttcctaatcgttca gacggagcctggtcgacttcgccggagactgccagatctcgttcctcttccctgtgtcat cttcttaattataaataatg (Seq ID No: 1208)

Homo sapiens hypoxia inducible factor 1, alpha subunit (basic helix-loop-helix transcription factor) (HIF1A):

caccctcttcgtcgcttcggccagtgtgtcgggctgggccctgacaagccacctgaggag aggctcggagccgggcccggaccccggcgattgccgcccgcttctctctagtctcacgag gggtttcccgcctcgcacccccacctctggacttgcctttccttctcttctccgcgtgtg gagggagccagcgcttaggccggagcgagcctgggggccgcccgccgtgaagacatcgcg gggaccgattcaccatg (Seq ID No: 1209)

Homo sapiens interleukin 21 receptor (IL21R):

cctcctcttcctccccactctgcacatgcggctgggtggcagccagcggcctcagacaga cccactggcgtctctctgctgagtgaccgtaagctcggcgtctggccctctgcctgcctc tccctgagtgtggctgacagccacgcagctgtgtctgtctgtctgcggcccgtgcatccc tgctgcggccgcctggtaccttccttgccgtctctttcctctgtctgctgctctgtggga cacctgcctggaggcccagctgcccgtcatcagagtgacaggtcttatgacagcctgatt ggtgactcgggctgggtgtggattctcaccccaggcctctgcctgctttctcagaccctc atctgtcacccccacgctgaacccagctgccaccccagaagcccatcagactgcccca gcacacggaatggatttctgagaaagaagccgaaacagaagatgaggcaatgaggctgcg agaggtagagtgattttccctcggtgactcaactgggacgtagcaggtcgggcagtcaag ccaggtgaccccatg (Seq ID No: 1210)

Homo sapiens DDB1 and CUL4 associated factor 4 (DCAF4):

tggtctttccgggtccttgcacgcttcgctccaactcctgcagagctgagccggagggga atccggaagggacacgctgaacaggtctgactcccgggcagcacagcccgctcacgattc cggccacggtgatgacgagtctccgtcaacctcgtctggcacagctgggacctcctctgt gccagagctacctgggtttactttgaccctgaaaagaaacgctacttccgcttgctccc tggacataacaactgcaacccccctgacgaaagagagcatccggcagaaggagatg (Seq ID No: 1211)

Homo sapiens oxidation resistance 1 (OXR1):

ccgcctcttgtgaggcgcgcggagccgcctcccctgggtcaggtctgatgggccggtggg

cgcgctagtggtggccgccaccgccgaaaccgtcgacctcctgggccccagttccgcgtc cagccccgcggcagcatg (Seq ID No: 1212)

Homo sapiens cut-like homeobox 1 (CUX1): ccccctctctatcagccgctcactccgtctcaatatgtctcaagatg (Seq ID No: 1213)

Homo sapiens atlastin GTPase 1 (ATL1):

ctccctttcctccccactccttcccaccagcgccacagcaacatcctcagagtctgagc gaactgcgcccagcgcgggcacggagcctcccaccgccagcaacctgcggccccggagaa ggcagcgagcgcagtgacagcgcctcaccgccaccagctcctggaccaccatg (Seq ID No: 1214)

Homo sapiens chemokine-like factor superfamily 5 (CKLFSF5):

ctgccttctctcccggggccctgtgggcaagcctcctgcttcactttcaggtttctcgaa gtgccttcttgctcctgtctgtttccccatcctgccagatttctgtttctcttgctgggc ttttggcagtaggggggctgtgttggtgggccctacgaagatg (Seq ID No: 1215)

Homo sapiens transmembrane emp24 protein transport domain co ntaining 7 (TMED7):

aggccttttccgcttctcttttacctccccaggtccgcccgtctgcgcccctcacaggaa gccggagggtcgctctgatcccgaatctcccacaggcgtgaacctgctctgctgtgtatc tttgcggggtggcctgcgctgaggcctgccgcgcgcggtgagtccgcgcagacctgaccc tgcgtctcgcagctcggttgaggccgccgccgccttctcgggatg (Seq ID No: 1216)

Homo sapiens ubiquitin-conjugating enzyme E2D 3 (UBE2D3):

cttcctttaccttcctcccatggtctccttccggttctcgatgcttctctgagcctaagg gtttccgccactcgttcaccctcccccagctcatgatcctcctccctcccccgccctcc tggtccaatctccgatctgtttagtaagaaggtgctgttccgagaagaaggaaaagggct tgacacgtattcactcggccccggacgtgggaagcaagccgtctggcttcggcctcacat cggtcttgtgctcgggacggcggcgttggcggactgatccgcggcggtgaagagaggccg ggaagttaaacttgtagccaccacctccgctcttcccgtcaccctcgcccccacttcggg ccgaaagcacggtacagaggctgttggtggctttgccacgccaccccacccaccccggat cgcggctgtcttaagggacctggattcatcaggggctcttcggggcctgtgcgagtgctg atctgctccgttttgcaaaaggcgcctgtgtctggcagagctggtgtgagacgagacaa tcctgccccgccgccgggataatcaagagttttggccggacctttgagcatacaccgaga gagtgaggagccagacgacaagcacacactatg (Seq ID No: 1217)

Homo sapiens zinc finger protein 595 (ZNF595):

tttcctctggctcctgcgagggcttggtttagggcttcagctctctgcgttctcggctcc gggaggcctcggtgattcagccacagcctctgcctcccgttgctctgtgacctgagggta ttggacaatttgtagctaagactcccggataccctgaagtcgggaaatg (Seq ID No: 1218)

Homo sapiens acyl-CoA synthetase medium-chain family member 2B (ACSM2B):

tgctctcttccaaggctgtaggagttctggagctgctggctggagaggagggtggacgaa gctctctccagaaagacatcctgagaggacttggcagcctgcagatggcctattgtggga ccttgtgatcatgcctgaacatg (Seq ID No: 1219)

Homo sapiens SRSF protein kinase 2 (SRPK2):

tttccctttatagcaccattgaatcccagtcctaacagaagtactgcgaatcttgtggcc
tcattctgaacaaaagggattagagaagaaaaatctcttgatataaggcttgaaagcaag
ggcaggcaatcttggttgtgaatatttctgattttccagaaatcaagcagaagattga
gctgctgatg (Seq ID No: 1220)

Homo sapiens synaptophysin-like 1 (SYPL1):

tgcccttcctcgccaccgggctgctctggtctcgtcggtcccctcctccgccccgtcgtc
ctgactctctctccctcctttcctcagaggatg (Seq ID No: 1221)

Homo sapiens thioredoxin reductase 1 (TXNRD1):

aaccctttcacctcagttttcttcactccggcatttgcagcagagcgaaaggtggtcgag
tcctgaaggagggcctgatgtcttcatcattctcaaattcttgtaagctctgcgtcgggt
gaaaccagacaaagccgcgagcccagggatgggagcacgcggggacggcctgccggcgg
ggacgacagcattgcgcctgggtgcagcagtgtgcgtctcggggaagggaagatattta
aggcgtgtctgagcagacggggaggcttttccaaacccaggcagcttcgtggcgtgtgcg
gtttcgacccggtcacacaaagcttcagcatgtcatgtggcttatcaggagggcagactt
caaaagctactaaaaatg (Seq ID No: 1222)

Homo sapiens minichromosome maintenance complex component 7 (MCM7):

tgtccttccgcgcggcggccgcggagagagctgcggcccgggggggcgtgcctgggatcc
ggagcttcgctcgggcccgggaaaggcggcagtgggctgggatcgcggtgtctctgggtg
tgatggccaatggctggactggctcccgccctgggcggaggaatcccgagctgtgaagcg
gctggaatccgggcccatgtgcttctttgtttactaagagcggaagcgatggcgggagcg
ggggtggggtgcggtggcggggtgcggtggcggaggtcccggtgaaatcaggggctaagg
ggacccaaagaaggcgggggatcataggggtggaaagaaagctgagaaccttgagaccgg
agtgtgaggggccaacggggaagggcgctagaattttaaactaaagtagggaccggaatt
cccctggggagatgttggatggccctgtgcactgccacgggctctttattcttcgctggt
tagaaacagacttgtgaaaaagagttatgcccactttggggagacttcgaaaaggttaag
aagttcttacaagagttctaccaggatgatgaactcgggaagaagcagttcaagtatggg
aaccagttggttcggctggctcatcgggaacaggtggctctgtatgtggacctggacgac
gtagccgaggatgaccccgagttggtggactcaatttgtgagaatgccaggcgctacgcg
aagctctttgctgatgccgtacaagagctgctgcctcagtacaaggagagggaagtggta
aataaagatgtcctggacgtttacattgagcatcggctaatgatggagcagcggagtcgg
gaccctgggatggtccgaagcccccagaaccagtaccctgctgaactcatgcgcagattg
tgagtggtctctgtcgggaaagatgtagggattggttctccaggatcttgtttgtgactg
ttttctccccttagtgagctgtatttcaaggccctagcagcaacaagcctcgtgtgatc
cgggaagtgcgggctgactctgtggggaagttggtaactgtgcgtggaatcgtcactcgt
gtctctgaagtcaaacccaagatg (Seq ID No: 1223)

Homo sapiens pre-B-cell colony enhancing factor 1 (PBEF1):

tttccccctctcccccctcctccgccgaccgagcagtgacttaagcaacggagcgcggtga
agctcattttctccttcctcgcagccgcgccagggagctcgcggcgcgcggcccctgtc
ctccggcccgagatg (Seq ID No: 1224)

Homo sapiens cyclin B1 interacting protein 1, E3 ubiquitin p rotein ligase (CCNB1IP1):

ctttctttccctctccgttttggtgggctggttgaagatgaaatccactgaggagggaag

tccagcaccctgtgtgccagtccagaactggcccatctgtagacccccctgaaaatcatat
gggcttggatttggatattctcaacagaaagggttaaaggctgatggtacctaaagcctg
gtacttgaattttgatcaagataagctgccttaagttctcttcattacacaaatgatcct
agataattgatagatcctgtggttcaactggatttctagatagaagctggattcatgtga
tgccagaggagtaaaatttcaagagactgaaaccagatctgagtttcgctgttccagtct
ggacctctttggtgctgtaaatcctggatatactgtagatgagtactgcgtttttctttt
atggcctctcttcagcttctggagacctcactatcctattatg
(Seq ID No: 1225)

Homo sapiens STEAP family member 3, metalloreductase (STEAP3):

ccgccttcgccgcggaccttcagctgccgcggtcgctccgagcggcgggccgcagagatg
acatttattcattttatgcatcctgggttctactggtcgtcccacctcagttcctgtagc
aaagagacttgagtctgagccactaattatcacccgtgaggtttcctccccgagcaggaa
gcagcaggccagagctgcgctctctcagtgcactctccaaccaagcatcagtcaccactc
ccggtccagcccctgtggccaagagctggcgtgcaggctgcgggaggcagctggctgtgc
aagaccctggcagggccctcgcctcctgagaaaccgagagtcagaaccaaagccaggctg
tcctggttggagactgagccagaaagggtggctcacctcacggtgaggctgtcgagtgac
ctgagagcctcagaccctcacgtcagccggatg (Seq ID No: 1226)

Homo sapiens nicotinamide nucleotide transhydrogenase (NNT):

tgttcttccgggttggaggcgcagcgccgcggggcccaagcccgggtctgccagcgcgac
gtcctctcgcggccctcagggcacagcccaaggctgtcagcctcccggcccagtgatttg
ccttcaaggaaactggggagtcagaaaattgggaactcatatcaacatg
(Seq ID No: 1227)

Homo sapiens SHC
(Src homology 2 domain containing) transforming protein 1 (SHC1):

gtccctctccctccccaggacttctgtgactcctgggccacagaggtccaaccaggctaa
gggcctggggataccccctgcctggccccccttgcccaaactggcagggggggccaggctgg
gcagcagcccctctttcacctcaactatg (Seq ID No: 1228)

Homo sapiens bromodomain containing 8 (BRD8): cggcccttccagaccgtctctcctcagggttggagacttcggggccaagatg (Seq ID No: 1229)

Homo sapiens ring finger protein 13 (RNF13):

tcgcctctttagtaggtcgggtgagtgtagtgtgcagggaagagacgcgtcagcgccagg
gccaggcccgcccgggggcagcccggcagccgaatcttgggctactctgtcccaacagcc
ggagcagatcagaccgaccggccctgcccgctcggtcccgcgccctccagaccctacggt
ctccgtttctaggggcacatggttagcggcaggcgcccacagccaatccactttgccagc
ctgccccttcctctgccaagagcagcttcttcagccgcgctccagttccgcagacgcctg
ccccaccctgctcttcccttccagggaagacggatcacgcggccaagaacgagactcgca
aactgggcatttctccgagccgggctagagcaagtagcgagactccgcgtgagagtggga
aagagccttaacaggcaaccatgttgcccagtgggttttctgtgcctttgggtgcggacc
aatgaggcgcgtggggcgggacttccgcttcgcctaggtgttgtcgtccctgctagtact
ccgggctgtgggggtcggtgcggatattcagtcatgaaatcagggtagggacttctcccg
cagcgacgcggctggcaagactgtttgtgttgcgggggccggacttcaagagagaaagag

agagtgggcagacatcgaagccaaacagcagtatcccggaagcactcatgcaactttggt
ggcggccactcagttttctctgccagtgtctggtgattttacaacgagatg
(Seq ID No: 1230)

Homo sapiens aldolase A, fructose-bisphosphate (ALDOA):

ccgcctcctgcgccgccccttccgaggctaaatcggctgcgttcctctcggaacgcgccg
cagaaggggtcctggtgacgagtcccgcgttctctccttgaatccactcgccagcccgcc
gccctctgccgccgcaccctgcacacccgcccctctcctgtgccaggaacttgctactac
cagcaccatg (Seq ID No: 1231)

Homo sapiens LY6/PLAUR domain containing 6 (LYPD6):

cgctccttccctgagctcccgggctccggcagcgcgctggcggggcgccgcattgcacac
tctgggggcgccgcagtgttcgtgggatggggcagcgggctgcagctggcggccggaatc
cgcgcgcagcccgggtgcaagttctctcctgttgccctgagtgcccactcccaggccctc
tgtatgagtgacacttcagtctgccatg (Seq ID No: 1232)

Homo sapiens butyrophilin, subfamily 3, member A1 (BTN3A1):

cagtctctgctttcttttcctttcttccagaaggagatttaaccatagtagaaagaatg
gagaactattaactgcctttcttctgtgggctgtgattttcagaggggaatgctaagagg
tgattttcaatgttgggactcaaaggtgaagacactgaaggacagaatttttggcagagg
aaagatcttcttcggtcaccatacttgagttagctctagggaagtggaggtttccatttg
gaattctatagcttcttccaggtcatagtgtctgccccccaccttccagtatctcctgat
atgcagcatgaatg (Seq ID No: 1233)

Homo sapiens lipoic acid synthetase (LIAS):

ctgtcctttcccgggagttagcgatccctcaacccctgcactgcgctagtcctaaagagg
aaatg (Seq ID No: 1234)

Homo sapiens C-type lectin domain family 7, member A (CLEC7A):

gattctcttttgtccacagacagtcatctcaggagcagaaagaaaagagctcccaaatgc
tatatctattcaggggctctcaagaacaatg (Seq ID No: 1235)

Homo sapiens CD247 molecule (CD247):

actccttttctcctaaccgtcccggccaccgctgcctcagcctctgcctcccagcctctt tctgagggaaaggacaagatg (Seq ID No: 1236)

Homo sapiens myeloid zinc finger 1 (MZF1):

aagcctttctccattttgcggtctaggaagtagcagaggccccttcctgtagggagttgc catggagacgcggtggggcaccgacggagttctaatgacggccgtgattggtgcaggatc ctgctaatctcaggaaggcccgtagagaagtgaggaaaacgtggtgggggcatgcgcga tctggtaggcggtgctgccgtctgttgtacctgagaggcttgcgcatgccgacgcacgga ttcgaggcggggagcatgggaagaagcggccaggagtatgacctgatcattgcgaccacc gctaggggaagggaggagagggtgtagaaacggggacgagggtgggggaagggcaaggag gcgctcgagctggtgcgcggagcatcctgggagacgtagtccagcgggaggggggaagtcg aagactgcgcgtgctcaggagcgcggagcggcccgctgagcgcagaggggcagacactgg cctcagatacctgacctggtaccctctatg (Seq ID No: 1237)

Homo sapiens E2F transcription factor 6 (E2F6):

cctcctcttttccgtctgcgtcgggagctcccgggcacgtgaggccgtgccgcgtttac

tggcgggcgggacggcctagccgggcggcgcctcggaggaagccgcggacccccttaggtg ctgggcccttggaaatcggcgcgtggggggcggtgctcgagctgagcgcgagagggcggg agagctcgtggggtgcgaggggagcaggacgcccggccgggcagcatg (Seq ID No: 1238)

Homo sapiens purinergic receptor P2Y, G-protein coupled, 10 (P2RY10):

cttcctctttcaacaacaaatgtgtcagttatcagcaggatccatgccgccagagtaaag ctttctaccctttactccctgcaaagaaacaagagtgcttatcccagctaagctccaggg taatgttatcatgacagcttcaactttagaccacaggcaaatgctttgttaaaactcta tgctggtcattcccttcaggatttggcactcaccaacatacccttctttcaagtgaaaag gcatctcttttaatggtcctgacctttggaataggaagcatgtaccctggacagagcact tcaaactagaggaaccataaatccatg (Seq ID No: 1239)

Homo sapiens chromosome 9 open reading frame 85 (C9orf85): catccttttgcctgctcccggcgaggggtggctttgatttcggcgatg (Seq ID No: 1240)

Homo sapiens ERGIC and golgi 3 (ERGIC3): cgtccccttt ccggccggtccccatg (Seq ID No: 1241)

Homo sapiens ankyrin repeat domain 46 (ANKRD46):

ccctcccctccgcccgtcaccgcctccttgaagctgccgctgtcgctgctgctcgttcga gtcgcagatccttgccagcacattacagaatattttgttgaaccttcttgagaattcag agaaactgctgagtgaccactgaacgaaaagatctaatcttaaggcttacgcctcacttt gatgcccaggctggagtgctgtggctcaatcacagctcatcgcaacctcgacctcccggg ctcaagtgatcctctcacctcagcgtcccgaacaggcgtgttccatccaccacatcagaa caatg (Seq ID No: 1242)

Homo sapiens Ras and Rab interactor-like (RINL):

tcctctctccacttcctgctactgcaggcctctcctccgagaacagaggccaggtcatga ctcactggcttcctgcaacctgacgatggcccagccagaagacaaggcacctgaagtccc cacagaggggtgaggtgaacaaagcagacaggacccctctaggggtcctcagcacccta gagccacttactcgcctgcagaggacatgggggtgtggcatgtgccagagctggatacc caggatgcggaggcccttgtggggctgtggccactagggagtttcttggtcacaggacgt gaccccagccaggccctggtgttgaggtcaggacctttaccaggagaagtcaatacctac cagatccagaagattcccagaggtgtgtccctggaatcctccaacctctgcatg (Seq ID No: 1243)

Homo sapiens embigin (EMB):

ccgccttttcttcagcgtcctacccgcggcactggctgcgagcgccgggccacctgcgag tgtgcgcagggactctggacacccgcggcggcgagctgagggagcagtctccacgaggac ccaggcggaccctctggcgccatg (Seq ID No: 1244)

Homo sapiens MMS22-like, DNA repair protein (MMS22L):

ccgcctttccggagcgcgggcgcgcggtggcgggaatttcgcctgtttgcggtttagacc ccaaagattcctgttggtggtctgggtcacaggaggcaggtttcgggagctggaaatgtg agcgggtacgacaggcaccgcgggtaaccgacgccccgggtccttgctgcagccgggtac gcgggataccggcaccccgccttctccgcccgagtgctgccaggcgtgggcctggaatct

cttcacaccttctctttggagcccttaatgatacgacgaaccccaagtgtttcagaacat gaagtaaacaatg (Seq ID No: 1245)

Homo sapiens chromosome 19 open reading frame 54 (C19orf54):

actcctttccttttttccagtggttatcgcggcgcccaccggcctctgatctctgagtctt ctccaacccacagacgtttttgttgctctggttccaggaccttctccacaactaggcca ttttccctgccaggtgtcctttttgacctcttgacctctgactcaaagggcctgctcccc ctcatgtcttcggcctggagaagagccagctcctgaaggaggcctttgataaggccggcc cggtccccaagggcagagaagatgtgaagaggcttctgaaactacacaaggaccggttcc gaggtgacctgcggtggatcctcttctgtgcagacctgccgtccctcatccaagaaggcc ctcaatg (Seq ID No: 1246)

Homo sapiens zinc finger protein 621 (ZNF621):

cgcccttccggctcggcctttagttagtgaccagctcctcggcgttctgcagagcgtggg tttcagcgagttctacgtgccaggtccgcccggtgccggcttcctcgctgcccctggcgg ctcgtcagcccccactacccctgaacttggtcccaatggcggcccgcccctccttcaccc ggaccgtgggcatctgggcctcgccgaagccgtcaaggtggctgctcgggcttctagagc ccgtgtccagccctttgccaccgaggcctgatcctcttttctgccctaaagaacttgccc tgacagcctctggctcccgctcttgaggatcttgcttgtccaaacccagaagacagtgca tgaagccaggggacatccgccatg (Seq ID No: 1247)

Homo sapiens family with sequence similarity 73, member A (FAM73A): ccgccttctccatg (Seq ID No: 1248)

Homo sapiens RNA binding motif protein 43 (RBM43):

ccgcccttttcttcgtagcctccaagggagctggaacaaaaacgaaaccaaaacctgcc tgctcgctcctctccccatcgcctgcgttccgctggttgtgggctttctgcggccgctga gggcgcgtctcccctccgccatg (Seq ID No: 1249)

Homo sapiens spermatogenesis and centriole associated 1 (SPATC1):

caccctccttcagcccaggcaaggcctggggccctgggcagcctccaggtgcagtgccct cccgtgggccgcacccttgccactgccccagggcatg (Seq ID No: 1250)

Homo sapiens carbonic anhydrase XIII (CA13): ctttctcttccttccaccccgagggaccatg (Seq ID No: 1251)

Homo sapiens transglutaminase 2
(C polypeptide, protein-glutamine-gamma-glutamyltransferase) (TGM2):

cgctctccgcctcggcagtgccagccgccagtggtcgcacttggagggtctcgccgccag tggaaggagccaccgcccccgcccgaccatg (Seq ID No: 1252)

Homo sapiens NOP2/Sun domain family, member 4 (NSUN4):

atttcctttccctttttcgctcgtgtcccgccgggtggcgctcaccacctccccggaac acgcgagtctcctgtcgcggttccggtcggaattaccccgtggagcacgccgatatg (Seq ID No: 1253)

Homo sapiens mitochondrial ribosome recycling factor (MRRF):

gagtctttccttagtaacctgggcgatagctgtggatgtttccaaggattgtcttcagtc atg (Seq ID No: 1254)

Homo sapiens PHD finger protein 17 (PHF17):

cttcctccataacaagccaaacgccagaccgagagtgcctccgtgcgcgagtgcccggtg tgtgcgcgccggcgagagcaggggcccgcccggctccccgcccgccgcggcccgaactca tgcagctccgagcgagcgagcggcgcccagcccagcgcctcggccgaacccctccgcagc aggctgcctgctgtttcccggggagatcatg (Seq ID No: 1255)

Homo sapiens prolylcarboxypeptidase (angiotensinase C) (PRCP): cctccttttcgccctcccacccgcactgcagtctccagcctgagccatg (Seq ID No: 1256)

Homo sapiens proteolipid protein 1 (PLP1):

aagcccttttcattgcaggagaagaggacaaagatactcagagagaaaaagtaaaagacc gaagaaggaggctggagagaccaggatccttccagctgaacaaagtcagccacaaagcag actagccagccggctacaattggagtcagagtcccaaagacatg (Seq ID No: 1257)

Homo sapiens coiled-coil domain containing 80 (CCDC80):

cagccttctcactcctcactgagtccactctgaacgtgctaaaatgggaaggaggcggtg
ttttgctgatctgttaaattcttagtgaagtttccttgatttccagtggctgctgttgtt
tgagtttggtttggagcaaaactgaggtagtcctaacatttctgggactgaatccaggca
agagaaagaagaaaaagaagaagaaaaagaggaggaaaaaggtagggagaaataaaggga
ggagagaagcacagtgaaagaaaaaaaaagtcccttttcgacatcacattcctgtgtttt
ccctcagcctggaaaacatattaatcccagtgcttttacgcccggaaacaaagagactaa
gccagactatgggggaaagggagataagaaggatcctggaactttaaagaggaaagagt
gagattcagaaatcgccaggactggactttaagggacgtcctgtgtcagcacaagggact
ggcacacacagacacacgagaccgaggagaaactgcagacaaatggagatacaaagactt
agaaggacagctcctttcacctcatcctacttgtccagaaggtaaaaagacacagccaga
aagaaaaggcatcggctcagctctcagatcaggacaggctgtggatctgtggcggtactc
tgaaagctggagctgcagcacacccctttttgtattgctcaccctcggtaaagagagagag
ggctgggaggaaaagtagttcatctaggaaactgtcctgggaaccaaacttctgatttct
tttgcaaccctctgcattccatctctatgagccaccattggattacacaatg
(Seq ID No: 1258)

Homo sapiens chromosome 20 open reading frame 44 (C20orf44):

cgacctctttgcgcctgcgccccccttgccagtctttcgccggcaaaaggaggacgtaga
aaaggggacaccggaaactcactcttcacccggaaatggttattgaggaacatg
(Seq ID No: 1259)

Homo sapiens tryptophanyl tRNA synthetase 2, mitochondrial (WARS2): cgcccttctcaagatg (Seq ID No: 1260)

Homo sapiens myotubularin related protein 2 (MTMR2):

ctttccctgtgctgcccctgccgcgcgatggagaagagctcgagctgcgagagtcttggc
tcccagccggcggcggctcggccgcccagcgtggactccttgtccagttaatgtgttaag
agccattgacatttgaagatcatcagaagtgaagataaaacatctcaaaaattataattg
cctccacttctcattcagagaattcagtgcatacaaaatcagcttctgttgtatcatcag

attccatttcaacttctgccgacaacttttctcctgatttgaggagggagtctcgctcta
tcccctaggctggagtgcattggcgccatctcggctcatttgcaacctctgtctcccggg
ttcaagcgattctcctgcctcagcttcccgaggagctgggattacaggtcctgagggagt
ctaacaagttagcagaaatg (Seq ID No: 1261)

Homo sapiens reticulon 3 (RTN3):

cgccctctagctgcgctcggctgagtcagtcagtctgtcggagtctgtcctcggagcagg
cggagtaaagggacttgagcgagccagttgccggattattctatttcccctccctctctc
ccgccccgtatctcttttcacccttctcccaccctcgctcgcgtagccatg
(Seq ID No: 1262)

Homo sapiens G protein-coupled receptor 56 (GPR56):

gtccctccctctccgcactagctgtctgccctgccctgccgtaggagatgggctgggagc
ctcccacgctctccagctcactcggcaggcagcggggaccagggctggcaggttaagcct
ctgggggtggatcctgaaaggtggtccagccgcctggccctgcgtgggaccctccacctg
gcagcagacagggtctcgctctgtcacacaggctggagtgcagtggtgtgatcttggctc
atcgtaacctccacctcccgggttcaagtgattctcatgcctcagcctcccgagtagctg
ggattacaggtggtgacttccaagagtgactccgtcggaggaaaatg
(Seq ID No: 1263)

Homo sapiens immunoglobulin superfamily containing leucine-r ich repeat (ISLR):

gctcctccctgccgcctcctctcagtggatggttccaggcaccctgtctggggcagggag
ggcacaggcctgcacatcgaaggtggggtgggaccaggctgcccctcgccccagcatcca
agtcctcccttgggcgcccgtggccctgcagactctcagggctaaggtcctctgttgctt
tttggttccaccttagaagaggctccgcttgactaagagtagcttgaaggaggcaccatg
(Seq ID No: 1264)

Homo sapiens glycoprotein M6A (GPM6A):

atttcttttccccattttaaatgcaaagcaagacttgtgaatcatagtgtctctgctcct
gggattcagaccaaatttccccccaaaattctcaggctatttgtttgaatacctgcttac
agtggtacacaatgggcagctttgagaagaaaaattgataatcttcacggaagagtaatt
tgaatgaaattacacttgacagcctgtctccaagcaaacaagaggaacgagggagcctga
gctaagctctgaggacttgcccaagccactgctgttggagcttcccaggaaaaaaaaaaa
aaaaaaaaaaaaaaaaaaaaaaaaaaacaccagtttttccaacatctaattgagcttt
tgattaattccgtgtaccagattctactgaagaaaggtagccatg
(Seq ID No: 1265)

Homo sapiens splicing factor 1 (SF1):

ctccctctttgtgcgtctcgcgccgccgccgcccgccgcgtgagaggacgggctccgcgc
gctccggcagcgcattcgggtcccctcccccgggaggcttgcgaaggagaagccgccgc
agaggaaaagcaggtgccggtgcctgtccccgggggcgccatg
(Seq ID No: 1266)

Homo sapiens cell cycle associated protein 1 (CAPRIN1):

ccgcccctcgcgacccagagggctgctggctggctaagtccctcccgctcccggctctcg
cctcactaggagcggctctcggtgcagcgggacagggcgaagcggcctgcgcccacggag
cgcgcgacactgcccggaagggaccgccacccttgccccctcagctgcccactcgtgatt
tccagcggcctccgcgcgcgcacgatg (Seq ID No: 1267)

Homo sapiens hypothetical protein FLJ90297 (LOC388152):

ctgccctcttgcgtgccccggccacccccgggcggcttgtagccggtgcgcggggtggct
ggggctacgtgcagagctgtcgcggagccggaacagcagcggtgaagcccctcggctcgg
ccgagaccgccgtgcccattgctcgcctcggttgccgccgctttagccgcagccgctgct
gccgccgccgggggagaggcagcctattgtctttctccgcggcgaaggtgaggagctgtc
tcggctcggcccgcgggggagccccgggagccgcacggagatggaggaggacatctggac
agtgagcaggaggcgcctcggcccatg (Seq ID No: 1268)

Homo sapiens kelch-like ECH-associated protein 1 (KEAP1):

cgccctctccccgcctccttttcgggcgtcccgaggccgctccccaaccgacaaccaaga
ccccgcaggccacgcagccctggagccgaggcccccgacggcggaggcgcccgcgggtc
ccctacagccaaggtccctgagtgccagaggtggtggtgttgcttatcttctggaacccc
atg (Seq ID No: 1269)

Homo sapiens F-box protein 38 (FBXO38):

ctccctctcaaccacaataacaggcggagggtcggcgtaggtactttgaactcaagtaaa
caaaagggaagatttctcgttgatactggagactgcacaacaatg
(Seq ID No: 1270)

Homo sapiens musculoskeletal, embryonic nuclear protein 1 (MUSTN1):

agatcttttccagcagctgctgcctgccagagaggcgccttcagagacccagcgcttaca
caatacccaccatg (Seq ID No: 1271)

Homo sapiens QKI, KH domain containing, RNA binding (QKI):

cctcctctccggcggcggcggcggcggcgggcggagtgagctgcggagcctggaata
tg (Seq ID No: 1272)

Homo sapiens protein phosphatase 1, catalytic subunit, beta isoform (PPP1CB):

gggcctctcttgtttatttatttattttccgtgggtgcctccgagtgtgcgcgcgctctc
gctacccggcggggagggggtgggggagggcccgggaaaaggggagttggagccgggg
tcgaaacgccgcgtgacttgtaggtgagagaacgccgagccgtcgccgcagcctccgccg
ccgagaagcccttgttcccgctgctgggaaggagagtctgtgccgacaagatg
(Seq ID No: 1273)

Homo sapiens methyltransferase like 21B (METTL21B):

cagcctctaccccgctccggatccgggatctgagcgccggccgcggtgcccaggcactcc
cttggcgggccggatg (Seq ID No: 1274)

Homo sapiens adaptor-related protein complex 3, mu 1 subunit (AP3M1):

cggccttctcggcttctccagcttcggtaggagaggatccggcgccgaatcactgactgg cacaggtgttgggatagtgtctcacttggtcacccaggctggagtgcagtggcgcaatct tagctctctacagcgtcgatcttcctcctgggctcaagcaattctcctgcttcatcctcc tgagtacctaggactacagaaaatg (Seq ID No: 1275)

Homo sapiens muscleblind-like splicing regulator 1 (MBNL1):

cagtcttttcactgcagctgaatgagttgtggcgcccacaatgctcccatgacaaggagc tgacaagttccatttccgtcgcgggcatcttggaatcatgactcccacaatgccttggg

cacttggtcgacagtggggccgcctctgaaaaaaaaatgtgagaggttggtactaagaag tgcctttcctgacgtctctgctgcttggaaccgcttctagagcagtctctgcttttgcct tgcttgctgccagctagactgtgacgacagcacatccaccctccacctctagcccagaca cccccatttctacttataatcaagagaaaagctctaagtatctggcattgccctaggctg ctttagtgttaaaagaaaagtttgctgaaaaagtaagatatcttctgccaggaaatcaag gaggaaaaaaaaaatcatttctcgattttgctctaaactgctgcatctgtctatgccaa actaatcaataccgattgcaccaccaaactccattgcaaattcagctgtgaggagattcc ctttcagacaactttgctgaaagcagcttggaaattcggtgtcgaagggtctgccacgtt ttcatgcttgcatttgggctccaaattggcactgggaaggggttactgagagcacaagg ctgataccaggccctacttttaaacgttcatctacttacaatcctagtatttctctaaaa accaaaacctctttgaattaacagtttcatgctgtgaatttctagtgggagatcttttcc ttgatattgacgacacaattttccatgtacttttaaagcagggagtggggaaaagtattt tgaggggacattttcatcatcagttcagcttttttttttggttgttgctctttttttggg ggggttgggtttgttggtttcactgaaacatttaactacctgtaaaatctaaacatg (Seq ID No: 1276)

Homo sapiens lipid phosphate phosphatase-related protein typ e 1 (LPPR1):

cagccttttgctctttcctttcattaaacaaacaggagatcctgaaacctggaccctgtg caagctgcagcgccaggaggaggcagcggaggaagcagagcgcgggatgggcgcccagcg gcatctgtgatcccgcgcacctccgccccacgggcgcgcgcacaaacacggacacacaca tacacacactcgcgcacacactcgcacaaacacacactcgtacacgcccgcgccgctcgc tcgccggcttgctctcccacgcaagcggaatgcagcagcgcctggagagcgtgtctcgga ccgccgcctgaatgtacctcgctcccgggagccggacggcccagtagggcgcactggagg acgctccgctgcgggagcctggacagtttttgacggtgcagtcttgctatatggtgtgag aaatg (Seq ID No: 1277)

Homo sapiens muscleblind-like splicing regulator 2 (MBNL2):

ctgtctttgcttcatcatctgaaggtaaaattttccagatacggcagacggctttcagag tacaataaacagggaatgagaactatttacatggaagtttctttctcatgatgcggtgga gaagcctcggccacttggttctgccagatgttcctggggttactgtaaatggaaggaca ggcagagctaaacaaggtttatcatttaaaagtgcctgtgtgaagtcacttttgctggaa aactgcagcttgggagctttctttgtattcacatcccactcttctgtcaagtacacttta ccctgaccttatgagtggatgaagatacctcagttgtctgactttgccaattgcttaatt tcagaatttaaaaaggggaaagaaaaacatcctgctaaaatatgaacatctgagtgtctt attttccaacatcgtcaatagctgtgagcgtcagcattaaatattctcccaaggagtgcc atgatattgaagtcactttattaataacagctgtatctgcaaaacagtcaagagactcgg acgttgaaagccagagatgacactgagcatgctttattgcggcctaccatctttaagtg ggacatattgattgatgagtgattgcctgtccatacactctctcatcatcctgttccttg gattggacttcactaagcaatttatcactcaccttcagacttacatgtgggagttttcac aacagtagttttggaatcattagaacttggattgatttcatcatttaacagaaacaaaca gcccaaattactttatcaccatg (Seq ID No: 1278)

Homo sapiens chromosome 3 open reading frame 25 (C3orf25):

gcgcctttcgcacgacttggagttacggtttatctgataccgcggtacccctacgcaagc aagcccacatcgacacacattcacacacgcccttcagcaccccctcccagcaccacgacc atg (Seq ID No: 1279)

Homo sapiens testis expressed 19 (TEX19):

cctcctcctttccctgggtgcccacatgaacagagacaccaggatgctctcctgagacca cagcaactgcagaagctgaagacatttccagaagttcaagcttccaccctctgcaggtcc ccactgagctgggacccaggtcatccaccccaccccaaatccctggataggaaacccctt tctcctcctgctccttgtccccttcatccctgccgcccagcatcctactggcctcagcac ctgtggccagaccgtccaagatcctctgaaggcccagctcttgctgtccaccccggcagt aggcaggcagcctggccatg (Seq ID No: 1280)

Homo sapiens protein kinase C, beta (PRKCB):

gcctccctcccccgcagctggggccagcggtgccaagcgcagctggacgagcggcagcag ctgggcgagtgacagccccggctccgcgcgccgcggccgccagagccggcgcaggggaag cgcccgcggccccgggtgcagcagcggccgccgcctcccgcgcctccccggcccgcagcc cgcggtcccgcggccccggggccggcacctctcgggctccggctccccgcgcgcaagatg (Seq ID No: 1281)

Homo sapiens protein kinase N1 (PKN1): ccctccctccgcgcggggacccctggcgggcggcaggaggacatg (Seq ID No: 1282)

Homo sapiens hemochromatosis type 2 (juvenile) (HFE2):

ccttctctggttccctgacctcagtgagacagcagccggcctggggacctggggagaca cggaggacccccctggctggagctgacccacagagtagggaatcatggctggagaattgga tagcagagtaatgtttgacctctggaaacatcacttacagggcttccggtcaaaattcac taggtaggagggtcatcagctgggaagaaccggcgcctgggaaacctggctggataggta tg (Seq ID No: 1283)

Homo sapiens ribosomal protein L9 (RPL9): cgttctttctttgctgcgtctactgcgagaatg (Seq ID No: 1284)

Homo sapiens ribosomal protein L3 (RPL3): cggcctctaccggcgggatttgatggcgtgatg (Seq ID No: 1285)

Homo sapiens ribosomal protein L4 (RPL4): acttccttttcctgtggcagcagccgggctgagaggagcgtggctgtctcctctctccgc catg (Seq ID No: 1286)

Homo sapiens ribosomal protein L5 (RPL5): tggcccttttcccacccccctagcgccgctgggcctgcaggtctctgtcgagcagcggacg ccg-gtctctgttccgcaggatg (Seq ID No: 1287)

Homo sapiens ribosomal protein L6 (RPL6): aattctctttcccatcttgcaagatg (Seq ID No: 1288)

Homo sapiens ribosomal protein L7 (RPL7): cttcctctttttccggctggaaccatg (Seq ID No: 1289)

Homo sapiens ribosomal protein L7a (RPL7A): ctttcctttctctctcctcccgccgcccaagatg (Seq ID No: 1290)

Homo sapiens ribosomal protein L11 (RPL11): ctttctcttcctgctctccatcatg (Seq ID No: 1291)

Homo sapiens ribosomal protein L12 (RPL12):

cggcctctcggctttcggctcggaggaggccaaggtgcaacttccttcggtcgtcccgaa tccgggttcatccgacaccagccgcctccaccatg (Seq ID No: 1292)

Homo sapiens ribosomal protein L13 (RPL13): gcttcctttccgctcggctgttttcctgcgcaggagccgcagggccgtaggcagccatg (Seq ID No: 1293)

Homo sapiens ribosomal protein L23 (RPL23): acttcctttttcttttttccggcgttcaagatg (Seq ID No: 1294)

Homo sapiens ribosomal protein L18 (RPL18): cgttctctctttccggacctggccgagcaggaggcgccatcatg (Seq ID No: 1295)

Homo sapiens ribosomal protein L18a (RPL18A): acttccttttgcgggtggcggcgaacgcggagagcacgccatg (Seq ID No: 1296)

Homo sapiens ribosomal protein L19 (RPL19): agctctttcctttcgctgctgcggccgcagccatg (Seq ID No: 1297)

Homo sapiens ribosomal protein L21 (RPL21): gcctctttcctttcggccggaaccgccatcttccagtaattcgccaaaatg (Seq ID No: 1298)

Homo sapiens ribosomal protein L22 (RPL22): acctccctttctaactccgctgccgccatg (Seq ID No: 1299)

Homo sapiens ribosomal protein L23a (RPL23A): agacccttttcacaagatg (Seq ID No: 1300)

Homo sapiens ribosomal protein L17 (RPL17):

cgctcttcctctttccctaagcagcctgagggttgactggattggtgaggcccgtgtggc tacttctgtggaagcagtgctgtagttactggaagataaaagggaaagcaagcccttggt gggggaaagtatggctgcgatgatggcatttcttaggacacctttggattaataatgaaa acaactactctctgagcagctgttcgaatcatctgatatttatactgaatgagttactgt aagtacgtattgacagaattacactgtactttcctctaggtgatctgtgaaaatg (Seq ID No: 1301)

Homo sapiens ribosomal protein L24 (RPL24): ttctctctttcttttcgccatcttttgtctttccgtggagctgtcgccatg (Seq ID No: 1302)

Homo sapiens ribosomal protein L26 (RPL26): agttctcttcccttttgcggccatcaccgaagcgggagcggccaaaatg (Seq ID No: 1303)

Homo sapiens ribosomal protein L27 (RPL27): ctttccttttgctggtagggccgggtggttgctgccgaaatg (Seq ID No: 1304)

Homo sapiens ribosomal protein L30 (RPL30):

aagtctttcctttctcgttccccggccatcttagcggctgctgttggttgggggccgtcc cgctcctaaggcaggaagatg (Seq ID No: 1305)

Homo sapiens ribosomal protein L27a (RPL27A): ccttcctttttcgtctgggctgccaacatg (Seq ID No: 1306)

Homo sapiens ribosomal protein L28 (RPL28): cttcctctttccgtctcaggtcgccgctgcgaagggagccgccgccatg (Seq ID No: 1307)

Homo sapiens ribosomal protein L29 (RPL29): cagccccttctcttccggttctaggcgcttcgggagccgcggcttatggtgcagacatg (Seq ID No: 1308)

Homo sapiens ribosomal protein L31 (RPL31): cgctcttcctttccaacttggacgctgcagaatg (Seq ID No: 1309)

Homo sapiens ribosomal protein L32 (RPL32):

ccgtcccttctctcttcctcggcgctgcctacggaggtggcagccatctccttctcggca tcatg (Seq ID No: 1310)

Homo sapiens ribosomal protein L35a (RPL35A):

cgtccttctcttaccgccatcttggctcctgtggaggcctgctgggaacgggacttctaa aaggaactatg (Seq ID No: 1311)

Homo sapiens ribosomal protein L37 (RPL37): ccttctcttccggtctttctggtctcggccgcagaagcgagatg (Seq ID No: 1312)

Homo sapiens ribosomal protein L37a (RPL37A): gcgtctcttcctttctgggctcggacctaggtcgcggcgacatg (Seq ID No: 1313)

Homo sapiens ribosomal protein L38 (RPL38):

cgttctttttcgtccttttccccggttgctgcttgctgtgagtgtctctagggtgatacg tgggtgagaaaggtcctggtccgcgccagagcccagcgcgcctcgtcgccatg (Seq ID No: 1314)

Homo sapiens ribosomal protein L39 (RPL39): ccctcctcttcctttctccgccatcgtggtgtgttcttgactccgctgctcgccatg (Seq ID No: 1315)

Homo sapiens ribosomal protein, large, P0 (RPLP0):

aggcccttctctcgccaggcgtcctcgtggaagtgacatcgtctttaaaccctgcgtggc aatccctgacgcaccgccgtgatg (Seq ID No: 1316)

Homo sapiens ribosomal protein, large, P1 (RPLP1):

cggtccttccgaggaagctaaggctgcgttggggtgaggccctcacttcatccggcgact agcaccgcgtccggcagcgccagccctacactcgcccgcgccatg (Seq ID No: 1317)

Homo sapiens ribosomal protein, large, P2 (RPLP2):

ccttccttttcctccctgtcgccaccgaggtcgcacgcgtgagacttctccgccgcctcc gccgcagacgccgccgcgatg (Seq ID No: 1318)

Homo sapiens ribosomal protein S3 (RPS3): acttcctttcctttcagcggagcgcggcggcaagatg (Seq ID No: 1319)

Homo sapiens ribosomal protein S3A (RPS3A): ccgcccttttggctctctgaccagcaccatg (Seq ID No: 1320)

Homo sapiens ribosomal protein S4, X-linked (RPS4X): ggtcctctttccttgcctaacgcagccatg (Seq ID No: 1321)

Homo sapiens ribosomal protein S4, Y-linked 1 (RPS4Y1): gattctcttccgtcgcagagtttcgccatg (Seq ID No: 1322)

Homo sapiens ribosomal protein S5 (RPS5):

ttttcttcccagttaaaagtgttggcccgcggcgcgcggcctcttcctgtctgtaccagg gcggcgcgtggtctacgccgagtgacagagacgctcaggctgtgttctcaggatg (Seq ID No: 1323)

Homo sapiens ribosomal protein S6 (RPS6): ggccctcttttccgtggcgcctcggaggcgttcagctgcttcaagatg (Seq ID No: 1324)

Homo sapiens ribosomal protein S7 (RPS7): gggtctcttcctaagccggcgctcggcaagttctcccaggagaaagccatg (Seq ID No: 1325)

Homo sapiens ribosomal protein S8 (RPS8): gtttctctttccagccagcgccgagcgatg (Seq ID No: 1326)

Homo sapiens ribosomal protein S9 (RPS9):

gcgcctctttctcagtgaccgggtggtttgcttaggcgcagacggggaagcggagccaac atg (Seq ID No: 1327)

Homo sapiens ribosomal protein S10 (RPS10): gctccttcctttccagccccggtaccggaccctgcagccgcagagatg (Seq ID No: 1328)

Homo sapiens ribosomal protein S11 (RPS11): ctgcccctttctttttttcaggcggccgggaagatg (Seq ID No: 1329)

Homo sapiens ribosomal protein S12 (RPS12):

aggcctctttccctgccgccgccgagtcgcgcggaggcggaggcttgggtgcgttcaaga ttcaacttcacccgtaacccaccgccatg (Seq ID No: 1330)

Homo sapiens ribosomal protein S13 (RPS13): cgctctcctttcgttgcctgatcgccgccatcatg (Seq ID No: 1331)

Homo sapiens ribosomal protein S15 (RPS15): cgatctcttctgaggatccggcaagatg (Seq ID No: 1332)

Homo sapiens ribosomal protein S15a (RPS15A):

cgtcctctttccgccatctttccgcgccggtgagtagcactctctgagagctccaatttc atccgtctgccatcggcgccatcctgcaatctaagccacaatg (Seq ID No: 1333)

Homo sapiens ribosomal protein S16 (RPS16):

ctttcctttccggttgcggcgccgcgcggtgaggttgtctagtccacgctcggagccatg (Seq ID No: 1334)

Homo sapiens ribosomal protein S19 (RPS19): cgttccctttcccctggctggcagcgcggaggccgcacgatg (Seq ID No: 1335)

Homo sapiens ribosomal protein S20 (RPS20):

ccacccctttctttttgaggaagacgcggtcgtaagggctgaggattttggtccgcacgctcctgctcctgactcaccgctgttcgctctcgccgaggaacaagtcggtcaggaagcccgcgcgcaacagccatg (Seq ID No: 1336)

Homo sapiens ribosomal protein S21 (RPS21): gcttcctttctctctcgcgcgcggtgtggtggcagcaggcgcagcccagcctcgaaatg (Seq ID No: 1337)

Homo sapiens ribosomal protein S23 (RPS23): gcttctctctttcgctcaggcccgtggcgccgacaggatg (Seq ID No: 1338)

Homo sapiens ribosomal protein S25 (RPS25):

gcttccttttgtccgacatcttgacgaggctgcggtgtctgctgctattctccgagcttcgcaatg (Seq ID No: 1339)

Homo sapiens ribosomal protein S26 (RPS26): ccgtctcctctctccggtccgtgcctccaagatg (Seq ID No: 1340)

Homo sapiens ribosomal protein S27 (RPS27): cgctcctttccggcggtgacgacctacgcacacgagaacatg (Seq ID No: 1341)

Homo sapiens ribosomal protein S28 (RPS28): actcctctccgccagaccgccgccgcgccgccatcatg (Seq ID No: 1342)

Homo sapiens ribosomal protein S29 (RPS29): gcttcttccttttacctcgttgcactgctgagagcaagatg (Seq ID No: 1343)

Homo sapiens ribosomal protein L15 (RPL15): agctctttcctttccgtctggcggcagccatcaggtaagccaagatg (Seq ID No: 1344)

Homo sapiens ribosomal protein S2 (RPS2): cgttcttcttttccgacaaaacaccaaatg (Seq ID No: 1345)

Homo sapiens ribosomal protein L14 (RPL14): gggtcttcttccttctcgcctaacgccgccaacatg (Seq ID No: 1346)

Homo sapiens ribosomal protein S14 (RPS14): ctctctttccggtgtggagtctggagacgacgtgcagaaatg (Seq ID No: 1347)

Homo sapiens ribosomal protein L10 (RPL10): gcgcctctttcccttcggtgtgccactgaagatcctggtgtcgccatg (Seq ID No: 1348)

Homo sapiens ribosomal protein L10a (RPL10A): tagtctcttttccggttagcgcggcgtgagaagccatg (Seq ID No: 1349)

Homo sapiens ribosomal protein L35 (RPL35): tcctctttccctcggagcgggcggcggcgttggcggcttgtgcagcaatg (Seq ID No: 1350)

Homo sapiens ribosomal protein L13a (RPL13A): cctcctccttttccaagcggctgccgaagatg (Seq ID No: 1351)

Homo sapiens ribosomal protein L36 (RPL36): cagcccttccgccacggccgtctctggagagcagcagccatg (Seq ID No: 1352)

Homo sapiens ribosomal protein L36a (RPL36A): gtttctttctttccgcgccgatagcgctcacgcaagcatg (Seq ID No: 1353)

Homo sapiens ribosomal protein L41 (RPL41): tcgcc tttctctcggccttagcgccatttttttggaaacctctgcgccatg (Seq ID No: 1354)

Homo sapiens ribosomal protein S18 (RPS18): cgctctctcttccacaggaggcctacacgccgccgcttgtgctgcagccatg (Seq ID No: 1355)

Homo sapiens ribosomal protein S24 (RPS24): ggttctcttttcctccttggctgtctgaagatagatcgccatcatg (Seq ID No: 1356)

Homo sapiens ribosomal protein L8 (RPL8): tttcctctttcggccgcgctggtgaacaggtaggtcatccttgcggccttgcggcatg (Seq ID No: 1357)

Homo sapiens ribosomal protein L34 (RPL34): cttcctcttccggggacgttgtctgcaggtatg (Seq ID No: 1358)

Homo sapiens ribosomal protein S17 (RPS17): gtttcctcttttaccaaggacccgccaacatg (Seq ID No: 1359)

Homo sapiens ribosomal protein SA (RPSA):

ctgtcttttccgtgctacctgcagaggggtccatacggcgttgttctggattcccgtcgt
aacttaaagggaaattttcacaatg (Seq ID No: 1360)

Homo sapiens eukaryotic translation initiation factor 3, sub unit C (EIF3C):

cttctctctcggcgtttccgctgtcagggccctgcggtgtgactcgcgggctcagctggt
ccggccgtagcacctccgcgccgtcgccatg (Seq ID No: 1361)

Homo sapiens poly(A) binding protein, cytoplasmic 1 (PABPC1):

cgctctcctcctctcacggaaaggtcgcggcctgtggccctgcgggcagccgtgccgaga
tg (Seq ID No: 1362)

Homo sapiens tubulin, beta 1 class VI (TUBB1):

cactcccttccaaaagcatgacaggcagaaagcagagaagggccaggactggctgagggc
ggggagctgggcctctggggtggacacacccttggtcacattgtgaggtagcttggttg
gccagtcccaccactgcagtgaccacagttgtgttgggctcacaccagtgaaccgaagct
ctggattctgagagtctgaggattccgtgaagatctcagacttgggctcagagcaaggat
g (Seq ID No: 1363)

PpLuc(GC)-ag-A64

GGGAGAAAGCTTGAGG*ATGGAGGACGCCAAGAACATCAAGAAGGGCCCGGCGCCCTTCTA*
*CCCGCTGGAGGACGGGACCGCCGGCGAGCAGCTCCACAAGGCCATGAAGCGGTACGCCCT*
*GGTGCCGGGCACGATCGCCTTCACCGACGCCCACATCGAGGTCGACATCACCTACGCGGA*
*GTACTTCGAGATGAGCGTGCGCCTGGCCGAGGCCATGAAGCGGTACGGCCTGAACACCAA*
*CCACCGGATCGTGGTGTGCTCGGAGAACAGCCTGCAGTTCTTCATGCCGGTGCTGGGCGC*
*CCTCTTCATCGGCGTGGCCGTCGCCCCGGCGAACGACATCTACAACGAGCGGGAGCTGCT*
*GAACAGCATGGGGATCAGCCAGCCGACCGTGGTGTTCGTGAGCAAGAAGGGCCTGCAGAA*
*GATCCTGAACGTGCAGAAGAAGCTGCCCATCATCCAGAAGATCATCATCATGGACAGCAA*
*GACCGACTACCAGGGCTTCCAGTCGATGTACACGTTCGTGACCAGCCACCTCCCGCCGGG*
*CTTCAACGAGTACGACTTCGTCCCGGAGAGCTTCGACCGGGACAAGACCATCGCCCTGAT*
*CATGAACAGCAGCGGCAGCACCGGCCTGCCGAAGGGGGTGGCCCTGCCGCACCGGACCGC*
*CTGCGTGCGCTTCTCGCACGCCCGGGACCCCATCTTCGGCAACCAGATCATCCCGGACAC*
*CGCCATCCTGAGCGTGGTGCCGTTCCACCACGGCTTCGGCATGTTCACGACCCTGGGCTA*
*CCTCATCTGCGGCTTCCGGGTGGTCCTGATGTACCGGTTCGAGGAGGAGCTGTTCCTGCG*
*GAGCCTGCAGGACTACAAGATCCAGAGCGCGCTGCTCGTGCCGACCCTGTTCAGCTTCTT*
*CGCCAAGAGCACCCTGATCGACAAGTACGACCTGTCGAACCTGCACGAGATCGCCAGCGG*
*GGGCGCCCCGCTGAGCAAGGAGGTGGGCGAGGCCGTGGCCAAGCGGTTCCACCTCCCGGG*
*CATCCGCCAGGGCTACGGCCTGACCGAGACCACGAGCGCGATCCTGATCACCCCCGAGGG*
*GGACGACAAGCCGGGCGCCGTGGGCAAGGTGGTCCCGTTCTTCGAGGCCAAGGTGGTGGA*
*CCTGGACACCGGCAAGACCCTGGGCGTGAACCAGCGGGGCGAGCTGTGCGTGCGGGGGCC*
*GATGATCATGAGCGGCTACGTGAACAACCCGGAGGCCACCAACGCCCTCATCGACAAGGA*
*CGGCTGGCTGCACAGCGGCGACATCGCCTACTGGGACGAGGACGAGCACTTCTTCATCGT*
*CGACCGGCTGAAGTCGCTGATCAAGTACAAGGGCTACCAGGTGGCGCCGGCCGAGCTGGA*

*GAGCATCCTGCTCCAGCACCCCAACATCTTCGACGCCGGCGTGGCCGGGCTGCCGGACGA*
*CGACGCCGGCGAGCTGCCGGCCGCGGTGGTGGTGCTGGAGCACGGCAAGACCATGACGGA*
*GAAGGAGATCGTCGACTACGTGGCCAGCCAGGTGACCACCGCCAAGAAGCTGCGGGGCGG*
*CGTGGTGTTCGTGGACGAGGTCCCGAAGGGCCTGACCGGGAAGCTCGACGCCCGGAAGAT*
*CCGCGAGATCCTGATCAAGGCCAAGAAGGGCGGCAAGATCGCCGTGTAA*GACTAGTTATA
AGACTGACTAGCCCGATGGGCCTCCCAACGGGCCCTCCTCCCCTCCTTGCACCGAGATTA
ATAGATCTAAAAAAAAAAAAAAAAAAAAAAAAAAAAAAAAAAAAAAAAAAAAAAAAAAAA
AAAAAAAAAAAA (SEQ ID No: 1364)

RPL32-PpLuc(GC)-ag-A64-C30-histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGCCCTCCTCCCCTCCTTGCACCGAGATTAATAGATCTAAAAAAAAAAAAAAA
AAAAAAAAAAAAAAAAAAAAAAAAAAAAAAAAAAAAAAAAAAAAAAAAAATGCATCCCCC
CCCCCCCCCCCCCCCCCCCCCCCCCCAAAGGCTCTTTTCAGAGCCACCAGAATT(SEQ ID No: 1365)

fragment of the 5'UTR of human ribosomal protein Large 32 ACGGAGGTGGCAGCCATCTCCTTCTCGGCATC (SEQ ID No: 1366)

fragment of the 5'UTR of human ribosomal protein Large 32 GGCGCTGCCTACGGAGGTGGCAGCCATCTCCT (SEQ ID No: 1367)

5'UTR of human ribosomal protein Large 32 lacking the 5' terminal oligopyrimidine tract GGCGCTGCCTACGGAGGTGGCAGCCATCTCCTTCTCGGCATC (SEQ ID No. 1368)

Human albumin 3'UTR

CATCACATTT AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA AAAATGGAAA GAATCT (SEQ ID No: 1369)

3'UTR of Homo sapiens hemoglobin, alpha 1 (HBA1)

gctggagcctcggtggccatgcttcttgccccttgggcctccccccagcccctcctcccc ttcctgcacccgtacccccgtggtctttgaataaagtctgagtgggcggc (SEQ ID No: 1370)

3'UTR of Homo sapiens hemoglobin, alpha 2 (HBA2)

gctggagcctcggtagccgttcctcctgcccgctgggcctcccaacgggccctcctcccctccttg caccggcccttcctggtctttgaataaagtctgagtgggcag (SEQ ID No: 1371)

3'UTR of Homo sapiens hemoglobin, beta (HBB)

Gctcgctttcttgctgtccaatttctattaaaggttcctttgttccctaagtccaactactaaact gggggatattatgaagggccttgagcatctggattctgcctaataaaaaacatttattttcattgc (SEQ ID No: 1372)

3'UTR of Homo sapiens tyrosine hydroxylase (TH)

gtgcacggcgtccctgagggcccttcccaacctcccctggtcctgcactgtcccggagct caggccctggtgaggggctgggtcccgggtgccccccatgccctccctgctgccaggctc ccactgcccctgcacctgcttctcagcgcaacagctgtgtgtgcccgtggtgaggttgtg ctgcctgtggtgaggtcctgtcctggctcccagggtcctgggggctgctgcactgccctc cgcccttccctgacactgtctgctgccccaatcaccgtcacaataaaagaaactgtggtc tcta (SEQ ID No: 1373)

3'UTR of Homo sapiens arachidonate 15-lipoxygenase (ALOX15)

gcgtcgccaccctttggttatttcagcccccatcacccaagccacaagctgaccccttcg tggttatagccctgccctcccaagtcccaccctcttcccatgtcccaccctccctagagg ggcaccttttcatggtctctgcacccagtgaacacatttactctagaggcatcacctgg gaccttactcctctttccttccttcctcctttcctatcttccttcctctctctcttcctc tttcttcattcagatctatatggcaaatagccacaattatataaatcatttcaagactag aatagggggatataatacatattactccacacctttatgaatcaaatatgatttttttg ttgttgttaagacagagtctcactttgacacccaggctggagtgcagtggtgccatcacc acggctcactgcagcctcagcgtcctgggctcaaatgatcctcccacctcagcctcctga gtagctgggactacaggctcatgccatcatgcccagctaatatttttttattttcgtgga gacggggcctcactatgttgcctaggctggaaataggattttgaacccaaattgagttta acaataataaaaagttgttttacgctaaagatggaaaagaactaggactgaactatttta aataaaatattggc (SEQ ID No: 1374)

3'UTR of Homo sapiens collagen, type I, alpha 1 (COL1A1)

actccctccatcccaacctggctccctcccacccaaccaactttccccccaacccggaaa cagacaagcaacccaaactgaaccccctcaaaagccaaaaaatgggagacaatttcacat ggactttggaaaatatttttttcctttgcattcatctctcaaacttagttttttatctttg

accaaccgaacatgaccaaaaaccaaaagtgcattcaaccttaccaaaaaaaaaaaaaa
aaaagaataaataaataacttttttaaaaaaggaagcttggtccacttgcttgaagaccca
tgcgggggtaagtccctttctgcccgttgggcttatgaaaccccaatgctgccctttctg
ctcctttctccacacccccccttggggcctcccctccactccttcccaaatctgtctcccc
agaagacacaggaaacaatgtattgtctgcccagcaatcaaaggcaatgctcaaacaccc
aagtggcccccaccctcagcccgctcctgcccgcccagcacccccaggccctgggggacc
tggggttctcagactgccaaagaagccttgccatctggcgctcccatggctcttgcaaca
tctccccttcgtttttgaggggtcatgccgggggagccaccagcccctcactgggttcg
gaggagagtcaggaagggccacgacaaagcagaaacatcggatttggggaacgcgtgtca
atcccttgtgccgcagggctgggcgggagagactgttctgttccttgtgtaactgtgttg
ctgaaagactacctcgttcttgtcttgatgtgtcaccggggcaactgcctgggggcgggg
atgggggcagggtggaagcggctccccattttataccaaaggtgctacatctatgtgatg
ggtggggtggggagggaatcactggtgctatagaaattgagatgcccccccaggccagca
aatgttccttttttgttcaaagtctatttttattccttgatatttttcttttttttttttt
tttttgtggatggggacttgtgaattttctaaaggtgctatttaacatgggaggagag
cgtgtgcggctccagcccagcccgctgctcactttccaccctctctccacctgcctctgg
cttctcaggcctctgctctccgacctctctcctctgaaaccctcctccacagctgcagcc
catcctcccggctccctcctagtctgtcctgcgtcctctgtccccgggtttcagagacaa
cttcccaaagcacaaagcagtttttcccccctaggggtgggaggaagcaaaagactctgta
cctattttgtatgtgtataataatttgagatgtttttaattattttgattgctggaataa
agcatgtggaaatgacccaaacataa (SEQ ID No: 1375)

albumin7 3'UTR

CATCACATTTAAAAGCATCTCAGCCTACCATGAGAATAAGAGAAAGAAAATGAAGATCAA
TAGCTTATTCATCTCTTTTTCTTTTTCGTTGGTGTAAAGCCAACACCCTGTCTAAAAAAC
ATAAATTTCTTTAATCATTTTGCCTCTTTTCTCTGTGCTTCAATTAATAAAAAATGGAAA
GAACCT (SEQ ID No: 1376)

Human albumin 3'UTR + poly(A) sequence

CATCACATTT AAAAGCATCT CAGCCTACCA TGAGAATAAG AGAAAGAAAA
TGAAGATCAA AAGCTTATTC ATCTGTTTTT CTTTTTCGTT GGTGTAAAGC
CAACACCCTG TCTAAAAAAC ATAAATTTCT TTAATCATTT TGCCTCTTTT
CTCTGTGCTT CAATTAATAA AAAATGGAAA GAATCTAGAT CTAAAAAAAA
AAAAAAAAAA AAAAAAAAAA AAAAAAAAAA AAAAAAAAAA AAAAAAAAAA
AAAAAA (SEQ ID No: 1377)

Human albumin 3'UTR fragment 1

AAAAGCATCT CAGCCTACCA TGAGAATAAG AGAAAGAAAA TGAAGATCAA
AAGCTTATTC ATCTGTTTTT CTTTTTCGTT GGTGTAAAGC CAACACCCTG
TCTAAAAAAC ATAAATTTCT TTAATCATTT TGCCTCTTTT CTCTGTGCTT CAATT
(SEQ ID No: 1378)

Human albumin 3'UTR fragment 2

CATCACATTT AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG (SEQ ID No: 1379)

Human albumin 3'UTR fragment 3

AAAAGCATCT CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC (SEQ ID No: 1380)

Human albumin 3'UTR fragment 4

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT (SEQ ID No: 1381)

Human albumin 3'UTR fragment 5

TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT (SEQ ID No: 1382)

Human albumin 3'UTR fragment 6

AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT (SEQ ID No: 1383)

Human albumin 3'UTR fragment 7

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT (SEQ ID No: 1384)

Human albumin 3'UTR fragment 8

AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA (SEQ ID No: 1385)

Human albumin 3'UTR fragment 9

ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA AAAATGGAAA (SEQ ID No: 1386)

Human albumin 3'UTR fragment 10

CAGCCTACCA TGAGAATAAG AGAAAGAAAA TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No: 1387)

Human albumin 3'UTR fragment 11

TGAAGATCAA AAGCTTATTC ATCTGTTTTT CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No: 1388)

Human albumin 3'UTR fragment 12

CTTTTTCGTT GGTGTAAAGC CAACACCCTG TCTAAAAAAC ATAAATTTCT TTAATCATTT TGCCTCTTTT CTCTGTGCTT CAATTAATAA A (SEQ ID No: 1389)

Human albumin 3'UTR fragment 13

AAGCTTATTC ATCTGTTTTT CTTTTTCGTT GGTGTAAAGC CAACACCCTG TCTAAAAAAC (SEQ ID No: 1390)

Sequence according to formula (Ic) NGNNNNNNUNNNNNNCN (SEQ ID NO: 1391)

Sequence according to formula (IIc) : N*N*NNNNGNNNNNNUNNNNNNCNNNN*N*N* (SEQ ID NO: 1392)

Sequence according to formula (Id): NCNNNNNNUNNNNNNGN (SEQ ID NO: 1393)

Sequence according to formula (IId) N*N*NNNNCNNNNNNUNNNNNNGNNNN*N*N* (SEQ ID NO: 1394)

Sequence according to formula (Ie) DGNNNNNNUNNNNNNCH (SEQ ID NO: 1395)

Sequence according to formula (IIe) N*N*NNNDGNNNNNNUNNNNNNCHNNN*N*N* (SEQ ID NO: 1396)

Sequence according to formula (If) NGNBYYNNUNVNDNCN (SEQ ID NO: 1397)

Sequence according to formula (IIf) N*N*NNNNGNBYYNNUNVNDNCNNNN*N*N* (SEQ ID NO: 1398)

Sequence according to formula (Ig) NGHYYYDNUHABRDCN (SEQ ID NO: 1399)

Sequence according to formula (IIg) N*N*HNNNGHYYYDNUHABRDCNNNN*N*H* (SEQ ID NO: 1400)

Sequence according to formula (Ih) DGHYCUDYUHASRRCC (SEQ ID NO: 1401)

Sequence according to formula (IIh) N*H*AAHDGHYCUDYUHASRRCCVHB*N*H* (SEQ ID NO: 1402)

Sequence according to formula (Ic) VGYYYYHHTHRVVRCB (SEQ ID NO: 1403)

Sequence according to formula (Ic) SGYYYTTYTMARRRCS (SEQ ID NO: 1404)

Sequence according to formula (Ic) SGYYCTTTTMAGRRCS (SEQ ID NO: 1405)

Sequence according to formula (Ie) DGNNNBNNTHVNNNCH (SEQ ID NO: 1406)

Sequence according to formula (Ie) RGNNNYHBTHRDNNCY (SEQ ID NO: 1407)

Sequence according to formula (Ie) RGNDBYHYTHRDHNCY (SEQ ID NO: 1408)

Sequence according to formula (If) VGYYYTYHTHRVRRCB (SEQ ID NO: 1409)

Sequence according to formula (If) SGYYCTTYTMAGRRCS (SEQ ID NO: 1410)

Sequence according to formula (If) SGYYCTTTTMAGRRCS (SEQ ID NO: 1411)

Sequence according to formula (Ig) GGYYCTTYTHAGRRCC (SEQ ID NO: 1412)

Sequence according to formula (Ig) GGCYCTTYTMAGRGCC (SEQ ID NO: 1413)

Sequence according to formula (Ig) GGCTCTTTTMAGRGCC (SEQ ID NO: 1414)

Sequence according to formula (Ih) DGHYCTDYTHASRRCC (SEQ ID NO: 1415)

Sequence according to formula (Ih) GGCYCTTTTHAGRGCC (SEQ ID NO: 1416)

Sequence according to formula (Ih) GGCYCTTTTMAGRGCC (SEQ ID NO: 1417)

Sequence according to formula (IIc) H*H*HHVVGYYYYHHTHRVVRCBVHH*N*N* (SEQ ID NO: 1418)

Sequence according to formula (IIc) M*H*MHMSGYYYTTYTMARRRCSMCH*H*H* (SEQ ID NO: 1419)

Sequence according to formula (IIc) M*M*MMMSGYYCTTTTMAGRRCSACH*M*H* (SEQ ID NO: 1420)

Sequence according to formula (IIe) N*N*NNNDGNNNBNNTHVNNNCHNHN*N*N* (SEQ ID NO: 1421)

Sequence according to formula (IIe) N*N*HHNRGNNNYHBTHRDNNCYDHH*N*N* (SEQ ID NO: 1422)

Sequence according to formula (IIe) N*H*HHVRGNDBYHYTHRDHNCYRHH*H*H* (SEQ ID NO: 1423)

Sequence according to formula (IIf) H*H*MHMVGYYYTYHTHRVRRCBVMH*H*N* (SEQ ID NO: 1424)

Sequence according to formula (IIf) M*M*MMMSGYYCTTYTMAGRRCSMCH*H*H* (SEQ ID NO: 1425)

Sequence according to formula (IIf) M*M*MMMSGYYCTTTTMAGRRCSACH*M*H* (SEQ ID NO: 1426)

Sequence according to formula (IIg) H*H*MAMGGYYCTTYTHAGRRCCVHN*N*M* (SEQ ID NO: 1427)

Sequence according to formula (IIg) H*H*AAMGGCYCTTYTMAGRGCCVCH*H*M* (SEQ ID NO: 1428)

Sequence according to formula (IIg) M*M*AAMGGCTCTTTTMAGRGCCMCY*M*M* (SEQ ID NO: 1429)

Sequence according to formula (IIh) N*H*AAHDGHYCTDYTHASRRCCVHB*N*H* (SEQ ID NO: 1430)

Sequence according to formula (IIh) H*H*AAMGGCYCTTTTHAGRGCCVMY*N*M* (SEQ ID NO: 1431)

Sequence according to formula (IIh) H*M*AAAGGCYCTTTTMAGRGCCRMY*H*M* (SEQ ID NO: 1432)

Specific histone stem-loop sequence CAAAGGCTCTTTTCAGAGCCACCA (SEQ ID NO: 1433)

Center, α-complex-binding portion of the 3'UTR of an α-globin gene GCCCGATGGGCCTCCCAACGGGCCCTCCTCCCCTCCTTGCACCG (SEQ ID NO: 1434)

ATP synthase lipid-binding protein, mitochondrial (atp5g2) tagttt ctcctctcga acgccaggtg gagcaaccgg ccggataccg

ccacagccct ggcaggcggc gctgtgatg (SEQ ID NO: 1435)

RPL35 - PpLuc(GC) - albumin7 - A64 - C30 - histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT

CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAAATGCATCCCCCCCCCCC
CCCCCCCCCCCCCCCCCCCAAAGGCTCTTTTCAGAGCCACCAGAATT (SEQ ID NO: 1436)

RPL21 - PpLuc(GC) - albumin7 - A64 - C30 - histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT

GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT (SEQ ID NO: 1437)

ATP5A1 - PpLuc(GC) - albumin7 - A64 - C30 - histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTGCATCACATTTAAAAGCATCTCAGCCTACCATGAGAATAAGAGAAAGAAAATG
AAGATCAATAGCTTATTCATCTCTTTTTCTTTTTCGTTGGTGTAAAGCCAACACCCTGTC
TAAAAAACATAAATTTCTTTAATCATTTTGCCTCTTTTCTCTGTGCTTCAATTAATAAAA
AATGGAAAGAACCTAGATCTAAAAAAAAAAAAAAAAAAAAAAAAAAAAAAAAAAAAAAAA
AAAAAAAAAAAAAAAAAAAAAAAATGCATCCCCCCCCCCCCCCCCCCCCCCCCCCCCCCC
AAAGGCTCTTTTCAGAGCCACCAGAATT (SEQ ID NO: 1438)

HSD17B4 - PpLuc(GC) - albumin7 - A64 - C30 - histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG

TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTGCATCA
CATTTAAAAGCATCTCAGCCTACCATGAGAATAAGAGAAAGAAAATGAAGATCAATAGCT
TATTCATCTCTTTTTCTTTTTCGTTGGTGTAAAGCCAACACCCTGTCTAAAAAACATAAA
TTTCTTTAATCATTTTGCCTCTTTTCTCTGTGCTTCAATTAATAAAAAATGGAAAGAACC
TAGATCTAAAAAAAAAAAAAAAAAAAAAAAAAAAAAAAAAAAAAAAAAAAAAAAAAAAAA
AAAAAAAAAAATGCATCCCCCCCCCCCCCCCCCCCCCCCCCCCCCCCAAAGGCTCTTTTC
AGAGCCACCAGAATT (SEQ ID NO: 1439)

AIG1 - PpLuc(GC) - albumin7 - A64 - C30 - histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC

GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTGCATCACATTTAAAAGCATCTCAGCCTACCATGAGAA
TAAGAGAAAGAAAATGAAGATCAATAGCTTATTCATCTCTTTTTCTTTTTCGTTGGTGTA
AAGCCAACACCCTGTCTAAAAAACATAAATTTCTTTAATCATTTTGCCTCTTTTCTCTGT
GCTTCAATTAATAAAAAATGGAAAGAACCTAGATCTAAAAAAAAAAAAAAAAAAAAAAAA
AAAAAAAAAAAAAAAAAAAAAAAAAAAAAAAAAAAAAAAATGCATCCCCCCCCCCCCCCC
CCCCCCCCCCCCCCCAAAGGCTCTTTTCAGAGCCACCAGAATT (SEQ ID NO: 1440)

COX6C - PpLuc (GC) - albumin7 - A64 - C30 - histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTGCATCACATTTA
AAAGCATCTCAGCCTACCATGAGAATAAGAGAAAGAAAATGAAGATCAATAGCTTATTCA
TCTCTTTTTCTTTTTCGTTGGTGTAAAGCCAACACCCTGTCTAAAAAACATAAATTTCTT
TAATCATTTTGCCTCTTTTCTCTGTGCTTCAATTAATAAAAAATGGAAAGAACCTAGATC
TAAAAAAAAAAAAAAAAAAAAAAAAAAAAAAAAAAAAAAAAAAAAAAAAAAAAAAAAAAA
AAAAATGCATCCCCCCCCCCCCCCCCCCCCCCCCCCCCCCAAAGGCTCTTTTCAGAGCC

ACCAGAATT (SEQ ID NO: 1441)

ASAH1 - PpLuc(GC) - albumin7 - A64 - C30 - histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT (SEQ ID NO: 1442)

mRPL21 - PpLuc (GC) - albumin7 - A64 - C30 - histoneSL

GGGGCCGCCGCAGCCATCTTCCAGTAACTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC

GAAGGGGGTGGCCCTGCCGCACCGGACCGCCTGCGTGCGCTTCTCGCACGCCCGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCCAAAGGCTCTTTTCAGAGCCACCAGAATT (SEQ ID NO: 1443)

mRPL35A - PpLuc(GC) - albumin7 - A64 - C30 - histoneSL

GGGCCATCTTGGCGCCTGTGGAGGCCTGCTGGGAACAGGACTTCTAACAGCAAGTAAGCT
TGAGGATGGAGGACGCCAAGAACATCAAGAAGGGCCCGGCGCCCTTCTACCCGCTGGAGG
ACGGGACCGCCGGCGAGCAGCTCCACAAGGCCATGAAGCGGTACGCCCTGGTGCCGGGCA
CGATCGCCTTCACCGACGCCCACATCGAGGTCGACATCACCTACGCGGAGTACTTCGAGA
TGAGCGTGCGCCTGGCCGAGGCCATGAAGCGGTACGGCCTGAACACCAACCACCGGATCG
TGGTGTGCTCGGAGAACAGCCTGCAGTTCTTCATGCCGGTGCTGGGCGCCCTCTTCATCG
GCGTGGCCGTCGCCCCGGCGAACGACATCTACAACGAGCGGGAGCTGCTGAACAGCATGG
GGATCAGCCAGCCGACCGTGGTGTTCGTGAGCAAGAAGGGCCTGCAGAAGATCCTGAACG
TGCAGAAGAAGCTGCCCATCATCCAGAAGATCATCATCATGGACAGCAAGACCGACTACC
AGGGCTTCCAGTCGATGTACACGTTCGTGACCAGCCACCTCCCGCCGGGCTTCAACGAGT
ACGACTTCGTCCCGGAGAGCTTCGACCGGGACAAGACCATCGCCCTGATCATGAACAGCA
GCGGCAGCACCGGCCTGCCGAAGGGGGTGGCCCTGCCGCACCGGACCGCCTGCGTGCGCT
TCTCGCACGCCCGGGACCCCATCTTCGGCAACCAGATCATCCCGGACACCGCCATCCTGA
GCGTGGTGCCGTTCCACCACGGCTTCGGCATGTTCACGACCCTGGGCTACCTCATCTGCG
GCTTCCGGGTGGTCCTGATGTACCGGTTCGAGGAGGAGCTGTTCCTGCGGAGCCTGCAGG
ACTACAAGATCCAGAGCGCGCTGCTCGTGCCGACCCTGTTCAGCTTCTTCGCCAAGAGCA
CCCTGATCGACAAGTACGACCTGTCGAACCTGCACGAGATCGCCAGCGGGGGCGCCCCGC
TGAGCAAGGAGGTGGGCGAGGCCGTGGCCAAGCGGTTCCACCTCCCGGGCATCCGCCAGG
GCTACGGCCTGACCGAGACCACGAGCGCGATCCTGATCACCCCCGAGGGGGACGACAAGC
CGGGCGCCGTGGGCAAGGTGGTCCCGTTCTTCGAGGCCAAGGTGGTGGACCTGGACACCG
GCAAGACCCTGGGCGTGAACCAGCGGGGCGAGCTGTGCGTGCGGGGGCCGATGATCATGA
GCGGCTACGTGAACAACCCGGAGGCCACCAACGCCCTCATCGACAAGGACGGCTGGCTGC
ACAGCGGCGACATCGCCTACTGGGACGAGGACGAGCACTTCTTCATCGTCGACCGGCTGA
AGTCGCTGATCAAGTACAAGGGCTACCAGGTGGCGCCGGCCGAGCTGGAGAGCATCCTGC
TCCAGCACCCCAACATCTTCGACGCCGGCGTGGCCGGGCTGCCGGACGACGACGCCGGCG
AGCTGCCGGCCGCGGTGGTGGTGCTGGAGCACGGCAAGACCATGACGGAGAAGGAGATCG
TCGACTACGTGGCCAGCCAGGTGACCACCGCCAAGAAGCTGCGGGGCGGCGTGGTGTTCG

TGGACGAGGTCCCGAAGGGCCTGACCGGGAAGCTCGACGCCCGGAAGATCCGCGAGATCC
TGATCAAGGCCAAGAAGGGCGGCAAGATCGCCGTGTAAGACTAGTGCATCACATTTAAAA
GCATCTCAGCCTACCATGAGAATAAGAGAAAGAAAATGAAGATCAATAGCTTATTCATCT
CTTTTTCTTTTTCGTTGGTGTAAAGCCAACACCCTGTCTAAAAAACATAAATTTCTTTAA
TCATTTTGCCTCTTTTCTCTGTGCTTCAATTAATAAAAAATGGAAAGAACCTAGATCTAA
AAAAAAAAAAAAAAAAAAAAAAAAAAAAAAAAAAAAAAAAAAAAAAAAAAAAAAAAAAAA
AATGCATCCCCCCCCCCCCCCCCCCCCCCCCCCCCCCCAAAGGCTCTTTTCAGAGCCACC
AGAATT (SEQ ID NO: 1444)

RPL35 - PpLuc(GC) - A64 - C30 - histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAATGCATCCCCCCCCCCCCCCCCCC
CCCCCCCCCCCCAAAGGCTCTTTTCAGAGCCACCAGAATT (SEQ ID NO: 1445)

RPL21 - PpLuc(GC) - A64 - C30 - histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT

CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTAGATCTAAAAAAAAAAAAAAAAAAAAAAAAAAAA
AAAAAAAAAAAAAAAAAAAAAAAAAAAAAAAAAAAAATGCATCCCCCCCCCCCCCCCCCC
CCCCCCCCCCCCAAAGGCTCTTTTCAGAGCCACCAGAATT (SEQ ID NO: 1446)

ATP5A1 - PpLuc(GC) - A64 - C30 - histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG

CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTAGATCTAAAAAAAAAAAAAAAAAAAAAAAAAAAAAAAAAAAAAAAAAAAAAAA
AAAAAAAAAAAAAAAAATGCATCCCCCCCCCCCCCCCCCCCCCCCCCCCCCCCAAAGGCT
CTTTTCAGAGCCACCAGAATT (SEQ ID NO: 1447)

HSD17B4 - PpLuc(GC) - A64 - C30 - histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTAGATCT
AAAAAAAAAAAAAAAAAAAAAAAAAAAAAAAAAAAAAAAAAAAAAAAAAAAAAAAAAAAA
AAAATGCATCCCCCCCCCCCCCCCCCCCCCCCCCCCCCCCAAAGGCTCTTTTCAGAGCCA
CCAGAATT (SEQ ID NO: 1448)

AIG1 - PpLuc(GC) - A64 - C30 - histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT

CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTAGATCTAAAAAAAAAAAAAAAAAAAAAAAAAAAAAAA
AAAAAAAAAAAAAAAAAAAAAAAAAAAAAAAAATGCATCCCCCCCCCCCCCCCCCCCCCC
CCCCCCCCCAAAGGCTCTTTTCAGAGCCACCAGAATT (SEQ ID NO: 1449)

COX6C - PpLuc(GC) - A64 - C30 - histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTAGATCTAAAAAA

AAAAAAAAAAAAAAAAAAAAAAAAAAAAAAAAAAAAAAAAAAAAAAAAAAAAAAAAAATG
CATCCCCCCCCCCCCCCCCCCCCCCCCCCCCCCCAAAGGCTCTTTTCAGAGCCACCAGAA
TT (SEQ ID NO: 1450)

ASAH1 - PpLuc(GC) - A64 - C30 - histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTAGATCTAAAAAAAAAAAAAAAAAAAAA
AAAAAAAAAAAAAAAAAAAAAAAAAAAAAAAAAAAAAAAAAAAATGCATCCCCCCCCCCC
CCCCCCCCCCCCCCCCCCCCAAAGGCTCTTTTCAGAGCCACCAGAATT (SEQ ID NO: 1451)

5'UTR of human ribosomal protein Large 35 (RPL35) lacking the 5' terminal oligopyrimidine tract GGAGCGGGCGGCGGCGTTGGCGGCTTGTGCAGCA (SEQ ID NO: 1452)

5'UTR of human ribosomal protein Large 21 (RPL21) lacking the 5' terminal oligopyrimidine tract GGCCGGAACCGCCATCTTCCAGTAATTCGCCAAA (SEQ ID NO: 1453)

5'UTR of human ATP synthase, H+ transporting, mitochondrial F1 complex, alpha subunit 1, cardiac muscle (ATP5A1) lacking the 5' terminal oligopyrimidine tract

GCGGCTCGGCCATTTTGTCCCAGTCAGTCCGGAGGCTGCGGCTGCAGAAGTACCGCCTGCGGAGTA
ACTGCAAAG (SEQ ID NO: 1454)

5'UTR of human hydroxysteroid (17-beta) dehydrogenase 4 (HSD17B4) lacking the 5' terminal oligopyrimidine tract GTCCCGCAGTCGGCGTCCAGCGGCTCTGCTTGTTCGTGTGTGTGTCGTTGCAGGCCTTATTC (SEQ ID NO: 1455)

5'UTR of human androgen-induced 1 (AIG1) lacking the 5' terminal oligopyrimidine tract GCCGCCCAGCCGGTCCAGGCCTCTGGCGAAC (SEQ ID NO: 1456)

5'UTR of human cytochrome c oxidase subunit VIc (COX6C) lacking the 5' terminal oligopyrimidine tract AGTCAGGAAGGACGTTGGTGTTGAGGTTAGCATACGTATCAAGGACAGTAACTACC (SEQ ID NO: 1457)

5'UTR of human N-acylsphingosine amidohydrolase (acid ceramidase) 1 (ASAH1) lacking the 5' terminal oligopyrimidine tract GCCTCTGCTGGAGTCCGGGGAGTGGCGTTGGCTGCTAGAGCG (SEQ ID NO: 1458)

5'UTR of mouse ribosomal protein Large 21 (mRPL21) lacking the 5' terminal oligopyrimidine tract GGCCGCCGCAGCCATCTTCCAGTAACTCGCCAAA (SEQ ID NO: 1459)

5'UTR of mouse ribosomal protein large 35A (mRPL35A) lacking the 5' terminal oligopyrimidine tract GCCATCTTGGCGCCTGTGGAGGCCTGCTGGGAACAGGACTTCTAACAGCAAGT (SEQ ID NO: 1460)

Mouse ribosomal protein Large 21 (mRPL21)

TCCTCCTTTCGGCCGCCGCAGCCATCTTCCAGTAACTCGCCAAAATGCCATCTTCCAGTAACTCGC
CAAAATG (SEQ ID NO: 1461)

mouse ribosomal protein large 35A (mRPL35A)

CTTCCTCTTTCCGCCATCTTGGCGCCTGTGGAGGCCTGCTGGGAACAGGACTTCTAACAGCAAGTA
TG (SEQ ID NO: 1462)

RPL32 - PpLuc (GC) - ag - A64

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG

GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAA (SEQ ID NO: 1463)

PpLuc(GC) - ag - A64 - histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAAATGCATCAAAGGCTCTT
TTCAGAGCCACCA (SEQ ID NO: 1464)

PpLuc(GC) - albumin7 - A64 - C30 - histoneSL

GGGGCGCTGCCTACGGAGGTGGCAGCCATCTCCTTCTCGGCATCAAGCTTGAGGATGGAG

GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT (SEQ ID NO: 1465)

RPL35 - PpLuc(GC) - ag - A64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA

CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA (SEQ ID NO: 1466)

RPL21 - PpLuc(GC) - ag - A64

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA (SEQ ID NO: 1467)

atp5a1 - PpLuc(GC) - ag - A64

GGGCGGCTCGGCCATTTTGTCCCAGTCAGTCCGGAGGCTGCGGCTGCAGAAGTACCGCCT

GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAAA
AAAAAAAAAAAAAAAAA (SEQ ID NO: 1468)

HSD17B4 - PpLuc(GC) - ag - A64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA

TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAA (SEQ ID NO: 1469)

AIG1 - PpLuc(GC) - ag - A64

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAA (SEQ ID NO: 1470)

COX6C - PpLuc(GC) - ag - A64

GGAGTCAGGAAGGACGTTGGTGTTGAGGTTAGCATACGTATCAAGGACAGTAACTACCAA

GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA (SEQ ID NO: 1471)

ASAH1 - PpLuc(GC) - ag - A64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG

GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAA (SEQ ID NO: 1472)

RPL35 - PpLuc(GC) - ag - A64 - histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA (SEQ ID NO: 1473)

RPL21 - PpLuc(GC) - ag - A64 - histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA

GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAAATGCATCAAAGGCTCTTTTCAGAGC
CACCA (SEQ ID NO: 1474)

atp5a1 - PpLuc(GC) - ag - A64 - histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC

AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAAA
AAAAAAAAAAAAAAAAATGCATCAAAGGCTCTTTTCAGAGCCACCA (SEQ ID NO: 1475)

HSD17B4 - PpLuc(GC) - ag - A64 - histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAATGCATCAAAGGCTCTTTTCAGAGCCACCA (SEQ ID NO: 1476)

AIG1 - PpLuc(GC) - ag - A64 - histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT

CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAAATGCATCAAAGGCTCTTTTCAGAGCCAC
CA(SEQ ID NO: 1477)

COX6C - PpLuc(GC) - ag - A64 - histoneSL

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA

CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA
TGCATCAAAGGCTCTTTTCAGAGCCACCA (SEQ ID NO: 1478)

ASAH1 - PpLuc(GC) - ag - A64 - histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAAAATGCATCAAAGGCTCTT
TTCAGAGCCACCA (SEQ ID NO: 1479)

RPL32 - PpLuc(GC) - ag - A64 - histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAAAATGCATCAAAGGCTCTT
TTCAGAGCCACCA (SEQ ID NO: 1480)

RPL32 - PpLuc(GC) - albumin7 - A64 - C30 - histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC

AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT (SEQ ID NO: 1481)

SEQUENCE LISTING

<110> CureVac AG

<120> Artificial nucleic acid molecules for improved protein or peptide expression

<130> CU01P132WO1EPT1

<160> 1481

<170> PatentIn version 3.5

<210> 1
<211> 21
<212> DNA

<213> Homo sapiens

<400> 1
gctccttctt tctgcaacat g 21

<210> 2
<211> 108
<212> DNA
<213> Homo sapiens

<400> 2

ggctctcttt ccgcgctgcg gtcagcctcg gcgtcccaca gagagggcca gaggtggaaa 60

cgcagaaaac caaaccagga ctatcagaga ttgcccggag aggggatg 108

<210> 3
<211> 69
<212> DNA
<213> Homo sapiens

<400> 3

cttcctcttc ttgatcgggg attcaggaag gagcccagga gcagaggaag tagagagaga 60

gacaacatg 69

<210> 4
<211> 159
<212> DNA
<213> Homo sapiens

<400> 4

tgtccttcct ctctggactg taagaatatg tctccagggc cagtgtctgc tgcgatcgag 60

tcccaccttc caagtcctgg catctcaatg catctgggaa gctacctgca ttaagtcagg 120

actgagcaca caggtgaact ccagaaagaa gaagctatg 159

<210> 5
<211> 57
<212> DNA
<213> Homo sapiens

<400> 5
tgaccttttc cctcccgcgg ctctctacct ctcgccgccc ctagggagga caccatg 57

<210> 6
<211> 168
<212> DNA
<213> Homo sapiens

<400> 6

gggcctctct agcttgcggc ctgtgtctat ggtcgggccc tctgcgtcca gctgctccgg 60

accgagctcg ggtgtatggg gccgtaggaa ccggctccgg ggccccgata acgggccgcc 120

cccacagcac cccgggctgg cgtgagggtc tcccttgatc tgagaatg 168

<210> 7
<211> 53
<212> DNA
<213> Homo sapiens

<400> 7
agctcttcta ctccactgct gtctatcttg cctgccggca cccagccacc atg 53

<210> 8
<211> 89
<212> DNA
<213> Homo sapiens

<400> 8

cttcctctac ccggttggca ggcggcctgg cccagcccct tctctaagga agcgcatttc 60

ctgcctccct gggccggccg ggctggatg 89

<210> 9
<211> 28
<212> DNA
<213> Homo sapiens

<400> 9
tcttctctct gctgctgtag ctgccatg 28

<210> 10
<211> 246
<212> DNA
<213> Homo sapiens

<400> 10

ctgtctttct tccgggaggc ggtgacagct gctgagacgt gttgcagcca gagtctctcc 60

gctttaatgc gctcccatta gtgccgtccc ccactggaaa accgtggctt ctgtattatt 120

tgccatcttt gttgtgtagg agcagggagg gcttcctccc ggggtcctag gcggcggtgc 180

agtccgtcgt agaagaatta gagtagaagt tgtcggggtc cgctcttagg acgcagccgc 240

ctcatg 246

<210> 11
<211> 211
<212> DNA
<213> Homo sapiens

<400> 11

cgtcccctcg cagttcggcg gtcccgcggg tctgtctctt gcttcaacag tgtttggacg 60

gaacagatcc ggggactctc ttccagcctc cgaccgccct ccgatttcct ctccgcttgc 120

aacctccggg accatcttct cggccatctc ctgcttctgg gacctgccag caccgttttt 180

gtggttagct ccttcttgcc aaccaaccat g 211

<210> 12
<211> 40
<212> DNA
<213> Homo sapiens

<400> 12
ccgcctccct gggcgccgga gtcatgtgac ccacacaatg 40

<210> 13
<211> 181
<212> DNA
<213> Homo sapiens

<400> 13

ttttctttca ttagggggaa ggcgtgagga aagtaccaaa cagcagcgga gttttaaact 60

ttaaatagac aggtctgagt gcctgaactt gccttttcat tttacttcat cctccaagga 120

gttcaatcac ttggcgtgac ttcactactt ttaagcaaaa gagtggtgcc caggcaacat 180

g 181

<210> 14
<211> 68
<212> DNA
<213> Homo sapiens

<400> 14

cattctctgg gaaagggcag cagcagccag gtgtggcagt gacagggagg tgtgaatgag 60

gcaggatg 68

<210> 15
<211> 55
<212> DNA
<213> Homo sapiens

<400> 15
cgctccttcc tcctcggctc gcgtctcact cagtgtacct tctagtcccg ccatg 55

<210> 16
<211> 22
<212> DNA
<213> Homo sapiens

<400> 16
ctgtcctctt cagctcaaga tg 22

<210> 17
<211> 111
<212> DNA
<213> Homo sapiens

<400> 17

gggccctttc tgggcaggac ccgccccttg gtcccgcaga gccttggtac ttggacctga 60

accttgctcc gagagggagt cctcgcggac gtcagccaag attccagaat g 111

<210> 18
<211> 113
<212> DNA
<213> Homo sapiens

<400> 18

cttcctttg cagcaagttc tttcctgcac taatcacaat tcttggaaga ggagaactgg 60

acgttgtgaa cagagttagc tggtaaatgt cctcttaaaa gatccaaaaa atg 113

<210> 19
<211> 146
<212> DNA
<213> Homo sapiens

<400> 19

agccctttct ccagggacag ttgctgaagc ttcatccttt gctctcattc tgtaagtcat 60

agaaaagttt gaaacattct gtctgtggta gagctcgggc cagctgtagt tcattcgcca 120

gtgtgctttt cttaatatct aagatg 146

<210> 20
<211> 79
<212> DNA
<213> Homo sapiens

<400> 20

ggtcccctat ccgcaccccg gcccctgaga gctggcactg cgactcgaga cagcggcccg 60

gcaggacagc tccagaatg 79

<210> 21
<211> 178
<212> DNA
<213> Homo sapiens

<400> 21

ccccctcttc ctcctcctca agggaaagct gcccacttct agctgccctg ccatcccctt 60

taaagggcga cttgctcagc gccaaaccgc ggctccagcc ctctccagcc tccggctcag 120

ccggctcatc agtcggtccg cgccttgcag ctcctccaga gggacgcgcc ccgagatg 178

<210> 22
<211> 21
<212> DNA
<213> Homo sapiens

<400> 22
ggctcttctg gcgccaaaat g 21

<210> 23
<211> 170
<212> DNA
<213> Homo sapiens

<400> 23

cttccttcct gaccggcgcg cgcagcctgc tgccgcggtc agcgcctgct cctgctcctc 60

cgctcctcct gcgcggggtg ctgaaacagc ccggggaagt agagccgcct ccggggagcc 120

caaccagccg aacgccgccg gcgtcagcag ccttgcgcgg ccacagcatg 170

<210> 24
<211> 615
<212> DNA
<213> Homo sapiens

<400> 24

gcgcctctct tccgccaggc atcccagagg tcctggtggt ttcatttccg ggtgcggctt 60

ctgtcataaa gcggagacct cccttcaaac gtggcgtcgt gggttgtttg cgcctcgcct 120

ggggtcagcg agcaaggacg ggcgcgggcg gggatactca aagccaacag ctggagtcag 180

cccttgtgtc ccgggctcac agtggcacga ctgaatcctc agagtcggct ggcttttgag 240

ctctcacgat tggggaggag gggcgtttc tggttcgcag ctccagagga ttgcgttcct 300

tcccccatac ctgtccccca cagtcacgct ctgccctgac gtgcagcatt tgacaagtta 360

ccccctcgcc acatactact tccacccacg tccgagttaa ctttgttctt aaccttcttg 420

agactaccct cggcctccag gtcttttttt cccagttcat ttttgcccat aagattgagt 480

ttcgagtttc agatatcatg cagaaagttt acctttaaga ctgagcaccc atctgatact 540

cttcctcccg aaaaagttca tgctcacgag agagtttgtg ggaaaagtga aagccagtac 600

acgcaggaaa ctatg 615

<210> 25
<211> 38
<212> DNA

<213> Homo sapiens

<400> 25
cgctccttca ccctcctcgt tggtgtcctg tcaccatg 38

<210> 26
<211> 177
<212> DNA
<213> Homo sapiens

<400> 26

gagccttctt gtcagcatct gttagtggag gttgggaagc ctctcctcct tccccctccc 60

tctttgcctc cacctggctc ctccccatgt tcgtccatca cccctccccc ctttcccaag 120

gacaatctgc aagaaagcag cggcggagga gagctaagac taaaagagtg gatcatg 177

<210> 27
<211> 54
<212> DNA
<213> Homo sapiens

<400> 27
ctgtctcctc agcttcaggc accaccactg acctgggaca gtgaatcgac aatg 54

<210> 28
<211> 848
<212> DNA
<213> Homo sapiens

<400> 28

```
agttctctcc tctcggaagc tgcagccatg atggaagttt gagagttgag ccgctgtgag        60
gcgaggccgg gctcaggcga gggagatgag agacggcggc ggccgcggcc cggagcccct       120
ctcagcgcct gtgagcagcc gcgggggcag cgccctcggg gagccggccg gcctgcggcg       180
gcggcagcgg cggcgtttct cgcctcctct tcgtcttttc taaccgtgca gcctcttcct       240
cggcttctcc tgaaagggaa ggtggaagcc gtgggctcgg gcgggagccg gctgaggcgc       300
ggcggcggcg gcggcacctc ccgctcctgg agcggggggg agaagcggcg gcggcggcgg       360
ccgcggcggc tgcagctcca gggagggggt ctgagtcgcc tgtcaccatt tccagggctg       420
ggaacgccgg agagttggtc tctccccttc tactgcctcc aacacggcgg cggcggcggc       480
ggcacatcca gggacccggg ccggttttaa acctcccgtc cgccgccgcc gcaccccccg       540
tggcccgggc tccggaggcc gccggcggag gcagccgttc ggaggattat tcgtcttctc       600
cccattccgc tgccgccgct gccaggcctc tggctgctga ggagaagcag gcccagtcgc       660
tgcaaccatc cagcagccgc cgcagcagcc attacccggc tgcggtccag agccaagcgg       720
cggcagagcg aggggcatca gctaccgcca agtccagagc catttccatc ctgcagaaga       780
agccccgcca ccagcagctt ctgccatctc tctcctcctt tttcttcagc cacaggctcc       840
cagacatg                                                                848
```

<210> 29
<211> 50
<212> DNA
<213> Homo sapiens

<400> 29
cctcccttac tgcaggaagg cactccgaag acataagtcg gtgagacatg 50

<210> 30
<211> 58
<212> DNA
<213> Homo sapiens

<400> 30
ccgcctccca ctccccagcg cccccggacc gtgcagttct ctgcaggacc aggccatg 58

<210> 31
<211> 187
<212> DNA
<213> Homo sapiens

<400> 31

gtttctccca gcctcagcct ccccgccgcc gccgccgccg ccgccgccga gccggtttcc 60

tttttccggc gctccgggtg cgagagacag gtcgggcccc ctaggcagcg agccgcagcg 120

caatcccggc gctcgcccaa ggaccctgga agctaccgtt accccgccgg gcagcgtggg 180

cgccatg 187

<210> 32
<211> 143
<212> DNA
<213> Homo sapiens

<400> 32

cctcctcctc ttacccaaat tttccagccg atcactggag ctgacttccg caatcccgat 60

ggaataaatc tagcacccct gatggtgtgc ccacactttg ctgccgaaac gaagccagac 120

aacagatttc catcagcagg atg 143

<210> 33
<211> 95
<212> DNA
<213> Homo sapiens

<400> 33

gcttctttgc gtaaccaata ctggaaggca tttaaaggca cctctgccgc cacagacctt 60

gcagttaact ccgccctgac ccacccttcc cgatg 95

<210> 34
<211> 77
<212> DNA
<213> Homo sapiens

<400> 34

ccgcctcctc ctgtcccgca gtcggcgtcc agcggctctg cttgttcgtg tgtgtgtcgt 60

tgcaggcctt attcatg 77

<210> 35
<211> 254
<212> DNA
<213> Homo sapiens

<400> 35

ctctctctgc ttctttcctt ttctctctca tggtagggtt atgagtcagt tgccaaaagg 60

tggggacatt tcctgatgca tttgcaacac tgagaagtta tcttaaggga ggctgggccc 120

cattctactc atctggccca gaaagtgaac accttggggg ccactaaggc agccctgcta 180

ggggagacgc tccaacctgt cttctctctg tctcctggca gctctcttgg cctcctagtt 240

tctacctaat catg 254

<210> 36
<211> 31
<212> DNA
<213> Homo sapiens

<400> 36
cagcctcctc ctgcctcacc gcccgaagat g 31

<210> 37
<211> 314
<212> DNA
<213> Homo sapiens

<400> 37

ccgccccttc tcgagaactc gcagagctgg gctggtaaaa ttgcagtgct gaagacactg 60

gacccgcaaa aggctgtccc tcccaaacct gggattctgg gctcactgag ttcacctgcg 120

agtcagccct acctgcactg ctctggtcta gtacaaacag gctgctggca ttgagggacg 180

gagtctccaa ctcctggcct ctagcagtcc tcctgtgtag gtctcccaaa gtgctagtgt 240

gtccggaatt ggtgggttct tggtctcact gacttcaaga atgaagccgc ggaccctcgc 300

agtctgctac aatg 314

<210> 38
<211> 39
<212> DNA
<213> Homo sapiens

<400> 38
ttttctcttc tgtcaacccc acacgccttt ggcacaatg 39

<210> 39
<211> 335
<212> DNA
<213> Homo sapiens

<400> 39

ctccctctag cgccttcccc ccggcccgac tccgctggtc agcgccaagt gacttacgcc 60

cccgaccctg agcccggacc gctaggcgag gaggatcaga tctccgctcg agaatctgaa 120

ggtgccctgg tcctggagga gttccgtccc agcccgcggt ctcccggtac tgtcgggccc 180

cggccctctg gagcttcagg aggcggccgt cagggtcggg gagtatttgg gtccggggtc 240

tcagggaagg gcggcgcctg ggtctgcggt atcggaaaga gcctgctgga gccaagtagc 300

cctccctctc ttgggacaga cccctcggtc ccatg 335

<210> 40
<211> 98
<212> DNA
<213> Homo sapiens

<400> 40

ctccctccct ctttccctca cagccgacga ggcaacaatt aggctttggg gataaaacga 60

ggtgcggaga gcgggctggg gcatttctcc ccgagatg 98

<210> 41
<211> 41
<212> DNA
<213> Homo sapiens

<400> 41
cactcttctg gtccccacag actcagagag aacccaccat g 41

<210> 42
<211> 84
<212> DNA
<213> Homo sapiens

<400> 42

cttcctctga tccgggccgg gcgggaagtc gggtcccgag gctccggctc ggcagaccgg 60

gcggaaagca gccgagcggc catg 84

<210> 43
<211> 48
<212> DNA
<213> Homo sapiens

<400> 43
cggcctttcc agggccgggg aaccccagga ggaagctgct gagccatg 48

<210> 44
<211> 166
<212> DNA
<213> Homo sapiens

<400> 44

cactctcttt acagtcagcc ttctgcttgc cacagtcata gtgggcagtc agtgaatctt 60

ccccaagtgc tgacaattaa tacctggttt agcggcaaag attcagagag gcgtgagcag 120

cccctctggc cttcagacaa aatctacgt accatcagaa actatg 166

<210> 45
<211> 132
<212> DNA
<213> Homo sapiens

<400> 45

tctcctttta cacaaatagc cccggatatc tgtgttacca gccttgtctc ggccacctca 60

aggataatca ctaaattctg ccgaaaggac tgaggaacgg tgcctggaaa agggcaagaa 120

tatcacggca tg 132

<210> 46
<211> 59
<212> DNA
<213> Homo sapiens

<400> 46
tggtcccttt agggctccgg atatctttgg tgacttgtcc actccagtgt ggcatcatg 59

<210> 47
<211> 87
<212> DNA
<213> Homo sapiens

<400> 47

aaacctcttc gaggcacaag gcacaacagg ctgctctggg attctcttca gccaatcttc 60

attgctcaag tgtctgaagc agccatg 87

<210> 48
<211> 209
<212> DNA
<213> Homo sapiens

<400> 48

ctgtctctct tcatttaagc acgactctgc agaaggaaca aagcaccctc cccactgggc 60

tcctggttgc agagctccaa gtcctcacac agatacgcct gtttgagaag cagcgggcaa 120

gaaagacgca agcccagagg ccctgccatt tctgtgggct caggtcccta ctggctcagg 180

cccctgcctc cctcggcaag gccacaatg 209

<210> 49
<211> 185
<212> DNA
<213> Homo sapiens

<400> 49

agccctctgc cctttctgag cccgagggac tgccacctcc actgtgtgca cactcagcta 60

cgggacacat ttcaggtatc caaggcagca gaggtgagtg ggtcccccga gctctgtgac 120

cttatgctcc acactaactc tggcagagcc tccgtttcct catagaacaa agaacagcca 180

ccatg 185

<210> 50
<211> 111
<212> DNA
<213> Homo sapiens

<400> 50

tgccctcctt ccctgtctct gcctctccct cccttcctca ggcatcagag cggagacttc 60

agggagacca gagcccagct tgccaggcac tgagctagaa gccctgccat g 111

<210> 51
<211> 72
<212> DNA
<213> Homo sapiens

<400> 51

ccgcccctcc cgcggccccg cccctcccgc ggcccgtcag cctctgccgc ggagctgcgt 60

ccgccactca tg 72

<210> 52
<211> 123
<212> DNA
<213> Homo sapiens

<400> 52

cttcctttaa aatcctataa aatcagaagc ccaagtctcc actgccagtg tgaaatcttc 60

agagaagaat ttctctttag ttctttgcaa gaaggtagag ataaagacac tttttcaaaa 120

atg 123

<210> 53
<211> 60
<212> DNA
<213> Homo sapiens

<400> 53
ttttctctct gattctccag cgacaggacc cggcgccggg cactgagcac cgccaccatg 60

<210> 54
<211> 110
<212> DNA
<213> Homo sapiens

<400> 54

ctttctctgt ctgccagggt ctccgactgt cccagacggg ctggtgtggg cttgggatcc 60

tcctggtgac ctctcccgct aaggtccctc agccactctg ccccaagatg 110

<210> 55
<211> 151
<212> DNA
<213> Homo sapiens

<400> 55

ccctccttcg cgctctctcg ctccctgccg ccgcccgcag ggctgcgggg ctcggtggca 60

tctcccgggc gcggcccgca gtccttgccc ctgcctccgg gccgctcccg cccccggcgc 120

cgctcgctcc cccgacccgg actcccccat g 151

<210> 56
<211> 85
<212> DNA
<213> Homo sapiens

<400> 56

gtgcctctgt ggccgcaggc gcaggcccgg gcgacagccg agacgtggag cgcgccggct 60

cgctgcagct ccgggactca acatg 85

<210> 57
<211> 71
<212> DNA
<213> Homo sapiens

<400> 57

gcttctctcg ttccgtcgat tgggaggagc ggtggcgacc tcggccttca gtgtttccga 60

cggagtgaat g 71

<210> 58
<211> 234
<212> DNA
<213> Homo sapiens

<400> 58

```
atctctctct tctcctggcg ctcgcgtgcg agagggaact agcgagaacg aggaagcagc        60
tggaggtgac gccgggcaga ttacgcctgt cagggccgag ccgagcggat cgctgggcgc       120
tgtgcagagg aaaggcggga gtgcccggct cgctgtcgca gagccgagcc tgtttctgcg       180
ccggaccagt cgaggactct ggacagtaga ggccccggga cgaccgagct gatg             234
```

<210> 59
<211> 67
<212> DNA
<213> Homo sapiens

<400> 59

```
aattctcctg gcggcctccg ttcagacgcg gcagctgtga cccacctgcc tcctccgcag        60
agcaatg                                                                  67
```

<210> 60
<211> 493
<212> DNA
<213> Homo sapiens

<400> 60

```
tcccctcttt ccccaacctc ctccctctct tctactccac ccctccgttt tcccactccc        60
cactgactcg gatgcctgga tgttctgcca ccgggcagtg gtccagcgtg cagccgggag       120
ggggcagggg cagggggcac tgtgacagga agctgcgcgc acaagttggc catttcgagg       180
gcaaaataag ttctcccttg gatttggaaa ggacaaagcc agtaagctac ctcttttgtg       240
tcggatgagg aggaccaacc atgagccaga gcccgggtgc aggctcaccg ccgccgctgc       300
caccgcggtc agctccagtt cctgccagga gttgtcggtg cgaggaattt tgtgacaggc       360
tctgttagtc tgttcctccc ttatttgaag gacaggccaa agatccagtt tggaaatgag       420
agaggactag catgacacat tggctccacc attgatatct cccagaggta cagaaacagg       480
attcatgaag atg                                                          493
```

<210> 61
<211> 864
<212> DNA
<213> Homo sapiens

<400> 61

```
ggttcctttg gggtgatcaa agagggagac acagacacag agagacaaag gcaaggagga        60
ctgtctggga gccacgcggg cgatacagtt tccgaggcac gccgcgtccc gcctagcctg       120
ttgaacaggt agacatgagc gacccaagct gcggatttgc gaggcgcgcc ctggagctgc       180
tagagatccg gaagcacagc cccgaggtgt gcgaagccac caagtcaagt tcctaacgag       240
tcttcagagg aggcagcagg aagctcagag agctgcaaag caaccgtgcc catctgtcaa       300
gacattcctg agaagaacat acaagaaagt cttcctcaaa gaaaaaccag tcggagccga       360
gtctatcttc acactttggc agagagtatt tgcaaactga ttttcccaga gtttgaacgg       420
ctgaatgttg cacttcagag aacattggca aagcacaaaa taaaagaaag caggaaatct       480
ttggaaagag aagactttga aaaaacaatt gcagagcaag cagttgcagc aggagttcca       540
gtggaggtta tcaaagaatc tcttggtgaa gaggttttta aaatatgtta cgaggaagat       600
gaaaacatcc ttggggtggt tggaggcacc cttaaagatt ttttaaacag cttcagtacc       660
cttctgaaac agagcagcca ttgccaagaa gcaggaaaaa ggggcaggct tgaggacgcc       720
tccattctat gcctggataa ggaggatgat tttctacatg tttactactt cttccctaag       780
agaaccacct ccctgattct tcccggcatc ataaaggcag ctgctcacgt attatatgaa       840
acggaagtgg aagtgtcgtt aatg                                              864
```

<210> 62
<211> 111
<212> DNA
<213> Homo sapiens

<400> 62

```
ggctccttcc gctccgcgac tgcgttaact ggagccaggc tgagcgtcgg cgccggggtt        60
cggtggcctc tagtgagatc tggaggatcc aaggattctg tagctacaat g                111
```

<210> 63
<211> 55
<212> DNA
<213> Homo sapiens

<400> 63
aggtctctgc ggcgcggtcc tcggagacac gcggcggtgt cctgtgttgg ccatg 55

<210> 64
<211> 140
<212> DNA
<213> Homo sapiens

<400> 64

acttcctttc ccggcgtgca ccgcgaatcc ctcctcctct tctttacctc tctccctcct 60

cctcaggttc tctatcgacg agtctggtag ctgagcgttg ggctgtaggt cgctgtgctg 120

tgtgatcccc cagagccatg 140

<210> 65
<211> 198
<212> DNA
<213> Homo sapiens

<400> 65

ccttctttaa taagcaggag cgaaaaagac aaattccaaa gaggattgtt cagttcaagg 60

gaatgaagaa ttcagaataa ttttggtaaa tggattccaa tatggggaat aagaataagc 120

tgaacagttg acctgctttg aagaaacata ctgtccattt gtctaaaata atctataaca 180

accaaaccaa tcaaaatg 198

<210> 66
<211> 49
<212> DNA
<213> Homo sapiens

<400> 66
cggcccctct gttgtcgttt ggcagcggat agaggacacg accaagatg 49

<210> 67
<211> 113
<212> DNA
<213> Homo sapiens

<400> 67

cttccctttc ctgttaggcg agagctgcga aaggcgagag ctgcgaaggg ccaggtgtcg 60

ggcgctgttt ctcgttttca tcatatagac aaaacagccc tgctgcaaag atg 113

<210> 68
<211> 326
<212> DNA
<213> Homo sapiens

<400> 68

gcttcttctc acgccgggag caggctcccg cctcgcaccg ctgccccgcg agcagctcct 60

cttctcccga ggcgcgcggg gcgccccgc gagccccgcg gctgagaccc cgcagcctgg 120

aggagggctg tccggggctt tggatgctgc tgctaggggt ggtgggagca gccgtgggac 180

gcgtggccgg gagcggggt gacagcctgg gattccgggg gcttctcttc cttgtcctcc 240

tcctctcctc tctattccca gtgtggccgt ggctgacact aaagactttg tagccatcaa 300

cccgagtgca gtttcgatgg aaaatg 326

<210> 69
<211> 342
<212> DNA
<213> Homo sapiens

<400> 69

ccgcctcttt cattgaagaa atttaagttc gtgtggtttt accttttccg ggagtctcca 60

gctggccctc atttgtgtcc ggagctcagg agttcccaaa ccgactcagt cgcaccaagt 120

ttccgtcttt tggaattggg gaaggagttt ctttctttct tttctttttt cttgagccag 180

ttttaatcgc tttgaataaa tactccctta agtagttaaa tataggagga gaaagaatac 240

atcggttgtt aaagcaggag aggaagagag acctgccctg tagcgtgact cctctagaaa 300

aaaaaaaaaa aagccggagt attttactaa gcccctaaaa tg 342

<210> 70
<211> 186
<212> DNA
<213> Homo sapiens

<400> 70

cctcctcctg ctcctgcctt ggctcctccg ccgcgcgtct cgcactccga gagccgcagc 60

ggcagcggcg cgtcctgcct gcagagagcc aggccggaga agccgagcgg cgcagaggac 120

gccagggcgc gcgccgcagc cacccaccct ccggaccgcg gcagctgctg acccgccatc 180

gccatg 186

<210> 71
<211> 214
<212> DNA
<213> Homo sapiens

<400> 71

ctgtctttat ggaccagtag gcagagcgaa attgacgctg acaagacttt tgcatcttgg 60

aagggactgt aatctactgt agtgaagaac agagcctctc aatcagacgg gtgtaaataa 120

gagacggagg ggagtccaaa agaaaaggaa gaggaggaaa aacaagtgtg tgttgggggg 180

aacaggggga aaagcatttt tggtggatgg tatg 214

<210> 72
<211> 261
<212> DNA
<213> Homo sapiens

<400> 72

```
gctcctttaa gcgtccacag gcggcggagc ggccacaatc acagctccgg gcattggggg      60
aacccgagcc ggctgcgccg gggaatccg tgcgggcgcc ttccgtcccg gtcccatcct      120
cgccgcgctc cagcacctct gaagttttgc agcgcccaga aaggaggcga ggaaggaggg     180
agtgtgtgag aggagggagc aaaaagctca ccctaaaaca tttatttcaa ggagaaaaga     240
aaaagggggg gcgcaaaaat g                                               261
```

<210> 73
<211> 97
<212> DNA
<213> Homo sapiens

<400> 73

```
ttttctcttt caaactgccc agacggttgg acaggacgta gacacacaga agaaaagaag      60
acaaagaacg ggtaggaaaa ttaaaaaggt taccatg                               97
```

<210> 74
<211> 97
<212> DNA
<213> Homo sapiens

<400> 74

```
cagcctcttt tgccggtatt cagtgaagaa agcaagtcta aatatgcagt tctctcactg      60
gagtgaaaga tgttttgttc atttctaatc aactatg                               97
```

<210> 75
<211> 472
<212> DNA
<213> Homo sapiens

<400> 75

```
ccgcctctcg gcgagcccgc cctcttctga agaggcgttt ctggaccact gagccccgcc      60

tcccactgtg agcggaaccc taccgttttt aaaaaaatct ttttcaaaac ttgccaggtt     120

gtctttccaa atatttttaa taatagtgct gctgctgtag accacagaga aaagaatccc     180

tcgctcttcc ttttcactta gtagaaactt ctaccgcgta ggtcccgcca ggagttcgcg     240

catgcgcagg agcgacaata agatggcggt gataatcgcc gcactttttt tcaaattagt     300

ggatcccaga aatcattgcg cgcatttgta acgaatttcc gttcgagttt gtattttagg     360

cgccattttc gagtgaagga cccggagccg aaacaccggt aggagcgggg aggtgggtac     420

tacacaaccg tctccagcct tggtctgagt ggactgtcct gcagcgacca tg              472
```

<210> 76
<211> 79
<212> DNA
<213> Homo sapiens

<400> 76

```
tggccttccc ggcggctgat tcgagggctt gtttggtcag aagggggcg tcagagaagc        60

tgccccttag ccaaccatg                                                    79
```

<210> 77
<211> 141
<212> DNA
<213> Homo sapiens

<400> 77

```
gagtctttcg gcctgggtgg aggacgcggc tgcttcaagt ccttggctct gatccaggcc      60

acagattcca ggattctaca ggcaggaaac atcttagaaa tcagggttgg gcaggcagga     120

gccaggagag tagctacaat g                                               141
```

<210> 78
<211> 217
<212> DNA
<213> Homo sapiens

<400> 78

```
tatccttttt tactgcagat ttactttaag gctcatattc tccaagtcta ttctgcttta      60

aaaagaagac aagaaaagaa gtggtttatc aaaatcacgt tataatcaga ttttgaccaa     120

gcattttgta agtatacaaa tgtcagccaa tgacatataa caaccatttc ttataaaacc     180

ttgatgttca aaagcctgac tagcagtggc atccatg                              217
```

<210> 79
<211> 181
<212> DNA

<213> Homo sapiens

<400> 79

tgctcttttc gatccgggac ggccggtcag gctcgccgcc gagctggaga actacgatga 60

cccgcacaaa acccctgcct ccccagttgt ccacatcagg ggcctgattg acggtgtggt 120

ggaagcagac cttgtggagg ccttgcagga gtttggaccc atcagctatg tggtggtaat 180

g 181

<210> 80
<211> 303
<212> DNA
<213> Homo sapiens

<400> 80

cattcttccg ggtccagaag gtgatctccg cccgtgctca gaatccaggg gcccggggct 60

gtagattcct tgacaaggat atcctagcgg cgaaacaaca ccgtactggg agtcagaacg 120

tctgggttct agtcttgact gccattaact agcggtatga cattggagaa gctttttga 180

cccttctgga tttccgtttc cttttctgta aaatgaggag cttggaagat ccggaaaatg 240

aggcccatag gaaacaagtg acttgctgag tccagataac actgactgtc agagagaaac 300

atg 303

<210> 81
<211> 289
<212> DNA
<213> Homo sapiens

<400> 81

tggcctcctc ttgccacgag gtcagacggc gagttcttag agaaaaaggc tgcttagctg 60

ctgcttatca tgtaacctca aaaggaaact gatcgtcttt ctcatgctgt cacgtacttg 120

ggttattatc gctgattaca gctggaaaca attgatttgc tcttacgtat ttgtgtgact 180

tgactcttca aacacaaagg ttaacaggaa gatctcgagg gccctggctg aacttcacct 240

tttggctttc ttggcctgat gctgaactct cgaggttgag ccccatatg 289

<210> 82
<211> 172
<212> DNA
<213> Homo sapiens

<400> 82

atccctcccc ggactccggc tccggctccg attgcaattt gcaacctccg ctgccgtcgc 60

cgcagcagcc accaattcgc cagcggttca ggtggctctt gcctcgatgt cctagcctag 120

gggcccccgg gccggacttg gctgggctcc cttcaccctc tgcggagtca tg 172

<210> 83
<211> 24
<212> DNA
<213> Homo sapiens

<400> 83
ccgcctcctc gggagagata aatg 24

<210> 84
<211> 221
<212> DNA
<213> Homo sapiens

<400> 84

gcgccccttt gtgcgtcacg ggtggcgggc gcgggaaggg gatttggatt gttgcgcctc 60

tgctctgaag aaagtgctgt ctggctccaa ctccagttct ttcccctgag cagcgcctgg 120

aacctaaccc ttcccactct gtcaccttct cgatcccgcc ggcgctttag agccgcagtc 180

cagtcttgga tccttcagag cctcagccac tagctgcgat g 221

<210> 85
<211> 222
<212> DNA
<213> Homo sapiens

<400> 85

gcgtcttttc ccccagcccc gctccaccag atccgcggga gccccactgc tctccgggtc 60

cttggcttgt ggctgtgggt cccatcgggc ccgccctcgc acgtcactcc gggacccccg 120

cggcctccgc aggttctgcg ctccaggccg gagtcagaga ctccaggatc ggttctttca 180

tcttcgccgc ccctgcgcgt ccagctcttc taagacgaga tg 222

<210> 86
<211> 143
<212> DNA
<213> Homo sapiens

<400> 86

aaccctttct gcagagtccc ggcagtgcgg gactccggta gccgcccctc cggtagccgc 60

ccctcctgcc cccgcgccgc cgccctatat gttgcccgcc gcggcctctg gggcatggag 120

cacgctgccc agccctggcg atg 143

<210> 87

<211> 99
<212> DNA
<213> Homo sapiens

<400> 87

gttccctttt ctacctgcag agcacggttc ccataagggc ggcgagatca gcctcctgtc 60

tcatctggaa gaccaccact ctggggtctc agaggaatg 99

<210> 88
<211> 50
<212> DNA
<213> Homo sapiens

<400> 88
ctatctctgt gtgtccgcgt gtgcgcccgg tccccgcctg ccgcaccatg 50

<210> 89
<211> 310
<212> DNA
<213> Homo sapiens

<400> 89

cctcctcctc cagctcttcc tgtcccgctg ttgcaacact gcctcactct tcccctccca 60

ccttctctcc cctcctctct gctttaattt tctcagaatt ctctggactg aggctccagt 120

tctggccttt ggggttcaag atcactggga ccaggccgtg atctctatgc ccgagtctca 180

accctcaact gtcaccccaa ggcacttggg acgtcctgga cagaccgagt cccgggaagc 240

cccagcactg ccgctgccac actgccctga gcccaaatgg gggagtgaga ggccatagct 300

gtctggcatg 310

<210> 90
<211> 84
<212> DNA
<213> Homo sapiens

<400> 90

aggtctctgc gcagcccagc ccgccggtcc acgccgcgca ccgctccgag ggccagcgcc 60

acccgctccg cagccggcac catg 84

<210> 91
<211> 50
<212> DNA
<213> Homo sapiens

<400> 91
tcgcctttgc cgatccgccg cccgtccaca cccgccgcca gctcaccatg 50

<210> 92

<211> 107
<212> DNA
<213> Homo sapiens

<400> 92

ggctccttct tcctctgcat gtggctggcg gccgcagagc agttcagttc gctcactcct 60

cgccggccgc ctctccttcg ggctctcctc gcgtcactgg agccatg 107

<210> 93
<211> 52
<212> DNA
<213> Homo sapiens

<400> 93
cgttctctgc ctggcctgag gctccctgag ccgcctcccc accatcacca tg 52

<210> 94
<211> 160
<212> DNA
<213> Homo sapiens

<400> 94

gcctcctttc tttctcagcc cagacctggc cctctggaga gggttttgga gtcctgggta 60

ggcagggtac ctcaggcagc aggcagcaca ccttggatgt gagctgaatg gattttcaaa 120

tttcacagaa ggagcctcca tgctggagaa agtatgtatg 160

<210> 95
<211> 126
<212> DNA
<213> Homo sapiens

<400> 95

cggcctccct ttagctgcca tcttgcgtcc ccgcgtgtgt gcgcctaatc tcaggtggtc 60

cacccgagac cccttgagca ccaaccctag tcccccgcgc ggccccttat tcgctccgac 120

aagatg 126

<210> 96
<211> 43
<212> DNA
<213> Homo sapiens

<400> 96
ttgtcctttg catctgcacg tgttcgcagt cgtttccgcg atg 43

<210> 97
<211> 485
<212> DNA
<213> Homo sapiens

<400> 97

tttcctttct taatatggcg atgagctctt aggccagtgt ggggaccggg gctgaggtgc 60
cctggacact ggaggagggg gagggaagga gcccctggga gcctggggta gaagtgtagg 120
aggtgggagg attccggccc gcatggagct gtcctggcct cagaaggtta tccgtctctc 180
ctgccaacca tggagacata tttagacagg accaggtggg gactgagggg tgccaatttc 240
agggggcagc tccggttccc tccccgcccc ctgctcctat tcctccacct gacccttttt 300
cccttggctc tgtcggcagt ttctccagga cccagcagtg ccctctgtcc actgctctgg 360
gccattcccc aatccccccct cccacttgag cccctaactc agaatctggg acccaggggc 420
ccctccctac cccagctaac ctcttctgga ccaggagagc caacccagat cccactacct 480
ccatg 485

<210> 98
<211> 92
<212> DNA
<213> Homo sapiens

<400> 98

gtctctctgc ccctctctgc cccaagtatt ttcagcccca gccggccaca cagctcggat 60
ctcctcctgt ggatcccccc agctctgcga tg 92

<210> 99
<211> 235
<212> DNA
<213> Homo sapiens

<400> 99

aggtctttgg ctttttttgg cggagctggg gcgccctccg gaagcgtttc caactttcca 60
gaagtttctc gggacgggca ggagggggtg gggactgcca tatatagatc ccgggagcag 120
gggagcgggc taagagtaga atcgtgtcgc ggctcgagag cgagagtcac gtcccggcgc 180
tagcccagcc cgacccaggc ccaccgtggt gcacgcaaac cacttcctgg ccatg 235

<210> 100
<211> 92
<212> DNA
<213> Homo sapiens

<400> 100

tttcctcctt tccaagagag ggctgaggga gcagggttga gcaactggtg cagacagcct 60
agctggactt tgggtgaggc ggttcagcca tg 92

<210> 101

<211> 97
<212> DNA
<213> Homo sapiens

<400> 101

gggtcccttt ctgtcccctg agcaccgtcg cctcctttcc tccagggctc cgtaggcacc 60

aactgcaagg acccctcccc ctgcgggcgc tcccatg 97

<210> 102
<211> 98
<212> DNA
<213> Homo sapiens

<400> 102

gagcctctcc tcaaagcctg gctcccacgg aaaatatgct cagtgcagcc gcgtgcatga 60

atgaaaacgc cgccgggcgc ttctagtcgg acaaaatg 98

<210> 103
<211> 380
<212> DNA
<213> Homo sapiens

<400> 103

cgctcccttc gtttccgtct cggccgggca cccgagcgca tcccgccgag gccgggccgt 60

ttcaggggga ggcgccaact catcgcggcg ccgggcccct gaccgtgcag taaccgctac 120

ccaggaggcg gagcggacaa ggctccggcc tgcgaggagt cacattaact ttgctctaga 180

agacaacttt acaaggatct aaaaggaaca ggattaaaga tgactgaata ctgggttcca 240

gaaatttaaa acaatcagct tagcaaatca tatattcttc tgtggagctg agaattgatg 300

tccgctcttc cccgtgattt ggaactttcc aatcccagag aaaagttgac aaagggactg 360

cccaggactg agtccatatg 380

<210> 104
<211> 94
<212> DNA
<213> Homo sapiens

<400> 104

cccccctca ctcgcgcgtt aggaggctcg ggtcgttgtg gtgcgctgtc ttcccgcttg 60

cgtcagggac ctgcccgact cagtggccgc catg 94

<210> 105
<211> 211

<212> DNA
<213> Homo sapiens

<400> 105

cctcctctcc tctccctctc ctctcctgct atagagggct ccgacagcag ttcccagcca 60

gcgtgttcag cctgcctgcc tgcctgcctc tgtgtgtgtg tgagcgtgtg tgcgtgcgtc 120

tactttgtac tgggaagaac acagcccatg tgctctgcat ggacgttact gatactctgt 180

ttagcttgat tttcgaaaag caggcaagat g 211

<210> 106
<211> 87
<212> DNA
<213> Homo sapiens

<400> 106

ctgcctcttc cagggcgggc ggtgtggtgc acgcattgct gtgctccaac tccctcaggg 60

cctgtgttgc cgcactctgc tgctatg 87

<210> 107
<211> 243
<212> DNA
<213> Homo sapiens

<400> 107

cctcctcctt ctcccgccct cctcgccgat ccgggcggtg ctggcagccg gagcggcggc 60

gggcgggccg agcagccggg gcagccgcgc gtgggcatcc acgggcgccg agcctccgtc 120

cgtgtctcta tccctcccgg gcctttgtca gcgcgcccgc tgggagcggg gccgagagcg 180

ccggttccag tcagacagcc ccgcaggtca gcggccgggc cgagggcgcc agagggggcc 240

atg 243

<210> 108
<211> 506
<212> DNA
<213> Homo sapiens

<400> 108

ttgcctttgg ctgggtgcaa cttccatttt aggtgttgga tctgaggggg aaaaaaaaga 60
gagagggaga gagagagaaa gaagagcagg aaagatcccg aaaggaggaa gaggtggcga 120
aaaatcaact gccctgctgg atttgtcttt ctcagcacct tggcgaagcc ttgggtttct 180
ttcttaaagg actgattttt agaactccac atttgaggtg tgtggctttt gaagaaaatg 240
tatgtactga cgggaaaagg aggataagca agtcgaattt ttgtcttacg ctctctcctt 300
cctgcttcct ccttgctgtg gtggctggga tgcttcttcc atgatttttt gaatctagac 360
tgggctgttc tctgtgttaa accaatcagt tgcgaccttc tcttaacagt gtgaagtgag 420
ggggtctctc tccctccttc tccttcctct gtgattcacc ttccttttta ccctgccctg 480
cggcggctcc gccccttacc ttcatg 506

<210> 109
<211> 528
<212> DNA
<213> Homo sapiens

<400> 109

ccgtcccctc cagcccagct cgggctccag ctccagcgcc ggcgcttcag ctgcgaccgc 60
gagccctctc aagcaagata taacttcccc aagtcacaca gtggtatcag agctaagaat 120
gggacccaga tatgactgat ctagttctgt tccaaaaccg tgctgtatta tattaacgcc 180
taccctctga agaggtccaa gcaacggaag tactactacg aagctgcctt tctggccatc 240
cttgagaaaa atagacagat gagttcctgc cagtgagtcc ctaggcctcc atctctctcc 300
cttgctgtac caccttcacc accatccatg cgaccccaag agccttaatg actctagaag 360
agactccagg caggggaagc tgaaaggacc tttcactccc tacttttggc cagggccttc 420
tgtgccacct gccaagacca gcaggcctac cctctgaaga ggtccaagca acggaagtac 480
tactacgaag ctgcctttct ggccatcctt gagaaaaata gacagatg 528

<210> 110
<211> 163
<212> DNA
<213> Homo sapiens

<400> 110

cgatctttct ggagccgcac ctccacgcgg agtccgagcg cgtgtgctga gaccccaggg 60
tcgggagggc ggagactggg agggagggag aagccccttt ggcctgcctt acggaagcct 120
gcgagggagg gtggtgtcca ctgcccagtt ccgtgtcccg atg 163

<210> 111
<211> 175

<212> DNA
<213> Homo sapiens

<400> 111

agttcttctc gatcgtgtca gtttgtaagg cgagggcgga agttggattc ctggcctgag 60

aatattaggc gtagttttcc agtttttggc aaagcggaaa tacttaaggc ccctgggttg 120

actgggttct ttgttttatc taccggcttc tgctttacga caggtcacaa acatg 175

<210> 112
<211> 97
<212> DNA
<213> Homo sapiens

<400> 112

tttcctcccc atcctgtcgc ggctcgaaag gaatggaaaa tggcggccta gacgcggagt 60

ttcctgcccg acccgcggcg gctccggcgg cgccatg 97

<210> 113
<211> 247
<212> DNA
<213> Homo sapiens

<400> 113

ctctctcttt tttttccgcc ctagctgggg ctgtgttgga ggagaggaag aaagagagac 60

agaggattgc attcatccgt tacgttcttg aaatttccta atagcaagac cagcgaagcg 120

gttgcaccct tttcaatctt gcaaaggaaa aaaacaaaac aaaacaaaaa aaacccaagt 180

ccccttcccg gcagtttttg ccttaaagct gccctcttga aattaatttt ttcccaggag 240

agagatg 247

<210> 114
<211> 107
<212> DNA
<213> Homo sapiens

<400> 114

tgttctcttt ggcttccggg cgcacgctac tctgtcgccg ccgtcagacc ggaattgccg 60

gtgccgccgc caccgctgtc tgtgcgccca cctctgctgc taccatg 107

<210> 115
<211> 70
<212> DNA
<213> Homo sapiens

<400> 115

cgttcttttt cgcaacgggt ttgccgccag aacacaggtg tcgtgaaaac tacccctaaa 60

agccaaaatg 70

<210> 116
<211> 89
<212> DNA
<213> Homo sapiens

<400> 116

tctcctcttt cccctccct tctctcccgg gcggcttact ttgcggcagc gccgagaacc 60

ccaccccctt tctttgcgga atcaccatg 89

<210> 117
<211> 31
<212> DNA
<213> Homo sapiens

<400> 117
atttccttcc tcttttggca acatggcggg c 31

<210> 118
<211> 35
<212> DNA
<213> Homo sapiens

<400> 118
gggtcttttg cgttctcttt ccctctccca acatg 35

<210> 119
<211> 40
<212> DNA
<213> Homo sapiens

<400> 119
tattcttttg aagattcttc gttgtcaagc cgccaaagtg 40

<210> 120
<211> 141
<212> DNA
<213> Homo sapiens

<400> 120

cttcccctca gtgcggtcac atacttccag aagagcggac cagggctgct gccagcacct 60

gccactcaga gcgcctctgt cgctgggacc cttcagaact ctctttgctc acaagttacc 120

aaaaaaaaaa gagccaacat g 141

<210> 121
<211> 86
<212> DNA
<213> Homo sapiens

<400> 121

```
cgctcttttc cacgtgcgaa agccccggac tcgtggagtt gtgaacgccg cggactccgg      60
agccgcacaa accagggctc gccatg                                           86
```

<210> 122
<211> 311
<212> DNA
<213> Homo sapiens

<400> 122

```
ctgtcccttg ctccaggcgc tcactttgcg ggcggcactt tttccaggtt gttaatccag      60
ctaatggaga aggatagatg cacgctactt ggtttagaaa aaaaaacaaa aatgagcaaa     120
cgagacgccc cttccgtttt atgataacta agctgcaggg aaataaatcg gctggcccta     180
ctgcaatcta ctgcactcga gaaacatcac agaaaattct ttgatttatc ttaatagtga     240
caagtgagcc tgcttctgtc aattactgaa gctataagga gattttttaa aaattaaact     300
tcaacacaat g                                                          311
```

<210> 123
<211> 212
<212> DNA
<213> Homo sapiens

<400> 123

```
ccgcctctgt tcaggacact gggtcccctt ggagcctccc caggcttaat gattgtccag      60
aaggcggcta taaagggagc ctgggaggct gggtggagga gggagcagaa aaaacccaac     120
tcagcagatc tgggaactgt gagagcggca agcaggaact gtggtcagag gctgtgcgtc     180
ttggctggta gggcctgctc ttttctacca tg                                   212
```

<210> 124
<211> 219
<212> DNA
<213> Homo sapiens

<400> 124

```
agccctcccc tcctcgctcc ctcccctcct ctccccgccc agttcttctc ttcccgtctg      60
aggtggcggt cggtctcgcc ttgtcgccag ctccatttc ctctctttct cttccccttt      120
ccttcgcgcc caagagcgcc tcccagcctc gtagggtggt cacggagccc ctgcgccttt     180
tccttgctcg ggtcctgcgt ccgcgcctgc cccgccatg                            219
```

<210> 125
<211> 30

<212> DNA
<213> Homo sapiens

<400> 125
cacccttctt aaagcggcgg cgggaagatg 30

<210> 126
<211> 190
<212> DNA
<213> Homo sapiens

<400> 126

ggccctccct gcacggcctc ccgtgcgccc ctgtcagact gtggcggccg gtcgcgcggt 60

gcgctctccc tccctgcccg cagcctggag aggcgcttcg tgctgcacac ccccgcgttc 120

ctgccggcac cgcgcctgcc ctctgccgcg ctccgccctg ccgccgaccg cacgcccgcc 180

gcgggacatg 190

<210> 127
<211> 246
<212> DNA
<213> Homo sapiens

<400> 127

ggccccctcc tcgcgagttg gtgccgctgc cacctccgat tccgagcttt cggcacctct 60

gccgggtggt accgagcctt cccggcgccc cctcctctcc tcccaccggc ctgcccttcc 120

ccgcgggact atcgccccca cgtttccctc agccctttc tctcccggcc gagccgcggc 180

ggcagcagca gcagcagcag cagcaggagg aggagcccgg tggcggcggt ggccggggag 240

cccatg 246

<210> 128
<211> 304
<212> DNA
<213> Homo sapiens

<400> 128

gggccttccc ggctgacggc ctgcgtgcac tgcgcttgcg cgggttgagg gcggtggctc 60

aggctcctgg aaaggaccgt ccacccctcc gcgctggcgg tgtggacgcg gaactcagcg 120

gagaaacgcg attgagagca gtgtgtggat tacactatca ctggaaaaat acgaattgag 180

aagaaggaaa agactggaag atgcagacct tggttcctgt tagtggaaac actgtaaggt 240

cccagaaatg gaaaagaaaa tgaaataaat cagcagttat gaggcagagc ctaagagaac 300

tatg 304

<210> 129
<211> 20
<212> DNA
<213> Homo sapiens

<400> 129
gttcctctct ccccaagatg 20

<210> 130
<211> 23
<212> DNA
<213> Homo sapiens

<400> 130
actccctttt ctttggcaag atg 23

<210> 131
<211> 515
<212> DNA
<213> Homo sapiens

<400> 131

gtccctctac tcagagcagc ccggagaccg ctgccgccgc tgccgctgct accaccgctg 60

ccacctgagg agacccgccg cccccccgtc gccgcctcct gcgagtcctt cttagcacct 120

ggcgtttcat gcacattgcc actgccatta ttattatcat tccaatacaa ggaaaataaa 180

agaagatacc agcgaaaaga accgcttaca cctttccgaa ttactcaagt gtctcctgga 240

aacagagggt cgttgtcccc ggaggagcag ccgaagggcc cgtgggctgg tgttgaccgg 300

gagggaggag gagttggggg cattgcgtgg tggaaagttg cgtgcggcag agaaccgaag 360

gtgcagcgcc acagcccagg ggacggtgtg tctgggagaa gacgctgccc ctgcgtcggg 420

acccgccagc gcgcgggcac cgcggggccc gggacgacgc cccctcctgc ggcgtggact 480

ccgtcagtgg cccaccaaga aggaggagga atatg 515

<210> 132
<211> 305
<212> DNA
<213> Homo sapiens

<400> 132

ttttctttat cctgcagtct ttacctcagc agaaccgcac accacagact ccctccagct 60

ctttgtgtgt ggctctctca gggtccaaca agagcaagct gtgggtctgt gagtgtttat 120

gtgtgctttt attcacttca cacttattga aaagtgtgta tgtgagaggg tggggtgtgt 180

gtgtcaaaga gagtgaggaa gagaaggaga gagagatcaa ttgattctgc agcctcagct 240

ccagcatccc tcagttggga gcttccaaag ccgggtgatc acttggggtg catagctcgg 300

agatg 305

<210> 133
<211> 151
<212> DNA
<213> Homo sapiens

<400> 133

ccgcccctta cccggcgtgc cccgcgcccg gaggcgctga cgtggccgcc gtcagagccg 60

ccatcttgtg ggagcaaaac caacgcctgg ctcggagcag cagcctctga ggtgtccctg 120

gccagtgtcc ttccacctgt ccacaagcat g 151

<210> 134
<211> 569
<212> DNA
<213> Homo sapiens

<400> 134

gcgccttttc cggcagcggc ggcggcagaa ctgggaggag gagttggagg ccggagggag 60

cccgcgctcg ggcggcggc tggaggcagc gcaccgagtt cccgcgagga tccatgacct 120

gacggggccc cggagccgcg ctgcctctcg ggtgtcctgg gtcggtgggg agcccagtgc 180

tcgcaggccg gcgggcgggc cggagggctg cagtctccct cgcggtgaga ggaaggcgga 240

ggagcgggaa ccgcggcggc gctcgcgcgg cgcctgcggg gggaagggca gttccgggcc 300

gggccgcgcc tcagcagggc ggcggctccc agcgcagtct cagggcccgg gtggcggcgg 360

cgactggaga aatcaagttg tgcggtcggt gatgcccgag tgagcggggg gcctgggcct 420

ctgcccttag gaggcaactc ccacgcaggc cgcaaaggcg ctctcgcggc cgagaggctt 480

cgtttcggtt tcgcggcggc ggcggcgttg ttggctgagg ggacccggga cacctgaatg 540

cccccggccc cggctcctcc gacgcgatg 569

<210> 135
<211> 187
<212> DNA
<213> Homo sapiens

<400> 135

cgccctcccg ccgccgcccg ccctcgctct ctcgcgctac cctcccgccg cccgcggtcc 60

tccgtcggtt ctctcgttag tccacggtct ggtcttcagc tacccgcctt cgtctccgag 120

tttgcgactc gcggaccggc gtccccggcg cgaagaggct ggactcggat tcgttgcctg 180

agcaatg 187

<210> 136
<211> 152
<212> DNA
<213> Homo sapiens

<400> 136

cggcctcccg ctctcacttc cttctcgagc ccggagccgc tgccgccgcc cccagctccc 60

ccgcctcggg gagggcacca ggtcactgca gccagagggg tccagaagag agaggaggca 120

ctgcctccac tacagcaact gcacccacga tg 152

<210> 137
<211> 46
<212> DNA
<213> Homo sapiens

<400> 137
ctctcttccc actcgggttt gacctacagc cgcccgggag aagatg 46

<210> 138
<211> 194
<212> DNA
<213> Homo sapiens

<400> 138

ccacctctgt ctgctgccgg cagaaagcca caagccatga aaactgattg agatgagaag 60

aattcatctg ggactggctt ttgctttagg atggtgttgg aagttgctcg ttgtcgctag 120

gagcctgctc cactgtaagg gtgtcaggat ctgaagagct atggtgaaac accactgaag 180

cattgccaag gatg 194

<210> 139
<211> 323
<212> DNA
<213> Homo sapiens

<400> 139

gcatctcttc gcctctcgga gctggaaatg cagctattga gatcttcgaa tgctgcggag 60

ctggaggcgg aggcagctgg ggaggtccga gcgatgtgac caggccgcca tcgctcgtct 120

cttcctctct cctgccgcct cctgtctcga aaataacttt tttagtctaa agaaagaaag 180

acaaaagtag tcgtccgccc ctcacgccct ctcttcctct cagccttccg cccggtgagg 240

aagcccgggg tggctgctcc gccgtcgggg ccgcgccgcc gagccccagc cgccccgggc 300

cgccccccgca cgccgccccc atg 323

<210> 140
<211> 80
<212> DNA
<213> Homo sapiens

<400> 140

cggcctctag tcatcgccct cgcagcggcg gccaacatca ccgccactgc cacccctccc 60

agactgtgga cgggaggatg 80

<210> 141
<211> 131
<212> DNA
<213> Homo sapiens

<400> 141

aggcctcttg gttctgcggc acgtgacggt cgggccgcct ccgcctctct ctttactgcg 60

gcgcggggca aggtgtgcgg gcgggaaggg gcacgggcac ccccgcggtc cccgggaggc 120

tagagatcat g 131

<210> 142
<211> 42
<212> DNA
<213> Homo sapiens

<400> 142
cgacctttct ctgcgcagta cggccgccgg gaccgcagca tg 42

<210> 143
<211> 108
<212> DNA
<213> Homo sapiens

<400> 143

gcgccttttt ttcccccccat acaatacaag atcttccttc ctcagttccc ttaaagcaca 60

gcccagggaa acctcctcac agttttcatc cagccacggg ccagcatg 108

<210> 144
<211> 220
<212> DNA

<213> Homo sapiens

<400> 144

cgacctcttt gcagctcgca cagctaaggg cgagggcgcc cttcggcaga agcagcaaac 60

cgccggcaag cccagcgagg agggctgccg ggtctgggc ttgggaattg gctggcaccc 120

agcggaaagg gacgtgagct gagcggcggg ggagaagagt gcgcaggtca gagggcggcg 180

cgcagcggcg ctccgcgagg tccccacgcc gggcgatatg 220

<210> 145
<211> 84
<212> DNA
<213> Homo sapiens

<400> 145

tctcctttg ctctcagatg ctgccagggt ccctgaagag ggaagacacg cggaaacagg 60

cttgcaccca gacacgacac catg 84

<210> 146
<211> 142
<212> DNA
<213> Homo sapiens

<400> 146

cttcctcttt tggggttctt cctttctctc tcagctctcc gtctctcttt ctctctcagc 60

ctctttcttt ctccctgtct cccccactgt cagcacctct tctgtgtggt gagtggaccg 120

cttaccccac taggtgaaga tg 142

<210> 147
<211> 70
<212> DNA
<213> Homo sapiens

<400> 147

gcctctctct tgctgcctag cctcctgccg gcctcatctt cgcccagcca accccgcctg 60

gagccctatg 70

<210> 148
<211> 66
<212> DNA
<213> Homo sapiens

<400> 148

cggccttttt ctagccaggc tctcaactgt ctcctgcgtt gctgggaagt tctggaagga 60

agcatg 66

<210> 149
<211> 37
<212> DNA
<213> Homo sapiens

<400> 149
cttcctttcc gcacaggggc cgccgagcgg ggccatg 37

<210> 150
<211> 502
<212> DNA
<213> Homo sapiens

<400> 150

ttccccttcc gtgggtcagg gccggtccgg tccggaacct gcagcccctt tcccagtgtt 60

ctagttcgcc cgtgacccgg aataatgagc aaggagggtg tggtgggttg aaagccatcc 120

tactttactc ccgagttaga gcatggattc agttttagtc ttaaggggga agtgagattg 180

gagattttta tttttaattt tgggcagaag caggttgact ctagggatct ccagagcgag 240

aggatttaac ttcatgttgc tcccgtgttt gaaggaggac aataaaagtc ccaccgggca 300

aaattttcgt aacctctgcg gtagaaaacg tcaggtatct tttaaatcgc gatagttttc 360

gctgtgtcag gctttcttcg gtggagctcc gagggtagct aggttctagg tttgaaacag 420

atgcagaatc caaaggcagc gcaaaaaaca gccaccgatt ttgctatgtc tctgagctgc 480

gagataatca gacagctaaa tg 502

<210> 151
<211> 502
<212> DNA
<213> Homo sapiens

<400> 151

ttccccttcc gtgggtcagg gccggtccgg tccggaacct gcagcccctt tcccagtgtt 60

ctagttcgcc cgtgacccgg aataatgagc aaggagggtg tggtgggttg aaagccatcc 120

tactttactc ccgagttaga gcatggattc agttttagtc ttaaggggga agtgagattg 180

gagattttta tttttaattt tgggcagaag caggttgact ctagggatct ccagagcgag 240

aggatttaac ttcatgttgc tcccgtgttt gaaggaggac aataaaagtc ccaccgggca 300

aaattttcgt aacctctgcg gtagaaaacg tcaggtatct tttaaatcgc gatagttttc 360

gctgtgtcag gctttcttcg gtggagctcc gagggtagct aggttctagg tttgaaacag 420

atgcagaatc caaaggcagc gcaaaaaaca gccaccgatt ttgctatgtc tctgagctgc 480

gagataatca gacagctaaa tg 502

<210> 152
<211> 153
<212> DNA
<213> Homo sapiens

<400> 152

ctacccttttt ccgctccacg gtgacctccg tgcggccggg tgcgggcgga gtcttcctcg 60

atcccgtggt gctccgcggc gcggccttgc tctcttccgg tcgcgggaca ccgggtgtag 120

agggcggtcg cggcgggcag tggcggcaga atg 153

<210> 153
<211> 122
<212> DNA
<213> Homo sapiens

<400> 153

ctttcttttc agtccttgcg caccggggaa caaggtcgtg aaaaaaaagg tcttggtgag 60

gtgccgccat ttcatctgtc ctcattctct gcgcctttcg cagagcttcc agcagcggta 120

tg 122

<210> 154
<211> 235
<212> DNA
<213> Homo sapiens

<400> 154

cagccttttc ctccaggggt gctttgtaaa cacggctgtg ctcagggctc gcgggtgacc 60

gaaaggatca tgaactagtg acctggaaag ggtactagat ggaaacttga gaaaggactg 120

cttattgata acagctaagg tattcctgga agcagagtaa ataaagctca tggcccacca 180

gctagaaagt attcttgcca tgagaaaaag aatgtgataa gttattcaac ttatg 235

<210> 155
<211> 488
<212> DNA
<213> Homo sapiens

<400> 155

agatcccttt cccagagtgc tctgcgccgt gaagaagcgg ctcccgggga ctgggggcat 60

tttgtgttgg ctggagctgg agtaacaaga tggcgtcgtc cgcggagtga caggggtccc 120

tctgggccgg agccggcggc agtggtggca gcggtatcgc cgccctagct caccgcgccc 180

cttttccagc ccgcgacgtc gccgcgcaag cgaggcagcg gcggccgccg agaaacaagt 240

ggcccagcct ggtaaccgcc gagaagccct tcacaaactg cggcctggca aaaagaaacc 300

tgactgagcg gcggtgatca ggttcccctc tgctgattct gggccccgaa ccccggtaaa 360

ggcctccgtg ttccgtttcc tgccgccctc ctccgtagcc ttgcctagtg taggagcccc 420

gaggcctccg tcctcttccc agaggtgtcg gggcttggcc ccagcctcca tcttcgtctc 480

tcaggatg 488

<210> 156
<211> 217
<212> DNA
<213> Homo sapiens

<400> 156

aagcctctcg gtctgtggca gcagcgttgg cccggccccg ggagcggaga gcgaggggag 60

gcggagacgg aggaaggtct gaggagcagc ttcagtcccc gccgagccgc caccgcaggt 120

cgaggacggt cggactcccg cggcgggagg agcctgttcc cctgagggta tttgaagtat 180

accatacaac tgttttgaaa atccagcgtg gacaatg 217

<210> 157
<211> 93
<212> DNA
<213> Homo sapiens

<400> 157

ccgcctcctc ccccgacttc cttccctgag cacggcggcg gcggggacga gcaccggcct 60

gcgcgcggag ccggcaccgg atgacccaac atg 93

<210> 158
<211> 73
<212> DNA
<213> Homo sapiens

<400> 158

ctgtcttttc gcttgtgtcc ctctttctag tgtcgcgctc gagtcccgac gggccgctcc 60

aagcctcgac atg 73

<210> 159
<211> 91
<212> DNA
<213> Homo sapiens

<400> 159

ctgtctcccc tcgccgcatc cactctccgg ccggccgcct gcccgccgcc tcctccgtgc 60

gcccgccagc ctcgcccgcg ccgtcaccat g 91

<210> 160
<211> 118
<212> DNA
<213> Homo sapiens

<400> 160

cgttccctac ttcctgtgct cttgcggaga cgcgcgcgtc ggggtttaac gcgtttctgg 60

gccgccgtaa gcccggccta ggggcagctt tgactcgaga gccggctata ggcgcatg 118

<210> 161
<211> 313
<212> DNA
<213> Homo sapiens

<400> 161

caccctttcg gatgcctccc ctagaaccct accactttcc accccttttcc gtctgttatt 60

tctcccaaac ttgcgcccgc acaggcccct ctggaacact cctgccccgt agtgcccctc 120

gtccccgctc cgtagagaaa gagcgtgcgt gccgcgcatt tctggcctgg ggagcgggtg 180

gagtaaacct gcgggaacca ttttacgaca acgtgcggct gtgcggtgtg gctgacggca 240

acgccgctgc tcttggagag gtcactccgg agacggcgtt ggttttgggg tgtggggggt 300

tggtggcact atg 313

<210> 162
<211> 711
<212> DNA
<213> Homo sapiens

<400> 162

```
ccttccctgg catctggagg gaccaccgtt gccgcgtctt cggcttccac gatctgcgtt      60
cgggctacgc ggccacggcg gcagccactg cgactcccac tgtgcctggc tctgtccata     120
ttagttccca ggcggccgtc gccgttccag cagcggcagc ggcagcggca gcggcggaca     180
tgttgtgagg cggcggcgcg ggtgtctgaa ggatggtttg gccgaggcgg cggcaacggc     240
tgctggcggc ggcggcagcg gcagcggggc ctcgggctct atagagccga gcccgctggg     300
tacccgcccg gtaccgcggc gaggccagtg cccctggatc ttgcctctgc tccgacgccg     360
ttggggacca gttaggcgac agcgcccgcc cctctgagga gacacgaagg tggttcccca     420
gccgctcaaa tttccggacc accgcgcttt cccctcctca gcctgggctg tgctctctct     480
agaatcctcg ggcccccact ttcttcccaa actcatccta aatctctcac acacgcgagt     540
gttcccagcc ctcaagccag ctgctcctcc gttcattttc tgcaccctct tcgcaaagca     600
ccccccggga tcactctccg agggcgactt tttgagaaat ctcggtggag tagtggacca     660
gagctgggga gtttttaaaa gccggggcgc gagaaacagg aaggtactat g              711
```

<210> 163
<211> 388
<212> DNA
<213> Homo sapiens

<400> 163

```
ttttcttttt cgtgcgagcc ctcgcgcgcg cgtacagtca tcccgctggt ctgacgattg      60
tggagaggcg gtggagaggc ttcatccatc ccacccggtc gtcgccgggg attggggtcc     120
cagcgagacc tccccgggag aagcagtgcc caggaggttt tctgaagccg gggaagctgt     180
gcagccgaag ccgccgccgc gccggagccc gggacaccgg ccaccctccg cgccacccac     240
cctcgccggc tccggcttcc tctggcccag gcgccgcgcg gacccggcag ctgtctgcgc     300
acgccgagct ccacggtgaa aaaaaagtga aggtgtaaaa gcagcacaag tgcaataaga     360
gatatttcct caaatttgcc tcaagatg                                        388
```

<210> 164
<211> 104
<212> DNA
<213> Homo sapiens

<400> 164

cagtccctct ggctgagacc tcggctccgg aatcactgca gcccccctcg ccctgagcca 60

gagcaccccg ggtcccgcca gcccctcaca ctcccagcaa aatg 104

<210> 165
<211> 90
<212> DNA
<213> Homo sapiens

<400> 165

cgttctcttc cgccgtcgtc gccgccatcc tcggcgcgac tcgcttcttt cggttctacc 60

tgggagaatc caccgccatc cgccaccatg 90

<210> 166
<211> 41
<212> DNA
<213> Homo sapiens

<400> 166
ctgtcttttc agtcgggcgc tgagtggttt ttcggatcat g 41

<210> 167
<211> 37
<212> DNA
<213> Homo sapiens

<400> 167
gtttcttttc tttgaatgac agaactacag cataatg 37

<210> 168
<211> 142
<212> DNA
<213> Homo sapiens

<400> 168

gcgcctcctc cgcccgccgc ccgggagccg cagccgccgc cgccactgcc actcccgctc 60

tctcagcgcc gccgtcgcca ccgccaccgc caccgccact accaccgtct gagtctgcag 120

tcccgagatc ccagccatca tg 142

<210> 169
<211> 105
<212> DNA
<213> Homo sapiens

<400> 169

ccgcctttta cttcggcccg cttcttctgg tcactccgcc accgtagaat cgcctaccat 60

ttggtgcaag caaaaagcaa tcagcaattg gacaggaaaa gaatg 105

<210> 170

<211> 60
<212> DNA
<213> Homo sapiens

<400> 170
cttcctcttt ctcgactcca tcttcgcggt agctgggacc gccgttcagt cgccaatatg 60

<210> 171
<211> 194
<212> DNA
<213> Homo sapiens

<400> 171

```
ctgtctcttt cgctccgcta caacaacaaa cgtgcacagg ggagtgaggg cagggcgctc     60
gcagggggca cgcagggagg gcccagggcg ccagggaggc cgcgccgggc taatccgaag    120
gggctgcgag gtcaggctgt aaccgggtca atgtgtggaa tattgggggg ctcggctgca    180
gacttggcca aatg                                                      194
```

<210> 172
<211> 183
<212> DNA
<213> Homo sapiens

<400> 172

```
gccccttttc acaatatttg attaggaatt tggggcggga ccctggtctg gcacaggcac     60
gcacactctc agtagactct ttcactcctc tctctcttcc tctctcacac gttctccaac    120
ccaaggaggc cagacagagg gacgtggtca ctctctgaaa agttcaactt gagagacaaa    180
atg                                                                  183
```

<210> 173
<211> 206
<212> DNA
<213> Homo sapiens

<400> 173

```
cgttcttcgc cgagagtcgt cggggtttcc tgcttcaaca gtgcttggac ggaacccggc     60
gctcgttccc caccccggcc ggccgcccat agccagccct ccgtcacctc ttcaccgcac    120
cctcggactg ccccaaggcc cccgccgccg ctccagcgcc gcgcagccac cgccgccgcc    180
gccgcctctc cttagtcgcc gccatg                                         206
```

<210> 174
<211> 80
<212> DNA
<213> Homo sapiens

<400> 174

cgctctctgc tcctcctgtt cgacagtcag ccgcatcttc ttttgcgtcg ccagccgagc 60

cacatcgctc agacaccatg 80

<210> 175
<211> 36
<212> DNA
<213> Homo sapiens

<400> 175
caccctctct ggacagccca gggccgcagg ctcatg 36

<210> 176
<211> 99
<212> DNA
<213> Homo sapiens

<400> 176

ctgtccttac cttcagcagg agccggttcc ctgtgtgtgt gtccgctcgc cctctgctcc 60

gtcctgcggc tgcccactgc cctcctacgg tccaccatg 99

<210> 177
<211> 176
<212> DNA
<213> Homo sapiens

<400> 177

gcgcctcttc cggttacctt ttcccagcgc cagaggcgcc tagggttggg gtcctcgctc 60

aggcacagag acccgacacc gagcggcggc ttccccggga tcgagggacg cgcacgccag 120

aggagacgaa aggaacccgg gtcggaccag atcggaacca ctgaccattg cccatg 176

<210> 178
<211> 190
<212> DNA
<213> Homo sapiens

<400> 178

cggcctcctt ctgcctaggt cccaacgctt cggggcaggg gtgcggtctt gcaataggaa 60

gccgagcgtc ttgcaagctt cccgtcgggc accagctact cggccccgca ccctacctgg 120

tgcattccct agacacctcc ggggtcccta cctggagatc cccggagccc cccttcctgc 180

gccagccatg 190

<210> 179
<211> 23
<212> DNA
<213> Homo sapiens

<400> 179
cattctcccc agaggccgag atg 23

<210> 180
<211> 71
<212> DNA
<213> Homo sapiens

<400> 180

gctcccttta gcgagtcctt cttttcctga ctgcagctct tttcattttg ccatcctttt 60

ccagctccat g 71

<210> 181
<211> 65
<212> DNA
<213> Homo sapiens

<400> 181

ctgtcctttc gtggctcact ccctttcctc tgctgccgct cggtcacgct tgctctttca 60

ccatg 65

<210> 182
<211> 62
<212> DNA
<213> Homo sapiens

<400> 182

ccgccttccc tgcgcagtcg gtgtctccgc gtcgctgggt gggacttggc tcggcggcca 60

tg 62

<210> 183
<211> 109
<212> DNA
<213> Homo sapiens

<400> 183

ctcccttctt gactctctgt tcacagaact caggctgcct ccagccagcc tttgcccgct 60

agactcactg gccctgagca cttgaaggtg cagcaagtca ctgagaatg 109

<210> 184
<211> 68
<212> DNA
<213> Homo sapiens

<400> 184

```
ctgtccctca ctcgccgccg acgacctgtc tcgccgagcg cacgccttgc cgccgccccg        60

cagaaatg                                                                 68
```

<210> 185
<211> 61
<212> DNA
<213> Homo sapiens

<400> 185

```
ccgccttttc ccctgcctgc ccttcgggca cctcaggaag gcaccttcct ctgtcagaat        60

g                                                                        61
```

<210> 186
<211> 217
<212> DNA
<213> Homo sapiens

<400> 186

```
cgtcctctgg ccgcgcctgc ggccgcacgc ccagcgcccc tcgcctaacc tcgcgcccgg        60

gccgcgcctc ctcctcctcc tgctccccgc cgcttccgtt tctcgaggga aaggctgctg       120

cctcctgctc tgtcctcatc cccggcttag ctgacggccc agagggtggg tgccaattcc       180

accagcagct gcaactgaaa agcaaggttc agaaatg                                217
```

<210> 187
<211> 186
<212> DNA
<213> Homo sapiens

<400> 187

```
gtttcctctc cttgttttgc tttcgatctg gactgttctc aggcaagccg gggagtaact        60

tttagttttg ctcctgcgat tattcaactg acgggctttc atttccattt cacataccct       120

agcaacactt ataccttgcg gaattgtatt ggtagcgtga aaaaagcaca ctgagagggc       180

accatg                                                                  186
```

<210> 188
<211> 136
<212> DNA
<213> Homo sapiens

<400> 188

ttttcttctt ttttcttctt tcttaaagcg aactgtactc ctctgctgtt cctttgaact 60

tggttcagta ggaagaagtg atatcctccc cagaccatct gctttgggga gcttggcaaa 120

actgtccagc aaaatg 136

<210> 189
<211> 351
<212> DNA
<213> Homo sapiens

<400> 189

ccgccttcct ccctccctcg ctccctccct gcgcgccgcc tctcactcac agcctccctt 60

ccttctttct ccctccgcct cccgagcacc agcgcgctct gagctgcccc cagggtccct 120

cccccgccgc cagcagccca tttggaggga ggaagtaagg gaagaggaga ggaaggggag 180

ccggaccgac tacccagaca gagccggtga atgggtttgt ggtgaccccc gcccccacc 240

ccaccctccc ttcccacccg acccccaacc cccatcccca gttcgagccg ccgcccgaaa 300

ggccgggccg tcgtcttagg aggagtcgcc gccgccgcca cctccgccat g 351

<210> 190
<211> 92
<212> DNA
<213> Homo sapiens

<400> 190

ctctccccct cctccccctc ccgctccaag attcgccgcc gccgccgccg cagccgcagg 60

agtagccgcc gccggagccg cgcgcagcca tg 92

<210> 191
<211> 64
<212> DNA
<213> Homo sapiens

<400> 191

gctcctcccg cgaatcgcag cttctgagac cagggttgct ccgtccgtgc tccgcctcgc 60

catg 64

<210> 192
<211> 145
<212> DNA
<213> Homo sapiens

<400> 192

attcccttct ttgggctcgg gggctcccgg agcagggcga gagctcgcgt cgccggaaag 60

gaagacggga agaaagggca ggcggctcgg cgggcgtctt ctccactcct ctgccgcgtc 120

cccgtggctg cagggagccg gcatg 145

<210> 193
<211> 85
<212> DNA
<213> Homo sapiens

<400> 193

ctgtcttttc cgtgctacct gcagaggggt ccatacggcg ttgttctgga ttcccgtcgt 60

aacttaaagg gaaattttca caatg 85

<210> 194
<211> 175
<212> DNA
<213> Homo sapiens

<400> 194

ttttctttcc tggctgatga tttgtcattc tagtcacttc ctgccttgtg accacacacc 60

caggcttgac aaagctgttc tgcagatcag aaagaagggg ttcctggtca tacaccagta 120

ctaccaagga cagctttttt cctgcaagat ctgttaccta aagcaataaa aaatg 175

<210> 195
<211> 72
<212> DNA
<213> Homo sapiens

<400> 195

ccatctctct cgggtggagt cttctgacag ctggtgcgcc tgcccgggaa catcctcctg 60

gactcaatca tg 72

<210> 196
<211> 128
<212> DNA
<213> Homo sapiens

<400> 196

ttctctcttt tttgcacatc tggctgaact gggagtcagg tggttgactt gtgcctggct 60

gcagtagcag cggcatctcc cttgcacagt tctcctcctc ggcctgccca agagtccacc 120

aggccatg 128

<210> 197

<211> 498
<212> DNA
<213> Homo sapiens

<400> 197

```
tgttcctttc ccctccgctt ctctgaccta gctgcgcggc cccggcccgg gagctgccga      60
acccgcgcct cccctgggtg aggaggacac gcctgccctc gtcgagaaaa cttttcctgc     120
cgactcagtt ggggcggcgg tggcaggaag tgcgggcagc gacctctcct ccgcctgccc     180
cgcgcgccct gccggaggtc ggcgctgagc ttgcgatcaa gtttgtgggg gcccccccttc    240
ccagttgccg gcgagtctcg cctcgagagg ggcgcccgac cccggggagg gcggcaggcc     300
agggcgaagg ccaagggcgt gtggtggcgc cggagactag gtgcggagca aggcggggac     360
tcgcacccgc atccgagagc gcggaggtcg cgcagcccgg gagaagggag cctccggcgg     420
ctgcttccta gagtccacag tgcgctgtct cctttggctg aggagagtgt cctggccccg     480
agtctatcga ggaaaatg                                                   498
```

<210> 198
<211> 127
<212> DNA
<213> Homo sapiens

<400> 198

```
ccgcctcttt tcccgagatg ccccggggag ggaggacaac accttcaaag acaggccctc      60
tgagtccgac gagctccaga ccatccaaga agacagtgca gccacctccg agagcctgga     120
tgtgatg                                                               127
```

<210> 199
<211> 401
<212> DNA
<213> Homo sapiens

<400> 199

```
cattcttccc aggacctcag cgcagccctg gcccaggaag gcaggagaca gaggccagga      60
cggtccagag gtgtcgaaat gtcctgggga cctgagcagc agccaccagg gaagaggcag     120
ggagggagct gaggaccagg cttggttgtg agaatccctg agcccaggcg gtagatgcca     180
ggaggtgtct ggactggctg ggccatgcct gggctgacct gtccagccag ggagagggtg     240
tgagggcaga tctgggggtg cccagatgga aggaggcagg catgggggac acccaaggcc     300
ccctggcagc accatgaact aagcaggaca cctggagggg aagaactgtg gggacctgga     360
ggcctccaac gactccttcc tgcttcctgg acaggactat g                         401
```

<210> 200
<211> 131
<212> DNA
<213> Homo sapiens

<400> 200

gggcctttcc cagtgcggcc gccgccgcca cagctgcagt cagcaccgtc accccagcag 60

catccgccgc ctgcaccgcg cgtgcggccc gccccggcct gaccccgccg ccgaacccgg 120

cgccagccat g 131

<210> 201
<211> 396
<212> DNA
<213> Homo sapiens

<400> 201

agctctctgc tcgctctgct cgcagtcaca gacacttgag cacacgcgta caccccagaca 60

tcttcgggct gctattggat tgactttgaa ggttctgtgt gggtcgccgt ggctgcatgt 120

ttgaatcagg tggagaagca cttcaacgct ggacgaagta aagattattg ttgttatttt 180

ttttttctct ctctctctct cttaagaaag gaaaatatcc caaggactaa tctgatcggg 240

tcttccttca tcaggaacga atgcaggaat ttgggaactg agctgtgcaa gtgctgaaga 300

aggagatttg tttggaggaa acaggaaaga gaaagaaaag gaaggaaaaa atacataatt 360

tcagggacga gagagagaag aaaaacgggg actatg 396

<210> 202
<211> 1338
<212> DNA
<213> Homo sapiens

<400> 202

agcccttctt ggggaagtca gctacccagc agcctgtagt cctcggctac ccaccctcac 60

cgcctggggt cccatggtga gacagctggg tgggcatcag gcttctgcag agggccaggc 120

cggagggagc tgggcgaggg agtggggctg gctcctggct tgcaccggcc tcgtggaatc 180

caggcctcag acctgatcgc tggcgaaact ggctctgtgc gctggagccc ctggtcttct 240

gcgtctgtcc tcctcccggc cagactttac tcctggctca gcgacaggta tttgctatgg 300

aagagctgtc cctccctccc ctcggtgggc ctgggtccac ctccacctcc tcttcaggtc 360

cgcaccttcc tcccctttaa aacaccagcc gggcgcagac ccgttctagg cttttccatg 420

gtgcttccgc caaagcttgt gaccgagtcc ttcccgccta gggctggtgg gcctccccctg 480

ctggtaggtc tctcttcgct ttctttactc agaactgaag ctctcattcc ccacccacca 540

aggaaaaaca aaagggaaga agccacagct ggccccggct tgctttggca caggtgtttc 600

cccccggccc cccgtcgggc accctggttc ctgttctgtc cctgccccac gcgaccctgg 660

ggctcccacc cgggctcctc agcctcccct gggttggggt ggggggactg gctcccagcc 720

cttggcctag ggtttggtga acgcctttcc tggactgcgg gcccacttca ggcgcggctc 780

caggctgggc agctgcgctg gagggccgag ggcaggggtg gggtcgggcg tccaccctca 840

gggttgcgcc agggagccgg aaagccgact cccgaagttg gggtcctggg aaaacttggg 900

tcctgggttg actgagaagc ggcggggaaa ggaggcgggc caggaggagg gggcctggcg 960

gacgccggcc ggggggcggg gcgcggcggg gctgtcggtc acgcccctca gtccgccccg 1020

ccccgccccg cctgccgggg aagggccacg ttgcccgccc ggccgtccgg ccccggcgcg 1080

ccgcagaaag ggctggcgag tcgaaaggcg aggcggccgc ggcagcgctt gggacgcgcc 1140

tgggcaccgg gctcgctccc tgcgccccgg agcaggccaa gttcggggcc aggacgtcgg 1200

gaggacctgg tgcatggctg cctcctaatc ccatagtcca gaggaggcat ccctaggact 1260

gcgggcaagg gagccgggca agcccagggc agccttgaac cgtcccctgg cctgccctcc 1320

ccggtggggg ccaggatg 1338

<210> 203
<211> 522
<212> DNA
<213> Homo sapiens

<400> 203

ctgcctcccg cccccggggc caaagtacaa agggaggagg aagaagggag cggggtcgga 60

gccgtcgggg ccaaaggaga cggggccagg aacaggcagt ctcggcccaa ctgcggacgc 120

tccctccacc ccctgcgcaa aaagacccaa ccggagttga ggcgctgccc ctgaaggccc 180

caccttacac ttggcggggg ccggagccag gctcccagga ctgctccaga accgagggaa 240

gctcgggtcc ctccaagcta gccatggtga ggcgccggag gccccggggc cccacccccc 300

cggcctgacc acactgccct gggtgccctc ctccagaagc ccgagatgcg gggggccggg 360

agacaacact cctggctccc cagagaggcg tgggtctggg gctgagggcc agggcccgga 420

tgcccaggtt ccgggactag ggccttggca gccagcgggg gtggggacca cgggcaccca 480

gagaaggtcc tccacacatc ccagcgccgg ctcccggcca tg 522

<210> 204
<211> 90

<212> DNA
<213> Homo sapiens

<400> **204**

ccgccttctc cgcagccccg caggccccgg gccctgtcat tcccagcgct gccctgtctt 60

gcgttccagt gttccagctt ctgcgagatg 90

<210> 205
<211> 562
<212> DNA
<213> Homo sapiens

<400> 205

ggccctttat aatgcgaggg tctggacggc tgaggacccc cgagctgtgc tgctcgcggc 60

cgccaccgcc gggccccggc cgtccctggc tcccctcctg cctcgagaag ggcagggctt 120

ctcagaggct tggcgggaaa aagaacggag ggagggatcg cgctgagtat aaaagccggt 180

tttcggggct ttatctaact cgctgtagta attccagcga gaggcagagg gagcgagcgg 240

gcggccggct agggtggaag agccgggcga gcagagctgc gctgcgggcg tcctgggaag 300

ggagatccgg agcgaatagg gggcttcgcc tctggcccag ccctcccgct gatcccccag 360

ccagcggtcc gcaacccttg ccgcatccac gaaactttgc ccatagcagc gggcgggcac 420

tttgcactgg aacttacaac acccgagcaa ggacgcgact ctcccgacgc ggggaggcta 480

ttctgcccat ttggggacac ttccccgccg ctgccaggac ccgcttctct gaaaggctct 540

ccttgcagct gcttagacgc tg 562

<210> 206
<211> 38
<212> DNA
<213> Homo sapiens

<400> 206
tggcctctcg gttccgcggc gcaccggagg gcagcatg 38

<210> 207
<211> 88
<212> DNA
<213> Homo sapiens

<400> 207

cttcctctcc aggaatccgc ggagggagcg caggctcgaa gagctcctgg acgcagaggc 60

cctgcccttg ccagacggcg cagacatg 88

<210> 208
<211> 29

<212> DNA
<213> Homo sapiens

<400> 208
ccttcttcct cctgcccgta gtagccatg 29

<210> 209
<211> 37
<212> DNA
<213> Homo sapiens

<400> 209
ccgtcctttc atcctggcgt ttgcctgcag caagatg 37

<210> 210
<211> 159
<212> DNA
<213> Homo sapiens

<400> 210

tgccccttcc ccggccaagc ccaactccgg atctcgctct ccaccggatc tcacccgcca 60

cacccggaca ggcggctgga ggaggcgggc gtctaaaatt ctgggaagca gaacctggcc 120

ggagccacta gacagagccg ggcctagccc agagacatg 159

<210> 211
<211> 101
<212> DNA
<213> Homo sapiens

<400> 211

gcccctcctc cgccgccggc tcccgggtgt ggtggtcgca ccagctctct gctctcccag 60

cgcagcgccg ccgcccggcc cctccagctt cccggaccat g 101

<210> 212
<211> 108
<212> DNA
<213> Homo sapiens

<400> 212

gcgtcctttc cctggtgtga ttccgtcctg cgcggttgtt ctctggagca gcgttctttt 60

atctccgtcc gccttctctc ctacctaagt gcgtgccgcc acccgatg 108

<210> 213
<211> 456
<212> DNA
<213> Homo sapiens

<400> 213

cattcctttt gtagaaaaac ccgtgcctcg aatgaggcga gactcagaga ggacccaggc 60

gcggggcgga cccctccaat tccttcctcg cgcccccgaa agagcggcgc accagcagcc 120

gaactgccgg cgcccaggct ccctggtccg gccgggatgc ggccggtacc cgctccccgc 180

cgggaacaac ctctccactc ttcctgcagg gagctggtgc cagccgacag ccgcgccagg 240

gccgctccgg gtaccagggt cggatcgggt gacgtcgcga acttgcgcct ggccgccaag 300

ccggcctcca ggctgaagaa ggacccgccc cggccttgac ccgggccccg cccctccagc 360

cggggcaccg agccccggcc ctagctgctc gcccctactc gccggcactc gcccggctcg 420

cccgctttcg cacccagttc acgcgccaca gctatg 456

<210> 214
<211> 104
<212> DNA
<213> Homo sapiens

<400> 214

gcgtcttccc gagccagtgt gctgagctct ccgcgtcgcc tctgtcgccc gcgcctggcc 60

taccgcggca ctcccggctg cacgctctgc ttggcctcgc catg 104

<210> 215
<211> 512
<212> DNA
<213> Homo sapiens

<400> 215

gcttcccctt ctccccggcg gttagtgctg agagtgcgga gtgtgtgctc cgggctcgga 60

acacacattt attattaaaa aatccaaaaa aaatctaaaa aaatctttta aaaaacccca 120

aaaaaattta caaaaaatcc gcgtctcccc cgccggagac ttttattttt tttcttcctc 180

ttttataaaa taacccggtg aagcagccga gaccgacccg cccgcccgcg gccccgcagc 240

agctccaaga aggaaccaag agaccgaggc cttcccgctg cccggacccg acaccgccac 300

cctcgctccc cgccggcagc cggcagccag cggcagtgga tcgaccccgt tctgcggccg 360

ttgagtagtt ttcaattccg gttgattttt gtccctctgc gcttgctccc cgctcccctc 420

cccccggctc cggcccccag ccccggcact cgctctcctc ctctcacgga aaggtcgcgg 480

cctgtggccc tgcgggcagc cgtgccgaga tg 512

<210> 216
<211> 270
<212> DNA
<213> Homo sapiens

<400> 216

cgctctttct ctccggtaca cacagctccc cacattcgca cccctgcccg cgcgccgggc 60

cgcctgactg cacggcttcc cctccagcca gatgctggag aacacacact gattcgctgc 120

tttccaagac cctgttcagt ctctttctct atacaaagat ttttttaaaa actatatata 180

agaattcttt atttgcaccc tccctccgag tcccctgctc cgccagcctg cgcgcctcct 240

agcaccactt ttcactccca aagaaggatg 270

<210> 217
<211> 65
<212> DNA
<213> Homo sapiens

<400> 217

gggtctttcc ctcactcgtc ctccgcgcgt cgccgctctt cggttctgct ctgtccgccg 60

ccatg 65

<210> 218
<211> 222
<212> DNA
<213> Homo sapiens

<400> 218

cgctcccctt tcccctcccg ccggacctgc caggaggtgg gctggcgcgg agggagggcc 60

ctgtcccctg tccctttaag gaggagggcc aaacgccggc ctagagtgcg gcgtagcccc 120

cacccgccgt gccctcaccc cagagcagct gcagcctcag ccggccgccc ctccgccagc 180

caagtccgcc gctctgaccc ccggcagcaa gtcgccacca tg 222

<210> 219
<211> 300
<212> DNA
<213> Homo sapiens

<400> 219

cggcctctgt gagccgcaac ctttccaagg gagtggttgt gtgatcgcca tcttagggag 60

tgagtgtggc cgggccttct cctgtggcgg gtgtggggag cggagcccag agctcctgtg 120

gggccgctgc tttggcggtg ggcccagccg ggagcagcct ctttcgaagg ccgccgtgac 180

ctcttcaagg gcgtggagac gggaaggaaa aggccccggt tggggttcca gggcgccggt 240

aacgttaacc ggcgccttgc ctgtcctcta accgtcgctc cctcctcccc tagaaagatg 300

<210> 220
<211> 437

<212> DNA
<213> Homo sapiens

<400> 220

```
cctcccettt actcctggct gcggggcgag ccgggcgtct gctgcagcgg ccgcggtggc      60
tgaggaggcc cgagaggagt cggtggcagc ggcggcggcg ggaccggcag cagcagcagc     120
agcagcagca gcagcaacca ctagcctcct gccccgcggc gctgccgcac gagccccacg     180
agccgctcac cccgccgttc tcagcgctgc ccgaccccgc tggcgcgccc tcccgccgcc     240
agtcccggca gcgccctcag ttgtcctccg actcgccctc ggccttccgc gccagccgca     300
gccacagccg caacgccacc cgcagccaca gccacagcca cagccccagg catagccttc     360
ggcacagccc cggctccggc tcctgcggca gctcctctgg gcaccgtccc tgcgccgaca     420
tcctggaggt tgggatg                                                    437
```

<210> 221
<211> 279
<212> DNA
<213> Homo sapiens

<400> 221

```
ggatctcttc gtctttgcag cgtagcccga gtcggtcagc gccggaggtg agcggtgcag      60
gaggctacgc catcagtccc caccaagggc cagtcgcccg gctagtgcgg aatcccggcg     120
cgccggccgg ccccgggcac gcaggcaggg cggcgcagga tccagggcgt ctgggatgca     180
gtggagctca gagagaggag aacggctcct cacgcctggg gcctgctctt cagaagtccc     240
cagcgccgtt ccttccagat caggacctca gcagccatg                            279
```

<210> 222
<211> 571
<212> DNA
<213> Homo sapiens

<400> 222

cggcctcctc ggcgcagcca tcctcttggc tgccgcgggc ggcaaagccc acggcatctg 60

ccatttgtca ttcagcccgt cggtaccgcc ccgagccttg atttagacac ggctggggcg 120

tgctctggcc tcactctccg ggcgggtgct ggacggacgg acggacgggg cagccgtgct 180

cacagctcag cagcgcgggg ccttggcgcg cggggcgctt ccccgggtcg ccgtcatggc 240

cgcggaggtg gcacgcccga gcggcctcgc ctgagctccg gggtcgtcg ccccgcaggg 300

attgctgtca cgtctaatgt ggctgctgcc tcgtgtcaca tctgaaactc atctgtacct 360

cacttagaaa gtggttctga ttagacaaga cttttcgttg cagtcgacag aaacctaatg 420

ggaccattga agaattccaa acaggtattt gcataggaat cagaggagtt aatcttgtct 480

cttctcacag gtttgaatct tcagacaaac ttctgggagg actcggtccc tgcctcgcag 540

cagatgttcc ctgtcactca gtaggcatat g 571

<210> 223
<211> 53
<212> DNA
<213> Homo sapiens

<400> 223
tgctctcttg gctccggaac ccccgcgggc gctggctccg tctgccaggg atg 53

<210> 224
<211> 110
<212> DNA
<213> Homo sapiens

<400> 224

ccccccttccc ggccagacgg cgggcaagac agctgggtgt acagcgtcct cgaaaccacg 60

agcaagtgag cagatcctcc gaggcaccag ggactccagc ccatgccatg 110

<210> 225
<211> 81
<212> DNA
<213> Homo sapiens

<400> 225

cagtcctttc gcgcctcggc ggcgcggcat agcccggctc ggcctgtaaa gcagtctcaa 60

gcctgccgca gggagaagat g 81

<210> 226
<211> 31
<212> DNA
<213> Homo sapiens

<400> 226
ggtccttcca cgtgctttcg gcggcgacat g 31

<210> 227
<211> 191
<212> DNA
<213> Homo sapiens

<400> 227

tgttcttctc gtggttccag tggggagaga aggaggaagt agggagcggg gtggcagggg 60

ggggacccgc cgcggctgct gccaccgccg ccaccaccgc ctctgctcgt ggcgtgggaa 120

aggaggtgtg agtcccgggc gcgagccggc ggcggcgccg ctgcgggagg gtcggcggtg 180

ggaaggcgat g 191

<210> 228
<211> 61
<212> DNA
<213> Homo sapiens

<400> 228

cggtcttcca gtttcccggc gtgcttaggg cgcgccaaat gggaggggga gacgcaagat 60

g 61

<210> 229
<211> 75
<212> DNA
<213> Homo sapiens

<400> 229

cggcctccgc cgctgccgcc gccgctgcta cagccgccgc cgccgctgtt gccgcggctt 60

gttattctta aaatg 75

<210> 230
<211> 156
<212> DNA
<213> Homo sapiens

<400> 230

ctttctttct gcagcaggaa ccgcggctgc tggacaagag gggtgcggtg gatactgacc 60

tttgctccgg cctcgtcgtg aagacacagc gcatctcccc gctgtaggct tcctcccaca 120

gaacccgttt cgggcctcag agcgtctggt gagatg 156

<210> 231
<211> 800
<212> DNA
<213> Homo sapiens

<400> 231

```
cgccccctct tcctcgccct ctctcgcggg tcggggttac atggcggcga ctgcggcaaa       60
gcgagagcct cggagacgcc gctgccgcca gcacagccgg agacctgagc cgacactggg      120
ggcagtccgc gagccccgca ctctctcgat gagtcggaga agtcccgttg tatcagagta      180
agatggacgg tagctttgat tgtgattgtg gtgagctgga gccacctgat cactaacaaa      240
agacatcttc tgttaaccaa cagccgccag ggcttcctgt tgaaataaat atatagcaac      300
aaaggaaaaa aagaagcaaa acggaaatag tgcttaccag caccttagaa tgatgctgct      360
caggaccagt ccaacactga atgtatctgc actgtgagga gaatgttcat agaagcctgt      420
tgtgtgcata tttattcaca tttttgttaa atgttaaatc gtttagcacg gtaatctgag      480
tgcacagtat gtcatttcat tccgtttgag tttcttgttt tcgttaaatg tctgcagagt      540
tgctgcccct ttcttgaact atgagtactg caatcttttt aattctcaat atgaatagag      600
ctttttgagc tttaaatcta aggggaactc gacaggcctg tttggcatat gcaatgaaca      660
tcaagaaacc atcttgctgt ggaagcataa ttatttttct tctccctttt tgaaagatct      720
ttccttttga tgccagtttt cttccttgtt tacacaagtt caatttgaaa ggaaaaggca      780
atagtaaggg tttcaaaatg                                                  800
```

<210> 232
<211> 131
<212> DNA
<213> Homo sapiens

<400> 232

```
cctccccttc cctacatcta gccgccgcgc tttcccgctc ccgcagcagc agcctcccgc       60
gtcgctgtcg ctgttgcctc cgccacctcc tccgccgccg cgcgcccctc ggagttccgc      120
gccccaccat g                                                           131
```

<210> 233
<211> 218
<212> DNA
<213> Homo sapiens

<400> 233

```
ttgcctctgg ctctgaggcg gcggcgccgg gcgctgcgaa ggctcggccg ctgtagtcag       60
tggtgtgggg tgcgcaaggg cacggacctc ggagctctcc ccgcttgcgc cgagtttctc      120
agcgccttcc ccacccaaac cggggtctcg cagtcggaag cactcagagt gcagccccgc      180
gcggggccgg tcgtaaccgc gccgcgggcc ggacgatg                              218
```

<210> 234
<211> 26
<212> DNA
<213> Homo sapiens

<400> 234
agttctttct gcccacacta gacatg 26

<210> 235
<211> 35
<212> DNA
<213> Homo sapiens

<400> 235
tgttcttctg tgccgggggt cttcctgctg tcatg 35

<210> 236
<211> 76
<212> DNA
<213> Homo sapiens

<400> 236

cagcccctct ggcaggctcc cgccagcgtc gctgcggctc cggcccggga gcgagcgccc 60

ggagctcgga aagatg 76

<210> 237
<211> 158
<212> DNA
<213> Homo sapiens

<400> 237

ctccctttgc aggacgtcac ggaggactgc aggggcctga gccgctgctg ccgccgccgc 60

cgcgcagccc cacatcaacg caccggggtc ctgtcaccgc caccgccaaa aaagtcaccg 120

ccgctagggt cgccgttgca tcggtgcagg gcaagatg 158

<210> 238
<211> 48
<212> DNA
<213> Homo sapiens

<400> 238
ctctctctct ctctctctcg ctcgttccct aacattaaag agaaaatg 48

<210> 239
<211> 174
<212> DNA
<213> Homo sapiens

<400> 239

gcgcccctct gctccggctc ggggcgggca ctggcggagg gactggccag tcccctcctc 60

cgcgccggcc ccaaccctgt cgctgccgcc gcgctccgag tccccattcc cgagctgccg 120

ctgttgtcgc tcgctcagcg tctccctctc ggccgccctc tcctcgggac gatg 174

<210> 240
<211> 246
<212> DNA
<213> Homo sapiens

<400> 240

ccgcctttc ccgcggaggc gccgagcggc catattgcgg agctgtctgc ggtggcggcg 60

gcgcctctcg tctcccgcgg cccagcgctc gcaccaccgc ttctccctcc ctgtcgcagc 120

cgcgccgccg cgcagcgccc cagccacacg ccggcgggca gaagccgccc gctctccgga 180

aagtgataac agaattcatt gaagtggaga atttttaaag aaggtaacaa aaagagaaag 240

aaaatg 246

<210> 241
<211> 120
<212> DNA
<213> Homo sapiens

<400> 241

tcgccttctt gcacttcgcg ggagaagttg ttggcgcgaa tggatcctga gcctcgataa 60

cagattcctc aaccggccca cccgccagcc agccagcgcc ttcatcctgg ggctgcgatg 120

<210> 242
<211> 284
<212> DNA
<213> Homo sapiens

<400> 242

gcatctttct cgaggagctc tcctgggcgg ctgaagaagg agcttcttct ccggagtgcg 60

ccggcggtgg cgcctgcgga cctaactagc tccaggttag gccgagcttt gcgggaaagc 120

agcggacttg aaaatactgg aaatctgtcc ggatccaaat tattttgcaa gccagatgag 180

taaccagagg gcatgaaagg ttgagaacat ttgacttccc tgcaaacctt ggtatagatc 240

acttcctttt ctgtaggaaa ggaaaggcac caaagagcac aatg 284

<210> 243
<211> 17
<212> DNA
<213> Homo sapiens

<400> 243
tgctcttccc ggtcatg 17

<210> 244
<211> 49
<212> DNA
<213> Homo sapiens

<400> 244
cgttcttcct tttcgatccg ccatctgcgg tggagccgcc accaaaatg 49

<210> 245
<211> 311
<212> DNA
<213> Homo sapiens

<400> 245

cttcctttag cgtgaaccgc gggtgcggtg cctcccgtga aaataataaa ttcaccgtca 60

cgcttgttgt gaacgcgggt ggttcccgaa acttggaggc ttcccgtaaa cccagctcct 120

tcctcatctg ggaggtgggt cccgcgcggg tccgccgcct cctccctggc ccctccctct 180

cgtgtctttc attttcctgg ggctccgggg cgcggagaag ctgcatccca gaggagcgcg 240

tccaggagcg gacccgggag tgtttcaaga gccagtgaca aggaccaggg gcccaagtcc 300

caccagccat g 311

<210> 246
<211> 125
<212> DNA
<213> Homo sapiens

<400> 246

ctttcttttg cgcaggcgtc gcgccctggg gccggggccg ggcggcaccg cggtgcgcaa 60

gcgcaaccgt cggtgggtcg gggatcggtc gcctgagagg tatcacctct tctgggctca 120

agatg 125

<210> 247
<211> 134
<212> DNA
<213> Homo sapiens

<400> 247

agttctcctt ccaccttccc ccacccttct ctgccaaccg ctgtttcagc ccctagctgg 60

attccagcca ttgctgcagc tgctccacag cccttttcag gacccaaaca accgcagccg 120

ctgttcccag gatg 134

<210> 248
<211> 339
<212> DNA
<213> Homo sapiens

<400> 248

```
cgccctccta cttccccgtc tgcgtccgcg ttcgcggctc ccgtttgcat catccccgtc      60
tgcgtccgcg ttcgcggctc ccgtttgcat catctccagc cggcggctgc tccagggagg     120
ctgggcgcga tcctctccgc ccgcggctcc aacccgcact ctgcgcctct cctcgccttt     180
ctcgcacctg ctcctgcgcc aggcccggag accccgggg cggcttccca gaacctgcgg      240
agcacaactg gccgaccgac ccattcattg ggaaccccgt cttttgccag agcccacgtc     300
ccctgccacc tctagctcgg agcggcgtgt agcgccatg                            339
```

<210> 249
<211> 22
<212> DNA
<213> Homo sapiens

<400> 249
ctgcctcttt ctgagcggca tg 22

<210> 250
<211> 53
<212> DNA
<213> Homo sapiens

<400> 250
ctatctctcg ataaagttgt tgttgcggct tccgccgcgg gtggaagaag atg 53

<210> 251
<211> 560
<212> DNA
<213> Homo sapiens

<400> 251

```
ctatctctca tctttccgct cttagctggg agtgctccgc ctagtcactt ttcttaaggt      60
ggctcgtcga ggcctgactt cttccccgaa atcacgtccc tagacagcct cctattttac     120
cactaacttt actcctgcag ttattcagcg gtaggaaact gaaaccaaaa accagtgtaa     180
gcaagtaaac atctaaactg tttcaggagc cgcgtagaag gaacgcggcg gtgtgccccg     240
gaagcggaag tagattctcc tatagaaagg ctggactacg cggagtggtg acgtttcctc     300
attgggcgga aggttcgctg gcactccgtt ggtcttccag ctggtgggag ttgacgacgt     360
ggtgctgggc gttgggaccc tactttatct agttcgggaa gttgggttgt ggggtcatac     420
ctgtctgtct gctcccagct ttcttgggtt tcttccgacg gcgtggggcc tcgctaagga     480
attcccggcc cctcagggcc acggctttag cggtgtcttt tgcgagttct tcgtaagtac     540
atcttaaagc tgtcaagatg                                                 560
```

<210> 252

<211> 71
<212> DNA
<213> Homo sapiens

<400> 252

cggtctttcg gatgctgacg ctctcttcct gtctttgtgg ctccggaaag gcgtttggga 60

tgccaacgat g 71

<210> 253
<211> 161
<212> DNA
<213> Homo sapiens

<400> 253

ttttcttttt ggtggtggtg gtggaagggg ggaggtgcta gcagggccag ccttgaactc 60

gctggacaga gctacagacc tatggggcct ggaagtgccc gctgagaaag ggagaagaca 120

gcagaggggt tgccgaggca acctccaagt cccagatcat g 161

<210> 254
<211> 23
<212> DNA
<213> Homo sapiens

<400> 254
tgttcttccc atcggcgaag atg 23

<210> 255
<211> 199
<212> DNA
<213> Homo sapiens

<400> 255

ggtccttccc ctaaaacttg gcactgccga tactcccagc ccgttccttc ccaagtcagg 60

aacttgcagg ggaccccttg gcaattcttt ttctctcaag agcagacagc cttcagtccc 120

agccgctgcc agggctggtg tgtctgaccc agctgtggtt tttccaggcc tgaaggcccc 180

ggagtgcacc agcggcatg 199

<210> 256
<211> 897
<212> DNA
<213> Homo sapiens

<400> 256

cagtctcctc cccgaggtgc cggtggcccc gccgccactc cctccggctc cctccctccc 60

gccgcggcgc gcatctcatt ccagccctca ttccgcgcat tccagcgtcc tcctcgcaca 120

ctcgaggcca gggggcggga gggccgcagc tccggcgccg ccgcgtcccg ccaggtgaga 180

ggcgcccgcg cccgccgcac ccgccggcgc cctcacgggc cgcgcgcccc acgccgccgc 240

agccgaccgc tcgcgccgcg tgctcggctg ctcttttctt tccgccgccc gcgttcccgc 300

cttggacctc tgcgctccga cgcgctccgt cccgacctct ggcttccctc cgcgctccgg 360

cgctgctcgc tgcccctctc ccgcttccct cctgtccgcc ccgcgctccc ctcctcgctc 420

ccggttgact cactcctcca ggaataggga tccccgtgtt ttcccgtcag tcccattctg 480

ggaaaactcc tccctccgcg cgctccgctc cgctccgctg ggcgcaccgg ggccggtcgg 540

cgcggggtgg gcttggcccc gcggccccgc cttcactgcg ccgcccgtcg gccccggccg 600

gagcccggct ctgcgcgctg acgccctgtc gtccccgcag aacgatcgcc gcggccggaa 660

gagttggcgc tcggggcgga ctccttggaa ctggcttagc gcacccatcc caccttcccg 720

caccctggga ccggtcggaa cgagctgatt gcccgctaca tcaagctccg gacagggaag 780

acccgcacca ggaagcaggt ctccagccac atccaggtgc tggctcgtcg caaagctcgc 840

gagatccagg ccaagctaaa ggaccaggca gctaaggaca aggccctgca gagcatg 897

<210> 257
<211> 86
<212> DNA
<213> Homo sapiens

<400> 257

ttttctttcc tccagtctcg gggctgcagg ctgagcgcga tgcgcggaga cccccgcggg 60

ggcggcggcg gccgtgagcc ccgatg 86

<210> 258
<211> 121
<212> DNA
<213> Homo sapiens

<400> 258

cggccttttc cgcccgctcc cccctccccc cgagcgccgc tccggctgca ccgcgctcgc 60

tccgagtttc aggctcgtgc taagctagcg ccgtcgtcgt ctcccttcag tcgccatcat 120

g 121

<210> 259
<211> 46
<212> DNA
<213> Homo sapiens

<400> 259
ctatcttctt tttcttcagc gaggcggccg agctgacgca aacatg 46

<210> 260
<211> 70
<212> DNA
<213> Homo sapiens

<400> 260

cggcctccgc caccatcttg ctttccttta atccggcagt gaccgtgtgt cagaacaatc 60

ttgaatcatg 70

<210> 261
<211> 539
<212> DNA
<213> Homo sapiens

<400> 261

ctgcctttcg tgtctctgca gcgtggagac tggaaccggc aatttcaaag gacgccacgt 60

tcaatcgcag cgctggcgcg ggcggaggct aaaacacggg ggtcctgaga ctgaggaaaa 120

cgcgccaagt tcccctcggt ggcggagtgc taaagaccct agcggttcag gcgttcggcg 180

agcggggccg ctgcttgttg cgctcctggc tctcccgggg cgggcgcaga tgggcgccgc 240

tcccgggatg tagttggtgt tggtgcaaga cgggagcgag cggcggtcgg ggttcccgct 300

cttgggagcg gatggtcact cccccgcggg gagggcgagc cgaccagatt ttcctggggc 360

cggggacccg gcgggctcgg ggcagggact cacctgtcgc acccacactc attcgggttg 420

gacttgccgg cgtcaccgcc gcggacttcg ctttgggcca tgaccagata taattggtga 480

ttacaacttt cctctataaa ttaactcttg acactccttg ggatttgaag aaaaaaatg 539

<210> 262
<211> 143
<212> DNA
<213> Homo sapiens

<400> 262

gtgtctcctt cacttcgccc tccagctgct ggagctgcag cccgaccgcg agcgtgccaa 60

gcggcttcag cagctagcgg agcggtggcg gcggcccccc tcaggacacc accagattcc 120

cctcttcccg cggcctcgcc atg 143

<210> 263
<211> 68
<212> DNA
<213> Homo sapiens

<400> 263

gcctcttctc cgggagccag tccgcgccac cgccgccgcc caggccatcg ccaccctccg 60

cagccatg 68

<210> 264
<211> 423
<212> **DNA**
<213> Homo sapiens

<400> 264

ccccctcccc gctgctcacc ccgctctccg gccgccgccg gtgcgggtgc tccgctaccg 60

gctcctctcc gttctgtgct ctcttctgct ctcggctccc cacccctct cccttccctc 120

ctctcccctt gcctcccctc ctctgcagcg cctgcattat tttctgcccg caggctcggc 180

ttgcactgct gctgcagccc ggggaggtgg ctgggtgggt ggggaggaga ctgtgcaagt 240

tgtaggggag gggtgccct cttcttcccc gctcccttcc cccgccaact ccttcccctc 300

cttctccccc tttcccctcc ccgcccccac cttcttcctc ctttcggaag gactggtaac 360

ttgtcgtgcg gagcgaacgg cggcggcggc ggcggcggcg gcaccatcca ggcgggcacc 420

atg 423

<210> 265
<211> 283
<212> DNA
<213> Homo sapiens

<400> 265

agtcctttgc tcgccggctt gctagctctc tcgatcactc cctcccttcc tccctccctt 60

cctcccggcg gccgcggcgg cgctggggaa gcggtgaaga ggagtggccc ggccctggaa 120

gaatgcggct ctgacaaggg gacagaaccc agcgcagtct ccccacggtt taagcagcac 180

tagtgaagcc caggcaaccc aaccgtgcct gtctcggacc ccgcacccaa accactggag 240

gtcctgatcg atctgcccac cggagcctcc gggcttcgac atg 283

<210> 266
<211> 95
<212> DNA
<213> Homo sapiens

<400> 266

cagcctctac cttgcgagcc gtcttcccca ggcctgcgtc cgagtctccg ccgctgcggg 60

cccgctccga cgcggaagat ctgactgcag ccatg 95

<210> 267
<211> 155
<212> DNA
<213> Homo sapiens

<400> 267

```
gggcctttgt ctctggctgc agttggagct ctgcgtctcg tcttcgttct tctgtgtcct        60
ctgctgctag aggtccagcc tctgtggctc tgtgacctgc gggtattggg ggatccacag       120
ctaagacgcc aggaccccc ggaagcctag aaatg                                   155
```

<210> 268
<211> 448
<212> DNA
<213> Homo sapiens

<400> 268

```
cagtcctttt gtgggagtcc ggtctgtcca cttgccggtc cctcagaccg tcggcggtct        60
ctgtccgctt cgggacctgt ccgctggtcg ctccgcgtcc gatggctcct ggccgcggaa       120
ccttaggcct ggccctggtc tccgagcgcg ggttcgccgg gaggagcgtg tggcgggggt       180
gtgccggggc gtgagtgcgc cgagcatggg gctgagcctg gtgtggggag tgggtatctg       240
cggagccggc ctgaacccca cctcagccgg gcgcggggag ggggctccgt gcgtgtgatc       300
gtgcagctgt gagcgcgtgg ccgccccgcg gggctccgct gcaggcccct cagccccagg       360
agcagtactc gctcttcagg gcctgccctg gatcctggag gctacacagc tgcccactcc       420
tcctggggag gctgccgtgg aggccatg                                          448
```

<210> 269
<211> 155
<212> DNA
<213> Homo sapiens

<400> 269

```
gggcctttgt ctctcgctgc agcctgagct ctaggtcttg ttttccctgc tttgtgtttt        60
ctgctcgtgg acgcccagcc tctgtggccc tgtggcctgc aggtattggg agatccacag       120
ctaagacgcc gggacccccct ggaagcctag aaatg                                 155
```

<210> 270
<211> 128
<212> DNA
<213> Homo sapiens

<400> 270

gggcctttgt ctctcgctgc cgccggagtt tccaggtctc gacttcactg ctctgtgtcc 60

tctgctccag gaggcccagc ctgtgtggcc ctgtgacctg caggtattgg agagccacag 120

ctaagatg 128

<210> 271
<211> 176
<212> DNA
<213> Homo sapiens

<400> 271

gggtctttgc gtctggctac taccagaccg cgggttaggg gcttcatctc tctgcgttct 60

cagttgtggg aggccttggt gattcggcca cagcctcagc ctccgtcgct ctgtgacctg 120

cgggtattgg atgattggta gctaagactc ccgaatactt cagaagtggg gaaatg 176

<210> 272
<211> 29
<212> DNA
<213> Homo sapiens

<400> 272
tgttctttct agctctgaaa tagaaaatg 29

<210> 273
<211> 305
<212> DNA
<213> Homo sapiens

<400> 273

ctccctttc ggagatttga atttccccca gcgaggcgag tgaggcgaaa tacccgtatg 60

gtgatagctg gccttttcgc gccaatactg aaaaaggcag aacgttcctc cgctggcgcc 120

agccaatcag caggactcct gccttccttc ggggcaaggt cgcagcatct gcctcggaaa 180

tcacgaaatc acggggcttc tttctgctgg ctcagccggg aggcccagag tgttctgcag 240

aggctgcgta ttgaaggctg ctctctgaag ctccctgccc caggtcacgc cgccggttcc 300

agatg 305

<210> 274
<211> 45
<212> DNA
<213> Homo sapiens

<400> 274
acttcttttc ttggctaagc cgcgtttgta ctgtgtctta ccatg 45

<210> 275
<211> 171
<212> DNA

<213> Homo sapiens

<400> 275

```
ccgcctttgc gctgcgcgcc tgcgcccgcg ccggcttcca gcgggtgtcg gacctgagag        60
ctggaggggc gtgcgcgcgc cctcgctctg ttgcgcgcgc ggtgtcacct tgggcgcgag       120
cggggccgcg cgcgcacggg acccggagcc gagggccatt gagtggcgat g                171
```

<210> 276
<211> 243
<212> DNA
<213> Homo sapiens

<400> 276

```
cctccccctt ccccggctgg ggcggctgga gagccgggag tcgctgggtg cgtggggctg        60
cctcgccgcg tctcgccacg ggctctgcca gcagacagcc ttggcacaca ggcacaaggg       120
ctggagccca gagatgagag tgcccaaggg agatgtgagc ctggcgggct gcccgctaac       180
ctgtcgctga agccccagaa gcgggccctc aggccaggcc taccctgcct ccggcccagc       240
atg                                                                     243
```

<210> 277
<211> 687
<212> DNA
<213> Homo sapiens

<400> 277

```
aagcctcttt tatacatctc ttcagggaag agagaagcaa tgggcatgtt agtatacaat        60
gatcacagcc acgcaggcct gcaagctgcc ttttggacag gctgttgact gccgttccaa       120
ttagctgatt ggagaatgtg gaatgcagag tgataatgct gcatatctgc tatcaggcag       180
cagcaaaggt ttttgtcttg ggaaggcaag ctttccctgc aatattatct cagcagctcc       240
ctagctgctt accctgaaaa cgagggatcc aaacggaggg tgttgcactc tgctaacgct       300
ggtcctgtgc gtggctgtgg catatgagcg gcaggtctga aaaagcaggt gtgtgctggg       360
acgggcactg gactggaacg caggcggacg ctctcgggtt tacctgcttc ctgttaacag       420
attgtgggct cccagggcat atgtctgcac gctgaggccg aggcggagaa ggggcttcct       480
gagcgtccca gtacactgac agagacactt ggattggact taatcttaaa cctctggagt       540
tcaagacctt ttaaaaaggg ctaaataaac aatctctaca tgtaaaaggc cactgactcc       600
tacttcctct gtatagagca actgttgaac tcagctgcct gtaggaaaac tgaagacttt       660
aataacaaac tctccaaggt gaaaatg                                           687
```

<210> 278
<211> 540
<212> DNA
<213> Homo sapiens

<400> 278

gtttctcttc ttgacttgat gcaggcacag atttatcaag ctcctcagtc aacaaacaca 60
tcaccggaag aaatatggaa ggaaaggaat tttaaaagga aataccaatc tctgtgcaaa 120
caaagccttg tatattcatg tttgcaccaa tctactgtga gatttatgaa gaaaaacaaa 180
ttgcggacaa ctctctatgt acacttacaa atgcctcagt tgatgcttgt gggctgtttg 240
tcagcgttct gtgataatga acacatggac ttctgtttat taaattcagt tgacccttt 300
agccaattgc caggagcctg gatttttact tccaactgct gatatctgtg taaaaattga 360
tctacatcca ccctttaaaa gcattgatga attaattaga actttagaca acaaagaaaa 420
attgaaaaag aattctcagt aaaagcgaat tcgatgttca aaacaaacta caaagagaca 480
agacttctct gtttactttc taagaactaa tataattgct accttaaaaa ggaaaaaatg 540

<210> 279
<211> 33
<212> DNA
<213> Homo sapiens

<400> 279
gggccttcct gcaacctttg cggctccaac atg 33

<210> 280
<211> 270
<212> DNA
<213> Homo sapiens

<400> 280

gcttctttgc agcgcttcag cgttttcccc tggagggcgc ctccatcctt ggaggcctag 60
tgccgtcgga gagagagcgg gagccgcgga cagagacgcg tgcgcaattc ggagccgact 120
ctgggtgcgg actgtgggag ctgactctgg gtagccggct gcgcgtggct ggggaggcga 180
ggccggacgc acctctgttt gggggtcctc agagattaat gattcatcaa gggatagttg 240
tacttgtctc gtgggaatca cttcatcatg 270

<210> 281
<211> 37
<212> DNA
<213> Homo sapiens

<400> 281
ctgccttcgc cgctcgggcc gcccggggga aaacatg 37

<210> 282
<211> 237
<212> DNA
<213> Homo sapiens

<400> 282

ccgcctcctc taggccgccg gccgcgaagc gctgagtcac ggtgaggcta ctggacccac 60

actctcttaa cctgccctcc ctgcactcgc tcccggcggc tcttcgcgtc acccccgccg 120

ctaaggctcc aggtgccgct accgcagcgt gagtacctgg ggctcctgca ggggtccact 180

agccctccat cctctacagc tcagcatcag aacactctct ttttagactc cgatatg 237

<210> 283
<211> 109
<212> DNA
<213> Homo sapiens

<400> 283

ccttccttac ttccggttct ctatggtgcg cgggcaagct ttgctccgcc tccggcagtg 60

gcttactccc ggtgccaggt tcttggagct gtgaggagga acaaccatg 109

<210> 284
<211> 112
<212> DNA
<213> Homo sapiens

<400> 284

gggcctttgc aaaattgccc tagtaacggc cgcatggtaa ctcaggcgcc gggcgcactg 60

tcctagctgc tggttttcca cgctggtttt agctcccggc gtctgcaaaa tg 112

<210> 285
<211> 153
<212> DNA
<213> Homo sapiens

<400> 285

tttccttttt agttgactga aacaaaacaa aacaaaaggg ccactggatg tctgccttct 60

tggggggtga gccagacaga ctgacaaaca aacagcccca actgtgttcg gggagggtt 120

tcgcctcccg ttttgcccgg cagcagcagc atg 153

<210> 286
<211> 250
<212> DNA
<213> Homo sapiens

<400> 286

gctcccctttt tgccttcaac cttcgagccg ccacgtaatg ccacgtcccc gcgcatgcgc 60

atcttggccg ctgctggcgg ctgtttccgg gcttagaggg ctggagtggc cgccgagttg 120

gaggcggtgg tggcagcagt aggagtgtgt agagtgcggg attgggggcc aggccctgcg 180

gagggcgggg gaagttgtct tctttttttt ccggaggggc cggtaaacct ggtggctgaa 240

cggcaagatg 250

<210> 287
<211> 171
<212> DNA
<213> Homo sapiens

<400> 287

cccccttttg gcccctgcct ttggagaaag tggagtgtgg cgcttggttg tcgttatttc 60

ttcggactgc ttcgcggtgc acggattcag cttctgccca gtggggcttt cagctgtttg 120

cgcgtctctc tgtccccctc ccctcccccc ggcacacctc tgtctacgat g 171

<210> 288
<211> 145
<212> DNA
<213> Homo sapiens

<400> 288

gcttcttccg ctttctcgtc aggctcctgc gccccaggca tgaaccaagg tttctgaact 60

actgggcggg agccaacgtc tcttctttct cccgctctgg cggaggcttt gtcgctgcgg 120

gctgggcccc agggtgtccc ccatg 145

<210> 289
<211> 152
<212> DNA
<213> Homo sapiens

<400> 289

ggctccttcc tttccgtctc tggccggctg ggcgcgggcg actgctggcg aggcgcgtgg 60

gaccttacgc tggttcccct tcgtctcctc tcccggcccg ggccactaga gagttcgctg 120

acgccgggtg agctgagcct gccgccaaga tg 152

<210> 290
<211> 109
<212> DNA
<213> Homo sapiens

<400> 290

gtttcctctt ttcctggttt ctcaagagtg ctgctgctaa cgcggtcccc ggcacgcacc 60

atctgttgcc atcccggccg gccgaggcca ttgcagattt tggaagatg 109

<210> 291
<211> 26
<212> DNA
<213> Homo sapiens

<400> 291
ccgcctcctt ctttctcgac aagatg 26

<210> 292
<211> 47
<212> DNA
<213> Homo sapiens

<400> 292
cgctctctgg ccgggcttgg gctgcgtgga gaatactttt tgcgatg 47

<210> 293
<211> 33
<212> DNA
<213> Homo sapiens

<400> 293
gtttctcttt cttcctgtct gcttggaaag atg 33

<210> 294
<211> 55
<212> DNA
<213> Homo sapiens

<400> 294
aaaccttttc cggtcttact cacgttgcgg ccttcctcgc gtcacagccg ggatg 55

<210> 295
<211> 36
<212> DNA
<213> Homo sapiens

<400> 295
ctccctctca cacacgctca cacccggctc gagatg 36

<210> 296
<211> 846
<212> DNA
<213> Homo sapiens

<400> 296

```
ccgcctctct ccgccccggg tcgctgccgc ctccgccgct ttcgggcttc gcagcctgag      60
gaaaaaaaga gaaaaagata aaaaaaatct gaaaacgctt caaaatcctg aaaaaaaaaa     120
aggaaaagaa aaaacgaatc ctcggagaac ccgcggggaa gtcactttcg tacgcttccg     180
gcctgccccg cgcccgccgc cgcagcgctt ggcgtccgtc ggtctccgtc cgtcggtccg     240
ggggtgagcc gcccgcccgg cccgccgtgc cctccccccg ctcgggcccc gagccccgcg     300
ccccgcgcct gccccggcgc accacgtgtc cgtgctgccc ttcgccgccc gcccggggct     360
cgccgagtcg gcgcccacaa agatttggtt tccctctgcc ccggcggttg taatcttaaa     420
ccgccggagc ccgaggccta tatttataga gaaacgcgtg tccccgaggc cgccgtgggc     480
agcgtccggt cgcctcttaa aggattttta cccttcggaa ggggattccc cgtttaattt     540
ttttcctact ttgatttttt gaaatttgga gcttcgcacc aggaccgcgg agaagtgcaa     600
agtcgcgggg agggccgtat tgtgcggaga gccttttgtc tgcggtgctg cggccgtggg     660
agccggcccc cgcctcccgt ttccgtcccg tctccaagcc cgccgactcc agctcgtcct     720
cgccgcgccg gtgccacctg tgagccgcgg cgcgggcccg ggctccgaag gcgccccttt     780
gtcctgcggc gggcccgata agaagtcctc ctggcggggc tcggggtggt gggggggcggg    840
gagatg                                                                846
```

<210> 297
<211> 252
<212> DNA
<213> Homo sapiens

<400> 297

```
agctctttcg cggcgctacg gcgttggcac cagtctctag aaaagaagtc agctctggtt      60
cggagaagca gcggctggcg tgggccatcc ggggaatggg cgccctcgtg acctagtgtt     120
gcggggcaaa aagggtcttg ccggcctcgc tcgtgcaggg gcgtatctgg gcgcctgagc     180
gcggcgtggg agccttggga gccgccgcag caggggggcac acccggaacc ggcctgagcg    240
cccgggacca tg                                                         252
```

<210> 298
<211> 147
<212> DNA
<213> Homo sapiens

<400> 298

ctttcttttc tccaagacgg gctgaggatt gtacagctct aggcggagtt ggggctcttc 60

ggatcgctta gattctcctc tttgctgcat ttccccccac gtcctcgttc tcccgcgtct 120

gcctgcggac ccggagaagg gagaatg 147

<210> 299
<211> 210
<212> DNA
<213> Homo sapiens

<400> 299

tggtcttctc tcccgcggcg ctggggcccg cgctccgctg ctgttgctcc attcggcgct 60

tttctggcgg ctggctcctc tccgctgccg gctgctcctc gaccaggcct ccttctcaac 120

ctcagcccgc ggcgccgacc cttccggcac cctcccgccc cgtctcgtac tgtcgccgtc 180

accgccgcgg ctccggccct ggccccgatg 210

<210> 300
<211> 75
<212> DNA
<213> Homo sapiens

<400> 300

ttttctttct ttcttagctg ttagctgaga ggaagtctct gaacaggcgg cagcggctct 60

tatagtgcaa ccatg 75

<210> 301
<211> 72
<212> DNA
<213> Homo sapiens

<400> 301

gatccttttt gtcccctact gcgtgcggtg gcagcttcct tgcggaagtg gtgaccgtga 60

gagaagaaga tg 72

<210> 302
<211> 133
<212> DNA
<213> Homo sapiens

<400> 302

gtttcctttc gctgatgcaa gagcctagtg cggtggtggg agaggtatcg gcaggggcag 60

cgctgccgcc ggggcctggg gctgacccgt ctgacttccc gtccgtgccg agcccactcg 120

agccgcagcc atg 133

<210> 303
<211> 156
<212> DNA
<213> Homo sapiens

<400> 303

cctcccctct ccgcctaagc ctgccctatg ccagccgggt gtcctcccca cagcaccacg 60

gcttctcttc ctcagcacgg cgacaggggc ttccccttcg ccgccgccgc cgccgccggc 120

caagctccgc cgcgcccgcg gcccgcggcc gccatg 156

<210> 304
<211> 91
<212> DNA
<213> Homo sapiens

<400> 304

cgcccccttc tgcgcggtca cgccgagcca gcgcctgggc ctggaaccgg gccgtagccc 60

ccccagtttc gcccaccacc tccctaccat g 91

<210> 305
<211> 578
<212> DNA
<213> Homo sapiens

<400> 305

ctccctttct taaatgcttg gggtgagaga gaagagaggc tagggtgggg catggaggac 60

acagagagag agagtgctgt gtattccttc cccgctactg tcctgtcctc agctaacttg 120

ctctgggaca gcttccccag ggctacagat actgcactca gctgactgtc ctttcttctg 180

ggcccctggt cccagagcag agctgacaaa ggagattcct gagagagcac cttcttatca 240

cagaaagtgc tgagccaaga gctcctagct gccccttttg cagatgtgaa gggccagtga 300

accttggacc cagatggttg cttaatactc ctttccccct ccctcactcc ttcctttgcg 360

ggctgcctca cctcctccac ccttcttgct taaatccata ggcatttgtc tggccttccc 420

ttttactgct ggctgggaag gaggagcatc agaccacaga tcctggaagg cacttctctc 480

cctgactgct gctcacactg ccgtgagaac ctgcttatat ccaggaccaa ggaggcaatg 540

ccaggaagct ggtgaagggt ttcctctcct ccaccatg 578

<210> 306
<211> 89
<212> DNA
<213> Homo sapiens

<400> 306

ctcccttttc tcctcaagtc ctgaagttga gtttgagagg cgacacggcg gcggcggccg 60

cgctgctccc gctcctctgc ctccccatg 89

<210> 307
<211> 74
<212> DNA
<213> Homo sapiens

<400> 307

agccctccta cctgcgcacg tggtgccgcc gctgctgcct cccgctcgcc ctgaacccag 60

tgcctgcagc catg 74

<210> 308
<211> 86
<212> DNA
<213> Homo sapiens

<400> 308

ccttctttgc ggctcggcca ttttgtccca gtcagtccgg aggctgcggc tgcagaagta 60

ccgcctgcgg agtaactgca aagatg 86

<210> 309
<211> 217
<212> DNA
<213> Homo sapiens

<400> 309

cggccccttc ggctccgagc tgaccctgat cagggccgag ttgtctcggc ggcgctgccg 60

aggcctccac ccaggacagt ccccctcccc gggcctctct cctcttgcct acgagtcccc 120

tctcctcgta ggcctctcgg atctgatatc gtggggtgag gtgagcaggc ccggggaggg 180

tggttaccgc tgaggagctg cagtctctgt caagatg 217

<210> 310
<211> 71
<212> DNA
<213> Homo sapiens

<400> 310

agctctcttc ttgcttggca gctggaccaa gggagccagt cttgggcgct ggagggcctg 60

tcctgaccat g 71

<210> 311
<211> 479
<212> DNA
<213> Homo sapiens

<400> 311

```
ttttcttctt cgtcagcctc ccttccaccg ccatattggg ccactaaaaa aagggggctc      60
gtcttttcgg ggtgtttttc tccccctccc ctgtccccgc ttgctcacgg ctctgcgact     120
ccgacgccgg caaggtttgg agagcggctg ggttcgcggg acccgcgggc ttgcacccgc     180
ccagactcgg acgggctttg ccaccctctc cgcttgcctg gtcccctctc ctctccgccc     240
tcccgctcgc cagtccattt gatcagcgga gactcggcgg ccgggccggg gcttccccgc     300
agcccctgcg cgctcctaga gctcgggccg tggctcgtcg gggtctgtgt cttttggctc     360
cgagggcagt cgctgggctt ccgagagggg ttcgggctgc gtaggggcgc tttgttttgt     420
tcggttttgt ttttttgaga gtgcgagaga ggcggtcgtg cagacccggg agaaagatg      479
```

<210> 312
<211> 540
<212> DNA
<213> Homo sapiens

<400> 312

```
tctcctcttc ttcctttgtg tgtgcgcgag cggagttggg gcggagggag aagggggagg      60
tcgctctgtc tgtccgtctc ccgccgcctc tgcccggtct actcgaagtg cggcgggaga     120
ggcgggagcc caggagaggg tgcgggagct ggcggggcgg ctcggagctg ccaggacgcc     180
ctggtcccag ccgcgcacag gggagcgtgg acggcagagg ggctcggcgg gagccgagat     240
ccgcccgtcc cggctgcccc tcggcctccc tctgctccca cctacccccct gacacccata    300
gaaaagcgtg caaaggcgcg gagcgggacg gaaaccacaa ataaatagcg gcggcggcag     360
cgcgtcatct ggtggagcag gaagtgcagg cagagtccgg aggctggtgc tttctgcgcg     420
tccccaggac tttgccatgg gctgggggcc gcggaggctg cgagcggccg ggcgagggca     480
gcggcggcgg cgtccgcacc ggggctgagc gagcagcgac gcgaggggcg cgcggagatg     540
```

<210> 313
<211> 466
<212> DNA
<213> Homo sapiens

<400> 313

gggcctcctt gaggaccccg ggctgggcgc cgccgccggt tcgtctactc tttccttcag 60
ccgcctcctt tcaaccttgt caacccgtcg gcgcggcctc tggtgcagcg gcggcggctc 120
ctgttcctgc cgcagctctc tccctttctt acctccccac cagatcccgg agatcgcccg 180
ccatggcttt acttactgcg gccgcccggc tcttgggaac caaggcaccc agtggcaagt 240
actagctgag catttgggag atgcttgtct tacttggctg ttgcttctcc tgctgctggg 300
gaaaaggaat gcatcttgtc ttgttcttgc agcccggcat gccagtgctt cctccacgaa 360
tttgaaagac atattggctg acctgatacc taaggagcag gccagaatta agactttcag 420
gcagcaacat ggcaagacgg tggtgggcca aatcactgtg gacatg 466

<210> 314
<211> 114
<212> DNA
<213> Homo sapiens

<400> 314

atttcttttc aagtcaattg aactgaaatc tccttgttgc tttgaaatct tagaagagag 60
cccactaatt caaggactct tactgtggga gcaactgctg gttctatcac aatg 114

<210> 315
<211> 68
<212> DNA
<213> Homo sapiens

<400> 315

cagtctccct cccttctgga gacaccacca gatgggccag ccagaggcag cagcagcctc 60
ttcccatg 68

<210> 316
<211> 54
<212> DNA
<213> Homo sapiens

<400> 316
cgctctccgc ctcgcttgct cctgccgggc gtgcagggcc ccgccgccgc catg 54

<210> 317
<211> 180
<212> DNA
<213> Homo sapiens

<400> 317

catcctttcc ctgcggagga ccagggcaag tttcctgcct gcacggcaca ggagagcaaa 60
cttctacaga cagaccaagg cttccatttg ctgctgacac atggaactga ggtgaaattg 120
tgctccatga ttttacagat ttcataacgt ttaagagacg ggactcaggt catcaaaatg 180

<210> 318
<211> 15
<212> DNA
<213> Homo sapiens

<400> 318
cgtcctctca gcatg 15

<210> 319
<211> 247
<212> DNA
<213> Homo sapiens

<400> 319

ttacctcttt ttcttgtctc tcgtcaggtc tctgacattg acagagcctg gacgttggag 60

gaagccccag gacgttggag gggtaaagta aaagtccaca gttaccgtga gagaaaaaag 120

agggagaaag cagtgcagcc aaactcggaa gaaaagagag gaggaaaagg actcgacttt 180

cacattggaa caaccttctt tccagtgcta aaggatctct gatctgggga acaacaccct 240

ggacatg 247

<210> 320
<211> 72
<212> DNA
<213> Homo sapiens

<400> 320

cgctctttct cccttcagca gccagccagc tctgtgtcag ggtcgggggg tgcagaaagt 60

caggacagaa tg 72

<210> 321
<211> 161
<212> DNA
<213> Homo sapiens

<400> 321

gttcctcttt tcctcggttc ccagtgttct ggcaggtaag gaacgccggc tcttcgcctc 60

tcagcgcggc ttgtcctttg ttccggacgc ccgctcctca gccctgcggc tcctggggtc 120

gctgctgcat cccgcacgcc tccaccggct gcagacccat g 161

<210> 322
<211> 78
<212> DNA
<213> Homo sapiens

<400> 322

cgctccctcc cggtgccggc ttctctgagt caccaacctg aggctgcccc ggccgcctgc 60

gcacccggca gcaccatg 78

<210> 323
<211> 59
<212> DNA
<213> Homo sapiens

<400> 323
agctctttgc cgtcggagcg cttgtttgct gcctcgtact cctccattta tccgccatg 59

<210> 324
<211> 118
<212> DNA
<213> Homo sapiens

<400> 324

cttccttctt tcctcccttg ccagtccgcc tgtcttcctc cccgtcttcc ctgcccggcc 60

tccccctct tcccccgctg gccccctccc cggagggata atatggtctc cggcgatg 118

<210> 325
<211> 182
<212> DNA
<213> Homo sapiens

<400> 325

ccaccttctt ttcatttcta gtgagacaca cgctttggtc ctggctttcg gcccgtagtt 60

gtagaaggag ccctgctggt gcaggttaga ggtgccgcat cccccggagc tctcgaagtg 120

gaggcggtag gaaacggagg gcttgcggct agccggagga agctttggag ccggaagcca 180

tg 182

<210> 326
<211> 96
<212> DNA
<213> Homo sapiens

<400> 326

cattcctttc tttcgattac ccgtggcgcg gagagtcagg gcggcggctg cggcagcaag 60

ggcggcggtg gcggcggcgg cagctgcagt gacatg 96

<210> 327
<211> 152
<212> DNA
<213> Homo sapiens

<400> 327

gagtcttttc agcgctgagg actggcgctg aggaggcggc ggtggctccc ggggcgtttg 60

agcgggctca cccgagcccg cgggccaacg cggatccagg cccgactggc gggaccgccc 120

cggattcccc gcgggccttc ctagccgcca tg 152

<210> 328
<211> 28
<212> DNA
<213> Homo sapiens

<400> 328
atttctcctc cccctcccgg ccaagatg 28

<210> 329
<211> 192
<212> DNA
<213> Homo sapiens

<400> 329

ggtcctctct cggctcctcg cggctcgcgg cggccgacgg ttcctgggac acctgcttgc 60

ttggcccgtc cggcggctca gggcttctct gctgcgctcc cggttcgctg gacgggaaga 120

agggctgggc cgtcccgtcc cgtccccatc ggaaccccaa gtcgcgccgc tgacccgtcg 180

cagggcgaga tg 192

<210> 330
<211> 23
<212> DNA
<213> Homo sapiens

<400> 330
ctaccttcct tctagcagaa atg 23

<210> 331
<211> 268
<212> DNA
<213> Homo sapiens

<400> 331

cggcccttcc tccgccttct gggcggagcc cgcgcgggat ccgggtggct gcaggctgct 60

ggcttctgcg gctgcggggt cggggtcgcg gccagggcca agccgcagcg agttcacagg 120

cggaacccct gcaggcggcg ccccctacgc gaggtcaccc ctgggaagga gcgcagccca 180

cccggcccct ccgcatccga gcaggacgcc cgtctcctct ccctgaggat ttcaggtctc 240

cctgtcccag gaggcttgtg ccaagatg 268

<210> 332
<211> 34
<212> DNA

<213> Homo sapiens

<400> 332
tcttctctcg gttcctcttt cctcgctcaa gatg 34

<210> 333
<211> 56
<212> DNA
<213> Homo sapiens

<400> 333
ggctcttctt tgcctctgct ggagtccggg gagtggcgtt ggctgctaga gcgatg 56

<210> 334
<211> 68
<212> DNA
<213> Homo sapiens

<400> 334

ttttccttta gtcaggaagg acgttggtgt tgaggttagc atacgtatca aggacagtaa 60

ctaccatg 68

<210> 335
<211> 81
<212> DNA
<213> Homo sapiens

<400> 335

gcttctcttt tccttggcgg aggagggaga ccacagagcc ctgggttgtg gaagaggtgg 60

ctgttccctg tcatcagtat g 81

<210> 336
<211> 292
<212> DNA
<213> Homo sapiens

<400> 336

cccccttccc ccgcctttct tccctccgcg acccgggccg tgcgtccgtc cccctgcctc 60

tgcctggcgg tccctcctcc cctctccttg cacccatacc tctttgtacc gcaccccctg 120

gggacccctg cgcccctccc ctcccccctg accgcatgga ccgtcccgca ggccgctgat 180

gccgcccgcg gcgaggtggc ccggaccgca gtgccccaag agagctctaa tggtaccaag 240

tgacaggttg gctttactgt gactcgggga cgccagagct cctgagaaga tg 292

<210> 337
<211> 261
<212> DNA
<213> Homo sapiens

<400> 337

gagcctttct ggggaagaac tccaggcgtg cggacgcaac agccgagaac attaggtgtt 60
gtggacagga gctgggacca agatcttcgg ccagccccgc atcctcccgc atcttccagc 120
accgtcccgc accctccgca tccttccccg ggccaccacg cttcctatgt gacccgcctg 180
ggcaacgccg aacccagtcg cgcagcgctg cagtgaattt tccccccaaa ctgcaataag 240
ccgccttcca aggccaagat g 261

<210> 338
<211> 243
<212> DNA
<213> Homo sapiens

<400> 338

gcgcctctta ggcttggcgg tggcggcggc ggcagcttcg cgccgaatcc ccggggagcg 60
gcggtggcgg cgtcctgggg ccaggaggag cgaacacctg ccgcggtcct cccgccggcg 120
ctgggctctg tgtgctccgg gatggagcag gtgtgcagag ggtgagaacc cagctctggg 180
accaagtcac ttgcttcctt acttagcaag actatcgact tgagcaaact tggacctggg 240
atg 243

<210> 339
<211> 526
<212> DNA
<213> Homo sapiens

<400> 339

ccccctcttt tggttacaga cgtgagggct ctttggagac gtaaacatct ccgagtggcg 60
agggtgggcg gggctgggct tgggaaaggg cggggtggct tgcttgaggt gtggaaagac 120
cagaagaagg tgaggtcaag agagtgcaga atgaggcatt ccaatggtgg gtgggccctg 180
acctgagaga gtggcgcggg gaggggtgaa agcgcggcga tcctggaacg ccagcgggcg 240
ttgcggccta tgcgcgaggg gcggggcgat taggtcatag agcggctccc agcgttccct 300
gcggcgtagg aggcggtcca gactataaaa gcggctgccg gaaagcggcc ggcacctcat 360
tcatttctac cggtctctag tagtgcagct tcggctggtg tcatcggtgt ccttcctccg 420
ctgccgcccc cgcaaggctt cgccgtcatc gaggccattt ccagcgactt gtcgcacgct 480
tttctatata cttcgttccc cgccaaccgc aaccattgac gccatg 526

<210> 340
<211> 251
<212> DNA
<213> Homo sapiens

<400> 340

gctcctctgc gcccttgccg ccctccgagc cacagctttc ctcccgctcc tgcccccggc 60
ccgtcgccgt ctccgcgctc gcagcggcct cgggagggcc caggtagcga gcagcgacct 120
cgcgagcctt ccgcactccc gcccggttcc ccggccgtcc gcctatcctt ggccccctcc 180
gctttctccg cgccggcccg cctcgcttat gcctcggcgc tgagccgctc tcccgattgc 240
ccgccgacat g 251

<210> 341
<211> 78
<212> DNA
<213> Homo sapiens

<400> 341

cttcctttcg cggggtcctc cgtagttctg gcacgagcca ggcgtactga caggtggacc 60
agcggactgg tggagatg 78

<210> 342
<211> 357
<212> DNA
<213> Homo sapiens

<400> 342

gctcctcctc gtcccagcgc tagcgggcac gcggttcctt tttgcgagct ttccgagtgc 60
caggcgccgg ccggctgcga agacgcggtg ggccgcccct ccgattgaaa tcacagaaga 120
tattcgtgtt cttcttaaga gaaaaagagg acattttagc tttctcagtt gaaggcgtac 180
tttattgtcg gcttccaaag attactaact tttatctgta tcactaagat tgaactgcct 240
tggctgtact gctattctta ctgctgcttc tattattgcc ttcttcagca caataaggct 300
ttcaaaagcc aaagaataac aagaaataag caccatttta gaagcctttc cactatg 357

<210> 343
<211> 86
<212> DNA
<213> Homo sapiens

<400> 343

tggtcctttt caacggtttt cacagatcca gtgacccacg ctctgaagac agaattagct 60
aactttcaaa aacatctgga aaaatg 86

<210> 344
<211> 303
<212> DNA
<213> Homo sapiens

<400> 344

ctgcctctcc aggcaacgcg ggaggcccag cgggaaggca ggaggcggcg gcggaggagg 60
agctctactg agccgcaact gtggcgacag caaccggagt cgcagccgcc gccacctgca 120
cctggcgcct agcccacgtc cagcgcctgc ccggccgccg cttcccgcca ccctgccctg 180
cccacccgcc aggtactacc attaaagata ccttcttctc agcaaatcta tgataaaaaa 240
tataagtaac agaagaagaa ataactgtta tttgtcaagt gacaagcttt taatgtcaga 300
atg 303

<210> 345
<211> 107
<212> DNA
<213> Homo sapiens

<400> 345

acgtctcttg ctcctcaggg ccactgccag gcttgccgag tcctgggact gctctcgctc 60
cggctgccac tctcccgcgc tctcctagct ccctgcgaag caggatg 107

<210> 346
<211> 78
<212> DNA
<213> Homo sapiens

<400> 346

acgcctcttc agttgtctgc tactcagagg aaggggcggt tggtgcggcc tccattgttc 60
gtgttttaag gcgccatg 78

<210> 347
<211> 146
<212> DNA
<213> Homo sapiens

<400> 347

gggtcttttt tagcgccatc tgctcgcggc gccgcctcct gctcctcccg ctgctgctgc 60
cgctgccgcc ctgagtcact gcctgcgcag ctccggccgc ctggctcccc atactagtcg 120
ccgatatttg gagttcttac aacatg 146

<210> 348
<211> 115
<212> DNA
<213> Homo sapiens

<400> 348

cgctctctga tgcaacgccg gaatcgcgga aaccgccggt gcacgttgga gtcataagac 60

ggcgtcggtg ttgcagtctg tgtccttgga ggtgaccagg gccactgcag gcatg 115

<210> 349
<211> 43
<212> DNA
<213> Homo sapiens

<400> 349
cgtccccttg ggtccttgat cctgagctga ccgggtagcc atg 43

<210> 350
<211> 35
<212> DNA
<213> Homo sapiens

<400> 350
ttctccttcc cgcagtctgc agccggagta agatg 35

<210> 351
<211> 91
<212> DNA
<213> Homo sapiens

<400> 351

catcctttac ggcaggcgtc cgcgtcgcta gctagtcgtt ctgaagcggc ggccagagaa 60

gagtcaaggg cacgagcatc gggtagccat g 91

<210> 352
<211> 133
<212> DNA
<213> Homo sapiens

<400> 352

ccctcctttt taagcgcctc ccgccagcct ctgctgtggc tcgcttcgcc gcgctccctc 60

cttccccgcc ttccatacct ccccggctcc gctcggttcc tggccacccc gcagcccctg 120

cccaggtgcc atg 133

<210> 353
<211> 146
<212> DNA
<213> Homo sapiens

<400> 353

ctcccttcgc gctccggacg ggcgacggta gctcgagacc cgggactccg cccgcctccc 60

cgcgagtatt tgaggtccgg ggcggctccg gcgcctctgc ccgccgttct gctcgctcgc 120

tccccgctct ggagtctgcc atcatg 146

<210> 354
<211> 69
<212> DNA
<213> Homo sapiens

<400> 354

ttctctcctc agacttcaag ggctaccact ggacccttcc cctgtcttga accctgagcc 60

ggcaccatg 69

<210> 355
<211> 25
<212> DNA
<213> Homo sapiens

<400> 355
ctctctcctt tccctgttag acatg 25

<210> 356
<211> 213
<212> DNA
<213> Homo sapiens

<400> 356

agttctctcc gcacgcgggc tggagaagcg ggtcctacgc acgctttgtt gtcgcgcttt 60

gcctccgtcc ttgcccctac tcccgcctta cctgacttcc ttttcggagg aagatccttg 120

agcagccgac gttgggacaa aggatttgga gaaacccagg gctaaagtca cgtttttcct 180

cctttaagac ttacctcaac acttcactcc atg 213

<210> 357
<211> 173
<212> DNA
<213> Homo sapiens

<400> 357

cgccctttct gtgcggcgcc cgggcgcaac gcaaacatgg cggcgggtgg cacccgtcgg 60

tgaggcggtg ccgggcgggg gttgtcgggt gtcatgggcg gtggcgacgg caccgccccc 120

gcgtctccct gagcgggacg gcaggggggg cttctgcgct gagccgggcg atg 173

<210> 358
<211> 242
<212> DNA
<213> Homo sapiens

<400> 358

ctgccctttg gacgcgcgcc tcggttccga acgcagcgga cggcgcctca ggcagcgcgg 60

cggacagccc gtcctccggc gcgccgcgag cctcggagga ccctagcgac ggtcgtggcg 120

taagaccggg gggacgcggc ggtagcggcg gccgttgcga ttgattgcgc tggttgcctg 180

cggcgtccac ttccttggcc gcccttgcta cactggctga ttgttgtgca gccggcgcca 240

tg 242

<210> 359
<211> 425
<212> DNA
<213> Homo sapiens

<400> 359

ccaccctttc tcgaggctgt ggcctccgcg agagccgagc gggccgcacc gccggccgtg 60

cgactgcccc agtcagacac gaccccggct tctagcccgc ctaagcctgt ttggggttgc 120

tgactcgttt cctccccgag tttcccgcgg gaactaactc ttcaagagga ccaaccgcag 180

cccagagctt cgcagacccg gccaaccaga ggcgaggttg agagcccggc gggccgcggg 240

gagagagcgt cccatctgtc ctggaaagcc tgggcgggtg gattgggacc ccgagagaag 300

caggggagct cggcggggtg cagaagtgcc caggcccctc cccgctgggg ttgggagctt 360

gggcaggcca gcttcaccct tcctaagtcc gcttctggtc tccgggccca gcctcggcca 420

ccatg 425

<210> 360
<211> 185
<212> DNA
<213> Homo sapiens

<400> 360

ttccctcctt cgtcgctgtt gctgccgcca tacgcgctct ccctgtttag ctcttctgtt 60

agaaatagta tctttgtttt cctttgctgt tcctcaatcc cctactcttc accccttgtt 120

ttcacctatt ttgcgagaac ccatccagat ccccccttccc ttcttcccct gccggcccag 180

ttatg 185

<210> 361
<211> 29
<212> DNA
<213> Homo sapiens

<400> 361
ccgccctttc gctcctcggc cgcgcaatg 29

<210> 362
<211> 368

<212> DNA
<213> Homo sapiens

<400> 362

agctctttcc cttttggttt gcaagcactg cctgtaaagc cctcgcatga gaggccagcc 60
tgctagggaa atccaggaat ctgcaacaaa aacgatgaca gtctgaaata ctctctggtg 120
ccaacctcca aattctcgtc tgtcacttca gacccccact agttgacaga gcagcagaat 180
ttcaactcca gtagacttga atatgcctct gggcaaagaa gcagagctaa cgaggaaagg 240
gatttaaaga gtttttcttg ggtgtttgtc aaacttttat tccctgtctg tgtgcagagg 300
ggattcaact tcaatttttc tgcagtggct ctgggtccag cccctttactt aaagatctgg 360
aaagcatg 368

<210> 363
<211> 65
<212> DNA
<213> Homo sapiens

<400> 363

ctccctttttc agtccgcgtc cctccctggg ccgggctggc actcttgcct tccccgtccc 60
tcatg 65

<210> 364
<211> 62
<212> DNA
<213> Homo sapiens

<400> 364

ggtccttctc tggggcggtc gcgttggcag cggatgcggg aagccggact ctgggcgtca 60
tg 62

<210> 365
<211> 136
<212> DNA
<213> Homo sapiens

<400> 365

cgccctctca gcaacccgca cagggcgcac ccggacgctc taccgctccc gccgcagtcg 60
ccgggccatg ggcctcgagc ccgccccgaa cccccgcgag cccgccttgt ctgcggcgtg 120
actggaggcc cagatg 136

<210> 366
<211> 79
<212> DNA

<213> Homo sapiens

<400> 366

```
cgttcttttg ttccggggcc gcagggcggg gcaggcccga ctttcgccgt cttcttgtct        60

actctccaga acggccatg                                                     79
```

<210> 367
<211> 117
<212> DNA
<213> Homo sapiens

<400> 367

```
cttcctctcc gcctccttcg cctagcctgc gagtgttctg agggaagcaa ggaggcggcg        60

gcggccgcag cgagtggcga gtagtggaaa cgttgcttct gaggggagcc caagatg         117
```

<210> 368
<211> 63
<212> DNA
<213> Homo sapiens

<400> 368

```
ctacctcttc ctctccacgc ggttgagaag accggtcggc ctgggcaacc tgcgctgaag        60

atg                                                                      63
```

<210> 369
<211> 404
<212> DNA
<213> Homo sapiens

<400> 369

```
tgctctctgc cgcattgata gcagcgagag ctggaggtgt tgggtcggga gaccagccgt        60

tcgatcccgc cgcaggtagg agctggtttc catcctggca ccacggcaca cacctccagc       120

ctcgagcccg gcgctgctgc ccgggggtct ccttcaggct ctttgacgcc gttccagggg       180

gcacctatcc aggcatcctc tgggcctcta gccagaggac tggctcccgg cttcagcact       240

ccgggctgca gtaagaagtg cccttatcgc tctgagccct gccaccatcc cgtgaaccac       300

cgaaaccctg gtccagcgcg acagccttgg acctgggact ggacggatcc aaaacgctca       360

gcctcggccc cccacagacg gggctctgca tcgtctctga tatg                       404
```

<210> 370
<211> 41
<212> DNA
<213> Homo sapiens

<400> 370

tgctcttcct gggctggctg tctcctgctc atccagccat g 41

<210> 371
<211> 152
<212> DNA
<213> Homo sapiens

<400> 371

ctgtctttct agcatgttgc ccttttttcaa ccacatttgt gtttcaggtg tagagaggag 60

agagagtgaa cagggagcgg ggcttttgtc tgttggtctc cctggactga agagagggag 120

aatagaagcc caagactaag attctcaaaa tg 152

<210> 372
<211> 55
<212> DNA
<213> Homo sapiens

<400> 372
gcttctctgc tgaccctctc tcgtcgccgc tgccgccgcc gcagctgcca aaatg 55

<210> 373
<211> 42
<212> DNA
<213> Homo sapiens

<400> 373
cctccttctg tttcccagac cgagagccgc gccggcacca tg 42

<210> 374
<211> 152
<212> DNA
<213> Homo sapiens

<400> 374

ccccctcttc tccgcgtagg cccagctccc tgaagcggct gtttcgagcc acgcgcccat 60

cgggtaccga ggcacgcgcc gggcgtcacg tgcgtttcgc ggcgagcgga aatgacgcga 120

gttgtgtgag ccgccagtat ggccgggcta tg 152

<210> 375
<211> 182
<212> DNA
<213> Homo sapiens

<400> 375

ctgtctctcc ccatccgggg cagcggggaa tggctgagcc aggggttcgc cgcccccgcc 60

gccgccgccg ccgccgccgc cgccgccgcc gcccgctttc ggctcgggcc tcaggaccgt 120

agcatcctga gacattttga attgacactt ctcaagattt gactggatca gagttcatca 180

tg 182

<210> 376
<211> 106
<212> DNA
<213> Homo sapiens

<400> 376

cttcctttat ctctggcggc cttgtagtcg tctccgagac tccccacccc tccttccctc 60

ttgacccccct aggtttgatt gccctttccc cgaaacaact atcatg 106

<210> 377
<211> 118
<212> DNA
<213> Homo sapiens

<400> 377

gagcctctgt cggccgcgga agcctggagt gggcggtacg cagacgcgcg cggtgagacc 60

cgctgtctgc tcagcggact ctgcccgccc ccacctcccc ctgcgtcggg ccgacatg 118

<210> 378
<211> 179
<212> DNA
<213> Homo sapiens

<400> 378

caccctcttt cagagtggta catggaagac agcacaaagt ggatccatac tctgaaatgc 60

agtaactctg atgcttgaat ttgtctccct tcttgccaga aaggattcta ataactcggt 120

gtcaaagcca agacataaac tcaacccctt ctcttccaaa agcttcacgt tacagcatg 179

<210> 379
<211> 92
<212> DNA
<213> Homo sapiens

<400> 379

gtacctttct cggggtgtct gcgtaactgc ccagacttgc cttggtttgg tcagatgaca 60

cctcctctgg gactggctag ccagcgttca tg 92

<210> 380
<211> 185
<212> DNA
<213> Homo sapiens

<400> 380

```
tttcctctgc tccgccgcgg ccggaggtat ccgcatcggc gagctgcgtc tcccgggtgt        60
cggccccggc ggctccccga ccgtgcccgg ctgtggcgag gcggctccag cccagcctgt       120
ggcagccgcg acccccgggg cgctccggag cccactgcgc ggcgcgcgtg ccggctgcct       180
gcatg                                                                   185
```

<210> 381
<211> 36
<212> DNA
<213> Homo sapiens

<400> 381
ctttcttccg ccttaggaag gtggcggcca gggatg 36

<210> 382
<211> 817
<212> DNA
<213> Homo sapiens

<400> 382

```
cggccctttt ctcggggcgc ccgagaggcc agctcagacc tcccggctcg acaggcggcg        60
cgggcggcgg tgagtgcggc gcggggacgc cggggcgcgg ggaccagcgg gagacagcgg       120
ggggccggtg gcgccagcac ctgctggggg ccccgggcac tgagcccttg gctggggcct       180
cctgggatgc caggggggcgc gggtcgggtc gcgggcatcg aggcgcggcg gagggcgtgg      240
gggcccggcc ggggcggggt ccggcctccc agcgctggtc ccggccgcgt ctccggttgg       300
gttcagctcc tgcgtcccag agtggcccga tcgcgcgtgg cggggtcgtc cggccccccac      360
ccgaacgagc gcccttcgcg gcccgccgcg tccccctccc cggagaggac ggcccctggg       420
ctttttagaa aaaggcgcga ttctctctag tgactcaggt tgagatttcc agaaatatcc       480
cccgggggtt cagaaacaaa accaaaacaa acaaaaaaac cccaacgaat tcccaaatgc       540
tatttgccaa acatttgact tctaggggcg cgggtacccg cgtttctctc cctgccccccg      600
cgacttcgcg caagatccgg gaaggacacc cgaggcccct gggagaccct ggggaggtga       660
aaatcagaga gcgaagcggg ccgtggcccc taggcctgac cctccccgc ggggtaaggc        720
gggcaccccg cgagcgcagg ggtcctctta ctgctgatgg cacccagctc tgggcccaga       780
cgccgctcac cgtccaccgc cggtgctggg taaaatg                                817
```

<210> 383
<211> 264
<212> DNA
<213> Homo sapiens

<400> 383

```
ccaccccttc ggctctgggc cccgcctcgt ggtgccggct ggttcttcgc gctcgcccga    60
cttcccagcg gccccgtgcg gcccgggcat gcccagtgcg ggcgcagcgg ccccggccct   120
ggaagcgccc cggcggagct ggcctgcggt gggctagggg cagggccgga gccgcggcgg   180
cggagctgtg gatccttcat gatgagagat ttggggacac ttctctctcc tgtgtgtagt   240
tgatagtttg gtggtgaaga gatg                                          264
```

<210> 384
<211> 64
<212> DNA
<213> Homo sapiens

<400> 384

```
tgacctttcc gagttggctg cagatttgtg gtgcgttctg agccgtctgt cctgcgccaa    60
gatg                                                                 64
```

<210> 385
<211> 91
<212> DNA
<213> Homo sapiens

<400> 385

```
attcctccgc gcgctgggac aggctgcttc ttcgccagaa ccaaccggtt gcttgctgtc    60
ccagcggcgc cccctcatca ccgtcgccat g                                   91
```

<210> 386
<211> 222
<212> DNA
<213> Homo sapiens

<400> 386

```
ctctcttccg cccggcggcc cacaccggtc aggcccggcg cgggctgcgc tctccagctg    60
tggctatggc cccagccccg agatgaggag ggagagaact aggggcccgc aggcctggga   120
atttccgtcc cccaccaagt ccggatgctc actccaaagt ctcagcaggc ccctgaggga   180
gggagctgtc agccagggaa aaccgagaac accatcacca tg                      222
```

<210> 387
<211> 430
<212> DNA
<213> Homo sapiens

<400> 387

cgaccttcct gccgggccgg gcggtccgag gctgctggag tgccgtgagc aggccgcggg 60
aacgtcgccg tcaccttgtc tcggggcctc ggcgctgctt cccgccaaaa cacgtttacc 120
gcgcgcccgg gcctcccacc ttgcggaagg gaccccacca ccacttggat ttctgttgca 180
ggttgagaac aaaaacatgc acctggagtt tccccggagc cctctgcgtg gttgagcttc 240
ggtggaattt cggggctctt ggctgccagc cgcgcttgcc tggtagcaac agaaaccagt 300
cctgctcgcc tccgtggaca tttcattacc atccagaagt gtctcccact gaaggcatcc 360
gtggttgttt ttaagccaca aaaaagccac acccaagatc acctgacacc caccctgaca 420
agtgtccatg 430

<210> 388
<211> 184
<212> DNA
<213> Homo sapiens

<400> 388

ccgccccttt ccctcgcctt cggctgacgc tgacgtcgga tgagtgatcc ggagggacgc 60
tccgaccgcg gccgggaggc tcctgggggc cggggctccg aggttataat ataacttatc 120
ctctcatgct tttttcctgc cccttctccc caaatcatca acaatagaag aagaagaaaa 180
catg 184

<210> 389
<211> 57
<212> DNA
<213> Homo sapiens

<400> 389
gtgtctctgt gggcggccgc cgggttgagc tgcggcacac gtgcgacggc cgtgatg 57

<210> 390
<211> 31
<212> DNA
<213> Homo sapiens

<400> 390
gtttctttta gtttccggtg tctctgcaat g 31

<210> 391
<211> 330
<212> DNA
<213> Homo sapiens

<400> 391

cggcctctgt ctcctccccc tcccgccctt acctccacgc gggaccgccc gcgccagtca 60

actcctcgca ctttgcccct gcttggcagc ggataaaagg gggctgagga aataccggac 120

acggtcaccc gttgccagct ctagccttta aattcccggc tcggggacct ccacgcaccg 180

cggctagcgc cgacaaccag ctagcgtgca aggcgccgcg gctcagcgcg taccggcggg 240

cttcgaaacc gcagtcctcc ggcgaccccg aactccgctc cggagcctca gccccctgga 300

aagtgatccc ggcatccgag agccaagatg 330

<210> 392
<211> 22
<212> DNA
<213> Homo sapiens

<400> 392
cggcctttgc ggttccaaca tg 22

<210> 393
<211> 273
<212> DNA
<213> Homo sapiens

<400> 393

tagccttttc aaaaggcgca gcttaccgcg gtgcgcgcgg attctggact tgggcgccaa 60

ctcgtagtcc acgctccccg gggtcagcag aggggcgctc acgctctcgc cacccacctc 120

gctttctcac cccgcgcttc ccggcctggg tttttagtct tccttggagc gctctctggc 180

ctccgcctcc gccagggagc ggaaggcgga gacagcgaga ctggccaggg gggaggaaag 240

aggacgcgtg tgggcaaggg ggacaacggg atg 273

<210> 394
<211> 81
<212> DNA
<213> Homo sapiens

<400> 394

cgacctttcc cctctgcgac agtttcccga ggtacctagt gtctgagcgg cacagacgag 60

atctcgatcg aaggcgagat g 81

<210> 395
<211> 311
<212> DNA
<213> Homo sapiens

<400> 395

```
ggtcctctgc gccctggcag ccaggagtcg ccgccacgac cgccgggtct cagtgggtgc      60
ctgcgccttc tccccgcccg cctgccccgg gccatccaga aacttgctct acccgccgcg     120
ggtgctcggc agtgctgccc atggcccagc ccaggagcct atttagggcg ccggacgggc     180
tggacagagg cgcggctcag taattgaagg cctgaaacgc ccatgtgcca ctgactagga     240
ggcttccctg ctgcggcact tcatgaccca gcggcgcgcg gcccagtgaa gccaccgtgg     300
tgtccagcat g                                                          311
```

<210> 396
<211> 121
<212> DNA
<213> Homo sapiens

<400> 396

```
ctgtctcttg taggcaggga tcacagtctg aaacgacagc aaggaagagg taggcaggga      60
aaactaactg gaaggaagtt taaatacaga aagagcaaag tattatctaa ctataacaat     120
g                                                                     121
```

<210> 397
<211> 190
<212> DNA
<213> Homo sapiens

<400> 397

```
cttcctccag ggtctggaga acccagaggc agctcctcct gagtgctggg aaggactctg      60
ggcatcttca gcccttctta ctctctgagg ctcaagccag aaattcaggc tgcttgcaga     120
gtgggtgaca gagccacgga gctggtgtcc ctgggaccct ctgcccgtct tctctccact     180
ccccagcatg                                                            190
```

<210> 398
<211> 91
<212> DNA
<213> Homo sapiens

<400> 398

```
cgctcttcct ggttgggccc tgccctgagc tgccaccggg aagccagcct cagggactgc      60
agcgaccccc aaacacccct cccccaggat g                                     91
```

<210> 399
<211> 148
<212> DNA
<213> Homo sapiens

<400> 399

cttcctttcg ccgtcgccgc cgcggagcgg agtcgagccg agctgatttg atcgaggagc 60

gcggttaccg gacgggctgg gtctatggtc gctccgcggg ccgctccgcc ggctggtgct 120

tttttatcag ggcaagctgt gttccatg 148

<210> 400
<211> 81
<212> DNA
<213> Homo sapiens

<400> 400

cttcctccct agtcgcgggg agtctgagaa agcgcgcctg tttcgcgacc atcacgcacc 60

tcccctccgc ttgtggccat g 81

<210> 401
<211> 293
<212> DNA
<213> Homo sapiens

<400> 401

cctccttcct cgccgccggg gcgccctctc ggtgccactg gctctcacgt gccagtagcc 60

caccccgcat catcctctcg cctcgctcct ggagggaagt gactatatct cccccgtccg 120

ccttccatcg ccgccgcggc ggtaattctg tcgggcccgc ccgctgacgt cacctgctag 180

ccccgcctcc tctagggtcc cgggcccctg cggcgggggc tgccccgggg ggcagtcagt 240

tgaggcggcg ggagctcggc ggagggcggg ccaggtgact ggtccgggcc atg 293

<210> 402
<211> 401
<212> DNA
<213> Homo sapiens

<400> 402

aggccttttt gtgtcctgtt tgctaaaggc atgcgggcta cagcattcaa gagagggagt 60

cgttaacaaa gggaaagaga taaatgtaaa taagctcaca tttacagaat gagcggtttg 120

cagtaaaaag ctgcggcagc ccagagtctg ctactttagg ctgggctaac ctttccctgt 180

aaaaaaaaaa aaaaaaaaaa aaaaaaaaaa atggataaaa atatgcactt ccaaagggcg 240

agttgcccat ttacatgttt attagctaat tatctacagg catcagcaca ttctctcatc 300

tagcacactc tttcttgggg aggaaaatat ttcctaccgg tccatagtgt cagagtggtg 360

aacccctgca gccagcaggc ctcctgaaaa aaaagtccat g 401

<210> 403
<211> 188

<212> DNA
<213> Homo sapiens

<400> 403

gctcctcttt gcagaggggg aaactcttgg gctgagagca ggaataatgc ggtaggcaag 60

gcgggctgct ggctccccccg gctccggcag cagcggcggc agcccgagca gcggcagcag 120

cagcggcagc accccaggcg ctgacagccc cgccggccgg ctccgttgct gaccgccgac 180

tgtcaatg 188

<210> 404
<211> 187
<212> DNA
<213> Homo sapiens

<400> 404

agccctttct gtccctccca gtgaggccag ctgcggtgaa gagggtgctc tcttgcctgg 60

agttccctct gctacggctg ccccctccca gccctggccc actaagccag acccagctgt 120

cgccattccc acttctggtc ctgccacctc ctgagctgcc ttcccgcctg gtctgggtag 180

agtcatg 187

<210> 405
<211> 36
<212> DNA
<213> Homo sapiens

<400> 405
gggtctcctc gctgtcgccg ccgctgccac accatg 36

<210> 406
<211> 198
<212> DNA
<213> Homo sapiens

<400> 406

tcttctcctt taccaagatg gcggcttgtc cctgtttcgc cacagttcct accttatgag 60

ctcggttttc ttatgcttat aagagtggaa cagcaaaagc tggcaggctg acagaggcgg 120

cctcaggacg gaccttctgg ctactgaccg ttttgctgtg gttttcccgg attgtgtgta 180

ggtgtgagat caaccatg 198

<210> 407
<211> 58
<212> DNA
<213> Homo sapiens

<400> 407
gttcctccct tcttccgagc ctctcctctg gccgccgcgc gggagagagg ccgagatg 58

<210> 408
<211> 183
<212> DNA
<213> Homo sapiens

<400> 408

```
ctacctttt cgagagtgac tcccgttgtc ccaaggcttc ccagagcgaa cctgtgcggc        60
tgcaggcacc ggcgcgtcga gtttccggcg tccggaagga ccgagctctt ctcgcggatc      120
cagtgttccg tttccagccc ccaatctcag agcggagccg acagagagca gggaaccggc      180
atg                                                                    183
```

<210> 409
<211> 148
<212> DNA
<213> Homo sapiens

<400> 409

```
cagtctttcg cctcagtctc gagctctcgc tggccttcgg gtgtacgtgc tccgggatct       60
tcagcacccg cggccgccat cgccgtcgct tggcttcttc tggactcatc tgcgccactt      120
gtccgcttca cactccgccg ccatcatg                                         148
```

<210> 410
<211> 218
<212> DNA
<213> Homo sapiens

<400> 410

```
ccgcccctttt ctttctcctc gccggcccga gagcaggaac acgataacga aggaggccca      60
acttcattca ataaggagcc tgacggattt atcccagacg gtagaacaaa aggaagaata      120
ttgatggatt ttaaaccaga gtttttaaag agcttgagaa tacggggaaa ttaatttgtt      180
ctcctacaca catagatagg gtaaggttgt ttctgatg                              218
```

<210> 411
<211> 106
<212> DNA
<213> Homo sapiens

<400> 411

```
cagtctctcc cagcgaccgc cgcgggggca aggcctggag ctgtggttcg aatttgtgca       60
ggcagcgggt gctggctttt agggtccgcc gcctctctgc ctaatg                     106
```

<210> 412
<211> 581
<212> DNA

<213> Homo sapiens

<400> 412

```
agttctttcc aactttttct cggcggagtg agcgcagcgg gcgcagactc gggggcaggt       60
tgctgtgctt ctccgggctc agccgcctgc tctcctggct caggtcctcg gggagcccta      120
gacagacatc aagtggccac tggcgctcct tcccctccca gctgagccat cctccccggc      180
ctcctcgggc gggacagccc cgtgcttagg tttttctcct tttctccccc ggtgcgcctc      240
tgctcggact ctcgcgccgg gatcgcggcg gaaacctccc tcccctttcg cctcctgcgg      300
ctccttccct tcgcccctcc tccgccagtc actggaatca attccgtggg gaatcggctc      360
cgccgccgcg aaggacagcc tttccgcgcg ggactccggg gcgccacggg ggccatgtaa      420
gcagctatct tccagagggc cacactgggc atggacaccc ttttccctgc ctggaggagc      480
acaggtgata gtgtaatttt ccagtcacga aactgctaag gccatctcag gggcgtgtgc      540
gccaggatag gcgggcggcg tccgaggacc acatagccat g                          581
```

<210> 413
<211> 218
<212> DNA
<213> Homo sapiens

<400> 413

```
ctttctttta agttgataac aatcagctca ggggtttgct ctgcttgcaa ggtcactgca       60
agaatgaaca ttgaactttg gactatacct gaggggtgag gtaaacaaca ggactataaa      120
tatcagagtg tgctgctgtg gctttgtgga gctgccagag taaagcaaag agaaaggaag      180
caggcccgtt ggaagtggtt gtgacaaccc cagcaatg                              218
```

<210> 414
<211> 103
<212> DNA
<213> Homo sapiens

<400> 414

```
agctcttctc gcgcatgcgt tctccgaacg gtcttcttcc gacagcttgc tgccctagac       60
cagagttggt ggctggacct cctgcgactt ccgagttgcg atg                       103
```

<210> 415
<211> 120
<212> DNA
<213> Homo sapiens

<400> 415

tttcctcttc ctccccagca ccgacccaca ctgaccaaca caggctgagc agtcaggccc 60

acagcatctg accccaggcc cagctcgtcc tggctggcct gggtcggcct ctggagtatg 120

<210> 416
<211> 29
<212> DNA
<213> Homo sapiens

<400> 416
cctcctttcc ccagcccgcc gcggccatg 29

<210> 417
<211> 97
<212> DNA
<213> Homo sapiens

<400> 417

ggacctttcg cactcgggtc aggggtaaag cagcctgtcg cttgccgggc agctggtgag 60

tcggtgacct ggcctgtgag gagcagtgag gagaatg 97

<210> 418
<211> 175
<212> DNA
<213> Homo sapiens

<400> 418

gtgcctctga ctgtccgggt ccctccagca ttttgcagct ttctcctgtc ttgaagaagt 60

agaacggtgc ccgagaaacg tttttcccct tcgagactca ggaggatgaa agtcatcact 120

tgtgaaatag cctggcacaa caaggagccc gtgtacagcc tggacttcca gcatg 175

<210> 419
<211> 87
<212> DNA
<213> Homo sapiens

<400> 419

agccccgccc cgctcgctgg cctgccctcc tcttgctacc ctcccggcgc agagaacccc 60

ggctgctcag cgcgctccgc ggtcatg 87

<210> 420
<211> 65
<212> DNA
<213> Homo sapiens

<400> 420

tgccctcctc ttgctaccct cccggcgcag agaaccccgg ctgctcagcg cgctccgcgg 60

tcatg 65

<210> 421
<211> 176
<212> DNA
<213> Homo sapiens

<400> 421

cagcctcctg ccagcgcgct ctctgtttct ctgcagcccc gaagctcgcg aatgtagcag 60

gcgccccaag ctcggtcctc aagaagccat ggcggaatcc aggggccgtc tgtacctttg 120

gatgtgcttg gctgctgcgc tggcatcttt cctgatggga tttatggtgg gtaagt 176

<210> 422
<211> 46
<212> DNA
<213> Homo sapiens

<400> 422
ctgtctcttt aacgcgagag gaagcgatgc agaggggtgg aaaatg 46

<210> 423
<211> 163
<212> DNA
<213> Homo sapiens

<400> 423

cttccttttc tgccttctct cctgctttct agctctgggc tttcccagct ccgaagtcaa 60

tactgagatc ccagatgtgt ccagagacat cctgaagagg ctcgggggtg gaggagcctt 120

agtgtgtcca caaaggggact cctgaaactg actgagagcc agt 163

<210> 424
<211> 20
<212> DNA
<213> Homo sapiens

<400> 424
ggccctctgg cgctaccatg 20

<210> 425
<211> 81
<212> DNA
<213> Homo sapiens

<400> 425

cgcccttccc ccacccgctt ccggccgcgg ctcggttctc ccgcctccgc ctccgccgcg 60

gctcgtggtt gtcccgccat g 81

<210> 426
<211> 67

<212> DNA
<213> Homo sapiens

<400> 426

```
ctccctcctt gcagttggat ccctggcggg tgcggcccgg cccggcccgt gagcggcgca        60

cagaatg                                                                   67
```

<210> 427
<211> 93
<212> DNA
<213> Homo sapiens

<400> 427

```
actcctcctt tcccccgccc cgcctccgtt cggagagccg gcgggcgggc gcctctcggc        60

caggaagcgc ctcttggacg cgtgtgaccg atg                                     93
```

<210> 428
<211> 248
<212> DNA
<213> Homo sapiens

<400> 428

```
aggcctcttc tgaggctcta ggtgccccag tagcagggcc ttctgcagca aggccgggaa        60

ctgctgcacc attggtgtgt tttaccttaa gggactccag gcagcttcct tgctgggaag       120

atattcattt gctggggtgg ggctgggggt gcagaggtag gaagtgctgt ggctagaagg       180

cggcctggcc agcgagtagg tggtggagcg agtgagagcg tgtgcgctgt aaacagtgtg       240

agtgcatg                                                                248
```

<210> 429
<211> 930
<212> DNA
<213> Homo sapiens

<400> 429

aggcctcttc tgaggctcta ggtgccccag tagcagggcc ttctgcagca aggccgggaa 60
ctgctgcacc attggtgtgt tttaccttaa gggactccag gcagcttcct tgctgggaag 120
atattcattt gctggggtgg ggctgggggt gcagaggtag gaagtgctgt ggctagaagg 180
cggcctggcc agcgagtagg tggtggagcg agtgagagcg tgtgcgctgt aaacagtgtg 240
agtgcatgtg cgccagcgcg tgcaaggaca cggtaaggga tgtacatgta ttgtctcgtg 300
agtaagagct tgtgtgtgtg ttgggatggg aagacacgta ctggtatgag agcccgcgtg 360
agaagtgtat gtgtgagtac tcgcgtggaa gttttgcact cgggtttgag gctgtgcaaa 420
agtacgcatg gctcaccagg tgtggggctg tgtgggctgc ctcgtgtgtg ccagcccgtg 480
tgcaggcctg ttttgtgaga gccttcaggg aacgcatgag cacgtgtgcc agtgcgagtg 540
cgggacgcgg ggaggcggga gagaccgagt gggaggcccc gcgaaggagt gggagtggga 600
gtgggagtgc cggcgggaga cctgcggggg cgcgcccggg ctgacgcgtg cgcgccagtg 660
cgcgtgagtg cgggcgcgcg ccgccgcccc ccgccggggt cggagccggt tgccatggga 720
acgcgccgcg gcccgagtta atcatttcct gtggaaagtg tgcgggaggg gcgcgagcgg 780
gctggccgag gaggaggcgg cggcgtggag ctgcctcctg ccggcgggcc gggccgggcc 840
gagccccggg cgctgcggcg acgcctggat cctgcctccg ccaggccggc tgcctggtgc 900
cccgaggagg ctgctgagcc ccaggccatg 930

<210> 430
<211> 28
<212> DNA
<213> Homo sapiens

<400> 430
cctcctccgc gttccagaat ccaagatg 28

<210> 431
<211> 263
<212> DNA
<213> Homo sapiens

<400> 431

cgttctctcc cgcggaattc aggtttacgg ccctgcgggt tctcagaggc aagttcagac 60
cgtgttgttt tcttttcacg gatcctgccc tttcttcccg aaaagaagac agccttgggt 120
cgcgattgtg ggcttcgaa gagtccagca gtgggaattt ctagaatttg gaatcgagtg 180
cattttctga catttgagta cagtacccag gggttcttgg agaagaacct ggtcccagag 240
gagcttgact gaccataaaa atg 263

<210> 432
<211> 383

<212> DNA
<213> Homo sapiens

<400> 432

cttccttctg caacaggcgt gggtcacgct ctcgctcggt ctttctgccg ccatcttggt 60
tccgcgttcc ctgcacagta agtactttct gtgccgctac tgtctatccg cagccatccg 120
cctttctttc gggctaagcc gccccgggga ctgagagtta aggagagttg gaggctttac 180
tgggccacag ggttcctact cgcccctggg cctccggaca aaatggggtc tgcggttggt 240
gtcctggcaa aagcagggta gaagggctgc ggggcgggcc cagaatccga gcctgcagag 300
atgggagcag ttgcagtgtt gagggcggaa gaggagtgcg tcttgttttg ggaactgctt 360
cacaggatcc agaaaaggaa atg 383

<210> 433
<211> 181
<212> DNA
<213> Homo sapiens

<400> 433

cgcccttcgc cgctgagctc gcagcctccg gcgcccacct ccacctccag tgtcccgcct 60
cgggccgtcg ccctccagcg gctcgcgagc gtgggagacg tacctgggca ggcactgtcc 120
agcccaggcc caggcacagc cgtgaggggc gaggcacggg gacatcctgg cggccaccat 180
g 181

<210> 434
<211> 56
<212> DNA
<213> Homo sapiens

<400> 434
ccgcccctcc cgcaacgctc gaccccagga ttcccccggc tcgcctgccc gccatg 56

<210> 435
<211> 170
<212> DNA
<213> Homo sapiens

<400> 435

ccctcttctt tagactgcca cgaggaaaaa gcagatgtga gaactcaagg ttcagggctg 60
ctcttctaag aaacaagtct gccataatct ccatctgtgt tggaatctgt taactaatga 120
actggtctct gtgcaaatcc tgagtgctaa agcttccaac aagactgatg 170

<210> 436
<211> 96
<212> DNA

<213> Homo sapiens

<400> 436

```
cgctctctta cactcgggcc tcagaagtcc gtgccagtga ccggaggcgg cggcggcgag        60
cggttccttg tggctagaa gaatcctgca aaaatg                                   96
```

<210> 437
<211> 517
<212> DNA
<213> Homo sapiens

<400> 437

```
agtccttctc attccttgcc cccgcccaag gctctcttca ccttccccgc gggggtcctc        60
tcgttttctg tctcccaaat gctggcttcc cgcctttcct cccccgctta tttacttaat       120
taaggccctg gggctgcacc ccaccggcag ctccttcggg ggtgtggccg aagagctccg       180
agggcggggc tgaccgagcc atattcgggc gtggccggtg gtgattggtg agggcggggc       240
ctgccgcagg gggcggggcc tgcaggtttg gcccccgcag ggagcgcagc tggcgccgct       300
gggagctggt ggcgcggcgc aggtcccggc cgagtgtggc gcagcagtgg cggcgcttcc       360
cattcgccat gcgccggggg tgggtgcccg aaggttgcat gatggaattt gaacattact       420
tcaagaggtt ttgtattttg gattagttaa ttgggtttgt cctctgctga ctgtttcttc       480
ggatgcattt tttggtgtgc tcttgaggga ttaaatg                                517
```

<210> 438
<211> 203
<212> DNA
<213> Homo sapiens

<400> 438

```
cgtccttccg gctctcggct ttgccacaaa gcttcccgaa gacgcggccg ctacccggag        60
acgcggtcgc cacccagaag cgctctcccg ggaagccccg ctcgtgggac cgcgccacct       120
gcgccgcctc tgcggcccgc agcccgacgg gcgccgccat gttggggtcc tagcgaggga       180
cgcgtaggtg tcttcataag atg                                               203
```

<210> 439
<211> 444
<212> DNA
<213> Homo sapiens

<400> 439

cagtcctttt gcaagagctg ctaagagcgc tgggtaagga gaggaagggg agagacatgg 60
aacttggctg gtctgcaggg aaatgccact gttttggccg ggagtagggg gcgggagtgg 120
cgggagaggg ggtggccggc tggggaggag ccagcctggt ggagaagctg ccctgtgggc 180
gggggtgagg aggggagggc tgtggtcacc aggcaggaag gaggggtggc ctgacccctc 240
ggcagtccct cccctcagcc tttccccaaa ttgctacttc tctggggctc caggtcctgc 300
ttgtgctcag ctccagctca ctggctggcc accgagactt ctggacagga aactgcacca 360
tcctcttctc ccagcaaggg ggctccagag actgcccacc caggaagtct ggtggcctgg 420
ggatttggtg ggtctgctcc ttag 444

<210> 440
<211> 656
<212> DNA
<213> Homo sapiens

<400> 440

cctcctttct ccttccctct tgcctccagt gactgtctcc aggatttctc tcttcctatt 60
tcaggaggac tctcacaggc tcccacagcc tgtgttaagc tgaggtttcc cctagatctc 120
gtatatcccc aacacatacc tccacgcaca cacatcccca agaacctcga gctcacacca 180
acagacacac gcgcgcatac acactcgctc tcgcttgtcc atctccctcc cgggggagcc 240
ggcgcgcgct cccacctttg ccgcacactc cggcgagccg agcccgcagc gctccaggat 300
tctgcggctc ggaactcgga ttgcagctct gaacccccat ggtggttttt taaacacttc 360
ttttccttct cttcctcgtt ttgattgcac cgtttccatc tgggggctag aggagcaagg 420
cagcagcctt cccagccagc ccttgttggc ttgccatcgt ccatctggct tataaaagtt 480
tgctgagcgc agtccagagg gctgcgctgc tcgtcccctc ggctggcaga aggggggtgac 540
gctgggcagc ggcgaggagc gcgccgctgc ctctggcggg ctttcggctt gaggggcaag 600
gtgaagagcg caccggccgt ggggtttacc gagctggatt tgtatgttgc accatg 656

<210> 441
<211> 80
<212> DNA
<213> Homo sapiens

<400> 441

cggccttctg ggcgcgcgcg acgtcagttt gagttctgtg ttctccccgc ccgtgtcccg 60
cccgacccgc gcccgcgatg 80

<210> 442

<211> 36
<212> DNA
<213> Homo sapiens

<400> 442
agttccttcc ccagaaggag agattcctct gccatg 36

<210> 443
<211> 309
<212> DNA
<213> Homo sapiens

<400> 443

ggctctttct gcgagcgggc gcgcgggcga gcggttgtgc ttgtgcttgt ggcgcgtggt 60

gcgggtttcg gcggcggctg aggaagaagc gcgggcggcg ccttcgggag gcgagcaggc 120

agcagttggc cgtgccgtag cagcgtcccg cgcgcggcgg gcagcggccc aggaggcgcg 180

tggcggcgct cggcctcgcg gcggcggcgg cggcagcggc ccagcagttg gcggcgagcg 240

cgtctgcgcc tgcgcggcgg gccccgcgcc cctcctcccc ccctgggcgc ccccggcggc 300

gtgtgaatg 309

<210> 444
<211> 121
<212> DNA
<213> Homo sapiens

<400> 444

cggccttcca tcccagtttc ttctaggaat tcggagcctc ccctgcagcg actcggaaga 60

ttcgaggcgg cgggggacaa gtcggcgccc cagagcggac gagtcaccag gtgtcaagat 120

g 121

<210> 445
<211> 54
<212> DNA
<213> Homo sapiens

<400> 445
ccgcctttct ccgctggcaa cggcgccgct ccccgctcct cctccccagc catg 54

<210> 446
<211> 49
<212> DNA
<213> Homo sapiens

<400> 446
ctcccttccc ttgcatgctg cattgtgtcg ggagttgctg acagccatg 49

<210> 447
<211> 210
<212> DNA

<213> Homo sapiens

<400> 447

ccgccttcct agtggagacg cgagtggggg aggagcagtc cgaggggaac gtgggttgaa 60

cgttgcaact agggtggaga tcaagctgga acaggagttc cgatcgaccc ggtaccaaga 120

aggggagtgc ccgcggcagg gttcattgaa aaaatcctta gtgatattga catgtctcaa 180

gtgacataaa ttagccaatg actcggaatg 210

<210> 448
<211> 63
<212> DNA
<213> Homo sapiens

<400> 448

tggcctttgt caagtcatcc cctcttctcc tcaggaactg ctcaaacctg tgccccaaag 60

atg 63

<210> 449
<211> 17
<212> DNA
<213> Homo sapiens

<400> 449
ggatctcttt cgccatg 17

<210> 450
<211> 104
<212> DNA
<213> Homo sapiens

<400> 450

ctgtctccct cggcctgtgc cgccgccgac gccgcttgtg ggcccgactc cgctctgtct 60

gcttcgccac cttctccccg agcactgccc ggccggccgc catg 104

<210> 451
<211> 134
<212> DNA
<213> Homo sapiens

<400> 451

catcctctct tccaccctct cttctccctg gtcaaccgct ctgcaaacaa ccatcaatct 60

gatcccacag gcctgagaaa gtctgctctc cagtacctgc tgctgatctg tttcagccga 120

caagaggcac catg 134

<210> 452

<211> 47
<212> DNA
<213> Homo sapiens

<400> 452
ctgcctttcg atctctccac atctcggtgg cgcgggatct caagatg 47

<210> 453
<211> 76
<212> DNA
<213> Homo sapiens

<400> 453

aatcctttct cctgccgcag tggagaggag cggccggagc gagacacttc gccgaggcac 60

agcagccggc aggatg 76

<210> 454
<211> 118
<212> DNA
<213> Homo sapiens

<400> 454

gagccttttc tcgctaacac cgctcgccct ctccgagtca gttccgcggt agaggtgacc 60

tgactctctg aggctcattt tgcagttgtt gaaattgtcc ccgcagtttt caatcatg 118

<210> 455
<211> 132
<212> DNA
<213> Homo sapiens

<400> 455

gtttctctat cagtcgcgca gctgtgttcg cggactcagg tggaaggaat ttcttctctt 60

cgttgacgtt gctggtgttc actgtttgga attagtcaag tttcgggaat caccgtcgct 120

gccatcaaca tg 132

<210> 456
<211> 72
<212> DNA
<213> Homo sapiens

<400> 456

ggttctctct ctccagaagg ttctgccggt tcccccagct ctgggtaccc ggctctgcat 60

cgcgtcgcca tg 72

<210> 457

<211> 56
<212> DNA
<213> Homo sapiens

<400> 457
caacctctcc tcttcgtctc cgccatcagc tcggcagtcg cgaagcagca accatg 56

<210> 458
<211> 34
<212> DNA
<213> Homo sapiens

<400> 458
ctttctcccg aacgccagcg ctgaggacac gatg 34

<210> 459
<211> 32
<212> DNA
<213> Homo sapiens

<400> 459
ctctctctgc accttccttc tgtcaataga tg 32

<210> 460
<211> 141
<212> DNA
<213> Homo sapiens

<400> 460

cggcctccat gtgcgacgtg ttctccttct gcgtgggcgt ggcgggccgc gcgcgggtct 60

ccgtggaagt ccgtttcgtg agcagcgcca aggtgaggtc ggggcgggtc ctgccgggag 120

cctctcccca gtccggccat g 141

<210> 461
<211> 84
<212> DNA
<213> Homo sapiens

<400> 461

ctgccttttt acagctagac ctgtgtgctg caaggagcta aggccttcag tgtccccttc 60

cttacccagg tttctcacag aatg 84

<210> 462
<211> 493
<212> DNA
<213> Homo sapiens

<400> 462

ccgccccttg caccgcccac gtggccagcg ccacctgcct cattgtgccc aggagttctc 60

caaacccgcg ctgcggagtg agtgaccaag ttccggccag ttcgacctcg aggatccaga 120

ggtggagacg gtactacctc ccagctctgt tttccatccc cttcaggtcc ttcctcggga 180

ggcggcgaag gcggtccacc ctgcgcgtga tcctttatgc ccggcccctg cccctccctc 240

cgggtggaac ttcccccctca ccgccagact taagctgagg atcgttggat ctctggcggg 300

gtgcagaact gagcccaggc cacagtaccc tattcacgct ctgtgcttgt gccaaggttt 360

caagtgatcc tcccgcctca gcctgcccag gtgctgagat tacatgtatg agccactgca 420

cctggaaagg agccagaaat gtgaagtgct agctgaagga tgagcagcag ctagccaggc 480

aaagggggca atg 493

<210> 463
<211> 275
<212> DNA
<213> Homo sapiens

<400> 463

tgttcttccc ccacctgcca cgtacagagc ccaagttctc gctaggcttg ttgggtcagc 60

gcgattggcc ggggcccgcg cgagcctgcg agcgaggtgc ggcggtcgcg aagggcaacc 120

gagggggccg tgaccaccgc ctccccgcga cgccccagtc cagtggcctc gcgtccgccc 180

attcagcgga gacctgcgga gaggcggcgg ccgcggcctc cgcaagccgt ctttctctag 240

agttgtatat atagaacatc ctggagtcca ccatg 275

<210> 464
<211> 133
<212> DNA
<213> Homo sapiens

<400> 464

catccttctc aaaagactta ttgacagtgc caaagctcgg tactggacac aacgagggac 60

ctgggtctac gataacgcgc ttttgctcct cctgaagtgt ctttggtcca acgttgttcc 120

agagtgtacc atg 133

<210> 465
<211> 117
<212> DNA
<213> Homo sapiens

<400> 465

ttttctctct ctctttcact gcaaggcggc ggcaggagag gttgtggtgc tagtttctct 60

aagccatcca gtgccatcct cgtcgctgca gcgacacacg ctctcgccgc cgccatg 117

<210> 466
<211> 79
<212> DNA
<213> Homo sapiens

<400> 466

caccctctcg gggagggagt tggggaagct gggttggctg ggttggtagc tcctacctac 60

tgtgtggcaa gaaggtatg 79

<210> 467
<211> 96
<212> DNA
<213> Homo sapiens

<400> 467

tctccttcct gcgcgcgcgc ctgaagtcgg cgtgggcgtt tgaggaagct gggatacagc 60

atttaatgaa aaatttatgc ttaagaagta aaaatg 96

<210> 468
<211> 165
<212> DNA
<213> Homo sapiens

<400> 468

cagtctttcc tgctaagcct cagcgtctcc tccaagccac atcaaaatct ttccttctgg 60

gcctttccca gaagtgaatt cttgctggaa ggtataaaag accagctcct ccaagcagag 120

caactccctg gctgccgtga aaagacaagg cactgggcag tgatg 165

<210> 469
<211> 49
<212> DNA
<213> Homo sapiens

<400> 469
ctgcctttac aacagaggga gacgatggac tgagctgatc cgcaccatg 49

<210> 470
<211> 36
<212> DNA
<213> Homo sapiens

<400> 470
cgccctctgc ggtgaaggag agaccacact gccatg 36

<210> 471
<211> 192
<212> DNA
<213> Homo sapiens

<400> 471

aagcctcttt catcggggca cagacttcct tttacttctt ccttttgccc tctcgcctcc 60
tcctcctggg aagaagcgga ggcgccggcg gtcggccggg atagcaacag gccgggccac 120
tgaggcggtg cggaaagttt ctgtctggga gtgcggaact ggggccgggt tggtgtactg 180
ctcggagcaa tg 192

<210> 472
<211> 531
<212> DNA
<213> Homo sapiens

<400> 472

cgccctttat tcttgctcgg cctcgccaca gagagcaaat cagattggct gggcgacaac 60
ctcaaagggc ggggctgcac acgttcacta cgggaatgag gtagcggtgg agggggcagt 120
tgggcgggga taggccgtcc tagctaaggt ggtaaaggcc aataactctt caggctgcct 180
ctcctcgaaa agtcatcttc tcgcgaacct ttaaaatgcc ttcctcccca agcacctcaa 240
gggactagaa ctgagtgctt catttgtctt ttttcctcct tgcaaaagtc ccgtttgcca 300
ccatggggat gtaccaagtg agaccgagta gggggaacga gtggtgattg acgcgccagg 360
ttactggcca ctgctcacct aggcgctagc aaacttctgc caagatcgga actgagtact 420
aaacagcctc cacagttctc cctggtgccg tctccggctt ggcgccgcat cctcctctgg 480
gctcgcgatg gccgcgtccc ctcccgctgc ggacgggtcc tttggtacat g 531

<210> 473
<211> 167
<212> DNA
<213> Homo sapiens

<400> 473

ctgcctcttt ccggctgtga ccctcctcgc cgccgccgct tggctgcgtc ctccgactcc 60
ccgcgccgcc gagaccaggc tcccgctccg gttgcggccg caccgccctc cgcggccgcc 120
ccctggggat ccagcgagcg cggtcgtcct tggtggaagg aaccatg 167

<210> 474
<211> 122
<212> DNA
<213> Homo sapiens

<400> 474

aactcctttc cccctgctgt gacgtacagg tgaggtaaac agtactgaag tccagggcgt 60
cggtgctcac tgctctggca atgcccggtg agactgaatt atgtttaaat ttattgtaga 120
tg 122

<210> 475
<211> 62
<212> DNA
<213> Homo sapiens

<400> 475

ggctccttcc cagcccccgg cctagctctg cgaacggtga ctgcccatcc ttggccgcaa 60

tg 62

<210> 476
<211> 91
<212> DNA
<213> Homo sapiens

<400> 476

gacccttttc ccctccccgg gccacccagc ccgcccaact cccagcggag agcaaggttt 60

tcttctgttt tcatagccag ccagaacaat g 91

<210> 477
<211> 33
<212> DNA
<213> Homo sapiens

<400> 477
ttttcttttc ctctaggcag agaagaggcg atg 33

<210> 478
<211> 259
<212> DNA
<213> Homo sapiens

<400> 478

ctccctcttt gctctaacag acagcagcga ctttaggctg gataatagtc aaattcttac 60

ctcgctcttt cactgctagt aagatcagat tgcgtttctt tcagttactc ttcaatcgcc 120

agtttcttga tctgcttcta aaagaagaag tagagaagat aaatcctgtc ttcaatacct 180

ggaaggaaaa acaaaataac ctcaactccg ttttgaaaaa aacattccaa gaactttcat 240

cagagatttt acttagatg 259

<210> 479
<211> 22
<212> DNA
<213> Homo sapiens

<400> 479
gcttctcttt ctggtcaaaa tg 22

<210> 480

<211> 74
<212> DNA
<213> Homo sapiens

<400> 480

ccgcctttg tgctgcggcc gcggagcccc cgagggccca gtgttcacca tcataccagg 60

ggccagaggc gatg 74

<210> 481
<211> 75
<212> DNA
<213> Homo sapiens

<400> 481

tggtctcttc ggtctcctgc cgcccccggg aagcgcgctg cgctgccgag gcgagctaag 60

cgcccgctcg ccatg 75

<210> 482
<211> 192
<212> DNA
<213> Homo sapiens

<400> 482

ccccctctcc ctccctcctt gcgggccctc ctccccttcc ctcccctccg ccccccttccc 60

cgtaggcagc ccgcccgcca gtccgcccgc accgcctcct tcccagcccc tagcgctccg 120

gctgggtctc tcccccgccc cccaggctcc cccggtcgct ctcctccggc ggtcgcccgc 180

gctcggtgga tg 192

<210> 483
<211> 470
<212> DNA
<213> Homo sapiens

<400> 483

tcgccttctg cccgggcgct cgcagccgag cgcggccggg gaagggctct cctcccagcg 60
ccgagcactg ggccctggca gacgccccaa gattgttgtg aggagtctag ccagttggtg 120
agcgctgtaa tctgaaccag ctgtgtccag actgaggccc catttgcatt gtttaacata 180
cttagaaaat gaagtgttca tttttaacat tcctcctcca attggtttaa tgctgaatta 240
ctgaagaggg ctaagcaaaa ccaggtgctt gcgctgaggg ctctgcagtg gctgggagga 300
ccccggcgct ctccccgtgt cctctccacg actcgctcgg cccctctgga ataaaacacc 360
cgcgagcccc gagggcccag aggaggccga cgtgcccgag ctcctccggg ggtcccgccc 420
gcgagctttc ttctcgcctt cgcatctcct cctcgcgcgt cttggacatg 470

<210> 484
<211> 42
<212> DNA
<213> Homo sapiens

<400> 484
cgctcttcct tccgcttgcg ctgtgagctg aggcggtgta tg 42

<210> 485
<211> 193
<212> DNA
<213> Homo sapiens

<400> 485

tcgtcttctc tgtcttaggg ctggtgctgg ccctgcccac gcctagggct ccggcgcgtc 60
acgggcctca gctgggattc ccgcgcccct cggacggcca cgagactcgg acatctttcc 120
aggaacagcg tgaggaggac agaagcaccc aacaggactg ctcaagccac ctgcgaacac 180

tgctgctacc atg 193

<210> 486
<211> 66
<212> DNA
<213> Homo sapiens

<400> 486

agttcccttt tccggtcggc gtggtcttgc gagtggagtg tccgctgtgc ccgggcctgc 60
accatg 66

<210> 487
<211> 36
<212> DNA
<213> Homo sapiens

<400> 487
cctcctcttg acgtggcaga ggcggcgcca gccatg 36

<210> 488
<211> 77
<212> DNA
<213> Homo sapiens

<400> 488

cgtcctcttt cctttccttc tccctcccct tttcccttcc ttcgtccctt ccttccttcc 60

tttcgccggg cgcgatg 77

<210> 489
<211> 110
<212> DNA
<213> Homo sapiens

<400> 489

ccgcccctcc cgctggatcc cgcagccgcg gctcttcccg acgcgttccg ccttccccag 60

ctgtgcactc tccatccagc tgtgcgctct cgtcgggagt cccagccatg 110

<210> 490
<211> 125
<212> DNA
<213> Homo sapiens

<400> 490

gcttctcttt cctttcgcgc cggttgccgc tgcggagcgc ggcgggtcca tgtgcgcagt 60

gagtggcgct attcctggcc cagtagcacc cgagccccgg gtttgaccga gtccgcgctg 120

cgatg 125

<210> 491
<211> 625
<212> DNA
<213> Homo sapiens

<400> 491

```
gctcctttgc gcacgcgcgc cgcttcccag tggcaagcgc gggcaggacc gcgttgcgtc        60
atcggggcgc gcgcctcaga gagagctgtg gttgccggaa gttgagcggc ggtaagtgag       120
ccgcggcggg cgagggtgta gtggggtctt gctgggccgg ttttggaggc ctggagtcaa       180
ggggcgagct cgccagggag ggcgagggtc acagcaagtc tcaggatcct cctctgccag       240
tttctgggtg gtccttcctc ctccagggac tcactgattc cggctggcgc ccttcgtctg       300
tagccgcgtc ccctcagact ggttcagtcc ggggtcttct gacttggaag ctcgtgctga       360
tttcctaagt cagcccctcc tgtcctcttg gtaggcagtg ctcagaatct tcagtgttgg       420
aacacgggag atgggacatt tggattccca gcctggctgt gtctggattt gctgtctctg       480
gcacgttcct tccccatcta agctgctttt ccatctgcaa aatgggaatg ataatccgcc       540
atttgtttaa gtgaggaggt taaataagtt tactttctga gaaagaagat tctcgattcc       600
ttggttacag ggttagaaac taatg                                             625
```

<210> 492
<211> 115
<212> DNA
<213> Homo sapiens

<400> 492

```
cgctcccttt gccgccgcct tagcccggga cccgaaccca gcctctcccc tacccgaaca        60
ccggccccgg ctccaccgag gcccgggtcc cccagcccgt ctcgccgccg ccatg            115
```

<210> 493
<211> 224
<212> DNA
<213> Homo sapiens

<400> 493

```
agcccccttt tccctccatg gtttctctcc gctcccgtga gtaacttggc tccgggggct        60
ccgctcgcct gcccgcacgc cgcccgccac ccaggaccgc gccgccggcc tccgccgcta       120
gcaaaccctt ccgacggccc tcgctgcgca agccgggacg cctctccccc ctccgccccc       180
gccgcggaaa gttaagtttg aagagggggg aagaggggaa catg                        224
```

<210> 494
<211> 86
<212> DNA
<213> Homo sapiens

<400> 494

```
acttctcttt tccctagact gcagccagcg gagcccgcag ccggcccgag ccaggaaccc        60
aggtccggag cctcaacttc aggatg                                             86
```

<210> 495
<211> 145
<212> DNA
<213> Homo sapiens

<400> 495

cgctccctct gggcttccgt cctccgcccg cgcccgacgg agcctgttcg cgtcgactgc 60

ccagagtccg cgaatcctcc gctccgagcc cgtccggact cccccgatcc cagctttctc 120

tcctttgaaa acactaagaa taatg 145

<210> 496
<211> 159
<212> DNA
<213> Homo sapiens

<400> 496

aggccccctt ctccgcctcc gcctcctccc gacgccggcg ccgctttctg gaaggttcgt 60

gaaggcagtg agggcttacc gttattacac tgcggccggc cagaatccgg gtccatccgt 120

ccttcccgag ccaacccaga cacagcggag tttgccatg 159

<210> 497
<211> 38
<212> DNA
<213> Homo sapiens

<400> 497
gcttctttcc cgagcttgga acttcgttat ccgcgatg 38

<210> 498
<211> 69
<212> DNA
<213> Homo sapiens

<400> 498

acccctcttt tctagagttc tgcctcgctt cccggcgcgg tcgcagccct cagcccactt 60

aggataatg 69

<210> 499
<211> 55
<212> DNA
<213> Homo sapiens

<400> 499
ctcccttctg cctgggacgt cagcggacgg ggcgctcgcg ggccggggct gtatg 55

<210> 500
<211> 1300
<212> DNA
<213> Homo sapiens

<400> 500

```
aagccctttc cgaggatggc aaaggatctg ggaatgcttc tccaaagata tgtggatgga      60
cgaaataggt ctctggtgat actgaggcgg ggtggggacg gggaggcaaa gacttggctt     120
cttaggaatt ggaagaaata agtaaacaat gtttggtagc aatttgtaat aaggaagtaa     180
tcataaaatt aactacgtcc gtttctgatt gtgtcaactt tgtcaaggag tagaagttta     240
agaattgaat actgtcctgc aaacaacgta acctcatctc ctgtttgaca caccctgttg     300
agaagcagtc ctttacctcc taaatttctt tttcgaaatt atcatttcct ttatggactg     360
agaataacac tgcctgttca ctcccaccga gctgtgaaca gtgaccttaa ttcttccaag     420
cagggaagtg tagaaactaa ggtctgtgac agaccgcaaa atcatctccc aatctttaag     480
gaaaatcaga atcacgcata atcccataga gataaatttg atgcatagtc ttttcctatg     540
catacatttt tccttttttt ttacaataat tgaattttta tattttttca gcttgcttct     600
gtcacttaat atattatgag taattttttt tggttttttt tgttttggag acagaatctc     660
gcactgtcgc ccgggttgga gtgcagtggc gcgatctcgg ctcactgcaa cctctgcctc     720
ccggcttcaa gcgattctcc tgtctcagcc tccctagtag ctgggattac aggcacccgc     780
caccacgccc agctaatttt tttgtgtgtt tttagtagag aaggggtttc actatattgg     840
ccaggctggt ctcaaactcc tgacctcatg atacgcccac ctcggtctcc caaagtgcta     900
ggattacagg cctgagccac cgcgccagcc tattatgaat aattttctac atgaatacgc     960
atcgtactaa ataactttaa atgttggtgt agtatgccat tgtatgggta tggcatcatt    1020
tattgttaga cgttagattg tttccactaa gtcggtatta taaagagaac taatgacttc    1080
attattatta gctttttctt tctttggaca caatatccaa aaagaaattg ttgtttcaaa    1140
gatatgcaag atttttaagg ctttttgata tgtattgtca aattgccctc cagaaagaat    1200
acatgaattt acactcagca gctctgcttc cagcgtgaaa gactttctat tgtaccattt    1260
tggtgttttt tccctagctc tcagactccc cagtacaatg                          1300
```

<210> 501
<211> 539
<212> DNA
<213> Homo sapiens

<400> 501

gtgtctcccg gtcgcgcgtg gaggtcggtc gctcagagct gctgggcgca gtttctccgc 60

ctgctgcttc ggcgcggctg tatcggcgag cgagcgagtt cccgcgagtt ctcggtggcg 120

ctccccccttc ctttcagtct ccacggactg gcccctcgtc cttctacttg accgctcccg 180

tcttccgccg ccttctggcg ctttccgttg ggccgattcc cgcccgcttc ctcctgcttc 240

ccatcgaagc tctagaaatg aatgtttcca tctcttcaga gatgaaccag attatgatgc 300

atcattatca cagaagaaat tcgtgtctat agcttttaag gacttgatta catcattttc 360

aagcctgata gttttggaat caccattaga gcttaagaca cacctgcctt catttcaacc 420

acctgtcttc ataccctgac gaagtgcacc ttttaacact cctttgtcct tggattactt 480

aagagttccc agaaatacat ttgccaccaa cagagtagcc aaatttataa ggaaaaatg 539

<210> 502
<211> 310
<212> DNA
<213> Homo sapiens

<400> 502

acccctcttt ttctccaaag gagtgcttgt ggagatcgga tcttttctcc agcaattggg 60

ggaaagaagg ctttttctct gaattcgctt agtgtaacca gcggcgtata ttttttaggc 120

gccttttcga aaacctagta gttaatattc atttgtttaa atcttatttt atttttaagc 180

tcaaactgct taagaatacc ttaattcctt aaagtgaaat aattttttgc aaaggggttt 240

cctcgatttg gagctttttt tttcttccac cgtcatttct aactcttaaa accaactcag 300

ttccatcatg 310

<210> 503
<211> 104
<212> DNA
<213> Homo sapiens

<400> 503

ttttcctttc ttagccaaat caccaaaatg tccagttaga acaagaattt agcattctgc 60

aaaagaagtt aacagctgag ataacgagga aatattctga aatg 104

<210> 504
<211> 134
<212> DNA
<213> Homo sapiens

<400> 504

ggttcttctg ggcgctaagg gagctgacgg agagggccac cgcccagcaa tagacggtgc 60

ctcagcctgc cgagccgcag tttccgtggt gtgagtgagt ccgggcccgt gtcccctctc 120

ccgccgccgc catg 134

<210> 505
<211> 296
<212> DNA
<213> Homo sapiens

<400> 505

cggcccttcg cagcgggcgc gctgtcagac ctcagtctgg cggctgcatt gctgggcgcg 60

ccgctctcgt ctgatccctg ctggggacgg ttgcccgggc aggatccttt acgatccctt 120

ctcggtttct ccgtcgtcac agggaataaa tctcgctcga aactcactgg accgctccta 180

gaaaggcgaa aagatattca ggagcccttc cattttcctt ccagtaggca ccgaacccag 240

cattttcggc aaccgctgct ggcagttttg ccaggtgttt gttaccttga aaaatg 296

<210> 506
<211> 231
<212> DNA
<213> Homo sapiens

<400> 506

cgctctctgg caagaggcaa gaggtagcaa cagcgagcgt gccggtcgct agtcgcgggt 60

ccccgagtga gcacgccagg gagcaggaga ccaaacgacg ggggtcggag tcagagtcgc 120

agtgggagtc cccggaccgg agcacgagcc tgagcgggag agcgccgctc gcacgcccgt 180

cgccacccgc gtacccggcg cagccagagc caccagcgca gcgctgccat g 231

<210> 507
<211> 152
<212> DNA
<213> Homo sapiens

<400> 507

gggtctttgt ctcgctgcag cgggtgctgc aggtctggcc ttcacttttc tgcgtcctct 60

tactcctaga ggcccagcct ctgtggcgct gtgatctggt tattgggaga ttcacagcta 120

agacgccagg atcccccgga agcctagaaa tg 152

<210> 508
<211> 150
<212> DNA
<213> Homo sapiens

<400> 508

ctttctttga cgcaagggct cgagacgcag ccgccgtcgg ccgagcgccc ggctagaagc 60

gacaccagac ggagcctccg gagttcctcc gcccccacct cgccgggtcc tggagccgca 120

gtcctcccag ctgccctcct cgtggccatg 150

<210> 509
<211> 608
<212> DNA
<213> Homo sapiens

<400> 509

ctgtcttttc tccagtttga gcgggggtgt cgggagcagg cggagagctt tcctgcgagg 60

ctgtggaagc agtgaacact cttctcagcg gctcgcctcc cagcagtgct atttttttgcc 120

atccgccctc acccccagca cacgcgctcg cacacacacg cacgcacgca cacacacaca 180

cacacacact cacacagaga cctctctggg tttctttgcc ttgagtctcc cggggctgtg 240

agaagccagg cgcatctcaa accgagctgg cagctccagg ctccggagcc atgccctgca 300

cggaccctcg tctttaccac gctcctgagg aatgaaagga acccagggac cctcagaagg 360

cagcagtgat gcggaccaac cccccggagc ctgcaccctt ccgagggcca taggcgaccc 420

agggaactgg agagagctcc agaaaggaaa tcccagcttt cccaaagtcc ctgtggatgc 480

tgacaaaagg agacctgaat ttttggaaga gcctgtacta ggttacccgg ctgcagagtg 540

attttcccct ccggcactga ctctccccct ccaaccccca gccgtccaga gtaccatgaa 600

gaattatg 608

<210> 510
<211> 32
<212> DNA
<213> Homo sapiens

<400> 510
ttccctctcc gctgcgtccc cgcgcgaaga tg 32

<210> 511
<211> 56
<212> DNA
<213> Homo sapiens

<400> 511
cttcctccgc ggtcttccga gcggtcgcgt gaactgcttc ctgcaggctg gccatg 56

<210> 512
<211> 197
<212> DNA
<213> Homo sapiens

<400> 512

cgctctttcc gcgcggtgca ttctggggcc cgaggtcgag cccgccgctg ccgccgtcgc 60
ctgagggaag cgagaagagg ccgcgaccgg agagaaaaag cggagtcgcc accggagaga 120
agtcgactcc ctagcagcag ccgccgccag agaggcccgc ccaccagttc gcccgtcccc 180
ctgccccgtt cacaatg 197

<210> 513
<211> 1250
<212> DNA
<213> Homo sapiens

<400> 513

ttttcttcct ggtggcgttt gggcttaata cagctttggc gaggtcggat gacgggtggg 60
agccagcggt ggaaggggtg gcgaaagtac cggtttgccc caggccgccg aggggcctcc 120
ttagagagac cttgcctgct ccgctcgcgt ccgccggggc cgcgcgggtc ctcctggcgc 180
cgccaggttc aaaaagccac tcgagttgtc actgcgacgg ccctgggcca ggagccgttt 240
cgggatctgt caaacaacga gttttcgtcg ttcgaatcag gttgactggt ccttcatccc 300
cccaatctcc cgtacctggc gagtccagct cgtcgcggca atgctaagaa aagagtgata 360
tgcaagctga gaccaaaaat atggtatgat ttagccatac tgaaggggaa ggaaataaga 420
gctgggcaaa gcattctgtg aattggctga ctccacttct atggtgagag agaggagtgc 480
atcaaagatt actcccagta gagatggttt cagcatgttg gccagtctgg tctcagactc 540
ctgacctcaa gtgatccacc cacctcggcc tcccaaaatg ctgggattac aggtataagc 600
cactgtgcct ggccaaagat accgttaacc ctggataaag agaatggagg ttacctctgt 660
ccgtgtagat tcctaagctg tcctggagtg atccttggag taaaggaaag gtgctttgaa 720
gcacattcag ccatcagccc tgtgggatgg cagccactga tttgtcctat ggtctttaca 780
gggacccagt ctgccttcaa gaaaagacag aagtagaaag ggtggtggct gactgtctga 840
caaattgtta tcaggtatgc aggaagtata tccttctcca aaatatcata cttgcatcac 900
caggtagaca catttccttc tacacagaat tatcttcaga gcttcttaaa gcaaataaag 960
cctgcttcaa ggactgagtc cctagtcgaa ttcccggaag gagtggagcc tgtcatattg 1020
tgtttatcta gcatctgctc aagagtgtgc tgcagtggag ggaaatcaga tgacctccca 1080
gtctggttgt gttacataca atcatgtgta agaagtgcca ttcaagccgt gtcactggag 1140
gggactgaca gtgagattca gtgacttttg atgatctggc tgtggacttc accccagaag 1200
aatggacttt actggaccca actcagagaa acctctacag agatgtgatg 1250

<210> 514
<211> 36

<212> DNA
<213> Homo sapiens

<400> 514
gcttccctcc cggcgcagtc accggcgcgg tctatg 36

<210> 515
<211> 182
<212> DNA
<213> Homo sapiens

<400> 515

cttcctttct cgtgttgtta tcgggtagct gcctgctcag aacccacaaa gcctgcccct 60

catcccaggc agagagcaac ccagctcttt ccccagacac tgagagctgg tggtgcctgc 120

tgtcccaggg agagttgcat cgccctccac agagcaggct tgcatctgac tgacccacca 180

tg 182

<210> 516
<211> 336
<212> DNA
<213> Homo sapiens

<400> 516

cctcccctttt tgggtcggta gttcagcgcc ggcgccggtg tgcgagccgc ggcagagtga 60

ggcaggcaac ccgaggtgcg gagcgacctg cggaggctga gccccgcttt ctcccagggt 120

ttcttatcag ccagccgccg ctgtccccgg gggagtagga ggctcctgac aggccgcggc 180

tgtctgtgtg tccttctgag tgtcagagga acggccagac cccgcgggcc ggagcagaac 240

gcggccaggg cagaaagcgg cggcaggaga agcaggcagg gggccggagg acgcagaccg 300

agacccgagg cggaggcgga ccgcgagccg gccatg 336

<210> 517
<211> 857
<212> DNA
<213> Homo sapiens

<400> 517

tggtcctttc ttttatgatt cacaaggaat gaccctcttc atcgcctctc ctaattcagt 60

cctcacaaca gtccttttac aaatgggaca acaggttaga ggaagtcagg cagatttcca 120

gcatcataga gagtaaagga ccagggaagg atcaggattc aaggactgca cccaggctct 180

gcttccagct tgctgtgtga ctttgggtaa ttttgttccc ttagggaact gagctttctc 240

atttgtaaat gcaaacaggc tgttgggagg atcaaatgag atccaggggt gaaaacagct 300

tagtttactt tcaggaattt acccacgcgg tatataaagg caaaatatta ttatagtcag 360

gtgattgtag attgaggaac ccatttcctc attctgcaaa ttgcaaacct gagggcccaa 420

agagggacag gggcttgccc caggtctcag caggctgtga gcaagagcta aagcctaatc 480

ctcctgcctt tgggcctgga gcccttcctt gtaccccagg ggtcagtgtc tttgttggat 540

acaggcttag attgactgac tgtaccctga gaacctaggg gagtccctgt tcccaattct 600

tctcctaccc ccaccttggc ctgatggagg aagaccctgc tgtgttgaga tgagcaccag 660

agccaagaag ctgaggagga tctggagaat tctggaggaa gaggagagtg ttgctggagc 720

tgtacagacc ctgcttctca ggtcccagga aggtggcgtc agcatctgca gccgcgtcga 780

cgttgtcgga gcctccgcgg aggacccagg agagccggac taggaccagg gccctgggcc 840

tccccacact ccccatg 857

<210> 518
<211> 54
<212> DNA
<213> Homo sapiens

<400> 518
ctttccttcg gcttccgttc ttggtccatg tgagagaagc tggctgctga aatg 54

<210> 519
<211> 67
<212> DNA
<213> Homo sapiens

<400> 519

ggttctctgt cagtcgcgag cgaacgacca agagggtgtt cgactgctag agccgagcga 60

agcgatg 67

<210> 520
<211> 96
<212> DNA
<213> Homo sapiens

<400> 520

ccgcctcttg tttttcccgc gaaactcggc ggctgagcgt ggaggttctt gtctcccctg 60

gtttgtgaag tgcggaaaac cagaggcgca gtcatg 96

<210> 521
<211> 114
<212> DNA
<213> Homo sapiens

<400> 521

tactctttat caatcgtctt ccggcgcagc cccgtccctg ttttttgtgc tcctccgagc 60

tcgctgttcg tccgggtttt ttacgtttta atttccagga cttgaactgc catg 114

<210> 522
<211> 219
<212> DNA
<213> Homo sapiens

<400> 522

ctccccctct cgctctcctc cctcttcccg gctccagctc cgccgccagc tccagccttt 60

gctccccctc ccaaagtccc ctccccggag cggagcgcac ctagggtccc tcttccgtcc 120

ccccagccca gctacccgtt cagaccagca gcctcggggg gcaccccccc gccagcctgc 180

ctccctcccg ctcagccctg ccagggttcc ccagccatg 219

<210> 523
<211> 146
<212> DNA
<213> Homo sapiens

<400> 523

agatcttctt ccgctctgag gcgctactga ggccgcggag ccggactgcg gttggggcgg 60

gaagagccgg ggccgtggct gacatggagc agccctgctg ctgaggccgc gccctccccg 120

ccctgaggtg gggggcccacc aggatg 146

<210> 524
<211> 304
<212> DNA
<213> Homo sapiens

<400> 524

cggcctcttt gtgtggtgcc cagatagggg agcggaggtg gcggcggcgg cggtagcggt 60

ggccttggtt gtcttccagt ctcctcggct cgccctttag ccggcaccgc tccccttccc 120

tccccccttcc tctcttcctt ccttccctcc ccttcccttt ttcccttccc cgtcggtgag 180

cggcgggggt ggctccagca acggctgggc ccaagctgtg tagaggcctt aaccaacgat 240

aacggcggcg acggcgaaac ctcggagctc gcagggcggg ggcaaggccc gggccttgga 300

gatg 304

<210> 525
<211> 737
<212> DNA
<213> Homo sapiens

<400> 525

ggctcttttg ttcggctgag gggagggccg ttggccgggg cctgcggtac gccgcttcag 60

tgagggacgc cactgcggcc acccggcttg ctgccttcct gggcgccact cccccaggcg 120

acccgacgcg acgcgccagc agcgcagcac cgattcctct cgggctcttg ggcgctgctc 180

tgaggtgagg agcccgctgg aggcgggaga gctgggggag ggggcgcggc ggcggcggcg 240

gcgggagccc tgcgtgaggg aacgcgcttt cgaggcggag gttaggagcg gggagcgcgc 300

ccgggtccag cgtcctgctt ctccgcttcc cgcgctgagc tcttcgcctg tcgctgaggc 360

gtcggtgcca gctgcgtgaa ggatggagag ggcggggcgc gaatcctgag ccagagactg 420

agtgcttggg ggtgggccga gcacttgggg gccgctcttc ggggcccggg tggtctggaa 480

caatgttgct tggctgggcg gctgcgggat agggcggaag gggacaggct tgaggcttgg 540

ataggcgtga ggaggcgcat acgaccgcac aacccgaggt ttgtaactgt attcggaaga 600

cgccgggtcc ggctgggact gccagaggaa cctggctttg caggactacg gaggagtaac 660

gtcgagtgaa ttggaagagg gcccagggcc gcacaagcag cgtcaccctt tacaccagaa 720

agctggcggg cactatg 737

<210> 526
<211> 204
<212> DNA
<213> Homo sapiens

<400> 526

cgcccttctt aggaggggct gcattgcagg gggagagtga actgacagac tcagtcactg 60

aagagggaaa aggagtgaga agacaaagcc gtcaaagccc caacagcttt gtatttctcc 120

agcccggcgc agaccccgga gctcccgagg cactccctcc atctttggaa cacgccagta 180

attgattgat aacaggaagc tatg 204

<210> 527
<211> 145
<212> DNA
<213> Homo sapiens

<400> 527

ttttctttct ttcctagagt ctctgaagcc acagatctct taagaacttt ctgtctccaa 60

accgtggctg ctcgataaat cagacagaac agttaatcct caatttaagc ctgatctaac 120

ccctagaaac agatatagaa caatg 145

<210> 528
<211> 592
<212> DNA
<213> Homo sapiens

<400> 528

acttcttcct cccttcccct ctcttcccct ccctccccag ccttccccgc gagcggacgc 60

ggcagcgcct ctgtctcgct ttttcttatt tttcccccct ttccccttc ttttttttt 120

tttcttttct tttctcccct cccccccttt caccatttcc cctcggaggc gctttccccg 180

ggcaggggca gagccggtct caccccccgc ctctccccgg cccccgccgc cctatggcga 240

gagggagccc cctcccaacc cgggctcgag cggcggcggc ctcaggccgg gggtcatcat 300

ggaactaatt cgctgaccga cccagcggcc gcagccgtgc gtcccgctcg agcgccagcg 360

cccgcgcccg cgcccccga tccgcttccc ctttctccct cctcagttgg ccgagtcgtc 420

ccgcgcgcac cgcctccgcg cgcctatgag aatgaggtgg taacgggccc ccggatgacc 480

ccgcgtcacc actgtgaggc ctacagctct gccggggagg aggaggagga ggaagaggag 540

gagaaggtag ctacagcaag ctgggtagca ggcagatcca aaggatatca tg 592

<210> 529
<211> 29
<212> DNA
<213> Homo sapiens

<400> 529
cattccctcg cgctctctcg ggcaacatg 29

<210> 530
<211> 71
<212> DNA
<213> Homo sapiens

<400> 530

gtctctttgt cctgccggca ctgaggactc atccatctgc acagctgggg cccctgggag 60

gagacgccat g 71

<210> 531
<211> 97
<212> DNA
<213> Homo sapiens

<400> 531

gggtctctcg gtcccgcagc cgtgaggagg acggtctgca tactcgctgc ccgccggctc 60

cctcccccgc gtccctgcga ccgccgcggc gaagatg 97

<210> 532
<211> 130
<212> DNA
<213> Homo sapiens

<400> 532

gggccctttt cgcggccggg ccccagcatg gctgccccca cggctgaggg cctggcagct 60

gctgcgccct cgctttcttg acattccctg gcttctgtgc tctcttcccc aggccacccc 120

agcagacatg 130

<210> 533
<211> 26
<212> DNA
<213> Homo sapiens

<400> 533
ctgtctttct cagaaaacca aatatg 26

<210> 534
<211> 400
<212> DNA
<213> Homo sapiens

<400> 534

gtttctctgc agttttcctc agctttgggt ggtggccgct gccgggcatc ggcttccagt 60

ccgcggaggg cgaggcggcg tggacagcgg ccccggcacc cagcgccccg ccgcccgcaa 120

gccgcgcgcc cgtccgccgc gccccgagcc cgccgcttcc tatctcagcg ccctgccgcc 180

gccgccgcgg cccagcgagc ggccctgatg caggccatca agtgtgtggt ggtgggagac 240

ggaaacaaga atctcagtgt aacccgagca aaatcgcgcg tctcagcgtt gcttgtatag 300

agctgtaggt aaaacttgcc tactgatcag ttacacaacc aatgcatttc ctggagaata 360

tatccctact gtctttgaca attattctgc caatgttatg 400

<210> 535
<211> 17
<212> DNA
<213> Homo sapiens

<400> 535
tcgtctcctc caagatg 17

<210> 536
<211> 152

<212> DNA
<213> Homo sapiens

<400> 536

ccgcctttcc ttttgtttgt ctcacgtttt gcgtgggagg cggtcccggg atttcagggg 60

tctaccggct ctcttatggc gaatgcaacc cgaagagaga gtgagctgta tcttcagagt 120

tgtctccgtc tttccaagaa cagaacaaaa tg 152

<210> 537
<211> 40
<212> DNA
<213> Homo sapiens

<400> 537
gcctcctttc caagcgcgac ccgttgaggt ccttgtcatg 40

<210> 538
<211> 152
<212> DNA
<213> Homo sapiens

<400> 538

ccgcctttcc ttttgttttt ctcaggtttt gcgtgggagg cggtcccggg atttcaaggg 60

tctacgcgct tttctatggc gaatgcaacc cgacgaggga gtgggctgta tcttcagagt 120

tgtctccgtc tttccaagaa cagaacaaaa tg 152

<210> 539
<211> 235
<212> DNA
<213> Homo sapiens

<400> 539

ctttcttttt cctttcttcg gaatgagaga ctcaaccata atagaaagaa tggagaacta 60

ttaaccacca ttcttcagtg ggctgtgatt ttcagagggg aatactaaga aatggttttc 120

catactggaa cccaaaggta aagacactca aggacagaca tttttggcag agctgctcac 180

tccttgctca gctcagtttt ctgtgcttgg accctctggg cccatcctgg ccatg 235

<210> 540
<211> 131
<212> DNA
<213> Homo sapiens

<400> 540

ctctttggga tgctttgttg tctggtggtg actgtgccca tgggtgagtt gtatcggaaa 60

atcgtcatgt gaggatcaga ggggaaaaga aaacagaggc ctctggtctc tgcctgccct 120

gggtgctcat g 131

<210> 541
<211> 137
<212> DNA
<213> Homo sapiens

<400> 541

acgcctcctc ttgcgctgtc ctgttaatgg cgggcagtag ccgctgaggg gattgcagat 60

aaccgcttcc cgcacgggga aagtctaccc tgcctgccac tttctgctcg ccgtcagcgc 120

cggagctcgc cagcatg 137

<210> 542
<211> 130
<212> DNA
<213> Homo sapiens

<400> 542

tgctctttct ctagcacggt cccactctgc agactcagtg ccttattcag tcttctctct 60

cgctctctcc gctgctgtag ccggaccctt tgccttcgcc actgctcagc gtctgcacat 120

ccctacaatg 130

<210> 543
<211> 323
<212> DNA
<213> Homo sapiens

<400> 543

caccctcttc cgccgctccc gcccagcgac ctcgctcccg gggcgacgcc ccgcgtgcgc 60

cagagtcgcc gaggtcgtcc ccggcaccgg aagtgaccct ggcgggtttg tcttcaaatt 120

ctcggcgagc aggagccgcg ccggcaggtg gtgttgacga ttgaactggg cagtactggg 180

gccgtgagcg gagagcaaag tgggctggac tgggtcaggc cctccttcct cgctgccggg 240

atctccactc cgccaatccc ctgtgcctgg cgttgggcgg tttcccgagg agcttgggcc 300

gccgcagctt acagttgaac atg 323

<210> 544
<211> 186
<212> DNA
<213> Homo sapiens

<400> 544

ctttctcttt ttcctttctt ccggatgaga ggctaagcca taatagaaag aatggagaat 60

tattgattga ccgtctttat tctgtgggct ctgattctcc aatgggaata ccaagggatg 120

gttttccata ctggaaccca aaggtaaaga cactcaagga cagacatttt tggcagagca 180

tagatg 186

<210> 545
<211> 100
<212> DNA
<213> Homo sapiens

<400> 545

cggcctttca tttccgcttc cggtgcgggc cgcgcgcgag cgcagcggtg ggaggcggcg 60

accagccggt tgaggcccca ggcttggcct caccacaatg 100

<210> 546
<211> 126
<212> DNA
<213> Homo sapiens

<400> 546

ctccctcctg gcgcttgtcc tcctctccca gtcggcacca cagcggtggc tgccgggcgt 60

ggtgtcggtg ggtcggttgg tttttgtctc accgttggtg tccgtgccgt tcagttgccc 120

gccatg 126

<210> 547
<211> 80
<212> DNA
<213> Homo sapiens

<400> 547

gcgtctctat cgcgccagtt cctcagcctc agtgctatga aggtgacagc gtgaggtgac 60

ccatctggcc cgccgcgatg 80

<210> 548
<211> 63
<212> DNA
<213> Homo sapiens

<400> 548

gggcctttgc ccgccttggc ggccggctct acgttccctg ttctcgcctg cagctccgcc 60

atg 63

<210> 549
<211> 309
<212> DNA

<213> Homo sapiens

<400> 549

gcttcccttc cgatgattcg gctcttctcg gctcagtctc agcgaagcgt ctgcgaccgt 60

cgtttgagtc gtcgctgccg ctgccgctgc cactgccact gccacctcgc ggatcaggag 120

ccagcgttgt tcgcccgacg cctcgctgcc ggtgggagga agcgagaggg aagccgcttg 180

cgggtttgtc gccgctgctc gcccaccgcc tggaagagcc gagccccggc ccagtcggtc 240

gcttgccacc gctcgtagcc gttacccgcg ggccgccaca gccgccggcc gggagaggcg 300

cgcgccatg 309

<210> 550
<211> 133
<212> DNA
<213> Homo sapiens

<400> 550

gggcctttgt cccgacagag ctccacttcc tgtccccgcg gctctgtgtc ccctgctagc 60

cgtaggtcgt gtgacccgca ggcaccggga gatccagaag tgaaacgcca ggctctctgg 120

aggccaggag atg 133

<210> 551
<211> 405
<212> DNA
<213> Homo sapiens

<400> 551

cagtctttcg cggcccggga gctcagcaga gctaccagct gccctgttgg cttcgctggt 60

cggatcgtcc tcctggcccc gccaaacagg cggggggagc ggccccgact gtggggccat 120

ggcagtagtc tcctcgttcg ccgccgccgc tagcctagct gagtcgccgg cttctgcgct 180

aggggctccc accgcctccg caggctaagg agccgctgcc accaacgagc tgtgagggtt 240

actatgctcc ctctttgccg ccgtctcctc ctcttgcccg cgcaggcacc cctctggctg 300

ctcagtcctg cctcagtgtc aaaccagaag agaagtaaaa ttcaacaaaa atttatgtgt 360

ggagttcctt cttaaaagaa gaaaaaagtg attatttaga ctatg 405

<210> 552
<211> 196
<212> DNA
<213> Homo sapiens

<400> 552

agccctcctt gctagtctgg gacttcccgg tggagtgagg aacccagcaa cacgctcctg 60

acttcccttc ccaaggactc gacctgagaa ggacacagca gtctctgaat ttcatgctct 120

cctctttgat gtgaagaaaa tgaaaagctg aacagttgtg gaactgtgga tagagttaga 180

caataaggcc gccatg 196

<210> 553
<211> 238
<212> DNA
<213> Homo sapiens

<400> 553

ccctccctcc ctttccctcc ctcgtcgact gttgcttgct ggtcgcagac tccctgaccc 60

ctccctcacc cctccctaac ctcggtgcca ccggattgcc cttcttttcc tgttgcccag 120

cccagcccta gtgtcagggc gggggcctgg agcagcccga ggcactgcag cagaagagag 180

aaaagacaac gacgaccctc agctcgccag tccggtcgct ggcttcgccg ccgccatg 238

<210> 554
<211> 166
<212> DNA
<213> Homo sapiens

<400> 554

ctttctttcc gtcgcagaga gcatcggccg gcgaccgttc cggcggccat tgcgaaaact 60

tccccacggc tactgcgtcc acgtggcggt ggcgtgggga ctccctgaaa gcagagcggc 120

agggcgcccg gaagtcgtga gtcgagtctt cccgggctaa tccatg 166

<210> 555
<211> 576
<212> DNA
<213> Homo sapiens

<400> 555

ctcccttccc cctccgcccc cggacggccg ctggggcgcg cgcctctcct cgcaccccca 60

ccctgagtcc ccacactccg cggggccacc gagctgctga ggcccctttg cgggcccgcc 120

gagcggttcc gggtttaggg ttcacaggtc agagttgact ccctgaaaag tgcagccggt 180

ttgaaatgca agatggcggc ggcgtggcgc tgagaggcgc ggcggcccct gcaggagaag 240

acagactgct gctttggacc tgttggtaat gatggcctga gctaaacatc taactagaag 300

ggataccctt ccatttcaaa gaacagaatg ctaaggaagc tgtggcaagt gattggagtt 360

gtgcttcaaa aatttcagaa attcagcagt attttatctg ccaacaataa gctctttact 420

tgattgcacc atgagaaagc tgctaatgag acttgttgag cacaaaaatg gacttgaaga 480

accaaaagcc attgttttca aatgaagaac actgaacagt tttaagcctc gatgcttttt 540

aatcaccact gagcttttcc tcataacatc agaatg 576

<210> 556
<211> 81
<212> DNA
<213> Homo sapiens

<400> 556

ccttccttcc ctccctcctt ccctcctgtc gccgtctctt ctggcgccgc tgctcccgga 60

ggagctcccg gcacggcgat g 81

<210> 557
<211> 180
<212> DNA
<213> Homo sapiens

<400> 557

ctttctctca ggatttccgc tggcttcagg ttccggtcag gcgtcgggac agagcctgat 60

ccaggcttcg gcggccggtg gcagctctcg atcagctctc gcagtcggag aggcggctaa 120

ggaaaggtgc cacagcagag acgcgaagga gaggccctag aacctttca aagaagaatg 180

<210> 558
<211> 58
<212> DNA
<213> Homo sapiens

<400> 558
gagcctcttt ggtagcagga ggctggaaga aaggacagaa gtagctctgg ctgtgatg 58

<210> 559
<211> 132
<212> DNA
<213> Homo sapiens

<400> 559

tgccctttct ttcgccagcc ttacgggccc gaaccctcgt gtgaagggtg cagtacctaa 60

gccggagcgg ggtagaggcg ggccggcacc cccttctgac ctccagtgcc gccggcctca 120

agatcagaca tg 132

<210> 560
<211> 334
<212> DNA
<213> Homo sapiens

<400> 560

ctgccttttc tcctgccggg tagtttcgct ttcctgcgca gagtctgcgg aggggctcgg 60

ctgcaccggg gggatcgcgc ctggcagacc ccagaccgag cagaggcgac ccagcgcgct 120

cgggagaggc tgcaccgccg cgcccccgcc tagcccttcc ggatcctgcg cgcagaaaag 180

tttcatttgc tgtatgccat cctcgagagc tgtctaggtt aacgttcgca ctctgtgtat 240

ataacctcga cagtcttggc acctaacgtg ctgtgcgtag ctgctccttt ggttgaatcc 300

ccaggccctt gttggggcac aaggtggcag gatg 334

<210> 561
<211> 102
<212> DNA
<213> Homo sapiens

<400> 561

agctctctcg agtcactccg gcgcagtgtt gggactgtct gggtatcgga aagcaagcct 60

acgttgctca ctattacgta taatcctttt cttttcaaga tg 102

<210> 562
<211> 16
<212> DNA
<213> Homo sapiens

<400> 562
cgttctccgt aagatg 16

<210> 563
<211> 16
<212> DNA
<213> Homo sapiens

<400> 563
gcttctctgc actatg 16

<210> 564
<211> 140
<212> DNA
<213> Homo sapiens

<400> 564

cgctcttttc tcgggacggg agaggccgtg tagcgtcgcc gttactccga ggagatacca 60

gtcggtagag gagaagtcga ggttagaggg aactgggagg cactttgctg tctgcaatcg 120

aagttgaggg tgcaaaaatg 140

<210> 565
<211> 85
<212> DNA
<213> Homo sapiens

<400> 565

atttctttgt taagtcgttc cctctacaaa ggacttccta gtgggtgtga aaggcagcgg 60

tggccacaga ggcggcggag agatg 85

<210> 566
<211> 46
<212> DNA
<213> Homo sapiens

<400> 566
cggtctccgc cggttggggg gaagtaattc cggttgttgc accatg 46

<210> 567
<211> 42
<212> DNA
<213> Homo sapiens

<400> 567
cttcctcctc gcccccaccc agacccagaa ggcgccacca tg 42

<210> 568
<211> 81
<212> DNA
<213> Homo sapiens

<400> 568
cttccttcct agtcgcgggg agtctgagaa agcgcacctg ttccgcgacc gtcacgcacc 60

cctcctccgc ctgccgcgat g 81

<210> 569
<211> 50
<212> DNA
<213> Homo sapiens

<400> 569
ggttctctgt cccggttcct ggggttgcac agacagaccc tgtaaacatg 50

<210> 570
<211> 292
<212> DNA
<213> Homo sapiens

<400> 570

gggtccctcg ctggctagta ggagagactg gtgcttgccc cgcccggtgg actaactcgc 60

ttaattttaa ataaaaagtc gaggacacgg cggtcgtttt cccgaagaca tgggccctcc 120

catgggccat ttgctccctg gaggccctcg cgtcttgctg agcccgggga gttaggatga 180

cgcgagcggt gagggagccc ggaacgattc cttcgcggaa caattgaggc gaggcctttg 240

ggagtacttt gtgggacgga ccctggcggg ccctgccaga cgcacaggga tg 292

<210> 571
<211> 82
<212> DNA
<213> Homo sapiens

<400> 571

ccgccttccc aagagcccct gcggccgggc gcgaaaatgg cggcggcggc gacggccggg 60

cgctcctgaa gcagcagtta tg 82

<210> 572
<211> 87
<212> DNA
<213> Homo sapiens

<400> 572

cggccttcct gcagcctctt ccgctcgccg gctgcggcgc ctgggacggt tgcggtgggt 60

ctgggcgctg ggaagtcgtc caagatg 87

<210> 573
<211> 128
<212> DNA
<213> Homo sapiens

<400> 573

cggtctctgg cggagtcggg gaatcggatc aaggcgagag gatccggcag ggaaggagct 60

tcggggccgg gggttgggcc gcacatttac gtgcgcgaag cggagtggac cgggagctgg 120

tgacgatg 128

<210> 574
<211> 51
<212> DNA
<213> Homo sapiens

<400> 574
tctcctcttt ctccaccacc tcgggccccg gtgtccccgg ccagcactat g 51

<210> 575
<211> 338

<212> DNA
<213> Homo sapiens

<400> 575

tcttccttcc gggtcgcgct aggccgggct tgcggcggtt gtgccgcatc tagagagtcg 60

gggagccgcc cccgcaccca ggccttctcg cgctgcctgg tcgctggtga agcccgcggc 120

gcgcgcctct cccggaccct gcagggtaaa agaatgtcac atgtcagcat ttgtacctga 180

agtcagcatg caaagttcag ggtacctgga tgaatgccaa cttttgcatt tcccatgtgt 240

atcctgtgac cattctatct gggaacatcc ttcaaagagt tcatgcatct tactgaggac 300

acctgacctt ttgaagcttc ataattcaca tctagatg 338

<210> 576
<211> 248
<212> DNA
<213> Homo sapiens

<400> 576

gctccttcta gcatccttca tccttcaggt accagccatc cagacagtgc ttgagctgca 60

gaaactgaga ccagacctct ggcctggccc tccccagggg cctcctttcg tatagtcact 120

gcttctgcat cagatacttt cagctgcaac tccctactgg gtggggcacc catttcaggc 180

agaaggtttt ggtaccctcc actgacccta cacccagggc tgctactgcc gcttgtggct 240

tcaggatg 248

<210> 577
<211> 47
<212> DNA
<213> Homo sapiens

<400> 577
aggccttttg ttcctgtccc ggaaagccgg cgtcctgccg cgcgatg 47

<210> 578
<211> 232
<212> DNA
<213> Homo sapiens

<400> 578

cctcctcctc ctcttctcgc cattgcagtt ggacccagca gcccggcgcg caccgcgtgg 60

cttttggggg cagaccccgg cgggctgtgg caggagggcg gcggcggcgg ctgcggtcga 120

agaaggggac gccgacaaga gttgaagtat tgataacacc aaggaactct atcacaattt 180

gaaaagataa gcaaaagttt gatttccaga cactacagaa gaagtaaaaa tg 232

<210> 579
<211> 122
<212> DNA
<213> Homo sapiens

<400> 579

gtttcctctg ctctccgctc tcgcccgcta gctctcctcc cttccgctcc tgcttctctc 60

cgggtctccc gctccagctc cagccccacc cggccggtcc cgcacggctc cgggtagcca 120

tg 122

<210> 580
<211> 79
<212> DNA
<213> Homo sapiens

<400> 580

tgccctctgc cgctgctccc gtctctttgg ttacgctcgt cagccggtcg gccgccgcct 60

ccagccgtgt gccgctatg 79

<210> 581
<211> 160
<212> DNA
<213> Homo sapiens

<400> 581

ccgcctcttc cttcccgttg tttaaggcag ttggttgccc tcctgtccgt cagaggtgca 60

gtaccagagg tggcgtgctg ccgatttcgc gtttgccttg ctggatgatt ccgcttgttt 120

gccggctgcg tgagtgctta gagcttttcg gtggaagatg 160

<210> 582
<211> 34
<212> DNA
<213> Homo sapiens

<400> 582
ggctctctac cggtgagggt ttgcggggaa gatg 34

<210> 583
<211> 159
<212> DNA
<213> Homo sapiens

<400> 583

cagtctctgt gcgttgaagc cggagaccgc ggcggcctca gcgaggaccc tccgccccgg 60

agccgccggc cggagccgca gcctctgccg cagcgccccc gccacctgtc ccctccccct 120

ccgcctccgc cggagccgcc tcgtgcactc tggggtatg 159

<210> 584
<211> 216
<212> DNA
<213> Homo sapiens

<400> 584

gtgccttttt ccgccgcgcg ccaccagaat gtccctgtct tgaggtctaa tggcggacgc 60

cagtatgttg gagttggtgg tggcttaagt tttgaaggga ggtagcatcc gttggatatc 120

cacaccatcc ttctcgctgc aggctttctt ggactccgta ctgttggtgt aaccaaggcc 180

tggaggtctg ggtggctcag gtttcctgca gccatg 216

<210> 585
<211> 163
<212> DNA
<213> Homo sapiens

<400> 585

ctgcctctcg ctggaggcca ggccgtgcag catcgaagac aggaggaact ggagcctcat 60

tggccggccc ggggcgccgg cctcgggctt aaataggagc tccgggctct ggctgggacc 120

cgaccgctgc cggccgcgct cccgctgctc ctgccgggtg atg 163

<210> 586
<211> 702
<212> DNA
<213> Homo sapiens

<400> 586

ttttcttttc tgctttgcag gcccaggctc aaggcaaatt ataagtaggg aaccaatttg 60

agggaaagac atgtgaacag agttaaggta ccacgtcctg ggagcgacca gcagccccac 120

ctgaagtccg catgcaactc tgacaagctc aggtgcttgt tttaaggaaa ggggctacta 180

gagtcttacc aacagcgagc ccaggtggga gatgaaacag gtactcccca aaataggtca 240

tccgagggag gaaaactgat ggagagcaca atgtgctctg agcgttttta atgtttttaa 300

gcttttaaat gatttcttca aggccgagca gcagcagcaa aggtgtggct taaaggatta 360

agggggtttc tgctgacacc tagaatgaag ttactctatt actaatcaag ccgagaggag 420

gcccactatg cccccgttta tcatcctttc ccagttcctt tttgctggtc acaaaacgat 480

gctcatcaat cccacctaaa gcaggaggcc aggagcccag cctcttgtag aaacagcgag 540

ggtataactg ccctcccgtt ctgcccccaa gacgaaggag gactctcgga agccaagaaa 600

ggtttaagaa gtctttctgg atagagagca gtgcccaggc aggaagcctt tcgccggcag 660

agcggggtcc aaggacgagc tggagaggac agaggcgcga tg 702

<210> 587
<211> 40
<212> DNA
<213> Homo sapiens

<400> 587
attcctttcc ttcctagcct tggtcgtcgc cgccaccatg 40

<210> 588
<211> 88
<212> DNA
<213> Homo sapiens

<400> 588

ccacctcttc ctgttcccgt ccttgaggac gccgtgccgg gtcagtgtta gcctccagcc 60

ctggttgtgg aaggcgacag aagtcatg 88

<210> 589
<211> 249
<212> DNA
<213> Homo sapiens

<400> 589

ccccctcccc cgcggcgccg tctcctcctc ccgcctgagg cgagtctggg ctcagcctag 60

agctctccgg cggcggcgca gcttcagggc agcgcgggct gcagcggcgg cggcggttag 120

ggctgtgtag ggcgaggcct ccccccttcct cctcgccatc ctactcctcc ctcctcgtca 180

tcctccccct tcgtcctcct cgccttcctc ctcctcgtca ggctcgaccc agctgtgagc 240

ggcaagatg 249

<210> 590
<211> 326
<212> DNA
<213> Homo sapiens

<400> 590

cttccttcct cacttcctct gcaggaggga gcgagagtaa agctacgccc tggcgcgcag 60

tctccgcgtc acaggaactt cagcacccac agggcggaca gcgctcccct ctacctggag 120

acttgactcc cgcgcgcccc aaccctgctt atcccttgac cgtcgagtgt cagagatcct 180

gcagccgccc agtcccggcc cctctcccgc cccacaccca ccctcctggc tcttcctgtt 240

tttactcctc cttttcattc ataacaaaag ctacagctcc aggagcccag cgccgggctg 300

tgacccaagc cgagcgtgga agaatg 326

<210> 591
<211> 69
<212> DNA
<213> Homo sapiens

<400> 591

cttccttccg tctcgctcgg agtttccctc tgcgttcgct ccgcgctgct ggaggctgtc 60

gtcccaatg 69

<210> 592
<211> 321
<212> DNA
<213> Homo sapiens

<400> 592

cctccttctg ttgcttcccg tctcctcggc ggctcccctc ccccgcccgg ctctccgcgc 60

cccttctggg cggcggggcg gcggagccgt cggcgtgcgg ccctccttgc gttcgtgcgt 120

gcgcccgtgg cccggcgcac gtcccgcgac accgaggccg agcggggcag ggggctgacc 180

gccatgaccc cccagagccc ggcgtgaggg ggccgagatg cggtgacctg ccagcacctg 240

ccgcagcctt cgtccgggag tcgccccatc tctccacgca tcggggccct gtgccccttg 300

ctgctgcagc cgggcaccat g 321

<210> 593
<211> 116
<212> DNA
<213> Homo sapiens

<400> 593

gtgtctctcg gcggagctgc tgtgcagtgg aacgcgctgg gccgcgggca gcgtcgcctc 60

acgcggagca gagctgagct gaagcgggac ccggagcccg agcagccgcc gccatg 116

<210> 594
<211> 96
<212> DNA
<213> Homo sapiens

<400> 594

cgttctctcc tccttcctcc ccgcctccag ctgccggcag gacctttctc tcgctgccgc 60

tgggaccccg tgtcatcgcc caggccgagc acgatg 96

<210> 595
<211> 55
<212> DNA
<213> Homo sapiens

<400> 595
aggcctctgt cccccacccc ctttccccgg tcccaggctc tccttcggaa agatg 55

<210> 596
<211> 274
<212> DNA
<213> Homo sapiens

<400> 596

cggccttttt ttcccggctg ggctcgggct cagctcgact gggctcggcg ggcggcggcg 60

gcggcgccgg cggctggcgg aggagggagg gcgagggcgg gcgcgggccg gcgggcgggc 120

ggaagaggga ggagaggcgc ggggagccag gcctcggggc ctcggagcaa ccacccgagc 180

agacggagta cacggagcag cggccccggc cccgccaacg ctgccgccgg ctactccctc 240

ttgatgccct ccccttttgcc cctcactcag gatg 274

<210> 597
<211> 28
<212> DNA
<213> Homo sapiens

<400> 597
tcatcttttc cccagaggcg tcggaatg 28

<210> 598
<211> 444
<212> DNA
<213> Homo sapiens

<400> 598

aattctctct ctttggctcc ctccttccgc gcgagtctct ggagaagccg cagcgcgagt 60

tgccgccgct gctgcccggg gccgggtaag tgggcctcac tcagagcccg accctcttgg 120

ccccggcttg cgtcgacccc cgccgggcac cgagcctgcg ccgcgcgcgg cccgggcgtc 180

ggggccgcgc ccgaccggga aaggccggga agccggttgg gcccgatcct cctggcagct 240

agaacgggcc gggcggggga gggggaacc gagcagagct taggggtgg ggcctcggag 300

ccaggccatg tcggggctcc tcaagaagag ggccagtggg actgctgggg tcgggctgga 360

ggggatctga ttgggggaag cgtctgggga ctgcttgggg cctgattggg ggacgtcgcg 420

aggatcggct tgccttgcgc catg 444

<210> 599
<211> 31
<212> DNA
<213> Homo sapiens

<400> 599
gggcctttgc tgtgtgggat aaacagtaat g 31

<210> 600
<211> 81
<212> DNA
<213> Homo sapiens

<400> 600

ctgtctcttc gccggttccc ggccccgtgg atcctacttc tctgtcgccc gcggttcgcc 60

gccccgctcg ccgccgcgat g 81

<210> 601
<211> 362
<212> DNA
<213> Homo sapiens

<400> 601

cctccttttc ctgcccccag actagaggcg ggatgtagtc tcttaggcta agagtgattg 60

gtcacaagga gactcggaag tgtctgatca gagccccaga ggaggccttg agagcctgtt 120

ggcgtaccgt tccacacttg gatccaggaa tcgggcgtgt tccaggctgc tctctatggt 180

agctttgggc ggatagaggg ggcgcgcaaa gtattaaggg acaataatgg ccgctttcaa 240

ggtgtggatt ttggctcctt gagcctgtct gagcgagggg tggcagcgcc ggcgccccag 300

aatccgggac agaagggtcc caagagtcgc gcttggtgag agaaatccca gatcctgtga 360

tg 362

<210> 602
<211> 130
<212> DNA
<213> Homo sapiens

<400> 602

catcctctaa gcttttaaat attgcttcga tggtctgaat ttttatttcc agggaaaaag 60

agagttttgt cccacagtca gcaggccact agtttattaa cttccagtca ccttgatttt 120

tgctaaaatg 130

<210> 603
<211> 25
<212> DNA
<213> Homo sapiens

<400> 603
gctcccctct cacgcagcca acatg 25

<210> 604
<211> 163
<212> DNA
<213> Homo sapiens

<400> 604

catcctttta gcaccgcgag aggcgccggt gtttcgagcc gtggcaccgg catcggctga 60

cactgctgcc tccagctagt tatttcgtcc tcttccgttc ttcaccccta caccttggag 120

gtgaacttct cacctgaggg ctgtaaagac tcgtttgaaa atg 163

<210> 605
<211> 43
<212> DNA
<213> Homo sapiens

<400> 605
cgtccctcac cgcaccaccc ctaaagacgc tagcgctgcg atg 43

<210> 606
<211> 82

<212> DNA
<213> Homo sapiens

<400> 606

gggcctctgc attgcccgac tccgtaggag cgcgggggcg gctcctgctc ttcctggact 60

cctgagcaga gttgtcgaga tg 82

<210> 607
<211> 90
<212> DNA
<213> Homo sapiens

<400> 607

ccgcccttcc ttcttctccc agcattgccc cccccacgtt tcagcacagc gctggccgca 60

gtctgacagg aaagggacgg agccaagatg 90

<210> 608
<211> 101
<212> DNA
<213> Homo sapiens

<400> 608

ccatctttcc gtcccgggca gccagcgcca gtcggagcca gcgcgagccg ccgccgccat 60

cactgccgct gccaagtcct ccacccgctg cccccgccat g 101

<210> 609
<211> 59
<212> DNA
<213> Homo sapiens

<400> 609
gattctcttt ccgcccgctc catggcggtg gatgcctgac tggaagcccg agtgggatg 59

<210> 610
<211> 121
<212> DNA
<213> Homo sapiens

<400> 610

aactctttct tcggctcgcg agctgagagg agcaggtaga ggggcagagg cgggactgtc 60

gtctggggga gccgcccagg aggctcctca ggccgacccc agaccctggc tggccaggat 120

g 121

<210> 611
<211> 44
<212> DNA

<213> Homo sapiens

<400> 611
gcgccctttc ccctgccggt gtcctgctcg ccgtccccgc catg 44

<210> 612
<211> 154
<212> DNA
<213> Homo sapiens

<400> 612

ctccctcccc gcgccccgtc ctctcccgcc ctacaggccc tagcagggca ggcgggaggt 60

gagcgcggcc atcccgctcc cggagttccg ggatcctgga gtccgtagtt cgtggtcctt 120

cgccggtgtc cccggagccc agcggctgtg gatg 154

<210> 613
<211> 23
<212> DNA
<213> Homo sapiens

<400> 613
cctccctttc tcctgcagcc atg 23

<210> 614
<211> 146
<212> DNA
<213> Homo sapiens

<400> 614

cctcctttc tcccgccggc tctaacccgc gcttggctaa ggtccgcggg aacccgtgag 60

ccaccgagag agcagagaac tcggcgccgc caaacagccc agctcgcgct tcagcgtccc 120

ggcgccgtcg cgccactcct ccgatg 146

<210> 615
<211> 187
<212> DNA
<213> Homo sapiens

<400> 615

gcgtctcttt cgctccgtgt cccgctgctg ctcctgtgag cgcccggcga gtccgtcccg 60

tccaccgtcc gcagctggta gccagcctgc ccctcgcctc gactcccttt caccaacacc 120

gacacccaca ttgacacctc cagtccggcc agccgctcca ctcgttgcct ttgcatctcc 180

acacatg 187

<210> 616
<211> 683
<212> DNA
<213> Homo sapiens

<400> 616

cgccctcttt tggtcgcccc ctccccaacc cagcactaag gagcaccctg ctctggtctc 60
cgccaccacc cagcgcctcc tggacccatc cccccaaacc cttgaacgtc ctcaggaccc 120
ccaggtgagc gcggcgcgct gcgggcgggg accctctctg cacctccccg cacccctggg 180
ggtcgctctg tccctacggt ccccgcctcc cctttctcct ttctaagcgc ctcgcgccca 240
ggccgccgcc cggggtggcg cagcccgcag ccctcccgct ccgggcgccc tccgccgctc 300
cgagaccccc tgggggcgcg tcctctcccg ctcccctgtt ccctcccccg gctcagggcg 360
ggcgcgtggt cccaggggag gctcccgccc agccccgcac tcctttgtgc ggccgggcgg 420
gcgctgcgtc aaggtggagg cgcggccaca cgcgcgcacc cacccgcgcg cacccagccc 480
ccgggagagg caggaaggga ggcggcggcg cgaggaggag ggagcggccg tggagcccaa 540
tcgttcgctc cccttcccgg gtccgcgcgc ggcgccgcct ccgccattgc tgcgagcagg 600
agcaggagac gcggagctcg gagcgctcag ctgacctgcc ggagccgggc gtgggctgca 660
gcctcggagc tcccggaacg atg 683

<210> 617
<211> 152
<212> DNA
<213> Homo sapiens

<400> 617

acgtcctttc gatgttgcgt catgcagtgc gccggaggaa ctgtgctctt tgaggccgac 60
gctaggggcc cggaagggaa actgcgaggc gaaggtgacc ggggaccgag catttcagat 120
ctgctcggta gacctggtgc accaccacca tg 152

<210> 618
<211> 322
<212> DNA
<213> Homo sapiens

<400> 618

tttccttcct ctttcactcc gcgctcacgg cggcggccaa agcggcggcg acggcggcgc 60
gagaacgacc cggcggccag ttctcttcct cctgcgcacc tgccccgctc ggtcagtcag 120
tcggcggccg gcgcccggct tgtgctcaga cctcgcgctt gcggcgccca ggcccagcgg 180
ccgtagctag cgtctggcct gagaacctcg gcgctccggc ggcgcgggca ccacgagccg 240
agcctcgcag cggctccaga ggaggcaggc gagtgagcga gtccgagggg tggccggggc 300
aggtggtggc gccgcgaaga tg 322

<210> 619

<211> 154
<212> DNA
<213> Homo sapiens

<400> 619

```
cggtctcctc acttccggct tcgctgctct tggttctggt tctggaggct gggttgagag      60
gtcgccggtc cgactgtcct cggcggttgg tcagtgtgaa tttgtgacag ctgcagttgc     120
tccccgcccc cgagcagccg aggagtctac catg                                 154
```

<210> 620
<211> 449
<212> DNA
<213> Homo sapiens

<400> 620

```
ccgccccttc ggcgccacca cgtgtgtccc tgcgcccggt ggccaccgac tcagtccctc      60
gccgaccagt ctgggcagcg gaggagggtg gttggcagtg gctggaagct tcgctatggg     120
aagttgttcc tttgctctct cgcgcccagt cctcctccct ggttctcctc agccgctgtc     180
ggaggagagc acccggagac gcgggctgca gtcgcggcgg cttctccccg cctgggcggc     240
cgcgccgctg ggcaggtgct gagcgcccct agagcctccc ttgccgcctc cctcctctgc     300
ccggccgcag cagtgcacat ggggtgttgg aggtagatgg gctcccggcc cgggaggcgg     360
cggtggatgc ggcgctgggc agaagcagcc gccgattcca gctgccccgc gcgccccggg     420
cgcccctgcg agtccccggt tcagccatg                                       449
```

<210> 621
<211> 444
<212> DNA
<213> Homo sapiens

<400> 621

```
ccccctcttc tgcagcagac ggactgagtt cctctaatcc ctgtgttcct tctcccccat      60
ctttctaaaa cccttctctg agagaggaat aactatagct tcagggataa tatagcttta     120
aggaaacttt tggcagatgt ggacgtcgta acatctgggc agtgttaaca gaatcccgga     180
ggccgggaca gaccaggagc cactcgttct aggaatgtta aagtagaagg tttttttccaa    240
ttgatgagag gagcagagag gaaggagaaa gaggaggaga gagaaaaagg gcacaaaata     300
ccataaaaca gatcccatat ttctgcttcc cctcactttt agaagttaat tgatggctga     360
cttctgaaag tcactttcct ttgccctggt acttcaggcc atatacatct tttcttgtct     420
ccataatcct ccctttcaag gatg                                            444
```

<210> 622
<211> 84
<212> DNA
<213> Homo sapiens

<400> 622

acctcccttt ctctgctcag ctccagcgtc atttcggcct cttagttctt ctgaaccctg 60

ctcctgagct aggtaggaaa catg 84

<210> 623
<211> 77
<212> DNA
<213> Homo sapiens

<400> 623

gggtctcttc cgcggaaact gacattgcgt ttccgttgtc ggcctcccac tgcaggagcc 60

atatattgaa gaccatg 77

<210> 624
<211> 371
<212> DNA
<213> Homo sapiens

<400> 624

ccaccttttc ttgggcttgt aggaaggtgg acatgggctc ccggagacaa gacaagtgat 60

atgttgaact gttcggtggc tggaatcaac tgctcctgga gtgacctaag gccagtgttt 120

atcagaactt agccagggcc agccaagcag gcacagatgc tctgctatga aatgccacgc 180

aggcagagac tgacaagcgg taggaactga gctttcccct tggactgctg cttcctgctg 240

tgttcagggg agggggtcac tttctggcaa ctctgctgct gctgctgctg ctgctgctac 300

ttcagcttcc tctccactca aggtaagcag gctaagggag ggcaggctgc tagggaaagc 360

tttgtaccat g 371

<210> 625
<211> 49
<212> DNA
<213> Homo sapiens

<400> 625
tggcccctct tctcacatca gcgggtccag gcccaaccga cagactatg 49

<210> 626
<211> 206
<212> DNA
<213> Homo sapiens

<400> 626

cgtcctcccc gctccgggcc ccacccggct cagacggctc cggacgggac cgcgagcaca 60

ggccgctccg cgggcgcttc ggatcctcgc gggaccccac cctctcccag cctgcccagc 120

ccgctgcagc cgccagcgcg ccccgtcggc agctctccat ctgcacgtct ctccgtgaac 180

cccgtgagcg gtgtgcagcc accatg 206

<210> 627
<211> 387
<212> DNA
<213> Homo sapiens

<400> 627

cgccctcttc ttccagactc tcggtctgtc cgctgggggc gcgcgcggtg tgtggcaggc 60

ggcagcggcg ctggcggccg agtgcgcttg tcacgcgtgg cggtgcgtgg ttgctagggg 120

cgcctgaggc tgccgggtag cccagcaggc cgagggagga agtagcgtgg agccggtgcc 180

gagccggggc gaagctggat cccctagata gactgtcttc aagctcactg atattttcct 240

ctgcttgatc cattgtgctg ttgagagcct ctagtaaatt tttcagactg acagacttca 300

aggatgcagc tgctactacc ggaggtgtgt ggcaccttac ctcagcaagg ccatgagacc 360

gtgtggccat gatgtgggcc cctcatg 387

<210> 628
<211> 116
<212> DNA
<213> Homo sapiens

<400> 628

acctctccct cctcctggcg ttagttccgg tcgcagagga gacaccgccg cagttgccgg 60

tacatcgggg atttctggct ctttcctctt cgccttaaat tcgggtgtct tttatg 116

<210> 629
<211> 209
<212> DNA
<213> Homo sapiens

<400> 629

ttgccctttc tgtgtaagct gtgagcgtag gcggccctga gggggtgtgt tgcaggggtt 60

tccaagccca gcaccagcac ccttgccctt ttccatcagg ggttcagcct agggtccccg 120

ctggtgggcg gctcccgagt cttggagaag agcacgagaa cctagaccgc ccccgaagtg 180

cggagacccc ctgggcaggc tgaaagatg 209

<210> 630
<211> 197

<212> DNA
<213> Homo sapiens

<400> 630

ctgccctttg cctcctgggc ggagaagctg cttcctcctg ggaacaaccg cctcccgctc 60

ctagcaggtt gctactgccc cgaacccgcg ctgcagggaa cagcggggca aacagtgagt 120

ggggttcagc gtagactctg gaccaggaga ggcccgcggt gaccgaggcc tgggccccgg 180

aaaccaatag agccatg 197

<210> 631
<211> 434
<212> DNA
<213> Homo sapiens

<400> 631

agccctcttt cacccccccc ccccggccat taccgaagcg gatgaaaaca aacactaacg 60

atggcggcgc cgggaagcga ccggctgctg ggcttaaggc gggagtgacc gcttaaccag 120

tgagggaagc actgaagagc gccagtcgac gtgggtgcga caactcgcgg agtcttagga 180

gcaaaacgtc tggggcctgc gagccaggac ccttctgaag ccttaggtgt ctatcggcga 240

cgtgtacggt cactgcagct ccggagcgcg gaaccctcag ccaggaggcg cggctggtcg 300

gtcccaggtc ccggcctccg taatgagagc ccggaaccac tctttgtgcc gcagcttcgc 360

agcatcttgg actcaagtga ttctcctgcc tcagcctcct gagtagctgg gactacagat 420

tcctataggc aatg 434

<210> 632
<211> 142
<212> DNA
<213> Homo sapiens

<400> 632

ccgccttccc acatcggatc gcagggctcc caaaatggcg agtgaggctg cggggactcg 60

ctgagcagcg gagggggagc gtgcagagcc gctgcggccc tcacagtccg gagcccggcc 120

gtgccgtgcc gtagggaaca tg 142

<210> 633
<211> 340
<212> DNA
<213> Homo sapiens

<400> 633

cgttctttct cccttctctg cctctctctc ctccacgctg ctttgatttc gctcttgcct 60

ctcttcttgc gctgctcagc tgggaacatc gtctcaccag gggcagcagc gacgcgctgc 120

acagccagac aggagctggc tgcggggcat ggaagcagcc tccttggcag ccgggagagg 180

agcaagcgca cgccactgcc cgtgacccag gcgtccggct gctgtcccct gccggggagc 240

tcatccacgc agaggtctct ccctgtcctc cctgcgagct tttcctctgc agagcccagt 300

ggagccagtc cccacaggag acaaccctga cgggagcatg 340

<210> 634
<211> 129
<212> DNA
<213> Homo sapiens

<400> 634

cggcctttct gtgttcctgg cccgcggccg tcgggtgtga gctgcgccga ccgctctgag 60

ggttcgtggc ccaccgctcc ttcgcggtcc ctgccgccac cgtccacgct cagcgttgta 120

gagaagatg 129

<210> 635
<211> 78
<212> DNA
<213> Homo sapiens

<400> 635

cggcctttct agccgctgtc ccaagggttg gtctcgcgct ttcggctgcg agctctctgt 60

ggtgctggca gcgacatg 78

<210> 636
<211> 188
<212> DNA
<213> Homo sapiens

<400> 636

ctccctcctt taaacagctt ctccgggtct cagcatgggc ttccagggca gcgattgagg 60

agaccttacc aaggagcacc acacagtaga tgctgagaca tcgtactcca ggataagaaa 120

cagtaacatg gcagcacctg cttgaaagaa attaaaaacc aacagactcc atttagaaag 180

gaacaatg 188

<210> 637
<211> 61
<212> DNA
<213> Homo sapiens

<400> 637

cctcctctcc ccttgcggcc tttctaacgt tggccctgct cttgtggcct cccgcagaat 60

g 61

<210> 638
<211> 256
<212> DNA
<213> Homo sapiens

<400> 638

cggtctttgc cgttaccgct atgtgtgggg cgtgtgtgga ataacgttat tgcccagcgg 60

agctgagggc cccggagctc gaccgcagcg gcagcgacga caacagcggc gacgacgacg 120

acgacgaggt gggggagga cggcgtgcga gagactcacg ggacgcgacg cgccccgcct 180

cccccgtccg gtccctctct ccacggtaag gggatgacgt agctttgcca aagacttaga 240

agctaagcag aaaatg 256

<210> 639
<211> 327
<212> DNA
<213> Homo sapiens

<400> 639

aggcctctcg aggtccagac agccgcccag cccgctctgc gacgcagcag tgaatagtgt 60

ggtacctcct tgtctcggtt caggtccaga cctccccgtc ttccggctgc cctgaacgtc 120

aggcgacctc aggaccctgt gattggcgcc tgcgccggcg gaccgtgacc gaggaaaccc 180

ctggagggac ttgggcattc cttgggctcc gtgcctgttc ttcgtgctcc tttcgggcaa 240

ggatctcaca ttatcagtct ttgaccgaca cagaatgcct ggcatttgat aaatgtttgt 300

tgaacttgaa gagacatatg gacaatg 327

<210> 640
<211> 83
<212> DNA
<213> Homo sapiens

<400> 640

ttttcctttt catttcagcc tgactgccgg aatcagagcc gcgggtgaga tccccagccc 60

tgtgagcctg taggagtaga atg 83

<210> 641
<211> 472
<212> DNA
<213> Homo sapiens

<400> 641

```
ggttctttga gatgctgttt ggcgactcgt cgccattccc ggagcaggtc ggcctcggcc        60
caggggcgag tatccgttgc tgtgtcggag acactagtcc ccgacaccga gacagccagc       120
cctctcccct gcctcgcggc gggagagcgt gtccggccgg ccggccggcg gggctcgcgc       180
aacctccctc gcctcccctt cccccgcagc ctccgccccg ccaggcccgg cccggactcc       240
cgagccccgg cctcctcgtc ctcggtcgcc gctgccgccg ggcttaacag ccccgtccgc       300
cgcttctctt cctagtttga gaagccaagg aaggaaacag ggaaaaatgt cgccatgaag       360
gccgagaacc gctgccgccg ccgacccccg ccggccctga acgccatgag cctgggtccc       420
cgccgcgccc gctccgctcc gactgccgtc gccgccgagg cccccgttga tg               472
```

<210> 642
<211> 301
<212> DNA
<213> Homo sapiens

<400> 642

```
agcccttctt gattggaaga agcgcctcgg accccggtcc ttggcgccgt agtggttagg        60
ttgagcccta ggcgtggggg agaactgggg aaactggaat ttcccgcgga gctgacagcg       120
cttgcgctcc ccctactcgt tctaattcca cgcgctccaa aatatccgcc atggagaaat       180
cttggccagg atgtccattc taggcccatc ggtgctgtct tgctgaaggt tgggtcaggc       240
atctaaaggg actgtggtaa gggagggtgt gacacaggtg taagctgcca tcgtcatcat       300
g                                                                       301
```

<210> 643
<211> 51
<212> DNA
<213> Homo sapiens

<400> 643
gctcctctcc ggccgcgcag ccgctgccgc ccacccgcac ccgccgtcat g 51

<210> 644
<211> 116
<212> DNA
<213> Homo sapiens

<400> 644

```
gactctttcc tgtcccggcc tgcgtggtgt gggcttgtgg gtctttgaga cccgaaaatt        60
gagagcgttt tcgcactcca gcggctgctc ctggcggctc tgcggccgtc accatg           116
```

<210> 645

<211> 58
<212> DNA
<213> Homo sapiens

<400> 645
acgccttttg ctggaagagc gctgctgggg ttaggattct gcgcggcgag gcaagatg 58

<210> 646
<211> 267
<212> DNA
<213> Homo sapiens

<400> 646

cctcctctgc gagcgttatt tcaaaagaag ttgagaacca gagaaaccga cctaagggga 60

ttctcccatt tggcccgtcc taccctaaag tcaccacctg ctgcttttct ggagcgctta 120

ccagtgacca agaggaacag aacacagagc agcctggcag tgtccaagca acaagcctcc 180

gctcctcctt cctgcaccct ggggctcctg aaactcacat gggtaaaaaa gatacagtaa 240

agacataaat accacatttg acaaatg 267

<210> 647
<211> 358
<212> DNA
<213> Homo sapiens

<400> 647

ccgcctctcg agcgctgccg gtggccgcag cggcgcaccc acgccggccc ggaggagcag 60

agtgttcatt tctgtgtcgg gcacagtgct aagtgctggg tgctcactgg tgatgaggca 120

gatgaaggtt accaaacttg tggacaggag cctcatatca gagacgtgga cctcactgta 180

gcctggtcat ggcttccagc ttttcgaatc tgaggctcca aaggaggaaa tgaccattca 240

gggatcttac tccagcttga ttacggagac tgaaccttca tagggtgcgc acttaccaag 300

gacaggaagg tttctctgtt tgaagggctt taaacttata acaaagaaaa taaaaatg 358

<210> 648
<211> 181
<212> DNA
<213> Homo sapiens

<400> 648

ccgcctctca accatcaggt tcggcagccc gcggcgccgc ctggcagctc ctcctcttct 60

ccgccccgcc ggccgcgggc gcgggggacg tcagcgctgc cagcgtggaa ggagctgcgg 120

ggcgcgggag gaggaagtag agcccgggac cgccaggcca ccaccggccg cctcagccat 180

g 181

<210> 649
<211> 85
<212> DNA
<213> Homo sapiens

<400> 649

ccttcctttc tccctctgca gacacaacga gacacaaaaa gagaggcaac ccctagacca 60

ccgcgaagga cccatctgca ccatg 85

<210> 650
<211> 26
<212> DNA
<213> Homo sapiens

<400> 650
tgttcctttt ggtacgctcc aagatg 26

<210> 651
<211> 131
<212> DNA
<213> Homo sapiens

<400> 651

tcccctcctt ccagcgcctt tcggtggagc actgcggcac tcagcccgag ctgccgtttt 60

cccctcgcgg ggaacgctgt gacccccccg caggagcggc ggggcggggt gggggggccc 120

gggagaagat g 131

<210> 652
<211> 96
<212> DNA
<213> Homo sapiens

<400> 652

gctcctttcc gtggtgtgta gccggcttgg cgtgaccctc gcctgatcca gttgttagag 60

ttggaagctt ggcagttggc ctcccttctt cccatg 96

<210> 653
<211> 291
<212> DNA
<213> Homo sapiens

<400> 653

cttcctttcc tagttgggtt ctgacagctc cgaggcagtg gtttacacaa ccaacacgaa 60

acatttctac gatccacccg attcctcccc tcattgatat tcaggaagca gctctccttc 120

ccctgccttc agctcaagtt tgctgagctt ttgtttcatt tgtgaatact tcttgctgga 180

agtccctcac ccagagacca gtgctcccaa cggcagagca gcgggggaga taaagaactg 240

gtgacacgtg gctgtacatt cagcacagct gtggtgtccc caagtgccat g 291

<210> 654
<211> 125
<212> DNA
<213> Homo sapiens

<400> 654

ctttcttttc cggattgggc atcccggcat ctgcacgtgg ttatgctgcc ggagtttggg 60

ccgccactgt aggaaaagta acttcagctg cagccccaaa gcgagtgagc cgagccggag 120

ccatg 125

<210> 655
<211> 30
<212> DNA
<213> Homo sapiens

<400> 655
cgctctctgc tcgcgcttgg gctcgcgatg 30

<210> 656
<211> 29
<212> DNA
<213> Homo sapiens

<400> 656
gcgccttttc tgacgatgcg aacaacatg 29

<210> 657
<211> 78
<212> DNA
<213> Homo sapiens

<400> 657

ccgccttctg ccctcagcag cagacgctct gtcccgcccg ggcagctctg cgaggcagcg 60

gctggagagg gaaccatg 78

<210> 658
<211> 135
<212> DNA
<213> Homo sapiens

<400> 658

gcttctcctt tttgtgttcc ggccgatccc acctctcctc gaccctggac gtctaccttc 60

cggaggccca catcttgccc actccgcgcg cggggctagc gcgggtttca gcgacgggag 120

ccctcaaggg acatg 135

<210> 659
<211> 271
<212> DNA
<213> Homo sapiens

<400> 659

caccctcttg cccggtcccc gggagggccg gtccgctcct cccggacgcc gaggacctac 60

caccgcgact tcgccccgcc cggcgcgggc ccaggaccct gatgtcgctt ttgaacagcc 120

cctgcacctg gcagccagcg agctactgta gtaggcattg ccgactgttt gcataccgga 180

tgggagtgac agtgtaatag aaaaacaagc aagaaacctt ttaggtagga ctcctaaggc 240

tcagaggaag ttacctccag ccgctgccat g 271

<210> 660
<211> 209
<212> DNA
<213> Homo sapiens

<400> 660

ccttcccttt cccggctcaa gtccttcctc tctctttcct ttctttccgc ctatcttttt 60

tctgctgccg ctccgggtcc gggccatttt ccgggccggg cgcactaagg tgcgcggccc 120

cggggcccag tatatgaccc gccgtcctgc tatccttcgc ttcccccgcc ccatgtggct 180

gcggggccgc ggcggcgctg cccactatg 209

<210> 661
<211> 31
<212> DNA
<213> Homo sapiens

<400> 661
ccgcctttcg taagtccccc cgcctcgcat g 31

<210> 662
<211> 37
<212> DNA
<213> Homo sapiens

<400> 662
caacctcttc tctcccgctt ctctcgctgt gaagatg 37

<210> 663
<211> 272
<212> DNA
<213> Homo sapiens

<400> 663

ctcccttctg ctggaccttc cttcgtctct ccatctctcc ctcctttccc cgcgttctct 60

ttccaccttt ctcttcttcc caccttagac ctcccttcct gccctccttt cctgcccacc 120

gctgcttcct ggcccttctc cgaccccgct ctagcagcag acctcctggg gtctgtgggt 180

tgatctgtgg cccctgtgcc tccgtgtcct tttcgtctcc cttcctcccg actccgctcc 240

cggaccagcg gcctgaccct ggggaaagga tg 272

<210> 664
<211> 142
<212> DNA
<213> Homo sapiens

<400> 664

actcctctag ccggaacctg ggggcccgga gccggggtag gcacagagtt gtcctcggag 60

gtccaggaca gcggccagcc cggcggcggg agtcagggcc acgccacctg cagggaagaa 120

cccgagtcga agcgggaaga tg 142

<210> 665
<211> 190
<212> DNA
<213> Homo sapiens

<400> 665

cttcctcttg cagttgaggc cggcgccgag ccggacttca ggcggatctc gtggcggagc 60

ccatcttgct ccctctccca ggcctttacc cgctccctag gattcccggg ccctgtaggt 120

gggagttggg agacgacagt actgctttta aagagacagt gttagggatc ttggaagcac 180

agccaacatg 190

<210> 666
<211> 84
<212> DNA
<213> Homo sapiens

<400> 666

gctcctttcc actcgggaaa ccttcagagg agtctcagaa aggacacggc tggctgcttt 60

tctcagcgcc gaagccgcgc catg 84

<210> 667
<211> 219
<212> DNA
<213> Homo sapiens

<400> 667

gcccctccct ctccgccccc accccctgtc ggcgtctggg cctcgtcccc ttctctctgt 60

ctcccttgcc tcccccatca cgtcccctga caccgacacc ccattgctcc cacagtctcc 120

ccagtctcca ctttggtccc cagcgctgtc tgcccgagga tttgcctgaa ggctgccccc 180

aactctgcac ccgcccccg agggccaccg aggaccatg 219

<210> 668
<211> 300
<212> DNA
<213> Homo sapiens

<400> 668

caccettccc gaagtttttc tgtcacctgt gttaggctcc gtccccttc cgcgttttat 60

ccccgtacca gaaaaggata catttagtgc ctcccaccca gctccactaa acgggttgga 120

tatctcattc tttgagttgg tgttccttcc ccggcgcccc catgtagctg ggaagtggga 180

cctgggggtg gttggacccc tgggatccta aaggaggggc agggagggcg cagaactccg 240

cttctgctcc ttgctaccag gacgcgcggc ctcctcagcc tctttcctcc cgctgccatg 300

<210> 669
<211> 133
<212> DNA
<213> Homo sapiens

<400> 669

cgttcttccg ggaaaatggc gactcccgct cgtgccccgg agtcaccgcc gtccgcggat 60

ccggcgctag tagcggggcc tgccgaggaa gccgagtgcc cgccgccgcg ccagcctcag 120

cccgcgcaga atg 133

<210> 670
<211> 226
<212> DNA
<213> Homo sapiens

<400> 670

tcgcctcttt ccgccagcgc ccgcaggacc cggatgagag cgcacgcttc ggggtctccg 60

ggaagtcgcg gcgccttcgg atgtggcgga tgcggccgtg agccggcggg ggaggtgctg 120

ctgctgcctc cactgtactc agacccaggt agcacaggat tgtccatcct ccagcagctc 180

agtgcaacgg tgtgaactca gcctgtttca gagcctccac accatg 226

<210> 671
<211> 126
<212> DNA

<213> Homo sapiens

<400> 671

cgatctctgc ggggcaagat ggcggcgccc agacaggcct ggagcacgga tgaataagag 60

ggaaccccca cacggagaca ctgctggaga gagtcgtact ggggaggcag ctggagcagc 120

aagatg 126

<210> 672
<211> 285
<212> DNA
<213> Homo sapiens

<400> 672

cctcctcttt ggtgcctcca gccaggaggc gggagcgatc cacagcagct gacccagctc 60

aggcactgcc tctctcacag ccctcaagac acaccatggg cccagaggca ggtttgctac 120

acagcagcga cgacgcaggc ggcggcccca gcgactcgca actgcctccc tgaccacagc 180

ggccaccgcc caacaccccc gagaagccat cgccaccacc ggcaggagaa cctagggtcc 240

ataaagccat cttcgcgatc gactaaagct acgtcaacaa ctatg 285

<210> 673
<211> 109
<212> DNA
<213> Homo sapiens

<400> 673

ccccctctct cggcccggcc atcttgtggg aagagctgaa gcaggcgctc ttggctcggc 60

gcggcccgct gcaatccgtg gaggaacgcg ccgccgagcc accatcatg 109

<210> 674
<211> 48
<212> DNA
<213> Homo sapiens

<400> 674
caccctttct gcgggggacg atttcgtcgg tggtaggctg ctaccatg 48

<210> 675
<211> 29
<212> DNA
<213> Homo sapiens

<400> 675
gcgcctcttc acgaggtgga aacaagatg 29

<210> 676
<211> 55
<212> DNA
<213> Homo sapiens

<400> 676
cttcctcttc ctgggcagcc tcgggacggg gcgccgcggc cgggcgggca gcatg 55

<210> 677
<211> 171
<212> DNA
<213> Homo sapiens

<400> 677

acttcctttg cctgctcacc gccagcgtag gtgctaccac cgctgccgtc gccgccgcca 60

ttttgatggc aggaagagtc cggttctggg acagctggag acagtggtgg tgactgaaat 120

aactttacca aaggaaagct attttgcgaa ctatcttctc cagcggagat g 171

<210> 678
<211> 21
<212> DNA
<213> Homo sapiens

<400> 678
agttcttcct ttgacaagat g 21

<210> 679
<211> 205
<212> DNA
<213> Homo sapiens

<400> 679

cagtcttctc gagcacatcg tcgcaaacgg ggccggaaag cgtggcagcg caggcgcaag 60

cgcagagagc ggaggcggtg gtggtggcgg ccgctggcca gttccttcag tgaatctaca 120

gacctatttt ctcaggagct cagcctggcc ttacttcagt gataaaagga ggaaaggctg 180

gctacagcaa acatcattca agatg 205

<210> 680
<211> 157
<212> DNA
<213> Homo sapiens

<400> 680

ctttcttctc gtcggtgttc ccggctgcta tagagccggg tgagagagcg agcgcccgtc 60

ggcgggtgtc gagggcgggt tgcctcgcgc tgacccttcc cgccctcctt ctcgtcacac 120

accaggtccc cgcggaagcc gcggtgtcgg cgccatg 157

<210> 681
<211> 749
<212> DNA
<213> Homo sapiens

<400> 681

ccgccttctc acactttcag gctctgatcg cggccgcagt ttttcctttt ttcttctgcc 60
gtcgccttct ctgcctcttc tcatcctttc tcgctctgct gctctgcagt gtgacgagtc 120
cgaatcctct tcccacccag cccgcgcctt tcttcttttg cctgcgctgt tctatttctc 180
cttcggccgc cgccgccact gctgcacaca gctggtgtcg gtgccgcgct tttaccccca 240
agtcgttccc gcagcctatg gcccaggccg ccttgggtat ttctgctcaa ggtaaccaca 300
tccctcttta aaaattccgc cgaaaaagag aagacgcttt acccgactct ttgggccgtt 360
atctcacggc gaactttctg accaagtata caactaccca gagggcctag gagaagtgct 420
gtatagagag cagttcgact tcaacgctga gccaccttgg gaacctagct gatgataggg 480
gggttccatc tcccaacttg tccatggagg tcttcacttc agaaatccaa gactcatatt 540
catccagctt ggtgtcaagt gggctgttgc tgccagaatt atcttgtgat tatttgagag 600
atgtatcagt ttcttctgaa gtacaatcaa ctgtagaagc ctttgtagca gtttgttgca 660
tattctaagg acccagacat aggcttggtg gcccgtctct tgtctttcct ggtttatgac 720
tttcggcttt gtggaatacg gctgagatg 749

<210> 682
<211> 55
<212> DNA
<213> Homo sapiens

<400> 682
gcctcttctt cttccgccct ggcagggtct ccgcagaaga tttgttgccg tcatg 55

<210> 683
<211> 44
<212> DNA
<213> Homo sapiens

<400> 683
gcgcctctcc agcctccgca ggcccaaccg ccgccagcac catg 44

<210> 684
<211> 69
<212> DNA
<213> Homo sapiens

<400> 684

tttcctcttt tgtgctgatt cctgaggact aggaaggtgc cccgaaaaga attcagagac 60
ctgacaatg 69

<210> 685
<211> 541
<212> DNA
<213> Homo sapiens

<400> 685

ctctcttttc tggagttaga ttagtctgaa gccgccacca gccccaggcc cccgtgcaga 60
agaaaagcgg gagggaacgg cggaggccgc cgctgccctg caccgccctc ctggaggcca 120
cttggagagt ccggccccga ggaggccatg gccacaagtg cccacagctg gccccaggtt 180
gccagcgtcg ctacagccca gaccaaggca gaataatctc cggatgagct ggtggcaccg 240
ctgagccttt ggtctcacca gggcttcctg ttgctggcag gcggggtgga gcggagctgc 300
tgggaggctg ctggatagga gaggggtcac ggctgcggaa gaggaggttc ttcgggacac 360
ccgtggatgg acacggcaag gaaacaccag gccaaccaca gctggggata aaatagcaca 420
accacaccct gccgtccagc gcctcccagc ctgtgcccct tcctagtacc accagcaacc 480
atcaatcccg tctcctcctg cctcctctcc tgcaatccac cccgccacga ctatcgccat 540
g 541

<210> 686
<211> 126
<212> DNA
<213> Homo sapiens

<400> 686

tcttctctgt ggcggagaca gccaggttgg cagctgacgg gacagccggg gtctattttg 60
ttgcgggttt tcagcaaatc cagggctggt ctggaggcgc gaaaacttaa ggcatacaga 120
acgatg 126

<210> 687
<211> 286
<212> DNA
<213> Homo sapiens

<400> 687

gcgcctctgt cattctactg cggccgccct ggcttccttc tacctgtgcg gccctcaacg 60
tctccttggt gcgggacccg cttcactttc ggctcccgga gtctccctcc actgctcaga 120
cctctggacc tgacaggaga cgcctacttg gctctgacgc ggcgccccag cccggctgtg 180
tccccggcgc cccggaccac cctccctgcc ggctttgggt gcgttgtggg gtcccgagga 240
ttcgcgagat ttgttgaaag acattcaaga ttacgaagtt tagatg 286

<210> 688
<211> 132
<212> DNA
<213> Homo sapiens

<400> 688

gggccttttc tctgcacgga gccggcgctt ttgcagttgc ttctgcggaa aggtggtagt 60

taagaatttg taaaggccag agaactacct acgattctct cagcggtctc tcttctcctc 120

aagtttgaaa tg 132

<210> 689
<211> 137
<212> DNA
<213> Homo sapiens

<400> 689

cctcctccct ccttccgtcc tccgcgcctt ccgtcggtcg gtccttgctt cctgcttcgc 60

ctccgcgcct cgcgctatgg gacagagccc ccgatccgcc agcaccacct gaggatccag 120

aaaccgcccc agcgatg 137

<210> 690
<211> 133
<212> DNA
<213> Homo sapiens

<400> 690

ctgtcctttc agcaccacaa gctcgggctg aggagggagg actcctggcc gtcctcctcc 60

tcttcaaatt ggcttgaatc ttctctgacc ccccacgagt gcagcacagt ctgggaagaa 120

aggcgtaagg atg 133

<210> 691
<211> 218
<212> DNA
<213> Homo sapiens

<400> 691

gcgccccttc cggcgcgggg agggcgctga agatcggggc cgctcggccg caggccgcct 60

ccagcgccgc gggatgtagc gcgggggacc gcggccccca gcagagcccg cctgcccggc 120

ttgtctacca tcagagggag atctctgccc cctggggctg agagacccca acctttcccc 180

aagctgaagc tgcagggtat tgaggtacca gccagatg 218

<210> 692
<211> 325
<212> DNA
<213> Homo sapiens

<400> 692

ttttcccttg ggacccgggt ccacacggcg gggtcgcccg tccatctccg gctcgcccgc 60
ggggcccatc gtcgacgtta gcggccgttc tccgagccga ctgacccatc cttggcgctg 120
ccgccgcgcg cttgttctcc tccctcgccc cgccttcatc ctccccgttc acggaaacga 180
cagctgcggc tgcggggctg gcgccgcctc cctccaccta ccacgtctgc cctcgccgct 240
ctagccctgc gccccagccc ggccgcggca cctccgcctc gccgccgcta ggtcggccgg 300
ctccgcccgg ctgccgccta ggatg 325

<210> 693
<211> 214
<212> DNA
<213> Homo sapiens

<400> 693

gtcccctctt cggcttccgt agaggaagtg gcgcggacct tcatttgggg tttcggttcc 60
ccccttccc cttccccggg gtctgggggt gacattgcac cgcgcccctc gtggggtcgc 120
gttgccaccc cacgcggact ccccagctgg cgcgcccctc ccatttgcct gtcctggtca 180
ggccccacc cccttccca cctgaccagc catg 214

<210> 694
<211> 53
<212> DNA
<213> Homo sapiens

<400> 694
ctgcctttcc cgggcgctga ttcctgagtg ctgagcgcga acccgaggag atg 53

<210> 695
<211> 73
<212> DNA
<213> Homo sapiens

<400> 695

atctccttta gccccgcccg cctccgtagc tgcctgaagt agtgcagggt cagcccgcaa 60
gttgcaggtc atg 73

<210> 696
<211> 28
<212> DNA
<213> Homo sapiens

<400> 696
tgatcttttc caaggctgta cagacatg 28

<210> 697
<211> 101
<212> DNA

<213> Homo sapiens

<400> 697

```
cgccctctcg ccgcgtcgcc ggtgcctgcg cctcccgctc cacctcgctt cttctctccc      60
ggccgaggcc cgggggacca gagcgagaag cggggaccat g                         101
```

<210> 698
<211> 164
<212> DNA
<213> Homo sapiens

<400> 698

```
gcttctcttt cggagggagt gttcgccgcc gccgcggccg ccacctggag tttcttcaga      60
ctccagattt ccctgtcaac cacgaggagt ccagagagga aacgcggagc ggagacaaca     120
gtacctgacg cctctttcag cccgggatcg ccccagcagg gatg                      164
```

<210> 699
<211> 44
<212> DNA
<213> Homo sapiens

<400> 699
gtttcttttg cggctccacg tcggcaccag ctgcggggca agat 44

<210> 700
<211> 98
<212> DNA
<213> Homo sapiens

<400> 700

```
ggttctttct gtgtttgttc tctgccctgc caaggccgta gagctggtgc gtgcgggtag      60
cggggctctc cgaggagccg cacgccggcg gcaccatg                              98
```

<210> 701
<211> 239
<212> DNA
<213> Homo sapiens

<400> 701

```
ctacctcctt ttccgcgggc cccgcccagg cggctgcccg tgacctgcct gggcgcgggg      60
aactgaaagc cggaaggggc aagacgggtt cagttcgtca tggggctgtt tggaaagacc     120
caggagaagc cgcccaaaga actgatatcc aaagagaaga agaaaaagtg aaacgatctg     180
tgaaagatgc tgccaagaag ggccagaagg atgtctgcat agttctggcc aaggagatg      239
```

<210> 702

<211> 41
<212> DNA
<213> Homo sapiens

<400> 702
cgaccttttg gccaggttag ggagggggcg acgctgagat g 41

<210> 703
<211> 38
<212> DNA
<213> Homo sapiens

<400> 703
cgctctttcc ggcggtgctc gcaagcgagg cagccatg 38

<210> 704
<211> 83
<212> DNA
<213> Homo sapiens

<400> 704
ccttcctttc cctccggcgt cctctcccgg ccctctcgcg ctgcactgtc tctccgacgc 60

aagactgtcc cggcccggat atg 83

<210> 705
<211> 44
<212> DNA
<213> Homo sapiens

<400> 705
cgccctcctt gccgcccagc cggtccaggc ctctggcgaa catg 44

<210> 706
<211> 83
<212> DNA
<213> Homo sapiens

<400> 706
cgccccttcg cggcgcttcc tagttcggct ggttcttctg tcgccggctt cagcagcccg 60

cgcccgggca ggaatagaag atg 83

<210> 707
<211> 118
<212> DNA
<213> Homo sapiens

<400> 707

**cgttccttcg ccgccgccag gggtagcggt gtagctgcgc agcgtcgcgc gcgctaccgc 60**

**acccaggttc ggcccgtagg cgtctggcag cccggcgcca tcttcatcga gcgccatg 118**

<210> 708
<211> 202
<212> DNA

<213> Homo sapiens

<400> 708

```
ccgcctctcg gccccgcggc ctggccggca agcagggctg cagtcacggg gcggcgcgga        60
gggccccagc ccagtcaggg gtgtggccgc cgccaccgta aggctaggcc gcgagcttag       120
tcctgggagc cgcctccgtc gccgccgtca gagccgccct atcagattat cttaacaaga       180
aaaccaactg gaaaaaaaaa tg                                                202
```

<210> 709
<211> 46
<212> DNA
<213> Homo sapiens

<400> 709
cgctcttttc attcacgaag gtagtgaggc ctagtggaaa gccatg 46

<210> 710
<211> 13
<212> DNA
<213> Homo sapiens

<400> 710
cctcccctcg atg 13

<210> 711
<211> 604
<212> DNA
<213> Homo sapiens

<400> 711

```
cccccttccc ggcaggctac tgggctccgc ccacacacct cccggcctgg ttcctaaacg        60
ccagctcgga gcaatcccct tgggctggag ccaaatccct gctgtgattt taaggaagac       120
cggcaggtcc gggcccccaa gggtcaaccc cacacacatc cccgcacttt cctgtatgca       180
ggcctgcgag cgtagaggga gtggaattca cagcctcccc acccatccgc aggggtctcc       240
tgggaggaac ccaccagcga taggaacact gaagctgggc tacggcgtcc gcccgagcct       300
tttcttaaag gcgccgaccc cggaagcggg gcgtccgagg gagcgcgcga cgggccacgc       360
acgtccgggc gtccagttcg gggcagcttc tccggctggt gggtgggtgg ggcagccttt       420
caggcagggt ggcaaccaac tatatctgag gaccagagcc attttggggc accagagctt       480
gtgacctctc catctccacc cagctgggtc caggggccac tctcagcact cacctcagca       540
gctgacatca taaagcagac ttgggaacct ggaagcactc tggagaacct ttccctgaga       600
catg                                                                    604
```

<210> 712

<211> 67
<212> DNA
<213> Homo sapiens

<400> 712
cgccctttca gttctgcttg ctgtcggcac cgctgcgtta cccggaaccg ccgggccgaa 60

cagcatg 67

<210> 713
<211> 99
<212> DNA
<213> Homo sapiens

<400> 713

ggttcttcct tttttattta ccggtggctg tgcttccaat ttaggaagac cccggcgacc 60

tgttcctcac ccccgcttcg ccctcacact ttcgggatg 99

<210> 714
<211> 23
<212> DNA
<213> Homo sapiens

<400> 714
tcgcctcctc cctccccaag atg 23

<210> 715
<211> 120
<212> DNA
<213> Homo sapiens

<400> 715

gttcctcctt gctctcgccc ctactctttc tggtgttaga tcgagctacc ctctaaaagc 60

agtttagagt ggtaaaaaaa aaaaaaaaca caccaaacgc tcgcagccac aaaagggatg 120

<210> 716
<211> 504
<212> DNA
<213> Homo sapiens

<400> 716

tagtctttcc aggtgttagt cgaaacctcg tggtgcgacc ctggtcgtcc caaacccccct 60
aggccttaat cctggggcgg tgggggcggg gaggccgtga gcacggcttc cgctcctcca 120
atccgccaga gggcgcagcg gccggcctct cccttcccgg ggttcttcgc gccgggcccc 180
ttccgcgtgg gtgagtgaat gtgagagtca gcgctcgcgc cgcgcgcgcc gcccgcctcc 240
gctgttcggc gctctgcttt aggcggtggg gggcgggcgc gcgcgtaaaa gcatagagac 300
gggcattgag ctcttgggct agagcgtcgc cgagtcggag ccggagcctg agccgcgcgc 360
tgtgtctccg ctgcgtccgc cgaggccccc gagtgtcagg gacaaaagcc tccgcctgct 420
cccgcagccg gggctcatct gccgccgccg ccgcgctgag gagagttcgc cgccgtcgcc 480
gcccgtgagg atctgagagc catg 504

<210> 717
<211> 86
<212> DNA
<213> Homo sapiens

<400> 717

agctctctag cagagcgccg ttgctggggg aatgcagaag cggccgcggg ctagcaagct 60

cccggagccg gcggcgcacc accatg 86

<210> 718
<211> 333
<212> DNA
<213> Homo sapiens

<400> 718

cctccttttc ttcctcagcg ggtccgcggc ccgctactct ccgggagggg cgcttcccga 60
cgccaaggta ggcctctccc gacgccgggg cggcccttcc tgatgccggg gtgtgtctct 120
cgcgacgcgg gggtgggctc cggacgccgg ggctggcctt gccgaagtcg ggggtgggtc 180
cctccggacg ccgaagtggg ctcgggatgc ggggctggga ccctcccgat tccggggcgg 240
attccggacg ccgggaccgg ccattactgg tgccgggttg ggcttctcca gatgccgggg 300
ctgggtcctt cccaaggttg agacaaaagg atg 333

<210> 719
<211> 63
<212> DNA
<213> Homo sapiens

<400> 719

ccgccccttc ccatcgtgta cggtcccgcg tggctgcgcg cggcgctctg ggagtacgac 60

atg 63

<210> 720
<211> 28
<212> DNA
<213> Homo sapiens

<400> 720
cagcctcctt tgcgggtaaa cagacatg 28

<210> 721
<211> 53
<212> DNA
<213> Homo sapiens

<400> 721
cttcctctca agcttggcgt ttgtttggtg gggttacacg cgggttcaac atg 53

<210> 722
<211> 203
<212> DNA
<213> Homo sapiens

<400> 722

taccctcttc tgttgctttc tccctgtggc tcgcgccgtc ccccgccgcc cgtcgacccc 60

gcttccatgt ccctggcgga cacagctccc aggaacctcc acgcccatgg ccactaggca 120

gagggaatcc tctatcacct cctgctgttc cacctcgagc tgcgacgcag acgacgaggg 180

cgtgcgcggc acctgcgaag atg 203

<210> 723
<211> 306
<212> DNA
<213> Homo sapiens

<400> 723

ccccttcttt tgtggtccg gcccattgcg agggtgacag gaaaccctgt gcagggagcg 60

ccgccatctt ggaccagccc gaggaagata ctgagggagc acaggagcag tcaccgctgc 120

cactgctact gccgctactg ctgccggcgc gtctgcacct ctcggcctgc cagtgtacct 180

gccggcgcct cggtcgaccg cccccgcccc ctctcccgct gcgtccgcac tcctgttcct 240

ggtcctgacg cccccctccc gcccggaaag ctgcccagcc accagcaacc ccccagtgcc 300

accatg 306

<210> 724
<211> 145

<212> DNA
<213> Homo sapiens

<400> 724

```
cttccttttc ctgtttggtt ttaagtaggc tataaaaatc aagttgctgt cttcagaggg      60
tctgtggtcc tctgatcaac ataggctggt gggagtacag gactcgcctc ctcagggttc     120
cctgtgctgc cacttttcag ccatg                                           145
```

<210> 725
<211> 513
<212> DNA
<213> Homo sapiens

<400> 725

```
ctctcttccc ctctccctct ccctctgccg ggtggatgct ttctccatgt ggcaaggctg      60
taactgttca cagctgtctg aaacagcagt ggaccaggag cagcttggag ttttaacttt     120
cattttacaa agaacaacat gtttgaatgt ttcagcaggc aagttataac tggcatctac     180
ttcttgttct tctagaacac cgaaaatctc tcccagcact ttagaaaggg gaccctgact     240
gtgttaaaga agaagtggga gaacccaggg ctgggagcag agtctcacac agactctcta     300
cggaacagca gcactgagat taggcacaga gcagaccatc ctcctgctga agtgacaagc     360
cacgctgctt ctggagccaa agctgaccaa gaagaacaaa tccaccccag atctagactc     420
aggtcacctc ctgaagccct cgttcagggt cgatatcccc acatcaagga cggtgaggat     480
cttaaagacc actcaacaga aagtaaaaaa atg                                 513
```

<210> 726
<211> 27
<212> DNA
<213> Homo sapiens

<400> 726
gtttctcttg cgccctggtc caagatg 27

<210> 727
<211> 320
<212> DNA
<213> Homo sapiens

<400> 727

caccccccttc cacgtcagcc aaggactctg gagccgccgc cgccgctgct gcggttcata 60

gccggagtag acggagccgc agtagacgga tccgcggctg caccaaacca ctgcccctcg 120

gagcctggta gtgggccaca agcccccagt cccagaggcg tggtgggtcg ggcagagtcg 180

gaagaactgg ctttctagct ggaagatgcg gaaggggagc gactaggccg cttgcgtctg 240

ggcctggcag aagggaccgg attttctggc atccttaaat cttgtgtcaa ggattggtta 300

taatataacc agaaaccatg 320

<210> 728
<211> 69
<212> DNA
<213> Homo sapiens

<400> 728

gggtcttttg cggctgcagc gggcttgtag gtgtccggct ttgctggccc agcaagcctg 60

ataagcatg 69

<210> 729
<211> 109
<212> DNA
<213> Homo sapiens

<400> 729

ctttcttttt ctccaaaagg ggaggaaatt gaaactgagt ggcccacgat gggaagaggg 60

gaagcccagg ggtacaggag gcctctgggt gaaggcagag gctaacatg 109

<210> 730
<211> 182
<212> DNA
<213> Homo sapiens

<400> 730

gtgcctttcc agtggacctg ggctgttgtt gcggttgttt tccttctctc cgtgcaacgc 60

tggcaagtct caaagtcgcc acagaaacat gcccctgatt cagtgcctct gcttagctgt 120

aacatgttaa tcagaactac ctggcatctt cctgaacaag actttcaata ggggccagta 180

tg 182

<210> 731
<211> 56
<212> DNA
<213> Homo sapiens

<400> 731
agatcttctt ccgggcggac gtggagccgg aagcggaggt tccgggctcc gggatg 56

<210> 732
<211> 77
<212> DNA
<213> Homo sapiens

<400> 732

cgtcctttag ccgggagcct gtctttgctt gcctttgcct ttgaggctct gtggctgtgg 60

ggctgagtgg catcatg 77

<210> 733
<211> 85
<212> DNA
<213> Homo sapiens

<400> 733

agctctttcc ccgcgactgc gccacgtctg aggcggctgt ggccgcgtcg gtgtccgcgt 60

cgaggagccg gggcagggca cgatg 85

<210> 734
<211> 152
<212> DNA
<213> Homo sapiens

<400> 734

ttctctcttt cggccggcgc cgccagttcc tggggcacac ccagaggtcc ccttctcgcc 60

gccgcctgca actgcgaggg tagcccgggg ccgcttggag tcgcccggac ctgagaggct 120

gctgcactgg gcctcagcca gccctccgga tg 152

<210> 735
<211> 359
<212> DNA
<213> Homo sapiens

<400> 735

cgtccttcta atcctagtct tcgtttggtc cggttgcact cttcctatag cccagagggc 60

gagagggcct gtggcctggg ggaaggagga cgaggttctg cctggatccc agcagtagga 120

cgctgtgcca tttgggaaca aaggaatagt ctgcctggaa tccctgcaga tcttggggcc 180

ggaggccagt ccaacccttg gagcaggaag aaacgcaaag ttgtcaagaa ccaagtcgag 240

ctgcctcaga gccggcccgc agtagctgca gactccgccc gcgacgtgtg cgcgcttctc 300

tgggccagag cgagcctgtt ttgtgctcgg gttaagagat ttgtcccagc tataccatg 359

<210> 736
<211> 188

<212> DNA
<213> Homo sapiens

<400> 736

cggcctcccg cacgcaccgc gcagcctgct gtgcccgtgg gtcccgagtg ctccgccgcc 60

cgccccgacc cgggcccagc cgcctccacg gcccgcgctc gtactggagc gaagagcggc 120

ctcctgaagg aggggaaggg acgtgggggc ggccacggca ggattaacct ccatttcagc 180

taatcatg 188

<210> 737
<211> 201
<212> DNA
<213> Homo sapiens

<400> 737

tctccctttt ttccttcttc cttcccctcc tcgccgccac cgcccaggac cgccggccgg 60

gggacgagct cggagcagca gccagagttt attaaccact taacctctca gaactgaaca 120

aagacaacat tgttcctgga acgccctctt tttaaaaaag aaagcataac ccctactgta 180

gaactaaatg cactgtgcat g 201

<210> 738
<211> 505
<212> DNA
<213> Homo sapiens

<400> 738

cggccttttg tcagcgcgca gggccaggag agctctcatt tcctcccagc ctcgtgcggg 60

aaatggcttt aattctgacg gcagggctgt gagggactag cgggaacccg agccttttgt 120

caaggaactg cggcgtcggt ggccagtcat ccccgccgcc gcggagccgc tgcactgctg 180

ggggatctcc cagcagctct gacgagcgcg ggctgcagca tgggcagaaa acgctgccct 240

gcagattagc tgggtggatt ttttaagcgc accccacccc ccaaacccat aaaataacaa 300

aaccaacccg cagtggccga ccggagatag ctaagatgcc gcgcaggagt ttccacctgg 360

atgtttgagg ttgtgtagat gtggccggca cccttgagag tggagctagg gggtgcagac 420

tgagcagtga acagaaggag ccttggacag ggctgggcca gcctcccgag ttccaggagc 480

gaattgcaaa cccaccggga aaatg 505

<210> 739
<211> 112
<212> DNA
<213> Homo sapiens

<400> 739

ccgccttccg gctccagtcc ccgggctcgg cctcggcgag gtgtaattcg cagcgcgggc 60

cggccccgga ggctctcggc gagcgcggcg cggtaacaag tgggcgagga tg 112

<210> 740
<211> 581
<212> DNA
<213> Homo sapiens

<400> 740

cgacctctac ttccacctct ggccccaagt acagcgccag ctgcggcctc gggagcgccc 60

gcgggggtgc ccgtgcaccg gccgcgcctc ctccctggcg cgggactcgg ccgcagctgc 120

ctcggacccc ggcacgatcg tgcacaactt ttcccgaacc gagccccgga ctgaaccggc 180

tggcggcagc cacagcgggt cgagctccaa gttgcaggcc ctcttcgccc acccgctgta 240

caacgtcccg gaggagccgc ctctcctggg agccgaggac tcgctcctgg ccagccagga 300

ggcgctgcgg tattaccgga ggaaggtggc ccgctggaac aggcctcagt tcctgctttt 360

gaaaggaaga gggggagtct gtgacccctg aggcctcctt gcaactctgt tttccaagct 420

ttgcacatct tccgaatttc ttcttcaaag tctaccctaa tgaaatatca gacaattttc 480

caagtgtgct tcatgaactt ctgggaggtg cttcacagtt tctgcaaatg attgattgaa 540

ttttcacttt gaaaaaatat actttaaggc gacacaagat g 581

<210> 741
<211> 45
<212> DNA
<213> Homo sapiens

<400> 741
ttttctttcc tggtgtcccg tcgcggcttg ggacccggca agatg 45

<210> 742
<211> 96
<212> DNA
<213> Homo sapiens

<400> 742

tgctccctct cccacaaggc agcgcgccgg ctcggacgcg gccggctacc gagccctttg 60

tgagggctgt gagctgcgcc tgacggtggc accatg 96

<210> 743
<211> 24
<212> DNA
<213> Homo sapiens

<400> 743

gaatcttttc cacagcccaa aatg 24

<210> 744
<211> 307
<212> DNA
<213> Homo sapiens

<400> 744

gtttcctttg ttgtgagctg cggcagagac tggtggctgg aggagacgcc ggcgctggag 60

agtgcgctgc gccgcccgcc gctgagggac cgcggggtta gccactgctg gctgcttcca 120

gtgttcgccg agaggtaccg gggtgacag ctccgggacc ggccgaaagg cgaggaaccg 180

gtgtggaaat taaaagaaca cacatatttt gactggggct ttgatcaacc aaatgctaaa 240

aagccacata aagaagatcc ctaatagtca tttctcaaca attatatagt caactgatgt 300

aacaatg 307

<210> 745
<211> 18
<212> DNA
<213> Homo sapiens

<400> 745
ctcccccttt ccaccatg 18

<210> 746
<211> 308
<212> DNA
<213> Homo sapiens

<400> 746

gcttccctct tctctcgccg cctcctggcc tccgcaccga cgcggcccgg gctggagccg 60

agccggggcc gagctgcagg ccggaccgga gccggatctg tacccgctga gacgtggaaa 120

catggaggcc tgagccggtg tgcgccacct gggctgcggc ggcgacagcg acttctcctg 180

acccctctgc caccctccca tccgtccgcg ggtccgtgga gctggagcag atcccccagc 240

cggctgagac aggttgtctt ttggaaatgc aggtttaagg acaaattatc tgcttaagct 300

agaagatg 308

<210> 747
<211> 144
<212> DNA
<213> Homo sapiens

<400> 747

cctcctcttt ctcctcctcc tcagggctcc agtcaggccg atccgctccg ctcacggaag 60

gaaaacagaa ataacttgct ggcttgtctg gagtcacatg tacttaggtg acaatttaca 120

gaaagtcatc tctgcagctt gatg 144

<210> 748
<211> 91
<212> DNA
<213> Homo sapiens

<400> 748

cgttcctttg tgctgcggcg gcggcttctc gagtcctccc cgacgcgtcc tctaggccag 60

cgagccccgc gctctccggt gacggaccat g 91

<210> 749
<211> 64
<212> DNA
<213> Homo sapiens

<400> 749

ctctcctttg gcttggggct ccggagttgc cactgccgcc ggcgctggta agcttttcag 60

gatg 64

<210> 750
<211> 87
<212> DNA
<213> Homo sapiens

<400> 750

tgacctcttt cgggtctctt tgaatctccg ctgtagcgtc acctggaagg cagatctaac 60

agagaacctg gactgtctcc tatcatg 87

<210> 751
<211> 284
<212> DNA
<213> Homo sapiens

<400> 751

ccgccttctg gacttggtct tagttcccag tcgcggccaa atcacgcctc agccacctcc 60

cgcaagcctc tcactgcctc agccacgctt tccaggctgg tttctggtcc ccatccgcgg 120

ctggtccggc cctgggaccg aatcacttcc cagcgagagg aaggtcaaat ttctcgaccg 180

gctacgggaa ggtcgcggcc gccgccctgt cagccgcctc ggcgccccca ggacccctcg 240

ggtctcttta accggaagcg gaagtgcgtg tcggcgggat catg 284

<210> 752
<211> 17
<212> DNA
<213> Homo sapiens

<400> 752
cagtccttct cagcatg 17

<210> 753
<211> 297
<212> DNA
<213> Homo sapiens

<400> 753

cgttctttgt tctgtccccg gtgtgtgggt ctgtgacagg gtccaacagg gcctggtccg 60

tgtccggtcc cccaaatctg tcgtccctgc ccccaggcat tggcatcaac aaaagtcaga 120

attcccggga acttgaacag aggctgctaa attcccagta attgctcctt tggccttcta 180

gggactgact tcaaagaagg aaggaaagaa tcaggcagtg cttcctcatt ctcttttaaa 240

acccgcttcc cgctgagtct gcacccagga gaccagagag caccttgccc ttccatg 297

<210> 754
<211> 250
<212> DNA
<213> Homo sapiens

<400> 754

ggttcttctc ctaggcggaa gccagaccag agagcgtgcg tgtttttccc agggtgcccc 60

gcgctgctgt tatggccgcc tccttgaggt agtatccgca catggaattc tagggccgca 120

ggtgtattta cggtaactgt cgccactaga tttcagcgcc tttggactct cctgttttca 180

ctttcttttg ttgactcccg tgtggccctc gtgggagcct gttttggctg cagcggtgtc 240

tggggtgatg 250

<210> 755
<211> 33
<212> DNA
<213> Homo sapiens

<400> 755
gtttctcttt cctctcagtt tgcgcacacc atg 33

<210> 756
<211> 81
<212> DNA
<213> Homo sapiens

<400> 756

tgctcttctc gttcccgaga tcagcggcgg cggtgaccgc gagtgggtcg gcaccgtctc 60

cggctccggg tgcgaacaat g 81

<210> 757
<211> 57
<212> DNA
<213> Homo sapiens

<400> 757
cctcctcctg gacggcggca gcggcggcgc gaggagccgg cgggcagcgg cgcgatg 57

<210> 758
<211> 149
<212> DNA
<213> Homo sapiens

<400> 758

gcgccttctc cttgcttctg ggggtcgtgg ccttgctccc gctgtgcggg aaaagaatcc 60

aggcccttcc acgcgcgtgt gggtgcgggg gccccgaagt gctcgtggtt ccccgctagg 120

tctccgctgg ggcaggaacc ggaatcatg 149

<210> 759
<211> 244
<212> DNA
<213> Homo sapiens

<400> 759

cctccttcgt cgcggcctct agtgcacttt cggctccttc cccttcccgg gcctttcagc 60

ttggtctttc cgggcctcgc ttcccccagc ccctgcgccc ggcccgaacg agaggttccg 120

gagccccggc gcgggcgggt tctggggtgt agacgctgct ggccagcccg ccccagccga 180

ggttctcggc accgccttga gagcttcagc tgccccagga ttagaatccc aagaaaatca 240

aatg 244

<210> 760
<211> 66
<212> DNA
<213> Homo sapiens

<400> 760

ccgcctcttt cccgccgccg cctgggaggg gacccgggct gccaggcgcc cagctgtgcc 60

cagatg 66

<210> 761
<211> 162
<212> DNA

<213> Homo sapiens

<400> 761

cttcctttcc agcctcccgc cctcgtctgc ttccggccct gtggcctggt ggggctctgc 60

aggctccctc gggagtggtc cttgggccgt ggcccctctg ggaggcctga gggagctcaa 120

tcctggtagc aacacccctg aattcctggt ggtgaaagga tg 162

<210> 762
<211> 277
<212> DNA
<213> Homo sapiens

<400> 762

agttccttta tccctgggcc caacctcccc gccgacccgc ggtccaggcc tcggtctctc 60

tcttcggcgg cgagccgcgg cccagacccc ggcagaggac acttgtcggc acgttctcac 120

ccctgtcatc tcagccccct gcctagctcc accccaggct tgggaacccg gcccctgacg 180

gcccattgtc cgcgggccca gcccccgcgc tgaacgcacg ctcgcccttg cccctaacca 240

gcgcgtctac cccggcaacg cgcagtgacc tgggatg 277

<210> 763
<211> 252
<212> DNA
<213> Homo sapiens

<400> 763

gtttcttttc tgcgcttgtg cgttttctgt tcggtttcct tcccgctagc ggggccacga 60

gggttgctag gcaacagccc ctgggtgact tggtcttagg gtcctgtccg gcttggggct 120

gatgaaagga gctgtccgcg cccgggctct tccgagaagt ggttgctgac agccacaaag 180

tgaaagggag tgaggcggcg tggacgagta aggagtgaca gtgaggattc acatttgggt 240

tatttcaaga tg 252

<210> 764
<211> 96
<212> DNA
<213> Homo sapiens

<400> 764

cgtccttcct ggtcctgcgg gtccaggact gtccgcgggg ttgagggaag gggccgtgcc 60

cggtgccagc ccaggtgctc gcggcctggc tccatg 96

<210> 765
<211> 205
<212> DNA

<213> Homo sapiens

<400> 765

```
ctccctctgg ggcgcgggcc tcagttccgg gctacagcag ccgacgccga gaggcaccgt      60
ttcttcttaa aagagaaacg ctgcgcgcgc gaggtgggcc cctgtcttcc agcagctccg     120
ggcctgctcg ctaggcccgg gaggcgcagg cgcaggcgca gtgggggtga gggcgcgtgg     180
gggcgcacag cctctggtgc acatg                                           205
```

<210> 766
<211> 28
<212> DNA
<213> Homo sapiens

<400> 766
tggccctttc ctttccgcgt gtagaatg 28

<210> 767
<211> 371
<212> DNA
<213> Homo sapiens

<400> 767

```
tctccccttc tacagagttc ctccggcgct tcctccaccc cgggatacac agaacctcat      60
ctcctacggt gctgaagcct gcagcagggc aggatgggca ggagagcaga gccgcggagt     120
ctgcggcgcg ggtgaagagc ggcgcgtaat tcccgcagca agattgttcc gcgcccgcag     180
cccctggact agcaggatcc gaaccccggc ggctgcgtgc ttataggcgc agacgtcaga     240
gagcccgcgg cttaaagcgc gtcgcctggc tagcgccacc ccctagcctt cttcaaggcc     300
tccagggctg ggcccaagcg cccgtcgacg gcaccctggg cccagaggac tcgcgggcct     360
catctccaat g                                                          371
```

<210> 768
<211> 512
<212> DNA
<213> Homo sapiens

<400> 768

agttctttct gatagcaggc agccatcttg cctggagcct gagaaaggga ggagagacag 60

aaggaaccgg cgacagtggt ctcagggccg ctccgggggg cctcaagaac cggaggcagc 120

cccggaggtg gtccccgatc ccgggctatg ctcttggatc tgagaaggga aggcggaggg 180

cggcggggac aagatgggtg gagaatgtca agcaaggaat gctaggcggg ggaggggcgt 240

tgctatggcg actggggagg ggcggtgtct gttctgaatc gctgtgtgtc acccgggcgc 300

tgcccaggaa gggcagggct ggggtgatga ccatggtaac acccgggggg gagttcgtga 360

catctccggc gcggagggac tcgatgtcta tggcaatggt cgcctggtgg aagggacgga 420

actagatccc ttcgctcggg acgctcacat tccaggccct tgtcctgcag gctgccgcgg 480

gcggacacgc cagaggagga ggccggggaa tg 512

<210> 769
<211> 71
<212> DNA
<213> Homo sapiens

<400> 769

ccgcctttta cgacgcgccg gaaagcaacg gcaagggcgg cagccagcac cgggcggaga 60

gggctaccat g 71

<210> 770
<211> 126
<212> DNA
<213> Homo sapiens

<400> 770

ctgcctcctt ctactcgggc gccccggcgg ccgccacctc tccccagccc aggagaggct 60

gcggagccgc agccgcccag accgcgcagc gcgggaggca ggttccgcac gaaataaatc 120

agaatg 126

<210> 771
<211> 126
<212> DNA
<213> Homo sapiens

<400> 771

ttccccttttt ggggacagat cccgaagttc gagcatccct cggataggcc gggtgtcagg 60

cctggtctct caggcccgtc caggcccatc ttgacgattc caagaccacc cccttgagca 120

agaatg 126

<210> 772
<211> 124
<212> DNA
<213> Homo sapiens

<400> 772

ccgccctttg ccactccccc tgcctcctct ccgcctttaa cttctcggga agatgaggca 60
gtttggcatc tgtggccgag ttgctgttgc cgggtgatag ttggagcgga gacttagcat 120
aatg 124

<210> 773
<211> 363
<212> DNA
<213> Homo sapiens

<400> 773

agtcctctcg cagctgcgcc aggacagccg gcgcgcggcc gtgcccacaa gttgccggca 60
gctgagcgcc gcgcctcctc ctgctcgcag ccccctacgc ccacccggcg gcggtggcca 120
gcgccaggac gcacatcccg cggacaccga ccccagatgt aaagcgggac cccagcccct 180
cgcccccccgg cgcgatcgac agtctcgcca gcgtctcctc tgccaaaacc cagggctgga 240
agatgtggca gccggccacg gagcgcctgc aggagagatt tgcagacaca gaagcggcac 300
agagaaggcc attgtgaaga tcaaggcaga aaccggagtt atggcatcat aagccaagga 360
atg 363

<210> 774
<211> 167
<212> DNA
<213> Homo sapiens

<400> 774

tttcctcctc ctcctcctcc gcctccgccg ccgttgcttg aatggtggag ccgaagctcg 60
gctcgtgaac acacactgac agctataggg caggcggcgg caccgtcccc gcttcccctc 120
ggcggcgggg tgtcccgtcg gcggccctga agtgacccat aaacatg 167

<210> 775
<211> 130
<212> DNA
<213> Homo sapiens

<400> 775

tggcctttttt tgggcgtctc cctgctccgc ggcccgggct ggcgggcggg cgctcggctg 60

gcggctgcag cagcagaggg agacccgcgg caaccccggc aacccagggc tcggcgtcgc 120

tgccaccatg 130

<210> 776
<211> 656
<212> DNA
<213> Homo sapiens

<400> 776

aggtctttta gtctttttcc ccctccctta ctcttcgtcc ccggtccctc ccctccccac 60

ccctttcctt ctagctccga cgtttgcggc cgcgggggcg gcggaggata tggagtaaag 120

ccagagtcag tggccaggca cgaaggcaga gcaggaacag ccaggaggcg tttattaggg 180

gggcgggggg aaagagcccc agcaccgccc ctcctggaag aaggaagagg taagtgaccg 240

gccgccggca ccgaccgacc tccctcaccg gcggctctct cgcctgggct cccggagccg 300

gcgaggaggg aatggaggac tcgcgcccgg gttaggcctc ccagggccgc tcaggctggt 360

gggtgttgcc tggtgacggg cctgccggcg gccggccggg cgatcggcgg tcggcgcccg 420

cgcaaagcgg ggctggacga gcagcgagct ccggggagcg gatccgagag ggccgagtcc 480

tcgaaagagg ccttgaggcg acgggagacc cgggatcgaa gtcagctgcc ggagggagag 540

ccccccatgc cggctcgaga gctcgggttt cggtggtgga gaacgtagta cctttcgggg 600

acattggaca ctactctagg accgggtaac tataactacc caatattgca gccatg 656

<210> 777
<211> 215
<212> DNA
<213> Homo sapiens

<400> 777

caccctctct cctagtactt cctgttctcg gctaaccctg gcgctgggcc gggggctgga 60

gagtgaccgt ggtctgagtg acctggggcg gctgcgtggg ccggggtggg cctcaaagcc 120

gggcaccaga cgggaggggc ggcgctcggg ccgcgcgctg cccgcgccgg gtcctggcgg 180

gcggcgaggc tggggctgac tcctgcctca ggatg 215

<210> 778
<211> 32
<212> DNA
<213> Homo sapiens

<400> 778
cgacctctgg ctaacctacc cccggagcca tg 32

<210> 779

<211> 34
<212> DNA
<213> Homo sapiens

<400> 779
cggccccttc cggttacgaa accttagcaa gatg 34

<210> 780
<211> 166
<212> DNA
<213> Homo sapiens

<400> 780

tctccttttg cgcgacacgg tctcagctgt tccgcctgag gcgagtgacg ctggccgcca 60

acgaggtata cgtactggga ccctcgccct cagtctcgtc tccggcgcgg ctacctgccc 120

cgttttccct gtgagttgac ctgctccggg ccgcgggccg ccaatg 166

<210> 781
<211> 42
<212> DNA
<213> Homo sapiens

<400> 781
cggtctcctg tacgccctag actaggggcc gccatctcca tg 42

<210> 782
<211> 133
<212> DNA
<213> Homo sapiens

<400> 782

ttgtcctctt cgctgctccg tagtgacggg gattgttgtg ttgcagaaat ccggcaatcg 60

acctgaggac ttgcgagccg ctcagctccc gggacgtttg gagctgctgc taaataattt 120

ctgctcagcc atg 133

<210> 783
<211> 33
<212> DNA
<213> Homo sapiens

<400> 783
ggctctttct cctccacgtg gggacgcagg atg 33

<210> 784
<211> 74
<212> DNA
<213> Homo sapiens

<400> 784

cgctctctct cactggcaca gcgaggtttt gctcagccct tgtctcggga ccgcagcctc 60

cgccgagcgc catg 74

<210> 785
<211> 181
<212> DNA
<213> Homo sapiens

<400> 785

cgctcccccc cacgtgtccg ccggagtttc tccaccagca acatggccgc cgcctgagag 60

gagagccggg ccgccgccgt ctctgcagcc cgcgggtaac tgggccgttg ccgccgtccg 120

cgctcggccc ccgcggagag atcgagctga aggactgcgc ggctggctct cctctagtat 180

g 181

<210> 786
<211> 217
<212> DNA
<213> Homo sapiens

<400> 786

gagcctttgc ccgccagcgc cttcgctctt tggctccctg agttagtccg gttgcttgcg 60

atcgccgcgg ccggggctgc gaaccgaagg gctcgctccg cgccgcctgg gtctctacct 120

catccgtagg tgtggccctg atggtgtggc aggctctgga ctcctaaagc tctggagcga 180

atttaagatt ttattcatgt gcatggcata gaagatg 217

<210> 787
<211> 270
<212> DNA
<213> Homo sapiens

<400> 787

ccgtctttct ggaaacaccg ctttgatctc ggcggtgcgg gacaggtacc tcccggctgc 60

tgcgggtgcc ctggatccag tcggctgcac caggcgagcg agacccttcc ctggtggagg 120

ctcagagttc cggcagggtg catccggcct gtgtgtggcg cgaggcaggg aagccggtac 180

ccgggtcctg gccccagcgc tgacgttttc tctcccnttt cttctctctt cgcggttgcg 240

gcgtcgcaga cgctagtgtg agccccccatg 270

<210> 788
<211> 104
<212> DNA
<213> Homo sapiens

<400> 788

cctccttcc ccggggtaga agtccagggt gagaaattgg ttccgaactc aaaggaaccc 60

agtgccgggc cacagccggg tcacgtggcc ggcggccccc catg 104

<210> 789
<211> 173
<212> DNA
<213> Homo sapiens

<400> 789

attccttctc tgcatcgaag gatcaggaag tttgtgctct ctgcgtggct aagtttttca 60

cctactagga cgggggtggg gtggggagaa caggtgtcct tctaaaatac agcacaagct 120

acagcctgcg tccagccata acccaggagt aacatcagaa acaggtgaga atg 173

<210> 790
<211> 240
<212> DNA
<213> Homo sapiens

<400> 790

cgctcctcct cttcgctgcc ggtgggcacc gccgctcgct cgcacttctg cgcccattgg 60

agcttcggag atccctgcgg tcccgcggga cggcgcggca gcagctgacc tcgcagacag 120

gatcttgctc tcttgcccag actggaatac agtggtgtga acacggctca ctgcagcctc 180

aacctcctgg actcagagat gtcggcttat ttataggaat tgcttgaagc cagagtcatg 240

<210> 791
<211> 205
<212> DNA
<213> Homo sapiens

<400> 791

cgtcccttta agagcggctg gccaggcacg gcctccgcct ctcagtacgc ggagcgccgg 60

cggtcacctg ggctcgcgg agcggccaga tcgcggcgga gtcggcgcgc ttccccgagg 120

gaaggtggga gaggggaccc ggacgcgagg tgccccgaag ccctctcgag cgtaaccgtc 180

ccgcgcctct ctgaggcgga ggatg 205

<210> 792
<211> 903
<212> DNA
<213> Homo sapiens

<400> 792

ctgtctcttg gctcaggctt ggaggcctcc gagcagcaac atcgtcccaa ttataccccg 60
ttggagcatc ttcagatctt ccactctttt cacaacgcaa tcaaaatctt cgtacccatt 120
ttgcagtagt gatctctgta agttgcttta caattcataa agtttattct atttgatctt 180
cactctaatt tacaaagaaa agcagggaag tctatttctg ttttacagag gtgtacaggg 240
aggctcacag gggctaagtt cacacagtaa gccctcgaag ctgccagggc tgcaaagccc 300
accctctttc caccgcaccg aactacctcc tttcgcctac aaaacgtagg tggggaccac 360
tggtgttgga atgacggccc acctcgagtt tcaggtgact tccactctgc aattaacttg 420
caggcagccc cagacctgca atgaacacac gggtggggga gagatatgca cgccagggtc 480
agtgggaacc aacagccgag gggtgagcgg ggctaggggc cccgggccgc cggcggggca 540
aacgcggttc agaaacgcag gccgcgctct ggcccgcccc ctgcagcagc acggcctgct 600
cgccatcgcc cggagagcgc cgcgggttcc cgagtccggg cgcggagggc gcgcgggcac 660
ggcggcaggg gcgtgctcgg aggacgcgcg ctgcgctgct cctccaaagg gcagctccgg 720
gggaaagagg gtggcgtccc ggggaagccc gcagccgccg ccgatgtcgc tgggactcgg 780
aagtgccgaa agaggggtgt tgggaactcg cggcgcgcgt gaacgttgcc gtcgccgccg 840
cccgggacag cccggagaaa ctctcagcgt aggcatcggg aaccttcgtg ccaaggagcc 900
atg 903

<210> 793
<211> 633
<212> DNA
<213> Homo sapiens

<400> 793

cctccttttt ctcccaaacc acttcttccc ccctaccccc cgccacgcga ggctgcggcg 60
cacggtatgg gtgtgtttgt gtgtatttgt gtggggaggg cgtttggagg gaaggttacc 120
gggagctccg aggccgctgg ggaacaggga tcccggtgac aaagatgggg atatttcctc 180
tgtcttccac ttggaaacct caaccccccgc ttcaggctcc ctagatactt tctggggccc 240
aaccgaaggc cgtagccatc caaagcgttc ccagcctttc tggggagtga aacttacccc 300
cggggttcgt cctagaggag cgtgagcggg gaatgcccag gtcaaccggg ctgtccgaat 360
tccgccccgg ctcagcctcc ggcctcagtc cgggagagag atctgcctgt cggtctgggc 420
tgggggaaac gcggcagtgg cctgggccac aggtgagggc agagtaacca gtgggaaggc 480
tgcgttttca cgaaggactc gggtgaagct gcagagctgc ctttgagccc tgactccttg 540
gcttcctggg tcggaggaga tcttgtaatg gagtggttct tcgtctcact agcaagatgc 600
ctgatttcct caggatcaag ggattgaaga atg 633

<210> 794
<211> 414
<212> DNA
<213> Homo sapiens

<400> 794

agtccttcgc cgcattgggg caaaataatc ccttcatttt tgtgaaggta ccgtggaaaa 60

tatttcattt ttcttctcac cggagcaatt gtaaatgcta tgcggtaaga ggagttacct 120

gtggaaaggt ggttaagaga ttaggtaaag aaaaggaaag gacaccaaaa taaagtgctg 180

cggaagaatt tttgtccagc tgtgagacga cgagtgcgtg aagtgaaggc gattgagagg 240

ggctgaggga attgtcctct gtggaaggga ctttcttttg gccctaggcc ccttcctgcc 300

cctgtcgtca gcagagtctc tacaaggaag ataacggact gtaaaattct ataaagcaaa 360

gctacacatc acttgacacc atacaccatc ttggttacat aatgaagaga gatg 414

<210> 795
<211> 71
<212> DNA
<213> Homo sapiens

<400> 795

atgtctcttg acagcggcgg cggcgcagcc ggttccgggt tcggcgcggg gcggggatgt 60

gaatcccgat g 71

<210> 796
<211> 99
<212> DNA
<213> Homo sapiens

<400> 796

ccatctcttc ccttaggtgt ttaagttccg cgcgcaggcc aggctgcaac ctgacggcca 60

gatccctcgc tgtcctagtc gctgctcctt ggagtcatg 99

<210> 797
<211> 52
<212> DNA
<213> Homo sapiens

<400> 797
cttccttcca agaaccttcg agatctgcgg tctggggtct ggttgaaaga tg 52

<210> 798
<211> 78
<212> DNA
<213> Homo sapiens

<400> 798

gcacccottc cgcgcagccc cctgacctgc agcctccgga cctcgctgca gcgcggaccc 60

ggcccgcccg cccgaatg 78

<210> 799
<211> 161
<212> DNA
<213> Homo sapiens

<400> 799

ccgcctcttc cgctctacag cggaggtggc tgtggcggtg gcgctggtgg ctgcggcggc 60

ggcggcggca gcggcgctcg agcggttcct gtcagggtca gccggcgggc cccctgggtg 120

gtccacctgc aaatcgcgga gcggcgcccc agggatcgat g 161

<210> 800
<211> 38
<212> DNA
<213> Homo sapiens

<400> 800
gcgtctcttg tttgtgcggc tgaccagttg gcgacatg 38

<210> 801
<211> 721
<212> DNA
<213> Homo sapiens

<400> 801

cgttcttttc tttgtatttc cgcctctcgc ctctctctaa aagccgcagt tagaggcgag 60

atttaggaaa aacctctgcc gagtgagcct ctggttggga atatgtatga gaaaaaaaaa 120

ctggcaaggc gttagtcaag caaagctgaa ggcagaggaa atttgatatc tggctggagt 180

ctagaggatt taatgcaaat aagatactct gagggcagcg tggcaaaaaa agactacaat 240

tcccggtggt cacagcgttt gagaagcgat gctttctgag acttgtagta actaggagct 300

gtgtttgaac tatccaggct caggacagcc tcttgaaaaa aaatttttta ttaataaagc 360

ggatttgagt gggatctttt tcctaatcga ttacgggccc acacgtatgg gaagaattct 420

aacaatgatt aaagggacat gctaccttta cgactatcct tttctaatcg atgactccta 480

aatctaggag taggtagtcg atgtttgtgg tctgggcgtc tgtagaaggg caacctcgtg 540

ctttctgcag aggagaccgg agggcagaag gcagagtcca ggcttagact gcagttcctc 600

gcttacctgt gcagtctaat tttgagctgc ctctttgtag tcttaaaagg caggagcttc 660

gtgttgtggg tctgctaacc cgtacgtttc cgtgggcaag tcgtgtgtac tcctcgccat 720

g 721

<210> 802
<211> 77
<212> DNA
<213> Homo sapiens

<400> 802

```
cgacctcttc aagatggcgg gcgccggaga ctagcttccg cttccggtgt gagcggcccg      60
gccggggggg caagatg                                                     77
```

<210> 803
<211> 72
<212> DNA
<213> Homo sapiens

<400> 803

```
ttttctttat ggcgtgggag aggccacagc ccggactcca tcgactcccc cggctcttag      60
actaaaatca tg                                                          72
```

<210> 804
<211> 131
<212> DNA
<213> Homo sapiens

<400> 804

```
ctccctcttt cttcccctct ggggaagctc agtgctggac ttccgaagac cttttacgac      60
attgagtctc ggagttggtc tcagcgccgg atccactttt cggcaaagtg acgtggacgt     120
caacagcaat g                                                          131
```

<210> 805
<211> 800
<212> DNA
<213> Homo sapiens

<400> 805

gctcctctct ggggagtgga gggtgttcag ttattaatga ccgctgagca ggcagcacca 60

tgtcagtgtg acaactgatc gggtgaacga tgcaccacta accaccatgg aaacaaggaa 120

aaataaagcc agctcacagg atctctcttc actggattga gagcctcagc ctgccgactg 180

agaaaaagag ttccaggaaa aagaaggaat cccggctgca gcctcctgcc ttcctttata 240

ttttaaaata gagagataag attgcgtgca tgtgtgcata tctatagtat atattttgta 300

cactttgtta cacagacaca caaatgcacc tatttatacc gggcaagaac acaaccatgt 360

gattatctca accaaggaac tgaggaatcc agcacgcaag gacatcggag gtgggctagc 420

actgaaactg cttttcaagc atcatgctgc tattcctgca aatactgaag aagcatggga 480

tttaaatatt ttacttctaa ataaatgaat tactcaatct cctatgacca tctatacata 540

ctccaccttc aaaaagtaca tcaatattat atcattaagg aaatagtaac cttctcttct 600

ccaatatgca tgacattttt ggacaatgca attgtggcac tggcacttat ttcagtgaag 660

aaaaactttg tggttctatg gcattcatca tttgacaaat gcaagcatct tccttatcaa 720

tcagctccta ttgaacttac tagcactgac tgtggaatcc ttaagggccc attacatttc 780

tgaagaagaa agctaagatg 800

<210> 806
<211> 162
<212> DNA
<213> Homo sapiens

<400> 806

ggccctctct ccgcgcggag ccgagccgga actgcggcag tctctccctg ccaggctctt 60

catccaaggt ttctgtggat cccttctgaa gttctatctg aaaattgcgc ttaagtgaat 120

tttctgttag aagaacttgg ttgctacttt cttgtcaaga tg 162

<210> 807
<211> 116
<212> DNA
<213> Homo sapiens

<400> 807

ccgccccttt aacctttagg gtgcgcgggt gcagtatatc tcgcgctctc tccccttcc 60

ccctcccctt tccccacccc gggcgctcag gttggtctgg accggaagcg aagatg 116

<210> 808
<211> 29
<212> DNA
<213> Homo sapiens

<400> 808

cttcctctag aacccgaccc accaccatg 29

<210> 809
<211> 104
<212> DNA
<213> Homo sapiens

<400> 809

gctcctcttt tccagtcctc cactgccggg gctgggcccg gccgcgggaa ggaccgaagg 60

ggatacagcg tgtccctgcg gcggctgcaa gaggactaag catg 104

<210> 810
<211> 383
<212> DNA
<213> Homo sapiens

<400> 810

cctcctcctt cctcctcctc ctcctccttc ttctcctcct tctcggccgg gaggaggcag 60

ggctggatcc ctcagccgcc gccgctcctc ctcctggcag gccggccgcg gagtcagctg 120

acgccggcgc tccagcctcg cctccccgcg ccgcgctctg cgctccccga aagtggctgc 180

aagccggccg cccactgtca gggttggggg gacagagaaa gtgatgtgcg ccttctaaag 240

cctcgcccag cgccgccgaa gcagcttcac ctctccaact ttctcccacc gactgcttgt 300

cttgaccctg ccctccaccc tccccagagc cacttcgggt gcgcgctctt gggtaaaggg 360

ggggtcaccg gctgtctggg atg 383

<210> 811
<211> 175
<212> DNA
<213> Homo sapiens

<400> 811

tctcctccat cgcctgcagt aagggcggcc gcggcgagcc tttgagggga acgacttgtc 60

ggagccctaa ccaggggtat ctctgagcct ggtgggatcc ccggagcgtc acatcacttt 120

ccgatcactt caaagtggtt aaaaactaat atttatatga cagaagaaaa agatg 175

<210> 812
<211> 367
<212> DNA
<213> Homo sapiens

<400> 812

tggccttttg ccctagggag cgagtgcgga gcgagtggga gcgagacggc cctgagtgga 60

agtgtctggc tccccgtaga ggcccttctg tacgccccgc cgcccatgag ctcgttctca 120

cgcgaacagc gccgtcgtta ggctggctct gtagcctcgg cttaccccgg gacaggccca 180

cgcctcgcca gggagggggc agcccgtcga ggcgcctccc tagtcagcgt cggcgtcgcg 240

ctgcgaccct ggaagcggga gccgccgcga gcgagaggag gagctccagt ggcggcggcg 300

gcggcggcag cggcagcggg cagcagctcc agcagcgcca gcaggcggga tcgaggccgt 360

caacatg 367

<210> 813
<211> 25
<212> DNA
<213> Homo sapiens

<400> 813
cgctcttcct caggcggcgg ccatg 25

<210> 814
<211> 91
<212> DNA
<213> Homo sapiens

<400> 814

cggtcttcct cctcgtcctg ccgcagggcc agaacccctg acggtattca gctgcgcgta 60

agtctggccg gtgccatctg tctccgcaat g 91

<210> 815
<211> 73
<212> DNA
<213> Homo sapiens

<400> 815

cggtctcctt cggcaacccc ggccgaacgg ccacccagag gctgtgctga gctggcgcag 60

cggcagcagc atg 73

<210> 816
<211> 170
<212> DNA
<213> Homo sapiens

<400> 816

gtttctttcc tacgcagccg ctcctgccgc cgtggtcgct ggagctttgc ctctctaggc 60

cggcagcgcc tctcctccat ggtcctgtct gtcagcgctg ttttgggagc ccgccggtga 120

ggccgggcca cgctcagaca cttcgatcgt cgagtctgtc actgggcatg 170

<210> 817
<211> 71
<212> DNA
<213> Homo sapiens

<400> 817

gattcttttt ggatagggtt gacgttcgtg gatagactca tatctgtgac cagtgtccgc 60

caccgcggat g 71

<210> 818
<211> 54
<212> DNA
<213> Homo sapiens

<400> 818
ccccctccct gcccacctcc tgcagcctcc tgcgccccgc cgagctggcg gatg 54

<210> 819
<211> 96
<212> DNA
<213> Homo sapiens

<400> 819

cgtcctccca agatggcgga gacagagtga agaaactgtg ttccccctt gggttgctat 60

cgatcaaggg taaaattcca ttctgatatc aaaatg 96

<210> 820
<211> 342
<212> DNA
<213> Homo sapiens

<400> 820

gcttcttttt ctggtagaag gcggggttct cctcgtacgc tgcggagtct ctgcggggtg 60

tagaccggaa tcctgctgac gggcagagtg gatcagggag ggagggtcga gacacggtgg 120

ctgcaggtct gagacaaggc tgctccgagg tagtagctct cttgcctgga ggtggccatt 180

cattcctgga gtgctgctga ggagcgaggg cccatctggg gtctctggaa gtcggtgccc 240

aggcctgaag gatagccccc cttgcgcttc cctgggctgc ggccggcctt ctcagaacga 300

agggcgtcct tccaccccgc ggcgcaggtg accgctgcca tg 342

<210> 821
<211> 134
<212> DNA
<213> Homo sapiens

<400> 821

aggcctctcc aagcccctac cgcacaggct catagcccca agcccggagg aggtggctac 60

attgtgtcta ttgtatccct tggctggtgt atttgtacat ctctcgggac gtgaaattga 120

cagtgaaaag tatg 134

<210> 822
<211> 245
<212> DNA
<213> Homo sapiens

<400> 822

cttcctctgg cggcgtccgg ccgcttctcc tctgctcctc gaagaaggcc agggcggcgc 60

tgccgcaagt tttgacattt tcgcagcgga gacgcgcgcg ggcactctcg ggccgacggc 120

tgcggcggcg gccgaccctc cagagcccct tagtcgcgcc ccggccctcc cgctgcccgg 180

agtccggcgg ccacgaggcc cagccgcgtc ctcccgcgct tgctcgcccg gcggccgcag 240

ccatg 245

<210> 823
<211> 362
<212> DNA
<213> Homo sapiens

<400> 823

cgtccttctc gcgcctcgct ctggccctgc aggttgtgtt tccgcctcta ccccgcctcc 60

attccgttgc tctctcagtc tcagacccgg gctctcggtc cgccgcttca ggtcttggcg 120

cagcctcaga gagttggcgc ggctctgtgt tgaccaaacc tagtggatgc agttagcgcc 180

ggagcccggc cccgcccgtc accagggtta ttcccgcctt ctaggtttgc caggactgcc 240

ggccctgcag ctgccttctg ccccaggttt ttggctactg atgttacaaa caataaaata 300

ttggagcata gagttgaaga acagactcaa accaggtttt tatttaatta gttaaaaata 360

tg 362

<210> 824
<211> 298
<212> DNA
<213> Homo sapiens

<400> 824

tttccttttc cctccccctg gagctgtagc ggcagcagca gcaggaagcc gagccgggtt 60

gagcgactcg gaggcgagcg gaggagctgg aatatgggga gtcagcgagg acggtggggc 120

caggagccct tgggagggcc tacggaggga gcggccccag gcgctttcta gagcgtgagc 180

ggtgggggag caggcgcagg gtggcacgag cggaggcggg gcccgggcgt ggggcacggc 240

tggggaagct gccgcctccg gccctgcccg gctgcctccg ccgcggccag tggctatg 298

<210> 825
<211> 357
<212> DNA
<213> Homo sapiens

<400> 825

gttccttttt gggttagctt tggcagtatt gagttttact tcctcctctt tttagtggaa 60

gacagaccat aatcccagtg tgagtgaaat tgattgtttc atttattacc gttttggctg 120

ggggttagtt ccgacacctt cacagttgaa gagcaggcag aaggagttgt gaagacagga 180

caatcttctt ggggatgctg gtcctggaag ccagcgggcc tcgctctgtc tttggcctca 240

ttgaccccag gttctctggt taaaactgaa agcctactac tggcctggtg cccatcaatc 300

cattgatcct tgaggctgtg cccctggggc acccacctgg cagggcctac caccatg 357

<210> 826
<211> 118
<212> DNA
<213> Homo sapiens

<400> 826

gcttctcttc cgctccgggt cggctccgtt tccctttccg ggcgggcagg cggcggaccc 60

cagtgtcttt atccctcttt tgcacagtca gcttctgcag ctctcccggg ctagcatg 118

<210> 827
<211> 62
<212> DNA
<213> Homo sapiens

<400> 827

cgacctcttg gctccgctag tgcccggcgc gccgccgcca gtgctgcggg ctccgggcaa 60

tg 62

<210> 828
<211> 879
<212> DNA
<213> Homo sapiens

<400> 828

```
ctttctctca ggaaactcca ctcccaactg acaggtgcta tttccagcca gtcctatgct      60
gttgcaaata gtgagtccat gaatgccctc tgccgtgtgc attacttatt ttcatcagca     120
gatcttcgta acacactcct ggaagtggga tgacggggtc aaaaggcgaa tccatacata     180
agttaaatag atattgctca attctcttcc acggggttca gaccattttg gatttctacg     240
agcaatgaag acagtgctat tcctctacac cctggccggc caactgagcg tggttaaacg     300
tggggaggga ggagggtgag gttaccaacc tgatggttga gaaagggcct ccgcccagcg     360
cgcccttcct ccaccccac ccgagagaca gctgaactcc ggccgggacg cgcgtgttgc      420
cagtccagcc ctgcaccgcg tcccctgagg gcgggctgca ggcggccggg aagccttgca     480
caaccggccc aaaagaggaa gcccagaaag tgctgaagta aacactttgg gagaccgttg     540
caacataaag cggcctctca gtctttggtg gaaccatcac taggccccaa tcccttagtc     600
cctcttgcgt cgaggctgca aaatggttcc attcgccagg agacgctcct gagagaaggg     660
cgcgcgcggc acaggggcct tccttgcacc tcggagcaaa gcagctcgga tagcgccaca     720
cgtctgcgcg ctgcgtggga agggcagggc tgacagcact tcctccccgg ggcagcgacc     780
tggagcccgg gtgcggcagt ctgcaccgcg cgtcgctttc ccggccggag tctcgccgcc     840
ttcccgcgcc ccgcagcgcc ccgcagagca gtcgagatg                            879
```

<210> 829
<211> 77
<212> DNA
<213> Homo sapiens

<400> 829

```
ccttccctct tcactgtgag ctcagagcag caggacaaag tgctcgggac aaggacatag      60
ggctgagagt agccatg                                                      77
```

<210> 830
<211> 77
<212> DNA
<213> Homo sapiens

<400> 830

```
acctcctttc cctttcggat tcccgacgct gtggttgctg taaggggtcc tccctgcgcc      60
acacggccgt cgccatg                                                      77
```

<210> 831
<211> 76
<212> DNA
<213> Homo sapiens

<400> 831

```
ttttctttta ttcttgtctg ttctgcctca ctcccgagct ctactgactc ccaacagagc        60
gcccaagaag aaaatg                                                        76
```

<210> 832
<211> 46
<212> DNA
<213> Homo sapiens

<400> 832
cctcctctac tatctcggta tcaccaaacc cttgccggct cttatg 46

<210> 833
<211> 350
<212> DNA
<213> Homo sapiens

<400> 833

```
ctgcctctta tgaagcaata ctagagagga aaaacaaaac ccattccttt aagaaagatt        60
ccgcctcctc tcataagcaa gcgcctaatg gtaattgtag agtttactaa gtcaaacact       120
tactactcag cattgagaga agctgctgct gctaatgctg ctgctgctgc tgccgccgcc       180
gccgctgctg ctgctgctgt tggtctgagg ctgcagtagg tttctgtgca gcattgcaga       240
atccacacct agagaacaga agacacagac acgtacgtct actacccttg ttagaaggaa       300
gctttggatc ttcggtggat aacaagagta atccacagac ttaaaacatg                  350
```

<210> 834
<211> 199
<212> DNA
<213> Homo sapiens

<400> 834

```
agcccttttg gatctaatgc gcagaggagg ttggcccaga gctcccgggc tcccccaagg        60
ctgaactccg tccaaggtgc ccgcaggctc cctgcccgcc ttccccatgc cagcccgcag       120
ctaggggcag gggcagcggc ggctggggtt gggggtgggt ggggagcttt tggggaggac       180
aggtcgcagc ttggctatg                                                    199
```

<210> 835
<211> 279
<212> DNA
<213> Homo sapiens

<400> 835

```
ttatcttttt gcctagcgac tgacaacagg ctggttgctt ggcgtggaat cctaaagtgg        60
cctggctttg agactggagt gagaccccag ccctaggctg gggttctttc cattatagag       120
gagacggatt cagaagggct acagaccaag gttgttgaaa accagacata tgatgagcgt       180
ctagagatta acgactccga agaggttgca agtatttata ctccaacccc aagacaccaa       240
ggacttcctc gttctgccca tcttcctaac aaggctatg                              279
```

<210> 836
<211> 1573
<212> DNA
<213> Homo sapiens

<400> 836

```
ccccctttc gggaggaggg aggcagggac ttgcaggcaa gagttgcacc tggtctagga         60
acctgcagag aaaagaactc tggggtaagt agtgttctgg cactggcacg gaaaggggta       120
aagggtgggg ggcatgagag ggacgaaatg gagagggcag ggaatgaatt atgcaaaaaa       180
atctccaata tttcgcagcg gagggagagc acagcacagc actcccagga tgagtcctgc       240
ctgggtctcc cgcgccgaac ccgcagcacg aagttctttt taagaagaga aactcgaaaa       300
tcctggaggg taacagaggc agccagggcg gggcggagtg cggaggcggc tgccagggac       360
tggggccgag gcggcggcca aggtggcctg aagctgtgac acccagcctc ctcctcctcc       420
tcctcatggc cgcgctcagc ctcacctccc cgcccgggcc tcctgcctcc gcccccgggt       480
gccgggctgc ggagctgacg ctgggacgcc cggcggcggc gaggacgctc acctggccaa       540
gcctccttct cctcctcccc ctcccgcccc cacctgtcct cctcctctct gagttgggaa       600
gcgtagggat ccgtaggcga ggaaataacg acccctgcag ttgtattgcg gaaaatctcg       660
acagcggcgc tagttgcggg cgatggaagc caggcaactg gggttctgg ggagttcagg        720
aaaatagcag aggagcagga agggcgcgcg cgacctggag agtctgtgtg cccccaccgc       780
gccccagtcc ccggggccca gcccttcccc tcggcgccct ggacgcactg ccggaacccg       840
gctgagaggc tgcaggctgc gcgcggacct ggggagcagg gagggtcggc ggaggctgcc       900
ggcggctggc ggtttcgggc aataatccct gcctctcttt ctctgtgtgt ctgctgtgtc       960
```

tgctccttcc ccgcccccg gaagcaggag aagaactgcc ccggagcgca gcagccaccc 1020

tccgaccatg ccccggtgag gggggcggac ttcgagggca acttgccgcg gactgcctgg 1080

gcttagccag cgagctacgc gctcccggga gcccggaatt gcacggcgca gcccggcggg 1140

gggctatcgt ctatgtcttc ttggggcgcc agacgaatcg gggtctcgtt tttgctggaa 1200

gagcccagtg ttggtggctt caggtggctg ctgccgccgc cgccgccgcc gccgctgcta 1260

gtgcggtttc cgccgctggt gcgaagagaa gagacacgcg agcggggaga cctccaaggc 1320

agcgaggcat cggacatgtg tcagcacatc tggggcgcac atccgtcgag cccgagggga 1380

gatttgccgg aacaattcaa actgcgatat tgatcttggg ggtgactgtc cctggccggc 1440

tgtcgggtgg gagtgcgagt gtgcactcgc tcggaagtgt gtgcgagtgt gtatgtgtgt 1500

gtgccgtgtc gggctccccc cttcccccg ttttcccgtc gagtgatgca cttggaatga 1560

gaatcagagg atg 1573

<210> 837
<211> 76
<212> DNA
<213> Homo sapiens

<400> 837

cctcctccct gtaacatgcc atagtgcgcc tgcgaccaca cggccggggc gctagcgttc 60

gccttcagcc accatg 76

<210> 838
<211> 38
<212> DNA
<213> Homo sapiens

<400> 838
gttccttcta ctctggcacc actctccagg ctgccatg 38

<210> 839
<211> 36
<212> DNA
<213> Homo sapiens

<400> 839
ccgtcttccc ttcccgcgtt ccccgggaga aacatg 36

<210> 840
<211> 35
<212> DNA
<213> Homo sapiens

<400> 840
cctcctttcc gcttccggtg tcccctacag tcatg 35

<210> 841

<211> 76
<212> DNA
<213> Homo sapiens

<400> 841

gctcctcctg ctggctgggg attttccagc ctgggcgctg acgccgcgga cctccctgcg 60

accgtcgcgg accatg 76

<210> 842
<211> 130
<212> DNA
<213> Homo sapiens

<400> 842

cctcctctcg cgagaggcgc aaggcgtgga gtcgacggct ggagagaagc cgggagcgag 60

cccaggcggc agtcttgatt cccttttggc cagcagtttt taggtctgtc agtactgcac 120

tgcaagaatg 130

<210> 843
<211> 113
<212> DNA
<213> Homo sapiens

<400> 843

taccctcctt ccccattttc tgtggtccaa ctaccctcgg cgatcccagg cttggcgggg 60

caccgcctgg cctctcccgt tcctttaggc tgccgccgct gcctgccgcc atg 113

<210> 844
<211> 174
<212> DNA
<213> Homo sapiens

<400> 844

agccctccct tccgcgcgct tactttgttt ataacttgaa aaatcctctc cgtctccctt 60

ccctgcctcc tttcctttcc ctttcctctg ccagtacaac tagacccggc gtctggcgtc 120

cccggtgccc agcattctgc ggggcaggcg gattaattgg aattcttcaa aatg 174

<210> 845
<211> 185
<212> DNA
<213> Homo sapiens

<400> 845

cctccttccg gctgggcaag gggccgcggg gagcagctcg ggactgaacc gagaggtgcc 60

gaaggaaccg gcgggccgct tgatcccgct gcagacgtag gagatgcctg ggacaaggag 120

gccaccttct cagggcaaaa gaaaaagaag gtgacaggcg ttgagaccac cgaagggaac 180

ccatg 185

<210> 846
<211> 125
<212> DNA
<213> Homo sapiens

<400> 846

agttctctct gggaacatct ggtgggtact acaggcccta ttccaggccc tatggcctgt 60

ggaacctcac cacgggggg agggctgggc cagacggaga catcacctgt ggtgtcagcc 120

ccatg 125

<210> 847
<211> 82
<212> DNA
<213> Homo sapiens

<400> 847

cctccttcgc gccggccctt ccgcgggtga tcagctggtc tgcgctcccc tgacgtgggc 60

tggggcacgt caccgccgaa tg 82

<210> 848
<211> 153
<212> DNA
<213> Homo sapiens

<400> 848

gggtctcttc cggagtcttt tcctggacgg ggtccctgcg gtgggtgtgt ttcggcctgg 60

cctgggcagg cgcttgtgct gccagggcgc cgggcccggg gaggccgggg tctcgggtgg 120

ccgccggccc aggcgctgga cggcagcagg atg 153

<210> 849
<211> 16
<212> DNA
<213> Homo sapiens

<400> 849
ttttctttcc aaaatg 16

<210> 850
<211> 54
<212> DNA
<213> Homo sapiens

<400> 850
ggttcttcac tcgcgactga cggagctgcg gtggcgtctc cacacgcaac catg 54

<210> 851
<211> 289
<212> DNA
<213> Homo sapiens

<400> 851

ccttcttcct cttgttcctc ctcctgcctc tcttcgcttc gcctgcaaac gcggtggggg 60

ctgctcggcg gtcaggagca ggttaccctc cgtctgcatg cccaccatca aggtatgagg 120

atggtagaag ctctcgtcga accagatgga tgaagaccac taacggcttt tgtttcctct 180

ggtaacagca agagacagag cgacatgaga gattggaccg cgggctgcac tggagaattt 240

actggtagga taattcatcc ctaaagagat tgaagtgagc ttcagaatg 289

<210> 852
<211> 151
<212> DNA
<213> Homo sapiens

<400> 852

cggcctccgc ccctgcagcc gcgggcacgc ggaggggctc ctggctgccc gcacctgcac 60

ccgcgcgtcg gcggcgccga agccccgctc cccgcctgcg cgtctgtctc gtccgcatct 120

ccgcggcctc ctgctccacg acgtgaccat g 151

<210> 853
<211> 113
<212> DNA
<213> Homo sapiens

<400> 853

ttttcttctc gggctgcaaa caaagggaag cctgcaacaa gttaagctga agaccgaagc 60

aagagctggt tcaggtggca gccacagcag cctcagggac ctcagcaact atg 113

<210> 854
<211> 198
<212> DNA
<213> Homo sapiens

<400> 854

ctgtctcttt aaggtgcccg aggctcgcgg gcgctgcgct gaggggacgg cgggaggcgc 60

ggcctggcct cgcactcaaa gccgccgcag cgcgccccgg gctcggccga cccggcgggg 120

atctaggggt gggcgacttc gcgggaccgt ggcgcatgtt tcctgggagt tactgatcat 180

cttctttgaa gaaacatg 198

<210> 855
<211> 313
<212> DNA
<213> Homo sapiens

<400> 855

gtcccctttg tgaggcccgg gatgggaggt gcccggttcc cccagggaca gcttcaagcg 60

gtagggacag acatctgagg acccagcctc agggatgctg tccccgggct tccaggctcc 120

agcgccgtag gactgaggca gactccacgg tgagaaagag acccgatcta acccaggcct 180

ttcatcagag cccaggaggg aaggcaggaa gtgggaccac gaggcccggg gggcttctaa 240

ctcgtctggc cagggagatc tgaattgggg tgaagagcag aatctccaga acaaggagga 300

ggtggtgatc atg 313

<210> 856
<211> 115
<212> DNA
<213> Homo sapiens

<400> 856

gctcctcctg tcttgtctca gcggctgcca acagatcatg agccatcagc tcctctgggg 60

ccagctatag gacaacagaa ctctcaccaa aggaccagac acagtgggca ccatg 115

<210> 857
<211> 81
<212> DNA
<213> Homo sapiens

<400> 857

tgctcttctc gttcccgaga tcagcggcgg cggtgaccgc gagtgggtcg gcaccgtctc 60

cggctccggg tgcgaacaat g 81

<210> 858
<211> 24
<212> DNA
<213> Homo sapiens

<400> 858

ctgtctttac ccagagctac catg 24

<210> 859
<211> 648
<212> DNA
<213> Homo sapiens

<400> 859

```
ctctcttttt cttgcagaac cgtctctctc ccttctctgt ctcttagcac agagctctta      60
ttcagccact agcttggccc ttcctgcttc aattgtaatg cttgttctgc ccgtccacag     120
actattggcg gcagaaacaa cgaatttcct ccaaactagg cggtgttggt ggctcttgca     180
ttcctctgga tgaggaaatc tagttggggg gttccagaag gggaaggctc ctgggctttc     240
aatacatcct cctgaatcat acctcgtttc gggttcccta gaaaaatctg gacgtgtaaa     300
aagaactctt aacggccgat gcagctcttc caaagctaag gctgccttgg agttttcata     360
agaaattgtc cctggaggtg ttggatgatc acagcttcct tggagcattg cagttgctgg     420
aatccagttt caggattaag ggagggctgc ctccttgcaa tgggctgcca agaaaacggc     480
tgtgcttgtt cttaacctca ggctctgtct gtgatcagtc tgagagtctc tcccaggtct     540
actgctccct ggaaagccct atctctctgc aggctcgcct ctgggctttg tctccttgga     600
gccacatcac tgggacagct gtggatgtgg atgcagattt gaaccatg                  648
```

<210> 860
<211> 144
<212> DNA
<213> Homo sapiens

<400> 860

```
ccgcctcctc gctgcgggaa gggtcctggg ccccgggcgg cggtcgccag gtctcagggc      60
cgggggtacc cgagtctcgt ttcctctcag tccatccacc cttcatgggg ccagagccct     120
ctctccagaa tctgagcagc aatg                                            144
```

<210> 861
<211> 49
<212> DNA
<213> Homo sapiens

<400> 861
ctgtctccat cttgtctgta tccgctgctc ttgtgacgtt gtggagatg 49

<210> 862
<211> 87
<212> DNA
<213> Homo sapiens

<400> 862

cctcccttt ttttccgcct tctgccagca gaagcagcag ccgcagcacc tgagccgcta 60

ctgccgctca ctcaggacaa cgctatg 87

<210> 863
<211> 161
<212> DNA
<213> Homo sapiens

<400> 863

ggacctccct ctttgcctcc tccctgttcc aggagctggt gccctgggct ctgcgctgtt 60

gttttcagcg ctccgaaagc cggcgcttga gatccaggca agtgaatcca gccaggcagt 120

tttcccttca gcacctcgga cagaacacgc agtaaaaaat g 161

<210> 864
<211> 187
<212> DNA
<213> Homo sapiens

<400> 864

cttccttctg gagctgggtc ctgactaggg accgcctggg tgaggtgagg acctggtggc 60

cgcagttgtg gcactgtgcg caggcgctga actgaccgga cggagcgggc ggctgtggcc 120

tcgccagctg gtttaaaaat atcctttttt gctgaaggaa cacatttgct ggtatagttt 180

cagaatg 187

<210> 865
<211> 388
<212> DNA
<213> Homo sapiens

<400> 865

ctatcctttc ctctcagtcc tgccatctag ctgccttggg tctcgcgctc cgcagagcgt 60

tccgacactc tccggcctcg ttctgccgcc tccgcgcgct ctccccgtgc ggccaccgcg 120

ccccccaagc ttgcctcctt cttgccggac ttggggccgc gcgccctgac tccttcccct 180

cccgcggacc cgcgcactcc cggcgcggcc tctcccccac gcaggccacc gtgcactctg 240

tggcctcccc ctccttcccc gctctcctcg cgcttctctg gctccctagc tgtcgcgctc 300

tcctcggcga gcgcgctccc ggcccgcgcg ctccgggctc cggtttctcc cggctcctgt 360

cagtgcggtg actgcgctgg gaaacatg 388

<210> 866
<211> 488
<212> DNA
<213> Homo sapiens

<400> 866

cctttctcctg agagtcggag ccacagccag agccctgccc aggccgagcc ggagctgcag 60

cccgagcgcg gtggtgccct cagccccgtc ctcttgtcct cctcagcctc ggtgccttgg 120

aatttgtgtc gctgagtcag caagcctttc agatttgccc ggtttttgtt gtttgtggtt 180

tgtatcaaga tgggaactca aacaagtcat tcctcctaag gagctggtgt cttcatccag 240

aagggacagt ttgtgccagc tctccagaga gaaaaggatc tggtactgtt ctggagtggc 300

ctgtagcaga cactgaacca ccagccagct gcatttgttg tcctggaagt cattgccaac 360

tctgccagtc acactggggt ccccagagaa gtcaagatct gccggaggcg ctgggcaatg 420

accccgggac tccaggccag aggggtctga agctgtttgg gaaagcagcg ggactccttg 480

ggaagatg 488

<210> 867
<211> 191
<212> DNA
<213> Homo sapiens

<400> 867

ctgccttttt caccacctct aatttcagct tcagcagttg cttggaactt tggttctggc 60

agcagcagca acatcattac cgctagcggc agttttgtgc cgaggcacct acacacctcc 120

cgtcctctct gccagatcgc gggcctgtcg gtgtctgctc ctacacgcca acgccggtgg 180

gcaggaccat g 191

<210> 868
<211> 61
<212> DNA
<213> Homo sapiens

<400> 868

cgccctttca ccccggacgt gggcgggaga ggaagcggct ggtgatgctg gaacaaacat 60

g 61

<210> 869
<211> 99
<212> DNA
<213> Homo sapiens

<400> 869

cgctctttct ccgacagctg ccgggggtgc cctgcaagct gttccgcgcg tcctgcccgt 60

ctgtccccgc gggtcgtcgc ccgccacagc cgcgccatg 99

<210> 870
<211> 90
<212> DNA
<213> Homo sapiens

<400> 870

cgctcttttg tttcttgctg cagcaacgcg agtgggagca ccaggatctc gggctcggaa 60

cgagactgca cggattgttt taagaaaatg 90

<210> 871
<211> 168
<212> DNA
<213> Homo sapiens

<400> 871

agaccctttt cagacccttt tccggctgac ttctgagaag gttgcgcagc agctgtgccc 60

ggcagtctag aggcgcagaa gaggaagcca tcgcctggcc ccggctctct ggaccttgtc 120

tcgctcggga gcggaaacag cggcagccag agaactgttt taatcatg 168

<210> 872
<211> 92
<212> DNA
<213> Homo sapiens

<400> 872

gggtcctttt tcctctcttc agcgtggggc gcccacaatt tgcgcgctct ctttctgctg 60

ctccccagct ctcggataca gccgacacca tg 92

<210> 873
<211> 79
<212> DNA
<213> Homo sapiens

<400> 873

ggctctctcc ttgtcagtcg gcgccgcgtg cgggctggtg gctctgtggc agcggcggcg 60

gcaggactcc ggcactatg 79

<210> 874
<211> 102
<212> DNA
<213> Homo sapiens

<400> 874

ggctctttcc gctatctgcc gcttgtccac cggaagcgag ttgcgacacg gcaggttccc 60

gcccggaaga agcgaccaaa gcgcctgagg accggcaaca tg 102

<210> 875
<211> 256
<212> DNA
<213> Homo sapiens

<400> 875

gatccctttc ccagtcctgc ttcccagtgc ctcgggccag ggaatcctgg cctccgcctg 60

cggagccggc ggaacccgct tcccgcctcc acggggcagc gccagcggcc tggtcctttc 120

accggcagct ccgtgccgac gctctcaccg ctcttcctat cgccgggagt ggcgggccga 180

ccagggggcg gccgggctac cgtccgccat tcccgtgtct ctgcgcccgc gggggccgcc 240

cgagccggcc accatg 256

<210> 876
<211> 193
<212> DNA
<213> Homo sapiens

<400> 876

ccgccttttc cagttccagg tgtgcagaag tgtcctctcc ccacgcgcgg cgggctgcac 60

ttggtcgctg gctccgagat cgcgcggggc cgccggaagc ccaagacggt accgggggcc 120

gcagccgcag ccggcgccgc cctccgccct ccccaacagc aggccgagtc ccgtagcatc 180

cggtagggaa atg 193

<210> 877
<211> 389
<212> DNA
<213> Homo sapiens

<400> 877

agctctttcc gggggcccgg ggaactactc tccttgcctc gctctgtctc cttcgaagtg 60

ctctgcgcga ggttcagagc ggccgccgcc tccaaaggga cggttttcta gagctccgac 120

gcctctcggt gcccctctgc tccggccctt gccctttgac ctcgctctcg cggcagggtg 180

agaggtcggg tggccatctt gtggcggcgg cgcgggcggc tgttactgcg gagacccatc 240

ccctccccct tctcgcaccc ctggcagtct gtcagtcggt aaaaagtccc gcagcctgtc 300

aggtgaggcc ccggcctcgt gccgtcgctc ttcccgccgc actgggcggc ccaggccgct 360

ccctgccggg cctcactgcc gccaccatg 389

<210> 878
<211> 274
<212> DNA
<213> Homo sapiens

<400> 878

```
ctatctttct agacaaggca gttgaggagg agggagcgct tgagggggac tggcctggcg      60
tgcactccgc acctcgggga cattattgcg cgtggaacgg ctgcttttgg aaggcacaac     120
ttcctgaatg gaccatgact cccaccaaag atccctgtct ctgattcacc aaacagcttc     180
aaccctgaaa ccaggacgag aagttgacaa catctgagtg gacagctaat tgacctaaga     240
cttcagacca gactattgcc cagaagaaaa gatg                                 274
```

<210> 879
<211> 513
<212> DNA
<213> Homo sapiens

<400> 879

```
gggcctttta tctcggtgct gccgggggag gcgggaggag gagacaccag gggtggccct      60
gagcgccggc gacacctttc ctggactata aattgagcac ctgggatggg taggggggcca     120
acgcagtcac cgccgtccgc agtcacagtc cagccactga ccgcagcagc gcccttgcgt     180
agcagccgct tgcagcgaga acactgaatt gccaacgagc aggagagtct caaggcgcaa     240
gaggaggcca gggctcgacc cacagagcac cctcagccat cgcgagtttc cgggcgccaa     300
agccaggaga agccgcccat cccgcagggc cggtctgcca gcgagacgag agttggcgag     360
ggcggaggag tgccgggaat cccgccacac cggctatagc caggccccca gcgcgggcct     420
tggagagcgc gtgaaggcgg gcatcccctt gacccggccg accatccccg tgcccctgcg     480
tccctgcgct ccaacgtccg cgcggccacc atg                                  513
```

<210> 880
<211> 137
<212> DNA
<213> Homo sapiens

<400> 880

```
ctctcccttt gtcattctag ctgcctgctg cctccgcagc gtccccccag ctctccctgt      60
gctaactgcc tgcaccttgg acagagcggg tgcgcaaatc agaaggatta gttgggacct     120
gccttggcga ccccatg                                                    137
```

<210> 881
<211> 60
<212> DNA

<213> Homo sapiens

<400> 881

cttcctcttt tctaagcttg tctcttaaaa cccactggac gttggcacag tgctgggatg 60

<210> 882
<211> 501
<212> DNA
<213> Homo sapiens

<400> 882

ctttctcttt ttgtttggct tctaacgcgt tgggactgag tcgccgccgt gagctccccg 60
aagactgcac aaactaccgc gggctcctcc gccccgtctg cgattcggaa gccggcctgg 120
gggtcgcgtc gggagccctg gcgctgcagc tccgcacctt agcagcccgg gtactcatcc 180
agatccacgc cggggacaca cacacagagt aactaaaagt gcggcgattc tgcacatcgc 240
cgactgcttt ggggtaacaa aaagacccga gttgcctgcc gaccgaggac ccccgggagc 300
cgggctcgga gcagacgagg tatccggcgg cgcccatttg gggcttcta actctttctc 360
cacgcagccc ctcttctgtc ccctcccctc tcgctccctt ttaaaatcag tggcaccgag 420
gcgcctgcag ccgcactcgc cagcgactca tctctccagc gggttttttt ttgtttgtcg 480
tgtgcgatcc tcacactcat g 501

<210> 883
<211> 424
<212> DNA
<213> Homo sapiens

<400> 883

cgtcctctcc gggggcggag cgggtcggcg ggcctgacag ggaacctccc tgaccgagcc 60
cacgtctccc cacggccaga gaaatctccg gcccggcccg catcgccagc ccccaggccc 120
ggaggaacgg cccgagccca ggagaaccac atcttcgtcc cagccccgga ggctcctgtg 180
ggcaagatcg tgagccaacg ggttcctgag gcccctcctg gccaggcagg gtttccccgc 240
gcgtttccga ggagccctgc ctggccgggc ggctggacaa acaggtcgta gcaccgatcg 300
cgcccgcccc cagcaggggt cccgcacagg cttgcccctg acccccaccc aaacctgtcc 360
ttccgctttg cccccaaaca gtgcacttgc cggcggtccc aacccagcag gagaagtgga 420
catg 424

<210> 884
<211> 22
<212> DNA

<213> Homo sapiens

<400> 884
ggctctcccc gcgtccaaga tg 22

<210> 885
<211> 153
<212> DNA
<213> Homo sapiens

<400> 885

gcgccttcct cttcccatcg cgcgggtcct agccaccggt gtctccttct acatccgcct 60

ctgcgccggc tgccacccgc gctccctccg ccgccgccgc cttgctgctg ctcaaagctg 120

ctgccgcccc ttgggctaaa aggttttcaa atg 153

<210> 886
<211> 304
<212> DNA
<213> Homo sapiens

<400> 886

ctttctcttt ccctttgcaa ttgccttggg tcctgccgca cagagcggcc tgtctttatc 60

agaggtccct ctgccagggg gagggcccca gagaaaacca gaaagagggt gagagactga 120

ggaagataaa gcgtcccagg gcctcctaca ccagcgcctg agcaggaagc gggaggggcc 180

atgactacga ggccctggga ggtcacttta gggagggctg tcctaaaacc agaagcttgg 240

agcagaaagt gaaaccctgg tgctccagac aaagatctta gtcgggacta gccggccaag 300

gatg 304

<210> 887
<211> 251
<212> DNA
<213> Homo sapiens

<400> 887

gctcctcctc ctcccctccg tcggtcagtc agtccgcgag gagagtccgc ggtggcggcg 60

acggtggcga gagccgcggg ggccgtagga agccaacctt ccctgcttct ccggggccct 120

cgccccctcc tccccacaaa atcagggatg gaggcgcctc cccggcaccc tcttagcagc 180

cctccccagg aaaagtgtcc cccctgagct cctaacgctc cccaacagct acccctgccc 240

cccacgccat g 251

<210> 888
<211> 71
<212> DNA
<213> Homo sapiens

<400> 888

cctccttctc ctcccagtgc cacagagccg aagcccgagc tgccgccgca gccacagccg 60

agggcactat g 71

<210> 889
<211> 20
<212> DNA
<213> Homo sapiens

<400> 889
tgacctttta ccccaacatg 20

<210> 890
<211> 22
<212> DNA
<213> Homo sapiens

<400> 890
ccgccttccc cggcgcgcca tg 22

<210> 891
<211> 256
<212> DNA
<213> Homo sapiens

<400> 891

agttctttgt agtgcctccc tcagactcta acacactcag cctggccccc tcctcctatt 60

gcaacccccct cccccgctcc tcccggccag gccagctcag tcttcccagc ccccattcca 120

cgtggaccag ccagggcggg ggtagggaaa gaggacagga agagggggag ccagttctgg 180

gaggcggggg gaaggaggtt ggtggcgact ccctcgctcg ccctcactgc cggcggtccc 240

aactccaggc accatg 256

<210> 892
<211> 148
<212> DNA
<213> Homo sapiens

<400> 892

cctcctcccg ttccagctgc cgctgccgct tcctgggctg agtccgcccg cggtcccggc 60

ggcgccaggt gcgttcactc tgcccggctc cagccagcgt ccgccgccgc cgtagctgcc 120

ccaggctccc cgccccgctg ccgagatg 148

<210> 893
<211> 429
<212> DNA
<213> Homo sapiens

<400> 893

cctcctttt gcacacacac gaatacaaag agccatacga ccttcggatg ccggaaggtc 60

cttctgaatc ccttccctgt tccttaggtt gcactagtcg ggggttccat gctgggggc 120

agaaggaatg ctctctaccg tctgaaaccg ttcatcagga aggccttgat ttgtgatgtg 180

ctaggagagc acaggatctg caaatagaag gcacctgtct cccttctgca ggccgaggag 240

aggccgccat ggactgtgtg cttcttcatg gcttgtttac tcttctttca cagaccctac 300

agcttggggc ctgggctcct ctgaccatcc tcattgagaa aggaaagtga gtccagagaa 360

gttgatgctt cctacctgtt ggagcggccc agcagtgtaa gcgtggttgt tactgcccca 420

tccgccatg 429

<210> 894
<211> 174
<212> DNA
<213> Homo sapiens

<400> 894

cgccctcttc cgaatgtcct gcggccccag cctctcctca cgctcgcgca gtctccgccg 60

cagtctcagc tgcagctgca ggactgagcc gtgcacccgg aggagacccc cggaggaggc 120

gacaaacttc gcagtgccgc gacccaaccc cagccctggg tagcctgcag catg 174

<210> 895
<211> 151
<212> DNA
<213> Homo sapiens

<400> 895

cggcctctct tcctcagtgc gggcggagaa gcgaaagcgg atcgtcctcg gctgccgccg 60

ccttctccgg gactcgcgcg cccctccccg cgcgcccacc cacccagtcc ggctggactg 120

cggcagccgc gcggctcacc ccggcaggat g 151

<210> 896
<211> 83
<212> DNA
<213> Homo sapiens

<400> 896

ccgcctctgg cgaatggctc gtctgtagtg cacgccgcgg gcccagctgc gaccccggcc 60

ccgcccccgg gaccccggcc atg 83

<210> 897
<211> 28
<212> DNA
<213> Homo sapiens

<400> 897
cctccctttt cttttctgcc gggtaatg 28

<210> 898
<211> 288
<212> DNA
<213> Homo sapiens

<400> 898

cttcctcttt tctgtctggc ccgcggcccc gctgcctgcc ctgctccagg ctccacctgc 60

gccgccgatc gcccgggtat cgcgggggcc caggccagct gagtccgttt tccgcgccgg 120

ggtggcgccc ctccaaccgt cctaacgccg ggccggcagc aaggagtgtt cctgggacct 180

cagagaccag gctcagagcc tgacatccct gcgaggggac agcctcatcc gcccaggcca 240

gtgggggtct ctacaagtgc ccaggctcag gtgcagcccc cagcaatg 288

<210> 899
<211> 46
<212> DNA
<213> Homo sapiens

<400> 899
ggtcctcctg ggagtctcgg aggggaccgg ctgtgcagac gccatg 46

<210> 900
<211> 153
<212> DNA
<213> Homo sapiens

<400> 900

tcctctctat gcttggggaa ggaacttcct gtaagcaagg cttgaggctt gctctcgcct 60

tcgtcagcag ccctcctcaa tcttctccaa actcccgtcc ccaggccaca cagattctcc 120

tcaagagagc cctataagga cattggtaaa atg 153

<210> 901
<211> 381
<212> DNA
<213> Homo sapiens

<400> 901

attcccttcc gcccccttct ctaagctgca cagcctgaat agaagggctg gtccagcggc 60

ggcggaggct ggcgctgtcc tgagagggag ggctctgtgc ggaagagtca gggcgaccct 120

tgggcgctgg agtacgcttg ggactggggc tgcgagtgag caccagcgat tggttcggaa 180

gcggacattt ggttcagaac gagcatttaa ctctgccagg gatccgctgg gctctgacga 240

ctgcggtaga tccatggctt cctggacgtt cacccgtaga gtcatcctag cttaactctt 300

gttccctggt ctcagttcac aagcctcacc tgtatcttcc tggctcggaa gataattgaa 360

accaagtctg acttctcaat g 381

<210> 902
<211> 35
<212> DNA
<213> Homo sapiens

<400> 902
ctttctcttc ccgacgcgtg agttaggccg taatg 35

<210> 903
<211> 292
<212> DNA
<213> Homo sapiens

<400> 903

ggccctctgg caagatggct gctgcggagg cgttggagcg cggaaatctg gaaccgggat 60

ggcgacgtct acactgagtc ggaggcgaag gagcttactc cacgggaaca gcctctagat 120

aatctgagtt gttgaaaata cgaagcctgt tactcgtgaa cagtggctga caacagtgtt 180

gttgtgagcc tggctgtctg cttggaccca gaggtttcgt ctgccagggt ttttggttgt 240

atttaggatt tcagggaaaa gtgtccaagc tttcagtgtt ggagcaggta tg 292

<210> 904
<211> 123
<212> DNA
<213> Homo sapiens

<400> 904

cggcctcttc gcttttgtgg cggcgcccgc gctcgcaggc cactctctgc tgtcgcccgt 60

cccgcgcgct cctccgaccc gctccgctcc gctccgctcg gccccgcgcc gcccgtcaac 120

atg 123

<210> 905
<211> 108
<212> DNA
<213> Homo sapiens

<400> 905

tcctctcttg actttgagcg tccggcggtc gcagagccag gaggcggagg cgcgcgggcc 60

agcctgggcc ccagcccaca ccttcaccag ggcccaggag ccaccatg 108

<210> 906
<211> 29
<212> DNA
<213> Homo sapiens

<400> 906
ggttcctctc ggagcggaga cggcaaatg 29

<210> 907
<211> 156
<212> DNA
<213> Homo sapiens

<400> 907

ccccctctcg cgggaattat ttgaacgttc gagcggtaaa tactccctgg ggctgtcata 60

gaagactact cggagagcgc tgcctctggg ttggcgggct ggcaggctgt agccgagcgc 120

gggcaggact cgtcccggca gggttccaga gccatg 156

<210> 908
<211> 33
<212> DNA
<213> Homo sapiens

<400> 908
tatcccctcc cacggtctct agttcgcgtt atg 33

<210> 909
<211> 275
<212> DNA
<213> Homo sapiens

<400> 909

ctgcctctac agctgtgtgt aggcctgggg gcgagggtct tcggaacgta gcgctggctg 60

cggccccgcc cgcctaccca cccgcccgtc cggcagccgg ctcccgccgc ctccgcgctc 120

tgtctggggc cagccacctg gcgggccgct ccggtgcgcc tgcccgcgct tttcactgac 180

aggcgctgtt ccccacagcc agcgccgccc gccacgtccc agctctcggc caacggagct 240

gcgcggcggg tgacctttcc gagcccagcg cgatg 275

<210> 910
<211> 243
<212> DNA
<213> Homo sapiens

<400> 910

ctaccctttc atctctgcaa ctccttcctc cctgggcctc ccttctggtg tgtctgtggg 60

tctgtctagg tgggcttggg aaaggggaag gaaggggcgt ctctttaggc agctcagact 120

ggacaagcct tctttgaaaa tggtcctttg aacacacgcc tgctggtggt tggtcagaca 180

gatgcgccag cgggagcccc ggggccccaa ggggacagct atctctgcag gaccagtgcg 240

atg 243

<210> 911
<211> 402
<212> DNA
<213> Homo sapiens

<400> 911

cagccccttt tccggctgag agctcatcca cacttccaat cactttccgg agtgcttccc 60

ctccctccgg cccgtgctgg tcccgacggc gggcctgggt ctcgcgcgcg tattgctggg 120

taacgggcct tctctcgcgt cggcccggcc cctcctgcct cggctcgtcc ctccttccag 180

aacgtcccgg gctcctgccg agtcagaaga aatgggactc cctccgcgac gtgcccggag 240

cagctccctt cgctgtggaa gcggcggtgt cttcgaagaa accggaagcc cgtggtgacc 300

cctggcgacc cggtttgttt tcggtccgtt tccaaacact aaggaatcga aactcggcgg 360

ccttgggggc ggccctacgt agcctggctt ctggttgtca tg 402

<210> 912
<211> 107
<212> DNA
<213> Homo sapiens

<400> 912

acgccttttc cggcccgcag cgcggcctgg gctcccgcgt gtttaaaagt gcgcttgtgg 60

ctgctgctgt cttaactcct gtgcttggcg gacagacagg cgagatg 107

<210> 913
<211> 65
<212> DNA
<213> Homo sapiens

<400> 913

agtcctttct cgtcgctgct cggctcgcgg cccgtggggt cggccccgcc accgttgccg 60

ccatg 65

<210> 914
<211> 312

<212> DNA
<213> Homo sapiens

<400> 914

cggcctccgc cgcacgcgct ggcggactaa gagtggctgg cgaagcgagc ggccggcgcg 60
ggcccctggc gggcgggcgg tacagcccca agcctgagac ccggacctga gcatcgcagg 120
ttcgagtccc gccccgcctg gggcgaagcc gggggtggcg gcgacctcgc ggcgttgcac 180
cggctctgtg agcacctccc ctctgagcac ttcccttgtg acaggccact tcccttgtga 240
caggcccagg acgaggtggc caggcggccc ccatggcgtc cctggtctag gcggagaacc 300
gcctgggcga tg 312

<210> 915
<211> 351
<212> DNA
<213> Homo sapiens

<400> 915

ccctccctcc acctcggagt ctgcgcggcg cggccaggcc cggccgaccg cgtctcggtc 60
ttcgcgtctg ccagcctggc tggcagtccg tctgtccatc ccgccgcgcc ggggcagtct 120
aggcggagcg ggggctcagg cggcggcggc ctcgacgcga gtgagtgtcg tggttggggt 180
gctggaccca gagtgcctac cctcgcctgc ctgggcctca gtttccacat ctgcacaatg 240
ggggtgacca tccctgccct gctggctgcc aggagcggct gtgagtcttc aggcgtggat 300
gcagcctggg ggaagccata gggcgctttc acaggcctgg ccttcaccat g 351

<210> 916
<211> 27
<212> DNA
<213> Homo sapiens

<400> 916
gaaccttttt tcacctcgtc tgaaatg 27

<210> 917
<211> 117
<212> DNA
<213> Homo sapiens

<400> 917

cgccctccca cccgctcaga cctggttgcc agcccaacag gaagcggccc ctcccggctt 60
cggagccgcc gccactcatc tctgcccagc tgctgccctc cccaggaggc ctccatg 117

<210> 918
<211> 254

<212> DNA
<213> Homo sapiens

<400> 918

```
gctcctttaa ggcagcgaac gggccaagag aagcgtgttt cgccccctcc gacgccaccg        60
aggtagcggc ttcaccttta aggcggcgcg gggctgctg ggaaggccgg cgggatggag       120
gcggcgggac cggctcgcgg gtgcgggtcc gggtgaagcg ggaggcagcc agagtcggag       180
ccgggcccga gcaccaggcg caggcccggc gcccgcctgc ccgcaccctc gtcctcacag       240
acgccacagc catg                                                        254
```

<210> 919
<211> 178
<212> DNA
<213> Homo sapiens

<400> 919

```
acttcctttt tctgcccact ctggtaactt attgctctgc tgggctcttt cccttagggt        60
ctctggccct gttcttgccc cagcatgact tttatcggga cgccgttgtg gaagcctcac       120
gcaggagccc tgccccgtg gagaagatcc cactggtgac tccaacccta ccaccatg          178
```

<210> 920
<211> 648
<212> DNA
<213> Homo sapiens

<400> 920

```
gctcctccca ctgccgttgt gggtaacgcg gacgtggaag aacctcgtct gcggaggaaa        60
aggtagatgt taaatggtaa ctacgcgcga ggttctgagg agccctggga acaggaagga       120
gaaaagaata ccaaaagtga caacagtttg ccaatcgcag tctttaatct gataaagcgg       180
ttatctcgtc ttgagtccca ggtgccgagt caatccccat acacagccgc cgccattgcc       240
tcgagtcctt gtgtctgact gtctgttcct gctgctgtat gacacagcac ctcgaggcaa       300
ggaaataaga aaactgcctc tgatccaagc agagaaggtc tgcctgtaga tctgctgtag       360
ggcttgtcac cattggaagc aaggtcctac ttcagtggca gatctggtgg ccttggagtg       420
gctgaagacc accaccctcc acagggctgg gcccatgcac agccatcctt ccctaccttg       480
agtgagcttc ctctgcatgt tttctatatc actggcagag cctgtagttg gaaaggggac       540
agagtgacta ctggactttg tgtgaaaaca ccaaccggga caaaacttca gtcaaggctg       600
agacgggtgg gggtatataa cttgtcctta cgttaaactt ggaacatg                    648
```

<210> 921

<211> 24
<212> DNA
<213> Homo sapiens

<400> 921
aatcctttgc ggtggttcaa gatg 24

<210> 922
<211> 65
<212> DNA
<213> Homo sapiens

<400> 922

```
ggtcctcccg taggaaccgg cggactcggt tggcgttgtg gggcaggggg tggtggagca        60
agatg                                                                    65
```

<210> 923
<211> 907
<212> DNA
<213> Homo sapiens

<400> 923

```
gggcctcctc gcctttgtgc catccgggtc tctcgcgcga gcgatttagt ctgaggcgaa        60
gcttcggagc ggccggtact gttgaaagcg acaagtggag gcgccgctct agcggccggg       120
actctgaact atggcggcta gtgatacaga gcgagatgga ctagccccag aaaagacatc       180
accagataga gataagaaaa aagagcagtc agaagtatct gtttctccta gagcttcaaa       240
acatcattat tcaagatcac gatcaaggtc aagagaaaga aaacgaaagt cagataatga       300
aggaagaaaa cacaggagcc ggagcagaag caaagagcgt gcttatgcgc gaagagactg       360
aactgaagac gctgcagact cagatagcaa aataataagc ctacttcatg ataagggaag       420
aagacatgaa tccaaagata aatcctctaa gaaacataag tctgaggaac ataatgacaa       480
agaacattct tctgataaag gaagagagcg actaaattca tctgaaaatg gtgaggacag       540
gcacaaacgc aaagaaagaa agtcatcaag aggcagaagt cactcaagat ctaggtctcg       600
tgaaagacgc catcgtagta gaagcaggga gcggaagaag tctcgatcca ggagtaggga       660
gcggaagaaa tcgagatcca gaagcagaga gaggaagaaa tcgagatcca gaagcaggga       720
aagaaaacgg cggatcaggt ctcgttcccg ctcaagatca agacacaggc ataggactag       780
aagcaggagt aggacaagga gtaggagtcg agatagaaag aagagaattg aaaagccgag       840
aagatttagc agaagtttaa gccggactcc aagtccacct cccttcagag gcagaaacac       900
agcaatg                                                                 907
```

<210> 924

<211> 590
<212> DNA
<213> Homo sapiens

<400> 924

```
ccgccttccc acccccccgcc cttccactat ggccgcttct gtgtggtgtg gggagacgct 60
ggtcctcccc gtcctcccat agcgcttatt gcctcaccct cacccccctag gggccggatc 120
caaaggcgct gcactcccca agccttgggg catcagccag gaaggtttcc tacctcctaa 180
ttcaggggca ggactcctct tttcccccca cggggaaaag aggcagaaac ttaggggttt 240
ccctcctttc ttagggtcag acgctcttag ggtccacttc ttcaggggcg gaagcctctc 300
ctacccttcc catagggggca caggccttta ccccactgta cttcggagcc aacgcctttc 360
cctcagcact gccaccccag agtcaggacc cagaggactg tgccttcgcc cccaacgcag 420
gcgcggcctt ttggagagga gggaggagtg gagaggacag gggcccttgc tctcccctcc 480
ccaacttgtt cctcttgccc cccagtccct ggcaatccag agatcccgat atctaggact 540
gtccatccat ccactccctg accttttccc ggctcctggc tgcagccatg 590
```

<210> 925
<211> 396
<212> DNA
<213> Homo sapiens

<400> 925

```
tctcctttcc tcttctcaga cccgggagcg tccgggacgc ggagcccgga gctggggcga 60
cgaggcgatt gcgggggcct gggctagctg ctggctacca atattctact ttctgtctct 120
atgaatgtga ctaccctggt tacctcatat aatctccctg gaaaaggaga catgaatgtc 180
tgcaatgata cttcctgaca agaagttgat acaagaaaag gaaaggagat taacagctag 240
tgagcagaat ttcgaacagc aggatttcgt attttttgct tccaactgca cacttccgtt 300
gcccactttt aaatcagaga tacctacact caaaacccag acaaggcaaa aggatacttt 360
tcttgtatat tttttgagat cgaagaaacg acaatg 396
```

<210> 926
<211> 269
<212> DNA
<213> Homo sapiens

<400> 926

ccgcccettt cacctcctgg ctccctcccg ggcgatccgc gcccccttggg tctcccctcc 60

cttccctccg tccgcgtctc ctgcgccccc tccctgcgct cgtcccgccg ctcttcccgc 120

cgcccaactt ttcctccaac tcgcgctcgg gagctggcga ggcggcggcg gctcctcagg 180

tcagtttgaa aaggaggatc gagctcactg tggagtatcc atggagatgt ggagccttgt 240

caccaacctc taactgcaga actgggatg 269

<210> 927
<211> 29
<212> DNA
<213> Homo sapiens

<400> 927
ctccctcttc cgctgccgcc gtgggaatg 29

<210> 928
<211> 37
<212> DNA
<213> Homo sapiens

<400> 928
cctccttctt tcctgcctct gattccgggc tgtcatg 37

<210> 929
<211> 31
<212> DNA
<213> Homo sapiens

<400> 929
agtcctttgc gcggcacctg gcgacaaaat g 31

<210> 930
<211> 1100
<212> DNA
<213> Homo sapiens

<400> 930

cccctctcttc tccaccagcc aacgtccggg aaaaacgagt aagtacaggt tccttctgcc 60

aatccccgcc ggccacagct aactttcccg cccggcccct ttctgtcata attgaggtgt 120

ccacaaccag ccaatcagga acgcgagagt atcccgcgtt tgctttcgct cgccgaggcg 180

cgtatcagtc ggaattttgg ggagccaacc gcgccgtctg tccctggcaa gccagcggcg 240

gtttaaagga ggtggcggga agcctgtgtg tgcttcaaat cgtcaccctc atggtcgctc 300

cggtaagtgc tgcggggcag cattttctct gaggaggagc ggggacgggc gagactggca 360

taagcgtctt cgcgagggag caaggcggcc tgtgggtcgg cctcaccccg gcctccgacc 420

tgaagatccc agcatgcagc gcgggcgcgg ggcccgacgg aagccgggag ccggccggaa 480

gcagttcctg cgctctggct tctgggtcct gtcctgcgcg atcgcggggt cttagacagc 540

tcaactcgcc gagatgacct gggcacctct gcgttgaatc ggcaaatact gatcaagccg 600

catttattct gctctcagga actctaagtc tagcagagaa gatgaggcgg tagaagttca 660

tcaatggctt ggctggagga caagcaaatt gaggacattg gcaacggagt gatcaaaatg 720

atagatcatg aggcctaaaa tgaataagga aagaagagaa gtggcagagg ctgagaacag 780

aaagagaggg tggaggggct gtaaatcttg aagattaggg tataatatga gtatatgggt 840

aagaattgga agaattgtgt aggaggcagt agtcaaaaag tagaagcagt ttggaagagt 900

agttacaaat atcaagagcc aggtggctaa aaggtggagc tataggtcat tgaagctcaa 960

gaaactgagt ctctagggca ttggttaagt catctgtcta gacttcaaag ttgtctagga 1020

tgataattca gaagactgat ctgtgccaaa gtcacaggtt tttcacgact gaaaacaaca 1080

tagcaaaata agccaagatg 1100

<210> 931
<211> 76
<212> DNA
<213> Homo sapiens

<400> 931

cttcctttca cgctgtcgct gcccgtaggt ggttgtggcc actgtgcccg gagggaggcg 60

gcggtggcca gtaatg 76

<210> 932
<211> 73
<212> DNA
<213> Homo sapiens

<400> 932

cggcctctgc gtgcacgcgc ctgcgtgctc gcgctcgcgg ttctggcgct gccggaataa 60

tgctgacagc atg 73

<210> 933
<211> 168
<212> DNA
<213> Homo sapiens

<400> 933

ccgccctttc ctgtcgtgac ttaacgcacg caagcggctc cagggtacgt ccccgccacg 60

cgcgctcgca ggatcggtgc gtggtgacgt ttcgccggcg cgggcgccat cccggaagcg 120

cgagcaaggc cgccagatgt gcaggcagcg gaggaggaga aagagatg 168

<210> 934
<211> 70
<212> DNA
<213> Homo sapiens

<400> 934

acttctttct tgcccgccaa gcccgcagcc acccgggcgc ggcgggactc ctagacccgg 60

cgctgcgatg 70

<210> 935
<211> 289
<212> DNA
<213> Homo sapiens

<400> 935

cgttcctttt gtgacgccgg ctgtgagcgc ctgagagtct ttttgccttt cagagttaag 60

gcctcactgg cctgggaaaa taattgctgc cttttgcatc cgcgttggct ccgtccccag 120

gatcttcccg gttcagggac ctggcgattt ctgagtgttc cggaatccca ataaccctgt 180

ttaaagagga atggagattg ccactgtcca tttagattaa tgaggtgtcc tgaagtgatg 240

gtgacatcaa tgaaaggagg gttctgacac gttctcacct cgcgggatg 289

<210> 936
<211> 208
<212> DNA
<213> Homo sapiens

<400> 936

caaccctttt cttccgcacg gttggaggag gtcggctggt tatcgggagt tggagggctg 60
aggtcgggag ggtggtgtgt acagagctct aggacaccag gccagtcgcg ggttttgggc 120
cgaggcctgg gttacaagca gcaagtgcgc ggttggggcc actgcgaggc cgttttagaa 180
aactgtttaa aacaaagagc aattgatg 208

<210> 937
<211> 225
<212> DNA
<213> Homo sapiens

<400> 937

ccgcctcctc ctcctgccgc tgccgctgct ttggctgctg cgtcatacgc cccagagccg 60
ccgggacgga ggggctgggc ctggggaccc cccggcctcc gcctgcacgc ccccccacgc 120
ccggacgtgc cctctccgcg cgggggactc gcctaggtct cctacgtctg cccctgcccg 180
gctcccggcg gccccagctg tcaccggccc ccccaggatg caatg 225

<210> 938
<211> 85
<212> DNA
<213> Homo sapiens

<400> 938

ccccttccg cctgacgcgc ccccggcggc ggccgcgcag ccctggctcc tcgcgggctc 60
gggcggcggc tgcggcgggg ctatg 85

<210> 939
<211> 358
<212> DNA
<213> Homo sapiens

<400> 939

ccgcctcctt tccggagccc gtctgttccc cttcgggtcc aaagcttttg gctcctcctt 60
gttccgagcc cgaaggcccg ccccttcacg tactcggagc tcggatccca gtgtggacct 120
ggactcgaat cccgttgccg actcgcgctc tcggcttctg ctccggggct tcttccctgc 180
ccgcccgggg ccctgaccgt ggcttcttcc ccggcctgat ctgcgcagcc cggcgggcgc 240
ccagaaggag caggcggcgc gggggcgcgc tgggcggggg aggcgtggcc ggagctgcgg 300
cggcaagcgg gctgggactg ctcggccgcc tcctgcccgg cgagcagctc agaccatg 358

<210> 940
<211> 284
<212> DNA
<213> Homo sapiens

<400> 940

acgccccttt tttgctcagc cgtcagcccc gtctccgtct gaagagtgct tctgccctca 60
tttgcctctc cctgtgaccc cggccccctc agactccgct gcgtcgtctc tcggccccgt 120
ccagccgttc ctgactgctc ttcgccggag tccgcttccc aaccccccttt cgccagagcc 180
cgagagctcc gtcggctctg cgtcctggcg gattgtcagt ggcttcgccc cgaggagagc 240
tgactgccct gggctgctgc ctccggcaga gctgagccaa aatg 284

<210> 941
<211> 465
<212> DNA
<213> Homo sapiens

<400> 941

ctcccttccc cctctgtggg tcccgcgagg agactctcgg gctttgaggt gagacctgaa 60
gttccgctgg ccggtagtgt agcaggaaag ggcaggtcct cccgggtcgt gagccagtag 120
cctcctgggg tggcaaggtg tagagagggg ggcgttgaaa ggacacccgc tacccggcct 180
gctttctagg ggtctctttg gattgaggac atcagcagca gtggaaggga ttttactgga 240
gacctgtcac tgtcagagcc ttaaaatatc accgacgggg ccttaatgtc accgaggtag 300
agagaaaagg gcagtagccc tagagactat tgcgacacag tgtgcccctc ataagttttt 360
ccagggaggg gttctgtact gagttgacgc cccaggagct gagcaccagg ctttgcatcc 420
ttgggaactc agcaaacgtt tgttcagcca attgcaggta gcatg 465

<210> 942
<211> 82
<212> DNA
<213> Homo sapiens

<400> 942

tactcccttc cctcaggccc caggaagttg caagagtacc atttgtcgca cactcgggga 60
ccgcgggtgg ccggaggaga tg 82

<210> 943
<211> 83
<212> DNA
<213> Homo sapiens

<400> 943

gctcctcctt cgcggcggta ccgcctctgt ttctgcggcg attgaacagc cgagctttgc 60
ggccgggatc gcggaaagtg atg 83

<210> 944
<211> 612
<212> DNA
<213> Homo sapiens

<400> 944

atttcctctc tgctgagagc cagggaaggc gagctctgcg cacacgggcg tccctgcagc 60
agccactctg ctttccagga ccggccaact gccctggagg catccacaca ggggcccagg 120
cagcacagag gagctgtgaa cccgctccac accggccacc ctgcccggag cctggcactc 180
acagcaggcc ggtgctaagg agtgtggcgc gggctcgact cccactgctg ccggcctccc 240
gagtgactct gttttccact gctgcaggcg agaagaggca cgcgcggcac aggccggcct 300
ccgcttcccg ggaagacggc gcactcctgg ccctgggttc ttgctgctgc ccaccctctg 360
ctccctggga tgggccccga ggcgagcagc ttcagcacag gcctggccct gctccaggtg 420
caggaaggag gataaggccg ggccgagagg cggcacacct ggaccatccc atgggcctcc 480
gcccgcgccg ccccgaggat gagtggtgat gtcctctagc cacccctagc agcgtcggct 540
ctccctggac gtgcggccgc ggactgggac ttggctttct ccggataagc ggcggcaccg 600
gcgtcagcga tg 612

<210> 945
<211> 67
<212> DNA
<213> Homo sapiens

<400> 945

tcgtctttcc cctcccatct cctcagatcg gtggacgtgc tcgcctccac tcggggccag 60
gtctatg 67

<210> 946
<211> 44
<212> DNA
<213> Homo sapiens

<400> 946
cctccccttt tcggcccagt agcggcggct cagttgctgc catg 44

<210> 947
<211> 98
<212> DNA
<213> Homo sapiens

<400> 947

ccttctcttt cggagttgtt ccgtgctccc acgtgcttcc ccttctccac tggctgggat 60
cccccgggct cggggcgcag taataatttt tcaccatg 98

<210> 948
<211> 81
<212> DNA
<213> Homo sapiens

<400> 948

aaccctttgc cttcggactt ctccggggcc agcagccgcc cgaccagggg cccggggcca 60

cgggctcagc cgacgaccat g 81

<210> 949
<211> 81
<212> DNA
<213> Homo sapiens

<400> 949

cggcctttcc tccgcgcccc cgcgtcccca gccggccgct ccgagaggac ccggaggagg 60

caggtggctt tctagaagat g 81

<210> 950
<211> 26
<212> DNA
<213> Homo sapiens

<400> 950
ccgcccctta cggcgccgga gagatg 26

<210> 951
<211> 297
<212> DNA
<213> Homo sapiens

<400> 951

cctccctttt tctacttccg aggctgcaaa gtgcaacagc agactcttct gactcaggaa 60

ggccggtgct cctacccact tcctgttcct ccatctccag cggacactgc tctttcaagg 120

gcaggtctcc agcccagctc tctgaaaaca ttttgctgaa aatataagca aacatcggcc 180

ttgtcctcct tgtgttcata cactgtggaa gcttttctct gcctcctccg tgagagtgcg 240

tggccgggag accagaaacg tggtcctttc tcttgcctgt gagctggtgc agagatg 297

<210> 952
<211> 64
<212> DNA
<213> Homo sapiens

<400> 952

cttcctccgg ctggcagcac gactcgcgta gccgtgcgcc gattgcctct cggcctgggc 60

aatg 64

<210> 953
<211> 27
<212> DNA
<213> Homo sapiens

<400> 953
aattcttttt tccccaggct tgccatg 27

<210> 954
<211> 254
<212> DNA
<213> Homo sapiens

<400> 954

acttcccttt ttccggtccg ccggattatg aatgacggcc ggcgcgagta ttttccacat 60

aaggtggctg tcgtttttct cctggcgtct gtggaggcga gtggtctgcg ggcagcagct 120

cccagaggca gccttggaat tccagctcgg actgggcggg aaggcgcagg cggcccaggt 180

cgccgacacg ctcacgcacc ctccctgcct ggccgcgcct ctgcgaccag gtgacccaat 240

gaaagaagaa aatg 254

<210> 955
<211> 311
<212> DNA
<213> Homo sapiens

<400> 955

actccttttt ctttccaaac agggaaaagt gttccacgaa gcggtagcgc ctttccgcct 60

cgcgttttcc tccctgaccc tggtcccggc tcccgtccgg gcgccagctg gtggggcgag 120

cgccgggagc ccatctgccc ccaggggcac ggggcgcggg gccggctccc gcccggcaca 180

tggctgcagc cacctcgcgc gcacccccgag gcgccgcgcc cagctcgccc gaggtccgtc 240

ggaggcgccc ggccgccccg gagccaagca gcagctgagc ggggaagcgc ccgcgtccgg 300

ggatcgggat g 311

<210> 956
<211> 118
<212> DNA
<213> Homo sapiens

<400> 956

cctcctcttg cggtgggggg aaagcggcct cttactctag gcctttcggt ttgcgcgagc 60

gggcaggaaa gcgtgcgtgc ggctaagaga gtgggcgctc tcgcggccgc tgacgatg 118

<210> 957
<211> 299
<212> DNA
<213> Homo sapiens

<400> 957

cctccttctt ccactccccg cggcgcgagc ggctgactgc ccgtagagga aacgacattc 60

ggagctgcgc tcccgcccag gccggccctg acgcgggcct cgtcagccag taacagggag 120

cagaggtggg agttagcgag gcgaccacga aaacggtgaa ggtcggaacc gacagcctcc 180

tccgagaagg gcaggagctg ggaggaggcg gcagcggcgg cggcagaaac agcagcggcg 240

gcggcggcgg cagctgggag gaggtggtga cggtggcaac ggcagcgtcg gggacgatg 299

<210> 958
<211> 816
<212> DNA
<213> Homo sapiens

<400> 958

ctttctcttt tagccccgcc tgcttcccgg ctccagctgg ggccggagag gctgagtggt 60

tggtacgctg ctcgctggcc tcccagtctt cccagcaacc ggtgacactg cccgcgccag 120

actgaccact agccgacgcg ggcgagaggg acaggagcgt gacctcccca tcccgagggg 180

ccggacgctc gggcgcctcc ccgctccccc cactcggagg ccgcgcgcgc cgttagcccc 240

ttcctcgctc ccccgcccca gtcccgcagt ccgggaggcg ggggtcggca gccggctgag 300

tgggaaccgc gcggtgtctg aggaggcagt cggcgaccgg tttccacttc aagcgtgacc 360

cttttgcctg tgggatgagc tccagcatgg ggtgaggtac agaagagaga cttgaagagc 420

gtgccttggg actcaagcgc caaacctgta ccctagcgag tgtcctactc cgcatccgta 480

atggaaggaa atgcacatct tactccagag gcacaagagg aggacatccc atgcggctac 540

tcctgcccag cgtggtgggg cagcagaagc tccagagccc agacttgcag gctcacggtg 600

cagggtgaac ctggccacag ctcaccctgg aacagccaca atgtctgccc cttagagaag 660

aaccctgaaa tcagaccagt ttttgcggcc tccccctttc ctctctgtta cagtgccctt 720

tccaggcctt aagagaagta aaacttagct gcagcgccag gaggtggacc ccagagtgtg 780

agtggcacgc ttccctgtga acccgtcctc accatg 816

<210> 959
<211> 148
<212> DNA

<213> Homo sapiens

<400> 959

```
ccgcctccgt cccggctgcg gcccctgccg gttacataac tcgttgcggg ctccgcgcgg      60
tcccacttcc cggctccctt cgcctccagg atgcgctgag ccctacaaca cccccagcgg     120
ccgccggctc cccacgaggt tgtgaatg                                         148
```

<210> 960
<211> 237
<212> DNA
<213> Homo sapiens

<400> 960

```
tgccctctgt ccccgcggct gggtctcgtc tgctccggtt cctgggctcc taattcttgg      60
tccagcttct tccaggtcag tgtgcgggcc ttccacgctg ccagcggaac actggaatgg     120
cggaagggga acgggtctgc gcgtctgttg ttcccagcgc tctgcgaagc ctgaaaagga     180
ggagcaacct gtccagaatc cccgcaggac aggaaaagga ggggaaatct cgacatg        237
```

<210> 961
<211> 673
<212> DNA
<213> Homo sapiens

<400> 961

```
tcccctttgt ctccccactc cccgcccagg cctggcccgc ctgcctggcc actcttcctc      60
catcagcctg gctggcagca gccttggact ccgcccgtgg agccctgggc ctgttgaccc     120
accagcttag gagcacccac caagctctgg gtaaggaagc tcaccttctg gggctcttct     180
gggaaaatag aggtcaacgt ggaggtacca ggccaccatg ctcagtctca agctgcccca     240
acttcttcaa gtccaccagg tcccccgggt gttctgggaa gatggcatca tgtctggcta     300
ccgccgcccc accagctcgg ctttggactg tgtcctcagc tccttccaga tgaccaacga     360
gacggtcaac atctggactc acttcctgcc cacctggtga ggggaggctc tgccccaggc     420
cgcggccttg agctcagagg gggtacccag gcgggcaggg accgtccagg cccacgggct     480
gcagcggcag tcgcgggggt ccgcggcggc ctgagcacgc gcccgccgca ggtacttcct     540
gtggcggctc ctggcgctgg cgggcggccc cggcttccgt gcggagccgt accactggcc     600
gctgctggtc ttcctgctgc ccgcctgcct ctacccctc gcgtcgtgct gcgcgcacac     660
cttcagctcc atg                                                        673
```

<210> 962
<211> 189
<212> DNA

<213> Homo sapiens

<400> 962

catccttctt gctcaaccac tgggtgcaca ggatggaaac ttctattccc tctctggaag 60

acagcgcgtg gcttggcttc acagagttgt ggctggagac cgaagcagcc cctttctcag 120

gcttactgtc accagtctgt ctgtgttagg ggagagggga gtccgctctg tcctgaaggc 180

ccagagatg 189

<210> 963
<211> 137
<212> DNA
<213> Homo sapiens

<400> 963

ccttcttctt tcctgcctca ccttccaatt cgtttgccgc cgccgtcccg cagctgctgt 60

ttccggagtt gccccttccc catgttccgg ggcaggagtc cgcaaagcga agatccgccc 120

gccggttcct catcatg 137

<210> 964
<211> 158
<212> DNA
<213> Homo sapiens

<400> 964

ccgcctttgc tcggcggaga cagcaggcag agagatgagg aaactgagac ccagaaaggt 60

ggaagcactt gtctaaggtc acgcctccag gaagcagtgt gtccacgact ccagtccaag 120

tggtcaggct ccagagccca cagtcccagg ggtccatg 158

<210> 965
<211> 101
<212> DNA
<213> Homo sapiens

<400> 965

agccctcctc acaccccccac tgggctcctg cattaagccc ggggttcgca gccgcagccg 60

ggatcgggca cccaggggcg ggcgggcacg gtagggccat g 101

<210> 966
<211> 509
<212> DNA
<213> Homo sapiens

<400> 966

catcctctct gggaatttac cgatgcccag aacgcccttc tttcccccac acgaccctct 60
cctagtctaa ctcctgggcg tgctttaagc tcagctcagg cagcgtcacc ttctctggaa 120
agcccaaacc cagccacccc actacccgct acccgcggcc cacgctgatg aagacagcag 180
aacacggagg ccccgcgttc ccgccgcgag agcaggagag aaagattacc tcccgcgagc 240
tctagcgcgc ccggctttcc ggcgcactcc agggggcgtg gctcgggtcc acccgggctg 300
cgagccggca gcacaggcca ataggcaatt agcgcgcgcc aggctgcctt ccccgcgccg 360
gacccgggac gtctgaacgg aagttcgacc catcggcgac ccgacggcga gaccccgccc 420
catccccgac tgcctgaacc gcgccaggag acggaccgca agtccagcgt acccacagac 480
gactcaggcg ggagacgagc ggtgtcatg 509

<210> 967
<211> 192
<212> DNA
<213> Homo sapiens

<400> 967

cgttctttta ggggtggagc cggcaggaaa tttaaactga agccgcggcc gaaaacgcca 60
agagattgat gctgtagctg ccctgagata accaggactg tggaatcggg aagagctcat 120
ggagctcgcg aatgtaatac ggaggcctct gaggaaggag tacggaggcc gagaaggagc 180
cggcatttga tg 192

<210> 968
<211> 13
<212> DNA
<213> Homo sapiens

<400> 968
atttcctttt atg 13

<210> 969
<211> 439
<212> DNA
<213> Homo sapiens

<400> 969

ggttcctttc ctcactgcac gctcttgccc ctcctctttt ctctcctgcc cgtgttcttc 60

ccgccgcctg acctggcccg cccgcctttc cagtctggcc gggcgggggc ctgaagcacg 120

gcggctcggg ccgtgggacc gtgttcacac cctttccaga aattcttggc tggtaaccgc 180

gaaaccgact ggagcaggag ctgggagaac tggagaaaac tgctctaatc tcacttgact 240

ccagctagga gctgatgctg catcgtaata acatttgcag agcgctttca caggcgctgg 300

agtgacttgt ctgagattcc tccagaactg agccctttgt tggaaccata ccccagccca 360

tggtcccatg actaggtgga tagtactcct tgtacctcct gcaacccaga accctggctg 420

accactttga aggaggatg 439

<210> 970
<211> 363
<212> DNA
<213> Homo sapiens

<400> 970

cctcccottt ctgctgttac cgggagcgcg gtggccacgg aacgctgccc ggagccgcgc 60

gagggaggac ccgacgcgcg gcgtttaccc agcgcagcgt tccaccgctc gggtttggct 120

ggataaaata aaaaatgggg atattgacct cctgtcacta ctgcatggac tttgatggtt 180

tccaatcatt actttctcct ctgtgtcaat ctgcctcttc gagaaattca tactcctgaa 240

tagctctcca gacccccagc tggccatgtg gtgagttcag ggcccaaatc aagtagtacc 300

agcaatcagg gaactcctat ctgttttgaa tggattcaca ccagccacaa gcctggaaag 360

atg 363

<210> 971
<211> 113
<212> DNA
<213> Homo sapiens

<400> 971

ctccctcttc ccccgccccg ccctgggcca ggtgttcgaa tcccgactcc agaactggcg 60

gcgtcccagt cccgcgggcg tggagcgccg gaggacccgc cctcgggctc atg 113

<210> 972
<211> 155
<212> DNA
<213> Homo sapiens

<400> 972

gggcctttgt ccctcgctgt ggcctgagct ccaggtctcg tcttcagcgc tctgtgtcct 60

ctgctcctag aggtccaggc tctgtggccc tgtgacccgc aggtattggg agatctacag 120

ctaagacgcc aggaacccct ggaagcctag aaatg 155

<210> 973
<211> 236
<212> DNA
<213> Homo sapiens

<400> 973

gctcctttg caggctcgtg gcggtcggtc agcggggcgt tctcccacct gtagcgactc 60

aggttactga aaaggcggga aaacgctgcg atggcggcag ctgggggagg aggaagataa 120

gcgcgtgagg ctggggtcct ggcgcgtggt tggcagaggc agagacataa gacgtgcacg 180

actcgcccca cagggccctc agacccttc cttccaaagg agcctccaag ctcatg 236

<210> 974
<211> 55
<212> DNA
<213> Homo sapiens

<400> 974
gccccttcct cactaccctc caaatcccgc tgcagccatt gccgcagaca cgatg 55

<210> 975
<211> 358
<212> DNA
<213> Homo sapiens

<400> 975

cgcccctttac caacatggct gctgacgcca cgccttctgg gactcgtagt ccggtcctcg 60

cgcgctttct tacctaactg gggcgctctg ggtgttgtac gaaagcgcgt ctgcggccgc 120

aatgtctgct gagagttgta gttctgtgcc ctatcacggc cactcccatt tctggtgccg 180

tcacgggaca gagcagtcgg tgacaggaca gagcagtcgg tgacgggaca cagtggttgg 240

tgacgggaca gagcggtcgg tgacagcctc aagggcttca gcaccgcgcc catggcagag 300

ccagaccgac tcagattcag actctgaggg aggagccgct ggtggagaag cagacatg 358

<210> 976
<211> 175
<212> DNA
<213> Homo sapiens

<400> 976

ttgtctctct gtcagtggcg gctgctgcct gctctggagg caggctgggc ggtggcggcc 60

gagactggcg gggtggacg cccgggccgg gctgcgcccg cttcttgcag ctgtgaattc 120

ctttggacaa ttgatgatat ttatcattgt gcccagtttc tacaaataaa agatg 175

<210> 977
<211> 123
<212> DNA
<213> Homo sapiens

<400> 977

ctgccttcat ctctccatct ctgcgctgct gccggctgcg ccatccagca cccagactcc 60

agcaccggcc gaggaccccc actccggctg cagggaccct gtcccagcga gaccgcaggc 120

atg 123

<210> 978
<211> 271
<212> DNA
<213> Homo sapiens

<400> 978

ccgcccctte cccggagcct cacttccgtc acagtcctgt ttctctccct gttgtccctg 60

cctctttttc cttcccgccg tgccccgcgg ccgggccggg gcagccggga agcgggtggg 120

gtggtgtgtt acccagtagc tcctgggaca tcgctcgggt acgctccacg ccgtcgcagc 180

cactgctgtg gtcgccggtc ggccgagggg ccgcgatact ggttgcccgc ggtgtaagca 240

gaattcgacg tgtatcgctg ccgtcaagat g 271

<210> 979
<211> 132
<212> DNA
<213> Homo sapiens

<400> 979

cgttctttgg ccctgtgaca cgtagcaacg gggctggttc agggtctgaa acagagtttg 60

ggggttgttt gggattagtg aagctactgc ctttgccgcc agcgcagcct cagagtttga 120

ttatttgcaa tg 132

<210> 980
<211> 272
<212> DNA
<213> Homo sapiens

<400> 980

cctccctttc cagagccccc agttccttag aaaccaggcg gcgcgttccc ggtggcggcg 60

ccctggactc ccgggcccgc gcatccccgc cagccttcct taaggcggat gggtggcccc 120

cgagaccccg tcggacccat ggtttccagt gcagcgcgga gtgggcgatg ccagcgtgcc 180

aggagccatg tctgaccagg acgtttggaa gatcatatcc atgccagagg ctcttgtgag 240

gagatgagtt ggtaaagaga gaggctggga tg 272

<210> 981
<211> 275
<212> DNA
<213> Homo sapiens

<400> 981

ttccctttcg aattccaggg tatatctggg aggccggagg acgtgtctgg ttattacaca 60

gatgcacagc tggacgtggg atccacacag ctcagaacag ttggatcttg ctcagtctct 120

gtcagaggaa gatcccttgg acaagaggac cctgccttgg tgtgagagtg agggaagagg 180

aagctggaac gagggttaag gaaaaccttc cagtctggac agtgactgga gagctccaag 240

gaaagcccct cggtaaccca gccgctggca ccatg 275

<210> 982
<211> 155
<212> DNA
<213> Homo sapiens

<400> 982

ccgcctccct gcgccccgcc cctccggcta gctcgctggc tcccggctcc tcccgacgtc 60

tcctacctcc tcacggctct tcccggcgct ctcctggctc ccttctgccc cagctccgtc 120

tcggcggcgg cgggcagttg cagtggtgca gaatg 155

<210> 983
<211> 63
<212> DNA
<213> Homo sapiens

<400> 983

cttccttctt tgccaggcag acgcccgttg tagccgttgg ggaaccgttg agaatccgcc 60

atg 63

<210> 984
<211> 189
<212> DNA
<213> Homo sapiens

<400> 984

ctgtctctaa tctctgcaac agccgcgctt cccgggtccc gcggctcccg cgcgcgatct 60

gccgcggccg gctgctgggc aaaaatcaga gccgcctccg ccccattacc catcatggaa 120

accctccagg aaaaagtggc cccggacgcg cgagcctgag gattctgcac aaaagaggtg 180

cccaaaatg 189

<210> 985
<211> 68
<212> DNA
<213> Homo sapiens

<400> 985

tgctcctttc tgcccgtgga cgccgccgaa gaagcatcgt taaagtctct cttcaccctg 60

ccgtcatg 68

<210> 986
<211> 155
<212> DNA
<213> Homo sapiens

<400> 986

gggtcctttg tctctcggtg cagccggagc tccaggtctc ctcttcacta ctctgtgtcc 60

tgtgctccta caggcccagc ctctgtggcc ctgtgacctg caggtattgg gagatccaca 120

gctaagacac caggacccct ggaagcctag aaatg 155

<210> 987
<211> 83
<212> DNA
<213> Homo sapiens

<400> 987

cggcctctag ccacaccgag tccgccgcgg cgtccagggt cggcagcaac cgcagccgag 60

cccgagcggg tggcggcgcc atg 83

<210> 988
<211> 126
<212> DNA
<213> Homo sapiens

<400> 988

cattcttctg cgacggcgcg gacctggagc ttccgcgcgg tggcttcact ctcctgtaaa 60

acgctagagc ggcgagttgt tacctgcgtc ctctgacctg agagcgaagg ggaaagcggc 120

gagatg 126

<210> 989
<211> 26
<212> DNA
<213> Homo sapiens

<400> 989
gtcccctttc ctcgcaggac ctcatg 26

<210> 990
<211> 159
<212> DNA
<213> Homo sapiens

<400> 990

ctttcttcct cttggcttat attagggata ggggatgtgg tttgttacaa aggatgagta 60

ttttgatagc ttctcattcc ttgaactatt ctgcaggttt ataacaaagc tcagaaaata 120

ctaaaggtta aaggagaatt gagagctgcc aaggaaatg 159

<210> 991
<211> 89
<212> DNA
<213> Homo sapiens

<400> 991

cttcctttct cggaaacgcg gcgcggccgg ctgccggaaa acagggcaga cctgtatggt 60

tcgtttattc ctggggttgt catatcatg 89

<210> 992
<211> 243
<212> DNA
<213> Homo sapiens

<400> 992

tattcttttt tagtgcagcg ggagagagcg ggagtgtgcg ccgcgcgaga gtgggaggcg 60

aagggggcag gccagggaga ggcgcaggag cctttgcagc cacgcgcgcg ccttccctgt 120

cttgtgtgct tcgcgaggta gagcgggcgc gcggcagcgg cggggattac tttgctgcta 180

gtttcggttc gcggcagcgg cgggtgtagt ctcggcggca gcggcggaga cactagcact 240

atg 243

<210> 993
<211> 219

<212> DNA
<213> Homo sapiens

<400> 993

```
atttctccat ctggctctcc tctacctcca ggcaggctca cccgagatcc ccgccccgaa     60
cccccctgc acactcggcc cagcgctgtt gcccccggag cggacgtttc tgcagctatt     120
ctgagcacac cttgacgtcg gctgagggag cgggacaggg tcagcggcga aggaggcagg     180
ccccgcgcgg ggatctcgga agccctgcgg tgcatcatg                            219
```

<210> 994
<211> 951
<212> DNA
<213> Homo sapiens

<400> 994

```
ccttcctttc cccagtgttg agcgcggtct cgcctccgct tcctcctcac tccgcctgcc     60
ggctgggaaa ctagggcacc agtacgatag ttccggcacc ggaaaagagg gctgatgact     120
gggcccgggg gccgccgcaa cgacccttgg ggccggcaaa gagccagaga gggtgctcac     180
acttccaagc accccacacc aaggacaggc tggacggcaa ggcggagacg cggggcttgg     240
gccctcagac cggggacagc aggaggttgg gccaagggcc aggacttccc gtcacaattt     300
catttgttga tcccggcacc gccaggtaag gggggccctg agtgaggcta ggtatctggt     360
acggataaag ttaggtatag agtagagcgg ctgcccgctc agggttatcc ctaaagacag     420
ttggaggaga gttgcttggg gcctcgggga tgcactgggc gggatcaggg cttacaccta     480
ggactggcaa aagagcggga cccggcagag gcggggcttg ccgaagggac gagcctctat     540
tcaggaaatg cacgagcttt ggggcggggc tcaaagaaag gggcggggct tccggggccc     600
gcgtcctggt gagctgcgcg tctgcgcgag gattgggcga gagggtgggg ccactcaacg     660
ctgaggcggc gaatggccgg agcagactta aatcaagagg ctggggacct ctaagatcaa     720
agtttggggc ggggcctaag gagggggcgg ggcctccaga ttcgagacct ggaagggctg     780
gggcggcgct tggggcggcc ctgccgccgc ctcccgttct cccctccgca gcggcggcgg     840
tggcggagaa ggaactcgac acgcaccgac cgccctcccg ccccagccga agcggaagct     900
gtagcccgct ctgggccggg gccatgggcg ccccgcgccg cccgggtcat g              951
```

<210> 995
<211> 42
<212> DNA
<213> Homo sapiens

<400> 995

ggctcttttt gacagccccc agtgcgaaag gctgccagca tg 42

<210> 996
<211> 60
<212> DNA
<213> Homo sapiens

<400> 996
ggttctctct gacgtgggag ccgccgtcgc tgccgccacc cggaggctct tgtcaggatg 60

<210> 997
<211> 177
<212> DNA
<213> Homo sapiens

<400> 997

aggtcttttct ccacaaaaga aataacagcg tgcattacgt attcagatac tgctttgctt 60

catcctctct aaaatttaac accgaggagt ttaagaaatg aagataagga actcgaatta 120

tttttaaact ttggatcaat gtaaaggcaa tctaatattt ggaaaatact tgcaatg 177

<210> 998
<211> 267
<212> DNA
<213> Homo sapiens

<400> 998

gcctctcctt gggccccttc tctccccctt tcccctccct gctggttcct ggcatcgcca 60

gatgctgcgc agcagtctcc gattccccat caccaattcg gctggcgtct ccgagaccgc 120

ggactcccgt agggtccccg tggccccgag ttgtagtcgg gacaccccgg ccgcgggtga 180

tcgtcgggtc tccacgcgcc cgggtcgctg acgcggatcc ggcctcggcg ccttctcagg 240

gcgccctgca aggccgcagg caggatg 267

<210> 999
<211> 86
<212> DNA
<213> Homo sapiens

<400> 999

gctcctcctg ctgtgggacc gctgaccgcg cggctgctcc gctctccccg ctccaagcgc 60

cgatctgggc acccgccacc agcatg 86

<210> 1000
<211> 882
<212> DNA
<213> Homo sapiens

<400> 1000

gggtctttta ggagagcact gctgcagccg gcagtggaga gcctgggcag ggagacaggg 60
agaaaactcc ggcagcaggg tggtctctag ggctgacctc ggagcctggg gacaggggag 120
cctatgccgc actgaaggcg ggacgctgta agcgaggagc agctgggcct gggcggactc 180
ctcggccaat cagcctcggt cagcagcacc ctcaggcgca gggcactgtt tgggcattgc 240
ctagagatcc gacaccccgc ccagatcagc gcagggaggc gaaagcgaca gccgggcgcg 300
ggaggagacc agggcagctg tcccctccgc gagggtggcc ctcgaggcaa tgcgggtggg 360
ggctggtgag gaggcggaag ggccgaggct gagtgggagg ggccggggcg ccagggctgg 420
agcgcgcggc tcgggggtgg aggctgcaga gccagcgagc gagcgagggg cgggggcgcc 480
cgggccggcg cgcaggaggg gcgggggcgg cggggagggg ggctcgggct gcgtgtgccg 540
gagccggcgg gggcggcggt gcgtgcgcat gacgcggggg gagggcctgg gccgcgcgct 600
cccggtcccg ttgttgttgc cgctggaggc tgctccgagg cagcgggatc acggcgctgg 660
gaagcgctcg gcagcggcgg ccacagcgtg cgcggcggcg cctcctggcc tcggcctccg 720
gcccccggcc cccggctcca tgcgctagcc ccgcgccgcc agcccagtag tcccggcccc 780
gccagccccg cgctcccgct cgccgctgcc gccgccgccg ccgccgccgc ctccgccgcg 840
ccgccccggg cccgcctcgg gccccacggc tccgaagcca tg 882

<210> 1001
<211> 52
<212> DNA
<213> Homo sapiens

<400> 1001
ctgcctctct cagtccgggt ttggagactc ctgcgtcctc cgacttttca tg 52

<210> 1002
<211> 225
<212> DNA
<213> Homo sapiens

<400> 1002

cattctctgc gaccggcagc cgccaatggg aagggagtga gtgccacgaa caggccaata 60
aggagggagc agtgcggggt ttaaatctga ggctaggctg gctcttctcg gcgtgctgcg 120
gcggaacggc tgttggtttc tgctgggtgt aggtccttgg ctggtcgggc ctccggtgtt 180
ctgcttctcc ccgctgagct gctgcctggt gaagaggaag ccatg 225

<210> 1003
<211> 23
<212> DNA
<213> Homo sapiens

<400> 1003

gtttctcttt ccgggacaac atg 23

<210> 1004
<211> 220
<212> DNA
<213> Homo sapiens

<400> 1004

```
cagcctctag cctgggattc cctgcgcagc caacaacaga aaagaaaacc agctcccaca      60
cagaggctcc cggctgcgca gaccttgccc agcacaccag attgccagct ccgagacccg     120
ggactcctcc tgtcctgggc cgaatgctct tttagcgcgg tagagtgcac tttctccaac     180
tggaaaagcg gggacccagc gagaacccga gcgaacgatg                           220
```

<210> 1005
<211> 547
<212> DNA
<213> Homo sapiens

<400> 1005

```
ggctctttcg gcttccttcc tcgctgggcc ggctaaaccc ggccgcagca gcaccggggt      60
gataagtgtc cagggcagga ggccagcgat gttgccttgc taaccgggta tctaagagaa     120
acagggtctt tttattctta ggctcgacag tctgacggcc ctttttctga acgggaccct     180
gcaggtcttc cgcctgctgt tgcattaaat ttgggggtgg aagaggcttc tgcgttgttc     240
cttacccgca acgatgacca tggctttgcc ttctttaaaa ttgaggcctc caactctgac     300
gctgactgga gaattgaaac ccgaacacac attgggctct tttggcactt gactagagct     360
aaaacctcgg gattcagcgg gcaagcgttg ctcagcaacg gcgcgtaggc tgtgtgcggt     420
tggctggagc cagaccccac cccggcctcg gcccatgctc tagaggggac gttgccccaa     480
tcctgaagga cttcggcact cgagacctgt ggatgccgcg ttgctgtggc ctgcggggt      540
gatcatg                                                               547
```

<210> 1006
<211> 80
<212> DNA
<213> Homo sapiens

<400> 1006

```
ccgtccctct acgcgttttg gttcccggtt ggtgcttcct gttcgcagct gcggcacttc      60
aaggttactg actttttatg                                                  80
```

<210> 1007
<211> 266

<212> DNA
<213> Homo sapiens

<400> 1007

gggcctttct ggacttggac tccttgggag tcgtttctcg gccatttgac ccgtgggact 60

tgtgggtttt gtgctgcttt ttctttcttt cttccccttt tccaacttca gcaatacacc 120

cagatgttag tcgagtcacg tcccgccgcc ctctgccctt gaaatgctgg caagtacgca 180

gccccgcgat cgtcacgtga cgccggggtt cagcgtatcc ttgctgggca accgtcttag 240

agaccagcac tgctggctgc accatg 266

<210> 1008
<211> 101
<212> DNA
<213> Homo sapiens

<400> 1008

cgctccctcg gtgcggcggg ctgcgtgcgc gagtgggagg tggcaggcct gcgactccgg 60

ccttgtccgc gcccgctctc ggcgcgacgt ctccagccat g 101

<210> 1009
<211> 225
<212> DNA
<213> Homo sapiens

<400> 1009

ctccctttct ctcagcatct tcttggtagc ctgcctgtag gtgaagaagc accagcagca 60

tccatggcct gtcttttggc ttaacactta tctcctttgg ctttgacagc ggacggaata 120

gacctcagca gcggcgtggt gaggacttag ctgggacctg gaatcgtatc ctcctgtgtt 180

ttttcagact ccttggaaat taaggaatgc aattctgcca ccatg 225

<210> 1010
<211> 18
<212> DNA
<213> Homo sapiens

<400> 1010
cagccctttt tgcaaatg 18

<210> 1011
<211> 300
<212> DNA
<213> Homo sapiens

<400> 1011

gagcctcttg cttgctgtga ctggtggagc tgccgcgctg tccgcgttat ctcctcccgg 60

tgagaacgaa ccgcagtgtc caccggcgag gagccagccc tgtcccggtc agagaaagac 120

gacgaggata cctgggagcg ggcggcggcc gggctgggcc gcgccggtgc gggctggcga 180

ctctgctcct ccgcttgctg ctgtctctgg gaactgggtg ccagcgctga ggggcttcca 240

gcggacaggg accccccttcc ccggctcccc tgcccaccct gccggggagg gcggaagatg 300

<210> 1012
<211> 33
<212> DNA
<213> Homo sapiens

<400> 1012
tgccctctct catgaccccg ctccgggatt atg 33

<210> 1013
<211> 27
<212> DNA
<213> Homo sapiens

<400> 1013
ccgccccttt ggagctactt cctcatg 27

<210> 1014
<211> 270
<212> DNA
<213> Homo sapiens

<400> 1014

tcgcctcttc gcattgtgag ctctcgcggt aagaggctga ggagccggcc tgcaacctgc 60

cggggcggct ccgctacgcg cagccgcctc agtggcttcc tccacagcca cctccggagg 120

gatctggctg aggaggaagt ggaggtgtca ctggccccgg cctttgcccc aatcttgtgt 180

gggcactgaa gggggactac aggttcgaga gttatgggtg ctacatgtgt gctttcagag 240

cagtagtgtg aggaagcttg gagtgggatg 270

<210> 1015
<211> 146
<212> DNA
<213> Homo sapiens

<400> 1015

cggtctttgt ggcttgcagc tcggggtggg tggctcattt cctggccgct cctgggcttc 60

gcggaaagaa gagattactc acactccttc gcaagcacag aaccagttgt actgagcttt 120

ttgctaagct gtttcagcca agaatg 146

<210> 1016
<211> 35

<212> DNA
<213> Homo sapiens

<400> 1016
gctcccttcc cggcggcctt tgcgggaaca agatg 35

<210> 1017
<211> 806
<212> DNA
<213> Homo sapiens

<400> 1017

```
cttccttctc catattgtat actggaattg aagccaagga ggtaccattt tgctcgaggg      60
catggcctaa gccggtcagc taaggccatg ttaatacggg gctgtcccat ctctctgcgg     120
ggcgcgacag ctggaagagc cgaacggata agagaagagg aggtgagagg agctgtacac     180
cacaagaggc actgagggac tcaggataac gggatgaagc cgtcagtgcc cccagaaacg     240
aagcggcccc ggacgaattt ctgagtcacc gtcgcgagaa agcgggctga gccgccattt     300
tgaagcctgg caaaccgaag caagaaatgc tgccgtgttg gatctttgcc agccttcgtg     360
ccgaatggga gcaggttgga gggagggaga gccaatatac actatgggct gattaagccc     420
ggttggctgc catgttgtta acgagcaccg atttcctcta cttttgtcga agaagtttat     480
tgtgggtcag ggacgtcagg tcgcttgcct tcgtttactg tggtcatgat tgagcatatg     540
aggacggcca ttattgttgg gggcaaatgg aaatgctcta ggcggggcca tttttcttag     600
gggcaagctg tcgtcaccct tgtcaactgg ttcggatgaa gcccctgtgg ccgccatctt     660
gatctcgggc ggccccgata agggaggcgg agtgtgcgga gaggaggcgg ggcaactgcg     720
cggacgtgac gcaaggcgcc gccatgtctt ttgagggcgg tgacggcgcc gggccggcca     780
tgctggctac gggcacggcg cggatg                                          806
```

<210> 1018
<211> 18
<212> DNA
<213> Homo sapiens

<400> 1018
ctgccctttc ccaagatg 18

<210> 1019
<211> 206
<212> DNA
<213> Homo sapiens

<400> 1019

ggccctccct ttcatcagtc ttcccgcgtc cgccgattcc tcctccttgg tcgccgcgtc 60

cttggctggc gttagagaca gggtttcaac gtgttagcca ggatggtctc agtctccaga 120

ccctgtgatc cgcccgcctc ggcctcccaa agtgttggga ttacaggtgt gagccaccgt 180

gcctggccga ggctccttct tttatg 206

<210> 1020
<211> 73
<212> DNA
<213> Homo sapiens

<400> 1020

cgccctcccc gccgtggatt ggcccgcggc gggacccgtc agccgcggtt gtgtctggga 60

aggagagaaa atg 73

<210> 1021
<211> 935
<212> DNA
<213> Homo sapiens

<400> 1021

atttctctcc tccctggggg tctcagtgca tctccttctc ctctctgcct gcctcctccc 60

tcaccgaagg gttagcggac acccatcctt ttctgcttgg ggaccccacc accacccgca 120

acactgccgc tgtctcttct tcaccgtatc cttctctacc caccctcttc tctcttctct 180

tctccctgcc cctttaaatc tgcctggccc agcctccccc gtgatgctgg gatggagcaa 240

acattgattt gtgctgggat ggaatcggaa ttttgattta tttttcctct cccaaccata 300

agaagaaaaa aataataaaa acaccccctc ttgagagccc cctcccccctt tgcatccagc 360

tcccagctct tcttccctat ctccatccaa ggcagatttt ttcccctaca ctattctcat 420

cttcccccac ccttgccact acctcgcccc cccacccagc ctgctcctcc agctggggag 480

agaggggact ctccggactc ccccaccttt cctctctggg ttggagcagt ctctccggaa 540

ggggaggggg cttggcttgt ccgggcgagg tgggagtgga ggtatcctgc catggatgct 600

gtgccgggga ggcagcctga gccccagccc acatgagacg ccgaagaacc ggggcagagg 660

ggtcctgaca gcagccaggg aaacgggtgc cctacgattc tgcccagccc cctctcagga 720

cccccaaact gccatccaca ctcgacactt cggggttcta gccactcagg atgagggtcc 780

ggccctgcct gccctcgctg gggccccccc gcccggcccc ggtctaactg cccccgcccc 840

gaggcctcgc ccggctccaa ggcccccagc aggctctcca gtcccaggat gcgctgagcc 900

gccgggggc tgaggccgcg ccaactacat gcatg 935

<210> 1022
<211> 154
<212> DNA
<213> Homo sapiens

<400> 1022

ttttctttct cctcctccaa ccttggcgga ggccacgact caggcgccac agctgggggc 60

tagaggccgc ggaccatggt gcggggcagc caccgctgaa gtcagcaaaa ccgagcctgg 120

cctgaggcag gctgcgcggg aggccaaagc catg 154

<210> 1023
<211> 194
<212> DNA
<213> Homo sapiens

<400> 1023

cgctccttct ttctggccgg atgtgtgctg agacccagag tcacccaggg gtctccgtca 60

cgtgccagga gtaggcagaa gtgggctgtg acagatcagg aaacagagct cagtgcagcc 120

cactaaattg ctcagggccc tacagctaac aagcggcaga ggcaggatct gcactcagga 180

gctgcttgga gatg 194

<210> 1024
<211> 73
<212> DNA
<213> Homo sapiens

<400> 1024

ggctctctct gcggcttggc ccgttagagg cggcttgtgt ccacgggacg cgggcggatc 60

ttctccggcc atg 73

<210> 1025
<211> 120
<212> DNA
<213> Homo sapiens

<400> 1025

agttctcttc acggagccgc gcggctgcgg gggcgcaaat agggtcagtg ggccgcttgg 60

cggtgtcgtt gcggtaccag gtccgcgtga ggggttcggg ggttctgggc aggcacaatg 120

<210> 1026
<211> 287
<212> DNA
<213> Homo sapiens

<400> 1026

gttcctttcc tgggcatcag cttgcctgct ctcagcctaa gctctctcgc caaccgtggt 60

ggctccttgc gttcctacat cctctcatct gagaatcaga gagcataatc ttcttacggg 120

cccgtgattt attaacgtgg cttaatctga aggttctcag tcaaattctt tgtgatctac 180

tgattgtggg ggcatggcaa ggtttgctta aaggagcttg gctggtttgg gcccttgtag 240

ctgacagaag gtggccaggg agaaggcagc acactgctcg gagaatg 287

<210> 1027
<211> 31
<212> DNA
<213> Homo sapiens

<400> 1027
tctccttcta cggatatctg tggaccttat g 31

<210> 1028
<211> 161
<212> DNA
<213> Homo sapiens

<400> 1028

gcttctttcc gcccggctcc ttcagaggcc cggcgacctc cagggctggg aagtcaaccg 60

agctcccttc caggtcaatc caaactggag ctcaactttc agaagagaaa gacgccccag 120

caagcctctt tcggggagtc ctctagctcc tcacctccat g 161

<210> 1029
<211> 197
<212> DNA
<213> Homo sapiens

<400> 1029

cctcctcctt tcctccctct actctgacac agcacttagc acctgaatct tcgtttctct 60

cccagggacc ctccattttc catatccagg aaaatgtgat gcgccacagg tatcagcgtc 120

tggatcgcca cttcacgttt tagccacaag tgactcagtg gaagatccag agtcaacaga 180

ggctcgtcag gaagatg 197

<210> 1030
<211> 112
<212> DNA
<213> Homo sapiens

<400> 1030

caccctttca ctacttctcc cccggactcc ttggtagtct gttagtggga gatccttgtt 60

gccgtccctt cgcctccttc accgccgcag accccttcaa gttctagtca tg 112

<210> 1031
<211> 147
<212> DNA
<213> Homo sapiens

<400> 1031

tttccttcct ctcttccctt cgcagaggtg agtgccgggc tcggcgctct gctcctggag 60

ctcccgcggg actgcctggg gacagggact gctgtggcgc tcggccctcc actgcggacc 120

tctcctgagt gggtgcgccg agtcatg 147

<210> 1032
<211> 128
<212> DNA
<213> Homo sapiens

<400> 1032

gcccctttct ttccttcgct tcctctttta gagaatgtcc ggattgctat tggactttgg 60

agcgtatggc tccaaatcaa ctcattggct aaaacttgac ggaaaatggt ggttaggtgg 120

ccagaatg 128

<210> 1033
<211> 314
<212> DNA
<213> Homo sapiens

<400> 1033

cggcctcttc ccagcgttcc tcctccggcc ccaggtcacc gccagcacgc gcctgcttcc 60

cgtctgcgcg agtccacgca gctccccaga tcaagaagct gaggccccag gttacacact 120

aaagtaaatg gcagaggcag aaataacacc tatgtcctcc tgaccccaag gcatgttctt 180

aaagttctgg aaacctcctg gaggcttcct tgctgctcct ctgggactgc caccctgggc 240

agggtgttct gtggcccctc atcatcgtgg ttttgaacca caggcccttc accagcacag 300

cagcagcagg catg 314

<210> 1034
<211> 127
<212> DNA
<213> Homo sapiens

<400> 1034

catcctcccg ccagcctgcc cgcctgctcg ccggcgcccg gagcccgctc tggccgcttg 60

ctttttgctg agaaagcttc ctgccctgga agatggcacc cttccccatc cagacacctt 120

gggaatg 127

<210> 1035
<211> 197
<212> DNA
<213> Homo sapiens

<400> 1035

cttcctcctt ttcacggcgt cttgcattac tattgtgcgg ctgcaggagg tgtcgagcgg 60

cgttattttt ttttgcggtt tgcctttttt tttctttttt ttttttttgg aaccgcggtt 120

gtttaaaagc ctgagggaac ctggagaggg gctcccactc cctaccctct ttcctccgag 180

tttgtgactc cgagatg 197

<210> 1036
<211> 180
<212> DNA
<213> Homo sapiens

<400> 1036

ggctctttgt cgaagctaga ggaccggcag gcggcagcag caactacggc ggcggcggca 60

gaacccagca gcgatgtgga ggtggagacc cacaggagcc ccggacttca cctgagctac 120

ctcagtggtc accaagagtg gcaagataaa gaaaaccctg agttggcgg gaccaggatg 180

<210> 1037
<211> 119
<212> DNA
<213> Homo sapiens

<400> 1037

ccgtctttca gtttcacttt tgttttcctg ctcccagcag ggttaggctt gctgaggggc 60

aggcacagga gtcctggctg agctcatggc ctgaggctgc ctagcggcca cggggaatg 119

<210> 1038
<211> 20
<212> DNA
<213> Homo sapiens

<400> 1038
ccgcccttcc ttgtaagatg 20

<210> 1039
<211> 509
<212> DNA
<213> Homo sapiens

<400> 1039

ctgcccttcc gcagcgatgg catcccgggt gagtatcggc cccggccgag cccccaaggc 60

gggcgggcag cgcggcaggg ccgggacttg agcggaggac cgagtaggcg caggtgtccg 120

ggcccaacag gaccaggaag gtgtcggggt tggaatgagt gggtacccgg gccggggacg 180

gtgcgagagg gtgccttgct tgggagcgga acgagaaggt acttgggtca gggaggtgat 240

gcccgggcct ggaacgtggc ggggattgga gcaggcgcgc aggtacccga tccgaggcgg 300

ggagagcacc cgggatggaa ggagcaggcg tgcgggccgt gagcggcgcc agagggtacc 360

tggctctgtg gaggggccct ctggtatgtg tgtccctgtc cttctggggc gtggatggtg 420

cctgggaccc agctggcaac cagttgaaga cgttctcctt ggaagctctt ggccctgagg 480

actttgcctg gggcattggc cctgccatg 509

<210> 1040
<211> 463
<212> DNA
<213> Homo sapiens

<400> 1040

gcgtctcttc cggcgtctag gggggtgtcc tgccggcgcg cgggccctgc ggccattttg 60

ggcttcgctt ccaccgcacc agccggccta cccagtcctt ccggtatcgc gttgctcagg 120

ggcttttcaa ccctctgtca gtcggaaaac catcgccgag gccgtggggg gactcctatc 180

catggtgttg aagcgtcgag ccgactaggg aacctccttc cccgccagga tggaagtcgc 240

atcagtcgcc gcctattgcg cgggctgttc ttccctgtgt tctgccgccc gctgccgcat 300

tcgctgccct ctgtggcttt tctgctggct cgaagatcgg cctggagcag cgacgccacc 360

gctgggcaag gccgagactc tgtaggcttc ctccgaatcc cgtcgacctc cagccgctga 420

gcgccgcggc cctacctgag agactgtcaa gaaaaaggag atg 463

<210> 1041
<211> 51
<212> DNA
<213> Homo sapiens

<400> 1041
ccctctcttc gcggagcggc gccgcgtagc ttccatccgc cagctgccat g 51

<210> 1042
<211> 159
<212> DNA
<213> Homo sapiens

<400> 1042

agccctttac actacggtgt ttccggcttc aagatggtcg cctaagctgt ttagtgaaac 60

ttcttccacc tttctccatt cctctaggtg ctttttctga acctggatgt gaggcattaa 120

aggatccgac ggaaatagaa ttgaaggcat tctaaaatg 159

<210> 1043
<211> 187
<212> DNA
<213> Homo sapiens

<400> 1043

cctcctccgt gtggggcagc tgctggctgg gctgcctgtt gagtcagcct tcttccctca 60

cggctcttct cccggtccct gaaactcggc tgccagggga gctggagcca cctgcgaagg 120

tgtcctccca tactggaccc ctacaggaag ctccgtgtgc ccagctgggg cacagcccca 180

gctgatg 187

<210> 1044
<211> 367
<212> DNA
<213> Homo sapiens

<400> 1044

gctccttttc ctttttgatc cattcaaaaa ttactcattg caaattcccg gactgctagg 60

cgaggagagg gaagggggcg gaggagacag ggctactgca ggcgcagagc tgggggcagc 120

cgggggcccg agtggctgag gctggtcccg cagcggccgc ttgccggcgt tctggctcct 180

gtggcctcac caggaagcgt cagagtcccg acactgggga agctcggagc gccgcctccg 240

ctgccgccgc ctcctgcctg gctctgggtc cccgagcccc ctcccctggc ccagcccgac 300

tccctcctcc ttcccgaacc atccggctcg ggctccttcc ctggcgatgg ctggccgctg 360

agccatg 367

<210> 1045
<211> 114
<212> DNA
<213> Homo sapiens

<400> 1045

ccgtctctgg gcggctgctg ccgctgccgc tgctgctgct gcgggggtcg ggcggcggcc 60

aggggatttg ggcaggcacc gtggatcccc gagaagggga cgagttgaca gatg 114

<210> 1046
<211> 372
<212> DNA
<213> Homo sapiens

<400> 1046

gagcctctgc cagccctgag ctgggaagaa gcagctacct cggaggcagg gcgcgcaggc 60
gggcggcgat gagaggggc gcagccgcag ccccgcgctg gggagcccac cgctaaccct 120
gcaccccacc cacccctgca caaaagagct ggcgggcgct ggccacgtcg ccctgggtga 180
ccttcctcgg atgcagaatc cgcccctgcg agcatcctct tcctcctagg ctctgaaggc 240
ccggggagcg tgagcgatgc ccagctgcac ccgggcaggg ctcgcctttg tttgccagta 300
aggaggagag gctgtctcag ctgcagaggg gtcatccctg cttcaagcca gtgcctcttc 360
ccagctccca tg 372

<210> 1047
<211> 189
<212> DNA
<213> Homo sapiens

<400> 1047

attcctctct cacccccacg cagaggagag aacttgcttc tggacccggg tgggtgccgg 60
ctcggctctc cttgtcttcc agagcggtgg cccggaagca cagtcctccc agacgccagc 120
gccagaagct cggatcgcgg ctgcaccggg agagcgccga tctgggtgcg aggcaggtgc 180
ggggccatg 189

<210> 1048
<211> 257
<212> DNA
<213> Homo sapiens

<400> 1048

gttcctctag gaaaattcct ttgtgcagat caggcccgtg gattggtgag tgaatcctaa 60
ccacgtcttc cctggcctgt cttcactctt ctccccagaa tcaccacttc tgcactggtg 120
tctgaaggtg tattgagtga ttttgtggag ggcagaagta ggaagtcttt gggacaaaac 180
tgtatttacc ttgggatctg tgaacaagag gaacctcagc agccaggaca ggcaggagca 240
gtggaatagc tactatg 257

<210> 1049
<211> 20
<212> DNA
<213> Homo sapiens

<400> 1049
cgccctctcg cgcggcgatg 20

<210> 1050

<211> 73
<212> DNA
<213> Homo sapiens

<400> 1050

ctccctccct gcagcccgca acgggaatgg agtaaaggga gacccgtcga cctggccacg 60

gggatcagcg atg 73

<210> 1051
<211> 260
<212> DNA
<213> Homo sapiens

<400> 1051

cattcttccg gtttcagaag ttaaggctgg tgtcctggcc ccagtccacc tctgggagcg 60

cctgcgccgc tccgcggaga gtccgtggat ctcacagtga aaaatgtttg ctgacccttg 120

acattgacaa actgctgaca gctcagatga tccatgattg gaaggatgtg gtcatcacca 180

agatgtcttt ctttctccgg ttcccagttt tccagacctg aagtgttttc caatcaaagc 240

gaagagacga tctgtggatg 260

<210> 1052
<211> 370
<212> DNA
<213> Homo sapiens

<400> 1052

ggctctcttg cgcaagcgcg ctgtccgctt cttctgggcg gacgctctgg aggcaaaaca 60

tttccctgct gggggcggcg accaccgtga gcgtcccgga aggggcggca aagacgcctc 120

cgtcgcgcac gaggtggcct cgttggcttt accttggttc gcggtcgtcc ttggttatcg 180

tgagcgtccg cgagtctctg ggaggccaag cctaggggcg ccacagcgcc tgcgcgcgta 240

cggcggccgg aaggggctag aggcggctcc ctgggtgaca accgcgcgcc ccacctttcc 300

ccacgtggcc gcgaagaccg gctcaggagc atctatcggc tgcacgccaa catcaacaca 360

ggcgaagatg 370

<210> 1053
<211> 36
<212> DNA
<213> Homo sapiens

<400> 1053
gctcctcccc cggcggcgag ccagggagaa aggatg 36

<210> 1054
<211> 63

<212> DNA
<213> Homo sapiens

<400> 1054

```
tgccctcttg tttttagtct cgcttttcgg ttgccgttgt ctttttttcct tgactcggaa      60
atg                                                                      63
```

<210> 1055
<211> 126
<212> DNA
<213> Homo sapiens

<400> 1055

```
ggctctccta ccttctcggg cagcccagtc tttgccatcc ttgcccagcc ggtgtggtgc      60
ttgtgtgtca cagccttgta gccgggagtc gctgccgagt gggcgctcag ttttcgggtc     120
gtcatg                                                                126
```

<210> 1056
<211> 563
<212> DNA
<213> Homo sapiens

<400> 1056

```
ccgcctctgc cccgcggcga gggtgtctat ggagaggcgg cggccgcggc tgctgaggcg      60
gaggctgagg cagtggcgat ggcgcccttt cctgaagaag tggacgtctt caccgcccca     120
cactggcgga tgaagcagct ggtggggctc tactgcgaca agctttctaa aaccaatttt     180
tccaacaaca acgatttccg tgctcttctg cagtctttgt atgctacttt caaggagttc     240
aaaatgcatg agcagattga aaatgaatac attattggtt tgcttcaaca acgcagccag     300
accatttata atgtacattc tgacaataaa ctctccgaga tgcttagcct ctttgaaaag     360
ggactgaaga atgttaagcc tactactgtt gactggaagc cttaccaata acataaaaca     420
atcgaataac aattatttca tgtattatat gtaaaatata tatactggat tcttacagta     480
agaatgaata tgaacagtta aattatgcaa aacaactgaa agagagattg gaggctttta     540
caagagattt tcttcctcac atg                                             563
```

<210> 1057
<211> 74
<212> DNA
<213> Homo sapiens

<400> 1057

ctgcctccac tcggcctcag ttcctcatca ctgttcctgt gctcacagtc atcaattata 60

gaccccacaa catg 74

<210> 1058
<211> 55
<212> DNA
<213> Homo sapiens

<400> 1058
tcgtctctct ctctgcgcct gggtcgggtg ggtgacgccg agagccagag agatg 55

<210> 1059
<211> 271
<212> DNA
<213> Homo sapiens

<400> 1059

ccgcccctcc cgatttcctc cgggctacag gcgacagagc tgagccaagc gtttactggg 60

cagctgttac ggtaagtgag gaggggctgg ggtgcccagc gttttggatc tcccactctg 120

gcccggcccc ggaataccac atagaggcct tgggacctga ttcatcccgt ccagacagcc 180

ctagagacct gagcgactga ggcctgggat ctggacgccg gaatttcctg cgtggttctg 240

gacgccctgc cctgggctca gattccaaat g 271

<210> 1060
<211> 238
<212> DNA
<213> Homo sapiens

<400> 1060

cctcctcttg tagtggcgcc ggcttgcatc ccaggtcgtg gcggttttgg tgcctgaagc 60

agggagcgcg gagtcgttcc cgagagaggc ggccaggcta tgctcgccgg tttccggcgt 120

tccgctccgg ccagccagag tctctgtctc aacctgtgtc cgtgctccag cagtctcctc 180

agcccggccc cgcggcgcgg ttggcggcgg cgccccaggc gcgccccctc ctccgatg 238

<210> 1061
<211> 26
<212> DNA
<213> Homo sapiens

<400> 1061
cgctctttcc cggaggctgg cagatg 26

<210> 1062
<211> 187
<212> DNA
<213> Homo sapiens

<400> 1062

ctttcccttt cggacatgcg cgctcggagc aaggcgccct cgcactcagc ttaccgcgca 60
tgtacgttgc caggggtaac gcaggtagcc aaagtggctt gtggagtggc gaccgttagt 120
gaggcggttg ctgagacaga cgctgaggcg ggtaggagga gcccgagccg taagggaagc 180
cgtgatg 187

<210> 1063
<211> 27
<212> DNA
<213> Homo sapiens

<400> 1063
agttcttccg gggcggaggt caccatg 27

<210> 1064
<211> 582
<212> DNA
<213> Homo sapiens

<400> 1064

ccgccccttc gcttataatg cagagcatgt gaagggagac cggctcggtc tctctctctc 60
ccagtggact agaaggagca gagagttatg ctgtttctcc cattctttac agctcaccgg 120
atgtaaaaga actctggcta gagaccctcc aaggacagag gcacagccac acgggagtga 180
aatccacccc tggacagtca gccgcaatac tgatgaagct gagaagcagc cacaatgctt 240
caaaaacact aaacgccaat aatatggaga cactaatcga atgtcaatca gagggtgata 300
tcaaggaaca tcccctgttg gcatcatgtg agagtgaaga cagtatttgc cagctcattg 360
gacattctca ctattctatg ccttaaaggc ccttcaacgg aagggatatt caggagagca 420
gccaacgaga aagcccgtaa ggagctgaag gaggagctca actctgggga tgcggtggat 480
ctggagaggc tccccgtgca cctcctcgct gtggtcttta aggacttcct cagaagtatc 540
ccccggaagc tactttcaag cgacctcttt gaggagtgga tg 582

<210> 1065
<211> 42
<212> DNA
<213> Homo sapiens

<400> 1065
ggtcctcctt ggctgactca ccgccctggc cgccgcacca tg 42

<210> 1066
<211> 122
<212> DNA
<213> Homo sapiens

<400> 1066

cccccctcctt cataaagtcc tggcctcggg acagcctgca cagctgccta gcctgtggag 60

acgggacagc cctgtcccac tcactctttc ccctgccgct cctgccggca gctccaacca 120

tg 122

<210> 1067
<211> 80
<212> DNA
<213> Homo sapiens

<400> 1067

cgctctcccc ggatcgtgcg gggcctgagc ctctccgccg gcgcaggctc tgctcgcgcc 60

agctcgctcc cgcagccatg 80

<210> 1068
<211> 164
<212> DNA
<213> Homo sapiens

<400> 1068

gcccctctcc ccgggcagcc gcggcggcag cagcagcagc agcagctgga gctgtggggc 60

tgtcaccgcc gcccgccccg ctcactcgcg gatcccgacc gcccatctcc gcctcgcttc 120

cagcccagga tgagacttct gtgagcagcg aggattttga tatg 164

<210> 1069
<211> 244
<212> DNA
<213> Homo sapiens

<400> 1069

caccccttctt tgtgctcggg ttaggaggag ctaggctgcc atcgggccgg tgcagatacg 60

gggttgctct tttgctcata agaggggctt cgctggcagt ctgaacggca agcttgagtc 120

aggacccctta attaagatcc tcaattggct ggagggcaga tctcgcgagt agggcaacgc 180

ggtaaaaata ttgcttcggt gggtgacgcg gtacagctgc ccaagggcgt tcgtaacggg 240

aatg 244

<210> 1070
<211> 336
<212> DNA
<213> Homo sapiens

<400> 1070

tttcctcttg agccatcatg cacatctgac tgcagcccca gcgagccctt ccttccttgt 60

ctgactgctc ttcttctcga tttcttcttg ttctgccttc tcggtttgca gccctgaccc 120

ccgctgtgtg tctggccctt ggtgactgtc cgtgtttctg ttcctgtcat tgtaactgtg 180

acttttctct ctgtctgccc cccttccta ctggttcatg cttctccccc attcccaccc 240

tctctgcccg gcctcccgct cccgcccttt ctcctcatgc acccggcctc gtctctgtag 300

tctctgcact tgtctcccat taaggtccca tccatg 336

<210> 1071
<211> 303
<212> DNA
<213> Homo sapiens

<400> 1071

cagtcttcct cccgcccctt ctttggtccc tacggacctg gggggcggtg gcggtcaatg 60

ccgggtcaag gtccgcgggc ctcgcagatc gtagcccggg cgcacgcgat cagatgatcc 120

tgttgtggac ggctaagttg taggcgggat ggctgagaaa gcggcgctag gacccccggg 180

cagaggctcg gggaagggag tcagggggga aatgccttac aaggtcgcct tgcggtcacc 240

atcattgccc gccgcccaaa atagcccccg gcgccagctg gcctgcccta tggccgagag 300

atg 303

<210> 1072
<211> 81
<212> DNA
<213> Homo sapiens

<400> 1072

ctccctcttt ccctccctcc tcctccgtcc gcccgtccgt ccgcgcgtct gtccgttcgg 60

cccggtccgg cccgaagcat g 81

<210> 1073
<211> 819
<212> DNA
<213> Homo sapiens

<400> 1073

cagcctccct tatttagtcc gcgatggctt ccctcgcgcc ccaccgtcct cttccggaag 60
gcggctccct ccctgcgcag cccggagccc ctgagatcag cctcgagcag gcgcccgagc 120
gagactatcc ctaaacggga acggcggtgg ccgactcgcg agtgaggaaa agaaggaaag 180
ggcagactgg tcgcgaagag aagatccagg cctcagagga ggagaaaggc cggagccagc 240
cgaggtttgc cgagggcggt gttccggacc cgcgcggtgc ggggaggaag gccgagggtg 300
ggagaggagg ggcccggcgg aaactgccga ggtttcccga aggcggcagc gtccgagttg 360
cccggatgta gttggtggag cggcagcggc ggcaccagcg gcggcggcgg cggcgggagg 420
aggaggagga gaagaaggac caggcggcgg cagcagcggc ggcggcgggg ggaggagggg 480
aggaggcggc ggagcaggag gaggagaagg cggaggaggc agtcgctctc cgcggggctg 540
agccggacgc gtcgtcttgc ccccctcccc ccggttcgcg gtgccgccgt gtagttggcg 600
ccgctgcccc ggctgagagt gagcgtggtg tcgacggagg gagatggccc gggagcgccg 660
gcgccagtaa ctgggagctg atgagagtcg ccgagggcgc gccgggccca ggtgccgggg 720
ctgcccgccg cccgccgccg ccgccgcctg cgcgcccgcc cgcctttcgc ggccgctctc 780
ccccctcccc gacacacact cacaggccgg gcattgatg 819

<210> 1074
<211> 19
<212> DNA
<213> Homo sapiens

<400> 1074
cgctccttca gtctcgatg 19

<210> 1075
<211> 91
<212> DNA
<213> Homo sapiens

<400> 1075

cagccttctg cactcacagc cgaagggaaa gcagcaggtt ggggcttctt gtggccaact 60
tcagagcctg tcaccaggaa aggtaagcat g 91

<210> 1076
<211> 352
<212> DNA
<213> Homo sapiens

<400> 1076

cgccctctag cccctcccg cgggagtcgc ggcgctgcgg gtaggagccg ggttgcggga 60

gaccccaggt tcggttggga ttcccagcca gaacggagct taagccgggc aggcgagcga 120

atgacggagt agcgagctgc acggcggcgt gctgcgctgt tgaggacgct gtcccgcgcg 180

ctcccaggcc gccccgaggc ttggggtctt cgaaggataa tcggcgcccg gggccgaaca 240

gcgggggcac acggggcgct gccgaagtgc aaggccacgg ccagagctcg agcccgacgc 300

gctgtctgga gtcgtaggac cctgacgtgg ctgaagcggc cccgggagca tg 352

<210> 1077
<211> 129
<212> DNA
<213> Homo sapiens

<400> 1077

agccctcccc gcggccggct cggctccttg gcgctgcctg gggtcctttc cgcccggtcc 60

ccgcttgcca gcccccgctg ctctgtgccc tgtccggcca ggcctggagc cgacaccacc 120

gccatcatg 129

<210> 1078
<211> 342
<212> DNA
<213> Homo sapiens

<400> 1078

ctccctcttt tctcagtacc ctcctcttta ctctccgagt taactgagag ccgacctgac 60

atctccaaca ttttcaccct cttcccccac ccccatcacc gagaatggag tcagggtttc 120

cggagagacc gaactctgct ctcagcacct ttcccagccg ctgttgctaa actgacctcg 180

gaggacgaga ggggaaggag gtgcgacgcc ccttacatca gtacataact accacaccaa 240

ccacctccac ttcaaagccg gattttgcat cctgggggcg ggacagacct cgtcccgggc 300

tgaattctct ctccactctt cgagattggc acacccagaa tg 342

<210> 1079
<211> 154
<212> DNA
<213> Homo sapiens

<400> 1079

ctgtctttcc ttccctccct gctcggcggc tccaccacag ttgcaacctg cagaggcccg 60

gagaacacaa ccctcccgag aagcccaggt ccagagccaa acccgtcact gaccccccag 120

cccaggcgcc cagccactcc ccaccgctac catg 154

<210> 1080

<211> 309
<212> DNA
<213> Homo sapiens

<400> 1080

```
aggtctcttg actctttccg cctttgttta caaccctgcc atgatctccc tcttgcaaaa      60
gcgagggcta cagaacaggc attcaggagt cctgtgctcc agtcacagcc ttttctgttc     120
ttcagctagg agacaccaaa ccctcaggaa gatttactat agctaagaga aaactgcagc     180
agaaagggcg cggctaccta cttcttaaat tccgtttgtg gaccctcaga ctcttagtcc     240
cctactccca gatacagcgg ccctaccgtg gctcctggca aggtggcatc cacttttgta     300
gtaagcatg                                                             309
```

<210> 1081
<211> 50
<212> DNA
<213> Homo sapiens

<400> 1081
ctgtccttct ggcggagcgt gcttcccgct gcggggacgt tcgagcaatg 50

<210> 1082
<211> 314
<212> DNA
<213> Homo sapiens

<400> 1082

```
cgttctctgg tagcgaccat tttggttaat gttgggtgtg tttctgcggt ttgtgaggtg      60
agaggcgctg gagctatggg tccgaaccgc ggtgtctgaa cccagaaggt gaagagtcct     120
tcttgctgca cagaggcaga tcttaggccc cgtaacggcg cccgccgctc ccggcagtgc     180
tttccccgcg tactcgggat ggcggcggcc gcgctgaggc tcccggctca ggcatcatct     240
ggctgcaaag aagagaacac actgtgtttg agggaggagg aaggaggatc agagtttaaa     300
ctcctgccat aatg                                                       314
```

<210> 1083
<211> 97
<212> DNA
<213> Homo sapiens

<400> 1083

```
gtttcttccg cttcctgtac cacccggctc aagtagcgga cacggaacag ggaactatca      60
gcccgtcggc ctccgggccc tgcattctct agccatg                               97
```

<210> 1084

<211> 88
<212> DNA
<213> Homo sapiens

<400> 1084

agccctttc gactgtgagc tgcggcagct gagcagaggc ggcggcgcgg gacctgcagt 60

cgccagggat tccctccagg tgacgatg 88

<210> 1085
<211> 465
<212> DNA
<213> Homo sapiens

<400> 1085

ccccctttc gtttccggcg ctcccgcctt ctctccgcag agctcttctc tgagcctgtt 60

ggggggaggg agggggcgt ggaggaactg gggttcgcgg gagcacgagc tgcagcacca 120

cttccgggtg agtgcaaggg gagggcagca aggagggggg gccacccact acctcgcgcc 180

cccgccctgc gggtgtctcg cgcgcgttcc gtgcgtgtga gtgtgtgggt ctgtctcgct 240

ccagaagtgc gtgcccgcgc gctgcgcctt gcgctttttc ccctccctcg ccccttcctg 300

gtcctcccac cctcctcggc tccctccttt cccagcaaac gccgcccctc ccgcgccctg 360

gctcaggctc tggcgccgcc gcagccgtcg ccgcccgaaa gttcaggagc cctggaaagg 420

agaaggaata agacggcagg aggaagagag agagagggta gaatg 465

<210> 1086
<211> 140
<212> DNA
<213> Homo sapiens

<400> 1086

ctctcctttc tcctccggca gccagcgcgc ctgtgtcctc tctaggaagg ggtaggggag 60

gggcgtctgg agaggacccc ccgcgaatgc ccacgtgacg tgcagtcccc ctggggctgt 120

tccggcctgc ggggaacatg 140

<210> 1087
<211> 90
<212> DNA
<213> Homo sapiens

<400> 1087

gctcctcctc ccgctcctcc tcggcctccc cttcgggcgc tctcgcgcta actgtgctcc 60

tccggggccc tccgcctgct cccagccatg 90

<210> 1088
<211> 161
<212> DNA
<213> Homo sapiens

<400> 1088

gcgcctttat ctgcatccgg gtccgtggga ttcgcgctcc actggtcagc tggggtcgct 60

ctcgggtggt tgggtgttgc ttgttcccgc tgttccagcg tcgaagaacc attgggtctg 120

ccggtttgaa cttgttctgg aagctgtgcg tcaccgtaat g 161

<210> 1089
<211> 34
<212> DNA
<213> Homo sapiens

<400> 1089
ccgcctcctc cgcttgcggc cggtctgcac catg 34

<210> 1090
<211> 106
<212> DNA
<213> Homo sapiens

<400> 1090

tttcctttcc gctcccaggg gcgttgggaa cggttgtagg acgtggctct ttattcgtga 60

gttttccatt tacctccgct gaacctagag cttcagacgc cctatg 106

<210> 1091
<211> 100
<212> DNA
<213> Homo sapiens

<400> 1091

ggaccttttc ggccaccgct cgcttcaata tggctgcccc cagggagaga cgaggctacc 60

atgaaggagc cgagcgcaga ccctgagtcc gtcacccatg 100

<210> 1092
<211> 132
<212> DNA
<213> Homo sapiens

<400> 1092

ctccctcctc cttacccccc cctccctgtc cggtccgggt tcgcttgcct cgtcagcgtc 60

cgcgtttttc ccggcccccc ccaacccccc cggacaggac cccctttgagc ttgtccctca 120

gctgccacca tg 132

<210> 1093
<211> 215
<212> DNA
<213> Homo sapiens

<400> 1093

cgccctctag tggcagccgg ttttgaggcc ggcctccggc tttgaagttc ctcaccgcgt 60

ctccttccct ctccccaaag cctggatcac cgcccagcgt caggcgaggg gcgacgtctc 120

gaggtaaaac ggaggaggtg cgggacgcgg agactgcgcg ggcccggtag ccctggagag 180

gccgaggctc taggccgcga ggggcgggtg caatg 215

<210> 1094
<211> 80
<212> DNA
<213> Homo sapiens

<400> 1094

agttctctgc ggagggccgg ttgatacagt tccggtggga gaacgcggct gcgaggtttt 60

cggctttggc tcctgatatg 80

<210> 1095
<211> 250
<212> DNA
<213> Homo sapiens

<400> 1095

caccccttccc cccgccaccg tgggttccag acttgggata agtaaacagc gggtggagcg 60

aggcctacgg acccaggcca ggtgggagtc tgcactcttc aaggggcctg ggctgctgct 120

cacgggtatt aaagaactcc gcgttgttca tggctgaggc gatgcattag gaagatcctg 180

gacctagaga acaagtcccc cgaacgctga gttggaggcg ggacttcggg tgcgcgttgg 240

cgggagcatg 250

<210> 1096
<211> 213
<212> DNA
<213> Homo sapiens

<400> 1096

aagcctcttt tcaggctgag tcctaaacct gaagaaagtt tagagcctgg ggctctaaac 60

tacctgagtc tttccaaacg acaagccaag aagacctgtt gaaagtttcc tcttaagttt 120

cgtggagaga gactcaggta tagaaatatc cttactgcca cctgacctga agcagaagaa 180

atcacagaca gcttccagac caggcccaac atg 213

<210> 1097
<211> 96
<212> DNA
<213> Homo sapiens

<400> 1097

acgccccttc tcctgtaaac ttgggtcgcc tctagcttag cgagcgctgg agtttgaaga 60

gcgggcagtg gctgcacacg ccaaactttc cctatg 96

<210> 1098
<211> 261
<212> DNA
<213> Homo sapiens

<400> 1098

cttccttccc ttccccgact ttgcagattt ctcttccccc aggcctccct cctccacctc 60

tccgccccct ccgggcttgg ctctcccagg aggctacgac tggagccact ggtcccgcag 120

gatccccgcg tcctcggtcg ccgcgtccac gtccctctcg cgtccccgcc cggcgccacg 180

ccgcctcctc tgggttcggc ctccgcgcgg tgcagcgcag tctcaggccg cgggacaagc 240

ccgacttaaa tctctgcaat g 261

<210> 1099
<211> 484
<212> DNA
<213> Homo sapiens

<400> 1099

cgtccttctc ccgcccccgc ccctgcctgc cagctccacc gggccgtagg tgcggacgac 60

ctcaaaattc ctcggcccgc gaaggccgcc agctgcgggg aggggagggg aggcgcggtc 120

ccgcagcgcc cccaggctca tgtcccaggt atgtccagac ccccgaggca ccgcttgcag 180

ggcagtgaca gcccgtgagg ctcggcctcg acccctggca cccttggtcc cagctacgcc 240

ggctcctggc cttcccccaa gtccgagaga gaggtgggat tctccccgac gcagttggaa 300

accgggaatc ccctttaggg tcccgttcgt gctgcactac tgactccacc atctgcaaag 360

ggattcttgt ccagaatccc cgaaggcttt aggacagcgc ttattttgtt gaatgaagag 420

tctctaattt tcggaaagac cacaggctaa aagtcaagtt gtgccttttt agccaagaag 480

catg 484

<210> 1100
<211> 82
<212> DNA
<213> Homo sapiens

<400> 1100

cggcctttcg gcagccgaac ggccgcggca gttcaggaca aagaggtgtg ggcaggccac 60

tgggccagct ggtaacatca tg 82

<210> 1101
<211> 728
<212> DNA
<213> Homo sapiens

<400> 1101

tgctcctttc ggttgccata gcaaccccat tccccaagcc ctctgtccgt ctcctctggt 60

aggttccaca atggtacagg cagcatcacg ctgcacaatg gtttccaggc agtgaaagag 120

ggtgattcag caagccactc ttcttctatt ttctttaacc tccccttcac tttttatttt 180

tatgggggtg ggtggtgctt gctatatgct tacctttttc ttttcttttt tcatttttac 240

aaatttcctt ttttgtcctc acccctcaat tcctaggggc ttgagtgagt ttaagattgg 300

gttttcttgg aaatcacctg tccatcgtta attttaaaca atctccatat ctccaaagaa 360

tctcttccat gttagtctgg aatgtggtta atgaaaaaca agtagggagg atttctgggg 420

caaacactgc cggatcagga tcgtagttct caggcacgga atggctagtg tgagaaacac 480

caacagcagg cccatctcag atcttcacta tggcaactta tgcaagaaac tgttgaatta 540

gacccgtttc ctatagatga gaaaccatac aagctgtggt atttatgagc ctccatttct 600

tatactactg cagtgaacca acattggatg tgaaaattgc cttttgtcag gtgtgtgttc 660

cttacaggta aaacaaggga ttcgataaac aagtggatgt gtcatatatt gccaaacatt 720

acaacatg 728

<210> 1102
<211> 143
<212> DNA
<213> Homo sapiens

<400> 1102

cgtcctcccc gccgccgccg gtcccggtgc gcgcccatcc ctgcccgcag ccccgcgcgc 60

cggccgagtc gctgagccgc ggctgccgga cgggacggga ccggctaggc tgggcgcgcc 120

ccccgggccc cgccgtgggc atg 143

<210> 1103
<211> 170
<212> DNA
<213> Homo sapiens

<400> 1103

acgccttttc cgctagtcgc cccgctctat cccatagtct cgctgccctg agcctcccgt 60

gccggccggc cggccggggg aacaggcggg cgctcggggg gcgctcgggg ggcgggggga 120

gttccggttc cggttctttg tgcggctgca tcggcggctc cgggaagatg 170

<210> 1104
<211> 35
<212> DNA
<213> Homo sapiens

<400> 1104
cgtcctcttg tgtagcctga ggcggcggta gcatg 35

<210> 1105
<211> 90
<212> DNA
<213> Homo sapiens

<400> 1105

aggcctcttt tgaaagatgc ggccctgacc ctgtgaacct cgcgcagagc ggcctgaagc 60

gagaggttga ggctgggagg tgagaaaatg 90

<210> 1106
<211> 102
<212> DNA
<213> Homo sapiens

<400> 1106

gcccctctac ggaggccccg cctctagttc ggcctgtttt ctcagtcccg gcacccgccg 60

cgaccgcaaa ggcggccgcg gttctaggaa cttgacgtga tg 102

<210> 1107
<211> 79
<212> DNA
<213> Homo sapiens

<400> 1107

cttcccttac tcaccggtgt ccggaaaggt gaacgctgcg ctcgggctgc ctcgcctgtt 60

acctccgccg ccgggcatg 79

<210> 1108
<211> 97
<212> DNA
<213> Homo sapiens

<400> 1108

gctccttttc agctagtggg tggaacccca ggagggaaaa ctcagggaag cccagggccc 60

gtgttgtgct tttggcccag gtaggtggac agacatg 97

<210> 1109
<211> 280
<212> DNA
<213> Homo sapiens

<400> 1109

agttccttca gtctcagccg ccaactccgg aggcgcggtg ctcggcccgg gagcgcgagc 60

gggaggagca gagacccgca gccgggagcc cgagcgcggg cgatgcaggc tccgcgagcg 120

gcacctgcgg ctcctctaag ctacgaccgt cgtctccgcg gcagcagcgc gggccccagc 180

agcctcggca gccacagccg ctgcagccgg ggcagcctcc gctgctgtcg cctcctctga 240

tgcgcttgcc ctctcccggc cccgggactc cgggagaatg 280

<210> 1110
<211> 396
<212> DNA
<213> Homo sapiens

<400> 1110

cattctttca tactgcctcc tcccttgttt ttctgtctca gagagatagt ctgtcctaaa 60

tatcccatgt agcccaggcc actgaattaa aacggagcgt attcgttctc tgccccaccc 120

cgcaactcct gaaagcggcg caactcaatt acttgatcct tatatgcccc acgcgggact 180

catactacgt ttcccgtgaa cacgtgcagt ccaaaccccg cccctgatat ttatctcagt 240

ggacggtggc cggaaaagga caatggtttc catgtcagcg gataaacgct ctcccctcgg 300

ctcccggacg cgacggaggt cgtagtagta gtgagtacgt gctgaggagc aaaggagtaa 360

ccaagagatc cagtgaccga cagagcaaga gccatg 396

<210> 1111
<211> 132
<212> DNA

<213> Homo sapiens

<400> 1111

tctcctcctt tgcttcataa aaagagggac aagtggctgg tgctgtggac agagaagctt 60

tatttttagt atgagacaac ctctattttc tttcaggaga gggaagttgg attatcaatt 120

cttttgtaaa tg 132

<210> 1112
<211> 103
<212> DNA
<213> Homo sapiens

<400> 1112

cccccccttt cccccttcgc ctcctgacag gaaaggttta agggggacag agccctggga 60

ggccgggccg ggctcggggg ccaccccggg ggcccgggcc atg 103

<210> 1113
<211> 119
<212> DNA
<213> Homo sapiens

<400> 1113

ggttctctgc tctggacttg ggaggctccg ttgcctgctc ccggagggag acgcgctgcc 60

gaggagaacc cagcgggaga acatttcagg ataggaatag gccaagtgct gagaagatg 119

<210> 1114
<211> 225
<212> DNA
<213> Homo sapiens

<400> 1114

ccatctcttc cagcaggaga gggctctact ctgagctcct attttccaag gctccgggcc 60

gcgctcggcg ctggcctgct gccccggcgg gtccgccggc cggaggcggg agtcacagga 120

agagccctcc acaaaaggag gcctcggcgg atcaggacag ctgcaggtgg gtgtgcagac 180

tggtgagctg ccagcagggg cccagacgcg ccaggcctgg agatg 225

<210> 1115
<211> 105
<212> DNA
<213> Homo sapiens

<400> 1115

cggtctctca gcggccggtt tctgcgtccg ctgccgcagg ttccaccgcg ctccaggtat 60

tttttttctt gaaggaaagc tgcttcctca tatgtttcaa gaatg 105

<210> 1116
<211> 271
<212> DNA
<213> Homo sapiens

<400> 1116

cctccttttc cgttgtccct tcgcgcccca aaccacatcc tggagcgcac tctccagcgt 60

ggctggcagc ggggacggtg cgccggggcg caggcccaag agtcgcgtgc gcggcccctt 120

gcaccatccc cccgggccca cccccgggcc gcgctgattg ggcaggtagg gactctgccc 180

agcggaaagt tttgggtgcc gggaggaagt ctaacctttg ggagactcca agacagcagc 240

tccgaggtcg gcgggggtct gggtggccat g 271

<210> 1117
<211> 252
<212> DNA
<213> Homo sapiens

<400> 1117

acttcttttc cgtgggagta aggaagtgct tttgaatgag gtactgaggg ccaaggtgtt 60

ggaagttcct aattctttcc tcggttaact gtgaaactct gcgtattggg aaggcctggc 120

ctcagtcatc aggccaggag aggtactgga cgccgcgcac gcactcgtct gccagcgagg 180

cccaaagggg aagcctagcg gagctcagtg tggcagctgc tggcctctgg gccgctactt 240

gtcaatacca tg 252

<210> 1118
<211> 638
<212> DNA
<213> Homo sapiens

<400> 1118

```
ccgccttcct cctcctcctc tcgccgctac cgccgtcgcc gccgccgcag ccgccgccgg      60
tccgcgcggc ctcgggtggc cggagctcag cctgcgcgcg ccgcgccctg tgtctccggg     120
tggggcagaa gactcgcccc ttgaacctcc cgcggggact ctccgtggtg tggcggccct     180
ggggctcttt cttaatagcc ccggactgag tcccctccag tcgaggaccc tctcctagtc     240
cactgacgag cggtggacac ctgccgctgt atctccccca aaccgagtcc ttgccctgct     300
gcctcctcat acccacacgg cggcagagac cttcaccata gcgttcgctc aactccagaa     360
ccttccgacc tccgctagtt cctgcgggcc tttgcccgct tcccggtgca ccctccccgg     420
gagacacctc agacccccga cagcctgggc aggctcggtg cctgcgggtg cgttcctgat     480
cacccctccc ctcttccctc cccctcatcc tccattccct tgttttcacc ctctgtcctc     540
tgcccgtcac tcccttgtc acctcttgga gcccccctcct aaccagcggc cagtgggttt     600
cccatacccc aggatgtgag cctctttaac ctgtaatg                              638
```

<210> 1119
<211> 107
<212> DNA
<213> Homo sapiens

<400> 1119

```
cactcttctt tagcatgcta ttatggggaa agtgaccact cctgggagcg gggtggtcg       60
gggcggtttg gtggcgggga agcggctgta acttctacgt gaccatg                   107
```

<210> 1120
<211> 90
<212> DNA
<213> Homo sapiens

<400> 1120

```
cttcctctgc tctgcttccc ttcggaggaa aatttcaggc tgaaggttta gcgggtgccg      60
cctctaaaga gagcaatcac tacacttatg                                       90
```

<210> 1121
<211> 271
<212> DNA
<213> Homo sapiens

<400> 1121

gctcctcttc ctgccggcat ccgggatccc tacgtcccgc gtcccccgag cgctcggagc 60

ctacgcgccc agcgctaccg aaacccagag tcctgcgccc tggagtcccc gcgccccgga 120

gcccgagcac ccgggagtcc cgagcctcgc gccccggagt gcccgagcct gcgccgccgc 180

acccggatac cccgcgtccc cgcgagctgc cgaggccgcc cgccgccgcc ccgcggacag 240

taccgccttc ctcccctctg tccgcgccat g 271

<210> 1122
<211> 159
<212> DNA
<213> Homo sapiens

<400> 1122

ctccctccct agctgacttg ctccctcccg ggctgcggct gctgcaaaag ccagcagcgg 60

cagcgggagc tgtccggagg ccggcgtcga gggtttgccg ctgtctctgc tattccatcc 120

tccccatagg ggctctctcc cctctcccat ctcaagatg 159

<210> 1123
<211> 136
<212> DNA
<213> Homo sapiens

<400> 1123

cggcctttgt ctctcgctgc agtcagagct ccaggtctgg ttcttctcct aaaggcccag 60

gctgtgtggc cccgtgtcct gcaggtattg ggagatccac agctaagaca ccgggacctc 120

ctggaagcca aaaatg 136

<210> 1124
<211> 31
<212> DNA
<213> Homo sapiens

<400> 1124
cggtcttccg ggcccgggtc ggggctcgat g 31

<210> 1125
<211> 78
<212> DNA
<213> Homo sapiens

<400> 1125

ggctctctga cgaaggactg gaaggtggcg gtggtgaagg tgcaggccgt tggggcggct 60

cagaggcagg tgactatg 78

<210> 1126
<211> 148

<212> DNA
<213> Homo sapiens

<400> 1126

ctgcctctac ccccgccacg gatcgccggg tagtaggact gcgcggctcc aggctgaggg 60

tcggtccgga ggcgggtggg cgcgggtctc acccggattg tccgggtggc accgttcccg 120

gccccaccgg gcgccgcgag ggatcatg 148

<210> 1127
<211> 93
<212> DNA
<213> Homo sapiens

<400> 1127

ctttctcttt gtcggaggag ctcctctgtt tcctgtgcag tagctcccgt tgcggcggca 60

cccgtggcag ccctggcgga cgcaggagcg atg 93

<210> 1128
<211> 248
<212> DNA
<213> Homo sapiens

<400> 1128

ctgcctctcc tcggccaggc ggaacctctc tgctgggccc ggtggccgca aaagaacttt 60

ctttctcccg cccgaacggt cgccgcggcc aactgcctcg cccgcctggc agcctaaccc 120

tccttctctt cttctcctct ccggcttcgc gcggccctgc ctccctctcg cccggcggca 180

tccgcttgct gctgccaccg cctcctcatc ttctgcccgg ccaaccggcc tgccccgctg 240

cagtgatg 248

<210> 1129
<211> 495
<212> DNA
<213> Homo sapiens

<400> 1129

ccctcccttg cactgcctct ggcacctggg gcagccgcgc ccgcggagtt ttccgcccgg 60

cgctgacggc tgctgcgccc gcggctcccc agtgccccga gtgccccgcg ggccccgcga 120

gcgggagtgg gacccagccc ctaggcagaa cccaggcgcc gcgcccggga cgcccgcgga 180

gagagccact cccgcccacg tcccatttcg cccctcgcgt ccggagtccc cgtggccagg 240

gattattgga cctgcctggt ttaaactatt gtcttagtta attttgtgct gctctaacaa 300

aatatcacag actgagtaat ttataagcaa tagtagctta tttggctcac agttctggag 360

gctgagaaga tcgtgaggct gcatctggca agggccttct tgctgcttca taacatggca 420

gaagacatca tgcgggtgtg tgtctgggga agagacttac agaagtggag ttgctgagtc 480

aaagatctaa ccatg 495

<210> 1130
<211> 164
<212> DNA
<213> Homo sapiens

<400> 1130

ttttctttct ttcctctccc ggcgttgatg agtgcttggc tcctgacaga agggatttgg 60

ctcccagctt tgtagttcgg aagaagttgg gtctatagat ttccccctaa ctctccattg 120

atgtgttgag cttcagaggg aataataact ctacgtaaag catg 164

<210> 1131
<211> 33
<212> DNA
<213> Homo sapiens

<400> 1131
cttcctcttc gttaagtcgg ccttcccaac atg 33

<210> 1132
<211> 256
<212> DNA
<213> Homo sapiens

<400> 1132

cgctcttttt aagctcccct gagccggtgc tgcgctcctc taattgggac tccgagccgg 60

ggctatttct ggcgctggcg cggctccaag aaggcatccg catttgctac cagcggcggc 120

cgcggcggag ccaggccggt cctcagcgcc cagcaccgcc gctcccggca acccggagcg 180

cgcaccgcag gccggcggcc gagctcgcgc atcccagcca tcactcttcc acctgctcct 240

tagagaaggg aagatg 256

<210> 1133

<211> 173
<212> DNA
<213> Homo sapiens

<400> 1133

cggcctttgc ggttgatcgg tcattggggt gctgcagccc cgccacctgt tccgtagctt 60

gccggtgccc cgaaggtgtc ttctcctaag gaagattaaa tcagaaaatt ttaaatcaca 120

gttatccctt tacttaaagc cagagtaagc cttccaaatt aaccccagga atg 173

<210> 1134
<211> 485
<212> DNA
<213> Homo sapiens

<400> 1134

ctttctcttc tcttagcagc acccagcttg cccacccatg ctcaagatgg gcgggatgcc 60

agcctgttac ataaatgtgc caaaagcctg gccatgcctg gaaaatggac caatccgccc 120

gccaagaggt tgggtctcgt tccctagaga gaaggaagtt tcctctcctt gaagtgagag 180

ctagaatcgc actttctgtc aagctgagag aaagactctt ttccagaggc taaaaggaca 240

agaaaatctg atttgcttgc ttctaacttt gcgttttaaa gggggaagga ggaaaggaaa 300

gagggggagg gtggttctgc ttagccccac ccctccggct accccaggtc cagccgtcca 360

ttccggtgga ggcagaggca gtcctggggc tctggggctc gggctttgtc accgggaccc 420

gcaggagcca gaaccactcg gcgccgcctg gtgcatggga ggggagccgg gccaggaaca 480

atatg 485

<210> 1135
<211> 79
<212> DNA
<213> Homo sapiens

<400> 1135

ccgcctcccc gcgggttccg ttggctgtgg cggcagctga cgcttgtggc ggcggtggct 60

tcggggtggg cgtaagatg 79

<210> 1136
<211> 115
<212> DNA
<213> Homo sapiens

<400> 1136

tgttcttcta cttacctggg cccggagaag gtggagggag acgagaagcc gccgagagcc 60

gactaccctc cgggcccagt ctgtctgtcc gtggtggatc taagaaacta gaatg 115

<210> 1137
<211> 101
<212> DNA
<213> Homo sapiens

<400> 1137

ggtccttcac gttccattcc caggctggtc tgagctccgg ggccgtggtc ccgctgcctc 60

ctccggtcgt cgtgcggaag ctgcgacgca ggcagaccat g 101

<210> 1138
<211> 87
<212> DNA
<213> Homo sapiens

<400> 1138

cattcttccg gtggagatgg ctgcggccgt ggcggggatg ctgcgagggg gtctcctgcc 60

ccaggcgggc tagagtgcag tggcatg 87

<210> 1139
<211> 94
<212> DNA
<213> Homo sapiens

<400> 1139

acttctctgt agatcgctga gcgatacttt cggcagcacc tccttgattc tcagttttgc 60

tggaggccgc aaccaggccc gcgccgccac catg 94

<210> 1140
<211> 134
<212> DNA
<213> Homo sapiens

<400> 1140

cggtctttct ctagacgcgt cttgctggga gagtgtccgt tgcttcccgt ccgtgtcgcg 60

gccctgcggt tggcggcctc ctcgtggagc ggagcaaggc caggcggccc ctgctcgagt 120

cccgcgtcgc catg 134

<210> 1141
<211> 301
<212> DNA
<213> Homo sapiens

<400> 1141

gggccccctc cgcgcgtact gcgggcccca cgggtgttag tggcgggggc ggcagagtcc 60
gggtgggttg tcgcgacgga gccgggcctc ttcgccgtct tgagacgggg ctggcgagaa 120
gggcccctca cggagttgcc atgggcgtct aaccgcggca gccaggcccc tctctacgtg 180
agaccccggc ccccctcccc tttctgcagc ccgcccgcca cctgcgcgcc gcgtggcctc 240
cgccggcgcc tgcccgcccc gcgcctccgt ctcccacgga gcaggccggg ctctcgccat 300
g 301

<210> 1142
<211> 204
<212> DNA
<213> Homo sapiens

<400> 1142

ccatcttttc cggcgctggc tcctctccgt cagtgcggtt tcgcctttat ggtggtggag 60
tctgcccagg ctgtggaccg caaataaccc tgtacaaaga ggaatggaga ttgcctctat 120
ccacctagat tcataagctg gcctgaggtg atcttggcat caaggaaggg atgcacatca 180
tcacaccatc agcttcagag aatg 204

<210> 1143
<211> 117
<212> DNA
<213> Homo sapiens

<400> 1143

ctttctcttc ttttgcttct agttaccatc ctcaaaggat tggctaaaag caagcaactg 60
gattgaacac cctaagaaga aagattcaca ctgcaccagg agacatcaga aagaatg 117

<210> 1144
<211> 134
<212> DNA
<213> Homo sapiens

<400> 1144

gcgcctttcg attgcatcag ctggtccagc cgaggccaag tcccgggcgc tagcccacct 60
cccacccgcc tcttggctcc tctcctctag gccgtcgctt tcgggttctc tcatcgcttc 120
gtcgttcgcc aatg 134

<210> 1145
<211> 158
<212> DNA
<213> Homo sapiens

<400> 1145

```
cagcctctgt gcggtgggac caacggacgg acggacggac gcgcgcacct accgaggcgc     60
gggcgctgca gaggctccca gcccaagcct gagcctgagc ccgccccgag gtccccgccc    120
cgcccgcctg gctctctcgc cgcggagccg ccaagatg                            158
```

<210> 1146
<211> 174
<212> DNA
<213> Homo sapiens

<400> 1146

```
cgtcctctca gtggtagcgc ggggactggc tgggaagcgg tcggtcgagt gtggcctgtg     60
tggactcgca tcttgcccga agccgggcgg aggagagctc aagctaaggg tgatcagccc    120
atgacctaaa cctccagaca aaataaaacg gaaaatttgc tagaatcaag aatg          174
```

<210> 1147
<211> 112
<212> DNA
<213> Homo sapiens

<400> 1147

```
tgaccttttc attcccgttg ttatggaggt aggctctcta ggaatctggg agtagtagct     60
gggggcaag agcaaataaa gagctcgagc ttctgtggtc tctggggaga tg             112
```

<210> 1148
<211> 1859
<212> DNA
<213> Homo sapiens

<400> 1148

```
gggccttctg gcagtttctg ggagctgcga acgcgccgcc ccggggctcg gcggccggaa     60
```

acgctggctt cggagcctta ggcgccgcgg cctttccttg ttttccgccc agtccacgcc 120
gccatggcca agtggggcca ggggaacccc cactggatcg tggaggagcg ggaggacggg 180
accaacgtga acaactggcg ctggcgcggc tggcggcggc ctccttccgg gatctgggga 240
gggccgggcc gcgggagccg gggctgccct ggggtctgtg cggggccgcg gggccagggg 300
gtcagggggc cgcccccccct cagctgctgg acgcagggct cggccttcgc ctctcggctc 360
gggagagtcc ttgagtacgg agaccggcta ggagggttgc agctgcctct ttttgaaagt 420
tgggttgggc cccaagagtg acttccgaca gacctttcca ctcccaccgt ctgtggcctg 480
agggccttcc cttctcctcc cgcccacccc tctggatgtt tcggggagtt agaagggagc 540
tggattgaga gactgtgtta ggggcggggg tatggaacgt agtggaaagg gcagaaattt 600
ggatctcagt tcgcgcccac cccgcaggcg cctcccgcga gccgggccct ctgtgagtga 660
gacaagctcc ccttccttta cgcgcctcac ctggcgcgtg ggagaggtc ggcagccctc 720
cgccgcagaa cctccggaag ggatgtcctc tgccctgcgc ctctggccgg ggctgtggtc 780
cctccaggcc gtcgagggga tgctgaggcc ggtccccaga ggagcatgac ttggctggtc 840
cggaggagct ctgagggcat gggcaatctt ggctcgctgc aacctcagct tccagagttc 900
aagcgagtct cctgcttcag cctcatgagt agctgggact acagatgcgt gccactacgt 960
ccgtctgatg tttgtatttt tagtagagac agggtttcac catgttggtc aggctgctct 1020
cgaactccag atctcgtgat ccgcccgcct gggcctacta aagtgctggg attacaggcg 1080
tgagctagat ctgactttct agtgtcctag ccttggcccg atggacatgt catttctctc 1140
agctcgtttc tgtcccctaa agtgagaata ttgcctggga agattacatt agacgatgta 1200
tatgcgaaga cacttgatag ctggtattgt catgattctg attagttcac tactgctact 1260
ttccctgtgg cctaggcttt gcctatttcc agtgggcgag ctagctagat cctcctccct 1320
taaataagcc agtgttttta agacagaata ctacttgcat agtggacaat aatatcttaa 1380
agaactgagc aggatgaaaa gaatttgata gaaagcaggt ttgaggagca cattggaggt 1440
tggcaggttt cgaggctgct tgagaggact tgggccgatc tgggctgggc ttggacgtga 1500
ccctggcacc caggcaggtg gatcccagct ggggcttcca ttcacgactt tctggtccct 1560
ggcaggacag agcgggatgc caccagcttg tccaaaggga agttccagga gctcctggtg 1620
ggcatcgttg tggagaatga cgctggccgc ggcgagatca acgagttgaa gcaggtggaa 1680
ggggaggctt cgtgcagcag ccgcaaagga aagctgattt tcttctatga gtggaacatc 1740
aaactgggct ggaaaggcat cgttaaagaa tctggagtga agcacaaggg attgattgaa 1800
atacccaatc tttctgagga aaatgaagta gatgacactg agaatttaca acgggaatg 1859

<210> 1149

<211> 28
<212> DNA
<213> Homo sapiens

<400> 1149
ctgcctctca gcccaaattg gaaacatg 28

<210> 1150
<211> 115
<212> DNA
<213> Homo sapiens

<400> 1150

ccgccccttt catggccgcc gcctggcgcc ggggctaagt ggccgccggc gtccgggtac 60

ccgagggctc tcccgcgttg ctggcaccgc tggcgccgcg gtctcgtagc gcatg 115

<210> 1151
<211> 110
<212> DNA
<213> Homo sapiens

<400> 1151

cctcctctgg ctgctgcctc cgcagctccc tcctcctacc ccacctcctc catctgggga 60

gcgtctgcgg gggcctgagg ggcggcggcg gcggcggcgg ctgcgatatg 110

<210> 1152
<211> 98
<212> DNA
<213> Homo sapiens

<400> 1152

ccctcctttg cgctgggctg agcccagagc cgagagcagg ggtcggctct gagttccctg 60

cttggttttt gggtggcagc agccagagga ggaatatg 98

<210> 1153
<211> 112
<212> DNA
<213> Homo sapiens

<400> 1153

cacccttctc tgtctacctc tgggcgggac tgccgggtga tgagatactc ggtcggcgac 60

ggtagaacgg gcgacggcga caaccgcaat cacatccacg acggtgatca tg 112

<210> 1154
<211> 213
<212> DNA

<213> Homo sapiens

<400> 1154

```
ggcccctttc ggtccaacgg caggacctgg gggctgtggc cgggggcggc cgttgacctg        60
gtgaccgcgg cgccgcccca gaccgggggc gcagtcccac tcgctccgag ccccggtccc       120
ccaagcctcc ctcccgggta cctggggccg cgcccgccct gcgcccagct ccgccctccg       180
tcggcccagg cctgacagag cccggcagcc atg                                    213
```

<210> 1155
<211> 294
<212> DNA
<213> Homo sapiens

<400> 1155

```
cttcctctct agccgccagt gctctatgct ccgcggtcgc gggccgccag cctccagccg        60
gccagccgcg aggggtgcgc agagggaggc ggggcggaaa ggcgagaggt gtctcctcca       120
ccggagccag gggagacccg agcaagctcc gtgacagcac gtcggccgcc atgtcgccga       180
gtggggctgg aaacagaccc ggcgcccagc ggtagccctc cttgcgcctc cgattcccag       240
acatggaagg tctttaatgt aactttaaat ggttcaccaa aggatgctct aatg             294
```

<210> 1156
<211> 154
<212> DNA
<213> Homo sapiens

<400> 1156

```
gggcctttgt ctctcgctgc agccggcgct ccacgtctag tcttcactgc tctgcgtcct        60
gtgctgataa aggctcgccg ctgtgaccct gttacctgca agaacttgga ggttcacagc       120
taagacgcca ggaccccctg gaagcctaga aatg                                   154
```

<210> 1157
<211> 66
<212> DNA
<213> Homo sapiens

<400> 1157

```
ccccttctc tttcagcctc gggcacgggg gaggctcggc ggacctgctg attgggaacc        60
gatatg                                                                   66
```

<210> 1158
<211> 137
<212> DNA

<213> Homo sapiens

<400> 1158

```
cctcctccct ccttccgtcc tccgcgcctt ccgtcggtcg gtccttgctt cctgcttcgc       60
ctccgcgcct cgcgctatgg gacagagccc ccgatccgcc agcaccacct gaggatccag      120
aaaccgcccc agcgatg                                                     137
```

<210> 1159
<211> 437
<212> DNA
<213> Homo sapiens

<400> 1159

```
acctccctcc cctcccaggc gccgccgcag ccggagcggc tcccgggccc tgggccgccg       60
ccggccagga agaaatactt gtgttggctg catttccagg gatgctacca gagctcaagg      120
ctgtcacctg gtcttgccca gaagagccgt tcttagaggc aggacttgat gaaggctttc      180
ctgctgatgg aataggtttg ctagagctgg ccttggaatt agaacccttc atgtggcctt      240
tataaatatg cgtttgagac agagttatat gcagaagttg aaaatgcctg gaagatttct      300
ggtttctttc actacttatc ctgccttttt gcatcgctgc cagatttgga tgatatgata      360
ttcagagggg caccttaatc aaagccattc ttcaacaaga cccacctggc ataagattgc      420
acacataatt caagatg                                                     437
```

<210> 1160
<211> 68
<212> DNA
<213> Homo sapiens

<400> 1160

```
cctcctctgt ggattctggc caggccgggt tcggcggttg ctgtgagagc gggcttccca       60
acaccatg                                                                68
```

<210> 1161
<211> 595
<212> DNA
<213> Homo sapiens

<400> 1161

aggcctctct gccgcgcgcg caggtacggg gcagaagtcg caggtaccca gctgctgccc 60
acatttctgg tccagagtcc cgaaccccga gcactgggat gcctggctac tccgagccaa 120
ggcactgatg tttgaactgg aaacttcaaa acgtttaata agagtcttca ggatgggttt 180
gaactagaca agctagaaat ttctttagaa caccagctct agcatgcatc tcccactttt 240
ggctttcctg gagaggagct tgaagaggtg gttctgcaga cagccacagt gatacttagg 300
aaaccagagg aatggatttg acttttctgc taggattctc tgttatagtt tctccctgag 360
ttgtaagagg catggaaata tacatgaaac tgaagaacct gcaaggaagg gaagtggaac 420
tttccatgct gagtgaaaac taaccaagtg gcagttgtga ctgaaaacac tgaaacctac 480
cacgtccaga ttcactggat tgggggatag aggaacggtc acagctaggg agaaagaagt 540
gataccggaa aagaaaacct aaatgaagag aatgaggatg actgcacagt agatg 595

<210> 1162
<211> 28
<212> DNA
<213> Homo sapiens

<400> 1162
agctcctttt cctgagcccg ccgcgatg 28

<210> 1163
<211> 311
<212> DNA
<213> Homo sapiens

<400> 1163

agttctcctg ggcgcctagc attgtcgccc acgctgcagt agcggcttct gcggctccaa 60
gccagcgggt cctgtgaagg cgagcagacg cggagaaagg acgcgggagt gagagagggt 120
gagtcagcca ctgtctaaac gataacggga ggcggctctg cggggtaggg ttgaattcag 180
taaatgggct cgtgctgctg tctcttcgga gacgctgcta tcttagcgtc agcgagggaa 240
ggttgaggag gagccagagc cgggtcctgc agcgtttctc gccatcagcg cccgtcgcca 300
tctccaccat g 311

<210> 1164
<211> 127
<212> DNA
<213> Homo sapiens

<400> 1164

ctttctttga ctggagcgga cccgccggac gcaaccgcct cgccagccgg agccagcgcg 60
agctcggcac ggtggacacc cggtccgagg ccggcaagcc ggctggtgcc cgagtcggcc 120
aagcatg 127

<210> 1165
<211> 137
<212> DNA
<213> Homo sapiens

<400> 1165

ggtcccctta tttggatctg cgggaatgtg ggctggagag gtcctgccgt ggtaccagcc 60

tccagcctgc ccccaggact gcccctgacc caggcgcgcc cgctgctcgg tggcaggagg 120

gccggcggag cgccatg 137

<210> 1166
<211> 90
<212> DNA
<213> Homo sapiens

<400> 1166

gattctcttt gttccgcagc catttcaggc cccggacagg aggcagtgcc gcttcggccg 60

aaggcccgag cgcccgaggc gtctgggatg 90

<210> 1167
<211> 43
<212> DNA
<213> Homo sapiens

<400> 1167
cttccttcgt tattggagcc aggcctacac cccagcaacc atg 43

<210> 1168
<211> 329
<212> DNA
<213> Homo sapiens

<400> 1168

ggctccttcc tccaggagtc tctggtgcag ctggggtgga atctggccag gccctgctta 60

ggcccccatc ctggggtcag gaaatttgga ggataaggcc cttcagcccc aaggacatcc 120

tggctgccat acctgctcct gacttctcag ggctggcagt catcgactgg gaggcatggc 180

gcccacgctg ggccttcaac tgggacacca aggacattta ccggcagcgc tcacgggcac 240

tggtacaggc acagcaccct gattggccag ctcctcaggt ggaggcagta gcccaggacc 300

agttccaggg agctgcacgg gcctggatg 329

<210> 1169
<211> 182
<212> DNA
<213> Homo sapiens

<400> 1169

agtcctcccg gtcgccccac tgcgcatggc acgttgcgta ctcccctccc agcaaccggt 60

ctggcggcgg cgcggcagta aaactgagga ggcggagcca agacggtcgg ggctgcttgc 120

taactccagg aacaggttta agtttttgaa actgaagtag gcctacacag taggaactca 180

tg 182

<210> 1170
<211> 113
<212> DNA
<213> Homo sapiens

<400> 1170

ccctctcttt cgctgtttga gagtctctcg gctcaaggac cgggaggtaa gaggtttggg 60

actgccccgg caactccagg gtgtctggtc cacgacctat cctaggcgcc atg 113

<210> 1171
<211> 79
<212> DNA
<213> Homo sapiens

<400> 1171

ggctctttcc agctcctggc agccgggcac ccgaaggaac gggtcgtgca acgacgcagc 60

tggacctggc ccagccatg 79

<210> 1172
<211> 115
<212> DNA
<213> Homo sapiens

<400> 1172

cagtcttttc tccttgctca gcttcaatgt gttccggagt ggggacgggg tggctgaacc 60

tcgcaggtgg cagagaggct cccctggggc tgtggggctc tacgtggatc cgatg 115

<210> 1173
<211> 145
<212> DNA
<213> Homo sapiens

<400> 1173

atcccttcct ctcttttctg ttcgccctct tctccctgct ctttttccct ttccaccccc 60

ctcctctgtt ctccctcacc tcctgcgccc cctccccctt cccgggttct gacagtacga 120

tgagctgccc cattacggcg ggatg 145

<210> 1174
<211> 169

<212> DNA
<213> Homo sapiens

<400> 1174

ttttcttttc gtttagatac attgcctttt gcctaggctg gcgtcgagac ttgaggccgt 60

tgcagacttt ggcgcggctc gcgcctcctg cttcaagagc ccagcggtga gagctggcct 120

gcggcacgcg gcctaatgcc agacagtaac agtttggagg atcaagatg 169

<210> 1175
<211> 238
<212> DNA
<213> Homo sapiens

<400> 1175

gagcctttgc cccggcgtcg gtgactcagt gttcgcggga gcgccgcacc tacaccagcc 60

aacccagatc ccgaggtccg acagcgcccg gcccagatcc ccacgcctgc caggagcaag 120

ccgagagcca gccggccggc gcactccgac tccgagcagt ctctgtcctt cgacccgagc 180

cccgcgccct ttccgggacc cctgccccgc gggcagcgct gccaacctgc cggccatg 238

<210> 1176
<211> 677
<212> DNA
<213> Homo sapiens

<400> 1176

cgctctttct ctcgccgcgc cgtcttgaag ccgcgcgggc tcgtgagcag cgcgaggccg 60

ccaaggtgcc tcgcttcgcc ggagccgctg ccgcccgccg gagggaagcc ggcctcgggc 120

gcgcacgctc gtcggagccc cggcgcgccc cgcgcctgag cctgctgaca gcggccgctg 180

ggctcaggct gtccgctctg ggctccgcgg cctcggcccc gctgcactcc acctccgccc 240

cctcggactc cctcccctct gcttctactc ctcctgctcc agtgcggatc gtttcgcaac 300

tgcttgccac tcgtcccgtg cctggctgtt tttccatttc ccggccccct cttcttgagt 360

actttacccc ctgcatttgg ggacagggac tggaaaaggg gcgggtggag cgtccagtgg 420

agaagaagga agcgaggccc gcaggaggag gaggatcggc ggactgtggg gaggagaccc 480

cacgccaccc tttctggtca tctcccctcc cgccccgccc ctgcgcacac tccctcgcgg 540

gcgagctact ttcggaccag gaaagtaaga gcggccctgg gtgacagcgc cgcggggcca 600

gtcccggggt tagccgcgcg tctgctcgct tctggtccgt cgcgctccca gccagggcac 660

agcccggacc gaggatg 677

<210> 1177

<211> 95
<212> DNA
<213> Homo sapiens

<400> 1177

ccgtctcctc ctcttgctcc ctcggccggg cggcggtgac tgtgcaccga cgtcggcgcg 60

ggctgcaccg ccgcgtccgc ccgcccgcca gcatg 95

<210> 1178
<211> 796
<212> DNA
<213> Homo sapiens

<400> 1178

ttttcttttc gccaggggtt gggactccgg gtggcaggcg cccgggggaa tcccagctga 60

ctcgctcact gccttcgaag tccggcgccc cccgggaggg aactgggtgg ccgcaccctc 120

ccggctgcgg tggctgtcgc cccccaccct gcagccagga ctcgatggag aatccattcc 180

aatatatggc catgtggctc tttggagcaa tgttccatca tgttccatgc tgctgacgtc 240

acatggagca cagaaatcaa tgttagcaga tagccagccc atacaagatc gttttcaact 300

agtggcccca ctgtgtccgg aattgatggg ttcttggtct cactgacttc aagaatgaag 360

ccgcggaccc tcgcggtgag tgttacagct cttaaggtgg cgcatctgga gtttgttcct 420

tctgatgttc ggatgtgttc ggagtttctt ccttctggtg ggttcgtggt ctcgctggct 480

caggagtgaa gctacagacc ttcgcggagg cattgtggat ggatggctgc tggaaacccc 540

ttgccatagc cagctcttct tcaatactta aggatttacc gtggctttga gtaatgagaa 600

tttcgaaacc acatttgaga agtatttcca tccagtgcta cttgtgttta cttctaaaca 660

gtcattttct aactgaagct ggcattcatg tcttcatttt gggatgcagc taatataccc 720

agttggccca aagcacctaa cctatagtta tataatctga ctctcagttc agttttactc 780

tactaatgcc ttcatg 796

<210> 1179
<211> 59
<212> DNA
<213> Homo sapiens

<400> 1179
gtccctcctt ccctccccga ctgtgcgccg cggctggctc gggttcccgg gccgacatg 59

<210> 1180
<211> 309
<212> DNA
<213> Homo sapiens

<400> 1180

```
cactctcttt ctctctccct ctggcatgca tgctgctggt aggagacccc caagtcaaca      60
ttgcttcaga aatcctttag cactcatttc tcaggagaac ttatggcttc agaatcacag     120
ctcggttttt aagatggaca taacctgtac gaccttctga tgggctttca actttgaact     180
ggatgtggac acttttctct cagatgacag aattactcca acttcccctt tgcagttgct     240
tcctttcctt gaaggtagct gtatcttatt ttctttaaaa agctttttct tccaaagcca     300
cttgccatg                                                             309
```

<210> 1181
<211> 173
<212> DNA
<213> Homo sapiens

<400> 1181

```
agccctttta ctcagccaca gcctccggag ccgttgcaca cctacctgcc cggccgactt      60
acctgtactt gccgccgtcc cggctcacct ggcggtgccc gaggagtagt cgctggagtc     120
cgcgcctccc tgggactgca atgtgccgat cttagctgct gcctgagagg atg            173
```

<210> 1182
<211> 666
<212> DNA
<213> Homo sapiens

<400> 1182

```
cttccttcct tctccagtcc cttccactgt gcgtcttctg tcccccgttc ttccccagcg      60
gacccctctt tcgagactcc ctagtggggt ccccagctcc cgggcgatcc tgcccttgcc     120
gagcgcgttt tctggagtca cctgggggag gggagtcctg ggcagggccg ggctggggaa     180
gacgcctggg gcactgcccg gcgttaacaa agggagccga taccgaccgg cgtgggcgcg     240
gagcgggcgg ccgccaccga gcgtgctgag caaccgcagc ctccgcggcc gagagtgcag     300
cgagcaaggg gagagccagt tgcgcagagc cctgcaacca gcagtccagg gagaagtggt     360
gaatgtcatg gagcccagct gaaatggact ggcccccttg agcctgtccc aagccctggt     420
gccaggtgtc catccccgtg ctgagatgag ttttgatcat cctgagaaaa atgggccttg     480
gcctgcagac ccaataaacc ttccctccca tggataatag tgctaattcc tgaggacctg     540
aagggcctgc cgcccctggg ggattagcca gaagcagatg atcatgacgc agtcctgagg     600
tttaatgggg cacccacagc caacttccaa caagatgtgg gcacaaaaac taccattcgc     660
ctgatg                                                                666
```

<210> 1183
<211> 60
<212> DNA
<213> Homo sapiens

<400> 1183
ctccccttcc gcgcccggct ccccttccgc gcccctcccg ccggagatga ggggaagatg 60

<210> 1184
<211> 37
<212> DNA
<213> Homo sapiens

<400> 1184
gcttcttttg ctgggctgct gctccttcgg catcatg 37

<210> 1185
<211> 420
<212> DNA
<213> Homo sapiens

<400> 1185

cggtcttccg ggtgtctttg acagggtttt ctacgccgct ttttcggcga ctttttgctc 60

ttccgctttt tgccaccgcc cccaaccttc tatatccttg cagcccctac cttttcttgt 120

gttgctcctc ccctggcagc cgtgaggggg gttagatctc agccggagcc ggagctgggc 180

ctagctgtcc cacgggccac cactacctcc tttggttcgg gagaaagcta cgaccaagta 240

cgcccagctc gggccttaga acttctgaac gggcagtgcg ggtaggccct gcttagccct 300

tcccggagga cacctgacca aaagaggaag atagtcttgg gacccttgca tggtgtttca 360

aagggtggtg aagaactaag gtagaagaat acatgttcac ttccagtgaa caagagcatg 420

<210> 1186
<211> 120
<212> DNA
<213> Homo sapiens

<400> 1186

ctcccttctg gtgtactggg tgggaggtgg aactagtcgg acaaagccct cgcgtcggac 60

ccttgccaga actcaattaa tggatgcctc gaagttgacg tacatatata ttcagaaatg 120

<210> 1187
<211> 230
<212> DNA
<213> Homo sapiens

<400> 1187

cgcccctttg cgcggctggc gcggccagcc ggccaggctc ccctcggcaa acctgtctaa 60

ttggggcggg gagcggagct tcctcctctg agggccgtgc cgcgctgcca gatttgttct 120

tccgcccctg cctccgcggc tcggaggcga gcggaaggtg ccccggggcc gaggcccgtg 180

acggggcggg cgggagcccc ggcagtccgg ggtcgccggc gagggccatg 230

<210> 1188
<211> 114
<212> DNA
<213> Homo sapiens

<400> 1188

ctacctctac ccacagccag tgcctttggc gcactgaggt gcacagggtc ccttagccgg 60

gcgcagggcg cgcagcccag gctgagatcc gcggcttccg tagaagtgag catg 114

<210> 1189
<211> 162
<212> DNA
<213> Homo sapiens

<400> 1189

cctcctctcg ctctctgccc gctaactttc ccgagccccg accggcggcg cagagctccg 60

gggtagcttt gtggccgaac gccgacctcg ggcggagagc gcggctgtgc ccagtatccc 120

atccccgcga cccccgcgcg ctccggagag aacaggacta tg 162

<210> 1190
<211> 206
<212> DNA
<213> Homo sapiens

<400> 1190

tcccctctgc ctcccggtgg ctcctcgctc tccttccatc tctctcgccc cctctccctc 60

cgtcccgtcc tcgccgctcc cctcaccccg cctctctccc cctcccccag cccctcctct 120

cctcacccca cccggcctcc ctccctccct cgcccgcccg gcgctcgcag agccgacacc 180

agggggctc tcgatgtagc accatg 206

<210> 1191
<211> 119
<212> DNA
<213> Homo sapiens

<400> 1191

ttcccttcct tggatccctg tgcacctact ggagccaggt tactctgggt cctggacctg 60

actgcctcat tctggaggct tccagacagc cacagttagt gcccaaacct gagaggatg 119

<210> 1192
<211> 52
<212> DNA
<213> Homo sapiens

<400> 1192
cgccctctct tcctgcagcc tgggaacttc agccggctgg agccccacca tg 52

<210> 1193
<211> 248
<212> DNA
<213> Homo sapiens

<400> 1193

cattctcttt ccttttcctt ctctcctgag cgctcctgca gttcctgggg cgtagtaggg 60

gatccacaag cgtttgtgac cagtgaagtt ctttacaagg gtgagatctg cacgggagga 120

cccgagcgag ggtctcggct tgccaggaag ccggggttcc ccgggaagcg tggagttcac 180

ccgcgcactc gaagtgcctt tgcaaaatta tatctgggtg ttggcaccca gccactattc 240

tgccaatg 248

<210> 1194
<211> 89
<212> DNA
<213> Homo sapiens

<400> 1194

agctcttttc cttcttcctc cacttcccct accctccacc gtccgggagc cgccgccacc 60

gccgccgagg agtcaggaag ttcaagatg 89

<210> 1195
<211> 693
<212> DNA
<213> Homo sapiens

<400> 1195

gcgcctttgc ggccgtgatt cggtcccgct gtcctaggcg ggatggtgcc gctgtgccag 60
gtaagggtgg cgggtgtgcg tgcgggcctg ggtgcggagc cctcctcgac gtgtctctcc 120
cgccctttcc ctccacatac ccagccttgg tcagtcggac ctccccacta gcccccaacc 180
tggccggcgt cttgggttcg ggggcgcccc cgccccccgcc cccgggccct tcctgtctcc 240
gggctttact gcgactgccc cagcagaagt cgggtcctct ccgagaactc ttgtcagctc 300
acggcagcaa ggacggactc gttctgaagg cgcctccacc ttttatgacc acctctttcc 360
cagattattc gttttgatga agctaaaatt ttaatctaaa aagaaatgca cctcatggag 420
aattcttgtg aagaactgtg cttcatctgt ggatttctac acccttgatc atttgcaaac 480
ctgtaattat ttcgtaaaga gttgtttgca cggagtgaca ggttgaagta ttgtattttg 540
caaaaagtgc tgaaataaca ggagttcgtt cagagaccat ttctgtgcct caagaaataa 600
aagcgttgca gctgtggaag gagatagaaa ctcgacatcc tggattggct gatgttagaa 660
atcagataat atttgctgtt cgtcaagaat atg 693

<210> 1196
<211> 24
<212> DNA
<213> Homo sapiens

<400> 1196
tttccttttt ccggagggga gatg 24

<210> 1197
<211> 45
<212> DNA
<213> Homo sapiens

<400> 1197
ctgcccctca ctcgtctcgc ccgccagtct ccctcccgcg cgatg 45

<210> 1198
<211> 512
<212> DNA
<213> Homo sapiens

<400> 1198

agtccttctt gtcctggtcg ttgttcccgt ctgagtacca gctccccact gccctgaggg 60

cgggccggcc tgcggcggag ggaaaaagga agaggagaag gaaattgtcc cgaatccctg 120

cagtgggtcc aagcctctcc cgggtggcca gtctttctgt aggttgcggc acaacgccag 180

gcaaaagaag aggaaggaat ttaatcctaa tcggtggagg tcgatttgag ggtctgctgt 240

agcaggtggc tccgcttgaa gcgagggagg aagtttcctc cgatcagtag agattggaaa 300

gattgttggg agtggcacac cactagggaa aagaagaagg ggcgaactgc ttgtcttgag 360

gaggtcaacc cccagaatca gctcttgtgg ccttgaagtg gctgaagacg atcaccctcc 420

acaggcttga gcccagtccc acagccttcc tcccccagcc tgagtgacta ctctattcct 480

tggtccctgc tattgtcggg gacgattgca tg 512

<210> 1199
<211> 518
<212> DNA
<213> Homo sapiens

<400> 1199

cgtcctcctc tgggtaccaa ctctattgcg cagctcgctg ccgtgcgttt aacccaggcg 60

aggaggagga ggagaaaatt cccccagatt cgggcaggcc cgcaccccac attccgtcct 120

gttttgagag gaggagggaa gagaaataaa cgtggcagcg catagaaggc cagcagggag 180

actgctttcc agacacctcc ggcccacaca gccgttcacc ccccgtcttt tcagtcctgg 240

aaaaggaatt cggtctgtcc ttaggatgaa gctctaactg aactgaagta aggagaaaca 300

gccttgaatc tttggagggt ctgtcttcct tttgggctct gtgcaactgc agctacagtg 360

gaaaaaagca aactgctctt gatcccaggc cctgcctaag cctcagcaga acttgtaagc 420

ctaaactgaa gagcctcacc cggacgagca ggcatccctt aaccttaagc aatccagttc 480

cacgccctgg atcagtgaat aaccccagct gcaccatg 518

<210> 1200
<211> 537
<212> DNA
<213> Homo sapiens

<400> 1200

cgccctctgg ggctccgagc ccggcgggac catgttcacc agcaccggct ccagtgggct 60
ctgtgagtac cggcctccgc catcctggct gccccctaca cgccacccta ggcacctctt 120
tgaggaggct ggggcagcgg ggaccctcgg gtttgccgga ggtggtgggg ccgaccctcc 180
agacccgcgt ccgaaccctg ctagttcccg gtcttggggg tcagcggaaa ccgcccccat 240
ttcggcctgg aggggcgaat ggggacaaag ccccgccgcc cgccccgacc ccacctggta 300
tccccaggtg ctctgcccag gagtctcttg ggccgctgc aagtgggcag gtgccctggt 360
gttctcgtgg gccggcccca ggccctttgc ggagcgtgtg ccgcgctgaa ggaaggggcc 420
gtccccctta ccatgcccca ttcttttagg cttgggggac cgaactaact ccccccgccc 480
ccacttgcaa agttcagcct ccgctttaga agctgacctc tcagtttcac ttggatg 537

<210> 1201
<211> 119
<212> DNA
<213> Homo sapiens

<400> 1201

cctcctccct cggccggccc tggggccgtg tccgccgggc aactccagcc gaggcctggg 60
cttctgcctg caggtgtctg cggcgaggcc cctagggtac agcccgattt ggccccatg 119

<210> 1202
<211> 181
<212> DNA
<213> Homo sapiens

<400> 1202

cgctccctca cagctcccgt cccgttaccg cctcctggcc ggcctcgcgc ctttcaccgg 60
caccttgcgt cggtcgcgcc gcggggcctg ctcctgccgc gcgcaccccc ggggcttcgg 120
ctccggcacg ggtcgcgccc agctttcctg cacctgaggc cgccggccag ccgccgccat 180
g 181

<210> 1203
<211> 130
<212> DNA
<213> Homo sapiens

<400> 1203

ctttcttttt aaaaatcgct gggtctgttg agctgtcctg ggctgggtgc cttgctcttt 60
gactgagact ggagacagac ggcaacagcc acaggcagac tgaggtggca ataggaaatc 120
tgccgagatg 130

<210> 1204
<211> 239
<212> DNA
<213> Homo sapiens

<400> 1204

gattctcccg cctcccagcc ccggcgcacg cgcgccccgc ccagcctgct ttccctccgc 60

gccctcccct ctcctttctc cctctcagaa ccttcctgcc gtcgcgtttg cacctcgctg 120

ctccagcctc tggggcgcat tccaaccttc cagcctgcga cctgcggaga aaaaaaatta 180

cttattttct tgccccatac ataccttgag gcgagcaaaa aaattaaatt ttaaccatg 239

<210> 1205
<211> 146
<212> DNA
<213> Homo sapiens

<400> 1205

tttccttctt cccgcgagtc agaagcttcg cgagggccca gagaggcggt ggggtgggcg 60

accctacgcc agctccgggc gggagaaagc ccaccctctc ccgcgcccca ggaaaccgcc 120

ggcgttcggc gctgcgcaga gccatg 146

<210> 1206
<211> 235
<212> DNA
<213> Homo sapiens

<400> 1206

ttttctttct ctcagcatct ccacccaacc agcagaaaac cggtgagtgg ggcttttaag 60

tgattttcaa gaagaatgta acagatgtca aacgggaaaa gcacaaggca aagcctgctc 120

tctctgtctc tctgtctcct cttctccttt tttgccttat tctatccgat tttttcccta 180

agcttctacc tgggattttc ctttggaaaa gtctctgagg ttccaccaaa atatg 235

<210> 1207
<211> 82
<212> DNA
<213> Homo sapiens

<400> 1207

ttgtcttttt ttttttaaac taaaatggag gctggtttct tgccttaagg agcccattgc 60

ctttcccgct gaagtctaga tg 82

<210> 1208
<211> 440
<212> DNA

<213> Homo sapiens

<400> 1208

ccacctttcc gggggaagcc acgcgcacca ggcatcgcac gcggctctgc acccgcgccg 60

ccggacctga aacccggcgg agggcacacg gggctgccgc tgcgggcccc ggaccaaccc 120

atgcttactc cggagcctgt accggcgccg acgggtcgga cctccctgcg cggtgtcgcc 180

cagcgggttc gtgcgaaagg cggggccgac tacacgcggt gccgcgccct gagaccgttt 240

atctgcagtc aacgcagcct cccggctcag cctgggaaga tgcgcgaatc gggaacccca 300

gagcgcggtg gctagaccgg gctccgccgc ctcccccaca gcccctttcc taatcgttca 360

gacggagcct ggtcgacttc gccggagact gccagatctc gttcctcttc cctgtgtcat 420

cttcttaatt ataaataatg 440

<210> 1209
<211> 257
<212> DNA
<213> Homo sapiens

<400> 1209

caccctcttc gtcgcttcgg ccagtgtgtc gggctgggcc ctgacaagcc acctgaggag 60

aggctcggag ccgggcccgg accccggcga ttgccgcccg cttctctcta gtctcacgag 120

gggtttcccg cctcgcaccc ccacctctgg acttgccttt ccttctcttc tccgcgtgtg 180

gagggagcca gcgcttaggc cggagcgagc ctgggggccg cccgccgtga agacatcgcg 240

gggaccgatt caccatg 257

<210> 1210
<211> 555
<212> DNA
<213> Homo sapiens

<400> 1210

cctcctcttc ctccccactc tgcacatgcg gctgggtggc agccagcggc ctcagacaga 60

cccactggcg tctctctgct gagtgaccgt aagctcggcg tctggccctc tgcctgcctc 120

tccctgagtg tggctgacag ccacgcagct gtgtctgtct gtctgcggcc cgtgcatccc 180

tgctgcggcc gcctggtacc ttccttgccg tctctttcct ctgtctgctg ctctgtggga 240

cacctgcctg gaggcccagc tgcccgtcat cagagtgaca ggtcttatga cagcctgatt 300

ggtgactcgg gctgggtgtg gattctcacc ccaggcctct gcctgctttc tcagaccctc 360

atctgtcacc cccacgctga acccagctgc cacccccaga agcccatcag actgccccca 420

gcacacggaa tggatttctg agaaagaagc cgaaacagaa gatgaggcaa tgaggctgcg 480

agaggtagag tgattttccc tcggtgactc aactgggacg tagcaggtcg ggcagtcaag 540

ccaggtgacc ccatg 555

<210> 1211
<211> 295
<212> DNA
<213> Homo sapiens

<400> 1211

tggtctttcc gggtccttgc acgcttcgct ccaactcctg cagagctgag ccggagggga 60

atccggaagg gacacgctga acaggtctga ctcccgggca gcacagcccg ctcacgattc 120

cggccacggt gatgacgagt ctccgtcaac ctcgtctggc acagctggga cctcctctgt 180

gccagagcta cctgggtttt actttgaccc tgaaaagaaa cgctacttcc gcttgctccc 240

tggacataac aactgcaacc ccctgacgaa agagagcatc cggcagaagg agatg 295

<210> 1212
<211> 138
<212> DNA
<213> Homo sapiens

<400> 1212

ccgcctcttg tgaggcgcgc ggagccgcct cccctgggtc aggtctgatg ggccggtggg 60

cgcgctagtg gtggccgcca ccgccgaaac cgtcgacctc ctgggcccca gttccgcgtc 120

cagccccgcg gcagcatg 138

<210> 1213
<211> 47
<212> DNA
<213> Homo sapiens

<400> 1213
ccccctctct atcagccgct cactccgtct caatatgtct caagatg 47

<210> 1214
<211> 173
<212> DNA
<213> Homo sapiens

<400> 1214

ctccctttc ctccccactc cttcccacca gcgccacagc aacatcctca gagtctgagc 60

gaactgcgcc cagcgcgggc acggagcctc ccaccgccag caacctgcgg ccccggagaa 120

ggcagcgagc gcagtgacag cgcctcaccg ccaccagctc ctggaccacc atg 173

<210> 1215
<211> 162
<212> DNA
<213> Homo sapiens

<400> 1215

ctgccttctc tcccggggcc ctgtgggcaa gcctcctgct tcactttcag gtttctcgaa 60

gtgccttctt gctcctgtct gtttccccat cctgccagat ttctgtttct cttgctgggc 120

ttttggcagt aggggctgt gttggtgggc cctacgaaga tg 162

<210> 1216
<211> 225
<212> DNA
<213> Homo sapiens

<400> 1216

aggccttttc cgcttctctt ttacctcccc aggtccgccc gtctgcgccc ctcacaggaa 60

gccggagggt cgctctgatc ccgaatctcc cacaggcgtg aacctgctct gctgtgtatc 120

tttgcggggt ggcctgcgct gaggcctgcc gcgcgcggtg agtccgcgca gacctgaccc 180

tgcgtctcgc agctcggttg aggccgccgc cgccttctcg ggatg 225

<210> 1217
<211> 633
<212> DNA
<213> Homo sapiens

<400> 1217

```
cttcctttac cttcctccca tggtctcctt ccggttctcg atgcttctct gagcctaagg      60
gtttccgcca ctcgttcacc ctccccccag ctcatgatcc tcctccctcc cccgccctcc     120
tggtccaatc tccgatctgt ttagtaagaa ggtgctgttc cgagaagaag gaaaagggct     180
tgacacgtat tcactcggcc ccggacgtgg gaagcaagcc gtctggcttc ggcctcacat     240
cggtcttgtg ctcgggacgg cggcgttggc ggactgatcc gcggcggtga agagaggccg     300
ggaagttaaa cttgtagcca ccacctccgc tcttcccgtc accctcgccc ccacttcggg     360
ccgaaagcac ggtacagagg ctgttggtgg ctttgccacg ccaccccacc caccccggat     420
cgcggctgtc ttaagggacc tggattcatc aggggctctt cggggcctgt gcgagtgctg     480
atctgctccg tttttgcaaa aggcgcctgt gtctggcaga gctggtgtga gacgagacaa     540
tcctgccccg ccgccgggat aatcaagagt tttggccgga cctttgagca tacaccgaga     600
gagtgaggag ccagacgaca agcacacact atg                                  633
```

<210> 1218
<211> 169
<212> DNA
<213> Homo sapiens

<400> 1218

```
tttcctctgg ctcctgcgag ggcttggttt agggcttcag ctctctgcgt tctcggctcc      60
gggaggcctc ggtgattcag ccacagcctc tgcctcccgt tgctctgtga cctgagggta     120
ttggacaatt tgtagctaag actcccggat accctgaagt cgggaaatg                169
```

<210> 1219
<211> 143
<212> DNA
<213> Homo sapiens

<400> 1219

```
tgctctcttc caaggctgta ggagttctgg agctgctggc tggagaggag ggtggacgaa      60
gctctctcca gaaagacatc ctgagaggac ttggcagcct gcagatggcc tattgtggga     120
ccttgtgatc atgcctgaac atg                                             143
```

<210> 1220
<211> 190
<212> DNA
<213> Homo sapiens

<400> 1220

tttccccttta tagcaccatt gaatcccagt cctaacagaa gtactgcgaa tcttgtggcc 60

tcattctgaa caaaagggat tagagaagaa aaatctcttg atataaggct tgaaagcaag 120

ggcaggcaat cttggttgtg aatattttct gatttttcca gaaatcaagc agaagattga 180

gctgctgatg 190

<210> 1221
<211> 93
<212> DNA
<213> Homo sapiens

<400> 1221

tgcccttcct cgccaccggg ctgctctggt ctcgtcggtc ccctcctccg ccccgtcgtc 60

ctgactctct ctccctcctt tcctcagagg atg 93

<210> 1222
<211> 378
<212> DNA
<213> Homo sapiens

<400> 1222

aaccctttca cctcagtttt cttcactccg gcatttgcag cagagcgaaa ggtggtcgag 60

tcctgaagga gggcctgatg tcttcatcat tctcaaattc ttgtaagctc tgcgtcgggt 120

gaaaccagac aaagccgcga gcccagggat gggagcacgc gggggacggc ctgccggcgg 180

ggacgacagc attgcgcctg ggtgcagcag tgtgcgtctc ggggaaggga agatatttta 240

aggcgtgtct gagcagacgg ggaggctttt ccaaacccag gcagcttcgt ggcgtgtgcg 300

gtttcgaccc ggtcacacaa agcttcagca tgtcatgtgg cttatcagga gggcagactt 360

caaaagctac taaaaatg 378

<210> 1223
<211> 1104
<212> DNA
<213> Homo sapiens

<400> 1223

tgtccttccg cgcggcggcc gcggagagag ctgcggcccg gggggcgtg cctgggatcc 60

ggagcttcgc tcgggcccgg gaaaggcggc agtgggctgg gatcgcggtg tctctgggtg 120

tgatggccaa tggctggact ggctcccgcc ctgggcggag gaatcccgag ctgtgaagcg 180

gctggaatcc gggcccatgt gcttctttgt ttactaagag cggaagcgat ggcgggagcg 240

ggggtggggt gcggtggcgg ggtgcggtgg cggaggtccc ggtgaaatca ggggctaagg 300

ggacccaaag aaggcggggg atcatagggg tggaaagaaa gctgagaacc ttgagaccgg 360

agtgtgaggg gccaacgggg aagggcgcta gaattttaaa ctaaagtagg gaccggaatt 420

cccctgggga gatgttggat ggccctgtgc actgccacgg gctctttatt cttcgctggt 480

tagaaacaga cttgtgaaaa agagttatgc ccactttggg gagacttcga aaaggttaag 540

aagttcttac aagagttcta ccaggatgat gaactcggga agaagcagtt caagtatggg 600

aaccagttgg ttcggctggc tcatcgggaa caggtggctc tgtatgtgga cctggacgac 660

gtagccgagg atgaccccga gttggtggac tcaatttgtg agaatgccag gcgctacgcg 720

aagctctttg ctgatgccgt acaagagctg ctgcctcagt acaaggagag ggaagtggta 780

aataaagatg tcctggacgt ttacattgag catcggctaa tgatggagca gcggagtcgg 840

gaccctggga tggtccgaag cccccagaac cagtaccctg ctgaactcat gcgcagattg 900

tgagtggtct ctgtcgggaa agatgtaggg attggttctc caggatcttg tttgtgactg 960

ttttctcccc ttagtgagct gtattttcaa ggccctagca gcaacaagcc tcgtgtgatc 1020

cgggaagtgc gggctgactc tgtggggaag ttggtaactg tgcgtggaat cgtcactcgt 1080

gtctctgaag tcaaacccaa gatg 1104

<210> 1224
<211> 135
<212> DNA
<213> Homo sapiens

<400> 1224

tttccccctc tccccctcct ccgccgaccg agcagtgact taagcaacgg agcgcggtga 60

agctcatttt tctccttcct cgcagccgcg ccagggagct cgcggcgcgc ggcccctgtc 120

ctccggcccg agatg 135

<210> 1225
<211> 463
<212> DNA
<213> Homo sapiens

<400> 1225

ctttctttcc ctctccgttt tggtgggctg gttgaagatg aaatccactg aggagggaag 60

tccagcaccc tgtgtgccag tccagaactg gcccatctgt agaccccctg aaaatcatat 120

gggcttggat ttggatattc tcaacagaaa gggttaaagg ctgatggtac ctaaagcctg 180

gtacttgaat tttgatcaag ataagctgcc ttaagttctc ttcattacac aaatgatcct 240

agataattga tagatcctgt ggttcaactg gatttctaga tagaagctgg attcatgtga 300

tgccagagga gtaaaatttc aagagactga aaccagatct gagtttcgct gttccagtct 360

ggacctcttt ggtgctgtaa atcctggata tactgtagat gagtactgcg tttttctttt 420

atggcctctc ttcagcttct ggagacctca ctatcctatt atg 463

<210> 1226
<211> 453
<212> DNA
<213> Homo sapiens

<400> 1226

ccgccttcgc cgcggacctt cagctgccgc ggtcgctccg agcggcgggc cgcagagatg 60

acatttattc attttatgca tcctgggttc tactggtcgt cccacctcag ttcctgtagc 120

aaagagactt gagtctgagc cactaattat cacccgtgag gtttcctccc cgagcaggaa 180

gcagcaggcc agagctgcgc tctctcagtg cactctccaa ccaagcatca gtcaccactc 240

ccggtccagc ccctgtggcc aagagctggc gtgcaggctg cgggaggcag ctggctgtgc 300

aagaccctgg cagggccctc gcctcctgag aaaccgagag tcagaaccaa agccaggctg 360

tcctggttgg agactgagcc agaaagggtg gctcacctca cggtgaggct gtcgagtgac 420

ctgagagcct cagaccctca cgtcagccgg atg 453

<210> 1227
<211> 169
<212> DNA
<213> Homo sapiens

<400> 1227

tgttcttccg ggttggaggc gcagcgccgc ggggcccaag cccgggtctg ccagcgcgac 60

gtcctctcgc ggccctcagg gcacagccca aggctgtcag cctcccggcc cagtgatttg 120

ccttcaagga aactggggag tcagaaaatt gggaactcat atcaacatg 169

<210> 1228
<211> 149
<212> DNA
<213> Homo sapiens

<400> 1228

gtccctctcc ctccccagga cttctgtgac tcctgggcca cagaggtcca accaggctaa 60

gggcctgggg atacccccctg cctggccccc ttgcccaaac tggcaggggg gccaggctgg 120

gcagcagccc ctctttcacc tcaactatg 149

<210> 1229
<211> 52
<212> DNA
<213> Homo sapiens

<400> 1229
cggcccttcc agaccgtctc tcctcagggt tggagacttc ggggccaaga tg 52

<210> 1230
<211> 771
<212> DNA
<213> Homo sapiens

<400> 1230

tcgcctcttt agtaggtcgg gtgagtgtag tgtgcaggga agagacgcgt cagcgccagg 60

gccaggcccg cccgggggca gcccggcagc cgaatcttgg gctactctgt cccaacagcc 120

ggagcagatc agaccgaccg gccctgcccg ctcggtcccg cgccctccag accctacggt 180

ctccgtttct aggggcacat ggttagcggc aggcgcccac agccaatcca ctttgccagc 240

ctgccccttc ctctgccaag agcagcttct tcagccgcgc tccagttccg cagacgcctg 300

ccccaccctg ctcttccctt ccagggaaga cggatcacgc ggccaagaac gagactcgca 360

aactgggcat ttctccgagc cgggctagag caagtagcga gactccgcgt gagagtggga 420

aagagcctta acaggcaacc atgttgccca gtgggttttc tgtgcctttg ggtgcggacc 480

aatgaggcgc gtggggcggg acttccgctt cgcctaggtg ttgtcgtccc tgctagtact 540

ccgggctgtg gggtcggtg cggatattca gtcatgaaat cagggtaggg acttctcccg 600

cagcgacgcg gctggcaaga ctgtttgtgt tgcgggggcc ggacttcaag agagaaagag 660

agagtgggca gacatcgaag ccaaacagca gtatcccgga agcactcatg caactttggt 720

ggcggccact cagttttctc tgccagtgtc tggtgatttt acaacgagat g 771

<210> 1231
<211> 190
<212> DNA
<213> Homo sapiens

<400> 1231

ccgcctcctg cgccgcccct tccgaggcta aatcggctgc gttcctctcg gaacgcgccg 60

cagaaggggt cctggtgacg agtcccgcgt tctctccttg aatccactcg ccagcccgcc 120

gccctctgcc gccgcaccct gcacacccgc ccctctcctg tgccaggaac ttgctactac 180

cagcaccatg 190

<210> 1232
<211> 208
<212> DNA
<213> Homo sapiens

<400> 1232

cgctccttcc ctgagctccc gggctccggc agcgcgctgg cggggcgccg cattgcacac 60

tctgggggcg ccgcagtgtt cgtgggatgg ggcagcgggc tgcagctggc ggccggaatc 120

cgcgcgcagc ccgggtgcaa gttctctcct gttgccctga gtgcccactc ccaggccctc 180

tgtatgagtg acacttcagt ctgccatg 208

<210> 1233
<211> 314
<212> DNA
<213> Homo sapiens

<400> 1233

cagtctctgc tttctttttc ctttcttcca gaaggagatt taaccatagt agaaagaatg 60

gagaactatt aactgccttt cttctgtggg ctgtgatttt cagaggggaa tgctaagagg 120

tgattttcaa tgttgggact caaaggtgaa gacactgaag gacagaattt ttggcagagg 180

aaagatcttc ttcggtcacc atacttgagt tagctctagg gaagtggagg tttccatttg 240

gaattctata gcttcttcca ggtcatagtg tctgcccccc accttccagt atctcctgat 300

atgcagcatg aatg 314

<210> 1234
<211> 65
<212> DNA
<213> Homo sapiens

<400> 1234

ctgtcctttc ccgggagtta gcgatccctc aacccctgca ctgcgctagt cctaaagagg 60

aaatg 65

<210> 1235
<211> 91
<212> DNA
<213> Homo sapiens

<400> 1235

gattctcttt tgtccacaga cagtcatctc aggagcagaa agaaaagagc tcccaaatgc 60

tatatctatt caggggctct caagaacaat g 91

<210> 1236
<211> 81
<212> DNA
<213> Homo sapiens

<400> 1236

actccttttc tcctaaccgt cccggccacc gctgcctcag cctctgcctc ccagcctctt 60

tctgagggaa aggacaagat g 81

<210> 1237
<211> 510
<212> DNA
<213> Homo sapiens

<400> 1237

aagcctttct ccattttgcg gtctaggaag tagcagaggc cccttcctgt agggagttgc 60

catggagacg cggtggggca ccgacggagt tctaatgacg gccgtgattg gtgcaggatc 120

ctgctaatct caggaaggcc cgtagagaag tgaggaaaac gtggtggggg gcatgcgcga 180

tctggtaggc ggtgctgccg tctgttgtac ctgagaggct tgcgcatgcc gacgcacgga 240

ttcgaggcgg ggagcatggg aagaagcggc caggagtatg acctgatcat tgcgaccacc 300

gctaggggaa gggaggagag ggtgtagaaa cggggacgag ggtgggggaa gggcaaggag 360

gcgctcgagc tggtgcgcgg agcatcctgg gagacgtagt ccagcgggag ggggaagtcg 420

aagactgcgc gtgctcagga gcgcggagcg gcccgctgag cgcagagggg cagacactgg 480

cctcagatac ctgacctggt accctctatg 510

<210> 1238
<211> 228
<212> DNA
<213> Homo sapiens

<400> 1238

cctcctcttt ttccgtctgc gtcgggagct cccgggcacg tgaggccgtg ccgcgtttac 60

tggcgggcgg gacggcctag ccgggcggcg cctcggagga agccgcggac cccttaggtg 120

ctgggccctt ggaaatcggc gcgtgggggg cggtgctcga gctgagcgcg agagggcggg 180

agagctcgtg ggtgcgaggg ggagcaggac gcccggccgg gcagcatg 228

<210> 1239
<211> 327
<212> DNA
<213> Homo sapiens

<400> 1239

cttcctcttt caacaacaaa tgtgtcagtt atcagcagga tccatgccgc cagagtaaag 60
ctttctaccc tttactccct gcaaagaaac aagagtgctt atcccagcta agctccaggg 120
taatgttatc atgacagctt caacttttag accacaggca aatgctttgt taaaactcta 180
tgctggtcat tcccttcagg atttggcact caccaacata cccttctttc aagtgaaaag 240
gcatctcttt taatggtcct gacctttgga ataggaagca tgtaccctgg acagagcact 300
tcaaactaga ggaaccataa atccatg 327

<210> 1240
<211> 48
<212> DNA
<213> Homo sapiens

<400> 1240
catccttttg cctgctcccg gcgaggggtg gctttgattt cggcgatg 48

<210> 1241
<211> 26
<212> DNA
<213> Homo sapiens

<400> 1241
cgtccccttt ccggccggtc cccatg 26

<210> 1242
<211> 305
<212> DNA
<213> Homo sapiens

<400> 1242

ccctcccctc cgcccgtcac cgcctccttg aagctgccgc tgtcgctgct gctcgttcga 60
gtcgcagatc cttgccagca cattacagaa tatttttgtt gaaccttctt gagaattcag 120
agaaactgct gagtgaccac tgaacgaaaa gatctaatct taaggcttac gcctcacttt 180
gatgcccagg ctggagtgct gtggctcaat cacagctcat cgcaacctcg acctcccggg 240
ctcaagtgat cctctcacct cagcgtcccg aacaggcgtg ttccatccac cacatcagaa 300
caatg 305

<210> 1243
<211> 414
<212> DNA
<213> Homo sapiens

<400> 1243

tcctctctcc acttcctgct actgcaggcc tctcctccga gaacagaggc caggtcatga 60
ctcactggct tcctgcaacc tgacgatggc ccagccagaa gacaaggcac ctgaagtccc 120
cacagagggg gtgaggtgaa caaagcagac aggacccctc taggggtcct cagcacccta 180
gagccactta ctcgcctgca gaggacatgg ggggtgtggc atgtgccaga gctggatacc 240
caggatgcgg aggcccttgt ggggctgtgg ccactaggga gtttcttggt cacaggacgt 300
gaccccagcc aggccctggt gttgaggtca ggacctttac caggagaagt caatacctac 360
cagatccaga agattcccag aggtgtgtcc ctggaatcct ccaacctctg catg 414

<210> 1244
<211> 144
<212> DNA
<213> Homo sapiens

<400> 1244

ccgccttttc ttcagcgtcc tacccgcggc actggctgcg agcgccgggc cacctgcgag 60
tgtgcgcagg gactctggac acccgcggcg gcgagctgag ggagcagtct ccacgaggac 120
ccaggcggac cctctggcgc catg 144

<210> 1245
<211> 313
<212> DNA
<213> Homo sapiens

<400> 1245

ccgcctttcc ggagcgcggg cgcgcggtgg cgggaatttc gcctgtttgc ggtttagacc 60
ccaaagattc ctgttggtgg tctgggtcac aggaggcagg tttcgggagc tggaaatgtg 120
agcgggtacg acaggcaccg cgggtaaccg acgccccggg tccttgctgc agccgggtac 180
gcgggatacc ggcaccccgc cttctccgcc cgagtgctgc caggcgtggg cctggaatct 240
cttcacacct tctctttgga gcccttaatg atacgacgaa ccccaagtgt ttcagaacat 300
gaagtaaaca atg 313

<210> 1246
<211> 367
<212> DNA
<213> Homo sapiens

<400> 1246

actcctttcc tttttccagt ggttatcgcg gcgcccaccg gcctctgatc tctgagtctt 60
ctccaaccca cagacgtttt ttgttgctct ggttccagga ccttctccac aactaggcca 120
ttttccctgc caggtgtcct ttttgacctc ttgacctctg actcaaaggg cctgctcccc 180
ctcatgtctt cggcctggag aagagccagc tcctgaagga ggcctttgat aaggccggcc 240
cggtccccaa gggcagagaa gatgtgaaga ggcttctgaa actacacaag gaccggttcc 300
gaggtgacct gcggtggatc ctcttctgtg cagacctgcc gtccctcatc caagaaggcc 360
ctcaatg 367

<210> 1247
<211> 384
<212> DNA
<213> Homo sapiens

<400> 1247

cgcccttccg gctcggcctt tagttagtga ccagctcctc ggcgttctgc agagcgtggg 60
tttcagcgag ttctacgtgc caggtccgcc cggtgccggc ttcctcgctg cccctggcgg 120
ctcgtcagcc cccactaccc ctgaacttgg tcccaatggc ggcccgcccc tccttcaccc 180
ggaccgtggg catctgggcc tcgccgaagc cgtcaaggtg gctgctcggg cttctagagc 240
ccgtgtccag ccctttgcca ccgaggcctg atcctctttt ctgccctaaa gaacttgccc 300
tgacagcctc tggctcccgc tcttgaggat cttgcttgtc caaacccaga agacagtgca 360
tgaagccagg ggacatccgc catg 384

<210> 1248
<211> 14
<212> DNA
<213> Homo sapiens

<400> 1248
ccgccttctc catg 14

<210> 1249
<211> 143
<212> DNA
<213> Homo sapiens

<400> 1249

ccgccctttt cttcgtagcc tccaagggag ctggaacaaa aaacgaaacc aaaacctgcc 60
tgctcgctcc tctccccatc gcctgcgttc cgctggttgt gggctttctg cggccgctga 120
gggcgcgtct cccctccgcc atg 143

<210> 1250
<211> 97

<212> DNA
<213> Homo sapiens

<400> 1250

caccctcctt cagcccaggc aaggcctggg gccctgggca gcctccaggt gcagtgccct 60

cccgtgggcc gcacccttgc cactgcccca gggcatg 97

<210> 1251
<211> 31
<212> DNA
<213> Homo sapiens

<400> 1251
ctttctcttc cttccacccc gagggaccat g 31

<210> 1252
<211> 91
<212> DNA
<213> Homo sapiens

<400> 1252

cgctctccgc ctcggcagtg ccagccgcca gtggtcgcac ttggagggtc tcgccgccag 60

tggaaggagc caccgccccc gcccgaccat g 91

<210> 1253
<211> 117
<212> DNA
<213> Homo sapiens

<400> 1253

atttcctttc cctttttcg ctcgtgtccc gccgggtggc gctcaccacc tccccggaac 60

acgcgagtct cctgtcgcgg ttccggtcgg aattaccccg tggagcacgc cgatatg 117

<210> 1254
<211> 63
<212> DNA
<213> Homo sapiens

<400> 1254

gagtctttcc ttagtaacct gggcgatagc tgtggatgtt tccaaggatt gtcttcagtc 60

atg 63

<210> 1255
<211> 211
<212> DNA
<213> Homo sapiens

<400> 1255

cttcctccat aacaagccaa acgccagacc gagagtgcct ccgtgcgcga gtgcccggtg 60

tgtgcgcgcc ggcgagagca ggggcccgcc cggctccccg cccgccgcgg cccgaactca 120

tgcagctccg agcgagcgag cggcgcccag cccagcgcct cggccgaacc cctccgcagc 180

aggctgcctg ctgtttcccg gggagatcat g 211

<210> 1256
<211> 49
<212> DNA
<213> Homo sapiens

<400> 1256
cctccttttc gccctcccac ccgcactgca gtctccagcc tgagccatg 49

<210> 1257
<211> 164
<212> DNA
<213> Homo sapiens

<400> 1257

aagccctttt cattgcagga gaagaggaca aagatactca gagagaaaaa gtaaaagacc 60

gaagaaggag gctggagaga ccaggatcct tccagctgaa caaagtcagc cacaaagcag 120

actagccagc cggctacaat tggagtcaga gtcccaaaga catg 164

<210> 1258
<211> 832
<212> DNA
<213> Homo sapiens

<400> 1258

cagccttctc actcctcact gagtccactc tgaacgtgct aaaatgggaa ggaggcggtg 60

ttttgctgat ctgttaaatt cttagtgaag tttccttgat ttccagtggc tgctgttgtt 120

tgagtttggt ttggagcaaa actgaggtag tcctaacatt tctgggactg aatccaggca 180

agagaaagaa gaaaaagaag aagaaaaaga ggaggaaaaa ggtagggaga aataaaggga 240

ggagagaagc acagtgaaag aaaaaaaaag tccctttcg acatcacatt cctgtgtttt 300
ccctcagcct ggaaaacata ttaatcccag tgcttttacg cccggaaaca aagagactaa 360
gccagactat gggggaaagg gagataagaa ggatcctgga actttaaaga gggaaagagt 420
gagattcaga aatcgccagg actggacttt aagggacgtc ctgtgtcagc acaaggact 480
ggcacacaca gacacacgag accgaggaga aactgcagac aaatggagat acaaagactt 540
agaaggacag ctcctttcac ctcatcctac ttgtccagaa ggtaaaaaga cacagccaga 600
aagaaaaggc atcggctcag ctctcagatc aggacaggct gtggatctgt ggcggtactc 660
tgaaagctgg agctgcagca caccccttt gtattgctca ccctcggtaa agagagagag 720
ggctgggagg aaaagtagtt catctaggaa actgtcctgg gaaccaaact tctgatttct 780
tttgcaaccc tctgcattcc atctctatga gccaccattg gattacacaa tg 832

<210> 1259
<211> 114
<212> DNA
<213> Homo sapiens

<400> 1259

cgacctcttt gcgcctgcgc ccccttgcc agtctttcgc cggcaaaagg aggacgtaga 60
aaaggggaca ccggaaactc actcttcacc cggaaatggt tattgaggaa catg 114

<210> 1260
<211> 16
<212> DNA
<213> Homo sapiens

<400> 1260
cgcccttctc aagatg 16

<210> 1261
<211> 440
<212> DNA
<213> Homo sapiens

<400> 1261

ctttccctgt gctgcccctg ccgcgcgatg gagaagagct cgagctgcga gagtcttggc 60
tcccagccgg cggcggctcg gccgcccagc gtggactcct tgtccagtta atgtgttaag 120
agccattgac atttgaagat catcagaagt gaagataaaa catctcaaaa attataattg 180
cctccacttc tcattcagag aattcagtgc atacaaaatc agcttctgtt gtatcatcag 240
attccatttc aacttctgcc gacaactttt ctcctgattt gaggagggag tctcgctcta 300
tcccctaggc tggagtgcat tggcgccatc tcggctcatt tgcaacctct gtctcccggg 360
ttcaagcgat tctcctgcct cagcttcccg aggagctggg attacaggtc ctgagggagt 420
ctaacaagtt agcagaaatg 440

<210> 1262
<211> 171
<212> DNA
<213> Homo sapiens

<400> 1262

cgccctctag ctgcgctcgg ctgagtcagt cagtctgtcg gagtctgtcc tcggagcagg 60
cggagtaaag ggacttgagc gagccagttg ccggattatt ctatttcccc tccctctctc 120
ccgccccgta tctcttttca cccttctccc accctcgctc gcgtagccat g 171

<210> 1263
<211> 347
<212> DNA
<213> Homo sapiens

<400> 1263

gtccctccct ctccgcacta gctgtctgcc ctgccctgcc gtaggagatg ggctgggagc 60
ctcccacgct ctccagctca ctcggcaggc agcggggacc agggctggca ggttaagcct 120
ctgggggtgg atcctgaaag gtggtccagc cgcctggccc tgcgtgggac cctccacctg 180
gcagcagaca gggtctcgct ctgtcacaca ggctggagtg cagtggtgtg atcttggctc 240
atcgtaacct ccacctcccg ggttcaagtg attctcatgc ctcagcctcc cgagtagctg 300
ggattacagg tggtgacttc caagagtgac tccgtcggag gaaaatg 347

<210> 1264
<211> 240
<212> DNA
<213> Homo sapiens

<400> 1264

gctcctccct gccgcctcct ctcagtggat ggttccaggc accctgtctg gggcagggag 60

ggcacaggcc tgcacatcga aggtggggtg ggaccaggct gcccctcgcc ccagcatcca 120

agtcctccct tgggcgcccg tggccctgca gactctcagg gctaaggtcc tctgttgctt 180

tttggttcca ccttagaaga ggctccgctt gactaagagt agcttgaagg aggcaccatg 240

<210> 1265
<211> 405
<212> DNA
<213> Homo sapiens

<400> 1265

atttcttttc cccattttaa atgcaaagca agacttgtga atcatagtgt ctctgctcct 60

gggattcaga ccaaatttcc ccccaaaatt ctcaggctat ttgtttgaat acctgcttac 120

agtggtacac aatgggcagc tttgagaaga aaaattgata atcttcacgg aagagtaatt 180

tgaatgaaat tacacttgac agcctgtctc caagcaaaca agaggaacga gggagcctga 240

gctaagctct gaggacttgc ccaagccact gctgttggag cttcccagga aaaaaaaaaa 300

aaaaaaaaaa aaaaaaaaaa aaaaaaaaac accagttttt ccaacatcta attgagcttt 360

tgattaattc cgtgtaccag attctactga agaaaggtag ccatg 405

<210> 1266
<211> 163
<212> DNA
<213> Homo sapiens

<400> 1266

ctccctcttt gtgcgtctcg cgccgccgcc gcccgccgcg tgagaggacg ggctccgcgc 60

gctccggcag cgcattcggg tcccctcccc ccgggaggct tgcgaaggag aagccgccgc 120

agaggaaaag caggtgccgg tgcctgtccc cgggggcgcc atg 163

<210> 1267
<211> 207
<212> DNA
<213> Homo sapiens

<400> 1267

ccgcccctcg cgacccagag ggctgctggc tggctaagtc cctcccgctc ccggctctcg 60

cctcactagg agcggctctc ggtgcagcgg gacagggcga agcggcctgc gcccacggag 120

cgcgcgacac tgcccggaag ggaccgccac ccttgccccc tcagctgccc actcgtgatt 180

tccagcggcc tccgcgcgcg cacgatg 207

<210> 1268
<211> 327
<212> DNA
<213> Homo sapiens

<400> 1268

ctgccctctt gcgtgccccg gccacccccg ggcggcttgt agccggtgcg cggggtggct 60

ggggctacgt gcagagctgt cgcggagccg gaacagcagc ggtgaagccc ctcggctcgg 120

ccgagaccgc cgtgcccatt gctcgcctcg gttgccgccg ctttagccgc agccgctgct 180

gccgccgccg ggggagaggc agcctattgt ctttctccgc ggcgaaggtg aggagctgtc 240

tcggctcggc ccgcggggga gccccgggag ccgcacggag atggaggagg acatctggac 300

agtgagcagg aggcgcctcg gcccatg 327

<210> 1269
<211> 183
<212> DNA
<213> Homo sapiens

<400> 1269

cgccctctcc ccgcctcctt ttcgggcgtc ccgaggccgc tccccaaccg acaaccaaga 60

ccccgcaggc cacgcagccc tggagccgag gccccccgac ggcggaggcg cccgcgggtc 120

ccctacagcc aaggtccctg agtgccagag gtggtggtgt tgcttatctt ctggaacccc 180

atg 183

<210> 1270
<211> 106
<212> DNA
<213> Homo sapiens

<400> 1270

ctccctctca accacaataa caggcggagg gtcggcgtag gtactttgaa ctcaagtaaa 60

caaaagggaa gattttctcg ttgatactgg agactgcaca acaatg 106

<210> 1271
<211> 74
<212> DNA
<213> Homo sapiens

<400> 1271

agatcttttc cagcagctgc tgcctgccag agaggcgcct tcagagaccc agcgcttaca 60

caatacccac catg 74

<210> 1272
<211> 62
<212> DNA
<213> Homo sapiens

<400> 1272

cctcctctcc ggcggcggcg gcggcggcgg cgggcggagt gagctgcgga gcctggaata 60

tg 62

<210> 1273
<211> 233
<212> DNA
<213> Homo sapiens

<400> 1273

gggcctctct tgtttattta tttattttcc gtgggtgcct ccgagtgtgc gcgcgctctc 60

gctacccggc ggggaggggg tgggggagg gcccgggaaa agggggagtt ggagccgggg 120

tcgaaacgcc gcgtgacttg taggtgagag aacgccgagc cgtcgccgca gcctccgccg 180

ccgagaagcc cttgttcccg ctgctgggaa ggagagtctg tgccgacaag atg 233

<210> 1274
<211> 76
<212> DNA
<213> Homo sapiens

<400> 1274

cagcctctac cccgctccgg atccgggatc tgagcgccgg ccgcggtgcc caggcactcc 60

cttggcgggc cggatg 76

<210> 1275
<211> 205
<212> DNA
<213> Homo sapiens

<400> 1275

cggccttctc ggcttctcca gcttcggtag gagaggatcc ggcgccgaat cactgactgg 60

cacaggtgtt gggatagtgt ctcacttggt cacccaggct ggagtgcagt ggcgcaatct 120

tagctctcta cagcgtcgat cttcctcctg ggctcaagca attctcctgc ttcatcctcc 180

tgagtaccta ggactacaga aaatg 205

<210> 1276
<211> 957
<212> DNA
<213> Homo sapiens

<400> 1276

```
cagtcttttc actgcagctg aatgagttgt ggcgcccaca atgctcccat gacaaggagc      60
tgacaagttc cattttccgt cgcgggcatc ttggaatcat gactcccaca atgccttggg     120
cacttggtcg acagtggggc cgcctctgaa aaaaaaatgt gagaggttgg tactaagaag     180
tgcctttcct gacgtctctg ctgcttggaa ccgcttctag agcagtctct gcttttgcct     240
tgcttgctgc cagctagact gtgacgacag cacatccacc ctccacctct agcccagaca     300
cccccatttc tacttataat caagagaaaa gctctaagta tctggcattg ccctaggctg     360
ctttagtgtt aaaagaaaag tttgctgaaa aagtaagata tcttctgcca ggaaatcaag     420
gaggaaaaaa aaaatcattt tctcgatttt gctctaaact gctgcatctg tctatgccaa     480
actaatcaat accgattgca ccaccaaact ccattgcaaa ttcagctgtg aggagattcc     540
ctttcagaca actttgctga aagcagcttg gaaattcggt gtcgaagggt ctgccacgtt     600
ttcatgcttg cattttgggc tccaaattgg cactgggaag gggttactga gagcacaagg     660
ctgataccag gccctacttt taaacgttca tctacttaca atcctagtat ttctctaaaa     720
accaaaacct ctttgaatta acagtttcat gctgtgaatt tctagtggga gatcttttcc     780
ttgatattga cgacacaatt ttccatgtac ttttaaagca gggagtgggg aaaagtattt     840
tgaggggaca ttttcatcat cagttcagct ttttttttt ggttgttgct ctttttttggg     900
ggggttgggt ttgttggttt cactgaaaca tttaactacc tgtaaaatct aaacatg        957
```

<210> 1277
<211> 425
<212> DNA
<213> Homo sapiens

<400> 1277

```
cagccttttg ctctttcctt tcattaaaca aacaggagat cctgaaacct ggaccctgtg      60
caagctgcag cgccaggagg aggcagcgga ggaagcagag cgcgggatgg gcgcccagcg     120
gcatctgtga tcccgcgcac ctccgcccca cgggcgcgcg cacaaacacg gacacacaca     180
tacacacact cgcgcacaca ctcgcacaaa cacacactcg tacacgcccg cgccgctcgc     240
tcgccggctt gctctcccac gcaagcggaa tgcagcagcg cctggagagc gtgtctcgga     300
ccgccgcctg aatgtacctc gctcccggga gccggacggc ccagtagggc gcactggagg     360
acgctccgct gcgggagcct ggacagtttt tgacggtgca gtcttgctat atggtgtgag     420
aaatg                                                                 425
```

<210> 1278

<211> 803
<212> DNA
<213> Homo sapiens

<400> 1278

```
ctgtctttgc ttcatcatct gaaggtaaaa ttttccagat acggcagacg gctttcagag      60
tacaataaac agggaatgag aactatttac atggaagttt ctttctcatg atgcggtgga     120
gaagcctcgg ccacttggtt ctgccagatg ttcctggggt tactgtaaat gggaaggaca     180
ggcagagcta aacaaggttt atcatttaaa agtgcctgtg tgaagtcact tttgctggaa     240
aactgcagct tgggagcttt ctttgtattc acatcccact cttctgtcaa gtacacttta     300
ccctgacctt atgagtggat gaagatacct cagttgtctg actttgccaa ttgcttaatt     360
tcagaattta aaaaggggaa agaaaaacat cctgctaaaa tatgaacatc tgagtgtctt     420
attttccaac atcgtcaata gctgtgagcg tcagcattaa atattctccc aaggagtgcc     480
atgatattga agtcacttta ttaataacag ctgtatctgc aaaacagtca agagactcgg     540
acgttgaaag ccagagatga cactgagcat gcttttattg cggcctacca tctttaagtg     600
ggacatattg attgatgagt gattgcctgt ccatacactc tctcatcatc ctgttccttg     660
gattggactt cactaagcaa tttatcactc accttcagac ttacatgtgg gagttttcac     720
aacagtagtt ttggaatcat tagaacttgg attgatttca tcatttaaca gaaacaaaca     780
gcccaaatta ctttatcacc atg                                             803
```

<210> 1279
<211> 123
<212> DNA
<213> Homo sapiens

<400> 1279

```
gcgcctttcg cacgacttgg agttacggtt tatctgatac cgcggtaccc ctacgcaagc      60
aagcccacat cgacacacat tcacacacgc ccttcagcac cccctcccag caccacgacc     120
atg                                                                   123
```

<210> 1280
<211> 320
<212> DNA
<213> Homo sapiens

<400> 1280

cctcctcctt tccctgggtg cccacatgaa cagagacacc aggatgctct cctgagacca 60
cagcaactgc agaagctgaa gacatttcca gaagttcaag cttccaccct ctgcaggtcc 120
ccactgagct gggacccagg tcatccaccc caccccaaat ccctggatag gaaacccctt 180
tctcctcctg ctccttgtcc ccttcatccc tgccgcccag catcctactg gcctcagcac 240
ctgtggccag accgtccaag atcctctgaa ggcccagctc ttgctgtcca ccccggcagt 300
aggcaggcag cctggccatg 320

<210> 1281
<211> 240
<212> DNA
<213> Homo sapiens

<400> 1281

gcctccctcc cccgcagctg gggccagcgg tgccaagcgc agctggacga gcggcagcag 60
ctgggcgagt gacagccccg gctccgcgcg ccgcggccgc cagagccggc gcaggggaag 120
cgcccgcggc cccgggtgca gcagcggccg ccgcctcccg cgcctccccg gcccgcagcc 180
cgcggtcccg cggccccggg gccggcacct ctcgggctcc ggctccccgc gcgcaagatg 240

<210> 1282
<211> 45
<212> DNA
<213> Homo sapiens

<400> 1282
ccctccctcc gcgcggggac ccctggcggg cggcaggagg acatg 45

<210> 1283
<211> 242
<212> DNA
<213> Homo sapiens

<400> 1283

ccttctctgg ttccctgacc tcagtgagac agcagccggc ctggggacct gggggagaca 60
cggaggaccc cctggctgga gctgacccac agagtaggga atcatggctg gagaattgga 120
tagcagagta atgtttgacc tctggaaaca tcacttacag ggcttccggt caaaattcac 180
taggtaggag ggtcatcagc tgggaagaac cggcgcctgg gaaacctggc tggataggta 240
tg 242

<210> 1284
<211> 33
<212> DNA
<213> Homo sapiens

<400> 1284

cgttctttct ttgctgcgtc tactgcgaga atg 33

<210> 1285
<211> 33
<212> DNA
<213> Homo sapiens

<400> 1285
cggcctctac cggcgggatt tgatggcgtg atg 33

<210> 1286
<211> 64
<212> DNA
<213> Homo sapiens

<400> 1286

**acttcctttt cctgtggcag cagccgggct gagaggagcg tggctgtctc ctctctccgc 60**

**catg 64**

<210> 1287
<211> 82
<212> DNA
<213> Homo sapiens

<400> 1287

**tggccctttt cccaccccct agcgccgctg ggcctgcagg tctctgtcga gcagcggacg 60**

**ccggtctctg ttccgcagga tg 82**

<210> 1288
<211> 26
<212> DNA
<213> Homo sapiens

<400> 1288
aattctcttt cccatcttgc aagatg 26

<210> 1289
<211> 27
<212> DNA
<213> Homo sapiens

<400> 1289
cttcctcttt ttccggctgg aaccatg 27

<210> 1290
<211> 34
<212> DNA
<213> Homo sapiens

<400> 1290
ctttcctttc tctctcctcc cgccgcccaa gatg 34

<210> 1291
<211> 25
<212> DNA
<213> Homo sapiens

<400> 1291
ctttctcttc ctgctctcca tcatg 25

<210> 1292
<211> 95
<212> DNA
<213> Homo sapiens

<400> 1292

**cggcctctcg gctttcggct cggaggaggc caaggtgcaa cttccttcgg tcgtcccgaa 60**

**tccgggttca tccgacacca gccgcctcca ccatg 95**

<210> 1293
<211> 59
<212> DNA
<213> Homo sapiens

<400> 1293
gcttcctttc cgctcggctg ttttcctgcg caggagccgc agggccgtag gcagccatg 59

<210> 1294
<211> 34
<212> DNA
<213> Homo sapiens

<400> 1294
acttcctttt ttcttttttc cggcgttcaa gatg 34

<210> 1295
<211> 44
<212> DNA
<213> Homo sapiens

<400> 1295
cgttctctct ttccggacct ggccgagcag gaggcgccat catg 44

<210> 1296
<211> 43
<212> DNA
<213> Homo sapiens

<400> 1296
acttcctttt gcgggtggcg gcgaacgcgg agagcacgcc atg 43

<210> 1297
<211> 35
<212> DNA
<213> Homo sapiens

<400> 1297
agctctttcc tttcgctgct gcggccgcag ccatg 35

<210> 1298
<211> 51
<212> DNA
<213> Homo sapiens

<400> 1298
gcctctttcc tttcggccgg aaccgccatc ttccagtaat tcgccaaaat g 51

<210> 1299
<211> 30
<212> DNA
<213> Homo sapiens

<400> 1299
acctcccttt ctaactccgc tgccgccatg 30

<210> 1300
<211> 19
<212> DNA
<213> Homo sapiens

<400> 1300
agaccctttt cacaagatg 19

<210> 1301
<211> 295
<212> DNA
<213> Homo sapiens

<400> 1301

cgctcttcct ctttccctaa gcagcctgag ggttgactgg attggtgagg cccgtgtggc 60

tacttctgtg gaagcagtgc tgtagttact ggaagataaa agggaaagca agcccttggt 120

gggggaaagt atggctgcga tgatggcatt tcttaggaca cctttggatt aataatgaaa 180

acaactactc tctgagcagc tgttcgaatc atctgatatt tatactgaat gagttactgt 240

aagtacgtat tgacagaatt acactgtact ttcctctagg tgatctgtga aaatg 295

<210> 1302
<211> 51
<212> DNA
<213> Homo sapiens

<400> 1302
ttctctcttt cttttcgcca tcttttgtct ttccgtggag ctgtcgccat g 51

<210> 1303
<211> 49
<212> DNA
<213> Homo sapiens

<400> 1303
agttctcttc ccttttgcgg ccatcaccga agcgggagcg gccaaaatg 49

<210> 1304

<211> 43
<212> DNA
<213> Homo sapiens

<400> 1304
ctttcctttt tgctggtagg gccgggtggt tgctgccgaa atg 43

<210> 1305
<211> 81
<212> DNA
<213> Homo sapiens

<400> 1305

aagtctttcc tttctcgttc cccggccatc ttagcggctg ctgttggttg ggggccgtcc 60

cgctcctaag gcaggaagat g 81

<210> 1306
<211> 30
<212> DNA
<213> Homo sapiens

<400> 1306
ccttcctttt tcgtctgggc tgccaacatg 30

<210> 1307
<211> 49
<212> DNA
<213> Homo sapiens

<400> 1307
cttcctcttt ccgtctcagg tcgccgctgc gaagggagcc gccgccatg 49

<210> 1308
<211> 60
<212> DNA
<213> Homo sapiens

<400> 1308
cagccccttt ctcttccggt tctaggcgct tcgggagccg cggcttatgg tgcagacatg 60

<210> 1309
<211> 34
<212> DNA
<213> Homo sapiens

<400> 1309
cgctcttcct ttccaacttg gacgctgcag aatg 34

<210> 1310
<211> 65
<212> DNA
<213> Homo sapiens

<400> 1310

ccgtcccttc tctcttcctc ggcgctgcct acggaggtgg cagccatctc cttctcggca 60

tcatg 65

<210> 1311
<211> 71
<212> DNA
<213> Homo sapiens

<400> 1311

cgtccttctc ttaccgccat cttggctcct gtggaggcct gctgggaacg ggacttctaa 60

aaggaactat g 71

<210> 1312
<211> 44
<212> DNA
<213> Homo sapiens

<400> 1312
ccttctcttc cggtctttct ggtctcggcc gcagaagcga gatg 44

<210> 1313
<211> 44
<212> DNA
<213> Homo sapiens

<400> 1313
gcgtctcttc ctttctgggc tcggacctag gtcgcggcga catg 44

<210> 1314
<211> 113
<212> DNA
<213> Homo sapiens

<400> 1314

cgttcttttt cgtccttttc cccggttgct gcttgctgtg agtgtctcta gggtgatacg 60

tgggtgagaa aggtcctggt ccgcgccaga gcccagcgcg cctcgtcgcc atg 113

<210> 1315
<211> 57
<212> DNA
<213> Homo sapiens

<400> 1315
ccctcctctt cctttctccg ccatcgtggt gtgttcttga ctccgctgct cgccatg 57

<210> 1316
<211> 84
<212> DNA
<213> Homo sapiens

<400> 1316

aggcccttct ctcgccaggc gtcctcgtgg aagtgacatc gtctttaaac cctgcgtggc 60

aatccctgac gcaccgccgt gatg 84

<210> 1317
<211> 105
<212> DNA
<213> Homo sapiens

<400> 1317

cggtccttcc gaggaagcta aggctgcgtt ggggtgaggc cctcacttca tccggcgact 60

agcaccgcgt ccggcagcgc cagccctaca ctcgcccgcg ccatg 105

<210> 1318
<211> 81
<212> DNA
<213> Homo sapiens

<400> 1318

ccttcctttt cctccctgtc gccaccgagg tcgcacgcgt gagacttctc cgccgcctcc 60

gccgcagacg ccgccgcgat g 81

<210> 1319
<211> 37
<212> DNA
<213> Homo sapiens

<400> 1319
acttcctttc ctttcagcgg agcgcggcgg caagatg 37

<210> 1320
<211> 31
<212> DNA
<213> Homo sapiens

<400> 1320
ccgccctttt ggctctctga ccagcaccat g 31

<210> 1321
<211> 30
<212> DNA
<213> Homo sapiens

<400> 1321
ggtcctcttt ccttgcctaa cgcagccatg 30

<210> 1322
<211> 30
<212> DNA
<213> Homo sapiens

<400> 1322
gattctcttc cgtcgcagag tttcgccatg 30

<210> 1323
<211> 115
<212> DNA
<213> Homo sapiens

<400> 1323

ttttcttccc agttaaaagt gttggcccgc ggcgcgcggc ctcttcctgt ctgtaccagg 60

gcggcgcgtg gtctacgccg agtgacagag acgctcaggc tgtgttctca ggatg 115

<210> 1324
<211> 48
<212> DNA
<213> Homo sapiens

<400> 1324
ggccctcttt tccgtggcgc ctcggaggcg ttcagctgct tcaagatg 48

<210> 1325
<211> 51
<212> DNA
<213> Homo sapiens

<400> 1325
gggtctcttc ctaagccggc gctcggcaag ttctcccagg agaaagccat g 51

<210> 1326
<211> 30
<212> DNA
<213> Homo sapiens

<400> 1326
gtttctcttt ccagccagcg ccgagcgatg 30

<210> 1327
<211> 63
<212> DNA
<213> Homo sapiens

<400> 1327

gcgcctcttt ctcagtgacc gggtggtttg cttaggcgca gacggggaag cggagccaac 60

atg 63

<210> 1328
<211> 48
<212> DNA
<213> Homo sapiens

<400> 1328
gctccttcct ttccagcccc ggtaccggac cctgcagccg cagagatg 48

<210> 1329
<211> 36
<212> DNA
<213> Homo sapiens

<400> 1329
ctgccccttt ctttttttca ggcggccggg aagatg 36

<210> 1330
<211> 89
<212> DNA
<213> Homo sapiens

<400> 1330

aggcctcttt ccctgccgcc gccgagtcgc gcggaggcgg aggcttgggt gcgttcaaga 60

ttcaacttca cccgtaaccc accgccatg 89

<210> 1331
<211> 35
<212> DNA
<213> Homo sapiens

<400> 1331
cgctctcctt tcgttgcctg atcgccgcca tcatg 35

<210> 1332
<211> 28
<212> DNA
<213> Homo sapiens

<400> 1332
cgatctcttc tgaggatccg gcaagatg 28

<210> 1333
<211> 103
<212> DNA
<213> Homo sapiens

<400> 1333

cgtcctcttt ccgccatctt tccgcgccgg tgagtagcac tctctgagag ctccaatttc 60

atccgtctgc catcggcgcc atcctgcaat ctaagccaca atg 103

<210> 1334
<211> 61
<212> DNA
<213> Homo sapiens

<400> 1334

ctttcctttt ccggttgcgg cgccgcgcgg tgaggttgtc tagtccacgc tcggagccat 60

g 61

<210> 1335
<211> 42
<212> DNA
<213> Homo sapiens

<400> 1335
cgttcccttt cccctggctg gcagcgcgga ggccgcacga tg 42

<210> 1336
<211> 136
<212> DNA
<213> Homo sapiens

<400> 1336

ccaccccttt ctttttgagg aagacgcggt cgtaagggct gaggattttt ggtccgcacg 60

ctcctgctcc tgactcaccg ctgttcgctc tcgccgagga acaagtcggt caggaagccc 120

gcgcgcaaca gccatg 136

<210> 1337
<211> 59
<212> DNA
<213> Homo sapiens

<400> 1337
gcttcctttc tctctcgcgc gcggtgtggt ggcagcaggc gcagcccagc ctcgaaatg 59

<210> 1338
<211> 40
<212> DNA
<213> Homo sapiens

<400> 1338
gcttctctct ttcgctcagg cccgtggcgc cgacaggatg 40

<210> 1339
<211> 67
<212> DNA
<213> Homo sapiens

<400> 1339

gcttcctttt tgtccgacat cttgacgagg ctgcggtgtc tgctgctatt ctccgagctt 60

cgcaatg 67

<210> 1340
<211> 34
<212> DNA
<213> Homo sapiens

<400> 1340
ccgtctcctc tctccggtcc gtgcctccaa gatg 34

<210> 1341

<211> 42
<212> DNA
<213> Homo sapiens

<400> 1341
cgctcctttc cggcggtgac gacctacgca cacgagaaca tg 42

<210> 1342
<211> 38
<212> DNA
<213> Homo sapiens

<400> 1342
actcctctcc gccagaccgc cgccgcgccg ccatcatg 38

<210> 1343
<211> 41
<212> DNA
<213> Homo sapiens

<400> 1343
gcttcttcct tttacctcgt tgcactgctg agagcaagat g 41

<210> 1344
<211> 30
<212> DNA
<213> Homo sapiens

<400> 1344
cgttcttctt ttccgacaaa acaccaaatg 30

<210> 1345
<211> 30
<212> DNA
<213> Homo sapiens

<400> 1345
cgttcttctt ttccgacaaa acaccaaatg 30

<210> 1346
<211> 36
<212> DNA
<213> Homo sapiens

<400> 1346
gggtcttctt ccttctcgcc taacgccgcc aacatg 36

<210> 1347
<211> 42
<212> DNA
<213> Homo sapiens

<400> 1347
ctctctttcc ggtgtggagt ctggagacga cgtgcagaaa tg 42

<210> 1348
<211> 48
<212> DNA

<213> Homo sapiens

<400> 1348
gcgcctcttt cccttcggtg tgccactgaa gatcctggtg tcgccatg 48

<210> 1349
<211> 38
<212> DNA
<213> Homo sapiens

<400> 1349
tagtctcttt tccggttagc gcggcgtgag aagccatg 38

<210> 1350
<211> 50
<212> DNA
<213> Homo sapiens

<400> 1350
tcctctttcc ctcggagcgg gcggcggcgt tggcggcttg tgcagcaatg 50

<210> 1351
<211> 32
<212> DNA
<213> Homo sapiens

<400> 1351
cctcctcctt ttccaagcgg ctgccgaaga tg 32

<210> 1352
<211> 42
<212> DNA
<213> Homo sapiens

<400> 1352
cagcccttcc gccacggccg tctctggaga gcagcagcca tg 42

<210> 1353
<211> 40
<212> DNA
<213> Homo sapiens

<400> 1353
gtttctttct ttccgcgccg atagcgctca cgcaagcatg 40

<210> 1354
<211> 51
<212> DNA
<213> Homo sapiens

<400> 1354
tcgcctttct ctcggcctta gcgccatttt tttggaaacc tctgcgccat g 51

<210> 1355
<211> 52
<212> DNA
<213> Homo sapiens

<400> 1355
cgctctctct tccacaggag gcctacacgc cgccgcttgt gctgcagcca tg 52

<210> 1356
<211> 46
<212> DNA
<213> Homo sapiens

<400> 1356
ggttctcttt tcctccttgg ctgtctgaag atagatcgcc atcatg 46

<210> 1357
<211> 58
<212> DNA
<213> Homo sapiens

<400> 1357
tttcctcttt cggccgcgct ggtgaacagg taggtcatcc ttgcggcctt gcggcatg 58

<210> 1358
<211> 33
<212> DNA
<213> Homo sapiens

<400> 1358
cttcctcttc cggggacgtt gtctgcaggt atg 33

<210> 1359
<211> 32
<212> DNA
<213> Homo sapiens

<400> 1359
gtttcctctt ttaccaagga cccgccaaca tg 32

<210> 1360
<211> 85
<212> DNA
<213> Homo sapiens

<400> 1360

ctgtcttttc cgtgctacct gcagaggggt ccatacggcg ttgttctgga ttcccgtcgt 60

aacttaaagg gaaattttca caatg 85

<210> 1361
<211> 91
<212> DNA
<213> Homo sapiens

<400> 1361

cttctctctc ggcgtttccg ctgtcagggc cctgcggtgt gactcgcggg ctcagctggt 60

ccggccgtag cacctccgcg ccgtcgccat g 91

<210> 1362
<211> 62
<212> DNA
<213> Homo sapiens

<400> 1362

cgctctcctc ctctcacgga aaggtcgcgg cctgtggccc tgcgggcagc cgtgccgaga 60

tg 62

<210> 1363
<211> 241
<212> DNA
<213> Homo sapiens

<400> 1363

cactcccttc caaaagcatg acaggcagaa agcagagaag ggccaggact ggctgagggc 60

ggggagctgg gcctctgggg tggacacacc cttggtcaca ttgtgagggt agcttggttg 120

gccagtccca ccactgcagt gaccacagtt gtgttgggct cacaccagtg aaccgaagct 180

ctggattctg agagtctgag gattccgtga agatctcaga cttgggctca gagcaaggat 240

g 241

<210> 1364
<211> 1812
<212> DNA
<213> Artificial Sequence

<220>
<223> PpLuc(GC) agA64

<400> 1364

gggagaaagc ttgaggatgg aggacgccaa gaacatcaag aagggcccgg cgcccttcta 60
cccgctggag gacgggaccg ccggcgagca gctccacaag gccatgaagc ggtacgccct 120
ggtgccgggc acgatcgcct tcaccgacgc ccacatcgag gtcgacatca cctacgcgga 180
gtacttcgag atgagcgtgc gcctggccga ggccatgaag cggtacggcc tgaacaccaa 240
ccaccggatc gtggtgtgct cggagaacag cctgcagttc ttcatgccgg tgctgggcgc 300
cctcttcatc ggcgtggccg tcgccccggc gaacgacatc tacaacgagc gggagctgct 360
gaacagcatg gggatcagcc agccgaccgt ggtgttcgtg agcaagaagg gcctgcagaa 420
gatcctgaac gtgcagaaga agctgcccat catccagaag atcatcatca tggacagcaa 480
gaccgactac cagggcttcc agtcgatgta cacgttcgtg accagccacc tcccgccggg 540
cttcaacgag tacgacttcg tcccggagag cttcgaccgg gacaagacca tcgccctgat 600
catgaacagc agcggcagca ccggcctgcc gaaggggggtg gccctgccgc accggaccgc 660
ctgcgtgcgc ttctcgcacg cccgggaccc catcttcggc aaccagatca tcccggacac 720
cgccatcctg agcgtggtgc cgttccacca cggcttcggc atgttcacga ccctgggcta 780
cctcatctgc ggcttccggg tggtcctgat gtaccggttc gaggaggagc tgttcctgcg 840
gagcctgcag gactacaaga tccagagcgc gctgctcgtg ccgaccctgt tcagcttctt 900
cgccaagagc accctgatcg acaagtacga cctgtcgaac ctgcacgaga tcgccagcgg 960
gggcgccccg ctgagcaagg aggtgggcga ggccgtggcc aagcggttcc acctcccggg 1020
catccgccag ggctacggcc tgaccgagac cacgagcgcg atcctgatca cccccgaggg 1080
ggacgacaag ccgggcgccg tgggcaaggt ggtcccgttc ttcgaggcca aggtggtgga 1140
cctggacacc ggcaagaccc tgggcgtgaa ccagcggggc gagctgtgcg tgcgggggcc 1200
gatgatcatg agcggctacg tgaacaaccc ggaggccacc aacgccctca tcgacaagga 1260
cggctggctg cacagcggcg acatcgccta ctgggacgag gacgagcact tcttcatcgt 1320
cgaccggctg aagtcgctga tcaagtacaa gggctaccag gtggcgccgg ccgagctgga 1380
gagcatcctg ctccagcacc ccaacatctt cgacgccggc gtggccgggc tgccggacga 1440
cgacgccggc gagctgccgg ccgcggtggt ggtgctggag cacggcaaga ccatgacgga 1500
gaaggagatc gtcgactacg tggccagcca ggtgaccacc gccaagaagc tgcggggcgg 1560
cgtggtgttc gtggacgagg tcccgaaggg cctgaccggg aagctcgacg cccggaagat 1620
ccgcgagatc ctgatcaagg ccaagaaggg cggcaagatc gccgtgtaag actagttata 1680
agactgacta gcccgatggg cctcccaacg ggccctcctc ccctccttgc accgagatta 1740
atagatctaa aaaaaaaaaa aaaaaaaaaa aaaaaaaaaa aaaaaaaaaa aaaaaaaaaa 1800
aaaaaaaaaa aa 1812

<210> 1365
<211> 1914
<212> DNA
<213> Artificial Sequence

<220>
<223> 32L PpLuc(GC) ag A64 C30 histoneSL

<400> 1365

```
ggggcgctgc ctacggaggt ggcagccatc tccttctcgg catcaagctt gaggatggag      60
gacgccaaga acatcaagaa gggcccggcg cccttctacc cgctggagga cgggaccgcc     120
ggcgagcagc tccacaaggc catgaagcgg tacgccctgg tgccgggcac gatcgccttc     180
accgacgccc acatcgaggt cgacatcacc tacgcggagt acttcgagat gagcgtgcgc     240
ctggccgagg ccatgaagcg gtacggcctg aacaccaacc accggatcgt ggtgtgctcg     300
gagaacagcc tgcagttctt catgccggtg ctgggcgccc tcttcatcgg cgtggccgtc     360
gccccggcga acgacatcta caacgagcgg gagctgctga acagcatggg gatcagccag     420
ccgaccgtgg tgttcgtgag caagaagggc ctgcagaaga tcctgaacgt gcagaagaag     480
ctgcccatca tccagaagat catcatcatg gacagcaaga ccgactacca gggcttccag     540
tcgatgtaca cgttcgtgac cagccacctc ccgccgggct tcaacgagta cgacttcgtc     600
ccggagagct tcgaccggga caagaccatc gccctgatca tgaacagcag cggcagcacc     660
ggcctgccga agggggtggc cctgccgcac cggaccgcct gcgtgcgctt ctcgcacgcc     720
cgggacccca tcttcggcaa ccagatcatc ccggacaccg ccatcctgag cgtggtgccg     780
```

ttccaccacg gcttcggcat gttcacgacc ctgggctacc tcatctgcgg cttccgggtg 840
gtcctgatgt accggttcga ggaggagctg ttcctgcgga gcctgcagga ctacaagatc 900
cagagcgcgc tgctcgtgcc gaccctgttc agcttcttcg ccaagagcac cctgatcgac 960
aagtacgacc tgtcgaacct gcacgagatc gccagcgggg gcgccccgct gagcaaggag 1020
gtgggcgagg ccgtggccaa gcggttccac ctcccgggca tccgccaggg ctacggcctg 1080
accgagacca cgagcgcgat cctgatcacc cccgaggggg acgacaagcc gggcgccgtg 1140
ggcaaggtgg tcccgttctt cgaggccaag gtggtggacc tggacaccgg caagaccctg 1200
ggcgtgaacc agcggggcga gctgtgcgtg cgggggccga tgatcatgag cggctacgtg 1260
aacaacccgg aggccaccaa cgccctcatc gacaaggacg gctggctgca cagcggcgac 1320
atcgcctact gggacgagga cgagcacttc ttcatcgtcg accggctgaa gtcgctgatc 1380
aagtacaagg gctaccaggt ggcgccggcc gagctggaga gcatcctgct ccagcacccc 1440
aacatcttcg acgccggcgt ggccgggctg ccggacgacg acgccggcga gctgccggcc 1500
gcggtggtgg tgctggagca cggcaagacc atgacggaga aggagatcgt cgactacgtg 1560
gccagccagg tgaccaccgc caagaagctg cggggcggcg tggtgttcgt ggacgaggtc 1620
ccgaagggcc tgaccgggaa gctcgacgcc cggaagatcc gcgagatcct gatcaaggcc 1680
aagaagggcg gcaagatcgc cgtgtaagac tagttataag actgactagc ccgatgggcc 1740
tcccaacggg ccctcctccc ctccttgcac cgagattaat agatctaaaa aaaaaaaaaa 1800
aaaaaaaaaa aaaaaaaaaa aaaaaaaaaa aaaaaaaaaa aaaaaaaaaa tgcatccccc 1860
cccccccccc cccccccccc ccccccaaag gctcttttca gagccaccag aatt 1914

<210> 1366
<211> 1848
<212> DNA
<213> Artificial Sequence

<220>
<223> 32L PpLuc(GC) A64N64

<400> 1366

```
ggggcgctgc ctacggaggt ggcagccatc tccttctcgg catcaagctt gaggatggag      60
gacgccaaga acatcaagaa gggcccggcg cccttctacc cgctggagga cgggaccgcc     120
ggcgagcagc tccacaaggc catgaagcgg tacgccctgg tgccgggcac gatcgccttc     180
accgacgccc acatcgaggt cgacatcacc tacgcggagt acttcgagat gagcgtgcgc     240
ctggccgagg ccatgaagcg gtacggcctg aacaccaacc accggatcgt ggtgtgctcg     300
gagaacagcc tgcagttctt catgccggtg ctgggcgccc tcttcatcgg cgtggccgtc     360
gccccggcga acgacatcta caacgagcgg gagctgctga acagcatggg gatcagccag     420
ccgaccgtgg tgttcgtgag caagaagggc ctgcagaaga tcctgaacgt gcagaagaag     480
```

ctgcccatca tccagaagat catcatcatg gacagcaaga ccgactacca gggcttccag 540
tcgatgtaca cgttcgtgac cagccacctc ccgccgggct tcaacgagta cgacttcgtc 600
ccggagagct tcgaccggga caagaccatc gccctgatca tgaacagcag cggcagcacc 660
ggcctgccga agggggtggc cctgccgcac cggaccgcct gcgtgcgctt ctcgcacgcc 720
cgggacccca tcttcggcaa ccagatcatc ccggacaccg ccatcctgag cgtggtgccg 780
ttccaccacg gcttcggcat gttcacgacc ctgggctacc tcatctgcgg cttccgggtg 840
gtcctgatgt accggttcga ggaggagctg ttcctgcgga gcctgcagga ctacaagatc 900
cagagcgcgc tgctcgtgcc gaccctgttc agcttcttcg ccaagagcac cctgatcgac 960
aagtacgacc tgtcgaacct gcacgagatc gccagcgggg gcgccccgct gagcaaggag 1020
gtgggcgagg ccgtggccaa gcggttccac ctcccgggca tccgccaggg ctacggcctg 1080
accgagacca cgagcgcgat cctgatcacc cccgaggggg acgacaagcc gggcgccgtg 1140
ggcaaggtgg tcccgttctt cgaggccaag gtggtggacc tggacaccgg caagaccctg 1200
ggcgtgaacc agcggggcga gctgtgcgtg cgggggccga tgatcatgag cggctacgtg 1260
aacaacccgg aggccaccaa cgccctcatc gacaaggacg gctggctgca cagcggcgac 1320
atcgcctact gggacgagga cgagcacttc ttcatcgtcg accggctgaa gtcgctgatc 1380
aagtacaagg gctaccaggt ggcgccggcc gagctggaga gcatcctgct ccagcacccc 1440
aacatcttcg acgccggcgt ggccgggctg ccggacgacg acgccggcga gctgccggcc 1500
gcggtggtgg tgctggagca cggcaagacc atgacggaga aggagatcgt cgactacgtg 1560
gccagccagg tgaccaccgc caagaagctg cggggcggcg tggtgttcgt ggacgaggtc 1620
ccgaagggcc tgaccgggaa gctcgacgcc cggaagatcc gcgagatcct gatcaaggcc 1680
aagaagggcg gcaagatcgc cgtgtaagac tagtagatct aaaaaaaaaa aaaaaaaaaa 1740
aaaaaaaaaa aaaaaaaaaa aaaaaaaaaa aaaaaaaaaa aaaatgcatc cccccccccc 1800
cccccccccc cccccccccc aaaggctctt ttcagagcca ccagaatt 1848

<210> 1367
<211> 2035
<212> DNA
<213> Artificial Sequence

<220>
<223> 32L PpLuc(GC) albumin7 A64N64

<400> 1367

```
ggggcgctgc ctacggaggt ggcagccatc tccttctcgg catcaagctt gaggatggag       60
gacgccaaga acatcaagaa gggcccggcg cccttctacc cgctggagga cgggaccgcc      120
ggcgagcagc tccacaaggc catgaagcgg tacgccctgg tgccgggcac gatcgccttc      180
```

```
accgacgccc acatcgaggt cgacatcacc tacgcggagt acttcgagat gagcgtgcgc      240
ctggccgagg ccatgaagcg gtacggcctg aacaccaacc accggatcgt ggtgtgctcg      300
gagaacagcc tgcagttctt catgccggtg ctgggcgccc tcttcatcgg cgtggccgtc      360
gccccggcga acgacatcta caacgagcgg gagctgctga acagcatggg gatcagccag      420
ccgaccgtgg tgttcgtgag caagaagggc ctgcagaaga tcctgaacgt gcagaagaag      480
ctgcccatca tccagaagat catcatcatg gacagcaaga ccgactacca gggcttccag      540
tcgatgtaca cgttcgtgac cagccacctc ccgccgggct tcaacgagta cgacttcgtc      600
ccggagagct tcgaccggga caagaccatc gccctgatca tgaacagcag cggcagcacc      660
ggcctgccga aggggtggc cctgccgcac cggaccgcct gcgtgcgctt ctcgcacgcc       720
cgggacccca tcttcggcaa ccagatcatc ccggacaccg ccatcctgag cgtggtgccg      780
ttccaccacg gcttcggcat gttcacgacc ctgggctacc tcatctgcgg cttccgggtg      840
gtcctgatgt accggttcga ggaggagctg ttcctgcgga gcctgcagga ctacaagatc      900
cagagcgcgc tgctcgtgcc gaccctgttc agcttcttcg ccaagagcac cctgatcgac      960
aagtacgacc tgtcgaacct gcacgagatc gccagcgggg gcgccccgct gagcaaggag     1020
gtgggcgagg ccgtggccaa gcggttccac ctcccgggca tccgccaggg ctacggcctg     1080
accgagacca cgagcgcgat cctgatcacc cccgaggggg acgacaagcc gggcgccgtg     1140
ggcaaggtgg tcccgttctt cgaggccaag gtggtggacc tggacaccgg caagaccctg     1200
ggcgtgaacc agcggggcga gctgtgcgtg cgggggccga tgatcatgag cggctacgtg     1260
aacaacccgg aggccaccaa cgccctcatc gacaaggacg gctggctgca cagcggcgac     1320
atcgcctact gggacgagga cgagcacttc ttcatcgtcg accggctgaa gtcgctgatc     1380
aagtacaagg gctaccaggt ggcgccggcc gagctggaga gcatcctgct ccagcacccc     1440
aacatcttcg acgccggcgt ggccgggctg ccggacgacg acgccggcga gctgccggcc     1500
gcggtggtgg tgctggagca cggcaagacc atgacggaga aggagatcgt cgactacgtg     1560
gccagccagg tgaccaccgc caagaagctg cggggcggcg tggtgttcgt ggacgaggtc     1620
ccgaagggcc tgaccgggaa gctcgacgcc cggaagatcc gcgagatcct gatcaaggcc     1680
aagaagggcg gcaagatcgc cgtgtaagac tagtgcatca catttaaaag catctcagcc     1740
taccatgaga ataagagaaa gaaaatgaag atcaatagct tattcatctc tttttctttt     1800
tcgttggtgt aaagccaaca ccctgtctaa aaaacataaa tttctttaat cattttgcct     1860
cttttctctg tgcttcaatt aataaaaaat ggaaagaacc tagatctaaa aaaaaaaaaa     1920
aaaaaaaaaa aaaaaaaaaa aaaaaaaaaa aaaaaaaaaa aaaaaaaaaa atgcatcccc     1980
cccccccccc cccccccccc cccccccaaa ggctcttttc agagccacca gaatt          2035
```

<210> 1368
<211> 42
<212> DNA
<213> Artificial Sequence

<220>
<223> 5UTR of human ribosomal protein Large 32 lacking the 5 terminal oligopyrimidine tract

<400> 1368
ggcgctgcct acggaggtgg cagccatctc cttctcggca tc 42

<210> 1369
<211> 186
<212> DNA
<213> Homo sapiens

<400> 1369

catcacattt aaaagcatct cagcctacca tgagaataag agaaagaaaa tgaagatcaa 60

aagcttattc atctgttttt ctttttcgtt ggtgtaaagc caacaccctg tctaaaaaac 120

ataaatttct ttaatcattt tgcctctttt ctctgtgctt caattaataa aaaatggaaa 180

gaatct 186

<210> 1370
<211> 110
<212> DNA
<213> Homo sapiens

<400> 1370

gctggagcct cggtggccat gcttcttgcc ccttgggcct ccccccagcc cctcctcccc 60

ttcctgcacc cgtacccccg tggtctttga ataaagtctg agtgggcggc 110

<210> 1371
<211> 108
<212> DNA
<213> Homo sapiens

<400> 1371

gctggagcct cggtagccgt tcctcctgcc cgctgggcct cccaacgggc cctcctcccc 60

tccttgcacc ggcccttcct ggtctttgaa taaagtctga gtgggcag 108

<210> 1372
<211> 132
<212> DNA
<213> Homo sapiens

<400> 1372

gctcgctttc ttgctgtcca atttctatta aaggttcctt tgttccctaa gtccaactac 60

taaactgggg gatattatga agggccttga gcatctggat tctgcctaat aaaaaacatt 120

tattttcatt gc 132

<210> 1373
<211> 304
<212> DNA
<213> Homo sapiens

<400> 1373

gtgcacggcg tccctgaggg cccttcccaa cctcccctgg tcctgcactg tcccggagct 60

caggccctgg tgaggggctg ggtcccgggt gccccccatg ccctccctgc tgccaggctc 120

ccactgcccc tgcacctgct tctcagcgca acagctgtgt gtgcccgtgg tgaggttgtg 180

ctgcctgtgg tgaggtcctg tcctggctcc cagggtcctg gggctgctg cactgccctc 240

cgcccttccc tgacactgtc tgctgcccca atcaccgtca caataaaaga aactgtggtc 300

tcta 304

<210> 1374
<211> 674
<212> DNA
<213> Homo sapiens

<400> 1374

gcgtcgccac cctttggtta tttcagcccc catcacccaa gccacaagct gaccccttcg 60

tggttatagc cctgccctcc caagtcccac cctcttccca tgtcccaccc tccctagagg 120

ggcacctttt catggtctct gcacccagtg aacacatttt actctagagg catcacctgg 180

gaccttactc ctctttcctt ccttcctcct ttcctatctt ccttcctctc tctcttcctc 240

tttcttcatt cagatctata tggcaaatag ccacaattat ataaatcatt tcaagactag 300

aataggggga tataatacat attactccac acctttttatg aatcaaatat gattttttttg 360

ttgttgttaa gacagagtct cactttgaca cccaggctgg agtgcagtgg tgccatcacc 420

acggctcact gcagcctcag cgtcctgggc tcaaatgatc ctcccacctc agcctcctga 480

gtagctggga ctacaggctc atgccatcat gcccagctaa tattttttta ttttcgtgga 540

gacggggcct cactatgttg cctaggctgg aaataggatt ttgaacccaa attgagttta 600

acaataataa aaagttgttt tacgctaaag atggaaaaga actaggactg aactatttta 660

aataaaatat tggc 674

<210> 1375
<211> 1406
<212> DNA

<213> Homo sapiens

<400> 1375

```
actccctcca tcccaacctg gctccctccc acccaaccaa ctttcccccc aacccggaaa      60
cagacaagca acccaaactg aaccccctca aaagccaaaa aatgggagac aatttcacat     120
ggactttgga aaatattttt ttcctttgca ttcatctctc aaacttagtt tttatctttg     180
accaaccgaa catgaccaaa aaccaaaagt gcattcaacc ttaccaaaaa aaaaaaaaaa     240
aaaagaataa ataaataact ttttaaaaaa ggaagcttgg tccacttgct tgaagaccca     300
tgcgggggta agtccctttc tgcccgttgg gcttatgaaa ccccaatgct gccctttctg     360
ctcctttctc cacacccccc ttggggcctc ccctccactc cttcccaaat ctgtctcccc     420
agaagacaca ggaaacaatg tattgtctgc ccagcaatca aaggcaatgc tcaaacaccc     480
aagtggcccc caccctcagc ccgctcctgc ccgcccagca cccccaggcc ctgggggacc     540
tggggttctc agactgccaa agaagccttg ccatctggcg ctcccatggc tcttgcaaca     600
tctccccttc gtttttgagg gggtcatgcc gggggagcca ccagcccctc actgggttcg     660
gaggagagtc aggaagggcc acgacaaagc agaaacatcg gatttgggga acgcgtgtca     720
atcccttgtg ccgcagggct gggcgggaga gactgttctg ttccttgtgt aactgtgttg     780
ctgaaagact acctcgttct tgtcttgatg tgtcaccggg gcaactgcct gggggcgggg     840
atgggggcag ggtggaagcg gctccccatt ttataccaaa ggtgctacat ctatgtgatg     900
ggtggggtgg ggagggaatc actggtgcta tagaaattga gatgcccccc caggccagca     960
aatgttcctt tttgttcaaa gtctattttt attccttgat atttttcttt tttttttttt    1020
tttttgtgg atggggactt gtgaattttt ctaaaggtgc tatttaacat gggaggagag     1080
cgtgtgcggc tccagcccag cccgctgctc actttccacc ctctctccac ctgcctctgg    1140
cttctcaggc ctctgctctc cgacctctct cctctgaaac cctcctccac agctgcagcc    1200
catcctcccg gctccctcct agtctgtcct gcgtcctctg tccccgggtt tcagagacaa    1260
cttcccaaag cacaaagcag tttttccccc taggggtggg aggaagcaaa agactctgta    1320
cctattttgt atgtgtataa taatttgaga tgtttttaat tattttgatt gctggaataa    1380
agcatgtgga aatgacccaa acataa                                        1406
```

<210> 1376
<211> 186
<212> DNA
<213> Homo sapiens

<400> 1376

catcacattt aaaagcatct cagcctacca tgagaataag agaaagaaaa tgaagatcaa 60

tagcttattc atctcttttt ctttttcgtt ggtgtaaagc caacaccctg tctaaaaaac 120

ataaatttct ttaatcattt tgcctctttt ctctgtgctt caattaataa aaaatggaaa 180

gaacct 186

<210> 1377
<211> 256
<212> DNA
<213> Artificial Sequence

<220>
<223> Human albumin 3UTR + poly(A) sequence

<400> 1377

catcacattt aaaagcatct cagcctacca tgagaataag agaaagaaaa tgaagatcaa 60

aagcttattc atctgttttt ctttttcgtt ggtgtaaagc caacaccctg tctaaaaaac 120

ataaatttct ttaatcattt tgcctctttt ctctgtgctt caattaataa aaaatggaaa 180

gaatctagat ctaaaaaaaa aaaaaaaaaa aaaaaaaaaa aaaaaaaaaa aaaaaaaaaa 240

aaaaaaaaaa aaaaaa 256

<210> 1378
<211> 155
<212> DNA
<213> Homo sapiens

<400> 1378

aaaagcatct cagcctacca tgagaataag agaaagaaaa tgaagatcaa aagcttattc 60

atctgttttt ctttttcgtt ggtgtaaagc caacaccctg tctaaaaaac ataaatttct 120

ttaatcattt tgcctctttt ctctgtgctt caatt 155

<210> 1379
<211> 110
<212> DNA
<213> Homo sapiens

<400> 1379

catcacattt aaaagcatct cagcctacca tgagaataag agaaagaaaa tgaagatcaa 60

aagcttattc atctgttttt ctttttcgtt ggtgtaaagc caacaccctg 110

<210> 1380
<211> 110
<212> DNA
<213> Homo sapiens

<400> 1380

aaaagcatct cagcctacca tgagaataag agaaagaaaa tgaagatcaa aagcttattc 60

atctgttttt ctttttcgtt ggtgtaaagc caacaccctg tctaaaaaac 110

<210> 1381
<211> 110
<212> DNA
<213> Homo sapiens

<400> 1381

cagcctacca tgagaataag agaaagaaaa tgaagatcaa aagcttattc atctgttttt 60

ctttttcgtt ggtgtaaagc caacaccctg tctaaaaaac ataaatttct 110

<210> 1382
<211> 110
<212> DNA
<213> Homo sapiens

<400> 1382

tgagaataag agaaagaaaa tgaagatcaa aagcttattc atctgttttt ctttttcgtt 60

ggtgtaaagc caacaccctg tctaaaaaac ataaatttct ttaatcattt 110

<210> 1383
<211> 110
<212> DNA
<213> Homo sapiens

<400> 1383

agaaagaaaa tgaagatcaa aagcttattc atctgttttt ctttttcgtt ggtgtaaagc 60

caacaccctg tctaaaaaac ataaatttct ttaatcattt tgcctctttt 110

<210> 1384
<211> 110
<212> DNA
<213> Homo sapiens

<400> 1384

tgaagatcaa aagcttattc atctgttttt ctttttcgtt ggtgtaaagc caacaccctg 60

tctaaaaaac ataaatttct ttaatcattt tgcctctttt ctctgtgctt 110

<210> 1385
<211> 110
<212> DNA
<213> Homo sapiens

<400> 1385

aagcttattc atctgttttt ctttttcgtt ggtgtaaagc caacaccctg tctaaaaaac 60

ataaatttct ttaatcattt tgcctctttt ctctgtgctt caattaataa 110

<210> 1386
<211> 110
<212> DNA
<213> Homo sapiens

<400> 1386

atctgttttt ctttttcgtt ggtgtaaagc caacaccctg tctaaaaaac ataaatttct 60

ttaatcattt tgcctctttt ctctgtgctt caattaataa aaaatggaaa 110

<210> 1387
<211> 151
<212> DNA
<213> Homo sapiens

<400> 1387

cagcctacca tgagaataag agaaagaaaa tgaagatcaa aagcttattc atctgttttt 60

ctttttcgtt ggtgtaaagc caacaccctg tctaaaaaac ataaatttct ttaatcattt 120

tgcctctttt ctctgtgctt caattaataa a 151

<210> 1388
<211> 121
<212> DNA
<213> Homo sapiens

<400> 1388

tgaagatcaa aagcttattc atctgttttt ctttttcgtt ggtgtaaagc caacaccctg 60

tctaaaaaac ataaatttct ttaatcattt tgcctctttt ctctgtgctt caattaataa 120

a 121

<210> 1389
<211> 91
<212> DNA
<213> Homo sapiens

<400> 1389

ctttttcgtt ggtgtaaagc caacaccctg tctaaaaaac ataaatttct ttaatcattt 60

tgcctctttt ctctgtgctt caattaataa a 91

<210> 1390
<211> 60
<212> DNA
<213> Homo sapiens

<400> 1390
aagcttattc atctgttttt ctttttcgtt ggtgtaaagc caacaccctg tctaaaaaac 60

<210> 1391
<211> 16
<212> RNA
<213> Artificial Sequence

<220>
<223> Sequence according to formula (Ic)

<220>
<221> misc_feature
<222> (1)..(1)
<223> n is a, u, t, g, and c, or a nucleotide analogue thereof

<220>
<221> misc_feature
<222> (3)..(8)
<223> n is a, u, t, g, and c, or a nucleotide analogue thereof

<220>
<221> misc_feature
<222> (10)..(14)
<223> n is a, u, t, g, and c, or a nucleotide analogue thereof

<220>
<221> misc_feature
<222> (16)..(16)
<223> n is a, u, t, g, and c, or a nucleotide analogue thereof

<400> 1391
ngnnnnnnun nnnncn 16

<210> 1392
<211> 26
<212> RNA
<213> Artificial Sequence

<220>
<223> Sequence according to formula (IIc)

<220>
<221> misc_feature
<222> (1)..(2)
<223> n is a, u, t, g, and c, or a nucleotide analogue thereof; may be present or not

<220>
<221> misc_feature
<222> (3)..(6)
<223> n is a, u, t, g, and c, or a nucleotide analogue thereof

<220>
<221> misc_feature
<222> (8)..(13)
<223> n is a, u, t, g, and c, or a nucleotide analogue thereof

<220>

<221> misc_feature
<222> (15)..(19)
<223> n is a, u, t, g, and c, or a nucleotide analogue thereof

<220>
<221> misc_feature
<222> (21)..(24)
<223> n is a, u, t, g, and c, or a nucleotide analogue thereof

<220>
<221> misc_feature
<222> (25)..(26)
<223> n is a, u, t, g, and c, or a nucleotide analogue thereof; may be present or not.

<400> 1392
nnnnnngnnn nnnunnnnnc nnnnnn 26

<210> 1393
<211> 16
<212> RNA
<213> Artificial Sequence

<220>
<223> Sequence according to formula (Id)

<220>
<221> misc_feature
<222> (1)..(1)
<223> n is a, u, t, g, and c, or a nucleotide analogue thereof

<220>
<221> misc_feature
<222> (3)..(8)
<223> n is a, u, t, g, and c, or a nucleotide analogue thereof

<220>
<221> misc_feature
<222> (10)..(14)
<223> n is a, u, t, g, and c, or a nucleotide analogue thereof

<220>
<221> misc_feature
<222> (16)..(16)
<223> n is a, u, t, g, and c, or a nucleotide analogue thereof

<400> 1393
ncnnnnnnun nnnngn 16

<210> 1394
<211> 26
<212> RNA
<213> Artificial Sequence

<220>
<223> Sequence according to formula (IId)

<220>
<221> misc_feature

<222> (1)..(2)
<223> n is a, u, t, g, and c, or a nucleotide analogue thereof; may be present or not.

<220>
<221> misc_feature
<222> (3)..(6)
<223> n is a, u, t, g, and c, or a nucleotide analogue thereof

<220>
<221> misc_feature
<222> (8)..(13)
<223> n is a, u, t, g, and c, or a nucleotide analogue thereof

<220>
<221> misc_feature
<222> (15)..(19)
<223> n is a, u, t, g, and c, or a nucleotide analogue thereof

<220>
<221> misc_feature
<222> (21)..(23)
<223> n is a, u, t, g, and c, or a nucleotide analogue thereof

<220>
<221> misc_feature
<222> (24)..(26)
<223> n is a, u, t, g, and c, or a nucleotide analogue thereof; may be present or not

<400> 1394
nnnnnncnnn nnnunnnnng nnnnnn 26

<210> 1395
<211> 16
<212> RNA
<213> Artificial Sequence

<220>
<223> Sequence according to formula (Ie)

<220>
<221> misc_feature
<222> (3)..(8)
<223> n is a, u, t, g, and c, or a nucleotide analogue thereof

<220>
<221> misc_feature
<222> (10)..(14)
<223> n is a, u, t, g, and c, or a nucleotide analogue thereof

<400> 1395
dgnnnnnnun nnnnch 16

<210> 1396
<211> 26
<212> RNA
<213> Artificial Sequence

<220>

<223> Sequence according to formula (IIe)

<220>
<221> misc_feature
<222> (1)..(2)
<223> n is a, u, t, g, and c, or a nucleotide analogue thereof; may be present or not

<220>
<221> misc_feature
<222> (3)..(5)
<223> n is a, u, t, g, and c, or a nucleotide analogue thereof

<220>
<221> misc_feature
<222> (8)..(13)
<223> n is a, u, t, g, and c, or a nucleotide analogue thereof

<220>
<221> misc_feature
<222> (15)..(19)
<223> n is a, u, t, g, and c, or a nucleotide analogue thereof

<220>
<221> misc_feature
<222> (22)..(23)
<223> n is a, u, t, g, and c, or a nucleotide analogue thereof

<220>
<221> misc_feature
<222> (24)..(26)
<223> n is a, u, t, g, and c, or a nucleotide analogue thereof; may be present or not

<400> 1396
nnnnndgnnn nnnunnnnnc hnnnnn 26

<210> 1397
<211> 16
<212> RNA
<213> Artificial Sequence

<220>
<223> Sequence according to formula (If)

<220>
<221> misc_feature
<222> (1)..(1)
<223> n is a, u, t, g, and c, or a nucleotide analogue thereof

<220>
<221> misc_feature
<222> (3)..(3)
<223> n is a, u, t, g, and c, or a nucleotide analogue thereof

<220>
<221> misc_feature
<222> (7)..(8)
<223> n is a, u, t, g, and c, or a nucleotide analogue thereof

<220>
<221> misc_feature
<222> (10)..(10)
<223> n is a, u, t, g, and c, or a nucleotide analogue thereof

<220>
<221> misc_feature
<222> (12)..(12)
<223> n is a, u, t, g, and c, or a nucleotide analogue thereof

<220>
<221> misc_feature
<222> (14)..(14)
<223> n is a, u, t, g, and c, or a nucleotide analogue thereof

<220>
<221> misc_feature
<222> (16)..(16)
<223> n is a, u, t, g, and c, or a nucleotide analogue thereof

<400> 1397
ngnbyynnun vndncn 16

<210> 1398
<211> 26
<212> RNA
<213> Artificial Sequence

<220>
<223> Sequence according to formula (IIf)

<220>
<221> misc_feature
<222> (1)..(2)
<223> n is a, u, t, g, and c, or a nucleotide analogue thereof; may be present or not

<220>
<221> misc_feature
<222> (3)..(6)
<223> n is a, u, t, g, and c, or a nucleotide analogue thereof

<220>
<221> misc_feature
<222> (8)..(8)
<223> n is a, u, t, g, and c, or a nucleotide analogue thereof

<220>
<221> misc_feature
<222> (12)..(13)
<223> n is a, u, t, g, and c, or a nucleotide analogue thereof

<220>
<221> misc_feature
<222> (15)..(15)
<223> n is a, u, t, g, and c, or a nucleotide analogue thereof

<220>
<221> misc_feature

<222> (17)..(17)
<223> n is a, u, t, g, and c, or a nucleotide analogue thereof

<220>
<221> misc_feature
<222> (19)..(19)
<223> n is a, u, t, g, and c, or a nucleotide analogue thereof

<220>
<221> misc_feature
<222> (21)..(23)
<223> n is a, u, t, g, and c, or a nucleotide analogue thereof

<220>
<221> misc_feature
<222> (24)..(26)
<223> n is a, u, t, g, and c, or a nucleotide analogue thereof; may be present or not

<400> 1398
nnnnnngnby ynnunvndnc nnnnnn 26

<210> 1399
<211> 16
<212> RNA
<213> Artificial Sequence

<220>
<223> Sequence according to formula (Ig)

<220>
<221> misc_feature
<222> (1)..(1)
<223> n is a, u, t, g, and c, or a nucleotide analogue thereof

<220>
<221> misc_feature
<222> (8)..(8)
<223> n is a, u, t, g, and c, or a nucleotide analogue thereof

<220>
<221> misc_feature
<222> (16)..(16)
<223> n is a, u, t, g, and c, or a nucleotide analogue thereof

<400> 1399
nghyyydnuh abrdcn 16

<210> 1400
<211> 26
<212> RNA
<213> Artificial Sequence

<220>
<223> Sequence according to formula (IIg)

<220>
<221> misc_feature
<222> (1)..(2)

<223> n is a, u, t, g, and c, or a nucleotide analogue thereof; may be present or not

<220>
<221> misc_feature
<222> (4)..(6)
<223> n is a, u, t, g, and c, or a nucleotide analogue thereof

<220>
<221> misc_feature
<222> (13)..(13)
<223> n is a, u, t, g, and c, or a nucleotide analogue thereof

<220>
<221> misc_feature
<222> (21)..(23)
<223> n is a, u, t, g, and c, or a nucleotide analogue thereof

<220>
<221> misc_feature
<222> (24)..(25)
<223> n is a, u, t, g, and c, or a nucleotide analogue thereof; may be present or not

<220>
<221> misc_feature
<222> (26)..(26)
<223> may be present or not

<400> 1400
nnhnnnghyy ydnuhabrdc nnnnnh 26

<210> 1401
<211> 16
<212> RNA
<213> Artificial Sequence

<220>
<223> Sequence according to formula (Ih)

<400> 1401
dghycudyuh asrrcc 16

<210> 1402
<211> 26
<212> RNA
<213> Artificial Sequence

<220>
<223> Sequence according to formula (IIh)

<220>
<221> misc_feature
<222> (1)..(1)
<223> n is a, u, t, g, and c, or a nucleotide analogue thereof; may be present or not

<220>
<221> misc_feature
<222> (2)..(2)
<223> may be present or not

<220>
<221> misc_feature
<222> (25)..(25)
<223> n is a, u, t, g, and c, or a nucleotide analogue thereof; may be present or not

<220>
<221> misc_feature
<222> (26)..(26)
<223> may be present or not

<400> 1402
nhaahdghyc udyuhasrrc cvhbnh 26

<210> 1403
<211> 16
<212> DNA
<213> Artificial Sequence

<220>
<223> Sequence according to formula (Ic)

<400> 1403
vgyyyyhhth rwrcb 16

<210> 1404
<211> 16
<212> DNA
<213> Artificial Sequence

<220>
<223> Sequence according to formula (Ic)

<400> 1404
sgyyyttytm arrrcs 16

<210> 1405
<211> 16
<212> DNA
<213> Artificial Sequence

<220>
<223> Sequence according to formula (Ic)

<400> 1405
sgyycttttm agrrcs 16

<210> 1406
<211> 16
<212> DNA
<213> Artificial Sequence

<220>
<223> Sequence according to formula (Ie)

<220>
<221> misc_feature
<222> (3)..(5)
<223> n is a, u, t, g, and c, or a nucleotide analogue thereof

<220>
<221> misc_feature
<222> (7)..(8)
<223> n is a, u, t, g, and c, or a nucleotide analogue thereof

<220>
<221> misc_feature
<222> (12)..(14)
<223> n is a, u, t, g, and c, or a nucleotide analogue thereof

<400> 1406
dgnnnbnnth vnnnch 16

<210> 1407
<211> 16
<212> DNA
<213> Artificial Sequence

<220>
<223> Sequence according to formula (Ie)

<220>
<221> misc_feature
<222> (3)..(5)
<223> n is a, u, t, g, and c, or a nucleotide analogue thereof

<220>
<221> misc_feature
<222> (13)..(14)
<223> n is a, u, t, g, and c, or a nucleotide analogue thereof

<400> 1407
rgnnnyhbth rdnncy 16

<210> 1408
<211> 16
<212> DNA
<213> Artificial Sequence

<220>
<223> Sequence according to formula (Ie)

<220>
<221> misc_feature
<222> (3)..(3)
<223> n is a, u, t, g, and c, or a nucleotide analogue thereof

<220>
<221> misc_feature
<222> (14)..(14)
<223> n is a, u, t, g, and c, or a nucleotide analogue thereof

<400> 1408
rgndbyhyth rdhncy 16

<210> 1409
<211> 16
<212> DNA

<213> Artificial Sequence

<220>
<223> Sequence according to formula (If)

<400> 1409
vgyyytyhth rvrrcb 16

<210> 1410
<211> 16
<212> DNA
<213> Artificial Sequence

<220>
<223> Sequence according to formula (If)

<400> 1410
sgyycttytm agrrcs 16

<210> 1411
<211> 16
<212> DNA
<213> Artificial Sequence

<220>
<223> Sequence according to formula (If)

<400> 1411
sgyycttttm agrrcs 16

<210> 1412
<211> 16
<212> DNA
<213> Artificial Sequence

<220>
<223> Sequence according to formula (Ig)

<400> 1412
ggyycttyth agrrcc 16

<210> 1413
<211> 16
<212> DNA
<213> Artificial Sequence

<220>
<223> Sequence according to formula (Ig)

<400> 1413
ggcycttytm agrgcc 16

<210> 1414
<211> 16
<212> DNA
<213> Artificial Sequence

<220>

<223> Sequence according to formula (Ig)

<400> 1414
ggctcttttm agrgcc 16

<210> 1415
<211> 16
<212> DNA
<213> Artificial Sequence

<220>
<223> Sequence according to formula (Ih)

<400> 1415
dghyctdyth asrrcc 16

<210> 1416
<211> 16
<212> DNA
<213> Artificial Sequence

<220>
<223> Sequence according to formula (Ih)

<400> 1416
ggcyctttth agrgcc 16

<210> 1417
<211> 16
<212> DNA
<213> Artificial Sequence

<220>
<223> Sequence according to formula (Ih)

<400> 1417
ggcyctttm agrgcc 16

<210> 1418
<211> 26
<212> DNA
<213> Artificial Sequence

<220>
<223> Sequence according to formula (IIc)

<220>
<221> misc_feature
<222> (1)..(2)
<223> may be present or not

<220>
<221> misc_feature
<222> (25)..(26)
<223> n is a, u, t, g, and c, or a nucleotide analogue thereof; may be present or not

<400> 1418
hhhhwgyyy yhhthrwrc bvhhnn 26

<210> 1419
<211> 26
<212> DNA
<213> Artificial Sequence

<220>
<223> Sequence according to formula (IIc)

<220>
<221> misc_feature
<222> (1)..(2)
<223> may be present or not

<220>
<221> misc_feature
<222> (25)..(26)
<223> may be present or not

<400> 1419
mhmhmsgyyy ttytmarrrc smchhh 26

<210> 1420
<211> 26
<212> DNA
<213> Artificial Sequence

<220>
<223> Sequence according to formula (IIc)

<220>
<221> misc_feature
<222> (1)..(2)
<223> may be present or not

<220>
<221> misc_feature
<222> (25)..(26)
<223> may be present or not

<400> 1420
mmmmmsgyyc ttttmagrrc sachmh 26

<210> 1421
<211> 26
<212> DNA
<213> Artificial Sequence

<220>
<223> Sequence according to formula (IIe)

<220>
<221> misc_feature
<222> (1)..(2)
<223> n is a, u, t, g, and c, or a nucleotide analogue thereof; may be present or not

<220>
<221> misc_feature
<222> (3)..(5)

<223> n is a, u, t, g, and c, or a nucleotide analogue thereof

<220>
<221> misc_feature
<222> (8)..(10)
<223> n is a, u, t, g, and c, or a nucleotide analogue thereof

<220>
<221> misc_feature
<222> (12)..(13)
<223> n is a, u, t, g, and c, or a nucleotide analogue thereof

<220>
<221> misc_feature
<222> (17)..(19)
<223> n is a, u, t, g, and c, or a nucleotide analogue thereof

<220>
<221> misc_feature
<222> (22)..(22)
<223> n is a, u, t, g, and c, or a nucleotide analogue thereof

<220>
<221> misc_feature
<222> (24)..(26)
<223> n is a, u, t, g, and c, or a nucleotide analogue thereof; may be present or not

<400> 1421
nnnnndgnnn bnnthvnnnc hnhnnn 26

<210> 1422
<211> 26
<212> DNA
<213> Artificial Sequence

<220>
<223> Sequence according to formula (IIe)

<220>
<221> misc_feature
<222> (1)..(1)
<223> n is a, u, t, g, and c, or a nucleotide analogue thereof; may be present or not

<220>
<221> misc_feature
<222> (2)..(2)
<223> n is a, u, t, g, and c, or a nucleotide analogue thereof; may be present or not

<220>
<221> misc_feature
<222> (5)..(5)
<223> n is a, u, t, g, and c, or a nucleotide analogue thereof

<220>
<221> misc_feature
<222> (8)..(10)
<223> n is a, u, t, g, and c, or a nucleotide analogue thereof

<220>
<221> misc_feature
<222> (18)..(19)
<223> n is a, u, t, g, and c, or a nucleotide analogue thereof

<220>
<221> misc_feature
<222> (24)..(24)
<223> may be present or not

<220>
<221> misc_feature
<222> (25)..(26)
<223> n is a, u, t, g, and c, or a nucleotide analogue thereof; may be present or not

<400> 1422
nnhhnrgnnn yhbthrdnnc ydhhnn 26

<210> 1423
<211> 26
<212> DNA
<213> Artificial Sequence

<220>
<223> Sequence according to formula (IIe)

<220>
<221> misc_feature
<222> (1)..(1)
<223> n is a, u, t, g, and c, or a nucleotide analogue thereof; may be present or not

<220>
<221> misc_feature
<222> (2)..(2)
<223> may be present or not

<220>
<221> misc_feature
<222> (8)..(8)
<223> n is a, u, t, g, and c, or a nucleotide analogue thereof

<220>
<221> misc_feature
<222> (19)..(19)
<223> n is a, u, t, g, and c, or a nucleotide analogue thereof

<220>
<221> misc_feature
<222> (24)..(26)
<223> may be present or not

<400> 1423
nhhhvrgndb yhythrdhnc yrhhhh 26

<210> 1424
<211> 26
<212> DNA
<213> Artificial Sequence

<220>
<223> Sequence according to formula (IIf)

<220>
<221> misc_feature
<222> (1)..(2)
<223> may be present or not

<220>
<221> misc_feature
<222> (25)..(25)
<223> may be present or not

<220>
<221> misc_feature
<222> (26)..(26)
<223> n is a, u, t, g, and c, or a nucleotide analogue thereof; may be present or not

<400> 1424
hhmhmvgyyy tyhthrvrrc bvmhhn 26

<210> 1425
<211> 26
<212> DNA
<213> Artificial Sequence

<220>
<223> Sequence according to formula (IIf)

<220>
<221> misc_feature
<222> (1)..(2)
<223> may be present or not

<220>
<221> misc_feature
<222> (25)..(26)
<223> may be present or not

<400> 1425
mmmmmsgyyc ttytmagrrc smchhh 26

<210> 1426
<211> 26
<212> DNA
<213> Artificial Sequence

<220>
<223> Sequence according to formula (IIf)

<220>
<221> misc_feature
<222> (1)..(2)
<223> may be present or not

<220>
<221> misc_feature
<222> (25)..(26)

<223> may be present or not

<400> 1426
mmmmmsgyyc ttttmagrrc sachmh 26

<210> 1427
<211> 26
<212> DNA
<213> Artificial Sequence

<220>
<223> Sequence according to formula (IIg)

<220>
<221> misc_feature
<222> (1)..(2)
<223> may be present or not

<220>
<221> misc_feature
<222> (24)..(25)
<223> n is a, u, t, g, and c, or a nucleotide analogue thereof; may be present or not

<220>
<221> misc_feature
<222> (26).. (26)
<223> may be present or not

<400> 1427
hhmamggyyc ttythagrrc cvhnnm 26

<210> 1428
<211> 26
<212> DNA
<213> Artificial Sequence

<220>
<223> Sequence according to formula (IIg)

<220>
<221> misc_feature
<222> (1)..(2)
<223> may be present or not

<220>
<221> misc_feature
<222> (25)..(26)
<223> may be present or not

<400> 1428
hhaamggcyc ttytmagrgc cvchhm 26

<210> 1429
<211> 26
<212> DNA
<213> Artificial Sequence

<220>

<223> Sequence according to formula (IIg)

<220>
<221> misc_feature
<222> (1)..(2)
<223> may be present or not

<220>
<221> misc_feature
<222> (24)..(26)
<223> may be present or not

<400> 1429
mmaamggctc ttttmagrgc cmcymm 26

<210> 1430
<211> 26
<212> DNA
<213> Artificial Sequence

<220>
<223> Sequence according to formula (IIh)

<220>
<221> misc_feature
<222> (1)..(1)
<223> n is a, u, t, g, and c, or a nucleotide analogue thereof; may be present or not

<220>
<221> misc_feature
<222> (2)..(2)
<223> may be present or not

<220>
<221> misc_feature
<222> (24)..(24)
<223> may be present or not

<220>
<221> misc_feature
<222> (25)..(25)
<223> n is a, u, t, g, and c, or a nucleotide analogue thereof; may be present or not

<220>
<221> misc_feature
<222> (26)..(26)
<223> may be present or not

<400> 1430
nhaahdghyc tdythasrrc cvhbnh 26

<210> 1431
<211> 26
<212> DNA
<213> Artificial Sequence

<220>
<223> Sequence according to formula (IIh)

<220>
<221> misc_feature
<222> (1)..(2)
<223> may be present or not

<220>
<221> misc_feature
<222> (24)..(24)
<223> may be present or not

<220>
<221> misc_feature
<222> (25)..(25)
<223> n is a, u, t, g, and c, or a nucleotide analogue thereof; may be present or not

<220>
<221> misc_feature
<222> (26)..(26)
<223> may be present or not

<400> 1431
hhaamggcyc tttthagrgc cvmynm 26

<210> 1432
<211> 26
<212> DNA
<213> Artificial Sequence

<220>
<223> Sequence according to formula (IIh)

<220>
<221> misc_feature
<222> (1)..(2)
<223> may be present or not

<220>
<221> misc_feature
<222> (24)..(26)
<223> may be present or not

<400> 1432
hmaaaggcyc ttttmagrgc crmyhm 26

<210> 1433
<211> 24
<212> DNA
<213> Artificial Sequence

<220>
<223> Specific histone stem-loop sequence

<400> 1433
caaaggctct tttcagagcc acca 24

<210> 1434
<211> 44
<212> DNA

<213> Homo sapiens

<400> 1434
gcccgatggg cctcccaacg ggccctcctc ccctccttgc accg 44

<210> 1435
<211> 75
<212> DNA
<213> Homo sapiens

<400> 1435

tagtttctcc tctcgaacgc caggtggagc aaccggccgg ataccgccac agccctggca 60

ggcggcgctg tgatg 75

<210> 1436
<211> 2027
<212> DNA
<213> Artificial Sequence

<220>
<223> RPL35 PpLuc(GC) albumin7 A64 C30 histoneSL

<400> 1436

```
ggggagcggg cggcggcgtt ggcggcttgt gcagcaaagc ttgaggatgg aggacgccaa      60
gaacatcaag aagggcccgg cgcccttcta cccgctggag gacgggaccg ccggcgagca     120
gctccacaag gccatgaagc ggtacgccct ggtgccgggc acgatcgcct tcaccgacgc     180
ccacatcgag gtcgacatca cctacgcgga gtacttcgag atgagcgtgc gcctggccga     240
ggccatgaag cggtacggcc tgaacaccaa ccaccggatc gtggtgtgct cggagaacag     300
cctgcagttc ttcatgccgg tgctgggcgc cctcttcatc ggcgtggccg tcgccccggc     360
gaacgacatc tacaacgagc gggagctgct gaacagcatg gggatcagcc agccgaccgt     420
ggtgttcgtg agcaagaagg gcctgcagaa gatcctgaac gtgcagaaga agctgcccat     480
catccagaag atcatcatca tggacagcaa gaccgactac cagggcttcc agtcgatgta     540
cacgttcgtg accagccacc tcccgccggg cttcaacgag tacgacttcg tcccggagag     600
cttcgaccgg gacaagacca tcgccctgat catgaacagc agcggcagca ccggcctgcc     660
gaagggggtg gccctgccgc accggaccgc ctgcgtgcgc ttctcgcacg cccgggaccc     720
catcttcggc aaccagatca tcccggacac cgccatcctg agcgtggtgc cgttccacca     780
cggcttcggc atgttcacga ccctgggcta cctcatctgc ggcttccggg tggtcctgat     840
gtaccggttc gaggaggagc tgttcctgcg gagcctgcag gactacaaga tccagagcgc     900
gctgctcgtg ccgaccctgt tcagcttctt cgccaagagc accctgatcg acaagtacga     960
cctgtcgaac ctgcacgaga tcgccagcgg gggcgccccg ctgagcaagg aggtgggcga    1020
ggccgtggcc aagcggttcc acctcccggg catccgccag ggctacggcc tgaccgagac    1080
cacgagcgcg atcctgatca cccccgaggg ggacgacaag ccgggcgccg tgggcaaggt    1140
ggtcccgttc ttcgaggcca aggtggtgga cctggacacc ggcaagaccc tgggcgtgaa    1200
ccagcggggc gagctgtgcg tgcgggggcc gatgatcatg agcggctacg tgaacaaccc    1260
ggaggccacc aacgccctca tcgacaagga cggctggctg cacagcggcg acatcgccta    1320
ctgggacgag gacgagcact tcttcatcgt cgaccggctg aagtcgctga tcaagtacaa    1380
gggctaccag gtggcgccgg ccgagctgga gagcatcctg ctccagcacc ccaacatctt    1440
cgacgccggc gtggccgggc tgccggacga cgacgccggc gagctgccgg ccgcggtggt    1500
ggtgctggag cacggcaaga ccatgacgga gaaggagatc gtcgactacg tggccagcca    1560
ggtgaccacc gccaagaagc tgcggggcgg cgtggtgttc gtggacgagg tcccgaaggg    1620
cctgaccggg aagctcgacg cccggaagat ccgcgagatc ctgatcaagg ccaagaaggg    1680
cggcaagatc gccgtgtaag actagtgcat cacatttaaa agcatctcag cctaccatga    1740
```

```
gaataagaga aagaaaatga agatcaatag cttattcatc tctttttctt tttcgttggt     1800
gtaaagccaa caccctgtct aaaaaacata aatttcttta atcattttgc ctcttttctc     1860
tgtgcttcaa ttaataaaaa atggaaagaa cctagatcta aaaaaaaaaa aaaaaaaaaa     1920
aaaaaaaaaa aaaaaaaaaa aaaaaaaaaa aaaaaaaaaa aaatgcatcc cccccccccc     1980
cccccccccc cccccccccca aaggctcttt tcagagccac cagaatt                  2027
```

<210> 1437
<211> 2027
<212> DNA
<213> Artificial Sequence

<220>
<223> RPL21 PpLuc(GC) albumin7 A64 C30 histoneSL

<400> 1437

gggcccggaa ccgccatctt ccagtaattc gccaaaaagc ttgaggatgg aggacgccaa 60
gaacatcaag aagggcccgg cgcccttcta cccgctggag gacgggaccg ccggcgagca 120
gctccacaag gccatgaagc ggtacgccct ggtgccgggc acgatcgcct tcaccgacgc 180
ccacatcgag gtcgacatca cctacgcgga gtacttcgag atgagcgtgc gcctggccga 240
ggccatgaag cggtacggcc tgaacaccaa ccaccggatc gtggtgtgct cggagaacag 300
cctgcagttc ttcatgccgg tgctgggcgc cctcttcatc ggcgtggccg tcgccccggc 360
gaacgacatc tacaacgagc gggagctgct gaacagcatg gggatcagcc agccgaccgt 420
ggtgttcgtg agcaagaagg gcctgcagaa gatcctgaac gtgcagaaga agctgcccat 480
catccagaag atcatcatca tggacagcaa gaccgactac cagggcttcc agtcgatgta 540
cacgttcgtg accagccacc tcccgccggg cttcaacgag tacgacttcg tcccggagag 600
cttcgaccgg gacaagacca tcgccctgat catgaacagc agcggcagca ccggcctgcc 660
gaagggggtg gccctgccgc accggaccgc ctgcgtgcgc ttctcgcacg cccgggaccc 720
catcttcggc aaccagatca tcccggacac cgccatcctg agcgtggtgc cgttccacca 780
cggcttcggc atgttcacga ccctgggcta cctcatctgc ggcttccggg tggtcctgat 840
gtaccggttc gaggaggagc tgttcctgcg gagcctgcag gactacaaga tccagagcgc 900
gctgctcgtg ccgaccctgt tcagcttctt cgccaagagc accctgatcg acaagtacga 960
cctgtcgaac ctgcacgaga tcgccagcgg gggcgccccg ctgagcaagg aggtgggcga 1020
ggccgtggcc aagcggttcc acctcccggg catccgccag ggctacggcc tgaccgagac 1080
cacgagcgcg atcctgatca cccccgaggg ggacgacaag ccgggcgccg tgggcaaggt 1140
ggtcccgttc ttcgaggcca aggtggtgga cctggacacc ggcaagaccc tgggcgtgaa 1200
ccagcggggc gagctgtgcg tgcgggggcc gatgatcatg agcggctacg tgaacaaccc 1260

ggaggccacc aacgccctca tcgacaagga cggctggctg cacagcggcg acatcgccta 1320

ctgggacgag gacgagcact tcttcatcgt cgaccggctg aagtcgctga tcaagtacaa 1380

gggctaccag gtggcgccgg ccgagctgga gagcatcctg ctccagcacc ccaacatctt 1440

cgacgccggc gtggccgggc tgccggacga cgacgccggc gagctgccgg ccgcggtggt 1500

ggtgctggag cacggcaaga ccatgacgga gaaggagatc gtcgactacg tggccagcca 1560

ggtgaccacc gccaagaagc tgcggggcgg cgtggtgttc gtggacgagg tcccgaaggg 1620

cctgaccggg aagctcgacg cccggaagat ccgcgagatc ctgatcaagg ccaagaaggg 1680

cggcaagatc gccgtgtaag actagtgcat cacatttaaa agcatctcag cctaccatga 1740

gaataagaga aagaaaatga agatcaatag cttattcatc tctttttctt tttcgttggt 1800

gtaaagccaa caccctgtct aaaaaacata aatttcttta atcattttgc ctcttttctc 1860

tgtgcttcaa ttaataaaaa atggaaagaa cctagatcta aaaaaaaaaa aaaaaaaaaa 1920

aaaaaaaaaa aaaaaaaaaa aaaaaaaaaa aaaaaaaaaa aaatgcatcc cccccccccc 1980

cccccccccc ccccccccca aaggctcttt tcagagccac cagaatt 2027

<210> 1438
<211> 2068
<212> DNA
<213> Artificial Sequence

<220>
<223> ATP5A1 PpLuc(GC) albumin7 A64 C30 histoneSL

<400> 1438

```
gggcggctcg gccattttgt cccagtcagt ccggaggctg cggctgcaga agtaccgcct      60
gcggagtaac tgcaaagaag cttgaggatg gaggacgcca agaacatcaa gaagggcccg     120
gcgcccttct acccgctgga ggacgggacc gccggcgagc agctccacaa ggccatgaag     180
cggtacgccc tggtgccggg cacgatcgcc ttcaccgacg cccacatcga ggtcgacatc     240
acctacgcgg agtacttcga gatgagcgtg cgcctggccg aggccatgaa gcggtacggc     300
ctgaacacca accaccggat cgtggtgtgc tcggagaaca gcctgcagtt cttcatgccg     360
gtgctgggcg ccctcttcat cggcgtggcc gtcgccccgg cgaacgacat ctacaacgag     420
cgggagctgc tgaacagcat ggggatcagc cagccgaccg tggtgttcgt gagcaagaag     480
ggcctgcaga agatcctgaa cgtgcagaag aagctgccca tcatccagaa gatcatcatc     540
atggacagca agaccgacta ccagggcttc cagtcgatgt acacgttcgt gaccagccac     600
ctcccgccgg gcttcaacga gtacgacttc gtcccggaga gcttcgaccg ggacaagacc     660
atcgccctga tcatgaacag cagcggcagc accggcctgc cgaagggggt ggccctgccg     720
caccggaccg cctgcgtgcg cttctcgcac gcccgggacc ccatcttcgg caaccagatc     780
atcccggaca ccgccatcct gagcgtggtg ccgttccacc acggcttcgg catgttcacg     840
```

```
accctgggct acctcatctg cggcttccgg gtggtcctga tgtaccggtt cgaggaggag      900
ctgttcctgc ggagcctgca ggactacaag atccagagcg cgctgctcgt gccgaccctg      960
ttcagcttct tcgccaagag caccctgatc gacaagtacg acctgtcgaa cctgcacgag     1020
atcgccagcg gggcgcccc gctgagcaag gaggtgggcg aggccgtggc caagcggttc     1080
cacctcccgg gcatccgcca gggctacggc ctgaccgaga ccacgagcgc gatcctgatc     1140
acccccgagg gggacgacaa gccgggcgcc gtgggcaagg tggtcccgtt cttcgaggcc     1200
aaggtggtgg acctggacac cggcaagacc ctgggcgtga accagcgggg cgagctgtgc     1260
gtgcgggggc cgatgatcat gagcggctac gtgaacaacc cggaggccac caacgccctc     1320
atcgacaagg acggctggct gcacagcggc gacatcgcct actgggacga ggacgagcac     1380
ttcttcatcg tcgaccggct gaagtcgctg atcaagtaca agggctacca ggtggcgccg     1440
gccgagctgg agagcatcct gctccagcac cccaacatct tcgacgccgg cgtggccggg     1500
ctgccggacg acgacgccgg cgagctgccg gccgcggtgg tggtgctgga gcacggcaag     1560
accatgacgg agaaggagat cgtcgactac gtggccagcc aggtgaccac cgccaagaag     1620
ctgcggggcg gcgtggtgtt cgtggacgag gtcccgaagg gcctgaccgg gaagctcgac     1680
gcccggaaga tccgcgagat cctgatcaag gccaagaagg gcggcaagat cgccgtgtaa     1740
gactagtgca tcacatttaa aagcatctca gcctaccatg agaataagag aaagaaaatg     1800
aagatcaata gcttattcat ctctttttct ttttcgttgg tgtaaagcca acaccctgtc     1860
taaaaaacat aaatttcttt aatcattttg cctcttttct ctgtgcttca attaataaaa     1920
aatggaaaga acctagatct aaaaaaaaaa aaaaaaaaaa aaaaaaaaaa aaaaaaaaaa     1980
aaaaaaaaaa aaaaaaaaaa aaaatgcatc cccccccccc cccccccccc cccccccccc     2040
aaaggctctt ttcagagcca ccagaatt                                         2068
```

<210> 1439
<211> 2055
<212> DNA
<213> Artificial Sequence

<220>
<223> HSD17B4 PpLuc(GC) albumin7 A64 C30 histoneSL

<400> 1439

```
gggtcccgca gtcggcgtcc agcggctctg cttgttcgtg tgtgtgtcgt tgcaggcctt       60
attcaagctt gaggatggag gacgccaaga acatcaagaa gggcccggcg cccttctacc      120
cgctggagga cgggaccgcc ggcgagcagc tccacaaggc catgaagcgg tacgccctgg      180
tgccgggcac gatcgccttc accgacgccc acatcgaggt cgacatcacc tacgcggagt      240
acttcgagat gagcgtgcgc ctggccgagg ccatgaagcg gtacggcctg aacaccaacc      300
```

accggatcgt ggtgtgctcg gagaacagcc tgcagttctt catgccggtg ctgggcgccc 360

tcttcatcgg cgtggccgtc gccccggcga acgacatcta caacgagcgg gagctgctga 420

acagcatggg gatcagccag ccgaccgtgg tgttcgtgag caagaagggc ctgcagaaga 480

tcctgaacgt gcagaagaag ctgcccatca tccagaagat catcatcatg gacagcaaga 540

ccgactacca gggcttccag tcgatgtaca cgttcgtgac cagccacctc ccgccgggct 600

tcaacgagta cgacttcgtc ccggagagct tcgaccggga caagaccatc gccctgatca 660

tgaacagcag cggcagcacc ggcctgccga aggggtggc cctgccgcac cggaccgcct 720

gcgtgcgctt ctcgcacgcc cgggacccca tcttcggcaa ccagatcatc ccggacaccg 780

ccatcctgag cgtggtgccg ttccaccacg gcttcggcat gttcacgacc ctgggctacc 840

tcatctgcgg cttccgggtg gtcctgatgt accggttcga ggaggagctg ttcctgcgga 900

gcctgcagga ctacaagatc cagagcgcgc tgctcgtgcc gaccctgttc agcttcttcg 960

ccaagagcac cctgatcgac aagtacgacc tgtcgaacct gcacgagatc gccagcgggg 1020

gcgccccgct gagcaaggag gtgggcgagg ccgtggccaa gcggttccac ctcccgggca 1080

tccgccaggg ctacggcctg accgagacca cgagcgcgat cctgatcacc cccgaggggg 1140

acgacaagcc gggcgccgtg ggcaaggtgg tcccgttctt cgaggccaag gtggtggacc 1200

tggacaccgg caagaccctg ggcgtgaacc agcggggcga gctgtgcgtg cgggggccga 1260

tgatcatgag cggctacgtg aacaacccgg aggccaccaa cgccctcatc gacaaggacg 1320

gctggctgca cagcggcgac atcgcctact gggacgagga cgagcacttc ttcatcgtcg 1380

accggctgaa gtcgctgatc aagtacaagg gctaccaggt ggcgccggcc gagctggaga 1440

gcatcctgct ccagcacccc aacatcttcg acgccggcgt ggccgggctg ccggacgacg 1500

acgccggcga gctgccggcc gcggtggtgg tgctggagca cggcaagacc atgacggaga 1560

aggagatcgt cgactacgtg gccagccagg tgaccaccgc caagaagctg cggggcggcg 1620

tggtgttcgt ggacgaggtc ccgaagggcc tgaccgggaa gctcgacgcc cggaagatcc 1680

gcgagatcct gatcaaggcc aagaagggcg gcaagatcgc cgtgtaagac tagtgcatca 1740

catttaaaag catctcagcc taccatgaga ataagagaaa gaaaatgaag atcaatagct 1800

tattcatctc tttttctttt tcgttggtgt aaagccaaca ccctgtctaa aaaacataaa 1860

tttctttaat cattttgcct cttttctctg tgcttcaatt aataaaaaat ggaaagaacc 1920

tagatctaaa aaaaaaaaaa aaaaaaaaaa aaaaaaaaaa aaaaaaaaaa aaaaaaaaaa 1980

aaaaaaaaaa atgcatcccc cccccccccc cccccccccc cccccccaaa ggctcttttc 2040

agagccacca gaatt 2055

<210> 1440

<211> 2024
<212> DNA
<213> Artificial Sequence

<220>
<223> AIG1 PpLuc(GC) albumin7 A64 C30 histoneSL

<400> 1440

gggccgccca gccggtccag gcctctggcg aacaagcttg aggatggagg acgccaagaa 60
catcaagaag ggcccggcgc ccttctaccc gctggaggac gggaccgccg gcgagcagct 120
ccacaaggcc atgaagcggt acgccctggt gccgggcacg atcgccttca ccgacgccca 180
catcgaggtc gacatcacct acgcggagta cttcgagatg agcgtgcgcc tggccgaggc 240
catgaagcgg tacggcctga acaccaacca ccggatcgtg gtgtgctcgg agaacagcct 300
gcagttcttc atgccggtgc tgggcgccct cttcatcggc gtggccgtcg ccccggcgaa 360
cgacatctac aacgagcggg agctgctgaa cagcatgggg atcagccagc cgaccgtggt 420
gttcgtgagc aagaagggcc tgcagaagat cctgaacgtg cagaagaagc tgcccatcat 480
ccagaagatc atcatcatgg acagcaagac cgactaccag ggcttccagt cgatgtacac 540
gttcgtgacc agccacctcc cgccgggctt caacgagtac gacttcgtcc cggagagctt 600
cgaccgggac aagaccatcg ccctgatcat gaacagcagc ggcagcaccg gcctgccgaa 660
gggggtggcc ctgccgcacc ggaccgcctg cgtgcgcttc tcgcacgccc gggaccccat 720
cttcggcaac cagatcatcc cggacaccgc catcctgagc gtggtgccgt tccaccacgg 780
cttcggcatg ttcacgaccc tgggctacct catctgcggc ttccgggtgg tcctgatgta 840
ccggttcgag gaggagctgt tcctgcggag cctgcaggac tacaagatcc agagcgcgct 900
gctcgtgccg accctgttca gcttcttcgc caagagcacc ctgatcgaca agtacgacct 960
gtcgaacctg cacgagatcg ccagcggggg cgccccgctg agcaaggagg tgggcgaggc 1020
cgtggccaag cggttccacc tcccgggcat ccgccagggc tacggcctga ccgagaccac 1080
gagcgcgatc ctgatcaccc ccgaggggga cgacaagccg ggcgccgtgg gcaaggtggt 1140
cccgttcttc gaggccaagg tggtggacct ggacaccggc aagaccctgg gcgtgaacca 1200
gcggggcgag ctgtgcgtgc gggggccgat gatcatgagc ggctacgtga acaacccgga 1260
ggccaccaac gccctcatcg acaaggacgg ctggctgcac agcggcgaca tcgcctactg 1320
ggacgaggac gagcacttct tcatcgtcga ccggctgaag tcgctgatca agtacaaggg 1380
ctaccaggtg gcgccggccg agctggagag catcctgctc cagcacccca acatcttcga 1440
cgccggcgtg gccgggctgc cggacgacga cgccggcgag ctgccggccg cggtggtggt 1500
gctggagcac ggcaagacca tgacggagaa ggagatcgtc gactacgtgg ccagccaggt 1560
gaccaccgcc aagaagctgc ggggcggcgt ggtgttcgtg gacgaggtcc cgaagggcct 1620
gaccgggaag ctcgacgccc ggaagatccg cgagatcctg atcaaggcca agaagggcgg 1680

caagatcgcc gtgtaagact agtgcatcac atttaaaagc atctcagcct accatgagaa 1740
taagagaaag aaaatgaaga tcaatagctt attcatctct tttctttttt cgttggtgta 1800
aagccaacac cctgtctaaa aaacataaat ttctttaatc attttgcctc ttttctctgt 1860
gcttcaatta ataaaaaatg gaaagaacct agatctaaaa aaaaaaaaaa aaaaaaaaaa 1920
aaaaaaaaaa aaaaaaaaaa aaaaaaaaaa aaaaaaaaaa tgcatccccc cccccccccc 1980
cccccccccc cccccaaag gctcttttca gagccaccag aatt 2024

<210> 1441
<211> 2049
<212> DNA
<213> Artificial Sequence

<220>
<223> COX6C PpLuc(GC) albumin7 A64 C30 histoneSL

<400> 1441

ggagtcagga aggacgttgg tgttgaggtt agcatacgta tcaaggacag taactaccaa 60
gcttgaggat ggaggacgcc aagaacatca agaagggccc ggcgcccttc tacccgctgg 120
aggacgggac cgccggcgag cagctccaca aggccatgaa gcggtacgcc ctggtgccgg 180
gcacgatcgc cttcaccgac gcccacatcg aggtcgacat cacctacgcg gagtacttcg 240
agatgagcgt gcgcctggcc gaggccatga agcggtacgg cctgaacacc aaccaccgga 300
tcgtggtgtg ctcggagaac agcctgcagt tcttcatgcc ggtgctgggc gccctcttca 360
tcggcgtggc cgtcgccccg gcgaacgaca tctacaacga gcgggagctg ctgaacagca 420
tggggatcag ccagccgacc gtggtgttcg tgagcaagaa gggcctgcag aagatcctga 480
acgtgcagaa gaagctgccc atcatccaga agatcatcat catggacagc aagaccgact 540
accagggctt ccagtcgatg tacacgttcg tgaccagcca cctcccgccg ggcttcaacg 600
agtacgactt cgtcccggag agcttcgacc gggacaagac catcgccctg atcatgaaca 660
gcagcggcag caccggcctg ccgaaggggg tggccctgcc gcaccggacc gcctgcgtgc 720
gcttctcgca cgcccgggac cccatcttcg gcaaccagat catcccggac accgccatcc 780
tgagcgtggt gccgttccac cacggcttcg gcatgttcac gaccctgggc tacctcatct 840
gcggcttccg ggtggtcctg atgtaccggt tcgaggagga gctgttcctg cggagcctgc 900
aggactacaa gatccagagc gcgctgctcg tgccgaccct gttcagcttc ttcgccaaga 960
gcaccctgat cgacaagtac gacctgtcga acctgcacga gatcgccagc gggggcgccc 1020
cgctgagcaa ggaggtgggc gaggccgtgg ccaagcggtt ccacctcccg ggcatccgcc 1080
agggctacgg cctgaccgag accacgagcg cgatcctgat cacccccgag gggacgaca 1140
agccgggcgc cgtgggcaag gtggtcccgt tcttcgaggc caaggtggtg gacctggaca 1200
ccggcaagac cctgggcgtg aaccagcggg gcgagctgtg cgtgcggggg ccgatgatca 1260

tgagcggcta cgtgaacaac ccggaggcca ccaacgccct catcgacaag gacggctggc 1320
tgcacagcgg cgacatcgcc tactgggacg aggacgagca cttcttcatc gtcgaccggc 1380
tgaagtcgct gatcaagtac aagggctacc aggtggcgcc ggccgagctg gagagcatcc 1440
tgctccagca ccccaacatc ttcgacgccg gcgtggccgg gctgccggac gacgacgccg 1500
gcgagctgcc ggccgcggtg gtggtgctgg agcacggcaa gaccatgacg gagaaggaga 1560
tcgtcgacta cgtggccagc caggtgacca ccgccaagaa gctgcggggc ggcgtggtgt 1620
tcgtggacga ggtcccgaag ggcctgaccg ggaagctcga cgcccggaag atccgcgaga 1680
tcctgatcaa ggccaagaag ggcggcaaga tcgccgtgta agactagtgc atcacattta 1740
aaagcatctc agcctaccat gagaataaga gaaagaaaat gaagatcaat agcttattca 1800
tctcttttc tttttcgttg gtgtaaagcc aacaccctgt ctaaaaaaca taaatttctt 1860
taatcatttt gcctcttttc tctgtgcttc aattaataaa aaatggaaag aacctagatc 1920
taaaaaaaaa aaaaaaaaaa aaaaaaaaaa aaaaaaaaaa aaaaaaaaaa aaaaaaaaaa 1980
aaaaatgcat cccccccccc cccccccccc cccccccccc caaaggctct tttcagagcc 2040
accagaatt 2049

<210> 1442
<211> 2035
<212> DNA
<213> Artificial Sequence

<220>
<223> ASAH1 PpLuc(GC) albumin7 A64 C30 histoneSL

<400> 1442

```
gggcctctgc tggagtccgg ggagtggcgt tggctgctag agcgaagctt gaggatggag      60
gacgccaaga acatcaagaa gggcccggcg cccttctacc cgctggagga cgggaccgcc     120
ggcgagcagc tccacaaggc catgaagcgg tacgccctgg tgccgggcac gatcgccttc     180
accgacgccc acatcgaggt cgacatcacc tacgcggagt acttcgagat gagcgtgcgc     240
ctggccgagg ccatgaagcg gtacggcctg aacaccaacc accggatcgt ggtgtgctcg     300
gagaacagcc tgcagttctt catgccggtg ctgggcgccc tcttcatcgg cgtggccgtc     360
gccccggcga acgacatcta caacgagcgg gagctgctga acagcatggg gatcagccag     420
ccgaccgtgg tgttcgtgag caagaagggc ctgcagaaga tcctgaacgt gcagaagaag     480
ctgcccatca tccagaagat catcatcatg gacagcaaga ccgactacca gggcttccag     540
tcgatgtaca cgttcgtgac cagccacctc ccgccgggct tcaacgagta cgacttcgtc     600
ccggagagct tcgaccggga caagaccatc gccctgatca tgaacagcag cggcagcacc     660
ggcctgccga agggggtggc cctgccgcac cggaccgcct gcgtgcgctt ctcgcacgcc     720
```

cgggacccca tcttcggcaa ccagatcatc ccggacaccg ccatcctgag cgtggtgccg 780
ttccaccacg gcttcggcat gttcacgacc ctgggctacc tcatctgcgg cttccgggtg 840
gtcctgatgt accggttcga ggaggagctg ttcctgcgga gcctgcagga ctacaagatc 900
cagagcgcgc tgctcgtgcc gaccctgttc agcttcttcg ccaagagcac cctgatcgac 960
aagtacgacc tgtcgaacct gcacgagatc gccagcgggg gcgccccgct gagcaaggag 1020
gtgggcgagg ccgtggccaa gcggttccac ctcccgggca tccgccaggg ctacggcctg 1080
accgagacca cgagcgcgat cctgatcacc cccgaggggg acgacaagcc gggcgccgtg 1140
ggcaaggtgg tcccgttctt cgaggccaag gtggtggacc tggacaccgg caagaccctg 1200
ggcgtgaacc agcggggcga gctgtgcgtg cgggggccga tgatcatgag cggctacgtg 1260
aacaacccgg aggccaccaa cgccctcatc gacaaggacg gctggctgca cagcggcgac 1320
atcgcctact gggacgagga cgagcacttc ttcatcgtcg accggctgaa gtcgctgatc 1380
aagtacaagg gctaccaggt ggcgccggcc gagctggaga gcatcctgct ccagcacccc 1440
aacatcttcg acgccggcgt ggccgggctg ccggacgacg acgccggcga gctgccggcc 1500
gcggtggtgg tgctggagca cggcaagacc atgacggaga aggagatcgt cgactacgtg 1560
gccagccagg tgaccaccgc caagaagctg cggggcggcg tggtgttcgt ggacgaggtc 1620
ccgaagggcc tgaccgggaa gctcgacgcc cggaagatcc gcgagatcct gatcaaggcc 1680
aagaagggcg gcaagatcgc cgtgtaagac tagtgcatca catttaaaag catctcagcc 1740
taccatgaga ataagagaaa gaaaatgaag atcaatagct tattcatctc tttttctttt 1800
tcgttggtgt aaagccaaca ccctgtctaa aaaacataaa tttctttaat cattttgcct 1860
cttttctctg tgcttcaatt aataaaaaat ggaaagaacc tagatctaaa aaaaaaaaaa 1920
aaaaaaaaaa aaaaaaaaaa aaaaaaaaaa aaaaaaaaaa aaaaaaaaaa atgcatcccc 1980
cccccccccc cccccccccc cccccccaaa ggctcttttc agagccacca gaatt 2035

<210> 1443
<211> 2027
<212> DNA
<213> Artificial Sequence

<220>
<223> mRPL21 PpLuc(GC) albumin7 A64 C30 histoneSL

<400> 1443

```
ggggccgccg cagccatctt ccagtaactc gccaaaaagc ttgaggatgg aggacgccaa      60
gaacatcaag aagggcccgg cgcccttcta cccgctggag gacgggaccg ccggcgagca     120
gctccacaag gccatgaagc ggtacgccct ggtgccgggc acgatcgcct tcaccgacgc     180
ccacatcgag gtcgacatca cctacgcgga gtacttcgag atgagcgtgc gcctggccga     240
ggccatgaag cggtacggcc tgaacaccaa ccaccggatc gtggtgtgct cggagaacag     300
```

cctgcagttc ttcatgccgg tgctgggcgc cctcttcatc ggcgtggccg tcgccccggc 360
gaacgacatc tacaacgagc gggagctgct gaacagcatg gggatcagcc agccgaccgt 420
ggtgttcgtg agcaagaagg gcctgcagaa gatcctgaac gtgcagaaga agctgcccat 480
catccagaag atcatcatca tggacagcaa gaccgactac cagggcttcc agtcgatgta 540
cacgttcgtg accagccacc tcccgccggg cttcaacgag tacgacttcg tcccggagag 600
cttcgaccgg gacaagacca tcgccctgat catgaacagc agcggcagca ccggcctgcc 660
gaagggggtg gccctgccgc accggaccgc ctgcgtgcgc ttctcgcacg cccgggaccc 720
catcttcggc aaccagatca tcccggacac cgccatcctg agcgtggtgc cgttccacca 780
cggcttcggc atgttcacga ccctgggcta cctcatctgc ggcttccggg tggtcctgat 840
gtaccggttc gaggaggagc tgttcctgcg gagcctgcag gactacaaga tccagagcgc 900
gctgctcgtg ccgaccctgt tcagcttctt cgccaagagc accctgatcg acaagtacga 960
cctgtcgaac ctgcacgaga tcgccagcgg gggcgccccg ctgagcaagg aggtgggcga 1020
ggccgtggcc aagcggttcc acctcccggg catccgccag ggctacggcc tgaccgagac 1080
cacgagcgcg atcctgatca cccccgaggg ggacgacaag ccgggcgccg tgggcaaggt 1140
ggtcccgttc ttcgaggcca aggtggtgga cctggacacc ggcaagaccc tgggcgtgaa 1200
ccagcggggc gagctgtgcg tgcgggggcc gatgatcatg agcggctacg tgaacaaccc 1260
ggaggccacc aacgccctca tcgacaagga cggctggctg cacagcggcg acatcgccta 1320
ctgggacgag gacgagcact tcttcatcgt cgaccggctg aagtcgctga tcaagtacaa 1380
gggctaccag gtggcgccgg ccgagctgga gagcatcctg ctccagcacc ccaacatctt 1440
cgacgccggc gtggccgggc tgccggacga cgacgccggc gagctgccgg ccgcggtggt 1500
ggtgctggag cacggcaaga ccatgacgga gaaggagatc gtcgactacg tggccagcca 1560
ggtgaccacc gccaagaagc tgcggggcgg cgtggtgttc gtggacgagg tcccgaaggg 1620
cctgaccggg aagctcgacg cccggaagat ccgcgagatc ctgatcaagg ccaagaaggg 1680
cggcaagatc gccgtgtaag actagtgcat cacatttaaa agcatctcag cctaccatga 1740
gaataagaga aagaaaatga agatcaatag cttattcatc tctttttctt tttcgttggt 1800
gtaaagccaa caccctgtct aaaaaacata aatttcttta atcattttgc ctcttttctc 1860
tgtgcttcaa ttaataaaaa atggaaagaa cctagatcta aaaaaaaaaa aaaaaaaaaa 1920
aaaaaaaaaa aaaaaaaaaa aaaaaaaaaa aaaaaaaaaa aaatgcatcc cccccccccc 1980
cccccccccc cccccccccca aaggctcttt tcagagccac cagaatt 2027

<210> 1444
<211> 2046
<212> DNA

<213> Artificial Sequence

<220>
<223> mRPL35A PpLuc(GC) albumin7 A64 C30 histoneSL

<400> 1444

gggccatctt ggcgcctgtg gaggcctgct gggaacagga cttctaacag caagtaagct 60

tgaggatgga ggacgccaag aacatcaaga agggcccggc gcccttctac ccgctggagg 120

acgggaccgc cggcgagcag ctccacaagg ccatgaagcg gtacgccctg gtgccgggca 180

cgatcgcctt caccgacgcc cacatcgagg tcgacatcac ctacgcggag tacttcgaga 240

tgagcgtgcg cctggccgag gccatgaagc ggtacggcct gaacaccaac caccggatcg 300

tggtgtgctc ggagaacagc ctgcagttct tcatgccggt gctgggcgcc ctcttcatcg 360

gcgtggccgt cgccccggcg aacgacatct acaacgagcg ggagctgctg aacagcatgg 420

ggatcagcca gccgaccgtg gtgttcgtga gcaagaaggg cctgcagaag atcctgaacg 480

tgcagaagaa gctgcccatc atccagaaga tcatcatcat ggacagcaag accgactacc 540

agggcttcca gtcgatgtac acgttcgtga ccagccacct cccgccgggc ttcaacgagt 600

acgacttcgt cccggagagc ttcgaccggg acaagaccat cgccctgatc atgaacagca 660

gcggcagcac cggcctgccg aagggggtgg ccctgccgca ccggaccgcc tgcgtgcgct 720

tctcgcacgc ccgggacccc atcttcggca accagatcat cccggacacc gccatcctga 780

gcgtggtgcc gttccaccac ggcttcggca tgttcacgac cctgggctac ctcatctgcg 840

gcttccgggt ggtcctgatg taccggttcg aggaggagct gttcctgcgg agcctgcagg 900

actacaagat ccagagcgcg ctgctcgtgc cgaccctgtt cagcttcttc gccaagagca 960

ccctgatcga caagtacgac ctgtcgaacc tgcacgagat cgccagcggg ggcgccccgc 1020

tgagcaagga ggtgggcgag gccgtggcca agcggttcca cctcccgggc atccgccagg 1080

gctacggcct gaccgagacc acgagcgcga tcctgatcac cccgaggggg gacgacaagc 1140

cgggcgccgt gggcaaggtg gtcccgttct tcgaggccaa ggtggtggac ctggacaccg 1200

gcaagaccct gggcgtgaac cagcggggcg agctgtgcgt gcgggggccg atgatcatga 1260

gcggctacgt gaacaacccg gaggccacca acgccctcat cgacaaggac ggctggctgc 1320

acagcggcga catcgcctac tgggacgagg acgagcactt cttcatcgtc gaccggctga 1380

agtcgctgat caagtacaag ggctaccagg tggcgccggc cgagctggag agcatcctgc 1440

tccagcaccc caacatcttc gacgccggcg tggccgggct gccggacgac gacgccggcg 1500

agctgccggc cgcggtggtg gtgctggagc acggcaagac catgacggag aaggagatcg 1560

tcgactacgt ggccagccag gtgaccaccg ccaagaagct gcggggcggc gtggtgttcg 1620

tggacgaggt cccgaagggc ctgaccggga agctcgacgc ccggaagatc cgcgagatcc 1680

tgatcaaggc caagaagggc ggcaagatcg ccgtgtaaga ctagtgcatc acatttaaaa 1740

gcatctcagc ctaccatgag aataagagaa agaaaatgaa gatcaatagc ttattcatct 1800

ctttttcttt ttcgttggtg taaagccaac accctgtcta aaaaacataa atttctttaa 1860

tcattttgcc tcttttctct gtgcttcaat taataaaaaa tggaaagaac ctagatctaa 1920

aaaaaaaaaa aaaaaaaaaa aaaaaaaaaa aaaaaaaaaa aaaaaaaaaa aaaaaaaaaa 1980

aatgcatccc cccccccccc cccccccccc cccccccaa aggctctttt cagagccacc 2040

agaatt 2046

<210> 1445
<211> 1840
<212> DNA
<213> Artificial Sequence

<220>
<223> RPL35 PpLuc(GC) A64 C30 histoneSL

<400> 1445

```
ggggagcggg cggcggcgtt ggcggcttgt gcagcaaagc ttgaggatgg aggacgccaa      60
gaacatcaag aagggcccgg cgcccttcta cccgctggag gacgggaccg ccggcgagca     120
gctccacaag gccatgaagc ggtacgccct ggtgccgggc acgatcgcct tcaccgacgc     180
ccacatcgag gtcgacatca cctacgcgga gtacttcgag atgagcgtgc gcctggccga     240
ggccatgaag cggtacggcc tgaacaccaa ccaccggatc gtggtgtgct cggagaacag     300
cctgcagttc ttcatgccgg tgctgggcgc cctcttcatc ggcgtggccg tcgccccggc     360
gaacgacatc tacaacgagc gggagctgct gaacagcatg gggatcagcc agccgaccgt     420
ggtgttcgtg agcaagaagg gcctgcagaa gatcctgaac gtgcagaaga agctgcccat     480
catccagaag atcatcatca tggacagcaa gaccgactac cagggcttcc agtcgatgta     540
cacgttcgtg accagccacc tcccgccggg cttcaacgag tacgacttcg tcccggagag     600
cttcgaccgg gacaagacca tcgccctgat catgaacagc agcggcagca ccggcctgcc     660
gaagggggtg gccctgccgc accggaccgc ctgcgtgcgc ttctcgcacg cccgggaccc     720
catcttcggc aaccagatca tcccggacac cgccatcctg agcgtggtgc cgttccacca     780
cggcttcggc atgttcacga ccctgggcta cctcatctgc ggcttccggg tggtcctgat     840
gtaccggttc gaggaggagc tgttcctgcg gagcctgcag gactacaaga tccagagcgc     900
gctgctcgtg ccgaccctgt tcagcttctt cgccaagagc accctgatcg acaagtacga     960
cctgtcgaac ctgcacgaga tcgccagcgg gggcgccccg ctgagcaagg aggtgggcga    1020
ggccgtggcc aagcggttcc acctcccggg catccgccag ggctacggcc tgaccgagac    1080
cacgagcgcg atcctgatca cccccgaggg ggacgacaag ccgggcgccg tgggcaaggt    1140
ggtcccgttc ttcgaggcca aggtggtgga cctggacacc ggcaagaccc tgggcgtgaa    1200
ccagcggggc gagctgtgcg tgcgggggcc gatgatcatg agcggctacg tgaacaaccc    1260
ggaggccacc aacgccctca tcgacaagga cggctggctg cacagcggcg acatcgccta    1320
ctgggacgag gacgagcact tcttcatcgt cgaccggctg aagtcgctga tcaagtacaa    1380
gggctaccag gtggcgccgg ccgagctgga gagcatcctg ctccagcacc ccaacatctt    1440
cgacgccggc gtggccgggc tgccggacga cgacgccggc gagctgccgg ccgcggtggt    1500
ggtgctggag cacggcaaga ccatgacgga gaaggagatc gtcgactacg tggccagcca    1560
ggtgaccacc gccaagaagc tgcggggcgg cgtggtgttc gtggacgagg tcccgaaggg    1620
cctgaccggg aagctcgacg cccggaagat ccgcgagatc ctgatcaagg ccaagaaggg    1680
cggcaagatc gccgtgtaag actagtagat ctaaaaaaaa aaaaaaaaaa aaaaaaaaaa    1740
aaaaaaaaaa aaaaaaaaaa aaaaaaaaaa aaaaaatgca tccccccccc cccccccccc    1800
cccccccccc ccaaaggctc ttttcagagc caccagaatt                          1840
```

<210> 1446
<211> 1840
<212> DNA
<213> Artificial Sequence

<220>
<223> RPL21 PpLuc(GC) A64 C30 histoneSL

<400> 1446

```
ggggccggaa ccgccatctt ccagtaattc gccaaaaagc ttgaggatgg aggacgccaa      60
gaacatcaag aagggcccgg cgcccttcta cccgctggag gacgggaccg ccggcgagca     120
gctccacaag gccatgaagc ggtacgccct ggtgccgggc acgatcgcct tcaccgacgc     180
ccacatcgag gtcgacatca cctacgcgga gtacttcgag atgagcgtgc gcctggccga     240
ggccatgaag cggtacggcc tgaacaccaa ccaccggatc gtggtgtgct cggagaacag     300
cctgcagttc ttcatgccgg tgctgggcgc cctcttcatc ggcgtggccg tcgccccggc     360
gaacgacatc tacaacgagc gggagctgct gaacagcatg gggatcagcc agccgaccgt     420
ggtgttcgtg agcaagaagg gcctgcagaa gatcctgaac gtgcagaaga agctgcccat     480
catccagaag atcatcatca tggacagcaa gaccgactac cagggcttcc agtcgatgta     540
cacgttcgtg accagccacc tcccgccggg cttcaacgag tacgacttcg tcccggagag     600
cttcgaccgg gacaagacca tcgccctgat catgaacagc agcggcagca ccggcctgcc     660
gaagggggtg gccctgccgc accggaccgc ctgcgtgcgc ttctcgcacg cccgggaccc     720
catcttcggc aaccagatca tcccggacac cgccatcctg agcgtggtgc cgttccacca     780
cggcttcggc atgttcacga ccctgggcta cctcatctgc ggcttccggg tggtcctgat     840
gtaccggttc gaggaggagc tgttcctgcg gagcctgcag gactacaaga tccagagcgc     900
gctgctcgtg ccgaccctgt tcagcttctt cgccaagagc accctgatcg acaagtacga     960
```

cctgtcgaac ctgcacgaga tcgccagcgg gggcgccccg ctgagcaagg aggtgggcga 1020

ggccgtggcc aagcggttcc acctcccggg catccgccag ggctacggcc tgaccgagac 1080

cacgagcgcg atcctgatca cccccgaggg ggacgacaag ccgggcgccg tgggcaaggt 1140

ggtcccgttc ttcgaggcca aggtggtgga cctggacacc ggcaagaccc tgggcgtgaa 1200

ccagcggggc gagctgtgcg tgcgggggcc gatgatcatg agcggctacg tgaacaaccc 1260

ggaggccacc aacgccctca tcgacaagga cggctggctg cacagcggcg acatcgccta 1320

ctgggacgag gacgagcact tcttcatcgt cgaccggctg aagtcgctga tcaagtacaa 1380

gggctaccag gtggcgccgg ccgagctgga gagcatcctg ctccagcacc ccaacatctt 1440

cgacgccggc gtggccgggc tgccggacga cgacgccggc gagctgccgg ccgcggtggt 1500

ggtgctggag cacggcaaga ccatgacgga gaaggagatc gtcgactacg tggccagcca 1560

ggtgaccacc gccaagaagc tgcggggcgg cgtggtgttc gtggacgagg tcccgaaggg 1620

cctgaccggg aagctcgacg cccggaagat ccgcgagatc ctgatcaagg ccaagaaggg 1680

cggcaagatc gccgtgtaag actagtagat ctaaaaaaaa aaaaaaaaaa aaaaaaaaaa 1740

aaaaaaaaaa aaaaaaaaaa aaaaaaaaaa aaaaaatgca tccccccccc cccccccccc 1800

cccccccccc ccaaaggctc ttttcagagc caccagaatt 1840

<210> 1447
<211> 1881
<212> DNA
<213> Artificial Sequence

<220>
<223> ATP5A1 PpLuc(GC) A64 - C30 histoneSL

<400> 1447

```
gggcggctcg gccattttgt cccagtcagt ccggaggctg cggctgcaga agtaccgcct        60
gcggagtaac tgcaaagaag cttgaggatg gaggacgcca agaacatcaa gaagggcccg       120
gcgcccttct acccgctgga ggacgggacc gccggcgagc agctccacaa ggccatgaag       180
cggtacgccc tggtgccggg cacgatcgcc ttcaccgacg cccacatcga ggtcgacatc       240
acctacgcgg agtacttcga gatgagcgtg cgcctggccg aggccatgaa gcggtacggc       300
ctgaacacca accaccggat cgtggtgtgc tcggagaaca gcctgcagtt cttcatgccg       360
gtgctgggcg ccctcttcat cggcgtggcc gtcgccccgg cgaacgacat ctacaacgag       420
cgggagctgc tgaacagcat ggggatcagc cagccgaccg tggtgttcgt gagcaagaag       480
ggcctgcaga agatcctgaa cgtgcagaag aagctgccca tcatccagaa gatcatcatc       540
atggacagca agaccgacta ccagggcttc cagtcgatgt acacgttcgt gaccagccac       600
ctcccgccgg gcttcaacga gtacgacttc gtcccggaga gcttcgaccg ggacaagacc       660
```

atcgccctga tcatgaacag cagcggcagc accggcctgc cgaagggggt ggccctgccg 720
caccggaccg cctgcgtgcg cttctcgcac gcccgggacc ccatcttcgg caaccagatc 780
atcccggaca ccgccatcct gagcgtggtg ccgttccacc acggcttcgg catgttcacg 840
accctgggct acctcatctg cggcttccgg gtggtcctga tgtaccggtt cgaggaggag 900
ctgttcctgc ggagcctgca ggactacaag atccagagcg cgctgctcgt gccgaccctg 960
ttcagcttct tcgccaagag caccctgatc gacaagtacg acctgtcgaa cctgcacgag 1020
atcgccagcg gggcgcccc gctgagcaag gaggtgggcg aggccgtggc caagcggttc 1080
cacctcccgg gcatccgcca gggctacggc ctgaccgaga ccacgagcgc gatcctgatc 1140
acccccgagg gggacgacaa gccgggcgcc gtgggcaagg tggtcccgtt cttcgaggcc 1200
aaggtggtgg acctggacac cggcaagacc ctgggcgtga accagcgggg cgagctgtgc 1260
gtgcgggggc cgatgatcat gagcggctac gtgaacaacc cggaggccac caacgccctc 1320
atcgacaagg acggctggct gcacagcggc gacatcgcct actgggacga ggacgagcac 1380
ttcttcatcg tcgaccggct gaagtcgctg atcaagtaca agggctacca ggtggcgccg 1440
gccgagctgg agagcatcct gctccagcac cccaacatct tcgacgccgg cgtggccggg 1500
ctgccggacg acgacgccgg cgagctgccg gccgcggtgg tggtgctgga gcacggcaag 1560
accatgacgg agaaggagat cgtcgactac gtggccagcc aggtgaccac cgccaagaag 1620
ctgcggggcg gcgtggtgtt cgtggacgag gtcccgaagg gcctgaccgg gaagctcgac 1680
gcccggaaga tccgcgagat cctgatcaag gccaagaagg gcggcaagat cgccgtgtaa 1740
gactagtaga tctaaaaaaa aaaaaaaaaa aaaaaaaaaa aaaaaaaaaa aaaaaaaaaa 1800
aaaaaaaaaa aaaaaaatgc atcccccccc cccccccccc cccccccccc cccaaaggct 1860
cttttcagag ccaccagaat t 1881

<210> 1448
<211> 1868
<212> DNA
<213> Artificial Sequence

<220>
<223> HSD17B4 PpLuc(GC) A64 C30 histoneSL

<400> 1448

```
gggtcccgca gtcggcgtcc agcggctctg cttgttcgtg tgtgtgtcgt tgcaggcctt       60
attcaagctt gaggatggag gacgccaaga acatcaagaa gggcccggcg cccttctacc      120
cgctggagga cgggaccgcc ggcgagcagc tccacaaggc catgaagcgg tacgccctgg      180
tgccgggcac gatcgccttc accgacgccc acatcgaggt cgacatcacc tacgcggagt      240
acttcgagat gagcgtgcgc ctggccgagg ccatgaagcg gtacggcctg aacaccaacc      300
accggatcgt ggtgtgctcg gagaacagcc tgcagttctt catgccggtg ctgggcgccc      360
```

tcttcatcgg cgtggccgtc gccccggcga acgacatcta caacgagcgg gagctgctga 420
acagcatggg gatcagccag ccgaccgtgg tgttcgtgag caagaagggc ctgcagaaga 480
tcctgaacgt gcagaagaag ctgcccatca tccagaagat catcatcatg gacagcaaga 540
ccgactacca gggcttccag tcgatgtaca cgttcgtgac cagccacctc ccgccgggct 600
tcaacgagta cgacttcgtc ccggagagct tcgaccggga caagaccatc gccctgatca 660
tgaacagcag cggcagcacc ggcctgccga agggggtggc cctgccgcac cggaccgcct 720
gcgtgcgctt ctcgcacgcc cgggacccca tcttcggcaa ccagatcatc ccggacaccg 780
ccatcctgag cgtggtgccg ttccaccacg gcttcggcat gttcacgacc ctgggctacc 840
tcatctgcgg cttccgggtg gtcctgatgt accggttcga ggaggagctg ttcctgcgga 900
gcctgcagga ctacaagatc cagagcgcgc tgctcgtgcc gaccctgttc agcttcttcg 960
ccaagagcac cctgatcgac aagtacgacc tgtcgaacct gcacgagatc gccagcgggg 1020
gcgccccgct gagcaaggag gtgggcgagg ccgtggccaa gcggttccac ctcccgggca 1080
tccgccaggg ctacggcctg accgagacca cgagcgcgat cctgatcacc cccgaggggg 1140
acgacaagcc gggcgccgtg ggcaaggtgg tcccgttctt cgaggccaag gtggtggacc 1200
tggacaccgg caagaccctg ggcgtgaacc agcggggcga gctgtgcgtg cgggggccga 1260
tgatcatgag cggctacgtg aacaacccgg aggccaccaa cgccctcatc gacaaggacg 1320
gctggctgca cagcggcgac atcgcctact gggacgagga cgagcacttc ttcatcgtcg 1380
accggctgaa gtcgctgatc aagtacaagg gctaccaggt ggcgccggcc gagctggaga 1440
gcatcctgct ccagcacccc aacatcttcg acgccggcgt ggccgggctg ccggacgacg 1500
acgccggcga gctgccggcc gcggtggtgg tgctggagca cggcaagacc atgacggaga 1560
aggagatcgt cgactacgtg gccagccagg tgaccaccgc caagaagctg cggggcggcg 1620
tggtgttcgt ggacgaggtc ccgaagggcc tgaccgggaa gctcgacgcc cggaagatcc 1680
gcgagatcct gatcaaggcc aagaagggcg gcaagatcgc cgtgtaagac tagtagatct 1740
aaaaaaaaaa aaaaaaaaaa aaaaaaaaaa aaaaaaaaaa aaaaaaaaaa aaaaaaaaaa 1800
aaaatgcatc cccccccccc cccccccccc cccccccccc aaaggctctt ttcagagcca 1860
ccagaatt 1868

<210> 1449
<211> 1837
<212> DNA
<213> Artificial Sequence

<220>
<223> AIG1 PpLuc(GC) A64 C30 histoneSL

<400> 1449

gggccgccca gccggtccag gcctctggcg aacaagcttg aggatggagg acgccaagaa 60

catcaagaag ggcccggcgc ccttctaccc gctggaggac gggaccgccg gcgagcagct 120

ccacaaggcc atgaagcggt acgccctggt gccgggcacg atcgccttca ccgacgccca 180

catcgaggtc gacatcacct acgcggagta cttcgagatg agcgtgcgcc tggccgaggc 240

catgaagcgg tacggcctga acaccaacca ccggatcgtg gtgtgctcgg agaacagcct 300

gcagttcttc atgccggtgc tgggcgccct cttcatcggc gtggccgtcg ccccggcgaa 360

cgacatctac aacgagcggg agctgctgaa cagcatgggg atcagccagc cgaccgtggt 420

gttcgtgagc aagaagggcc tgcagaagat cctgaacgtg cagaagaagc tgcccatcat 480

ccagaagatc atcatcatgg acagcaagac cgactaccag ggcttccagt cgatgtacac 540

gttcgtgacc agccacctcc cgccgggctt caacgagtac gacttcgtcc cggagagctt 600

cgaccgggac aagaccatcg ccctgatcat gaacagcagc ggcagcaccg gcctgccgaa 660

gggggtggcc ctgccgcacc ggaccgcctg cgtgcgcttc tcgcacgccc gggaccccat 720

cttcggcaac cagatcatcc cggacaccgc catcctgagc gtggtgccgt tccaccacgg 780

cttcggcatg ttcacgaccc tgggctacct catctgcggc ttccgggtgg tcctgatgta 840

ccggttcgag gaggagctgt tcctgcggag cctgcaggac tacaagatcc agagcgcgct 900

gctcgtgccg accctgttca gcttcttcgc caagagcacc ctgatcgaca agtacgacct 960

gtcgaacctg cacgagatcg ccagcggggg cgccccgctg agcaaggagg tgggcgaggc 1020

cgtggccaag cggttccacc tcccgggcat ccgccagggc tacggcctga ccgagaccac 1080

gagcgcgatc ctgatcaccc ccgaggggga cgacaagccg ggcgccgtgg gcaaggtggt 1140

cccgttcttc gaggccaagg tggtggacct ggacaccggc aagaccctgg gcgtgaacca 1200

gcgggggcgag ctgtgcgtgc gggggccgat gatcatgagc ggctacgtga acaacccgga 1260

ggccaccaac gccctcatcg acaaggacgg ctggctgcac agcggcgaca tcgcctactg 1320

ggacgaggac gagcacttct tcatcgtcga ccggctgaag tcgctgatca agtacaaggg 1380

ctaccaggtg gcgccggccg agctggagag catcctgctc cagcacccca acatcttcga 1440

cgccggcgtg gccgggctgc cggacgacga cgccggcgag ctgccggccg cggtggtggt 1500

gctggagcac ggcaagacca tgacggagaa ggagatcgtc gactacgtgg ccagccaggt 1560

gaccaccgcc aagaagctgc ggggcggcgt ggtgttcgtg gacgaggtcc cgaagggcct 1620

gaccgggaag ctcgacgccc ggaagatccg cgagatcctg atcaaggcca agaagggcgg 1680

caagatcgcc gtgtaagact agtagatcta aaaaaaaaaa aaaaaaaaaa aaaaaaaaaa 1740

aaaaaaaaaa aaaaaaaaaa aaaaaaaaaa aaatgcatcc cccccccccc cccccccccc 1800

cccccccccca aaggctcttt tcagagccac cagaatt 1837

<210> 1450
<211> 1862
<212> DNA
<213> Artificial Sequence

<220>
<223> COX6C PpLuc(GC) A64 C30 histoneSL

<400> 1450

ggagtcagga aggacgttgg tgttgaggtt agcatacgta tcaaggacag taactaccaa 60
gcttgaggat ggaggacgcc aagaacatca agaagggccc ggcgcccttc tacccgctgg 120
aggacgggac cgccggcgag cagctccaca aggccatgaa gcggtacgcc ctggtgccgg 180
gcacgatcgc cttcaccgac gcccacatcg aggtcgacat cacctacgcg gagtacttcg 240
agatgagcgt gcgcctggcc gaggccatga agcggtacgg cctgaacacc aaccaccgga 300
tcgtggtgtg ctcggagaac agcctgcagt tcttcatgcc ggtgctgggc gccctcttca 360
tcggcgtggc cgtcgccccg gcgaacgaca tctacaacga gcgggagctg ctgaacagca 420
tggggatcag ccagccgacc gtggtgttcg tgagcaagaa gggcctgcag aagatcctga 480
acgtgcagaa gaagctgccc atcatccaga agatcatcat catggacagc aagaccgact 540
accagggctt ccagtcgatg tacacgttcg tgaccagcca cctcccgccg ggcttcaacg 600
agtacgactt cgtcccggag agcttcgacc gggacaagac catcgccctg atcatgaaca 660
gcagcggcag caccggcctg ccgaaggggg tggccctgcc gcaccggacc gcctgcgtgc 720
gcttctcgca cgcccgggac cccatcttcg gcaaccagat catcccggac accgccatcc 780
tgagcgtggt gccgttccac cacggcttcg gcatgttcac gaccctgggc tacctcatct 840
gcggcttccg ggtggtcctg atgtaccggt tcgaggagga gctgttcctg cggagcctgc 900
aggactacaa gatccagagc gcgctgctcg tgccgaccct gttcagcttc ttcgccaaga 960
gcaccctgat cgacaagtac gacctgtcga acctgcacga gatcgccagc gggggcgccc 1020
cgctgagcaa ggaggtgggc gaggccgtgg ccaagcggtt ccacctcccg ggcatccgcc 1080
agggctacgg cctgaccgag accacgagcg cgatcctgat cacccccgag ggggacgaca 1140
agccgggcgc cgtgggcaag gtggtcccgt tcttcgaggc caaggtggtg gacctggaca 1200
ccggcaagac cctgggcgtg aaccagcggg gcgagctgtg cgtgcggggg ccgatgatca 1260
tgagcggcta cgtgaacaac ccggaggcca ccaacgccct catcgacaag gacggctggc 1320
tgcacagcgg cgacatcgcc tactgggacg aggacgagca cttcttcatc gtcgaccggc 1380
tgaagtcgct gatcaagtac aagggctacc aggtggcgcc ggccgagctg gagagcatcc 1440
tgctccagca ccccaacatc ttcgacgccg gcgtggccgg gctgccggac gacgacgccg 1500
gcgagctgcc ggccgcggtg gtggtgctgg agcacggcaa gaccatgacg gagaaggaga 1560
tcgtcgacta cgtggccagc caggtgacca ccgccaagaa gctgcggggc ggcgtggtgt 1620

```
tcgtggacga ggtcccgaag ggcctgaccg ggaagctcga cgcccggaag atccgcgaga     1680
tcctgatcaa ggccaagaag ggcggcaaga tcgccgtgta agactagtag atctaaaaaa     1740
aaaaaaaaaa aaaaaaaaaa aaaaaaaaaa aaaaaaaaaa aaaaaaaaaa aaaaaaaatg     1800
catccccccc cccccccccc cccccccccc ccccaaaggc tcttttcaga gccaccagaa     1860
tt                                                                    1862
```

<210> 1451
<211> 1848
<212> DNA
<213> Artificial Sequence

<220>
<223> ASAH1 PpLuc(GC) A64 C30 histoneSL

<400> 1451

```
gggcctctgc tggagtccgg ggagtggcgt tggctgctag agcgaagctt gaggatggag      60
gacgccaaga acatcaagaa gggcccggcg cccttctacc cgctggagga cgggaccgcc     120
ggcgagcagc tccacaaggc catgaagcgg tacgccctgg tgccgggcac gatcgccttc     180
accgacgccc acatcgaggt cgacatcacc tacgcggagt acttcgagat gagcgtgcgc     240
ctggccgagg ccatgaagcg gtacggcctg aacaccaacc accggatcgt ggtgtgctcg     300
gagaacagcc tgcagttctt catgccggtg ctgggcgccc tcttcatcgg cgtggccgtc     360
gccccggcga acgacatcta caacgagcgg gagctgctga acagcatggg gatcagccag     420
ccgaccgtgg tgttcgtgag caagaagggc ctgcagaaga tcctgaacgt gcagaagaag     480
ctgcccatca tccagaagat catcatcatg gacagcaaga ccgactacca gggcttccag     540
tcgatgtaca cgttcgtgac cagccacctc ccgccgggct tcaacgagta cgacttcgtc     600
ccggagagct tcgaccggga caagaccatc gccctgatca tgaacagcag cggcagcacc     660
ggcctgccga agggggtggc cctgccgcac cggaccgcct gcgtgcgctt ctcgcacgcc     720
cgggacccca tcttcggcaa ccagatcatc ccggacaccg ccatcctgag cgtggtgccg     780
ttccaccacg gcttcggcat gttcacgacc ctgggctacc tcatctgcgg cttccgggtg     840
gtcctgatgt accggttcga ggaggagctg ttcctgcgga gcctgcagga ctacaagatc     900
cagagcgcgc tgctcgtgcc gaccctgttc agcttcttcg ccaagagcac cctgatcgac     960
aagtacgacc tgtcgaacct gcacgagatc gccagcgggg gcgccccgct gagcaaggag    1020
gtgggcgagg ccgtggccaa gcggttccac ctcccgggca tccgccaggg ctacggcctg    1080
accgagacca cgagcgcgat cctgatcacc cccgaggggg acgacaagcc gggcgccgtg    1140
ggcaaggtgg tcccgttctt cgaggccaag gtggtggacc tggacaccgg caagaccctg    1200
ggcgtgaacc agcggggcga gctgtgcgtg cgggggccga tgatcatgag cggctacgtg    1260
aacaacccgg aggccaccaa cgccctcatc gacaaggacg gctggctgca cagcggcgac    1320
```

atcgcctact gggacgagga cgagcacttc ttcatcgtcg accggctgaa gtcgctgatc 1380

aagtacaagg gctaccaggt ggcgccggcc gagctggaga gcatcctgct ccagcacccc 1440

aacatcttcg acgccggcgt ggccgggctg ccggacgacg acgccggcga gctgccggcc 1500

gcggtggtgg tgctggagca cggcaagacc atgacggaga aggagatcgt cgactacgtg 1560

gccagccagg tgaccaccgc caagaagctg cggggcggcg tggtgttcgt ggacgaggtc 1620

ccgaagggcc tgaccgggaa gctcgacgcc cggaagatcc gcgagatcct gatcaaggcc 1680

aagaagggcg gcaagatcgc cgtgtaagac tagtagatct aaaaaaaaaa aaaaaaaaaa 1740

aaaaaaaaaa aaaaaaaaaa aaaaaaaaaa aaaaaaaaaa aaaatgcatc cccccccccc 1800

cccccccccc cccccccccc aaaggctctt ttcagagcca ccagaatt 1848

<210> 1452
<211> 34
<212> DNA
<213> Homo sapiens

<400> 1452
ggagcgggcg gcggcgttgg cggcttgtgc agca 34

<210> 1453
<211> 34
<212> DNA
<213> Homo sapiens

<400> 1453
ggccggaacc gccatcttcc agtaattcgc caaa 34

<210> 1454
<211> 75
<212> DNA
<213> Homo sapiens

<400> 1454

gcggctcggc cattttgtcc cagtcagtcc ggaggctgcg gctgcagaag taccgcctgc 60

ggagtaactg caaag 75

<210> 1455
<211> 62
<212> DNA
<213> Homo sapiens

<400> 1455

gtcccgcagt cggcgtccag cggctctgct tgttcgtgtg tgtgtcgttg caggccttat 60

tc 62

<210> 1456

<211> 31
<212> DNA
<213> Homo sapiens

<400> 1456
gccgcccagc cggtccaggc ctctggcgaa c 31

<210> 1457
<211> 56
<212> DNA
<213> Homo sapiens

<400> 1457
agtcaggaag gacgttggtg ttgaggttag catacgtatc aaggacagta actacc 56

<210> 1458
<211> 42
<212> DNA
<213> Homo sapiens

<400> 1458
gcctctgctg gagtccgggg agtggcgttg gctgctagag cg 42

<210> 1459
<211> 34
<212> DNA
<213> Mus musculus

<400> 1459
ggccgccgca gccatcttcc agtaactcgc caaa 34

<210> 1460
<211> 53
<212> DNA
<213> Mus musculus

<400> 1460
gccatcttgg cgcctgtgga ggcctgctgg gaacaggact tctaacagca agt 53

<210> 1461
<211> 73
<212> DNA
<213> Mus musculus

<400> 1461

**tcctcctttc ggccgccgca gccatcttcc agtaactcgc caaaatgcca tcttccagta 60**

**actcgccaaa atg 73**

<210> 1462
<211> 68
<212> DNA
<213> Mus musculus

<400> 1462

cttcctcttt ccgccatctt ggcgcctgtg gaggcctgct gggaacagga cttctaacag 60

caagtatg 68

<210> 1463
<211> 1850
<212> DNA
<213> Artificial Sequence

<220>
<223> RPL32 PpLuc(GC) ag -64

<400> 1463

gggcgctgc ctacggaggt ggcagccatc tccttctcgg catcaagctt gaggatggag 60
gacgccaaga acatcaagaa gggcccggcg cccttctacc cgctggagga cgggaccgcc 120
ggcgagcagc tccacaaggc catgaagcgg tacgccctgg tgccgggcac gatcgccttc 180
accgacgccc acatcgaggt cgacatcacc tacgcggagt acttcgagat gagcgtgcgc 240
ctggccgagg ccatgaagcg gtacggcctg aacaccaacc accggatcgt ggtgtgctcg 300
gagaacagcc tgcagttctt catgccggtg ctgggcgccc tcttcatcgg cgtggccgtc 360
gccccggcga acgacatcta caacgagcgg gagctgctga acagcatggg gatcagccag 420
ccgaccgtgg tgttcgtgag caagaagggc ctgcagaaga tcctgaacgt gcagaagaag 480
ctgcccatca tccagaagat catcatcatg gacagcaaga ccgactacca gggcttccag 540
tcgatgtaca cgttcgtgac cagccacctc ccgccgggct tcaacgagta cgacttcgtc 600
ccggagagct tcgaccggga caagaccatc gccctgatca tgaacagcag cggcagcacc 660
ggcctgccga aggggtggc cctgccgcac cggaccgcct gcgtgcgctt ctcgcacgcc 720
cgggacccca tcttcggcaa ccagatcatc ccggacaccg ccatcctgag cgtggtgccg 780
ttccaccacg gcttcggcat gttcacgacc ctgggctacc tcatctgcgg cttccgggtg 840
gtcctgatgt accggttcga ggaggagctg ttcctgcgga gcctgcagga ctacaagatc 900
cagagcgcgc tgctcgtgcc gaccctgttc agcttcttcg ccaagagcac cctgatcgac 960
aagtacgacc tgtcgaacct gcacgagatc gccagcgggg gcgccccgct gagcaaggag 1020
gtgggcgagg ccgtggccaa gcggttccac ctcccgggca tccgccaggg ctacggcctg 1080
accgagacca cgagcgcgat cctgatcacc cccgaggggg acgacaagcc gggcgccgtg 1140
ggcaaggtgg tcccgttctt cgaggccaag gtggtggacc tggacaccgg caagaccctg 1200
ggcgtgaacc agcggggcga gctgtgcgtg cgggggccga tgatcatgag cggctacgtg 1260
aacaacccgg aggccaccaa cgccctcatc gacaaggacg gctggctgca cagcggcgac 1320
atcgcctact gggacgagga cgagcacttc ttcatcgtcg accggctgaa gtcgctgatc 1380
aagtacaagg gctaccaggt ggcgccggcc gagctggaga gcatcctgct ccagcacccc 1440
aacatcttcg acgccggcgt ggccgggctg ccggacgacg acgccggcga gctgccggcc 1500
gcggtggtgg tgctggagca cggcaagacc atgacggaga aggagatcgt cgactacgtg 1560
gccagccagg tgaccaccgc caagaagctg cggggcggcg tggtgttcgt ggacgaggtc 1620
ccgaagggcc tgaccgggaa gctcgacgcc cggaagatcc gcgagatcct gatcaaggcc 1680
aagaagggcg gcaagatcgc cgtgtaagac tagttataag actgactagc ccgatgggcc 1740
tcccaacggg ccctcctccc ctccttgcac cgagattaat agatctaaaa aaaaaaaaaa 1800
aaaaaaaaaa aaaaaaaaaa aaaaaaaaaa aaaaaaaaaa aaaaaaaaaa 1850

<210> 1464
<211> 1873
<212> DNA
<213> Artificial Sequence

<220>
<223> PpLuc(GC) ag A64 histoneSL

<400> 1464

```
ggggcgctgc ctacggaggt ggcagccatc tccttctcgg catcaagctt gaggatggag      60
gacgccaaga acatcaagaa gggcccggcg cccttctacc cgctggagga cgggaccgcc     120
ggcgagcagc tccacaaggc catgaagcgg tacgccctgg tgccgggcac gatcgccttc     180
accgacgccc acatcgaggt cgacatcacc tacgcggagt acttcgagat gagcgtgcgc     240
ctggccgagg ccatgaagcg gtacggcctg aacaccaacc accggatcgt ggtgtgctcg     300
gagaacagcc tgcagttctt catgccggtg ctgggcgccc tcttcatcgg cgtggccgtc     360
gccccggcga acgacatcta caacgagcgg gagctgctga acagcatggg gatcagccag     420
ccgaccgtgg tgttcgtgag caagaagggc ctgcagaaga tcctgaacgt gcagaagaag     480
ctgcccatca tccagaagat catcatcatg gacagcaaga ccgactacca gggcttccag     540
tcgatgtaca cgttcgtgac cagccacctc ccgccgggct tcaacgagta cgacttcgtc     600
ccggagagct tcgaccggga caagaccatc gccctgatca tgaacagcag cggcagcacc     660
ggcctgccga agggggtggc cctgccgcac cggaccgcct gcgtgcgctt ctcgcacgcc     720
cgggacccca tcttcggcaa ccagatcatc ccggacaccg ccatcctgag cgtggtgccg     780
ttccaccacg gcttcggcat gttcacgacc ctgggctacc tcatctgcgg cttccgggtg     840
gtcctgatgt accggttcga ggaggagctg ttcctgcgga gcctgcagga ctacaagatc     900
cagagcgcgc tgctcgtgcc gaccctgttc agcttcttcg ccaagagcac cctgatcgac     960
aagtacgacc tgtcgaacct gcacgagatc gccagcgggg gcgccccgct gagcaaggag    1020
gtgggcgagg ccgtggccaa gcggttccac ctcccgggca tccgccaggg ctacggcctg    1080
accgagacca cgagcgcgat cctgatcacc cccgaggggg acgacaagcc gggcgccgtg    1140
ggcaaggtgg tcccgttctt cgaggccaag gtggtggacc tggacaccgg caagaccctg    1200
ggcgtgaacc agcggggcga gctgtgcgtg cgggggccga tgatcatgag cggctacgtg    1260
aacaacccgg aggccaccaa cgccctcatc gacaaggacg gctggctgca cagcggcgac    1320
```

```
atcgcctact gggacgagga cgagcacttc ttcatcgtcg accggctgaa gtcgctgatc      1380
aagtacaagg gctaccaggt ggcgccggcc gagctggaga gcatcctgct ccagcacccc      1440
aacatcttcg acgccggcgt ggccgggctg ccggacgacg acgccggcga gctgccggcc      1500
gcggtggtgg tgctggagca cggcaagacc atgacggaga aggagatcgt cgactacgtg      1560
gccagccagg tgaccaccgc caagaagctg cggggcggcg tggtgttcgt ggacgaggtc      1620
ccgaagggcc tgaccgggaa gctcgacgcc cggaagatcc gcgagatcct gatcaaggcc      1680
aagaagggcg gcaagatcgc cgtgtaagac tagttataag actgactagc ccgatgggcc      1740
tcccaacggg ccctcctccc ctccttgcac cgagattaat aaaaaaaaaa aaaaaaaaaa      1800
aaaaaaaaaa aaaaaaaaaa aaaaaaaaaa aaaaaaaaaa aaaatgcatc aaaggctctt      1860
ttcagagcca cca                                                         1873
```

<210> 1465
<211> 2035
<212> DNA
<213> Artificial Sequence

<220>
<223> PpLuc(GC) albumin7 A64 C30 histoneSL

<400> 1465

```
gggggcgctgc ctacggaggt ggcagccatc tccttctcgg catcaagctt gaggatggag      60
gacgccaaga acatcaagaa gggcccggcg cccttctacc cgctggagga cgggaccgcc      120
ggcgagcagc tccacaaggc catgaagcgg tacgccctgg tgccgggcac gatcgccttc      180
accgacgccc acatcgaggt cgacatcacc tacgcggagt acttcgagat gagcgtgcgc      240
ctggccgagg ccatgaagcg gtacggcctg aacaccaacc accggatcgt ggtgtgctcg      300
gagaacagcc tgcagttctt catgccggtg ctgggcgccc tcttcatcgg cgtggccgtc      360
gccccggcga acgacatcta caacgagcgg gagctgctga acagcatggg gatcagccag      420
ccgaccgtgg tgttcgtgag caagaagggc ctgcagaaga tcctgaacgt gcagaagaag      480
ctgcccatca tccagaagat catcatcatg gacagcaaga ccgactacca gggcttccag      540
tcgatgtaca cgttcgtgac cagccacctc ccgccgggct tcaacgagta cgacttcgtc      600
ccggagagct tcgaccggga caagaccatc gccctgatca tgaacagcag cggcagcacc      660
ggcctgccga agggggtggc cctgccgcac cggaccgcct gcgtgcgctt ctcgcacgcc      720
cgggacccca tcttcggcaa ccagatcatc ccggacaccg ccatcctgag cgtggtgccg      780
ttccaccacg gcttcggcat gttcacgacc ctgggctacc tcatctgcgg cttccgggtg      840
gtcctgatgt accggttcga ggaggagctg ttcctgcgga gcctgcagga ctacaagatc      900
cagagcgcgc tgctcgtgcc gaccctgttc agcttcttcg ccaagagcac cctgatcgac      960
aagtacgacc tgtcgaacct gcacgagatc gccagcgggg gcgccccgct gagcaaggag     1020
```

gtgggcgagg ccgtggccaa gcggttccac ctcccgggca tccgccaggg ctacggcctg 1080
accgagacca cgagcgcgat cctgatcacc cccgaggggg acgacaagcc gggcgccgtg 1140
ggcaaggtgg tcccgttctt cgaggccaag gtggtggacc tggacaccgg caagaccctg 1200
ggcgtgaacc agcggggcga gctgtgcgtg cgggggccga tgatcatgag cggctacgtg 1260
aacaacccgg aggccaccaa cgccctcatc gacaaggacg gctggctgca cagcggcgac 1320
atcgcctact gggacgagga cgagcacttc ttcatcgtcg accggctgaa gtcgctgatc 1380
aagtacaagg gctaccaggt ggcgccggcc gagctggaga gcatcctgct ccagcacccc 1440
aacatcttcg acgccggcgt ggccgggctg ccggacgacg acgccggcga gctgccggcc 1500
gcggtggtgg tgctggagca cggcaagacc atgacggaga aggagatcgt cgactacgtg 1560
gccagccagg tgaccaccgc caagaagctg cggggcggcg tggtgttcgt ggacgaggtc 1620
ccgaagggcc tgaccgggaa gctcgacgcc cggaagatcc gcgagatcct gatcaaggcc 1680
aagaagggcg gcaagatcgc cgtgtaagac tagtgcatca catttaaaag catctcagcc 1740
taccatgaga ataagagaaa gaaaatgaag atcaatagct tattcatctc tttttctttt 1800
tcgttggtgt aaagccaaca ccctgtctaa aaaacataaa tttctttaat cattttgcct 1860
cttttctctg tgcttcaatt aataaaaaat ggaaagaacc tagatctaaa aaaaaaaaaa 1920
aaaaaaaaaa aaaaaaaaaa aaaaaaaaaa aaaaaaaaaa aaaaaaaaaa atgcatcccc 1980
cccccccccc cccccccccc cccccccaaa ggctcttttc agagccacca gaatt 2035

<210> 1466
<211> 1836
<212> DNA
<213> Artificial Sequence

<220>
<223> RPL35 PpLuc(GC) ag A64

<400> 1466

```
ggggagcggg cggcggcgtt ggcggcttgt gcagcaaagc ttgaggatgg aggacgccaa      60
gaacatcaag aagggcccgg cgcccttcta cccgctggag gacgggaccg ccggcgagca     120
gctccacaag gccatgaagc ggtacgccct ggtgccgggc acgatcgcct tcaccgacgc     180
ccacatcgag gtcgacatca cctacgcgga gtacttcgag atgagcgtgc gcctggccga     240
ggccatgaag cggtacggcc tgaacaccaa ccaccggatc gtggtgtgct cggagaacag     300
cctgcagttc ttcatgccgg tgctgggcgc cctcttcatc ggcgtggccg tcgccccggc     360
gaacgacatc tacaacgagc gggagctgct gaacagcatg gggatcagcc agccgaccgt     420
ggtgttcgtg agcaagaagg gcctgcagaa gatcctgaac gtgcagaaga agctgcccat     480
catccagaag atcatcatca tggacagcaa gaccgactac cagggcttcc agtcgatgta     540
```

cacgttcgtg accagccacc tcccgccggg cttcaacgag tacgacttcg tcccggagag 600
cttcgaccgg gacaagacca tcgccctgat catgaacagc agcggcagca ccggcctgcc 660
gaagggggtg gccctgccgc accggaccgc ctgcgtgcgc ttctcgcacg cccgggaccc 720
catcttcggc aaccagatca tcccggacac cgccatcctg agcgtggtgc cgttccacca 780
cggcttcggc atgttcacga ccctgggcta cctcatctgc ggcttccggg tggtcctgat 840
gtaccggttc gaggaggagc tgttcctgcg gagcctgcag gactacaaga tccagagcgc 900
gctgctcgtg ccgaccctgt tcagcttctt cgccaagagc accctgatcg acaagtacga 960
cctgtcgaac ctgcacgaga tcgccagcgg gggcgccccg ctgagcaagg aggtgggcga 1020
ggccgtggcc aagcggttcc acctcccggg catccgccag ggctacggcc tgaccgagac 1080
cacgagcgcg atcctgatca cccccgaggg ggacgacaag ccgggcgccg tgggcaaggt 1140
ggtcccgttc ttcgaggcca aggtggtgga cctggacacc ggcaagaccc tgggcgtgaa 1200
ccagcggggc gagctgtgcg tgcgggggcc gatgatcatg agcggctacg tgaacaaccc 1260
ggaggccacc aacgccctca tcgacaagga cggctggctg cacagcggcg acatcgccta 1320
ctgggacgag gacgagcact tcttcatcgt cgaccggctg aagtcgctga tcaagtacaa 1380
gggctaccag gtggcgccgg ccgagctgga gagcatcctg ctccagcacc ccaacatctt 1440
cgacgccggc gtggccgggc tgccggacga cgacgccggc gagctgccgg ccgcggtggt 1500
ggtgctggag cacggcaaga ccatgacgga gaaggagatc gtcgactacg tggccagcca 1560
ggtgaccacc gccaagaagc tgcggggcgg cgtggtgttc gtggacgagg tcccgaaggg 1620
cctgaccggg aagctcgacg cccggaagat ccgcgagatc ctgatcaagg ccaagaaggg 1680
cggcaagatc gccgtgtaag actagttata agactgacta gcccgatggg cctcccaacg 1740
ggccctcctc ccctccttgc accgagatta ataaaaaaaa aaaaaaaaaa aaaaaaaaaa 1800
aaaaaaaaaa aaaaaaaaaa aaaaaaaaaa aaaaaa 1836

<210> 1467
<211> 1836
<212> DNA
<213> Artificial Sequence

<220>
<223> RPL21 PpLuc(GC) ag A64

<400> 1467

```
ggggccggaa ccgccatctt ccagtaattc gccaaaaagc ttgaggatgg aggacgccaa      60
gaacatcaag aagggcccgg cgcccttcta cccgctggag gacgggaccg ccggcgagca     120
gctccacaag gccatgaagc ggtacgccct ggtgccgggc acgatcgcct tcaccgacgc     180
ccacatcgag gtcgacatca cctacgcgga gtacttcgag atgagcgtgc gcctggccga     240
ggccatgaag cggtacggcc tgaacaccaa ccaccggatc gtggtgtgct cggagaacag     300
cctgcagttc ttcatgccgg tgctgggcgc cctcttcatc ggcgtggccg tcgccccggc     360
gaacgacatc tacaacgagc gggagctgct gaacagcatg gggatcagcc agccgaccgt     420
ggtgttcgtg agcaagaagg gcctgcagaa gatcctgaac gtgcagaaga agctgcccat     480
catccagaag atcatcatca tggacagcaa gaccgactac cagggcttcc agtcgatgta     540
cacgttcgtg accagccacc tcccgccggg cttcaacgag tacgacttcg tcccggagag     600
cttcgaccgg gacaagacca tcgccctgat catgaacagc agcggcagca ccggcctgcc     660
gaagggggtg gccctgccgc accggaccgc ctgcgtgcgc ttctcgcacg cccgggaccc     720
catcttcggc aaccagatca tcccggacac cgccatcctg agcgtggtgc cgttccacca     780
cggcttcggc atgttcacga ccctgggcta cctcatctgc ggcttccggg tggtcctgat     840
gtaccggttc gaggaggagc tgttcctgcg gagcctgcag gactacaaga tccagagcgc     900
gctgctcgtg ccgaccctgt tcagcttctt cgccaagagc accctgatcg acaagtacga     960
cctgtcgaac ctgcacgaga tcgccagcgg gggcgccccg ctgagcaagg aggtgggcga    1020
ggccgtggcc aagcggttcc acctcccggg catccgccag ggctacggcc tgaccgagac    1080
cacgagcgcg atcctgatca cccccgaggg ggacgacaag ccgggcgccg tgggcaaggt    1140
ggtcccgttc ttcgaggcca aggtggtgga cctggacacc ggcaagaccc tgggcgtgaa    1200
ccagcggggc gagctgtgcg tgcgggggcc gatgatcatg agcggctacg tgaacaaccc    1260
ggaggccacc aacgccctca tcgacaagga cggctggctg cacagcggcg acatcgccta    1320
ctgggacgag gacgagcact tcttcatcgt cgaccggctg aagtcgctga tcaagtacaa    1380
gggctaccag gtggcgccgg ccgagctgga gagcatcctg ctccagcacc ccaacatctt    1440
cgacgccggc gtggccgggc tgccggacga cgacgccggc gagctgccgg ccgcggtggt    1500
ggtgctggag cacggcaaga ccatgacgga gaaggagatc gtcgactacg tggccagcca    1560
ggtgaccacc gccaagaagc tgcggggcgg cgtggtgttc gtggacgagg tcccgaaggg    1620
cctgaccggg aagctcgacg cccggaagat ccgcgagatc ctgatcaagg ccaagaaggg    1680
cggcaagatc gccgtgtaag actagttata agactgacta gcccgatggg cctcccaacg    1740
ggccctcctc ccctccttgc accgagatta ataaaaaaaa aaaaaaaaaa aaaaaaaaaa    1800
aaaaaaaaaa aaaaaaaaaa aaaaaaaaaa aaaaaa                              1836
```

<210> 1468
<211> 1877
<212> DNA
<213> Artificial Sequence

<220>
<223> atp5a1 PpLuc(GC) -g A64

<400> 1468

gggcggctcg gccattttgt cccagtcagt ccggaggctg cggctgcaga agtaccgcct 60
gcggagtaac tgcaaagaag cttgaggatg gaggacgcca agaacatcaa gaagggcccg 120
gcgcccttct acccgctgga ggacgggacc gccggcgagc agctccacaa ggccatgaag 180
cggtacgccc tggtgccggg cacgatcgcc ttcaccgacg cccacatcga ggtcgacatc 240
acctacgcgg agtacttcga gatgagcgtg cgcctggccg aggccatgaa gcggtacggc 300
ctgaacacca accaccggat cgtggtgtgc tcggagaaca gcctgcagtt cttcatgccg 360
gtgctgggcg ccctcttcat cggcgtggcc gtcgccccgg cgaacgacat ctacaacgag 420
cgggagctgc tgaacagcat ggggatcagc cagccgaccg tggtgttcgt gagcaagaag 480
ggcctgcaga agatcctgaa cgtgcagaag aagctgccca tcatccagaa gatcatcatc 540
atggacagca agaccgacta ccagggcttc cagtcgatgt acacgttcgt gaccagccac 600
ctcccgccgg gcttcaacga gtacgacttc gtcccggaga gcttcgaccg ggacaagacc 660
atcgccctga tcatgaacag cagcggcagc accggcctgc cgaagggggt ggccctgccg 720
caccggaccg cctgcgtgcg cttctcgcac gcccgggacc ccatcttcgg caaccagatc 780
atcccggaca ccgccatcct gagcgtggtg ccgttccacc acggcttcgg catgttcacg 840
accctgggct acctcatctg cggcttccgg gtggtcctga tgtaccggtt cgaggaggag 900
ctgttcctgc ggagcctgca ggactacaag atccagagcg cgctgctcgt gccgaccctg 960
ttcagcttct tcgccaagag caccctgatc gacaagtacg acctgtcgaa cctgcacgag 1020
atcgccagcg gggcgccccc gctgagcaag gaggtgggcg aggccgtggc caagcggttc 1080
cacctcccgg gcatccgcca gggctacggc ctgaccgaga ccacgagcgc gatcctgatc 1140
acccccgagg gggacgacaa gccgggcgcc gtgggcaagg tggtcccgtt cttcgaggcc 1200
aaggtggtgg acctggacac cggcaagacc ctgggcgtga accagcgggg cgagctgtgc 1260
gtgcgggggc cgatgatcat gagcggctac gtgaacaacc cggaggccac caacgccctc 1320
atcgacaagg acggctggct gcacagcggc gacatcgcct actgggacga ggacgagcac 1380
ttcttcatcg tcgaccggct gaagtcgctg atcaagtaca agggctacca ggtggcgccg 1440
gccgagctgg agagcatcct gctccagcac cccaacatct tcgacgccgg cgtggccggg 1500
ctgccggacg acgacgccgg cgagctgccg gccgcggtgg tggtgctgga gcacggcaag 1560
accatgacgg agaaggagat cgtcgactac gtggccagcc aggtgaccac cgccaagaag 1620
ctgcggggcg gcgtggtgtt cgtggacgag gtcccgaagg gcctgaccgg gaagctcgac 1680
gcccggaaga tccgcgagat cctgatcaag gccaagaagg gcggcaagat cgccgtgtaa 1740
gactagttat aagactgact agcccgatgg gcctcccaac gggccctcct cccctccttg 1800
caccgagatt aataaaaaaa aaaaaaaaaa aaaaaaaaaa aaaaaaaaaa aaaaaaaaaa 1860
aaaaaaaaaa aaaaaaa 1877

<210> 1469
<211> 1864
<212> DNA
<213> Artificial Sequence

<220>
<223> HSD17B4 PpLuc(GC) ag A64

<400> 1469

```
gggtcccgca gtcggcgtcc agcggctctg cttgttcgtg tgtgtgtcgt tgcaggcctt      60
attcaagctt gaggatggag gacgccaaga acatcaagaa gggcccggcg cccttctacc     120
cgctggagga cgggaccgcc ggcgagcagc tccacaaggc catgaagcgg tacgccctgg     180
tgccgggcac gatcgccttc accgacgccc acatcgaggt cgacatcacc tacgcggagt     240
acttcgagat gagcgtgcgc ctggccgagg ccatgaagcg gtacggcctg aacaccaacc     300
accggatcgt ggtgtgctcg gagaacagcc tgcagttctt catgccggtg ctgggcgccc     360
tcttcatcgg cgtggccgtc gccccggcga acgacatcta caacgagcgg gagctgctga     420
acagcatggg gatcagccag ccgaccgtgg tgttcgtgag caagaagggc ctgcagaaga     480
tcctgaacgt gcagaagaag ctgcccatca tccagaagat catcatcatg gacagcaaga     540
ccgactacca gggcttccag tcgatgtaca cgttcgtgac cagccacctc ccgccgggct     600
tcaacgagta cgacttcgtc ccggagagct tcgaccggga caagaccatc gccctgatca     660
tgaacagcag cggcagcacc ggcctgccga agggggtggc cctgccgcac cggaccgcct     720
gcgtgcgctt ctcgcacgcc cgggacccca tcttcggcaa ccagatcatc ccggacaccg     780
ccatcctgag cgtggtgccg ttccaccacg gcttcggcat gttcacgacc ctgggctacc     840
tcatctgcgg cttccgggtg gtcctgatgt accggttcga ggaggagctg ttcctgcgga     900
gcctgcagga ctacaagatc cagagcgcgc tgctcgtgcc gaccctgttc agcttcttcg     960
ccaagagcac cctgatcgac aagtacgacc tgtcgaacct gcacgagatc gccagcgggg    1020
gcgccccgct gagcaaggag gtgggcgagg ccgtggccaa gcggttccac ctcccgggca    1080
tccgccaggg ctacggcctg accgagacca cgagcgcgat cctgatcacc cccgaggggg    1140
acgacaagcc gggcgccgtg ggcaaggtgg tcccgttctt cgaggccaag gtggtggacc    1200
tggacaccgg caagaccctg ggcgtgaacc agcggggcga gctgtgcgtg cgggggccga    1260
tgatcatgag cggctacgtg aacaacccgg aggccaccaa cgccctcatc gacaaggacg    1320
gctggctgca cagcggcgac atcgcctact gggacgagga cgagcacttc ttcatcgtcg    1380
accggctgaa gtcgctgatc aagtacaagg gctaccaggt ggcgccggcc gagctggaga    1440
gcatcctgct ccagcacccc aacatcttcg acgccggcgt ggccgggctg ccggacgacg    1500
acgccggcga gctgccggcc gcggtggtgg tgctggagca cggcaagacc atgacggaga    1560
```

```
aggagatcgt cgactacgtg gccagccagg tgaccaccgc caagaagctg cggggcggcg      1620
tggtgttcgt ggacgaggtc ccgaagggcc tgaccgggaa gctcgacgcc cggaagatcc      1680
gcgagatcct gatcaaggcc aagaagggcg gcaagatcgc cgtgtaagac tagttataag      1740
actgactagc ccgatgggcc tcccaacggg ccctcctccc ctccttgcac cgagattaat      1800
aaaaaaaaaa aaaaaaaaaa aaaaaaaaaa aaaaaaaaaa aaaaaaaaaa aaaaaaaaaa      1860
aaaa                                                                    1864
```

<210> 1470
<211> 1833
<212> DNA
<213> Artificial Sequence

<220>
<223> AIG1 PpLuc(GC) ag A64

<400> 1470

```
gggccgccca gccggtccag gcctctggcg aacaagcttg aggatggagg acgccaagaa        60
catcaagaag ggcccggcgc ccttctaccc gctggaggac gggaccgccg gcgagcagct       120
ccacaaggcc atgaagcggt acgccctggt gccgggcacg atcgccttca ccgacgccca       180
catcgaggtc gacatcacct acgcggagta cttcgagatg agcgtgcgcc tggccgaggc       240
catgaagcgg tacggcctga acaccaacca ccggatcgtg gtgtgctcgg agaacagcct       300
gcagttcttc atgccggtgc tgggcgccct cttcatcggc gtggccgtcg ccccggcgaa       360
cgacatctac aacgagcggg agctgctgaa cagcatgggg atcagccagc cgaccgtggt       420
gttcgtgagc aagaagggcc tgcagaagat cctgaacgtg cagaagaagc tgcccatcat       480
ccagaagatc atcatcatgg acagcaagac cgactaccag ggcttccagt cgatgtacac       540
gttcgtgacc agccacctcc cgcccgggctt caacgagtac gacttcgtcc cggagagctt       600
cgaccgggac aagaccatcg ccctgatcat gaacagcagc ggcagcaccg gcctgccgaa       660
gggggtggcc ctgccgcacc ggaccgcctg cgtgcgcttc tcgcacgccc gggaccccat       720
cttcggcaac cagatcatcc cggacaccgc catcctgagc gtggtgccgt tccaccacgg       780
cttcggcatg ttcacgaccc tgggctacct catctgcggc ttccgggtgg tcctgatgta       840
ccggttcgag gaggagctgt tcctgcggag cctgcaggac tacaagatcc agagcgcgct       900
gctcgtgccg accctgttca gcttcttcgc caagagcacc ctgatcgaca agtacgacct       960
gtcgaacctg cacgagatcg ccagcggggg cgccccgctg agcaaggagg tgggcgaggc      1020
cgtggccaag cggttccacc tcccgggcat ccgccagggc tacggcctga ccgagaccac      1080
gagcgcgatc ctgatcaccc ccgaggggga cgacaagccg ggcgccgtgg gcaaggtggt      1140
cccgttcttc gaggccaagg tggtggacct ggacaccggc aagaccctgg gcgtgaacca      1200
gcggggcgag ctgtgcgtgc gggggccgat gatcatgagc ggctacgtga acaacccgga      1260
ggccaccaac gccctcatcg acaaggacgg ctggctgcac agcggcgaca tcgcctactg      1320
ggacgaggac gagcacttct tcatcgtcga ccggctgaag tcgctgatca agtacaaggg      1380
ctaccaggtg gcgccggccg agctggagag catcctgctc cagcacccca acatcttcga      1440
cgccggcgtg gccgggctgc cggacgacga cgccggcgag ctgccggccg cggtggtggt      1500
gctggagcac ggcaagacca tgacggagaa ggagatcgtc gactacgtgg ccagccaggt      1560
gaccaccgcc aagaagctgc ggggcggcgt ggtgttcgtg gacgaggtcc cgaagggcct      1620
gaccgggaag ctcgacgccc ggaagatccg cgagatcctg atcaaggcca agaagggcgg      1680
caagatcgcc gtgtaagact agttataaga ctgactagcc cgatgggcct cccaacgggc      1740
cctcctcccc tccttgcacc gagattaata aaaaaaaaaa aaaaaaaaaa aaaaaaaaaa      1800
aaaaaaaaaa aaaaaaaaaa aaaaaaaaaa aaa                                  1833
```

<210> 1471
<211> 1858
<212> DNA
<213> Artificial Sequence

<220>
<223> COX6C PpLuc(GC) ag -64

<400> 1471

```
ggagtcagga aggacgttgg tgttgaggtt agcatacgta tcaaggacag taactaccaa      60
gcttgaggat ggaggacgcc aagaacatca agaagggccc ggcgcccttc tacccgctgg     120
aggacgggac cgccggcgag cagctccaca aggccatgaa gcggtacgcc ctggtgccgg     180
gcacgatcgc cttcaccgac gcccacatcg aggtcgacat cacctacgcg gagtacttcg     240
agatgagcgt gcgcctggcc gaggccatga agcggtacgg cctgaacacc aaccaccgga     300
tcgtggtgtg ctcggagaac agcctgcagt tcttcatgcc ggtgctgggc gccctcttca     360
tcggcgtggc cgtcgccccg gcgaacgaca tctacaacga gcgggagctg ctgaacagca     420
tggggatcag ccagccgacc gtggtgttcg tgagcaagaa gggcctgcag aagatcctga     480
acgtgcagaa gaagctgccc atcatccaga agatcatcat catggacagc aagaccgact     540
accagggctt ccagtcgatg tacacgttcg tgaccagcca cctcccgccg ggcttcaacg     600
agtacgactt cgtcccggag agcttcgacc gggacaagac catcgccctg atcatgaaca     660
gcagcggcag caccggcctg ccgaaggggg tggccctgcc gcaccggacc gcctgcgtgc     720
gcttctcgca cgcccgggac cccatcttcg gcaaccagat catcccggac accgccatcc     780
tgagcgtggt gccgttccac cacggcttcg gcatgttcac gaccctgggc tacctcatct     840
gcggcttccg ggtggtcctg atgtaccggt tcgaggagga gctgttcctg cggagcctgc     900
aggactacaa gatccagagc gcgctgctcg tgccgaccct gttcagcttc ttcgccaaga     960
```

gcaccctgat cgacaagtac gacctgtcga acctgcacga gatcgccagc gggggcgccc 1020

cgctgagcaa ggaggtgggc gaggccgtgg ccaagcggtt ccacctcccg ggcatccgcc 1080

agggctacgg cctgaccgag accacgagcg cgatcctgat cacccccgag gggacgaca 1140

agccgggcgc cgtgggcaag gtggtcccgt tcttcgaggc caaggtggtg gacctggaca 1200

ccggcaagac cctgggcgtg aaccagcggg gcgagctgtg cgtgcggggg ccgatgatca 1260

tgagcggcta cgtgaacaac ccggaggcca ccaacgccct catcgacaag gacggctggc 1320

tgcacagcgg cgacatcgcc tactgggacg aggacgagca cttcttcatc gtcgaccggc 1380

tgaagtcgct gatcaagtac aagggctacc aggtggcgcc ggccgagctg gagagcatcc 1440

tgctccagca ccccaacatc ttcgacgccg gcgtggccgg gctgccggac gacgacgccg 1500

gcgagctgcc ggccgcggtg gtggtgctgg agcacggcaa gaccatgacg gagaaggaga 1560

tcgtcgacta cgtggccagc caggtgacca ccgccaagaa gctgcggggc ggcgtggtgt 1620

tcgtggacga ggtcccgaag ggcctgaccg ggaagctcga cgcccggaag atccgcgaga 1680

tcctgatcaa ggccaagaag ggcggcaaga tcgccgtgta agactagtta taagactgac 1740

tagcccgatg ggcctcccaa cgggccctcc tcccctcctt gcaccgagat taataaaaaa 1800

aaaaaaaaaa aaaaaaaaaa aaaaaaaaaa aaaaaaaaaa aaaaaaaaaa aaaaaaaa 1858

<210> 1472
<211> 1844
<212> DNA
<213> Artificial Sequence

<220>
<223> ASAH1 PpLuc(GC) ag A64

<400> 1472

gggcctctgc tggagtccgg ggagtggcgt tggctgctag agcgaagctt gaggatggag 60

gacgccaaga acatcaagaa gggcccggcg cccttctacc cgctggagga cgggaccgcc 120

ggcgagcagc tccacaaggc catgaagcgg tacgccctgg tgccgggcac gatcgccttc 180

accgacgccc acatcgaggt cgacatcacc tacgcggagt acttcgagat gagcgtgcgc 240

ctggccgagg ccatgaagcg gtacggcctg aacaccaacc accggatcgt ggtgtgctcg 300

gagaacagcc tgcagttctt catgccggtg ctgggcgccc tcttcatcgg cgtggccgtc 360

gccccggcga acgacatcta caacgagcgg gagctgctga acagcatggg gatcagccag 420

ccgaccgtgg tgttcgtgag caagaagggc ctgcagaaga tcctgaacgt gcagaagaag 480

ctgcccatca tccagaagat catcatcatg gacagcaaga ccgactacca gggcttccag 540

tcgatgtaca cgttcgtgac cagccacctc ccgccgggct tcaacgagta cgacttcgtc 600

ccggagagct tcgaccggga caagaccatc gccctgatca tgaacagcag cggcagcacc 660

ggcctgccga agggggtggc cctgccgcac cggaccgcct gcgtgcgctt ctcgcacgcc 720

cgggacccca tcttcggcaa ccagatcatc ccggacaccg ccatcctgag cgtggtgccg 780

ttccaccacg gcttcggcat gttcacgacc ctgggctacc tcatctgcgg cttccgggtg 840

gtcctgatgt accggttcga ggaggagctg ttcctgcgga gcctgcagga ctacaagatc 900

cagagcgcgc tgctcgtgcc gaccctgttc agcttcttcg ccaagagcac cctgatcgac 960

aagtacgacc tgtcgaacct gcacgagatc gccagcgggg gcgccccgct gagcaaggag 1020

gtgggcgagg ccgtggccaa gcggttccac ctcccgggca tccgccaggg ctacggcctg 1080

accgagacca cgagcgcgat cctgatcacc cccgaggggg acgacaagcc gggcgccgtg 1140

ggcaaggtgg tcccgttctt cgaggccaag gtggtggacc tggacaccgg caagaccctg 1200

ggcgtgaacc agcggggcga gctgtgcgtg cgggggccga tgatcatgag cggctacgtg 1260

aacaacccgg aggccaccaa cgccctcatc gacaaggacg gctggctgca cagcggcgac 1320

atcgcctact gggacgagga cgagcacttc ttcatcgtcg accggctgaa gtcgctgatc 1380

aagtacaagg gctaccaggt ggcgccggcc gagctggaga gcatcctgct ccagcacccc 1440

aacatcttcg acgccggcgt ggccgggctg ccggacgacg acgccggcga gctgccggcc 1500

gcggtggtgg tgctggagca cggcaagacc atgacggaga aggagatcgt cgactacgtg 1560

gccagccagg tgaccaccgc caagaagctg cggggcggcg tggtgttcgt ggacgaggtc 1620

ccgaagggcc tgaccgggaa gctcgacgcc cggaagatcc gcgagatcct gatcaaggcc 1680

aagaagggcg gcaagatcgc cgtgtaagac tagttataag actgactagc ccgatgggcc 1740

tcccaacggg ccctcctccc ctccttgcac cgagattaat aaaaaaaaaa aaaaaaaaaa 1800

aaaaaaaaaa aaaaaaaaaa aaaaaaaaaa aaaaaaaaaa aaaa 1844

<210> 1473
<211> 1865
<212> DNA
<213> Artificial Sequence

<220>
<223> RPL35 PpLuc(GC) ag A64 histoneSL

<400> 1473

```
ggggagcggg cggcggcgtt ggcggcttgt gcagcaaagc ttgaggatgg aggacgccaa        60
gaacatcaag aagggcccgg cgcccttcta cccgctggag gacgggaccg ccggcgagca       120
gctccacaag gccatgaagc ggtacgccct ggtgccgggc acgatcgcct tcaccgacgc       180
ccacatcgag gtcgacatca cctacgcgga gtacttcgag atgagcgtgc gcctggccga       240
ggccatgaag cggtacggcc tgaacaccaa ccaccggatc gtggtgtgct cggagaacag       300
cctgcagttc ttcatgccgg tgctgggcgc cctcttcatc ggcgtggccg tcgccccggc       360
gaacgacatc tacaacgagc gggagctgct gaacagcatg ggcatcagcc agccgaccgt       420
```

ggtgttcgtg agcaagaagg gcctgcagaa gatcctgaac gtgcagaaga agctgcccat 480
catccagaag atcatcatca tggacagcaa gaccgactac cagggcttcc agtcgatgta 540
cacgttcgtg accagccacc tcccgccggg cttcaacgag tacgacttcg tcccggagag 600
cttcgaccgg gacaagacca tcgccctgat catgaacagc agcggcagca ccggcctgcc 660
gaagggggtg gccctgccgc accggaccgc ctgcgtgcgc ttctcgcacg cccgggaccc 720
catcttcggc aaccagatca tcccggacac cgccatcctg agcgtggtgc cgttccacca 780
cggcttcggc atgttcacga ccctgggcta cctcatctgc ggcttccggg tggtcctgat 840
gtaccggttc gaggaggagc tgttcctgcg gagcctgcag gactacaaga tccagagcgc 900
gctgctcgtg ccgaccctgt tcagcttctt cgccaagagc accctgatcg acaagtacga 960
cctgtcgaac ctgcacgaga tcgccagcgg gggcgccccg ctgagcaagg aggtgggcga 1020
ggccgtggcc aagcggttcc acctcccggg catccgccag ggctacggcc tgaccgagac 1080
cacgagcgcg atcctgatca cccccgaggg ggacgacaag ccgggcgccg tgggcaaggt 1140
ggtcccgttc ttcgaggcca aggtggtgga cctggacacc ggcaagaccc tgggcgtgaa 1200
ccagcggggc gagctgtgcg tgcgggggcc gatgatcatg agcggctacg tgaacaaccc 1260
ggaggccacc aacgccctca tcgacaagga cggctggctg cacagcggcg acatcgccta 1320
ctgggacgag gacgagcact tcttcatcgt cgaccggctg aagtcgctga tcaagtacaa 1380
gggctaccag gtggcgccgg ccgagctgga gagcatcctg ctccagcacc ccaacatctt 1440
cgacgccggc gtggccgggc tgccggacga cgacgccggc gagctgccgg ccgcggtggt 1500
ggtgctggag cacggcaaga ccatgacgga gaaggagatc gtcgactacg tggccagcca 1560
ggtgaccacc gccaagaagc tgcggggcgg cgtggtgttc gtggacgagg tcccgaaggg 1620
cctgaccggg aagctcgacg cccggaagat ccgcgagatc ctgatcaagg ccaagaaggg 1680
cggcaagatc gccgtgtaag actagttata agactgacta gcccgatggg cctcccaacg 1740
ggccctcctc ccctccttgc accgagatta ataaaaaaaa aaaaaaaaaa aaaaaaaaaa 1800
aaaaaaaaaa aaaaaaaaaa aaaaaaaaaa aaaaaatgca tcaaaggctc ttttcagagc 1860
cacca 1865

<210> 1474
<211> 1865
<212> DNA
<213> Artificial Sequence

<220>
<223> RPL21 PpLuc(GC) ag A64 histoneSL

<400> 1474

gggggccggaa ccgccatctt ccagtaattc gccaaaaagc ttgaggatgg aggacgccaa 60

gaacatcaag aagggcccgg cgcccttcta cccgctggag gacgggaccg ccggcgagca 120

gctccacaag gccatgaagc ggtacgccct ggtgccgggc acgatcgcct tcaccgacgc 180
ccacatcgag gtcgacatca cctacgcgga gtacttcgag atgagcgtgc gcctggccga 240
ggccatgaag cggtacggcc tgaacaccaa ccaccggatc gtggtgtgct cggagaacag 300
cctgcagttc ttcatgccgg tgctgggcgc cctcttcatc ggcgtggccg tcgccccggc 360
gaacgacatc tacaacgagc gggagctgct gaacagcatg gggatcagcc agccgaccgt 420
ggtgttcgtg agcaagaagg gcctgcagaa gatcctgaac gtgcagaaga agctgcccat 480
catccagaag atcatcatca tggacagcaa gaccgactac cagggcttcc agtcgatgta 540
cacgttcgtg accagccacc tcccgccggg cttcaacgag tacgacttcg tcccggagag 600
cttcgaccgg gacaagacca tcgccctgat catgaacagc agcggcagca ccggcctgcc 660
gaagggggtg gccctgccgc accggaccgc ctgcgtgcgc ttctcgcacg cccgggaccc 720
catcttcggc aaccagatca tcccggacac cgccatcctg agcgtggtgc cgttccacca 780
cggcttcggc atgttcacga ccctgggcta cctcatctgc ggcttccggg tggtcctgat 840
gtaccggttc gaggaggagc tgttcctgcg gagcctgcag gactacaaga tccagagcgc 900
gctgctcgtg ccgaccctgt tcagcttctt cgccaagagc accctgatcg acaagtacga 960
cctgtcgaac ctgcacgaga tcgccagcgg gggcgccccg ctgagcaagg aggtgggcga 1020
ggccgtggcc aagcggttcc acctcccggg catccgccag ggctacggcc tgaccgagac 1080
cacgagcgcg atcctgatca cccccgaggg ggacgacaag ccgggcgccg tgggcaaggt 1140
ggtcccgttc ttcgaggcca aggtggtgga cctggacacc ggcaagaccc tgggcgtgaa 1200
ccagcggggc gagctgtgcg tgcgggggcc gatgatcatg agcggctacg tgaacaaccc 1260
ggaggccacc aacgccctca tcgacaagga cggctggctg cacagcggcg acatcgccta 1320
ctgggacgag gacgagcact tcttcatcgt cgaccggctg aagtcgctga tcaagtacaa 1380
gggctaccag gtggcgccgg ccgagctgga gagcatcctg ctccagcacc ccaacatctt 1440
cgacgccggc gtggccgggc tgccggacga cgacgccggc gagctgccgg ccgcggtggt 1500
ggtgctggag cacggcaaga ccatgacgga gaaggagatc gtcgactacg tggccagcca 1560
ggtgaccacc gccaagaagc tgcggggcgg cgtggtgttc gtggacgagg tcccgaaggg 1620
cctgaccggg aagctcgacg cccggaagat ccgcgagatc ctgatcaagg ccaagaaggg 1680
cggcaagatc gccgtgtaag actagttata agactgacta gcccgatggg cctcccaacg 1740
ggccctcctc ccctccttgc accgagatta ataaaaaaaa aaaaaaaaaa aaaaaaaaaa 1800
aaaaaaaaaa aaaaaaaaaa aaaaaaaaaa aaaaaatgca tcaaaggctc ttttcagagc 1860
cacca 1865

<210> 1475

<211> 1906
<212> DNA
<213> Artificial Sequence

<220>
<223> atp5a1 PpLuc(GC) ag A64 histoneSL

<400> 1475

gggcggctcg gccattttgt cccagtcagt ccggaggctg cggctgcaga agtaccgcct 60
gcggagtaac tgcaaagaag cttgaggatg gaggacgcca agaacatcaa gaagggcccg 120
gcgcccttct acccgctgga ggacgggacc gccggcgagc agctccacaa ggccatgaag 180
cggtacgccc tggtgccggg cacgatcgcc ttcaccgacg cccacatcga ggtcgacatc 240
acctacgcgg agtacttcga gatgagcgtg cgcctggccg aggccatgaa gcggtacggc 300
ctgaacacca accaccggat cgtggtgtgc tcggagaaca gcctgcagtt cttcatgccg 360
gtgctgggcg ccctcttcat cggcgtggcc gtcgccccgg cgaacgacat ctacaacgag 420
cgggagctgc tgaacagcat ggggatcagc cagccgaccg tggtgttcgt gagcaagaag 480
ggcctgcaga agatcctgaa cgtgcagaag aagctgccca tcatccagaa gatcatcatc 540
atggacagca agaccgacta ccagggcttc cagtcgatgt acacgttcgt gaccagccac 600
ctcccgccgg gcttcaacga gtacgacttc gtcccggaga gcttcgaccg ggacaagacc 660
atcgccctga tcatgaacag cagcggcagc accggcctgc cgaagggggt ggccctgccg 720
caccggaccg cctgcgtgcg cttctcgcac gcccgggacc ccatcttcgg caaccagatc 780
atcccggaca ccgccatcct gagcgtggtg ccgttccacc acggcttcgg catgttcacg 840
accctgggct acctcatctg cggcttccgg gtggtcctga tgtaccggtt cgaggaggag 900
ctgttcctgc ggagcctgca ggactacaag atccagagcg cgctgctcgt gccgaccctg 960
ttcagcttct tcgccaagag caccctgatc gacaagtacg acctgtcgaa cctgcacgag 1020
atcgccagcg gggcgccccc gctgagcaag gaggtgggcg aggccgtggc caagcggttc 1080
cacctcccgg gcatccgcca gggctacggc ctgaccgaga ccacgagcgc gatcctgatc 1140
acccccgagg gggacgacaa gccgggcgcc gtgggcaagg tggtcccgtt cttcgaggcc 1200
aaggtggtgg acctggacac cggcaagacc ctgggcgtga accagcgggg cgagctgtgc 1260
gtgcgggggc cgatgatcat gagcggctac gtgaacaacc cggaggccac caacgccctc 1320
atcgacaagg acggctggct gcacagcggc gacatcgcct actgggacga ggacgagcac 1380
ttcttcatcg tcgaccggct gaagtcgctg atcaagtaca agggctacca ggtggcgccg 1440
gccgagctgg agagcatcct gctccagcac cccaacatct tcgacgccgg cgtggccggg 1500
ctgccggacg acgacgccgg cgagctgccg gccgcggtgg tggtgctgga gcacggcaag 1560
accatgacgg agaaggagat cgtcgactac gtggccagcc aggtgaccac cgccaagaag 1620
ctgcggggcg gcgtggtgtt cgtggacgag gtcccgaagg gcctgaccgg gaagctcgac 1680

gcccggaaga tccgcgagat cctgatcaag gccaagaagg gcggcaagat cgccgtgtaa 1740

gactagttat aagactgact agcccgatgg gcctcccaac gggccctcct cccctccttg 1800

caccgagatt aataaaaaaa aaaaaaaaaa aaaaaaaaaa aaaaaaaaaa aaaaaaaaaa 1860

aaaaaaaaaa aaaaaaatgc atcaaaggct cttttcagag ccacca 1906

<210> 1476
<211> 1893
<212> DNA
<213> Artificial Sequence

<220>
<223> HSD17B4 PpLuc(GC) ag A64 histoneSL

<400> 1476

```
gggtcccgca gtcggcgtcc agcggctctg cttgttcgtg tgtgtgtcgt tgcaggcctt      60
attcaagctt gaggatggag gacgccaaga acatcaagaa gggcccggcg cccttctacc     120
cgctggagga cgggaccgcc ggcgagcagc tccacaaggc catgaagcgg tacgccctgg     180
tgccgggcac gatcgccttc accgacgccc acatcgaggt cgacatcacc tacgcggagt     240
acttcgagat gagcgtgcgc ctggccgagg ccatgaagcg gtacggcctg aacaccaacc     300
accggatcgt ggtgtgctcg gagaacagcc tgcagttctt catgccggtg ctgggcgccc     360
tcttcatcgg cgtggccgtc gccccggcga acgacatcta caacgagcgg gagctgctga     420
acagcatggg gatcagccag ccgaccgtgg tgttcgtgag caagaagggc ctgcagaaga     480
tcctgaacgt gcagaagaag ctgcccatca tccagaagat catcatcatg gacagcaaga     540
ccgactacca gggcttccag tcgatgtaca cgttcgtgac cagccacctc ccgccgggct     600
tcaacgagta cgacttcgtc ccggagagct tcgaccggga caagaccatc gccctgatca     660
tgaacagcag cggcagcacc ggcctgccga agggggtggc cctgccgcac cggaccgcct     720
gcgtgcgctt ctcgcacgcc cgggacccca tcttcggcaa ccagatcatc ccggacaccg     780
ccatcctgag cgtggtgccg ttccaccacg gcttcggcat gttcacgacc ctgggctacc     840
tcatctgcgg cttccgggtg gtcctgatgt accggttcga ggaggagctg ttcctgcgga     900
gcctgcagga ctacaagatc cagagcgcgc tgctcgtgcc gaccctgttc agcttcttcg     960
ccaagagcac cctgatcgac aagtacgacc tgtcgaacct gcacgagatc gccagcgggg    1020
gcgccccgct gagcaaggag gtgggcgagg ccgtggccaa gcggttccac ctcccgggca    1080
tccgccaggg ctacggcctg accgagacca cgagcgcgat cctgatcacc cccgaggggg    1140
acgacaagcc gggcgccgtg ggcaaggtgg tcccgttctt cgaggccaag gtggtggacc    1200
tggacaccgg caagaccctg ggcgtgaacc agcggggcga gctgtgcgtg cgggggccga    1260
tgatcatgag cggctacgtg aacaacccgg aggccaccaa cgccctcatc gacaaggacg    1320
```

gctggctgca cagcggcgac atcgcctact gggacgagga cgagcacttc ttcatcgtcg 1380

accggctgaa gtcgctgatc aagtacaagg gctaccaggt ggcgccggcc gagctggaga 1440

gcatcctgct ccagcacccc aacatcttcg acgccggcgt ggccgggctg ccggacgacg 1500

acgccggcga gctgccggcc gcggtggtgg tgctggagca cggcaagacc atgacggaga 1560

aggagatcgt cgactacgtg gccagccagg tgaccaccgc caagaagctg cggggcggcg 1620

tggtgttcgt ggacgaggtc ccgaagggcc tgaccgggaa gctcgacgcc cggaagatcc 1680

gcgagatcct gatcaaggcc aagaagggcg gcaagatcgc cgtgtaagac tagttataag 1740

actgactagc ccgatgggcc tcccaacggg ccctcctccc ctccttgcac cgagattaat 1800

aaaaaaaaaa aaaaaaaaaa aaaaaaaaaa aaaaaaaaaa aaaaaaaaaa aaaaaaaaaa 1860

aaaatgcatc aaaggctctt ttcagagcca cca 1893

<210> 1477
<211> 1862
<212> DNA
<213> Artificial Sequence

<220>
<223> AIG1 PpLuc(GC) ag A64 histoneSL

<400> 1477

```
gggccgccca gccggtccag gcctctggcg aacaagcttg aggatggagg acgccaagaa      60
catcaagaag ggcccggcgc ccttctaccc gctggaggac gggaccgccg gcgagcagct     120
ccacaaggcc atgaagcggt acgccctggt gccgggcacg atcgccttca ccgacgccca     180
catcgaggtc gacatcacct acgcggagta cttcgagatg agcgtgcgcc tggccgaggc     240
catgaagcgg tacggcctga acaccaacca ccggatcgtg gtgtgctcgg agaacagcct     300
gcagttcttc atgccggtgc tgggcgccct cttcatcggc gtggccgtcg ccccggcgaa     360
cgacatctac aacgagcggg agctgctgaa cagcatgggg atcagccagc cgaccgtggt     420
gttcgtgagc aagaagggcc tgcagaagat cctgaacgtg cagaagaagc tgcccatcat     480
ccagaagatc atcatcatgg acagcaagac cgactaccag ggcttccagt cgatgtacac     540
gttcgtgacc agccacctcc cgccgggctt caacgagtac gacttcgtcc cggagagctt     600
cgaccgggac aagaccatcg ccctgatcat gaacagcagc ggcagcaccg gcctgccgaa     660
gggggtggcc ctgccgcacc ggaccgcctg cgtgcgcttc tcgcacgccc gggaccccat     720
cttcggcaac cagatcatcc cggacaccgc catcctgagc gtggtgccgt tccaccacgg     780
cttcggcatg ttcacgaccc tgggctacct catctgcggc ttccgggtgg tcctgatgta     840
ccggttcgag gaggagctgt tcctgcggag cctgcaggac tacaagatcc agagcgcgct     900
gctcgtgccg accctgttca gcttcttcgc caagagcacc ctgatcgaca agtacgacct     960
gtcgaacctg cacgagatcg ccagcggggg cgccccgctg agcaaggagg tgggcgaggc    1020
```

cgtggccaag cggttccacc tcccgggcat ccgccagggc tacggcctga ccgagaccac 1080

gagcgcgatc ctgatcaccc ccgaggggga cgacaagccg ggcgccgtgg gcaaggtggt 1140

cccgttcttc gaggccaagg tggtggacct ggacaccggc aagaccctgg gcgtgaacca 1200

gcggggcgag ctgtgcgtgc gggggccgat gatcatgagc ggctacgtga acaacccgga 1260

ggccaccaac gccctcatcg acaaggacgg ctggctgcac agcggcgaca tcgcctactg 1320

ggacgaggac gagcacttct tcatcgtcga ccggctgaag tcgctgatca agtacaaggg 1380

ctaccaggtg gcgccggccg agctggagag catcctgctc cagcacccca acatcttcga 1440

cgccggcgtg gccgggctgc cggacgacga cgccggcgag ctgccggccg cggtggtggt 1500

gctggagcac ggcaagacca tgacggagaa ggagatcgtc gactacgtgg ccagccaggt 1560

gaccaccgcc aagaagctgc ggggcggcgt ggtgttcgtg gacgaggtcc cgaagggcct 1620

gaccgggaag ctcgacgccc ggaagatccg cgagatcctg atcaaggcca agaagggcgg 1680

caagatcgcc gtgtaagact agttataaga ctgactagcc cgatgggcct cccaacgggc 1740

cctcctcccc tccttgcacc gagattaata aaaaaaaaaa aaaaaaaaaa aaaaaaaaaa 1800

aaaaaaaaaa aaaaaaaaaa aaaaaaaaaa aaatgcatca aaggctcttt tcagagccac 1860

ca 1862

<210> 1478
<211> 1887
<212> DNA
<213> Artificial Sequence

<220>
<223> COX6C PpLuc(GC) ag A64 histoneSL

<400> 1478

```
ggagtcagga aggacgttgg tgttgaggtt agcatacgta tcaaggacag taactaccaa      60
gcttgaggat ggaggacgcc aagaacatca agaagggccc ggcgcccttc tacccgctgg     120
aggacgggac cgccggcgag cagctccaca aggccatgaa gcggtacgcc ctggtgccgg     180
gcacgatcgc cttcaccgac gcccacatcg aggtcgacat cacctacgcg gagtacttcg     240
agatgagcgt gcgcctggcc gaggccatga agcggtacgg cctgaacacc aaccaccgga     300
tcgtggtgtg ctcggagaac agcctgcagt tcttcatgcc ggtgctgggc gccctcttca     360
tcggcgtggc cgtcgccccg gcgaacgaca tctacaacga gcgggagctg ctgaacagca     420
tggggatcag ccagccgacc gtggtgttcg tgagcaagaa gggcctgcag aagatcctga     480
acgtgcagaa gaagctgccc atcatccaga agatcatcat catggacagc aagaccgact     540
accagggctt ccagtcgatg tacacgttcg tgaccagcca cctcccgccg ggcttcaacg     600
agtacgactt cgtcccggag agcttcgacc gggacaagac catcgccctg atcatgaaca     660
```

gcagcggcag caccggcctg ccgaaggggg tggccctgcc gcaccggacc gcctgcgtgc 720

gcttctcgca cgcccgggac cccatcttcg gcaaccagat catcccggac accgccatcc 780

tgagcgtggt gccgttccac cacggcttcg gcatgttcac gaccctgggc tacctcatct 840

gcggcttccg ggtggtcctg atgtaccggt tcgaggagga gctgttcctg cggagcctgc 900

aggactacaa gatccagagc gcgctgctcg tgccgaccct gttcagcttc ttcgccaaga 960

gcaccctgat cgacaagtac gacctgtcga acctgcacga gatcgccagc gggggcgccc 1020

cgctgagcaa ggaggtgggc gaggccgtgg ccaagcggtt ccacctcccg ggcatccgcc 1080

agggctacgg cctgaccgag accacgagcg cgatcctgat cacccccgag gggacgaca 1140

agccgggcgc cgtgggcaag gtggtcccgt tcttcgaggc caaggtggtg gacctggaca 1200

ccggcaagac cctgggcgtg aaccagcggg gcgagctgtg cgtgcggggg ccgatgatca 1260

tgagcggcta cgtgaacaac ccggaggcca ccaacgccct catcgacaag gacggctggc 1320

tgcacagcgg cgacatcgcc tactgggacg aggacgagca cttcttcatc gtcgaccggc 1380

tgaagtcgct gatcaagtac aagggctacc aggtggcgcc ggccgagctg gagagcatcc 1440

tgctccagca ccccaacatc ttcgacgccg gcgtggccgg gctgccggac gacgacgccg 1500

gcgagctgcc ggccgcggtg gtggtgctgg agcacggcaa gaccatgacg gagaaggaga 1560

tcgtcgacta cgtggccagc caggtgacca ccgccaagaa gctgcggggc ggcgtggtgt 1620

tcgtggacga ggtcccgaag ggcctgaccg ggaagctcga cgcccggaag atccgcgaga 1680

tcctgatcaa ggccaagaag ggcggcaaga tcgccgtgta agactagtta taagactgac 1740

tagcccgatg ggcctcccaa cgggccctcc tcccctcctt gcaccgagat taataaaaaa 1800

aaaaaaaaaa aaaaaaaaaa aaaaaaaaaa aaaaaaaaaa aaaaaaaaaa aaaaaaaatg 1860

catcaaaggc tcttttcaga gccacca 1887

<210> 1479
<211> 1873
<212> DNA
<213> Artificial Sequence

<220>
<223> ASAH1 PpLuc(GC) ag A64 histoneSL

<400> 1479

gggcctctgc tggagtccgg ggagtggcgt tggctgctag agcgaagctt gaggatggag 60

gacgccaaga acatcaagaa gggcccggcg cccttctacc cgctggagga cgggaccgcc 120

ggcgagcagc tccacaaggc catgaagcgg tacgccctgg tgccgggcac gatcgccttc 180

accgacgccc acatcgaggt cgacatcacc tacgcggagt acttcgagat gagcgtgcgc 240

ctggccgagg ccatgaagcg gtacggcctg aacaccaacc accggatcgt ggtgtgctcg 300

gagaacagcc tgcagttctt catgccggtg ctgggcgccc tcttcatcgg cgtggccgtc 360

gccccggcga acgacatcta caacgagcgg gagctgctga acagcatggg gatcagccag 420
ccgaccgtgg tgttcgtgag caagaagggc ctgcagaaga tcctgaacgt gcagaagaag 480
ctgcccatca tccagaagat catcatcatg gacagcaaga ccgactacca gggcttccag 540
tcgatgtaca cgttcgtgac cagccacctc ccgccgggct tcaacgagta cgacttcgtc 600
ccggagagct tcgaccggga caagaccatc gccctgatca tgaacagcag cggcagcacc 660
ggcctgccga agggggtggc cctgccgcac cggaccgcct gcgtgcgctt ctcgcacgcc 720
cgggacccca tcttcggcaa ccagatcatc ccggacaccg ccatcctgag cgtggtgccg 780
ttccaccacg gcttcggcat gttcacgacc ctgggctacc tcatctgcgg cttccgggtg 840
gtcctgatgt accggttcga ggaggagctg ttcctgcgga gcctgcagga ctacaagatc 900
cagagcgcgc tgctcgtgcc gaccctgttc agcttcttcg ccaagagcac cctgatcgac 960
aagtacgacc tgtcgaacct gcacgagatc gccagcgggg gcgccccgct gagcaaggag 1020
gtgggcgagg ccgtggccaa gcggttccac ctcccgggca tccgccaggg ctacggcctg 1080
accgagacca cgagcgcgat cctgatcacc cccgaggggg acgacaagcc gggcgccgtg 1140
ggcaaggtgg tcccgttctt cgaggccaag gtggtggacc tggacaccgg caagaccctg 1200
ggcgtgaacc agcggggcga gctgtgcgtg cgggggccga tgatcatgag cggctacgtg 1260
aacaacccgg aggccaccaa cgccctcatc gacaaggacg gctggctgca cagcggcgac 1320
atcgcctact gggacgagga cgagcacttc ttcatcgtcg accggctgaa gtcgctgatc 1380
aagtacaagg gctaccaggt ggcgccggcc gagctggaga gcatcctgct ccagcacccc 1440
aacatcttcg acgccggcgt ggccgggctg ccggacgacg acgccggcga gctgccggcc 1500
gcggtggtgg tgctggagca cggcaagacc atgacggaga aggagatcgt cgactacgtg 1560
gccagccagg tgaccaccgc caagaagctg cggggcggcg tggtgttcgt ggacgaggtc 1620
ccgaagggcc tgaccgggaa gctcgacgcc cggaagatcc gcgagatcct gatcaaggcc 1680
aagaagggcg gcaagatcgc cgtgtaagac tagttataag actgactagc ccgatgggcc 1740
tcccaacggg ccctcctccc ctccttgcac cgagattaat aaaaaaaaaa aaaaaaaaaa 1800
aaaaaaaaaa aaaaaaaaaa aaaaaaaaaa aaaaaaaaaa aaaatgcatc aaaggctctt 1860
ttcagagcca cca 1873

<210> 1480
<211> 1873
<212> DNA
<213> Artificial Sequence

<220>
<223> RPL32 PpLuc(GC) ag A64 histoneSL

<400> 1480

ggggcgctgc ctacggaggt ggcagccatc tccttctcgg catcaagctt gaggatggag 60

gacgccaaga acatcaagaa gggcccggcg cccttctacc cgctggagga cgggaccgcc 120

ggcgagcagc tccacaaggc catgaagcgg tacgccctgg tgccgggcac gatcgccttc 180

accgacgccc acatcgaggt cgacatcacc tacgcggagt acttcgagat gagcgtgcgc 240

ctggccgagg ccatgaagcg gtacggcctg aacaccaacc accggatcgt ggtgtgctcg 300

gagaacagcc tgcagttctt catgccggtg ctgggcgccc tcttcatcgg cgtggccgtc 360

gccccggcga acgacatcta caacgagcgg gagctgctga acagcatggg gatcagccag 420

ccgaccgtgg tgttcgtgag caagaagggc ctgcagaaga tcctgaacgt gcagaagaag 480

ctgcccatca tccagaagat catcatcatg gacagcaaga ccgactacca gggcttccag 540

tcgatgtaca cgttcgtgac cagccacctc ccgccgggct tcaacgagta cgacttcgtc 600

ccggagagct tcgaccggga caagaccatc gccctgatca tgaacagcag cggcagcacc 660

ggcctgccga agggggtggc cctgccgcac cggaccgcct gcgtgcgctt ctcgcacgcc 720

cgggacccca tcttcggcaa ccagatcatc ccggacaccg ccatcctgag cgtggtgccg 780

ttccaccacg gcttcggcat gttcacgacc ctgggctacc tcatctgcgg cttccgggtg 840

gtcctgatgt accggttcga ggaggagctg ttcctgcgga gcctgcagga ctacaagatc 900

cagagcgcgc tgctcgtgcc gaccctgttc agcttcttcg ccaagagcac cctgatcgac 960

aagtacgacc tgtcgaacct gcacgagatc gccagcgggg gcgccccgct gagcaaggag 1020

gtgggcgagg ccgtggccaa gcggttccac ctcccgggca tccgccaggg ctacggcctg 1080

accgagacca cgagcgcgat cctgatcacc cccgaggggg acgacaagcc gggcgccgtg 1140

ggcaaggtgg tcccgttctt cgaggccaag gtggtggacc tggacaccgg caagaccctg 1200

ggcgtgaacc agcggggcga gctgtgcgtg cgggggccga tgatcatgag cggctacgtg 1260

aacaacccgg aggccaccaa cgccctcatc gacaaggacg gctggctgca cagcggcgac 1320

atcgcctact gggacgagga cgagcacttc ttcatcgtcg accggctgaa gtcgctgatc 1380

aagtacaagg gctaccaggt ggcgccggcc gagctggaga gcatcctgct ccagcacccc 1440

aacatcttcg acgccggcgt ggccgggctg ccggacgacg acgccggcga gctgccggcc 1500

gcggtggtgg tgctggagca cggcaagacc atgacggaga aggagatcgt cgactacgtg 1560

gccagccagg tgaccaccgc caagaagctg cggggcggcg tggtgttcgt ggacgaggtc 1620

ccgaagggcc tgaccgggaa gctcgacgcc cggaagatcc gcgagatcct gatcaaggcc 1680

aagaagggcg gcaagatcgc cgtgtaagac tagttataag actgactagc ccgatgggcc 1740

tcccaacggg ccctcctccc ctccttgcac cgagattaat aaaaaaaaaa aaaaaaaaaa 1800

aaaaaaaaaa aaaaaaaaaa aaaaaaaaaa aaaaaaaaaa aaaatgcatc aaaggctctt 1860

ttcagagcca cca 1873

<210> 1481
<211> 2035
<212> DNA
<213> Artificial Sequence

<220>
<223> RPL32 PpLuc(GC) albumin7 A64 C30 histoneSL

<400> 1481

```
ggggcgctgc ctacggaggt ggcagccatc tccttctcgg catcaagctt gaggatggag      60
gacgccaaga acatcaagaa gggcccggcg cccttctacc cgctggagga cgggaccgcc     120
ggcgagcagc tccacaaggc catgaagcgg tacgccctgg tgccgggcac gatcgccttc     180
accgacgccc acatcgaggt cgacatcacc tacgcggagt acttcgagat gagcgtgcgc     240
ctggccgagg ccatgaagcg gtacggcctg aacaccaacc accggatcgt ggtgtgctcg     300
gagaacagcc tgcagttctt catgccggtg ctgggcgccc tcttcatcgg cgtggccgtc     360
gccccggcga acgacatcta caacgagcgg gagctgctga acagcatggg gatcagccag     420
ccgaccgtgg tgttcgtgag caagaagggc ctgcagaaga tcctgaacgt gcagaagaag     480
ctgcccatca tccagaagat catcatcatg gacagcaaga ccgactacca gggcttccag     540
tcgatgtaca cgttcgtgac cagccacctc ccgccgggct tcaacgagta cgacttcgtc     600
ccggagagct tcgaccggga caagaccatc gccctgatca tgaacagcag cggcagcacc     660
ggcctgccga aggggtggc cctgccgcac cggaccgcct gcgtgcgctt ctcgcacgcc      720
cgggacccca tcttcggcaa ccagatcatc ccggacaccg ccatcctgag cgtggtgccg     780
ttccaccacg gcttcggcat gttcacgacc ctgggctacc tcatctgcgg cttccgggtg     840
gtcctgatgt accggttcga ggaggagctg ttcctgcgga gcctgcagga ctacaagatc     900
cagagcgcgc tgctcgtgcc gaccctgttc agcttcttcg ccaagagcac cctgatcgac     960
aagtacgacc tgtcgaacct gcacgagatc gccagcgggg gcgccccgct gagcaaggag    1020
gtgggcgagg ccgtggccaa gcggttccac ctcccgggca tccgccaggg ctacggcctg    1080
accgagacca cgagcgcgat cctgatcacc cccgaggggg acgacaagcc gggcgccgtg    1140
ggcaaggtgg tcccgttctt cgaggccaag gtggtggacc tggacaccgg caagaccctg    1200
ggcgtgaacc agcggggcga gctgtgcgtg cgggggccga tgatcatgag cggctacgtg    1260
aacaacccgg aggccaccaa cgccctcatc gacaaggacg gctggctgca cagcggcgac    1320
atcgcctact gggacgagga cgagcacttc ttcatcgtcg accggctgaa gtcgctgatc    1380
aagtacaagg gctaccaggt ggcgccggcc gagctggaga gcatcctgct ccagcacccc    1440
aacatcttcg acgccggcgt ggccgggctg ccggacgacg acgccggcga gctgccggcc    1500
gcggtggtgg tgctggagca cggcaagacc atgacggaga aggagatcgt cgactacgtg    1560
```

```
gccagccagg tgaccaccgc caagaagctg cggggcggcg tggtgttcgt ggacgaggtc     1620
ccgaagggcc tgaccgggaa gctcgacgcc cggaagatcc gcgagatcct gatcaaggcc     1680
aagaagggcg gcaagatcgc cgtgtaagac tagtgcatca catttaaaag catctcagcc     1740
taccatgaga ataagagaaa gaaaatgaag atcaatagct tattcatctc tttttctttt     1800
tcgttggtgt aaagccaaca ccctgtctaa aaaacataaa tttctttaat cattttgcct     1860
cttttctctg tgcttcaatt aataaaaaat ggaaagaacc tagatctaaa aaaaaaaaaa     1920
aaaaaaaaaa aaaaaaaaaa aaaaaaaaaa aaaaaaaaaa aaaaaaaaaa atgcatcccc     1980
cccccccccc cccccccccc cccccccaaa ggctcttttc agagccacca gaatt          2035
```

## Claims

1. An artificial nucleic acid molecule comprising:

   a. at least one 5'-untranslated region element (5'UTR element) which comprises or consists of
   a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
   or
   a functional fragment of a nucleic acid sequence, which has an identity of at least 95% to a nucleic acid sequence according to SEQ ID NOs. 1368,1452, 1453, 1454, 1455, 1456, 1457 or 1458, or a corresponding RNA sequence;
   and
   b. at least one open reading frame (ORF),
   and wherein the 5'UTR element is suitable for increasing protein production from the artificial nucleic acid molecule.

2. The artificial nucleic acid molecule according to claim 1, further comprising:
   c. at least one histone stem-loop.

3. The artificial nucleic acid molecule according to claim 1 or 2, wherein the 5'UTR element and the open reading frame are heterologous.

4. The artificial nucleic acid molecule according to any one of claims 1-3, wherein the 5'UTR element does not comprise a TOP-motif, preferably wherein the nucleic acid sequence having an identity of at least 95% to a nucleic acid sequence according to SEQ ID NOs. 1368, 1452, 1453, 1454, 1455, 1456, 1457 or 1458, or a corresponding RNA sequence, preferably the 5'UTR element, starts at its 5'-end with a nucleotide located at position 1, 2, 3, 4, 5, 6, 7, 8, 9 or 10 downstream of the polypyrimidine tract.

5. The artificial nucleic acid molecule according to any one of claims 1-4, wherein the nucleic acid sequence having an identity of at least 95% to a nucleic acid sequence according to SEQ ID NOs. 1368, 1452, 1453, 1454, 1455, 1456, 1457 or 1458, or a corresponding RNA sequence, preferably the 5'UTR element, terminates at its 3'-end with a nucleotide located at position 1, 2, 3, 4, 5, 6, 7, 8, 9 or 10 upstream of the start codon of the gene it is derived from.

6. The artificial nucleic acid molecule according to any one of claims 1-5, wherein the 5'UTR element does not comprise a start codon or an open reading frame.

7. The artificial nucleic acid molecule according to any one of claims 2-6, wherein the at least one histone stem-loop is selected from following formulae (I) or (II):

formula (I) (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

formula (II) (stem-loop sequence with stem bordering elements):

$$\underbrace{N_{1-6}}_{\substack{\text{stem1}\\ \text{bordering element}}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\substack{\text{stem2}\\ \text{bordering element}}}$$

wherein:

stem1 or stem2 bordering elements $N_{1-6}$ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
stem1 $[N_{0-2}GN_{3-5}]$ is reverse complementary or partially reverse complementary with element stem2, and is a consecutive sequence between of 5 to 7 nucleotides;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine;
loop sequence $[N_{0-4}(U/T)N_{0-4}]$ is located between elements stem1 and stem2, and is a consecutive sequence of 3 to 5 nucleotides, more preferably of 4 nucleotides;
wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;
stem2 $[N_{3-5}CN_{0-2}]$ is reverse complementary or partially reverse complementary with element stem1, and is a consecutive sequence between of 5 to 7 nucleotides;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof; and
wherein C is cytidine or an analogue thereof, and may be optionally replaced by a guanosine or an analogue thereof provided that its complementary nucleotide guanosine in stem1 is replaced by cytidine;
wherein
stem1 and stem2 are capable of base pairing with each other
forming a reverse complementary sequence, wherein base pairing may occur between stem1 and stem2, or forming a partially reverse complementary sequence, wherein an incomplete base pairing may occur between stem1 and stem2.

**8.** The artificial nucleic acid molecule according to any one of claims 2-7, wherein the at least one histone stem-loop is selected from at least one of following formulae (Ia) or (IIa):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (Ia) (stem-loop sequence without stem bordering elements)

$$\underbrace{N_{2-5}}_{\substack{\text{stem1}\\ \text{bordering element}}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\substack{\text{stem2}\\ \text{bordering element}}}$$

formula (IIa) (stem-loop sequence with stem bordering elements)

**9.** The artificial nucleic acid molecule according to any one of claims 1-8, further comprising
d. a poly(A) sequence and/or a polyadenylation signal.

**10.** The artificial nucleic acid molecule according to any of claims 1-9, further comprising:
e. a poly(C) sequence.

**11.** The artificial nucleic acid molecule according to any one of claims 1-10, further comprising:
f. at least one 3'UTR element.

**12.** The artificial nucleic acid molecule according to claim 11, wherein the at least one 3'UTR element comprises or consists of a nucleic acid sequence corresponding to a 3'UTR of a gene providing a stable mRNA or to a functional variant of the 3'UTR of a gene providing a stable mRNA, or to a functional fragment of said 3'UTR or of said functional variant.

**13.** The artificial nucleic acid molecule according to any one of claims 2-12, wherein the at least one histone stem-loop comprises or consists of a nucleic acid sequence selected from the group consisting of SEQ ID NOs. 1391-1433, preferably from the group consisting of SEQ ID NOs. 1403-1433.

**14.** The artificial nucleic acid molecule according to any one of claims 2-13, wherein the histone stem-loop comprises or consists of a nucleic acid sequence having a sequence identity of at least about 75%, preferably of at least about 80%, preferably of at least about 85%, more preferably of at least about 90%, even more preferably of at least about 95% to the sequence according to SEQ ID NO. 1433 or to the corresponding RNA sequence, wherein preferably positions 6, 13 and 20 of the sequence having a sequence identity of at least about 75%, preferably of at least about 80%, preferably at least about 85%, more preferably at least about 90%, even more preferably at least about 95% to the sequence according to SEQ ID NO. 1433 or to the corresponding RNA sequence are conserved, i.e. are identical to the nucleotides at positions 6, 13 and 20 of SEQ ID NO. 1433 or to the corresponding RNA nucleotides.

**15.** The artificial nucleic acid molecule according to any one of claims 11-14, wherein the 3'UTR element comprises or consists of a 3'UTR of a gene selected from the group consisting of an albumin gene, an $\alpha$-globin gene, a $\beta$-globin gene, a tyrosine hydroxylase gene, a lipoxygenase gene, and a collagen alpha gene, or a functional variant of a 3'UTR of a gene selected from the group consisting of an albumin gene, an $\alpha$-globin gene, a $\beta$-globin gene, a tyrosine hydroxylase gene, a lipoxygenase gene, and a collagen alpha gene, or a functional fragment of said 3'UTR or of said variant, wherein the at least one 3'UTR element preferably comprises or consists of
a nucleic acid sequence, which is derived from the 3'UTR of a vertebrate albumin gene or from a variant thereof, preferably from the 3'UTR of a mammalian albumin gene or from a variant thereof, more preferably from the 3'UTR of a human albumin gene or from a variant thereof, even more preferably from the 3'UTR of the human albumin

gene according to GenBank Accession number NM_000477.5 or from a variant thereof; or
a nucleic acid sequence, which is derived from the 3'UTR of a vertebrate α-globin gene or from a variant thereof, preferably from the 3'UTR of a mammalian α-globin gene or from a variant thereof, more preferably from the 3'UTR of a human α-globin gene or from a variant thereof.

**16.** The artificial nucleic acid molecule according to any one of claims 11-15, wherein the at least one 3'UTR element comprises or consists of a nucleic acid sequence, which has an identity of at least about 95%, preferably of at least about 99%, to a nucleic acid sequence selected from SEQ ID NOs. 1369-1377 and 1434 or to a corresponding RNA sequence, or wherein the at least one 3'UTR element comprises or consists of a functional fragment of a nucleic acid sequence, which has an identity of at least about 95%, preferably of at least about 99%, to a nucleic acid sequence selected from SEQ ID NOs. 1369-1377 and 1434 or to a corresponding RNA sequence.

**17.** The artificial nucleic acid molecule according to any one of claims 1-16, wherein the artificial nucleic acid molecule, preferably the open reading frame, is at least partially G/C modified, preferably wherein the G/C content of the open reading frame is increased compared to the wild type open reading frame.

**18.** The artificial nucleic acid molecule according to any one of claims 1-17, which is an RNA, preferably an mRNA molecule.

**19.** A vector comprising:

a. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;
and
b. at least one open reading frame (ORF) and/or at least one cloning site, and wherein the 5'UTR element is suitable for increasing protein production from the artificial nucleic acid molecule.

**20.** The vector according to claim 19, further comprising:
c. at least one histone-stem loop.

**21.** The vector according to claim 19 or 20, which is a DNA vector, preferably a plasmid vector, or a viral vector.

**22.** The vector according to any one of claims 19-21, which comprises or codes for an artificial nucleic acid molecule according to any one of claims 1-18.

**23.** A cell comprising the artificial nucleic acid molecule according to any one of claims 1-18 or the vector according to any one of claims 19-22, wherein the cell is preferably a mammalian cell, more preferably an isolated cell of a mammalian subject, most preferably of a human subject.

**24.** A pharmaceutical composition comprising the artificial nucleic acid molecule according to any one of claims 1-18, the vector according to any one of claims 19-22, or the cell according to claim 23, optionally further comprising one or more pharmaceutically acceptable diluents and/or excipients and/or one or more adjuvants.

**25.** The artificial nucleic acid molecule according to any one of claims 1-18, the vector according to any one of claims 19-22, the cell according to claim 23, or the pharmaceutical composition according to claim 24 for use as a medicament, preferably for use as a vaccine or for use in gene therapy.

**26.** An in vitro method for increasing protein production from an artificial nucleic acid molecule, comprising the step of providing the artificial nucleic acid molecule with

i. at least one 5'-untranslated region element (5'UTR element) which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or

a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence;
ii. preferably at least one histone stem-loop; and
iii. optionally, a poly(A) sequence and/or a polyadenylation signal, and wherein the 5'UTR element is suitable for increasing protein production from the artificial nucleic acid molecule.

**27.** In vitro use of a 5'UTR element, which comprises or consists of
a nucleic acid sequence which has an identity of at least 95% to a nucleic acid sequence according to SEQ ID NOs. 1368, 1452, 1453, 1454, 1455, 1456, 1457 or 1458, or a corresponding RNA sequence,
or
a functional fragment of a nucleic acid sequence, which has an identity of at least 95% to a nucleic acid sequence according to SEQ ID NOs. 1368, 1452, 1453, 1454, 1455, 1456, 1457 or 1458, or a corresponding RNA sequence and preferably further comprises at least one histone stem-loop
for increasing protein production from a nucleic acid molecule.

**28.** A kit or kit of parts comprising an artificial nucleic acid molecule according to any one of claims 1-18, the vector according to any one of claims 19-22, the cell according to claim 23, and/or the pharmaceutical composition according to claim 24, the kit or kit of parts preferably further comprising instructions for use, cells for transfection, an adjuvant, a means for administration of the pharmaceutical composition, a pharmaceutically acceptable carrier and/or a pharmaceutically acceptable solution for dissolution or dilution of the artificial nucleic acid molecule, the vector or the pharmaceutical composition.

**Patentansprüche**

**1.** Artifizielles Nukleinsäuremolekül umfassend:

a. mindestens ein Element einer 5'-untranslatierten Region ("5'-untranslated region element") (5'-UTR-Element), welches umfasst oder besteht aus eine(r) Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat, oder
ein(em) funktionales/n Fragment einer Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat;
und
b. mindestens ein offenes Leseraster ("open reading frame") (ORF),

und wobei das 5'-UTR-Element geeignet ist, die Proteinproduktion von dem artifiziellen Nukleinsäuremolekül zu steigern.

**2.** Das artifizielle Nukleinsäuremolekül gemäß Anspruch 1, welches weiter umfasst:
c. mindestens einen Histon-Stem-Loop.

**3.** Das artifizielle Nukleinsäuremolekül gemäß Anspruch 1 oder 2, worin das 5'-UTR-Element und das offene Leseraster heterolog sind.

**4.** Das artifizielle Nukleinsäuremolekül gemäß einem der Ansprüche 1 bis 3, worin das 5'-UTR-Element kein TOP-Motiv enthält, bevorzugt worin die Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat, bevorzugt das 5'-UTR-Element, an ihrem 5'-Ende mit einem Nukleotid beginnt, welches sich an Position 1, 2, 3, 4, 5, 6, 7, 8, 9 oder 10 stromabwärts des Polypyrimidinabschnitts befindet.

**5.** Das artifizielle Nukleinsäuremolekül gemäß einem der Ansprüche 1 bis 4, worin die Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat, bevorzugt das 5'-UTR-Element, an ihrem 3'-Ende mit einem Nukleotid endet, welches sich an Position 1, 2, 3, 4, 5, 6, 7, 8, 9 oder 10 stromaufwärts des Startkodons des Gens, von dem es abgeleitet ist, befindet.

6. Das artifizielle Nukleinsäuremolekül gemäß einem der Ansprüche 1 bis 5, worin das 5'-UTR-Element kein Startkodon oder offenes Leseraster umfasst.

7. Das artifizielle Nukleinsäuremolekül gemäß einem der Ansprüche 2 bis 6, wobei der mindestens eine Histon-Stem-Loop aus den folgenden Formeln (I) oder (II) ausgewählt ist:

Formel (I) (Stem-Loop-Sequenz ohne an den Stamm („stem") angrenzende Elemente):

$$\underbrace{[N_{0-2}GN_{3-5}]}_{\text{stem1}}\ \underbrace{[N_{0-4}(U/T)N_{0-4}]}_{\text{Loop}}\ \underbrace{[N_{3-5}CN_{0-2}]}_{\text{stem2}}$$

Formel (II) (Stem-Loop-Sequenz mit an den Stamm („stem") angrenzenden Elementen):

$$\underbrace{N_{1-6}}_{\substack{\text{stem1}\\ \text{angrenzendes Element}}}\ \underbrace{[N_{0-2}GN_{3-5}]}_{\text{stem1}}\ \underbrace{[N_{0-4}(U/T)N_{0-4}]}_{\text{Loop}}\ \underbrace{[N_{3-5}CN_{0-2}]}_{\text{stem2}}\ \underbrace{N_{1-6}}_{\substack{\text{stem2}\\ \text{angrenzendes Element}}}$$

wobei:

an stem1 oder stem2 angrenzende Elemente $N_{1-6}$ eine konsekutive Sequenz von 1 bis 6, bevorzugt von 2 bis 6, bevorzugter von 2 bis 5, noch bevorzugter von 3 bis 5, am meisten bevorzugt von 4 bis 5 oder 5 N ist, wobei jedes N unabhängig voneinander ausgewählt ist aus einem Nukleotid ausgewählt aus A, U, T, G und C, oder einem Nukleotidanalog davon;
stem1 $[N_{0-2}GN_{3-5}]$ revers-komplementär oder teilweise revers-komplementär zu Element stem2 ist, und eine konsekutive Sequenz von 5 bis 7 Nukleotiden ist;
wobei $N_{0-2}$ eine konsekutive Sequenz von 0 bis 2, bevorzugt von 0 bis 1, bevorzugter von 1 N ist, wobei jedes N unabhängig voneinander ausgewählt ist aus einem Nukleotid, ausgewählt aus A, U, T, G und C oder einem Nukleotidanalog davon;
wobei $N_{3-5}$ eine konsekutive Sequenz von 3 bis 5, bevorzugt von 4 bis 5, bevorzugter von 4 N ist, wobei jedes N unabhängig voneinander ausgewählt ist aus einem Nukleotid, ausgewählt aus A, U, T, G und C oder einem Nukleotidanalog davon, und
wobei G Guanosin oder ein Analog davon ist und optional durch Cytidin oder ein Analog davon ersetzt sein kann, vorausgesetzt, dass sein komplementäres Nukleotid Cytidin in stem2 durch Guanosin ersetzt wird;
Loop-Sequenz $[N_{0-4}(U/T)N_{0-4}]$ zwischen Elementen stem1 und stem2 gelegen ist, und eine konsekutive Sequenz von 3 bis 5 Nukleotiden, bevorzugter von 4 Nukleotiden ist;
wobei jedes $N_{0-4}$ unabhängig voneinander eine konsekutive Sequenz von 0 bis 4, bevorzugt von 1 bis 3, bevorzugter von 1 bis 2 N ist, wobei jedes N unabhängig voneinander ausgewählt ist aus einem Nukleotid, ausgewählt aus A, U, T, G und C oder einem Nukleotidanalog davon; und
wobei U/T Uridin darstellt, oder optional Thymidin;
stem2 $[N_{3-5}CN_{0-2}]$ revers-komplementär oder teilweise revers-komplementär zu Element stem1 ist und eine konsekutive Sequenz von 5 bis 7 Nukleotiden ist;
wobei $N_{3-5}$ eine konsekutive Sequenz von 3 bis 5, bevorzugt von 4 bis 5, bevorzugter von 4 N ist, wobei jedes N unabhängig voneinander ausgewählt ist aus einem Nukleotid, ausgewählt aus A, U, T, G und C oder einem Nukleotidanalog davon;
wobei $N_{0-2}$ eine konsekutive Sequenz von 0 bis 2, bevorzugt von 0 bis 1, bevorzugter von 1 N ist, wobei jedes N unabhängig voneinander ausgewählt ist aus einem Nukleotid, ausgewählt aus A, U, T, G und C oder einem

Nukleotidanalog davon; und
wobei C Cytidin oder ein Analog davon ist, und optional durch ein Guanosin oder ein Analog davon ersetzt sein kann, vorausgesetzt, dass sein komplementäres Nukleotid Guanosin in stem1 durch Cytidin ersetzt wird; wobei
stem1 und stem2 zur Basenpaarung miteinander fähig sind und
eine revers-komplementäre Sequenz bilden, wobei zwischen stem1 und stem2 Basenpaarung auftreten kann, oder
eine teilweise revers-komplementäre Sequenz bilden, wobei eine unvollständige Basenpaarung zwischen stem1 und stem2 auftreten kann.

**8.** Das artifizielle Nukleinsäuremolekül gemäß einem der Ansprüche 2 bis 7, wobei der mindestens eine Histon-Stem-Loop ausgewählt ist aus mindestens einer der folgenden Formeln (Ia) oder (IIa):

$$\underbrace{[N_{0-1}GN_{3-5}]}_{\text{stem1}}\ \underbrace{[N_{1-3}(U/T)N_{0-2}]}_{\text{Loop}}\ \underbrace{[N_{3-5}CN_{0-1}]}_{\text{stem2}}$$

Formel (Ia) (Stem-Loop-Sequenz ohne an den Stamm („stem“) angrenzende Elemente)

$$\underbrace{N_{2-5}}_{\substack{\text{stem1}\\ \text{angrenzendes Element}}}\ \underbrace{[N_{0-1}GN_{3-5}]}_{\text{stem1}}\ \underbrace{[N_{1-3}(U/T)N_{0-2}]}_{\text{Loop}}\ \underbrace{[N_{3-5}CN_{0-1}]}_{\text{stem2}}\ \underbrace{N_{2-5}}_{\substack{\text{stem2}\\ \text{angrenzendes Element}}}$$

Formel (IIa) (Stem-Loop-Sequenz mit an den Stamm („stem“) angrenzenden Elementen)

**9.** Das artifizielle Nukleinsäuremolekül gemäß einem der Ansprüche 1 bis 8, weiter umfassend:
d. eine Poly(A)-Sequenz und/oder ein Polyadenylierungssignal.

**10.** Das artifizielle Nukleinsäuremolekül gemäß einem der Ansprüche 1 bis 9, weiter umfassend:
e. eine Poly(C)-Sequenz.

**11.** Das artifizielle Nukleinsäuremolekül gemäß einem der Ansprüche 1 bis 10, weiter umfassend:
f. mindestens ein 3'-UTR-Element.

**12.** Das artifizielle Nukleinsäuremolekül gemäß Anspruch 11, worin das mindestens eine 3'-UTR-Element umfasst oder besteht aus eine(r) Nukleinsäuresequenz korrespondierend zu einer 3'-UTR eines Gens, welches eine stabile mRNA bereitstellt, oder zu einer funktionalen Variante des 3'-UTR eines Gens, welches eine stabile mRNA bereitstellt, oder zu einem funktionalen Fragment dieser 3'-UTR oder dieser funktionalen Variante.

**13.** Das artifizielle Nukleinsäuremolekül gemäß einem der Ansprüche 2 bis 12, worin der mindestens eine Histon-Stem-Loop umfasst oder besteht aus eine(r) Nukleinsäuresequenz ausgewählt aus der Gruppe bestehend aus SEQ ID NOs. 1391-1433, bevorzugt aus der Gruppe bestehend aus SEQ ID NOs. 1403-1433.

**14.** Das artifizielle Nukleinsäuremolekül gemäß einem der Ansprüche 2 bis 13, worin der Histon-Stem-Loop umfasst oder besteht aus eine(r) Nukleinsäuresequenz, welche eine Sequenzidentität von mindestens ungefähr 75%, bevorzugt von mindestens ungefähr 80 %, bevorzugt von mindestens ungefähr 85 %, weiter bevorzugt von mindestens ungefähr 90 %, noch weiter bevorzugt von mindestens ungefähr 95 % mit der Sequenz gemäß SEQ ID NO. 1433 oder mit der entsprechenden RNA-Sequenz hat,
worin bevorzugt die Positionen 6, 13 und 20 der Sequenz, welche eine Sequenzidentität von mindestens ungefähr 75%, bevorzugt von mindestens ungefähr 80 %, bevorzugt von mindestens ungefähr 85 %, weiter bevorzugt von mindestens ungefähr 90 %, noch weiter bevorzugt von mindestens ungefähr 95 % mit der Sequenz gemäß SEQ ID NO. 1433 oder mit der entsprechenden RNA-Sequenz hat, konserviert sind, d.h. identisch sind mit den Nukleotiden

an den Positionen 6, 13 und 20 der SEQ ID NO. 1433 oder mit den entsprechenden RNA-Nukleotiden.

**15.** Das artifizielle Nukleinsäuremolekül gemäß einem der Ansprüche 11 bis 14, worin das 3'-UTR-Element umfasst oder besteht aus eine(r) 3'-UTR eines Gens ausgewählt aus der Gruppe bestehend aus einem Albumin-Gen, einem $\alpha$-Globin-Gen, einem $\beta$-Globin-Gen, einem Tyrosinhydroxylase-Gen, einem Lipoxygenase-Gen, und einem Collagen-Alpha-Gen, oder eine(r) funktionale(n) Variante einer 3'-UTR eines Gens ausgewählt aus der Gruppe bestehend aus einem Albumin-Gen, einem $\alpha$-Globin-Gen, einem $\beta$-Globin-Gen, einem Tyrosinhydroxylase-Gen, einem Lipoxygenase-Gen, und einem Collagen-Alpha-Gen, oder ein(em) funktionales/n Fragment dieser 3'-UTR oder dieser Variante, worin das mindestens eine 3'-UTR-Element bevorzugt umfasst oder besteht aus
einer Nukleinsäuresequenz, welche abgeleitet ist von der 3'-UTR eines Vertebraten-Albumingens oder einer Variante davon, bevorzugt von der 3'-UTR eines Säugetier-Albumingens oder einer Variante davon, weiter bevorzugt von der 3'-UTR eines humanen Albumingens oder einer Variante davon, noch weiter bevorzugt von der 3'-UTR des humanen Albumingens gemäß GenBank-Zugangsnummer NM_000477.5 oder einer Variante davon; oder
einer Nukleinsäuresequenz, welche abgeleitet ist von der 3'-UTR eines Vertebraten-$\alpha$-Globingens oder einer Variante davon, bevorzugt von der 3'-UTR eines Säugetier-$\alpha$-Globingens oder einer Variante davon, weiter bevorzugt von der 3'-UTR eines humanen $\alpha$-Globingens oder einer Variante davon.

**16.** Das artifizielle Nukleinsäuremolekül gemäß einem der Ansprüche 11 bis 15, worin das mindestens eine 3'-UTR-Element umfasst oder besteht aus eine(r) Nukleinsäuresequenz, welche eine Identität von mindestens ungefähr 95 %, bevorzugt von mindestens ungefähr 99 %, mit einer Nukleinsäuresequenz ausgewählt aus SEQ ID NOs. 1369-1377 und 1434 oder zu einer entsprechenden RNA-Sequenz hat, oder
worin das mindestens eine 3'-UTR-Element umfasst oder besteht aus ein(em) funktionales/en Fragment einer Nukleinsäuresequenz, welche eine Identität von mindestens ungefähr 95 %, bevorzugt von mindestens ungefähr 99 %, mit einer Nukleinsäuresequenz ausgewählt aus SEQ ID. NOs. 1369-1377 und 1434 oder mit einer entsprechenden RNA-Sequenz hat.

**17.** Das artifizielle Nukleinsäuremolekül gemäß einem der Ansprüche 1 bis 16, wobei das artifizielle Nukleinsäuremolekül, bevorzugt das offene Leseraster, mindestens teilweise G/C-modifiziert ist, bevorzugt wobei der G/C-Gehalt des offenen Leserasters erhöht ist im Vergleich zum offenen Leseraster des Wildtyps.

**18.** Das artifizielle Nukleinsäuremolekül gemäß einem der Ansprüche 1 bis 17, welches eine RNA ist, bevorzugt ein mRNA-Molekül.

**19.** Vektor umfassend:

a. mindestens ein Element einer 5'-untranslatierten Region ("5'-untranslated region element") (5'-UTR-Element), welches umfasst oder besteht aus eine(r) Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat, oder
ein(em) funktionales/n Fragment einer Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat;
und
b. mindestens ein offenes Leseraster (ORF) und/oder mindestens eine Klonierungsstelle,

und wobei das 5'-UTR-Element geeignet ist, die Proteinproduktion von dem artifiziellen Nukleinsäuremolekül zu steigern.

**20.** Der Vektor gemäß Anspruch 19, weiter umfassend:
c. mindestens einen Histon-Stem-Loop.

**21.** Der Vektor gemäß Anspruch 19 oder 20, welcher ein DNA-Vektor, bevorzugt ein Plasmid-Vektor, oder ein viraler Vektor ist.

**22.** Der Vektor gemäß einem der Ansprüche 19 bis 21, welcher umfasst oder kodiert für ein artifizielles Nukleinsäuremolekül gemäß einem der Ansprüche 1 bis 18.

**23.** Zelle umfassend das artifizielle Nukleinsäuremolekül gemäß einem der Ansprüche 1 bis 18 oder den Vektor gemäß

einem der Ansprüche 19 bis 22, wobei die Zelle bevorzugt eine Säugetierzelle, weiter bevorzugt eine isolierte Zelle eines Säugetiersubjekts, am meisten bevorzugt eines humanen Subjekts, ist.

**24.** Pharmazeutische Zusammensetzung, umfassend das artifizielle Nukleinsäuremolekül gemäß einem der Ansprüche 1 bis 18, den Vektor gemäß einem der Ansprüche 19 bis 22, oder die Zelle gemäß Anspruch 23, welche optional weiter einen oder mehrere pharmazeutisch annehmbare Verdünnungsmittel und/oder Zusatzstoffe und/oder ein oder mehrere Adjuvanzien umfasst.

**25.** Das artifizielle Nukleinsäuremolekül gemäß einem der Ansprüche 1 bis 18, der Vektor gemäß einem der Ansprüche 19 bis 22, die Zelle gemäß Anspruch 23, oder die pharmazeutische Zusammensetzung gemäß Anspruch 24 zur Verwendung als Medikament, bevorzugt zur Verwendung als Vakzine oder zur Verwendung in der Gentherapie.

**26.** In-vitro-Verfahren zur Steigerung der Proteinproduktion von einem artifiziellen Nukleinsäuremolekül, umfassend den Schritt der Bereitstellung des artifiziellen Nukleinsäuremoleküls mit

i. mindestens einem Element einer 5'-untranslatierten Region ("5'-untranslated region element") (5'-UTR-Element), welches umfasst oder besteht aus eine(r) Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat, oder
ein(em) funktionales/n Fragment einer Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat;
ii. bevorzugt mindestens einen Histon-Stem-Loop; und
iii. optional, eine Poly(A)-Sequenz und/oder ein Polyadenylierungssignal,

und wobei das 5'-UTR-Element geeignet ist, die Proteinproduktion von dem artifiziellen Nukleinsäuremolekül zu steigern.

**27.** In-vitro-Verwendung eines 5'-UTR-Elements, welches umfasst oder besteht aus eine(r) Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat,
oder
ein(em) funktionales/n Fragment einer Nukleinsäuresequenz, welche eine Identität von mindestens 95% mit einer Nukleinsäuresequenz gemäß SEQ ID NO. 1368, 1452, 1453, 1454, 1455, 1456, 1457 oder 1458, oder einer korrespondierenden RNA-Sequenz hat, und
bevorzugt zudem mindestens einen Histon-Stem-Loop umfasst zur Steigerung der Proteinproduktion von einem Nukleinsäuremolekül.

**28.** Kit oder Kit von Teilen ("kit of parts") umfassend ein artifizielles Nukleinsäuremolekül gemäß einem der Ansprüche 1 bis 18, den Vektor gemäß einem der Ansprüche 19 bis 22, die Zelle gemäß Anspruch 23, und/oder die pharmazeutische Zusammensetzung gemäß Anspruch 24, wobei das Kit oder Kit von Teilen zudem bevorzugt Anleitungen zur Verwendung, Zellen für die Transfektion, ein Adjuvans, ein Mittel zur Verabreichung der pharmazeutischen Zusammensetzung, ein pharmazeutisch akzeptabler Träger und/oder eine pharmazeutische akzeptable Lösung zum Auflösen des artifiziellen Nukleinsäuremoleküls, des Vektors oder der pharmazeutischen Zusammensetzung umfasst.

## Revendications

**1.** Molécule d'acide nucléique artificielle comprenant :

a. au moins un élément de région non traduite en 5' (élément 5'UTR) qui comprend ou est constitué
d'une séquence d'acide nucléique qui a une identité d'au moins 95 % avec une séquence d'acide nucléique selon SEQ ID NO 1368, 1452, 1453, 1454, 1455, 1456, 1457 ou 1458, ou une séquence d'ARN correspondante,
ou
d'un fragment fonctionnel d'une séquence d'acide nucléique, qui a une identité d'au moins 95 % avec une séquence d'acide nucléique selon SEQ ID NO 1368, 1452, 1453, 1454, 1455, 1456, 1457 ou 1458, ou une séquence d'ARN correspondante ;

et

b. au moins un cadre de lecture ouvert (ORF),

et dans laquelle l'élément 5'UTR est approprié pour augmenter la production de protéine à partir de la molécule d'acide nucléique artificielle.

2. Molécule d'acide nucléique artificielle selon la revendication 1, comprenant en outre :
c. au moins une tige-boucle d'histone.

3. Molécule d'acide nucléique artificielle selon la revendication 1 ou 2, dans laquelle l'élément 5'UTR et le cadre de lecture ouvert sont hétérologues.

4. Molécule d'acide nucléique artificielle selon l'une quelconque des revendications 1-3, dans laquelle l'élément 5'UTR ne comprend pas de motif TOP, de préférence dans laquelle l'acide nucléique ayant une identité d'au moins 95 % avec une séquence d'acide nucléique selon SEQ ID NO 1368, 1452, 1453, 1454, 1455, 1456, 1457 ou 1458, ou une séquence d'ARN correspondante, de préférence l'élément 5'UTR, commence à son extrémité 5' avec un nucléotide localisé à la position 1, 2, 3, 4, 5, 6, 7, 8, 9 ou 10 en aval de l'étendue de polypyrimidine.

5. Molécule d'acide nucléique artificielle selon l'une quelconque des revendications 1-4, dans laquelle l'acide nucléique ayant une identité d'au moins 95 % avec une séquence d'acide nucléique selon SEQ ID NO 1368, 1452, 1453, 1454, 1455, 1456, 1457 ou 1458, ou une séquence d'ARN correspondante, de préférence l'élément 5'UTR, se termine à son extrémité 3' avec un nucléotide localisé à la position 1, 2, 3, 4, 5, 6, 7, 8, 9 ou 10 en amont du codon de départ du gène dont elle est dérivée.

6. Molécule d'acide nucléique artificielle selon l'une quelconque des revendications 1-5, dans laquelle l'élément 5'UTR ne comprend pas de codon de départ ou de cadre de lecture ouvert.

7. Molécule d'acide nucléique artificielle selon l'une quelconque des revendications 2-6, dans laquelle l'au moins une tige-boucle d'histone est choisie parmi les formules (I) ou (II) suivantes :

formule (I) (séquence de tige-boucle sans élément bordant la boucle) :

$$\underbrace{[N_{0-2}GN_{3-5}]}_{\text{tige1}}\ \underbrace{[N_{0-4}(U/T)N_{0-4}]}_{\text{boucle}}\ \underbrace{[N_{3-5}CN_{0-2}]}_{\text{tige2}}$$

formule (II) (séquence de tige-boucle avec des éléments bordant la tige) :

$$\underbrace{N_{1-6}}_{\text{tige1 élément bordant}}\ \underbrace{[N_{0-2}GN_{3-5}]}_{\text{tige1}}\ \underbrace{[N_{0-4}(U/T)N_{0-4}]}_{\text{boucle}}\ \underbrace{[N_{3-5}CN_{0-2}]}_{\text{tige2}}\ \underbrace{N_{1-6}}_{\text{tige2 élément bordant}}$$

dans laquelle :

éléments bordant la tige1 ou la tige2 $N_{1-6}$ consistent en une séquence consécutive de 1 à 6, de préférence de 2 à 6, de façon davantage préférée de 2 à 5, de façon même davantage préférée de 3 à 5, de façon préférée

entre toutes de 4 à 5 ou 5 N, dans laquelle chaque N est choisi indépendamment d'un autre d'après un nucléotide choisi parmi A, U, T, G et C, ou un analogue nucléotidique de celui-ci ;

tige1 $[N_{0-2}GN_{3-5}]$ est le complémentaire inverse ou partiellement le complémentaire inverse de l'élément tige2, et est une séquence consécutive de 5 à 7 nucléotides ;

dans laquelle $N_{0-2}$ est une séquence consécutive de 0 à 2, de préférence de 0 à 1, de façon davantage préférée de 1 N, dans laquelle chaque N est choisi indépendamment d'un autre d'après un nucléotide choisi parmi A, U, T, G et C ou un analogue nucléotidique de celui-ci ;

dans laquelle $N_{3-5}$ est une séquence consécutive de 3 à 5, de préférence de 4 à 5, de façon davantage préférée de 4 N, dans laquelle chaque N est choisi indépendamment d'un autre d'après un nucléotide choisi parmi A, U, T, G et C ou un analogue nucléotidique de celui-ci, et

dans laquelle G représente la guanosine ou un analogue de celle-ci, et peut être éventuellement remplacée par une cytidine ou un analogue de celle-ci, à condition que son nucléotide complémentaire de cytidine dans la tige2 soit remplacé par une guanosine ;

séquence de boucle $[N_{0-4}(U/T)N_{0-4}]$ est localisée entre les éléments tige1 et tige2, et est une séquence consécutive de 3 à 5 nucléotides, de façon davantage préférée de 4 nucléotides ;

dans laquelle chaque $N_{0-4}$ est indépendante d'une autre une séquence consécutive de 0 à 4, de préférence de 1 à 3, de façon davantage préférée de 1 à 2 N, dans laquelle chaque N est choisi indépendamment d'un autre d'après un nucléotide choisi parmi A, U, T, G et C ou un analogue nucléotidique de celui-ci ; et

dans laquelle U/T représente l'uridine, ou éventuellement la thymidine ;

tige2 $[N_{3-5}CN_{0-2}]$ est le complémentaire inverse ou partiellement le complémentaire inverse de l'élément tige1, et est une séquence consécutive de 5 à 7 nucléotides ;

dans laquelle $N_{3-5}$ est une séquence consécutive de 3 à 5, de préférence de 4 à 5, de façon davantage préférée de 4 N, dans laquelle chaque N est choisi indépendamment d'un autre d'après un nucléotide choisi parmi A, U, T, G et C ou un analogue nucléotidique de celui-ci ;

dans laquelle $N_{0-2}$ est une séquence consécutive de 0 à 2, de préférence de 0 à 1, de façon davantage préférée de 1 N, dans laquelle chaque N est choisi indépendamment d'un autre d'après un nucléotide choisi parmi A, U, T, G et C ou un analogue nucléotidique de celui-ci ;

dans laquelle C représente la cytidine ou un analogue de celle-ci, et peut être éventuellement remplacée par une guanosine ou un analogue de celle-ci, à condition que son nucléotide complémentaire de guanosine dans la tigel soit remplacé par une cytidine ;

dans laquelle

tige1 et tige2 sont capables d'un appariement des bases de l'une avec l'autre formant une séquence complémentaire inverse, dans laquelle l'appariement des bases peut survenir entre tige1 et tige2, ou formant une séquence partiellement complémentaire inverse, dans laquelle un appariement des bases incomplet peut survenir entre tige1 et tige2.

**8.** Molécule d'acide nucléique artificielle selon l'une quelconque des revendications 2-7, dans laquelle l'au moins une tige-boucle d'histone est choisie parmi au moins l'une des formules (Ia) ou (IIa) suivantes :

$$\underbrace{[N_{0-1}GN_{3-5}]}_{\text{tige1}}\ \underbrace{[N_{1-3}(U/T)N_{0-2}]}_{\text{boucle}}\ \underbrace{[N_{3-5}CN_{0-1}]}_{\text{tige2}}$$

formule (Ia) (séquence de tige-boucle sans élément bordant la tige)

$$\underbrace{N_{2-5}}_{\text{tige1}}\ \underbrace{[N_{0-1}GN_{3-5}]}_{\text{tige1}}\ \underbrace{[N_{1-3}(U/T)N_{0-2}]}_{\text{boucle}}\ \underbrace{[N_{3-5}CN_{0-1}]}_{\text{tige2}}\ \underbrace{N_{2-5}}_{\text{tige2}}$$

élément bordant                    élément bordant

formule (IIa) (séquence de tige-boucle avec des éléments bordant la tige)

**9.** Molécule d'acide nucléique artificielle selon l'une quelconque des revendications 1-8, comprenant en outre
d. une séquence poly(A) et/ou un signal de polyadénylation.

**10.** Molécule d'acide nucléique artificielle selon l'une quelconque des revendications 1-9, comprenant en outre :
e. une séquence poly(C).

**11.** Molécule d'acide nucléique artificielle selon l'une quelconque des revendications 1-10, comprenant en outre :
f. au moins un élément 3'UTR.

**12.** Molécule d'acide nucléique artificielle selon la revendication 11, dans laquelle l'au moins un élément 3'UTR comprend ou est constitué d'une séquence d'acide nucléique correspondant à une 3'UTR d'un gène produisant un ARNm stable ou à un variant fonctionnel de la 3'UTR d'un gène produisant un ARNm stable, ou à un fragment fonctionnel de ladite 3'UTR ou dudit variant fonctionnel.

**13.** Molécule d'acide nucléique artificielle selon l'une quelconque des revendications 2-12, où l'au moins une tige-boucle d'histone comprend ou est constituée d'une séquence d'acide nucléique choisie dans le groupe constitué de SEQ ID NO 1391-1433, de préférence dans le groupe constitué de SEQ ID NO 1403-1433.

**14.** Molécule d'acide nucléique artificielle selon l'une quelconque des revendications 2-13, où la tige-boucle d'histone comprend ou est constituée d'une séquence d'acide nucléique ayant une identité de séquence d'au moins environ 75 %, de préférence d'au moins environ 80 %, de préférence d'au moins environ 85 %, de façon d'avantage préférée d'au moins environ 90 %, de façon même d'avantage préférée d'au moins environ 95 % avec la séquence selon SEQ ID NO 1433 ou avec la séquence d'ARN correspondante, où de préférence les positions 6, 13 et 20 de la séquence ayant une identité de séquence d'au moins environ 75 %, de préférence d'au moins environ 80 %, de préférence d'au moins environ 85 %, de façon d'avantage préférée d'au moins environ 90 %, de façon même d'avantage préférée d'au moins environ 95 % avec la séquence selon SEQ ID NO 1433 ou avec la séquence d'ARN correspondante sont conservées, c'est-à-dire sont identiques aux nucléotides au niveau des positions 6, 13 et 20 de SEQ ID NO 1433 ou aux nucléotides d'ARN correspondants.

**15.** Molécule d'acide nucléique artificielle selon l'une quelconque des revendications 11-14, dans laquelle l'élément 3'UTR comprend ou est constitué d'une 3'UTR d'un gène choisi dans le groupe constitué d'un gène de l'albumine, un gène de l'$\alpha$-globine, un gène de la $\beta$-globine, un gène de la tyrosine hydroxylase, un gène de la lipoxygénase, et un gène du collagène alpha, ou un variant fonctionnel d'une 3'UTR d'un gène choisi dans le groupe constitué d'un gène de l'albumine, un gène de l'$\alpha$-globine, un gène de la $\beta$-globine, un gène de la tyrosine hydroxylase, un gène de la lipoxygénase, et un gène du collagène alpha, ou un variant fonctionnel de ladite 3'UTR ou dudit variant, où l'au moins un élément 3'UTR comprend de préférence ou est constitué de
une séquence d'acide nucléique, qui est dérivée de la 3'UTR d'un gène d'albumine de vertébré ou d'un variant de celui-ci, de préférence de la 3'UTR d'un gène d'albumine de mammifère ou d'un variant de celui-ci, de façon d'avantage préférée de la 3'UTR d'un gène d'albumine humain ou d'un variant de celui-ci, de façon même d'avantage préférée de la 3'UTR du gène d'albumine humain selon GenBank numéro d'ordre NM-000477.5 ou d'un variant de celui-ci ; ou
d'une séquence d'acide nucléique, qui est dérivée de la 3'UTR d'un gène d'$\alpha$-globine de vertébré ou d'un variant de celui-ci, de préférence de la 3'UTR d'un gène d'$\alpha$-globine de mammifère ou d'un variant de celui-ci, de façon davantage préférée de la 3'UTR d'un gène d'$\alpha$-globine humain ou d'un variant de celui-ci.

**16.** Molécule d'acide nucléique artificielle selon l'une quelconque des revendications 11-15, dans laquelle l'au moins un élément 3'UTR comprend ou est constitué d'une séquence d'acide nucléique, qui présente une identité d'au moins environ 95 %, de préférence d'au moins environ 99 %, avec une séquence d'acide nucléique choisie parmi

SEQ ID NO : 1369-1377 et 1434 ou avec une séquence d'ARN correspondante, ou dans laquelle l'au moins un élément 3'UTR comprend ou est constitué d'un fragment fonctionnel d'une séquence d'acide nucléique, qui présente une identité d'au moins environ 95 %, de préférence d'au moins environ 99 %, avec une séquence d'acide nucléique choisie parmi SEQ ID NO : 1369 à 1377 et 1434 ou avec une séquence d'ARN correspondante.

**17.** Molécule d'acide nucléique artificielle selon l'une quelconque des revendications 1-16, dans laquelle la molécule d'acide nucléique artificielle, de préférence le cadre de lecture ouvert, est au moins partiellement modifiée en G/C, de préférence dans laquelle la teneur en G/C du cadre de lecture ouvert est augmentée comparativement au cadre de lecture ouvert de type sauvage.

**18.** Molécule d'acide nucléique artificielle selon l'une quelconque des revendications 1-17, qui est un ARN, de préférence une molécule d'ARNm.

**19.** Vecteur comprenant :

a. au moins un élément de région non traduite en 5' (élément 5'UTR) qui comprend ou est constitué
d'une séquence d'acide nucléique qui a une identité d'au moins 95 % avec une séquence d'acide nucléique selon SEQ ID NO 1368, 1452, 1453, 1454, 1455, 1456, 1457 ou 1458, ou une séquence d'ARN correspondante,
ou
d'un fragment fonctionnel d'une séquence d'acide nucléique, qui a une identité d'au moins 95 % avec une séquence d'acide nucléique selon SEQ ID NO 1368, 1452, 1453, 1454, 1455, 1456, 1457 ou 1458, ou une séquence d'ARN correspondante ;
et
b. au moins un cadre de lecture ouvert (ORF) et/ou au moins un site de clonage,

et dans laquelle l'élément 5'UTR est approprié pour augmenter la production de protéine à partir de la molécule d'acide nucléique artificielle.

**20.** Vecteur selon la revendication 19, comprenant en outre :
c. au moins une tige-boucle d'histone.

**21.** Vecteur selon la revendication 19 ou 20, qui est un vecteur d'ADN, de préférence un vecteur plasmidique, ou un vecteur viral.

**22.** Vecteur selon l'une quelconque des revendications 19-21, qui comprend ou code pour une molécule d'acide nucléique artificielle selon l'une quelconque des revendications 1-18.

**23.** Cellule comprenant la molécule d'acide nucléique artificielle selon l'une quelconque des revendications 1-18 ou le vecteur selon l'une quelconque des revendications 19-22, où la cellule est de préférence une cellule de mammifère, de manière d'avantage préférée une cellule isolée d'un sujet mammifère, de manière même d'avantage préférée d'un sujet humain.

**24.** Composition pharmaceutique comprenant la molécule d'acide nucléique artificielle selon l'une quelconque des revendications 1-18, le vecteur selon l'une quelconque des revendications 19-22, ou la cellule selon la revendication 23, comprenant éventuellement en outre un ou plusieurs diluants et/ou excipients pharmaceutiquement acceptables et/ou un ou plusieurs adjuvants.

**25.** Molécule d'acide nucléique artificielle selon l'une quelconque des revendications 1-18, vecteur selon l'une quelconque des revendications 19-22, cellule selon la revendication 23, ou composition pharmaceutique selon la revendication 24 pour une utilisation en tant que médicament, de préférence pour une utilisation en tant que vaccin ou pour une utilisation en thérapie génique.

**26.** Procédé in vitro pour augmenter la production de protéine à partir d'une molécule d'acide nucléique artificielle, comprenant l'étape de munir la molécule d'acide nucléique artificielle de

i. au moins un élément de région non traduite en 5' (élément 5'UTR) qui comprend ou est constitué
d'une séquence d'acide nucléique qui a une identité d'au moins 95 % avec une séquence d'acide nucléique selon SEQ ID NO 1368, 1452, 1453, 1454, 1455, 1456, 1457 ou 1458, ou une séquence d'ARN correspondante,

ou

d'un fragment fonctionnel d'une séquence d'acide nucléique, qui a une identité d'au moins 95 % avec une séquence d'acide nucléique selon SEQ ID NO 1368, 1452, 1453, 1454, 1455, 1456, 1457 ou 1458, ou une séquence d'ARN correspondante ;

ii. de préférence au moins une tige-boucle d'histone ; et

iii. éventuellement, une séquence poly(A) et/ou un signal de polyadénylation,

et dans laquelle l'élément 5'UTR est approprié pour augmenter la production de protéine à partir de la molécule d'acide nucléique artificielle.

**27.** Utilisation in vitro d'un élément 5'UTR, qui comprend ou est constitué d'une séquence d'acide nucléique qui a une identité d'au moins 95 % avec une séquence d'acide nucléique selon SEQ ID NO 1368, 1452, 1453, 1454, 1455, 1456, 1457 ou 1458, ou une séquence d'ARN correspondante,

ou

d'un fragment fonctionnel d'une séquence d'acide nucléique, qui a une identité d'au moins 95 % avec une séquence d'acide nucléique selon SEQ ID NO 1368, 1452, 1453, 1454, 1455, 1456, 1457 ou 1458, ou une séquence d'ARN correspondante et comprend en outre de préférence au moins une tige-boucle d'histone

pour augmenter la production de protéine à partir d'une molécule d'acide nucléique.

**28.** Kit ou kit de parties comprenant une molécule d'acide nucléique artificielle selon l'une quelconque des revendications 1-18, le vecteur selon l'une quelconque des revendications 19-22, la cellule selon la revendication 23, et/ou la composition pharmaceutique selon la revendication 24, le kit ou kit de parties comprenant de préférence en outre des instructions pour l'utilisation, des cellules pour la transfection, un adjuvant, un moyen pour l'administration de la composition pharmaceutique, un vecteur pharmaceutiquement acceptable et/ou une solution pharmaceutiquement acceptable pour la dissolution ou la dilution de la molécule d'acide nucléique artificielle, du vecteur ou de la composition pharmaceutique.

| #A | #T | #C | #G | Cons | 99% | 95% | 90% | | |
|---|---|---|---|---|---|---|---|---|---|
| 2224 | 172 | 1557 | 25 | N• | H• | M• | M• | | |
| 1586 | 188 | 2211 | 16 | N• | H• | H• | M• | | |
| 3075 | 47 | 875 | 4 | N | H | M | M | | |
| 2872 | 205 | 918 | 6 | N | H | H | M | | |
| 1284 | 19 | 2675 | 23 | N | V | M | M | | |
| 184 | 6 | 270 | 3541 | N | V | S | S | ^ | Stem 1 |
| 0 | 0 | 0 | 4001 | G | G | G | G | ^ | Stem 1 |
| 13 | 569 | 3394 | 25 | N | Y | Y | Y | ^ | Stem 1 |
| 12 | 1620 | 2342 | 27 | N | Y | Y | Y | ^ | Stem 1 |
| 9 | 199 | 3783 | 10 | N | Y | Y | C | ^ | Stem 1 |
| 1 | 3947 | 51 | 2 | N | Y | T | T | ^ | Stem 1 |
| 47 | 3830 | 119 | 5 | N | H | T | T | • | Loop |
| 59 | 3704 | 227 | 11 | N | H | Y | T | • | Loop |
| 0 | 4001 | 0 | 0 | T | T | T | T | • | Loop |
| 675 | 182 | 3140 | 4 | N | H | M | M | • | Loop |
| 3818 | 1 | 7 | 175 | N | R | A | A | v | Stem 2 |
| 195 | 21 | 50 | 3735 | N | V | R | G | v | Stem 2 |
| 1596 | 15 | 31 | 2359 | N | V | R | R | v | Stem 2 |
| 523 | 11 | 16 | 3451 | N | R | R | R | v | Stem 2 |
| 0 | 0 | 4001 | 0 | C | C | C | C | v | Stem 2 |
| 14 | 179 | 3543 | 265 | N | B | S | S | v | Stem 2 |
| 3727 | 8 | 154 | 112 | N | V | M | A | | |
| 61 | 64 | 3070 | 4 | N | H | C | C | | |
| 771 | 557 | 2636 | 37 | N• | H• | H• | H• | | |
| 2012 | 201 | 1744 | 43 | N• | N• | H• | M• | | |
| 2499 | 690 | 674 | 138 | N• | N• | H• | H• | | |

Figure 1

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| # A | 52 | 32 | 71 | 82 | 76 | 13 | 0 | 12 | 12 | 9 | 1 | 46 | 3 | 0 | 75 | 82 | 53 | 79 | 20 | 0 | 4 | 94 | 17 | 35 | 74 | 56 |
| # T | 20 | 32 | 37 | 21 | 8 | 3 | 0 | 21 | 85 | 58 | 86 | 70 | 65 | 131 | 28 | 1 | 17 | 13 | 10 | 0 | 15 | 7 | 31 | 32 | 20 | 28 |
| # C | 45 | 59 | 20 | 25 | 38 | 0 | 0 | 86 | 8 | 54 | 42 | 13 | 58 | 0 | 27 | 2 | 6 | 31 | 10 | 131 | 112 | 5 | 82 | 58 | 30 | 40 |
| # G | 14 | 8 | 3 | 3 | 9 | 115 | 131 | 12 | 26 | 10 | 2 | 2 | 5 | 0 | 1 | 46 | 55 | 8 | 91 | 0 | 0 | 25 | 1 | 6 | 7 | 7 |
| Cons | N• | N• | N | N | N | D | G | N | N | N | N | N | N | T | N | N | N | N | N | C | H | N | N | N• | N• | N• |
| 99% | N• | N• | N | N | N | D | G | N | N | N | B | N | N | T | H | V | N | N | N | C | H | N | H | N• | N• | N• |
| 95% | N• | N• | H | H | N | R | G | N | N | N | Y | H | B | T | H | R | D | N | N | C | Y | D | H | H• | N• | N• |
| 90% | N• | H• | H | H | V | R | G | N | D | B | Y | H | Y | T | H | R | D | H | N | C | Y | R | H | H• | H• | H• |

Figure 2

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| # A | 2172 | 1554 | 3004 | 2790 | 1208 | 171 | 0 | 1 | 0 | 0 | 0 | 1 | 56 | 0 | 600 | 3736 | 142 | 1517 | 503 | 0 | 10 | 3633 | 44 | 736 | 1938 | 2443 |
| # T | 152 | 156 | 10 | 184 | 11 | 3 | 0 | 548 | 1535 | 141 | 3861 | 3760 | 3639 | 3870 | 154 | 0 | 4 | 2 | 1 | 0 | 164 | 1 | 33 | 525 | 181 | 662 |
| # C | 1512 | 2152 | 855 | 893 | 2637 | 270 | 0 | 3308 | 2334 | 3729 | 9 | 106 | 169 | 0 | 3113 | 5 | 44 | 0 | 6 | 3870 | 3431 | 149 | 3788 | 2578 | 1714 | 634 |
| # G | 11 | 6 | 1 | 3 | 14 | 3426 | 3870 | 13 | 1 | 0 | 0 | 3 | 6 | 0 | 3 | 129 | 3680 | 2351 | 3360 | 0 | 265 | 87 | 3 | 31 | 36 | 131 |
| Cons | N* | N* | N | N | N | N | G | N | B | Y | Y | N | N | T | N | V | N | D | N | C | N | N | N | N* | N* | N* |
| 99% | H* | H* | M | H | M | V | G | Y | Y | Y | T | Y | H | T | H | R | V | R | R | C | B | V | M | H* | H* | N* |
| 95% | M* | M* | M | M | M | S | G | Y | Y | C | T | T | Y | T | M | A | G | R | R | C | S | M | C | H* | H* | H* |
| 90% | M* | M* | M | M | M | S | G | Y | Y | C | T | T | T | T | M | A | G | R | R | C | S | A | C | H* | M* | H* |

Figure 3

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| # A | 661 | 146 | 1315 | 1323 | 920 | 8 | 0 | 1 | 0 | 0 | 0 | 1 | 4 | 0 | 441 | 1333 | 0 | 1199 | 21 | 0 | 1 | 1126 | 26 | 81 | 380 | 960 |
| # T | 63 | 121 | 2 | 2 | 6 | 2 | 0 | 39 | 1217 | 2 | 1331 | 1329 | 1207 | 1333 | 30 | 0 | 1 | 0 | 1 | 0 | 2 | 1 | 22 | 91 | 91 | 12 |
| # C | 601 | 1062 | 16 | 6 | 403 | 1 | 0 | 1293 | 116 | 1331 | 2 | 0 | 121 | 0 | 862 | 0 | 2 | 0 | 0 | 1333 | 1328 | 128 | 1284 | 1143 | 834 | 361 |
| # G | 8 | 4 | 0 | 2 | 4 | 1322 | 1333 | 0 | 0 | 0 | 0 | 3 | 1 | 0 | 0 | 0 | 1330 | 134 | 1311 | 0 | 2 | 78 | 1 | 18 | 28 | 0 |
| Cons | N• | N• | H | N | N | N | G | H | Y | Y | Y | D | N | T | H | A | B | R | D | C | N | N | N | N• | N• | H• |
| 99% | H• | H• | M | A | M | G | G | Y | Y | C | T | T | Y | T | H | A | G | R | R | C | C | V | H | N• | N• | M• |
| 95% | H• | H• | A | A | M | G | G | C | Y | C | T | T | Y | T | M | A | G | R | G | C | C | V | C | H• | H• | M• |
| 90% | M• | M• | A | A | M | G | G | C | T | C | T | T | T | T | M | A | G | R | G | C | C | M | C | Y• | M• | M• |

Figure 4

| | | | | | | Stem 1 | | | | | | Loop | | | | Stem 2 | | | | | | | | | | |
|---|---|---|---|---|---|---|---|---|---|---|---|---|---|---|---|---|---|---|---|---|---|---|---|---|---|---|
| | | | | | | < | < | < | < | < | < | • | • | • | • | > | > | > | > | > | > | | | | | |
| # A | 10 | 17 | 84 | 84 | 76 | 1 | 0 | 1 | 0 | 0 | 0 | 1 | 0 | 0 | 12 | 84 | 0 | 65 | 3 | 0 | 0 | 69 | 5 | 0 | 10 | 64 |
| # T | 8 | 6 | 0 | 0 | 2 | 2 | 0 | 1 | 67 | 0 | 84 | 80 | 81 | 84 | 5 | 0 | 0 | 0 | 0 | 0 | 0 | 0 | 4 | 25 | 24 | 3 |
| # C | 62 | 61 | 0 | 0 | 6 | 0 | 0 | 82 | 17 | 84 | 0 | 0 | 3 | 0 | 67 | 0 | 1 | 0 | 0 | 84 | 84 | 5 | 75 | 57 | 44 | 17 |
| # G | 4 | 0 | 0 | 0 | 0 | 81 | 84 | 0 | 0 | 0 | 0 | 3 | 0 | 0 | 0 | 0 | 83 | 19 | 81 | 0 | 0 | 10 | 0 | 2 | 6 | 0 |
| Cons | N* | H* | A | A | H | D | G | H | Y | C | T | D | Y | T | H | A | S | R | R | C | C | V | H | B* | N* | H* |
| 99% | N* | H* | A | A | H | D | G | H | Y | C | T | D | Y | T | H | A | S | R | R | C | C | V | H | B* | N* | H* |
| 95% | H* | H* | A | A | M | G | G | C | Y | C | T | T | T | T | H | A | G | R | G | C | C | V | M | Y* | N* | M* |
| 90% | H* | M* | A | A | A | G | G | C | Y | C | T | T | T | T | M | A | G | R | G | C | C | R | M | Y* | H* | M* |

Figure 5

mRNA

nucleotide sequence of PpLuc(GC) – ag – A64

GGGAGAAAGCTTGAGG*ATGGAGGACGCCAAGAACATCAAGAAGGGCCCGGCGCCCTTCTA*
*CCCGCTGGAGGACGGGACCGCCGGCGAGCAGCTCCACAAGGCCATGAAGCGGTACGCCCT*
*GGTGCCGGGCACGATCGCCTTCACCGACGCCCACATCGAGGTCGACATCACCTACGCGGA*
*GTACTTCGAGATGAGCGTGCGCCTGGCCGAGGCCATGAAGCGGTACGGCCTGAACACCAA*
*CCACCGGATCGTGGTGTGCTCGGAGAACAGCCTGCAGTTCTTCATGCCGGTGCTGGGCGC*
*CCTCTTCATCGGCGTGGCCGTCGCCCCGGCGAACGACATCTACAACGAGCGGGAGCTGCT*
*GAACAGCATGGGGATCAGCCAGCCGACCGTGGTGTTCGTGAGCAAGAAGGGCCTGCAGAA*
*GATCCTGAACGTGCAGAAGAAGCTGCCCATCATCCAGAAGATCATCATCATGGACAGCAA*
*GACCGACTACCAGGGCTTCCAGTCGATGTACACGTTCGTGACCAGCCACCTCCCGCCGGG*
*CTTCAACGAGTACGACTTCGTCCCGGAGAGCTTCGACCGGGACAAGACCATCGCCCTGAT*
*CATGAACAGCAGCGGCAGCACCGGCCTGCCGAAGGGGGTGGCCCTGCCGCACCGGACCGC*
*CTGCGTGCGCTTCTCGCACGCCCGGGACCCCATCTTCGGCAACCAGATCATCCCGGACAC*
*CGCCATCCTGAGCGTGGTGCCGTTCCACCACGGCTTCGGCATGTTCACGACCCTGGGCTA*
*CCTCATCTGCGGCTTCCGGGTGGTCCTGATGTACCGGTTCGAGGAGGAGCTGTTCCTGCG*
*GAGCCTGCAGGACTACAAGATCCAGAGCGCGCTGCTCGTGCCGACCCTGTTCAGCTTCTT*
*CGCCAAGAGCACCCTGATCGACAAGTACGACCTGTCGAACCTGCACGAGATCGCCAGCGG*
*GGGCGCCCCGCTGAGCAAGGAGGTGGGCGAGGCCGTGGCCAAGCGGTTCCACCTCCCGGG*
*CATCCGCCAGGGCTACGGCCTGACCGAGACCACGAGCGCGATCCTGATCACCCCCGAGGG*
*GGACGACAAGCCGGGCGCCGTGGGCAAGGTGGTCCCGTTCTTCGAGGCCAAGGTGGTGGA*
*CCTGGACACCGGCAAGACCCTGGGCGTGAACCAGCGGGGCGAGCTGTGCGTGCGGGGGCC*
*GATGATCATGAGCGGCTACGTGAACAACCCGGAGGCCACCAACGCCCTCATCGACAAGGA*
*CGGCTGGCTGCACAGCGGCGACATCGCCTACTGGGACGAGGACGAGCACTTCTTCATCGT*
*CGACCGGCTGAAGTCGCTGATCAAGTACAAGGGCTACCAGGTGGCGCCGGCCGAGCTGGA*
*GAGCATCCTGCTCCAGCACCCCAACATCTTCGACGCCGGCGTGGCCGGGCTGCCGGACGA*
*CGACGCCGGCGAGCTGCCGGCCGCGGTGGTGGTGCTGGAGCACGGCAAGACCATGACGGA*
*GAAGGAGATCGTCGACTACGTGGCCAGCCAGGTGACCACCGCCAAGAAGCTGCGGGGCGG*
*CGTGGTGTTCGTGGACGAGGTCCCGAAGGGCCTGACCGGGAAGCTCGACGCCCGGAAGAT*
*CCGCGAGATCCTGATCAAGGCCAAGAAGGGCGGCAAGATCGCCGTGTAA*GACTAGTTATA
AGACTGACTAGCCCGATGGGCCTCCCAACGGGCCCTCCTCCCCTCCTTGCACCGAGATTA
ATAGATCTAAAAAAAAAAAAAAAAAAAAAAAAAAAAAAAAAAAAAAAAAAAAAAAAAAAA
AAAAAAAAAAAA

Figure 6

nucleotide sequence of RPL32 – PpLuc(GC) – ag – A64 – C30 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAAATGCATCCCCC
CCCCCCCCCCCCCCCCCCCCCCCCCAAAGGCTCTTTTCAGAGCCACCAGAATT

Figure 7

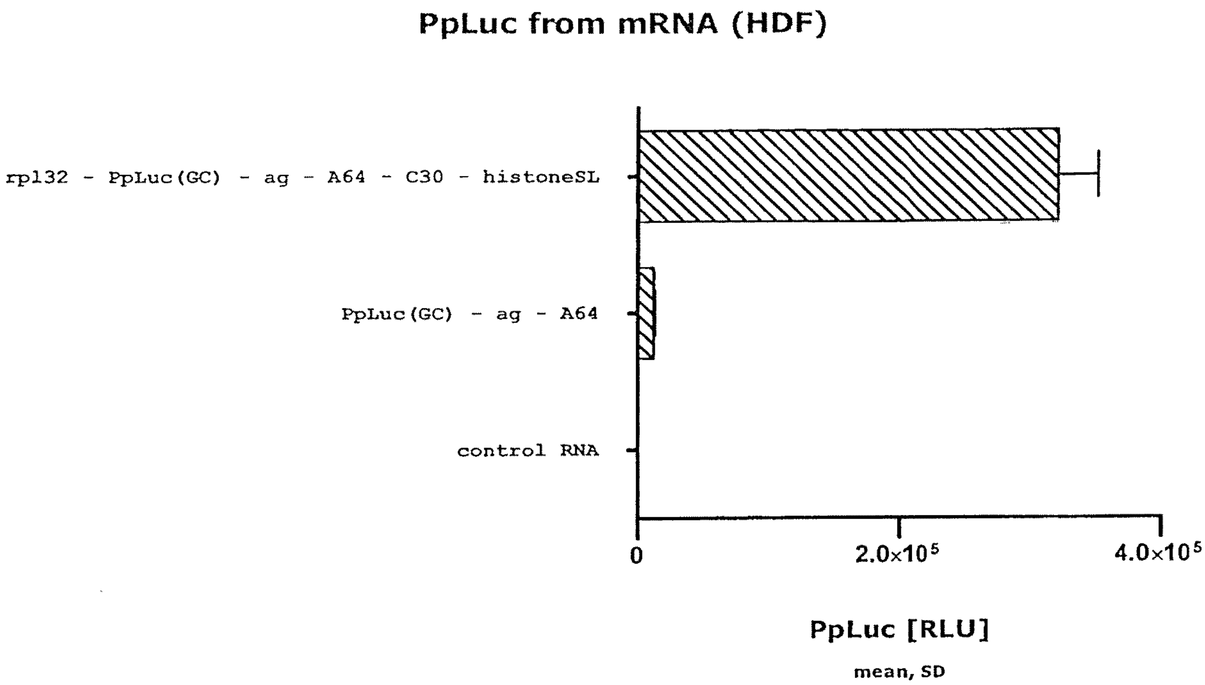
PpLuc from mRNA (HDF)
rpl32 - PpLuc(GC) - ag - A64 - C30 - histoneSL
PpLuc(GC) - ag - A64
control RNA
0
2.0×10⁵
4.0×10⁵
PpLuc [RLU]
mean, SD

Figure 8

nucleotide sequence of PpLuc(GC) – ag – A64 – histoneSL

GGGAGAAAGCTTGAGGATGGAGGACGCCAAGAACATCAAGAAGGGCCCGGCGCCCTTCTA
CCCGCTGGAGGACGGGACCGCCGGCGAGCAGCTCCACAAGGCCATGAAGCGGTACGCCCT
GGTGCCGGGCACGATCGCCTTCACCGACGCCCACATCGAGGTCGACATCACCTACGCGGA
GTACTTCGAGATGAGCGTGCGCCTGGCCGAGGCCATGAAGCGGTACGGCCTGAACACCAA
CCACCGGATCGTGGTGTGCTCGGAGAACAGCCTGCAGTTCTTCATGCCGGTGCTGGGCGC
CCTCTTCATCGGCGTGGCCGTCGCCCCGGCGAACGACATCTACAACGAGCGGGAGCTGCT
GAACAGCATGGGGATCAGCCAGCCGACCGTGGTGTTCGTGAGCAAGAAGGGCCTGCAGAA
GATCCTGAACGTGCAGAAGAAGCTGCCCATCATCCAGAAGATCATCATCATGGACAGCAA
GACCGACTACCAGGGCTTCCAGTCGATGTACACGTTCGTGACCAGCCACCTCCCGCCGGG
CTTCAACGAGTACGACTTCGTCCCGGAGAGCTTCGACCGGGACAAGACCATCGCCCTGAT
CATGAACAGCAGCGGCAGCACCGGCCTGCCGAAGGGGGTGGCCCTGCCGCACCGGACCGC
CTGCGTGCGCTTCTCGCACGCCCGGGACCCCATCTTCGGCAACCAGATCATCCCGGACAC
CGCCATCCTGAGCGTGGTGCCGTTCCACCACGGCTTCGGCATGTTCACGACCCTGGGCTA
CCTCATCTGCGGCTTCCGGGTGGTCCTGATGTACCGGTTCGAGGAGGAGCTGTTCCTGCG
GAGCCTGCAGGACTACAAGATCCAGAGCGCGCTGCTCGTGCCGACCCTGTTCAGCTTCTT
CGCCAAGAGCACCCTGATCGACAAGTACGACCTGTCGAACCTGCACGAGATCGCCAGCGG
GGGCGCCCCGCTGAGCAAGGAGGTGGGCGAGGCCGTGGCCAAGCGGTTCCACCTCCCGGG
CATCCGCCAGGGCTACGGCCTGACCGAGACCACGAGCGCGATCCTGATCACCCCCGAGGG
GGACGACAAGCCGGGCGCCGTGGGCAAGGTGGTCCCGTTCTTCGAGGCCAAGGTGGTGGA
CCTGGACACCGGCAAGACCCTGGGCGTGAACCAGCGGGGCGAGCTGTGCGTGCGGGGGCC
GATGATCATGAGCGGCTACGTGAACAACCCGGAGGCCACCAACGCCCTCATCGACAAGGA
CGGCTGGCTGCACAGCGGCGACATCGCCTACTGGGACGAGGACGAGCACTTCTTCATCGT
CGACCGGCTGAAGTCGCTGATCAAGTACAAGGGCTACCAGGTGGCGCCGGCCGAGCTGGA
GAGCATCCTGCTCCAGCACCCCAACATCTTCGACGCCGGCGTGGCCGGGCTGCCGGACGA
CGACGCCGGCGAGCTGCCGGCCGCGGTGGTGGTGCTGGAGCACGGCAAGACCATGACGGA
GAAGGAGATCGTCGACTACGTGGCCAGCCAGGTGACCACCGCCAAGAAGCTGCGGGGCGG
CGTGGTGTTCGTGGACGAGGTCCCGAAGGGCCTGACCGGGAAGCTCGACGCCCGGAAGAT
CCGCGAGATCCTGATCAAGGCCAAGAAGGGCGGCAAGATCGCCGTGTAAGACTAGTTATA
AGACTGACTAGCCCGATGGGCCTCCCAACGGGCCCTCCTCCCCTCCTTGCACCGAGATTA
ATAAAAAAAAAAAAAAAAAAAAAAAAAAAAAAAAAAAAAAAAAAAAAAAAAAAAAAAA
AAAAAATGCATCAAAGGCTCTTTTCAGAGCCACCA

Figure 9

nucleotide sequence of RPL32 – PpLuc(GC) – ag – A64

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAGATCTAAAAAAAAAAAAAA
AAAAAAAAAAAAAAAAAAAAAAAAAAAAAAAAAAAAAAAAAAAAAAAAAA

Figure 10

nucleotide sequence of RPL32 – PpLuc(GC) – ag – A64 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAA
AAAAAAAAAAAAAAAAAAAAAAAAAAAAAAAAAAAAAAAAAAAAATGCATCAAAGGCTCTT
TTCAGAGCCACCA

Figure 11

PpLuc from mRNA with combined UTRs (HDF)

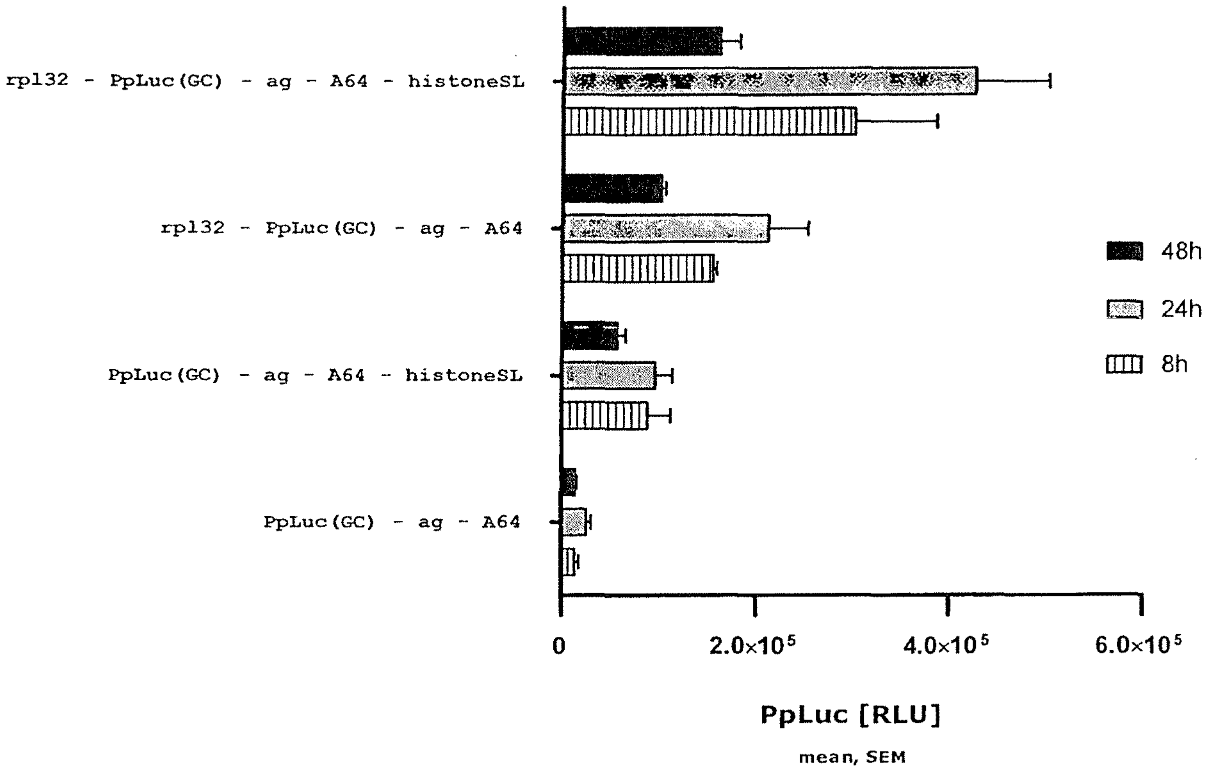


Figure 12

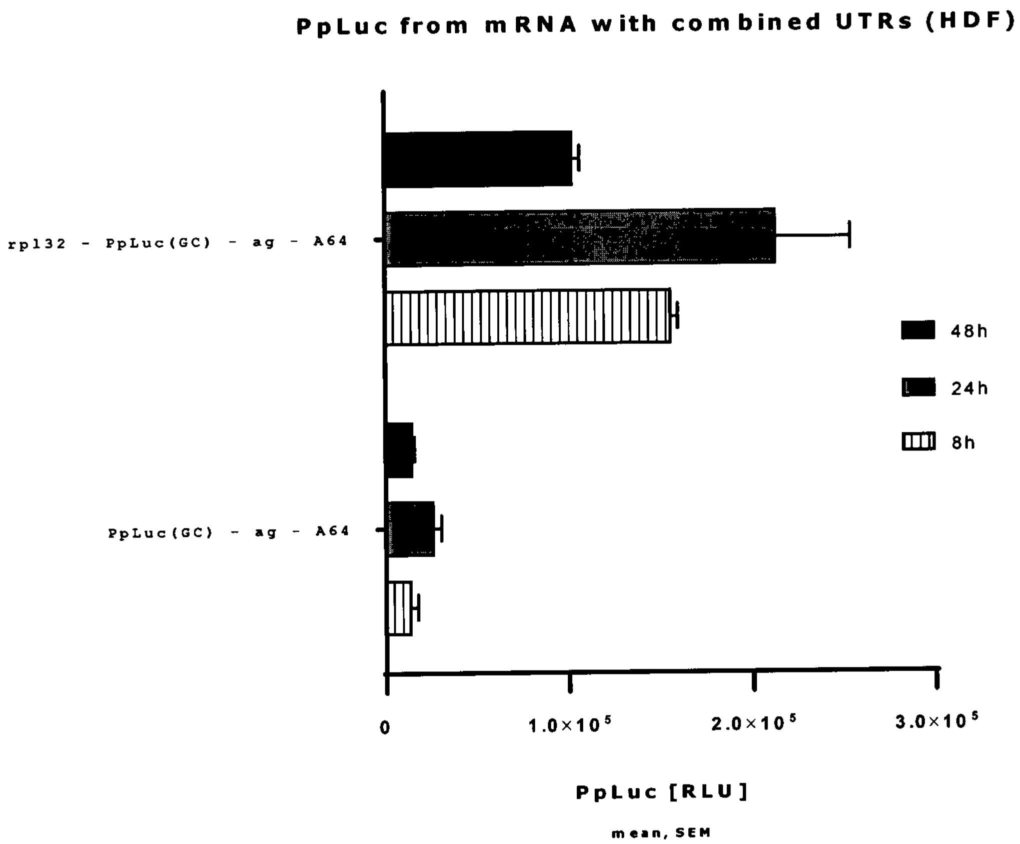
PpLuc from mRNA with combined UTRs (HDF)
rpl32 - PpLuc(GC) - ag - A64
PpLuc(GC) - ag - A64
48h
24h
8h
0
1.0×10⁵
2.0×10⁵
3.0×10⁵
PpLuc [RLU]
mean, SEM

Fig. 12A

nucleotide sequence of RPL32 – PpLuc(GC) – albumin7 – A64 – C30 – histoneSL

GGGGCGCTGCCTACGGAGGTGGCAGCCATCTCCTTCTCGGCATCAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT

Figure 13

nucleotide sequence of RPL35 – PpLuc(GC) – albumin7 – A64 – C30 – histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT

**Figure 14**

nucleotide sequence of RPL21 – PpLuc(GC) – albumin7 – A64 – C30 – histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT

**Figure 15**

nucleotide sequence of atp5a1 – PpLuc(GC) – albumin7 – A64 – C30 – histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGGATGGAGGACGCCAAGAACATCAAGAAGGGCCCG
GCGCCCTTCTACCCGCTGGAGGACGGGACCGCCGGCGAGCAGCTCCACAAGGCCATGAAG
CGGTACGCCCTGGTGCCGGGCACGATCGCCTTCACCGACGCCCACATCGAGGTCGACATC
ACCTACGCGGAGTACTTCGAGATGAGCGTGCGCCTGGCCGAGGCCATGAAGCGGTACGGC
CTGAACACCAACCACCGGATCGTGGTGTGCTCGGAGAACAGCCTGCAGTTCTTCATGCCG
GTGCTGGGCGCCCTCTTCATCGGCGTGGCCGTCGCCCCGGCGAACGACATCTACAACGAG
CGGGAGCTGCTGAACAGCATGGGGATCAGCCAGCCGACCGTGGTGTTCGTGAGCAAGAAG
GGCCTGCAGAAGATCCTGAACGTGCAGAAGAAGCTGCCCATCATCCAGAAGATCATCATC
ATGGACAGCAAGACCGACTACCAGGGCTTCCAGTCGATGTACACGTTCGTGACCAGCCAC
CTCCCGCCGGGCTTCAACGAGTACGACTTCGTCCCGGAGAGCTTCGACCGGGACAAGACC
ATCGCCCTGATCATGAACAGCAGCGGCAGCACCGGCCTGCCGAAGGGGGTGGCCCTGCCG
CACCGGACCGCCTGCGTGCGCTTCTCGCACGCCCGGGACCCCATCTTCGGCAACCAGATC
ATCCCGGACACCGCCATCCTGAGCGTGGTGCCGTTCCACCACGGCTTCGGCATGTTCACG
ACCCTGGGCTACCTCATCTGCGGCTTCCGGGTGGTCCTGATGTACCGGTTCGAGGAGGAG
CTGTTCCTGCGGAGCCTGCAGGACTACAAGATCCAGAGCGCGCTGCTCGTGCCGACCCTG
TTCAGCTTCTTCGCCAAGAGCACCCTGATCGACAAGTACGACCTGTCGAACCTGCACGAG
ATCGCCAGCGGGGGCGCCCCGCTGAGCAAGGAGGTGGGCGAGGCCGTGGCCAAGCGGTTC
CACCTCCCGGGCATCCGCCAGGGCTACGGCCTGACCGAGACCACGAGCGCGATCCTGATC
ACCCCCGAGGGGGACGACAAGCCGGGCGCCGTGGGCAAGGTGGTCCCGTTCTTCGAGGCC
AAGGTGGTGGACCTGGACACCGGCAAGACCCTGGGCGTGAACCAGCGGGGCGAGCTGTGC
GTGCGGGGGCCGATGATCATGAGCGGCTACGTGAACAACCCGGAGGCCACCAACGCCCTC
ATCGACAAGGACGGCTGGCTGCACAGCGGCGACATCGCCTACTGGGACGAGGACGAGCAC
TTCTTCATCGTCGACCGGCTGAAGTCGCTGATCAAGTACAAGGGCTACCAGGTGGCGCCG
GCCGAGCTGGAGAGCATCCTGCTCCAGCACCCCAACATCTTCGACGCCGGCGTGGCCGGG
CTGCCGGACGACGACGCCGGCGAGCTGCCGGCCGCGGTGGTGGTGCTGGAGCACGGCAAG
ACCATGACGGAGAAGGAGATCGTCGACTACGTGGCCAGCCAGGTGACCACCGCCAAGAAG
CTGCGGGGCGGCGTGGTGTTCGTGGACGAGGTCCCGAAGGGCCTGACCGGGAAGCTCGAC
GCCCGGAAGATCCGCGAGATCCTGATCAAGGCCAAGAAGGGCGGCAAGATCGCCGTGTAA
GACTAGTGCATCACATTTAAAAGCATCTCAGCCTACCATGAGAATAAGAGAAAGAAAATG
AAGATCAATAGCTTATTCATCTCTTTTTCTTTTTCGTTGGTGTAAAGCCAACACCCTGTC
TAAAAAACATAAATTTCTTTAATCATTTTGCCTCTTTTCTCTGTGCTTCAATTAATAAAA
AATGGAAAGAACCTAGATCTAAAAAAAAAAAAAAAAAAAAAAAAAAAAAAAAAAAAAAAA
AAAAAAAAAAAAAAAAAAAAAAAATGCATCCCCCCCCCCCCCCCCCCCCCCCCCCCCCC
AAAGGCTCTTTTCAGAGCCACCAGAATT

**Figure 16**

nucleotide sequence of HSD17B4 – PpLuc(GC) – albumin7 – A64 – C30 – histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTGCATCA
CATTTAAAAGCATCTCAGCCTACCATGAGAATAAGAGAAAGAAAATGAAGATCAATAGCT
TATTCATCTCTTTTTCTTTTTCGTTGGTGTAAAGCCAACACCCTGTCTAAAAAACATAAA
TTTCTTTAATCATTTTGCCTCTTTTCTCTGTGCTTCAATTAATAAAAAATGGAAAGAACC
TAGATCTAAAAAAAAAAAAAAAAAAAAAAAAAAAAAAAAAAAAAAAAAAAAAAAAAAAAA
AAAAAAAAAAATGCATCCCCCCCCCCCCCCCCCCCCCCCCCCCCCCCAAAGGCTCTTTTC
AGAGCCACCAGAATT

**Figure 17**

nucleotide sequence of AIG1 – PpLuc(GC) – albumin7 – A64 – C30 – histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTGCATCACATTTAAAAGCATCTCAGCCTACCATGAGAA
TAAGAGAAAGAAAATGAAGATCAATAGCTTATTCATCTCTTTTTCTTTTTCGTTGGTGTA
AAGCCAACACCCTGTCTAAAAAACATAAATTTCTTTAATCATTTTGCCTCTTTTCTCTGT
GCTTCAATTAATAAAAAATGGAAAGAACCTAGATCTAAAAAAAAAAAAAAAAAAAAAAAA
AAAAAAAAAAAAAAAAAAAAAAAAAAAAAAAAAAAAAAAATGCATCCCCCCCCCCCCCCC
CCCCCCCCCCCCCCCAAAGGCTCTTTTCAGAGCCACCAGAATT

# Figure 18

nucleotide sequence of COX6C – PpLuc(GC) – albumin7 – A64 – C30 – histoneSL

GGAGTCAGGAAGGACGTTGGTGTTGAGGTTAGCATACGTATCAAGGACAGTAACTACCAA
GCTTGAGG*ATGGAGGACGCCAAGAACATCAAGAAGGGCCCGGCGCCCTTCTACCCGCTGG*
*AGGACGGGACCGCCGGCGAGCAGCTCCACAAGGCCATGAAGCGGTACGCCCTGGTGCCGG*
*GCACGATCGCCTTCACCGACGCCCACATCGAGGTCGACATCACCTACGCGGAGTACTTCG*
*AGATGAGCGTGCGCCTGGCCGAGGCCATGAAGCGGTACGGCCTGAACACCAACCACCGGA*
*TCGTGGTGTGCTCGGAGAACAGCCTGCAGTTCTTCATGCCGGTGCTGGGCGCCCTCTTCA*
*TCGGCGTGGCCGTCGCCCCGGCGAACGACATCTACAACGAGCGGGAGCTGCTGAACAGCA*
*TGGGGATCAGCCAGCCGACCGTGGTGTTCGTGAGCAAGAAGGGCCTGCAGAAGATCCTGA*
*ACGTGCAGAAGAAGCTGCCCATCATCCAGAAGATCATCATCATGGACAGCAAGACCGACT*
*ACCAGGGCTTCCAGTCGATGTACACGTTCGTGACCAGCCACCTCCCGCCGGGCTTCAACG*
*AGTACGACTTCGTCCCGGAGAGCTTCGACCGGGACAAGACCATCGCCCTGATCATGAACA*
*GCAGCGGCAGCACCGGCCTGCCGAAGGGGGTGGCCCTGCCGCACCGGACCGCCTGCGTGC*
*GCTTCTCGCACGCCCGGGACCCCATCTTCGGCAACCAGATCATCCCGGACACCGCCATCC*
*TGAGCGTGGTGCCGTTCCACCACGGCTTCGGCATGTTCACGACCCTGGGCTACCTCATCT*
*GCGGCTTCCGGGTGGTCCTGATGTACCGGTTCGAGGAGGAGCTGTTCCTGCGGAGCCTGC*
*AGGACTACAAGATCCAGAGCGCGCTGCTCGTGCCGACCCTGTTCAGCTTCTTCGCCAAGA*
*GCACCCTGATCGACAAGTACGACCTGTCGAACCTGCACGAGATCGCCAGCGGGGGCGCCC*
*CGCTGAGCAAGGAGGTGGGCGAGGCCGTGGCCAAGCGGTTCCACCTCCCGGGCATCCGCC*
*AGGGCTACGGCCTGACCGAGACCACGAGCGCGATCCTGATCACCCCCGAGGGGGACGACA*
*AGCCGGGCGCCGTGGGCAAGGTGGTCCCGTTCTTCGAGGCCAAGGTGGTGGACCTGGACA*
*CCGGCAAGACCCTGGGCGTGAACCAGCGGGGCGAGCTGTGCGTGCGGGGGCCGATGATCA*
*TGAGCGGCTACGTGAACAACCCGGAGGCCACCAACGCCCTCATCGACAAGGACGGCTGGC*
*TGCACAGCGGCGACATCGCCTACTGGGACGAGGACGAGCACTTCTTCATCGTCGACCGGC*
*TGAAGTCGCTGATCAAGTACAAGGGCTACCAGGTGGCGCCGGCCGAGCTGGAGAGCATCC*
*TGCTCCAGCACCCCAACATCTTCGACGCCGGCGTGGCCGGGCTGCCGGACGACGACGCCG*
*GCGAGCTGCCGGCCGCGGTGGTGGTGCTGGAGCACGGCAAGACCATGACGGAGAAGGAGA*
*TCGTCGACTACGTGGCCAGCCAGGTGACCACCGCCAAGAAGCTGCGGGGCGGCGTGGTGT*
*TCGTGGACGAGGTCCCGAAGGGCCTGACCGGGAAGCTCGACGCCCGGAAGATCCGCGAGA*
*TCCTGATCAAGGCCAAGAAGGGCGGCAAGATCGCCGTGTAA*GACTAGTGCATCACATTTA
AAAGCATCTCAGCCTACCATGAGAATAAGAGAAAGAAAATGAAGATCAATAGCTTATTCA
TCTCTTTTTCTTTTTCGTTGGTGTAAAGCCAACACCCTGTCTAAAAAACATAAATTTCTT
TAATCATTTTGCCTCTTTTCTCTGTGCTTCAATTAATAAAAAATGGAAAGAACCTAGATC
TAAAAAAAAAAAAAAAAAAAAAAAAAAAAAAAAAAAAAAAAAAAAAAAAAAAAAAAAAAA
AAAAATGCATCCCCCCCCCCCCCCCCCCCCCCCCCCCCCCAAAGGCTCTTTTCAGAGCC
ACCAGAATT

**Figure 19**

nucleotide sequence of ASAH1 – PpLuc(GC) – albumin7 – A64 – C30 – histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTGCATCACATTTAAAAGCATCTCAGCC
TACCATGAGAATAAGAGAAAGAAAATGAAGATCAATAGCTTATTCATCTCTTTTTCTTTT
TCGTTGGTGTAAAGCCAACACCCTGTCTAAAAAACATAAATTTCTTTAATCATTTTGCCT
CTTTTCTCTGTGCTTCAATTAATAAAAAATGGAAAGAACCTAGATCTAAAAAAAAAAAAA
AAAAAAAAAAAAAAAAAAAAAAAAAAAAAAAAAAAAAAAAAAAAAAAAAAATGCATCCCC
CCCCCCCCCCCCCCCCCCCCCCCCCCAAAGGCTCTTTTCAGAGCCACCAGAATT

**Figure 20**

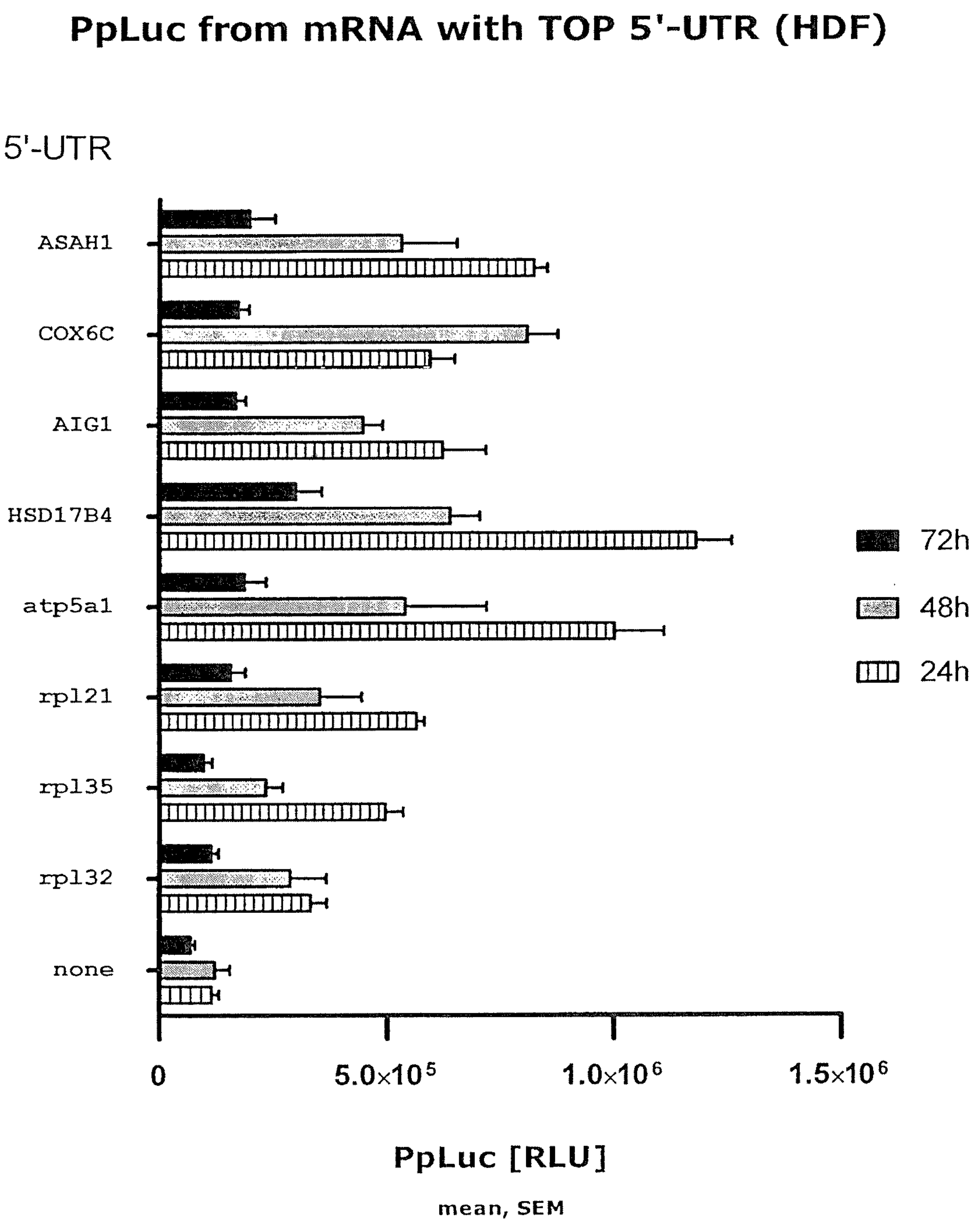
PpLuc from mRNA with TOP 5'-UTR (HDF)
5'-UTR
ASAH1
COX6C
AIG1
HSD17B4
atp5a1
rpl21
rpl35
rpl32
none
72h
48h
24h
0
5.0×10^5
1.0×10^6
1.5×10^6
PpLuc [RLU]
mean, SEM

Figure 21

nucleotide sequence of RPL35 – PpLuc(GC) – ag – A64

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA

Figure 22

nucleotide sequence of rpl21 – PpLuc(GC) – ag – A64

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAA

Figure 23

nucleotide sequence of atp5a1 – PpLuc(GC) – ag – A64

GGGCGGCTCGGCCATTTTGTCCCAGTCAGTCCGGAGGCTGCGGCTGCAGAAGTACCGCCT
GCGGAGTAACTGCAAAGAAGCTTGAGG*ATGGAGGACGCCAAGAACATCAAGAAGGGCCCG*
*GCGCCCTTCTACCCGCTGGAGGACGGGACCGCCGGCGAGCAGCTCCACAAGGCCATGAAG*
*CGGTACGCCCTGGTGCCGGGCACGATCGCCTTCACCGACGCCCACATCGAGGTCGACATC*
*ACCTACGCGGAGTACTTCGAGATGAGCGTGCGCCTGGCCGAGGCCATGAAGCGGTACGGC*
*CTGAACACCAACCACCGGATCGTGGTGTGCTCGGAGAACAGCCTGCAGTTCTTCATGCCG*
*GTGCTGGGCGCCCTCTTCATCGGCGTGGCCGTCGCCCCGGCGAACGACATCTACAACGAG*
*CGGGAGCTGCTGAACAGCATGGGGATCAGCCAGCCGACCGTGGTGTTCGTGAGCAAGAAG*
*GGCCTGCAGAAGATCCTGAACGTGCAGAAGAAGCTGCCCATCATCCAGAAGATCATCATC*
*ATGGACAGCAAGACCGACTACCAGGGCTTCCAGTCGATGTACACGTTCGTGACCAGCCAC*
*CTCCCGCCGGGCTTCAACGAGTACGACTTCGTCCCGGAGAGCTTCGACCGGGACAAGACC*
*ATCGCCCTGATCATGAACAGCAGCGGCAGCACCGGCCTGCCGAAGGGGGTGGCCCTGCCG*
*CACCGGACCGCCTGCGTGCGCTTCTCGCACGCCCGGGACCCCATCTTCGGCAACCAGATC*
*ATCCCGGACACCGCCATCCTGAGCGTGGTGCCGTTCCACCACGGCTTCGGCATGTTCACG*
*ACCCTGGGCTACCTCATCTGCGGCTTCCGGGTGGTCCTGATGTACCGGTTCGAGGAGGAG*
*CTGTTCCTGCGGAGCCTGCAGGACTACAAGATCCAGAGCGCGCTGCTCGTGCCGACCCTG*
*TTCAGCTTCTTCGCCAAGAGCACCCTGATCGACAAGTACGACCTGTCGAACCTGCACGAG*
*ATCGCCAGCGGGGGCGCCCCGCTGAGCAAGGAGGTGGGCGAGGCCGTGGCCAAGCGGTTC*
*CACCTCCCGGGCATCCGCCAGGGCTACGGCCTGACCGAGACCACGAGCGCGATCCTGATC*
*ACCCCCGAGGGGGACGACAAGCCGGGCGCCGTGGGCAAGGTGGTCCCGTTCTTCGAGGCC*
*AAGGTGGTGGACCTGGACACCGGCAAGACCCTGGGCGTGAACCAGCGGGGCGAGCTGTGC*
*GTGCGGGGGCCGATGATCATGAGCGGCTACGTGAACAACCCGGAGGCCACCAACGCCCTC*
*ATCGACAAGGACGGCTGGCTGCACAGCGGCGACATCGCCTACTGGGACGAGGACGAGCAC*
*TTCTTCATCGTCGACCGGCTGAAGTCGCTGATCAAGTACAAGGGCTACCAGGTGGCGCCG*
*GCCGAGCTGGAGAGCATCCTGCTCCAGCACCCCAACATCTTCGACGCCGGCGTGGCCGGG*
*CTGCCGGACGACGACGCCGGCGAGCTGCCGGCCGCGGTGGTGGTGCTGGAGCACGGCAAG*
*ACCATGACGGAGAAGGAGATCGTCGACTACGTGGCCAGCCAGGTGACCACCGCCAAGAAG*
*CTGCGGGGCGGCGTGGTGTTCGTGGACGAGGTCCCGAAGGGCCTGACCGGGAAGCTCGAC*
*GCCCGGAAGATCCGCGAGATCCTGATCAAGGCCAAGAAGGGCGGCAAGATCGCCGTGTAA*
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAAA
AAAAAAAAAAAAAAAAA

Figure 24

nucleotide sequence of HSD17B4 – PpLuc(GC) – ag – A64

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAA

Figure 25

nucleotide sequence of AIG1 – PpLuc(GC) – ag – A64

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAA

Figure 26

nucleotide sequence of COX6C – PpLuc(GC) – ag – A64

GGAGTCAGGAAGGACGTTGGTGTTGAGGTTAGCATACGTATCAAGGACAGTAACTACCAA
GCTTGAGGATGGAGGACGCCAAGAACATCAAGAAGGGCCCGGCGCCCTTCTACCCGCTGG
AGGACGGGACCGCCGGCGAGCAGCTCCACAAGGCCATGAAGCGGTACGCCCTGGTGCCGG
GCACGATCGCCTTCACCGACGCCCACATCGAGGTCGACATCACCTACGCGGAGTACTTCG
AGATGAGCGTGCGCCTGGCCGAGGCCATGAAGCGGTACGGCCTGAACACCAACCACCGGA
TCGTGGTGTGCTCGGAGAACAGCCTGCAGTTCTTCATGCCGGTGCTGGGCGCCCTCTTCA
TCGGCGTGGCCGTCGCCCCGGCGAACGACATCTACAACGAGCGGGAGCTGCTGAACAGCA
TGGGGATCAGCCAGCCGACCGTGGTGTTCGTGAGCAAGAAGGGCCTGCAGAAGATCCTGA
ACGTGCAGAAGAAGCTGCCCATCATCCAGAAGATCATCATCATGGACAGCAAGACCGACT
ACCAGGGCTTCCAGTCGATGTACACGTTCGTGACCAGCCACCTCCCGCCGGGCTTCAACG
AGTACGACTTCGTCCCGGAGAGCTTCGACCGGGACAAGACCATCGCCCTGATCATGAACA
GCAGCGGCAGCACCGGCCTGCCGAAGGGGGTGGCCCTGCCGCACCGGACCGCCTGCGTGC
GCTTCTCGCACGCCCGGGACCCCATCTTCGGCAACCAGATCATCCCGGACACCGCCATCC
TGAGCGTGGTGCCGTTCCACCACGGCTTCGGCATGTTCACGACCCTGGGCTACCTCATCT
GCGGCTTCCGGGTGGTCCTGATGTACCGGTTCGAGGAGGAGCTGTTCCTGCGGAGCCTGC
AGGACTACAAGATCCAGAGCGCGCTGCTCGTGCCGACCCTGTTCAGCTTCTTCGCCAAGA
GCACCCTGATCGACAAGTACGACCTGTCGAACCTGCACGAGATCGCCAGCGGGGGCGCCC
CGCTGAGCAAGGAGGTGGGCGAGGCCGTGGCCAAGCGGTTCCACCTCCCGGGCATCCGCC
AGGGCTACGGCCTGACCGAGACCACGAGCGCGATCCTGATCACCCCCGAGGGGGACGACA
AGCCGGGCGCCGTGGGCAAGGTGGTCCCGTTCTTCGAGGCCAAGGTGGTGGACCTGGACA
CCGGCAAGACCCTGGGCGTGAACCAGCGGGGCGAGCTGTGCGTGCGGGGGCCGATGATCA
TGAGCGGCTACGTGAACAACCCGGAGGCCACCAACGCCCTCATCGACAAGGACGGCTGGC
TGCACAGCGGCGACATCGCCTACTGGGACGAGGACGAGCACTTCTTCATCGTCGACCGGC
TGAAGTCGCTGATCAAGTACAAGGGCTACCAGGTGGCGCCGGCCGAGCTGGAGAGCATCC
TGCTCCAGCACCCCAACATCTTCGACGCCGGCGTGGCCGGGCTGCCGGACGACGACGCCG
GCGAGCTGCCGGCCGCGGTGGTGGTGCTGGAGCACGGCAAGACCATGACGGAGAAGGAGA
TCGTCGACTACGTGGCCAGCCAGGTGACCACCGCCAAGAAGCTGCGGGGCGGCGTGGTGT
TCGTGGACGAGGTCCCGAAGGGCCTGACCGGGAAGCTCGACGCCCGGAAGATCCGCGAGA
TCCTGATCAAGGCCAAGAAGGGCGGCAAGATCGCCGTGTAAGACTAGTTATAAGACTGAC
TAGCCCGATGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA

Figure 27

nucleotide sequence of ASAH1 – PpLuc(GC) – ag – A64

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGGCCCTCCTCCCCTCCTTGCACCGAGATTAATAAAAAAAAAAAAAAAAAAAA
AAAAAAAAAAAAAAAAAAAAAAAAAAAAAAAAAAAAAAAAAAAA

Figure 28

nucleotide sequence of RPL35 – PpLuc(GC) – ag – A64 – histoneSL

GGGGAGCGGGCGGCGGCGTTGGCGGCTTGTGCAGCAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA

Figure 29

nucleotide sequence of rpl21 – PpLuc(GC) – ag – A64 – histoneSL

GGGGCCGGAACCGCCATCTTCCAGTAATTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTTATAAGACTGACTAGCCCGATGGGCCTCCCAACG
GGCCCTCCTCCCCTCCTTGCACCGAGATTAATAAAAAAAAAAAAAAAAAAAAAAAAAAAA
AAAAAAAAAAAAAAAAAAAAAAAAAAAAAAAAAAAATGCATCAAAGGCTCTTTTCAGAGC
CACCA

Figure 30

nucleotide sequence of atp5a1 – PpLuc(GC) – ag – A64 – histoneSL

GGGCGGCTCGGCCATTTTGTCCCAGTCAGTCCGGAGGCTGCGGCTGCAGAAGTACCGCCT
GCGGAGTAACTGCAAAGAAGCTTGAGG*ATGGAGGACGCCAAGAACATCAAGAAGGGCCCG*
*GCGCCCTTCTACCCGCTGGAGGACGGGACCGCCGGCGAGCAGCTCCACAAGGCCATGAAG*
*CGGTACGCCCTGGTGCCGGGCACGATCGCCTTCACCGACGCCCACATCGAGGTCGACATC*
*ACCTACGCGGAGTACTTCGAGATGAGCGTGCGCCTGGCCGAGGCCATGAAGCGGTACGGC*
*CTGAACACCAACCACCGGATCGTGGTGTGCTCGGAGAACAGCCTGCAGTTCTTCATGCCG*
*GTGCTGGGCGCCCTCTTCATCGGCGTGGCCGTCGCCCCGGCGAACGACATCTACAACGAG*
*CGGGAGCTGCTGAACAGCATGGGGATCAGCCAGCCGACCGTGGTGTTCGTGAGCAAGAAG*
*GGCCTGCAGAAGATCCTGAACGTGCAGAAGAAGCTGCCCATCATCCAGAAGATCATCATC*
*ATGGACAGCAAGACCGACTACCAGGGCTTCCAGTCGATGTACACGTTCGTGACCAGCCAC*
*CTCCCGCCGGGCTTCAACGAGTACGACTTCGTCCCGGAGAGCTTCGACCGGGACAAGACC*
*ATCGCCCTGATCATGAACAGCAGCGGCAGCACCGGCCTGCCGAAGGGGGTGGCCCTGCCG*
*CACCGGACCGCCTGCGTGCGCTTCTCGCACGCCCGGGACCCCATCTTCGGCAACCAGATC*
*ATCCCGGACACCGCCATCCTGAGCGTGGTGCCGTTCCACCACGGCTTCGGCATGTTCACG*
*ACCCTGGGCTACCTCATCTGCGGCTTCCGGGTGGTCCTGATGTACCGGTTCGAGGAGGAG*
*CTGTTCCTGCGGAGCCTGCAGGACTACAAGATCCAGAGCGCGCTGCTCGTGCCGACCCTG*
*TTCAGCTTCTTCGCCAAGAGCACCCTGATCGACAAGTACGACCTGTCGAACCTGCACGAG*
*ATCGCCAGCGGGGGCGCCCCGCTGAGCAAGGAGGTGGGCGAGGCCGTGGCCAAGCGGTTC*
*CACCTCCCGGGCATCCGCCAGGGCTACGGCCTGACCGAGACCACGAGCGCGATCCTGATC*
*ACCCCCGAGGGGGACGACAAGCCGGGCGCCGTGGGCAAGGTGGTCCCGTTCTTCGAGGCC*
*AAGGTGGTGGACCTGGACACCGGCAAGACCCTGGGCGTGAACCAGCGGGGCGAGCTGTGC*
*GTGCGGGGGCCGATGATCATGAGCGGCTACGTGAACAACCCGGAGGCCACCAACGCCCTC*
*ATCGACAAGGACGGCTGGCTGCACAGCGGCGACATCGCCTACTGGGACGAGGACGAGCAC*
*TTCTTCATCGTCGACCGGCTGAAGTCGCTGATCAAGTACAAGGGCTACCAGGTGGCGCCG*
*GCCGAGCTGGAGAGCATCCTGCTCCAGCACCCCAACATCTTCGACGCCGGCGTGGCCGGG*
*CTGCCGGACGACGACGCCGGCGAGCTGCCGGCCGCGGTGGTGGTGCTGGAGCACGGCAAG*
*ACCATGACGGAGAAGGAGATCGTCGACTACGTGGCCAGCCAGGTGACCACCGCCAAGAAG*
*CTGCGGGGCGGCGTGGTGTTCGTGGACGAGGTCCCGAAGGGCCTGACCGGGAAGCTCGAC*
*GCCCGGAAGATCCGCGAGATCCTGATCAAGGCCAAGAAGGGCGGCAAGATCGCCGTGTAA*
GACTAGTTATAAGACTGACTAGCCCGATGGGCCTCCCAACGGGCCCTCCTCCCCTCCTTG
CACCGAGATTAATAAAAAAAAAAAAAAAAAAAAAAAAAAAAAAAAAAAAAAAAAAAAAAA
AAAAAAAAAAAAAAAAATGCATCAAAGGCTCTTTTCAGAGCCACCA

Figure 31

nucleotide sequence of HSD17B4 – PpLuc(GC) – ag – A64 – histoneSL

GGGTCCCGCAGTCGGCGTCCAGCGGCTCTGCTTGTTCGTGTGTGTGTCGTTGCAGGCCTT
ATTCAAGCTTGAGGATGGAGGACGCCAAGAACATCAAGAAGGGCCCGGCGCCCTTCTACC
CGCTGGAGGACGGGACCGCCGGCGAGCAGCTCCACAAGGCCATGAAGCGGTACGCCCTGG
TGCCGGGCACGATCGCCTTCACCGACGCCCACATCGAGGTCGACATCACCTACGCGGAGT
ACTTCGAGATGAGCGTGCGCCTGGCCGAGGCCATGAAGCGGTACGGCCTGAACACCAACC
ACCGGATCGTGGTGTGCTCGGAGAACAGCCTGCAGTTCTTCATGCCGGTGCTGGGCGCCC
TCTTCATCGGCGTGGCCGTCGCCCCGGCGAACGACATCTACAACGAGCGGGAGCTGCTGA
ACAGCATGGGGATCAGCCAGCCGACCGTGGTGTTCGTGAGCAAGAAGGGCCTGCAGAAGA
TCCTGAACGTGCAGAAGAAGCTGCCCATCATCCAGAAGATCATCATCATGGACAGCAAGA
CCGACTACCAGGGCTTCCAGTCGATGTACACGTTCGTGACCAGCCACCTCCCGCCGGGCT
TCAACGAGTACGACTTCGTCCCGGAGAGCTTCGACCGGGACAAGACCATCGCCCTGATCA
TGAACAGCAGCGGCAGCACCGGCCTGCCGAAGGGGGTGGCCCTGCCGCACCGGACCGCCT
GCGTGCGCTTCTCGCACGCCCGGGACCCCATCTTCGGCAACCAGATCATCCCGGACACCG
CCATCCTGAGCGTGGTGCCGTTCCACCACGGCTTCGGCATGTTCACGACCCTGGGCTACC
TCATCTGCGGCTTCCGGGTGGTCCTGATGTACCGGTTCGAGGAGGAGCTGTTCCTGCGGA
GCCTGCAGGACTACAAGATCCAGAGCGCGCTGCTCGTGCCGACCCTGTTCAGCTTCTTCG
CCAAGAGCACCCTGATCGACAAGTACGACCTGTCGAACCTGCACGAGATCGCCAGCGGGG
GCGCCCCGCTGAGCAAGGAGGTGGGCGAGGCCGTGGCCAAGCGGTTCCACCTCCCGGGCA
TCCGCCAGGGCTACGGCCTGACCGAGACCACGAGCGCGATCCTGATCACCCCCGAGGGGG
ACGACAAGCCGGGCGCCGTGGGCAAGGTGGTCCCGTTCTTCGAGGCCAAGGTGGTGGACC
TGGACACCGGCAAGACCCTGGGCGTGAACCAGCGGGGCGAGCTGTGCGTGCGGGGGCCGA
TGATCATGAGCGGCTACGTGAACAACCCGGAGGCCACCAACGCCCTCATCGACAAGGACG
GCTGGCTGCACAGCGGCGACATCGCCTACTGGGACGAGGACGAGCACTTCTTCATCGTCG
ACCGGCTGAAGTCGCTGATCAAGTACAAGGGCTACCAGGTGGCGCCGGCCGAGCTGGAGA
GCATCCTGCTCCAGCACCCCAACATCTTCGACGCCGGCGTGGCCGGGCTGCCGGACGACG
ACGCCGGCGAGCTGCCGGCCGCGGTGGTGGTGCTGGAGCACGGCAAGACCATGACGGAGA
AGGAGATCGTCGACTACGTGGCCAGCCAGGTGACCACCGCCAAGAAGCTGCGGGGCGGCG
TGGTGTTCGTGGACGAGGTCCCGAAGGGCCTGACCGGGAAGCTCGACGCCCGGAAGATCC
GCGAGATCCTGATCAAGGCCAAGAAGGGCGGCAAGATCGCCGTGTAAGACTAGTTATAAG
ACTGACTAGCCCGATGGGCCTCCCAACGGGCCCTCCTCCCCTCCTTGCACCGAGATTAAT
AAAAAAAAAAAAAAAAAAAAAAAAAAAAAAAAAAAAAAAAAAAAAAAAAAAAAAAAAAAA
AAAATGCATCAAAGGCTCTTTTCAGAGCCACCA

Figure 32

nucleotide sequence of AIG1 – PpLuc(GC) – ag – A64 – histoneSL

GGGCCGCCCAGCCGGTCCAGGCCTCTGGCGAACAAGCTTGAGGATGGAGGACGCCAAGAA
CATCAAGAAGGGCCCGGCGCCCTTCTACCCGCTGGAGGACGGGACCGCCGGCGAGCAGCT
CCACAAGGCCATGAAGCGGTACGCCCTGGTGCCGGGCACGATCGCCTTCACCGACGCCCA
CATCGAGGTCGACATCACCTACGCGGAGTACTTCGAGATGAGCGTGCGCCTGGCCGAGGC
CATGAAGCGGTACGGCCTGAACACCAACCACCGGATCGTGGTGTGCTCGGAGAACAGCCT
GCAGTTCTTCATGCCGGTGCTGGGCGCCCTCTTCATCGGCGTGGCCGTCGCCCCGGCGAA
CGACATCTACAACGAGCGGGAGCTGCTGAACAGCATGGGGATCAGCCAGCCGACCGTGGT
GTTCGTGAGCAAGAAGGGCCTGCAGAAGATCCTGAACGTGCAGAAGAAGCTGCCCATCAT
CCAGAAGATCATCATCATGGACAGCAAGACCGACTACCAGGGCTTCCAGTCGATGTACAC
GTTCGTGACCAGCCACCTCCCGCCGGGCTTCAACGAGTACGACTTCGTCCCGGAGAGCTT
CGACCGGGACAAGACCATCGCCCTGATCATGAACAGCAGCGGCAGCACCGGCCTGCCGAA
GGGGGTGGCCCTGCCGCACCGGACCGCCTGCGTGCGCTTCTCGCACGCCCGGGACCCCAT
CTTCGGCAACCAGATCATCCCGGACACCGCCATCCTGAGCGTGGTGCCGTTCCACCACGG
CTTCGGCATGTTCACGACCCTGGGCTACCTCATCTGCGGCTTCCGGGTGGTCCTGATGTA
CCGGTTCGAGGAGGAGCTGTTCCTGCGGAGCCTGCAGGACTACAAGATCCAGAGCGCGCT
GCTCGTGCCGACCCTGTTCAGCTTCTTCGCCAAGAGCACCCTGATCGACAAGTACGACCT
GTCGAACCTGCACGAGATCGCCAGCGGGGGCGCCCCGCTGAGCAAGGAGGTGGGCGAGGC
CGTGGCCAAGCGGTTCCACCTCCCGGGCATCCGCCAGGGCTACGGCCTGACCGAGACCAC
GAGCGCGATCCTGATCACCCCCGAGGGGGACGACAAGCCGGGCGCCGTGGGCAAGGTGGT
CCCGTTCTTCGAGGCCAAGGTGGTGGACCTGGACACCGGCAAGACCCTGGGCGTGAACCA
GCGGGGCGAGCTGTGCGTGCGGGGGCCGATGATCATGAGCGGCTACGTGAACAACCCGGA
GGCCACCAACGCCCTCATCGACAAGGACGGCTGGCTGCACAGCGGCGACATCGCCTACTG
GGACGAGGACGAGCACTTCTTCATCGTCGACCGGCTGAAGTCGCTGATCAAGTACAAGGG
CTACCAGGTGGCGCCGGCCGAGCTGGAGAGCATCCTGCTCCAGCACCCCAACATCTTCGA
CGCCGGCGTGGCCGGGCTGCCGGACGACGACGCCGGCGAGCTGCCGGCCGCGGTGGTGGT
GCTGGAGCACGGCAAGACCATGACGGAGAAGGAGATCGTCGACTACGTGGCCAGCCAGGT
GACCACCGCCAAGAAGCTGCGGGGCGGCGTGGTGTTCGTGGACGAGGTCCCGAAGGGCCT
GACCGGGAAGCTCGACGCCCGGAAGATCCGCGAGATCCTGATCAAGGCCAAGAAGGGCGG
CAAGATCGCCGTGTAAGACTAGTTATAAGACTGACTAGCCCGATGGGCCTCCCAACGGGC
CCTCCTCCCCTCCTTGCACCGAGATTAATAAAAAAAAAAAAAAAAAAAAAAAAAAAAAAA
AAAAAAAAAAAAAAAAAAAAAAAAAAAAAAAAATGCATCAAAGGCTCTTTTCAGAGCCAC
CA

Figure 33

nucleotide sequence of COX6C – PpLuc(GC) – ag – A64 – histoneSL

GGAGTCAGGAAGGACGTTGGTGTTGAGGTTAGCATACGTATCAAGGACAGTAACTACCAA
GCTTGAGG*ATGGAGGACGCCAAGAACATCAAGAAGGGCCCGGCGCCCTTCTACCCGCTGG*
*AGGACGGGACCGCCGGCGAGCAGCTCCACAAGGCCATGAAGCGGTACGCCCTGGTGCCGG*
*GCACGATCGCCTTCACCGACGCCCACATCGAGGTCGACATCACCTACGCGGAGTACTTCG*
*AGATGAGCGTGCGCCTGGCCGAGGCCATGAAGCGGTACGGCCTGAACACCAACCACCGGA*
*TCGTGGTGTGCTCGGAGAACAGCCTGCAGTTCTTCATGCCGGTGCTGGGCGCCCTCTTCA*
*TCGGCGTGGCCGTCGCCCCGGCGAACGACATCTACAACGAGCGGGAGCTGCTGAACAGCA*
*TGGGGATCAGCCAGCCGACCGTGGTGTTCGTGAGCAAGAAGGGCCTGCAGAAGATCCTGA*
*ACGTGCAGAAGAAGCTGCCCATCATCCAGAAGATCATCATCATGGACAGCAAGACCGACT*
*ACCAGGGCTTCCAGTCGATGTACACGTTCGTGACCAGCCACCTCCCGCCGGGCTTCAACG*
*AGTACGACTTCGTCCCGGAGAGCTTCGACCGGGACAAGACCATCGCCCTGATCATGAACA*
*GCAGCGGCAGCACCGGCCTGCCGAAGGGGGTGGCCCTGCCGCACCGGACCGCCTGCGTGC*
*GCTTCTCGCACGCCCGGGACCCCATCTTCGGCAACCAGATCATCCCGGACACCGCCATCC*
*TGAGCGTGGTGCCGTTCCACCACGGCTTCGGCATGTTCACGACCCTGGGCTACCTCATCT*
*GCGGCTTCCGGGTGGTCCTGATGTACCGGTTCGAGGAGGAGCTGTTCCTGCGGAGCCTGC*
*AGGACTACAAGATCCAGAGCGCGCTGCTCGTGCCGACCCTGTTCAGCTTCTTCGCCAAGA*
*GCACCCTGATCGACAAGTACGACCTGTCGAACCTGCACGAGATCGCCAGCGGGGGCGCCC*
*CGCTGAGCAAGGAGGTGGGCGAGGCCGTGGCCAAGCGGTTCCACCTCCCGGGCATCCGCC*
*AGGGCTACGGCCTGACCGAGACCACGAGCGCGATCCTGATCACCCCCGAGGGGGACGACA*
*AGCCGGGCGCCGTGGGCAAGGTGGTCCCGTTCTTCGAGGCCAAGGTGGTGGACCTGGACA*
*CCGGCAAGACCCTGGGCGTGAACCAGCGGGGCGAGCTGTGCGTGCGGGGGCCGATGATCA*
*TGAGCGGCTACGTGAACAACCCGGAGGCCACCAACGCCCTCATCGACAAGGACGGCTGGC*
*TGCACAGCGGCGACATCGCCTACTGGGACGAGGACGAGCACTTCTTCATCGTCGACCGGC*
*TGAAGTCGCTGATCAAGTACAAGGGCTACCAGGTGGCGCCGGCCGAGCTGGAGAGCATCC*
*TGCTCCAGCACCCCAACATCTTCGACGCCGGCGTGGCCGGGCTGCCGGACGACGACGCCG*
*GCGAGCTGCCGGCCGCGGTGGTGGTGCTGGAGCACGGCAAGACCATGACGGAGAAGGAGA*
*TCGTCGACTACGTGGCCAGCCAGGTGACCACCGCCAAGAAGCTGCGGGGCGGCGTGGTGT*
*TCGTGGACGAGGTCCCGAAGGGCCTGACCGGGAAGCTCGACGCCCGGAAGATCCGCGAGA*
*TCCTGATCAAGGCCAAGAAGGGCGGCAAGATCGCCGTGTAA*GACTAGTTATAAGACTGAC
TAGCCCGATGGGCCTCCCAACGGGCCCTCCTCCCCTCCTTGCACCGAGATTAATAAAAAA
AAAAAAAAAAAAAAAAAAAAAAAAAAAAAAAAAAAAAAAAAAAAAAAAAAAAAAAAAA
TGCATCAAAGGCTCTTTTCAGAGCCACCA

Figure 34

nucleotide sequence of ASAH1 – PpLuc(GC) – ag – A64 – histoneSL

GGGCCTCTGCTGGAGTCCGGGGAGTGGCGTTGGCTGCTAGAGCGAAGCTTGAGGATGGAG
GACGCCAAGAACATCAAGAAGGGCCCGGCGCCCTTCTACCCGCTGGAGGACGGGACCGCC
GGCGAGCAGCTCCACAAGGCCATGAAGCGGTACGCCCTGGTGCCGGGCACGATCGCCTTC
ACCGACGCCCACATCGAGGTCGACATCACCTACGCGGAGTACTTCGAGATGAGCGTGCGC
CTGGCCGAGGCCATGAAGCGGTACGGCCTGAACACCAACCACCGGATCGTGGTGTGCTCG
GAGAACAGCCTGCAGTTCTTCATGCCGGTGCTGGGCGCCCTCTTCATCGGCGTGGCCGTC
GCCCCGGCGAACGACATCTACAACGAGCGGGAGCTGCTGAACAGCATGGGGATCAGCCAG
CCGACCGTGGTGTTCGTGAGCAAGAAGGGCCTGCAGAAGATCCTGAACGTGCAGAAGAAG
CTGCCCATCATCCAGAAGATCATCATCATGGACAGCAAGACCGACTACCAGGGCTTCCAG
TCGATGTACACGTTCGTGACCAGCCACCTCCCGCCGGGCTTCAACGAGTACGACTTCGTC
CCGGAGAGCTTCGACCGGGACAAGACCATCGCCCTGATCATGAACAGCAGCGGCAGCACC
GGCCTGCCGAAGGGGGTGGCCCTGCCGCACCGGACCGCCTGCGTGCGCTTCTCGCACGCC
CGGGACCCCATCTTCGGCAACCAGATCATCCCGGACACCGCCATCCTGAGCGTGGTGCCG
TTCCACCACGGCTTCGGCATGTTCACGACCCTGGGCTACCTCATCTGCGGCTTCCGGGTG
GTCCTGATGTACCGGTTCGAGGAGGAGCTGTTCCTGCGGAGCCTGCAGGACTACAAGATC
CAGAGCGCGCTGCTCGTGCCGACCCTGTTCAGCTTCTTCGCCAAGAGCACCCTGATCGAC
AAGTACGACCTGTCGAACCTGCACGAGATCGCCAGCGGGGGCGCCCCGCTGAGCAAGGAG
GTGGGCGAGGCCGTGGCCAAGCGGTTCCACCTCCCGGGCATCCGCCAGGGCTACGGCCTG
ACCGAGACCACGAGCGCGATCCTGATCACCCCCGAGGGGGACGACAAGCCGGGCGCCGTG
GGCAAGGTGGTCCCGTTCTTCGAGGCCAAGGTGGTGGACCTGGACACCGGCAAGACCCTG
GGCGTGAACCAGCGGGGCGAGCTGTGCGTGCGGGGGCCGATGATCATGAGCGGCTACGTG
AACAACCCGGAGGCCACCAACGCCCTCATCGACAAGGACGGCTGGCTGCACAGCGGCGAC
ATCGCCTACTGGGACGAGGACGAGCACTTCTTCATCGTCGACCGGCTGAAGTCGCTGATC
AAGTACAAGGGCTACCAGGTGGCGCCGGCCGAGCTGGAGAGCATCCTGCTCCAGCACCCC
AACATCTTCGACGCCGGCGTGGCCGGGCTGCCGGACGACGACGCCGGCGAGCTGCCGGCC
GCGGTGGTGGTGCTGGAGCACGGCAAGACCATGACGGAGAAGGAGATCGTCGACTACGTG
GCCAGCCAGGTGACCACCGCCAAGAAGCTGCGGGGCGGCGTGGTGTTCGTGGACGAGGTC
CCGAAGGGCCTGACCGGGAAGCTCGACGCCCGGAAGATCCGCGAGATCCTGATCAAGGCC
AAGAAGGGCGGCAAGATCGCCGTGTAAGACTAGTTATAAGACTGACTAGCCCGATGGGCC
TCCCAACGGCCCTCCTCCCCTCCTTGCACCGAGATTAATAAAAAAAAAAAAAAAAAAAAA
AAAAAAAAAAAAAAAAAAAAAAAAAAAAAAAAAAAAAAAAAAAATGCATCAAAGGCTCTT
TTCAGAGCCACCA

Figure 35

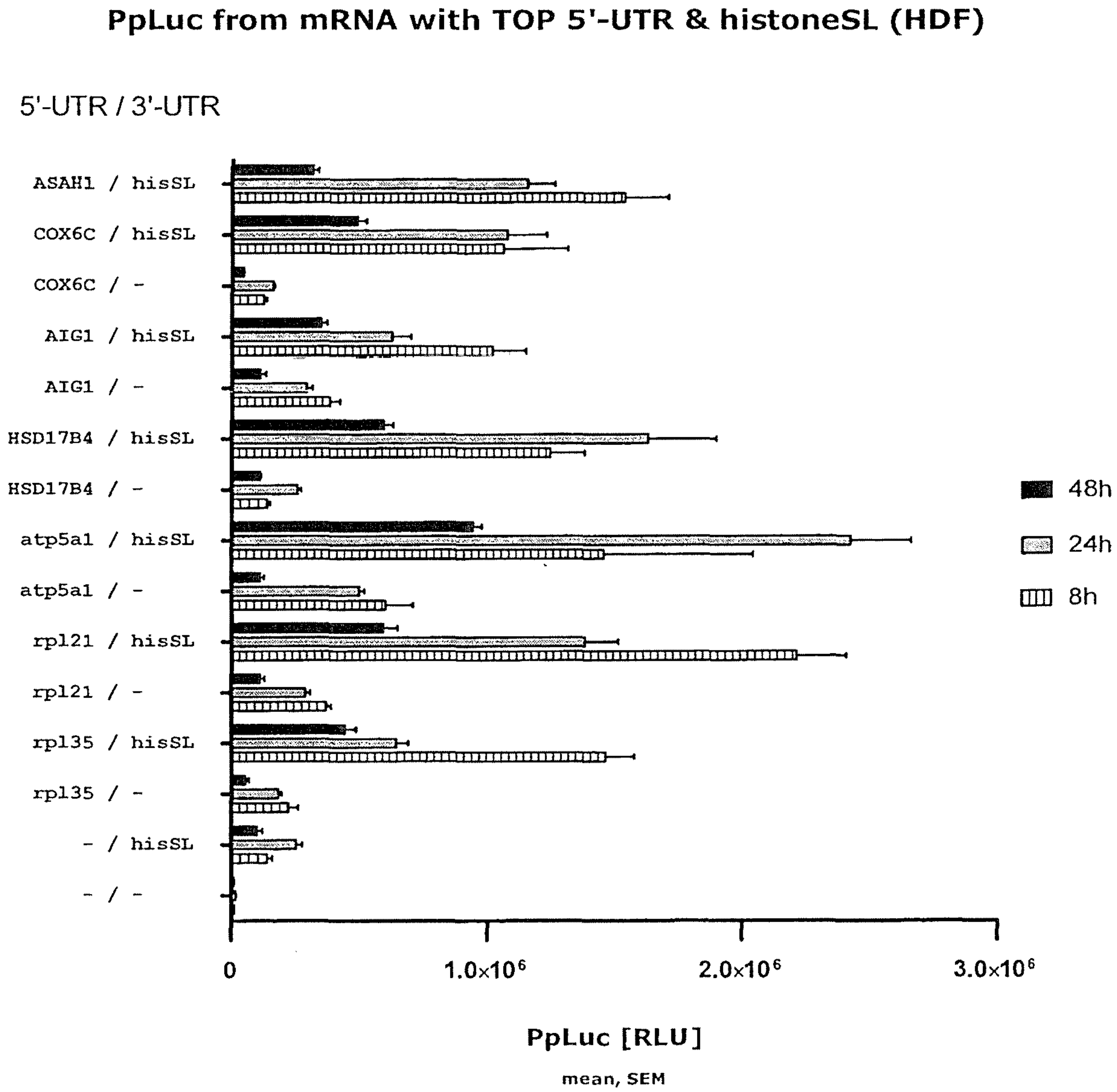
PpLuc from mRNA with TOP 5'-UTR & histoneSL (HDF)
5'-UTR / 3'-UTR
ASAH1 / hisSL
COX6C / hisSL
COX6C / -
AIG1 / hisSL
AIG1 / -
HSD17B4 / hisSL
HSD17B4 / -
atp5a1 / hisSL
atp5a1 / -
rpl21 / hisSL
rpl21 / -
rpl35 / hisSL
rpl35 / -
- / hisSL
- / -
48h
24h
8h
0
1.0×10⁶
2.0×10⁶
3.0×10⁶
PpLuc [RLU]
mean, SEM

Figure 36

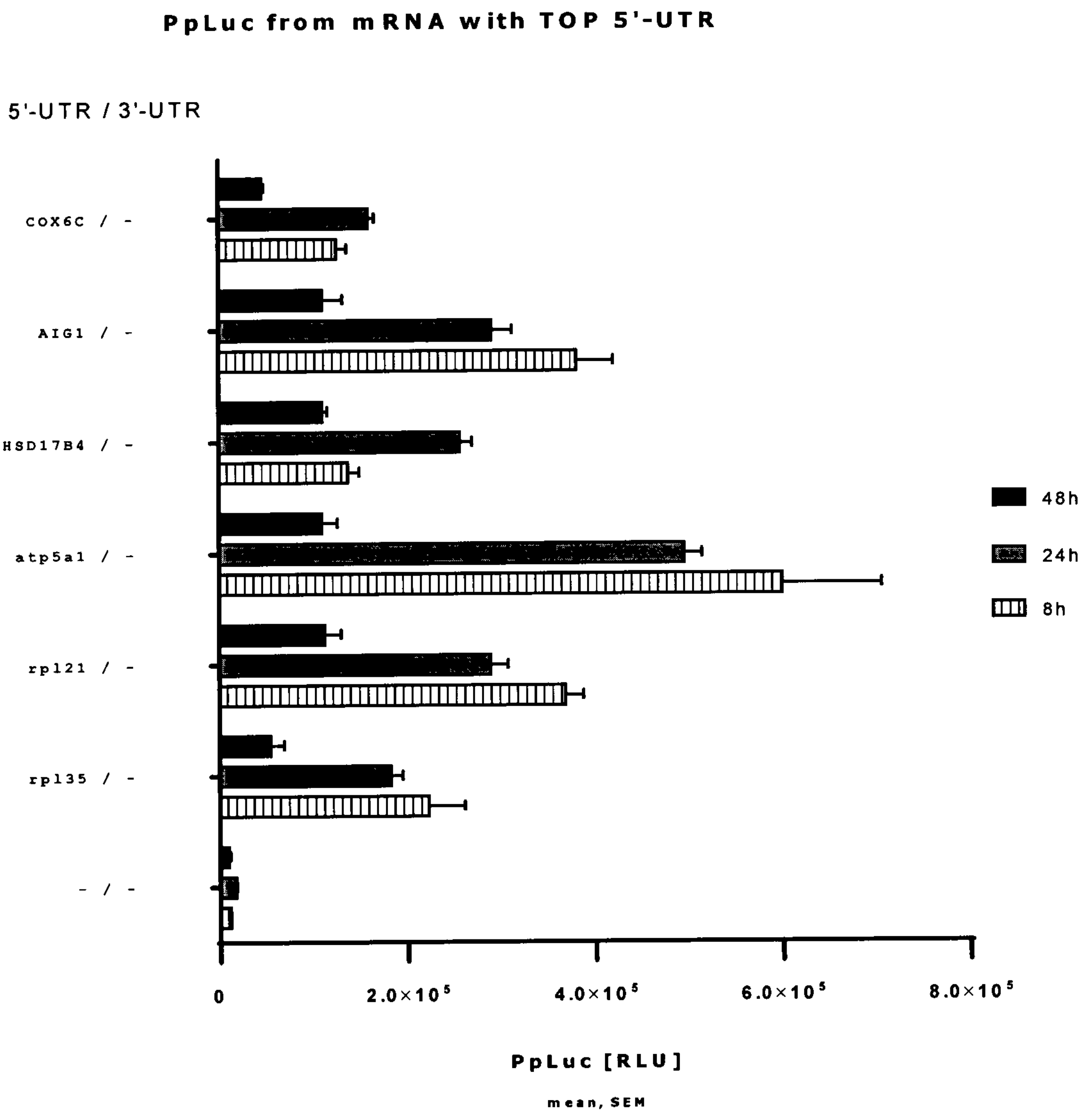
PpLuc from mRNA with TOP 5'-UTR
5'-UTR / 3'-UTR
COX6C / -
AIG1 / -
HSD17B4 / -
atp5a1 / -
rpl21 / -
rpl35 / -
- / -
48h
24h
8h
0
2.0×10⁵
4.0×10⁵
6.0×10⁵
8.0×10⁵
PpLuc [RLU]
mean, SEM

Fig. 36A

nucleotide sequence of mRPL21 – PpLuc(GC) – albumin7 – A64 – C30 – histoneSL

GGGGCCGCCGCAGCCATCTTCCAGTAACTCGCCAAAAAGCTTGAGGATGGAGGACGCCAA
GAACATCAAGAAGGGCCCGGCGCCCTTCTACCCGCTGGAGGACGGGACCGCCGGCGAGCA
GCTCCACAAGGCCATGAAGCGGTACGCCCTGGTGCCGGGCACGATCGCCTTCACCGACGC
CCACATCGAGGTCGACATCACCTACGCGGAGTACTTCGAGATGAGCGTGCGCCTGGCCGA
GGCCATGAAGCGGTACGGCCTGAACACCAACCACCGGATCGTGGTGTGCTCGGAGAACAG
CCTGCAGTTCTTCATGCCGGTGCTGGGCGCCCTCTTCATCGGCGTGGCCGTCGCCCCGGC
GAACGACATCTACAACGAGCGGGAGCTGCTGAACAGCATGGGGATCAGCCAGCCGACCGT
GGTGTTCGTGAGCAAGAAGGGCCTGCAGAAGATCCTGAACGTGCAGAAGAAGCTGCCCAT
CATCCAGAAGATCATCATCATGGACAGCAAGACCGACTACCAGGGCTTCCAGTCGATGTA
CACGTTCGTGACCAGCCACCTCCCGCCGGGCTTCAACGAGTACGACTTCGTCCCGGAGAG
CTTCGACCGGGACAAGACCATCGCCCTGATCATGAACAGCAGCGGCAGCACCGGCCTGCC
GAAGGGGGTGGCCCTGCCGCACCGGACCGCCTGCGTGCGCTTCTCGCACGCCCGGGACCC
CATCTTCGGCAACCAGATCATCCCGGACACCGCCATCCTGAGCGTGGTGCCGTTCCACCA
CGGCTTCGGCATGTTCACGACCCTGGGCTACCTCATCTGCGGCTTCCGGGTGGTCCTGAT
GTACCGGTTCGAGGAGGAGCTGTTCCTGCGGAGCCTGCAGGACTACAAGATCCAGAGCGC
GCTGCTCGTGCCGACCCTGTTCAGCTTCTTCGCCAAGAGCACCCTGATCGACAAGTACGA
CCTGTCGAACCTGCACGAGATCGCCAGCGGGGGCGCCCCGCTGAGCAAGGAGGTGGGCGA
GGCCGTGGCCAAGCGGTTCCACCTCCCGGGCATCCGCCAGGGCTACGGCCTGACCGAGAC
CACGAGCGCGATCCTGATCACCCCCGAGGGGGACGACAAGCCGGGCGCCGTGGGCAAGGT
GGTCCCGTTCTTCGAGGCCAAGGTGGTGGACCTGGACACCGGCAAGACCCTGGGCGTGAA
CCAGCGGGGCGAGCTGTGCGTGCGGGGGCCGATGATCATGAGCGGCTACGTGAACAACCC
GGAGGCCACCAACGCCCTCATCGACAAGGACGGCTGGCTGCACAGCGGCGACATCGCCTA
CTGGGACGAGGACGAGCACTTCTTCATCGTCGACCGGCTGAAGTCGCTGATCAAGTACAA
GGGCTACCAGGTGGCGCCGGCCGAGCTGGAGAGCATCCTGCTCCAGCACCCCAACATCTT
CGACGCCGGCGTGGCCGGGCTGCCGGACGACGACGCCGGCGAGCTGCCGGCCGCGGTGGT
GGTGCTGGAGCACGGCAAGACCATGACGGAGAAGGAGATCGTCGACTACGTGGCCAGCCA
GGTGACCACCGCCAAGAAGCTGCGGGGCGGCGTGGTGTTCGTGGACGAGGTCCCGAAGGG
CCTGACCGGGAAGCTCGACGCCCGGAAGATCCGCGAGATCCTGATCAAGGCCAAGAAGGG
CGGCAAGATCGCCGTGTAAGACTAGTGCATCACATTTAAAAGCATCTCAGCCTACCATGA
GAATAAGAGAAAGAAAATGAAGATCAATAGCTTATTCATCTCTTTTTCTTTTTCGTTGGT
GTAAAGCCAACACCCTGTCTAAAAAACATAAATTTCTTTAATCATTTTGCCTCTTTTCTC
TGTGCTTCAATTAATAAAAAATGGAAAGAACCTAGATCTAAAAAAAAAAAAAAAAAAAAA
AAAAAAAAAAAAAAAAAAAAAAAAAAAAAAAAAAAAAAAAAAATGCATCCCCCCCCCCCC
CCCCCCCCCCCCCCCCCCAAAGGCTCTTTTCAGAGCCACCAGAATT

Figure 37

nucleotide sequence of mRPL35A – PpLuc(GC) – albumin7 – A64 – C30 – histoneSL

GGGCCATCTTGGCGCCTGTGGAGGCCTGCTGGGAACAGGACTTCTAACAGCAAGTAAGCT
TGAGGATGGAGGACGCCAAGAACATCAAGAAGGGCCCGGCGCCCTTCTACCCGCTGGAGG
ACGGGACCGCCGGCGAGCAGCTCCACAAGGCCATGAAGCGGTACGCCCTGGTGCCGGGCA
CGATCGCCTTCACCGACGCCCACATCGAGGTCGACATCACCTACGCGGAGTACTTCGAGA
TGAGCGTGCGCCTGGCCGAGGCCATGAAGCGGTACGGCCTGAACACCAACCACCGGATCG
TGGTGTGCTCGGAGAACAGCCTGCAGTTCTTCATGCCGGTGCTGGGCGCCCTCTTCATCG
GCGTGGCCGTCGCCCCGGCGAACGACATCTACAACGAGCGGGAGCTGCTGAACAGCATGG
GGATCAGCCAGCCGACCGTGGTGTTCGTGAGCAAGAAGGGCCTGCAGAAGATCCTGAACG
TGCAGAAGAAGCTGCCCATCATCCAGAAGATCATCATCATGGACAGCAAGACCGACTACC
AGGGCTTCCAGTCGATGTACACGTTCGTGACCAGCCACCTCCCGCCGGGCTTCAACGAGT
ACGACTTCGTCCCGGAGAGCTTCGACCGGGACAAGACCATCGCCCTGATCATGAACAGCA
GCGGCAGCACCGGCCTGCCGAAGGGGGTGGCCCTGCCGCACCGGACCGCCTGCGTGCGCT
TCTCGCACGCCCGGGACCCCATCTTCGGCAACCAGATCATCCCGGACACCGCCATCCTGA
GCGTGGTGCCGTTCCACCACGGCTTCGGCATGTTCACGACCCTGGGCTACCTCATCTGCG
GCTTCCGGGTGGTCCTGATGTACCGGTTCGAGGAGGAGCTGTTCCTGCGGAGCCTGCAGG
ACTACAAGATCCAGAGCGCGCTGCTCGTGCCGACCCTGTTCAGCTTCTTCGCCAAGAGCA
CCCTGATCGACAAGTACGACCTGTCGAACCTGCACGAGATCGCCAGCGGGGGCGCCCCGC
TGAGCAAGGAGGTGGGCGAGGCCGTGGCCAAGCGGTTCCACCTCCCGGGCATCCGCCAGG
GCTACGGCCTGACCGAGACCACGAGCGCGATCCTGATCACCCCCGAGGGGGACGACAAGC
CGGGCGCCGTGGGCAAGGTGGTCCCGTTCTTCGAGGCCAAGGTGGTGGACCTGGACACCG
GCAAGACCCTGGGCGTGAACCAGCGGGGCGAGCTGTGCGTGCGGGGGCCGATGATCATGA
GCGGCTACGTGAACAACCCGGAGGCCACCAACGCCCTCATCGACAAGGACGGCTGGCTGC
ACAGCGGCGACATCGCCTACTGGGACGAGGACGAGCACTTCTTCATCGTCGACCGGCTGA
AGTCGCTGATCAAGTACAAGGGCTACCAGGTGGCGCCGGCCGAGCTGGAGAGCATCCTGC
TCCAGCACCCCAACATCTTCGACGCCGGCGTGGCCGGGCTGCCGGACGACGACGCCGGCG
AGCTGCCGGCCGCGGTGGTGGTGCTGGAGCACGGCAAGACCATGACGGAGAAGGAGATCG
TCGACTACGTGGCCAGCCAGGTGACCACCGCCAAGAAGCTGCGGGGCGGCGTGGTGTTCG
TGGACGAGGTCCCGAAGGGCCTGACCGGGAAGCTCGACGCCCGGAAGATCCGCGAGATCC
TGATCAAGGCCAAGAAGGGCGGCAAGATCGCCGTGTAAGACTAGTGCATCACATTTAAAA
GCATCTCAGCCTACCATGAGAATAAGAGAAAGAAAATGAAGATCAATAGCTTATTCATCT
CTTTTTCTTTTTCGTTGGTGTAAAGCCAACACCCTGTCTAAAAAACATAAATTTCTTTAA
TCATTTTGCCTCTTTTCTCTGTGCTTCAATTAATAAAAAATGGAAAGAACCTAGATCTAA
AAAAAAAAAAAAAAAAAAAAAAAAAAAAAAAAAAAAAAAAAAAAAAAAAAAAAAAAAAAA
AATGCATCCCCCCCCCCCCCCCCCCCCCCCCCCCCCCCAAAGGCTCTTTTCAGAGCCACC
AGAATT

Figure 38

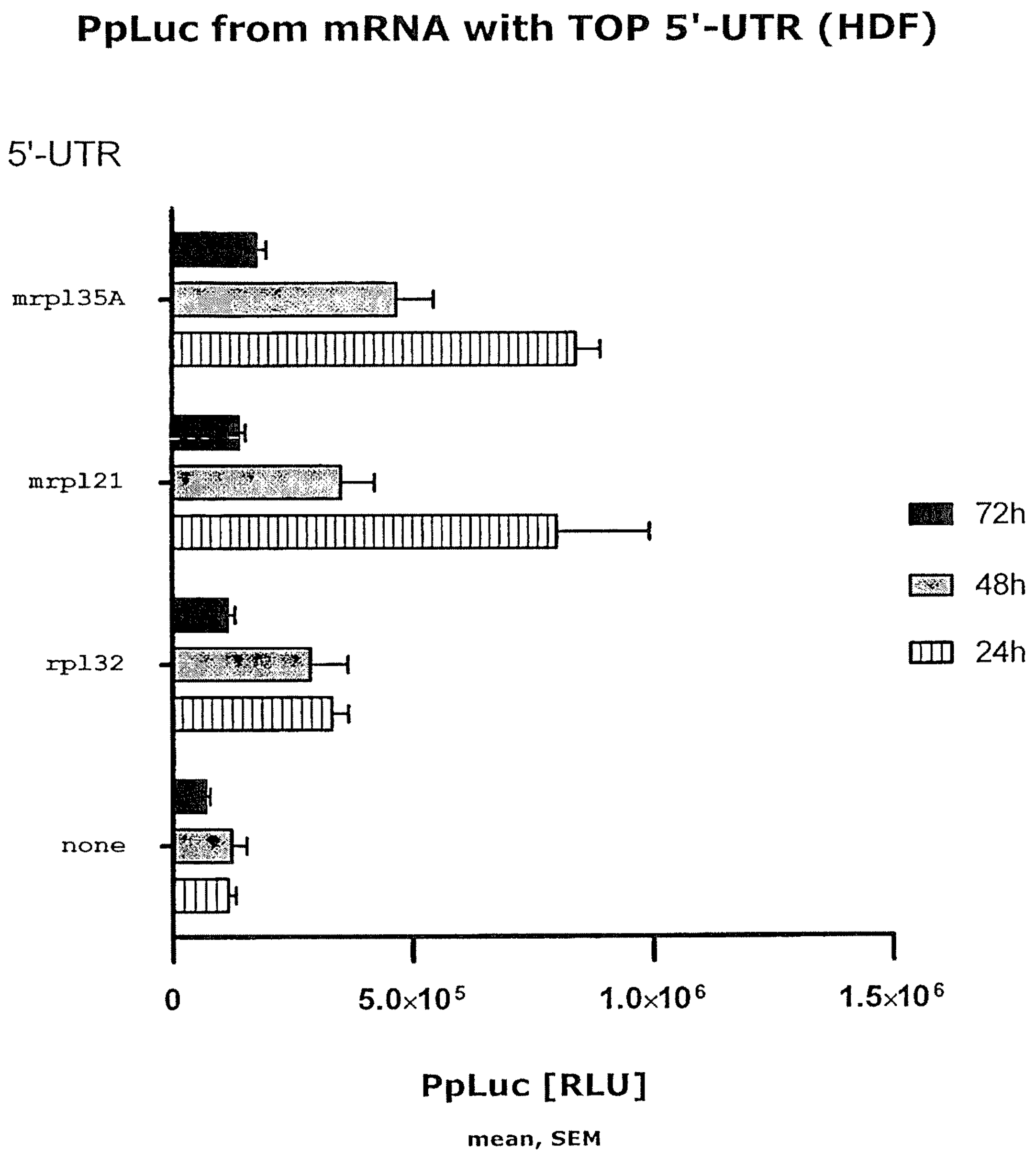
PpLuc from mRNA with TOP 5'-UTR (HDF)
5'-UTR
mrpl35A
mrpl21
rpl32
none
72h
48h
24h
0
5.0×10^5
1.0×10^6
1.5×10^6
PpLuc [RLU]
mean, SEM

Figure 39

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02098443 A
- WO 0112824 A


### Non-patent literature cited in the description


- **FRIEDEL et al.** Conserved principles of mammalian transcriptional regulation revealed by RNA half-life. *Nucleic Acid Research,* 2009, 1-12
- **JOHNSON et al.** Newly synthesized RNA: Simultaneous measurement in intact cells of transcription rates and RNA stability of insulin-like growth factor I, actin, and albumin in growth hormone-stimulated hepatocytes. *Proc. Natl. Acad. Sci.,* 1991, vol. 88, 5287-5291
- **DOMINSKI, Z. ; W. F. MARZLUFF.** *Gene,* 2007, vol. 396 (2), 373-90
- **DÁVILA LOPEZ, M. ; SAMUELSSON, T.** *RNA,* 2008, vol. 14 (1), 1-10
- **DÁVILA LÓPEZ, M. ; SAMUELSSON, T.** *RNA,* 2008, vol. 14 (1), 1-10
- **PANDEY, N. B. et al.** *Molecular and Cellular Biology,* 1994, vol. 14 (3), 1709-1720
- **WILLIAMS, A. S. ; MARZLUFF, W. F.** *Nucleic Acids Research,* 1995, vol. 23 (4), 654-662
- **SANCHEZ, R. ; W. F. MARZLUFF.** *Mol Cell Biol,* 2004, vol. 24 (6), 2513-25
- **WHITELAW, E. et al.** *Nucleic Acids Research,* 1986, vol. 14 (17), 7059-7070
- **PANDEY, N. B. ; MARZLUFF, W. F.** *Molecular and Cellular Biology,* 1987, vol. 7 (12), 4557-4559
- **PANDEY, N. B. et al.** *Nucleic Acids Research,* 1990, vol. 18 (11), 3161-3170
- **RODGERS et al.** Regulated α-globin mRNA decay is a cytoplasmic event proceeding through 3'-to-5' exosome-dependent decapping. *RNA,* 2002, vol. 8, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **CHAKRABARTI P. et al.** The Mammalian Target of Rapamycin Complex 1 Regulates Leptin Biosynthesis in Adipocytes at the Level of Translation: The Role of the 5'-Untranslated Region in the Expression of Leptin Messenger Ribonucleic Acid. *Molecular Endocrinology,* 2008, vol. 22 (10), 2260-2267
- **LADEVAIA et al.** All translation elongation factors and the e, f, and h subunits of translation initiation factor 3 are encoded by 5'-terminal oligopyrimidine (TOP) mRNAs. *RNA,* 2008, vol. 14, 1730-1736
- **AVNI et al.** Vertebrate mRNAs with a 5'-terminal pyrimidine tract are Candidates for translational repression in quiescent cells: characterization of the translational cis-regulatory element. *Molecular and Cellular Biology,* 1994, 3822-3833
- Translational Control of Ribosomal Protein mRNAs in Eukaryotes. **MEYUHAS et al.** Translational Control. Cold Spring Harbor Monograph Archive, 1996, 363-388
- **ZHU J.** Binding of the La autoantigen to the 5' untranslated region of a chimeric human translation elongation factor 1A reporter mRNA inhibits translation in vitro. *Biochemica et Biophysica Acta,* 2001, vol. 1521, 19-29
- **LEDDA, M. et al.** Effect of 3' UTR length on the translational regulation of 5'-terminal oligopyrimidine mRNAs. *Gene,* 2005, vol. 344, 213-220
- **AKASHI.** *Curr. Opin. Genet. Dev.,* 2001, vol. 11 (6), 660-666